# EUROPEAN PATENT APPLICATION

(11) **EP 2 006 271 A2**
(43) Date of publication of application: **24.12.2008**
(21) Application number: 07740229.5
(22) Date of filing: 29.03.2007
(51) Int. Cl.: C07C 1/00

(54) **SUBSTITUTED BICYCLIC CYCLIC DERIVATIVE AND USE THEREOF**

(30) Priority: 30.03.2006 JP 2006095008
(71) Applicant: Asahi Kasei Pharma Corporation, Tokyo 101-8101 (JP)
(72) Inventor: MATSUMOTO, Akiko, Tokyo 100-8440 (JP); SHODA, Motoshi, Tokyo 100-8440 (JP); KURIYAMA, Hiroshi, Tokyo 100-8440 (JP)
(74) Representative: Forstmeyer, Dietmar
(86) International application number: PCT/JP2007/056791
(87) International publication number: WO 2007/114213

(57) **Abstract**

[Object] To provide a compound having prostaglandin production-suppressing action and leukotriene production-suppressing action.

[Means for solution] A compound represented by the formula (I): 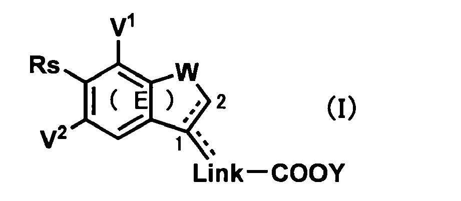 [In the formula, ---- represents a single bond, or a double bond, Link represents a single bond, or a saturated or unsaturated straight hydrocarbon having 1 or 2 carbon atoms, W represents a single bond, oxygen atom, sulfur atom, N(Rw) etc., Rw represents hydrogen atom, an alkyl group having 1 to 8 carbon atoms etc, Rs represents -D-Rx etc., D represents a single bond, oxygen atom, sulfur atom etc., Rx represents a linear or branched saturated alkyl group having 3 to 8 carbon atoms etc., one of V¹ and V² represents Zx, and the other represents AR, Zx represents hydrogen atom, a linear or branched saturated alkyl group having 1 to 4 carbon atoms etc., AR represents a partially unsaturated or completely unsaturated condensed bicyclic carbon ring or heterocyclic ring, and Y represents hydrogen atom, a lower alkyl group having 1 to 4 carbon atoms etc.], or a salt thereof.

## Description

### Field of the Invention

The present invention relates to a novel substituted bicyclic derivative. More specifically, the present invention relates to a substituted bicyclic derivative useful as an active ingredient of medicaments.

### Background Art

Various kinds of prostaglandins and various kinds of leukotrienes are produced in the bodies of mammals in response to variety of stimuli such as inflammatory stimuli and physical stimuli.
Both of prostaglandins and leukotrienes are metabolites of arachidonic acid, and they are physiologically active substances referred to as lipid mediators, and they cause various physiological responses of mammals by binding to receptors expressed on surfaces of various cells or in the cells.
Arachidonic acid is produced from a phospholipid as a substrate, such as phosphatidylcholine which is a cell membrane component, with the aid of an enzymatic activity of phospholipase A₂ (PLA₂).

Arachidonic acid produced by the action of PLA₂ is converted into prostaglandin (PG) H₂ with the aid of an enzymatic activity of constitutive type cyclooxygenase (COX) 1 or inducible type COX-2, and further converted into PGE₂, PGD₂, PGF₂α, PGI₂, thromboxane (TX) A₂ and the like with the aid of each synthetic enzyme. Further, arachidonic acid is also metabolized by the action of 5-lipoxygenase (5-LO) and thereby converted to leukotriene (LT) A₄, and further converted to LTB₄, LTC₄, LTD₄, LTE₄ and the like by the enzymatic activities of LTA₄ hydrolase, LTC₄ synthase, glutathione S-transferase and the like [Goodman & Gilman, Pharmacological Basis of Therapeutics, 9th edition, p.801, 1999 (Hirokawa Shoten); Funk, C.D., SCIENCE, vol. 294, p.1871, 2001].

Prostaglandins bind to each specific receptor to cause inflammatory reactions such as fervescence, enhancement of vascular permeability, vasodilation, swelling and pain, bronchial smooth muscle contraction, platelet aggregation, tumor cell proliferation, enhancement of bone resorption, nerve cell degeneration and the like, and thus play important roles in expression of symptoms or pathological formation for various diseases.
Leukotrienes are physiologically active substances which bind to each specific receptor to cause inflammatory reactions such as excessive accumulation of leucocytes and enhancement of vascular permeability, smooth muscle contraction, mucus secretion, proliferation of tumor cells and the like, and thus play important roles in expression of symptoms or pathological formation for various diseases.

Although inflammatory reactions themselves are essential reactions for living bodies to survive when they face pathogenic substances and affections, they are sometimes excessively caused or continue without any reason for providing evident benefit under certain situations or in certain diseases [Goodman & Gilman, Pharmacological Basis of Therapeutics, 9th edition, p.827, 1999 (Hirokawa Shoten)]. The condition of living body referred to in this specification wherein an acute or chronic inflammatory reaction is observed means a condition that an excessive or unbeneficial acute or temporary inflammatory reaction or chronic and persistent inflammatory reaction is caused. Further, an inflammatory reaction refers to a series of events caused by stimuli, for example, physical hazards such as heat, infective substances, ischemia, antigen/antibody reaction and the like, and it is accompanied by flare, swelling, hyperalgesia, algesic onset and the like as well-known macroscopic clinical symptoms. It is known that, as histological mechanisms for these reactions, vasodilation, enhancement of vascular permeability, infiltration of leucocytes and phagocytes, histological decomposition and fibrosing and the like are caused [Goodman & Gilman, Pharmacological Basis of Therapeutics, 9th edition, p.827, 1999 (Hirokawa Shoten)]. It is known that many of these histological reactions are caused by prostaglandins and/or leukotrienes, and prostaglandins and/or leukotrienes plays important roles in inflammatory reactions.

For example, it was reported that, in a pathological tissue of rheumatoid arthritis, which is an autoimmune and chronic inflammatory disease, expression of COX-2 and production of PGE₂ and TXA₂ as well as expression of 5-LO and production of LTB₄ were observed (Bonnet et al., Prostaglandins, 1995, vol. 50, p.127), and in a mouse deficient in FLAP, which is a protein required for activation of 5-LO, symptom of collagen-induced arthritis, which is a pathological model of chronic rheumatoid arthritis, was milder compared with that in a wild-type mouse (Griffiths et al., J. Exp. Med., 1997, vol. 185, p.1123), and thus it has been suggested that prostaglandins and leukotrienes play important roles in the pathological formation of chronic rheumatoid arthritis.

It was reported that, in a pathological tissue of bronchial asthma, one of chronic allergic diseases, excessive production of PGD₂ and TXA₂ as well as excessive production of LTC₄ and LTD₄ were observed (Wenzel et al., Am Rev. Respir. Dis., 1990, vol. 142, p.112), and an airway hypersensitive reaction, which is a pathological model of bronchial asthma, was unlikely to occur in a PGD₂ receptor-deficient mouse (Matsuoka et al., SCIENCE, vol. 287, p.2013, 2000). Thus, it has been demonstrated that roles of prostaglandins and leukotrienes are important in bronchial asthma.

In a cerebral tissue after ischemic reperfusion, expression of COX-2 increased, and concentrations of PGE₂ and TXA₂ increased, whereas activity of 5-LO increased, and production amount of LTC₄ increased (Ohtsuki et al., Am. J. Physiol., 1995, vol. 268, p.1249). Thus, it is known that prostaglandins and leukotrienes play important roles in formation of infarct that is accepted as an ischemic reperfusion injury.
It has been revealed that, in a pathological tissue of Alzheimer's disease, one of the diseases with neurodegeneration, the COX activity and 5-LO activity increased, prostaglandins and leukotrienes cause formation of the β-amyloid protein, one of the pathogenic substances of Alzheimer's disease, and further cause degeneration of nerve cells (Sugaya et al., Jpn. J. Pharmacol., 2000, vol. 82, p.85), and thus it is believed that prostaglandins and leukotrienes play important roles in the formation of neurodegenerative diseases such as Alzheimer's disease.

Furthermore, for example, it was reported that, in a pathological tissue of colon cancer, COX and 5-LO were expressed, and amounts of production of prostaglandins and leukotrienes were increased (Dreyling et al., Biochim. Biophys. Acta., 1986, vol. 878, p. 184), and leukotriene caused increase in colon cancer cells (Qiao et al., Biochim. Biophys. Acta, 1995, vol. 1258, p.215; Hong et al., Cancer Res., 1999, vol. 59, p.2223). Thus, it is believed that prostaglandins and leukotrienes play important roles also in tissues of large bowel cancer.
Involvement of prostaglandins and/or leukotrienes in diseases and pathological conditions is not limited to those diseases exemplified above, and it has been demonstrated that prostaglandins and/or leukotrienes are involved in variety of conditions, various diseases, or various pathological conditions where acute or chronic inflammatory reactions are observed and their roles are important.

For the above reason, various prostaglandin production suppressors or leukotriene production suppressors are used as agents for prophylactic or therapeutic treatment of conditions, various diseases or pathological conditions where an acute or chronic inflammatory reaction is recognized. Various non-steroidal antiinflammatory drugs (NSAIDS) as medicaments having a prostaglandin production-suppressing action are available and used as therapeutic agents for chronic rheumatoid arthritis and osteoarthritis, antiphlogistic- analgesic agents for injury and the like, prophylactic agents for cerebral infarction or myocardial infarction, prophylactic agents for colon polyposis and the like. However, the class of NSAIDS suppress only production of prostaglandins, and as a result, they increase amounts of production of leukotrienes, and exhibit side effects such as asthmatic attack and gastrointestinal injury as well as side effects such as renal disturbance.
Furthermore, a difference between an effective dose and a dose inducing the side effects is small in these NSAIDS, and no satisfactory agent is available from a viewpoint of therapeutic effect. A 5-LO inhibitor is available which is described in European Patent No. 279263 as a medicament having a leukotriene production-suppressing action, and the inhibitor is known as a prophylactic agent for asthma. However, since the agent causes side effects such as hepatic disorder, its dosage is limited, and the agent is not satisfactory also from a viewpoint of therapeutic effect. Since steroid agents suppress production of both of prostaglandins and leukotrienes, they are used as prophylactic agents or therapeutic agents for conditions of living bodies, various diseases and pathological conditions where various acute or chronic inflammatory reactions are observed. However, their actions are not limited to the lipid mediator production-suppressing action, and they exhibit severe side effects such as induction and exacerbation of infectious diseases due to the immunosuppression action, growth retardation due to normal cell antiproliferative activity, anetoderma and peptic ulcer. Therefore, their uses are limited.

Furthermore, for the above reasons, it is considered that compounds, that suppress the production of both of prostaglandins and leukotrienes and have reduced side effect, are effective as therapeutic agents or prophylactic agents for such conditions of living bodies, diseases or pathological conditions in mammals as described above, and methods of using such compounds together with medicaments available at present are more effective therapeutic or prophylactic methods. Therefore, development of compounds suppressing the production of both of prostaglandins and leukotrienes, and manufacture of pharmaceutical preparations thereof are strongly desired.

As compounds structurally similar to the compounds of the present invention, for example, biphenyl-5-alkanoic acid derivatives and use thereof are reported in WO99/19291. However, the moieties of these compounds that correspond to the ring (E) and "AR" included in the formula (I) of the compounds of the present invention are phenyl groups, and thus structural features of the above compounds are different. Further, biaryl phospholipase A₂ inhibitors are described in U.S. Patent No. 5,391,817 [Japanese Patent Unexamined Publication (Kokai) No. 7-22399]. However, the moieties of these compounds that correspond to the ring (E) and "AR" included in the formula (I) of the compounds of the present invention are defined only to be phenyl group, and thus the structural features of the above compounds are different.
Bicyclic heterocyclic compounds are reported in WO00/35886 as protease inhibitors. However, the moieties of these compounds that correspond to the ring (E) and the substituents on "AR" included in the formula (I) of the compounds of the present invention are different, and further, the publication is completely silent about whether or not the compounds disclosed in the above patent document have any prostaglandin production-suppressing action or leukotriene production-suppressing action. Indanacetic acid derivatives are reported in WO03/011842, and indane-, dihydrobenzofuran-, and tetrahydronaphthalene-carboxylic acid derivatives are reported in WO04/011446. However, the moieties of these compounds that correspond to "AR" included in the formula (I) of the compounds of the present invention are different, and further, the publication is completely silent about whether or not the compounds disclosed in the above patent specifications have any prostaglandin production-suppressing action or leukotriene production-suppressing action. Moreover, substituted phenylalkanoic acid derivatives and uses thereof are reported in WO03/07686. However, the moieties of these compounds that correspond to the ring (E) included in the formula (I) of the compounds of the present invention are limited to benzene ring, and thus structural features thereof are different. Substituted arylalkanoic acid derivatives and uses thereof are reported in WO05/016862. However, the moieties of these compounds that correspond to the ring (E) included in the formula (I) of the compounds of the present invention are limited to benzene ring and pyridine ring, and thus structural features thereof are different.
Patent document 1: WO99/19291
Patent document 2: U.S. Patent No. 5,391,817
Patent document 3: WO00/35886
Patent document 4: WO03/011842
Patent document 5: WO04/011446
Patent document 6: WO03/07686
Patent document 7: WO05/016862

### Disclosure of the Invention

### Object to be Achieved by the Invention

An object of the present invention is to provide a novel compound having superior prostaglandin production-suppressing action and leukotriene production-suppressing action. Another object of the present invention is to provide a compound for prophylactic and/or therapeutic treatment of various inflammatory diseases, autoimmune diseases, allergic diseases, pain and fibrosis in mammals caused by lipid mediators. A further object of the present invention is to provide a pharmaceutical composition containing such a compound.

### Means for Achieving the Object

In order to achieve the aforementioned objects, the inventors of the present invention conducted various researches. As a result, they found that the substituted bicyclic derivatives represented by the following general formula, which are novel compounds, had superior prostaglandin production-suppressing action and leukotriene production-suppressing action, and thus accomplished the present invention.
The present invention thus relates to the followings.
<1> A compound represented by the formula (I): 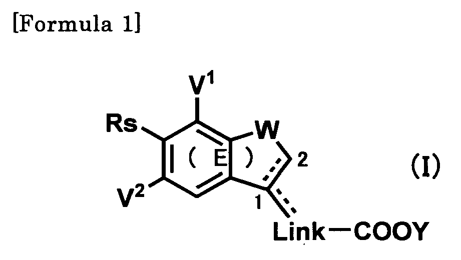 [In the formula, ---- represents a single bond or a double bond (provided that both the bond between the ring-constituting carbon atom at the 1-position of the ring (E) and the ring-constituting carbon atom at the 2-position of the ring (E), and the bond between the ring-constituting carbon atom at the 1-position of the ring (E) and the Link moiety do not simultaneously represent a double bond, and when Link represents a single bond, the bond between the ring-constituting carbon atom at the 1-position of the ring (E) and the Link moiety represents a single bond);
   Link represents a single bond, or a saturated or unsaturated straight hydrocarbon having 1 or 2 carbon atoms ;
   W represents a single bond, methylene group, oxygen atom, sulfur atom, or N(Rw);
   Rw represents hydrogen atom, an alkyl group having 1 to 8 carbon atoms, or - A⁶-Qp;
   A⁶ represents a single bond or methylene group;
   Qp represents phenyl group, and the phenyl group may be substituted with one of T¹ or two or more of the same or different T¹;
   T¹ represents a linear or branched saturated alkyl group having 1 to 4 carbon atoms, hydroxyl group, fluorine atom, chlorine atom, bromine atom, trifluoromethyl group, nitro group, an alkoxyl group having 1 to 4 carbon atoms, or a mono- or dialkylamino group having 1 to 4 carbon atoms;
   Rs represents -D-Rx or -N(Ry)(Rz);
   D represents a single bond, oxygen atom, sulfur atom, -S(O)-, -S(O)₂-, or - C(O)-;
   Rx represents a linear or branched saturated alkyl group having 3 to 8 carbon atoms, or represents Ra represented by the following formula:

   R¹-A^{a-} (Ra)

   Rb represented by the following formula: 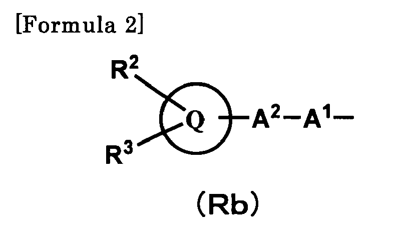 or Rc represented by the following formula: 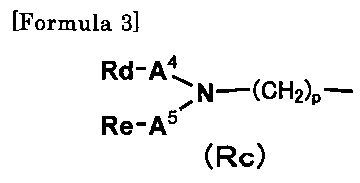 A^{a} in Ra represents a single bond, an alkylene (aa) having 1 to 3 carbon atoms, or an alkenylene (aa') having 2 or 3 carbon atoms, and the alkylene (aa) and the alkenylene (aa') may be independently substituted with a lower alkyl group having 1 to 4 carbon atoms;
   R¹ represents a saturated cyclic alkyl group having 3 to 7 carbon atoms or a condensed saturated cyclic alkyl group having 6 to 8 carbon atoms, and R¹ may be substituted with one of lower alkyl group having 1 to 4 carbon atoms or two or more of the same or different lower alkyl groups having 1 to 4 carbon atoms;
   Q in Rb represents a partially unsaturated or completely unsaturated monocyclic or condensed bicyclic carbon ring or a heterocyclic ring (q), and binds to A² at an arbitrary position, and the heterocyclic ring (q) contains 1 to 4 of the same or different ring-constituting heteroatoms selected from the group consisting of nitrogen atom, oxygen atom, and sulfur atom;
   A¹ represents a single bond, an alkylene (al) having 1 to 3 carbon atoms, or an alkenylene (a1') having 2 or 3 carbon atoms, and the alkylene (a1) and the alkenylene (a1') may be independently substituted with a lower alkyl group having 1 to 4 carbon atoms or phenyl group;
   A² represents a single bond, oxygen atom, sulfur atom, -S(O)-, -S(O)₂-, or - N(R⁴)-(provided that when A² represents oxygen atom, sulfur atom, -S(O)-, -S(O)₂., or -N(R⁴)-, A¹ represents ethylene or trimethylene);
   R² and R³ independently represents hydrogen atom, a linear or branched saturated alkyl group having 1 to 4 carbon atoms, oxo group, thioxo group, fluorine atom, chlorine atom, bromine atom, trifluoromethyl group, -OR⁵, -N(R⁶)(R⁶'), - NHCOR⁷, -NHSO₂R⁸, or -A⁶-Qa, or they bind to each other to represent methylenedioxy group;
   Qa represents a partially unsaturated or completely unsaturated monocyclic or condensed bicyclic carbon ring or heterocyclic ring (qa), binds to A⁶ at an arbitrary position on the ring, and may be substituted with one of T¹ or two or more of the same or different T¹, and the heterocyclic ring (qa) contains 1 to 4 of the same or different ring-constituting heteroatoms selected from the group consisting of nitrogen atom, oxygen atom, and sulfur atom;
   R⁴ and R⁶ independently represent hydrogen atom or a lower alkyl group having 1 to 4 carbon atoms;
   R⁵ and R⁷ independently represent hydrogen atom, a lower alkyl group having 1 to 4 carbon atoms, or -A⁶-Qa;
   R⁸ represents a lower alkyl group having 1 to 4 carbon atoms;
   R⁶ has the same meaning as that of R⁶, or binds to R⁶ to form a 3- to 6-membered ring together with the nitrogen atom to which they bind to represent a saturated nitrogen-containing cycloalkyl group, or morpholino group;
   symbol p in Rc represents an integer of 2 to 4;
   A⁴ represents a single bond, methylene, or ethylene;
   A⁵ represents -C(O)-, -C(S)-, or -S(O)₂-;
   Rd represents hydrogen atom, an alkyl group having 1 to 8 carbon atoms, or Qa;
   Re represents an alkyl group having 1 to 8 carbon atoms, -A⁶-Qa, -(CH₂)ᵢR¹⁴, - OR²⁸, -SR²⁸, or -N(R²⁹)(R³⁰);
   symbol i represents an integer of 1 to 3;
   R¹⁴ represents hydroxyl group, an alkoxyl group having 1 to 4 carbon atoms, carboxyl group, or an N,N-dialkylcarbamoyl group having 1 to 4 carbon atoms;
   R²⁸ represents an alkyl group having 1 to 8 carbon atoms or -A⁶-Qa;
   R²⁹ represents an alkyl group having 1 to 8 carbon atoms, an alkoxycarbonyl group having 1 to 4 carbon atoms, or -A⁶-Qa;
   R³⁰ represents hydrogen atom or a lower alkyl group having 1 to 4 carbon atoms, or binds to R²⁹ to form a 3- to 6-membered ring together with the nitrogen atom to which they bind to represent a saturated nitrogen-containing cycloalkyl group, or morpholino group;
   Rz has the same meaning as that of Rx, or represents methyl group, ethyl group, or -A⁵-Re;
   Ry represents hydrogen atom, an alkyl group having 1 to 8 carbon atoms, or -A⁶-Qp represents, or binds to Rz to form, together with the nitrogen atom to which they bind, a saturated or unsaturated cyclic substituent (qy) having 3 to 7 atoms, the cyclic substituent (qy) may contain one of nitrogen atom, oxygen atom, or sulfur atom besides the nitrogen atom, and the cyclic substituent (qy) may further be substituted with one of Rf or two of the same or different Rf;
   Rf represents an alkyl group having 1 to 8 carbon atoms or -A⁶-Qp;
   one of V¹ and V² represents Zx, and the other represents AR;
   Zx represents hydrogen atom, a linear or branched saturated alkyl group having 1 to 4 carbon atoms, fluorine atom, chlorine atom, bromine atom, nitro group, - OR⁹, or -N(Rn¹)(Rn²);
   R⁹ represents hydrogen atom, a lower alkyl group having 1 to 4 carbon atoms, or -A⁶-Qp;
   Rn¹ represents hydrogen atom or a linear or branched saturated alkyl group having 1 to 4 carbon atoms;
   Rn² has the same meaning as that of Rn¹, or represents -COR²³ or -SO₂R²⁴, or binds to Rn¹ to form a 3- to 6-membered ring together with the nitrogen atom to
   which they bind to form a saturated nitrogen-containing cycloalkyl group, or morpholino group;
   R²³ represents hydrogen atom, a lower alkyl group having 1 to 4 carbon atoms, a lower alkoxyl group having 1 to 4 carbon atoms, -O-A⁶-Qp, or -N(R²⁵)(R²⁶);
   R²⁵ represents hydrogen atom or a linear or branched saturated alkyl group having 1 to 4 carbon atoms;
   R²⁶ has the same meaning as that of R²⁵, or binds to R²⁵ to form a 3- to 6-membered ring together with the nitrogen atom to which they bind to form a saturated nitrogen-containing cycloalkyl group, or morpholino group;
   R²⁴ represents a lower alkyl group having 1 to 4 carbon atoms, amino group, or a mono- or dialkylamino group having 1 to 4 carbon atoms;
   AR represents a partially unsaturated or completely unsaturated condensed bicyclic carbon ring or heterocyclic ring (ar), and may be substituted with one of Xa or two or more of the same or different Xa;
   the heterocyclic ring (ar) contains 1 to 4 of the same or different ring-constituting heteroatoms selected from the group consisting of nitrogen atom, oxygen atom, and sulfur atom;
   Xa represents a linear or branched saturated alkyl group having 1 to 4 carbon atoms, a saturated cyclic alkyl group having 3 to 7 carbon atoms, oxo group, thioxo group, fluorine atom, chlorine atom, trifluoromethyl group, -(CH₂)ᵢR¹⁴, -OR¹⁰, - N(R¹¹)(R¹²), -SO₂R¹³, or -COR²⁷;
   R¹⁰ represents hydrogen atom, a lower alkyl group having 1 to 4 carbon atoms or -(CH₂)ᵢR¹⁴;
   R¹¹ represents hydrogen atom or a lower alkyl group having 1 to 4 carbon atoms;
   R¹² represents hydrogen atom, a lower alkyl group having 1 to 4 carbon atoms, a hydroxyalkyl group having 2 to 4 carbon atoms, -COR¹⁵, or -SO₂R¹⁶, or binds to R¹¹ to form a 3- to 6-membered ring together with the nitrogen atom to which they bind to represent a saturated nitrogen-containing cycloalkyl group, or morpholino group;
   R¹⁵ represents a lower alkyl group having 1 to 4 carbon atoms, a hydroxyalkyl group having 2 to 4 carbon atoms, amino group, a mono- or dialkylamino group having 1 to 4 carbon atoms, or -A⁶-Qa;
   R¹³ and R¹⁶ independently represent a lower alkyl group having 1 to 4 carbon atoms, amino group, or a mono- or dialkylamino group having 1 to 4 carbon atoms;
   R²⁷ represents hydrogen atom, hydroxyl group, an alkoxyl group having 1 to 4 carbon atoms, a lower alkyl group having 1 to 4 carbon atoms, amino group, or a mono- or dialkylamino group having 1 to 4 carbon atoms;
   Y represents hydrogen atom, a lower alkyl group having 1 to 4 carbon atoms, -(CH₂)ₘN(R¹⁸)(R¹⁹), or -C(R²⁰)₂OC(O)A³R²¹;
   symbol m represents an integer of 2 or 3;
   R¹⁸ has the same meaning as that of R¹⁹, or binds to R¹⁹ to form a 3- to 6-membered ring together with the nitrogen atom to which they bind to represent a saturated nitrogen-containing cycloalkyl group, or morpholino group;
   R¹⁹ represents methyl group, ethyl group, or propyl group;
   R²⁰ represents hydrogen atom, methyl group, ethyl group, or propyl group;
   R²¹ represents a lower alkyl group having 1 to 4 carbon atoms, a saturated cyclic alkyl group having 3 to 6 carbon atoms, or phenyl group; and
   A³ represents a single bond or oxygen atom] (this compound may sometimes be hereinafter referred to simply as "Compound (I)" of the present invention"), or a salt thereof.

<2> The compound or salt thereof according to <1> mentioned above, wherein, in the formula (I), V¹ is Zx, and V² is AR.
<3> The compound or salt thereof according to <1> or <2> mentioned above, wherein, in the formula (I), W is methylene group.
<4> The compound or salt thereof according to <1> or <2> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond or a double bond;
   when the bond between the ring-constituting carbon atom at the 1-position of the ring (E) and the Link moiety is a single bond, Link is CH₂ or CH₂CH₂, or when the bond between the ring-constituting carbon atom at the 1-position of the ring (E) and the Link moiety is a double bond, Link is CH; and W is methylene group.
<5> The compound or salt thereof according to <1> or <2> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond or a double bond;
   when the bond between the ring-constituting carbon atom at the 1-position of the ring (E) and the Link moiety is a single bond, Link is CH₂, or when the bond between the ring-constituting carbon atom at the 1-position of the ring (E) and the Link moiety is a double bond, Link is CH; and
   W is methylene group.

<6> The compound or salt thereof according to <1> or <2> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond;
   Link is CH₂ and
   W is methylene group.
<7> The compound or salt thereof according to <1> or <2> mentioned above, wherein, in the formula (I), the bond between the ring-constituting carbon atom at the 1-position of the ring (E) and the ring-constituting carbon atom at the 2-position of the ring (E) is a double bond; the bond between the ring-constituting carbon atom at the 1-position of the ring (E) and the Link moiety is a single bond;
   Link is CH₂, or CH₂CH₂; and
   W is oxygen atom.
<8> The compound or salt thereof according to <1> or <2> mentioned above, wherein, in the formula (I), the bond between the ring-constituting carbon atom at the 1-position of the ring (E) and the ring-constituting carbon atom at the 2-position of the ring (E) is a double bond;
   the bond between the ring-constituting carbon atom at the 1-position of the ring (E) and the Link moiety is a single bond;
   Link is CH₂, or CH₂CH₂; and
   W is NH or N(methyl).

<9> The compound or salt thereof according to any one of <1> to <8> mentioned above, wherein, in the formula (I), AR as V¹ or V² is a residue of naphthalene, benzofuran, benzo[b]thiophene, indole, benzothiazole, dihydro-3H-benzothiazole, quinoline, dihydro-1H-quinoline, benzo[d]isothiazole, 1H-indazole, benzo[c]isothiazole, 2H-indazole, imidazo[1,2-a]pyridine, 1H-pyrrolo[2,3-b]pyridine, isoquinoline, dihydro-2H-isoquinoline, cinnoline, quinazoline, quinoxaline, 1H-benzimidazole, benzoxazole, 1H-pyrrolo[3,2-b]pyridine, benzo[1,2,5]thiadiazole, 1H-benzotriazole, 1,3-dihydropyrrolo[2,3-b]pyridine, 1,3-dihydrobenzimidazole, dihydro-3H-benzoxazole, phthalazine, [1,8]naphthylidine, [1,5]naphthylidine, 1H-pyrrolo[3,2-c]pyridine, 1H-pyrrolo[2,3-c]pyridine, 1H-pyrazolo[4,3-b]pyridine, 1H-pyrazolo[4,3-c]pyridine, 1H-pyrazolo[3,4-c]pyridine, 1H-pyrazolo[3,4-b]pyridine, [1,2,4]triazolo[4,3-a]pyridine, thieno[3,2-c]pyridine, thieno[3,2-b]pyridine, 1H-thieno[3,2-c]pyrazole, benzo[d]isoxazole, benzo[c]isoxazole, indolizine, 1,3-dihydroindole, 1H-pyrazolo[3,4-d]thiazole, 2H-isoindole, [1,2,4]triazolo[1,5-a]pyrimidine, 1H-pyrazolo[3,4-b]pyrazine, 1H-imidazo[4,5-b]pyrazine, 7H-purine, or 4H-chromene ring (the aforementioned residue may be substituted with one of Xa or two or more of the same or different Xa).

<10> The compound or salt thereof according to any one of <1> to <8> mentioned above, wherein, in the formula (I), AR as V¹ or V² is naphthalen-2-yl group, naphthalen-1-yl group, benzofuran-5-yl group, benzofuran-4-yl group, benzofuran-2-yl group, benzo[b]thiophen-5-yl group, benzo[b]thiophen-4-yl group, benzo[b]thiophen-2-yl group, indol-5-yl group, indol-4-yl group, indol-6-yl group, benzothiazol-6-yl group, benzothiazol-7-yl group, benzothiazol-5-yl group, benzothiazol-4-yl group, dihydro-3H-benzothiazol-6-yl group, dihydro-3H-benzothiazol-7-yl group, dihydro-3H-benzothiazol-5-yl group, dihydro-3H-benzothiazol-4-yl group, quinolin-6-yl group, quinolin-3-yl group, quinolin-5-yl group, quinolin-7-yl group, dihydro-1H-quinolin-6-yl group, dihydro-1H-quinolin-5-yl group, benzo[d]isothiazol-5-yl group, benzo[d]isothiazol-4-yl group, benzo[d]isothiazol-6-yl group, benzo[d]isothiazol-7-yl group, 1H-indazol-5-yl group, 1H-indazol-4-yl group, 1H-indazol-6-yl group, benzo[c]isothiazol-5-yl group, benzo[c]isothiazol-4-yl group, benzo[c]isothiazol-6-yl group, benzo[c]isothiazol-7-yl group, 2H-indazol-5-yl group, 2H-indazol-4-yl group, 2H-indazol-6-yl group, imidazo[1,2-a]pyridin-6-yl group, imidazo[1,2-a]pyridin-7-yl group, 1H-pyrrolo[2,3-b]pyridin-5-yl group, 1H-pyrrolo[2,3-b]pyridin-4-yl group, isoquinolin-6-yl group, isoquinolin-3-yl group, isoquinolin-5-yl group, isoquinolin-7-yl group, dihydro-2H-isoquinolin-6-yl group, dihydro-2H-isoquinolin-5-yl group, cinnolin-6-yl group, cinnolin-5-yl group, quinazolin-6-yl group, quinazolin-7-yl group, quinazolin-5-yl group, quinoxalin-2-yl group, quinoxalin-6-yl group, quinoxalin-5-yl group, 1H-benzimidazol-5-yl group, 1H-benzimidazol-4-yl group, benzoxazol-5-yl group, benzoxazol-6-yl group, benzoxazol-4-yl group, benzoxazol-7-yl group, 1H-pyrrolo[3,2-b]pyridin-5-yl group, 1H-pyrrolo[3,2-b]pyridin-6-yl group, benzo[1,2,5]thiadiazol-5-yl group, benzo[1,2,5]thiadiazol-4-yl group, 1H-benzotriazol-5-yl group, 1H-benzotriazol-4-yl group, 1,3-dihydropyrrolo[2,3-b]pyridin-5-yl group, 1,3-dihydropyrrolo[2,3-b]pyridin-4-yl group, 1,3-dihydrobenzimidazol-5-yl group, 1,3-dihydrobenzimidazol-4-yl group, dihydro-3H-benzoxazol-6-yl group, dihydro-3H-benzoxazol-7-yl group, dihydro-3H-benzoxazol-5-yl group, dihydro-3H-benzoxazol-4-yl group, phthalazin-6-yl group, phthalazin-5-yl group, [1,8]naphthylidin-3-yl group, [1,8]naphthylidin-4-yl group, [1,5]naphthylidin-3-yl group, [1,5]naphthylidin-4-yl group, 1H-pyrrolo[3,2-c]pyridin-6-yl group, 1H-pyrrolo[3,2-c]pyridin-4-yl group, 1H-pyrrolo[2,3-c]pyridin-5-yl group, 1H-pyrrolo[2,3-c]pyridin-4-yl group, 1H-pyrazolo[4,3-b]pyridin-5-yl group, 1H-pyrazolo[4,3-b]pyridin-6-yl group, 1H-pyrazolo[4,3-c]pyridin-6-yl group, 1H-pyrazolo[4,3-c]pyridin-4-yl group, 1H-pyrazolo[3,4-c]pyridin-5-yl group, 1H-pyrazolo[3,4-c]pyridin-4-yl group, 1H-pyrazolo[3,4-b]pyridin-5-yl group, 1H-pyrazolo[3,4-b]pyridin-4-yl group, [1,2,4]triazolo[4,3-a]pyridin-6-yl group, [1,2,4]triazolo[4,3-a]pyridin-7-yl group, thieno[3,2-c]pyridin-2-yl group, thieno[3,2-c]pyridin-3-yl group, thieno[3,2-c]pyridin-6-yl group, thieno[3,2-b]pyridin-2-yl group, thieno[3,2-b]pyridin-3-yl group, thieno[3,2-b]pyridin-5-yl group, thieno[3,2-b]pyridin-6-yl group, 1H-thieno[3,2-c]pyrazol-5-yl group, 1H-thieno[3,2-c]pyrazol-4-yl group, benzo[d]isoxazol-5-yl group, benzo[d]isoxazol-4-yl group, benzo[d]isoxazol-6-yl group, benzo[d]isoxazol-7-yl group, benzo[c]isoxazol-5-yl group, benzo[c]isoxazol-4-yl group, benzo[c]isoxazol-6-yl group, benzo[c]isoxazol-7-yl group, indolizin-7-yl group, indolizin-6-yl group, indolizin-8-yl group, 1,3-dihydroindol-5-yl group, 1,3-dihydroindol-4-yl group, 1,3-dihydroindol-6-yl group, 1H-pyrazolo[3,4-d]thiazol-5-yl group, 2H-isoindol-5-yl group, 2H-isoindol-4-yl group, [1,2,4]triazolo[1,5-a]pyrimidin-6-yl group, 1H-pyrazolo[3,4-b]pyrazin-5-yl group, 1H-imidazo[4,5-b]pyrazin-5-yl group, 7H-purin-2-yl group, 4H-chromen-6-yl group, or 4H-chromen-5-yl group (the aforementioned groups may be substituted with one of Xa or two or more of the same or different Xa).

<11> The compound or salt thereof according to any one of <1> to <8> mentioned above, wherein, in the formula (I), AR as V¹ or V² is naphthalen-2-yl group, 6-hydroxynaphthalen-2-yl group, 6-methoxynaphthalen-2-yl group, 6-(2-hydroxyethyloxy)naphthalen-2-yl group, 6-aminonaphthalen-2-yl group, 6-(N-methylamino)naphthalen-2-yl group, 6-(N,N-dimethylamino)naphthalen-2-yl group, 6-(2-hydroxyethylamino)naphthalen-2-yl group, benzo[b]furan-5-yl group, 2-methylbenzo[b]furan-5-yl group, 3-methylbenzo[b]furan-5-yl group, 2,3-dimethylbenzo[b]furan-5-yl group, benzo[b]thiophen-5-yl group, 2-methylbenzo[b]thiophen-5-yl group, 3-methylbenzo[b]thiophen-5-yl group, 2,3-dimethylbenzo[b]thiophen-5-yl group, 1H-indol-5-yl group, 2-methyl-1H-indol-5-yl group, 3-methyl-1H-indol-5-yl group, 2,3-dimethyl-1H-indol-5-yl group, 1-methyl-1H-indol-5-yl group, 1,2-dimethyl-1H-indol-5-yl group, 1,3-dimethyl-1H-indol-5-yl group, 1,2,3-trimethyl-1H-indol-5-yl group, 1-ethyl-1H-indol-5-yl group, 1-ethyl-2-methyl-1H-indol-5-yl group, 1-ethyl-3-methyl-1H-indol-5-yl group, 1-ethyl-2,3-dimethyl-1H-indol-5-yl group, 1-propyl-1H-indol-5-yl group, 2-methyl-1-propyl-1H-indol-5-yl group, 3-methyl-1-propyl-1H-indol-5-yl group, 2,3-dimethyl-1-propyl-1H-indol-5-yl group, 1-(2-hydroxyethyl)-1H-indol-5-yl group, 1-(2-hydroxyethyl)-2-methyl-1H-indol-5-yl group, 1-(2-hydroxyethyl)-3-methyl-1H-indol-5-yl group, 2,3-dimethyl-1-(2-hydroxyethyl)-1H-indol-5-yl group, benzothiazol-6-yl group, 2-methylbenzothiazol-6-yl group, 2-methoxybenzothiazol-6-yl group, 2-aminobenzothiazol-6-yl group, 2-oxo-2,3-dihydrobenzothiazol-6-yl group, 2-oxo-3-methyl-2,3-dihydrobenzothiazol-6-yl group, 2-thioxo-2,3-dihydrobenzothiazol-6-yl group, 2-thioxo-3-methyl-2,3-dihydrobenzothiazol-6-yl group, quinolin-3-yl group, quinolin-6-yl group, 2-oxo-1,2-dihydroquinolin-6-yl group, benzo[d]isothiazol-5-yl group, 1H-indazol-5-yl group, 1-methyl-1H-indazol-5-yl group, 1-ethyl-1H-indazol-5-yl group, 1-propyl-1H-indazol-5-yl group, 1-(2-hydroxyethyl)-1H-indazol-5-yl group, 3-hydroxy-1H-indazol-5-yl group, 3-hydroxy-1-methyl-1H-indazol-5-yl group, 1-ethyl-3-hydroxy-1H-indazol-5-yl group, imidazo[1,2-a]pyridin-6-yl group, 1H-pyrrolo[2,3-b]pyridin-5-yl group, 1-methyl-1H-pyrrolo[2,3-b]pyridin-5-yl group, 1-ethyl-1H-pyrrolo[2,3-b]pyridin-5-yl group, 1-propyl-1H-pyrrolo[2,3-b]pyridin-5-yl group, 1-(2-hydroxyethyl)-1H-pyrrolo[2,3-b]pyridin-5-yl group, isoquinolin-6-yl group, 1-oxo-1,2-dihydroisoquinolin-6-yl group, cinnolin-6-yl group, or benzoxazol-5-yl group.

<12> The compound or salt thereof according to any one of <1> to <8> mentioned above, wherein, in the formula (I), AR as V¹ or V² is naphthalen-2-yl group, 6-hydroxynaphthalen-2-yl group, 6-methoxynaphthalen-2-yl group, 6-aminonaphthalen-2-yl group, 6-(N,N-dimethylamino)naphthalen-2-yl group, benzo[b]furan-5-yl group, benzo[b]thiophen-5-yl group, 1H-indol-5-yl group, 1-methyl-1H-indol-5-yl group, 1-ethyl-1H-indol-5-yl group, benzothiazol-6-yl group, 2-aminobenzothiazol-6-yl group, 2-oxo-2,3-dihydrobenzothiazol-6-yl group, quinolin-3-yl group, quinolin-6-yl group, 2-oxo-1,2-dihydroquinolin-6-yl group, 1H-indazol-5-yl group, 1-methyl-1H-indazol-5-yl group, 1-ethyl-1H-indazol-5-yl group, isoquinolin-6-yl group, 1-oxo-1,2-dihydroisoquinolin-6-yl group, cinnolin-6-yl group, or benzoxazol-5-yl group.
<13> The compound or salt thereof according to any one of <1> to <12>, wherein, in the formula (I), when Rb is contained in Rs, Q in Rb is phenyl group, thienyl group, furyl group, pyrrolyl group, pyridyl group, oxazolyl group, isoxazolyl group, thiazolyl group, isothiazolyl group, imidazolyl group, pyrazolyl group, oxadiazolyl group, thiadiazolyl group, triazolyl group, tetrazolyl group, naphthyl group, tetrahydronaphthyl group, indanyl group, indenyl group, quinolyl group, isoquinolyl group, indolyl group, benzofuryl group, benzothienyl group, benzimidazolyl group, benzoxazolyl group, benzothiazolyl group, indazolyl group, 4H-chromenyl group, dihydrobenzodioxyl group, benzisoxazolyl group, pyrrolopyridinyl group, pyrazolopyridinyl group, triazolopyridinyl group, thienopyridinyl group, thienopyrazolyl group, 1,3-dihydrobenzimidazole group, dihydro-3H-benzoxazole group, or dihydro-3H-benzothiazole group (the aforementioned groups bind to A² at an arbitrary position on the ring); and
   when Qa exists in a moiety of the structure, Qa is phenyl group, pyridyl group, oxazolyl group, isoxazolyl group, thiazolyl group, isothiazolyl group, imidazolyl group, pyrazolyl group, oxadiazolyl group, thiadiazolyl group, triazolyl group, tetrazolyl group, naphthyl group, indanyl group, indenyl group, quinolyl group, isoquinolyl group, indolyl group, benzofuryl group, benzothienyl group, benzimidazolyl group, benzoxazolyl group, benzothiazolyl group, or indazolyl group (the aforementioned groups may be substituted with one of T¹ or two or more of the same or different T¹, and bind to A⁶ at an arbitrary position on the ring).

<14> The compound or salt thereof according to any one of <1> to <13>, wherein, in the formula (I), when Rs represents -D-Rx, D is a single bond, oxygen atom, or sulfur atom.
<15> The compound or salt thereof according to any one of <1> to <13>, wherein, in the formula (I), when Rs represents -D-Rx, D is a single bond, oxygen atom, or sulfur atom;
   Rx is propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb mentioned above;
   when Rx represents Rb, Q in Rb is phenyl group, thienyl group, furyl group, pyridyl group, oxazolyl group, naphthyl group, tetrahydronaphthyl group, indanyl group, indolyl group, or dihydrobenzodioxyl group;
   A² in Rb is a single bond, oxygen atom, sulfur atom, -N(methyl)-, or -N(ethyl)-;
   A¹ in Rb is a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² is oxygen atom, sulfur atom, -N(methyl)-, or -N(ethyl)-, A¹ is ethylene group, methylethylene group, dimethylethylene group, ethylethylene group, phenylethylene group, ethenylene group, trimethylene group, or methyltrimethylene group);
   R² and R³ independently represent hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group;
   when Rs is -N(Ry)(Rz), Rz has the same meaning as that of Rx, or is methyl group, or ethyl group;
   Ry is hydrogen atom, methyl group, ethyl group, propyl group, isopropyl group, butyl group, isobutyl group, pentyl group, or isopentyl group (provided that when Rz is methyl group, or ethyl group, Ry is a substituent other than hydrogen atom), or may bind to Rz to form, together with the nitrogen atom, a cyclic substituent (qy) selected from pyrrolidino group, piperidino group, homopiperidino group, morpholino group, piperazino group, homopiperazino group, pyrrol-1-yl group, imidazol-1-yl group, pyrazol-1-yl group, and thiomorpholino group, and the cyclic substituent (qy) may be substituted with one of alkyl group having 1 to 8 carbon atoms or two of the same or different alkyl groups having 1 to 8 carbon atoms, phenyl group, or phenylmethyl group (provided that the benzene ring of the phenyl group or the phenylmethyl group may be substituted with one of substituent or two of the
   same or different substituents selected from the group consisting of methyl group, fluorine atom, chlorine atom, trifluoromethyl group, hydroxyl group, methoxy group, and dimethylamino group).

<16> The compound or salt thereof according to any one of <1> to <13>, wherein, in the formula (I), when Rs represents -D-Rx, D is a single bond or oxygen atom;
   Rx is propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, or cycloheptylmethyl group, or Rb mentioned above;
   when Rx represents Rb, Q in Rb is phenyl group, 2-thienyl group, 3-thienyl group, 2-furyl group, 3-furyl group, or indan-2-yl group;
   A² in Rb is a single bond, oxygen atom, sulfur atom, -N(methyl)-, or -N(ethyl)-;
   A¹ in Rb is a single bond, or methylene group, methylmethylene group, or ethylene group when A² is a single bond, or ethylene group when A² is oxygen atom, sulfur atom, -N(methyl)-, or -N(ethyl)-;
   R² and R³ independently represent hydrogen atom, methyl group, fluorine atom, chlorine atom, trifluoromethyl group, hydroxyl group, methoxy group, or dimethylamino group;
   when Rs is -N(Ry)(Rz), Rz has the same meaning as that of Rx, or is methyl group, or ethyl group;
   Ry is hydrogen atom, methyl group, ethyl group, propyl group, isopropyl group, butyl group, isobutyl group, pentyl group, or isopentyl group (provided that when Rz is methyl group, or ethyl group, Ry is a substituent other than hydrogen atom), or may bind to Rz to form, together with the nitrogen atom, a cyclic substituent (qy) selected from the group consisting of pyrrolidino group, piperidino group, morpholino group, and piperazino group, and the cyclic substituent (qy) may be substituted with one of substituent or two of the same or different substituents selected from methyl group, ethyl group, propyl group, isopropyl group, butyl group, isobutyl group, cyclopentyl group, cyclohexyl group, cyclopentylmethyl group, cyclohexylmethyl group, phenyl group, and phenylmethyl group (provided that the benzene ring of the phenyl group or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group).

<17> The compound or salt thereof according to any one of <1> to <13>, wherein, in the formula (I), when Rs represents -D-Rx, D is a single bond or oxygen atom;
   Rx is propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group; and
   when Rs represents -N(Ry)(Rz), -N(Ry)(Rz) is N,N-dimethylamino group, N-ethyl-N-methylamino group, N,N-diethylamino group, N-methyl-N-propylamino group, N-ethyl-N-propylamino group, N-isopropyl-N-methylamino group, N-ethyl-N-isopropylamino group, N-butylamino group, N-butyl-N-methylamino group, N-butyl-N-ethylamino group, N-isobutylamino group, N-isobutyl-N-methylamino group, N-ethyl-N-isobutylamino group, N-isopentylamino group, N-isopentyl-N-methylamino group, N-ethyl-N-isopentylamino group, N-(2-ethylbutyl)amino group, N-(2-ethylbutyl)-N-methylamino group, N-cyclopentylamino group, N-cyclopentyl-N-methylamino group, N-cyclohexylamino group, N-cyclohexyl-N-methylamino group, N-cycloheptylamino group, N-(cyclopentylmethyl)amino group, N-(cyclopentylmethyl)-N-methylamino group, N-(cyclohexylmethyl)amino group, N-(cyclohexylmethyl)-N-methylamino group, N-phenylamino group, N-(2-methylphenyl)amino group, N-(4-methylphenyl)amino group, N-(2-fluorophenyl)amino group, N-(3-fluorophenyl)amino group, N-(4-fluorophenyl)amino group, N-(2-chlorophenyl)amino group, N-(3-chlorophenyl)amino group, N-(4-chlorophenyl)amino group, N-(indan-2-yl)amino group, N-(1-phenylethyl)amino group, N-[1-(2-fluorophenyl)ethyl]amino group, N-[1-(3-fluorophenyl)ethyl]amino group, N-[1-(4-fluorophenyl)ethyl]amino group, N-[1-(2-chlorophenyl)ethyl]amino group, N-[1-(3-chlorophenyl)ethyl]amino group, N-[1-(4-chlorophenyl)ethyl]amino group, N-benzylamino group, N-benzyl-N-methylamino group, N-benzyl-N-ethylamino group, N-(2-methylphenylmethyl)amino group, N-methyl-N-(2-methylphenylmethyl)amino group, N-(3-methylphenylmethyl)amino group, N-methyl-N-(3-methylphenylmethyl)amino group, N-(4-methylphenylmethyl)amino group, N-methyl-N-(4-methylphenylmethyl)amino group, N-(2-fluorophenylmethyl)amino group, N-(2-fluorophenylmethyl)-N-methylamino group, N-(3-fluorophenylmethyl)amino group, N-(3-fluorophenylmethyl)-N-methylamino group, N-(4-fluorophenylmethyl)amino group, N-(4-fluorophenylmethyl)-N-methylamino group, N-(2-chlorophenylmethyl)amino group, N-(2-chlorophenylmethyl)-N-methylamino group, N-(3-chlorophenylmethyl)amino group, N-(3-chlorophenylmethyl)-N-methylamino group, N-(4-chlorophenylmethyl)amino group, N-(4-chlorophenylmethyl)-N-methylamino group, N-(2,3-difluorophenylmethyl)amino group, N-(2,3-difluorophenylmethyl)-N-methylamino group, N-(2,4-difluorophenylmethyl)amino group, N-(2,4-difluorophenylmethyl)-N-methylamino group, N-(2,5-difluorophenylmethyl)amino group, N-(2,5-difluorophenylmethyl)-N-methylamino group, N-(3,4-difluorophenylmethyl)amino group, N-(3,4-difluorophenylmethyl)-N-methylamino group, N-(3,5-diffuorophenylmethyl)amino group, N-(3,5-difluorophenylmethyl)-N-methylamino group, N-(2,3-dichlorophenylmethyl)amino group, N-(2,3-dichlorophenylmethyl)-N-methylamino group, N-(2,4-dichlorophenylmethyl)amino group, N-(2,4-dichlorophenylmethyl)-N-methylamino group, N-(2,5-dichlorophenylmethyl)amino group, N-(2,5-dichlorophenylmethyl)-N-methylamino group, N-(2,6-dichlorophenylmethyl)amino group, N-(2,6-dichlorophenylmethyl)-N-methylamino group, N-(3,4-dichlorophenylmethyl)amino group, N-(3,4-dichlorophenylmethyl)-N-methylamino group, N-(3,5-dichlorophenylmethyl)amino group, N-(3,5-dichlorophenylmethyl)-N-methylamino group, N-[2-(trifluoromethyl)phenylmethyl]amino group, N-methyl-N-[2-(trifluoromethyl)phenylmethyl]amino group, N-[3-(trifluoromethyl)phenylmethyl]amino group, N-methyl-N-[3-(triffuoromethyl)phenylmethyl]amino group, N-[4-(trifluoromethyl)phenylmethyl]amino group, N-methyl-N-[4-(trifluoromethyl)phenylmethyl]amino group, pyrrolidino group, 2-methylpyrrolidino group, 3-methylpyrrolidino group, 2,5-dimethylpyrrolidino group, 3,4-dimethylpyrrolidino group, piperidino group, 2-methylpiperidino group, 3-methylpiperidino group, 4-methylpiperidino group, 4-ethylpiperidino group, 4-propylpiperidino group, 4-isopropylpiperidino group, 4-butylpiperidino group, 4-isobutylpiperidino group, 4-cyclopentylpiperidino group, 4-cyclohexylpiperidino group, 4-(cyclopentylmethyl)piperidino group, 4-(cyclohexylmethyl)piperidino group, 4-phenylpiperidino group, 4-(2-methylphenyl)piperidino group, 4-(4-methylphenyl)piperidino group, 4-(2-fluorophenyl)piperidino group, 4-(3-fluorophenyl)piperidino group, 4-(4-fluorophenyl)piperidino group, 4-(2-chlorophenyl)piperidino group, 4-(3-chlorophenyl)piperidino group, 4-(4-chlorophenyl)piperidino group, 4-benzylpiperidino group, 4-(2-methylphenylmethyl)piperidino group, 4-(3-methylphenylmethyl)piperidino group, 4-methylphenylmethyl)piperidino group, 4-(2,3-dimethylphenylmethyl)piperidino group, 4-(3,5-dimethylphenylmethyl)piperidino group, 4-(2-fluorophenylmethyl)piperidino group, 4-(3-fluorophenylmethyl)piperidino group, 4-(4-fluorophenylmethyl)piperidino group, 4-(2-chlorophenylmethyl)piperidino group, 4-(3-chlorophenylmethyl)piperidino group, 4-(4-chlorophenylmethyl)piperidino group, 4-(2,3-difluorophenylmethyl)piperidino group, 4-(2,4-difluorophenylmethyl)piperidino group, 4-(2,5-difluorophenylmethyl)piperidino group, 4-(3,4-difluorophenylmethyl)piperidino group, 4-(2,3-dichlorophenylmethyl)piperidino group, 4-(2,4-dichlorophenylmethyl)piperidino group, 4-(2,5-dichlorophenylmethyl)piperidino group, 4-(2,6-dichlorophenylmethyl)piperidino group, 4-(3,4-dichlorophenylmethyl)piperidino group, 4-(3,5-dichlorophenylmethyl)piperidino group, 4-(3,6-dichlorophenylmethyl)piperidino group, 4-(2-methoxyphenylmethyl)piperidino group, 4-(3-methoxyphenylmethyl)piperidino group, 4-(4-methoxyphenylmethyl)piperidino group, 2,6-dimethylpiperidino group, 3,5-dimethylpiperidino group, 4,4-dimethylpiperidino group, homopiperidino group, morpholino group, 3,5-dimethylmorpholino group, 4-phenylpiperazino group, 4-(2-methylphenyl)piperazino group, 4-(4-methylphenyl)piperazino group, 4-(2-fluorophenyl)piperazino group, 4-(3-fluorophenyl)piperazino group, 4-(4-fluorophenyl)piperazino group, 4-(2-chlorophenyl)piperazino group, 4-(3-chlorophenyl)piperazino group, 4-(4-chlorophenyl)piperazino group, 4-benzylpiperazino group, 4-(2-methylphenylmethyl)piperazino group, 4-(3-methylphenylmethyl)piperazino group, 4-methylphenylmethyl)piperazino group, 4-(2,3-dimethylphenylmethyl)piperazino group, 4-(3,5-dimethylphenylmethyl)piperazino group, 4-(2-fluorophenylmethyl)piperazino group, 4-(3-fluorophenylmethyl)piperazino group, 4-(4-fluorophenylmethyl)piperazino group, 4-(2-chlorophenylmethyl)piperazino group, 4-(3-chlorophenylmethyl)piperazino group, 4-(4-chlorophenylmethyl)piperazino group, 4-(2,3-difluorophenylmethyl)piperazino group, 4-(2,4-difluorophenylmethyl)piperazino group, 4-(2,5-difluorophenylmethyl)piperazino group, 4-(3,4-difluorophenylmethyl)piperazino group, 4-(2,3-dichlorophenylmethyl)piperazino group, 4-(2,4-dichlorophenylmethyl)piperazino group, 4-(2,5-dichlorophenylmethyl)piperazino group, 4-(2,6-dichlorophenylmethyl)piperazino group, 4-(3,4-dichlorophenylmethyl)piperazino group, 4-(3,5-dichlorophenylmethyl)piperazino group, 4-(3,6-dichlorophenylmethyl)piperazino group, 4-(2-methoxyphenylmethyl)piperazino group, 4-(3-methoxyphenylmethyl)piperazino group, or 4-(4-methoxyphenylmethyl)piperazino group.

<18> The compound or salt thereof according to any one of <1> to <17> mentioned above, wherein, in the formula (I), when Rs represents -D-Rx, D is a single bond.
<19> The compound or salt thereof according to any one of <1> to <17> mentioned above, wherein, in the formula (I), Rs is -O-Rx.
<20> The compound or salt thereof according to any one of <1> to <15> mentioned above, wherein, in the formula (I), Rs is -S-Rx.
<21> The compound or salt thereof according to any one of <1> to <17> mentioned above, wherein, in the formula (I), Rs is -N(Ry)(Rz).
<22> The compound or salt thereof according to any one of <1> to <13>, wherein, in the formula (I), Rs is phenyl group, 2-methylphenyl group, 3-methylphenyl group, 4-methylphenyl group, 2,3-dimethylphenyl group, 3,5-dimethylphenyl group, 2-methoxyphenyl group, 3-methoxyphenyl group, 4-methoxyphenyl group, 2-fluorophenyl group, 3-fluorophenyl group, 4-fluorophenyl group, 2-chlorophenyl group, 3-chlorophenyl group, 4-chlorophenyl group, 2,3-difluorophenyl group, 2,4-difluorophenyl group, 2,5-difluorophenyl group, 3,4-difluorophenyl group, 2,3-dichlorophenyl group, 2,4-dichlorophenyl group, 2,5-dichlorophenyl group, 2,6-dichlorophenyl group, 3,4-dichlorophenyl group, 3,5-dichlorophenyl group, 2-trifluoromethylphenyl group, 3-trifluoromethylphenyl group, 4-trifluoromethylphenyl group, 4-(N,N-dimethylamino)phenyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-methoxyindan-2-yl group, 5-methoxyindan-2-yl group, 4,7-dimethoxyindan-2-yl group, 5,6-dimethoxyindan-2-yl group, furan-2-yl group, furan-3-yl group, thiophen-2-yl group, or thiophen-3-yl group.

<23> The compound or salt thereof according to any one of <1> to <22> mentioned above, wherein, in the formula (I), Zx as V¹ or V² is hydrogen atom, fluorine atom, chlorine atom, bromine atom, nitro group, methyl group, hydroxyl group, methoxy group, amino group, N-methylamino group, N-ethylamino group, N-propylamino group, N-isopropylamino group, or N,N-dimethylamino group.
<24> The compound or salt thereof according to any one of <1> to <22> mentioned above, wherein, in the formula (I), Zx as V¹ or V² is hydrogen atom, fluorine atom, chlorine atom, bromine atom, methyl group, hydroxyl group, amino group, or N-methylamino group.
<25> The compound or salt thereof according to any one of <1> to <24> mentioned above, wherein, Y is hydrogen atom, or a lower alkyl group having 1 to 4 carbon atoms.
<26> The compound or salt thereof according to any one of <1> to <24> mentioned above, wherein, Y is hydrogen atom, methyl group, or ethyl group.

<27> The compound or salt thereof according to <1> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond or a double bond;
   when the bond between the ring-constituting carbon atom at the 1-position of the ring (E) and the Link moiety is a single bond, Link is CH₂ or CH₂CH₂, or when the bond between the ring-constituting carbon atom at the 1-position of the ring (E) and the Link moiety is a double bond, Link is CH;
   W is methylene group;
   Rs is -D-Rx or -N(Ry)(Rz);
   D is a single bond, oxygen atom, or sulfur atom;
   Rx is propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb mentioned above;
   Q in Rb is phenyl group, thienyl group, furyl group, pyridyl group, oxazolyl group, naphthyl group, tetrahydronaphthyl group, indanyl group, indolyl group, or dihydrobenzodioxyl group;
   A² in Rb is a single bond, oxygen atom, sulfur atom, -N(methyl)-, or -N(ethyl)-;
   A¹ in Rb is a single bond, methylene group, methylmethylene group, ethylmethylene group, phenylmethylene group, ethylene group, methylethylene group, dimethylethylene group, ethylethylene group, phenylethylene group, trimethylene group, or methyltrimethylene group (provided that when A² is oxygen atom, sulfur atom, -N(methyl)-, or -N(ethyl)-, A¹ is ethylene group, methylethylene group, dimethylethylene group, ethylethylene group, phenylethylene group, trimethylene group, or methyltrimethylene group);
   R² and R³ independently represent hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group;
   Rz has the same meaning as that of Rx, or is methyl group, or ethyl group;
   Ry is hydrogen atom, methyl group, ethyl group, propyl group, isopropyl group, butyl group, isobutyl group, pentyl group, or isopentyl group (provided that when Rz is methyl group, or ethyl group, Ry is a substituent other than hydrogen atom), or may bind to Rz to form, together with the nitrogen atom, a cyclic substituent (qy) selected from pyrrolidino group, piperidino group, homopiperidino group, morpholino group, piperazino group, homopiperazino group, pyrrol-1-yl group, imidazol-1-yl group, pyrazol-1-yl group, and thiomorpholino group, and the cyclic substituent (qy) may be substituted with one of alkyl group having 1 to 8 carbon atoms or two of the same or different alkyl groups having 1 to 8 carbon atoms, phenyl group, or phenylmethyl group (provided that the benzene ring of the phenyl group or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group);
   the substituent V¹ on the ring (E) is Zx, and V² on the ring (E) is AR;
   Zx is hydrogen atom, fluorine atom, chlorine atom, bromine atom, nitro group, methyl group, hydroxyl group, methoxy group, amino group, N-methylamino group, N-ethylamino group, N-propylamino group, N-isopropylamino group, or N,N-dimethylamino group;
   AR is a residue of naphthalene, benzofuran, benzo[b]thiophene, indole, benzothiazole, dihydro-3H-benzothiazole, quinoline, dihydro-1H-quinoline, benzo[d]isothiazole, 1H-indazole, benzo[c]isothiazole, 2H-indazole, imidazo[1,2-a]pyridine, 1H-pyrrolo[2,3-b]pyridine, isoquinoline, dihydro-2H-isoquinoline, cinnoline, quinazoline, quinoxaline, 1H-benzimidazole, benzoxazole, 1H-pyrrolo[3,2-b]pyridine, benzo[1,2,5]thiadiazole, 1H-benzotriazole, 1,3-dihydropyrrolo[2,3-b]pyridine, 1,3-dihydrobenzimidazole, dihydro-3H-benzoxazole, phthalazine, [1,8]naphthylidine, [1,5]naphthylidine, 1H-pyrrolo[3,2-c]pyridine, 1H-pyrrolo[2,3-c]pyridine, 1H-pyrazolo[4,3-b]pyridine, 1H-pyrazolo[4,3-c]pyridine, 1H-pyrazolo[3,4-c]pyridine, 1H-pyrazolo[3,4-b]pyridine, [1,2,4]triazolo[4,3-a]pyridine, thieno[3,2-c]pyridine, thieno[3,2-b]pyridine, 1H-thieno[3,2-c]pyrazole, benzo[d]isoxazole, benzo[c]isoxazole, indolizine, 1,3-dihydroindole, 1H-pyrazolo[3,4-d]thiazole, 2H-isoindole, [1,2,4]triazolo[1,5-a]pyrimidine, 1H-pyrazolo[3,4-b]pyrazine, 1H-imidazo[4,5-b]pyrazine, 7H-purine, or 4H-chromene (the aforementioned residue may be substituted with one of Xa or two or more of the same or different Xa);
   Xa is oxo group, thioxo group, fluorine atom, chlorine atom, trifluoromethyl group, methyl group, ethyl group, propyl group, 2-hydroxyethyl group, carboxymethyl group, 2-carboxyethyl group, N,N-dimethylcarbamoylmethyl group, hydroxyl group, methoxy group, 2-hydroxyethyloxy group, carboxymethyloxy group, 2-carboxyethyloxy group, N,N-dimethylcarbamoylmethyloxy group, amino group, methylamino group, dimethylamino group, 2-hydroxyethylamino group, carbamoylamino group, acetylamino group, furan-2-carboxyamino group, 2-hydroxyacetylamino group, 2-aminoacetylamino group, methylsulfonylamino group, (N,N-dimethylsulfamoyl)amino group, methanesulfonyl group, sulfamoyl group, N-methylsulfamoyl group, N,N-dimethylsulfamoyl group, carboxyl group, acetyl group, carbamoyl group, or N,N-dimethylcarbamoyl group; and
   Y is hydrogen atom, or a lower alkyl group having 1 to 4 carbon atoms.

<28> The compound or salt thereof according to <1> mentioned above, wherein, in the formula (I), the bond between the ring-constituting carbon atom at the 1-position of the ring (E) and the ring-constituting carbon atom at the 2-position of the ring (E) is a double bond;
   the bond between the ring-constituting carbon atom at the 1-position of the ring (E) and the Link moiety is a single bond;
   Link is CH₂, or CH₂CH₂;
   W is oxygen atom;
   Rs is -D-Rx or -N(Ry)(Rz);
   D is a single bond, oxygen atom, or sulfur atom;
   Rx is propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb mentioned above;
   Q in Rb is phenyl group, thienyl group, furyl group, pyridyl group, oxazolyl group, naphthyl group, tetrahydronaphthyl group, indanyl group, indolyl group, or dihydrobenzodioxyl group;
   A² in Rb is a single bond, oxygen atom, sulfur atom, -N(methyl)-, or -N(ethyl)-;
   A¹ in Rb is a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² is oxygen atom, sulfur atom, -N(methyl)-, or -N(ethyl)-, A¹ represents ethylene group, methylethylene group, dimethylethylene group, ethylethylene group, phenylethylene group, ethenylene group, trimethylene group, or methyltrimethylene group);
   R² and R³ independently represent hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group;
   Rz has the same meaning as that of Rx, or is methyl group, or ethyl group;
   Ry is hydrogen atom, methyl group, ethyl group, propyl group, isopropyl group, butyl group, isobutyl group, pentyl group, or isopentyl group (provided that when Rz is methyl group, or ethyl group, Ry is a substituent other than hydrogen atom), or may bind to Rz to form, together with the nitrogen atom, a cyclic substituent (qy) selected from pyrrolidino group, piperidino group, homopiperidino group, morpholino group, piperazino group, homopiperazino group, pyrrol-1-yl group, imidazol-1-yl group, pyrazol-1-yl group, and thiomorpholino group, and the cyclic substituent (qy) may be substituted with one of alkyl group having 1 to 8 carbon atoms or two of the same or different alkyl groups having 1 to 8 carbon atoms, phenyl group, or phenylmethyl group (provided that the benzene ring of the phenyl group or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group);
   the substituent V¹ on the ring (E) is Zx, and V² on the ring (E) is AR;
   Zx is hydrogen atom, fluorine atom, chlorine atom, bromine atom, nitro group, methyl group, hydroxyl group, methoxy group, amino group, N-methylamino group, N-ethylamino group, N-propylamino group, N-isopropylamino group, or N,N-dimethylamino group;
   AR is a residue of naphthalene, benzofuran, benzo[b]thiophene, indole, benzothiazole, dihydro-3H-benzothiazole, quinoline, dihydro-1H-quinoline; benzo[d]isothiazole, 1H-indazole, benzo[c]isothiazole, 2H-indazole, imidazo[1,2-a]pyridine, 1H-pyrrolo[2,3-b]pyridine, isoquinoline, dihydro-2H-isoquinoline, cinnoline, quinazoline, quinoxaline, 1H-benzimidazole, benzoxazole, 1H-pyrrolo[3,2-b]pyridine, benzo[1,2,5]thiadiazole, 1H-benzotriazole, 1,3-dihydropyrrolo[2,3-b]pyridine, 1,3-dihydrobenzimidazole, dihydro-3H-benzoxazole, phthalazine, [1,8]naphthylidine, [1,5]naphthylidine, 1H-pyrrolo[3,2-c]pyridine, 1H-pyrrolo[2,3-c]pyridine, 1H-pyrazolo[4,3-b]pyridine, 1H-pyrazolo[4,3-c]pyridine, 1H-pyrazolo[3,4-c]pyridine, 1H-pyrazolo[3,4-b]pyridine, [1,2,4]triazolo[4,3-a]pyridine, thieno[3,2-c]pyridine, thieno[3,2-b]pyridine, 1H-thieno[3,2-c]pyrazole, benzo[d]isoxazole, benzo[c]isoxazole, indolizine, 1,3-dihydroindole, 1H-pyrazolo[3,4-d]thiazole, 2H-isoindole, [1,2,4]triazolo[1,5-a]pyrimidine, 1H-pyrazolo[3,4-b]pyrazine, 1H-imidazo[4,5-b]pyrazine, 7H-purine, or 4H-chromene (the aforementioned residue may be substituted with one of Xa or two or more of the same or different Xa);
   Xa is oxo group, thioxo group, fluorine atom, chlorine atom, trifluoromethyl group, methyl group, ethyl group, propyl group, 2-hydroxyethyl group, carboxymethyl group, 2-carboxyethyl group, N,N-dimethylcarbamoylmethyl group, hydroxyl group, methoxy group, 2-hydroxyethyloxy group, carboxymethyloxy group, 2-carboxyethyloxy group, N,N-dimethylcarbamoylmethyloxy group, amino group, methylamino group, dimethylamino group, 2-hydroxyethylamino group, carbamoylamino group, acetylamino group, furan-2-carboxyamino group, 2-hydroxyacetylamino group, 2-aminoacetylamino group, methylsulfonylamino group, (N,N-dimethylsulfamoyl)amino group, methanesulfonyl group, sulfamoyl group, N-methylsulfamoyl group, N,N-dimethylsulfamoyl group, carboxyl group, acetyl group, carbamoyl group, or N,N-dimethylcarbamoyl group; and
   Y is hydrogen atom, or a lower alkyl group having 1 to 4 carbon atoms.

<29> The compound or salt thereof according to <1> mentioned above, wherein, in the formula (I), the bond between the ring-constituting carbon atom at the 1-position of the ring (E) and the ring-constituting carbon atom at the 2-position of the ring (E) is a double bond;
   the bond between the ring-constituting carbon atom at the 1-position of the ring (E) and the Link moiety is a single bond;
   Link is CH₂, or CH₂CH₂;
   W is NH, or N(methyl);
   Rs is -D-Rx or -N(Ry)(Rz);
   D is a single bond, oxygen atom, or sulfur atom;
   Rx is propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb mentioned above;
   Q in Rb is phenyl group, thienyl group, furyl group, pyridyl group, oxazolyl group, naphthyl group, tetrahydronaphthyl group, indanyl group, indolyl group, or dihydrobenzodioxyl group;
   A² in Rb is a single bond, oxygen atom, sulfur atom, -N(methyl)-, or -N(ethyl)-;
   A¹ in Rb is a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² is oxygen atom, sulfur atom, -N(methyl)-, or -N(ethyl)-, A¹ is ethylene group, methylethylene group, dimethylethylene group, ethylethylene group, phenylethylene group, ethenylene group, trimethylene group, or methyltrimethylene group);
   R² and R³ independently represent hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group;
   Rz has the same meaning as that of Rx, or is methyl group, or ethyl group;
   Ry is hydrogen atom, methyl group, ethyl group, propyl group, isopropyl group, butyl group, isobutyl group, pentyl group, or isopentyl group (provided that when Rz is methyl group, or ethyl group, Ry is a substituent other than hydrogen atom), or may bind to Rz to form, together with the nitrogen atom, a cyclic substituent (qy) selected from pyrrolidino group, piperidino group, homopiperidino group, morpholino group, piperazino group, homopiperazino group, pyrrol-1-yl group, imidazol-1-yl group, pyrazol-1-yl group, and thiomorpholino group, and the cyclic substituent (qy) may be substituted with one of alkyl group having 1 to 8 carbon atoms or two of the same or different alkyl groups having 1 to 8 carbon atoms, phenyl group, or phenylmethyl group (provided that the benzene ring of the phenyl group or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group);
   the substituent V¹ on the ring (E) is Zx, and V² on the ring (E) is AR;
   Zx is hydrogen atom, fluorine atom, chlorine atom, bromine atom, nitro group, methyl group, hydroxyl group, methoxy group, amino group, N-methylamino group, N-ethylamino group, N-propylamino group, N-isopropylamino group, or N,N-dimethylamino group;
   AR is a residue of naphthalene, benzofuran, benzo[b]thiophene, indole, benzothiazole, dihydro-3H-benzothiazole, quinoline, dihydro-1H-quinoline, benzo[d]isothiazole, 1H-indazole, benzo[c]isothiazole, 2H-indazole, imidazo[1,2-a]pyridine, 1H-pyrrolo[2,3-b]pyridine, isoquinoline, dihydro-2H-isoquinoline, cinnoline, quinazoline, quinoxaline, 1H-benzimidazole, benzoxazole, 1H-pyrrolo[3,2-b]pyridine, benzo[1,2,5]thiadiazole, 1H-benzotriazole, 1,3-dihydropyrrolo[2,3-b]pyridine, 1,3-dihydrobenzimidazole, dihydro-3H-benzoxazole, phthalazine, [1,8]naphthylidine, [1,5]naphthylidine, 1H-pyrrolo[3,2-c]pyridine, 1H-pyrrolo[2,3-c]pyridine, 1H-pyrazolo[4,3-b]pyridine, 1H-pyrazolo[4,3-c]pyridine, 1H-pyrazolo[3,4-c]pyridine, 1H-pyrazolo[3,4-b]pyridine, [1,2,4]triazolo[4,3-a]pyridine, thieno[3,2-c]pyridine, thieno[3,2-b]pyridine, 1H-thieno[3,2-c]pyrazole, benzo[d]isoxazole, benzo[c]isoxazole, indolizine, 1,3-dihydroindole, 1H-pyrazolo[3,4-dlthiazole, 2H-isoindole, [1,2,4]triazolo[1,5-a]pyrimidine, 1H-pyrazolo[3,4-b]pyrazine, 1H-imidazo[4,5-b]pyrazine, 7H-purine, or 4H-chromene (the aforementioned residue may be substituted with one of Xa or two or more of the same or different Xa);
   Xa is oxo group, thioxo group, fluorine atom, chlorine atom, trifluoromethyl group, methyl group, ethyl group, propyl group, 2-hydroxyethyl group, carboxymethyl group, 2-carboxyethyl group, N,N-dimethylcarbamoylmethyl group, hydroxyl group, methoxy group, 2-hydroxyethyloxy group, carboxymethyloxy group, 2-carboxyethyloxy group, N,N-dimethylcarbamoylmethyloxy group, amino group, methylamino group, dimethylamino group, 2-hydroxyethylamino group, carbamoylamino group, acetylamino group, furan-2-carboxyamino group, 2-hydroxyacetylamino group, 2-aminoacetylamino group, methylsulfonylamino group, (N,N-dimethylsulfamoyl)amino group, methanesulfonyl group, sulfamoyl group, N-methylsulfamoyl group, N,N-dimethylsulfamoyl group, carboxyl group, acetyl group, carbamoyl group, or N,N-dimethylcarbamoyl group; and
   Y is hydrogen atom, or a lower alkyl group having 1 to 4 carbon atoms.

<30> The compound or salt thereof according to <1> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond or a double bond;
   when the bond between the ring-constituting carbon atom at the 1-position of the ring (E) and the Link moiety is a single bond, Link is CH₂ or CH₂CH₂, or when the bond between the ring-constituting carbon atom at the 1-position of the ring (E) and the Link moiety is a double bond, Link is CH;
   W is methylene group;
   Rs is -D-Rx or -N(Ry)(Rz);
   D is a single bond, oxygen atom, or sulfur atom;
   Rx is propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb mentioned above;
   Q in Rb is phenyl group, thienyl group, furyl group, pyridyl group, oxazolyl group, naphthyl group, tetrahydronaphthyl group, indanyl group, indolyl group, or dihydrobenzodioxyl group;
   A² in Rb is a single bond, oxygen atom, sulfur atom, -N(methyl)-, or -N(ethyl)-;
   A¹ in Rb is a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² is oxygen atom, sulfur atom, -N(methyl)-, or -N(ethyl)-, A¹ is ethylene group, methylethylene group, dimethylethylene group, ethylethylene group, phenylethylene group, ethenylene group, trimethylene group, or methyltrimethylene group);
   R² and R³ independently represent hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group;
   Rz has the same meaning as that of Rx, or is methyl group, or ethyl group;
   Ry is hydrogen atom, methyl group, ethyl group, propyl group, isopropyl group, butyl group, isobutyl group, pentyl group, or isopentyl group (provided that when Rz is methyl group, or ethyl group, Ry is a substituent other than hydrogen atom), or may bind to Rz to form, together with the nitrogen atom, a cyclic substituent (qy) selected from pyrrolidino group, piperidino group, homopiperidino group, morpholino group, piperazino group, homopiperazino group, pyrrol-1-yl group, imidazol-1-yl group, pyrazol-1-yl group, and thiomorpholino group, and the cyclic substituent (qy) may be substituted with one of alkyl group having 1 to 8 carbon atoms or two of the same or different alkyl groups having 1 to 8 carbon atoms, phenyl group, or phenylmethyl group (provided that the benzene ring of the phenyl group or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group);
   the substituent V¹ on the ring (E) is Zx, and V² on the ring (E) is AR;
   Zx is hydrogen atom, fluorine atom, chlorine atom, bromine atom, nitro group, methyl group, hydroxyl group, methoxy group, amino group, N-methylamino group, N-ethylamino group, N-propylamino group, N-isopropylamino group, or N,N-dimethylamino group;
   AR is naphthalen-2-yl group, naphthalen-1-yl group, benzofuran-5-yl group, benzofuran-4-yl group, benzofuran-2-yl group, benzo[b]thiophen-5-yl group, benzo[b]thiophen-4-yl group, benzo[b]thiophen-2-yl group, indol-5-yl group, indol-4-yl group, indol-6-yl group, benzothiazol-6-yl group, benzothiazol-7-yl group, benzothiazol-5-yl group, benzothiazol-4-yl group, dihydro-3H-benzothiazol-6-yl group, dihydro-3H-benzothiazol-7-yl group, dihydro-3H-benzothiazol-5-yl group, dihydro-3H-benzothiazol-4-yl group, quinolin-6-yl group, quinolin-3-yl group, quinolin-5-yl group, quinolin-7-yl group, dihydro-1H-quinolin-6-yl group, dihydro-1H-quinolin-5-yl group, benzo[d]isothiazol-5-yl group, benzo[d]isothiazol-4-yl group, benzo[d]isothiazol-6-yl group, benzo[d]isothiazol-7-yl group, 1H-indazol-5-yl group, 1H-indazol-4-yl group, 1H-indazol-6-yl group, benzo[c]isothiazol-5-yl group, benzo[c]isothiazol-4-yl group, benzo[c]isothiazol-6-yl group, benzo[c]isothiazol-7-yl group, 2H-indazol-5-yl group, 2H-indazol-4-yl group, 2H-indazol-6-yl group, imidazo[1,2-a]pyridin-6-yl group, imidazo[1,2-a]pyridin-7-yl group, 1H-pyrrolo[2,3-b]pyridin-5-yl group, 1H-pyrrolo[2,3-b]pyridin-4-yl group, isoquinolin-6-yl group, isoquinolin-3-yl group, isoquinolin-5-yl group, isoquinolin-7-yl group, dihydro-2H-isoquinolin-6-yl group, dihydro-2H-isoquinolin-5-yl group, cinnolin-6-yl group, cinnolin-5-yl group, quinazolin-6-yl group, quinazolin-7-yl group, quinazolin-5-yl group, quinoxalin-2-yl group, quinoxalin-6-yl group, quinoxalin-5-yl group, 1H-benzimidazol-5-yl group, 1H-benzimidazol-4-yl group, benzoxazol-5-yl group, benzoxazol-6-yl group, benzoxazol-4-yl group, benzoxazol-7-yl group, 1H-pyrrolo[3,2-b]pyridin-5-yl group, 1H-pyrrolo[3,2-b]pyridin-6-yl group, benzo[1,2,5]thiadiazol-5-yl group, benzo[1,2,5]thiadiazol-4-yl group, 1H-benzotriazol-5-yl group, 1H-benzotriazol-4-yl group, 1,3-dihydropyrrolo[2,3-b]pyridin-5-yl group, 1,3-dihydropyrrolo[2,3-b]pyridin-4-yl group, 1,3-dihydrobenzimidazol-5-yl group, 1,3-dihydrobenzimidazol-4-yl group, dihydro-3H-benzoxazol-6-yl group, dihydro-3H-benzoxazol-7-yl group, dihydro-3H-benzoxazol-5-yl group, dihydro-3H-benzoxazol-4-yl group, phthalazin-6-yl group, phthalazin-5-yl group, [1,8]naphthylidin-3-yl group, [1,8]naphthylidin-4-yl group, [1,5]naphthylidin-3-yl group, [1,5]naphthylidin-4-yl group, 1H-pyrrolo[3,2-c]pyridin-6-yl group, 1H-pyrrolo[3,2-c]pyridin-4-yl group, 1H-pyrrolo[2,3-c]pyridin-5-yl group, 1H-pyrrolo[2,3-c]pyridin-4-yl group, 1H-pyrazolo[4,3-b]pyridin-5-yl group, 1H-pyrazolo[4,3-b]pyridin-6-yl group, 1H-pyrazolo[4,3-c]pyridin-6-yl group, 1H-pyrazolo[4,3-c]pyridin-4-yl group, 1H-pyrazolo[3,4-c]pyridin-5-yl group, 1H-pyrazolo[3,4-c]pyridin-4-yl group, 1H-pyrazolo[3,4-b]pyridin-5-yl group, 1H-pyrazolo[3,4-b]pyridin-4-yl group, [1,2,4]triazolo[4,3-a]pyridin-6-yl group, [1,2,4]triazolo[4,3-a]pyridin-7-yl group, thieno[3,2-c]pyridin-2-yl group, thieno[3,2-c]pyridin-3-yl group, thieno[3,2-c]pyridin-6-yl group, thieno[3,2-b]pyridin-2-yl group, thieno[3,2-b]pyridin-3-yl group, thieno[3,2-b]pyridin-5-yl group, thieno[3,2-b]pyridin-6-yl group, 1H-thieno[3,2-c]pyrazol-5-yl group, 1H-thieno[3,2-c]pyrazol-4-yl group, benzo[d]isoxazol-5-yl group, benzo[d]isoxazol-4-yl group, benzo[d]isoxazol-6-yl group, benzo[d]isoxazol-7-yl group, benzo[c]isoxazol-5-yl group, benzo[c]isoxazol-4-yl group, benzo[c]isoxazol-6-yl group, benzo[c]isoxazol-7-yl group, indolizin-7-yl group, indolizin-6-yl group, indolizin-8-yl group, 1,3-dihydroindol-5-yl group, 1,3-dihydroindol-4-yl group, 1,3-dihydroindol-6-yl group, 1H-pyrazolo[3,4-d]thiazol-5-yl group, 2H-isoindol-5-yl group, 2H-isoindol-4-yl group, [1,2,4]triazolo[1,5-a]pyrimidin-6-yl group, 1H-pyrazolo[3,4-b]pyrazin-5-yl group, 1H-imidazo[4,5-b]pyrazin-5-yl group, 7H-purin-2-yl group, 4H-chromen-6-yl group, or 4H-chromen-5-yl group (the aforementioned groups may be substituted with one of Xa or two or more of the same or different Xa);
   Xa is oxo group, thioxo group, fluorine atom, chlorine atom, trifluoromethyl group, methyl group, ethyl group, propyl group, 2-hydroxyethyl group, carboxymethyl group, 2-carboxyethyl group, N,N-dimethylcarbamoylmethyl group, hydroxyl group, methoxy group, 2-hydroxyethyloxy group, carboxymethyloxy group, 2-carboxyethyloxy group, N,N-dimethylcarbamoylmethyloxy group, amino group, methylamino group, dimethylamino group, 2-hydroxyethylamino group, carbamoylamino group, acetylamino group, furan-2-carboxyamino group, 2-hydroxyacetylamino group, 2-aminoacetylamino group, methylsulfonylamino group, (N,N-dimethylsulfamoyl)amino group, methanesulfonyl group, sulfamoyl group, N-methylsulfamoyl group, N,N-dimethylsulfamoyl group, carboxyl group, acetyl group, carbamoyl group, or N,N-dimethylcarbamoyl group; and
   Y is hydrogen atom, or a lower alkyl group having 1 to 4 carbon atoms.

<31> The compound or salt thereof according to <1> mentioned above, wherein, in the formula (I), the bond between the ring-constituting carbon atom at the 1-position of the ring (E) and the ring-constituting carbon atom at the 2-position of the ring (E) is a double bond;
   the bond between the ring-constituting carbon atom at the 1-position of the ring (E) and the Link moiety is a single bond;
   Link is CH₂, or CH₂CH₂;
   W is oxygen atom;
   Rs is -D-Rx or -N(Ry)(Rz);
   D is a single bond, oxygen atom, or sulfur atom;
   Rx is propyl group, isopropyl group, butyl group, isobutyl group, pentyl group,
   isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb mentioned above;
   Q in Rb is phenyl group, thienyl group, furyl group, pyridyl group, oxazolyl group, naphthyl group, tetrahydronaphthyl group, indanyl group, indolyl group, or dihydrobenzodioxyl group;
   A² in Rb is a single bond, oxygen atom, sulfur atom, -N(methyl)-, or -N(ethyl)-;
   A¹ in Rb is a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² is oxygen atom, sulfur atom, -N(methyl)-, or -N(ethyl)-, A¹ is ethylene group, methylethylene group, dimethylethylene group, ethylethylene group, phenylethylene group, ethenylene group, trimethylene group, or methyltrimethylene group);
   R² and R³ independently represent hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group;
   Rz has the same meaning as that of Rx, or is methyl group, or ethyl group;
   Ry is hydrogen atom, methyl group, ethyl group, propyl group, isopropyl group, butyl group, isobutyl group, pentyl group, or isopentyl group (provided that when Rz is methyl group, or ethyl group, Ry is a substituent other than hydrogen atom), or may bind to Rz to form, together with the nitrogen atom, a cyclic substituent (qy) selected from pyrrolidino group, piperidino group, homopiperidino group, morpholino group, piperazino group, homopiperazino group, pyrrol-1-yl group, imidazol-1-yl group, pyrazol-1-yl group, and thiomorpholino group, and the cyclic substituent (qy) may be substituted with one of alkyl group having 1 to 8 carbon atoms or two of the same or different alkyl groups having 1 to 8 carbon atoms, phenyl group, or phenylmethyl group (provided that the benzene ring of the phenyl group or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group);
   the substituent V¹ on the ring (E) is Zx, and V² on the ring (E) is AR;
   Zx is hydrogen atom, fluorine atom, chlorine atom, bromine atom, nitro group,
   methyl group, hydroxyl group, methoxy group, amino group, N-methylamino group, N-ethylamino group, N-propylamino group, N-isopropylamino group, or N,N-dimethylamino group;
   AR is naphthalen-2-yl group, naphthalen-1-yl group, benzofuran-5-yl group, benzofuran-4-yl group, benzofuran-2-yl group, benzo[b]thiophen-5-yl group, benzo[b]thiophen-4-yl group, benzo[b]thiophen-2-yl group, indol-5-yl group, indol-4-yl group, indol-6-yl group, benzothiazol-6-yl group, benzothiazol-7-yl group, benzothiazol-5-yl group, benzothiazol-4-yl group, dihydro-3H-benzothiazol-6-yl group, dihydro-3H-benzothiazol-7-yl group, dihydro-3H-benzothiazol-5-yl group, dihydro-3H-benzothiazol-4-yl group, quinolin-6-yl group, quinolin-3-yl group, quinolin-5-yl group, quinolin-7-yl group, dihydro-1H-quinolin-6-yl group, dihydro-1H-quinolin-5-yl group, benzo[d]isothiazol-5-yl group, benzo[d]isothiazol-4-yl group, benzo[d]isothiazol-6-yl group, benzo[d]isothiazol-7-yl group, 1H-indazol-5-yl group, 1H-indazol-4-yl group, 1H-indazol-6-yl group, benzo[c]isothiazol-5-yl group, benzo[c]isothiazol-4-yl group, benzo[c]isothiazol-6-yl group, benzo[c]isothiazol-7-yl group, 2H-indazol-5-yl group, 2H-indazol-4-yl group, 2H-indazol-6-yl group, imidazo[1,2-a]pyridin-6-yl group, imidazo[1,2-a]pyridin-7-yl group, 1H-pyrrolo[2,3-b]pyridin-5-yl group, 1H-pyrrolo[2,3-b]pyridin-4-yl group, isoquinolin-6-yl group, isoquinolin-3-yl group, isoquinolin-5-yl group, isoquinolin-7-yl group, dihydro-2H-isoquinolin-6-yl group, dihydro-2H-isoquinolin-5-yl group, cinnolin-6-yl group, cinnolin-5-yl group, quinazolin-6-yl group, quinazolin-7-yl group, quinazolin-5-yl group, quinoxalin-2-yl group, quinoxalin-6-yl group, quinoxalin-5-yl group, 1H-benzimidazol-5-yl group, 1H-benzimidazol-4-yl group, benzoxazol-5-yl group, benzoxazol-6-yl group, benzoxazol-4-yl group, benzoxazol-7-yl group, 1H-pyrrolo[3,2-b]pyridin-5-yl group, 1H-pyrrolo[3,2-b]pyridin-6-yl group, benzo[1,2,5]thiadiazol-5-yl group, benzo[1,2,5]thiadiazol-4-yl group, 1H-benzotriazol-5-yl group, 1H-benzotriazol-4-yl group, 1,3-dihydropyrrolo[2,3-b]pyridin-5-yl group, 1,3-dihydropyrrolo[2,3-b]pyridin-4-yl group, 1,3-dihydrobenzimidazol-5-yl group, 1,3-dihydrobenzimidazol-4-yl group, dihydro-3H-benzoxazol-6-yl group, dihydro-3H-benzoxazol-7-yl group, dihydro-3H-benzoxazol-5-yl group, dihydro-3H-benzoxazol-4-yl group, phthalazin-6-yl group, phthalazin-5-yl group, [1,8]naphthylidin-3-yl group, [1,8]naphthylidin-4-yl group, [1,5]naphthylidin-3-yl group, [1,5]naphthylidin-4-yl group, 1H-pyrrolo[3,2-c]pyridin-6-yl group, 1H-pyrrolo[3,2-c]pyridin-4-yl group, 1H-pyrrolo[2,3-c]pyridin-5-yl group, 1H-pyrrolo[2,3-c]pyridin-4-yl group, 1H-pyrazolo[4,3-b]pyridin-5-yl group, 1H-pyrazolo[4,3-b]pyridin-6-yl group, 1H-pyrazolo[4,3-c]pyridin-6-yl group, 1H-pyrazolo[4,3-c]pyridin-4-yl group, 1H-pyrazolo[3,4-c]pyridin-5-yl group, 1H-pyrazolo[3,4-c]pyridin-4-yl group, 1H-pyrazolo[3,4-b]pyridin-5-yl group, 1H-pyrazolo[3,4-b]pyridin-4-yl group, [1,2,4]triazolo[4,3-a]pyridin-6-yl group, [1,2,4]triazolo[4,3-a]pyridin-7-yl group, thieno[3,2-c]pyridin-2-yl group, thieno[3,2-c]pyridin-3-yl group, thieno[3,2-c]pyridin-6-yl group, thieno[3,2-b]pyridin-2-yl group, thieno[3,2-b]pyridin-3-yl group, thieno[3,2-b]pyridin-5-yl group, thieno[3,2-b]pyridin-6-yl group, 1H-thieno[3,2-c]pyrazol-5-yl group, 1H-thieno[3,2-c]pyrazol-4-yl group, benzo[d]isoxazol-5-yl group, benzo[d]isoxazol-4-yl group, benzo[d]isoxazol-6-yl group, benzo[d]isoxazol-7-yl group, benzo[c]isoxazol-5-yl group, benzo[c]isoxazol-4-yl group, benzo[c]isoxazol-6-yl group, benzo[c]isoxazol-7-yl group, indolizin-7-yl group, indolizin-6-yl group, indolizin-8-yl group, 1,3-dihydroindol-5-yl group, 1,3-dihydroindol-4-yl group, 1,3-dihydroindol-6-yl group, 1H-pyrazolo[3,4-d]thiazol-5-yl group, 2H-isoindol-5-yl group, 2H-isoindol-4-yl group, [1,2,4]triazolo[1,5-a]pyrimidin-6-yl group, 1H-pyrazolo[3,4-b]pyrazin-5-yl group, 1H-imidazo[4,5-b]pyrazin-5-yl group, 7H-purin-2-yl group, 4H-chromen-6-yl group, or 4H-chromen-5-yl group (the aforementioned groups may be substituted with one of Xa or two or more of the same or different Xa);
   Xa is oxo group, thioxo group, fluorine atom, chlorine atom, trifluoromethyl group, methyl group, ethyl group, propyl group, 2-hydroxyethyl group, carboxymethyl group, 2-carboxyethyl group, N,N-dimethylcarbamoylmethyl group, hydroxyl group, methoxy group, 2-hydroxyethyloxy group, carboxymethyloxy group, 2-carboxyethyloxy group, N,N-dimethylcarbamoylmethyloxy group, amino group, methylamino group, dimethylamino group, 2-hydroxyethylamino group, carbamoylamino group, acetylamino group, furan-2-carboxyamino group, 2-hydroxyacetylamino group, 2-aminoacetylamino group, methylsulfonylamino group, (N,N-dimethylsulfamoyl)amino group, methanesulfonyl group, sulfamoyl group, N-methylsulfamoyl group, N,N-dimethylsulfamoyl group, carboxyl group, acetyl group, carbamoyl group, or N,N-dimethylcarbamoyl group; and
   Y is hydrogen atom, or a lower alkyl group having 1 to 4 carbon atoms.

<32> The compound or salt thereof according to <1> mentioned above, wherein, in the formula (I), the bond between the ring-constituting carbon atom at the 1-position of the ring (E) and the ring-constituting carbon atom at the 2-position of the ring (E) is a double bond;
   the bond between the ring-constituting carbon atom at the 1-position of the ring (E) and the Link moiety is a single bond;
   Link is CH₂, or CH₂CH₂;
   W is NH, or N(methyl);
   Rs is -D-Rx or -N(Ry)(Rz);
   D is a single bond, oxygen atom, or sulfur atom;
   Rx is propyl group, isopropyl group, butyl group, isobutyl group, pentyl group,
   isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb mentioned above;
   Q in Rb is phenyl group, thienyl group, furyl group, pyridyl group, oxazolyl group, naphthyl group, tetrahydronaphthyl group, indanyl group, indolyl group, or dihydrobenzodioxyl group;
   A² in Rb is a single bond, oxygen atom, sulfur atom, -N(methyl)-, or -N(ethyl)-;
   A¹ in Rb is a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² is oxygen atom, sulfur atom, -N(methyl)-, or -N(ethyl)-, A¹ is ethylene group, methylethylene group, dimethylethylene group, ethylethylene group, phenylethylene group, ethenylene group, trimethylene group, or methyltrimethylene group);
   R² and R³ independently represent hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group;
   Rz has the same meaning as that of Rx, or is methyl group, or ethyl group;
   Ry is hydrogen atom, methyl group, ethyl group, propyl group, isopropyl group, butyl group, isobutyl group, pentyl group, or isopentyl group (provided that when Rz is methyl group, or ethyl group, Ry is a substituent other than hydrogen atom), or may bind to Rz to form, together with the nitrogen atom, a cyclic substituent (qy) selected from pyrrolidino group, piperidino group, homopiperidino group, morpholino group, piperazino group, homopiperazino group, pyrrol-1-yl group, imidazol-1-yl group, pyrazol-1-yl group, and thiomorpholino group, and the cyclic substituent (qy) may be substituted with one of alkyl group having 1 to 8 carbon atoms or two of the same or different alkyl groups having 1 to 8 carbon atoms, phenyl group, or phenylmethyl group (provided that the benzene ring of the phenyl group or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group);
   the substituent V¹ on the ring (E) is Zx, and V² on the ring (E) is AR;
   Zx is hydrogen atom, fluorine atom, chlorine atom, bromine atom, nitro group, methyl group, hydroxyl group, methoxy group, amino group, N-methylamino group, N-ethylamino group, N-propylamino group, N-isopropylamino group, or N,N-dimethylamino group;
   AR is naphthalen-2-yl group, naphthalen-1-yl group, benzofuran-5-yl group, benzofuran-4-yl group, benzofuran-2-yl group, benzo[b]thiophen-5-yl group, benzo[b]thiophen-4-yl group, benzo[b]thiophen-2-yl group, indol-5-yl group, indol-4-yl group, indol-6-yl group, benzothiazol-6-yl group, benzothiazol-7-yl group, benzothiazol-5-yl group, benzothiazol-4-yl group, dihydro-3H-benzothiazol-6-yl group, dihydro-3H-benzothiazol-7-yl group, dihydro-3H-benzothiazol-5-yl group, dihydro-3H-benzothiazol-4-yl group, quinolin-6-yl group, quinolin-3-yl group, quinolin-5-yl group, quinolin-7-yl group, dihydro-1H-quinolin-6-yl group, dihydro-1H-quinolin-5-yl group, benzo[d]isothiazol-5-yl group, benzo[d]isothiazol-4-yl group, benzo[d]isothiazol-6-yl group, benzo[d]isothiazol-7-yl group, 1H-indazol-5-yl group, 1H-indazol-4-yl group, 1H-indazol-6-yl group, benzo[c]isothiazol-5-yl group, benzo[c]isothiazol-4-yl group, benzo[c]isothiazol-6-yl group, benzo[c]isothiazol-7-yl group, 2H-indazol-5-yl group, 2H-indazol-4-yl group, 2H-indazol-6-yl group, imidazo[1,2-a]pyridin-6-yl group, imidazo[1,2-a]pyridin-7-yl group, 1H-pyrrolo[2,3-b]pyridin-5-yl group, 1H-pyrrolo[2,3-b]pyridin-4-yl group, isoquinolin-6-yl group, isoquinolin-3-yl group, isoquinolin-5-yl group, isoquinolin-7-yl group, dihydro-2H-isoquinolin-6-yl group, dihydro-2H-isoquinolin-5-yl group, cinnolin-6-yl group, cinnolin-5-yl group, quinazolin-6-yl group, quinazolin-7-yl group, quinazolin-5-yl group, quinoxalin-2-yl group, quinoxalin-6-yl group, quinoxalin-5-yl group, 1H-benzimidazol-5-yl group, 1H-benzimidazol-4-yl group, benzoxazol-5-yl group, benzoxazol-6-yl group, benzoxazol-4-yl group, benzoxazol-7-yl group, 1H-pyrrolo[3,2-b]pyridin-5-yl group, 1H-pyrrolo[3,2-b]pyridin-6-yl group, benzo[1,2,5]thiadiazol-5-yl group, benzo[1,2,5]thiadiazol-4-yl group, 1H-benzotriazol-5-yl group, 1H-benzotriazol-4-yl group, 1,3-dihydropyrrolo[2,3-b]pyridin-5-yl group, 1,3-dihydropyrrolo[2,3-b]pyridin-4-yl group, 1,3-dihydrobenzimidazol-5-yl group, 1,3-dihydrobenzimidazol-4-yl group, dihydro-3H-benzoxazol-6-yl group, dihydro-3H-benzoxazol-7-yl group, dihydro-3H-benzoxazol-5-yl group, dihydro-3H-benzoxazol-4-yl group, phthalazin-6-yl group, phthalazin-5-yl group, [1,8]naphthylidin-3-yl group, [1,8]naphthylidin-4-yl group, [1,5]naphthylidin-3-yl group, [1,5]naphthylidin-4-yl group, 1H-pyrrolo[3,2-c]pyridin-6-yl group, 1H-pyrrolo[3,2-c]pyridin-4-yl group, 1H-pyrrolo[2,3-c]pyridin-5-yl group, 1H-pyrrolo[2,3-c]pyridin-4-yl group, 1H-pyrazolo[4,3-b]pyridin-5-yl group, 1H-pyrazolo[4,3-b]pyridin-6-yl group, 1H-pyrazolo[4,3-c]pyridin-6-yl group, 1H-pyrazolo[4,3-c]pyridin-4-yl group, 1H-pyrazolo[3,4-c]pyridin-5-yl group, 1H-pyrazolo[3,4-c]pyridin-4-yl group, 1H-pyrazolo[3,4-b]pyridin-5-yl group, 1H-pyrazolo[3,4-b]pyridin-4-yl group, [1,2,4]triazolo[4,3-a]pyridin-6-yl group, [1,2,4]triazolo[4,3-a]pyridin-7-yl group, thieno[3,2-c]pyridin-2-yl group, thieno[3,2-c]pyridin-3-yl group, thieno[3,2-c]pyridin-6-yl group, thieno[3,2-b]pyridin-2-yl group, thieno[3,2-b]pyridin-3-yl group, thieno[3,2-b]pyridin-5-yl group, thieno[3,2-b]pyridin-6-yl group, 1H-thieno[3,2-c]pyrazol-5-yl group, 1H-thieno[3,2-c]pyrazol-4-yl group, benzo[d]isoxazol-5-yl group, benzo[d]isoxazol-4-yl group, benzo[d]isoxazol-6-yl group, benzo[d]isoxazol-7-yl group, benzo[c]isoxazol-5-yl group, benzo[c]isoxazol-4-yl group, benzo[c]isoxazol-6-yl group, benzo[c]isoxazol-7-yl group, indolizin-7-yl group, indolizin-6-yl group, indolizin-8-yl group, 1,3-dihydroindol-5-yl group, 1,3-dihydroindol-4-yl group, 1,3-dihydroindol-6-yl group, 1H-pyrazolo[3,4-d]thiazol-5-yl group, 2H-isoindol-5-yl group, 2H-isoindol-4-yl group, [1,2,4]triazolo[1,5-a]pyrimidin-6-yl group, 1H-pyrazolo[3,4-b]pyrazin-5-yl group, 1H-imidazo[4,5-b]pyrazin-5-yl group, 7H-purin-2-yl group, 4H-chromen-6-yl group, or 4H-chromen-5-yl group (the aforementioned groups may be substituted with one of Xa or two or more of the same or different Xa);
   Xa is oxo group, thioxo group, fluorine atom, chlorine atom, trifluoromethyl group, methyl group, ethyl group, propyl group, 2-hydroxyethyl group, carboxymethyl group, 2-carboxyethyl group, N,N-dimethylcarbamoylmethyl group, hydroxyl group, methoxy group, 2-hydroxyethyloxy group, carboxymethyloxy group, 2-carboxyethyloxy group, N,N-dimethylcarbamoylmethyloxy group, amino group, methylamino group, dimethylamino group, 2-hydroxyethylamino group, carbamoylamino group, acetylamino group, furan-2-carboxyamino group, 2-hydroxyacetylamino group, 2-aminoacetylamino group, methylsulfonylamino group, (N,N-dimethylsulfamoyl)amino group, methanesulfonyl group, sulfamoyl group, N-methylsulfamoyl group, N,N-dimethylsulfamoyl group, carboxyl group, acetyl group, carbamoyl group, or N,N-dimethylcarbamoyl group; and
   Y is hydrogen atom, or a lower alkyl group having 1 to 4 carbon atoms.

<33> The compound or salt thereof according to <1> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond or a double bond;
   when the bond between the ring-constituting carbon atom at the 1-position of the ring (E) and the Link moiety is a single bond, Link is CH₂ or CH₂CH₂, or when the bond between the ring-constituting carbon atom at the 1-position of the ring (E) and the Link moiety is a double bond, Link is CH;
   W is methylene group;
   Rs is Rx;
   Rx is propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb mentioned above;
   Q in Rb is phenyl group, thienyl group, furyl group, pyridyl group, oxazolyl group, naphthyl group, tetrahydronaphthyl group, indanyl group, indolyl group, or dihydrobenzodioxyl group;
   A² in Rb is a single bond, oxygen atom, sulfur atom, -N(methyl)-, or -N(ethyl)-;
   A¹ in Rb is a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² is oxygen atom, sulfur atom, -N(methyl)-, or -N(ethyl)-, A¹ is ethylene group, methylethylene group, dimethylethylene group, ethylethylene group, phenylethylene group, ethenylene group, trimethylene group, or methyltrimethylene group);
   R² and R³ independently represent hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group;
   the substituent V¹ on the ring (E) is Zx, and V² on the ring (E) is AR;
   Zx is hydrogen atom, fluorine atom, chlorine atom, bromine atom, nitro group, methyl group, hydroxyl group, methoxy group, amino group, N-methylamino group, N-ethylamino group, N-propylamino group, N-isopropylamino group, or N,N-dimethylamino group;
   AR is naphthalen-2-yl group, naphthalen-1-yl group, benzofuran-5-yl group, benzofuran-4-yl group, benzofuran-2-yl group, benzo[b]thiophen-5-yl group, benzo[b]thiophen-4-yl group, benzo[b]thiophen-2-yl group, indol-5-yl group, indol-4-yl group, indol-6-yl group, benzothiazol-6-yl group, benzothiazol-7-yl group, benzothiazol-5-yl group, benzothiazol-4-yl group, dihydro-3H-benzothiazol-6-yl group, dihydro-3H-benzothiazol-7-yl group, dihydro-3H-benzothiazol-5-yl group, dihydro-3H-benzothiazol-4-yl group, quinolin-6-yl group, quinolin-3-yl group, quinolin-5-yl group, quinolin-7-yl group, dihydro-1H-quinolin-6-yl group, dihydro-1H-quinolin-5-yl group, benzo[d]isothiazol-5-yl group, benzo[d]isothiazol-4-yl group, benzo[d]isothiazol-6-yl group, benzo[d]isothiazol-7-yl group, 1H-indazol-5-yl group, 1H-indazol-4-yl group, 1H-indazol-6-yl group, benzo[c]isothiazol-5-yl group, benzo[c]isothiazol-4-yl group, benzo[c]isothiazol-6-yl group, benzo[c]isothiazol-7-yl group, 2H-indazol-5-yl group, 2H-indazol-4-yl group, 2H-indazol-6-yl group, imidazo[1,2-a]pyridin-6-yl group, imidazo[1,2-a]pyridin-7-yl group, 1H-pyrrolo[2,3-b]pyridin-5-yl group, 1H-pyrrolo[2,3-b]pyridin-4-yl group, isoquinolin-6-yl group, isoquinolin-3-yl group, isoquinolin-5-yl group, isoquinolin-7-yl group, dihydro-2H-isoquinolin-6-yl group, dihydro-2H-isoquinolin-5-yl group, cinnolin-6-yl group, cinnolin-5-yl group, quinazolin-6-yl group, quinazolin-7-yl group, quinazolin-5-yl group, quinoxalin-2-yl group, quinoxalin-6-yl group, quinoxalin-5-yl group, 1H-benzimidazol-5-yl group, 1H-benzimidazol-4-yl group, benzoxazol-5-yl group, benzoxazol-6-yl group, benzoxazol-4-yl group, benzoxazol-7-yl group, 1H-pyrrolo[3,2-b]pyridin-5-yl group, 1H-pyrrolo[3,2-b]pyridin-6-yl group, benzo[1,2,5]thiadiazol-5-yl group, benzo[1,2,5]thiadiazol-4-yl group, 1H-benzotriazol-5-yl group, 1H-benzotriazol-4-yl group, 1,3-dihydropyrrolo[2,3-b]pyridin-5-yl group, 1,3-dihydropyrrolo[2,3-b]pyridin-4-yl group, 1,3-dihydrobenzimidazol-5-yl group, 1,3-dihydrobenzimidazol-4-yl group, dihydro-3H-benzoxazol-6-yl group, dihydro-3H-benzoxazol-7-yl group, dihydro-3H-benzoxazol-5-yl group, dihydro-3H-benzoxazol-4-yl group, phthalazin-6-yl group, phthalazin-5-yl group, [1,8]naphthylidin-3-yl group, [1,8]naphthylidin-4-yl group, [1,5]naphthylidin-3-yl group, [1,5]naphthylidin-4-yl group, 1H-pyrrolo[3,2-c]pyridin-6-yl group, 1H-pyrrolo[3,2-c]pyridin-4-yl group, 1H-pyrrolo[2,3-c]pyridin-5-yl group, 1H-pyrrolo[2,3-c]pyridin-4-yl group, 1H-pyrazolo[4,3-b]pyridin-5-yl group, 1H-pyrazolo[4,3-b]pyridin-6-yl group, 1H-pyrazolo[4,3-c]pyridin-6-yl group, 1H-pyrazolo[4,3-c]pyridin-4-yl group, 1H-pyrazolo[3,4-c]pyridin-5-yl group, 1H-pyrazolo[3,4-c]pyridin-4-yl group, 1H-pyrazolo[3,4-b]pyridin-5-yl group, 1H-pyrazolo[3,4-b]pyridin-4-yl group, [1,2,4]triazolo[4,3-a]pyridin-6-yl group, [1,2,4]triazolo[4,3-a]pyridin-7-yl group, thieno[3,2-c]pyridin-2-yl group, thieno[3,2-c]pyridin-3-yl group, thieno[3,2-c]pyridin-6-yl group, thieno[3,2-b]pyridin-2-yl group, thieno[3,2-b]pyridin-3-yl group, thieno[3,2-b]pyridin-5-yl group, thieno[3,2-b]pyridin-6-yl group, 1H-thieno[3,2-c]pyrazol-5-yl group, 1H-thieno[3,2-c]pyrazol-4-yl group, benzo[d]isoxazol-5-yl group, benzo[d]isoxazol-4-yl group, benzo[d]isoxazol-6-yl group, benzo[d]isoxazol-7-yl group, benzo[c]isoxazol-5-yl group, benzo[c]isoxazol-4-yl group, benzo[c]isoxazol-6-yl group, benzo[c]isoxazol-7-yl group, indolizin-7-yl group, indolizin-6-yl group, indolizin-8-yl group, 1,3-dihydroindol-5-yl group, 1,3-dihydroindol-4-yl group, 1,3-dihydroindol-6-yl group, 1H-pyrazolo[3,4-d]thiazol-5-yl group, 2H-isoindol-5-yl group, 2H-isoindol-4-yl group, [1,2,4]triazolo[1,5-a]pyrimidin-6-yl group, 1H-pyrazolo[3,4-b]pyrazin-5-yl group, 1H-imidazo[4,5-b]pyrazin-5-yl group, 7H-purin-2-yl group, 4H-chromen-6-yl group, or 4H-chromen-5-yl group (the aforementioned groups may be substituted with one of Xa or two or more of the same or different Xa);
   Xa is oxo group, thioxo group, fluorine atom, chlorine atom, trifluoromethyl group, methyl group, ethyl group, propyl group, 2-hydroxyethyl group, carboxymethyl group, 2-carboxyethyl group, N,N-dimethylcarbamoylmethyl group, hydroxyl group, methoxy group, 2-hydroxyethyloxy group, carboxymethyloxy group, 2-carboxyethyloxy group, N,N-dimethylcarbamoylmethyloxy group, amino group, methylamino group, dimethylamino group, 2-hydroxyethylamino group, carbamoylamino group, acetylamino group, furan-2-carboxyamino group, 2-hydroxyacetylamino group, 2-aminoacetylamino group, methylsulfonylamino group, (N,N-dimethylsulfamoyl)amino group, methanesulfonyl group, sulfamoyl group, N-methylsulfamoyl group, N,N-dimethylsulfamoyl group, carboxyl group, acetyl group, carbamoyl group, or N,N-dimethylcarbamoyl group; and
   Y is hydrogen atom, or a lower alkyl group having 1 to 4 carbon atoms.

<34> The compound or salt thereof according to <1> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond or a double bond;
   when the bond between the ring-constituting carbon atom at the 1-position of the ring (E) and the Link moiety is a single bond, Link is CH₂ or CH₂CH₂, or when the bond between the ring-constituting carbon atom at the 1-position of the ring (E) and the Link moiety is a double bond, Link is CH;
   W is methylene group;
   Rs is -O-Rx;
   Rx is propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb mentioned above;
   Q in Rb is phenyl group, thienyl group, furyl group, pyridyl group, oxazolyl group, naphthyl group, tetrahydronaphthyl group, indanyl group, indolyl group, or dihydrobenzodioxyl group;
   A² in Rb is a single bond, oxygen atom, sulfur atom, -N(methyl)-, or -N(ethyl)-;
   A¹ in Rb is a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² is oxygen atom, sulfur atom, -N(methyl)-, or -N(ethyl)-, A¹ is ethylene group, methylethylene group, dimethylethylene group, ethylethylene group, phenylethylene group, ethenylene group, trimethylene group, or methyltrimethylene group);
   R² and R³ independently represent hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group;
   the substituent V¹ on the ring (E) is Zx, and V² on the ring (E) is AR;
   Zx is hydrogen atom, fluorine atom, chlorine atom, bromine atom, nitro group, methyl group, hydroxyl group, methoxy group, amino group, N-methylamino group, N-ethylamino group, N-propylamino group, N-isopropylamino group, or N,N-dimethylamino group;
   AR is naphthalen-2-yl group, naphthalen-1-yl group, benzofuran-5-yl group, benzofuran-4-yl group, benzofuran-2-yl group, benzo[b]thiophen-5-yl group, benzo[b]thiophen-4-yl group, benzo[b]thiophen-2-yl group, indol-5-yl group, indol-4-yl group, indol-6-yl group, benzothiazol-6-yl group, benzothiazol-7-yl group, benzothiazol-5-yl group, benzothiazol-4-yl group, dihydro-3H-benzothiazol-6-yl group, dihydro-3H-benzothiazol-7-yl group, dihydro-3H-benzothiazol-5-yl group, dihydro-3H-benzothiazol-4-yl group, quinolin-6-yl group, quinolin-3-yl group, quinolin-5-yl group, quinolin-7-yl group, dihydro-1H-quinolin-6-yl group, dihydro-1H-quinolin-5-yl group, benzo[d]isothiazol-5-yl group, benzo[d]isothiazol-4-yl group, benzo[d]isothiazol-6-yl group, benzo[d]isothiazol-7-yl group, 1H-indazol-5-yl group, 1H-indazol-4-yl group, 1H-indazol-6-yl group, benzo[c]isothiazol-5-yl group, benzo[c]isothiazol-4-yl group, benzo[c]isothiazol-6-yl group, benzo[c]isothiazol-7-yl group, 2H-indazol-5-yl group, 2H-indazol-4-yl group, 2H-indazol-6-yl group, imidazo[1,2-a]pyridin-6-yl group, imidazo[1,2-a]pyridin-7-yl group, 1H-pyrrolo[2,3-b]pyridin-5-yl group, 1H-pyrrolo[2,3-b]pyridin-4-yl group, isoquinolin-6-yl group, isoquinolin-3-yl group, isoquinolin-5-yl group, isoquinolin-7-yl group, dihydro-2H-isoquinolin-6-yl group, dihydro-2H-isoquinolin-5-yl group, cinnolin-6-yl group, cinnolin-5-yl group, quinazolin-6-yl group, quinazolin-7-yl group, quinazolin-5-yl group, quinoxalin-2-yl group, quinoxalin-6-yl group, quinoxalin-5-yl group, 1H-benzimidazol-5-yl group, 1H-benzimidazol-4-yl group, benzoxazol-5-yl group, benzoxazol-6-yl group, benzoxazol-4-yl group, benzoxazol-7-yl group, 1H-pyrrolo[3,2-b]pyridin-5-yl group, 1H-pyrrolo[3,2-b]pyridin-6-yl group, benzo[1,2,5]thiadiazol-5-yl group, benzo[1,2,5]thiadiazol-4-yl group, 1H-benzotriazol-5-yl group, 1H-benzotriazol-4-yl group, 1,3-dihydropyrrolo[2,3-b]pyridin-5-yl group, 1,3-dihydropyrrolo[2,3-b]pyridin-4-yl group, 1,3-dihydrobenzimidazol-5-yl group, 1,3-dihydrobenzimidazol-4-yl group, dihydro-3H-benzoxazol-6-yl group, dihydro-3H-benzoxazol-7-yl group, dihydro-3H-benzoxazol-5-yl group, dihydro-3H-benzoxazol-4-yl group, phthalazin-6-yl group, phthalazin-5-yl group, [1,8]naphthylidin-3-yl group, [1,8]naphthylidin-4-yl group, [1,5]naphthylidin-3-yl group, [1,5]naphthylidin-4-yl group, 1H-pyrrolo[3,2-c]pyridin-6-yl group, 1H-pyrrolo[3,2-c]pyridin-4-yl group, 1H-pyrrolo[2,3-c]pyridin-5-yl group, 1H-pyrrolo[2,3-c]pyridin-4-yl group, 1H-pyrazolo[4,3-b]pyridin-5-yl group, 1H-pyrazolo[4,3-b]pyridin-6-yl group, 1H-pyrazolo[4,3-c]pyridin-6-yl group, 1H-pyrazolo[4,3-c]pyridin-4-yl group, 1H-pyrazolo[3,4-c]pyridin-5-yl group, 1H-pyrazolo[3,4-c]pyridin-4-yl group, 1H-pyrazolo[3,4-b]pyridin-5-yl group, 1H-pyrazolo[3,4-b]pyridin-4-yl group, [1,2,4]triazolo[4,3-a]pyridin-6-yl group, [1,2,4]triazolo[4,3-a]pyridin-7-yl group, thieno[3,2-c]pyridin-2-yl group, thieno[3,2-c]pyridin-3-yl group, thieno[3,2-c]pyridin-6-yl group, thieno[3,2-b]pyridin-2-yl group, thieno[3,2-b]pyridin-3-yl group, thieno[3,2-b]pyridin-5-yl group, thieno[3,2-b]pyridin-6-yl group, 1H-thieno[3,2-c]pyrazol-5-yl group, 1H-thieno[3,2-c]pyrazol-4-yl group, benzo[d]isoxazol-5-yl group, benzo[d]isoxazol-4-yl group, benzo[d]isoxazol-6-yl group, benzo[d]isoxazol-7-yl group, benzo[c]isoxazol-5-yl group, benzo[c]isoxazol-4-yl group, benzo[c]isoxazol-6-yl group, benzo[c]isoxazol-7-yl group, indolizin-7-yl group, indolizin-6-yl group, indolizin-8-yl group, 1,3-dihydroindol-5-yl group, 1,3-dihydroindol-4-yl group, 1,3-dihydroindol-6-yl group, 1H-pyrazolo[3,4-d]thiazol-5-yl group, 2H-isoindol-5-yl group, 2H-isoindol-4-yl group, [1,2,4]triazolo[1,5-a]pyrimidin-6-yl group, 1H-pyrazolo[3,4-b]pyrazin-5-yl group, 1H-imidazo[4,5-b]pyrazin-5-yl group, 7H-purin-2-yl group, 4H-chromen-6-yl group, or 4H-chromen-5-yl group (the aforementioned groups may be substituted with one of Xa or two or more of the same or different Xa);
   Xa is oxo group, thioxo group, fluorine atom, chlorine atom, trifluoromethyl group, methyl group, ethyl group, propyl group, 2-hydroxyethyl group, carboxymethyl group, 2-carboxyethyl group, N,N-dimethylcarbamoylmethyl group, hydroxyl group, methoxy group, 2-hydroxyethyloxy group, carboxymethyloxy group, 2-carboxyethyloxy group, N,N-dimethylcarbamoylmethyloxy group, amino group, methylamino group, dimethylamino group, 2-hydroxyethylamino group, carbamoylamino group, acetylamino group, furan-2-carboxyamino group, 2-hydroxyacetylamino group, 2-aminoacetylamino group, methylsulfonylamino group, (N,N-dimethylsulfamoyl)amino group, methanesulfonyl group, sulfamoyl group, N-methylsulfamoyl group, N,N-dimethylsulfamoyl group, carboxyl group, acetyl group, carbamoyl group, or N,N-dimethylcarbamoyl group; and
   Y is hydrogen atom, or a lower alkyl group having 1 to 4 carbon atoms.

<35> The compound or salt thereof according to <1> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond or a double bond;
   when the bond between the ring-constituting carbon atom at the 1-position of the ring (E) and the Link moiety is a single bond, Link is CH₂ or CH₂CH₂, or when the bond between the ring-constituting carbon atom at the 1-position of the ring (E) and the Link moiety is a double bond, Link is CH;
   W is methylene group;
   Rs is -S-Rx;
   Rx is propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb mentioned above;
   Q in Rb is phenyl group, thienyl group, furyl group, pyridyl group, oxazolyl group, naphthyl group, tetrahydronaphthyl group, indanyl group, indolyl group, or dihydrobenzodioxyl group;
   A² in Rb is a single bond, oxygen atom, sulfur atom, -N(methyl)-, or -N(ethyl)-;
   A¹ in Rb is a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² is oxygen atom, sulfur atom, -N(methyl)-, or -N(ethyl)-, A¹ is ethylene group, methylethylene group, dimethylethylene group, ethylethylene group, phenylethylene group, ethenylene group, trimethylene group, or methyltrimethylene group);
   R² and R³ independently represent hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group;
   the substituent V¹ on the ring (E) is Zx, and V² on the ring (E) is AR;
   Zx is hydrogen atom, fluorine atom, chlorine atom, bromine atom, nitro group, methyl group, hydroxyl group, methoxy group, amino group, N-methylamino group, N-ethylamino group, N-propylamino group, N-isopropylamino group, or N,N-dimethylamino group;
   AR is naphthalen-2-yl group, naphthalen-1-yl group, benzofuran-5-yl group, benzofuran-4-yl group, benzofuran-2-yl group, benzo[b]thiophen-5-yl group, benzo[b]thiophen-4-yl group, benzo[b]thiophen-2-yl group, indol-5-yl group, indol-4-yl group, indol-6-yl group, benzothiazol-6-yl group, benzothiazol-7-yl group, benzothiazol-5-yl group, benzothiazol-4-yl group, dihydro-3H-benzothiazol-6-yl group, dihydro-3H-benzothiazol-7-yl group, dihydro-3H-benzothiazol-5-yl group, dihydro-3H-benzothiazol-4-yl group, quinolin-6-yl group, quinolin-3-yl group, quinolin-5-yl group, quinolin-7-yl group, dihydro-1H-quinolin-6-yl group, dihydro-1H-quinolin-5-yl group, benzo[d]isothiazol-5-yl group, benzo[d]isothiazol-4-yl group, benzo[d]isothiazol-6-yl group, benzo[d]isothiazol-7-yl group, 1H-indazol-5-yl group, 1H-indazol-4-yl group, 1H-indazol-6-yl group, benzo[c]isothiazol-5-yl group, benzo[c]isothiazol-4-yl group, benzo[c]isothiazol-6-yl group, benzo[c]isothiazol-7-yl group, 2H-indazol-5-yl group, 2H-indazol-4-yl group, 2H-indazol-6-yl group, imidazo[1,2-a]pyridin-6-yl group, imidazo[1,2-a]pyridin-7-yl group, 1H-pyrrolo[2,3-b]pyridin-5-yl group, 1H-pyrrolo[2,3-b]pyridin-4-yl group, isoquinolin-6-yl group, isoquinolin-3-yl group, isoquinolin-5-yl group, isoquinolin-7-yl group, dihydro-2H-isoquinolin-6-yl group, dihydro-2H-isoquinolin-5-yl group, cinnolin-6-yl group, cinnolin-5-yl group, quinazolin-6-yl group, quinazolin-7-yl group, quinazolin-5-yl group, quinoxalin-2-yl group, quinoxalin-6-yl group, quinoxalin-5-yl group, 1H-benzimidazol-5-yl group, 1H-benzimidazol-4-yl group, benzoxazol-5-yl group, benzoxazol-6-yl group, benzoxazol-4-yl group, benzoxazol-7-yl group, 1H-pyrrolo[3,2-b]pyridin-5-yl group, 1H-pyrrolo[3,2-b]pyridin-6-yl group, benzo[1,2,5]thiadiazol-5-yl group, benzo[1,2,5]thiadiazol-4-yl group, 1H-benzotriazol-5-yl group, 1H-benzotriazol-4-yl group, 1,3-dihydropyrrolo[2,3-b]pyridin-5-yl group, 1,3-dihydropyrrolo[2,3-b]pyridin-4-yl group, 1,3-dihydrobenzimidazol-5-yl group, 1,3-dihydrobenzimidazol-4-yl group, dihydro-3H-benzoxazol-6-yl group, dihydro-3H-benzoxazol-7-yl group, dihydro-3H-benzoxazol-5-yl group, dihydro-3H-benzoxazol-4-yl group, phthalazin-6-yl group, phthalazin-5-yl group, [1,8]naphthylidin-3-yl group, [1,8]naphthylidin-4-yl group, [1,5]naphthylidin-3-yl group, [1,5]naphthylidin-4-yl group, 1H-pyrrolo[3,2-c]pyridin-6-yl group, 1H-pyrrolo[3,2-c]pyridin-4-yl group, 1H-pyrrolo[2,3-c]pyridin-5-yl group, 1H-pyrrolo[2,3-c]pyridin-4-yl group, 1H-pyrazolo[4,3-b]pyridin-5-yl group, 1H-pyrazolo[4,3-b]pyridin-6-yl group, 1H-pyrazolo[4,3-c]pyridin-6-yl group, 1H-pyrazolo[4,3-c]pyridin-4-yl group, 1H-pyrazolo[3,4-c]pyridin-5-yl group, 1H-pyrazolo[3,4-c]pyridin-4-yl group, 1H-pyrazolo[3,4-b]pyridin-5-yl group, 1H-pyrazolo[3,4-b]pyridin-4-yl group, [1,2,4]triazolo[4,3-a]pyridin-6-yl group, [1,2,4]triazolo[4,3-a]pyridin-7-yl group, thieno[3,2-c]pyridin-2-yl group, thieno[3,2-c]pyridin-3-yl group, thieno[3,2-c]pyridin-6-yl group, thieno[3,2-b]pyridin-2-yl group, thieno[3,2-b]pyridin-3-yl group, thieno[3,2-b]pyridin-5-yl group, thieno[3,2-b]pyridin-6-yl group, 1H-thieno[3,2-c]pyrazol-5-yl group, 1H-thieno[3,2-c]pyrazol-4-yl group, benzo[d]isoxazol-5-yl group, benzo[d]isoxazol-4-yl group, benzo[d]isoxazol-6-yl group, benzo[d]isoxazol-7-yl group, benzo[c]isoxazol-5-yl group, benzo[c]isoxazol-4-yl group, benzo[c]isoxazol-6-yl group, benzo[c]isoxazol-7-yl group, indolizin-7-yl group, indolizin-6-yl group, indolizin-8-yl group, 1,3-dihydroindol-5-yl group, 1,3-dihydroindol-4-yl group, 1,3-dihydroindol-6-yl group, 1H-pyrazolo[3,4-d]thiazol-5-yl group, 2H-isoindol-5-yl group, 2H-isoindol-4-yl group, [1,2,4]triazolo[1,5-a]pyrimidin-6-yl group, 1H-pyrazolo[3,4-b]pyrazin-5-yl group, 1H-imidazo[4,5-b]pyrazin-5-yl group, 7H-purin-2-yl group, 4H-chromen-6-yl group, or 4H-chromen-5-yl group (the aforementioned groups may be substituted with one of Xa or two or more of the same or different Xa);
   Xa is oxo group, thioxo group, fluorine atom, chlorine atom, trifluoromethyl group, methyl group, ethyl group, propyl group, 2-hydroxyethyl group, carboxymethyl group, 2-carboxyethyl group, N,N-dimethylcarbamoylmethyl group, hydroxyl group, methoxy group, 2-hydroxyethyloxy group, carboxymethyloxy group, 2-carboxyethyloxy group, N,N-dimethylcarbamoylmethyloxy group, amino group, methylamino group, dimethylamino group, 2-hydroxyethylamino group, carbamoylamino group, acetylamino group, furan-2-carboxyamino group, 2-hydroxyacetylamino group, 2-aminoacetylamino group, methylsulfonylamino group, (N,N-dimethylsulfamoyl)amino group, methanesulfonyl group, sulfamoyl group, N-methylsulfamoyl group, N,N-dimethylsulfamoyl group, carboxyl group, acetyl group, carbamoyl group, or N,N-dimethylcarbamoyl group; and
   Y is hydrogen atom, or a lower alkyl group having 1 to 4 carbon atoms.

<36> The compound or salt thereof according to <1> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond or a double bond;
   when the bond between the ring-constituting carbon atom at the 1-position of the ring (E) and the Link moiety is a single bond, Link is CH₂ or CH₂CH₂, or when the bond between the ring-constituting carbon atom at the 1-position of the ring (E) and the Link moiety is a double bond, Link is CH;
   W is methylene group;
   Rs is -N(Ry)(Rz);
   Rz is methyl group, ethyl group, propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb mentioned above;
   Q in Rb is phenyl group, thienyl group, furyl group, pyridyl group, oxazolyl group, naphthyl group, tetrahydronaphthyl group, indanyl group, indolyl group, or dihydrobenzodioxyl group;
   A² in Rb is a single bond, oxygen atom, sulfur atom, -N(methyl)-, or -N(ethyl)-;
   A¹ in Rb is a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² is oxygen atom, sulfur atom, -N(methyl)-, or -N(ethyl)-, A¹ is ethylene group, methylethylene group, dimethylethylene group, ethylethylene group, phenylethylene group, ethenylene group, trimethylene group, or methyltrimethylene group);
   R² and R³ independently represent hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group;
   Ry is hydrogen atom, methyl group, ethyl group, propyl group, isopropyl group, butyl group, isobutyl group, pentyl group, or isopentyl group (provided that when Rz is methyl group, or ethyl group, Ry is a substituent other than hydrogen atom), or may bind to Rz to form, together with the nitrogen atom, a cyclic substituent (qy) selected from pyrrolidino group, piperidino group, homopiperidino group, morpholino group, piperazino group, homopiperazino group, pyrrol-1-yl group, imidazol-1-yl group, pyrazol-1-yl group, and thiomorpholino group, and the cyclic substituent (qy) may be substituted with one of alkyl group having 1 to 8 carbon atoms or two of the same or different alkyl groups having 1 to 8 carbon atoms, phenyl group, or phenylmethyl group (provided that the benzene ring of the phenyl group or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group);
   the substituent V¹ on the ring (E) is Zx, and V² on the ring (E) is AR;
   Zx is hydrogen atom, fluorine atom, chlorine atom, bromine atom, nitro group, methyl group, hydroxyl group, methoxy group, amino group, N-methylamino group, N-ethylamino group, N-propylamino group, N-isopropylamino group, or N,N-dimethylamino group;
   AR is naphthalen-2-yl group, naphthalen-1-yl group, benzofuran-5-yl group, benzofuran-4-yl group, benzofuran-2-yl group, benzo[b]thiophen-5-yl group, benzo[b]thiophen-4-yl group, benzo[b]thiophen-2-yl group, indol-5-yl group, indol-4-yl group, indol-6-yl group, benzothiazol-6-yl group, benzothiazol-7-yl group, benzothiazol-5-yl group, benzothiazol-4-yl group, dihydro-3H-benzothiazol-6-yl group, dihydro-3H-benzothiazol-7-yl group, dihydro-3H-benzothiazol-5-yl group, dihydro-3H-benzothiazol-4-yl group, quinolin-6-yl group, quinolin-3-yl group, quinolin-5-yl group, quinolin-7-yl group, dihydro-1H-quinolin-6-yl group, dihydro-1H-quinolin-5-yl group, benzo[d]isothiazol-5-yl group, benzo[d]isothiazol-4-yl group, benzo[d]isothiazol-6-yl group, benzo[d]isothiazol-7-yl group, 1H-indazol-5-yl group, 1H-indazol-4-yl group, 1H-indazol-6-yl group, benzo[c]isothiazol-5-yl group, benzo[c]isothiazol-4-yl group, benzo[c]isothiazol-6-yl group, benzo[c]isothiazol-7-yl group, 2H-indazol-5-yl group, 2H-indazol-4-yl group, 2H-indazol-6-yl group, imidazo[1,2-a]pyridin-6-yl group, imidazo[1,2-a]pyridin-7-yl group, 1H-pyrrolo[2,3-b]pyridin-5-yl group, 1H-pyrrolo[2,3-b]pyridin-4-yl group, isoquinolin-6-yl group, isoquinolin-3-yl group, isoquinolin-5-yl group, isoquinolin-7-yl group, dihydro-2H-isoquinolin-6-yl group, dihydro-2H-isoquinolin-5-yl group, cinnolin-6-yl group, cinnolin-5-yl group, quinazolin-6-yl group, quinazolin-7-yl group, quinazolin-5-yl group, quinoxalin-2-yl group, quinoxalin-6-yl group, quinoxalin-5-yl group, 1H-benzimidazol-5-yl group, 1H-benzimidazol-4-yl group, benzoxazol-5-yl group, benzoxazol-6-yl group, benzoxazol-4-yl group, benzoxazol-7-yl group, 1H-pyrrolo[3,2-b]pyridin-5-yl group, 1H-pyrrolo[3,2-b]pyridin-6-yl group, benzo[1,2,5]thiadiazol-5-yl group, benzo[1,2,5]thiadiazol-4-yl group, 1H-benzotriazol-5-yl group, 1H-benzotriazol-4-yl group, 1,3-dihydropyrrolo[2,3-b]pyridin-5-yl group, 1,3-dihydropyrrolo[2,3-b]pyridin-4-yl group, 1,3-dihydrobenzimidazol-5-yl group, 1,3-dihydrobenzimidazol-4-yl group, dihydro-3H-benzoxazol-6-yl group, dihydro-3H-benzoxazol-7-yl group, dihydro-3H-benzoxazol-5-yl group, dihydro-3H-benzoxazol-4-yl group, phthalazin-6-yl group, phthalazin-5-yl group, [1,8]naphthylidin-3-yl group, [1,8]naphthylidin-4-yl group, [1,5]naphthylidin-3-yl group, [1,5]naphthylidin-4-yl group, 1H-pyrrolo[3,2-c]pyridin-6-yl group, 1H-pyrrolo[3,2-c]pyridin-4-yl group, 1H-pyrrolo[2,3-c]pyridin-5-yl group, 1H-pyrrolo[2,3-c]pyridin-4-yl group, 1H-pyrazolo[4,3-b]pyridin-5-yl group, 1H-pyrazolo[4,3-b]pyridin-6-yl group, 1H-pyrazolo[4,3-c]pyridin-6-yl group, 1H-pyrazolo[4,3-c]pyridin-4-yl group, 1H-pyrazolo[3,4-c]pyridin-5-yl group, 1H-pyrazolo[3,4-c]pyridin-4-yl group, 1H-pyrazolo[3,4-b]pyridin-5-yl group, 1H-pyrazolo[3,4-b]pyridin-4-yl group, [1,2,4]triazolo[4,3-a]pyridin-6-yl group, [1,2,4]triazolo[4,3-a]pyridin-7-yl group, thieno[3,2-c]pyridin-2-yl group, thieno[3,2-c]pyridin-3-yl group, thieno[3,2-c]pyridin-6-yl group, thieno[3,2-b]pyridin-2-yl group, thieno[3,2-b]pyridin-3-yl group, thieno[3,2-b]pyridin-5-yl group, thieno[3,2-b]pyridin-6-yl group, 1H-thieno[3,2-c]pyrazol-5-yl group, 1H-thieno[3,2-c]pyrazol-4-yl group, benzo[d]isoxazol-5-yl group, benzo[d]isoxazol-4-yl group, benzo[d]isoxazol-6-yl group, benzo[d]isoxazol-7-yl group, benzo[c]isoxazol-5-yl group, benzo[c]isoxazol-4-yl group, benzo[c]isoxazol-6-yl group, benzo[c]isoxazol-7-yl group, indolizin-7-yl group, indolizin-6-yl group, indolizin-8-yl group, 1,3-dihydroindol-5-yl group, 1,3-dihydroindol-4-yl group, 1,3-dihydroindol-6-yl group, 1H-pyrazolo[3,4-d]thiazol-5-yl group, 2H-isoindol-5-yl group, 2H-isoindol-4-yl group, [1,2,4]triazolo[1,5-a]pyrimidin-6-yl group, 1H-pyrazolo[3,4-b]pyrazin-5-yl group, 1H-imidazo[4,5-b]pyrazin-5-yl group, 7H-purin-2-yl group, 4H-chromen-6-yl group, or 4H-chromen-5-yl group (the aforementioned groups may be substituted with one of Xa or two or more of the same or different Xa);
   Xa is oxo group, thioxo group, fluorine atom, chlorine atom, trifluoromethyl group, methyl group, ethyl group, propyl group, 2-hydroxyethyl group, carboxymethyl group, 2-carboxyethyl group, N,N-dimethylcarbamoylmethyl group, hydroxyl group, methoxy group, 2-hydroxyethyloxy group, carboxymethyloxy group, 2-carboxyethyloxy group, N,N-dimethylcarbamoylmethyloxy group, amino group, methylamino group, dimethylamino group, 2-hydroxyethylamino group, carbamoylamino group, acetylamino group, furan-2-carboxyamino group, 2-hydroxyacetylamino group, 2-aminoacetylamino group, methylsulfonylamino group, (N,N-dimethylsulfamoyl)amino group, methanesulfonyl group, sulfamoyl group, N-methylsulfamoyl group, N,N-dimethylsulfamoyl group, carboxyl group, acetyl group, carbamoyl group, or N,N-dimethylcarbamoyl group; and
   Y is hydrogen atom, or a lower alkyl group having 1 to 4 carbon atoms.

<37> The compound or salt thereof according to <1> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond or a double bond;
   when the bond between the ring-constituting carbon atom at the 1-position of the ring (E) and the Link moiety is a single bond, Link is CH₂, or when the bond between the ring-constituting carbon atom at the 1-position of the ring (E) and the Link moiety is a double bond, Link is CH;
   W is methylene group;
   Rs is -D-Rx or -N(Ry)(Rz);
   D represents a single bond, or oxygen atom;
   Rx is a substituent of propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, or Rb mentioned above;
   Q in Rb is phenyl group, 2-thienyl group, 3-thienyl group, 2-furyl group, 3-furyl group, or indan-2-yl group;
   A² in Rb is a single bond, oxygen atom, sulfur atom, -N(methyl)-, or -N(ethyl)-;
   A¹ in Rb is a single bond, or methylene group, methylmethylene group, or ethylene group when A² is a single bond, or ethylene group when A² is oxygen atom, sulfur atom, -N(methyl)-, or -N(ethyl)-;
   R² and R³ independently represent hydrogen atom, methyl group, fluorine atom, chlorine atom, trifluoromethyl group, hydroxyl group, methoxy group, or dimethylamino group;
   Rz has the same meaning as that of Rx, or is methyl group, or ethyl group;
   Ry is hydrogen atom, methyl group, ethyl group, propyl group, isopropyl group, butyl group, isobutyl group, pentyl group, or isopentyl group(provided that when Rz is methyl group, or ethyl group, Ry is a substituent other than hydrogen atom), or may bind to Rz to form, together with the nitrogen atom, a cyclic substituent (qy) selected from the group consisting of pyrrolidino group, piperidino group, morpholino group, and piperazino group, and the cyclic substituent (qy) may be substituted with one of substituent or two of the same or different substituents selected from methyl group, ethyl group, propyl group, isopropyl group, butyl group, isobutyl group, cyclopentyl group, cyclohexyl group, cyclopentylmethyl group, cyclohexylmethyl group, phenyl group, and phenylmethyl group (provided that the benzene ring of the phenyl group or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group);
   the substituent V¹ on the ring (E) is Zx, and V² on the ring (E) is AR;
   Zx is hydrogen atom, fluorine atom, chlorine atom, bromine atom, methyl group, hydroxyl group, amino group, or N-methylamino group;
   AR is naphthalen-2-yl group, naphthalen-1-yl group, benzofuran-5-yl group, benzofuran-4-yl group, benzo[b]thiophen-5-yl group, benzo[b]thiophen-4-yl group, indol-5-yl group, indol-4-yl group, benzothiazol-6-yl group, benzothiazol-7-yl group, quinolin-6-yl group, quinolin-3-yl group, dihydro-1H-quinolin-6-yl group, benzo[d]isothiazol-5-yl group, 1H-indazol-5-yl group, 1H-indazol-4-yl group, imidazo[1,2-a]pyridin-6-yl group, 1H-pyrrolo[2,3-b]pyridin-5-yl group, isoquinolin-6-yl group, dihydro-2H-isoquinolin-6-yl group, cinnolin-6-yl group, or benzoxazol-5-yl group (the aforementioned groups may be substituted with one of Xa or two or more of the same or different Xa);
   Xa is oxo group, methyl group, ethyl group, propyl group, 2-hydroxyethyl group, hydroxyl group, methoxy group, 2-hydroxyethyloxy group, amino group, N-methylamino group, N,N-dimethylamino group, 2-hydroxyethylamino group, or carboxyl group; and
   Y is hydrogen atom, methyl group, or ethyl group.

<38> The compound or salt thereof according to <1> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond or a double bond;
   when the bond between the ring-constituting carbon atom at the 1-position of the ring (E) and the Link moiety is a single bond, Link is CH₂, or when the bond between the ring-constituting carbon atom at the 1-position of the ring (E) and the Link moiety is a double bond, Link is CH;
   W is methylene group;
   Rs is -D-Rx or -N(Ry)(Rz);
   D is a single bond or oxygen atom;
   Rx is propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group;
   the substituent -N(Ry)(Rz) is N,N-dimethylamino group, N-ethyl-N-methylamino group, N,N-diethylamino group, N-methyl-N-propylamino group, N-ethyl-N-propylamino group, N-isopropyl-N-methylamino group, N-ethyl-N-isopropylamino group, N-butylamino group, N-butyl-N-methylamino group, N-butyl-N-ethylamino group, N-isobutylamino group, N-isobutyl-N-methylamino group, N-ethyl-N-isobutylamino group, N-isopentylamino group, N-isopentyl-N-methylamino group, N-ethyl-N-isopentylamino group, N-(2-ethylbutyl)amino group, N-(2-ethylbutyl)-N-methylamino group, N-cyclopentylamino group, N-cyclopentyl-N-methylamino group, N-cyclohexylamino group, N-cyclohexyl-N-methylamino group, N-cycloheptylamino group, N-(cyclopentylmethyl)amino group, N-(cyclopentylmethyl)-N-methylamino group, N-(cyclohexylmethyl)amino group, N-(cyclohexylmethyl)-N-methylamino group, N-phenylamino group, N-(2-methylphenyl)amino group, N-(4-methylphenyl)amino group, N-(2-fluorophenyl)amino group, N-(3-fluorophenyl)amino group, N-(4-fluorophenyl)amino group, N-(2-chlorophenyl)amino group, N-(3-chlorophenyl)amino group, N-(4-chlorophenyl)amino group, N-(indan-2-yl)amino group, N-(1-phenylethyl)amino group, N-[1-(2-fluorophenyl)ethyl]amino group, N-[1-(3-fluorophenyl)ethyl]amino group, N-[1-(4-fluorophenyl)ethyl]amino group, N-[1-(2-chlorophenyl)ethyl]amino group, N-[1-(3-chlorophenyl)ethyl]amino group, N-[1-(4-chlorophenyl)ethyl]amino group, N-benzylamino group, N-benzyl-N-methylamino group, N-benzyl-N-ethylamino group, N-(2-methylphenylmethyl)amino group, N-methyl-N-(2-methylphenylmethyl)amino group, N-(3-methylphenylmethyl)amino group, N-methyl-N-(3-methylphenylmethyl)amino group, N-(4-methylphenylmethyl)amino group, N-methyl-N-(4-methylphenylmethyl)amino group, N-(2-fluorophenylmethyl)amino group, N-(2-fluorophenylmethyl)-N-methylamino group, N-(3-fluorophenylmethyl)amino group, N-(3-fluorophenylmethyl)-N-methylamino group, N-(4-fluorophenylmethyl)amino group, N-(4-fluorophenylmethyl)-N-methylamino group, N-(2-chlorophenylmethyl)amino group, N-(2-chlorophenylmethyl)-N-methylamino group, N-(3-chlorophenylmethyl)amino group, N-(3-chlorophenylmethyl)-N-methylamino group, N-(4-chlorophenylmethyl)amino group, N-(4-chlorophenylmethyl)-N-methylamino group, N-(2,3-difluorophenylmethyl)amino group, N-(2,3-difluorophenylmethyl)-N-methylamino group, N-(2,4-difluorophenylmethyl)amino group, N-(2,4-difluorophenylmethyl)-N-methylamino group, N-(2,5-difluorophenylmethyl)amino group, N-(2,5-difluorophenylmethyl)-N-methylamino group, N-(3,4-difluorophenylmethyl)amino group, N-(3,4-difluorophenylmethyl)-N-methylamino group, N-(3,5-difluorophenylmethyl)amino group, N-(3,5-difluorophenylmethyl)-N-methylamino group, N-(2,3-dichlorophenylmethyl)amino group, N-(2,3-dichlorophenylmethyl)-N-methylamino group, N-(2,4-dichlorophenylmethyl)amino group, N-(2,4-dichlorophenylmethyl)-N-methylamino group, N-(2,5-dichlorophenylmethyl)amino group, N-(2,5-dichlorophenylmethyl)-N-methylamino group, N-(2,6-dichlorophenylmethyl)amino group, N-(2,6-dichlorophenylmethyl)-N-methylamino group, N-(3,4-dichlorophenylmethyl)amino group, N-(3,4-dichlorophenylmethyl)-N-methylamino group, N-(3,5-dichlorophenylmethyl)amino group, N-(3,5-dichlorophenylmethyl)-N-methylamino group, N-[2-(trifluoromethyl)phenylmethyl]amino group, N-methyl-N-[2-(trifluoromethyl)phenylmethyl]amino group, N-[3-(trifluoromethyl)phenylmethyl]amino group, N-methyl-N-[3-(trifluoromethyl)phenylmethyl]amino group, N-[4-(trifluoromethyl)phenylmethyl]amino group, N-methyl-N-[4-(trifluoromethyl)phenylmethyl]amino group, pyrrolidino group, 2-methylpyrrolidino group, 3-methylpyrrolidino group, 2,5-dimethylpyrrolidino group, 3,4-dimethylpyrrolidino group, piperidino group, 2-methylpiperidino group, 3-methylpiperidino group, 4-methylpiperidino group, 4-ethylpiperidino group, 4-propylpiperidino group, 4-isopropylpiperidino group, 4-butylpiperidino group, 4-isobutylpiperidino group, 4-cyclopentylpiperidino group, 4-cyclohexylpiperidino group, 4-(cyclopentylmethyl)piperidino group, 4-(cyclohexylmethyl)piperidino group, 4-phenylpiperidino group, 4-(2-methylphenyl)piperidino group, 4-(4-methylphenyl)piperidino group, 4-(2-fluorophenyl)piperidino group, 4-(3-fluorophenyl)piperidino group, 4-(4-fluorophenyl)piperidino group, 4-(2-chlorophenyl)piperidino group, 4-(3-chlorophenyl)piperidino group, 4-(4-chlorophenyl)piperidino group, 4-benzylpiperidino group, 4-(2-methylphenylmethyl)piperidino group, 4-(3-methylphenylmethyl)piperidino group, 4-methylphenylmethyl)piperidino group, 4-(2,3-dimethylphenylmethyl)piperidino group, 4-(3,5-dimethylphenylmethyl)piperidino group, 4-(2-fluorophenylmethyl)piperidino group, 4-(3-fluorophenylmethyl)piperidino group, 4-(4-fluorophenylmethyl)piperidino group, 4-(2-chlorophenylmethyl)piperidino group, 4-(3-chlorophenylmethyl)piperidino group, 4-(4-chlorophenylmethyl)piperidino group, 4-(2,3-difluorophenylmethyl)piperidino group, 4-(2,4-difluorophenylmethyl)piperidino group, 4-(2,5-difluorophenylmethyl)piperidino group, 4-(3,4-difluorophenylmethyl)piperidino group, 4-(2,3-dichlorophenylmethyl)piperidino group, 4-(2,4-dichlorophenylmethyl)piperidino group, 4-(2,5-dichlorophenylmethyl)piperidino group, 4-(2,6-dichlorophenylmethyl)piperidino group, 4-(3,4-dichlorophenylmethyl)piperidino group, 4-(3,5-dichlorophenylmethyl)piperidino group, 4-(3,6-dichlorophenylmethyl)piperidino group, 4-(2-methoxyphenylmethyl)piperidino group, 4-(3-methoxyphenylmethyl)piperidino group, 4-(4-methoxyphenylmethyl)piperidino group, 2,6-dimethylpiperidino group, 3,5-dimethylpiperidino group, 4,4-dimethylpiperidino group, homopiperidino group, morpholino group, 3,5-dimethylmorpholino group, 4-phenylpiperazino group, 4-(2-methylphenyl)piperazino group, 4-(4-methylphenyl)piperazino group, 4-(2-fluorophenyl)piperazino group, 4-(3-fluorophenyl)piperazino group, 4-(4-fluorophenyl)piperazino group, 4-(2-chlorophenyl)piperazino group, 4-(3-chlorophenyl)piperazino group, 4-(4-chlorophenyl)piperazino group, 4-benzylpiperazino group, 4-(2-methylphenylmethyl)piperazino group, 4-(3-methylphenylmethyl)piperazino group, 4-methylphenylmethyl)piperazino group, 4-(2,3-dimethylphenylmethyl)piperazino group, 4-(3,5-dimethylphenylmethyl)piperazino group, 4-(2-fluorophenylmethyl)piperazino group, 4-(3-fluorophenylmethyl)piperazino group, 4-(4-fluorophenylmethyl)piperazino group, 4-(2-chlorophenylmethyl)piperazino group, 4-(3-chlorophenylmethyl)piperazino group, 4-(4-chlorophenylmethyl)piperazino group, 4-(2,3-difluorophenylmethyl)piperazino group, 4-(2,4-difluorophenylmethyl)piperazino group, 4-(2,5-difluorophenylmethyl)piperazino group, 4-(3,4-difluorophenylmethyl)piperazino group, 4-(2,3-dichlorophenylmethyl)piperazino group, 4-(2,4-dichlorophenylmethyl)piperazino group, 4-(2,5-dichlorophenylmethyl)piperazino group, 4-(2,6-dichlorophenylmethyl)piperazino group, 4-(3,4-dichlorophenylmethyl)piperazino group, 4-(3,5-dichlorophenylmethyl)piperazino group, 4-(3,6-dichlorophenylmethyl)piperazino group, 4-(2-methoxyphenylmethyl)piperazino group, 4-(3-methoxyphenylmethyl)piperazino group, or 4-(4-methoxyphenylmethyl)piperazino group;
   the substituent V¹ on the ring (E) is Zx, and V² on the ring (E) is AR;
   Zx is hydrogen atom, fluorine atom, chlorine atom, bromine atom, methyl group, hydroxyl group, amino group, or N-methylamino group;
   AR is naphthalen-2-yl group, 6-hydroxynaphthalen-2-yl group, 6-methoxynaphthalen-2-yl group, 6-(2-hydroxyethyloxy)naphthalen-2-yl group, 6-aminonaphthalen-2-yl group, 6-(N-methylamino)naphthalen-2-yl group, 6-(N,N-dimethylamino)naphthalen-2-yl group, 6-(2-hydroxyethylamino)naphthalen-2-yl group, benzo[b]furan-5-yl group, 2-methylbenzo[b]furan-5-yl group, 3-methylbenzo[b]furan-5-yl group, 2,3-dimethylbenzo[b]furan-5-yl group, benzo[b]thiophen-5-yl group, 2-methylbenzo[b]thiophen-5-yl group, 3-methylbenzo[b]thiophen-5-yl group, 2,3-dimethylbenzo[b]thiophen-5-yl group, 1H-indol-5-yl group, 2-methyl-1H-indol-5-yl group, 3-methyl-1H-indol-5-yl group, 2,3-dimethyl-1H-indol-5-yl group, 1-methyl-1H-indol-5-yl group, 1,2-dimethyl-1H-indol-5-yl group, 1,3-dimethyl-1H-indol-5-yl group, 1,2,3-trimethyl-1H-indol-5-yl group, 1-ethyl-1H-indol-5-yl group, 1-ethyl-2-methyl-1H-indol-5-yl group, 1-ethyl-3-methyl-1H-indol-5-yl group, 1-ethyl-2,3-dimethyl-1H-indol-5-yl group, 1-propyl-1H-indol-5-yl group, 2-methyl-1-propyl-1H-indol-5-yl group, 3-methyl-1-propyl-1H-indol-5-yl group, 2,3-dimethyl-1-propyl-1H-indol-5-yl group, 1-(2-hydroxyethyl)-1H-indol-5-yl group, 1-(2-hydroxyethyl)-2-methyl-1H-indol-5-yl group, 1-(2-hydroxyethyl)-3-methyl-1H-indol-5-yl group, 2,3-dimethyl-1-(2-hydroxyethyl)-1H-indol-5-yl group, benzothiazol-6-yl group, 2-methylbenzothiazol-6-yl group, 2-methoxybenzothiazol-6-yl group, 2-aminobenzothiazol-6-yl group, 2-oxo-2,3-dihydrobenzothiazol-6-yl group, 2-oxo-3-methyl-2,3-dihydrobenzothiazol-6-yl group, 2-thioxo-2,3-dihydrobenzothiazol-6-yl group, 2-thioxo-3-methyl-2,3-dihydrobenzothiazol-6-yl group, quinolin-3-yl group, quinolin-6-yl group, 2-oxo-1,2-dihydroquinolin-6-yl group, benzo[d]isothiazol-5-yl group, 1H-indazol-5-yl group, 1-methyl-1H-indazol-5-yl group, 1-ethyl-1H-indazol-5-yl group, 1-propyl-1H-indazol-5-yl group, 1-(2-hydroxyethyl)-1H-indazol-5-yl group, 3-hydroxy-1H-indazol-5-yl group, 3-hydroxy-1-methyl-1H-indazol-5-yl group, 1-ethyl-3-hydroxy-1H-indazol-5-yl group, imidazo[1,2-a]pyridin-6-yl group, 1H-pyrrolo[2,3-b]pyridin-5-yl group, 1-methyl-1H-pyrrolo[2,3-b]pyridin-5-yl group, 1-ethyl-1H-pyrrolo[2,3-b]pyridin-5-yl group, 1-propyl-1H-pyrrolo[2,3-b]pyridin-5-yl group, 1-(2-hydroxyethyl)-1H-pyrrolo[2,3-b]pyridin-5-yl group, isoquinolin-6-yl group, 1-oxo-1,2-dihydroisoquinolin-6-yl group, cinnolin-6-yl group, or benzoxazol-5-yl group; and
   Y is hydrogen atom, methyl group, or ethyl group.

<39> The compound or salt thereof according to <1> mentioned above, wherein, in the formula (I), the bond between the ring-constituting carbon atom at the 1-position of the ring (E) and the ring-constituting carbon atom at the 2-position of the ring (E) is a double bond;
   the bond between the ring-constituting carbon atom at the 1-position of the ring (E) and the Link moiety is a single bond;
   Link is CH₂; and
   W is oxygen atom;
   Rs is -D-Rx;
   D is a single bond or oxygen atom;
   Rx is propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group;
   the substituent V¹ on the ring (E) is Zx, and V² on the ring (E) is AR;
   Zx is hydrogen atom, fluorine atom, chlorine atom, bromine atom, methyl group, hydroxyl group, amino group, or N-methylamino group;
   AR is naphthalen-2-yl group, 6-hydroxynaphthalen-2-yl group, 6-methoxynaphthalen-2-yl group, 6-(2-hydroxyethyloxy)naphthalen-2-yl group, 6-aminonaphthalen-2-yl group, 6-(N-methylamino)naphthalen-2-yl group, 6-(N,N-dimethylamino)naphthalen-2-yl group, 6-(2-hydroxyethylamino)naphthalen-2-yl group, benzo[b]furan-5-yl group, 2-methylbenzo[b]furan-5-yl group, 3-methylbenzo[b]furan-5-yl group, 2,3-dimethylbenzo[b]furan-5-yl group, benzo[b]thiophen-5-yl group, 2-methylbenzo[b]thiophen-5-yl group, 3-methylbenzo[b]thiophen-5-yl group, 2,3-dimethylbenzo[b]thiophen-5-yl group, 1H-indol-5-yl group, 2-methyl-1H-indol-5-yl group, 3-methyl-1H-indol-5-yl group, 2,3-dimethyl-1H-indol-5-yl group, 1-methyl-1H-indol-5-yl group, 1,2-dimethyl-1H-indol-5-yl group, 1,3-dimethyl-1H-indol-5-yl group, 1,2,3-trimethyl-1H-indol-5-yl group, 1-ethyl-1H-indol-5-yl group, 1-ethyl-2-methyl-1H-indol-5-yl group, 1-ethyl-3-methyl-1H-indol-5-yl group, 1-ethyl-2,3-dimethyl-1H-indol-5-yl group, 1-propyl-1H-indol-5-yl group, 2-methyl-1-propyl-1H-indol-5-yl group, 3-methyl-1-propyl-1H-indol-5-yl group, 2,3-dimethyl-1-propyl-1H-indol-5-yl group, 1-(2-hydroxyethyl)-1H-indol-5-yl group, 1-(2-hydroxyethyl)-2-methyl-1H-indol-5-yl group, 1-(2-hydroxyethyl)-3-methyl-1H-indol-5-yl group, 2,3-dimethyl-1-(2-hydroxyethyl)-1H-indol-5-yl group, benzothiazol-6-yl group, 2-methylbenzothiazol-6-yl group, 2-methoxybenzothiazol-6-yl group, 2-aminobenzothiazol-6-yl group, 2-oxo-2,3-dihydrobenzothiazol-6-yl group, 2-oxo-3-methyl-2,3-dihydrobenzothiazol-6-yl group, 2-thioxo-2,3-dihydrobenzothiazol-6-yl group, 2-thioxo-3-methyl-2,3-dihydrobenzothiazol-6-yl group, quinolin-3-yl group, quinolin-6-yl group, 2-oxo-1,2-dihydroquinolin-6-yl group, benzo[d]isothiazol-5-yl group, 1H-indazol-5-yl group, 1-methyl-1H-indazol-5-yl group, 1-ethyl-1H-indazol-5-yl group, 1-propyl-1H-indazol-5-yl group, 1-(2-hydroxyethyl)-1H-indazol-5-yl group, 3-hydroxy-1H-indazol-5-yl group, 3-hydroxy-1-methyl-1H-indazol-5-yl group, 1-ethyl-3-hydroxy-1H-indazol-5-yl group, imidazo[1,2-a]pyridin-6-yl group, 1H-pyrrolo[2,3-b]pyridin-5-yl group, 1-methyl-1H-pyrrolo[2,3-b]pyridin-5-yl group, 1-ethyl-1H-pyrrolo[2,3-b]pyridin-5-yl group, 1-propyl-1H-pyrrolo[2,3-b]pyridin-5-yl group, 1-(2-hydroxyethyl)-1H-pyrrolo[2,3-b]pyridin-5-yl group, isoquinolin-6-yl group, 1-oxo-1,2-dihydroisoquinolin-6-yl group, cinnolin-6-yl group, or benzoxazol-5-yl group; and
   Y is hydrogen atom, methyl group, or ethyl group.

<40> The compound or salt thereof according to <1> mentioned above, wherein, in the formula (I), the bond between the ring-constituting carbon atom at the 1-position of the ring (E) and the ring-constituting carbon atom at the 2-position of the ring (E) is a double bond;
   the bond between the ring-constituting carbon atom at the 1-position of the ring (E) and the Link moiety is a single bond;
   Link is CH₂;
   W is oxygen atom;
   Rs is -N(Ry)(Rz);
   the substituent -N(Ry)(Rz) is N,N-dimethylamino group, N-ethyl-N-methylamino group, N,N-diethylamino group, N-methyl-N-propylamino group, N-ethyl-N-propylamino group, N-isopropyl-N-methylamino group, N-ethyl-N-isopropylamino group, N-butylamino group, N-butyl-N-methylamino group, N-butyl-N-ethylamino group, N-isobutylamino group, N-isobutyl-N-methylamino group, N-ethyl-N-isobutylamino group, N-isopentylamino group, N-isopentyl-N-methylamino group, N-ethyl-N-isopentylamino group, N-(2-ethylbutyl)amino group, N-(2-ethylbutyl)-N-methylamino group, N-cyclopentylamino group, N-cyclopentyl-N-methylamino group, N-cyclohexylamino group, N-cyclohexyl-N-methylamino group, N-cycloheptylamino group, N-(cyclopentylmethyl)amino group, N-(cyclopentylmethyl)-N-methylamino group, N-(cyclohexylmethyl)amino group, N-(cyclohexylmethyl)-N-methylamino group, N-phenylamino group, N-(2-methylphenyl)amino group, N-(4-methylphenyl)amino group, N-(2-fluorophenyl)amino group, N-(3-fluorophenyl)amino group, N-(4-fluorophenyl)amino group, N-(2-chlorophenyl)amino group, N-(3-chlorophenyl)amino group, N-(4-chlorophenyl)amino group, N-(indan-2-yl)amino group, N-(1-phenylethyl)amino group, N-[1-(2-fluorophenyl)ethyl]amino group, N-[1-(3-fluorophenyl)ethyl]amino group, N-[1-(4-fluorophenyl)ethyl]amino group, N-[1-(2-chlorophenyl)ethyl]amino group, N-[1-(3-chlorophenyl)ethyl]amino group, N-[1-(4-chlorophenyl)ethyl]amino group, N-benzylamino group, N-benzyl-N-methylamino group, N-benzyl-N-ethylamino group, N-(2-methylphenylmethyl)amino group, N-methyl-N-(2-methylphenylmethyl)amino group, N-(3-methylphenylmethyl)amino group, N-methyl-N-(3-methylphenylmethyl)amino group, N-(4-methylphenylmethyl)amino group, N-methyl-N-(4-methylphenylmethyl)amino group, N-(2-fluorophenylmethyl)amino group, N-(2-fluorophenylmethyl)-N-methylamino group, N-(3-fluorophenylmethyl)amino group, N-(3-fluorophenylmethyl)-N-methylamino group, N-(4-fluorophenylmethyl)amino group, N-(4-fluorophenylmethyl)-N-methylamino group, N-(2-chlorophenylmethyl)amino group, N-(2-chlorophenylmethyl)-N-methylamino group, N-(3-chlorophenylmethyl)amino group, N-(3-chlorophenylmethyl)-N-methylamino group, N-(4-chlorophenylmethyl)amino group, N-(4-chlorophenylmethyl)-N-methylamino group, N-(2,3-difluorophenylmethyl)amino group, N-(2,3-difluorophenylmethyl)-N-methylamino group, N-(2,4-difluorophenylmethyl)amino group, N-(2,4-difluorophenylmethyl)-N-methylamino group, N-(2,5-difluorophenylmethyl)amino group, N-(2,5-difluorophenylmethyl)-N-methylamino group, N-(3,4-difluorophenylmethyl)amino group, N-(3,4-difluorophenylmethyl)-N-methylamino group, N-(3,5-difluorophenylmethyl)amino group, N-(3,5-difluorophenylmethyl)-N-methylamino group, N-(2,3-dichlorophenylmethyl)amino group, N-(2,3-dichlorophenylmethyl)-N-methylamino group, N-(2,4-dichlorophenylmethyl)amino group, N-(2,4-dichlorophenylmethyl)-N-methylamino group, N-(2,5-dichlorophenylmethyl)amino group, N-(2,5-dichlorophenylmethyl)-N-methylamino group, N-(2,6-dichlorophenylmethyl)amino group, N-(2,6-dichlorophenylmethyl)-N-methylamino group, N-(3,4-dichlorophenylmethyl)amino group, N-(3,4-dichlorophenylmethyl)-N-methylamino group, N-(3,5-dichlorophenylmethyl)amino group, N-(3,5-dichlorophenylmethyl)-N-methylamino group, N-[2-(trifluoromethyl)phenylmethyl]amino group, N-methyl-N-[2-(trifluoromethyl)phenylmethyl]amino group, N-[3-(trifluoromethyl)phenylmethyl]amino group, N-methyl-N-[3-(trifluoromethyl)phenylmethyl]amino group, N-[4-(trifluoromethyl)phenylmethyl]amino group, N-methyl-N-[4-(trifluoromethyl)phenylmethyl]amino group, pyrrolidino group, 2-methylpyrrolidino group, 3-methylpyrrolidino group, 2,5-dimethylpyrrolidino group, 3,4-dimethylpyrrolidino group, piperidino group, 2-methylpiperidino group, 3-methylpiperidino group, 4-methylpiperidino group, 4-ethylpiperidino group, 4-propylpiperidino group, 4-isopropylpiperidino group, 4-butylpiperidino group, 4-isobutylpiperidino group, 4-cyclopentylpiperidino group, 4-cyclohexylpiperidino group, 4-(cyclopentylmethyl)piperidino group, 4-(cyclohexylmethyl)piperidino group, 4-phenylpiperidino group, 4-(2-methylphenyl)piperidino group, 4-(4-methylphenyl)piperidino group, 4-(2-fluorophenyl)piperidino group, 4-(3-fluorophenyl)piperidino group, 4-(4-fluorophenyl)piperidino group, 4-(2-chlorophenyl)piperidino group, 4-(3-chlorophenyl)piperidino group, 4-(4-chlorophenyl)piperidino group, 4-benzylpiperidino group, 4-(2-methylphenylmethyl)piperidino group, 4-(3-methylphenylmethyl)piperidino group, 4-methylphenylmethyl)piperidino group, 4-(2,3-dimethylphenylmethyl)piperidino group, 4-(3,5-dimethylphenylmethyl)piperidino group, 4-(2-fluorophenylmethyl)piperidino group, 4-(3-fluorophenylmethyl)piperidino group, 4-(4-fluorophenylmethyl)piperidino group, 4-(2-chlorophenylmethyl)piperidino group, 4-(3-chlorophenylmethyl)piperidino group, 4-(4-chlorophenylmethyl)piperidino group, 4-(2,3-difluorophenylmethyl)piperidino group, 4-(2,4-difluorophenylmethyl)piperidino group, 4-(2,5-difluorophenylmethyl)piperidino group, 4-(3,4-difluorophenylmethyl)piperidino group, 4-(2,3-dichlorophenylmethyl)piperidino group, 4-(2,4-dichlorophenylmethyl)piperidino group, 4-(2,5-dichlorophenylmethyl)piperidino group, 4-(2,6-dichlorophenylmethyl)piperidino group, 4-(3,4-dichlorophenylmethyl)piperidino group, 4-(3,5-dichlorophenylmethyl)piperidino group, 4-(3,6-dichlorophenylmethyl)piperidino group, 4-(2-methoxyphenylmethyl)piperidino group, 4-(3-methoxyphenylmethyl)piperidino group, 4-(4-methoxyphenylmethyl)piperidino group, 2,6-dimethylpiperidino group, 3,5-dimethylpiperidino group, 4,4-dimethylpiperidino group, homopiperidino group, morpholino group, 3,5-dimethylmorpholino group, 4-phenylpiperazino group, 4-(2-methylphenyl)piperazino group, 4-(4-methylphenyl)piperazino group, 4-(2-fluorophenyl)piperazino group, 4-(3-fluorophenyl)piperazino group, 4-(4-fluorophenyl)piperazino group, 4-(2-chlorophenyl)piperazino group, 4-(3-chlorophenyl)piperazino group, 4-(4-chlorophenyl)piperazino group, 4-benzylpiperazino group, 4-(2-methylphenylmethyl)piperazino group, 4-(3-methylphenylmethyl)piperazino group, 4-methylphenylmethyl)piperazino group, 4-(2,3-dimethylphenylmethyl)piperazino group, 4-(3,5-dimethylphenylmethyl)piperazino group, 4-(2-fluorophenylmethyl)piperazino group, 4-(3-fluorophenylmethyl)piperazino group, 4-(4-fluorophenylmethyl)piperazino group, 4-(2-chlorophenylmethyl)piperazino group, 4-(3-chlorophenylmethyl)piperazino group, 4-(4-chlorophenylmethyl)piperazino group, 4-(2,3-difluorophenylmethyl)piperazino group, 4-(2,4-difluorophenylmethyl)piperazino group, 4-(2,5-difluorophenylmethyl)piperazino group, 4-(3,4-difluorophenylmethyl)piperazino group, 4-(2,3-dichlorophenylmethyl)piperazino group, 4-(2,4-dichlorophenylmethyl)piperazino group, 4-(2,5-dichlorophenylmethyl)piperazino group, 4-(2,6-dichlorophenylmethyl)piperazino group, 4-(3,4-dichlorophenylmethyl)piperazino group, 4-(3,5-dichlorophenylmethyl)piperazino group, 4-(3,6-dichlorophenylmethyl)piperazino group, 4-(2-methoxyphenylmethyl)piperazino group, 4-(3-methoxyphenylmethyl)piperazino group, or 4-(4-methoxyphenylmethyl)piperazino group;
   the substituent V¹ on the ring (E) is Zx, and V² on the ring (E) is AR;
   Zx is hydrogen atom, fluorine atom, chlorine atom, bromine atom, methyl group, hydroxyl group, amino group, or N-methylamino group;
   AR is naphthalen-2-yl group, 6-hydroxynaphthalen-2-yl group, 6-methoxynaphthalen-2-yl group, 6-(2-hydroxyethyloxy)naphthalen-2-yl group, 6-aminonaphthalen-2-yl group, 6-(N-methylamino)naphthalen-2-yl group, 6-(N,N-dimethylamino)naphthalen-2-yl group, 6-(2-hydroxyethylamino)naphthalen-2-yl group, benzo[b]furan-5-yl group, 2-methylbenzo[b]furan-5-yl group, 3-methylbenzo[b]furan-5-yl group, 2,3-dimethylbenzo[b]furan-5-yl group, benzo[b]thiophen-5-yl group, 2-methylbenzo[b]thiophen-5-yl group, 3-methylbenzo[b]thiophen-5-yl group, 2,3-dimethylbenzo[b]thiophen-5-yl group, 1H-indol-5-yl group, 2-methyl-1H-indol-5-yl group, 3-methyl-1H-indol-5-yl group, 2,3-dimethyl-1H-indol-5-yl group, 1-methyl-1H-indol-5-yl group, 1,2-dimethyl-1H-indol-5-yl group, 1,3-dimethyl-1H-indol-5-yl group, 1,2,3-trimethyl-1H-indol-5-yl group, 1-ethyl-1H-indol-5-yl group, 1-ethyl-2-methyl-1H-indol-5-yl group, 1-ethyl-3-methyl-1H-indol-5-yl group, 1-ethyl-2,3-dimethyl-1H-indol-5-yl group, 1-propyl-1H-indol-5-yl group, 2-methyl-1-propyl-1H-indol-5-yl group, 3-methyl-1-propyl-1H-indol-5-yl group, 2,3-dimethyl-1-propyl-1H-indol-5-yl group, 1-(2-hydroxyethyl)-1H-indol-5-yl group, 1-(2-hydroxyethyl)-2-methyl-1H-indol-5-yl group, 1-(2-hydroxyethyl)-3-methyl-1H-indol-5-yl group, 2,3-dimethyl-1-(2-hydroxyethyl)-1H-indol-5-yl group, benzothiazol-6-yl group, 2-methylbenzothiazol-6-yl group, 2-methoxybenzothiazol-6-yl group, 2-aminobenzothiazol-6-yl group, 2-oxo-2,3-dihydrobenzothiazol-6-yl group, 2-oxo-3-methyl-2,3-dihydrobenzothiazol-6-yl group, 2-thioxo-2,3-dihydrobenzothiazol-6-yl group, 2-thioxo-3-methyl-2,3-dihydrobenzothiazol-6-yl group, quinolin-3-yl group, quinolin-6-yl group, 2-oxo-1,2-dihydroquinolin-6-yl group, benzo[d]isothiazol-5-yl group, 1H-indazol-5-yl group, 1-methyl-1H-indazol-5-yl group, 1-ethyl-1H-indazol-5-yl group, 1-propyl-1H-indazol-5-yl group, 1-(2-hydroxyethyl)-1H-indazol-5-yl group, 3-hydroxy-1H-indazol-5-yl group, 3-hydroxy-1-methyl-1H-indazol-5-yl group, 1-ethyl-3-hydroxy-1H-indazol-5-yl group, imidazo[1,2-a]pyridin-6-yl group, 1H-pyrrolo[2,3-b]pyridin-5-yl group, 1-methyl-1H-pyrrolo[2,3-b]pyridin-5-yl group, 1-ethyl-1H-pyrrolo[2,3-b]pyridin-5-yl group, 1-propyl-1H-pyrrolo[2,3-b]pyridin-5-yl group, 1-(2-hydroxyethyl)-1H-pyrrolo[2,3-b]pyridin-5-yl group, isoquinolin-6-yl group, 1-oxo-1,2-dihydroisoquinolin-6-yl group, cinnolin-6-yl group, or benzoxazol-5-yl group; and
   Y is hydrogen atom, methyl group, or ethyl group.

<41> The compound or salt thereof according to <1> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond or a double bond;
   when the bond between the ring-constituting carbon atom at the 1-position of the ring (E) and the Link moiety is a single bond, Link is CH₂, or when the bond between the ring-constituting carbon atom at the 1-position of the ring (E) and the Link moiety is a double bond, Link is CH;
   W is methylene group;
   Rs is propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group;
   the substituent V¹ on the ring (E) is Zx, and V² on the ring (E) is AR;
   Zx is hydrogen atom, fluorine atom, chlorine atom, bromine atom, methyl group, hydroxyl group, amino group, or N-methylamino group;
   AR is naphthalen-2-yl group, 6-hydroxynaphthalen-2-yl group, 6-methoxynaphthalen-2-yl group, 6-(2-hydroxyethyloxy)naphthalen-2-yl group, 6-aminonaphthalen-2-yl group, 6-(N-methylamino)naphthalen-2-yl group, 6-(N,N-dimethylamino)naphthalen-2-yl group, 6-(2-hydroxyethylamino)naphthalen-2-yl group, benzo[b]furan-5-yl group, 2-methylbenzo[b]furan-5-yl group, 3-methylbenzo[b]furan-5-yl group, 2,3-dimethylbenzo[b]furan-5-yl group, benzo[b]thiophen-5-yl group, 2-methylbenzo[b]thiophen-5-yl group, 3-methylbenzo[b]thiophen-5-yl group, 2,3-dimethylbenzo[b]thiophen-5-yl group, 1H-indol-5-yl group, 2-methyl-1H-indol-5-yl group, 3-methyl-1H-indol-5-yl group, 2,3-dimethyl-1H-indol-5-yl group, 1-methyl-1H-indol-5-yl group, 1,2-dimethyl-1H-indol-5-yl group, 1,3-dimethyl-1H-indol-5-yl group, 1,2,3-trimethyl-1H-indol-5-yl group, 1-ethyl-1H-indol-5-yl group, 1-ethyl-2-methyl-1H-indol-5-yl group, 1-ethyl-3-methyl-1H-indol-5-yl group, 1-ethyl-2,3-dimethyl-1H-indol-5-yl group, 1-propyl-1H-indol-5-yl group, 2-methyl-1-propyl-1H-indol-5-yl group, 3-methyl-1-propyl-1H-indol-5-yl group, 2,3-dimethyl-1-propyl-1H-indol-5-yl group, 1-(2-hydroxyethyl)-1H-indol-5-yl group, 1-(2-hydroxyethyl)-2-methyl-1H-indol-5-yl group, 1-(2-hydroxyethyl)-3-methyl-1H-indol-5-yl group, 2,3-dimethyl-1-(2-hydroxyethyl)-1H-indol-5-yl group, benzothiazol-6-yl group, 2-methylbenzothiazol-6-yl group, 2-methoxybenzothiazol-6-yl group, 2-aminobenzothiazol-6-yl group, 2-oxo-2,3-dihydrobenzothiazol-6-yl group, 2-oxo-3-methyl-2,3-dihydrobenzothiazol-6-yl group, 2-thioxo-2,3-dihydrobenzothiazol-6-yl group, 2-thioxo-3-methyl-2,3-dihydrobenzothiazol-6-yl group, quinolin-3-yl group, quinolin-6-yl group, 2-oxo-1,2-dihydroquinolin-6-yl group, benzo[d]isothiazol-5-yl group, 1H-indazol-5-yl group, 1-methyl-1H-indazol-5-yl group, 1-ethyl-1H-indazol-5-yl group, 1-propyl-1H-indazol-5-yl group, 1-(2-hydroxyethyl)-1H-indazol-5-yl group, 3-hydroxy-1H-indazol-5-yl group, 3-hydroxy-1-methyl-1H-indazol-5-yl group, 1-ethyl-3-hydroxy-1H-indazol-5-yl group, imidazo[1,2-a]pyridin-6-yl group, 1H-pyrrolo[2,3-b]pyridin-5-yl group, 1-methyl-1H-pyrrolo[2,3-b]pyridin-5-yl group, 1-ethyl-1H-pyrrolo[2,3-b]pyridin-5-yl group, 1-propyl-1H-pyrrolo[2,3-b]pyridin-5-yl group, 1-(2-hydroxyethyl)-1H-pyrrolo[2,3-b]pyridin-5-yl group, isoquinolin-6-yl group, 1-oxo-1,2-dihydroisoquinolin-6-yl group, cinnolin-6-yl group, or benzoxazol-5-yl group; and
   Y is hydrogen atom, methyl group, or ethyl group.

<42> The compound or salt thereof according to <1> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond or a double bond;
   when the bond between the ring-constituting carbon atom at the 1-position of the ring (E) and the Link moiety is a single bond, Link is CH₂, or when the bond between the ring-constituting carbon atom at the 1-position of the ring (E) and the Link moiety is a double bond, Link is CH;
   W is methylene group;
   Rs is -O-Rx;
   Rx is propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group;
   the substituent V¹ on the ring (E) is Zx, and V² on the ring (E) is AR;
   Zx is hydrogen atom, fluorine atom, chlorine atom, bromine atom, methyl group, hydroxyl group, amino group, or N-methylamino group;
   AR is naphthalen-2-yl group, 6-hydroxynaphthalen-2-yl group, 6-methoxynaphthalen-2-yl group, 6-(2-hydroxyethyloxy)naphthalen-2-yl group, 6-aminonaphthalen-2-yl group, 6-(N-methylamino)naphthalen-2-yl group, 6-(N,N-dimethylamino)naphthalen-2-yl group, 6-(2-hydroxyethylamino)naphthalen-2-yl group, benzo[b]furan-5-yl group, 2-methylbenzo[b]furan-5-yl group, 3-methylbenzo[b]furan-5-yl group, 2,3-dimethylbenzo[b]furan-5-yl group, benzo[b]thiophen-5-yl group, 2-methylbenzo[b]thiophen-5-yl group, 3-methylbenzo[b]thiophen-5-yl group, 2,3-dimethylbenzo[b]thiophen-5-yl group, 1H-indol-5-yl group, 2-methyl-1H-indol-5-yl group, 3-methyl-1H-indol-5-yl group, 2,3-dimethyl-1H-indol-5-yl group, 1-methyl-1H-indol-5-yl group, 1,2-dimethyl-1H-indol-5-yl group, 1,3-dimethyl-1H-indol-5-yl group, 1,2,3-trimethyl-1H-indol-5-yl group, 1-ethyl-1H-indol-5-yl group, 1-ethyl-2-methyl-1H-indol-5-yl group, 1-ethyl-3-methyl-1H-indol-5-yl group, 1-ethyl-2,3-dimethyl-1H-indol-5-yl group, 1-propyl-1H-indol-5-yl group, 2-methyl-1-propyl-1H-indol-5-yl group, 3-methyl-1-propyl-1H-indol-5-yl group, 2,3-dimethyl-1-propyl-1H-indol-5-yl group, 1-(2-hydroxyethyl)-1H-indol-5-yl group, 1-(2-hydroxyethyl)-2-methyl-1H-indol-5-yl group, 1-(2-hydroxyethyl)-3-methyl-1H-indol-5-yl group, 2,3-dimethyl-1-(2-hydroxyethyl)-1H-indol-5-yl group, benzothiazol-6-yl group, 2-methylbenzothiazol-6-yl group, 2-methoxybenzothiazol-6-yl group, 2-aminobenzothiazol-6-yl group, 2-oxo-2,3-dihydrobenzothiazol-6-yl group, 2-oxo-3-methyl-2,3-dihydrobenzothiazol-6-yl group, 2-thioxo-2,3-dihydrobenzothiazol-6-yl group, 2-thioxo-3-methyl-2,3-dihydrobenzothiazol-6-yl group, quinolin-3-yl group, quinolin-6-yl group, 2-oxo-1,2-dihydroquinolin-6-yl group, benzo[d]isothiazol-5-yl group, 1H-indazol-5-yl group, 1-methyl-1H-indazol-5-yl group, 1-ethyl-1H-indazol-5-yl group, 1-propyl-1H-indazol-5-yl group, 1-(2-hydroxyethyl)-1H-indazol-5-yl group, 3-hydroxy-1H-indazol-5-yl group, 3-hydroxy-1-methyl-1H-indazol-5-yl group, 1-ethyl-3-hydroxy-1H-indazol-5-yl group, imidazo[1,2-a]pyridin-6-yl group, 1H-pyrrolo[2,3-b]pyridin-5-yl group, 1-methyl-1H-pyrrolo[2,3-b]pyridin-5-yl group, 1-ethyl-1H-pyrrolo[2,3-b]pyridin-5-yl group, 1-propyl-1H-pyrrolo[2,3-b]pyridin-5-yl group, 1-(2-hydroxyethyl)-1H-pyrrolo[2,3-b]pyridin-5-yl group, isoquinolin-6-yl group, 1-oxo-1,2-dihydroisoquinolin-6-yl group, cinnolin-6-yl group, or benzoxazol-5-yl group; and
   Y is hydrogen atom, methyl group, or ethyl group.

<43> The compound or salt thereof according to <1> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond or a double bond;
   when the bond between the ring-constituting carbon atom at the 1-position of the ring (E) and the Link moiety is a single bond, Link is CH₂, or when the bond between the ring-constituting carbon atom at the 1-position of the ring (E) and the Link moiety is a double bond, Link is CH;
   W is methylene group;
   Rs is -N(Ry)(Rz);
   the substituent -N(Ry)(Rz) is N,N-dimethylamino group, N-ethyl-N-methylamino group, N,N-diethylamino group, N-methyl-N-propylamino group, N-ethyl-N-propylamino group, N-isopropyl-N-methylamino group, N-ethyl-N-isopropylamino group, N-butylamino group, N-butyl-N-methylamino group, N-butyl-N-ethylamino group, N-isobutylamino group, N-isobutyl-N-methylamino group, N-ethyl-N-isobutylamino group, N-isopentylamino group, N-isopentyl-N-methylamino group, N-ethyl-N-isopentylamino group, N-(2-ethylbutyl)amino group, N-(2-ethylbutyl)-N-methylamino group, N-cyclopentylamino group, N-cyclopentyl-N-methylamino group, N-cyclohexylamino group, N-cyclohexyl-N-methylamino group, N-cycloheptylamino group, N-(cyclopentylmethyl)amino group, N-(cyclopentylmethyl)-N-methylamino group, N-(cyclohexylmethyl)amino group, N-(cyclohexylmethyl)-N-methylamino group, N-phenylamino group, N-(2-methylphenyl)amino group, N-(4-methylphenyl)amino group, N-(2-fluorophenyl)amino group, N-(3-fluorophenyl)amino group, N-(4-fluorophenyl)amino group, N-(2-chlorophenyl)amino group, N-(3-chlorophenyl)amino group, N-(4-chlorophenyl)amino group, N-(indan-2-yl)amino group, N-(1-phenylethyl)amino group, N-[1-(2-fluorophenyl)ethyl]amino group, N-[1-(3-fluorophenyl)ethyl]amino group, N-[1-(4-fluorophenyl)ethyl]amino group, N-[1-(2-chlorophenyl)ethyl]amino group, N-[1-(3-chlorophenyl)ethyl]amino group, N-[1-(4-chlorophenyl)ethyl]amino group, N-benzylamino group, N-benzyl-N-methylamino group, N-benzyl-N-ethylamino group, N-(2-methylphenylmethyl)amino group, N-methyl-N-(2-methylphenylmethyl)amino group, N-(3-methylphenylmethyl)amino group, N-methyl-N-(3-methylphenylmethyl)amino group, N-(4-methylphenylmethyl)amino group, N-methyl-N-(4-methylphenylmethyl)amino group, N-(2-fluorophenylmethyl)amino group, N-(2-fluorophenylmethyl)-N-methylamino group, N-(3-fluorophenylmethyl)amino group, N-(3-fluorophenylmethyl)-N-methylamino group, N-(4-fluorophenylmethyl)amino group, N-(4-fluorophenylmethyl)-N-methylamino group, N-(2-chlorophenylmethyl)amino group, N-(2-chlorophenylmethyl)-N-methylamino group, N-(3-chlorophenylmethyl)amino group, N-(3-chlorophenylmethyl)-N-methylamino group, N-(4-chlorophenylmethyl)amino group, N-(4-chlorophenylmethyl)-N-methylamino group, N-(2,3-difluorophenylmethyl)amino group, N-(2,3-difluorophenylmethyl)-N-methylamino group, N-(2,4-difluorophenylmethyl)amino group, N-(2,4-difluorophenylmethyl)-N-methylamino group, N-(2,5-difluorophenylmethyl)amino group, N-(2,5-difluorophenylmethyl)-N-methylamino group, N-(3,4-difluorophenylmethyl)amino group, N-(3,4-difluorophenylmethyl)-N-methylamino group, N-(3,5-difluorophenylmethyl)amino group, N-(3,5-difluorophenylmethyl)-N-methylamino group, N-(2,3-dichlorophenylmethyl)amino group, N-(2,3-dichlorophenylmethyl)-N-methylamino group, N-(2,4-dichlorophenylmethyl)amino group, N-(2,4-dichlorophenylmethyl)-N-methylamino group, N-(2,5-dichlorophenylmethyl)amino group, N-(2,5-dichlorophenylmethyl)-N-methylamino group, N-(2,6-dichlorophenylmethyl)amino group, N-(2,6-dichlorophenylmethyl)-N-methylamino group, N-(3,4-dichlorophenylmethyl)amino group, N-(3,4-dichlorophenylmethyl)-N-methylamino group, N-(3,5-dichlorophenylmethyl)amino group, N-(3,5-dichlorophenylmethyl)-N-methylamino group, N-[2-(trifluoromethyl)phenylmethyl]amino group, N-methyl-N-[2-(trifluoromethyl)phenylmethyl]amino group, N-[3-(trifluoromethyl)phenylmethyl]amino group, N-methyl-N-[3-(trifluoromethyl)phenylmethyl]amino group, N-[4-(trifluoromethyl)phenylmethyl]amino group, N-methyl-N-[4-(trifluoromethyl)phenylmethyl]amino group, pyrrolidino group, 2-methylpyrrolidino group, 3-methylpyrrolidino group, 2,5-dimethylpyrrolidino group, 3,4-dimethylpyrrolidino group, piperidino group, 2-methylpiperidino group, 3-methylpiperidino group, 4-methylpiperidino group, 4-ethylpiperidino group, 4-propylpiperidino group, 4-isopropylpiperidino group, 4-butylpiperidino group, 4-isobutylpiperidino group, 4-cyclopentylpiperidino group, 4-cyclohexylpiperidino group, 4-(cyclopentylmethyl)piperidino group, 4-(cyclohexylmethyl)piperidino group, 4-phenylpiperidino group, 4-(2-methylphenyl)piperidino group, 4-(4-methylphenyl)piperidino group, 4-(2-fluorophenyl)piperidino group, 4-(3-fluorophenyl)piperidino group, 4-(4-fluorophenyl)piperidino group, 4-(2-chlorophenyl)piperidino group, 4-(3-chlorophenyl)piperidino group, 4-(4-chlorophenyl)piperidino group, 4-benzylpiperidino group, 4-(2-methylphenylmethyl)piperidino group, 4-(3-methylphenylmethyl)piperidino group, 4-methylphenylmethyl)piperidino group, 4-(2,3-dimethylphenylmethyl)piperidino group, 4-(3,5-dimethylphenylmethyl)piperidino group, 4-(2-fluorophenylmethyl)piperidino group, 4-(3-fluorophenylmethyl)piperidino group, 4-(4-fluorophenylmethyl)piperidino group, 4-(2-chlorophenylmethyl)piperidino group, 4-(3-chlorophenylmethyl)piperidino group, 4-(4-chlorophenylmethyl)piperidino group, 4-(2,3-difluorophenylmethyl)piperidino group, 4-(2,4-difluorophenylmethyl)piperidino group, 4-(2,5-difluorophenylmethyl)piperidino group, 4-(3,4-difluorophenylmethyl)piperidino group, 4-(2,3-dichlorophenylmethyl)piperidino group, 4-(2,4-dichlorophenylmethyl)piperidino group, 4-(2,5-dichlorophenylmethyl)piperidino group, 4-(2,6-dichlorophenylmethyl)piperidino group, 4-(3,4-dichlorophenylmethyl)piperidino group, 4-(3,5-dichlorophenylmethyl)piperidino group, 4-(3,6-dichlorophenylmethyl)piperidino group, 4-(2-methoxyphenylmethyl)piperidino group, 4-(3-methoxyphenylmethyl)piperidino group, 4-(4-methoxyphenylmethyl)piperidino group, 2,6-dimethylpiperidino group, 3,5-dimethylpiperidino group, 4,4-dimethylpiperidino group, homopiperidino group, morpholino group, 3,5-dimethylmorpholino group, 4-phenylpiperazino group, 4-(2-methylphenyl)piperazino group, 4-(4-methylphenyl)piperazino group, 4-(2-fluorophenyl)piperazino group, 4-(3-fluorophenyl)piperazino group, 4-(4-fluorophenyl)piperazino group, 4-(2-chlorophenyl)piperazino group, 4-(3-chlorophenyl)piperazino group, 4-(4-chlorophenyl)piperazino group, 4-benzylpiperazino group, 4-(2-methylphenylmethyl)piperazino group, 4-(3-methylphenylmethyl)piperazino group, 4-methylphenylmethyl)piperazino group, 4-(2,3-dimethylphenylmethyl)piperazino group, 4-(3,5-dimethylphenylmethyl)piperazino group, 4-(2-fluorophenylmethyl)piperazino group, 4-(3-fluorophenylmethyl)piperazino group, 4-(4-fluorophenylmethyl)piperazino group, 4-(2-chlorophenylmethyl)piperazino group, 4-(3-chlorophenylmethyl)piperazino group, 4-(4-chlorophenylmethyl)piperazino group, 4-(2,3-difluorophenylmethyl)piperazino group, 4-(2,4-difluorophenylmethyl)piperazino group, 4-(2,5-difluorophenylmethyl)piperazino group, 4-(3,4-difluorophenylmethyl)piperazino group, 4-(2,3-dichlorophenylmethyl)piperazino group, 4-(2,4-dichlorophenylmethyl)piperazino group, 4-(2,5-dichlorophenylmethyl)piperazino group, 4-(2,6-dichlorophenylmethyl)piperazino group, 4-(3,4-dichlorophenylmethyl)piperazino group, 4-(3,5-dichlorophenylmethyl)piperazino group, 4-(3,6-dichlorophenylmethyl)piperazino group, 4-(2-methoxyphenylmethyl)piperazino group, 4-(3-methoxyphenylmethyl)piperazino group, or 4-(4-methoxyphenylmethyl)piperazino group;
   the substituent V¹ on the ring (E) is Zx, and V² on the ring (E) is AR;
   Zx is hydrogen atom, fluorine atom, chlorine atom, bromine atom, methyl group, hydroxyl group, amino group, or N-methylamino group;
   AR is naphthalen-2-yl group, 6-hydroxynaphthalen-2-yl group, 6-methoxynaphthalen-2-yl group, 6-(2-hydroxyethyloxy)naphthalen-2-yl group, 6-aminonaphthalen-2-yl group, 6-(N-methylamino)naphthalen-2-yl group, 6-(N,N-dimethylamino)naphthalen-2-yl group, 6-(2-hydroxyethylamino)naphthalen-2-yl group, benzo[b]furan-5-yl group, 2-methylbenzo[b]furan-5-yl group, 3-methylbenzo[b]furan-5-yl group, 2,3-dimethylbenzo[b]furan-5-yl group, benzo[b]thiophen-5-yl group, 2-methylbenzo[b]thiophen-5-yl group, 3-methylbenzo[b]thiophen-5-yl group, 2,3-dimethylbenzo[b]thiophen-5-yl group, 1H-indol-5-yl group, 2-methyl-1H-indol-5-yl group, 3-methyl-1H-indol-5-yl group, 2,3-dimethyl-1H-indol-5-yl group, 1-methyl-1H-indol-5-yl group, 1,2-dimethyl-1H-indol-5-yl group, 1,3-dimethyl-1H-indol-5-yl group, 1,2,3-trimethyl-1H-indol-5-yl group, 1-ethyl-1H-indol-5-yl group, 1-ethyl-2-methyl-1H-indol-5-yl group, 1-ethyl-3-methyl-1H-indol-5-yl group, 1-ethyl-2,3-dimethyl-1H-indol-5-yl group, 1-propyl-1H-indol-5-yl group, 2-methyl-1-propyl-1H-indol-5-yl group, 3-methyl-1-propyl-1H-indol-5-yl group, 2,3-dimethyl-1-propyl-1H-indol-5-yl group, 1-(2-hydroxyethyl)-1H-indol-5-yl group, 1-(2-hydroxyethyl)-2-methyl-1H-indol-5-yl group, 1-(2-hydroxyethyl)-3-methyl-1H-indol-5-yl group, 2,3-dimethyl-1-(2-hydroxyethyl)-1H-indol-5-yl group, benzothiazol-6-yl group, 2-methylbenzothiazol-6-yl group, 2-methoxybenzothiazol-6-yl group, 2-aminobenzothiazol-6-yl group, 2-oxo-2,3-dihydrobenzothiazol-6-yl group, 2-oxo-3-methyl-2,3-dihydrobenzothiazol-6-yl group, 2-thioxo-2,3-dihydrobenzothiazol-6-yl group, 2-thioxo-3-methyl-2,3-dihydrobenzothiazol-6-yl group, quinolin-3-yl group, quinolin-6-yl group, 2-oxo-1,2-dihydroquinolin-6-yl group, benzo[d]isothiazol-5-yl group, 1H-indazol-5-yl group, 1-methyl-1H-indazol-5-yl group, 1-ethyl-1H-indazol-5-yl group, 1-propyl-1H-indazol-5-yl group, 1-(2-hydroxyethyl)-1H-indazol-5-yl group, 3-hydroxy-1H-indazol-5-yl group, 3-hydroxy-1-methyl-1H-indazol-5-yl group, 1-ethyl-3-hydroxy-1H-indazol-5-yl group, imidazo[1,2-a]pyridin-6-yl group, 1H-pyrrolo[2,3-b]pyridin-5-yl group, 1-methyl-1H-pyrrolo[2,3-b]pyridin-5-yl group, 1-ethyl-1H-pyrrolo[2,3-b]pyridin-5-yl group, 1-propyl-1H-pyrrolo[2,3-b]pyridin-5-yl group, 1-(2-hydroxyethyl)-1H-pyrrolo[2,3-b]pyridin-5-yl group, isoquinolin-6-yl group, 1-oxo-1,2-dihydroisoquinolin-6-yl group, cinnolin-6-yl group, or benzoxazol-5-yl group; and
   Y is hydrogen atom, methyl group, or ethyl group.

<44> The compound or salt thereof according to <1> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond or a double bond;
   when the bond between the ring-constituting carbon atom at the 1-position of the ring (E) and the Link moiety is a single bond, Link is CH₂, or when the bond between the ring-constituting carbon atom at the 1-position of the ring (E) and the Link moiety is a double bond, Link is CH;
   W is methylene group;
   Rs is phenyl group, 2-methylphenyl group, 3-methylphenyl group, 4-methylphenyl group, 2,3-dimethylphenyl group, 3,5-dimethylphenyl group, 2-methoxyphenyl group, 3-methoxyphenyl group, 4-methoxyphenyl group, 2-fluorophenyl group, 3-fluorophenyl group, 4-fluorophenyl group, 2-chlorophenyl group, 3-chlorophenyl group, 4-chlorophenyl group, 2,3-difluorophenyl group, 2,4-difluorophenyl group, 2,5-difluorophenyl group, 3,4-difluorophenyl group, 2,3-dichlorophenyl group, 2,4-dichlorophenyl group, 2,5-dichlorophenyl group, 2,6-dichlorophenyl group, 3,4-dichlorophenyl group, 3,5-dichlorophenyl group, 2-trifluoromethylphenyl group, 3-trifluoromethylphenyl group, 4-trifluoromethylphenyl group, 4-(N,N-dimethylamino)phenyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-methoxyindan-2-yl group, 5-methoxyindan-2-yl group, 4,7-dimethoxyindan-2-yl group, 5,6-dimethoxyindan-2-yl group, furan-2-yl group, furan-3-yl group, thiophen-2-yl group, or thiophen-3-yl group;
   the substituent V¹ on the ring (E) is Zx, and V² on the ring (E) is AR;
   Zx is hydrogen atom, fluorine atom, chlorine atom, bromine atom, methyl group, hydroxyl group, amino group, or N-methylamino group;
   AR is naphthalen-2-yl group, 6-hydroxynaphthalen-2-yl group, 6-methoxynaphthalen-2-yl group, 6-(2-hydroxyethyloxy)naphthalen-2-yl group, 6-aminonaphthalen-2-yl group, 6-(N-methylamino)naphthalen-2-yl group, 6-(N,N-dimethylamino)naphthalen-2-yl group, 6-(2-hydroxyethylamino)naphthalen-2-yl group, benzo[b]furan-5-yl group, 2-methylbenzo[b]furan-5-yl group, 3-methylbenzo[b]furan-5-yl group, 2,3-dimethylbenzo[b]furan-5-yl group, benzo[b]thiophen-5-yl group, 2-methylbenzo[b]thiophen-5-yl group, 3-methylbenzo[b]thiophen-5-yl group, 2,3-dimethylbenzo[b]thiophen-5-yl group, 1H-indol-5-yl group, 2-methyl-1H-indol-5-yl group, 3-methyl-1H-indol-5-yl group, 2,3-dimethyl-1H-indol-5-yl group, 1-methyl-1H-indol-5-yl group, 1,2-dimethyl-1H-indol-5-yl group, 1,3-dimethyl-1H-indol-5-yl group, 1,2,3-trimethyl-1H-indol-5-yl group, 1-ethyl-1H-indol-5-yl group, 1-ethyl-2-methyl-1H-indol-5-yl group, 1-ethyl-3-methyl-1H-indol-5-yl group, 1-ethyl-2,3-dimethyl-1H-indol-5-yl group, 1-propyl-1H-indol-5-yl group, 2-methyl-1-propyl-1H-indol-5-yl group, 3-methyl-1-propyl-1H-indol-5-yl group, 2,3-dimethyl-1-propyl-1H-indol-5-yl group, 1-(2-hydroxyethyl)-1H-indol-5-yl group, 1-(2-hydroxyethyl)-2-methyl-1H-indol-5-yl group, 1-(2-hydroxyethyl)-3-methyl-1H-indol-5-yl group, 2,3-dimethyl-1-(2-hydroxyethyl)-1H-indol-5-yl group, benzothiazol-6-yl group, 2-methylbenzothiazol-6-yl group, 2-methoxybenzothiazol-6-yl group, 2-aminobenzothiazol-6-yl group, 2-oxo-2,3-dihydrobenzothiazol-6-yl group, 2-oxo-3-methyl-2,3-dihydrobenzothiazol-6-yl group, 2-thioxo-2,3-dihydrobenzothiazol-6-yl group, 2-thioxo-3-methyl-2,3-dihydrobenzothiazol-6-yl group, quinolin-3-yl group, quinolin-6-yl group, 2-oxo-1,2-dihydroquinolin-6-yl group, benzo[d]isothiazol-5-yl group, 1H-indazol-5-yl group, 1-methyl-1H-indazol-5-yl group, 1-ethyl-1H-indazol-5-yl group, 1-propyl-1H-indazol-5-yl group, 1-(2-hydroxyethyl)-1H-indazol-5-yl group, 3-hydroxy-1H-indazol-5-yl group, 3-hydroxy-1-methyl-1H-indazol-5-yl group, 1-ethyl-3-hydroxy-1H-indazol-5-yl group, imidazo[1,2-a]pyridin-6-yl group, 1H-pyrrolo[2,3-b]pyridin-5-yl group, 1-methyl-1H-pyrrolo[2,3-b]pyridin-5-yl group, 1-ethyl-1H-pyrrolo[2,3-b]pyridin-5-yl group, 1-propyl-1H-pyrrolo[2,3-b]pyridin-5-yl group, 1-(2-hydroxyethyl)-1H-pyrrolo[2,3-b]pyridin-5-yl group, isoquinolin-6-yl group, 1-oxo-1,2-dihydroisoquinolin-6-yl group, cinnolin-6-yl group, or benzoxazol-5-yl group; and
   Y is hydrogen atom, methyl group, or ethyl group.

<45> The compound or salt thereof according to <1> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond;
   Link is CH₂;
   W is methylene group;
   Rs is -D-Rx or -N(Ry)(Rz);
   D is a single bond or oxygen atom;
   Rx is propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, or cycloheptylmethyl group, or Rb mentioned above;
   Q in Rb is phenyl group, 2-thienyl group, 3-thienyl group, 2-furyl group, 3-furyl group, or indan-2-yl group;
   A² in Rb is a single bond, oxygen atom, sulfur atom, -N(methyl)-, or -N(ethyl)-;
   A¹ in Rb is a single bond, or methylene group, methylmethylene group, or ethylene group when A² is a single bond, or ethylene group when A² is oxygen atom, sulfur atom, -N(methyl)-, or -N(ethyl)-;
   R² and R³ independently represent hydrogen atom, methyl group, fluorine atom, chlorine atom, trifluoromethyl group, hydroxyl group, methoxy group, or dimethylamino group;
   Rz has the same meaning as that of Rx, or is methyl group, or ethyl group;
   Ry is hydrogen atom, methyl group, ethyl group, propyl group, isopropyl group, butyl group, isobutyl group, pentyl group, or isopentyl group (provided that when Rz is methyl group, or ethyl group, Ry is a substituent other than hydrogen atom), or may bind to Rz to form, together with the nitrogen atom, a cyclic substituent (qy) selected from the group consisting of pyrrolidino group, piperidino group, morpholino group, and piperazino group, and the cyclic substituent (qy) may be substituted with one of substituent or two of the same or different substituents selected from methyl group, ethyl group, propyl group, isopropyl group, butyl group, isobutyl group, cyclopentyl group, cyclohexyl group, cyclopentylmethyl group, cyclohexylmethyl group, phenyl group, and phenylmethyl group (provided that the benzene ring of the phenyl group or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group);
   the substituent V¹ on the ring (E) is Zx, and V² on the ring (E) is AR;
   Zx is hydrogen atom, fluorine atom, chlorine atom, bromine atom, methyl group, hydroxyl group, amino group, or N-methylamino group;
   AR is naphthalen-2-yl group, benzofuran-5-yl group, benzo[b]thiophen-5-yl group, indol-5-yl group, benzothiazol-6-yl group, quinolin-6-yl group, quinolin-3-yl group, 1H-indazol-5-yl group, isoquinolin-6-yl group, cinnolin-6-yl group, or benzoxazol-5-yl group (the aforementioned groups may be substituted with one of Xa or two or more of the same or different Xa);
   Xa is oxo group, methyl group, ethyl group, propyl group, 2-hydroxyethyl group, hydroxyl group, methoxy group, 2-hydroxyethyloxy group, amino group, N-methylamino group, N,N-dimethylamino group, 2-hydroxyethylamino group, or carboxyl group; and
   Y is hydrogen atom, methyl group, or ethyl group.

<46> The compound or salt thereof according to <1> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond;
   Link is CH₂;
   W is methylene group;
   Rs is -D-Rx or -N(Ry)(Rz);
   D is a single bond or oxygen atom;
   Rx is propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group;
   the substituent -N(Ry)(Rz) is N,N-dimethylamino group, N-ethyl-N-methylamino group, N,N-diethylamino group, N-methyl-N-propylamino group, N-ethyl-N-propylamino group, N-isopropyl-N-methylamino group, N-ethyl-N-isopropylamino group, N-butylamino group, N-butyl-N-methylamino group, N-butyl-N-ethylamino group, N-isobutylamino group, N-isobutyl-N-methylamino group, N-ethyl-N-isobutylamino group, N-isopentylamino group, N-isopentyl-N-methylamino group, N-ethyl-N-isopentylamino group, N-(2-ethylbutyl)amino group, N-(2-ethylbutyl)-N-methylamino group, N-cyclopentylamino group, N-cyclopentyl-N-methylamino group, N-cyclohexylamino group, N-cyclohexyl-N-methylamino group, N-cycloheptylamino group, N-(cyclopentylmethyl)amino group, N-(cyclopentylmethyl)-N-methylamino group, N-(cyclohexylmethyl)amino group, N-(cyclohexylmethyl)-N-methylamino group, N-phenylamino group, N-(2-methylphenyl)amino group, N-(4-methylphenyl)amino group, N-(2-fluorophenyl)amino group, N-(3-fluorophenyl)amino group, N-(4-fluorophenyl)amino group, N-(2-chlorophenyl)amino group, N-(3-chlorophenyl)amino group, N-(4-chlorophenyl)amino group, N-(indan-2-yl)amino group, N-(1-phenylethyl)amino group, N-[1-(2-fluorophenyl)ethyl]amino group, N-[1-(3-fluorophenyl)ethyl]amino group, N-[1-(4-fluorophenyl)ethyl]amino group, N-[1-(2-chlorophenyl)ethyl]amino group, N-[1-(3-chlorophenyl)ethyl]amino group, N-[1-(4-chlorophenyl)ethyl]amino group, N-benzylamino group, N-benzyl-N-methylamino group, N-benzyl-N-ethylamino group, N-(2-methylphenylmethyl)amino group, N-methyl-N-(2-methylphenylmethyl)amino group, N-(3-methylphenylmethyl)amino group, N-methyl-N-(3-methylphenylmethyl)amino group, N-(4-methylphenylmethyl)amino group, N-methyl-N-(4-methylphenylmethyl)amino group, N-(2-fluorophenylmethyl)amino group, N-(2-fluorophenylmethyl)-N-methylamino group, N-(3-fluorophenylmethyl)amino group, N-(3-fluorophenylmethyl)-N-methylamino group, N-(4-fluorophenylmethyl)amino group, N-(4-fluorophenylmethyl)-N-methylamino group, N-(2-chlorophenylmethyl)amino group, N-(2-chlorophenylmethyl)-N-methylamino group, N-(3-chlorophenylmethyl)amino group, N-(3-chlorophenylmethyl)-N-methylamino group, N-(4-chlorophenylmethyl)amino group, N-(4-chlorophenylmethyl)-N-methylamino group, N-(2,3-difluorophenylmethyl)amino group, N-(2,3-difluorophenylmethyl)-N-methylamino group, N-(2,4-difluorophenylmethyl)amino group, N-(2,4-difluorophenylmethyl)-N-methylamino group, N-(2,5-difluorophenylmethyl)amino group, N-(2,5-difluorophenylmethyl)-N-methylamino group, N-(3,4-difluorophenylmethyl)amino group, N-(3,4-difluorophenylmethyl)-N-methylamino group, N-(3,5-difluorophenylmethyl)amino group, N-(3,5-difluorophenylmethyl)-N-methylamino group, N-(2,3-dichlorophenylmethyl)amino group, N-(2,3-dichlorophenylmethyl)-N-methylamino group, N-(2,4-dichlorophenylmethyl)amino group, N-(2,4-dichlorophenylmethyl)-N-methylamino group, N-(2,5-dichlorophenylmethyl)amino group, N-(2,5-dichlorophenylmethyl)-N-methylamino group, N-(2,6-dichlorophenylmethyl)amino group, N-(2,6-dichlorophenylmethyl)-N-methylamino group, N-(3,4-dichlorophenylmethyl)amino group, N-(3,4-dichlorophenylmethyl)-N-methylamino group, N-(3,5-dichlorophenylmethyl)amino group, N-(3,5-dichlorophenylmethyl)-N-methylamino group, N-[2-(trifluoromethyl)phenylmethyl]amino group, N-methyl-N-[2-(trifluoromethyl)phenylmethyl]amino group, N-[3-(trifluoromethyl)phenylmethyl]amino group, N-methyl-N-[3-(trifluoromethyl)phenylmethyl]amino group, N-[4-(trifluoromethyl)phenylmethyl]amino group, N-methyl-N-[4-(trifluoromethyl)phenylmethyl]amino group, pyrrolidino group, 2-methylpyrrolidino group, 3-methylpyrrolidino group, 2,5-dimethylpyrrolidino group, 3,4-dimethylpyrrolidino group, piperidino group, 2-methylpiperidino group, 3-methylpiperidino group, 4-methylpiperidino group, 4-ethylpiperidino group, 4-propylpiperidino group, 4-isopropylpiperidino group, 4-butylpiperidino group, 4-isobutylpiperidino group, 4-cyclopentylpiperidino group, 4-cyclohexylpiperidino group, 4-(cyclopentylmethyl)piperidino group, 4-(cyclohexylmethyl)piperidino group, 4-phenylpiperidino group, 4-(2-methylphenyl)piperidino group, 4-(4-methylphenyl)piperidino group, 4-(2-fluorophenyl)piperidino group, 4-(3-fluorophenyl)piperidino group, 4-(4-fluorophenyl)piperidino group, 4-(2-chlorophenyl)piperidino group, 4-(3-chlorophenyl)piperidino group, 4-(4-chlorophenyl)piperidino group, 4-benzylpiperidino group, 4-(2-methylphenylmethyl)piperidino group, 4-(3-methylphenylmethyl)piperidino group, 4-methylphenylmethyl)piperidino group, 4-(2,3-dimethylphenylmethyl)piperidino group, 4-(3,5-dimethylphenylmethyl)piperidino group, 4-(2-fluorophenylmethyl)piperidino group, 4-(3-fluorophenylmethyl)piperidino group, 4-(4-fluorophenylmethyl)piperidino group, 4-(2-chlorophenylmethyl)piperidino group, 4-(3-chlorophenylmethyl)piperidino group, 4-(4-chlorophenylmethyl)piperidino group, 4-(2,3-difluorophenylmethyl)piperidino group, 4-(2,4-difluorophenylmethyl)piperidino group, 4-(2,5-difluorophenylmethyl)piperidino group, 4-(3,4-difluorophenylmethyl)piperidino group, 4-(2,3-dichlorophenylmethyl)piperidino group, 4-(2,4-dichlorophenylmethyl)piperidino group, 4-(2,5-dichlorophenylmethyl)piperidino group, 4-(2,6-dichlorophenylmethyl)piperidino group, 4-(3,4-dichlorophenylmethyl)piperidino group, 4-(3,5-dichlorophenylmethyl)piperidino group, 4-(3,6-dichlorophenylmethyl)piperidino group, 4-(2-methoxyphenylmethyl)piperidino group, 4-(3-methoxyphenylmethyl)piperidino group, 4-(4-methoxyphenylmethyl)piperidino group, 2,6-dimethylpiperidino group, 3,5-dimethylpiperidino group, 4,4-dimethylpiperidino group, homopiperidino group, morpholino group, 3,5-dimethylmorpholino group, 4-phenylpiperazino group, 4-(2-methylphenyl)piperazino group, 4-(4-methylphenyl)piperazino group, 4-(2-fluorophenyl)piperazino group, 4-(3-fluorophenyl)piperazino group, 4-(4-fluorophenyl)piperazino group, 4-(2-chlorophenyl)piperazino group, 4-(3-chlorophenyl)piperazino group, 4-(4-chlorophenyl)piperazino group, 4-benzylpiperazino group, 4-(2-methylphenylmethyl)piperazino group, 4-(3-methylphenylmethyl)piperazino group, 4-methylphenylmethyl)piperazino group, 4-(2,3-dimethylphenylmethyl)piperazino group, 4-(3,5-dimethylphenylmethyl)piperazino group, 4-(2-fluorophenylmethyl)piperazino group, 4-(3-fluorophenylmethyl)piperazino group, 4-(4-fluorophenylmethyl)piperazino group, 4-(2-chlorophenylmethyl)piperazino group, 4-(3-chlorophenylmethyl)piperazino group, 4-(4-chlorophenylmethyl)piperazino group, 4-(2,3-difluorophenylmethyl)piperazino group, 4-(2,4-difluorophenylmethyl)piperazino group, 4-(2,5-difluorophenylmethyl)piperazino group, 4-(3,4-difluorophenylmethyl)piperazino group, 4-(2,3-dichlorophenylmethyl)piperazino group, 4-(2,4-dichlorophenylmethyl)piperazino group, 4-(2,5-dichlorophenylmethyl)piperazino group, 4-(2,6-dichlorophenylmethyl)piperazino group, 4-(3,4-dichlorophenylmethyl)piperazino group, 4-(3,5-dichlorophenylmethyl)piperazino group, 4-(3,6-dichlorophenylmethyl)piperazino group, 4-(2-methoxyphenylmethyl)piperazino group, 4-(3-methoxyphenylmethyl)piperazino group, or 4-(4-methoxyphenylmethyl)piperazino group;
   the substituent V¹ on the ring (E) is Zx, and V² on the ring (E) is AR;
   Zx is hydrogen atom, fluorine atom, chlorine atom, bromine atom, methyl group, hydroxyl group, amino group, or N-methylamino group;
   AR is naphthalen-2-yl group, 6-hydroxynaphthalen-2-yl group, 6-methoxynaphthalen-2-yl group, 6-aminonaphthalen-2-yl group, 6-(N,N-dimethylamino)naphthalen-2-yl group, benzo[b]furan-5-yl group, benzo[b]thiophen-5-yl group, 1H-indol-5-yl group, 1-methyl-1H-indol-5-yl group, 1-ethyl-1H-indol-5-yl group, benzothiazol-6-yl group, 2-aminobenzothiazol-6-yl group, 2-oxo-2,3-dihydrobenzothiazol-6-yl group, quinolin-3-yl group, quinolin-6-yl group, 2-oxo-1,2-dihydroquinolin-6-yl group, 1H-indazol-5-yl group, 1-methyl-1H-indazol-5-yl group, 1-ethyl-1H-indazol-5-yl group, isoquinolin-6-yl group, 1-oxo-1,2-dihydroisoquinolin-6-yl group, cinnolin-6-yl group, or benzoxazol-5-yl group; and
   Y is hydrogen atom, methyl group, or ethyl group.

<47> The compound or salt thereof according to <1> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond;
   Link is CH₂;
   W is methylene group;
   Rs is propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group;
   the substituent V¹ on the ring (E) is Zx, and V² on the ring (E) is AR;
   Zx is hydrogen atom, fluorine atom, chlorine atom, bromine atom, methyl group, hydroxyl group, amino group, or N-methylamino group;
   AR is naphthalen-2-yl group, 6-hydroxynaphthalen-2-yl group, 6-methoxynaphthalen-2-yl group, 6-aminonaphthalen-2-yl group, 6-(N,N-dimethylamino)naphthalen-2-yl group, benzo[b]furan-5-yl group, benzo[b]thiophen-5-yl group, 1H-indol-5-yl group, 1-methyl-1H-indol-5-yl group, 1-ethyl-1H-indol-5-yl group, benzothiazol-6-yl group, 2-aminobenzothiazol-6-yl group, 2-oxo-2,3-dihydrobenzothiazol-6-yl group, quinolin-3-yl group, quinolin-6-yl group, 2-oxo-1,2-dihydroquinolin-6-yl group, 1H-indazol-5-yl group, 1-methyl-1H-indazol-5-yl group, 1-ethyl-1H-indazol-5-yl group, isoquinolin-6-yl group, 1-oxo-1,2-dihydroisoquinolin-6-yl group, cinnolin-6-yl group, or benzoxazol-5-yl group; and
   Y is hydrogen atom, methyl group, or ethyl group.

<48> The compound or salt thereof according to <1> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond;
   Link is CH₂;
   W is methylene group;
   Rs is -O-Rx;
   Rx is propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group;
   the substituent V¹ on the ring (E) is Zx, and V² on the ring (E) is AR;
   Zx is hydrogen atom, fluorine atom, chlorine atom, bromine atom, methyl group, hydroxyl group, amino group, or N-methylamino group;
   AR is naphthalen-2-yl group, 6-hydroxynaphthalen-2-yl group, 6-methoxynaphthalen-2-yl group, 6-aminonaphthalen-2-yl group, 6-(N,N-dimethylamino)naphthalen-2-yl group, benzo[b]furan-5-yl group, benzo[b]thiophen-5-yl group, 1H-indol-5-yl group, 1-methyl-1H-indol-5-yl group, 1-ethyl-1H-indol-5-yl group, benzothiazol-6-yl group, 2-aminobenzothiazol-6-yl group, 2-oxo-2,3-dihydrobenzothiazol-6-yl group, quinolin-3-yl group, quinolin-6-yl group, 2-oxo-1,2-dihydroquinolin-6-yl group, 1H-indazol-5-yl group, 1-methyl-1H-indazol-5-yl group, 1-ethyl-1H-indazol-5-yl group, isoquinolin-6-yl group, 1-oxo-1,2-dihydroisoquinolin-6-yl group, cinnolin-6-yl group, or benzoxazol-5-yl group; and
   Y is hydrogen atom, methyl group, or ethyl group.

<49> The compound or salt thereof according to <1> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond;
   Link is CH₂;
   W is methylene group;
   Rs is -N(Ry)(Rz);
   the substituent -N(Ry)(Rz) is N,N-dimethylamino group, N-ethyl-N-methylamino group, N,N-diethylamino group, N-methyl-N-propylamino group, N-ethyl-N-propylamino group, N-isopropyl-N-methylamino group, N-ethyl-N-isopropylamino group, N-butylamino group, N-butyl-N-methylamino group, N-butyl-N-ethylamino group, N-isobutylamino group, N-isobutyl-N-methylamino group, N-ethyl-N-isobutylamino group, N-isopentylamino group, N-isopentyl-N-methylamino group, N-ethyl-N-isopentylamino group, N-(2-ethylbutyl)amino group, N-(2-ethylbutyl)-N-methylamino group, N-cyclopentylamino group, N-cyclopentyl-N-methylamino group, N-cyclohexylamino group, N-cyclohexyl-N-methylamino group, N-cycloheptylamino group, N-(cyclopentylmethyl)amino group, N-(cyclopentylmethyl)-N-methylamino group, N-(cyclohexylmethyl)amino group, N-(cyclohexylmethyl)-N-methylamino group, N-phenylamino group, N-(2-methylphenyl)amino group, N-(4-methylphenyl)amino group, N-(2-fluorophenyl)amino group, N-(3-fluorophenyl)amino group, N-(4-fluorophenyl)amino group, N-(2-chlorophenyl)amino group, N-(3-chlorophenyl)amino group, N-(4-chlorophenyl)amino group, N-(indan-2-yl)amino group, N-(1-phenylethyl)amino group, N-[1-(2-fluorophenyl)ethyl]amino group, N-[1-(3-fluorophenyl)ethyl]amino group, N-[1-(4-fluorophenyl)ethyl]amino group, N-[1-(2-chlorophenyl)ethyl]amino group, N-[1-(3-chlorophenyl)ethyl]amino group, N-[1-(4-chlorophenyl)ethyl]amino group, N-benzylamino group, N-benzyl-N-methylamino group, N-benzyl-N-ethylamino group, N-(2-methylphenylmethyl)amino group, N-methyl-N-(2-methylphenylmethyl)amino group, N-(3-methylphenylmethyl)amino group, N-methyl-N-(3-methylphenylmethyl)amino group, N-(4-methylphenylmethyl)amino group, N-methyl-N-(4-methylphenylmethyl)amino group, N-(2-fluorophenylmethyl)amino group, N-(2-fluorophenylmethyl)-N-methylamino group, N-(3-fluorophenylmethyl)amino group, N-(3-fluorophenylmethyl)-N-methylamino group, N-(4-fluorophenylmethyl)amino group, N-(4-fluorophenylmethyl)-N-methylamino group, N-(2-chlorophenylmethyl)amino group, N-(2-chlorophenylmethyl)-N-methylamino group, N-(3-chlorophenylmethyl)amino group, N-(3-chlorophenylmethyl)-N-methylamino group, N-(4-chlorophenylmethyl)amino group, N-(4-chlorophenylmethyl)-N-methylamino group, N-(2,3-difluorophenylmethyl)amino group, N-(2,3-difluorophenylmethyl)-N-methylamino group, N-(2,4-difluorophenylmethyl)amino group, N-(2,4-difluorophenylmethyl)-N-methylamino group, N-(2,5-difluorophenylmethyl)amino group, N-(2,5-difluorophenylmethyl)-N-methylamino group, N-(3,4-difluorophenylmethyl)amino group, N-(3,4-difluorophenylmethyl)-N-methylamino group, N-(3,5-difluorophenylmethyl)amino group, N-(3,5-difluorophenylmethyl)-N-methylamino group, N-(2,3-dichlorophenylmethyl)amino group, N-(2,3-dichlorophenylmethyl)-N-methylamino group, N-(2,4-dichlorophenylmethyl)amino group, N-(2,4-dichlorophenylmethyl)-N-methylamino group, N-(2,5-dichlorophenylmethyl)amino group, N-(2,5-dichlorophenylmethyl)-N-methylamino group, N-(2,6-dichlorophenylmethyl)amino group, N-(2,6-dichlorophenylmethyl)-N-methylamino group, N-(3,4-dichlorophenylmethyl)amino group, N-(3,4-dichlorophenylmethyl)-N-methylamino group, N-(3,5-dichlorophenylmethyl)amino group, N-(3,5-dichlorophenylmethyl)-N-methylamino group, N-[2-(trifluoromethyl)phenylmethyl]amino group, N-methyl-N-[2-(trifluoromethyl)phenylmethyl]amino group, N-[3-(trifluoromethyl)phenylmethyl]amino group, N-methyl-N-[3-(trifluoromethyl)phenylmethyl]amino group, N-[4-(trifluoromethyl)phenylmethyl]amino group, N-methyl-N-[4-(trifluoromethyl)phenylmethyl]amino group, pyrrolidino group, 2-methylpyrrolidino group, 3-methylpyrrolidino group, 2,5-dimethylpyrrolidino group, 3,4-dimethylpyrrolidino group, piperidino group, 2-methylpiperidino group, 3-methylpiperidino group, 4-methylpiperidino group, 4-ethylpiperidino group, 4-propylpiperidino group, 4-isopropylpiperidino group, 4-butylpiperidino group, 4-isobutylpiperidino group, 4-cyclopentylpiperidino group, 4-cyclohexylpiperidino group, 4-(cyclopentylmethyl)piperidino group, 4-(cyclohexylmethyl)piperidino group, 4-phenylpiperidino group, 4-(2-methylphenyl)piperidino group, 4-(4-methylphenyl)piperidino group, 4-(2-fluorophenyl)piperidino group, 4-(3-fluorophenyl)piperidino group, 4-(4-fluorophenyl)piperidino group, 4-(2-chlorophenyl)piperidino group, 4-(3-chlorophenyl)piperidino group, 4-(4-chlorophenyl)piperidino group, 4-benzylpiperidino group, 4-(2-methylphenylmethyl)piperidino group, 4-(3-methylphenylmethyl)piperidino group, 4-methylphenylmethyl)piperidino group, 4-(2,3-dimethylphenylmethyl)piperidino group, 4-(3,5-dimethylphenylmethyl)piperidino group, 4-(2-fluorophenylmethyl)piperidino group, 4-(3-fluorophenylmethyl)piperidino group, 4-(4-fluorophenylmethyl)piperidino group, 4-(2-chlorophenylmethyl)piperidino group, 4-(3-chlorophenylmethyl)piperidino group, 4-(4-chlorophenylmethyl)piperidino group, 4-(2,3-difluorophenylmethyl)piperidino group, 4-(2,4-difluorophenylmethyl)piperidino group, 4-(2,5-difluorophenylmethyl)piperidino group, 4-(3,4-difluorophenylmethyl)piperidino group, 4-(2,3-dichlorophenylmethyl)piperidino group, 4-(2,4-dichlorophenylmethyl)piperidino group, 4-(2,5-dichlorophenylmethyl)piperidino group, 4-(2,6-dichlorophenylmethyl)piperidino group, 4-(3,4-dichlorophenylmethyl)piperidino group, 4-(3,5-dichlorophenylmethyl)piperidino group, 4-(3,6-dichlorophenylmethyl)piperidino group, 4-(2-methoxyphenylmethyl)piperidino group, 4-(3-methoxyphenylmethyl)piperidino group, 4-(4-methoxyphenylmethyl)piperidino group, 2,6-dimethylpiperidino group, 3,5-dimethylpiperidino group, 4,4-dimethylpiperidino group, homopiperidino group, morpholino group, 3,5-dimethylmorpholino group, 4-phenylpiperazino group, 4-(2-methylphenyl)piperazino group, 4-(4-methylphenyl)piperazino group, 4-(2-fluorophenyl)piperazino group, 4-(3-fluorophenyl)piperazino group, 4-(4-fluorophenyl)piperazino group, 4-(2-chlorophenyl)piperazino group, 4-(3-chlorophenyl)piperazino group, 4-(4-chlorophenyl)piperazino group, 4-benzylpiperazino group, 4-(2-methylphenylmethyl)piperazino group, 4-(3-methylphenylmethyl)piperazino group, 4-methylphenylmethyl)piperazino group, 4-(2,3-dimethylphenylmethyl)piperazino group, 4-(3,5-dimethylphenylmethyl)piperazino group, 4-(2-fluorophenylmethyl)piperazino group, 4-(3-fluorophenylmethyl)piperazino group, 4-(4-fluorophenylmethyl)piperazino group, 4-(2-chlorophenylmethyl)piperazino group, 4-(3-chlorophenylmethyl)piperazino group, 4-(4-chlorophenylmethyl)piperazino group, 4-(2,3-difluorophenylmethyl)piperazino group, 4-(2,4-difluorophenylmethyl)piperazino group, 4-(2,5-difluorophenylmethyl)piperazino group, 4-(3,4-difluorophenylmethyl)piperazino group, 4-(2,3-dichlorophenylmethyl)piperazino group, 4-(2,4-dichlorophenylmethyl)piperazino group, 4-(2,5-dichlorophenylmethyl)piperazino group, 4-(2,6-dichlorophenylmethyl)piperazino group, 4-(3,4-dichlorophenylmethyl)piperazino group, 4-(3,5-dichlorophenylmethyl)piperazino group, 4-(3,6-dichlorophenylmethyl)piperazino group, 4-(2-methoxyphenylmethyl)piperazino group, 4-(3-methoxyphenylmethyl)piperazino group, or 4-(4-methoxyphenylmethyl)piperazino group;
   the substituent V¹ on the ring (E) is Zx, and V² on the ring (E) is AR;
   Zx is hydrogen atom, fluorine atom, chlorine atom, bromine atom, methyl group, hydroxyl group, amino group, or N-methylamino group;
   AR is naphthalen-2-yl group, 6-hydroxynaphthalen-2-yl group, 6-methoxynaphthalen-2-yl group, 6-aminonaphthalen-2-yl group, 6-(N,N-dimethylamino)naphthalen-2-yl group, benzo[b]furan-5-yl group, benzo[b]thiophen-5-yl group, 1H-indol-5-yl group, 1-methyl-1H-indol-5-yl group, 1-ethyl-1H-indol-5-yl group, benzothiazol-6-yl group, 2-aminobenzothiazol-6-yl group, 2-oxo-2,3-dihydrobenzothiazol-6-yl group, quinolin-3-yl group, quinolin-6-yl group, 2-oxo-1,2-dihydroquinolin-6-yl group, 1H-indazol-5-yl group, 1-methyl-1H-indazol-5-yl group, 1-ethyl-1H-indazol-5-yl group, isoquinolin-6-yl group, 1-oxo-1,2-dihydroisoquinolin-6-yl group, cinnolin-6-yl group, or benzoxazol-5-yl group; and
   Y is hydrogen atom, methyl group, or ethyl group.
<50> The compound or salt thereof according to <1> mentioned above, wherein, in the formula (I), W is methylene group, or oxygen atom;
   when Rs represents -D-Rx, D is a single bond or oxygen atom;
   the substituent V¹ on the ring (E) is Zx, and V² on the ring (E) is AR;
   Zx is hydrogen atom or amino group; and
   AR is a residue of indole or 1H-indazole.
<51> The compound or salt thereof according to <1> mentioned above, wherein, in the formula (I), in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond or a double bond;
   when the bond between the ring-constituting carbon atom at the 1-position of the ring (E) and the Link moiety is a single bond, Link is CH₂ or CH₂CH₂, or when the bond between the ring-constituting carbon atom at the 1-position of the ring (E) and the Link moiety is a double bond, Link is CH;
   W is methylene group;
   when Rs represents -D-Rx, D is a single bond or oxygen atom;
   the substituent V¹ on the ring (E) is Zx, and V² on the ring (E) is AR;
   Zx is hydrogen atom or amino group; and
   AR is a residue of indole or 1H-indazole.
<52> The compound or salt thereof according to <1> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond or a double bond;
   when the bond between the ring-constituting carbon atom at the 1-position of the ring (E) and the Link moiety is a single bond, Link is CH₂, or when the bond between the ring-constituting carbon atom at the 1-position of the ring (E) and the Link moiety is a double bond, Link is CH;
   W is methylene group;
   when Rs represents -D-Rx, D is a single bond or oxygen atom;
   the substituent V¹ on the ring (E) is Zx, and V² on the ring (E) is AR;
   Zx is hydrogen atom or amino group; and
   AR is a residue of indole or 1H-indazole.
<53> The compound or salt thereof according to <1> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond;
   Link is CH₂;
   W is methylene group;
   when Rs represents -D-Rx, D is a single bond or oxygen atom;
   the substituent V¹ on the ring (E) is Zx, and V² on the ring (E) is AR;
   Zx is hydrogen atom or amino group; and
   AR is a residue of indole or 1H-indazole.
<54> The compound or salt thereof according to <1> mentioned above, wherein, in the formula (I), the bond between the ring-constituting carbon atom at the 1-position of the ring (E) and the ring-constituting carbon atom at the 2-position of the ring (E) is a double bond;
   the bond between the ring-constituting carbon atom at the 1-position of the ring (E) and the Link moiety is a single bond;
   Link is CH₂, or CH₂CH₂; and
   W is oxygen atom;
   when Rs represents -D-Rx, D is a single bond or oxygen atom;
   the substituent V¹ on the ring (E) is Zx, and V² on the ring (E) is AR;
   Zx is hydrogen atom or amino group; and
   AR is a residue of indole or 1H-indazole.
<55> The compound or salt thereof according to <1> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond;
   Link is CH₂;
   W is methylene group;
   Rs is -D-Rx or -N(Ry)(Rz);
   D is a single bond or oxygen atom;
   the substituent V¹ on the ring (E) is Zx, and V² on the ring (E) is AR;
   Zx is hydrogen atom or amino group; and
   AR is a residue of indole or 1H-indazole.
<56> The compound or salt thereof according to any one of <50> to <55> mentioned above, wherein Rx is propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb mentioned above;
   Q in Rb is phenyl group, thienyl group, furyl group, pyridyl group, oxazolyl group, naphthyl group, tetrahydronaphthyl group, indanyl group, indolyl group, or dihydrobenzodioxyl group;
   A² in Rb is a single bond, oxygen atom, sulfur atom, -N(methyl)-, or -N(ethyl)-;
   A¹ in Rb is a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² is oxygen atom, sulfur atom, -N(methyl)-, or -N(ethyl)-, A¹ is ethylene group, methylethylene group, dimethylethylene group, ethylethylene group, phenylethylene group, ethenylene group, trimethylene group, or methyltrimethylene group); and
   R² and R³ independently represent hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group. <57> The compound or salt thereof according to any one of <50> to <55> mentioned above, wherein Rx is propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group.
<58> The compound.or salt thereof according to any one of <50> to <57> mentioned above, wherein Rz is methyl group, ethyl group, propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb mentioned above;
   Q in Rb is phenyl group, thienyl group, furyl group, pyridyl group, oxazolyl group, naphthyl group, tetrahydronaphthyl group, indanyl group, indolyl group, or dihydrobenzodioxyl group;
   A² in Rb is a single bond, oxygen atom, sulfur atom, -N(methyl)-, or -N(ethyl)-;
   A¹ in Rb is a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² is oxygen atom, sulfur atom, -N(methyl)-, or -N(ethyl)-, A¹ is ethylene group, methylethylene group, dimethylethylene group, ethylethylene group, phenylethylene group, ethenylene group, trimethylene group, or methyltrimethylene group);
   R² and R³ independently represent hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group; and
   Ry is hydrogen atom, methyl group, ethyl group, propyl group, isopropyl group, butyl group, isobutyl group, pentyl group, or isopentyl group (provided that when Rz is methyl group, or ethyl group, Ry is a substituent other than hydrogen atom), or may bind to Rz to form, together with the nitrogen atom, a cyclic substituent (qy) selected from pyrrolidino group, piperidino group, homopiperidino group, morpholino group, piperazino group, homopiperazino group, pyrrol-1-yl group, imidazol-1-yl group, pyrazol-1-yl group, and thiomorpholino group, and the cyclic substituent (qy) may be substituted with one of alkyl group having 1 to 8 carbon atoms or two of the same or different alkyl groups having 1 to 8 carbon atoms, phenyl group, or phenylmethyl group (provided that the benzene ring of the phenyl group or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group).
<59> The compound or salt thereof according to any one of <50> to <57> mentioned above, wherein the substituent -N(Ry)(Rz) is N,N-dimethylamino group, N-ethyl-N-methylamino group, N,N-diethylamino group, N-methyl-N-propylamino group, N-ethyl-N-propylamino group, N-isopropyl-N-methylamino group, N-ethyl-N-isopropylamino group, N-butylamino group, N-butyl-N-methylamino group, N-butyl-N-ethylamino group, N-isobutylamino group, N-isobutyl-N-methylamino group, N-ethyl-N-isobutylamino group, N-isopentylamino group, N-isopentyl-N-methylamino group, N-ethyl-N-isopentylamino group, N-(2-ethylbutyl)amino group, N-(2-ethylbutyl)-N-methylamino group, N-cyclopentylamino group, N-cyclopentyl-N-methylamino group, N-cyclohexylamino group, N-cyclohexyl-N-methylamino group, N-cycloheptylamino group, N-(cyclopentylmethyl)amino group, N-(cyclopentylmethyl)-N-methylamino group, N-(cyclohexylmethyl)amino group, N-(cyclohexylmethyl)-N-methylamino group, N-phenylamino group, N-(2-methylphenyl)amino group, N-(4-methylphenyl)amino group, N-(2-fluorophenyl)amino group, N-(3-fluorophenyl)amino group, N-(4-fluorophenyl)amino group, N-(2-chlorophenyl)amino group, N-(3-chlorophenyl)amino group, N-(4-chlorophenyl)amino group, N-(indan-2-yl)amino group, N-(1-phenylethyl)amino group, N-[1-(2-fluorophenyl)ethyl]amino group, N-[1-(3-fluorophenyl)ethyl]amino group, N-[1-(4-fluorophenyl)ethyl]amino group, N-[1-(2-chlorophenyl)ethyl]amino group, N-[1-(3-chlorophenyl)ethyl]amino group, N-[1-(4-chlorophenyl)ethyl]amino group, N-benzylamino group, N-benzyl-N-methylamino group, N-benzyl-N-ethylamino group, N-(2-methylphenylmethyl)amino group, N-methyl-N-(2-methylphenylmethyl)amino group, N-(3-methylphenylmethyl)amino group, N-methyl-N-(3-methylphenylmethyl)amino group, N-(4-methylphenylmethyl)amino group, N-methyl-N-(4-methylphenylmethyl)amino group, N-(2-fluorophenylmethyl)amino group, N-(2-fluorophenylmethyl)-N-methylamino group, N-(3-fluorophenylmethyl)amino group, N-(3-fluorophenylmethyl)-N-methylamino group, N-(4-fluorophenylmethyl)amino group, N-(4-fluorophenylmethyl)-N-methylamino group, N-(2-chlorophenylmethyl)amino group, N-(2-chlorophenylmethyl)-N-methylamino group, N-(3-chlorophenylmethyl)amino group, N-(3-chlorophenylmethyl)-N-methylamino group, N-(4-chlorophenylmethyl)amino group, N-(4-chlorophenylmethyl)-N-methylamino group, N-(2,3-difluorophenylmethyl)amino group, N-(2,3-difluorophenylmethyl)-N-methylamino group, N-(2,4-difluorophenylmethyl)amino group, N-(2,4-difluorophenylmethyl)-N-methylamino group, N-(2,5-difluorophenylmethyl)amino group, N-(2,5-difluorophenylmethyl)-N-methylamino group, N-(3,4-difluorophenylmethyl)amino group, N-(3,4-difluorophenylmethyl)-N-methylamino group, N-(3,5-difluorophenylmethyl)amino group, N-(3,5-difluorophenylmethyl)-N-methylamino group, N-(2,3-dichlorophenylmethyl)amino group, N-(2,3-dichlorophenylmethyl)-N-methylamino group, N-(2,4-dichlorophenylmethyl)amino group, N-(2,4-dichlorophenylmethyl)-N-methylamino group, N-(2,5-dichlorophenylmethyl)amino group, N-(2,5-dichlorophenylmethyl)-N-methylamino group, N-(2,6-dichlorophenylmethyl)amino group, N-(2,6-dichlorophenylmethyl)-N-methylamino group, N-(3,4-dichlorophenylmethyl)amino group, N-(3,4-dichlorophenylmethyl)-N-methylamino group, N-(3,5-dichlorophenylmethyl)amino group, N-(3,5-dichlorophenylmethyl)-N-methylamino group, N-[2-(trifluoromethyl)phenylmethyl]amino group, N-methyl-N-[2-(trifluoromethyl)phenylmethyl]amino group, N-[3-(trifluoromethyl)phenylmethyl]amino group, N-methyl-N-[3-(trifluoromethyl)phenylmethyl]amino group, N-[4-(trifluoromethyl)phenylmethyl]amino group, N-methyl-N-[4-(trifluoromethyl)phenylmethyl]amino group, pyrrolidino group, 2-methylpyrrolidino group, 3-methylpyrrolidino group, 2,5-dimethylpyrrolidino group, 3,4-dimethylpyrrolidino group, piperidino group, 2-methylpiperidino group, 3-methylpiperidino group, 4-methylpiperidino group, 4-ethylpiperidino group, 4-propylpiperidino group, 4-isopropylpiperidino group, 4-butylpiperidino group, 4-isobutylpiperidino group, 4-cyclopentylpiperidino group, 4-cyclohexylpiperidino group, 4-(cyclopentylmethyl)piperidino group, 4-(cyclohexylmethyl)piperidino group, 4-phenylpiperidino group, 4-(2-methylphenyl)piperidino group, 4-(4-methylphenyl)piperidino group, 4-(2-fluorophenyl)piperidino group, 4-(3-fluorophenyl)piperidino group, 4-(4-fluorophenyl)piperidino group, 4-(2-chlorophenyl)piperidino group, 4-(3-chlorophenyl)piperidino group, 4-(4-chlorophenyl)piperidino group, 4-benzylpiperidino group, 4-(2-methylphenylmethyl)piperidino group, 4-(3-methylphenylmethyl)piperidino group, 4-methylphenylmethyl)piperidino group, 4-(2,3-dimethylphenylmethyl)piperidino group, 4-(3,5-dimethylphenylmethyl)piperidino group, 4-(2-fluorophenylmethyl)piperidino group, 4-(3-fluorophenylmethyl)piperidino group, 4-(4-fluorophenylmethyl)piperidino group, 4-(2-chlorophenylmethyl)piperidino group, 4-(3-chlorophenylmethyl)piperidino group, 4-(4-chlorophenylmethyl)piperidino group, 4-(2,3-difluorophenylmethyl)piperidino group, 4-(2,4-difluorophenylmethyl)piperidino group, 4-(2,5-difluorophenylmethyl)piperidino group, 4-(3,4-difluorophenylmethyl)piperidino group, 4-(2,3-dichlorophenylmethyl)piperidino group, 4-(2,4-dichlorophenylmethyl)piperidino group, 4-(2,5-dichlorophenylmethyl)piperidino group, 4-(2,6-dichlorophenylmethyl)piperidino group, 4-(3,4-dichlorophenylmethyl)piperidino group, 4-(3,5-dichlorophenylmethyl)piperidino group, 4-(3,6-dichlorophenylmethyl)piperidino group, 4-(2-methoxyphenylmethyl)piperidino group, 4-(3-methoxyphenylmethyl)piperidino group, 4-(4-methoxyphenylmethyl)piperidino group, 2,6-dimethylpiperidino group, 3,5-dimethylpiperidino group, 4,4-dimethylpiperidino group, homopiperidino group, morpholino group, 3,5-dimethylmorpholino group, 4-phenylpiperazino group, 4-(2-methylphenyl)piperazino group, 4-(4-methylphenyl)piperazino group, 4-(2-fluorophenyl)piperazino group, 4-(3-fluorophenyl)piperazino group, 4-(4-fluorophenyl)piperazino group, 4-(2-chlorophenyl)piperazino group, 4-(3-chlorophenyl)piperazino group, 4-(4-chlorophenyl)piperazino group, 4-benzylpiperazino group, 4-(2-methylphenylmethyl)piperazino group, 4-(3-methylphenylmethyl)piperazino group, 4-methylphenylmethyl)piperazino group, 4-(2,3-dimethylphenylmethyl)piperazino group, 4-(3,5-dimethylphenylmethyl)piperazino group, 4-(2-fluorophenylmethyl)piperazino group, 4-(3-fluorophenylmethyl)piperazino group, 4-(4-fluorophenylmethyl)piperazino group, 4-(2-chlorophenylmethyl)piperazino group, 4-(3-chlorophenylmethyl)piperazino group, 4-(4-chlorophenylmethyl)piperazino group, 4-(2,3-difluorophenylmethyl)piperazino group, 4-(2,4-difluorophenylmethyl)piperazino group, 4-(2,5-difluorophenylmethyl)piperazino group, 4-(3,4-difluorophenylmethyl)piperazino group, 4-(2,3-dichlorophenylmethyl)piperazino group, 4-(2,4-dichlorophenylmethyl)piperazino group, 4-(2,5-dichlorophenylmethyl)piperazino group, 4-(2,6-dichlorophenylmethyl)piperazino group, 4-(3,4-dichlorophenylmethyl)piperazino group, 4-(3,5-dichlorophenylmethyl)piperazino group, 4-(3,6-dichlorophenylmethyl)piperazino group, 4-(2-methoxyphenylmethyl)piperazino group, 4-(3-methoxyphenylmethyl)piperazino group, or 4-(4-methoxyphenylmethyl)piperazino group.
<60> The compound or salt thereof according to any one of <50> to <59> mentioned above, wherein AR is indol-5-yl group, indol-4-yl group, indol-6-yl group, 1H-indazol-5-yl group, 1H-indazol-4-yl group, or 1H-indazol-6-yl group (the aforementioned groups may be substituted with one of Xa or two or more of the same or different Xa). <61> The compound or salt thereof according to any one of <50> to <59> mentioned above, wherein AR is 1H-indol-5-yl group, 2-methyl-1H-indol-5-yl group, 3-methyl-1H-indol-5-yl group, 2,3-dimethyl-1H-indol-5-yl group, 1-methyl-1H-indol-5-yl group, 1,2-dimethyl-1H-indol-5-yl group, 1,3-dimethyl-1H-indol-5-yl group, 1,2,3-trimethyl-1H-indol-5-yl group, 1-ethyl-1H-indol-5-yl group, 1-ethyl-2-methyl-1H-indol-5-yl group, 1-ethyl-3-methyl-1H-indol-5-yl group, 1-ethyl-2,3-dimethyl-1H-indol-5-yl group, 1-propyl-1H-indol-5-yl group, 2-methyl-1-propyl-1H-indol-5-yl group, 3-methyl-1-propyl-1H-indol-5-yl group, 2,3-dimethyl-1-propyl-1H-indol-5-yl group, 1-(2-hydroxyethyl)-1H-indol-5-yl group, 1-(2-hydroxyethyl)-2-methyl-1H-indol-5-yl group, 1-(2-hydroxyethyl)-3-methyl-1H-indol-5-yl group, 2,3-dimethyl-1-(2-hydroxyethyl)-1H-indol-5-yl group, 1H-indazol-5-yl group, 1-methyl-1H-indazol-5-yl group, 1-ethyl-1H-indazol-5-yl group, 1-propyl-1H-indazol-5-yl group, 1-(2-hydroxyethyl)-1H-indazol-5-yl group, 3-hydroxy-1H-indazol-5-yl group, or 3-hydroxy-1-methyl-1H-indazol-5-yl group. <62> The compound or salt thereof according to any one of <50> to <59> mentioned above, whereinAR is 1H-indol-5-yl group, 1-methyl-1H-indol-5-yl group, 1-ethyl-1H-indol-5-yl group, 1H-indazol-5-yl group, 1-methyl-1H-indazol-5-yl group, or 1-ethyl-1H-indazol-5-yl group.
<63> The compound or salt thereof according to <1> mentioned above, wherein, in the formula (I), the bond between the ring-constituting carbon atom at the 1-position of the ring (E) and the ring-constituting carbon atom at the 2-position of the ring (E) is a single bond;
   the bond between the ring-constituting carbon atom at the 1-position of the ring (E) and the Link moiety is a single bond or a double bond;
   when the bond between the ring-constituting carbon atom at the 1-position of the ring (E) and the Link moiety is a single bond, Link is CH₂, or when the bond between the ring-constituting carbon atom at the 1-position of the ring (E) and the Link moiety is a double bond, Link is CH;
   W is methylene group;
   Rs is -D-Rx or -N(Ry)(Rz);
   D is a single bond or oxygen atom;
   Rx is propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group;
   the substituent -N(Ry)(Rz) is N,N-dimethylamino group, N-ethyl-N-methylamino group, N,N-diethylamino group, N-methyl-N-propylamino group, N-ethyl-N-propylamino group, N-isopropyl-N-methylamino group, N-ethyl-N-isopropylamino group, N-butylamino group, N-butyl-N-methylamino group, N-butyl-N-ethylamino group, N-isobutylamino group, N-isobutyl-N-methylamino group, N-ethyl-N-isobutylamino group, N-isopentylamino group, N-isopentyl-N-methylamino group, N-ethyl-N-isopentylamino group, N-(2-ethylbutyl)amino group, N-(2-ethylbutyl)-N-methylamino group, N-cyclopentylamino group, N-cyclopentyl-N-methylamino group, N-cyclohexylamino group, N-cyclohexyl-N-methylamino group, N-cycloheptylamino group, N-(cyclopentylmethyl)amino group, N-(cyclopentylmethyl)-N-methylamino group, N-(cyclohexylmethyl)amino group, N-(cyclohexylmethyl)-N-methylamino group, N-phenylamino group, N-(2-methylphenyl)amino group, N-(4-methylphenyl)amino group, N-(2-fluorophenyl)amino group, N-(3-fluorophenyl)amino group, N-(4-fluorophenyl)amino group, N-(2-chlorophenyl)amino group, N-(3-chlorophenyl)amino group, N-(4-chlorophenyl)amino group, N-(indan-2-yl)amino group, N-(1-phenylethyl)amino group, N-[1-(2-fluorophenyl)ethyl]amino group, N-[1-(3-fluorophenyl)ethyl]amino group, N-[1-(4-fluorophenyl)ethyl]amino group, N-[1-(2-chlorophenyl)ethyl]amino group, N-[1-(3-chlorophenyl)ethyl]amino group, N-[1-(4-chlorophenyl)ethyl]amino group, N-benzylamino group, N-benzyl-N-methylamino group, N-benzyl-N-ethylamino group, N-(2-methylphenylmethyl)amino group, N-methyl-N-(2-methylphenylmethyl)amino group, N-(3-methylphenylmethyl)amino group, N-methyl-N-(3-methylphenylmethyl)amino group, N-(4-methylphenylmethyl)amino group, N-methyl-N-(4-methylphenylmethyl)amino group, N-(2-fluorophenylmethyl)amino group, N-(2-fluorophenylmethyl)-N-methylamino group, N-(3-fluorophenylmethyl)amino group, N-(3-fluorophenylmethyl)-N-methylamino group, N-(4-fluorophenylmethyl)amino group, N-(4-fluorophenylmethyl)-N-methylamino group, N-(2-chlorophenylmethyl)amino group, N-(2-chlorophenylmethyl)-N-methylamino group, N-(3-chlorophenylmethyl)amino group, N-(3-chlorophenylmethyl)-N-methylamino group, N-(4-chlorophenylmethyl)amino group, N-(4-chlorophenylmethyl)-N-methylamino group, N-(2,3-difluorophenylmethyl)amino group, N-(2,3-difluorophenylmethyl)-N-methylamino group, N-(2,4-difluorophenylmethyl)amino group, N-(2,4-difluorophenylmethyl)-N-methylamino group, N-(2,5-difluorophenylmethyl)amino group, N-(2,5-diffuorophenylmethyl)-N-methylamino group, N-(3,4-difluorophenylmethyl)amino group, N-(3,4-difluorophenylmethyl)-N-methylamino group, N-(3,5-difluorophenylmethyl)amino group, N-(3,5-difluorophenylmethyl)-N-methylamino group, N-(2,3-dichlorophenylmethyl)amino group, N-(2,3-dichlorophenylmethyl)-N-methylamino group, N-(2,4-dichlorophenylmethyl)amino group, N-(2,4-dichlorophenylmethyl)-N-methylamino group, N-(2,5-dichlorophenylmethyl)amino group, N-(2,5-dichlorophenylmethyl)-N-methylamino group, N-(2,6-dichlorophenylmethyl)amino group, N-(2,6-dichlorophenylmethyl)-N-methylamino group, N-(3,4-dichlorophenylmethyl)amino group, N-(3,4-dichlorophenylmethyl)-N-methylamino group, N-(3,5-dichlorophenylmethyl)amino group, N-(3,5-dichlorophenylmethyl)-N-methylamino group, N-[2-(trifluoromethyl)phenylmethyl]amino group, N-methyl-N-[2-(trifluoromethyl)phenylmethyl]amino group, N-[3-(trifluoromethyl)phenylmethyl]amino group, N-methyl-N-[3-(trifluoromethyl)phenylmethyl]amino group, N-[4-(trifluoromethyl)phenylmethyl]amino group, N-methyl-N-[4-(trifluoromethyl)phenylmethyl]amino group, pyrrolidino group, 2-methylpyrrolidino group, 3-methylpyrrolidino group, 2,5-dimethylpyrrolidino group, 3,4-dimethylpyrrolidino group, piperidino group, 2-methylpiperidino group, 3-methylpiperidino group, 4-methylpiperidino group, 4-ethylpiperidino group, 4-propylpiperidino group, 4-isopropylpiperidino group, 4-butylpiperidino group, 4-isobutylpiperidino group, 4-cyclopentylpiperidino group, 4-cyclohexylpiperidino group, 4-(cyclopentylmethyl)piperidino group, 4-(cyclohexylmethyl)piperidino group, 4-phenylpiperidino group, 4-(2-methylphenyl)piperidino group, 4-(4-methylphenyl)piperidino group, 4-(2-fluorophenyl)piperidino group, 4-(3-fluorophenyl)piperidino group, 4-(4-fluorophenyl)piperidino group, 4-(2-chlorophenyl)piperidino group, 4-(3-chlorophenyl)piperidino group, 4-(4-chlorophenyl)piperidino group, 4-benzylpiperidino group, 4-(2-methylphenylmethyl)piperidino group, 4-(3-methylphenylmethyl)piperidino group, 4-methylphenylmethyl)piperidino group, 4-(2,3-dimethylphenylmethyl)piperidino group, 4-(3,5-dimethylphenylmethyl)piperidino group, 4-(2-fluorophenylmethyl)piperidino group, 4-(3-fluorophenylmethyl)piperidino group, 4-(4-fluorophenylmethyl)piperidino group, 4-(2-chlorophenylmethyl)piperidino group, 4-(3-chlorophenylmethyl)piperidino group, 4-(4-chlorophenylmethyl)piperidino group, 4-(2,3-difluorophenylmethyl)piperidino group, 4-(2,4-difluorophenylmethyl)piperidino group, 4-(2,5-difluorophenylmethyl)piperidino group, 4-(3,4-difluorophenylmethyl)piperidino group, 4-(2,3-dichlorophenylmethyl)piperidino group, 4-(2,4-dichlorophenylmethyl)piperidino group, 4-(2,5-dichlorophenylmethyl)piperidino group, 4-(2,6-dichlorophenylmethyl)piperidino group, 4-(3,4-dichlorophenylmethyl)piperidino group, 4-(3,5-dichlorophenylmethyl)piperidino group, 4-(3,6-dichlorophenylmethyl)piperidino group, 4-(2-methoxyphenylmethyl)piperidino group, 4-(3-methoxyphenylmethyl)piperidino group, 4-(4-methoxyphenylmethyl)piperidino group, 2,6-dimethylpiperidino group, 3,5-dimethylpiperidino group, 4,4-dimethylpiperidino group, homopiperidino group, morpholino group, 3,5-dimethylmorpholino group, 4-phenylpiperazino group, 4-(2-methylphenyl)piperazino group, 4-(4-methylphenyl)piperazino group, 4-(2-fluorophenyl)piperazino group, 4-(3-fluorophenyl)piperazino group, 4-(4-fluorophenyl)piperazino group, 4-(2-chlorophenyl)piperazino group, 4-(3-chlorophenyl)piperazino group, 4-(4-chlorophenyl)piperazino group, 4-benzylpiperazino group, 4-(2-methylphenylmethyl)piperazino group, 4-(3-methylphenylmethyl)piperazino group, 4-methylphenylmethyl)piperazino group, 4-(2,3-dimethylphenylmethyl)piperazino group, 4-(3,5-dimethylphenylmethyl)piperazino group, 4-(2-fluorophenylmethyl)piperazino group, 4-(3-fluorophenylmethyl)piperazino group, 4-(4-fluorophenylmethyl)piperazino group, 4-(2-chlorophenylmethyl)piperazino group, 4-(3-chlorophenylmethyl)piperazino group, 4-(4-chlorophenylmethyl)piperazino group, 4-(2,3-difluorophenylmethyl)piperazino group, 4-(2,4-difluorophenylmethyl)piperazino group, 4-(2,5-difluorophenylmethyl)piperazino group, 4-(3,4-difluorophenylmethyl)piperazino group, 4-(2,3-dichlorophenylmethyl)piperazino group, 4-(2,4-dichlorophenylmethyl)piperazino group, 4-(2,5-dichlorophenylmethyl)piperazino group, 4-(2,6-dichlorophenylmethyl)piperazino group, 4-(3,4-dichlorophenylmethyl)piperazino group, 4-(3,5-dichlorophenylmethyl)piperazino group, 4-(3,6-dichlorophenylmethyl)piperazino group, 4-(2-methoxyphenylmethyl)piperazino group, 4-(3-methoxyphenylmethyl)piperazino group, or 4-(4-methoxyphenylmethyl)piperazino group;
   the substituent V¹ on the ring (E) is Zx, and V² on the ring (E) is AR;
   Zx is hydrogen atom or amino group;
   AR is 1H-indol-5-yl group, 2-methyl-1H-indol-5-yl group, 3-methyl-1H-indol-5-yl group, 2,3-dimethyl-1H-indol-5-yl group, 1-methyl-1H-indol-5-yl group, 1,2-dimethyl-1H-indol-5-yl group, 1,3-dimethyl-1H-indol-5-yl group, 1,2,3-trimethyl-1H-indol-5-yl group, 1-ethyl-1H-indol-5-yl group, 1-ethyl-2-methyl-1H-indol-5-yl group, 1-ethyl-3-methyl-1H-indol-5-yl group, 1-ethyl-2,3-dimethyl-1H-indol-5-yl group, 1-propyl-1H-indol-5-yl group, 2-methyl-1-propyl-1H-indol-5-yl group, 3-methyl-1-propyl-1H-indol-5-yl group, 2,3-dimethyl-1-propyl-1H-indol-5-yl group, 1-(2-hydroxyethyl)-1H-indol-5-yl group, 1-(2-hydroxyethyl)-2-methyl-1H-indol-5-yl group, 1-(2-hydroxyethyl)-3-methyl-1H-indol-5-yl group, 2,3-dimethyl-1-(2-hydroxyethyl)-1H-indol-5-yl group, 1H-indazol-5-yl group, 1-methyl-1H-indazol-5-yl group, 1-ethyl-1H-indazol-5-yl group, 1-propyl-1H-indazol-5-yl group, 1-(2-hydroxyethyl)-1H-indazol-5-yl group, 3-hydroxy-1H-indazol-5-yl group, 3-hydroxy-1-methyl-1H-indazol-5-yl group, or 1-ethyl-3-hydroxy-1H-indazol-5-yl group; and
   Y is hydrogen atom, methyl group, or ethyl group.

From another aspect of the present invention, there is provided:
<64> A medicament comprising a compound represented by the aforementioned formula (I) having an arbitrary combination of choices selected from all of those described in this specification or a pharmacologically acceptable salt thereof as an active ingredient.
   The medicament of the present invention can be applied to various diseases as:
<65> An agent for suppressing production of a prostaglandin and/or a leukotriene, which comprises a compound represented by the aforementioned formula (I) having an arbitrary combination of choices selected from all of those described in this specification or a pharmacologically acceptable salt thereof as an active ingredient.
   Specifically, there is provided:
<66> The medicament according to <64> mentioned above, which is for prophylactic and/or therapeutic treatment of a disease induced by production of a prostaglandin and/or a leukotriene;
   and as the aforementioned medicament for prophylactic and/or therapeutic treatment of a disease, the present invention provides, for example:
<67> The medicament according to <64> mentioned above, which is for prophylactic and/or therapeutic treatment of an inflammatory disease of mammal;
<68> The medicament according to <64> mentioned above, which is for prophylactic and/or therapeutic treatment of an autoimmune disease of mammal;
<69> The medicament according to <64> mentioned above, which is for prophylactic and/or therapeutic treatment of an allergic disease of mammal;
<70> The medicament according to <64> mentioned above, which is for antipyresis and/or analgesis of mammal;
<71> The medicament according to <64> mentioned above, which is for prophylactic and/or therapeutic treatment of a fibrosis of mammal;
   and the like.

From a further aspect of the present invention, there is also provided:
<72> The medicament according to <64> mentioned above, which is for prophylactic and/or therapeutic treatment of a biological condition of mammal in which an acute or chronic inflammatory reaction is observed.
   According to preferred embodiments of these medicaments, there is provided:
<73> The medicament according to any one of <64> to <72> mentioned above, which is in the form of a pharmaceutical composition comprising a prophylactically and/or therapeutically effective amount of a compound represented by the aforementioned formula (I) having an arbitrary combination of choices selected from all of those described in this specification or a pharmacologically acceptable salt thereof and a pharmacologically acceptable carrier.

From a still further aspect, the present invention provides:
<74> A method for prophylactic and/or therapeutic treatment of a disease induced by production of a prostaglandin and/or a leukotriene, which comprises administering a prophylactically and/or therapeutically effective amount of a compound represented by the aforementioned formula (I) having an arbitrary combination of choices selected from all of those described in this specification or a pharmacologically acceptable salt thereof to a mammal.
   As the aforementioned method for prophylactic and/or therapeutic treatment, the present invention provides:
<75> The method according to <74> mentioned above, which is a method for prophylactic and/or therapeutic treatment of an inflammatory disease of mammal; <76> The method according to <74> mentioned above, which is a method for prophylactic and/or therapeutic treatment of an autoimmune disease of mammal; <77> The method according to <74> mentioned above, which is a method for prophylactic and/or therapeutic treatment of an allergic disease of mammal;
<78> The method according to <74> mentioned above, which is a method for antipyresis and/or analgesis of mammal;
<79> The method according to <74> mentioned above, which is a method for prophylactic and/or therapeutic treatment of a fibrosis of mammal;
   and the like.

Examples of the diseases which can be an object of application of the medicament and the method for prophylactic and/or therapeutic treatment of the present invention include, for example, diseases diagnosed as arthritis, chronic rheumatoid arthritis, malignant rheumatoid arthritis, juvenile rheumatoid arthritis, Felty's syndrome, adult Still's disease, osteoarthritis, synovitis, gout, slack of artificial joint implant, fervescence, common cold, algesia, burn, thermal injury, keloplasty, menstrual pain, dysmenorrhea, menstrual cramp, allergic reaction, allergic contact hypersensitivity, allergic rhinitis, pollinosis, allergic conjunctivitis, hypersensitivity pneumonitis, allergic bronchopulmonary mycosis, emphysema, acute respiratory distress syndrome, asthma, bronchitis, chronic obstructive pulmonary disease, chronic bronchitis, pulmonary emphysema, diffuse panbronchiolitis, respiratory obstruction, graft versus host syndrome, urticaria, ultraviolet radiation dermatitis, atopic dermatitis, cancer, myelogenous leukemia, sarcomata, brain tumor, cachexia, tissue ulcer, digestive ulcer, gastritis, acute and chronic pancreatitis, regional enteritis, ulcerative colitis, diverticulitis, recurrent gastroenteric disorder, gastroenteric bleeding, inflammatory bowel disease, Crohn's disease, intestinal tract type Behcet's disease, infectious enteritis, ischemic enteritis, radiation enteritis, drug-induced enteritis, irritable bowel syndrome, hepatic diseases (hepatopathies, liver failures) such as acute hepatitis, fulminant hepatitis, chronic hepatitis, hepatic cirrhosis, fatty liver, alcoholic liver injury, drug liver injury (drug-induced hepatitis), congestive hepatitis, autoimmune hepatitis, primary biliary cirrhosis and hepatic porphyria, coagulation, anemia, ankylosing spondilitis, restenosis, periodontosis, epidermolysis bullosa, atherosclerosis, aortic aneurysm, periarteritis nodosa, congestive cardiac failure, arrhythmia, myocardial infarction, cerebral infarction, attack, cerebral ischemia, head injury, spinal cord injury, myelopathic muscular atrophy, neuralgia, neurodegenerative disease, Alzheimer's disease, Lewy body disease, Shy-Drager syndrome, Reye's syndrome, progressive supranuclear palsy, progressive multifocal leukoencephalopathy, normal pressure hydrocephalus, subacute sclerosing panencephalitis, frontal lobe type dementia, acute anterior poliomyelitis (poliomyelitis), poliomyelitis neurosis, viral encephalitis, Creutzfeldt-Jakob disease, Kuru disease, bovine spongiform encephalopathy (mad cow disease), scrapie, epilepsy, cerebral amyloid angiopathy, autoimmune disease, Huntington's disease, Parkinson's disease, migraine, depression, mania, manic-depressive psychosis, hereditary cerebellar ataxia, peripheral neuropathy, glaucoma, pain, gingivitis, postoperative pain, amyotrophic lateral sclerosis, osteoporosis, multiple sclerosis, ocular angiogenesis, cornea damage, macular degeneration, conjunctivitis, abnormal wound healing, sprain or strain of muscle or joint, tendinitis, skin disease, psoriasis vulgaris, pustular psoriasis, erythroderma psoriaticum, arthritic psoriasis, myasthenia gravis, multiple myositis, myositis, bursitis, diabetes mellitus, tumor invasion, tumor growth, tumor metastasis, cornea scar, scleritis, immunodeficiency disease, pachydermia, eosinophilic fasciitis, sepsis, endotoxin shock, premature delivery, hypoprothrombinemia, hemophilia, thyroiditis, sarcoidosis, Behcet's syndrome, hypersensitivity, renal disease, rickettsial infectious disease, protozoal disease, reproduction disease, sepsis shock, toothache, pain after tooth extraction, back or low back pain, periarthritis humeroscapularis, cervico-omo-brachial syndrome, tenosynovitis, acute upper respiratory inflammation, herpes zoster, fibrosis, pulmonary fibrosis, drug induced pulmonary fibrosis, pneumoconiosis, chronic interstitial pneumonia, granulomatous interstitial pneumonia, fibrosing interstitial pneumonia, renal fibrosis, nephropyelitis, various types of secondary contracted kidney, glomerular nephritis, chronic nephritis, glomerulosclerosis, hepatic fibrosis, cardiac fibrosis after myocardial infarction, idiopathic cardiomyopathy, pancreatic sclerosis, pancreatic fibrosis, pancreatolithiasis, Takayasu's arteritis, chronic thyroiditis, dermatomyositis, multiple myositis, myelofibrosis, Banti disease, retroperitoneal fibrosis, and various radiation injuries, pathological conditions suspected to be these diseases, and the like.

### Effect of the Invention

The compound (I) of the present invention or a pharmacologically acceptable salt thereof has an action of suppressing the production of both of prostaglandins and leukotrienes, and said compound has features that, when administered to a human or animal, the compound exerts superior prophylactic and/or therapeutic effect on diseases or pathological conditions in which a prostaglandin and/or leukotriene is involved, and the compound has extremely low toxicity.

### Best Mode for Carrying out the Invention

This application is a patent application filed with claiming a conventional priority based on Japanese Patent Application No. 2006-095008 filed in Japan on March 30, 2006. The entire disclosures of Japanese Patent Application No. 2006-095008 including those of the specification and the claims are incorporated herein by reference.
In the present specification, carbon atom may sometimes be represented simply by "C", hydrogen atom by "H", oxygen atom by "O", sulfur atom by "S", and nitrogen atom by "N".
- - - - in the aforementioned formula (I) represents a single bond, or a double bond. However, both the bond between the ring-constituting carbon atom at the 1-position of the ring (E) and the ring-constituting carbon atom at the 2-position of the ring (E), and the bond between the ring-constituting carbon atom at the 1-position of the ring (E) and the Link moiety do not simultaneously represent a double bond, and when Link represents a single bond, the bond between the ring-constituting carbon atom at the 1-position of the ring (E) and the Link moiety represents a single bond.
When the bond between the ring-constituting carbon atom at the 1-positi on of the ring (E) and the ring-constituting carbon atom at the 2-position of the ring (E), and the bond between the ring-constituting carbon atom at the 1-posit ion of the ring (E) and the Link moiety in the formula (I) both represent a sing le bond, the ring-constituting carbon atom at the 1-position of the ring (E) is an asymmetric carbon atom, and at least two kinds of optical isomers exist. Stere oisomers such as the aforementioned optical isomers and diastereomers occurring due to an asymmetric carbon existing in a substituent other than the aforemen tioned asymmetric carbon in pure forms, arbitrary mixtures of such stereoisomer s, racemates and the like all fall within the scope of the present invention.

Examples of Link in the aforementioned formula (I) include a single bond, and a saturated or unsaturated straight hydrocarbon chain having 1 or 2 carbon atoms.
When Link represents a single bond, it is meant that the ring-constituting carbon atom at the 1-position of the ring (E) and COOY in the formula (I) are directly bound, and in such case, the ring-constituting carbon atom at the 1-position of the ring (E) and COOY are bound with a single bond.
As the saturated straight hydrocarbon chain having 1 or 2 carbon atoms, CH₂ or CH₂CH₂ is preferred, CH₂ is particularly preferred. When Link represents any one of these saturated hydrocarbon chains, the bond between the ring-constituting carbon atom at the 1-position of the ring (E) and the Link moiety are bound with a single bond.
The unsaturated hydrocarbon chain having 1 or 2 carbon atoms means a hydrocarbon chain in which the carbon-carbon bonds including the bond with the ring-constituting carbon atom at the 1-position of the ring (E) include an unsaturated bond which is a double bond or a triple bond. Specific examples of the unsaturated hydrocarbon chain including bond between the ring-constituting carbon atom at the 1-position of the ring (E) and COOY include:
the ring-constituting carbon atom at the 1-position of the ring (E)(double bond)CH-COOY;
the ring-constituting carbon atom at the 1-position of the ring (E)(double bond)CHCH₂-COOY;
the ring-constituting carbon atom at the 1-position of the ring (E)-CH(double bond)CH-COOY;
the ring-constituting carbon atom at the 1-position of the ring (E)(double bond)C-(double bond)CH-COOY;
the ring-constituting carbon atom at the 1-position of the ring (E)-C(triple bond)C-COOY;
and the like. Among them,
the ring-constituting carbon atom at the 1-position of the ring (E)(double bond)CH-COOY;
is a particularly preferred example.
As Link, CH, CH₂, and CH₂CH₂ are preferred examples, CH, and CH₂ are particularly preferred examples, and CH₂ is an extremely preferred example.

W in the aforementioned formula (I) represents a single bond, methylene group, oxygen atom, sulfur atom, or N(Rw). Specifically, when W represents a single bond, the ring (E) represents benzocyclobutane ring or benzocyclobutene ring, when W represents methylene group, the ring (E) represents indan ring or indene ring, when W represents oxygen atom, the ring (E) represents dihydrobenzofuran ring or benzofuran ring, when W represents sulfur atom, the ring (E) represents dihydrobenzothiophene ring or benzothiophenering, and when W represents N(Rw), the ring (E) represents indoline ring or indole ring. Preferred examples of W include methylene group, oxygen atom, N(Rw) and the like, and methylene group is a particularly preferred example.
Rw in N(Rw) as W represents hydrogen atom, an alkyl group having 1 to 8 carbon atoms or -A⁶-Qp. The alkyl group having 1 to 8 carbon atoms represents such a linear or branched saturated alkyl group, linear or branched alkyl group partially containing unsaturated bond, or alkyl group which may contain a cycloalkyl group having 3 to 7 carbon atoms, and examples include, for example, methyl group, ethyl group, propyl group, isopropyl group, butyl group, isobutyl group, t-butyl group, pentyl group, isopentyl group, 2-methylbutyl group, 2,2-dimethylpropyl group, hexyl group, 4-methylpentyl group, 2,3-dimethylbutyl group, 2-ethylbutyl group, heptyl group, octyl group, cyclopropyl group, cyclobutyl group, cyclopentyl group, cyclohexyl group, cycloheptyl group, cyclopropylmethyl group, cyclobutylmethyl group, cyclopentylmethyl group, cyclohexylmethyl group, cycloheptylmethyl group, 2-cyclopentylethyl group, 2-cyclohexylethyl group, 2-methylcyclopentyl group, 3-methylcyclopentyl group, 3,4-dimethylcyclopentyl group, 4-methylcyclohexyl group, 4,4-dimethylcyclohexyl group, 4-ethylcyclohexyl group, 4-methylcyclohexylmethyl group, and the like. As Rw, hydrogen atom, methyl group and the like are preferred examples.

A⁶ in the substituent -A⁶-Qp represents a single bond or methylene group, and Qp represents a phenyl group which may be substituted with one of T¹ or two or more of the same or different T¹. The substituent T¹ is a linear or branched saturated alkyl group having 1 to 4 carbon atoms, hydroxyl group, fluorine atom, chlorine atom, bromine atom, trifluoromethyl group, nitro group, an alkoxy group having 1 to 4 carbon atoms, or a mono- or dialkylamino group having 1 to 4 carbon atoms. Specific examples of -A⁶-Qp include phenyl group, methylphenyl group, chlorophenyl group, benzyl group, methylbenzyl group, chlorobenzyl group, dichlorobenzyl group, fluorobenzyl group, trifluoromethylbenzyl group, nitrobenzyl group, methoxyphenyl group, N-methylaminobenzyl group, N,N-dimethylaminobenzyl group, and the like.
In this specification, as represented by A⁶, Qp and T¹, for example, the same symbols may sometimes be used simultaneously at different positions. These symbols are used to mean the same class of groups of substituents. However, because each substituent is independently chosen from each other, the same symbols do not mean that an identical substituent should be necessarily chosen, and as a result, selection of the same or different kind of substituent is not prohibited.

A preferred example of the ring (E) containing W is the ring wherein W represents methylene group, and the bond between the ring-constituting carbon atom at the 1-position of the ring (E) and the ring-constituting carbon atom at the 2-position of the ring (E) represents a single bond, i.e., the ring (E) contains indane ring. Another preferred example is the ring wherein W represents oxygen atom, and the bond between the ring-constituting carbon atom at the 1-position of the ring (E) and the ring-constituting carbon atom at the 2-position of the ring (E) represents a double bond, i.e., the ring (E) contains benzofuran ring. A still more preferred other example is the ring wherein W represents NH, or N(methyl), and the bond between the ring-constituting carbon atom at the 1-position of the ring (E) and the ring-constituting carbon atom at the 2-position of the ring (E) represents a double bond, i.e., the ring (E) represents 1H-indole ring, or 1-methylindole ring. Among them, those wherein the ring (E) represents indan ring are particularly preferred.

In the aforementioned formula (I), Rs represents -D-Rx or -N(Ry)(Rz).
D represents a single bond, oxygen atom, sulfur atom, -S(O)-, -S(O)₂-, or - C(O)-. Among them, a single bond, oxygen atom and sulfur atom are preferred examples, and a single bond, and oxygen atom are particularly preferred examples.
Rx represents a linear or branched saturated alkyl group having 3 to 8 carbon atoms, or represents Ra, Rb, or Rc mentioned above.
As for Rx, examples of the linear or branched saturated alkyl group having 3 to 8 carbon atoms include, for example, propyl group, isopropyl group, butyl group, isobutyl group, 1-methylpropyl group, t-butyl group, pentyl group, isopentyl group, 2-methylbutyl group, 2,2-dimethylpropyl group, hexyl group, 4-methylpentyl group, 2,3-dimethylbutyl group, 2-ethylbutyl group, heptyl group, octyl group, and the like, and propyl group, isopropyl group, butyl group, isobutyl group, pentyl group, isopentyl group, and 2-ethylbutyl group are particularly preferred.
As for Rx, R¹ of Ra is defined to be a saturated cyclic alkyl group having 3 to 7 carbon atoms substituted with a lower alkyl group having 1 to 4 carbon atoms or an unsubstituted saturated cyclic alkyl group having 3 to 7 carbon atoms, or a condensed saturated cyclic alkyl group having 6 to 8 carbon atoms substituted with a lower alkyl group having 1 to 4 carbon atoms or an unsubstituted condensed saturated cyclic alkyl group having 6 to 8 carbon atoms. As for R¹, examples of the saturated cyclic alkyl group having 3 to 7 carbon atoms include cyclopropyl group, cyclobutyl group, cyclopentyl group, cyclohexyl group, cycloheptyl group, and the like, and cyclopentyl group, cyclohexyl group, and cycloheptyl group are particularly preferred. As for R¹, examples of the condensed saturated cyclic alkyl group having 6 to 8 carbon atoms group include bicyclo[2,2,1]heptyl group, bicyclo[2,2,2]octyl group, and the like.

Examples of the lower alkyl group having 1 to 4 carbon atoms substituting on R¹ include methyl group, ethyl group, propyl group, isopropyl group, butyl group, isobutyl group, t-butyl group, and the like. Examples of R¹ substituted with a lower alkyl group having 1 to 4 carbon atoms include methylcyclopentyl group, methylcyclohexyl group, methylbicyclo[2,2,1]heptyl group, and the like.
A^{a} is defined to be a single bond, an alkylene (aa) having 1 to 3 carbon atoms, i.e., methylene group, ethylene group, or trimethylene group, or an alkenylene (aa') having 2 or 3 carbon atoms, i.e., ethenylene group, or propenylene group. The alkylene (a) and the alkenylene (a') include those substituted with a lower alkyl group having 1 to 4 carbon atoms. In such case, examples of the lower alkyl group having 1 to 4 carbon atoms include methyl group, ethyl group, propyl group, isopropyl group, butyl group, isobutyl group, t-butyl group and the like. Preferred examples of A^{a} include a single bond, methylene group, ethylene group, trimethylene group and the like, and particularly preferred examples include a single bond, methylene group, and ethylene group.
Specific examples of Ra include cyclopropyl group, cyclobutyl group, cyclopentyl group, cyclohexyl group, cycloheptyl group, cyclopropylmethyl group, cyclobutylmethyl group, cyclopentylmethyl group, cyclohexylmethyl group, cycloheptylmethyl group, 2-cyclopentylethyl group, 2-cyclohexylethyl group, 3-cyclohexylpropyl group, 2-methylcyclopentyl group, 3-methylcyclopentyl group, 3,4-dimethylcyclopentyl group, 4-methylcyclohexyl group, 4,4-dimethylcyclohexyl group, 4-ethylcyclohexyl group, 4-methylcyclohexylmethyl group, bicyclo[2,2,1]heptan-2-ylmethyl group, bicyclo[2,2,2]octan-2-ylmethyl group, 3-methylbicyclo[2,2,1]heptan-2-ylmethyl group, bicyclo[2,2,1]hept-1-ylmethyl group, bicyclo[2,2,2]oct-1-ylmethyl group and the like, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, 2-cycloheptylethyl group and the like are preferred, and cyclopentyl group, cyclohexyl group, cycloheptyl group, cyclopentylmethyl group, cyclohexylmethyl group, cycloheptylmethyl group and the like are particularly preferred.

As for Rx, A² in Rb is defined to be a single bond, oxygen atom, sulfur atom, - S(O)-, -S(O)₂-, or -N(R⁴)-. R⁴ is defined to be a lower alkyl group having 1 to 4 carbon atoms. Preferred examples are methyl group, ethyl group, propyl group, isopropyl group, butyl group, isobutyl group, t-butyl group, and the like, and methyl group and ethyl group are particularly preferred examples. Therefore, particularly preferred examples of A² include a single bond, oxygen atom, sulfur atom, -N(methyl)-, and - N(ethyl)-.
A¹ is defined to be a single bond, an alkylene (a1) having 1 to 3 carbon atoms, i.e., methylene group, ethylene group, or trimethylene group, or an alkenylene (a1') having 2 or 3 carbon atoms, i.e., ethenylene group, or propenylene group. However, when A² represents oxygen atom, sulfur atom, -S(O)-, -S(O)₂- or -N(R⁴)-, A¹ is the alkylene (a1), or the alkenylene (a1'). Further, the alkylene (a1) and the alkenylene (a1') include those substituted with a lower alkyl group having 1 to 4 carbon atoms or phenyl group. In such case, examples of the lower alkyl group having 1 to 4 carbon atoms include methyl group, ethyl group, propyl group, isopropyl group, butyl group, isobutyl group, t-butyl group and the like, and methyl group, and ethyl group are preferred examples. Preferred examples of A¹ include a single bond, methylene group, methylmethylene group, ethylmethylene group, phenylmethylene group, ethylene group, methylethylene group, dimethylethylene group, ethylethylene group, phenylethylene group, ethenylene group, trimethylene group, methyltrimethylene group and the like (provided that when A² represents oxygen atom, sulfur atom, - S(O)-, -S(O)₂- or -N(R⁴)-, ethylene group, methylethylene group, dimethylethylene group, ethylethylene group, phenylethylene group, ethenylene group, trimethylene group, or methyltrimethylene group is preferred). Further, when A² represents a single bond, it is particularly preferred that A¹ is a single bond, or methylene group, methylmethylene group, or ethylene group (provided that when A² represents oxygen atom, sulfur atom, -S(O)-, -S(O)₂- or -N(R⁴)-, it is particularly preferred that A¹ is ethylene group).

Q in Rb is defined to be a residue of a partially unsaturated or completely unsaturated monocyclic or condensed bicyclic carbon ring or heterocyclic ring (q), and the heterocyclic ring (q) means a ring containing 1 to 4 the same or different ring-constituting heteroatoms selected from the group consisting of nitrogen atom, oxygen atom, and sulfur atom. The term "residue" means a monovalent group formed by eliminating hydrogen atom bonding to a ring-constituting atom. The residue of monocyclic carbon ring or heterocyclic ring is a partially unsaturated or completely unsaturated substituent having 5 to 7 atoms, and examples include, for example, phenyl group, thienyl group, furyl group, pyrrolyl group, pyridyl group, oxazolyl group, isoxazolyl group, thiazolyl group, isothiazolyl group, imidazolyl group, pyrazolyl group, oxadiazolyl group, thiadiazolyl group, triazolyl group, tetrazolyl group, and the like. Among them, phenyl group, thienyl group, furyl group, pyridyl group, and oxazolyl group are preferred examples, and phenyl group, thienyl group, and furyl group are particularly preferred examples.
The condensed bicyclic carbon ring or heterocyclic ring is a partially unsaturated or completely unsaturated ring having 8 to 11 atoms, and examples of residue thereof include, for example, naphthyl group, tetrahydronaphthyl group, indanyl group, indenyl group, quinolyl group, isoquinolyl group, indolyl group, benzofuryl group, benzothienyl group, benzimidazolyl group, benzoxazolyl group, benzothiazolyl group, indazolyl group, 4H-chromenyl group, dihydrobenzodioxyl group, benzoisoxazolyl group, pyrrolopyridinyl group, pyrazolopyridinyl group, triazolopyridinyl group, thienopyridinyl group, thienopyrazolyl group, 1,3-dihydrobenzimidazole group, dihydro-3H-benzoxazole group, dihydro-3H-benzothiazole group, and the like. Among them, naphthyl group, tetrahydronaphthyl group, indanyl group, indolyl group, dihydrobenzodioxyl group, and the like are preferred examples, and indanyl group is one of particularly preferred examples.

Q binds to A² at an arbitrary position on the ring. Preferred examples of Q with indication of bonding position include phenyl group, 2- or 3-thienyl group, 2- or 3-furyl group, 2-, 3- or 4-pyridyl group, 2-, 4- or 5-oxazolyl group, 1- or 2-naphthyl group, 1-, 2-, 5- or 6-tetrahydronaphthyl group, indan-1-yl group, indan-2-yl group, indan-4-yl group, indan-5-yl group, 1-, 2-, 3-, 4-, 5-, 6-, or 7-indolyl group, 2-, 5- or 6-dihydrobenzodioxyl group, and the like. Among them, phenyl group, 2- or 3-thienyl group, 2- or 3-furyl group, indan-2-yl group and the like are particularly preferred.
In Rb, R² and R³ are defined to be substituents of Q, and independently represent hydrogen atom, a linear or branched saturated alkyl group having 1 to 4 carbon atoms, oxo group, thioxo group, fluorine atom, chlorine atom, bromine atom, trifluoromethyl group, -OR⁵, -N(R⁶)(R^{6'}), -NHCOR⁷, -NHSO₂R⁸, or -A⁶-Qa, or bind to each other to represent methylenedioxy group. Among them, hydrogen atom, fluorine atom, chlorine atom, trifluoromethyl group and the like are particularly preferred examples. Examples of the linear or branched saturated alkyl group having 1 to 4 carbon atoms include methyl group, ethyl group, propyl group, isopropyl group, butyl group, isobutyl group, t-butyl group and the like, and methyl group is a particularly preferred example.

R⁶ in the substituent N(R⁶)(R^{6'}) represents hydrogen atom or a lower alkyl group having 1 to 4 carbon atoms. R^{6'} has the same meaning as that of R⁶, or binds to R⁶ to form a 3- to 6-membered ring together with the nitrogen atom to which they bind to form a saturated nitrogen-containing cycloalkyl group, or morpholino group. Examples of the compounds where "R^{6'} binds to R⁶ to form a 3- to 6-membered ring together with the nitrogen atom to which they bind to form a saturated nitrogen-containing cycloalkyl group, or morpholino group" include those wherein pyrrolidino group, piperidino group, morpholino group, or the like is formed. Therefore, specific examples of -N(R⁶)(R^{6'}) include amino group, N-methylamino group, N-ethylamino group, N-propylamino group, N-isopropylamino group, N,N-dimethylamino group, N,N-diethylamino group, piperidino group, pyrrolidino group, morpholino group, and the like. N,N-Dimethylamino group, piperidino group, morpholino group, and the like are preferred examples, and N,N-dimethylamino group is a particularly preferred example.
R⁵ and R⁷ are defined to independently represent hydrogen atom, a lower alkyl group having 1 to 4 carbon atoms, or a -A⁶-Qa group. As for R⁵, hydrogen atom is a particularly preferred example. Examples of the lower alkyl group having 1 to 4 carbon atoms include methyl group, ethyl group, propyl group, isopropyl group, butyl group, isobutyl group, t-butyl group, and the like, and among them, methyl group is a preferred example.

A⁶ in the substituent -A⁶-Qa has the same meaning as that of that defined above. Qa is defined to be a partially unsaturated or completely unsaturated monocyclic or condensed bicyclic carbon ring or heterocyclic ring (qa), and the heterocyclic ring (qa) means a substituent containing 1 to 4 the same or different ring-constituting heteroatoms selected from the group consisting of nitrogen atom, oxygen atom, and sulfur atom. The monocyclic carbon ring or heterocyclic ring is a partially unsaturated or completely unsaturated ring having 5 to 7 atoms, and examples of residue thereof include, for example, phenyl group, thienyl group, furyl group, pyrrolyl group, pyridyl group, oxazolyl group, isoxazolyl group, thiazolyl group, isothiazolyl group, imidazolyl group, pyrazolyl group, oxadiazolyl group, thiadiazolyl group, triazolyl group, tetrazolyl group, and the like. The condensed bicyclic carbon ring or heterocyclic ring is a partially unsaturated or completely unsaturated ring having 8 to 11 atoms, and examples of residue thereof include, for example, naphthyl group, indanyl group, indenyl group, quinolyl group, isoquinolyl group, indolyl group, benzofuryl group, benzothienyl group, benzimidazolyl group, benzoxazolyl group, benzothiazolyl group, indazolyl group, and the like.
Qa binds to A⁶ at an arbitrary position on the ring. Further, Qa may be substituted with two or more of the same or different T¹. T¹ has the same meaning as that defined above.

Specific examples of -A⁶-Qa include phenyl group, methylphenyl group, chlorophenyl group, benzyl group, methylbenzyl group, chlorobenzyl group, dichlorobenzyl group, fluorobenzyl group, trifluoromethylbenzyl group, nitrobenzyl group, methoxyphenyl group, N-methylaminobenzyl group, N,N-dimethylaminobenzyl group, furyl group, thienyl group, pyrrolyl group, pyridyl group, oxazolyl group, isoxazolyl group, thiazolyl group, isothiazolyl group, imidazolyl group, pyrazolyl group, oxadiazolyl group, thiadiazolyl group, triazolyl group, tetrazolyl group, naphthyl group, indanyl group, indenyl group, quinolyl group, isoquinolyl group, indolyl group, benzofuryl group, benzothienyl group, benzimidazolyl group, benzoxazolyl group, benzothiazolyl group, indazolyl group, and the like.
R⁸ each defined to be a lower alkyl group having 1 to 4 carbon atoms, and examples of the lower alkyl group having 1 to 4 carbon atoms include methyl group, ethyl group, propyl group, isopropyl group, butyl group, isobutyl group, t-butyl group, and the like.
Preferred examples of R² and R³ include hydrogen atom, methyl group, fluorine atom, chlorine atom, trifluoromethyl group, hydroxyl group, methoxy group, N,N-dimethylamino group, piperidino group, morpholino group, acetylamino group, and methylsulfonylamino group, and hydrogen atom, methyl group, fluorine atom, chlorine atom, trifluoromethyl group, hydroxyl group, methoxy group, and dimethylamino group are particularly preferred.

Particularly preferred examples of Rb include phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, 2-(N-ethyl-N-phenylamino)ethyl group and the like.

Symbol p in Rc is defined to be an integer of 2 to 4, and specifically, the alkylene represents ethylene when p is 2, trimethylene when p is 3, and tetramethylene when p is 4.
A⁴ represents a single bond, methylene, or ethylene.
Rd represents hydrogen atom, an alkyl group having 1 to 8 carbon atoms, or a group Qa.
Specific examples of the substituent -A⁴-Rd include hydrogen atom, methyl group, ethyl group, propyl group, isopropyl group, butyl group, isobutyl group, pentyl group, isoamyl group, cyclopropyl group, cyclopropylmethyl group, 2-(cyclopropyl)ethyl group, cyclopentyl group, cyclopentylmethyl group, 2-(cyclopentyl)ethyl group, cyclohexyl group, cyclohexylmethyl group, 2-(cyclohexyl)ethyl group, phenyl group, 4-methylphenyl group, 4-chlorophenyl group, 4-fluorophenyl group, benzyl group, 4-chlorophenylmethyl group, 4-fluorophenylmethyl group, 2-(4-chlorophenyl)ethyl group, 2-(4-fluorophenyl)ethyl group, pyridin-2-yl group, pyridin-3-yl group, pyridin-4-yl group, (pyridin-2-yl)methyl group, (pyridin-3-yl)methyl group, (pyridin-4-yl)methyl group and the like.
A⁵ represents -C(O)-, -C(S)-, or -S(O)₂-.
Re represents an alkyl group having 1 to 8 carbon atoms, a -A⁶-Qa group, a - (CH₂)ᵢR¹⁴ group, a -OR²⁸ group, a -SR²⁸ group, or a -N(R²⁹)(R³⁰) group. The groups Qa and -A⁶-Qa have the same meanings as mentioned above.

The alkyl group having 1 to 8 carbon atoms represents such a linear or branched saturated alkyl group, linear or branched alkyl group partially containing unsaturated bond, or alkyl group which may contain a cycloalkyl group having 3 to 7 carbon atoms, and examples include, for example, methyl group, ethyl group, propyl group, isopropyl group, butyl group, isobutyl group, t-butyl group, pentyl group, isopentyl group, 2-methylbutyl group, 2,2-dimethylpropyl group, hexyl group, 4-methylpentyl group, 2,3-dimethylbutyl group, 2-ethylbutyl group, heptyl group, octyl group, cyclopropyl group, cyclobutyl group, cyclopentyl group, cyclohexyl group, cycloheptyl group, cyclopropylmethyl group, cyclobutylmethyl group, cyclopentylmethyl group, cyclohexylmethyl group, cycloheptylmethyl group, 2-cyclopentylethyl group, 2-cyclohexylethyl group, 2-methylcyclopentyl group, 3-methylcyclopentyl group, 3,4-dimethylcyclopentyl group, 4-methylcyclohexyl group, 4,4-dimethylcyclohexyl group, 4-ethylcyclohexyl group, 4-methylcyclohexylmethyl group and the like.
Symbol i in the substituent -(CH₂)_{¡}R¹⁴ represents an integer of 1 to 3, and R¹⁴ represents hydroxyl group, an alkoxy group having 1 to 4 carbon atoms, carboxyl group, or an N,N-dialkylcarbamoyl group having 1 to 4 carbon atoms. Examples of the alkoxy group having 1 to 4 carbon atoms include methoxy group, ethoxy group, propyloxy group, isopropyloxy group, butoxy group, isobutyloxy group, t-butyloxy group, and the like. Examples of the N,N-dialkylcarbamoyl group having 1 to 4 carbon atoms include N,N-dimethylcarbamoyl group, N,N-diethylcarbamoyl group, and the like.
R²⁸ in the substituent -OR²⁸ or -SR²⁸ represents an alkyl group having 1 to 8 carbon atoms, or -A⁶-Qa, and these have the same meanings as defined above.

R²⁹ in the substituent -N(R²⁹)(R³⁰) represents an alkyl group having 1 to 8 carbon atoms, an alkoxycarbonyl group having 1 to 4 carbon atoms, or -A⁶-Qa.
Among them, the alkyl group having 1 to 8 carbon atoms and -A⁶-Qa have the same meanings as those defined above. Examples of the alkoxycarbonyl group having 1 to 4 carbon atoms include methyloxycarbonyl group, ethyloxycarbonyl group, propyloxycarbonyl group, isopropyloxycarbonyl group, butyloxycarbonyl group, isobutyloxycarbonyl group, t-butyloxycarbonyl group, and the like. R³⁰ represents hydrogen atom or a lower alkyl group having 1 to 4 carbon atoms, or binds to R²⁹ to form a 3- to 6-membered ring together with the nitrogen atom to which they bind to form a saturated nitrogen-containing cycloalkyl group, or morpholino group. The lower alkyl group having 1 to 4 carbon atoms has the same meaning as that defined above. Examples of the compound where "R³⁰ binds to R²⁹ to form a 3- to 6-membered ring together with the nitrogen atom to which they bind to form a saturated nitrogen-containing cycloalkyl group, or morpholino group" include, for example, a compound wherein a cyclic aminoalkyl group containing nitrogen atom such as pyrrolidino group, piperazino group, and morpholino group is formed.

Specific examples of the substituent -A⁵-Re include acetyl group, thioacetyl group, methanesulfonyl group, propionyl group, ethylthiocarbonyl group, butyryl group, propylthiocarbonyl group, isobutyryl group, isopropylthiocarbonyl group, isopropylsulfonyl group, valeryl group, butylthiocarbonyl group, isovaleryl group, isobutylthiocarbonyl group, pivaloyl group, t-butylthiocarbonyl group, cyclopropylcarbonyl group, cyclopropylthiocarbonyl group, cyclopentylcarbonyl group, cyclopentylthiocarbonyl group, cyclohexylcarbonyl group, cyclohexylthiocarbonyl group, cyclopentylmethylcarbonyl group, cyclopentylmethylthiocarbonyl group, cyclohexylmethylcarbonyl group, cyclohexylmethylthiocarbonyl group, benzoyl group, thiobenzoyl group, phenylsulfonyl group, 4-methylphenylcarbonyl group, 4-methylphenylthiocarbonyl group, 4-methylphenylsulfonyl group, 4-chlorophenylcarbonyl group, 4-chlorophenylthiocarbonyl group, 4-fluorophenylcarbonyl group, 4-fluorophenylthiocarbonyl group, phenylmethylcarbonyl group, 4-methylphenylmethylcarbonyl group, 4-chlorophenylmethylcarbonyl group, 4-fluorophenylmethylcarbonyl group, (pyridin-2-yl)carbonyl group, (pyridin-2-yl)thiocarbonyl group, (pyridin-3-yl)carbonyl group, (pyridin-4-yl)carbonyl group, (furan-2-yl)carbonyl group, (thiophen-2-yl)carbonyl group, methyloxycarbonyl group, methylsulfanylcarbonyl group, methyloxythiocarbonyl group, methyloxycarbonylaminocarbonyl group, carbamoyl group, N-methylcarbamoyl group, N-methylthiocarbamoyl group, N,N-dimethylcarbamoyl group, N,N-dimethylthiocarbamoyl group, N,N-dimethylsulfamoyl group, ethyloxycarbonyl group, ethyloxycarbonylaminocarbonyl group, N-ethylcarbamoyl group, N-ethylthiocarbamoyl group, N,N-diethylcarbamoyl group, N,N-diethylthiocarbamoyl group, N,N-diethylsulfamoyl group, propyloxycarbonyl group, N-propylcarbamoyl group, N-propylthiocarbamoyl group, isopropyloxycarbonyl group, N-isopropylcarbamoyl group, N-isopropylthiocarbamoyl group, butyloxycarbonyl group, N-butylcarbamoyl group, N-butylthiocarbamoyl group, isobutyloxycarbonyl group, N-isobutylcarbamoyl group, N-isobutylthiocarbamoyl group, t-butyloxycarbonyl group, N-t-butylcarbamoyl group, N-t-butylthiocarbamoyl group, cyclopropyloxycarbonyl group, N-cyclopropylcarbamoyl group, N-cyclopropylthiocarbamoyl group, cyclopentyloxycarbonyl group, N-cyclopentylcarbamoyl group, N-cyclopentylthiocarbamoyl group, cyclohexyloxycarbonyl group, N-cyclohexylcarbamoyl group, N-cyclohexylthiocarbamoyl group, cyclopentylmethyloxycarbonyl group, cyclohexylmethyloxycarbonyl group, phenyloxycarbonyl group, N-phenylcarbamoyl group, N-phenylthiocarbamoyl group, 4-methylphenyloxycarbonyl group, N-(4-methylphenyl)carbamoyl group, N-(4-methylphenyl)thiocarbamoyl group, 4-chlorophenyloxycarbonyl group, N-(4-chlorophenyl)carbamoyl group, N-(4-chlorophenyl)thiocarbamoyl group, 4-fluorophenyloxycarbonyl group, N-(4-fluorophenyl)carbamoyl group, N-(4-fluorophenyl)thiocarbamoyl group, phenylmethyloxycarbonyl group, 4-methylphenylmethyloxycarbonyl group, 4-chlorophenylmethyloxycarbonyl group, 4-fluorophenylmethyloxycarbonyl group, N-(pyridin-2-yl)carbamoyl group, N-(pyridin-2-yl)thiocarbamoyl group, N-(pyridin-3-yl)carbamoyl group, N-(pyridin-3-yl)thiocarbamoyl group, N-(pyridin-4-yl)carbamoyl group, N-(pyridin-4-yl)thiocarbamoyl group, N-(furan-2-yl)carbamoyl group, N-(thiophen-2-yl)carbamoyl group, (pyrrolidino-1-yl)carbonyl group, (piperidino-1-yl)carbonyl group, (morpholino-4-yl)carbonyl group and the like.

Specific examples of Rc include 2-(N-isobutyryl-N-methylamino)ethyl group, 2-(N-ethyl-N-isobutyrylamino)ethyl group, 2-(N-isobutyryl-N-propylamino)ethyl group, 2-(N-isobutyryl-N-isopropylamino)ethyl group, 2-(N-butyl-N-isobutyrylamino)ethyl group, 2-(N-isobutyl-N-isobutyrylamino)ethyl group, 2-(N-cyclopropyl-N-isobutyrylamino)ethyl group, 2-(N-cyclopentyl-N-isobutyrylamino)ethyl group, 2-(N-cyclopentylmethyl-N-isobutyrylamino)ethyl group, 2-(N-cyclohexyl-N-isobutyrylamino)ethyl group, 2-(N-cyclohexylmethyl-N-isobutyrylamino)ethyl group, 2-(N-isobutyryl-N-phenylamino)ethyl group, 2-[N-isobutyryl-N-(4-methylphenyl)amino]ethyl group, 2-[N-(4-chlorophenyl)-N-isobutyrylamino]ethyl group, 2-[N-(4-fluorophenyl)-N-isobutyrylamino]ethyl group, 2-(N-benzyl-N-isobutyrylamino)ethyl group, 2-[N-(4-chlorophenylmethyl)-N-isobutyrylamino]ethyl group, 2-[N-(4-fluorophenylmethyl)-N-isobutyrylamino]ethyl group, 2-[N-[2-(4-chlorophenyl)ethyl]-N-isobutyrylamino]ethyl group, 2-[N-[2-(4-fluorophenyl)ethyl]-N-isobutyrylamino]ethyl group, 2-(N-isobutylthiocarbonyl-N-methylamino)ethyl group, 2-(N-isobutylthiocarbonyl-N-isopropylamino)ethyl group, 2-(N-butyl-N-isobutylthiocarbonylamino)ethyl group, 2-(N-isobutyl-N-isobutylthiocarbonylamino)ethyl group, 2-(N-cyclopentyl-N-isobutylthiocarbonylamino)ethyl group, 2-(N-cyclopentylmethyl-N-isobutylthiocarbonylamino)ethyl group, 2-(N-isobutylthiocarbonyl-N-phenylamino)ethyl group, 2-(N-benzyl-N-isobutylthiocarbonylamino)ethyl group, 2-[N-(4-fluorophenylmethyl)-N-isobutylthiocarbonylamino]ethyl group, 2-(N-methyl-N-pivaloylamino)ethyl group, 2-(N-isopropyl-N-pivaloylamino)ethyl group, 2-(N-butyl-N-pivaloylamino)ethyl group, 2-(N-isobutyl-N-pivaloylamino)ethyl group, 2-(N-cyclohexyl-N-pivaloylamino)ethyl group, 2-(N-cyclohexylmethyl-N-pivaloylamino)ethyl group, 2-(N-phenyl-N-pivaloylamino)ethyl group, 2-(N-benzyl-N-pivaloylamino)ethyl group, 2-(N-cyclopentylcarbonyl-N-methylamino)ethyl group, 2-(N-butyl-N-cyclopentylcarbonylamino)ethyl group, 2-(N-cyclopentylcarbonyl-N-isobutylamino)ethyl group, 2-(N-cyclopentylcarbonyl-N-cyclopentylmethylamino)ethyl group, 2-(N-cyclopentylcarbonyl-N-phenylamino)ethyl group, 2-[N-cyclopentylcarbonyl-N-(4-fluorophenyl)amino]ethyl group, 2-(N-benzyl-N-cyclopentylcarbonylamino)ethyl group, 2-[N-cyclopentylcarbonyl-N-(4-fluorophenylmethyl)amino]ethyl group, 2-(N-methyl-N-phenylsulfonylamino)ethyl group, 2-(N-ethyl-N-phenylsulfonylamino)ethyl group, 2-(N-phenylsulfonyl-N-propylamino)ethyl group, 2-(N-isopropyl-N-phenylsulfonylamino)ethyl group, 2-(N-butyl-N-phenylsulfonylamino)ethyl group, 2-(N-isobutyl-N-phenylsulfonylamino)ethyl group, 2-(N-cyclopropyl-N-phenylsulfonylamino)ethyl group, 2-(N-cyclopentyl-N-phenylsulfonylamino)ethyl group, 2-(N-cyclopentylmethyl-N-phenylsulfonylamino)ethyl group, 2-(N-cyclohexyl-N-phenylsulfonylamino)ethyl group, 2-(N-cyclohexylmethyl-N-phenylsulfonylamino)ethyl group, 2-(N-phenyl-N-phenylsulfonylamino)ethyl group, 2-[N-(4-fluorophenyl)-N-phenylsulfonylamino]ethyl group, 2-(N-benzyl-N-phenylsulfonylamino)ethyl group, 2-[N-(N-butylcarbamoyl)-N-methylamino]ethyl group, 2-[N-butyl-N-(N-butylcarbamoyl)amino]ethyl group, 2-[N-(N-butylcarbamoyl)-N-isobutylamino]ethyl group, 2-[N-(N-butylcarbamoyl)-N-cyclopentylamino]ethyl group, 2-[N-(N-butylcarbamoyl)-N-cyclohexylmethylamino]ethyl group, 2-[N-(N-butylcarbamoyl)-N-phenylamino]ethyl group, 2-{N-(N-butylcarbamoyl)-N-(4-fluorophenyl)amino}ethyl group, 2-[N-benzyl-N-(N-butylcarbamoyl)amino]ethyl group, 2-{N-(N-butylcarbamoyl)-N-(4-fluorophenylmethyl)amino}ethyl group, 2-{N-(N-butylcarbamoyl)-N-[2-(4-fluorophenyl)ethyl]amino}ethyl group, 2-[N-(N-isopropylthiocarbamoyl)-N-methylamino]ethyl group, 2-[N-butyl-N-(N-isopropylthiocarbamoyl)amino]ethyl group, 2-[N-isobutyl-N-(N-isopropylthiocarbamoyl)amino]ethyl group, 2-[N-cyclopentyl-N-(N-isopropylthiocarbamoyl)amino]ethyl group, 2-[N-cyclohexylmethyl-N-(N-isopropylthiocarbamoyl)amino]ethyl group, 2-[N-(N-isopropylthiocarbamoyl)-N-phenylamino]ethyl group, 2-{N-(4-fluorophenyl)-N-(N-isopropylthiocarbamoyl)amino}ethyl group, 2-[N-benzyl-N-(N-isopropylthiocarbamoyl)amino]ethyl group, 2-(N-isobutyloxycarbonyl-N-methylamino)ethyl group, 2-(N-butyl-N-isobutyloxycarbonylamino)ethyl group, 2-(N-isobutyl-N-isobutyloxycarbonylamino)ethyl group, 2-(N-cyclopentyl-N-isobutyloxycarbonylamino)ethyl group, 2-(N-cyclohexylmethyl-N-isobutyloxycarbonylamino)ethyl group, 2-(N-isobutyloxycarbonyl-N-phenylamino)ethyl group, 2-[N-(4-fluorophenyl)-N-isobutyloxycarbonylamino]ethyl group, 2-(N-benzyl-N-isobutyloxycarbonylamino)ethyl group, 2-[N-(N-cyclopentylcarbamoyl)-N-methylamino]ethyl group, 2-[N-butyl-N-(N-cyclopentylcarbamoyl)amino]ethyl group, 2-[N-(N-cyclopentylcarbamoyl)-N-isobutylamino]ethyl group, 2-[N-cyclopentyl-N-(N-cyclopentylcarbamoyl)amino]ethyl group, 2-[N-cyclohexylmethyl-N-(N-cyclopentylcarbamoyl)amino]ethyl group, 2-[N-(N-cyclopentylcarbamoyl)-N-phenylamino]ethyl group, 2-[N-benzyl-N-(N-cyclopentylcarbamoyl)amino]ethyl group, 2-[N-(N-cyclohexylthiocarbamoyl)-N-methylamino]ethyl group, 2-[N-butyl-N-(N-cyclohexylthiocarbamoyl)amino]ethyl group, 2-[N-(N-cyclohexylthiocarbamoyl)-N-isobutylamino]ethyl group, 2-[N-(N-cyclohexylthiocarbamoyl)-N-cyclopentylamino]ethyl group, 2-[N-cyclohexylmethyl-N-(N-cyclohexylthiocarbamoyl)amino]ethyl group, 2-[N-(N-cyclohexylthiocarbamoyl)-N-phenylamino]ethyl group, 2-[N-benzyl-N-(N-cyclohexylthiocarbamoyl)amino]ethyl group, 2-(N-methyl-N-phenyloxycarbonylamino)ethyl group, 2-(N-butyl-N-phenyloxycarbonylamino)ethyl group, 2-(N-isobutyl-N-phenyloxycarbonylamino)ethyl group, 2-(N-cyclopentyl-N-phenyloxycarbonylamino)ethyl group, 2-(N-cyclohexylmethyl-N-phenyloxycarbonylamino)ethyl group, 2-(N-phenyl-N-phenyloxycarbonylamino)ethyl group, 2-(N-benzyl-N-phenyloxycarbonylamino)ethyl group, 2-[N-methyl-N-(N-phenylcarbamoyl)amino]ethyl group, 2-[N-butyl-N-(N-phenylcarbamoyl)amino]ethyl group, 2-[N-isobutyl-N-(N-phenylcarbamoyl)amino]ethyl group, 2-[N-cyclopentyl-N-(N-phenylcarbamoyl)amino]ethyl group, 2-[N-cyclohexylmethyl-N-(N-phenylcarbamoyl)amino]ethyl group, 2-[N-phenyl-N-(N-phenylcarbamoyl)amino]ethyl group, 2-[N-benzyl-N-(N-phenylcarbamoyl)amino]ethyl group and the like.

Particularly preferred specific examples of Rx include propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, 2-(N-ethyl-N-phenylamino)ethyl group and the like.

When Rs in the aforementioned formula (I) represents -N(Ry)(Rz), Rz is defined to have the same meaning as that of Rx, or represent methyl group, ethyl group, or a -A⁵-Re group. -A⁵-Re has the same meaning as that defined above.
Preferred examples of Rz are similar to the preferred examples of Rx, or methyl group and ethyl group when Ry represents a substituent other than hydrogen atom, and particularly preferred examples are similar to the particularly preferred examples of Rx, or methyl group and ethyl group when Ry represents a substituent other than hydrogen atom. Particularly preferred examples of Rz other than methyl group and ethyl group include propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, 2-(N-ethyl-N-phenylamino)ethyl group and the like.

Ry represents hydrogen atom, an alkyl group having 1 to 8 carbon atoms, or - A⁶-Qp. Specific examples include methyl group, ethyl group, propyl group, isopropyl group, butyl group, isobutyl group, t-butyl group, pentyl group, isopentyl group, 2-methylbutyl group, 2,2-dimethylpropyl group, hexyl group, 4-methylpentyl group, 2,3-dimethylbutyl group, 2-ethylbutyl group, heptyl group, octyl group, cyclopropyl group, cyclobutyl group, cyclopentyl group, cyclohexyl group, cycloheptyl group, cyclopropylmethyl group, cyclobutylmethyl group, cyclopentylmethyl group, cyclohexylmethyl group, cycloheptylmethyl group, 2-cyclopentylethyl group, 2-cyclohexylethyl group, 2-methylcyclopentyl group, 3-methylcyclopentyl group, 3,4-dimethylcyclopentyl group, 4-methylcyclohexyl group, 4,4-dimethylcyclohexyl group, 4-ethylcyclohexyl group, 4-methylcyclohexylmethyl group and the like. -A⁶-Qp has the same meaning as that defined above.
Preferred examples of Ry include hydrogen atom, methyl group, ethyl group, propyl group, isopropyl group, butyl group, isobutyl group, pentyl group, isopentyl group and the like, and particularly preferred examples include hydrogen atom, methyl group, ethyl group, propyl group, isopropyl group, butyl group, isobutyl group and the like.

Ry may also bind to Rz to form, together with the nitrogen atom, a saturated or unsaturated cyclic substituent (qy) having 3 to 7 atoms, the cyclic substituent (qy) may contain one of nitrogen atom, oxygen atom, or sulfur atom besides the nitrogen atom, and the cyclic substituent (qy) may be substituted with one of Rf or two of the same or different Rf. Preferred specific examples of the cyclic substituent (qy) include a nitrogen atom-containing cyclic substituent such as pyrrolidino group, piperidino group, homopiperidino group, morpholino group, piperazino group, homopiperazino group, pyrrol-1-yl group, imidazol-1-yl group, pyrazol-1-yl group, and thiomorpholino group, and pyrrolidino group, piperidino group, piperazino group, morpholino group and the like are particularly preferred examples. Rf represents an alkyl group having 1 to 8 carbon atoms, or -A⁶-Qp, and the substituent -A⁶-Qp has the same meaning as that defined above. Preferred examples of Rf include an alkyl group having 1 to 8 carbon atoms, phenyl group, and phenylmethyl group (provided that the benzene ring of the phenyl group and the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group), and particularly preferred examples include methyl group, ethyl group, propyl group, isopropyl group, butyl group, isobutyl group, cyclopentyl group, cyclohexyl group, cyclopentylmethyl group, cyclohexylmethyl group, phenyl group, and phenylmethyl group (provided that the benzene ring of the phenyl group and the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group).

Particularly preferred specific examples of Rf include methyl group, ethyl group, propyl group, isopropyl group, butyl group, isobutyl group, cyclopentyl group, cyclohexyl group, cyclopentylmethyl group, cyclohexylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-methoxyphenylmethyl group, 3-methoxyphenylmethyl group, 4-methoxyphenylmethyl group and the like.

Further, when the cyclic substituent (qy) is piperazino group, or homopiperazino group, which comprises a saturated ring containing two of nitrogen atoms, it is preferred that a nitrogen atom not bound to the ring E is substituted with Rf. In such case, particularly preferred examples of Rf include -A⁶-Qp, and specific examples thereof include phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-methoxyphenylmethyl group, 3-methoxyphenylmethyl group, 4-methoxyphenylmethyl group and the like.

Preferred examples of the substituent -N(Ry)(Rz) include those satisfying the following conditions:
[Rz represents a substituent which is methyl group, ethyl group, propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group represents, or Rb mentioned above;
Q in Rb represents a group which is phenyl group, thienyl group, furyl group, pyridyl group, oxazolyl group, naphthyl group, tetrahydronaphthyl group, indanyl group, indolyl group, or dihydrobenzodioxyl group;
A² in Rb represents a single bond, oxygen atom, sulfur atom, -N(methyl)-, or - N(ethyl)-;
A¹ in Rb represents a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² represents oxygen atom, sulfur atom, -N(methyl)-, or -N(ethyl)-, it represents ethylene group, methylethylene group, dimethylethylene group, ethylethylene group, phenylethylene group, ethenylene group, trimethylene group, or methyltrimethylene group);
R² and R³ independently represent hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group; and
Ry represents hydrogen atom, methyl group, ethyl group, propyl group, isopropyl group, butyl group, isobutyl group, pentyl group, or isopentyl group (provided that when Rz represents methyl group, or ethyl group, Ry represents a substituent other than hydrogen atom), or may bind to Rz to form, together with the nitrogen atom, a cyclic substituent (qy) selected from pyrrolidino group, piperidino group, homopiperidino group, morpholino group, piperazino group, homopiperazino group, pyrrol-1-yl group, imidazol-1-yl group, pyrazol-1-yl group, and thiomorpholino group, and the cyclic substituent (qy) may be substituted with one of alkyl group having 1 to 8 carbon atoms or two of the same or different alkyl groups having 1 to 8 carbon atoms, phenyl group, or phenylmethyl group (provided that the benzene ring
of the phenyl group and the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group)].

Further, particularly preferred examples of the substituent -N(Ry)(Rz) include those satisfying the following conditions:
[Rz represents a substituent which is methyl group, ethyl group, propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, or cycloheptylmethyl group, or Rb mentioned above;
Q in Rb represents phenyl group, 2-thienyl group, 3-thienyl group, 2-furyl group, 3-furyl group, or indan-2-yl group;
A² in Rb represents a single bond, oxygen atom, sulfur atom, -N(methyl)-, or - N(ethyl)-;
A¹ in Rb represents a single bond, or methylene group, methylmethylene group, or represents ethylene group when A² represents a single bond, or A¹ in Rb represents ethylene group when A² represents oxygen atom, sulfur atom, -N(methyl)-, or -N(ethyl)-;
R² and R³ independently represent hydrogen atom, methyl group, fluorine atom, chlorine atom, trifluoromethyl group, hydroxyl group, methoxy group, or dimethylamino group; and
Ry represents hydrogen atom, methyl group, ethyl group, propyl group, isopropyl group, butyl group, isobutyl group, pentyl group, or isopentyl group (provided that when Rz represents methyl group, or ethyl group, Ry represents a substituent other than hydrogen atom), or may bind to Rz to form, together with the nitrogen atom, a cyclic substituent (qy) selected from pyrrolidino group, piperidino group, morpholino group, and piperazino group, and the cyclic substituent (qy) may be substituted with one substituent or two of the same or different substituent selected from methyl group, ethyl group, propyl group, isopropyl group, butyl group, isobutyl group, cyclopentyl group, cyclohexyl group, cyclopentylmethyl group, cyclohexylmethyl group, phenyl group, and phenylmethyl group (provided that the benzene ring of the phenyl group and the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group)].

Particularly preferred specific examples of the substituent -N(Ry)(Rz) include N,N-dimethylamino group, N-ethyl-N-methylamino group, N,N-diethylamino group, N-methyl-N-propylamino group, N-ethyl-N-propylamino group, N-isopropyl-N-methylamino group, N-ethyl-N-isopropylamino group, N-butylamino group, N-butyl-N-methylamino group, N-butyl-N-ethylamino group, N-isobutylamino group, N-isobutyl-N-methylamino group, N-ethyl-N-isobutylamino group, N-isopentylamino group, N-isopentyl-N-methylamino group, N-ethyl-N-isopentylamino group, N-(2-ethylbutyl)amino group, N-(2-ethylbutyl)-N-methylamino group, N-cyclopentylamino group, N-cyclopentyl-N-methylamino group, N-cyclohexylamino group, N-cyclohexyl-N-methylamino group, N-cycloheptylamino group, N-(cyclopentylmethyl)amino group, N-(cyclopentylmethyl)-N-methylamino group, N-(cyclohexylmethyl)amino group, N-(cyclohexylmethyl)-N-methylamino group, N-phenylamino group, N-(2-methylphenyl)amino group, N-(4-methylphenyl)amino group, N-(2-fluorophenyl)amino group, N-(3-fluorophenyl)amino group, N-(4-fluorophenyl)amino group, N-(2-chlorophenyl)amino group, N-(3-chlorophenyl)amino group, N-(4-chlorophenyl)amino group, N-(indan-2-yl)amino group, N-(1-phenylethyl)amino group, N-[1-(2-fluorophenyl)ethyl]amino group, N-[1-(3-fluorophenyl)ethyl]amino group, N-[1-(4-fluorophenyl)ethyl]amino group, N-[1-(2-chlorophenyl)ethyl]amino group, N-[1-(3-chlorophenyl)ethyl]amino group, N-[1-(4-chlorophenyl)ethyl]amino group, N-benzylamino group, N-benzyl-N-methylamino group, N-benzyl-N-ethylamino group, N-(2-methylphenylmethyl)amino group, N-methyl-N-(2-methylphenylmethyl)amino group, N-(3-methylphenylmethyl)amino group, N-methyl-N-(3-methylphenylmethyl)amino group, N-(4-methylphenylmethyl)amino group, N-methyl-N-(4-methylphenylmethyl)amino group, N-(2-fluorophenylmethyl)amino group, N-(2-fluorophenylmethyl)-N-methylamino group, N-(3-fluorophenylmethyl)amino group, N-(3-fluorophenylmethyl)-N-methylamino group, N-(4-fluorophenylmethyl)amino group, N-(4-fluorophenylmethyl)-N-methylamino group, N-(2-chlorophenylmethyl)amino group, N-(2-chlorophenylmethyl)-N-methylamino group, N-(3-chlorophenylmethyl)amino group, N-(3-chlorophenylmethyl)-N-methylamino group, N-(4-chlorophenylmethyl)amino group, N-(4-chlorophenylmethyl)-N-methylamino group, N-(2,3-difluorophenylmethyl)amino group, N-(2,3-difluorophenylmethyl)-N-methylamino group, N-(2,4-difluorophenylmethyl)amino group, N-(2,4-difluorophenylmethyl)-N-methylamino group, N-(2,5-difluorophenylmethyl)amino group, N-(2,5-difluorophenylmethyl)-N-methylamino group, N-(3,4-difluorophenylmethyl)amino group, N-(3,4-diffuorophenylmethyl)-N-methylamino group, N-(3,5-difluorophenylmethyl)amino group, N-(3,5-difluorophenylmethyl)-N-methylamino group, N-(2,3-dichlorophenylmethyl)amino group, N-(2,3-dichlorophenylmethyl)-N-methylamino group, N-(2,4-dichlorophenylmethyl)amino group, N-(2,4-dichlorophenylmethyl)-N-methylamino group, N-(2,5-dichlorophenylmethyl)amino group, N-(2,5-dichlorophenylmethyl)-N-methylamino group, N-(2,6-dichlorophenylmethyl)amino group, N-(2,6-dichlorophenylmethyl)-N-methylamino group, N-(3,4-dichlorophenylmethyl)amino group, N-(3,4-dichlorophenylmethyl)-N-methylamino group, N-(3,5-dichlorophenylmethyl)amino group, N-(3,5-dichlorophenylmethyl)-N-methylamino group, N-[2-(trifluoromethyl)phenylmethyl]amino group, N-methyl-N-[2-(trifluoromethyl)phenylmethyl]amino group, N-[3-(trifluoromethyl)phenylmethyl]amino group, N-methyl-N-[3-(triffuoromethyl)phenylmethyl]amino group, N-[4-(trifluoromethyl)phenylmethyl]amino group, N-methyl-N-[4-(triffuoromethyl)phenylmethyl]amino group, pyrrolidino group, 2-methylpyrrolidino group, 3-methylpyrrolidino group, 2,5-dimethylpyrrolidino group, 3,4-dimethylpyrrolidino group, piperidino group, 2-methylpiperidino group, 3-methylpiperidino group, 4-methylpiperidino group, 4-ethylpiperidino group, 4-propylpiperidino group, 4-isopropylpiperidino group, 4-butylpiperidino group, 4-isobutylpiperidino group, 4-cyclopentylpiperidino group, 4-cyclohexylpiperidino group, 4-(cyclopentylmethyl)piperidino group, 4-(cyclohexylmethyl)piperidino group, 4-phenylpiperidino group, 4-(2-methylphenyl)piperidino group, 4-(4-methylphenyl)piperidino group, 4-(2-fluorophenyl)piperidino group, 4-(3-fluorophenyl)piperidino group, 4-(4-fluorophenyl)piperidino group, 4-(2-chlorophenyl)piperidino group, 4-(3-chlorophenyl)piperidino group, 4-(4-chlorophenyl)piperidino group, 4-benzylpiperidino group, 4-(2-methylphenylmethyl)piperidino group, 4-(3-methylphenylmethyl)piperidino group, 4-methylphenylmethyl)piperidino group, 4-(2,3-dimethylphenylmethyl)piperidino group, 4-(3,5-dimethylphenylmethyl)piperidino group, 4-(2-fluorophenylmethyl)piperidino group, 4-(3-fluorophenylmethyl)piperidino group, 4-(4-fluorophenylmethyl)piperidino group, 4-(2-chlorophenylmethyl)piperidino group, 4-(3-chlorophenylmethyl)piperidino group, 4-(4-chlorophenylmethyl)piperidino group, 4-(2,3-difluorophenylmethyl)piperidino group, 4-(2,4-difluorophenylmethyl)piperidino group, 4-(2,5-difluorophenylmethyl)piperidino group, 4-(3,4-difluorophenylmethyl)piperidino group, 4-(2,3-dichlorophenylmethyl)piperidino group, 4-(2,4-dichlorophenylmethyl)piperidino group, 4-(2,5-dichlorophenylmethyl)piperidino group, 4-(2,6-dichlorophenylmethyl)piperidino group, 4-(3,4-dichlorophenylmethyl)piperidino group, 4-(3,5-dichlorophenylmethyl)piperidino group, 4-(3,6-dichlorophenylmethyl)piperidino group, 4-(2-methoxyphenylmethyl)piperidino group, 4-(3-methoxyphenylmethyl)piperidino group, 4-(4-methoxyphenylmethyl)piperidino group, 2,6-dimethylpiperidino group, 3,5-dimethylpiperidino group, 4,4-dimethylpiperidino group, homopiperidino group, morpholino group, 3,5-dimethylmorpholino group, 4-phenylpiperazino group, 4-(2-methylphenyl)piperazino group, 4-(4-methylphenyl)piperazino group, 4-(2-fluorophenyl)piperazino group, 4-(3-fluorophenyl)piperazino group, 4-(4-fluorophenyl)piperazino group, 4-(2-chlorophenyl)piperazino group, 4-(3-chlorophenyl)piperazino group, 4-(4-chlorophenyl)piperazino group, 4-benzylpiperazino group, 4-(2-methylphenylmethyl)piperazino group, 4-(3-methylphenylmethyl)piperazino group, 4-methylphenylmethyl)piperazino group, 4-(2,3-dimethylphenylmethyl)piperazino group, 4-(3,5-dimethylphenylmethyl)piperazino group, 4-(2-fluorophenylmethyl)piperazino group, 4-(3-fluorophenylmethyl)piperazino group, 4-(4-fluorophenylmethyl)piperazino group, 4-(2-chlorophenylmethyl)piperazino group, 4-(3-chlorophenylmethyl)piperazino group, 4-(4-chlorophenylmethyl)piperazino group, 4-(2,3-difluorophenylmethyl)piperazino group, 4-(2,4-difluorophenylmethyl)piperazino group, 4-(2,5-difluorophenylmethyl)piperazino group, 4-(3,4-difluorophenylmethyl)piperazino group, 4-(2,3-dichlorophenylmethyl)piperazino group, 4-(2,4-dichlorophenylmethyl)piperazino group, 4-(2,5-dichlorophenylmethyl)piperazino group, 4-(2,6-dichlorophenylmethyl)piperazino group, 4-(3,4-dichlorophenylmethyl)piperazino group, 4-(3,5-dichlorophenylmethyl)piperazino group, 4-(3,6-dichlorophenylmethyl)piperazino group, 4-(2-methoxyphenylmethyl)piperazino group, 4-(3-methoxyphenylmethyl)piperazino group, 4-(4-methoxyphenylmethyl)piperazino group and the like.

Preferred examples of Rs in the aforementioned formula (I) include those satisfying the following conditions:
[Rs represents -D-Rx or -N(Ry)(Rz);
D represents a single bond, oxygen atom, or sulfur atom;
Rx represents propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb mentioned above;
Q in Rb represents a group which is phenyl group, thienyl group, furyl group, pyridyl group, oxazolyl group, naphthyl group, tetrahydronaphthyl group, indanyl group, indolyl group, or dihydrobenzodioxyl group;
A² in Rb represents a single bond, oxygen atom, sulfur atom, -N(methyl)-, or - N(ethyl)-;
A¹ in Rb represents a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² represents oxygen atom, sulfur atom, -N(methyl)-, or -N(ethyl)-, A¹ represents ethylene group, methylethylene group, dimethylethylene group, ethylethylene group, phenylethylene group, ethenylene group, trimethylene group, or methyltrimethylene group);
R² and R³ independently represent hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group;
Rz has the same meaning as that of Rx, or represents methyl group, or ethyl group; and
Ry represents hydrogen atom, methyl group, ethyl group, propyl group, isopropyl group, butyl group, isobutyl group, pentyl group, or isopentyl group (provided that when Rz represents methyl group, or ethyl group, Ry represents a substituent other than hydrogen atom), or may bind to Rz to form, together with the nitrogen atom, a cyclic substituent (qy) selected from pyrrolidino group, piperidino group, homopiperidino group, morpholino group, piperazino group, homopiperazino group, pyrrol-1-yl group, imidazol-1-yl group, pyrazol-1-yl group, and thiomorpholino group, and the cyclic substituent (qy) may be substituted with one of alkyl group having 1 to 8 carbon atoms or two of the same or different alkyl groups having 1 to 8 carbon atoms, phenyl group, or phenylmethyl group (provided that the benzene ring of the phenyl group and the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group)].

Further, particularly preferred examples of Rs in the aforementioned formula (I) include those satisfying the following conditions:
[Rs represents -D-Rx or -N(Ry)(Rz);
D represents a single bond, or oxygen atom;
Rx represents a substituent which is propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, or cycloheptylmethyl group, or Rb mentioned above;
Q in Rb represents phenyl group, 2-thienyl group, 3-thienyl group, 2-furyl group, 3-furyl group, or indan-2-yl group;
A² in Rb represents a single bond, oxygen atom, sulfur atom, -N(methyl)-, or - N(ethyl)-;
A¹ in Rb represents a single bond, or methylene group, methylmethylene group, or represents ethylene group when A² represents a single bond, or represents ethylene group when A² represents oxygen atom, sulfur atom, -N(methyl)-, or - N(ethyl)-;
R² and R³ independently represent hydrogen atom, methyl group, fluorine atom, chlorine atom, trifluoromethyl group, hydroxyl group, methoxy group, or dimethylamino group;
Rz has the same meaning as that of Rx, or represents or methyl group, or ethyl group; and
Ry represents hydrogen atom, methyl group, ethyl group, propyl group, isopropyl group, butyl group, isobutyl group, pentyl group, or isopentyl group (provided that when Rz represents methyl group, or ethyl group, Ry represents a substituent other than hydrogen atom), or may bind to Rz to form, together with the nitrogen atom, a cyclic substituent (qy) selected from pyrrolidino group, piperidino group, morpholino group, and piperazino group, and cyclic substituent (qy) may be substituted with one of substituent or two of the same or different substituents selected from methyl group, ethyl group, propyl group, isopropyl group, butyl group, isobutyl group, cyclopentyl group, cyclohexyl group, cyclopentylmethyl group, cyclohexylmethyl group, phenyl group, and phenylmethyl group (provided that the benzene ring of the phenyl group and the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group)].

Particularly preferred other embodiments of Rs in the aforementioned formula (I) include those satisfying the following conditions:
[Rs represents -D-Rx or -N(Ry)(Rz);
when Rs represents -D-Rx, D represents a single bond, or oxygen atom;
Rx represents propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group; and
when Rs represents -N(Ry)(Rz), -N(Ry)(Rz) represents N,N-dimethylamino group, N-ethyl-N-methylamino group, N,N-diethylamino group, N-methyl-N-propylamino group, N-ethyl-N-propylamino group, N-isopropyl-N-methylamino group, N-ethyl-N-isopropylamino group, N-butylamino group, N-butyl-N-methylamino group, N-butyl-N-ethylamino group, N-isobutylamino group, N-isobutyl-N-methylamino group, N-ethyl-N-isobutylamino group, N-isopentylamino group, N-isopentyl-N-methylamino group, N-ethyl-N-isopentylamino group, N-(2-ethylbutyl)amino group, N-(2-ethylbutyl)-N-methylamino group, N-cyclopentylamino group, N-cyclopentyl-N-methylamino group, N-cyclohexylamino group, N-cyclohexyl-N-methylamino group, N-cycloheptylamino group, N-(cyclopentylmethyl)amino group, N-(cyclopentylmethyl)-N-methylamino group, N-(cyclohexylmethyl)amino group, N-(cyclohexylmethyl)-N-methylamino group, N-phenylamino group, N-(2-methylphenyl)amino group, N-(4-methylphenyl)amino group, N-(2-fluorophenyl)amino group, N-(3-fluorophenyl)amino group, N-(4-fluorophenyl)amino group, N-(2-chlorophenyl)amino group, N-(3-chlorophenyl)amino group, N-(4-chlorophenyl)amino group, N-(indan-2-yl)amino group, N-(1-phenylethyl)amino group, N-[1-(2-fluorophenyl)ethyl]amino group, N-[1-(3-fluorophenyl)ethyl]amino group, N-[1-(4-fluorophenyl)ethyl]amino group, N-[1-(2-chlorophenyl)ethyl]amino group, N-[1-(3-chlorophenyl)ethyl]amino group, N-[1-(4-chlorophenyl)ethyl]amino group, N-benzylamino group, N-benzyl-N-methylamino group, N-benzyl-N-ethylamino group, N-(2-methylphenylmethyl)amino group, N-methyl-N-(2-methylphenylmethyl)amino group, N-(3-methylphenylmethyl)amino group, N-methyl-N-(3-methylphenylmethyl)amino group, N-(4-methylphenylmethyl)amino group, N-methyl-N-(4-methylphenylmethyl)amino group, N-(2-fluorophenylmethyl)amino group, N-(2-fluorophenylmethyl)-N-methylamino group, N-(3-fluorophenylmethyl)amino group, N-(3-fluorophenylmethyl)-N-methylamino group, N-(4-fluorophenylmethyl)amino group, N-(4-fluorophenylmethyl)-N-methylamino group, N-(2-chlorophenylmethyl)amino group, N-(2-chlorophenylmethyl)-N-methylamino group, N-(3-chlorophenylmethyl)amino group, N-(3-chlorophenylmethyl)-N-methylamino group, N-(4-chlorophenylmethyl)amino group, N-(4-chlorophenylmethyl)-N-methylamino group, N-(2,3-difluorophenylmethyl)amino group, N-(2,3-difluorophenylmethyl)-N-methylamino group, N-(2,4-difluorophenylmethyl)amino group, N-(2,4-difluorophenylmethyl)-N-methylamino group, N-(2,5-difluorophenylmethyl)amino group, N-(2,5-difluorophenylmethyl)-N-methylamino group, N-(3,4-difluorophenylmethyl)amino group, N-(3,4-difluorophenylmethyl)-N-methylamino group, N-(3,5-difluorophenylmethyl)amino group, N-(3,5-difluorophenylmethyl)-N-methylamino group, N-(2,3-dichlorophenylmethyl)amino group, N-(2,3-dichlorophenylmethyl)-N-methylamino group, N-(2,4-dichlorophenylmethyl)amino group, N-(2,4-dichlorophenylmethyl)-N-methylamino group, N-(2,5-dichlorophenylmethyl)amino group, N-(2,5-dichlorophenylmethyl)-N-methylamino group, N-(2,6-dichlorophenylmethyl)amino group, N-(2,6-dichlorophenylmethyl)-N-methylamino group, N-(3,4-dichlorophenylmethyl)amino group, N-(3,4-dichlorophenylmethyl)-N-methylamino group, N-(3,5-dichlorophenylmethyl)amino group, N-(3,5-dichlorophenylmethyl)-N-methylamino group, N-[2-(trifluoromethyl)phenylmethyl]amino group, N-methyl-N-[2-(trifluoromethyl)phenylmethyl]amino group, N-[3-(trifluoromethyl)phenylmethyl]amino group, N-methyl-N-[3-(trifluoromethyl)phenylmethyl]amino group, N-[4-(trifluoromethyl)phenylmethyl]amino group, N-methyl-N-[4-(trifluoromethyl)phenylmethyl]amino group, pyrrolidino group, 2-methylpyrrolidino group, 3-methylpyrrolidino group, 2,5-dimethylpyrrolidino group, 3,4-dimethylpyrrolidino group, piperidino group, 2-methylpiperidino group, 3-methylpiperidino group, 4-methylpiperidino group, 4-ethylpiperidino group, 4-propylpiperidino group, 4-isopropylpiperidino group, 4-butylpiperidino group, 4-isobutylpiperidino group, 4-cyclopentylpiperidino group, 4-cyclohexylpiperidino group, 4-(cyclopentylmethyl)piperidino group, 4-(cyclohexylmethyl)piperidino group, 4-phenylpiperidino group, 4-(2-methylphenyl)piperidino group, 4-(4-methylphenyl)piperidino group, 4-(2-fluorophenyl)piperidino group, 4-(3-fluorophenyl)piperidino group, 4-(4-fluorophenyl)piperidino group, 4-(2-chlorophenyl)piperidino group, 4-(3-chlorophenyl)piperidino group, 4-(4-chlorophenyl)piperidino group, 4-benzylpiperidino group, 4-(2-methylphenylmethyl)piperidino group, 4-(3-methylphenylmethyl)piperidino group, 4-methylphenylmethyl)piperidino group, 4-(2,3-dimethylphenylmethyl)piperidino group, 4-(3,5-dimethylphenylmethyl)piperidino group, 4-(2-fluorophenylmethyl)piperidino group, 4-(3-fluorophenylmethyl)piperidino group, 4-(4-fluorophenylmethyl)piperidino group, 4-(2-chlorophenylmethyl)piperidino group, 4-(3-chlorophenylmethyl)piperidino group, 4-(4-chlorophenylmethyl)piperidino group, 4-(2,3-difluorophenylmethyl)piperidino group, 4-(2,4-difluorophenylmethyl)piperidino group, 4-(2,5-difluorophenylmethyl)piperidino group, 4-(3,4-difluorophenylmethyl)piperidino group, 4-(2,3-dichlorophenylmethyl)piperidino group, 4-(2,4-dichlorophenylmethyl)piperidino group, 4-(2,5-dichlorophenylmethyl)piperidino group, 4-(2,6-dichlorophenylmethyl)piperidino group, 4-(3,4-dichlorophenylmethyl)piperidino group, 4-(3,5-dichlorophenylmethyl)piperidino group, 4-(3,6-dichlorophenylmethyl)piperidino group, 4-(2-methoxyphenylmethyl)piperidino group, 4-(3-methoxyphenylmethyl)piperidino group, 4-(4-methoxyphenylmethyl)piperidino group, 2,6-dimethylpiperidino group, 3,5-dimethylpiperidino group, 4,4-dimethylpiperidino group, homopiperidino group, morpholino group, 3,5-dimethylmorpholino group, 4-phenylpiperazino group, 4-(2-methylphenyl)piperazino group, 4-(4-methylphenyl)piperazino group, 4-(2-fluorophenyl)piperazino group, 4-(3-fluorophenyl)piperazino group, 4-(4-fluorophenyl)piperazino group, 4-(2-chlorophenyl)piperazino group, 4-(3-chlorophenyl)piperazino group, 4-(4-chlorophenyl)piperazino group, 4-benzylpiperazino group, 4-(2-methylphenylmethyl)piperazino group, 4-(3-methylphenylmethyl)piperazino group, 4-methylphenylmethyl)piperazino group, 4-(2,3-dimethylphenylmethyl)piperazino group, 4-(3,5-dimethylphenylmethyl)piperazino group, 4-(2-fluorophenylmethyl)piperazino group, 4-(3-fluorophenylmethyl)piperazino group, 4-(4-fluorophenylmethyl)piperazino group, 4-(2-chlorophenylmethyl)piperazino group, 4-(3-chlorophenylmethyl)piperazino group, 4-(4-chlorophenylmethyl)piperazino group, 4-(2,3-difluorophenylmethyl)piperazino group, 4-(2,4-difluorophenylmethyl)piperazino group, 4-(2,5-difluorophenylmethyl)piperazino group, 4-(3,4-difluorophenylmethyl)piperazino group, 4-(2,3-dichlorophenylmethyl)piperazino group, 4-(2,4-dichlorophenylmethyl)piperazino group, 4-(2,5-dichlorophenylmethyl)piperazino group, 4-(2,6-dichlorophenylmethyl)piperazino group, 4-(3,4-dichlorophenylmethyl)piperazino group, 4-(3,5-dichlorophenylmethyl)piperazino group, 4-(3,6-dichlorophenylmethyl)piperazino group, 4-(2-methoxyphenylmethyl)piperazino group, 4-(3-methoxyphenylmethyl)piperazino group, or 4-(4-methoxyphenylmethyl)piperazino group].

Further, particularly preferred other embodiments of Rs in the aforementioned formula (I) include those satisfying all the following conditions: Rs represents -D-Rx, D represents a single bond, Rx represents Rb, and A¹ and A² in Rb represent a single bond. Specifically, particularly preferred examples include those
wherein Rs represents phenyl group, 2-methylphenyl group, 3-methylphenyl group, 4-methylphenyl group, 2,3-dimethylphenyl group, 3,5-dimethylphenyl group, 2-methoxyphenyl group, 3-methoxyphenyl group, 4-methoxyphenyl group, 2-fluorophenyl group, 3-fluorophenyl group, 4-fluorophenyl group, 2-chlorophenyl group, 3-chlorophenyl group, 4-chlorophenyl group, 2,3-difluorophenyl group, 2,4-difluorophenyl group, 2,5-difluorophenyl group, 3,4-difluorophenyl group, 2,3-dichlorophenyl group, 2,4-dichlorophenyl group, 2,5-dichlorophenyl group, 2,6-dichlorophenyl group, 3,4-dichlorophenyl group, 3,5-dichlorophenyl group, 2-trifluoromethylphenyl group, 3-trifluoromethylphenyl group, 4-trifluoromethylphenyl group, 4-(N,N-dimethylamino)phenyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-methoxyindan-2-yl group, 5-methoxyindan-2-yl group, 4,7-dimethoxyindan-2-yl group, 5,6-dimethoxyindan-2-yl group, furan-2-yl group, furan-3-yl group, thiophen-2-yl group, thiophen-3-yl group or the like.

One of the substituents V¹ and V² on the ring (E) in the aforementioned formula (I) represents Zx, and the other represents AR. Specifically, when V¹ is Zx, V² is AR, and when V² is Zx, V¹ is AR. Those wherein V¹ is Zx, and V² is AR are preferred examples.
Zx is defined to be hydrogen atom, a linear or branched saturated alkyl group having 1 to 4 carbon atoms, fluorine atom, chlorine atom, bromine atom, nitro group, - OR⁹, or -N(Rn¹)(Rn²). Among them, preferred examples include hydrogen atom, fluorine atom, chlorine atom, bromine atom, nitro group and the like, and particularly preferred examples include hydrogen atom, fluorine atom, chlorine atom, bromine atom and the like.
Examples of the linear or branched saturated alkyl group having 1 to 4 carbon atoms as Zx include methyl group, ethyl group, propyl group, isopropyl group, butyl group, isobutyl group, t-butyl group and the like, and among them, methyl group is a particularly preferred example.
R⁹ represents hydrogen atom, a lower alkyl group having 1 to 4 carbon atoms, or -A⁶-Qp. Among them, a particularly preferred example is hydrogen atom. Examples of the lower alkyl group having 1 to 4 carbon atoms include methyl group, ethyl group, propyl group, isopropyl group, butyl group, isobutyl group, t-butyl group and the like, and methyl group is a particularly preferred example. A⁶ and Qp in - A⁶-Qp have the same meanings as defined above. Preferred examples of -OR⁹ include hydroxyl group, methoxy group and the like, and hydroxyl group is a particularly preferred example.

Rn¹ represents hydrogen atom or a linear or branched saturated alkyl group having 1 to 4 carbon atoms, and hydrogen atom is a particularly preferred example. Examples of the linear or branched saturated alkyl group having 1 to 4 carbon atoms include methyl group, ethyl group, propyl group, isopropyl group, butyl group, isobutyl group, t-butyl group and the like, among them, methyl group, ethyl group, propyl group, isopropyl group and the like are preferred examples, and methyl group is a particularly preferred example.
Rn² has the same meaning as that of Rn¹, or represents a -COR²³ group, or a - SO₂R²⁴ group, or binds to Rn¹ to form a 3- to 6-membered ring together with the nitrogen atom to which they bind to form a saturated nitrogen-containing cycloalkyl group, or morpholino group.
R²³ represents hydrogen atom, a lower alkyl group having 1 to 4 carbon atoms, a lower alkoxyl group having 1 to 4 carbon atoms, -O-A⁶-Qp, or -N(R²⁵)(R²⁶). R²⁵ represents hydrogen atom, or a linear or branched saturated alkyl group having 1 to 4 carbon atoms. R²⁶ has the same meaning as that of R²⁵, or binds to R²⁵ form a 3- to 6-membered ring together with the nitrogen atom to which they bind to represent a saturated nitrogen-containing cycloalkyl group, or morpholino group. Examples of the compounds where "R²⁶ binds to R²⁵ to form a 3- to 6-membered ring together with the nitrogen atom to which they bind to form a saturated nitrogen-containing cycloalkyl group, or morpholino group" include, for example, a compound where a nitrogen atom-containing cyclic aminoalkyl group such as pyrrolidino group, piperazino group, and morpholino group is formed.
Specific examples of -COR²³ include formyl group, acetyl group, t-butyloxycarbonyl group, phenyloxycarbonyl group, benzyloxycarbonyl group, carbamoyl group, N-methylcarbamoyl group, N,N-dimethylcarbamoyl group, piperidine-1-carbonyl group, morpholine-4-carbonyl group and the like.

R²⁴ represents a lower alkyl group having 1 to 4 carbon atoms, amino group, or a mono- or dialkylamino group having 1 to 4 carbon atoms. Specific examples of - SO₂R²⁴ include mesyl group, sulfamoyl group, N-methylsulfamoyl group, N,N-dimethylsulfamoyl group and the like.
As Rn², hydrogen atom, methyl group and the like are preferred examples, and hydrogen atom is a particularly preferred example.
Specific examples of -N(Rn¹)(Rn²) include amino group, N-methylamino group, N-ethylamino group, N-propylamino group, N-isopropylamino group, N,N-dimethylamino group, N,N-diethylamino group, piperidino group, pyrrolidino group, morpholino group, formylamino group, acetylamino group, t-butyloxycarbonylamino group, phenyloxycarbonylamino group, benzyloxycarbonylamino group, carbamoylamino group, N-methylcarbamoylamino group, N,N-dimethylcarbamoylamino group, piperidine-1-carbonylamino group, morpholine-4-carbonylamino group, mesylamino group, sulfamoylamino group, N-methylsulfamoylamino group, N,N-dimethylsulfamoylamino group and the like, among them, preferred examples include amino group, N-methylamino group, N-ethylamino group, N-propylamino group, N-isopropylamino group, N,N-dimethylamino group and the like, and amino group, N-methylamino group and the like are particularly preferred examples.
Preferred examples of Zx include hydrogen atom, fluorine atom, chlorine atom, bromine atom, nitro group, methyl group, hydroxyl group, methoxy group, amino group, N-methylamino group, N-ethylamino group, N-propylamino group, N-isopropylamino group, N,N-dimethylamino group and the like, and particularly preferred examples include hydrogen atom, fluorine atom, chlorine atom, bromine atom, methyl group, hydroxyl group, amino group, N-methylamino group and the like. The most preferred example is hydrogen atom or amino group.

AR in the aforementioned formula (I) is defined to be a residue of a partially unsaturated or completely unsaturated condensed bicyclic carbon ring or heterocyclic ring (ar). Further, AR may be substituted with one of Xa or two or more of the same or different Xa. The heterocyclic ring (ar) means a ring containing 1 to 4 the same or different ring-constituting heteroatoms selected from the group consisting of nitrogen atom, oxygen atom, and sulfur atom.
The "condensed bicyclic carbon ring or heterocyclic ring" means a partially unsaturated or completely unsaturated ring having 8 to 11 atoms. Preferred examples include a partially unsaturated or completely unsaturated ring consisting of 8 atoms formed by fusion of 5-membered heterocyclic rings containing 1 or 2 ring-constituting heteroatoms selected from the group consisting of nitrogen, oxygen, and sulfur atoms, a partially unsaturated or completely unsaturated ring consisting of 9 atoms formed by fusion of a 5-membered heterocyclic ring containing 1 or 2 ring-constituting heteroatoms selected from the group consisting of nitrogen, oxygen, and sulfur atoms and a 6-membered carbon ring or a 6-membered heterocyclic ring containing 1 or 2 ring-constituting heteroatoms selected from the group consisting of nitrogen, oxygen, and sulfur atoms, and a partially unsaturated or completely unsaturated substituent consisting of 10 atoms formed by fusion of a 6-membered carbon ring or a 6-membered heterocyclic ring containing 1 or 2 ring-constituting heteroatoms selected from the group consisting of nitrogen, oxygen, and sulfur atoms and a 6-membered carbon ring or 6-membered heterocyclic ring containing 1 or 2 ring-constituting heteroatoms selected from the group consisting of nitrogen, oxygen, and sulfur atom. As the carbon ring constituting AR not containing a heteroatom, among the rings constituting AR, naphthalene ring is particularly preferred.
Further, as the heterocyclic ring (ar) containing a heteroatom, among the rings constituting AR, those containing 1 or 2 ring-constituting heteroatoms are preferred.

As for AR in the formula (I), specific examples of preferred ring constituting AR include naphthalene, benzofuran, benzo[b]thiophene, indole, benzothiazole, dihydro-3H-benzothiazole, quinoline, dihydro-1H-quinoline, benzo[d]isothiazole, 1H-indazole, benzo[c]isothiazole, 2H-indazole, imidazo[1,2-a]pyridine, 1H-pyrrolo[2,3-b]pyridine, isoquinoline, dihydro-2H-isoquinoline, cinnoline, quinazoline, quinoxaline, 1H-benzimidazole, benzoxazole, 1H-pyrrolo[3,2-b]pyridine, benzo[1,2,5]thiadiazole, 1H-benzotriazole, 1,3-dihydropyrrolo[2,3-b]pyridine, 1,3-dihydrobenzimidazole, dihydro-3H-benzoxazole, phthalazine, [1,8]naphthylidine, [1,5]naphthylidine, 1H-pyrrolo[3,2-c]pyridine, 1H-pyrrolo[2,3-c]pyridine, 1H-pyrazolo[4,3-b]pyridine, 1H-pyrazolo[4,3-c]pyridine, 1H-pyrazolo[3,4-c]pyridine, 1H-pyrazolo[3,4-b]pyridine, [1,2,4]triazolo[4,3-a]pyridine, thieno[3,2-c]pyridine, thieno[3,2-b]pyridine, 1H-thieno[3,2-c]pyrazole, benzo[d]isoxazole, benzo[c]isoxazole, indolizine, 1,3-dihydroindol, 1H-pyrazolo[3,4-d]thiazole, 2H-isoindol, [1,2,4]triazolo[1,5-a]pyrimidine, 1H-pyrazolo[3,4-b]pyrazine, 1H-imidazo[4,5-b]pyrazine, 7H-purine, 4H-chromene, and the like. Among them, naphthalene, benzofuran, benzo[b]thiophene, indole, benzothiazole, dihydro-3H-benzothiazole, quinoline, dihydro-1H-quinoline, benzo[d]isothiazole, 1H-indazole, benzo[c]isothiazole, 2H-indazole, imidazo[1,2-a]pyridine, 1H-pyrrolo[2,3-b]pyridine, isoquinoline, dihydro-2H-isoquinoline, cinnoline, quinazoline, quinoxaline, 1H-benzimidazole, benzoxazole, 1H-pyrrolo[3,2-b]pyridine, benzo[1,2,5]thiadiazole, 1H-benzotriazole, 1,3-dihydropyrrolo[2,3-b]pyridine, 1,3-dihydrobenzimidazole and dihydro-3H-benzoxazole constitute a particularly preferred group. Further, naphthalene, benzofuran, benzo[b]thiophene, indole, benzothiazole, quinoline, 1H-indazole and isoquinoline are particularly preferred. Furthermore, indole and 1H-indazole are most preferred examples.

AR binds with a bond between an arbitrary carbon atom of AR and a ring-constituting carbon atom of the aromatic ring (E) in the aforementioned formula (I). Preferred examples of the ring constituting AR with indications of the aromatic ring (E) and the substitution position include naphthalen-2-yl group, naphthalen-1-yl group, benzofuran-5-yl group, benzofuran-4-yl group, benzofuran-2-yl group, benzo[b]thiophen-5-yl group, benzo[b]thiophen-4-yl group, benzo[b]thiophen-2-yl group, indol-5-yl group, indol-4-yl group, indol-6-yl group, benzothiazol-6-yl group, benzothiazol-7-yl group, benzothiazol-5-yl group, benzothiazol-4-yl group, dihydro-3H-benzothiazol-6-yl group, dihydro-3H-benzothiazol-7-yl group, dihydro-3H-benzothiazol-5-yl group, dihydro-3H-benzothiazol-4-yl group, quinolin-6-yl group, quinolin-3-yl group, quinolin-5-yl group, quinolin-7-yl group, dihydro-1H-quinolin-6-yl group, dihydro-1H-quinolin-5-yl group, benzo[d]isothiazol-5-yl group, benzo[d]isothiazol-4-yl group, benzo[d]isothiazol-6-yl group, benzo[d]isothiazol-7-yl group, 1H-indazol-5-yl group, 1H-indazol-4-yl group, 1H-indazol-6-yl group, benzo[c]isothiazol-5-yl group, benzo[c]isothiazol-4-yl group, benzo[c]isothiazol-6-yl group, benzo[c]isothiazol-7-yl group, 2H-indazol-5-yl group, 2H-indazol-4-yl group, 2H-indazol-6-yl group, imidazo[1,2-a]pyridin-6-yl group, imidazo[1,2-a]pyridin-7-yl group, 1H-pyrrolo[2,3-b]pyridin-5-yl group, 1H-pyrrolo[2,3-b]pyridin-4-yl group, isoquinolin-6-yl group, isoquinolin-3-yl group, isoquinolin-5-yl group, isoquinolin-7-yl group, dihydro-2H-isoquinolin-6-yl group, dihydro-2H-isoquinolin-5-yl group, cinnolin-6-yl group, cinnolin-5-yl group, quinazolin-6-yl group, quinazolin-7-yl group, quinazolin-5-yl group, quinoxalin-2-yl group, quinoxalin-6-yl group, quinoxalin-5-yl group, 1H-benzimidazol-5-yl group, 1H-benzimidazol-4-yl group, benzoxazol-5-yl group, benzoxazol-6-yl group, benzoxazol-4-yl group, benzoxazol-7-yl group, 1H-pyrrolo[3,2-b]pyridin-5-yl group, 1H-pyrrolo[3,2-b]pyridin-6-yl group, benzo[1,2,5]thiadiazol-5-yl group, benzo[1,2,5]thiadiazol-4-yl group, 1H-benzotriazol-5-yl group, 1H-benzotriazol-4-yl group, 1,3-dihydropyrrolo[2,3-b]pyridin-5-yl group, 1,3-dihydropyrrolo[2,3-b]pyridin-4-yl group, 1,3-dihydrobenzimidazol-5-yl group, 1,3-dihydrobenzimidazol-4-yl group, dihydro-3H-benzoxazol-6-yl group, dihydro-3H-benzoxazol-7-yl group, dihydro-3H-benzoxazol-5-yl group, dihydro-3H-benzoxazol-4-yl group, phthalazin-6-yl group, phthalazin-5-yl group, [1,8]naphthylidin-3-yl group, [1,8]naphthylidin-4-yl group, [1,5]naphthylidin-3-yl group, [1,5]naphthylidin-4-yl group, 1H-pyrrolo[3,2-c]pyridin-6-yl group, 1H-pyrrolo[3,2-c]pyridin-4-yl group, 1H-pyrrolo[2,3-c]pyridin-5-yl group, 1H-pyrrolo[2,3-c]pyridin-4-yl group, 1H-pyrazolo[4,3-b]pyridin-5-yl group, 1H-pyrazolo[4,3-b]pyridin-6-yl group, 1H-pyrazolo[4,3-c]pyridin-6-yl group, 1H-pyrazolo[4,3-c]pyridin-4-yl group, 1H-pyrazolo[3,4-c]pyridin-5-yl group, 1H-pyrazolo[3,4-c]pyridin-4-yl group, 1H-pyrazolo[3,4-b]pyridin-5-yl group, 1H-pyrazolo[3,4-b]pyridin-4-yl group, [1,2,4]triazolo[4,3-a]pyridin-6-yl group, [1,2,4]triazolo[4,3-a]pyridin-7-yl group, thieno[3,2-c]pyridin-2-yl group, thieno[3,2-c]pyridin-3-yl group, thieno[3,2-c]pyridin-6-yl group, thieno[3,2-b]pyridin-2-yl group, thieno[3,2-b]pyridin-3-yl group, thieno[3,2-b]pyridin-5-yl group, thieno[3,2-b]pyridin-6-yl group, 1H-thieno[3,2-c]pyrazol-5-yl group, 1H-thieno[3,2-c]pyrazol-4-yl group, benzo[d]isoxazol-5-yl group, benzo[d]isoxazol-4-yl group, benzo[d]isoxazol-6-yl group, benzo[d]isoxazol-7-yl group, benzo[c]isoxazol-5-yl group, benzo[c]isoxazol-4-yl group, benzo[c]isoxazol-6-yl group, benzo[c]isoxazol-7-yl group, indolizin-7-yl group, indolizin-6-yl group, indolizin-8-yl group, 1,3-dihydroindol-5-yl group, 1,3-dihydroindol-4-yl group, 1,3-dihydroindol-6-yl group, 1H-pyrazolo[3,4-d]thiazol-5-yl group, 2H-isoindol-5-yl group, 2H-isoindol-4-yl group, [1,2,4]triazolo[1,5-a]pyrimidin-6-yl group, 1H-pyrazolo[3,4-b]pyrazin-5-yl group, 1H-imidazo[4,5-b]pyrazin-5-yl group, 7H-purin-2-yl group, 4H-chromen-6-yl group, 4H-chromen-5-yl group, and the like. Among them, naphthalen-2-yl group, naphthalen-1-yl group, benzofuran-5-yl group, benzofuran-4-yl group, benzo[b]thiophen-5-yl group, benzo[b]thiophen-4-yl group, indol-5-yl group, indol-4-yl group, benzothiazol-6-yl group, benzothiazol-7-yl group, quinolin-6-yl group, quinolin-3-yl group, dihydro-1H-quinolin-6-yl group, benzo[d]isothiazol-5-yl group, 1H-indazol-5-yl group, 1H-indazol-4-yl group, imidazo[1,2-a]pyridin-6-yl group, 1H-pyrrolo[2,3-b]pyridin-5-yl group, isoquinolin-6-yl group, dihydro-2H-isoquinolin-6-yl group, cinnolin-6-yl group, benzoxazol-5-yl group, and the like constitute a particularly preferred group, and naphthalen-2-yl group, benzofuran-5-yl group, benzo[b]thiophen-5-yl group, indol-5-yl group, benzothiazol-6-yl group, quinolin-6-yl group, quinolin-3-yl group, benzo[d]isothiazol-5-yl group, 1H-indazol-5-yl group, imidazo[1,2-a]pyridin-6-yl group, 1H-pyrrolo[2,3-b]pyridin-5-yl group, isoquinolin-6-yl group, cinnolin-6-yl group, benzoxazol-5-yl group and the like are particularly preferred. Furthermore, indol-5-yl group, indol-4-yl group, indol-6-yl group, 1H-indazol-5-yl group, 1H-indazol-4-yl group, and 1H-indazol-6-yl group are most preferred examples.

Further, AR may be substituted with one of Xa or the same or different two or more of Xa. Examples of substitution position of Xa include a carbon atom of AR not bonding to the aromatic ring (E), and/or when nitrogen atom is present, that nitrogen atom.
The substituent Xa represents a linear or branched saturated alkyl group having 1 to 4 carbon atoms, a saturated cyclic alkyl group having 3 to 7 carbon atoms, oxo group, thioxo group, fluorine atom, chlorine atom, trifluoromethyl group, - (CH₂)ᵢR¹⁴, -OR¹⁰, -N(R¹¹)(R¹²), -SO₂R¹³, or -COR²⁷. However, when nitrogen atom is present in AR, Xa which may substitute on the nitrogen atom represents a linear or branched saturated alkyl group having 1 to 4 carbon atoms, a saturated cyclic alkyl group having 3 to 7 carbon atoms, or -(CH₂)ᵢR¹⁴.
Preferred examples of the substituent Xa are oxo group, thioxo group, fluorine atom, chlorine atom, and trifluoromethyl group.
Examples of the linear or branched saturated alkyl group having 1 to 4 carbon atoms as the substituent Xa include methyl group, ethyl group, propyl group, isopropyl group, butyl group, isobutyl group, t-butyl group, and the like, and among them, methyl group, ethyl group, and propyl group are particularly preferred.
Further, examples of the saturated cyclic alkyl group having 3 to 7 carbon atoms include cyclopropyl group, cyclobutyl group, cyclopentyl group, cyclohexyl group, cycloheptyl group, and the like.
-(CH₂)ᵢR¹⁴ has the same meaning as that defined above. Preferred examples are 2-hydroxyethyl group, carboxymethyl group, 2-carboxyethyl group, and N,N-dimethylcarbamoylmethyl group, and a particularly preferred example is 2-hydroxyethyl group.

R¹⁰ in -OR¹⁰ represents hydrogen atom, a lower alkyl group having 1 to 4 carbon atoms, or a -(CH₂)ᵢR¹⁴ group, and among them, hydrogen atom is a particularly preferred example. Examples of the lower alkyl group having 1 to 4 carbon atoms include methyl group, ethyl group, propyl group, isopropyl group, butyl group, isobutyl group, t-butyl group, and the like. Among them, methyl group is particularly preferred. -(CH₂)ᵢR¹⁴ has the same meaning as that defined above. Therefore, preferred examples of -OR¹⁰ are hydroxyl group, methoxy group, 2-hydroxyethyloxy group, carboxymethyloxy group, 2-carboxyethyloxy group, N,N-dimethylcarbamoylmethyloxy group, and the like, and hydroxyl group, methoxy group, and 2-hydroxyethyloxy group are particularly preferred.
R¹¹ in -N(R¹¹)(R¹²) represents hydrogen atom, or a lower alkyl group having 1 to 4 carbon atoms, and R¹² represents hydrogen atom, a lower alkyl group having 1 to 4 carbon atoms, a hydroxyalkyl group having 2 to 4 carbon atoms, -COR¹⁵, or -SO₂R¹⁶, or binds to R¹¹ to form a 3- to 6-membered ring together with the nitrogen atom to which they bind to form a saturated nitrogen-containing cycloalkyl group, or morpholino group. R¹⁵ in -COR¹⁵ represents a lower alkyl group having 1 to 4 carbon atoms, a hydroxyalkyl group having 2 to 4 carbon atoms, amino group, a mono- or dialkylamino group having 1 to 4 carbon atoms, or -A⁶-Qa. R¹⁶ in -SO₂R¹⁶ represents a lower alkyl group having 1 to 4 carbon atoms, amino group, or a mono- or dialkylamino group having 1 to 4 carbon atoms. Specific examples of -N(R¹¹)(R¹²) include amino group, N-methylamino group, N-ethylamino group, N-propylamino group, N-isopropylamino group, N,N-dimethylamino group, N,N-diethylamino group, piperidino group, pyrrolidino group, morpholino group, 2-hydroxyethylamino group, formylamino group, acetylamino group, benzoyl group, furan-2-carboxyamino group, 2-hydroxyacetylamino group, 2-aminoacetylamino group, carbamoylamino group, N-methylcarbamoylamino group, N,N-dimethylcarbamoylamino group, methylsulfonylamino group, sulfamoylamino group, N-methylsulfamoylamino group, N,N-dimethylsulfamoylamino group, and the like. Among them, preferred examples are amino group, methylamino group, dimethylamino group, 2-hydroxyethylamino group, carbamoylamino group, acetylamino group, furan-2-carboxyamino group, 2-hydroxyacetylamino group, 2-aminoacetylamino group, methylsulfonylamino group, (N,N-dimethylsulfamoyl)amino group, and the like, and amino group, N-methylamino group, N,N-dimethylamino group, and 2-hydroxyethylamino group are particularly preferred.

R¹³ in -SO₂R¹³ represents a lower alkyl group having 1 to 4 carbon atoms, amino group, or a mono- or dialkylamino group having 1 to 4 carbon atoms.
Preferred examples of -SO₂R¹³ include methanesulfonyl group, sulfamoyl group, N-methylsulfamoyl group, N,N-dimethylsulfamoyl group, and the like.
R²⁷ in -COR²⁷ represents hydrogen atom, hydroxyl group, an alkoxy group having 1 to 4 carbon atoms, a lower alkyl group having 1 to 4 carbon atoms, amino group, or a mono- or dialkylamino group having 1 to 4 carbon atoms. Specific examples of -COR²⁷ include formyl group, carboxyl group, methoxycarbonyl group, ethoxycarbonyl group, acetyl group, propionyl group, carbamoyl group, N-methylcarbamoyl group, N,N-dimethylcarbamoyl group, and the like. Carboxyl group, acetyl group, carbamoyl group, N,N-dimethylcarbamoyl group, and the like are preferred examples, and carboxyl group is particularly preferred.
Preferred examples of the group Xa include oxo group, thioxo group, fluorine atom, chlorine atom, trifluoromethyl group, methyl group, ethyl group, propyl group, 2-hydroxyethyl group, carboxymethyl group, 2-carboxyethyl group, N,N-dimethylcarbamoylmethyl group, hydroxyl group, methoxy group, 2-hydroxyethyloxy group, carboxymethyloxy group, 2-carboxyethyloxy group, N,N-dimethylcarbamoylmethyloxy group, amino group, methylamino group, dimethylamino group, 2-hydroxyethylamino group, carbamoylamino group, acetylamino group, furan-2-carboxyamino group, 2-hydroxyacetylamino group, 2-aminoacetylamino group, methylsulfonylamino group, (N,N-dimethylsulfamoyl)amino group, methanesulfonyl group, sulfamoyl group, N-methylsulfamoyl group, N,N-dimethylsulfamoyl group, carboxyl group, acetyl group, carbamoyl group, N,N-dimethylcarbamoyl group, and the like. Particularly preferred examples of the group Xa include oxo group, methyl group, ethyl group, propyl group, 2-hydroxyethyl group, hydroxyl group, methoxy group, 2-hydroxyethyloxy group, amino group, N-methylamino group, N,N-dimethylamino group, 2-hydroxyethylamino group, carboxyl group, and the like. Preferred examples of the group Xa which may substitute on nitrogen atom include methyl group, ethyl group, propyl group, hydroxymethyl group, 2-hydroxyethyl group, carboxymethyl group, 2-carboxyethyl group, and N,N-dimethylcarbamoylmethyl group. Among them, particularly preferred examples are methyl group, ethyl group, propyl group, and 2-hydroxyethyl group.

Preferred examples of AR substituted with the group Xa or unsubstituted AR include naphthalen-1-yl group, naphthalen-2-yl group, 6-fluoronaphthalen-2-yl group, 6-chloronaphthalen-2-yl group, 6-(trifluoromethyl)naphthalen-2-yl group, 5-hydroxynaphthalen-1-yl group, 5-hydroxynaphthalen-2-yl group, 6-hydroxynaphthalen-1-yl group, 6-hydroxynaphthalen-2-yl group, 7-hydroxynaphthalen-1-yl group, 7-hydroxynaphthalen-2-yl group, 5-methoxynaphthalen-1-yl group, 5-methoxynaphthalen-2-yl group, 6-methoxynaphthalen-1-yl group, 6-methoxynaphthalen-2-yl group, 7-methoxynaphthalen-1-yl group, 7-methoxynaphthalen-2-yl group, 5-(2-hydroxyethyloxy)naphthalen-2-yl group, 6-(2-hydroxyethyloxy)naphthalen-2-yl group, 7-(2-hydroxyethyloxy)naphthalen-2-yl group, 5-(carboxymethyloxy)naphthalen-2-yl group, 6-(carboxymethyloxy)naphthalen-2-yl group, 7-(carboxymethyloxy)naphthalen-2-yl group, 5-(N,N-dimethylcarbamoylmethyloxy)naphthalen-2-yl group, 6-(N,N-dimethylcarbamoylmethyloxy)naphthalen-2-yl group, 7-(N,N-dimethylcarbamoylmethyloxy)naphthalen-2-yl group, 5-aminonaphthalen-1-yl group, 5-aminonaphthalen-2-yl group, 6-aminonaphthalen-1-yl group, 6-aminonaphthalen-2-yl group, 7-aminonaphthalen-1-yl group, 7-aminonaphthalen-2-yl group, 5-(N-methylamino)naphthalen-1-yl group, 5-(N-methylamino)naphthalen-2-yl group, 6-(N-methylamino)naphthalen-1-yl group, 6-(N-methylamino)naphthalen-2-yl group, 7-(N-methylamino)naphthalen-1-yl group, 7-(N-methylamino)naphthalen-2-yl group, 5-(N,N-dimethylamino)naphthalen-1-yl group, 5-(N,N-dimethylamino)naphthalen-2-yl group, 6-(N,N-dimethylamino)naphthalen-1-yl group, 6-(N,N-dimethylamino)naphthalen-2-yl group, 7-(N,N-dimethylamino)naphthalen-1-yl group, 7-(N,N-dimethylamino)naphthalen-2-yl group, 5-(2-hydroxyethylamino)naphthalen-2-yl group, 6-(2-hydroxyethylamino)naphthalen-2-yl group, 7-(2-hydroxyethylamino)naphthalen-2-yl group, 5-acetylaminonaphthalen-2-yl group, 6-acetylaminonaphthalen-2-yl group, 6-(2-aminoacetylamino)naphthalen-2-yl group, 6-(2-hydroxyacetylamino)naphthalen-2-yl group, 7-(2-hydroxyacetylamino)naphthalen-2-yl group, 6-[(furan-2-carbonyl)amino]naphthalen-2-yl group, 7-[(furan-2-carbonyl)amino]naphthalen-2-yl group, 6-[(benzene-2-carbonyl)amino]naphthalen-2-yl group, 7-[(benzene-2-carbonyl)amino]naphthalen-2-yl group, 6-carbamoylaminonaphthalen-2-yl group, 6-methylsulfonylaminonaphthalen-2-yl group, 6-sulfamoylaminonaphthalen-2-yl group, 6-(N,N-dimethylsulfamoylamino)naphthalen-2-yl group, 6-methanesulfonylnaphthalen-2-yl group, 6-sulfamoylnaphthalen-2-yl group, 6-(N-methylsulfamoyl)naphthalen-2-yl group, 6-(N,N-dimethylsulfamoyl)naphthalen-2-yl group, 6-carboxynaphthalen-2-yl group, benzo[b]furan-4-yl group, benzo[b]furan-5-yl group, 2-methylbenzo[b]furan-4-yl group, 2-methylbenzo[b]furan-5-yl group, 3-methylbenzo[b]furan-4-yl group, 3-methylbenzo[b]furan-5-yl group, 2,3-dimethylbenzo[b]furan-4-yl group, 2,3-dimethylbenzo[b]furan-5-yl group, 2-carboxybenzo[b]furan-4-yl group, 2-carboxybenzo[b]furan-5-yl group, 2-carboxy-3-methylbenzo[b]furan-4-yl group, 2-carboxy-3-methylbenzo[b]furan-5-yl group, 3-acetylbenzo[b]furan-4-yl group, 3-acetylbenzo[b]furan-5-yl group, 3-acetyl-2-methylbenzo[b]furan-4-yl group, 3-acetyl-2-methylbenzo[b]furan-5-yl group, 3-hydroxymethylbenzo[b]furan-4-yl group, 3-hydroxymethylbenzo[b]furan-5-yl group, 3-hydroxymethyl-2-methylbenzo[b]furan-4-yl group, 3-hydroxymethyl-2-methylbenzo[b]furan-5-yl group, benzo[b]thiophen-4-yl group, benzo[b]thiophen-5-yl group, 2-methylbenzo[b]thiophen-4-yl group, 2-methylbenzo[b]thiophen-5-yl group, 3-methylbenzo[b]thiophen-4-yl group, 3-methylbenzo[b]thiophen-5-yl group, 2,3-dimethylbenzo[b]thiophen-4-yl group, 2,3-dimethylbenzo[b]thiophen-5-yl group, 2-carboxybenzo[b]thiophen-4-yl group, 2-carboxybenzo[b]thiophen-5-yl group, 2-carboxy-3-methylbenzo[b]thiophen-4-yl group, 2-carboxy-3-methylbenzo[b]thiophen-5-yl group, 3-acetylbenzo[b]thiophen-4-yl group, 3-acetylbenzo[b]thiophen-5-yl group, 3-acetyl-2-methylbenzo[b]thiophen-4-yl group, 3-acetyl-2-methylbenzo[b]thiophen-5-yl group, 3-hydroxymethylbenzo[b]thiophen-4-yl group, 3-hydroxymethylbenzo[b]thiophen-5-yl group, 3-hydroxymethyl-2-methylbenzo[b]thiophen-4-yl group, 3-hydroxymethyl-2-methylbenzo[b]thiophen-5-yl group, 1H-indol-4-yl group, 1H-indol-5-yl group, 2-methyl-1H-indol-4-yl group, 2-methyl-1H-indol-5-yl group, 3-methyl-1H-indol-4-yl group, 3-methyl-1H-indol-5-yl group, 2,3-dimethyl-1H-indol-4-yl group, 2,3-dimethyl-1H-indol-5-yl group, 2-carboxy-1H-indol-4-yl group, 2-carboxy-1H-indol-5-yl group, 2-carboxy-3-methyl-1H-indol-4-yl group, 2-carboxy-3-methyl-1H-indol-5-yl group, 3-acetyl-1H-indol-4-yl group, 3-acetyl-1H-indol-5-yl group, 3-acetyl-2-methyl-1H-indol-4-yl group, 3-acetyl-2-methyl-1H-indol-5-yl group, 3-hydroxymethyl-1H-indol-4-yl group, 3-hydroxymethyl-1H-indol-5-yl group, 3-hydroxymethyl-2-methyl-1H-indol-4-yl group, 3-hydroxymethyl-2-methyl-1H-indol-5-yl group, 1-methyl-1H-indol-4-yl group, 1-methyl-1H-indol-5-yl group, 1,2-dimethyl-1H-indol-4-yl group, 1,2-dimethyl-1H-indol-5-yl group, 1,3-dimethyl-1H-indol-4-yl group, 1,3-dimethyl-1H-indol-5-yl group, 1,2,3-trimethyl-1H-indol-4-yl group, 1,2,3-trimethyl-1H-indol-5-yl group, 2-carboxy-1-methyl-1H-indol-4-yl group, 2-carboxy-1-methyl-1H-indol-5-yl group, 2-carboxy-1,3-dimethyl-1H-indol-4-yl group, 2-carboxy-1,3-dimethyl-1H-indol-5-yl group, 3-acetyl-1-methyl-1H-indol-4-yl group, 3-acetyl-1-methyl-1H-indol-5-yl group, 3-acetyl-1,2-dimethyl-1H-indol-4-yl group, 3-acetyl-1,2-dimethyl-1H-indol-5-yl group, 3-hydroxymethyl-1-methyl-1H-indol-4-yl group, 3-hydroxymethyl-1-methyl-1H-indol-5-yl group, 3-hydroxymethyl-1,2-dimethyl-1H-indol-4-yl group, 3-hydroxymethyl-1,2-dimethyl-1H-indol-5-yl group, 1-ethyl-1H-indol-4-yl group, 1-ethyl-1H-indol-5-yl group, 1-ethyl-2-methyl-1H-indol-4-yl group, 1-ethyl-2-methyl-1H-indol-5-yl group, 1-ethyl-3-methyl-1H-indol-4-yl group, 1-ethyl-3-methyl-1H-indol-5-yl group, 1-ethyl-2,3-dimethyl-1H-indol-4-yl group, 1-ethyl-2,3-dimethyl-1H-indol-5-yl group, 2-carboxy-1-ethyl-1H-indol-4-yl group, 2-carboxy-1-ethyl-1H-indol-5-yl group, 2-carboxy-1-ethyl-3-methyl-1H-indol-4-yl group, 2-carboxy-1-ethyl-3-methyl-1H-indol-5-yl group, 3-acetyl-1-ethyl-1H-indol-4-yl group, 3-acetyl-1-ethyl-1H-indol-5-yl group, 3-acetyl-1-ethyl-2-methyl-1H-indol-4-yl group, 3-acetyl-1-ethyl-2-methyl-1H-indol-5-yl group, 1-ethyl-3-hydroxymethyl-1H-indol-4-yl group, 1-ethyl-3-hydroxymethyl-1H-indol-5-yl group, 1-ethyl-3-hydroxymethyl-2-methyl-1H-indol-4-yl group, 1-ethyl-3-hydroxymethyl-2-methyl-1H-indol-5-yl group, 1-propyl-1H-indol-4-yl group, 1-propyl-1H-indol-5-yl group, 2-methyl-1-propyl-1H-indol-4-yl group, 2-methyl-1-propyl-1H-indol-5-yl group, 3-methyl-1-propyl-1H-indol-4-yl group, 3-methyl-1-propyl-1H-indol-5-yl group, 2,3-dimethyl-1-propyl-1H-indol-4-yl group, 2,3-dimethyl-1-propyl-1H-indol-5-yl group, 2-carboxy-1-propyl-1H-indol-4-yl group, 2-carboxy-1-propyl-1H-indol-5-yl group, 2-carboxy-3-methyl-1-propyl-1H-indol-4-yl group, 2-carboxy-3-methyl-1-propyl-1H-indol-5-yl group, 3-acetyl-1-propyl-1H-indol-4-yl group, 3-acetyl-1-propyl-1H-indol-5-yl group, 3-acetyl-2-methyl-1-propyl-1H-indol-4-yl group, 3-acetyl-2-methyl-1-propyl-1H-indol-5-yl group, 3-hydroxymethyl-1-propyl-1H-indol-4-yl group, 3-hydroxymethyl-1-propyl-1H-indol-5-yl group, 3-hydroxymethyl-2-methyl-1-propyl-1H-indol-4-yl group, 3-hydroxymethyl-2-methyl-1-propyl-1H-indol-5-yl group, 1-(2-hydroxyethyl)-1H-indol-4-yl group, 1-(2-hydroxyethyl)-1H-indol-5-yl group, 1-(2-hydroxyethyl)-2-methyl-1H-indol-4-yl group, 1-(2-hydroxyethyl)-2-methyl-1H-indol-5-yl group, 1-(2-hydroxyethyl)-3-methyl-1H-indol-4-yl group, 1-(2-hydroxyethyl)-3-methyl-1H-indol-5-yl group, 2,3-dimethyl-1-(2-hydroxyethyl)-1H-indol-4-yl group, 2,3-dimethyl-1-(2-hydroxyethyl)-1H-indol-5-yl group, 2-carboxy-1-(2-hydroxyethyl)-1H-indol-4-yl group, 2-carboxy-1-(2-hydroxyethyl)-1H-indol-5-yl group, 2-carboxy-1-(2-hydroxyethyl)-3-methyl-1H-indol-4-yl group, 2-carboxy-1-(2-hydroxyethyl)-3-methyl-1H-indol-5-yl group, 3-acetyl-1-(2-hydroxyethyl)-1H-indol-4-yl group, 3-acetyl-1-(2-hydroxyethyl)-1H-indol-5-yl group, 3-acetyl-1-(2-hydroxyethyl)-2-methyl-1H-indol-4-yl group, 3-acetyl-1-(2-hydroxyethyl)-2-methyl-1H-indol-5-yl group, 1-(2-hydroxyethyl)-3-hydroxymethyl-1H-indol-4-yl group, 1-(2-hydroxyethyl)-3-hydroxymethyl-1H-indol-5-yl group, 1-(2-hydroxyethyl)-3-hydroxymethyl-2-methyl-1H-indol-4-yl group, 1-(2-hydroxyethyl)-3-hydroxymethyl-2-methyl-1H-indol-5-yl group, 1-carboxymethyl-1H-indol-4-yl group, 1-carboxymethyl-1H-indol-5-yl group, 1-carboxymethyl-2-methyl-1H-indol-4-yl group, 1-carboxymethyl-2-methyl-1H-indol-5-yl group, 1-carboxymethyl-3-methyl-1H-indol-4-yl group, 1-carboxymethyl-3-methyl-1H-indol-5-yl group, 1-carboxymethyl-2,3-dimethyl-1H-indol-4-yl group, 1-carboxymethyl-2,3-dimethyl-1H-indol-5-yl group, 2-carboxy-1-carboxymethyl-1H-indol-4-yl group, 2-carboxy-1-carboxymethyl-1H-indol-5-yl group, 2-carboxy-1-carboxymethyl-3-methyl-1H-indol-4-yl group, 2-carboxy-1-carboxymethyl-3-methyl-1H-indol-5-yl group, 3-acetyl-1-carboxymethyl-1H-indol-4-yl group, 3-acetyl-1-carboxymethyl-1H-indol-5-yl group, 3-acetyl-1-carboxymethyl-2-methyl-1H-indol-4-yl group, 3-acetyl-1-carboxymethyl-2-methyl-1H-indol-5-yl group, 1-carboxymethyl-3-hydroxymethyl-1H-indol-4-yl group, 1-carboxymethyl-3-hydroxymethyl-1H-indol-5-yl group, 1-carboxymethyl-3-hydroxymethyl-2-methyl-1H-indol-4-yl group, 1-carboxymethyl-3-hydroxymethyl-2-methyl-1H-indol-5-yl group, benzothiazol-6-yl group, 2-methylbenzothiazol-6-yl group, 2-methoxybenzothiazol-6-yl group, 2-aminobenzothiazol-6-yl group, 2-(N-methylamino)benzothiazol-6-yl group, 2-(N,N-dimethylamino)benzothiazol-6-yl group, 2-oxo-2,3-dihydrobenzothiazol-6-yl group, 2-oxo-3-methyl-2,3-dihydrobenzothiazol-6-yl group, 2-thioxo-2,3-dihydrobenzothiazol-6-yl group, 2-thioxo-3-methyl-2,3-dihydrobenzothiazol-6-yl group, quinolin-3-yl group, 2-methylquinolin-3-yl group, quinolin-6-yl group, 2-methylquinolin-6-yl group, 2-oxo-1,2-dihydroquinolin-6-yl group, benzo[d]isothiazol-5-yl group, 3-methylbenzo[d]isothiazol-5-yl group, 1H-indazol-5-yl group, 3-methyl-1H-indazol-5-yl group, 1-methyl-1H-indazol-5-yl group, 1,3-dimethyl-1H-indazol-5-yl group, 1-ethyl-1H-indazol-5-yl group, 1-ethyl-3-methyl-1H-indazol-5-yl group, 1-propyl-1H-indazol-5-yl group, 3-methyl-1-propyl-1H-indazol-5-yl group, 1-(2-hydroxyethyl)-1H-indazol-5-yl group, 1-(2-hydroxyethyl)-3-methyl-1H-indazol-5-yl group, 1-(carboxymethyl)-1H-indazol-5-yl group, 1-(carboxymethyl)-3-methyl-1H-indazol-5-yl group, 3-hydroxy-1H-indazol-5-yl group, 3-hydroxy-1-methyl-1H-indazol-5-yl group, 1-ethyl-3-hydroxy-1H-indazol-5-yl group, benzo[c]isothiazol-5-yl group, 3-methylbenzo[c]isothiazol-5-yl group, 2-methyl-2H-indazol-5-yl group, 2,3-dimethyl-2H-indazol-5-yl group, 2-ethyl-2H-indazol-5-yl group, 2-ethyl-3-methyl-2H-indazol-5-yl group, 2-propyl-2H-indazol-5-yl group, 3-methyl-2-propyl-2H-indazol-5-yl group, 2-(2-hydroxyethyl)-2H-indazol-5-yl group, 2-(2-hydroxyethyl)-3-methyl-2H-indazol-5-yl group, 2-(carboxymethyl)-2H-indazol-5-yl group, 2-(carboxymethyl)-3-methyl-2H-indazol-5-yl group, imidazo[1,2-a]pyridin-6-yl group, 2-methyl-imidazo[1,2-a]pyridin-6-yl group, 3-methyl-imidazo[1,2-a]pyridin-6-yl group, 2,3-dimethyl-imidazo[1,2-a]pyridin-6-yl group, 1H-pyrrolo[2,3-b]pyridin-5-yl group, 2-methyl-1H-pyrrolo[2,3-b]pyridin-5-yl group, 3-methyl-1H-pyrrolo[2,3-b]pyridin-5-yl group, 1-methyl-1H-pyrrolo[2,3-b]pyridin-5-yl group, 1,2-dimethyl-1H-pyrrolo[2,3-b]pyridin-5-yl group, 1,3-dimethyl-1H-pyrrolo[2,3-b]pyridin-5-yl group, 2,3-dimethyl-1H-pyrrolo[2,3-b]pyridin-5-yl group, 1,2,3-trimethyl-1H-pyrrolo[2,3-b]pyridin-5-yl group, 1-ethyl-1H-pyrrolo[2,3-b]pyridin-5-yl group, 1-ethyl-2-methyl-1H-pyrrolo[2,3-b]pyridin-5-yl group, 1-ethyl-3-methyl-1H-pyrrolo[2,3-b]pyridin-5-yl group, 1-ethyl-2,3-dimethyl-1H-pyrrolo[2,3-b]pyridin-5-yl group, 1-propyl-1H-pyrrolo[2,3-b]pyridin-5-yl group, 2-methyl-1-propyl-1H-pyrrolo[2,3-b]pyridin-5-yl group, 3-methyl-1-propyl-1H-pyrrolo[2,3-b]pyridin-5-yl group, 2,3-dimethyl-1-propyl-1H-pyrrolo[2,3-b]pyridin-5-yl group, 1-(2-hydroxyethyl)-1H-pyrrolo[2,3-b]pyridin-5-yl group, 1-(2-hydroxyethyl)-2-methyl-1H-pyrrolo[2,3-b]pyridin-5-yl group, 1-(2-hydroxyethyl)-3-methyl-1H-pyrrolo[2,3-b]pyridin-5-yl group, 2,3-dimethyl-1-(2-hydroxyethyl)-1H-pyrrolo[2,3-b]pyridin-5-yl group, 1-(carboxymethyl)1H-pyrrolo[2,3-b]pyridin-5-yl group, 1-(carboxymethyl)-2-methyl-1H-pyrrolo[2,3-b]pyridin-5-yl group, 1-(carboxymethyl)-3-methyl-1H-pyrrolo[2,3-b]pyridin-5-yl group, 1-(carboxymethyl)-2,3-dimethyl-1H-pyrrolo[2,3-b]pyridin-5-yl group, isoquinolin-6-yl group, 1-methylisoquinolin-6-yl group, 1-oxo-1,2-dihydroisoquinolin-6-yl group, cinnolin-6-yl group, cinnolin-5-yl group, quinazolin-6-yl group, quinazolin-7-yl group, quinazolin-5-yl group, 2-methylquinazolin-6-yl group, quinoxalin-6-yl group, quinoxalin-5-yl group, 2-methylquinoxalin-6-yl group, 1H-benzimidazol-5-yl group, 1H-benzimidazol-4-yl group, 1-methyl-1H-benzimidazol-5-yl group, 2-methyl-1H-benzimidazol-5-yl group, 1,2-dimethyl-1H-benzimidazol-5-yl group, benzoxazol-5-yl group, benzoxazol-6-yl group, benzoxazol-4-yl group, benzoxazol-7-yl group, 2-methylbenzoxazol-5-yl group, 1H-pyrrolo[3,2-b]pyridin-5-yl group, 1H-pyrrolo[3,2-b]pyridin-6-yl group, 1-methyl-1H-pyrrolo[3,2-b]pyridin-5-yl group, 1-ethyl-1H-pyrrolo[3,2-b]pyridin-5-yl group, 2-methyl-1H-pyrrolo[3,2-b]pyridin-5-yl group, 3-methyl-1H-pyrrolo[3,2-b]pyridin-5-yl group, 1,3-dimethyl-1H-pyrrolo[3,2-b]pyridin-5-yl group, benzo[1,2,5]thiadiazol-5-yl group, benzo[1,2,5]thiadiazol-4-yl group, 1H-benzotriazol-5-yl group, 1H-benzotriazol-4-yl group, 1-methyl-1H-benzotriazol-5-yl group, 1-ethyl-1H-benzotriazol-5-yl group, 1,3-dihydropyrrolo[2,3-b]pyridin-2-on-5-yl group, 1,3-dihydropyrrolo[2,3-b]pyridin-2-on-4-yl group, 1-methyl-1,3-dihydropyrrolo[2,3-b]pyridin-2-on-5-yl group, 1,3-dihydrobenzimidazol-2-on-5-yl group, 1,3-dihydrobenzimidazol-2-on-4-yl group, 1-methyl-1,3-dihydrobenzimidazol-2-on-5-yl group, 1,3-dihydrobenzimidazol-2-thion-5-yl group, 1,3-dihydrobenzimidazol-2-thion-4-yl group, 1-methyl-1,3-dihydrobenzimidazol-2-thion-5-yl group, 3H-benzoxazol-2-on-6-yl group, 3H-benzoxazol-2-on-7-yl group, 3H-benzoxazol-2-on-5-yl group, 3H-benzoxazol-2-on-4-yl group, 3-methyl-3H-benzoxazol-2-on-6-yl group, 3H-benzoxazole-2-thion-6-yl group, 3H-benzoxazole-2-thion-7-yl group, 3H-benzoxazole-2-thion-5-yl group, 3H-benzoxazole-2-thion-4-yl group, 3-methyl-3H-benzoxazole-2-thion-6-yl group, phthalazin-6-yl group, phthalazin-5-yl group, [1,8]naphthalidin-3-yl group, [1,8]naphthalidin-4-yl group, [1,5]naphthalidin-3-yl group, [1,5]naphthalidin-4-yl group, 1H-pyrrolo[3,2-c]pyridin-6-yl group, 1H-pyrrolo[3,2-c]pyridin-4-yl group, 1-methyl-1H-pyrrolo[3,2-c]pyridin-6-yl group, 1-ethyl-1H-pyrrolo[3,2-c]pyridin-6-yl group, 2-methyl-1H-pyrrolo[3,2-c]pyridin-6-yl group, 3-methyl-1H-pyrrolo[3,2-c]pyridin-6-yl group, 1,3-dimethyl-1H-pyrrolo[3,2-c]pyridin-6-yl group, 1H-pyrrolo[2,3-c]pyridin-5-yl group, 1H-pyrrolo[2,3-c]pyridin-4-yl group, 1-methyl-1H-pyrrolo[2,3-c]pyridin-5-yl group, 1-ethyl-1H-pyrrolo[2,3-c]pyridin-5-yl group, 2-methyl-1H-pyrrolo[2,3-c]pyridin-5-yl group, 3-methyl-1H-pyrrolo[2,3-c]pyridin-5-yl group, 1,3-dimethyl-1H-pyrrolo[2,3-c]pyridin-5-yl group, 1H-pyrazolo[4,3-b]pyridin-5-yl group, 1H-pyrazolo[4,3-b]pyridin-6-yl group, 1-methyl-1H-pyrazolo[4,3-b]pyridin-5-yl group, 1-ethyl-1H-pyrazolo[4,3-b]pyridin-5-yl group, 3-methyl-1H-pyrazolo[4,3-b]pyridin-5-yl group, 1,3-dimethyl-1H-pyrazolo[4,3-b]pyridin-5-yl group, 1H-pyrazolo[4,3-c]pyridin-6-yl group, 1H-pyrazolo[4,3-c]pyridin-4-yl group, 1-methyl-1H-pyrazolo[4,3-c]pyridin-6-yl group, 1-ethyl-1H-pyrazolo[4,3-c]pyridin-6-yl group, 3-methyl-1H-pyrazolo[4,3-c]pyridin-6-yl group, 1,3-dimethyl-1H-pyrazolo[4,3-c]pyridin-6-yl group, 1H-pyrazolo[3,4-c]pyridin-5-yl group, 1H-pyrazolo[3,4-c]pyridin-4-yl group, 1-methyl-1H-pyrazolo[3,4-c]pyridin-5-yl group, 1-ethyl-1H-pyrazolo[3,4-c]pyridin-5-yl group, 3-methyl-1H-pyrazolo[3,4-c]pyridin-5-yl group, 1,3-dimethyl-1H-pyrazolo[3,4-c]pyridin-5-yl group, 1H-pyrazolo[3,4-b]pyridin-5-yl group, 1H-pyrazolo[3,4-b]pyridin-4-yl group, 1-methyl-1H-pyrazolo[3,4-b]pyridin-5-yl group, 1-ethyl-1H-pyrazolo[3,4-b]pyridin-5-yl group, 3-methyl-1H-pyrazolo[3,4-b]pyridin-5-yl group, 1,3-dimethyl-1H-pyrazolo[3,4-b]pyridin-5-yl group, [1,2,4]triazolo[4,3-a]pyridin-6-yl group, [1,2,4]triazolo[4,3-a]pyridin-7-yl group, 3-methyl[1,2,4]triazolo[4,3-a]pyridin-6-yl group, thieno[3,2-c]pyridin-2-yl group, thieno[3,2-c]pyridin-3-yl group, thieno[3,2-c]pyridin-6-yl group, 2-methylthieno[3,2-c]pyridin-2-yl group, 3-methylthieno[3,2-c]pyridin-2-yl group, thieno[3,2-b]pyridin-2-yl group, thieno[3,2-b]pyridin-3-yl group, thieno[3,2-b]pyridin-5-yl group, thieno[3,2-b]pyridin-6-yl group, 2-methylthieno[3,2-b]pyridin-2-yl group, 3-methylthieno[3,2-b]pyridin-2-yl group, 1H-thieno[3,2-c]pyrazol-5-yl group, 1H-thieno[3,2-c]pyrazol-4-yl group, 1-methyl-1H-thieno[3,2-c]pyrazol-5-yl group, 1-ethyl-1H-thieno[3,2-c]pyrazol-5-yl group, 3-methyl-1H-thieno[3,2-c]pyrazol-5-yl group, 1,3-dimethyl-1H-thieno[3,2-c]pyrazol-5-yl group, benzo[d]isoxazol-5-yl group, benzo[d]isoxazol-4-yl group, benzo[d]isoxazol-6-yl group, benzo[d]isoxazol-7-yl group, 3-methylbenzo[d]isoxazol-5-yl group, benzo[c]isoxazol-5-yl group, benzo[c]isoxazol-4-yl group, benzo[c]isoxazol-6-yl group, benzo[c]isoxazol-7-yl group, 3-methylbenzo[c]isoxazol-5-yl group, indolizin-7-yl group, indolizin-6-yl group, indolizin-8-yl group, 1,3-dihydroindol-2-on-5-yl group, 1,3-dihydroindol-2-on-4-yl group, 1,3-dihydroindol-2-on-6-yl group, 1-methyl-1,3-dihydro-indol-2-on-5-yl group, 1H-pyrazolo[3,4-d]thiazol-5-yl group, 2H-isoindol-5-yl group, 2H-isoindol-4-yl group, 2-methyl-2H-isoindol-5-yl group, 4H-chromen-6-yl group, 4H-chromen-5-yl group, chromen-4-on-7-yl group, chromen-4-on-6-yl group, and the like.

Particularly preferred examples include naphthalen-2-yl group, 6-hydroxynaphthalen-2-yl group, 6-methoxynaphthalen-2-yl group, 6-(2-hydroxyethyloxy)naphthalen-2-yl group, 6-aminonaphthalen-2-yl group, 6-(N-methylamino)naphthalen-2-yl group, 6-(N,N-dimethylamino)naphthalen-2-yl group, 6-(2-hydroxyethylamino)naphthalen-2-yl group, benzo[b]furan-5-yl group, 2-methylbenzo[b]furan-5-yl group, 3-methylbenzo[b]furan-5-yl group, 2,3-dimethylbenzo[b]furan-5-yl group, benzo[b]thiophen-5-yl group, 2-methylbenzo[b]thiophen-5-yl group, 3-methylbenzo[b]thiophen-5-yl group, 2,3-dimethylbenzo[b]thiophen-5-yl group, 1H-indol-5-yl group, 2-methyl-1H-indol-5-yl group, 3-methyl-1H-indol-5-yl group, 2,3-dimethyl-1H-indol-5-yl group, 1-methyl-1H-indol-5-yl group, 1,2-dimethyl-1H-indol-5-yl group, 1,3-dimethyl-1H-indol-5-yl group, 1,2,3-trimethyl-1H-indol-5-yl group, 1-ethyl-1H-indol-5-yl group, 1-ethyl-2-methyl-1H-indol-5-yl group, 1-ethyl-3-methyl-1H-indol-5-yl group, 1-ethyl-2,3-dimethyl-1H-indol-5-yl group, 1-propyl-1H-indol-5-yl group, 2-methyl-1-propyl-1H-indol-5-yl group, 3-methyl-1-propyl-1H-indol-5-yl group, 2,3-dimethyl-1-propyl-1H-indol-5-yl group, 1-(2-hydroxyethyl)-1H-indol-5-yl group, 1-(2-hydroxyethyl)-2-methyl-1H-indol-5-yl group, 1-(2-hydroxyethyl)-3-methyl-1H-indol-5-yl group, 2,3-dimethyl-1-(2-hydroxyethyl)-1H-indol-5-yl group, benzothiazol-6-yl group, 2-methylbenzothiazol-6-yl group, 2-methoxybenzothiazol-6-yl group, 2-aminobenzothiazol-6-yl group, 2-oxo-2,3-dihydrobenzothiazol-6-yl group, 2-oxo-3-methyl-2,3-dihydrobenzothiazol-6-yl group, 2-thioxo-2,3-dihydrobenzothiazol-6-yl group, 2-thioxo-3-methyl-2,3-dihydrobenzothiazol-6-yl group, quinolin-3-yl group, quinolin-6-yl group, 2-oxo-1,2-dihydroquinolin-6-yl group, benzo[d]isothiazol-5-yl group, 1H-indazol-5-yl group, 1-methyl-1H-indazol-5-yl group, 1-ethyl-1H-indazol-5-yl group, 1-propyl-1H-indazol-5-yl group, 1-(2-hydroxyethyl)-1H-indazol-5-yl group, 3-hydroxy-1H-indazol-5-yl group, 3-hydroxy-1-methyl-1H-indazol-5-yl group, 1-ethyl-3-hydroxy-1H-indazol-5-yl group, imidazo[1,2-a]pyridin-6-yl group, 1H-pyrrolo[2,3-b]pyridin-5-yl group, 1-methyl-1H-pyrrolo[2,3-b]pyridin-5-yl group, 1-ethyl-1H-pyrrolo[2,3-b]pyridin-5-yl group, 1-propyl-1H-pyrrolo[2,3-b]pyridin-5-yl group, 1-(2-hydroxyethyl)-1H-pyrrolo[2,3-b]pyridin-5-yl group, isoquinolin-6-yl group, 1-oxo-1,2-dihydroisoquinolin-6-yl group, cinnolin-6-yl group, benzoxazol-5-yl group, and the like.

Particularly preferred examples include naphthalen-2-yl group, 6-hydroxynaphthalen-2-yl group, 6-methoxynaphthalen-2-yl group, 6-aminonaphthalen-2-yl group, 6-(N,N-dimethylamino)naphthalen-2-yl group, benzo[b]furan-5-yl group, benzo[b]thiophen-5-yl group, 1H-indol-5-yl group, 1-methyl-1H-indol-5-yl group, 1-ethyl-1H-indol-5-yl group, benzothiazol-6-yl group, 2-aminobenzothiazol-6-yl group, 2-oxo-2,3-dihydrobenzothiazol-6-yl group, quinolin-3-yl group, quinolin-6-yl group, 2-oxo-1,2-dihydroquinolin-6-yl group, 1H-indazol-5-yl group, 1-methyl-1H-indazol-5-yl group, 1-ethyl-1H-indazol-5-yl group, isoquinolin-6-yl group, 1-oxo-1,2-dihydroisoquinolin-6-yl group, cinnolin-6-yl group, benzoxazol-5-yl group, and the like. Further, most preferred examples include 1H-indol-5-yl group, 1-methyl-1H-indol-5-yl group, 1-ethyl-1H-indol-5-yl group, 1H-indazol-5-yl group, 1-methyl-1H-indazol-5-yl group, and 1-ethyl-1H-indazol-5-yl group.

In the aforementioned formula (I), the group Y is defined to be hydrogen atom, a lower alkyl group having 1 to 4 carbon atoms, -(CH₂)ₘN(R¹⁸)(R¹⁹), or - C(R²⁰)₂OC(O)A³R²¹. Among them, hydrogen atom and a lower alkyl group having 1 to 4 carbon atoms are preferred examples, and hydrogen atom is a particularly preferred example.
Examples of the lower alkyl group having 1 to 4 carbon atoms include methyl group, ethyl group, propyl group, isopropyl group, butyl group, isobutyl group, t-butyl group, and the like. Among them, methyl group, and ethyl group are particularly preferred.
Symbol m in -(CH₂)ₘN(R¹⁸)(R¹⁹) is defined to be an integer of 2 or 3. R¹⁸ is the same as R¹⁹, or binds to R¹⁹ to represent a saturated nitrogen-containing cycloalkyl group forming a 3- to 6-membered ring together with nitrogen atom, or form morpholino group together with nitrogen atom, and R¹⁹ is defined to be methyl group, ethyl group, or propyl group. Examples of -(CH₂)ₘN(R¹⁸)(R¹⁹) include 2-(N,N-dimethylamino)ethyl group, 2-(N,N-diethylamino)ethyl group, 2-(N,N-dipropylamino)ethyl group, 3-(N,N-dimethylamino)propyl group, 3-(N,N-diethylamino)propyl group, 2-(N,N-dipropylamino)propyl group, 2-pyrrolidin-1-ylethyl group, 2-piperidin-1-ylethyl group, 2-morpholin-4-ylethyl group, 3-pyrrolidin-1-ylpropyl group, 3-piperidin-1-ylpropyl group, 3-morpholin-4-ylpropyl group, and the like.

R²⁰ in -C(R²⁰)₂OC(O)A³R²¹ is defined to be hydrogen atom, methyl group, ethyl group, or propyl group. R²¹ is defined to be a lower alkyl group having 1 to 4 carbon atoms, a cyclic saturated alkyl group having 3 to 6 carbon atoms group, or phenyl group. Examples of the lower alkyl group having 1 to 4 carbon atoms include methyl group, ethyl group, propyl group, isopropyl group, butyl group, isobutyl group, t-butyl group, and the like, and examples of the cyclic saturated alkyl group having 3 to 6 carbon atoms group include cyclopropyl group, cyclobutyl group, cyclopentyl group, and cyclohexyl group. A³ is defined to be a single bond, or oxygen atom. Examples of -C(R²⁰)₂OC(O)A³R²¹ include acetoxymethyl group, propionyloxymethyl group, butyryloxymethyl group, (2-methylpropionyl)oxymethyl group, (2,2-dimethylpropionyl)oxymethyl group, cyclopropionyloxymethyl group, cyclopentanoyloxymethyl group, cyclohexanoyloxymethyl group, phenylcarboxymethyl group, 1-acetoxy-1-methylethyl group, 1-methyl-1-(2-methylpropionyloxy)ethyl group, 1-cyclopentanoyloxy-1-methylethyl group, 1-cyclohexanoyloxy-1-methylethyl group, methoxycarbonyloxymethyl group, ethoxycarbonyloxymethyl group, isopropyloxycarbonyloxymethyl group, t-butyloxycarbonyloxymethyl group, cyclopropyloxycarbonyloxymethyl group, cyclopentyloxycarbonyloxymethyl group, cyclohexyloxycarbonyloxymethyl group, phenyloxycarbonyloxymethyl group, 1-methoxycarbonyloxy-1-methylethyl group, 1-ethoxycarbonyloxy-1-methylethyl group, 1-isopropyloxycarbonyloxy-1-methylethyl group, 1-t-butyloxycarbonyloxy-1-methylethyl group, 1-cyclopropyloxycarbonyloxy-1-methylethyl group, 1-cyclopentyloxycarbonyloxy-1-methylethyl group, 1-cyclohexyloxycarbonyloxy-1-methylethyl group, 1-methyl-1-phenyloxycarbonyloxyethyl group, and the like.

In a preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a single bond;
the bond between the ring-constituting carbon atom at the 1-position of the ring (E) and the Link moiety represents a single bond or a double bond;
when the bond between the ring-constituting carbon atom at the 1-position of the ring (E) and the Link moiety represents a single bond, Link represents CH₂ or CH₂CH₂, and when the bond between the ring-constituting carbon atom at the 1-position of the ring (E) and the Link moiety represents a double bond, Link represents CH;
W represents methylene group;
Rs represents -D-Rx or -N(Ry)(Rz);
D represents a single bond, oxygen atom, or sulfur atom;
Rx represents propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb mentioned above;
Q in Rb represents a group which is phenyl group, thienyl group, furyl group, pyridyl group, oxazolyl group, naphthyl group, tetrahydronaphthyl group, indanyl group, indolyl group, or dihydrobenzodioxyl group;
A² in Rb represents a single bond, oxygen atom, sulfur atom, -N(methyl)-, or - N(ethyl)-;
A¹ in Rb represents a single bond, methylene group, methylmethylene group, ethylmethylene group, phenylmethylene group, ethylene group, methylethylene group, dimethylethylene group, ethylethylene group, phenylethylene group, trimethylene group, or methyltrimethylene group (provided that when A² represents oxygen atom, sulfur atom, -N(methyl)-, or -N(ethyl)-, A¹ represents ethylene group, methylethylene group, dimethylethylene group, ethylethylene group, phenylethylene group, trimethylene group, or methyltrimethylene group);
R² and R³ independently represents hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group;
Rz has the same meaning as that of Rx, or represents methyl group, or ethyl group;
Ry represents hydrogen atom, methyl group, ethyl group, propyl group, isopropyl group, butyl group, isobutyl group, pentyl group, or isopentyl group (provided that when Rz represents methyl group, or ethyl group, Ry represents a substituent other than hydrogen atom), or may bind to Rz to form, together with the nitrogen atom, a cyclic substituent (qy) selected from pyrrolidino group, piperidino group, homopiperidino group, morpholino group, piperazino group, homopiperazino group, pyrrol-1-yl group, imidazol-1-yl group, pyrazol-1-yl group, and thiomorpholino group, and the cyclic substituent (qy) may be substituted with one of alkyl group having 1 to 8 carbon atoms or two of the same or different alkyl groups having 1 to 8 carbon atoms, phenyl group, or phenylmethyl group (provided that the benzene ring of the phenyl group, or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group);
the substituent V¹ on the ring (E) represents Zx, and V² on the ring (E) represents AR;
Zx represents hydrogen atom, fluorine atom, chlorine atom, bromine atom, nitro group, methyl group, hydroxyl group, methoxy group, amino group, N-methylamino group, N-ethylamino group, N-propylamino group, N-isopropylamino group, or N,N-dimethylamino group;
AR represents a residue of naphthalene, benzofuran, benzo[b]thiophene, indole, benzothiazole, dihydro-3H-benzothiazole, quinoline, dihydro-1H-quinoline, benzo[d]isothiazole, 1H-indazole, benzo[c]isothiazole, 2H-indazole, imidazo[1,2-a]pyridine, 1H-pyrrolo[2,3-b]pyridine, isoquinoline, dihydro-2H-isoquinoline, cinnoline, quinazoline, quinoxaline, 1H-benzimidazole, benzoxazole, 1H-pyrrolo[3,2-b]pyridine, benzo[1,2,5]thiadiazole, 1H-benzotriazole, 1,3-dihydropyrrolo[2,3-b]pyridine, 1,3-dihydrobenzimidazole, dihydro-3H-benzoxazole, phthalazine, [1,8]naphthylidine, [1,5]naphthylidine, 1H-pyrrolo[3,2-c]pyridine, 1H-pyrrolo[2,3-c]pyridine, 1H-pyrazolo[4,3-b]pyridine, 1H-pyrazolo[4,3-c]pyridine, 1H-pyrazolo[3,4-c]pyridine, 1H-pyrazolo[3,4-b]pyridine, [1,2,4]triazolo[4,3-a]pyridine, thieno[3,2-c]pyridine, thieno[3,2-b]pyridine, 1H-thieno[3,2-c]pyrazole, benzo[d]isoxazole, benzo[c]isoxazole, indolizine, 1,3-dihydroindole, 1H-pyrazolo[3,4-d]thiazole, 2H-isoindole, [1,2,4]triazolo[1,5-a]pyrimidine, 1H-pyrazolo[3,4-b]pyrazine, 1H-imidazo[4,5-b]pyrazine, 7H-purine, or 4H-chromene (the aforementioned residue may be substituted with one of Xa or two or more of the same or different Xa);
Xa represents oxo group, thioxo group, fluorine atom, chlorine atom, trifluoromethyl group, methyl group, ethyl group, propyl group, 2-hydroxyethyl group, carboxymethyl group, 2-carboxyethyl group, N,N-dimethylcarbamoylmethyl group, hydroxyl group, methoxy group, 2-hydroxyethyloxy group, carboxymethyloxy group, 2-carboxyethyloxy group, N,N-dimethylcarbamoylmethyloxy group, amino group, methylamino group, dimethylamino group, 2-hydroxyethylamino group, carbamoylamino group, acetylamino group, furan-2-carboxyamino group, 2-hydroxyacetylamino group, 2-aminoacetylamino group, methylsulfonylamino group,
(N,N-dimethylsulfamoyl)amino group, methanesulfonyl group, sulfamoyl group, N-methylsulfamoyl group, N,N-dimethylsulfamoyl group, carboxyl group, acetyl group, carbamoyl group, or N,N-dimethylcarbamoyl group; and
   Y represents hydrogen atom, or a lower alkyl group having 1 to 4 carbon atoms.

In another preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a double bond;
the bond between the ring-constituting carbon atom at the 1-position of the ring (E) and the Link moiety represents a single bond;
Link represents either CH₂ or CH₂CH₂;
W represents oxygen atom;
Rs represents -D-Rx or -N(Ry)(Rz);
D represents a single bond, oxygen atom, or sulfur atom;
Rx represents propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb mentioned above;
Q in Rb represents a group which is phenyl group, thienyl group, furyl group, pyridyl group, oxazolyl group, naphthyl group, tetrahydronaphthyl group, indanyl group, indolyl group, or dihydrobenzodioxyl group;
A² in Rb represents a single bond, oxygen atom, sulfur atom, -N(methyl)-, or - N(ethyl)-;
A¹ in Rb represents a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² represents oxygen atom, sulfur atom, -N(methyl)-, or -N(ethyl)-, A¹ represents ethylene group, methylethylene group, dimethylethylene group, ethylethylene group, phenylethylene group, ethenylene group, trimethylene group, or methyltrimethylene group);
R² and R³ independently represents hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group;
Rz has the same meaning as that of Rx, or represents methyl group, or ethyl group;
Ry represents hydrogen atom, methyl group, ethyl group, propyl group, isopropyl group, butyl group, isobutyl group, pentyl group, or isopentyl group (provided that when Rz represents methyl group, or ethyl group, Ry represents a substituent other than hydrogen atom), or may bind to Rz to form, together with the nitrogen atom, a cyclic substituent (qy) selected from pyrrolidino group, piperidino group, homopiperidino group, morpholino group, piperazino group, homopiperazino group, pyrrol-1-yl group, imidazol-1-yl group, pyrazol-1-yl group, and thiomorpholino group, and the cyclic substituent (qy) may be substituted with one of alkyl group having 1 to 8 carbon atoms or two of the same or different alkyl groups having 1 to 8 carbon atoms, phenyl group, or phenylmethyl group (provided that the benzene ring of the phenyl group, or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group);
the substituent V¹ on the ring (E) represents Zx, and V² on the ring (E) represents AR;
Zx represents hydrogen atom, fluorine atom, chlorine atom, bromine atom, nitro group, methyl group, hydroxyl group, methoxy group, amino group, N-methylamino group, N-ethylamino group, N-propylamino group, N-isopropylamino group, or N,N-dimethylamino group;
AR represents a residue of naphthalene, benzofuran, benzo[b]thiophene, indole, benzothiazole, dihydro-3H-benzothiazole, quinoline, dihydro-1H-quinoline, benzo[d]isothiazole, 1H-indazole, benzo[c]isothiazole, 2H-indazole, imidazo[1,2-a]pyridine, 1H-pyrrolo[2,3-b]pyridine, isoquinoline, dihydro-2H-isoquinoline, cinnoline, quinazoline, quinoxaline, 1H-benzimidazole, benzoxazole, 1H-pyrrolo[3,2-b]pyridine, benzo[1,2,5]thiadiazole, 1H-benzotriazole, 1,3-dihydropyrrolo[2,3-b]pyridine, 1,3-dihydrobenzimidazole, dihydro-3H-benzoxazole, phthalazine, [1,8]naphthylidine, [1,5]naphthylidine, 1H-pyrrolo[3,2-c]pyridine, 1H-pyrrolo[2,3-c]pyridine, 1H-pyrazolo[4,3-b]pyridine, 1H-pyrazolo[4,3-c]pyridine, 1H-pyrazolo[3,4-c]pyridine, 1H-pyrazolo[3,4-b]pyridine, [1,2,4]triazolo[4,3-a]pyridine, thieno[3,2-c]pyridine, thieno[3,2-b]pyridine, 1H-thieno[3,2-c]pyrazole, benzo[d]isoxazole, benzo[c]isoxazole, indolizine, 1,3-dihydroindole, 1H-pyrazolo[3,4-d]thiazole, 2H-isoindole, [1,2,4]triazolo[1,5-a]pyrimidine, 1H-pyrazolo[3,4-b]pyrazine, 1H-imidazo[4,5-b]pyrazine, 7H-purine, or 4H-chromene (the aforementioned residue may be substituted with one of Xa or two or more of the same or different Xa);
Xa represents oxo group, thioxo group, fluorine atom, chlorine atom, trifluoromethyl group, methyl group, ethyl group, propyl group, 2-hydroxyethyl group, carboxymethyl group, 2-carboxyethyl group, N,N-dimethylcarbamoylmethyl group, hydroxyl group, methoxy group, 2-hydroxyethyloxy group, carboxymethyloxy group, 2-carboxyethyloxy group, N,N-dimethylcarbamoylmethyloxy group, amino group, methylamino group, dimethylamino group, 2-hydroxyethylamino group, carbamoylamino group, acetylamino group, furan-2-carboxyamino group, 2-hydroxyacetylamino group, 2-aminoacetylamino group, methylsulfonylamino group, (N,N-dimethylsulfamoyl)amino group, methanesulfonyl group, sulfamoyl group, N-methylsulfamoyl group, N,N-dimethylsulfamoyl group, carboxyl group, acetyl group, carbamoyl group, or N,N-dimethylcarbamoyl group; and
Y represents hydrogen atom, or a lower alkyl group having 1 to 4 carbon atoms.

In a still other preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a double bond;
the bond between the ring-constituting carbon atom at the 1-position of the ring (E) and the Link moiety represents a single bond;
Link represents either CH₂ or CH₂CH₂;
W represents NH, or N(methyl);
Rs represents -D-Rx or -N(Ry)(Rz);
D represents a single bond, oxygen atom, or sulfur atom;
Rx represents propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb mentioned above;
Q in Rb represents a group which is phenyl group, thienyl group, furyl group, pyridyl group, oxazolyl group, naphthyl group, tetrahydronaphthyl group, indanyl group, indolyl group, or dihydrobenzodioxyl group;
A² in Rb represents a single bond, oxygen atom, sulfur atom, -N(methyl)-, or - N(ethyl)-;
A¹ in Rb represents a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² represents oxygen atom, sulfur atom, -N(methyl)-, or -N(ethyl)-, A¹ represents ethylene group, methylethylene group, dimethylethylene group, ethylethylene group, phenylethylene group, ethenylene group, trimethylene group, or methyltrimethylene group);
R² and R³ independently represents hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group;
Rz has the same meaning as that of Rx, or represents methyl group, or ethyl group;
Ry represents hydrogen atom, methyl group, ethyl group, propyl group, isopropyl group, butyl group, isobutyl group, pentyl group, or isopentyl group (provided that when Rz represents methyl group, or ethyl group, Ry represents a substituent other than hydrogen atom), or may bind to Rz to form, together with the nitrogen atom, a cyclic substituent (qy) selected from pyrrolidino group, piperidino group, homopiperidino group, morpholino group, piperazino group, homopiperazino group, pyrrol-1-yl group, imidazol-1-yl group, pyrazol-1-yl group, and thiomorpholino group, and the cyclic substituent (qy) may be substituted with one of alkyl group having 1 to 8 carbon atoms or two of the same or different alkyl groups having 1 to 8 carbon atoms, phenyl group, or phenylmethyl group (provided that the benzene ring of the phenyl group, or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group);
the substituent V¹ on the ring (E) represents Zx, and V² on the ring (E) represents AR;
Zx represents hydrogen atom, fluorine atom, chlorine atom, bromine atom, nitro group, methyl group, hydroxyl group, methoxy group, amino group, N-methylamino group, N-ethylamino group, N-propylamino group, N-isopropylamino group, or N,N-dimethylamino group;
AR represents a residue of naphthalene, benzofuran, benzo[b]thiophene, indole, benzothiazole, dihydro-3H-benzothiazole, quinoline, dihydro-1H-quinoline, benzo[d]isothiazole, 1H-indazole, benzo[c]isothiazole, 2H-indazole, imidazo[1,2-a]pyridine, 1H-pyrrolo[2,3-b]pyridine, isoquinoline, dihydro-2H-isoquinoline, cinnoline, quinazoline, quinoxaline, 1H-benzimidazole, benzoxazole, 1H-pyrrolo[3,2-b]pyridine, benzo[1,2,5]thiadiazole, 1H-benzotriazole, 1,3-dihydropyrrolo[2,3-b]pyridine, 1,3-dihydrobenzimidazole, dihydro-3H-benzoxazole, phthalazine, [1,8]naphthylidine, [1,5]naphthylidine, 1H-pyrrolo[3,2-c]pyridine, 1H-pyrrolo[2,3-c]pyridine, 1H-pyrazolo[4,3-b]pyridine, 1H-pyrazolo[4,3-c]pyridine, 1H-pyrazolo[3,4-c]pyridine, 1H-pyrazolo[3,4-b]pyridine, [1,2,4]triazolo[4,3-a]pyridine, thieno[3,2-c]pyridine, thieno[3,2-b]pyridine, 1H-thieno[3,2-c]pyrazole, benzo[d]isoxazole, benzo[c]isoxazole, indolizine, 1,3-dihydroindole, 1H-pyrazolo[3,4-d]thiazole, 2H-isoindole, [1,2,4]triazolo[1,5-a]pyrimidine, 1H-pyrazolo[3,4-b]pyrazine, 1H-imidazo[4,5-b]pyrazine, 7H-purine, or 4H-chromene (the aforementioned residue may be substituted with one of Xa or two or more of the same or different Xa);
Xa represents oxo group, thioxo group, fluorine atom, chlorine atom, trifluoromethyl group, methyl group, ethyl group, propyl group, 2-hydroxyethyl group, carboxymethyl group, 2-carboxyethyl group, N,N-dimethylcarbamoylmethyl group, hydroxyl group, methoxy group, 2-hydroxyethyloxy group, carboxymethyloxy group, 2-carboxyethyloxy group, N,N-dimethylcarbamoylmethyloxy group, amino group, methylamino group, dimethylamino group, 2-hydroxyethylamino group, carbamoylamino group, acetylamino group, furan-2-carboxyamino group, 2-hydroxyacetylamino group, 2-aminoacetylamino group, methylsulfonylamino group, (N,N-dimethylsulfamoyl)amino group, methanesulfonyl group, sulfamoyl group, N-methylsulfamoyl group, N,N-dimethylsulfamoyl group, carboxyl group, acetyl group, carbamoyl group, or N,N-dimethylcarbamoyl group; and
Y represents hydrogen atom, or a lower alkyl group having 1 to 4 carbon atoms.

In another preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a single bond;
the bond between the ring-constituting carbon atom at the 1-position of the ring (E) and the Link moiety represents a single bond or a double bond;
when the bond between the ring-constituting carbon atom at the 1-position of the ring (E) and the Link moiety represents a single bond, Link represents CH₂ or CH₂CH₂, and when the bond between the ring-constituting carbon atom at the 1-position of the ring (E) and the Link moiety represents a double bond, Link represents CH;
W represents methylene group;
Rs represents -D-Rx or -N(Ry)(Rz);
D represents a single bond, oxygen atom, or sulfur atom;
Rx represents propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb mentioned above;
Q in Rb represents a group which is phenyl group, thienyl group, furyl group, pyridyl group, oxazolyl group, naphthyl group, tetrahydronaphthyl group, indanyl group, indolyl group, or dihydrobenzodioxyl group;
A² in Rb represents a single bond, oxygen atom, sulfur atom, -N(methyl)-, or - N(ethyl)-;
A¹ in Rb represents a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² represents oxygen atom, sulfur atom, -N(methyl)-, or -N(ethyl)-, A¹ represents ethylene group, methylethylene group, dimethylethylene group, ethylethylene group, phenylethylene group, ethenylene group, trimethylene group, or methyltrimethylene group);
R² and R³ independently represents hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group;
Rz has the same meaning as that of Rx, or represents methyl group, or ethyl group;
Ry represents hydrogen atom, methyl group, ethyl group, propyl group, isopropyl group, butyl group, isobutyl group, pentyl group, or isopentyl group (provided that when Rz represents methyl group, or ethyl group, Ry represents a substituent other than hydrogen atom), or may bind to Rz to form, together with the nitrogen atom, a cyclic substituent (qy) selected from pyrrolidino group, piperidino group, homopiperidino group, morpholino group, piperazino group, homopiperazino group, pyrrol-1-yl group, imidazol-1-yl group, pyrazol-1-yl group, and thiomorpholino group, and the cyclic substituent (qy) may be substituted with one of alkyl group having 1 to 8 carbon atoms or two of the same or different alkyl groups having 1 to 8 carbon atoms, phenyl group, or phenylmethyl group (provided that the benzene ring of the phenyl group, or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group);
the substituent V¹ on the ring (E) represents Zx, and V² on the ring (E) represents AR;
Zx represents hydrogen atom, fluorine atom, chlorine atom, bromine atom, nitro group, methyl group, hydroxyl group, methoxy group, amino group, N-methylamino group, N-ethylamino group, N-propylamino group, N-isopropylamino group, or N,N-dimethylamino group;
AR represents naphthalen-2-yl group, naphthalen-1-yl group, benzofuran-5-yl group, benzofuran-4-yl group, benzofuran-2-yl group, benzo[b]thiophen-5-yl group, benzo[b]thiophen-4-yl group, benzo[b]thiophen-2-yl group, indol-5-yl group, indol-4-yl group, indol-6-yl group, benzothiazol-6-yl group, benzothiazol-7-yl group, benzothiazol-5-yl group, benzothiazol-4-yl group, dihydro-3H-benzothiazol-6-yl group, dihydro-3H-benzothiazol-7-yl group, dihydro-3H-benzothiazol-5-yl group, dihydro-3H-benzothiazol-4-yl group, quinolin-6-yl group, quinolin-3-yl group, quinolin-5-yl group, quinolin-7-yl group, dihydro-1H-quinolin-6-yl group, dihydro-1H-quinolin-5-yl group, benzo[d]isothiazol-5-yl group, benzo[d]isothiazol-4-yl group, benzo[d]isothiazol-6-yl group, benzo[d]isothiazol-7-yl group, 1H-indazol-5-yl group, 1H-indazol-4-yl group, 1H-indazol-6-yl group, benzo[c]isothiazol-5-yl group, benzo[c]isothiazol-4-yl group, benzo[c]isothiazol-6-yl group, benzo[c]isothiazol-7-yl group, 2H-indazol-5-yl group, 2H-indazol-4-yl group, 2H-indazol-6-yl group, imidazo[1,2-a]pyridin-6-yl group, imidazo[1,2-a]pyridin-7-yl group, 1H-pyrrolo[2,3-b]pyridin-5-yl group, 1H-pyrrolo[2,3-b]pyridin-4-yl group, isoquinolin-6-yl group, isoquinolin-3-yl group, isoquinolin-5-yl group, isoquinolin-7-yl group, dihydro-2H-isoquinolin-6-yl group, dihydro-2H-isoquinolin-5-yl group, cinnolin-6-yl group, cinnolin-5-yl group, quinazolin-6-yl group, quinazolin-7-yl group, quinazolin-5-yl group, quinoxalin-2-yl group, quinoxalin-6-yl group, quinoxalin-5-yl group, 1H-benzimidazol-5-yl group, 1H-benzimidazol-4-yl group, benzoxazol-5-yl group, benzoxazol-6-yl group, benzoxazol-4-yl group, benzoxazol-7-yl group, 1H-pyrrolo[3,2-b]pyridin-5-yl group, 1H-pyrrolo[3,2-b]pyridin-6-yl group, benzo[1,2,5]thiadiazol-5-yl group, benzo[1,2,5]thiadiazol-4-yl group, 1H-benzotriazol-5-yl group, 1H-benzotriazol-4-yl group, 1,3-dihydropyrrolo[2,3-b]pyridin-5-yl group, 1,3-dihydropyrrolo[2,3-b]pyridin-4-yl group, 1,3-dihydrobenzimidazol-5-yl group, 1,3-dihydrobenzimidazol-4-yl group, dihydro-3H-benzoxazol-6-yl group, dihydro-3H-benzoxazol-7-yl group, dihydro-3H-benzoxazol-5-yl group, dihydro-3H-benzoxazol-4-yl group, phthalazin-6-yl group, phthalazin-5-yl group, [1,8]naphthylidin-3-yl group, [1,8]naphthylidin-4-yl group, [1,5]naphthylidin-3-yl group, [1,5]naphthylidin-4-yl group, 1H-pyrrolo[3,2-c]pyridin-6-yl group, 1H-pyrrolo[3,2-c]pyridin-4-yl group, 1H-pyrrolo[2,3-c]pyridin-5-yl group, 1H-pyrrolo[2,3-c]pyridin-4-yl group, 1H-pyrazolo[4,3-b]pyridin-5-yl group, 1H-pyrazolo[4,3-b]pyridin-6-yl group, 1H-pyrazolo[4,3-c]pyridin-6-yl group, 1H-pyrazolo[4,3-c]pyridin-4-yl group, 1H-pyrazolo[3,4-c]pyridin-5-yl group, 1H-pyrazolo[3,4-c]pyridin-4-yl group, 1H-pyrazolo[3,4-b]pyridin-5-yl group, 1H-pyrazolo[3,4-b]pyridin-4-yl group, [1,2,4]triazolo[4,3-a]pyridin-6-yl group, [1,2,4]triazolo[4,3-a]pyridin-7-yl group, thieno[3,2-c]pyridin-2-yl group, thieno[3,2-c]pyridin-3-yl group, thieno[3,2-c]pyridin-6-yl group, thieno[3,2-b]pyridin-2-yl group, thieno[3,2-b]pyridin-3-yl group, thieno[3,2-b]pyridin-5-yl group, thieno[3,2-b]pyridin-6-yl group, 1H-thieno[3,2-c]pyrazol-5-yl group, 1H-thieno[3,2-c]pyrazol-4-yl group, benzo[d]isoxazol-5-yl group, benzo[d]isoxazol-4-yl group, benzo[d]isoxazol-6-yl group, benzo[d]isoxazol-7-yl group, benzo[c]isoxazol-5-yl group, benzo[c]isoxazol-4-yl group, benzo[c]isoxazol-6-yl group, benzo[c]isoxazol-7-yl group, indolizin-7-yl group, indolizin-6-yl group, indolizin-8-yl group, 1,3-dihydroindol-5-yl group, 1,3-dihydroindol-4-yl group, 1,3-dihydroindol-6-yl group, 1H-pyrazolo[3,4-d]thiazol-5-yl group, 2H-isoindol-5-yl group, 2H-isoindol-4-yl group, [1,2,4]triazolo[1,5-a]pyrimidin-6-yl group, 1H-pyrazolo[3,4-b]pyrazin-5-yl group, 1H-imidazo[4,5-b]pyrazin-5-yl group, 7H-purin-2-yl group, 4H-chromen-6-yl group, or 4H-chromen-5-yl group (the aforementioned groups may be substituted with one of Xa or two or more of the same or different Xa);
Xa represents a group which is oxo group, thioxo group, fluorine atom, chlorine atom, trifluoromethyl group, methyl group, ethyl group, propyl group, 2-hydroxyethyl group, carboxymethyl group, 2-carboxyethyl group, N,N-dimethylcarbamoylmethyl group, hydroxyl group, methoxy group, 2-hydroxyethyloxy group, carboxymethyloxy group, 2-carboxyethyloxy group, N,N-dimethylcarbamoylmethyloxy group, amino group, methylamino group, dimethylamino group, 2-hydroxyethylamino group, carbamoylamino group, acetylamino group, furan-2-carboxyamino group, 2-hydroxyacetylamino group, 2-aminoacetylamino group, methylsulfonylamino group, (N,N-dimethylsulfamoyl)amino group, methanesulfonyl group, sulfamoyl group, N-methylsulfamoyl group, N,N-dimethylsulfamoyl group, carboxyl group, acetyl group, carbamoyl group, or N,N-dimethylcarbamoyl group; and
Y represents hydrogen atom, or a lower alkyl group having 1 to 4 carbon atoms.

In another preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a single bond;
the bond between the ring-constituting carbon atom at the 1-position of the ring (E) and the Link moiety represents a single bond or a double bond;
when the bond between the ring-constituting carbon atom at the 1-position of the ring (E) and the Link moiety represents a single bond, Link represents CH₂ or CH₂CH₂, and when the bond between the ring-constituting carbon atom at the 1-position of the ring (E) and the Link moiety represents a double bond, Link represents CH;
W represents methylene group;
Rs represents Rx;
Rx represents propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb mentioned above;
Q in Rb represents a group which is phenyl group, thienyl group, furyl group, pyridyl group, oxazolyl group, naphthyl group, tetrahydronaphthyl group, indanyl group, indolyl group, or dihydrobenzodioxyl group;
A² in Rb represents a single bond, oxygen atom, sulfur atom, -N(methyl)-, or -N(ethyl)-;
A¹ in Rb represents a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² represents oxygen atom, sulfur atom, -N(methyl)-, or -N(ethyl)-, A¹ represents ethylene group, methylethylene group, dimethylethylene group, ethylethylene group, phenylethylene group, ethenylene group, trimethylene group, or methyltrimethylene group);
R² and R³ independently represents hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group
the substituent V¹ on the ring (E) represents Zx, and V² on the ring (E) represents AR;
Zx represents hydrogen atom, fluorine atom, chlorine atom, bromine atom, nitro group, methyl group, hydroxyl group, methoxy group, amino group, N-methylamino group, N-ethylamino group, N-propylamino group, N-isopropylamino group, or N,N-dimethylamino group;
AR represents naphthalen-2-yl group, naphthalen-1-yl group, benzofuran-5-yl group, benzofuran-4-yl group, benzofuran-2-yl group, benzo[b]thiophen-5-yl group, benzo[b]thiophen-4-yl group, benzo[b]thiophen-2-yl group, indol-5-yl group, indol-4-yl group, indol-6-yl group, benzothiazol-6-yl group, benzothiazol-7-yl group, benzothiazol-5-yl group, benzothiazol-4-yl group, dihydro-3H-benzothiazol-6-yl group, dihydro-3H-benzothiazol-7-yl group, dihydro-3H-benzothiazol-5-yl group, dihydro-3H-benzothiazol-4-yl group, quinolin-6-yl group, quinolin-3-yl group, quinolin-5-yl group, quinolin-7-yl group, dihydro-1H-quinolin-6-yl group, dihydro-1H-quinolin-5-yl group, benzo[d]isothiazol-5-yl group, benzo[d]isothiazol-4-yl group, benzo[d]isothiazol-6-yl group, benzo[d]isothiazol-7-yl group, 1H-indazol-5-yl group, 1H-indazol-4-yl group, 1H-indazol-6-yl group, benzo[c]isothiazol-5-yl group, benzo[c]isothiazol-4-yl group, benzo[c]isothiazol-6-yl group, benzo[c]isothiazol-7-yl group, 2H-indazol-5-yl group, 2H-indazol-4-yl group, 2H-indazol-6-yl group, imidazo[1,2-a]pyridin-6-yl group, imidazo[1,2-a]pyridin-7-yl group, 1H-pyrrolo[2,3-b]pyridin-5-yl group, 1H-pyrrolo[2,3-b]pyridin-4-yl group, isoquinolin-6-yl group, isoquinolin-3-yl group, isoquinolin-5-yl group, isoquinolin-7-yl group, dihydro-2H-isoquinolin-6-yl group, dihydro-2H-isoquinolin-5-yl group, cinnolin-6-yl group, cinnolin-5-yl group, quinazolin-6-yl group, quinazolin-7-yl group, quinazolin-5-yl group, quinoxalin-2-yl group, quinoxalin-6-yl group, quinoxalin-5-yl group, 1H-benzimidazol-5-yl group, 1H-benzimidazol-4-yl group, benzoxazol-5-yl group, benzoxazol-6-yl group, benzoxazol-4-yl group, benzoxazol-7-yl group, 1H-pyrrolo[3,2-b]pyridin-5-yl group, 1H-pyrrolo[3,2-b]pyridin-6-yl group, benzo[1,2,5]thiadiazol-5-yl group, benzo[1,2,5]thiadiazol-4-yl group, 1H-benzotriazol-5-yl group, 1H-benzotriazol-4-yl group, 1,3-dihydropyrrolo[2,3-b]pyridin-5-yl group, 1,3-dihydropyrrolo[2,3-b]pyridin-4-yl group, 1,3-dihydrobenzimidazol-5-yl group, 1,3-dihydrobenzimidazol-4-yl group, dihydro-3H-benzoxazol-6-yl group, dihydro-3H-benzoxazol-7-yl group, dihydro-3H-benzoxazol-5-yl group, dihydro-3H-benzoxazol-4-yl group, phthalazin-6-yl group, phthalazin-5-yl group, [1,8]naphthylidin-3-yl group, [1,8]naphthylidin-4-yl group, [1,5]naphthylidin-3-yl group, [1,5]naphthylidin-4-yl group, 1H-pyrrolo[3,2-c]pyridin-6-yl group, 1H-pyrrolo[3,2-c]pyridin-4-yl group, 1H-pyrrolo[2,3-c]pyridin-5-yl group, 1H-pyrrolo[2,3-c]pyridin-4-yl group, 1H-pyrazolo[4,3-b]pyridin-5-yl group, 1H-pyrazolo[4,3-b]pyridin-6-yl group, 1H-pyrazolo[4,3-c]pyridin-6-yl group, 1H-pyrazolo[4,3-c]pyridin-4-yl group, 1H-pyrazolo[3,4-c]pyridin-5-yl group, 1H-pyrazolo[3,4-c]pyridin-4-yl group, 1H-pyrazolo[3,4-b]pyridin-5-yl group, 1H-pyrazolo[3,4-b]pyridin-4-yl group, [1,2,4]triazolo[4,3-a]pyridin-6-yl group, [1,2,4]triazolo[4,3-a]pyridin-7-yl group, thieno[3,2-c]pyridin-2-yl group, thieno[3,2-c]pyridin-3-yl group, thieno[3,2-c]pyridin-6-yl group, thieno[3,2-b]pyridin-2-yl group, thieno[3,2-b]pyridin-3-yl group, thieno[3,2-b]pyridin-5-yl group, thieno[3,2-b]pyridin-6-yl group, 1H-thieno[3,2-c]pyrazol-5-yl group, 1H-thieno[3,2-c]pyrazol-4-yl group, benzo[d]isoxazol-5-yl group, benzo[d]isoxazol-4-yl group, benzo[d]isoxazol-6-yl group, benzo[d]isoxazol-7-yl group, benzo[c]isoxazol-5-yl group, benzo[c]isoxazol-4-yl group, benzo[c]isoxazol-6-yl group, benzo[c]isoxazol-7-yl group, indolizin-7-yl group, indolizin-6-yl group, indolizin-8-yl group, 1,3-dihydroindol-5-yl group, 1,3-dihydroindol-4-yl group, 1,3-dihydroindol-6-yl group, 1H-pyrazolo[3,4-d]thiazol-5-yl group, 2H-isoindol-5-yl group, 2H-isoindol-4-yl group, [1,2,4]triazolo[1,5-a]pyrimidin-6-yl group, 1H-pyrazolo[3,4-b]pyrazin-5-yl group, 1H-imidazo[4,5-b]pyrazin-5-yl group, 7H-purin-2-yl group, 4H-chromen-6-yl group, or 4H-chromen-5-yl group (the aforementioned groups may be substituted with one of Xa or two or more of the same or different Xa);
Xa represents oxo group, thioxo group, fluorine atom, chlorine atom, trifluoromethyl group, methyl group, ethyl group, propyl group, 2-hydroxyethyl group, carboxymethyl group, 2-carboxyethyl group, N,N-dimethylcarbamoylmethyl group, hydroxyl group, methoxy group, 2-hydroxyethyloxy group, carboxymethyloxy group, 2-carboxyethyloxy group, N,N-dimethylcarbamoylmethyloxy group, amino group, methylamino group, dimethylamino group, 2-hydroxyethylamino group, carbamoylamino group, acetylamino group, furan-2-carboxyamino group, 2-hydroxyacetylamino group, 2-aminoacetylamino group, methylsulfonylamino group, (N,N-dimethylsulfamoyl)amino group, methanesulfonyl group, sulfamoyl group, N-methylsulfamoyl group, N,N-dimethylsulfamoyl group, carboxyl group, acetyl group, carbamoyl group, or N,N-dimethylcarbamoyl group; and
Y represents hydrogen atom, or a lower alkyl group having 1 to 4 carbon atoms.

In a still other preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a single bond;
the bond between the ring-constituting carbon atom at the 1-position of the ring (E) and the Link moiety represents a single bond or a double bond;
when the bond between the ring-constituting carbon atom at the 1-position of the ring (E) and the Link moiety represents a single bond, Link represents CH₂ or CH₂CH₂, and when the bond between the ring-constituting carbon atom at the 1-position of the ring (E) and the Link moiety represents a double bond, Link represents CH;
W represents methylene group;
Rs represents -O-Rx,;
Rx represents propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb mentioned above;
Q in Rb represents a group which is phenyl group, thienyl group, furyl group, pyridyl group, oxazolyl group, naphthyl group, tetrahydronaphthyl group, indanyl group, indolyl group, or dihydrobenzodioxyl group;
A² in Rb represents a single bond, oxygen atom, sulfur atom, -N(methyl)-, or -N(ethyl)-;
A¹ in Rb represents a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² represents oxygen atom, sulfur atom, -N(methyl)-, or -N(ethyl)-, A¹ represents ethylene group, methylethylene group, dimethylethylene group, ethylethylene group, phenylethylene group, ethenylene group, trimethylene group, or methyltrimethylene group);
R² and R³ independently represents hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group;
the substituent V¹ on the ring (E) represents Zx, and V² on the ring (E) represents AR;
Zx represents hydrogen atom, fluorine atom, chlorine atom, bromine atom, nitro group, methyl group, hydroxyl group, methoxy group, amino group, N-methylamino group, N-ethylamino group, N-propylamino group, N-isopropylamino group, or N,N-dimethylamino group;
AR represents naphthalen-2-yl group, naphthalen-1-yl group, benzofuran-5-yl group, benzofuran-4-yl group, benzofuran-2-yl group, benzo[b]thiophen-5-yl group, benzo[b]thiophen-4-yl group, benzo[b]thiophen-2-yl group, indol-5-yl group, indol-4-yl group, indol-6-yl group, benzothiazol-6-yl group, benzothiazol-7-yl group, benzothiazol-5-yl group, benzothiazol-4-yl group, dihydro-3H-benzothiazol-6-yl group, dihydro-3H-benzothiazol-7-yl group, dihydro-3H-benzothiazol-5-yl group, dihydro-3H-benzothiazol-4-yl group, quinolin-6-yl group, quinolin-3-yl group, quinolin-5-yl group, quinolin-7-yl group, dihydro-1H-quinolin-6-yl group, dihydro-1H-quinolin-5-yl group, benzo[d]isothiazol-5-yl group, benzo[d]isothiazol-4-yl group, benzo[d]isothiazol-6-yl group, benzo[d]isothiazol-7-yl group, 1H-indazol-5-yl group, 1H-indazol-4-yl group, 1H-indazol-6-yl group, benzo[c]isothiazol-5-yl group, benzo[c]isothiazol-4-yl group, benzo[c]isothiazol-6-yl group, benzo[c]isothiazol-7-yl group, 2H-indazol-5-yl group, 2H-indazol-4-yl group, 2H-indazol-6-yl group, imidazo[1,2-a]pyridin-6-yl group, imidazo[1,2-a]pyridin-7-yl group, 1H-pyrrolo[2,3-b]pyridin-5-yl group, 1H-pyrrolo[2,3-b]pyridin-4-yl group, isoquinolin-6-yl group, isoquinolin-3-yl group, isoquinolin-5-yl group, isoquinolin-7-yl group, dihydro-2H-isoquinolin-6-yl group, dihydro-2H-isoquinolin-5-yl group, cinnolin-6-yl group, cinnolin-5-yl group, quinazolin-6-yl group, quinazolin-7-yl group, quinazolin-5-yl group, quinoxalin-2-yl group, quinoxalin-6-yl group, quinoxalin-5-yl group, 1H-benzimidazol-5-yl group, 1H-benzimidazol-4-yl group, benzoxazol-5-yl group, benzoxazol-6-yl group, benzoxazol-4-yl group, benzoxazol-7-yl group, 1H-pyrrolo[3,2-b]pyridin-5-yl group, 1H-pyrrolo[3,2-b]pyridin-6-yl group, benzo[1,2,5]thiadiazol-5-yl group, benzo[1,2,5]thiadiazol-4-yl group, 1H-benzotriazol-5-yl group, 1H-benzotriazol-4-yl group, 1,3-dihydropyrrolo[2,3-b]pyridin-5-yl group, 1,3-dihydropyrrolo[2,3-b]pyridin-4-yl group, 1,3-dihydrobenzimidazol-5-yl group, 1,3-dihydrobenzimidazol-4-yl group, dihydro-3H-benzoxazol-6-yl group, dihydro-3H-benzoxazol-7-yl group, dihydro-3H-benzoxazol-5-yl group, dihydro-3H-benzoxazol-4-yl group, phthalazin-6-yl group, phthalazin-5-yl group, [1,8]naphthylidin-3-yl group, [1,8]naphthylidin-4-yl group, [1,5]naphthylidin-3-yl group, [1,5]naphthylidin-4-yl group, 1H-pyrrolo[3,2-c]pyridin-6-yl group, 1H-pyrrolo[3,2-c]pyridin-4-yl group, 1H-pyrrolo[2,3-c]pyridin-5-yl group, 1H-pyrrolo[2,3-c]pyridin-4-yl group, 1H-pyrazolo[4,3-b]pyridin-5-yl group, 1H-pyrazolo[4,3-b]pyridin-6-yl group, 1H-pyrazolo[4,3-c]pyridin-6-yl group, 1H-pyrazolo[4,3-c]pyridin-4-yl group, 1H-pyrazolo[3,4-c]pyridin-5-yl group, 1H-pyrazolo[3,4-c]pyridin-4-yl group, 1H-pyrazolo[3,4-b]pyridin-5-yl group, 1H-pyrazolo[3,4-b]pyridin-4-yl group, [1,2,4]triazolo[4,3-a]pyridin-6-yl group, [1,2,4]triazolo[4,3-a]pyridin-7-yl group, thieno[3,2-c]pyridin-2-yl group, thieno[3,2-c]pyridin-3-yl group, thieno[3,2-c]pyridin-6-yl group, thieno[3,2-b]pyridin-2-yl group, thieno[3,2-b]pyridin-3-yl group, thieno[3,2-b]pyridin-5-yl group, thieno[3,2-b]pyridin-6-yl group, 1H-thieno[3,2-c]pyrazol-5-yl group, 1H-thieno[3,2-c]pyrazol-4-yl group, benzo[d]isoxazol-5-yl group, benzo[d]isoxazol-4-yl group, benzo[d]isoxazol-6-yl group, benzo[d]isoxazol-7-yl group, benzo[c]isoxazol-5-yl group, benzo[c]isoxazol-4-yl group, benzo[c]isoxazol-6-yl group, benzo[c]isoxazol-7-yl group, indolizin-7-yl group, indolizin-6-yl group, indolizin-8-yl group, 1,3-dihydroindol-5-yl group, 1,3-dihydroindol-4-yl group, 1,3-dihydroindol-6-yl group, 1H-pyrazolo[3,4-d]thiazol-5-yl group, 2H-isoindol-5-yl group, 2H-isoindol-4-yl group, [1,2,4]triazolo[1,5-a]pyrimidin-6-yl group, 1H-pyrazolo[3,4-b]pyrazin-5-yl group, 1H-imidazo[4,5-b]pyrazin-5-yl group, 7H-purin-2-yl group, 4H-chromen-6-yl group, or 4H-chromen-5-yl group (the aforementioned groups may be substituted with one of Xa or two or more of the same or different Xa);
Xa represents oxo group, thioxo group, fluorine atom, chlorine atom, trifluoromethyl group, methyl group, ethyl group, propyl group, 2-hydroxyethyl group, carboxymethyl group, 2-carboxyethyl group, N,N-dimethylcarbamoylmethyl group, hydroxyl group, methoxy group, 2-hydroxyethyloxy group, carboxymethyloxy group, 2-carboxyethyloxy group, N,N-dimethylcarbamoylmethyloxy group, amino group, methylamino group, dimethylamino group, 2-hydroxyethylamino group, carbamoylamino group, acetylamino group, furan-2-carboxyamino group, 2-hydroxyacetylamino group, 2-aminoacetylamino group, methylsulfonylamino group, (N,N-dimethylsulfamoyl)amino group, methanesulfonyl group, sulfamoyl group, N-methylsulfamoyl group, N,N-dimethylsulfamoyl group, carboxyl group, acetyl group, carbamoyl group, or N,N-dimethylcarbamoyl group; and
Y represents hydrogen atom, or a lower alkyl group having 1 to 4 carbon atoms.

In a still other preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a single bond;
the bond between the ring-constituting carbon atom at the 1-position of the ring (E) and the Link moiety represents a single bond or a double bond;
when the bond between the ring-constituting carbon atom at the 1-position of the ring (E) and the Link moiety represents a single bond, Link represents CH₂ or CH₂CH₂, and when the bond between the ring-constituting carbon atom at the 1-position of the ring (E) and the Link moiety represents a double bond, Link represents CH;
W represents methylene group;
Rs represents -S-Rx;
Rx represents propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb mentioned above;
Q in Rb represents a group which is phenyl group, thienyl group, furyl group, pyridyl group, oxazolyl group, naphthyl group, tetrahydronaphthyl group, indanyl group, indolyl group, or dihydrobenzodioxyl group;
A² in Rb represents a single bond, oxygen atom, sulfur atom, -N(methyl)-, or - N(ethyl)-;
A¹ in Rb represents a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² represents oxygen atom, sulfur atom, -N(methyl)-, or -N(ethyl)-, A¹ represents ethylene group, methylethylene group, dimethylethylene group, ethylethylene group, phenylethylene group, ethenylene group, trimethylene group, or methyltrimethylene group);
R² and R³ independently represents hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group;
the substituent V¹ on the ring (E) represents Zx, and V² on the ring (E) represents AR;
Zx represents hydrogen atom, fluorine atom, chlorine atom, bromine atom, nitro group, methyl group, hydroxyl group, methoxy group, amino group, N-methylamino group, N-ethylamino group, N-propylamino group, N-isopropylamino group, or N,N-dimethylamino group;
AR represents naphthalen-2-yl group, naphthalen-1-yl group, benzofuran-5-yl group, benzofuran-4-yl group, benzofuran-2-yl group, benzo[b]thiophen-5-yl group, benzo[b]thiophen-4-yl group, benzo[b]thiophen-2-yl group, indol-5-yl group, indol-4-yl group, indol-6-yl group, benzothiazol-6-yl group, benzothiazol-7-yl group, benzothiazol-5-yl group, benzothiazol-4-yl group, dihydro-3H-benzothiazol-6-yl group, dihydro-3H-benzothiazol-7-yl group, dihydro-3H-benzothiazol-5-yl group, dihydro-3H-benzothiazol-4-yl group, quinolin-6-yl group, quinolin-3-yl group, quinolin-5-yl group, quinolin-7-yl group, dihydro-1H-quinolin-6-yl group, dihydro-1H-quinolin-5-yl group, benzo[d]isothiazol-5-yl group, benzo[d]isothiazol-4-yl group, benzo[d]isothiazol-6-yl group, benzo[d]isothiazol-7-yl group, 1H-indazol-5-yl group, 1H-indazol-4-yl group, 1H-indazol-6-yl group, benzo[c]isothiazol-5-yl group, benzo[c]isothiazol-4-yl group, benzo[c]isothiazol-6-yl group, benzo[c]isothiazol-7-yl group, 2H-indazol-5-yl group, 2H-indazol-4-yl group, 2H-indazol-6-yl group, imidazo[1,2-a]pyridin-6-yl group, imidazo[1,2-a]pyridin-7-yl group, 1H-pyrrolo[2,3-b]pyridin-5-yl group, 1H-pyrrolo[2,3-b]pyridin-4-yl group, isoquinolin-6-yl group, isoquinolin-3-yl group, isoquinolin-5-yl group, isoquinolin-7-yl group, dihydro-2H-isoquinolin-6-yl group, dihydro-2H-isoquinolin-5-yl group, cinnolin-6-yl group, cinnolin-5-yl group, quinazolin-6-yl group, quinazolin-7-yl group, quinazolin-5-yl group, quinoxalin-2-yl group, quinoxalin-6-yl group, quinoxalin-5-yl group, 1H-benzimidazol-5-yl group, 1H-benzimidazol-4-yl group, benzoxazol-5-yl group, benzoxazol-6-yl group, benzoxazol-4-yl group, benzoxazol-7-yl group, 1H-pyrrolo[3,2-b]pyridin-5-yl group, 1H-pyrrolo[3,2-b]pyridin-6-yl group, benzo[1,2,5]thiadiazol-5-yl group, benzo[1,2,5]thiadiazol-4-yl group, 1H-benzotriazol-5-yl group, 1H-benzotriazol-4-yl group, 1,3-dihydropyrrolo[2,3-b]pyridin-5-yl group, 1,3-dihydropyrrolo[2,3-b]pyridin-4-yl group, 1,3-dihydrobenzimidazol-5-yl group, 1,3-dihydrobenzimidazol-4-yl group, dihydro-3H-benzoxazol-6-yl group, dihydro-3H-benzoxazol-7-yl group, dihydro-3H-benzoxazol-5-yl group, dihydro-3H-benzoxazol-4-yl group, phthalazin-6-yl group, phthalazin-5-yl group, [1,8]naphthylidin-3-yl group, [1,8]naphthylidin-4-yl group, [1,5]naphthylidin-3-yl group, [1,5]naphthylidin-4-yl group, 1H-pyrrolo[3,2-c]pyridin-6-yl group, 1H-pyrrolo[3,2-c]pyridin-4-yl group, 1H-pyrrolo[2,3-c]pyridin-5-yl group, 1H-pyrrolo[2,3-c]pyridin-4-yl group, 1H-pyrazolo[4,3-b]pyridin-5-yl group, 1H-pyrazolo[4,3-b]pyridin-6-yl group, 1H-pyrazolo[4,3-c]pyridin-6-yl group, 1H-pyrazolo[4,3-c]pyridin-4-yl group, 1H-pyrazolo[3,4-c]pyridin-5-yl group, 1H-pyrazolo[3,4-c]pyridin-4-yl group, 1H-pyrazolo[3,4-b]pyridin-5-yl group, 1H-pyrazolo[3,4-b]pyridin-4-yl group, [1,2,4]triazolo[4,3-a]pyridin-6-yl group, [1,2,4]triazolo[4,3-a]pyridin-7-yl group, thieno[3,2-c]pyridin-2-yl group, thieno[3,2-c]pyridin-3-yl group, thieno[3,2-c]pyridin-6-yl group, thieno[3,2-b]pyridin-2-yl group, thieno[3,2-b]pyridin-3-yl group, thieno[3,2-b]pyridin-5-yl group, thieno[3,2-b]pyridin-6-yl group, 1H-thieno[3,2-c]pyrazol-5-yl group, 1H-thieno[3,2-c]pyrazol-4-yl group, benzo[d]isoxazol-5-yl group, benzo[d]isoxazol-4-yl group, benzo[d]isoxazol-6-yl group, benzo[d]isoxazol-7-yl group, benzo[c]isoxazol-5-yl group, benzo[c]isoxazol-4-yl group, benzo[c]isoxazol-6-yl group, benzo[c]isoxazol-7-yl group, indolizin-7-yl group, indolizin-6-yl group, indolizin-8-yl group, 1,3-dihydroindol-5-yl group, 1,3-dihydroindol-4-yl group, 1,3-dihydroindol-6-yl group, 1H-pyrazolo[3,4-d]thiazol-5-yl group, 2H-isoindol-5-yl group, 2H-isoindol-4-yl group, [1,2,4]triazolo[1,5-a]pyrimidin-6-yl group, 1H-pyrazolo[3,4-b]pyrazin-5-yl group, 1H-imidazo[4,5-b]pyrazin-5-yl group, 7H-purin-2-yl group, 4H-chromen-6-yl group, or 4H-chromen-5-yl group (the aforementioned groups may be substituted with one of Xa or two or more of the same or different Xa);
Xa represents oxo group, thioxo group, fluorine atom, chlorine atom, trifluoromethyl group, methyl group, ethyl group, propyl group, 2-hydroxyethyl group, carboxymethyl group, 2-carboxyethyl group, N,N-dimethylcarbamoylmethyl group, hydroxyl group, methoxy group, 2-hydroxyethyloxy group, carboxymethyloxy group, 2-carboxyethyloxy group, N,N-dimethylcarbamoylmethyloxy group, amino group, methylamino group, dimethylamino group, 2-hydroxyethylamino group, carbamoylamino group, acetylamino group, furan-2-carboxyamino group, 2-hydroxyacetylamino group, 2-aminoacetylamino group, methylsulfonylamino group, (N,N-dimethylsulfamoyl)amino group, methanesulfonyl group, sulfamoyl group, N-methylsulfamoyl group, N,N-dimethylsulfamoyl group, carboxyl group, acetyl group, carbamoyl group, or N,N-dimethylcarbamoyl group; and
Y represents hydrogen atom, or a lower alkyl group having 1 to 4 carbon atoms.

In a still other preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a single bond;
the bond between the ring-constituting carbon atom at the 1-position of the ring (E) and the Link moiety represents a single bond or a double bond;
when the bond between the ring-constituting carbon atom at the 1-position of the ring (E) and the Link moiety represents a single bond, Link represents CH₂ or CH₂CH₂, and when the bond between the ring-constituting carbon atom at the 1-position of the ring (E) and the Link moiety represents a double bond, Link represents CH;
W represents methylene group;
Rs represents -N(Ry)(Rz);
Rz represents a substituent which is methyl group, ethyl group, propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, Rb mentioned above;
Q in Rb represents a group which is phenyl group, thienyl group, furyl group, pyridyl group, oxazolyl group, naphthyl group, tetrahydronaphthyl group, indanyl group, indolyl group, or dihydrobenzodioxyl group;
A² in Rb represents a single bond, oxygen atom, sulfur atom, -N(methyl)-, or - N(ethyl)-;
A¹ in Rb represents a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² represents oxygen atom, sulfur atom, -N(methyl)-, or -N(ethyl)-, A¹ represents ethylene group, methylethylene group, dimethylethylene group, ethylethylene group, phenylethylene group, ethenylene group, trimethylene group, or methyltrimethylene group);
R² and R⁸ independently represents hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group;
Ry represents hydrogen atom, methyl group, ethyl group, propyl group, isopropyl group, butyl group, isobutyl group, pentyl group, or isopentyl group (provided that when Rz represents methyl group, or ethyl group, Ry represents a substituent other than hydrogen atom), or may bind to Rz to form, together with the nitrogen atom, a cyclic substituent (qy) selected from pyrrolidino group, piperidino group, homopiperidino group, morpholino group, piperazino group, homopiperazino group, pyrrol-1-yl group, imidazol-1-yl group, pyrazol-1-yl group, and thiomorpholino group, and the cyclic substituent (qy) may be substituted with one of alkyl group having 1 to 8 carbon atoms or two of the same or different alkyl groups having 1 to 8 carbon atoms, phenyl group, or phenylmethyl group (provided that the benzene ring of the phenyl group, or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group);
the substituent V¹ on the ring (E) represents Zx, and V² on the ring (E) represents AR;
Zx represents hydrogen atom, fluorine atom, chlorine atom, bromine atom, nitro group, methyl group, hydroxyl group, methoxy group, amino group, N-methylamino group, N-ethylamino group, N-propylamino group, N-isopropylamino group, or N,N-dimethylamino group;
AR represents naphthalen-2-yl group, naphthalen-1-yl group, benzofuran-5-yl group, benzofuran-4-yl group, benzofuran-2-yl group, benzo[b]thiophen-5-yl group, benzo[b]thiophen-4-yl group, benzo[b]thiophen-2-yl group, indol-5-yl group, indol-4-yl group, indol-6-yl group, benzothiazol-6-yl group, benzothiazol-7-yl group, benzothiazol-5-yl group, benzothiazol-4-yl group, dihydro-3H-benzothiazol-6-yl group, dihydro-3H-benzothiazol-7-yl group, dihydro-3H-benzothiazol-5-yl group, dihydro-3H-benzothiazol-4-yl group, quinolin-6-yl group, quinolin-3-yl group, quinolin-5-yl group, quinolin-7-yl group, dihydro-1H-quinolin-6-yl group, dihydro-1H-quinolin-5-yl group, benzo[d]isothiazol-5-yl group, benzo[d]isothiazol-4-yl group, benzo[d]isothiazol-6-yl group, benzo[d]isothiazol-7-yl group, 1H-indazol-5-yl group, 1H-indazol-4-yl group, 1H-indazol-6-yl group, benzo[c]isothiazol-5-yl group, benzo[c]isothiazol-4-yl group, benzo[c]isothiazol-6-yl group, benzo[c]isothiazol-7-yl group, 2H-indazol-5-yl group, 2H-indazol-4-yl group, 2H-indazol-6-yl group, imidazo[1,2-a]pyridin-6-yl group, imidazo[1,2-a]pyridin-7-yl group, 1H-pyrrolo[2,3-b]pyridin-5-yl group, 1H-pyrrolo[2,3-b]pyridin-4-yl group, isoquinolin-6-yl group, isoquinolin-3-yl group, isoquinolin-5-yl group, isoquinolin-7-yl group, dihydro-2H-isoquinolin-6-yl group, dihydro-2H-isoquinolin-5-yl group, cinnolin-6-yl group, cinnolin-5-yl group, quinazolin-6-yl group, quinazolin-7-yl group, quinazolin-5-yl group, quinoxalin-2-yl group, quinoxalin-6-yl group, quinoxalin-5-yl group, 1H-benzimidazol-5-yl group, 1H-benzimidazol-4-yl group, benzoxazol-5-yl group, benzoxazol-6-yl group, benzoxazol-4-yl group, benzoxazol-7-yl group, 1H-pyrrolo[3,2-b]pyridin-5-yl group, 1H-pyrrolo[3,2-b]pyridin-6-yl group, benzo[1,2,5]thiadiazol-5-yl group, benzo[1,2,5]thiadiazol-4-yl group, 1H-benzotriazol-5-yl group, 1H-benzotriazol-4-yl group, 1,3-dihydropyrrolo[2,3-b]pyridin-5-yl group, 1,3-dihydropyrrolo[2,3-b]pyridin-4-yl group, 1,3-dihydrobenzimidazol-5-yl group, 1,3-dihydrobenzimidazol-4-yl group, dihydro-3H-benzoxazol-6-yl group, dihydro-3H-benzoxazol-7-yl group, dihydro-3H-benzoxazol-5-yl group, dihydro-3H-benzoxazol-4-yl group, phthalazin-6-yl group, phthalazin-5-yl group, [1,8]naphthylidin-3-yl group, [1,8]naphthylidin-4-yl group, [1,5]naphthylidin-3-yl group, [1,5]naphthylidin-4-yl group, 1H-pyrrolo[3,2-c]pyridin-6-yl group, 1H-pyrrolo[3,2-c]pyridin-4-yl group, 1H-pyrrolo[2,3-c]pyridin-5-yl group, 1H-pyrrolo[2,3-c]pyridin-4-yl group, 1H-pyrazolo[4,3-b]pyridin-5-yl group, 1H-pyrazolo[4,3-b]pyridin-6-yl group, 1H-pyrazolo[4,3-c]pyridin-6-yl group, 1H-pyrazolo[4,3-c]pyridin-4-yl group, 1H-pyrazolo[3,4-c]pyridin-5-yl group, 1H-pyrazolo[3,4-c]pyridin-4-yl group, 1H-pyrazolo[3,4-b]pyridin-5-yl group, 1H-pyrazolo[3,4-b]pyridin-4-yl group, [1,2,4]triazolo[4,3-a]pyridin-6-yl group, [1,2,4]triazolo[4,3-a]pyridin-7-yl group, thieno[3,2-c]pyridin-2-yl group, thieno[3,2-c]pyridin-3-yl group, thieno[3,2-c]pyridin-6-yl group, thieno[3,2-b]pyridin-2-yl group, thieno[3,2-b]pyridin-3-yl group, thieno[3,2-b]pyridin-5-yl group, thieno[3,2-b]pyridin-6-yl group, 1H-thieno[3,2-c]pyrazol-5-yl group, 1H-thieno[3,2-c]pyrazol-4-yl group, benzo[d]isoxazol-5-yl group, benzo[d]isoxazol-4-yl group, benzo[d]isoxazol-6-yl group, benzo[d]isoxazol-7-yl group, benzo[c]isoxazol-5-yl group, benzo[c]isoxazol-4-yl group, benzo[c]isoxazol-6-yl group, benzo[c]isoxazol-7-yl group, indolizin-7-yl group, indolizin-6-yl group, indolizin-8-yl group, 1,3-dihydroindol-5-yl group, 1,3-dihydroindol-4-yl group, 1,3-dihydroindol-6-yl group, 1H-pyrazolo[3,4-d]thiazol-5-yl group, 2H-isoindol-5-yl group, 2H-isoindol-4-yl group, [1,2,4]triazolo[1,5-a]pyrimidin-6-yl group, 1H-pyrazolo[3,4-b]pyrazin-5-yl group, 1H-imidazo[4,5-b]pyrazin-5-yl group, 7H-purin-2-yl group, 4H-chromen-6-yl group, or 4H-chromen-5-yl group (the aforementioned groups may be substituted with one of Xa or two or more of the same or different Xa);
Xa represents oxo group, thioxo group, fluorine atom, chlorine atom, trifluoromethyl group, methyl group, ethyl group, propyl group, 2-hydroxyethyl group, carboxymethyl group, 2-carboxyethyl group, N,N-dimethylcarbamoylmethyl group, hydroxyl group, methoxy group, 2-hydroxyethyloxy group, carboxymethyloxy group, 2-carboxyethyloxy group, N,N-dimethylcarbamoylmethyloxy group, amino group, methylamino group, dimethylamino group, 2-hydroxyethylamino group, carbamoylamino group, acetylamino group, furan-2-carboxyamino group, 2-hydroxyacetylamino group, 2-aminoacetylamino group, methylsulfonylamino group, (N,N-dimethylsulfamoyl)amino group, methanesulfonyl group, sulfamoyl group, N-methylsulfamoyl group, N,N-dimethylsulfamoyl group, carboxyl group, acetyl group, carbamoyl group, or N,N-dimethylcarbamoyl group; and
Y represents hydrogen atom, or a lower alkyl group having 1 to 4 carbon atoms.

In a particularly preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a single bond;
the bond between the ring-constituting carbon atom at the 1-position of the ring (E) and the Link moiety represents a single bond, or a double bond;
when the bond between the ring-constituting carbon atom at the 1-position of the ring (E) and the Link moiety represents a single bond, Link represents CH₂, and when the bond between the ring-constituting carbon atom at the 1-position of the ring (E) and the Link moiety represents a double bond, Link represents CH;
W represents methylene group;
Rs represents -D-Rx or -N(Ry)(Rz);
D represents a single bond, or oxygen atom;
Rx represents propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, or cycloheptylmethyl group, or Rb mentioned above;
Q in Rb represents phenyl group, 2-thienyl group, 3-thienyl group, 2-furyl group, 3-furyl group, or indan-2-yl group;
A² in Rb represents a single bond, oxygen atom, sulfur atom, -N(methyl)-, or - N(ethyl)-;
A¹ in Rb represents a single bond, or methylene group, methylmethylene group, or ethylene group when A² represents a single bond, or ethylene group when A² represents oxygen atom, sulfur atom, -N(methyl)-, or -N(ethyl)-;
R² and R³ independently represent hydrogen atom, methyl group, fluorine atom, chlorine atom, trifluoromethyl group, hydroxyl group, methoxy group, or dimethylamino group;
Rz has the same meaning as that of Rx, or represents methyl group, or ethyl group;
Ry represents hydrogen atom, methyl group, ethyl group, propyl group, isopropyl group, butyl group, isobutyl group, pentyl group, or isopentyl group (provided that when Rz represents methyl group, or ethyl group, Ry represents a substituent other than hydrogen atom), or may bind to Rz to form, together with the nitrogen atom, a cyclic substituent (qy) selected from pyrrolidino group, piperidino group, morpholino group, and piperazino group, and the cyclic substituent (qy) may be substituted with one substituent or two of the same or different substituents selected from methyl group, ethyl group, propyl group, isopropyl group, butyl group, isobutyl group, cyclopentyl group, cyclohexyl group, cyclopentylmethyl group, cyclohexylmethyl group, phenyl group, and phenylmethyl group (provided that the benzene ring of the phenyl group, or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group);
the substituent V¹ on the ring (E) represents Zx, and V² on the ring (E) represents AR;
Zx represents hydrogen atom, fluorine atom, chlorine atom, bromine atom, methyl group, hydroxyl group, amino group, or N-methylamino group;
AR represents a substituent which is naphthalen-2-yl group, naphthalen-1-yl group, benzofuran-5-yl group, benzofuran-4-yl group, benzo[b]thiophen-5-yl group, benzo[b]thiophen-4-yl group, indol-5-yl group, indol-4-yl group, benzothiazol-6-yl group, benzothiazol-7-yl group, quinolin-6-yl group, quinolin-3-yl group, dihydro-1H-quinolin-6-yl group, benzo[d]isothiazol-5-yl group, 1H-indazol-5-yl group, 1H-indazol-4-yl group, imidazo[1,2-a]pyridin-6-yl group, 1H-pyrrolo[2,3-b]pyridin-5-yl group, isoquinolin-6-yl group, dihydro-2H-isoquinolin-6-yl group, cinnolin-6-yl group, or benzoxazol-5-yl group (the aforementioned groups may be substituted with one of Xa or two or more of the same or different Xa);
Xa represents oxo group, methyl group, ethyl group, propyl group, 2-hydroxyethyl group, hydroxyl group, methoxy group, 2-hydroxyethyloxy group, amino group, N-methylamino group, N,N-dimethylamino group, 2-hydroxyethylamino group, or carboxyl group; and
Y represents hydrogen atom, methyl group, or ethyl group.

In another particularly preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a single bond;
the bond between the ring-constituting carbon atom at the 1-position of the ring (E) and the Link moiety represents a single bond, or a double bond;
when the bond between the ring-constituting carbon atom at the 1-position of the ring (E) and the Link moiety represents a single bond, Link represents CH₂, and when the bond between the ring-constituting carbon atom at the 1-position of the ring (E) and the Link moiety represents a double bond, Link represents CH;
W represents methylene group;
Rs represents -D-Rx or -N(Ry)(Rz);
D represents a single bond, or oxygen atom;
Rx represents propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group;
the substituent -N(Ry)(Rz) represents N,N-dimethylamino group, N-ethyl-N-methylamino group, N,N-diethylamino group, N-methyl-N-propylamino group, N-ethyl-N-propylamino group, N-isopropyl-N-methylamino group, N-ethyl-N-isopropylamino group, N-butylamino group, N-butyl-N-methylamino group, N-butyl-N-ethylamino group, N-isobutylamino group, N-isobutyl-N-methylamino group, N-ethyl-N-isobutylamino group, N-isopentylamino group, N-isopentyl-N-methylamino group, N-ethyl-N-isopentylamino group, N-(2-ethylbutyl)amino group, N-(2-ethylbutyl)-N-methylamino group, N-cyclopentylamino group, N-cyclopentyl-N-methylamino group, N-cyclohexylamino group, N-cyclohexyl-N-methylamino group, N-cycloheptylamino group, N-(cyclopentylmethyl)amino group, N-(cyclopentylmethyl)-N-methylamino group, N-(cyclohexylmethyl)amino group, N-(cyclohexylmethyl)-N-methylamino group, N-phenylamino group, N-(2-methylphenyl)amino group, N-(4-methylphenyl)amino group, N-(2-fluorophenyl)amino group, N-(3-fluorophenyl)amino group, N-(4-fluorophenyl)amino group, N-(2-chlorophenyl)amino group, N-(3-chlorophenyl)amino group, N-(4-chlorophenyl)amino group, N-(indan-2-yl)amino group, N-(1-phenylethyl)amino group, N-[1-(2-fluorophenyl)ethyl]amino group, N-[1-(3-fluorophenyl)ethyl]amino group, N-[1-(4-fluorophenyl)ethyl]amino group, N-[1-(2-chlorophenyl)ethyl]amino group, N-[1-(3-chlorophenyl)ethyl]amino group, N-[1-(4-chlorophenyl)ethyl]amino group, N-benzylamino group, N-benzyl-N-methylamino group, N-benzyl-N-ethylamino group, N-(2-methylphenylmethyl)amino group, N-methyl-N-(2-methylphenylmethyl)amino group, N-(3-methylphenylmethyl)amino group, N-methyl-N-(3-methylphenylmethyl)amino group, N-(4-methylphenylmethyl)amino group, N-methyl-N-(4-methylphenylmethyl)amino group, N-(2-fluorophenylmethyl)amino group, N-(2-fluorophenylmethyl)-N-methylamino group, N-(3-fluorophenylmethyl)amino group, N-(3-fluorophenylmethyl)-N-methylamino group, N-(4-fluorophenylmethyl)amino group, N-(4-fluorophenylmethyl)-N-methylamino group, N-(2-chlorophenylmethyl)amino group, N-(2-chlorophenylmethyl)-N-methylamino group, N-(3-chlorophenylmethyl)amino group, N-(3-chlorophenylmethyl)-N-methylamino group, N-(4-chlorophenylmethyl)amino group, N-(4-chlorophenylmethyl)-N-methylamino group, N-(2,3-difluorophenylmethyl)amino group, N-(2,3-difluorophenylmethyl)-N-methylamino group, N-(2,4-difluorophenylmethyl)amino group, N-(2,4-difluorophenylmethyl)-N-methylamino group, N-(2,5-difluorophenylmethyl)amino group, N-(2,5-difluorophenylmethyl)-N-methylamino group, N-(3,4-difluorophenylmethyl)amino group, N-(3,4-difluorophenylmethyl)-N-methylamino group, N-(3,5-difluorophenylmethyl)amino group, N-(3,5-difluorophenylmethyl)-N-methylamino group, N-(2,3-dichlorophenylmethyl)amino group, N-(2,3-dichlorophenylmethyl)-N-methylamino group, N-(2,4-dichlorophenylmethyl)amino group, N-(2,4-dichlorophenylmethyl)-N-methylamino group, N-(2,5-dichlorophenylmethyl)amino group, N-(2,5-dichlorophenylmethyl)-N-methylamino group, N-(2,6-dichlorophenylmethyl)amino group, N-(2,6-dichlorophenylmethyl)-N-methylamino group, N-(3,4-dichlorophenylmethyl)amino group, N-(3,4-dichlorophenylmethyl)-N-methylamino group, N-(3,5-dichlorophenylmethyl)amino group, N-(3,5-dichlorophenylmethyl)-N-methylamino group, N-[2-(trifluoromethyl)phenylmethyl]amino group, N-methyl-N-[2-(trifluoromethyl)phenylmethyl]amino group, N-[3-(trifluoromethyl)phenylmethyl]amino group, N-methyl-N-[3-(trifluoromethyl)phenylmethyl]amino group, N-[4-(trifluoromethyl)phenylmethyl]amino group, N-methyl-N-[4-(trifluoromethyl)phenylmethyl]amino group, pyrrolidino group, 2-methylpyrrolidino group, 3-methylpyrrolidino group, 2,5-dimethylpyrrolidino group, 3,4-dimethylpyrrolidino group, piperidino group, 2-methylpiperidino group, 3-methylpiperidino group, 4-methylpiperidino group, 4-ethylpiperidino group, 4-propylpiperidino group, 4-isopropylpiperidino group, 4-butylpiperidino group, 4-isobutylpiperidino group, 4-cyclopentylpiperidino group, 4-cyclohexylpiperidino group, 4-(cyclopentylmethyl)piperidino group, 4-(cyclohexylmethyl)piperidino group, 4-phenylpiperidino group, 4-(2-methylphenyl)piperidino group, 4-(4-methylphenyl)piperidino group, 4-(2-fluorophenyl)piperidino group, 4-(3-fluorophenyl)piperidino group, 4-(4-fluorophenyl)piperidino group, 4-(2-chlorophenyl)piperidino group, 4-(3-chlorophenyl)piperidino group, 4-(4-chlorophenyl)piperidino group, 4-benzylpiperidino group, 4-(2-methylphenylmethyl)piperidino group, 4-(3-methylphenylmethyl)piperidino group, 4-methylphenylmethyl)piperidino group, 4-(2,3-dimethylphenylmethyl)piperidino group, 4-(3,5-dimethylphenylmethyl)piperidino group, 4-(2-fluorophenylmethyl)piperidino group, 4-(3-fluorophenylmethyl)piperidino group, 4-(4-fluorophenylmethyl)piperidino group, 4-(2-chlorophenylmethyl)piperidino group, 4-(3-chlorophenylmethyl)piperidino group, 4-(4-chlorophenylmethyl)piperidino group, 4-(2,3-difluorophenylmethyl)piperidino group, 4-(2,4-difluorophenylmethyl)piperidino group, 4-(2,5-difluorophenylmethyl)piperidino group, 4-(3,4-difluorophenylmethyl)piperidino group, 4-(2,3-dichlorophenylmethyl)piperidino group, 4-(2,4-dichlorophenylmethyl)piperidino group, 4-(2,5-dichlorophenylmethyl)piperidino group, 4-(2,6-dichlorophenylmethyl)piperidino group, 4-(3,4-dichlorophenylmethyl)piperidino group, 4-(3,5-dichlorophenylmethyl)piperidino group, 4-(3,6-dichlorophenylmethyl)piperidino group, 4-(2-methoxyphenylmethyl)piperidino group, 4-(3-methoxyphenylmethyl)piperidino group, 4-(4-methoxyphenylmethyl)piperidino group, 2,6-dimethylpiperidino group, 3,5-dimethylpiperidino group, 4,4-dimethylpiperidino group, homopiperidino group, morpholino group, 3,5-dimethylmorpholino group, 4-phenylpiperazino group, 4-(2-methylphenyl)piperazino group, 4-(4-methylphenyl)piperazino group, 4-(2-fluorophenyl)piperazino group, 4-(3-fluorophenyl)piperazino group, 4-(4-fluorophenyl)piperazino group, 4-(2-chlorophenyl)piperazino group, 4-(3-chlorophenyl)piperazino group, 4-(4-chlorophenyl)piperazino group, 4-benzylpiperazino group, 4-(2-methylphenylmethyl)piperazino group, 4-(3-methylphenylmethyl)piperazino group, 4-methylphenylmethyl)piperazino group, 4-(2,3-dimethylphenylmethyl)piperazino group, 4-(3,5-dimethylphenylmethyl)piperazino group, 4-(2-fluorophenylmethyl)piperazino group, 4-(3-fluorophenylmethyl)piperazino group, 4-(4-fluorophenylmethyl)piperazino group, 4-(2-chlorophenylmethyl)piperazino group, 4-(3-chlorophenylmethyl)piperazino group, 4-(4-chlorophenylmethyl)piperazino group, 4-(2,3-difluorophenylmethyl)piperazino group, 4-(2,4-difluorophenylmethyl)piperazino group, 4-(2,5-difluorophenylmethyl)piperazino group, 4-(3,4-difluorophenylmethyl)piperazino group, 4-(2,3-dichlorophenylmethyl)piperazino group, 4-(2,4-dichlorophenylmethyl)piperazino group, 4-(2,5-dichlorophenylmethyl)piperazino group, 4-(2,6-dichlorophenylmethyl)piperazino group, 4-(3,4-dichlorophenylmethyl)piperazino group, 4-(3,5-dichlorophenylmethyl)piperazino group, 4-(3,6-dichlorophenylmethyl)piperazino group, 4-(2-methoxyphenylmethyl)piperazino group, 4-(3-methoxyphenylmethyl)piperazino group, or 4-(4-methoxyphenylmethyl)piperazino group;
the substituent V¹ on the ring (E) represents Zx, and V² on the ring (E) represents AR;
Zx represents hydrogen atom, fluorine atom, chlorine atom, bromine atom, methyl group, hydroxyl group, amino group, or N-methylamino group;
AR represents a group which is naphthalen-2-yl group, 6-hydroxynaphthalen-2-yl group, 6-methoxynaphthalen-2-yl group, 6-(2-hydroxyethyloxy)naphthalen-2-yl group, 6-aminonaphthalen-2-yl group, 6-(N-methylamino)naphthalen-2-yl group, 6-(N,N-dimethylamino)naphthalen-2-yl group, 6-(2-hydroxyethylamino)naphthalen-2-yl group, benzo[b]furan-5-yl group, 2-methylbenzo[b]furan-5-yl group, 3-methylbenzo[b]furan-5-yl group, 2,3-dimethylbenzo[b]furan-5-yl group, benzo[b]thiophen-5-yl group, 2-methylbenzo[b]thiophen-5-yl group, 3-methylbenzo[b]thiophen-5-yl group, 2,3-dimethylbenzo[b]thiophen-5-yl group, 1H-indol-5-yl group, 2-methyl-1H-indol-5-yl group, 3-methyl-1H-indol-5-yl group, 2,3-dimethyl-1H-indol-5-yl group, 1-methyl-1H-indol-5-yl group, 1,2-dimethyl-1H-indol-5-yl group, 1,3-dimethyl-1H-indol-5-yl group, 1,2,3-trimethyl-1H-indol-5-yl group, 1-ethyl-1H-indol-5-yl group, 1-ethyl-2-methyl-1H-indol-5-yl group, 1-ethyl-3-methyl-1H-indol-5-yl group, 1-ethyl-2,3-dimethyl-1H-indol-5-yl group, 1-propyl-1H-indol-5-yl group, 2-methyl-1-propyl-1H-indol-5-yl group, 3-methyl-1-propyl-1H-indol-5-yl group, 2,3-dimethyl-1-propyl-1H-indol-5-yl group, 1-(2-hydroxyethyl)-1H-indol-5-yl group, 1-(2-hydroxyethyl)-2-methyl-1H-indol-5-yl group, 1-(2-hydroxyethyl)-3-methyl-1H-indol-5-yl group, 2,3-dimethyl-1-(2-hydroxyethyl)-1H-indol-5-yl group, benzothiazol-6-yl group, 2-methylbenzothiazol-6-yl group, 2-methoxybenzothiazol-6-yl group, 2-aminobenzothiazol-6-yl group, 2-oxo-2,3-dihydrobenzothiazol-6-yl group, 2-oxo-3-methyl-2,3-dihydrobenzothiazol-6-yl group, 2-thioxo-2,3-dihydrobenzothiazol-6-yl group, 2-thioxo-3-methyl-2,3-dihydrobenzothiazol-6-yl group, quinolin-3-yl group, quinolin-6-yl group, 2-oxo-1,2-dihydroquinolin-6-yl group, benzo[d]isothiazol-5-yl group, 1H-indazol-5-yl group, 1-methyl-1H-indazol-5-yl group, 1-ethyl-1H-indazol-5-yl group, 1-propyl-1H-indazol-5-yl group, 1-(2-hydroxyethyl)-1H-indazol-5-yl group, 3-hydroxy-1H-indazol-5-yl group, 3-hydroxy-1-methyl-1H-indazol-5-yl group, 1-ethyl-3-hydroxy-1H-indazol-5-yl group, imidazo[1,2-a]pyridin-6-yl group, 1H-pyrrolo[2,3-b]pyridin-5-yl group, 1-methyl-1H-pyrrolo[2,3-b]pyridin-5-yl group, 1-ethyl-1H-pyrrolo[2,3-b]pyridin-5-yl group, 1-propyl-1H-pyrrolo[2,3-b]pyridin-5-yl group, 1-(2-hydroxyethyl)-1H-pyrrolo[2,3-b]pyridin-5-yl group, isoquinolin-6-yl group, 1-oxo-1,2-dihydroisoquinolin-6-yl group, cinnolin-6-yl group, or benzoxazol-5-yl group; and
Y represents hydrogen atom, methyl group, or ethyl group.

In a still other particularly preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a single bond;
the bond between the ring-constituting carbon atom at the 1-position of the ring (E) and the Link moiety represents a single bond, or a double bond;
when the bond between the ring-constituting carbon atom at the 1-position of the ring (E) and the Link moiety represents a single bond, Link represents CH₂, and when the bond between the ring-constituting carbon atom at the 1-position of the ring (E) and the Link moiety represents a double bond, Link represents CH;
W represents methylene group;
Rs represents propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group;
the substituent V¹ on the ring (E) represents Zx, and V² on the ring (E) represents AR;
Zx represents hydrogen atom, fluorine atom, chlorine atom, bromine atom, methyl group, hydroxyl group, amino group, or N-methylamino group;
AR represents a group which is naphthalen-2-yl group, 6-hydroxynaphthalen-2-yl group, 6-methoxynaphthalen-2-yl group, 6-(2-hydroxyethyloxy)naphthalen-2-yl group, 6-aminonaphthalen-2-yl group, 6-(N-methylamino)naphthalen-2-yl group, 6-(N,N-dimethylamino)naphthalen-2-yl group, 6-(2-hydroxyethylamino)naphthalen-2-yl group, benzo[b]furan-5-yl group, 2-methylbenzo[b]furan-5-yl group, 3-methylbenzo[b]furan-5-yl group, 2,3-dimethylbenzo[b]furan-5-yl group, benzo[b]thiophen-5-yl group, 2-methylbenzo[b]thiophen-5-yl group, 3-methylbenzo[b]thiophen-5-yl group, 2,3-dimethylbenzo[b]thiophen-5-yl group, 1H-indol-5-yl group, 2-methyl-1H-indol-5-yl group, 3-methyl-1H-indol-5-yl group, 2,3-dimethyl-1H-indol-5-yl group, 1-methyl-1H-indol-5-yl group, 1,2-dimethyl-1H-indol-5-yl group, 1,3-dimethyl-1H-indol-5-yl group, 1,2,3-trimethyl-1H-indol-5-yl group, 1-ethyl-1H-indol-5-yl group, 1-ethyl-2-methyl-1H-indol-5-yl group, 1-ethyl-3-methyl-1H-indol-5-yl group, 1-ethyl-2,3-dimethyl-1H-indol-5-yl group, 1-propyl-1H-indol-5-yl group, 2-methyl-1-propyl-1H-indol-5-yl group, 3-methyl-1-propyl-1H-indol-5-yl group, 2,3-dimethyl-1-propyl-1H-indol-5-yl group, 1-(2-hydroxyethyl)-1H-indol-5-yl group, 1-(2-hydroxyethyl)-2-methyl-1H-indol-5-yl group, 1-(2-hydroxyethyl)-3-methyl-1H-indol-5-yl group, 2,3-dimethyl-1-(2-hydroxyethyl)-1H-indol-5-yl group, benzothiazol-6-yl group, 2-methylbenzothiazol-6-yl group, 2-methoxybenzothiazol-6-yl group, 2-aminobenzothiazol-6-yl group, 2-oxo-2,3-dihydrobenzothiazol-6-yl group, 2-oxo-3-methyl-2,3-dihydrobenzothiazol-6-yl group, 2-thioxo-2,3-dihydrobenzothiazol-6-yl group, 2-thioxo-3-methyl-2,3-dihydrobenzothiazol-6-yl group, quinolin-3-yl group, quinolin-6-yl group, 2-oxo-1,2-dihydroquinolin-6-yl group, benzo[d]isothiazol-5-yl group, 1H-indazol-5-yl group, 1-methyl-1H-indazol-5-yl group, 1-ethyl-1H-indazol-5-yl group, 1-propyl-1H-indazol-5-yl group, 1-(2-hydroxyethyl)-1H-indazol-5-yl group, 3-hydroxy-1H-indazol-5-yl group, 3-hydroxy-1-methyl-1H-indazol-5-yl group, 1-ethyl-3-hydroxy-1H-indazol-5-yl group, imidazo[1,2-a]pyridin-6-yl group, 1H-pyrrolo[2,3-b]pyridin-5-yl group, 1-methyl-1H-pyrrolo[2,3-b]pyridin-5-yl group, 1-ethyl-1H-pyrrolo[2,3-b]pyridin-5-yl group, 1-propyl-1H-pyrrolo[2,3-b]pyridin-5-yl group, 1-(2-hydroxyethyl)-1H-pyrrolo[2,3-b]pyridin-5-yl group, isoquinolin-6-yl group, 1-oxo-1,2-dihydroisoquinolin-6-yl group, cinnolin-6-yl group, or benzoxazol-5-yl group; and
Y represents hydrogen atom, methyl group, or ethyl group.

In a still other particularly preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a single bond;
the bond between the ring-constituting carbon atom at the 1-position of the ring (E) and the Link moiety represents a single bond, or a double bond;
when the bond between the ring-constituting carbon atom at the 1-position of the ring (E) and the Link moiety represents a single bond, Link represents CH₂, and when the bond between the ring-constituting carbon atom at the 1-position of the ring (E) and the Link moiety represents a double bond, Link represents CH;
W represents methylene group;
Rs represents -O-Rx;
Rx represents propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group;
the substituent V¹ on the ring (E) represents Zx, and V² on the ring (E) represents AR;
Zx represents hydrogen atom, fluorine atom, chlorine atom, bromine atom, methyl group, hydroxyl group, amino group, or N-methylamino group;
AR represents a group which is naphthalen-2-yl group, 6-hydroxynaphthalen-2-yl group, 6-methoxynaphthalen-2-yl group, 6-(2-hydroxyethyloxy)naphthalen-2-yl group, 6-aminonaphthalen-2-yl group, 6-(N-methylamino)naphthalen-2-yl group, 6-(N,N-dimethylamino)naphthalen-2-yl group, 6-(2-hydroxyethylamino)naphthalen-2-yl group, benzo[b]furan-5-yl group, 2-methylbenzo[b]furan-5-yl group, 3-methylbenzo[b]furan-5-yl group, 2,3-dimethylbenzo[b]furan-5-yl group, benzo[b]thiophen-5-yl group, 2-methylbenzo[b]thiophen-5-yl group, 3-methylbenzo[b]thiophen-5-yl group, 2,3-dimethylbenzo[b]thiophen-5-yl group, 1H-indol-5-yl group, 2-methyl-1H-indol-5-yl group, 3-methyl-1H-indol-5-yl group, 2,3-dimethyl-1H-indol-5-yl group, 1-methyl-1H-indol-5-yl group, 1,2-dimethyl-1H-indol-5-yl group, 1,3-dimethyl-1H-indol-5-yl group, 1,2,3-trimethyl-1H-indol-5-yl group, 1-ethyl-1H-indol-5-yl group, 1-ethyl-2-methyl-1H-indol-5-yl group, 1-ethyl-3-methyl-1H-indol-5-yl group, 1-ethyl-2,3-dimethyl-1H-indol-5-yl group, 1-propyl-1H-indol-5-yl group, 2-methyl-1-propyl-1H-indol-5-yl group, 3-methyl-1-propyl-1H-indol-5-yl group, 2,3-dimethyl-1-propyl-1H-indol-5-yl group, 1-(2-hydroxyethyl)-1H-indol-5-yl group, 1-(2-hydroxyethyl)-2-methyl-1H-indol-5-yl group, 1-(2-hydroxyethyl)-3-methyl-1H-indol-5-yl group, 2,3-dimethyl-1-(2-hydroxyethyl)-1H-indol-5-yl group, benzothiazol-6-yl group, 2-methylbenzothiazol-6-yl group, 2-methoxybenzothiazol-6-yl group, 2-aminobenzothiazol-6-yl group, 2-oxo-2,3-dihydrobenzothiazol-6-yl group, 2-oxo-3-methyl-2,3-dihydrobenzothiazol-6-yl group, 2-thioxo-2,3-dihydrobenzothiazol-6-yl group, 2-thioxo-3-methyl-2,3-dihydrobenzothiazol-6-yl group, quinolin-3-yl group, quinolin-6-yl group, 2-oxo-1,2-dihydroquinolin-6-yl group, benzo[d]isothiazol-5-yl group, 1H-indazol-5-yl group, 1-methyl-1H-indazol-5-yl group, 1-ethyl-1H-indazol-5-yl group, 1-propyl-1H-indazol-5-yl group, 1-(2-hydroxyethyl)-1H-indazol-5-yl group, 3-hydroxy-1H-indazol-5-yl group, 3-hydroxy-1-methyl-1H-indazol-5-yl group, 1-ethyl-3-hydroxy-1H-indazol-5-yl group, imidazo[1,2-a]pyridin-6-yl group, 1H-pyrrolo[2,3-b]pyridin-5-yl group, 1-methyl-1H-pyrrolo[2,3-b]pyridin-5-yl group, 1-ethyl-1H-pyrrolo[2,3-b]pyridin-5-yl group, 1-propyl-1H-pyrrolo[2,3-b]pyridin-5-yl group, 1-(2-hydroxyethyl)-1H-pyrrolo[2,3-b]pyridin-5-yl group, isoquinolin-6-yl group, 1-oxo-1,2-dihydroisoquinolin-6-yl group, cinnolin-6-yl group, or benzoxazol-5-yl group; and
Y represents hydrogen atom, methyl group, or ethyl group.

In a still other particularly preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a single bond;
the bond between the ring-constituting carbon atom at the 1-position of the ring (E) and the Link moiety represents a single bond, or a double bond;
when the bond between the ring-constituting carbon atom at the 1-position of the ring (E) and the Link moiety represents a single bond, Link represents CH₂, and when the bond between the ring-constituting carbon atom at the 1-position of the ring (E) and the Link moiety represents a double bond, Link represents CH;
W represents methylene group;
Rs represents -N(Ry)(Rz);
the substituent -N(Ry)(Rz) represents N,N-dimethylamino group, N-ethyl-N-methylamino group, N,N-diethylamino group, N-methyl-N-propylamino group, N-ethyl-N-propylamino group, N-isopropyl-N-methylamino group, N-ethyl-N-isopropylamino group, N-butylamino group, N-butyl-N-methylamino group, N-butyl-N-ethylamino group, N-isobutylamino group, N-isobutyl-N-methylamino group, N-ethyl-N-isobutylamino group, N-isopentylamino group, N-isopentyl-N-methylamino group, N-ethyl-N-isopentylamino group, N-(2-ethylbutyl)amino group, N-(2-ethylbutyl)-N-methylamino group, N-cyclopentylamino group, N-cyclopentyl-N-methylamino group, N-cyclohexylamino group, N-cyclohexyl-N-methylamino group, N-cycloheptylamino group, N-(cyclopentylmethyl)amino group, N-(cyclopentylmethyl)-N-methylamino group, N-(cyclohexylmethyl)amino group, N-(cyclohexylmethyl)-N-methylamino group, N-phenylamino group, N-(2-methylphenyl)amino group, N-(4-methylphenyl)amino group, N-(2-fluorophenyl)amino group, N-(3-fluorophenyl)amino group, N-(4-fluorophenyl)amino group, N-(2-chlorophenyl)amino group, N-(3-chlorophenyl)amino group, N-(4-chlorophenyl)amino group, N-(indan-2-yl)amino group, N-(1-phenylethyl)amino group, N-[1-(2-fluorophenyl)ethyl]amino group, N-[1-(3-fluorophenyl)ethyl]amino group, N-[1-(4-fluorophenyl)ethyl]amino group, N-[1-(2-chlorophenyl)ethyl]amino group, N-[1-(3-chlorophenyl)ethyl]amino group, N-[1-(4-chlorophenyl)ethyl]amino group, N-benzylamino group, N-benzyl-N-methylamino group, N-benzyl-N-ethylamino group, N-(2-methylphenylmethyl)amino group, N-methyl-N-(2-methylphenylmethyl)amino group, N-(3-methylphenylmethyl)amino group, N-methyl-N-(3-methylphenylmethyl)amino group, N-(4-methylphenylmethyl)amino group, N-methyl-N-(4-methylphenylmethyl)amino group, N-(2-fluorophenylmethyl)amino group, N-(2-fluorophenylmethyl)-N-methylamino group, N-(3-fluorophenylmethyl)amino group, N-(3-fluorophenylmethyl)-N-methylamino group, N-(4-fluorophenylmethyl)amino group, N-(4-fluorophenylmethyl)-N-methylamino group, N-(2-chlorophenylmethyl)amino group, N-(2-chlorophenylmethyl)-N-methylamino group, N-(3-chlorophenylmethyl)amino group, N-(3-chlorophenylmethyl)-N-methylamino group, N-(4-chlorophenylmethyl)amino group, N-(4-chlorophenylmethyl)-N-methylamino group, N-(2,3-difluorophenylmethyl)amino group, N-(2,3-difluorophenylmethyl)-N-methylamino group, N-(2,4-difluorophenylmethyl)amino group, N-(2,4-difluorophenylmethyl)-N-methylamino group, N-(2,5-difluorophenylmethyl)amino group, N-(2,5-difluorophenylmethyl)-N-methylamino group, N-(3,4-difluorophenylmethyl)amino group, N-(3,4-difluorophenylmethyl)-N-methylamino group, N-(3,5-difluorophenylmethyl)amino group, N-(3,5-difluorophenylmethyl)-N-methylamino group, N-(2,3-dichlorophenylmethyl)amino group, N-(2,3-dichlorophenylmethyl)-N-methylamino group, N-(2,4-dichlorophenylmethyl)amino group, N-(2,4-dichlorophenylmethyl)-N-methylamino group, N-(2,5-dichlorophenylmethyl)amino group, N-(2,5-dichlorophenylmethyl)-N-methylamino group, N-(2,6-dichlorophenylmethyl)amino group, N-(2,6-dichlorophenylmethyl)-N-methylamino group, N-(3,4-dichlorophenylmethyl)amino group, N-(3,4-dichlorophenylmethyl)-N-methylamino group, N-(3,5-dichlorophenylmethyl)amino group, N-(3,5-dichlorophenylmethyl)-N-methylamino group, N-[2-(trifluoromethyl)phenylmethyl]amino group, N-methyl-N-[2-(trifluoromethyl)phenylmethyl]amino group, N-[3-(trifluoromethyl)phenylmethyl]amino group, N-methyl-N-[3-(trifluoromethyl)phenylmethyl]amino group, N-[4-(trifluoromethyl)phenylmethyl]amino group, N-methyl-N-[4-(trifluoromethyl)phenylmethyl]amino group, pyrrolidino group, 2-methylpyrrolidino group, 3-methylpyrrolidino group, 2,5-dimethylpyrrolidino group, 3,4-dimethylpyrrolidino group, piperidino group, 2-methylpiperidino group, 3-methylpiperidino group, 4-methylpiperidino group, 4-ethylpiperidino group, 4-propylpiperidino group, 4-isopropylpiperidino group, 4-butylpiperidino group, 4-isobutylpiperidino group, 4-cyclopentylpiperidino group, 4-cyclohexylpiperidino group, 4-(cyclopentylmethyl)piperidino group, 4-(cyclohexylmethyl)piperidino group, 4-phenylpiperidino group, 4-(2-methylphenyl)piperidino group, 4-(4-methylphenyl)piperidino group, 4-(2-fluorophenyl)piperidino group, 4-(3-fluorophenyl)piperidino group, 4-(4-fluorophenyl)piperidino group, 4-(2-chlorophenyl)piperidino group, 4-(3-chlorophenyl)piperidino group, 4-(4-chlorophenyl)piperidino group, 4-benzylpiperidino group, 4-(2-methylphenylmethyl)piperidino group, 4-(3-methylphenylmethyl)piperidino group, 4-methylphenylmethyl)piperidino group, 4-(2,3-dimethylphenylmethyl)piperidino group, 4-(3,5-dimethylphenylmethyl)piperidino group, 4-(2-fluorophenylmethyl)piperidino group, 4-(3-fluorophenylmethyl)piperidino group, 4-(4-fluorophenylmethyl)piperidino group, 4-(2-chlorophenylmethyl)piperidino group, 4-(3-chlorophenylmethyl)piperidino group, 4-(4-chlorophenylmethyl)piperidino group, 4-(2,3-difluorophenylmethyl)piperidino group, 4-(2,4-difluorophenylmethyl)piperidino group, 4-(2,5-difluorophenylmethyl)piperidino group, 4-(3,4-difluorophenylmethyl)piperidino group, 4-(2,3-dichlorophenylmethyl)piperidino group, 4-(2,4-dichlorophenylmethyl)piperidino group, 4-(2,5-dichlorophenylmethyl)piperidino group, 4-(2,6-dichlorophenylmethyl)piperidino group, 4-(3,4-dichlorophenylmethyl)piperidino group, 4-(3,5-dichlorophenylmethyl)piperidino group, 4-(3,6-dichlorophenylmethyl)piperidino group, 4-(2-methoxyphenylmethyl)piperidino group, 4-(3-methoxyphenylmethyl)piperidino group, 4-(4-methoxyphenylmethyl)piperidino group, 2,6-dimethylpiperidino group, 3,5-dimethylpiperidino group, 4,4-dimethylpiperidino group, homopiperidino group, morpholino group, 3,5-dimethylmorpholino group, 4-phenylpiperazino group, 4-(2-methylphenyl)piperazino group, 4-(4-methylphenyl)piperazino group, 4-(2-fluorophenyl)piperazino group, 4-(3-fluorophenyl)piperazino group, 4-(4-fluorophenyl)piperazino group, 4-(2-chlorophenyl)piperazino group, 4-(3-chlorophenyl)piperazino group, 4-(4-chlorophenyl)piperazino group, 4-benzylpiperazino group, 4-(2-methylphenylmethyl)piperazino group, 4-(3-methylphenylmethyl)piperazino group, 4-methylphenylmethyl)piperazino group, 4-(2,3-dimethylphenylmethyl)piperazino group, 4-(3,5-dimethylphenylmethyl)piperazino group, 4-(2-fluorophenylmethyl)piperazino group, 4-(3-fluorophenylmethyl)piperazino group, 4-(4-fluorophenylmethyl)piperazino group, 4-(2-chlorophenylmethyl)piperazino group, 4-(3-chlorophenylmethyl)piperazino group, 4-(4-chlorophenylmethyl)piperazino group, 4-(2,3-difluorophenylmethyl)piperazino group, 4-(2,4-difluorophenylmethyl)piperazino group, 4-(2,5-difluorophenylmethyl)piperazino group, 4-(3,4-difluorophenylmethyl)piperazino group, 4-(2,3-dichlorophenylmethyl)piperazino group, 4-(2,4-dichlorophenylmethyl)piperazino group, 4-(2,5-dichlorophenylmethyl)piperazino group, 4-(2,6-dichlorophenylmethyl)piperazino group, 4-(3,4-dichlorophenylmethyl)piperazino group, 4-(3,5-dichlorophenylmethyl)piperazino group, 4-(3,6-dichlorophenylmethyl)piperazino group, 4-(2-methoxyphenylmethyl)piperazino group, 4-(3-methoxyphenylmethyl)piperazino group, or 4-(4-methoxyphenylmethyl)piperazino group;
the substituent V¹ on the ring (E) represents Zx, and V² on the ring (E) represents AR;
Zx represents hydrogen atom, fluorine atom, chlorine atom, bromine atom, methyl group, hydroxyl group, amino group, or N-methylamino group;
AR represents a group which is naphthalen-2-yl group, 6-hydroxynaphthalen-2-yl group, 6-methoxynaphthalen-2-yl group, 6-(2-hydroxyethyloxy)naphthalen-2-yl group, 6-aminonaphthalen-2-yl group, 6-(N-methylamino)naphthalen-2-yl group, 6-(N,N-dimethylamino)naphthalen-2-yl group, 6-(2-hydroxyethylamino)naphthalen-2-yl group, benzo[b]furan-5-yl group, 2-methylbenzo[b]furan-5-yl group, 3-methylbenzo[b]furan-5-yl group, 2,3-dimethylbenzo[b]furan-5-yl group, benzo[b]thiophen-5-yl group, 2-methylbenzo[b]thiophen-5-yl group, 3-methylbenzo[b]thiophen-5-yl group, 2,3-dimethylbenzo[b]thiophen-5-yl group, 1H-indol-5-yl group, 2-methyl-1H-indol-5-yl group, 3-methyl-1H-indol-5-yl group, 2,3-dimethyl-1H-indol-5-yl group, 1-methyl-1H-indol-5-yl group, 1,2-dimethyl-1H-indol-5-yl group, 1,3-dimethyl-1H-indol-5-yl group, 1,2,3-trimethyl-1H-indol-5-yl group, 1-ethyl-1H-indol-5-yl group, 1-ethyl-2-methyl-1H-indol-5-yl group, 1-ethyl-3-methyl-1H-indol-5-yl group, 1-ethyl-2,3-dimethyl-1H-indol-5-yl group, 1-propyl-1H-indol-5-yl group, 2-methyl-1-propyl-1H-indol-5-yl group, 3-methyl-1-propyl-1H-indol-5-yl group, 2,3-dimethyl-1-propyl-1H-indol-5-yl group, 1-(2-hydroxyethyl)-1H-indol-5-yl group, 1-(2-hydroxyethyl)-2-methyl-1H-indol-5-yl group, 1-(2-hydroxyethyl)-3-methyl-1H-indol-5-yl group, 2,3-dimethyl-1-(2-hydroxyethyl)-1H-indol-5-yl group, benzothiazol-6-yl group, 2-methylbenzothiazol-6-yl group, 2-methoxybenzothiazol-6-yl group, 2-aminobenzothiazol-6-yl group, 2-oxo-2,3-dihydrobenzothiazol-6-yl group, 2-oxo-3-methyl-2,3-dihydrobenzothiazol-6-yl group, 2-thioxo-2,3-dihydrobenzothiazol-6-yl group, 2-thioxo-3-methyl-2,3-dihydrobenzothiazol-6-yl group, quinolin-3-yl group, quinolin-6-yl group, 2-oxo-1,2-dihydroquinolin-6-yl group, benzo[d]isothiazol-5-yl group, 1H-indazol-5-yl group, 1-methyl-1H-indazol-5-yl group, 1-ethyl-1H-indazol-5-yl group, 1-propyl-1H-indazol-5-yl group, 1-(2-hydroxyethyl)-1H-indazol-5-yl group, 3-hydroxy-1H-indazol-5-yl group, 3-hydroxy-1-methyl-1H-indazol-5-yl group, 1-ethyl-3-hydroxy-1H-indazol-5-yl group, imidazo[1,2-a]pyridin-6-yl group, 1H-pyrrolo[2,3-b]pyridin-5-yl group, 1-methyl-1H-pyrrolo[2,3-b]pyridin-5-yl group, 1-ethyl-1H-pyrrolo[2,3-b]pyridin-5-yl group, 1-propyl-1H-pyrrolo[2,3-b]pyridin-5-yl group, 1-(2-hydroxyethyl)-1H-pyrrolo[2,3-b]pyridin-5-yl group, isoquinolin-6-yl group, 1-oxo-1,2-dihydroisoquinolin-6-yl group, cinnolin-6-yl group, or benzoxazol-5-yl group; and
Y represents hydrogen atom, methyl group, or ethyl group.

In a still other particularly preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a single bond;
the bond between the ring-constituting carbon atom at the 1-position of the ring (E) and the Link moiety represents a single bond, or a double bond;
when the bond between the ring-constituting carbon atom at the 1-position of the ring (E) and the Link moiety represents a single bond, Link represents CH₂, and when the bond between the ring-constituting carbon atom at the 1-position of the ring (E) and the Link moiety represents a double bond, Link represents CH;
W represents methylene group;
Rs represents phenyl group, 2-methylphenyl group, 3-methylphenyl group, 4-methylphenyl group, 2,3-dimethylphenyl group, 3,5-dimethylphenyl group, 2-methoxyphenyl group, 3-methoxyphenyl group, 4-methoxyphenyl group, 2-fluorophenyl group, 3-fluorophenyl group, 4-fluorophenyl group, 2-chlorophenyl group, 3-chlorophenyl group, 4-chlorophenyl group, 2,3-difluorophenyl group, 2,4-difluorophenyl group, 2,5-difluorophenyl group, 3,4-difluorophenyl group, 2,3-dichlorophenyl group, 2,4-dichlorophenyl group, 2,5-dichlorophenyl group, 2,6-dichlorophenyl group, 3,4-dichlorophenyl group, 3,5-dichlorophenyl group, 2-trifluoromethylphenyl group, 3-trifluoromethylphenyl group, 4-trifluoromethylphenyl group, 4-(N,N-dimethylamino)phenyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-methoxyindan-2-yl group, 5-methoxyindan-2-yl group, 4,7-dimethoxyindan-2-yl group, 5,6-dimethoxyindan-2-yl group, furan-2-yl group, furan-3-yl group, thiophen-2-yl group, or thiophen-3-yl group;
the substituent V¹ on the ring (E) represents Zx, and V² on the ring (E) represents AR;
Zx represents hydrogen atom, fluorine atom, chlorine atom, bromine atom, methyl group, hydroxyl group, amino group, or N-methylamino group;
AR represents a group which is naphthalen-2-yl group, 6-hydroxynaphthalen-2-yl group, 6-methoxynaphthalen-2-yl group, 6-(2-hydroxyethyloxy)naphthalen-2-yl group, 6-aminonaphthalen-2-yl group, 6-(N-methylamino)naphthalen-2-yl group, 6-(N,N-dimethylamino)naphthalen-2-yl group, 6-(2-hydroxyethylamino)naphthalen-2-yl group, benzo[b]furan-5-yl group, 2-methylbenzo[b]furan-5-yl group, 3-methylbenzo[b]furan-5-yl group, 2,3-dimethylbenzo[b]furan-5-yl group, benzo[b]thiophen-5-yl group, 2-methylbenzo[b]thiophen-5-yl group, 3-methylbenzo[b]thiophen-5-yl group, 2,3-dimethylbenzo[b]thiophen-5-yl group, 1H-indol-5-yl group, 2-methyl-1H-indol-5-yl group, 3-methyl-1H-indol-5-yl group, 2,3-dimethyl-1H-indol-5-yl group, 1-methyl-1H-indol-5-yl group, 1,2-dimethyl-1H-indol-5-yl group, 1,3-dimethyl-1H-indol-5-yl group, 1,2,3-trimethyl-1H-indol-5-yl group, 1-ethyl-1H-indol-5-yl group, 1-ethyl-2-methyl-1H-indol-5-yl group, 1-ethyl-3-methyl-1H-indol-5-yl group, 1-ethyl-2,3-dimethyl-1H-indol-5-yl group, 1-propyl-1H-indol-5-yl group, 2-methyl-1-propyl-1H-indol-5-yl group, 3-methyl-1-propyl-1H-indol-5-yl group, 2,3-dimethyl-1-propyl-1H-indol-5-yl group, 1-(2-hydroxyethyl)-1H-indol-5-yl group, 1-(2-hydroxyethyl)-2-methyl-1H-indol-5-yl group, 1-(2-hydroxyethyl)-3-methyl-1H-indol-5-yl group, 2,3-dimethyl-1-(2-hydroxyethyl)-1H-indol-5-yl group, benzothiazol-6-yl group, 2-methylbenzothiazol-6-yl group, 2-methoxybenzothiazol-6-yl group, 2-aminobenzothiazol-6-yl group, 2-oxo-2,3-dihydrobenzothiazol-6-yl group, 2-oxo-3-methyl-2,3-dihydrobenzothiazol-6-yl group, 2-thioxo-2,3-dihydrobenzothiazol-6-yl group, 2-thioxo-3-methyl-2,3-dihydrobenzothiazol-6-yl group, quinolin-3-yl group, quinolin-6-yl group, 2-oxo-1,2-dihydroquinolin-6-yl group, benzo[d]isothiazol-5-yl group, 1H-indazol-5-yl group, 1-methyl-1H-indazol-5-yl group, 1-ethyl-1H-indazol-5-yl group, 1-propyl-1H-indazol-5-yl group, 1-(2-hydroxyethyl)-1H-indazol-5-yl group, 3-hydroxy-1H-indazol-5-yl group, 3-hydroxy-1-methyl-1H-indazol-5-yl group, 1-ethyl-3-hydroxy-1H-indazol-5-yl group, imidazo[1,2-a]pyridin-6-yl group, 1H-pyrrolo[2,3-b]pyridin-5-yl group, 1-methyl-1H-pyrrolo[2,3-b]pyridin-5-yl group, 1-ethyl-1H-pyrrolo[2,3-b]pyridin-5-yl group, 1-propyl-1H-pyrrolo[2,3-b]pyridin-5-yl group, 1-(2-hydroxyethyl)-1H-pyrrolo[2,3-b]pyridin-5-yl group, isoquinolin-6-yl group, 1-oxo-1,2-dihydroisoquinolin-6-yl group, cinnolin-6-yl group, or benzoxazol-5-yl group; and
Y represents hydrogen atom, methyl group, or ethyl group.

In an extremely preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a single bond;
the bond between the ring-constituting carbon atom at the 1-position of the ring (E) and the Link moiety represents a single bond;
Link represents CH₂;
W represents methylene group;
Rs represents -D-Rx or -N(Ry)(Rz);
D represents a single bond, or oxygen atom;
Rx represents a substituent which is propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, or cycloheptylmethyl group, or Rb mentioned above;
Q in Rb represents phenyl group, 2-thienyl group, 3-thienyl group, 2-furyl group, 3-furyl group, or indan-2-yl group;
A² in Rb represents a single bond, oxygen atom, sulfur atom, -N(methyl)-, or - N(ethyl)-;
A¹ in Rb represents a single bond, or methylene group, methylmethylene group, or ethylene group when A² represents a single bond, or ethylene group when A² represents oxygen atom, sulfur atom, -N(methyl)-, or -N(ethyl)-;
R² and R³ independently represent hydrogen atom, methyl group, fluorine atom, chlorine atom, trifluoromethyl group, hydroxyl group, methoxy group, or dimethylamino group;
Rz has the same meaning as that of Rx, or represents methyl group, or ethyl group;
Ry represents hydrogen atom, methyl group, ethyl group, propyl group, isopropyl group, butyl group, isobutyl group, pentyl group, or isopentyl group (provided that when Rz represents methyl group, or ethyl group, Ry represents a substituent other than hydrogen atom), or may bind to Rz to form, together with the nitrogen atom, a cyclic substituent (qy) selected from pyrrolidino group, piperidino group, morpholino group, and piperazino group, and the cyclic substituent (qy) may be substituted with one substituent or two of the same or different substituents selected from methyl group, ethyl group, propyl group, isopropyl group, butyl group, isobutyl group, cyclopentyl group, cyclohexyl group, cyclopentylmethyl group, cyclohexylmethyl group, phenyl group, and phenylmethyl group (provided that the benzene ring of the phenyl group, or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group);
the substituent V¹ on the ring (E) represents Zx, and V² on the ring (E) represents AR;
Zx represents hydrogen atom, fluorine atom, chlorine atom, bromine atom, methyl group, hydroxyl group, amino group, or N-methylamino group;
AR represents a substituent which is naphthalen-2-yl group, benzofuran-5-yl group, benzo[b]thiophen-5-yl group, indol-5-yl group, benzothiazol-6-yl group, quinolin-6-yl group, quinolin-3-yl group, 1H-indazol-5-yl group, isoquinolin-6-yl group, cinnolin-6-yl group, or benzoxazol-5-yl group (the aforementioned groups may be substituted with one of Xa or two or more of the same or different Xa);
Xa represents oxo group, methyl group, ethyl group, propyl group, 2-hydroxyethyl group, hydroxyl group, methoxy group, 2-hydroxyethyloxy group, amino group, N-methylamino group, N,N-dimethylamino group, 2-hydroxyethylamino group, or carboxyl group; and
Y represents hydrogen atom, methyl group, or ethyl group.

In another extremely preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a single bond;
the bond between the ring-constituting carbon atom at the 1-position of the ring (E) and the Link moiety represents a single bond;
Link represents CH₂;
W represents methylene group;
Rs represents -D-Rx or -N(Ry)(Rz);
D represents a single bond, or oxygen atom;
Rx represents a group which is propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group;
the substituent -N(Ry)(Rz) represents N,N-dimethylamino group, N-ethyl-N-methylamino group, N,N-diethylamino group, N-methyl-N-propylamino group, N-ethyl-N-propylamino group, N-isopropyl-N-methylamino group, N-ethyl-N-isopropylamino group, N-butylamino group, N-butyl-N-methylamino group, N-butyl-N-ethylamino group, N-isobutylamino group, N-isobutyl-N-methylamino group, N-ethyl-N-isobutylamino group, N-isopentylamino group, N-isopentyl-N-methylamino group, N-ethyl-N-isopentylamino group, N-(2-ethylbutyl)amino group, N-(2-ethylbutyl)-N-methylamino group, N-cyclopentylamino group, N-cyclopentyl-N-methylamino group, N-cyclohexylamino group, N-cyclohexyl-N-methylamino group, N-cycloheptylamino group, N-(cyclopentylmethyl)amino group, N-(cyclopentylmethyl)-N-methylamino group, N-(cyclohexylmethyl)amino group, N-(cyclohexylmethyl)-N-methylamino group, N-phenylamino group, N-(2-methylphenyl)amino group, N-(4-methylphenyl)amino group, N-(2-fluorophenyl)amino group, N-(3-fluorophenyl)amino group, N-(4-fluorophenyl)amino group, N-(2-chlorophenyl)amino group, N-(3-chlorophenyl)amino group, N-(4-chlorophenyl)amino group, N-(indan-2-yl)amino group, N-(1-phenylethyl)amino group, N-[1-(2-fluorophenyl)ethyl]amino group, N-[1-(3-fluorophenyl)ethyl]amino group, N-[1-(4-fluorophenyl)ethyl]amino group, N-[1-(2-chlorophenyl)ethyl]amino group, N-[1-(3-chlorophenyl)ethyl]amino group, N-[1-(4-chlorophenyl)ethyl]amino group, N-benzylamino group, N-benzyl-N-methylamino group, N-benzyl-N-ethylamino group, N-(2-methylphenylmethyl)amino group, N-methyl-N-(2-methylphenylmethyl)amino group, N-(3-methylphenylmethyl)amino group, N-methyl-N-(3-methylphenylmethyl)amino group, N-(4-methylphenylmethyl)amino group, N-methyl-N-(4-methylphenylmethyl)amino group, N-(2-fluorophenylmethyl)amino group, N-(2-fluorophenylmethyl)-N-methylamino group, N-(3-fluorophenylmethyl)amino group, N-(3-fluorophenylmethyl)-N-methylamino group, N-(4-fluorophenylmethyl)amino group, N-(4-fluorophenylmethyl)-N-methylamino group, N-(2-chlorophenylmethyl)amino group, N-(2-chlorophenylmethyl)-N-methylamino group, N-(3-chlorophenylmethyl)amino group, N-(3-chlorophenylmethyl)-N-methylamino group, N-(4-chlorophenylmethyl)amino group, N-(4-chlorophenylmethyl)-N-methylamino group, N-(2,3-difluorophenylmethyl)amino group, N-(2,3-difluorophenylmethyl)-N-methylamino group, N-(2,4-difluorophenylmethyl)amino group, N-(2,4-difluorophenylmethyl)-N-methylamino group, N-(2,5-difluorophenylmethyl)amino group, N-(2,5-difluorophenylmethyl)-N-methylamino group, N-(3,4-difluorophenylmethyl)amino group, N-(3,4-difluorophenylmethyl)-N-methylamino group, N-(3,5-difluorophenylmethyl)amino group, N-(3,5-difluorophenylmethyl)-N-methylamino group, N-(2,3-dichlorophenylmethyl)amino group, N-(2,3-dichlorophenylmethyl)-N-methylamino group, N-(2,4-dichlorophenylmethyl)amino group, N-(2,4-dichlorophenylmethyl)-N-methylamino group, N-(2,5-dichlorophenylmethyl)amino group, N-(2,5-dichlorophenylmethyl)-N-methylamino group, N-(2,6-dichlorophenylmethyl)amino group, N-(2,6-dichlorophenylmethyl)-N-methylamino group, N-(3,4-dichlorophenylmethyl)amino group, N-(3,4-dichlorophenylmethyl)-N-methylamino group, N-(3,5-dichlorophenylmethyl)amino group, N-(3,5-dichlorophenylmethyl)-N-methylamino group, N-[2-(trifluoromethyl)phenylmethyl]amino group, N-methyl-N-[2-(trifluoromethyl)phenylmethyl]amino group, N-[3-(trifluoromethyl)phenylmethyl]amino group, N-methyl-N-[3-(trifluoromethyl)phenylmethyl]amino group, N-[4-(trifluoromethyl)phenylmethyl]amino group, N-methyl-N-[4-(trifluoromethyl)phenylmethyl]amino group, pyrrolidino group, 2-methylpyrrolidino group, 3-methylpyrrolidino group, 2,5-dimethylpyrrolidino group, 3,4-dimethylpyrrolidino group, piperidino group, 2-methylpiperidino group, 3-methylpiperidino group, 4-methylpiperidino group, 4-ethylpiperidino group, 4-propylpiperidino group, 4-isopropylpiperidino group, 4-butylpiperidino group, 4-isobutylpiperidino group, 4-cyclopentylpiperidino group, 4-cyclohexylpiperidino group, 4-(cyclopentylmethyl)piperidino group, 4-(cyclohexylmethyl)piperidino group, 4-phenylpiperidino group, 4-(2-methylphenyl)piperidino group, 4-(4-methylphenyl)piperidino group, 4-(2-fluorophenyl)piperidino group, 4-(3-fluorophenyl)piperidino group, 4-(4-fluorophenyl)piperidino group, 4-(2-chlorophenyl)piperidino group, 4-(3-chlorophenyl)piperidino group, 4-(4-chlorophenyl)piperidino group, 4-benzylpiperidino group, 4-(2-methylphenylmethyl)piperidino group, 4-(3-methylphenylmethyl)piperidino group, 4-methylphenylmethyl)piperidino group, 4-(2,3-dimethylphenylmethyl)piperidino group, 4-(3,5-dimethylphenylmethyl)piperidino group, 4-(2-fluorophenylmethyl)piperidino group, 4-(3-fluorophenylmethyl)piperidino group, 4-(4-fluorophenylmethyl)piperidino group, 4-(2-chlorophenylmethyl)piperidino group, 4-(3-chlorophenylmethyl)piperidino group, 4-(4-chlorophenylmethyl)piperidino group, 4-(2,3-difluorophenylmethyl)piperidino group, 4-(2,4-difluorophenylmethyl)piperidino group, 4-(2,5-difluorophenylmethyl)piperidino group, 4-(3,4-difluorophenylmethyl)piperidino group, 4-(2,3-dichlorophenylmethyl)piperidino group, 4-(2,4-dichlorophenylmethyl)piperidino group, 4-(2,5-dichlorophenylmethyl)piperidino group, 4-(2,6-dichlorophenylmethyl)piperidino group, 4-(3,4-dichlorophenylmethyl)piperidino group, 4-(3,5-dichlorophenylmethyl)piperidino group, 4-(3,6-dichlorophenylmethyl)piperidino group, 4-(2-methoxyphenylmethyl)piperidino group, 4-(3-methoxyphenylmethyl)piperidino group, 4-(4-methoxyphenylmethyl)piperidino group, 2,6-dimethylpiperidino group, 3,5-dimethylpiperidino group, 4,4-dimethylpiperidino group, homopiperidino group, morpholino group, 3,5-dimethylmorpholino group, 4-phenylpiperazino group, 4-(2-methylphenyl)piperazino group, 4-(4-methylphenyl)piperazino group, 4-(2-fluorophenyl)piperazino group, 4-(3-fluorophenyl)piperazino group, 4-(4-fluorophenyl)piperazino group, 4-(2-chlorophenyl)piperazino group, 4-(3-chlorophenyl)piperazino group, 4-(4-chlorophenyl)piperazino group, 4-benzylpiperazino group, 4-(2-methylphenylmethyl)piperazino group, 4-(3-methylphenylmethyl)piperazino group, 4-methylphenylmethyl)piperazino group, 4-(2,3-dimethylphenylmethyl)piperazino group, 4-(3,5-dimethylphenylmethyl)piperazino group, 4-(2-fluorophenylmethyl)piperazino group, 4-(3-fluorophenylmethyl)piperazino group, 4-(4-fluorophenylmethyl)piperazino group, 4-(2-chlorophenylmethyl)piperazino group, 4-(3-chlorophenylmethyl)piperazino group, 4-(4-chlorophenylmethyl)piperazino group, 4-(2,3-difluorophenylmethyl)piperazino group, 4-(2,4-difluorophenylmethyl)piperazino group, 4-(2,5-difluorophenylmethyl)piperazino group, 4-(3,4-difluorophenylmethyl)piperazino group, 4-(2,3-dichlorophenylmethyl)piperazino group, 4-(2,4-dichlorophenylmethyl)piperazino group, 4-(2,5-dichlorophenylmethyl)piperazino group, 4-(2,6-dichlorophenylmethyl)piperazino group, 4-(3,4-dichlorophenylmethyl)piperazino group, 4-(3,5-dichlorophenylmethyl)piperazino group, 4-(3,6-dichlorophenylmethyl)piperazino group, 4-(2-methoxyphenylmethyl)piperazino group, 4-(3-methoxyphenylmethyl)piperazino group, or 4-(4-methoxyphenylmethyl)piperazino group;
the substituent V¹ on the ring (E) represents Zx, and V² on the ring (E) represents AR;
Zx represents hydrogen atom, fluorine atom, chlorine atom, bromine atom, methyl group, hydroxyl group, amino group, or N-methylamino group;
AR represents naphthalen-2-yl group, 6-hydroxynaphthalen-2-yl group, 6-methoxynaphthalen-2-yl group, 6-aminonaphthalen-2-yl group, 6-(N,N-dimethylamino)naphthalen-2-yl group, benzo[b]furan-5-yl group, benzo[b]thiophen-5-yl group, 1H-indol-5-yl group, 1-methyl-1H-indol-5-yl group, 1-ethyl-1H-indol-5-yl group, benzothiazol-6-yl group, 2-aminobenzothiazol-6-yl group, 2-oxo-2,3-dihydrobenzothiazol-6-yl group, quinolin-3-yl group, quinolin-6-yl group, 2-oxo-l,2-dihydroquinolin-6-yl group, 1H-indazol-5-yl group, 1-methyl-1H-indazol-5-yl group, 1-ethyl-1H-indazol-5-yl group, isoquinolin-6-yl group, 1-oxo-1,2-dihydroisoquinolin-6-yl group, cinnolin-6-yl group, or benzoxazol-5-yl group; and
Y represents hydrogen atom, methyl group, or ethyl group.

In a still other extremely preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a single bond;
the bond between the ring-constituting carbon atom at the 1-position of the ring (E) and the Link moiety represents a single bond;
Link represents CH₂;
W represents methylene group;
Rs represents propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group;
the substituent V¹ on the ring (E) represents Zx, and V² on the ring (E) represents AR;
Zx represents hydrogen atom, fluorine atom, chlorine atom, bromine atom, methyl group, hydroxyl group, amino group, or N-methylamino group;
AR represents naphthalen-2-yl group, 6-hydroxynaphthalen-2-yl group, 6-methoxynaphthalen-2-yl group, 6-aminonaphthalen-2-yl group, 6-(N,N-dimethylamino)naphthalen-2-yl group, benzo[b]furan-5-yl group, benzo[b]thiophen-5-yl group, 1H-indol-5-yl group, 1-methyl-1H-indol-5-yl group, 1-ethyl-1H-indol-5-yl group, benzothiazol-6-yl group, 2-aminobenzothiazol-6-yl group, 2-oxo-2,3-dihydrobenzothiazol-6-yl group, quinolin-3-yl group, quinolin-6-yl group, 2-oxo-1,2-dihydroquinolin-6-yl group, 1H-indazol-5-yl group, 1-methyl-1H-indazol-5-yl group, 1-ethyl-1H-indazol-5-yl group, isoquinolin-6-yl group, 1-oxo-1,2-dihydroisoquinolin-6-yl group, cinnolin-6-yl group, or benzoxazol-5-yl group; and
Y represents hydrogen atom, methyl group, or ethyl group.

In a still other extremely preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a single bond;
the bond between the ring-constituting carbon atom at the 1-position of the ring (E) and the Link moiety represents a single bond;
Link represents CH₂;
W represents methylene group;
Rs represents -O-Rx;
Rx represents propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, and 2-(N-ethyl-N-phenylamino)ethyl group;
the substituent V¹ on the ring (E) represents Zx, and V² on the ring (E) represents AR;
Zx represents hydrogen atom, fluorine atom, chlorine atom, bromine atom, methyl group, hydroxyl group, amino group, and N-methylamino group;
AR represents naphthalen-2-yl group, 6-hydroxynaphthalen-2-yl group, 6-methoxynaphthalen-2-yl group, 6-aminonaphthalen-2-yl group, 6-(N,N-dimethylamino)naphthalen-2-yl group, benzo[b]furan-5-yl group, benzo[b]thiophen-5-yl group, 1H-indol-5-yl group, 1-methyl-1H-indol-5-yl group, 1-ethyl-1H-indol-5-yl group, benzothiazol-6-yl group, 2-aminobenzothiazol-6-yl group, 2-oxo-2,3-dihydrobenzothiazol-6-yl group, quinolin-3-yl group, quinolin-6-yl group, 2-oxo-1,2-dihydroquinolin-6-yl group, 1H-indazol-5-yl group, 1-methyl-1H-indazol-5-yl group, 1-ethyl-1H-indazol-5-yl group, isoquinolin-6-yl group, 1-oxo-1,2-dihydroisoquinolin-6-yl group, cinnolin-6-yl group, or benzoxazol-5-yl group; and
Y represents hydrogen atom, methyl group, or ethyl group.

In a still other extremely preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a single bond;
the bond between the ring-constituting carbon atom at the 1-position of the ring (E) and the Link moiety represents a single bond;
Link represents CH₂;
W represents methylene group;
Rs represents -N(Ry)(Rz);
the substituent -N(Ry)(Rz) represents N,N-dimethylamino group, N-ethyl-N-methylamino group, N,N-diethylamino group, N-methyl-N-propylamino group, N-ethyl-N-propylamino group, N-isopropyl-N-methylamino group, N-ethyl-N-isopropylamino group, N-butylamino group, N-butyl-N-methylamino group, N-butyl-N-ethylamino group, N-isobutylamino group, N-isobutyl-N-methylamino group, N-ethyl-N-isobutylamino group, N-isopentylamino group, N-isopentyl-N-methylamino group, N-ethyl-N-isopentylamino group, N-(2-ethylbutyl)amino group, N-(2-ethylbutyl)-N-methylamino group, N-cyclopentylamino group, N-cyclopentyl-N-methylamino group, N-cyclohexylamino group, N-cyclohexyl-N-methylamino group, N-cycloheptylamino group, N-(cyclopentylmethyl)amino group, N-(cyclopentylmethyl)-N-methylamino group, N-(cyclohexylmethyl)amino group, N-(cyclohexylmethyl)-N-methylamino group, N-phenylamino group, N-(2-methylphenyl)amino group, N-(4-methylphenyl)amino group, N-(2-fluorophenyl)amino group, N-(3-fluorophenyl)amino group, N-(4-fluorophenyl)amino group, N-(2-chlorophenyl)amino group, N-(3-chlorophenyl)amino group, N-(4-chlorophenyl)amino group, N-(indan-2-yl)amino group, N-(1-phenylethyl)amino group, N-[1-(2-fluorophenyl)ethyl]amino group, N-[1-(3-fluorophenyl)ethyl]amino group, N-[1-(4-fluorophenyl)ethyl]amino group, N-[1-(2-chlorophenyl)ethyl]amino group, N-[1-(3-chlorophenyl)ethyl]amino group, N-[1-(4-chlorophenyl)ethyl]amino group, N-benzylamino group, N-benzyl-N-methylamino group, N-benzyl-N-ethylamino group, N-(2-methylphenylmethyl)amino group, N-methyl-N-(2-methylphenylmethyl)amino group, N-(3-methylphenylmethyl)amino group, N-methyl-N-(3-methylphenylmethyl)amino group, N-(4-methylphenylmethyl)amino group, N-methyl-N-(4-methylphenylmethyl)amino group, N-(2-fluorophenylmethyl)amino group, N-(2-fluorophenylmethyl)-N-methylamino group, N-(3-fluorophenylmethyl)amino group, N-(3-fluorophenylmethyl)-N-methylamino group, N-(4-fluorophenylmethyl)amino group, N-(4-fluorophenylmethyl)-N-methylamino group, N-(2-chlorophenylmethyl)amino group, N-(2-chlorophenylmethyl)-N-methylamino group, N-(3-chlorophenylmethyl)amino group, N-(3-chlorophenylmethyl)-N-methylamino group, N-(4-chlorophenylmethyl)amino group, N-(4-chlorophenylmethyl)-N-methylamino group, N-(2,3-difluorophenylmethyl)amino group, N-(2,3-difluorophenylmethyl)-N-methylamino group, N-(2,4-difluorophenylmethyl)amino group, N-(2,4-difluorophenylmethyl)-N-methylamino group, N-(2,5-difluorophenylmethyl)amino group, N-(2,5-difluorophenylmethyl)-N-methylamino group, N-(3,4-difluorophenylmethyl)amino group, N-(3,4-difluorophenylmethyl)-N-methylamino group, N-(3,5-difluorophenylmethyl)amino group, N-(3,5-difluorophenylmethyl)-N-methylamino group, N-(2,3-dichlorophenylmethyl)amino group, N-(2,3-dichlorophenylmethyl)-N-methylamino group, N-(2,4-dichlorophenylmethyl)amino group, N-(2,4-dichlorophenylmethyl)-N-methylamino group, N-(2,5-dichlorophenylmethyl)amino group, N-(2,5-dichlorophenylmethyl)-N-methylamino group, N-(2,6-dichlorophenylmethyl)amino group, N-(2,6-dichlorophenylmethyl)-N-methylamino group, N-(3,4-dichlorophenylmethyl)amino group, N-(3,4-dichlorophenylmethyl)-N-methylamino group, N-(3,5-dichlorophenylmethyl)amino group, N-(3,5-dichlorophenylmethyl)-N-methylamino group, N-[2-(trifluoromethyl)phenylmethyl]amino group, N-methyl-N-[2-(trifluoromethyl)phenylmethyl]amino group, N-[3-(trifluoromethyl)phenylmethyl]amino group, N-methyl-N-[3-(trifluoromethyl)phenylmethyl]amino group, N-[4-(trifluoromethyl)phenylmethyl]amino group, N-methyl-N-[4-(trifluoromethyl)phenylmethyl]amino group, pyrrolidino group, 2-methylpyrrolidino group, 3-methylpyrrolidino group, 2,5-dimethylpyrrolidino group, 3,4-dimethylpyrrolidino group, piperidino group, 2-methylpiperidino group, 3-methylpiperidino group, 4-methylpiperidino group, 4-ethylpiperidino group, 4-propylpiperidino group, 4-isopropylpiperidino group, 4-butylpiperidino group, 4-isobutylpiperidino group, 4-cyclopentylpiperidino group, 4-cyclohexylpiperidino group, 4-(cyclopentylmethyl)piperidino group, 4-(cyclohexylmethyl)piperidino group, 4-phenylpiperidino group, 4-(2-methylphenyl)piperidino group, 4-(4-methylphenyl)piperidino group, 4-(2-fluorophenyl)piperidino group, 4-(3-fluorophenyl)piperidino group, 4-(4-fluorophenyl)piperidino group, 4-(2-chlorophenyl)piperidino group, 4-(3-chlorophenyl)piperidino group, 4-(4-chlorophenyl)piperidino group, 4-benzylpiperidino group, 4-(2-methylphenylmethyl)piperidino group, 4-(3-methylphenylmethyl)piperidino group, 4-methylphenylmethyl)piperidino group, 4-(2,3-dimethylphenylmethyl)piperidino group, 4-(3,5-dimethylphenylmethyl)piperidino group, 4-(2-fluorophenylmethyl)piperidino group, 4-(3-fluorophenylmethyl)piperidino group, 4-(4-fluorophenylmethyl)piperidino group, 4-(2-chlorophenylmethyl)piperidino group, 4-(3-chlorophenylmethyl)piperidino group, 4-(4-chlorophenylmethyl)piperidino group, 4-(2,3-difluorophenylmethyl)piperidino group, 4-(2,4-difluorophenylmethyl)piperidino group, 4-(2,5-difluorophenylmethyl)piperidino group, 4-(3,4-difluorophenylmethyl)piperidino group, 4-(2,3-dichlorophenylmethyl)piperidino group, 4-(2,4-dichlorophenylmethyl)piperidino group, 4-(2,5-dichlorophenylmethyl)piperidino group, 4-(2,6-dichlorophenylmethyl)piperidino group, 4-(3,4-dichlorophenylmethyl)piperidino group, 4-(3,5-dichlorophenylmethyl)piperidino group, 4-(3,6-dichlorophenylmethyl)piperidino group, 4-(2-methoxyphenylmethyl)piperidino group, 4-(3-methoxyphenylmethyl)piperidino group, 4-(4-methoxyphenylmethyl)piperidino group, 2,6-dimethylpiperidino group, 3,5-dimethylpiperidino group, 4,4-dimethylpiperidino group, homopiperidino group, morpholino group, 3,5-dimethylmorpholino group, 4-phenylpiperazino group, 4-(2-methylphenyl)piperazino group, 4-(4-methylphenyl)piperazino group, 4-(2-fluorophenyl)piperazino group, 4-(3-fluorophenyl)piperazino group, 4-(4-fluorophenyl)piperazino group, 4-(2-chlorophenyl)piperazino group, 4-(3-chlorophenyl)piperazino group, 4-(4-chlorophenyl)piperazino group, 4-benzylpiperazino group, 4-(2-methylphenylmethyl)piperazino group, 4-(3-methylphenylmethyl)piperazino group, 4-methylphenylmethyl)piperazino group, 4-(2,3-dimethylphenylmethyl)piperazino group, 4-(3,5-dimethylphenylmethyl)piperazino group, 4-(2-fluorophenylmethyl)piperazino group, 4-(3-fluorophenylmethyl)piperazino group, 4-(4-fluorophenylmethyl)piperazino group, 4-(2-chlorophenylmethyl)piperazino group, 4-(3-chlorophenylmethyl)piperazino group, 4-(4-chlorophenylmethyl)piperazino group, 4-(2,3-difluorophenylmethyl)piperazino group, 4-(2,4-difluorophenylmethyl)piperazino group, 4-(2,5-difluorophenylmethyl)piperazino group, 4-(3,4-difluorophenylmethyl)piperazino group, 4-(2,3-dichlorophenylmethyl)piperazino group, 4-(2,4-dichlorophenylmethyl)piperazino group, 4-(2,5-dichlorophenylmethyl)piperazino group, 4-(2,6-dichlorophenylmethyl)piperazino group, 4-(3,4-dichlorophenylmethyl)piperazino group, 4-(3,5-dichlorophenylmethyl)piperazino group, 4-(3,6-dichlorophenylmethyl)piperazino group, 4-(2-methoxyphenylmethyl)piperazino group, 4-(3-methoxyphenylmethyl)piperazino group, or 4-(4-methoxyphenylmethyl)piperazino group;
the substituent V¹ on the ring (E) represents Zx, and V² on the ring (E) represents AR;
Zx represents hydrogen atom, fluorine atom, chlorine atom, bromine atom, methyl group, hydroxyl group, amino group, or N-methylamino group;
AR represents naphthalen-2-yl group, 6-hydroxynaphthalen-2-yl group, 6-methoxynaphthalen-2-yl group, 6-aminonaphthalen-2-yl group, 6-(N,N-dimethylamino)naphthalen-2-yl group, benzo[b]furan-5-yl group, benzo[b]thiophen-5-yl group, 1H-indol-5-yl group, 1-methyl-1H-indol-5-yl group, 1-ethyl-1H-indol-5-yl group, benzothiazol-6-yl group, 2-aminobenzothiazol-6-yl group, 2-oxo-2,3-dihydrobenzothiazol-6-yl group, quinolin-3-yl group, quinolin-6-yl group, 2-oxo-1,2-dihydroquinolin-6-yl group, 1H-indazol-5-yl group, 1-methyl-1H-indazol-5-yl group, 1-ethyl-1H-indazol-5-yl group, isoquinolin-6-yl group, 1-oxo-1,2-dihydroisoquinolin-6-yl group, cinnolin-6-yl group, or benzoxazol-5-yl group; and
Y represents hydrogen atom, methyl group, or ethyl group.

In a most preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a single bond;
the bond between the ring-constituting carbon atom at the 1-position of the ring (E) and the Link moiety represents a single bond;
Link represents CH₂;
W represents methylene group;
Rs represents -D-Rx or -N(Ry)(Rz);
D represents a single bond, or oxygen atom;
Rx represents propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group;
the substituent -N(Ry)(Rz) represents N,N-dimethylamino group, N-ethyl-N-methylamino group, N,N-diethylamino group, N-methyl-N-propylamino group, N-ethyl-N-propylamino group, N-isopropyl-N-methylamino group, N-ethyl-N-isopropylamino group, N-butylamino group, N-butyl-N-methylamino group, N-butyl-N-ethylamino group, N-isobutylamino group, N-isobutyl-N-methylamino group, N-ethyl-N-isobutylamino group, N-isopentylamino group, N-isopentyl-N-methylamino group, N-ethyl-N-isopentylamino group, N-(2-ethylbutyl)amino group, N-(2-ethylbutyl)-N-methylamino group, N-cyclopentylamino group, N-cyclopentyl-N-methylamino group, N-cyclohexylamino group, N-cyclohexyl-N-methylamino group, N-cycloheptylamino group, N-(cyclopentylmethyl)amino group, N-(cyclopentylmethyl)-N-methylamino group, N-(cyclohexylmethyl)amino group, N-(cyclohexylmethyl)-N-methylamino group, N-phenylamino group, N-(2-methylphenyl)amino group, N-(4-methylphenyl)amino group, N-(2-fluorophenyl)amino group, N-(3-fluorophenyl)amino group, N-(4-fluorophenyl)amino group, N-(2-chlorophenyl)amino group, N-(3-chlorophenyl)amino group, N-(4-chlorophenyl)amino group, N-(indan-2-yl)amino group, N-(1-phenylethyl)amino group, N-[1-(2-fluorophenyl)ethyl]amino group, N-[1-(3-fluorophenyl)ethyl]amino group, N-[1-(4-fluorophenyl)ethyl]amino group, N-[1-(2-chlorophenyl)ethyl]amino group, N-[1-(3-chlorophenyl)ethyl]amino group, N-[1-(4-chlorophenyl)ethyl]amino group, N-benzylamino group, N-benzyl-N-methylamino group, N-benzyl-N-ethylamino group, N-(2-methylphenylmethyl)amino group, N-methyl-N-(2-methylphenylmethyl)amino group, N-(3-methylphenylmethyl)amino group, N-methyl-N-(3-methylphenylmethyl)amino group, N-(4-methylphenylmethyl)amino group, N-methyl-N-(4-methylphenylmethyl)amino group, N-(2-fluorophenylmethyl)amino group, N-(2-fluorophenylmethyl)-N-methylamino group, N-(3-fluorophenylmethyl)amino group, N-(3-fluorophenylmethyl)-N-methylamino group, N-(4-fluorophenylmethyl)amino group, N-(4-fluorophenylmethyl)-N-methylamino group, N-(2-chlorophenylmethyl)amino group, N-(2-chlorophenylmethyl)-N-methylamino group, N-(3-chlorophenylmethyl)amino group, N-(3-chlorophenylmethyl)-N-methylamino group, N-(4-chlorophenylmethyl)amino group, N-(4-chlorophenylmethyl)-N-methylamino group, N-(2,3-difluorophenylmethyl)amino group, N-(2,3-difluorophenylmethyl)-N-methylamino group, N-(2,4-difluorophenylmethyl)amino group, N-(2,4-difluorophenylmethyl)-N-methylamino group, N-(2,5-difluorophenylmethyl)amino group, N-(2,5-difluorophenylmethyl)-N-methylamino group, N-(3,4-difluorophenylmethyl)amino group, N-(3,4-difluorophenylmethyl)-N-methylamino group, N-(3,5-difluorophenylmethyl)amino group, N-(3,5-difluorophenylmethyl)-N-methylamino group, N-(2,3-dichlorophenylmethyl)amino group, N-(2,3-dichlorophenylmethyl)-N-methylamino group, N-(2,4-dichlorophenylmethyl)amino group, N-(2,4-dichlorophenylmethyl)-N-methylamino group, N-(2,5-dichlorophenylmethyl)amino group, N-(2,5-dichlorophenylmethyl)-N-methylamino group, N-(2,6-dichlorophenylmethyl)amino group, N-(2,6-dichlorophenylmethyl)-N-methylamino group, N-(3,4-dichlorophenylmethyl)amino group, N-(3,4-dichlorophenylmethyl)-N-methylamino group, N-(3,5-dichlorophenylmethyl)amino group, N-(3,5-dichlorophenylmethyl)-N-methylamino group, N-[2-(trifluoromethyl)phenylmethyl]amino group, N-methyl-N-[2-(trifluoromethyl)phenylmethyl]amino group, N-[3-(trifluoromethyl)phenylmethyl]amino group, N-methyl-N-[3-(trifluoromethyl)phenylmethyl]amino group, N-[4-(trifluoromethyl)phenylmethyl]amino group, N-methyl-N-[4-(trifluoromethyl)phenylmethyl]amino group, pyrrolidino group, 2-methylpyrrolidino group, 3-methylpyrrolidino group, 2,5-dimethylpyrrolidino group, 3,4-dimethylpyrrolidino group, piperidino group, 2-methylpiperidino group, 3-methylpiperidino group, 4-methylpiperidino group, 4-ethylpiperidino group, 4-propylpiperidino group, 4-isopropylpiperidino group, 4-butylpiperidino group, 4-isobutylpiperidino group, 4-cyclopentylpiperidino group, 4-cyclohexylpiperidino group, 4-(cyclopentylmethyl)piperidino group, 4-(cyclohexylmethyl)piperidino group, 4-phenylpiperidino group, 4-(2-methylphenyl)piperidino group, 4-(4-methylphenyl)piperidino group, 4-(2-fluorophenyl)piperidino group, 4-(3-fluorophenyl)piperidino group, 4-(4-fluorophenyl)piperidino group, 4-(2-chlorophenyl)piperidino group, 4-(3-chlorophenyl)piperidino group, 4-(4-chlorophenyl)piperidino group, 4-benzylpiperidino group, 4-(2-methylphenylmethyl)piperidino group, 4-(3-methylphenylmethyl)piperidino group, 4-methylphenylmethyl)piperidino group, 4-(2,3-dimethylphenylmethyl)piperidino group, 4-(3,5-dimethylphenylmethyl)piperidino group, 4-(2-fluorophenylmethyl)piperidino group, 4-(3-fluorophenylmethyl)piperidino group, 4-(4-fluorophenylmethyl)piperidino group, 4-(2-chlorophenylmethyl)piperidino group, 4-(3-chlorophenylmethyl)piperidino group, 4-(4-chlorophenylmethyl)piperidino group, 4-(2,3-difluorophenylmethyl)piperidino group, 4-(2,4-difluorophenylmethyl)piperidino group, 4-(2,5-difluorophenylmethyl)piperidino group, 4-(3,4-difluorophenylmethyl)piperidino group, 4-(2,3-dichlorophenylmethyl)piperidino group, 4-(2,4-dichlorophenylmethyl)piperidino group, 4-(2,5-dichlorophenylmethyl)piperidino group, 4-(2,6-dichlorophenylmethyl)piperidino group, 4-(3,4-dichlorophenylmethyl)piperidino group, 4-(3,5-dichlorophenylmethyl)piperidino group, 4-(3,6-dichlorophenylmethyl)piperidino group, 4-(2-methoxyphenylmethyl)piperidino group, 4-(3-methoxyphenylmethyl)piperidino group, 4-(4-methoxyphenylmethyl)piperidino group, 2,6-dimethylpiperidino group, 3,5-dimethylpiperidino group, 4,4-dimethylpiperidino group, homopiperidino group, morpholino group, 3,5-dimethylmorpholino group, 4-phenylpiperazino group, 4-(2-methylphenyl)piperazino group, 4-(4-methylphenyl)piperazino group, 4-(2-fluorophenyl)piperazino group, 4-(3-fluorophenyl)piperazino group, 4-(4-fluorophenyl)piperazino group, 4-(2-chlorophenyl)piperazino group, 4-(3-chlorophenyl)piperazino group, 4-(4-chlorophenyl)piperazino group, 4-benzylpiperazino group, 4-(2-methylphenylmethyl)piperazino group, 4-(3-methylphenylmethyl)piperazino group, 4-methylphenylmethyl)piperazino group, 4-(2,3-dimethylphenylmethyl)piperazino group, 4-(3,5-dimethylphenylmethyl)piperazino group, 4-(2-fluorophenylmethyl)piperazino group, 4-(3-fluorophenylmethyl)piperazino group, 4-(4-fluorophenylmethyl)piperazino group, 4-(2-chlorophenylmethyl)piperazino group, 4-(3-chlorophenylmethyl)piperazino group, 4-(4-chlorophenylmethyl)piperazino group, 4-(2,3-difluorophenylmethyl)piperazino group, 4-(2,4-difluorophenylmethyl)piperazino group, 4-(2,5-difluorophenylmethyl)piperazino group, 4-(3,4-difluorophenylmethyl)piperazino group, 4-(2,3-dichlorophenylmethyl)piperazino group, 4-(2,4-dichlorophenylmethyl)piperazino group, 4-(2,5-dichlorophenylmethyl)piperazino group, 4-(2,6-dichlorophenylmethyl)piperazino group, 4-(3,4-dichlorophenylmethyl)piperazino group, 4-(3,5-dichlorophenylmethyl)piperazino group, 4-(3,6-dichlorophenylmethyl)piperazino group, 4-(2-methoxyphenylmethyl)piperazino group, 4-(3-methoxyphenylmethyl)piperazino group, or 4-(4-methoxyphenylmethyl)piperazino group;
the substituent V¹ on the ring (E) represents Zx, and V² on the ring (E) represents AR;
Zx represents hydrogen atom or amino group;
AR represents 1H-indol-5-yl group, 1-methyl-1H-indol-5-yl group, 1-ethyl-1H-indol-5-yl group, 1H-indazol-5-yl group, 1-methyl-1H-indazol-5-yl group, or 1-ethyl-1H-indazol-5-yl group; and
Y represents hydrogen atom, methyl group, or ethyl group.

In another most preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a single bond;
the bond between the ring-constituting carbon atom at the 1-position of the ring (E) and the Link moiety represents a single bond;
Link represents CH₂;
W represents methylene group;
Rs represents propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group;
the substituent V¹ on the ring (E) represents Zx, and V² on the ring (E) represents AR;
Zx represents hydrogen atom or amino group;
AR represents 1H-indol-5-yl group, 1-methyl-1H-indol-5-yl group, 1-ethyl-1H-indol-5-yl group, 1H-indazol-5-yl group, 1-methyl-1H-indazol-5-yl group, or 1-ethyl-1H-indazol-5-yl group; and
Y represents hydrogen atom, methyl group, or ethyl group.

In a still other most preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a single bond;
the bond between the ring-constituting carbon atom at the 1-position of the ring (E) and the Link moiety represents a single bond;
Link represents CH₂;
W represents methylene group;
Rs represents -O-Rx;
Rx represents propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group;
the substituent V¹ on the ring (E) represents Zx, and V² on the ring (E) represents AR;
Zx represents hydrogen atom or amino group;
AR represents 1H-indol-5-yl group, 1-methyl-1H-indol-5-yl group, 1-ethyl-1H-indol-5-yl group, 1H-indazol-5-yl group, 1-methyl-1H-indazol-5-yl group, or 1-ethyl-1H-indazol-5-yl group; and
Y represents hydrogen atom, methyl group, or ethyl group.

In a still other most preferred embodiment of the present invention, the compound represented by the formula (I) or a salt thereof satisfies all of the following requirements:
the bond between the ring-constituting carbon atom at the 1-position of the ring (E) and the ring-constituting carbon atom at the 2-position of the ring (E) represents a single bond;
the bond between the ring-constituting carbon atom at the 1-position of the ring (E) and the Link moiety represents a single bond;
Link represents CH₂;
W represents methylene group;
Rs represents -N(Ry)(Rz);
the substituent -N(Ry)(Rz) represents N,N-dimethylamino group, N-ethyl-N-methylamino group, N,N-diethylamino group, N-methyl-N-propylamino group, N-ethyl-N-propylamino group, N-isopropyl-N-methylamino group, N-ethyl-N-isopropylamino group, N-butylamino group, N-butyl-N-methylamino group, N-butyl-N-ethylamino group, N-isobutylamino group, N-isobutyl-N-methylamino group, N-ethyl-N-isobutylamino group, N-isopentylamino group, N-isopentyl-N-methylamino group, N-ethyl-N-isopentylamino group, N-(2-ethylbutyl)amino group, N-(2-ethylbutyl)-N-methylamino group, N-cyclopentylamino group, N-cyclopentyl-N-methylamino group, N-cyclohexylamino group, N-cyclohexyl-N-methylamino group, N-cycloheptylamino group, N-(cyclopentylmethyl)amino group, N-(cyclopentylmethyl)-N-methylamino group, N-(cyclohexylmethyl)amino group, N-(cyclohexylmethyl)-N-methylamino group, N-phenylamino group, N-(2-methylphenyl)amino group, N-(4-methylphenyl)amino group, N-(2-fluorophenyl)amino group, N-(3-fluorophenyl)amino group, N-(4-fluorophenyl)amino group, N-(2-chlorophenyl)amino group, N-(3-chlorophenyl)amino group, N-(4-chlorophenyl)amino group, N-(indan-2-yl)amino group, N-(1-phenylethyl)amino group, N-[1-(2-fluorophenyl)ethyl]amino group, N-[1-(3-fluorophenyl)ethyl]amino group, N-[1-(4-fluorophenyl)ethyl]amino group, N-[1-(2-chlorophenyl)ethyl]amino group, N-[1-(3-chlorophenyl)ethyl]amino group, N-[1-(4-chlorophenyl)ethyl]amino group, N-benzylamino group, N-benzyl-N-methylamino group, N-benzyl-N-ethylamino group, N-(2-methylphenylmethyl)amino group, N-methyl-N-(2-methylphenylmethyl)amino group, N-(3-methylphenylmethyl)amino group, N-methyl-N-(3-methylphenylmethyl)amino group, N-(4-methylphenylmethyl)amino group, N-methyl-N-(4-methylphenylmethyl)amino group, N-(2-fluorophenylmethyl)amino group, N-(2-fluorophenylmethyl)-N-methylamino group, N-(3-fluorophenylmethyl)amino group, N-(3-fluorophenylmethyl)-N-methylamino group, N-(4-fluorophenylmethyl)amino group, N-(4-fluorophenylmethyl)-N-methylamino group, N-(2-chlorophenylmethyl)amino group, N-(2-chlorophenylmethyl)-N-methylamino group, N-(3-chlorophenylmethyl)amino group, N-(3-chlorophenylmethyl)-N-methylamino group, N-(4-chlorophenylmethyl)amino group, N-(4-chlorophenylmethyl)-N-methylamino group, N-(2,3-difluorophenylmethyl)amino group, N-(2,3-difluorophenylmethyl)-N-methylamino group, N-(2,4-difluorophenylmethyl)amino group, N-(2,4-difluorophenylmethyl)-N-methylamino group, N-(2,5-difluorophenylmethyl)amino group, N-(2,5-difluorophenylmethyl)-N-methylamino group, N-(3,4-difluorophenylmethyl)amino group, N-(3,4-difluorophenylmethyl)-N-methylamino group, N-(3,5-difluorophenylmethyl)amino group, N-(3,5-difluorophenylmethyl)-N-methylamino group, N-(2,3-dichlorophenylmethyl)amino group, N-(2,3-dichlorophenylmethyl)-N-methylamino group, N-(2,4-dichlorophenylmethyl)amino group, N-(2,4-dichlorophenylmethyl)-N-methylamino group, N-(2,5-dichlorophenylmethyl)amino group, N-(2,5-dichlorophenylmethyl)-N-methylamino group, N-(2,6-dichlorophenylmethyl)amino group, N-(2,6-dichlorophenylmethyl)-N-methylamino group, N-(3,4-dichlorophenylmethyl)amino group, N-(3,4-dichlorophenylmethyl)-N-methylamino group, N-(3,5-dichlorophenylmethyl)amino group, N-(3,5-dichlorophenylmethyl)-N-methylamino group, N-[2-(trifluoromethyl)phenylmethyl]amino group, N-methyl-N-[2-(trifluoromethyl)phenylmethyl]amino group, N-[3-(trifluoromethyl)phenylmethyl]amino group, N-methyl-N-[3-(trifluoromethyl)phenylmethyl]amino group, N-[4-(trifluoromethyl)phenylmethyl]amino group, N-methyl-N-[4-(trifluoromethyl)phenylmethyl]amino group, pyrrolidino group, 2-methylpyrrolidino group, 3-methylpyrrolidino group, 2,5-dimethylpyrrolidino group, 3,4-dimethylpyrrolidino group, piperidino group, 2-methylpiperidino group, 3-methylpiperidino group, 4-methylpiperidino group, 4-ethylpiperidino group, 4-propylpiperidino group, 4-isopropylpiperidino group, 4-butylpiperidino group, 4-isobutylpiperidino group, 4-cyclopentylpiperidino group, 4-cyclohexylpiperidino group, 4-(cyclopentylmethyl)piperidino group, 4-(cyclohexylmethyl)piperidino group, 4-phenylpiperidino group, 4-(2-methylphenyl)piperidino group, 4-(4-methylphenyl)piperidino group, 4-(2-fluorophenyl)piperidino group, 4-(3-fluorophenyl)piperidino group, 4-(4-fluorophenyl)piperidino group, 4-(2-chlorophenyl)piperidino group, 4-(3-chlorophenyl)piperidino group, 4-(4-chlorophenyl)piperidino group, 4-benzylpiperidino group, 4-(2-methylphenylmethyl)piperidino group, 4-(3-methylphenylmethyl)piperidino group, 4-methylphenylmethyl)piperidino group, 4-(2,3-dimethylphenylmethyl)piperidino group, 4-(3,5-dimethylphenylmethyl)piperidino group, 4-(2-fluorophenylmethyl)piperidino group, 4-(3-fluorophenylmethyl)piperidino group, 4-(4-fluorophenylmethyl)piperidino group, 4-(2-chlorophenylmethyl)piperidino group, 4-(3-chlorophenylmethyl)piperidino group, 4-(4-chlorophenylmethyl)piperidino group, 4-(2,3-difluorophenylmethyl)piperidino group, 4-(2,4-difluorophenylmethyl)piperidino group, 4-(2,5-difluorophenylmethyl)piperidino group, 4-(3,4-difluorophenylmethyl)piperidino group, 4-(2,3-dichlorophenylmethyl)piperidino group, 4-(2,4-dichlorophenylmethyl)piperidino group, 4-(2,5-dichlorophenylmethyl)piperidino group, 4-(2,6-dichlorophenylmethyl)piperidino group, 4-(3,4-dichlorophenylmethyl)piperidino group, 4-(3,5-dichlorophenylmethyl)piperidino group, 4-(3,6-dichlorophenylmethyl)piperidino group, 4-(2-methoxyphenylmethyl)piperidino group, 4-(3-methoxyphenylmethyl)piperidino group, 4-(4-methoxyphenylmethyl)piperidino group, 2,6-dimethylpiperidino group, 3,5-dimethylpiperidino group, 4,4-dimethylpiperidino group, homopiperidino group, morpholino group, 3,5-dimethylmorpholino group, 4-phenylpiperazino group, 4-(2-methylphenyl)piperazino group, 4-(4-methylphenyl)piperazino group, 4-(2-fluorophenyl)piperazino group, 4-(3-fluorophenyl)piperazino group, 4-(4-fluorophenyl)piperazino group, 4-(2-chlorophenyl)piperazino group, 4-(3-chlorophenyl)piperazino group, 4-(4-chlorophenyl)piperazino group, 4-benzylpiperazino group, 4-(2-methylphenylmethyl)piperazino group, 4-(3-methylphenylmethyl)piperazino group, 4-methylphenylmethyl)piperazino group, 4-(2,3-dimethylphenylmethyl)piperazino group, 4-(3,5-dimethylphenylmethyl)piperazino group, 4-(2-fluorophenylmethyl)piperazino group, 4-(3-fluorophenylmethyl)piperazino group, 4-(4-fluorophenylmethyl)piperazino group, 4-(2-chlorophenylmethyl)piperazino group, 4-(3-chlorophenylmethyl)piperazino group, 4-(4-chlorophenylmethyl)piperazino group, 4-(2,3-difluorophenylmethyl)piperazino group, 4-(2,4-difluorophenylmethyl)piperazino group, 4-(2,5-difluorophenylmethyl)piperazino group, 4-(3,4-difluorophenylmethyl)piperazino group, 4-(2,3-dichlorophenylmethyl)piperazino group, 4-(2,4-dichlorophenylmethyl)piperazino group, 4-(2,5-dichlorophenylmethyl)piperazino group, 4-(2,6-dichlorophenylmethyl)piperazino group, 4-(3,4-dichlorophenylmethyl)piperazino group, 4-(3,5-dichlorophenylmethyl)piperazino group, 4-(3,6-dichlorophenylmethyl)piperazino group, 4-(2-methoxyphenylmethyl)piperazino group, 4-(3-methoxyphenylmethyl)piperazino group, or 4-(4-methoxyphenylmethyl)piperazino group;
the substituent V¹ on the ring (E) represents Zx, and V² on the ring (E) represents AR;
Zx represents hydrogen atom or amino group;
AR represents 1H-indol-5-yl group, 1-methyl-1H-indol-5-yl group, 1-ethyl-1H-indol-5-yl group, 1H-indazol-5-yl group, 1-methyl-1H-indazol-5-yl group, or 1-ethyl-1H-indazol-5-yl group; and
Y represents hydrogen atom, methyl group, or ethyl group.

Compound (I) of the present invention may have one or more asymmetric carbons depending on types of substituents. For example, as for a compound wherein both the bond between the ring-constituting carbon atom at the 1-position of the ring (E) and the ring-constituting carbon atom at the 2-position of the ring (E) and the bond between the ring-constituting carbon atom at the 1-position of the ring (E) and the Link moiety represent a single bond, or the group Rs contains one or more asymmetric carbons, two kinds of optical isomers exist when the number of asymmetric carbon is 1, and when the number of asymmetric carbons is 2, four kinds of optical isomers and two kinds of diastereomers exist. Pure stereoisomers including optical isomers and diastereoisomers, any mixtures thereof, racemates and the like of the stereoisomers fall within the scope of the present invention. Further, Compound (I) of the present invention may exist as geometrical isomers based on presence of a double bond, or a cycloalkyl ring structure, and any geometrical isomers in pure forms, and any mixtures of the geometrical isomers also fall within the scope of the present invention. Mixtures such as racemates may sometimes be preferred from a viewpoint of easiness for manufacture.
As a salt of Compound (I) of the present invention, a pharmaceutically acceptable salt is preferred. It is meant that, when at least one of the conditions (1) to (3) is satisfied: (1) Y is hydrogen atom; (2) the group AR contains carboxyl group, or phenolic hydroxyl group; (3) the group Zx is phenolic hydroxyl group, and the like, then the compound forms 1 to 3 alkali salts depending on the number of acidic groups. Examples the alkali salts include, for example, salts with inorganic bases such as sodium and ammonia and salts with organic bases such as triethylamine.
Alternatively, it is meant that, when at least one of the conditions (1) to (4) is satisfied: (1) the group Rs has properties as a base as in a compound wherein Rs contains a substituted or unsubstituted amino group and the like; (2) AR itself is a cyclic substituent having properties as a base; (3) the group Ar contains a substituted or unsubstituted amino group; (4) Zx is a substituted or unsubstituted amino group and the like, then the compound forms 1 to 4 acidic salts depending on the number of basic groups. Examples of the acidic salts include, for example, salts with inorganic acids such as hydrochloric acid and sulfuric acid and salts with organic acids such as acetic acid and citric acid.

### <Preparation methods of the compounds of the present invention>

Compound (I) of the present invention can be produced by, for example, employing the reactions according to the following various methods.
When carboxyl (COOH) group, hydroxyl (OH) group, thiol (SH) group, carbonyl or ketone (C(O)) group including formyl (CHO) group, or amino (NH) group is contained in the structures of the compounds of the present invention or synthetic intermediates thereof, those substituents may be protected with a protective group as required. When a heterocyclic ring containing NH in the ring such as indole ring and indazole ring is contained in the structures of the compounds of the present invention or synthetic intermediates thereof, that NH is also an amino group which may be protected.
As for types of the protective groups, examples include those mentioned below, for example. However, they are not limited to these examples, and types, selection, and introduction of protective groups can be selected or attained by referring to and examining ordinary chemical literatures, for example, Protective Groups In Organic Synthesis, references cited in the literatures, and the like.

In the structures of the compounds of the present invention or synthetic intermediates thereof, when carboxyl (COOH) group is contained, the carboxyl group may be protected with a group Rp¹, when hydroxyl (OH) group is contained, the hydroxyl group may be protected with a group Rp², when formyl (CHO) group is contained, the formyl group may be protected with a group Rp³, and when amino group (NH) is contained, the amino group may be protected with a group Rp⁴.
Examples of Rp¹ include, for example, an alkyl group having 1 to 4 carbon atoms, an alkenyl group having 2 to 4 carbon atoms, an alkyl group having 1 to 4 carbon atoms substituted with an alkoxy group having 1 to 4 carbon atoms, an alkyl group having 1 to 4 carbon atoms substituted with 1 to 3 halogen atoms, and the like, and specific examples include methyl group, ethyl group, t-butyl group, allyl group, methoxyethyl group, trichloroethyl group, and the like. Further, examples of Rp¹ also include, for example, a group -Ap¹-Rp⁵, and the like. Ap¹ in the group -Ap¹-Rp⁵ represents a single bond, methylene group, or -CH₂C(O)-, and Rp⁵ represents phenyl group which may be substituted with one Xp or two or more of the same or different Xp. The substituent Xp represents an alkyl group having 1 to 4 carbon atoms, hydroxyl group, a halogen atom, trifluoromethyl group, nitro group, an alkoxy group having 1 to 4 carbon atoms, or a mono- or dialkylamino group having 1 to 4 carbon atoms in each alkyl group. Specific examples of -Ap¹-Rp⁵ include phenyl group, methylphenyl group, chlorophenyl group, benzyl (Bn) group, methylbenzyl group, chlorobenzyl group, dichlorobenzyl group, fluorobenzyl group, trifluoromethylbenzyl group, nitrobenzyl group, methoxyphenyl group, N-methylaminobenzyl group, N,N-dimethylaminobenzyl group, phenacyl group, and the like. Among them, an alkyl group having 1 to 4 carbon atoms, and the like are particularly preferred examples.

Rp² represents, for example, an alkyl group having 1 to 4 carbon atoms, an alkenyl group having 2 to 4 carbon atoms, an alkyl group having 1 to 4 carbon atoms substituted with an alkoxy group having 1 to 4 carbon atoms, an alkyl group having 1 to 4 carbon atoms substituted with 1 to 3 halogen atoms, a silyl group substituted with three of the same or different alkyl groups having 1 to 4 carbon atoms or phenyl groups, tetrahydropyranyl group, tetrahydrofuryl group, propargyl group, a group - Ap¹-Rp⁵, a group -CH₂-Ap²-Rp⁶, a group -C(O)Rp⁶, a group -COORp⁶, or the like. Ap² represents oxygen atom, or sulfur atom, and Rp⁶ represents hydrogen atom, an alkyl group having 1 to 4 carbon atoms, trimethylsilylethyl group, chloromethyl group, trichloromethyl group, trifluoromethyl group, 9-fluorenylmethyl group, adamantyl group, allyl group, a group -Ap¹-Rp⁵, or the like. Specific examples of Rp² include methyl group, ethyl group, t-butyl group, allyl group, methoxymethyl (MOM) group, methoxyethyl (MEM) group, trichloroethyl group, phenyl group, methylphenyl group, chlorophenyl group, benzyl group, methylbenzyl group, chlorobenzyl group, dichlorobenzyl group, fluorobenzyl group, trifluoromethylbenzyl group, nitrobenzyl group, methoxyphenyl group, N-methylaminobenzyl group, N,N-dimethylaminobenzyl group, phenacyl group, trityl group, 1-ethoxyethyl (EE) group, tetrahydropyranyl (THP) group, tetrahydrofuryl group, propargyl group, trimethylsilyl (TMS) group, triethylsilyl (TES) group, t-butyldimethylsilyl (TBDMS) group, t-butyldiphenylsilyl (TBDPS) group, acetyl (Ac) group, pivaloyl group, benzoyl group, allyloxycarbonyl (Alloc) group, 2,2,2-trichloroethoxycarbonyl (Troc) group, and the like.

Rp³ represents, for example, an acetal group, or the like, and specific examples include dimethylacetal, and the like.
Rp⁴ represents, for example, one or two or more of the same or different groups -Ap¹-Rp⁵, groups -C(O)Rp⁶, groups -COORp⁶, and the like. Specific examples include benzyl group, methylbenzyl group, chlorobenzyl group, dichlorobenzyl group, fluorobenzyl group, trifluoromethylbenzyl group, nitrobenzyl group, methoxyphenyl group, N-methylaminobenzyl group, N,N-dimethylaminobenzyl group, phenacyl group, acetyl group, trifluoroacetyl group, pivaloyl group, benzoyl group, allyloxycarbonyl group, 2,2,2-trichloroethoxycarbonyl group, benzyloxycarbonyl group, t-butoxycarbonyl (Boc) group, 1-methyl-1-(4-biphenyl)ethoxycarbonyl (Bpoc) group, 9-fluorenylmethoxycarbonyl group, benzyloxymethyl (BOM) group, 2-(trimethylsilyl)ethoxymethyl (SEM) group, and the like.

By removing these protective groups simultaneously with the preparation or stepwise during the preparation process or at the final step, protected compounds can be converted into objective compounds. Deprotection reactions for carboxyl group, hydroxyl group, thiol group, ketone or carbonyl group including formyl group, and amino group are well known, and examples include, for example, (1) alkali hydrolysis, (2) deprotection reaction under an acidic condition, (3) deprotection reaction by hydrogenolysis, (4) deprotection reaction of silyl group, (5) deprotection reaction using a metal, (6) deprotection reaction using a metal complex, and the like.
These methods are specifically performed as follows.
(1) The deprotection reaction by alkali hydrolysis is performed by, for example, reacting a protected compound with a base in a polar solvent. Examples of the base used in this reaction include, for example, alkali metal bases such as sodium hydroxide, potassium hydroxide, lithium hydroxide, barium hydroxide, calcium hydroxide, sodium carbonate, potassium carbonate, sodium methoxide, and potassium t-butoxide, and organic bases such as triethylamine. They are usually used in an amount of 1 to 20 moles, preferably 1 to 10 moles, based on the reactant, when an alkali metal base is used, or 1 fold mole to a large excess amount, when an organic base is used. As for the reaction solvent, it is usually preferred that the reaction is performed in an inactive medium that does not inhibit the reaction, preferably a polar solvent. Examples of the polar solvent include water, methanol, ethanol, tetrahydrofuran, dioxane, and the like, and these can be used as a mixture as required. As the reaction temperature, a suitable temperature, for example, from - 10°C to the reflux temperature of the solvent, is chosen. The reaction time is, for example, usually 0.5 to 72 hours, preferably 1 to 48 hours, when an alkali metal base is used, or 5 hours to 14 days, when an organic base is used. However, since the progress of the reaction can be monitored by thin layer chromatography (TLC), high performance liquid chromatography (HPLC), or the like, it is usually preferred that the reaction is terminated when the maximum yield of the objective compound is obtained.

(2) The deprotection reaction under an acidic condition is performed, for example, in an organic solvent (dichloromethane, chloroform, dioxane, ethyl acetate, anisole and the like) in the presence of an organic acid (acetic acid, trifluoroacetic acid, methanesulfonic acid, p-toluenesulfonic acid and the like), a Lewis acid (boron tribromide, boron trifluoride, aluminum bromide, aluminum chloride and the like), or an inorganic acid (hydrochloric acid, sulfuric acid and the like), or a mixture thereof (hydrogen bromide/acetic acid and the like) at a temperature of -10 to 100°C. There is also a method of adding ethanethiol, 1,2-ethanedithiol, or the like as an additive.
(3) The deprotection reaction by hydrogenolysis is performed, for example, in a solvent [ether type solvents (tetrahydrofuran, dioxane, dimethoxyethane, diethyl ether and the like), alcohol type solvents (methanol, ethanol and the like), benzene type solvents (benzene, toluene and the like), ketone type solvents (acetone, methyl ethyl ketone and the like), nitrile type solvents (acetonitrile and the like), amide type solvents (dimethylformamide and the like), ester type solvents (ethyl acetate and the like), water, acetic acid, mixtures of two or more types of those solvents, and the like] in the presence of a catalyst (palladium/carbon powder, platinum oxide (PtO₂), activated nickel and the like) and a hydrogen source such as hydrogen gas of ordinary pressure or under pressurization, ammonium formate, or hydrazine hydrate at a temperature of -10 to 60°C.
(4) The deprotection reaction of silyl group is performed, for example, by using tetra-n-butylammonium fluoride or the like in a water-miscible organic solvent (tetrahydrofuran, acetonitrile and the like) at a temperature of -10 to 60°C.

(5) The deprotection reaction using a metal is performed, for example, in an acidic solvent (acetic acid, buffer of pH 4.2 to 7.2, a mixture of such a solution and an organic solvent such as tetrahydrofuran) in the presence of zinc powder with or without ultrasonication at a temperature of -10 to 60°C.
(6) The deprotection reaction using a metal complex is performed, for example, in an organic solvent (dichloromethane, dimethylformamide, tetrahydrofuran, ethyl acetate, acetonitrile, dioxane, ethanol and the like), water, or a mixture thereof in the presence of a trap reagent (tributyltin hydride, triethylsilane, dimedone, morpholine, diethylamine, pyrrolidine and the like), an organic acid (acetic acid, formic acid, 2-ethylhexanoic acid and the like) and/or an organic acid salt (sodium 2-ethylhexanoate, potassium 2-ethylhexanoate and the like) in the presence or absence of a phosphine type regent (triphenylphosphine and the like) by using a metal complex [tetrakistriphenylphosphine palladium(0), bis(triphenylphosphine) palladium(II) dichloride, palladium(II) acetate, tris(triphenylphosphine) rhodium(I) chloride and the like] at a temperature of -10 to 60°C.
Further, besides the aforementioned methods, the deprotection reaction can be performed by referring to and examining ordinary chemical literatures, for example, Protective Groups In Organic Synthesis, mentioned above, references cited in the literatures, and the like.
As readily understood by those skilled in the art, protected compounds can be easily derived into the desired compounds of the present invention or synthetic intermediates thereof by using a combination of these protection/deprotection reactions, or appropriately choosing and using the reactions.

### [Preparation Method 1] (Step a)

As shown in the following scheme 1: 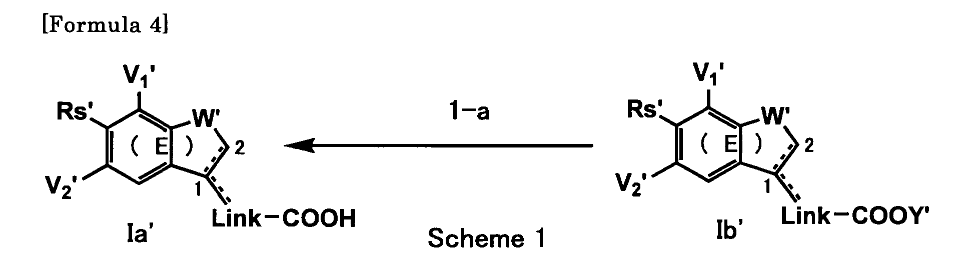 a compound of the present invention represented by the formula (Ia') wherein Link has the same meaning as defined above, Rs' has the same meaning as that of Rs mentioned above, or may be, when a protectable functional group such as hydroxyl group and amino group is contained, a protected hydroxyl group or a protected amino group, one of V1' and V2' represents Zx', the other represents Ar', Zx' has the same meaning as that of Zx mentioned above, or may be, when hydroxyl group or amino group is contained in Zx, a protected hydroxyl group or a protected amino group, Ar' has the same meaning as that of Ar mentioned above, or may be, when hydroxyl group, carboxyl group, or amino group is contained in Ar, a protected hydroxyl group, a protected carboxyl group, or a protected amino group, and W' has the same meaning as that of W mentioned above, or may be, when W is nitrogen atom, a protected nitrogen atom, which falls within the scope of Compound (I) of the present invention, and corresponds to Compound (I) wherein the group Y represents hydrogen atom [hereinafter simply referred to as "Compound (Ia')"], can be prepared by hydrolyzing a compound represented by the formula (Ib') wherein Rs', V1', V2', W', and Link have the same meanings as defined above, and Y' represents a lower alkyl group having 1 to 4 carbon atoms [hereinafter simply referred to as "Compound (Ib')"] to convert the group OY' into hydroxyl group (OH), and removing protective groups, which are hydroxyl group, amino group, or carboxyl group, if present, simultaneously or successively.

Although Y' in Compound (Ib') is preferably a lower alkyl group having 1 to 4 carbon atoms, since it is eventually removed in the reaction of the scheme 1, a protective group usually used for carboxyl group may also be employed. It would be readily understood by those skilled in the art that, in such case, the preparation can be performed by appropriately using a compound having a protective group used in a step previous to the step of preparing Compound (Ib') or a step previous to the foregoing step.
For the reaction of converting Compound (Ib') into Compound (Ia'), in general, the compound is preferably reacted in a base. Further, for the reaction of converting Compound (Ib') to Compound (Ia'), in general, the compound is preferably reacted in an inert medium that does not inhibit the reaction, preferably a polar solvent.
Examples of the base used in the above reaction include, for example, alkali metal bases such as sodium hydroxide, potassium hydroxide, sodium carbonate, potassium carbonate, sodium methoxide and potassium t-butoxide and organic bases such as triethylamine. As for amounts of the bases, generally 1 to 20 moles, preferably 1 to 10 moles, for alkali metal bases, or 1 to a large excess moles for organic bases based on Compound (Ia').

Examples of the polar solvent include water, methanol, ethanol, tetrahydrofuran, dioxane and the like, and these solvents may be used as a mixture as required. As the reaction temperature, an appropriate temperature of, for example, from room temperature to a refluxing temperature of solvent is chosen.
The reaction time is, for example, generally 0.5 to 72 hours, preferably 1 to 48 hours, when an alkali metal base is used, or generally 5 hours to 14 days when an organic base is used. Since progress of the reaction can be monitored by thin layer chromatography (TLC), high performance liquid chromatography (HPLC) or the like, the reaction can generally be terminated appropriately so as to maximize the yield of Compound (Ia').
For collection of Compound (Ia') obtained as described above from the reaction solution as a free carboxylic acid, operations may preferably be carried out by, when the polar solvent is a water-soluble solvent, evaporating the solvent, neutralizing the residue with an inorganic acid such as aqueous hydrochloric acid, dissolving the residue in a water-insoluble solvent, then washing the solution with a weakly acidic aqueous solution, water or the like, and evaporating the solvent. When the polar solvent is a water-insoluble solvent, operations may preferably carried out by neutralizing the reaction solution with an inorganic acid, washing the solution with a weakly acidic aqueous solution, water or the like, and then evaporating the solvent.
Further, when Compound (Ia') forms a salt with the base used after the reaction to give a solid, the salt of Compound (Ia') can be obtained by isolation and purification of the solid in a conventional manner.

### [Preparation Method 2] (Step b)

As shown in the following scheme 2: 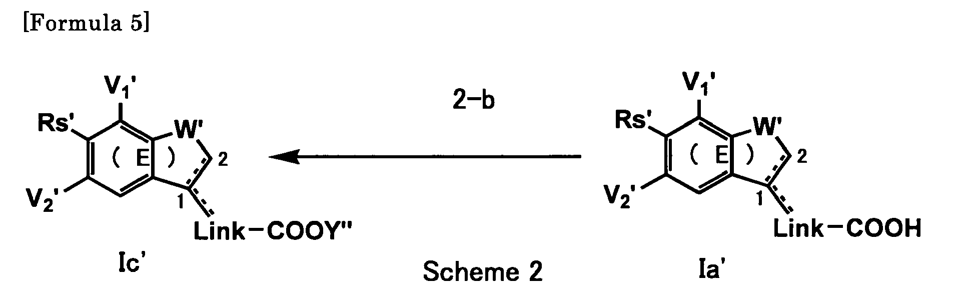 a compound represented by the formula (Ic') wherein Rs', V1', V2', W', and Link have the same meanings as defined above, and Y" represents a lower alkyl group having 1 to 4 carbon atoms, -(CH₂)ₘN (R¹⁸)(R¹⁹) or -C(R²⁰)₂OC(O)A³R²¹, which falls within the scope of Compound (I) of the present invention, and corresponds to Compound (I)
wherein Y represents Y" [hereinafter simply referred to as "Compound (Ic')"], can be produced by esterifying the carboxyl group (COOH) of Compound (Ib') in a conventional manner.

Examples of the method for producing Compound (Ic') include a method of allowing Compound (Ib') to react with an inorganic halide without solvent or in an inert solvent to convert the compound into an acid halide and then allowing the acid halide per se or the same dissolved in an inert solvent to react with an excess amount of hydroxide of the targeted Y", and the like. Examples of the inorganic halide used in this method include thionyl chloride, phosphoryl chloride, phosphorus pentachloride, phosphorus trichloride and the like, and thionyl chloride is a preferred example. Examples of an amount used include generally an equimolar to a large excess amount, preferably 1.5 to 5 moles, based on Compound (Ib'). Examples of the inert solvent used in this reaction include, for example, halogenated hydrocarbons such as dichloromethane, chloroform and 1,2-dichloroethane, ethers such as tetrahydrofuran and dioxane, and benzene compounds such as benzene, toluene, xylene and chlorobenzene. These solvents can be used, for example, each alone or as a mixed solvent. In order to promote the reaction, a catalytic amount of N,N-dimethylformamide may be added. As a reaction temperature, an appropriate temperature of from room temperature to a refluxing temperature of the solvent is generally chosen.

Examples of the inert solvent used for the reaction with hydroxide of the targeted Y" include, for example, halogenated hydrocarbons such as dichloromethane, chloroform and 1,2-dichloroethane, ethers such as tetrahydrofuran and dioxane, and benzene compounds such as benzene, toluene, and xylene. The reaction can also be performed with an excess amount of the hydroxide of the targeted Y" without using a solvent. As the reaction temperature, an appropriate temperature of from -10°C to room temperature is chosen.
Other methods for producing Compound (Ic') include, for example, the "esterification using an alcohol" described in Shin Jikken Kagaku Koza (edited by the Chemical Society of Japan, published by Maruzen Co., Ltd.), vol. 14, p.1002, "esterification using an O-alkylating agent", ibid, the same volume, p.1002, "esterification using an alkyl halide", ibid, the same volume, p.1008, "esterification reaction using a dehydrating agent", ibid, vol. 22, p.45 and the like. When hydroxyl group or amino group reactive under the aforementioned reaction conditions exists in V¹, V², or W in the ring (E), this substituent is preferably protected.
When a protective group of hydroxyl group or amino group exists in the groups V1', V2', and W' in the ring (E) of Compound (Ic') prepared as described above, such a protecting group can be eliminated during or after the preparation of Compound (Ic') to convert the compound into Compound (I) of the present invention.

### [Preparation Method 3] (Step c)

As shown in the following scheme 3: 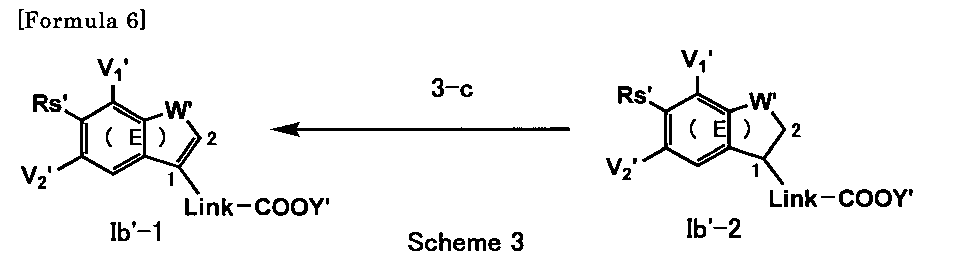 a compound represented by the formula (Ib'-1) wherein Rs', V1', V2', W', Link and Y' have the same meanings as defined above, which falls within the scope of Compound (I) of the present invention, and corresponds to Compound (I) wherein the bond between the ring-constituting carbon atom at the 1-position of the ring (E) and the ring-constituting carbon atom at the 2-position of the ring (E) represents a double bond [hereinafter simply referred to as "Compound (Ib'-1)"], can be prepared by a dehydrogenation reaction of a compound represented by the formula (Ib'-2) wherein Rs', V1', V2', W', Link and Y' have the same meanings as defined above, which falls within the scope of Compound (I) of the present invention, and corresponds to Compound (I) wherein the bond between the ring-constituting carbon atom at the 1-position of the ring (E) and the ring-constituting carbon atom at the 2-position of the ring (E) represents a single bond [hereinafter simply referred to as "Compound (Ib'-2)"].
Examples of the method for the dehydrogenation reaction include a method of performing the preparation according to a method described in ordinary literature, for example, Shin Jikken Kagaku Koza (edited by the Chemical Society of Japan, published by Maruzen Co., Ltd.), vol. 15, p.1088, and the like. For example, methods of using a metal catalyst such as platinum, palladium, iridium, rhodium, and nickel, sulfur, or selenium in an organic solvent under a heating condition, and the like are available. Examples of the method also include a method of adding a hydrogen receptor such as benzene, tetralin, nitrobenzene, oleic acid, cinnamic acid, and maleic acid, or bubbling nitrogen, or carbon dioxide in the system. Examples of the metal catalyst include palladium/carbon, rhodium/alumina, and the like, and examples of the organic solvent include toluene, triglyme, cymene, and the like.

### [Preparation Method 4]

As shown in the following scheme 4: 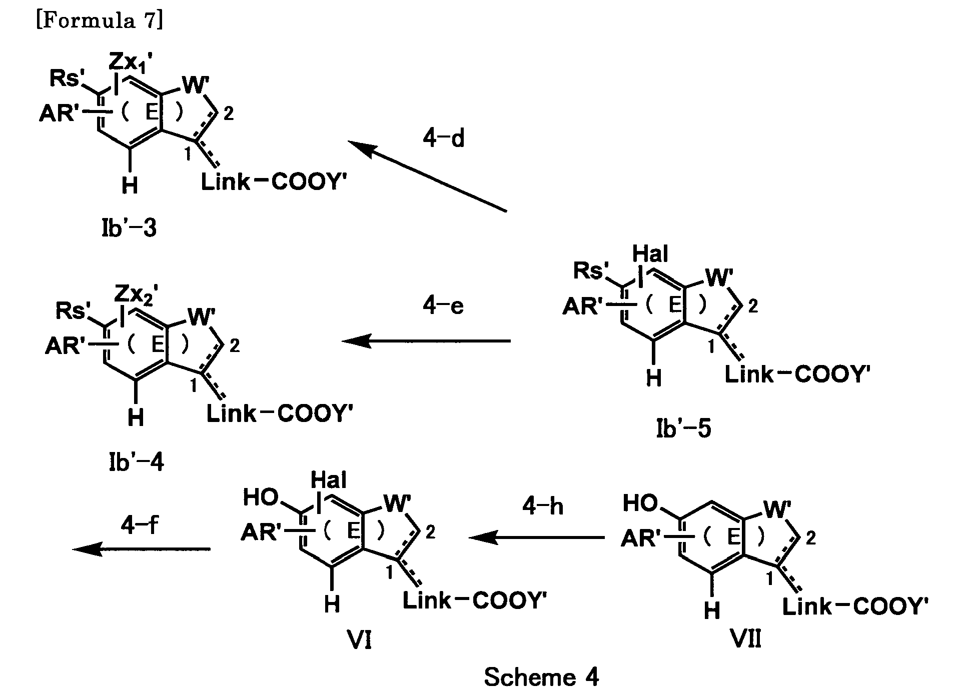 a compound represented by the formula (Ib'-3) wherein Rs', AR', W', Link and Y' have the same meanings as defined above, and H represents hydrogen atom [hereinafter simply referred to as "Compound (Ib'-3)"] as Compound (I) of the present invention wherein Zx represents Zx1', and Zx1' represents NRn1Rn2 or an amino group which may be protected (-NH-R^{P4}), and a compound represented by the formula (Ib'-4)
wherein Rs', AR', W', Link, Y', and H have the same meanings as defined above [hereinafter simply referred to as "Compound (Ib'-4)"] as Compound (I) of the present invention wherein Zx represents Zx2', and Zx2' represents a linear or branched saturated an alkyl group having 1 to 4 carbon atoms can also be prepared by the methods described below. Further, when hydroxyl group, amino group or the like reactive under the aforementioned reaction conditions or inhibiting the reactions exists in the group Rs, AR, Zx or W in the ring (E), this substituent is preferably protected.

### [Preparation Method 4] (Step d)

Compound (Ib'-3) can also be prepared from a compound represented by the formula (Ib'-5) wherein Rs', AR', W', Link, Y', and H have the same meanings as defined above [hereinafter simply referred to as "Compound Ib'-5"], which falls within the scope of Compound (I) of the present invention, and corresponds to Compound (I)
wherein the group Hal represents a halogen atom which is bromine atom, chlorine atom, iodine atom by the Buchwald-Hartwig reaction described in literature [for example, Buchwald et al., Journal ofAmerican Chemical Society, 1994, p.7901; Hartwig et al., Journal of American Chemical Society, 1994, p.5969] and the like. Specifically, there is a method of obtaining the compound by reacting Compound (Ib'-5) and a substituted amine [-NH(Rn1)(Rn2)], an amine which may be protected [-NH (R^{P4})], or the like, which constitutes the substituent Z1', and is commercially available, or can be prepared by a known method or a method similar thereto, in an organic solvent in the presence of a phosphine ligand such as 1,1'-bis(diphenylphosphino)-ferrocene, 2,2'-bis(diphenylphosphino)-1,1'-binaphthyl, 2-(di-t-butylphosphino)-biphenyl, 2-(dicyclohexylphosphino)-2'-(N,N-dimethylamino)-biphenyl, 4,5-bis(diphenylphosphino)-9,9'-dimethylxanthine, triphenylphosphine, trio-tolylphosphine, and tri-t-butylphosphine, a palladium catalyst such as palladium acetate, tris(dibenzylideneacetone)dipalladium, tetrakis(triphenylphosphine)palladium, palladium chloride, and [1,1'-bis(diphenylphosphino)-ferrocene]dichloropalladium/dichloromethane complex, and a base such as potassium phosphate, potassium carbonate, cesium carbonate, potassium t-butoxide, sodium t-butoxide, cesium fluoride, potassium fluoride, barium hydroxide, and triethylamine. The valence of palladium may be 0 or may be +2. Examples of the solvent include hydrocarbon type solvents such as toluene, xylene, and hexane, amide type solvents such as dimethylformamide, ether type solvents such as dioxane, and ethylene glycol dimethyl ether, and the like, and these solvents may be used as a mixture as required.

### [Preparation Method 4] (Step e)

Compound (Ib'-4) can also be prepared from Compound (Ib'-5) by performing the Suzuki reaction described in, for example, Jikken Kagaku Koza, 4th Edition (edited by Chemical Society of Japan, published by Maruzen Co., Ltd.), vol. 25, p.403. Specifically, examples of the preparation include a reaction of Compound (Ib'-5) and a boronic acid derivative, which is commercially available, or can be prepared by a known method or a method similar thereto, and constitutes the substituent Z2', in an organic solvent in the presence of a commercially available palladium catalyst or a catalyst prepared from a palladium complex and a ligand, and a base.
As the palladium catalyst, a commercially available catalyst such as tetrakis(triphenylphosphine)palladium, tetrakis(methyldiphenylphosphine)palladium, dichlorobis(triphenylphosphine)palladium, dichlorobis(tri-o-tolylphosphine)palladium, dichlorobis(tricyclohexylphosphine)palladium, dichlorobis(triethylphosphine)palladium, palladium acetate, palladium chloride, bis(acetonitrile)palladium chloride, tris(dibenzylideneacetone)dipalladium and bis(diphenylphosphinoferrocene)palladium chloride may be purchased and added to the reaction system, per se, or a catalyst may be added which is separately prepared from palladium acetate, tris(dibenzylideneacetone)dipalladium or the like and arbitrary ligands and isolated. Further, a catalyst considered to actually participate in the reaction may also be prepared by mixing palladium acetate, tris(dibenzylideneacetone)dipalladium or the like and arbitrary ligands in the
reaction system. The valence of palladium may be 0 or may be +2. Examples of the ligand include phosphine ligands such as trifurylphosphine, tri(o-tolyl)phosphine, tri(cyclohexyl)phosphine, tri(t-butyl)phosphine, dicyclohexylphenylphosphine, 1,1'-bis(di-t-butylphosphino)ferrocene, 2-dicyclohexylphosphino-2'-dimethylamino-1,1'-biphenyl and 2-(di-t-butylphosphino)biphenyl and phosphine mimic ligands such as imidazol-2-ylidenecarbenes.

Examples of the base include sodium carbonate, potassium carbonate, cesium carbonate, cesium fluoride, potassium fluoride, potassium phosphate, potassium acetate, triethylamine, potassium hydroxide, sodium hydroxide, sodium methoxide, lithium methoxide and the like.
The reaction system may be either a two-phase system of water and an organic solvent, or a homogeneous system of a water-containing organic solvent or an organic solvent. As for the organic solvent, examples include uses of hydrocarbon-type solvents such as toluene, xylene and hexane, halogen-type solvents such as methylene chloride, sulfoxide-type solvents such as dimethyl sulfoxide, amide-type solvents such as dimethylformamide, ether-type solvents such as tetrahydrofuran, dioxane and diglyme, alcohol-type solvents such as methanol and ethanol, nitrile-type solvents such as acetonitrile, ketone-type solvents such as acetone and cyclohexanone, ester-type solvents such as ethyl acetate, heterocyclic-type solvents such as pyridine and the like. Two or more kinds of organic solvents may be mixed and used.

For the reaction conditions, Miyaura, N., Suzuki, A., Chemical Review, 1995, vol. 95, p.2457; Snieckus, V., Chemical Review, 1990, vol. 90, p.879 and the like and references cited therein can be referred to.
Further, when a double bond is produced in the aforementioned reaction, the preparation can also be performed by reducing the double bond using a reduction reaction described in ordinary publications in the filed of chemistry. Examples of the reaction include a method of converting the double bond into a single bond by hydrogenation using a hydrogen source such as hydrogen gas, ammonium formate, and hydrazine hydrate in a single solvent or a mixed solvent of alcoholic-type solvents such as methanol, or ester-type solvents such as ethyl acetate in the presence of a catalyst such as palladium/carbon powder, platinum oxide (PtO₂), and activated nickel.
When hydroxyl group or amino group reactive under the aforementioned reaction conditions or inhibiting the reactions exists in the group AR, Rs or W in the ring (E), this substituent is preferably protected.

### [Preparation Method 4] (Step f)

Compound (Ib'-5) can be prepared by using a compound represented by the formula (VI) wherein AR', W', Link, Y', H, and Hal have the same meanings as defined above, and OH represents hydroxyl group [hereinafter simply referred to as "Compound (VI)"], which is commercially available, or can be synthesized by a known method or a method similar thereto, according to any of the methods described below.

### [Preparation Method 4] (Step f-1)

Examples of the method for preparing Compound (Ib'-5) from Compound (VI) include, for example, a method of performing the preparation by reacting Compound (VI) with an alkylating agent which is a compound represented by:

Rx-G (XXX)

wherein Rx has the same meanings as defined above, and G represents chlorine, bromine, iodine, mesylate group, triflate group or arenesulfonate group [hereinafter simply referred to as "alkylating agent"], for example, in an inert solvent in the presence of an appropriate base.
Examples of the alkylating agent used in this reaction include an iodide, bromide, or chloride of alkyl or aryl, which is commercially available, or can be prepared by a known method or a method similar thereto, a sulfuric acid ester of alkyl or aryl obtainable by mesylation, arenesulfonylation, or trifluoromethanesulfonylation of an alkyl alcohol or an aryl alcohol in a conventional manner, and the like. Examples of the inert solvent used in the reaction include, for example, alcohols such as methanol and ethanol, ethers such as tetrahydrofuran and dioxane, benzene compounds such as benzene, toluene and xylene, N,N-dimethylformamide, acetonitrile, acetone, and the like, and these solvents may be used as a mixture as required. Examples of the base used in the above reaction include, for example, alkali metal compounds such as sodium hydroxide, potassium hydroxide, sodium carbonate, potassium carbonate, sodium hydride, sodium methoxide, and potassium t-butoxide, and organic tertiary amines such as pyridine, 4-dimethylaminopyridine, 1,8-diazabicyclo[5,4,0]-undecene, trimethylamine, and triethylamine. When progress of the reaction is insufficient, a catalyst such as potassium iodide and copper powder may be added in an amount of 0.1 to 1.5 moles based on the starting material as required.

### [Preparation Method 4] (Step f-2)

Further, Compound (Ib'-5) can also be prepared from Compound (VI) by, for example, the Mitsunobu reaction described in publications [O. Mitsunobu, SYNTHESIS, 1981, p.1]. Specifically, there is a method of reacting Compound (Ib'-5) and an alkyl alcohol (RxOH), which constitutes the substituent Rx and is commercially available, or can be prepared by a known method or a method similar thereto, in an organic solvent in the presence of a phosphine such as triphenylphosphine and tributylphosphine and an azo compound such as diethyl azodicarboxylate, diisopropyl azodicarboxylate, N,N,N',N'-tetramethylazodicarboxamide, 1,1'-(azodicarbonyl)dipiperidine, and N,N,N',N'-tetraisopropylcarboxamide. Examples of the solvent include ethers such as diethyl ether, tetrahydrofuran and dimethoxyethane, halogen-type solvents such as methylene chloride, benzene compounds such as benzene, toluene, and xylene, and these solvents may be used as a mixture as required.

### [Preparation Method 4] (Step f-3)

Examples of the preparation of Compound (Ib'-5) include preparation by adding an alkene, which is commercially available or can be prepared by a known method or a method similar thereto, to the phenolic hydroxyl group of Compound (VI) in the presence of an acid catalyst for conversion into the substituent Rx, as described in Jikken Kagaku Koza, 4th Edition (edited by Chemical Society of Japan, published by Maruzen Co., Ltd.), vol. 20, p.200. Examples of the alkene used in this reaction include, for example, isobutylene, cyclopentene, cyclohexene, cycloheptene, alkenes having an aromatic ring such as substituted or unsubstituted styrene and α-methylstyrene, and the like. Examples of the acid catalyst used include mineral acids such as hydrochloric acid and sulfuric acid, boron trifluoride (including solvent complex thereof), tetrafluoroboric acid, trifluorosulfonic acid and the like. Examples of the solvent include ethers such as diethyl ether, tetrahydrofuran and dimethoxyethane, halogen-type solvents such as methylene chloride and benzene compounds such as benzene, toluene and xylene, and these solvents can be used as a mixture as required. Further, the alkene to be reacted may be used as a solvent.

### [Preparation Method 4] (Step f-4)

Examples of the method for preparing Compound (Ib'-5) wherein the substituent Rx represents the aforementioned group Rb, and both A¹ and A² represent a single bond include a method of performing the preparation by reacting Compound (VI) with an aryl halide under a basic condition, as described in Jikken Kagaku Koza, 4th Edition (edited by Chemical Society of Japan, published by Maruzen Co., Ltd.), vol. 20, p.191. Examples of the aryl halide used in this reaction include chlorides, bromides, and iodides of a substituted or unsubstituted aryl, which are commercially available or can be synthesized by a known method or a method similar thereto, and bromides and iodides are preferred. Alternatively, an aryl triflate may also be used instead of the aryl halide. Examples of the base used for this reaction include, for example, alkali metal compounds such as sodium hydroxide, potassium hydroxide, sodium carbonate, potassium carbonate, sodium hydride, sodium methoxide and potassium t-butoxide and organic tertiary amines such as pyridine, 4-dimethylaminopyridine, 1,8-diazabicyclo[5,4,0]undecene, trimethylamine, and triethylamine. To the reaction system, copper powder, cuprous halide, or copper alkoxide may be added as a catalyst. Further, for example, a phase transfer catalyst or crown ether may also be added. As the reaction solvent, hydrocarbon-type solvents such as toluene, xylene, chlorobenzene, dichlorobenzene and nitrobenzene, sulfoxide-type solvents such as dimethyl sulfoxide, amide-type solvents such as dimethylformamide, ether-type solvents such as dioxane and diglyme, heterocyclic-type solvents such as pyridine and the like may be used.
Example of the method for preparation of Compound (Ib'-5) wherein the substituent Rx represents the group Rb, and both A¹ and A² represent a single bond also include a method of performing the preparation according to the methods described in J. Tsuji, Journal of Organic Synthesis Association, 2001, vol. 59, No. 6, p.609] or references cited therein. Specifically, the compound can be prepared by reacting Compound (VI) with an aryl halide or aryl triflate, which is commercially available or can be prepared by a known method or a method similar thereto, in a solvent in the presence of a commercially available palladium catalyst or catalyst prepared from a palladium complex and a ligand and a base.

As the palladium catalyst, a commercially available catalyst such as tetrakis(triphenylphosphine)palladium, tetrakis(methyldiphenylphosphine)palladium, dichlorobis(triphenylphosphine) palladium, dichlorobis(tri-o-tolylphosphine)palladium, dichlorobis(tricyclohexylphosphine)palladium, dichlorobis(triethylphosphine)palladium, palladium acetate, palladium chloride, bis(acetonitrile)palladium chloride, tris(dibenzylideneacetone)dipalladium and bis(diphenylphosphinoferrocene)palladium chloride may be purchased and added to the reaction system, per se, or a catalyst may be added which is separately prepared from palladium acetate, tris(dibenzylideneacetone)dipalladium or the like and arbitrary ligands and isolated. Further, a catalyst considered to actually participate in the reaction may also be prepared by mixing palladium acetate, tris(dibenzylideneacetone)dipalladium or the like and arbitrary ligands in the reaction system. The valence of palladium may be 0 or may be +2. Examples of the ligand include phosphine ligands such as trifurylphosphine, tri(o-tolyl)phosphine, tri(cyclohexyl)phosphine, tri(t-butyl)phosphine, dicyclohexylphenylphosphine, 1,1'-bis(di-t-butylphosphino)ferrocene, 2-dicyclohexylphosphino-2'-dimethylamino-1,1'-biphenyl and 2-(di-t-butylphosphino)biphenyl, and phosphine mimic ligands such as imidazol-2-ylidenecarbenes.
Examples of the base include sodium carbonate, potassium carbonate, cesium carbonate, cesium fluoride, potassium fluoride, potassium phosphate, potassium acetate, triethylamine, potassium hydroxide, sodium hydroxide, sodium methoxide, lithium methoxide and the like.
Examples of the organic solvent include hydrocarbon-type solvents such as toluene, xylene and hexane, halogen-type solvents such as methylene chloride, sulfoxide-type solvents such as dimethyl sulfoxide, amide-type solvents such as dimethylformamide, ether-type solvents such as tetrahydrofuran, dioxane, and diglyme, heterocyclic-type solvents such as pyridine and the like. Two or more kinds of organic solvents may be mixed and used.

### [Preparation Method 4] (Step h)

Compound (VI) can be prepared by halogenating a compound represented by the formula (VII) wherein AR', W', Link, Y', H, and group OH have the same meanings as defined above [hereinafter simply referred to as "Compound (VII)"], which is commercially available, or can be synthesized by a known method or a method similar thereto. As for the halogenation, examples of chlorination include a preparation method described in ordinary publications in the filed of chemistry, for example, Shin Jikken Kagaku Koza (edited by Chemical Society of Japan, published by Maruzen Co., Ltd.), vol. 14, p.354. Examples of the method include a method utilizing chlorine (Cl₂), a method utilizing sulfuryl chloride, and the like. Examples of bromination include a preparation method described in ordinary publications in the filed of chemistry, for example, Shin Jikken Kagaku Koza (edited by Chemical Society of Japan, published by Maruzen Co., Ltd.), vol. 14, p.354. Examples of the method include a method utilizing bromine (Br₂), a method utilizing N-bromosuccinimide, and the like. Examples of iodination include a preparation method described in ordinary publications in the filed of chemistry, for example, Shin Jikken Kagaku Koza (edited by Chemical Society of Japan, published by Maruzen Co., Ltd.), vol. 14, p.423. Examples of the method include a method utilizing iodine (I₂), a method utilizing potassium triiodide, and the like.

### [Preparation Method 5]

As shown in the following scheme 5: 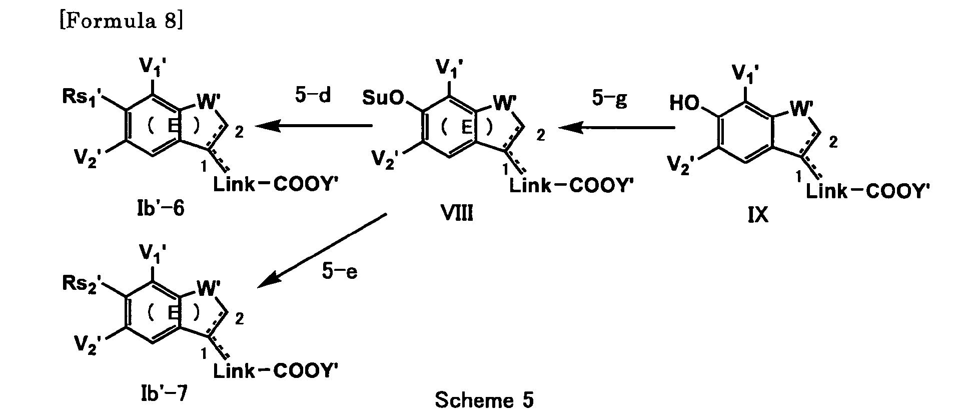 a compound represented by the formula (Ib'-6) wherein V1', V2', W', Link, and Y' have the same meanings as defined above [hereinafter simply referred to as "Compound (Ib'-6)"] as Compound (I) of the present invention wherein Rs' represents Rs1', and Rs1' represents a group [-N(Ry)(Rz)] which may be protected, or A1 and A2 in the group Rb which may be protected represent a single bond and [-N(R4)], respectively, and a compound represented by the formula (Ib'-7) wherein V1', V2', W', Link, and Y' have the same meanings as defined above [hereinafter simply referred to as "Compound (Ib'-7)"] as Compound (I) of the present invention wherein Rs' represents Rs2', and Rs2' represents a single bond-Rx' can also be prepared by the following methods. When hydroxyl group or amino group reactive under the aforementioned reaction conditions or inhibiting the reactions exists in the group Rs, V1, V2, or W in the ring (E), this substituent is preferably protected.

### [Preparation Method 5] (Step d)

Compound (Ib'-6) can be prepared from a compound represented by the formula (VIII) wherein V1', V2', W', Link, and Y' have the same meanings as defined above, the group SuO represents methanesulfonate group, trifluoromethanesulfonate group, or an arenesulfonate group of which aromatic ring moiety may be substituted with one of T¹ or two or more of the same or different T¹ [hereinafter simply referred to as "Compound (VIII)"], which is commercially available, or can be synthesized by a known method or a method similar thereto, by introduction of a substituted amine constituting the substituent Rs1', which is commercially available, or can be prepared by a known method or a method similar thereto, according to the method described in Preparation Method 4, Step d.

### [Preparation Method 5] (Step e)

Compound (Ib'-7) can be prepared from Compound (VIII) by a reaction with a boronic acid derivative constituting the substituent Rs2', which is commercially available, or can be prepared by a known method or a method similar thereto, according to the method described in Preparation Method 4, Step e..

### [Preparation Method 5] (Step g)

Compound (VIII) can be prepared by sulfonic acid esterification of a compound represented by the formula (IX) wherein V1', V2', W', Link, Y', and the group OH have the same meanings as defined above [hereinafter simply referred to as "Compound (IX)"], which is commercially available, or can be synthesized by a known method or a method similar thereto. As for the sulfonic acid esterification, examples of the method include a method of performing the preparation according to a method described in ordinary literature in the chemical field, for example, Shin Jikken Kagaku Koza (edited by the Chemical Society of Japan, published by Maruzen Co., Ltd.), vol. 14, p.1793. For example, a method of using sulfonyl chloride, a method of using sulfonic anhydride, and the like are available.

### [Preparation Method 6] (Step e)

As shown in the following scheme 6: 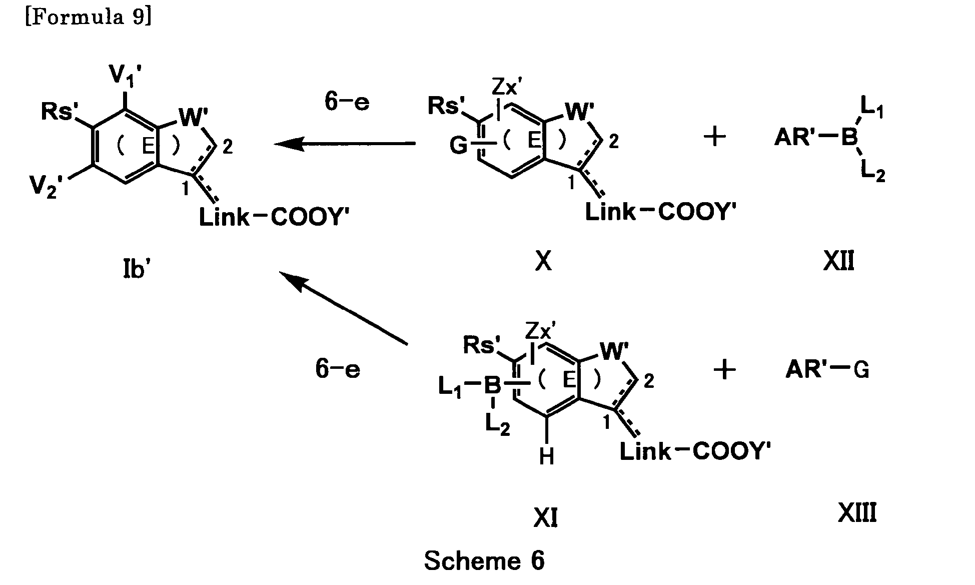 Compound (Ib') can also be prepared by reacting a compound represented by the formula (X) wherein Rs', Zx', G,W', Link, and Y' have the same meanings as defined above [hereinafter simply referred to as "Compound (X)"], which is commercially available, or can be synthesized by a known method or a method similar thereto, and a boronic acid derivative represented by the formula (XII) wherein AR' has the same meaning as defined above, B represents boron atom, L¹ and L² independently represent hydroxyl group, an alkoxyl group having 1 to 8 carbon atoms (for example, methoxy group, ethoxy group, propoxy group, isopropoxy group, cyclohexyloxy group), or a substituted or unsubstituted phenyloxy group, or L¹ and L² binds each other to represents a 5- or 6-membered cyclic ester of an arylboronic acid forming a ring containing boron atom [this ring may be saturated or unsaturated, may be a ring containing a heteroatom other than boron (for example, oxygen atom), and may be further substituted] (e.g., 9-borabicyclo[3,3,1]nonane, 1,3,2-dioxaborolane, 4,4,5,5-tetramethyl-1,3,2-dioxaborolane) [hereinafter simply referred to as "Compound (XII)"].
Examples of the preparation method of Compound (Ib') also include a method of reacting a combination of a compound represented by the formula (XI) wherein Rs', Zx', W', Link, Y', B, L1, and L2 have the same meanings as defined above [hereinafter simply referred to as "Compound (XI)"] and a compound represented by the formula (XIII) wherein AR' and G have the same meanings as defined above [hereinafter simply referred to as "Compound (XIII)"].
Specifically, the preparation can be performed with one or both combinations shown in the scheme 6 according to the method described in Preparation Method 4, Step e.

### [Preparation Method 7] (Step i)

As Compound (XII) and Compound (XI), commercially available regents can be used, or as shown in the following scheme 7: 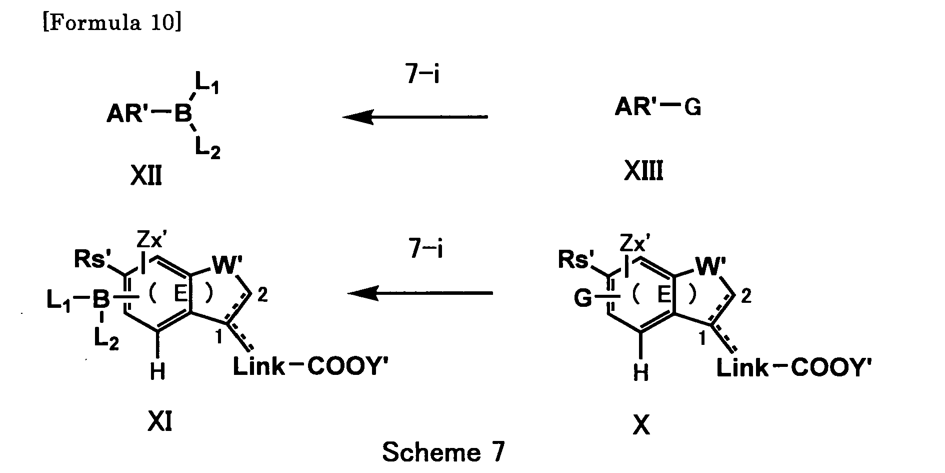 they can be prepared from a compound represented by the formula (XIII) wherein AR', and G have the same meanings as defined above [hereinafter simply referred to as "Compound (XIII)"] and a compound represented by the formula (X) (Rs', G, W', Link, Y' and H have the same meanings as defined above [hereinafter simply referred to as "Compound (X)"], which are commercially available, or can be synthesized by a known method or a method similar thereto, according to the methods described in the aforementioned literatures [Chemical Review, 1995, vol. 95, p.2457; Satoh, Y., SYNTHESIS, 1994, p.1146] or references cited therein. When hydroxyl group or amino group reactive under the aforementioned reaction conditions or inhibiting the reactions exists in Rs, Zx, or W in the ring (E), this substituent is preferably protected.
Examples include, for example, a method of preparing Compound (XII) or Compound (XI) by converting Compound (XIII) or Compound (X) into a lithio-compound using an alkyl lithium such as n-butyl lithium and t-butyl lithium, then reacting the product with a trialkyl borate and treating the product with a mineral acid such as hydrochloric acid, sulfuric acid, and phosphoric acid, a method of preparing Compound (XII) or Compound (XI) by performing a cross-coupling reaction of Compound (XIII) or Compound (X) and an (alkoxyl)diboron in the presence of a palladium catalyst and a base, and the like.

### [Preparation Method 8] (Step d)

As shown in the following scheme 8: 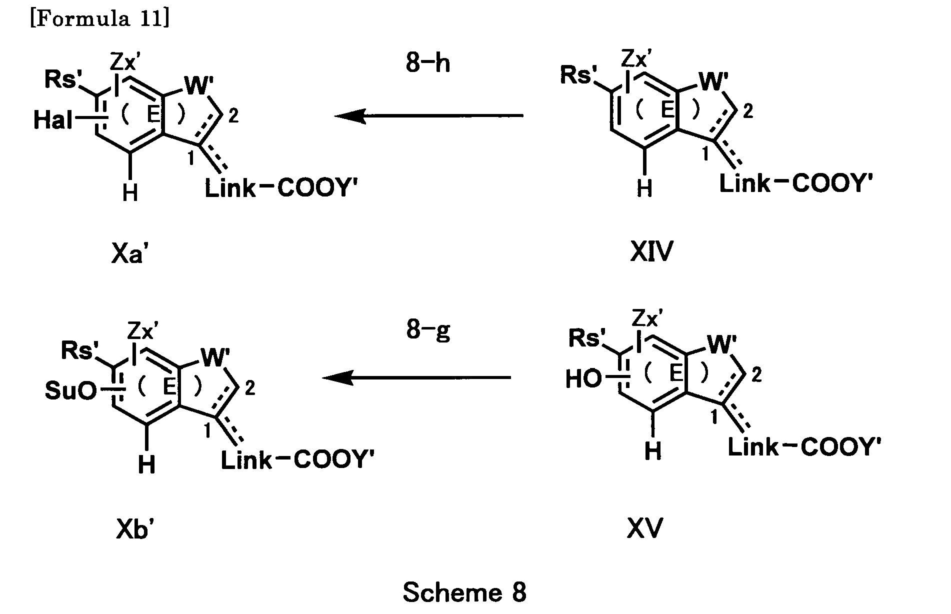 a compound represented by the formula (Xa') wherein Rs', Zx', W', Link, Y', and the group Hal have the same meanings as defined above [hereinafter simply referred to as "Compound (Xa')"] as Compound (X) wherein G represents a halogen atom such as chlorine atom, bromine atom, and iodine atom, and a compound represented by the formula (Xb') wherein Rs', Zx', W', Link, Y', and the group SuO have the same meanings as defined above [hereinafter simply referred to as "Compound (Xb')"] as Compound (X) wherein G represents a sulfonic acid ester group such as mesylate group, triflate group, and arenesulfonate group can be prepared by the methods described below. When hydroxyl group, amino group or the like reactive under the aforementioned reaction conditions or inhibiting the reactions exists in Rs, Zx, or W in the ring (E), this substituent is preferably protected.

### [Preparation Method 8] (Step h)

Compound (Xa') can also be prepared by halogenating a compound represented by the formula (XIV) wherein Rs', Zx', W', Link, Y', and H have the same meanings as defined above [hereinafter simply referred to as "Compound (XIV)"], which is commercially available, or can be synthesized by a known method or a method similar thereto, according to the method described in Preparation Method 4, Step h.

### [Preparation Method 8] (Step g)

Compound (Xb') can also be prepared by sulfonic acid esterification of a compound represented by the formula (XV) wherein Rs', Zx', W', Link, Y', and H have the same meanings as defined above, and group OH represents hydroxyl group [hereinafter simply referred to as "Compound (XV)"], which is commercially available or can be synthesized by a known method or a method similar thereto, according to the method described in Preparation Method 5, Step g.

### [Preparation Method 9]

As shown in the following scheme 9: 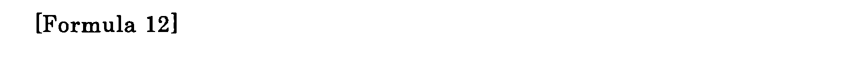 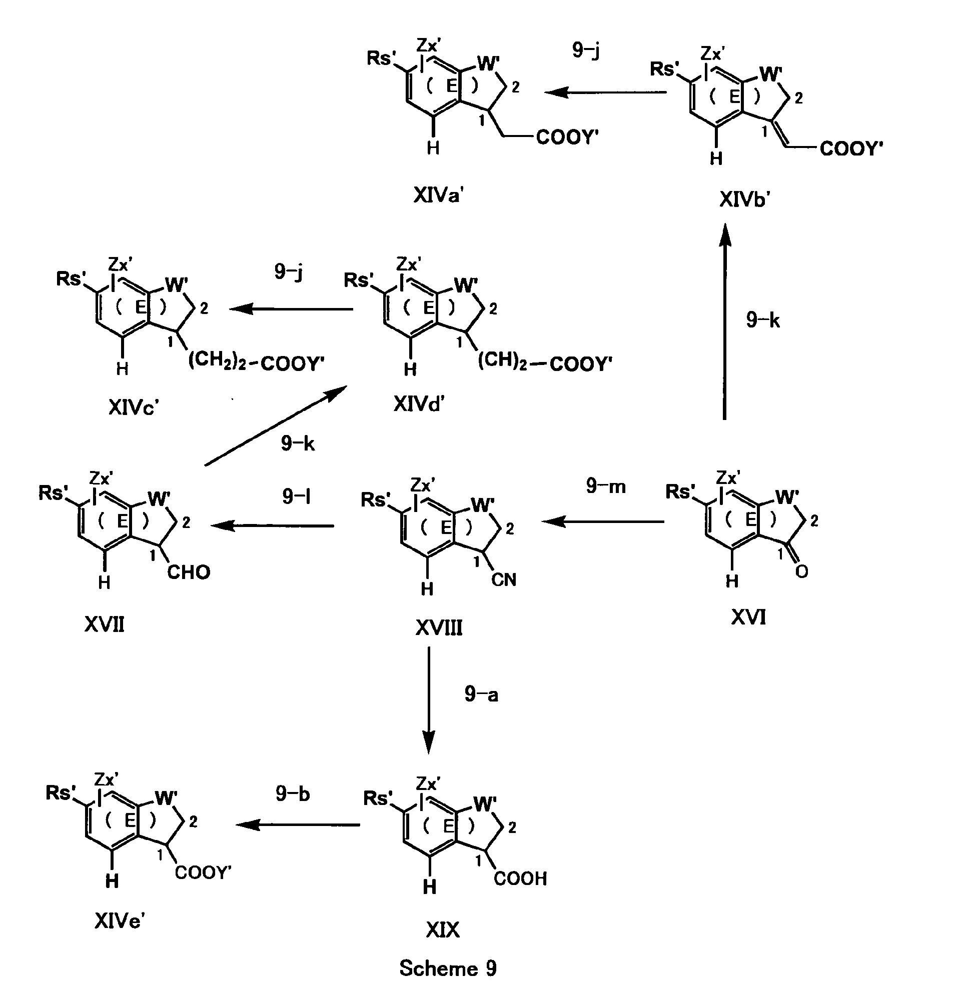 a compound represented by the formula (XIVa') wherein Rs', Zx', W', Y', and H have the same meanings as defined above [hereinafter simply referred to as "Compound (XIVa')"] as Compound (XIV) mentioned above wherein Link represents a saturated hydrocarbon having one carbon atom, a compound represented by the formula (XIVb')
wherein Rs', Zx', W', Y', and H have the same meanings as defined above [hereinafter simply referred to as "Compound (XIVb')"] as Compound (XIV) mentioned above wherein Link represents an unsaturated hydrocarbon having one carbon atom, a compound represented by the formula (XIVc') wherein Rs', Zx', W', Y', and H have the same meanings as defined above [hereinafter simply referred to as "Compound (XIVc')"] as Compound (XIV) mentioned above wherein Link represents a saturated hydrocarbon having two carbon atoms, a compound represented by the formula (XIVd') wherein Rs', Zx', W', Y', and H have the same meanings as defined above) [hereinafter simply referred to as "Compound (XIVd')"] as Compound (XIV) mentioned above wherein Link represents an unsaturated hydrocarbon having two carbon atoms, and a compound represented by the formula (XIVe') wherein Rs', Zx', W', Y', and H have the same meanings as defined above) [hereinafter simply referred to as "Compound (XIVe')"] as Compound (XIV) mentioned above wherein Link represents a single bond can also be prepared by a combination of the following methods. When hydroxyl group, amino group or the like reactive under the aforementioned reaction conditions or inhibiting the reactions exists in Rs, Zx, or W in the ring (E), this substituent is preferably protected.

### [Preparation Method 9] (Step j)

Compound (XIVa') and Compound (XIVc') can be prepared by reducing the double bond of Compound (XIVb') or Compound (XIVd') using a reduction reaction described in ordinary publications in the filed of chemistry. Examples of the reaction include a method of converting the double bond of Compound (XIVb') or Compound (XIVd') into a single bond by hydrogenation using a hydrogen source such as hydrogen gas, ammonium formate, and hydrazine hydrate in a single solvent or a mixed solvent of alcoholic-type solvents such as methanol and ethanol, and ester-type solvents such as ethyl acetate in the presence of a catalyst such as palladium/carbon powder, platinum oxide (PtO₂), and activated nickel.

### [Preparation Method 9] (Step k)

Compound (XIVb') and Compound (XIVd') can be prepared from a compound represented by the formula (XVI) wherein Rs', Zx'W', and H have the same meanings as defined above, and O represents oxygen atom [hereinafter simply referred to as "Compound (XVI)"] and a compound represented by the formula (XVII) wherein Rs', Zx'W', and H have the same meanings as defined above, and the group CHO represents an aldehyde group [hereinafter simply referred to as "Compound (XVII)"], which are commercially available, or can be synthesized by a known method or a method similar thereto, by any one of the following methods.

### [Preparation Method 9] (Step k-1)

Examples of the preparation method includes a method utilizing the Horner-Emonds reaction described in Shin Jikken Kagaku Koza (edited by Chemical Society of Japan, published by Maruzen Co., Ltd.), vol. 14, p.238. Specifically, the compound can be obtained by reacting Compound (XVI) or Compound (XVII) with a dialkylphosphonoacetic acid ester, which is commercially available, or can be synthesized by a known method or a method similar thereto, in an inert solvent, for example, an alcohol-type solvent such as methanol and ethanol or ether-type solvent such as tetrahydrofuran and dimethoxyethane in the presence of a base such as sodium hydride and sodium alkoxide.

### [Preparation Method 9] (Step k-2)

Examples also include a method of performing the preparation by the Reformatsky reaction described in Jikken Kagaku Koza (edited by Chemical Society of Japan, published by Maruzen Co., Ltd.), vol. 25, p.73.. Specifically, the compounds can be obtained by reacting Compound (XVI) or Compound (XVII) with an α-haloester, which is commercially available, or can be synthesized by a known method or a method similar thereto, in an inert solvent, for example, a benzene type solvent such as benzene, toluene, and xylene, an ether such as tetrahydrofuran and dioxane, or the like, in the presence of zing powder. Examples also include a method of adding iodine flakes, or copper chloride, and a method of using ultrasonication.

### [Preparation Method 9] (Step k-3)

Examples further include a method of performing the preparation by the Wittig reaction described in Shin Jikken Kagaku Koza (edited by the Chemical Society of Japan, published by Maruzen Co., Ltd.), vol. 14, p.1045. Specifically, the compounds can be obtained by reacting Compound (XVI) or Compound (XVII) with a carboalkoxytriphenylphospholane, which is commercially available, or can be synthesized by a known method or a method similar thereto, in a benzene solvent such as benzene, toluene, and xylene, an ether such as tetrahydrofuran and dioxane, or the like.

### [Preparation Method 9] (Step 1)

Compound (XVII) can be prepared from a compound represented by the formula (XVIII) wherein Rs', Zx', W', and H have the same meanings as defined above, and the group CN represents cyano group [hereinafter simply referred to as "Compound (XVIII)"], which is commercially available, or can be synthesized by a known method or a method similar thereto, according to, for example, the method described in Jikken Kagaku Koza (edited by the Chemical Society of Japan, published by Maruzen Co., Ltd.), vol. 21, p.89. Specifically, the compound can be prepared by making an aluminum hydride regent such as lithium aluminum hydride, diisobutylaluminum hydride, sodium triethoxyaluminum hydride, triethoxylithium aluminum hydride, and diethoxylithium aluminum hydride act on Compound (XVIII) in an inert solvent, for example, a benzene solvent such as benzene, toluene and xylene, an ether such as tetrahydrofuran and dioxane, or the like.

### [Preparation Method 9] (Step m)

Compound (XVIII) can also be synthesized from Compound (XVI) by any one of the following methods.

### [Preparation Method 9] (Step m-1)

For example, the preparation can be performed according to the method described in literatures [Dalko et al., Journal of Organic Chemistry, 1998, p.8107; Crombie et al., Journal of Chemical Society PERKIN TRANSI, 1982, p.1477]. Specifically, the compound can be prepared by reacting Compound (XVI) with commercially available p-tolylsulfonylmethyl isocyanide in an ether type solvent such as tetrahydrofuran, dioxane and ethylene glycol dimethyl ether in the presence of a base such as potassium t-butoxide and sodium ethoxide.

### [Preparation Method 9] (Step m-2)

The compound can also be prepared from Compound (XVI) according to the method described in Jikken Kagaku Koza, 4th Edition (edited by the Chemical Society of Japan, published by Maruzen Co., Ltd.), vol. 20, p.445. Specifically, the reaction is performed with trimethylsilyl cyanide and a Lewis acid, especially zinc iodide, as catalysts in an inert solvent such as tetrahydrofuran. Then, the reduction reaction using a hydrosilane described in Jikken Kagaku Koza, 4th Edition (edited by the Chemical Society of Japan, published by Maruzen Co., Ltd.), vol. 26, p.197 is performed. Examples of the method for the reduction reaction include a method of performing the reduction in a halogenated hydrocarbon solvent such as dichloromethane with a hydrosilane such as triethylsilane, a proton acid such as trifluoroacetic acid or a Lewis acid such as boron trifluoride, and the like.

### [Preparation Method 9] (Step b)

Compound (XIVe') can be prepared by esterifying a compound represented by the formula (XIX) wherein Rs', Zx', W', and H have the same meanings as defined above, and the group COOH represents carboxyl group [hereinafter simply referred to as "Compound (XIX)"], which is commercially available, or can be synthesized by a known method or a method similar thereto, according to the method described in Preparation Method 2, Step b.

### [Preparation Method 9] (Step a)

Compound (XIX) can be prepared by hydrolyzing Compound (XVIII) according to the method described in Preparation Method 1, Step a.

### [Preparation Method 10]

As shown in the following scheme 10: 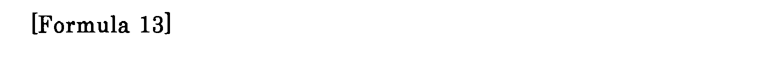 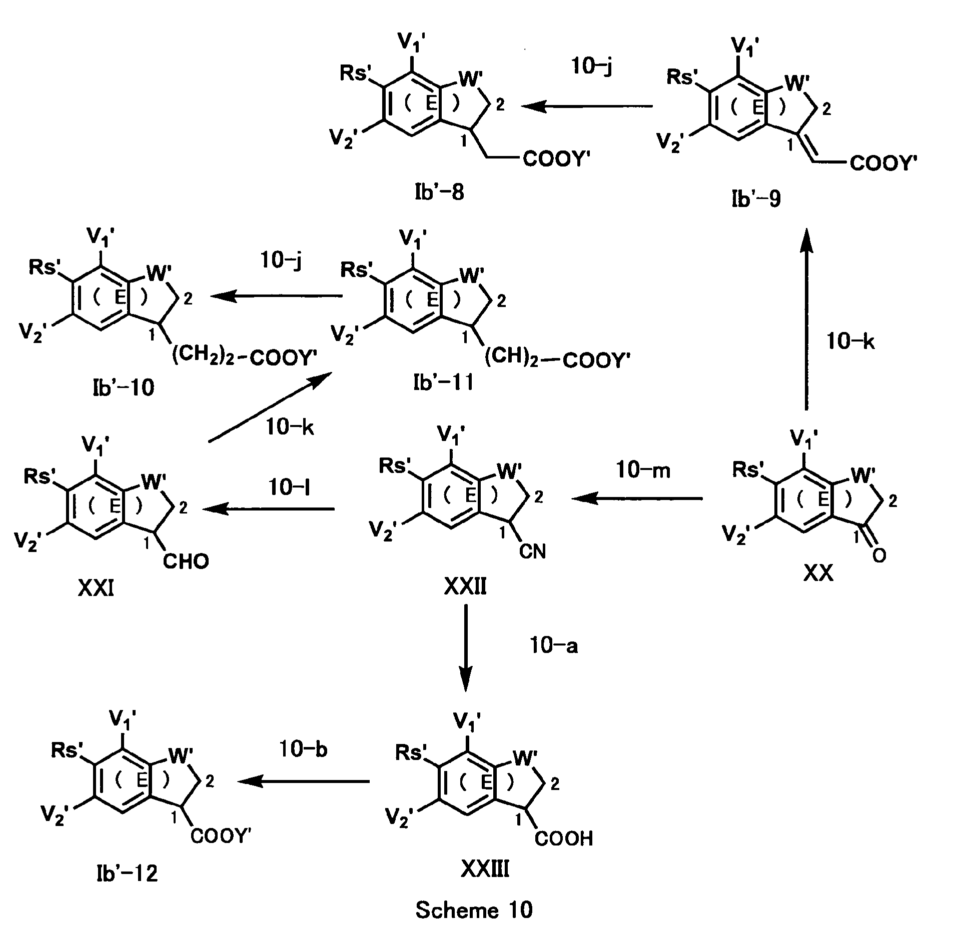 a compound represented by the formula (Ib'-8) wherein Rs', V1', V2', W, and Y' have the same meanings as defined above [hereinafter simply referred to as "Compound (Ib'-8)"], which falls within the scope of Compound (I) of the present invention, and corresponds to Compound (I) wherein Link represents a saturated hydrocarbon having one carbon atom, a compound represented by the formula (Ib'-9) wherein Rs', V1', V2', W', and Y' have the same meanings as defined above [hereinafter simply referred to as "Compound (Ib'-9)"], which falls within the scope of Compound (I) of the present invention, and corresponds to Compound (I) wherein Link represents an unsaturated hydrocarbon having one carbon atom, a compound represented by the formula (Ib'-10) wherein Rs', V1', V2', W', and Y' have the same meanings as defined above [hereinafter simply referred to as "Compound (Ib'-10)"], which falls within the scope of Compound (I) of the present invention, and corresponds to Compound (I) wherein Link represents a saturated hydrocarbon having two carbon atoms, a compound represented by the formula (Ib'-11) wherein Rs', V1', V2', W, and Y' have the same meanings as defined above) [hereinafter simply referred to as "Compound (Ib'-11)"], which falls within the scope of Compound (I) of the present invention, and corresponds to Compound (I) wherein Link represents an unsaturated hydrocarbon having two carbon atoms, and a compound represented by the formula (Ib'-12)
wherein Rs', V1', V2', W', and Y' have the same meanings as defined above [hereinafter simply referred to as "Compound (Ib'-12)"], which falls within the scope of Compound (I) of the present invention, and corresponds to Compound (I) wherein Link represents a single bond can also be prepared by a combination of the following methods. When hydroxyl group, amino group or the like reactive under the following reaction conditions or inhibiting the reactions exists in V1, V2 or W in the ring (E), this substituent is preferably protected.

### [Preparation Method 10] (Step j)

Compound (Ib'-8) and Compound (Ib'-10) can be prepared by reducing the double bond of Compound (Ib'-9) or Compound (Ib'-11) according to the method described in Preparation Method 9, Step j.

### [Preparation Method 10] (Step k)

Compound (Ib'-9) and Compound (Ib'-11) can be prepared from a compound represented by the formula (XX) wherein Rs', V1', V2', W' and O have the same meanings as defined above [hereinafter simply referred to as "Compound (XX)"], and a compound represented by the formula (XXI) wherein Rs', V1', V2', W' and the group CHO have the same meanings as defined above [hereinafter simply referred to as "Compound (XXI)"], which are commercially available, or can be synthesized by a known method or a method similar thereto, according to the method described in Preparation Method 9, Step k.

### [Preparation Method 10] (Step 1)

Compound (XXI) can be prepared from a compound represented by the formula (XXII) wherein Rs', V1', V2', W' and the group CN have the same meanings as defined above [hereinafter simply referred to as "Compound (XXII)"], which is commercially available, or can be synthesized by a known method or a method similar thereto, according to the method described in Preparation Method 9, Step 1.

### [Preparation Method 10] (Step m)

Compound (XXII) can be prepared from Compound (XX) according to the method described in Preparation Method 9, Step m.

### [Preparation Method 10] (Step b)

Compound (Ib'-12) can be prepared by esterifying a compound represented by the formula (XXIII) wherein Rs', V1', V2', W' and the group COOH have the same meanings as defined above [hereinafter simply referred to as "Compound (XXIII)"], which is commercially available, or can be synthesized by a known method or a method similar thereto, according to the method described in Preparation Method 2, Step b.

### [Preparation Method 10] (Step a)

Compound (XXIII) can be prepared by hydrolyzing Compound (XXII) according to the method described in Preparation Method 1, Step a.

### [Preparation Method 11] (Step e)

As shown in the following scheme 11: 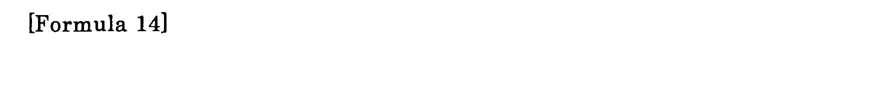 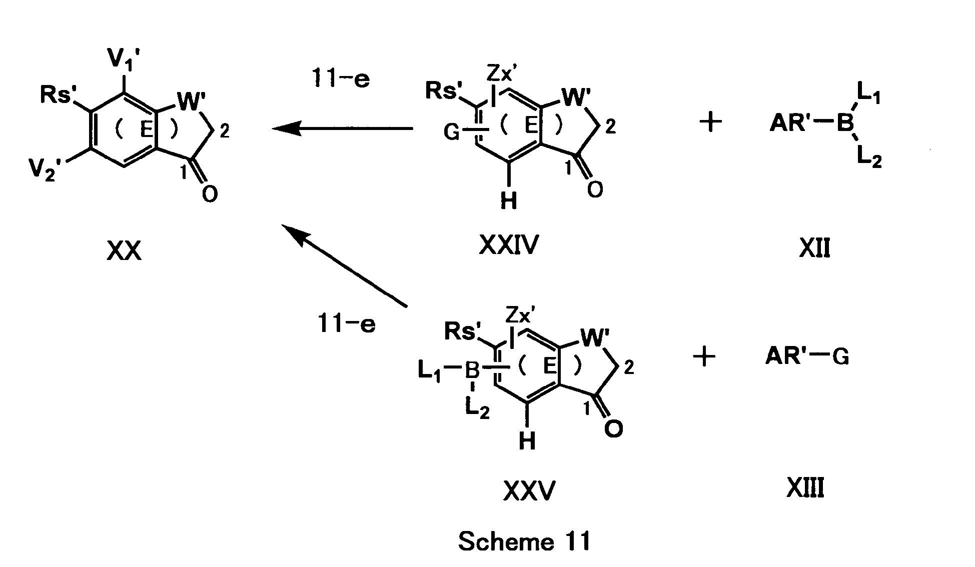 Compound (XX) can be prepared by reacting a compound represented by the formula (XXIV) wherein Rs', Zx', G, W', H, and O have the same meanings as defined above [hereinafter simply referred to as "Compound (XXIV)"], which is commercially available, or can be synthesized by a known method or a method similar thereto, and Compound (XII).

Examples of the preparation method of Compound (XX) further include a method of reacting a combination of a compound represented by the formula (XXV)
wherein Rs', Zx', B, L1', L2', W', H, and O have the same meanings as defined above [hereinafter simply referred to as "Compound (XXV)"], which is commercially available, or can be synthesized by a known method or a method similar thereto, and Compound (XIII).
Specifically, the preparation can be attained with both or either one of the combinations shown in the scheme 6 according to the method described in

### Preparation Method 4, Step e.

### [Preparation Method 12] (Step i)

As shown in the following scheme 12: 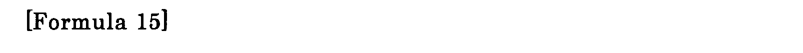 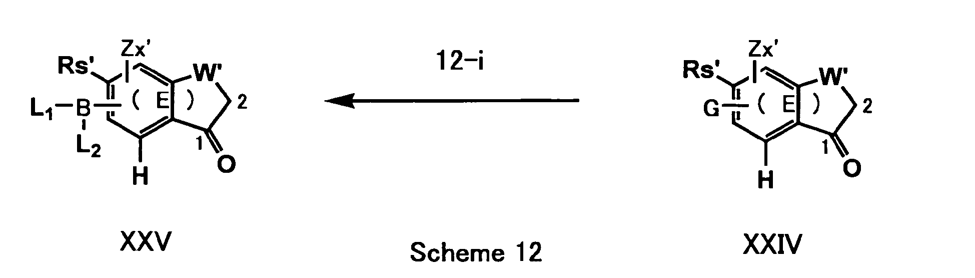 Compound (XXV) can be prepared from Compound (XXIV) according to the method described in Preparation Method 7, Step i mentioned above.

### [Preparation Method 13]

As shown in the following scheme 13: 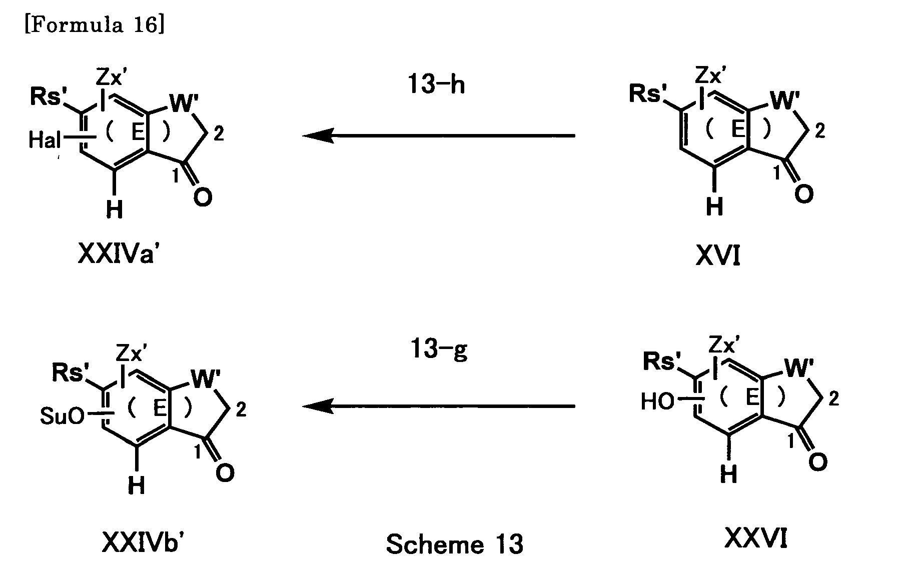 a compound represented by the formula (XXIVa') wherein Rs', Zx', W', H, O, and the group Hal have the same meanings as defined above [hereinafter simply referred to as "Compound (XXIVa')"] as Compound (XXIV) wherein G represents a halogen atom such as chlorine atom, bromine atom or iodine atom, and a compound represented by the formula (XXIVb') wherein Rs', Zx', W', H, O, and the group SuO have the same meanings as defined above [hereinafter simply referred to as "Compound (XXIVb')"] as Compound (XXIV) wherein G represents a sulfonic acid ester group such as mesylate group, triflate group or arenesulfonate group can be prepared by the following methods. When hydroxyl group, amino group or the like reactive under the aforementioned reaction conditions or inhibiting the reactions exists in Rs, Zx, or W in the ring (E), this substituent is preferably protected.

### [Preparation Method 13] (Step h)

Compound (XXIVa') can be prepared by halogenating Compound (XVI) according to the method described in Preparation Method 4, Step h mentioned above. [Preparation Method 13] (Step g)
Compound (XXIVb') can be prepared by sulfonic acid esterification of a compound represented by the formula (XXVI) wherein Rs', Zx', W', H, O, and the group OH have the same meanings as defined above [hereinafter simply referred to as "Compound (XXVI)"], which is commercially available or can be synthesized by a known method or a method similar thereto, according to the method described in Preparation Method 5, Step g mentioned above.

### [Preparation Method 14]

As shown in the following scheme 14: 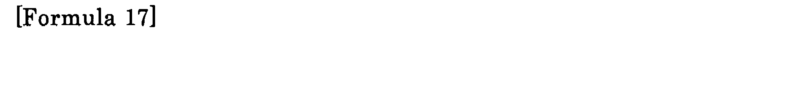 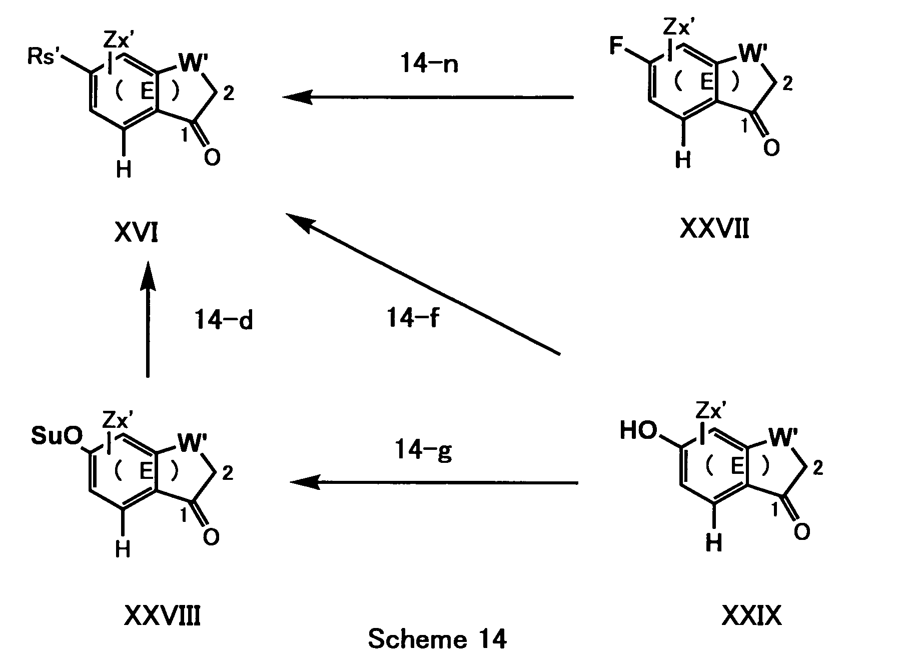 Compound (XVI) can also be prepared by a combination of the following methods.

### [Preparation Method 14] (Step n-1)

Compound (XVI) wherein Rs represents the aforementioned [-N(Ry)(Rz)] which may be protected, or Rs represents the aforementioned [Rs wherein A1 represents a single bond and A2 represents -N(R4) in Rb] which may be protected, or Rs represents [-S-Rx] can be prepared according to, for example, the method described in Shin Jikken Kagaku Koza (edited by the Chemical Society of Japan, published by Maruzen Co., Ltd.), vol. 14, p.1346, or the method described in Shin Jikken Kagaku Koza (edited by the Chemical Society of Japan, published by Maruzen Co., Ltd.), vol. 14, p.1716. Specifically, the compound can be prepared by reacting a compound represented by the formula (XXVII) wherein Zx', W', the groups SuO, H, and O have the same meanings as defined above, and F represents fluorine atom [hereinafter simply referred to as "Compound (XXVII)"], which is commercially available, or can be synthesized by a known method or a method similar thereto, with an amine derivative or thiol derivative constituting the aforementioned substituent Rs, which is commercially available, or can be prepared by a known method or a method similar thereto, in an inert solvent, for example, a halogenated hydrocarbon such as dichloromethane, chloroform, and 1,2-dichloroethane, an ether such as tetrahydrofuran and dioxane, an amide type solvent such as N,N-dimethylformamide or the like in the presence of a base such as potassium carbonate and sodium carbonate.

### [Preparation Method 14] (Step n-2)

Further, Compound (XVI) wherein Rs represents the aforementioned [-N(Ry)(Rz)] which may be protected, or Rs represents the aforementioned [Rs wherein A1 represents a single bond and A2 represents -N(R4) in Rb] which may be protected can be prepared according to the method of Preparation Method 14, Step n-1 by introducing [NH(Rz)] or [NH(Ry)] which may be protected, the aforementioned [primary amine wherein A1 represents a single bond and A2 represents NH in Rb] which may be protected, or the like, and then performing the reductive alkylation described in Shin Jikken Kagaku Koza (edited by the Chemical Society of Japan, published by Maruzen Co., Ltd.), vol. 14, p.1385. Specifically, the preparation can be attained by introducing the aforementioned [NH(Rz)] which may be protected, [NH(Ry)] which may be protected, or [primary amine wherein A1 represents a single bond and A2 represents NH in Rb] which may be protected, or the like into a compound represented by the formula (XXVII) wherein Zx', W', the groups SuO, H, and O have the same meanings as defined above, and F represents fluorine atom [hereinafter simply referred to as "Compound (XXVII)"], which is commercially available, or can be synthesized by a known method or a method similar thereto, according to the method of Preparation Method 14, Step n-1, then performing deprotection as required, reacting the resultant with an aldehyde or a ketone constituting Ry, Rz, or R4, which is commercially available, or can be prepared by a known method or a method similar thereto, in an organic solvent, for example, a halogenated hydrocarbon such as dichloromethane, chloroform, and 1,2-dichloroethane, an alcohol type solvent such as methanol and ethanol, or the like, and performing the reduction reaction simultaneously or stepwise in the presence of a reducing agent such as sodium cyanoborohydride, sodium triacetoxyborohydride and sodium borohydride.

### [Preparation Method 14] (Step f)

Compound (XVI) wherein Rs represents the aforementioned [-O-Rx] which may be protected can be prepared from a compound represented by the formula (XXIX) wherein Zx', W', O, H, and the group OH have the same meanings as defined above) [hereinafter simply referred to as "Compound (XXIX)"], which is commercially available, or can be synthesized by a known method or a method similar thereto, according to the method described in Preparation Method 4, Step f. Further, Compound (XXIV) can also be prepared by referring to the methods of the following known literatures. Compound (XXIV) wherein W on the ring (E) represents carbon atom can also be prepared by referring to Ingold et al., Journal of Chemical Society, 1923, p.1508; Johnson et al., Journal of American Chemical Society, 1944, p.218; Heinzelmann et al., Journal of American Chemical Society, 1948, p.1386; Hartmann et al., Journal of Medicinal Chemistry, 1994, p.1275) and the like, and references cited in these literatures. Compound (XXIV) wherein W on the ring (E) represents oxygen atom can also be prepared by referring to Slater et al., Journal of Chemical Society, 1920, p.314, and the like, and references cited in these literatures. Further, Compound (XXIV) wherein W on the ring (E) represents nitrogen atom can also be prepared by referring to Nimitz et al., Liebigs ofAnnnual Chemistry, 1987, p.765 and the like, references cited in these literatures.

### [Preparation Method 14] (Step d)

Compound (XVI) wherein Rs is the aforementioned [-N(Ry)(Rz)] which may be protected, or Rs is the aforementioned [Rs wherein A1 represents a single bond and A2 in Rb represents -N(R4) in Rb] which may be protected can be prepared by introducing a substituted amine constituting the substituent Rs', which is commercially available, or can be prepared by a known method or a method similar thereto, into Compound (XXVII) according to the method described in Preparation Method 4, Step d.

### [Preparation Method 14] (Step g)

Compound (XXVIII) can be prepared by sulfonic acid esterification of Compound (XXIX) according to the method described in Preparation Method 5, Step g.

The preparation method of Compound (I) is not limited to the methods described herein. For example, the compounds of the present invention can be produced by modifying or converting a substituent of a compound serving as a precursor of the compounds according to a method or a combination of methods described in ordinary publications in the filed of chemistry, and the like.
Examples of the preparation method for Compound (I) of the present invention which contains an asymmetric carbon in one of Rs and the ring (E) include a method of using a starting material in which a moiety corresponding to the asymmetric carbon in Rs or the ring (E) is already optically active, which is commercially available or can be prepared by a known method or a method similar thereto. A method is also available in which the compound of the present invention or a precursor thereof is separated as an optically active isomer in a conventional manner. Examples of such method include, for example, a method utilizing high performance liquid chromatography (HPLC) using a chiral column, a method comprising formation of a salt with an optically active acid or base, separation and purification of the formed salt, and the following neutralization of the salt to again obtain the compounds of the present invention or precursors thereof in free forms, a method comprising condensation with an optically active regent to form a diastereomer, successive separation and purification, followed by decomposition, and the like. Further, examples of the preparation method for Compound (I) of the present invention which contains an asymmetric carbon in both Rs and the ring (E) include a method of using starting materials in which moieties corresponding to the asymmetric carbons in Rs or the ring (E) are already optically active, which are commercially available or can be prepared by a known method or a method similar thereto. Examples of such method also include a method of separating diastereomers of the compounds of the present invention or precursors thereof in a conventional manner. When a precursor is separated to obtain an optical isomer, optically active Compound (I) of the present invention can then be prepared by performing the aforementioned preparation methods.

Examples of the method for preparing optically active Compound (I) of the present invention or precursor thereof include the following methods and the like.

### [Preparation Method 15]

As shown in the following scheme 15: 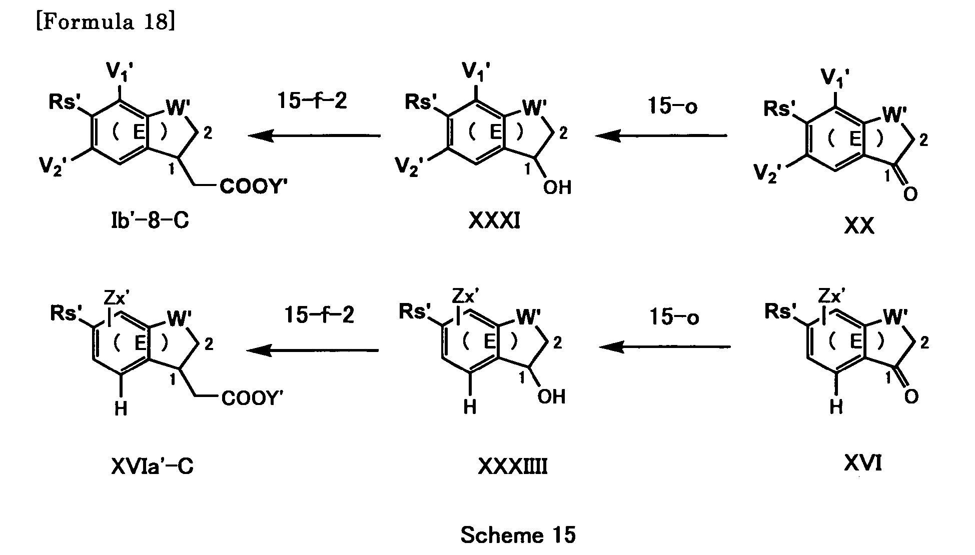 a compound represented by the formula (Ib'-8-C) wherein Rs', V1', V2', W', and Y' have the same meanings as defined above, and Link represents a saturated hydrocarbon having one carbon atom [hereinafter simply referred to as "Compound (Ib'-8-C)"], which falls within the scope of Compound (Ib'-8), wherein the carbon atom at the 1-position of the ring (E) is optically active, and which represents one of the stereoisomers, and a compound represented by the formula (XVIa'-C) wherein Rs', Zx', W', Y', and H have the same meanings as defined above, and Link represents a saturated hydrocarbon having one carbon atom [hereinafter simply referred to as "Compound (XVIa'-C)"] , which falls within the scope of Compound (XVIa'), wherein the carbon atom at the 1-position of the ring (E) is optically active, and which represents one of the stereoisomers, can also be prepared by a combination of the following methods.

### [Preparation Method 15] (Step f-2)

Compound (Ib'-8-C) and Compound (XVIa'-C) can be prepared by reacting a compound represented by the formula (XXXI) wherein Rs', V1', V2', W, and the group OH have the same meanings as defined above [hereinafter simply referred to as "Compound (XXXI)"], or a compound represented by the formula (XXXIII) wherein Rs', Zx', W', Y', and the group OH have the same meanings as defined above [hereinafter simply referred to as "Compound (XXXIII)"], which is commercially available, or can be synthesized by a known method or a method similar thereto, is optically active, and represents one of the stereoisomers, with a methanetricarboxylic acid triester such as triethyl methanetricarboxylate according to the method described in Preparation Method 4, Step f-2, then performing hydrolysis according to the method described in Preparation Method 1, Step a, further performing decarbonylation of the produced tricarboxylic acid according to a method described in an ordinary literature in the field of chemistry, and then esterifying the carboxylic acid according to the method of Preparation Method 2, Step b. As for the reaction conditions, known literatures (Hillier et al., Organic Letters, 2004, p.573 and the like) and references cited in these literatures can be referred to. When the stereoselectivity is
insufficient in the aforementioned reactions, Compound (Ib'-8-C) or Compound (XVIa'-C) as a free single stereoisomer can be obtained by forming a salt of optically active Compound (Ib'-8-C) or Compound (XVIa'-C) with an optically active base such as α-methylbenzylamine, separating and purifying the produced salt by crystallization or the like in an organic solvent, and then neutralizing the salt.

### [Preparation Method 15] (Step o)

Optically active Compound (XXXI) and Compound (XXXIII) can be prepared from Compound (XX) or Compound (XVI) according to the method described in known literatures (Hashiguchi et al., Journal of American Chemical Society, 1995, p.7562; Fujii et al., Journal of American Chemical Society, 1996, p.2521; Liu et al., Organic Letters, 2004, p.169, and the like) and references cited in these literatures. Specifically, optically active Compound (XXXI) and Compound (XXXIII) having an objective steric configuration can be prepared by making a combination of a ruthenium catalyst such as ruthenium chloride/[(S,S)-N-(p-toluenesulfonyl)-1,2-diphenylethylenediamine](p-cymene), ruthenium chloride/[(R,R)-N-(p-toluenesulfonyl)-1,2-diphenylethylenediamine](p-cymene), ruthenium chloride/[(S,S)-N-(p-methanesulfonyl)-1,2-diphenylethylenediamine](p-cymene) and ruthenium chloride/[(R,R)-N-(p-methanesulfonyl)-1,2-diphenylethylenediamine](p-cymene) and a ligand act on Compound (XX) or Compound (XVI) in a mixture of formic acid and triethylamine.

When Compound (I) of the present invention contains an acidic functional group such as carboxyl group or phenolic hydroxyl group, the compound can be converted into pharmaceutically acceptable salt (e.g., inorganic salts with sodium, ammonia and the like, or organic salts with triethylamine and the like) by a known means. For example, when an inorganic salt is to be obtained, it is preferable to dissolve Compound (I) of the present invention in water containing at least 1 equivalence of hydroxide, carbonate, bicarbonate or the like corresponding to a desired inorganic salt. For the reaction, an inactive water-miscible organic solvent such as methanol, ethanol, acetone, and dioxane may be mixed. For example, by using sodium hydroxide, sodium carbonate, or sodium hydrogencarbonate, a solution of sodium salt can be obtained.
When Compound (I) of the present invention contains a basic functional group such as amino group in the compound, or when Compound (I) of the present invention contains an aromatic ring which itself has properties of base (e.g., pyridine ring) in the compound, the compound can be converted into a pharmaceutically acceptable salt (e.g., salt with inorganic acids such as hydrochloric acid and sulfuric acid, or salts with organic acids such as acetic acid and citric acid) by a known means. For example, when an inorganic salt is to be obtained, it is preferable to dissolve Compound (I) of the present invention in water containing at least 1 equivalence of a desired inorganic acid. For the reaction, an inactive water-miscible organic solvent such as methanol, ethanol, acetone, and dioxane may be mixed. For example, by using hydrochloric acid, a solution of hydrochloride can be obtained.
When a solid salt is desired, a solution may be evaporated, or a water-miscible organic solvent having polarity to some extent, such as butanol or ethyl methyl ketone, can be added to obtain a solid salt thereof.
The various compounds disclosed by the present invention can be purified by known methods such as recrystallization, and variety of chromatography techniques (column chromatography, flash column chromatography, thin layer chromatography, high performance liquid chromatography).

Compound (I) of the present invention and pharmacologically acceptable salts thereof have an action of suppressing the production of both of prostaglandins and leukotrienes. The action of suppressing the production of prostaglandins and/or leukotrienes includes, for example, an action of suppressing PGE₂ production, observed when cells of MG-63 which is a cultured human osteosarcoma cell line are stimulated with IL-1β and/or PGD₂ and LTB₄ production observed when cells of RBL-2H3 which is a cultured rat mastocytoma cell line are stimulated with IgE, by 10% or more, preferably 30% or more, most preferably 50% or more, compared with a positive control at a concentration of the compound not having cytotoxicity. As for a mode of action at a molecular level, it is considered that the compound of the present invention inhibits both of COX-1 and/or COX-2, which produce prostaglandins, and 5-LO, which produces leukotrienes. It is also considered that the compound of the present invention suppresses the production of arachidonic acid by inhibiting enzymatic activity of type 2A, 4, or 5 PLA₂ involved in prostaglandin and leukotrien production.

It is considered that, in these molecular action mechanisms, Compound (I) of the present invention inhibits the enzymatic activity of type 4 PLA₂. For the judgment, for example, the enzyme inhibitory action against type 4 PLA₂ can be examined, and known methods for measuring the enzymatic activity of type 4 PLA₂ are preferably utilized [Clark et al., Proceeding of National Academy of Science USA (Proc. Natl. Acad. Sci. USA), 1990, vol. 87, p.7708; Gronich et al., Biochemical Journal (Biochem. J.), 1990, vol. 271, p.37; Clark et al., Cell, 1991, vol. 65, p.1043; Kramer et al., Journal of Biological Chemistry (J. Biol. Chem), 1991, vol. 266, p.5268]. The type 4 PLA2 inhibitory action of the compounds of the present invention can be elucidated by employing these methods.

Compounds (I) of the present invention and pharmacologically acceptable salts thereof inhibited mouse inflammatory edema, allergic edema, acetic acid writhing reaction, and rat adjuvant arthritis by oral administration at a dose of 0.1 to 500 mg/kg, and caused no death of the mice by oral administration at a dose of 500 mg/kg/day for 3 days. Therefore, they are safe compounds as drugs for mammals, preferably humans, pets or companion animals such as dogs and cats, and farm animals, and they are useful substances as active ingredients of medicaments. Preferred examples of the medicaments for mammals, preferably humans, pets or companion animals such as dogs and cats, and farm animals include agents for prophylactic and/or therapeutic treatment of various conditions, various diseases, and pathological conditions in which an acute or chronic inflammatory reaction resulted from production of prostaglandin and/or leukotriene is observed, specifically inflammatory diseases, allergic diseases, autoimmune diseases, and pain.

More specifically, examples of the conditions and diseases to which the medicament of the present invention is applicable include arthritis, chronic rheumatoid arthritis, malignant rheumatoid arthritis, juvenile rheumatoid arthritis, Felty's syndrome, adult Still's disease, osteoarthritis, synovitis, gout, slack of artificial joint implant, fervescence, common cold, algesia, burn, thermal injury, keloplasty, menstrual pain, dysmenorrhea, menstrual cramp, allergic reaction, allergic contact hypersensitivity, allergic rhinitis, pollinosis, allergic conjunctivitis, hypersensitivity pneumonitis, allergic bronchopulmonary mycosis, emphysema, acute respiratory distress syndrome, asthma, bronchitis, chronic obstructive pulmonary disease, chronic bronchitis, pulmonary emphysema, diffuse panbronchiolitis, respiratory obstruction, graft versus host syndrome, urticaria, ultraviolet radiation dermatitis, atopic dermatitis, cancer, myelogenous leukemia, sarcomata, brain tumor, cachexia, tissue ulcer, digestive ulcer, gastritis, acute and chronic pancreatitis, regional enteritis, ulcerative colitis, diverticulitis, recurrent gastroenteric disorder, gastroenteric bleeding, inflammatory bowel disease, Crohn's disease, intestinal tract type Behcet's disease, infectious enteritis, ischemic enteritis, radiation enteritis, drug-induced enteritis, irritable bowel syndrome, hepatic diseases (hepatopathies, liver failures) such as acute hepatitis, fulminant hepatitis, chronic hepatitis, hepatic cirrhosis, fatty liver, alcoholic liver injury, drug liver injury (drug-induced hepatitis), congestive hepatitis, autoimmune hepatitis, primary biliary cirrhosis and hepatic porphyria, coagulation, anemia, ankylosing spondilitis, restenosis, periodontosis, epidermolysis bullosa, atherosclerosis, aortic aneurysm, periarteritis nodosa, congestive cardiac failure, arrhythmia, myocardial infarction, cerebral infarction, attack, cerebral ischemia, head injury, spinal cord injury, myelopathic muscular atrophy, neuralgia, neurodegenerative disease, Alzheimer's disease, Lewy body disease, Shy-Drager syndrome, Reye's syndrome, progressive supranuclear palsy, progressive multifocal leukoencephalopathy, normal pressure hydrocephalus, subacute sclerosing panencephalitis, frontal lobe type dementia, acute anterior poliomyelitis (poliomyelitis), poliomyelitis neurosis, viral encephalitis, Creutzfeldt-Jakob disease, Kuru disease, bovine spongiform encephalopathy (mad cow disease), scrapie, epilepsy, cerebral amyloid angiopathy, autoimmune disease, Huntington's disease, Parkinson's disease, migraine, depression, mania, manic-depressive psychosis, hereditary cerebellar ataxia, peripheral neuropathy, glaucoma, pain, gingivitis, postoperative pain, amyotrophic lateral sclerosis, osteoporosis, multiple sclerosis, ocular angiogenesis, cornea damage, macular degeneration, conjunctivitis, abnormal wound healing, sprain or strain of muscle or joint, tendinitis, skin disease, psoriasis vulgaris, pustular psoriasis, erythroderma psoriaticum, arthritic psoriasis, myasthenia gravis, multiple myositis, myositis, bursitis, diabetes mellitus, tumor invasion, tumor growth, tumor metastasis, cornea scar, scleritis, immunodeficiency disease, pachydermia, eosinophilic fasciitis, sepsis, endotoxin shock, premature delivery, hypoprothrombinemia, hemophilia, thyroiditis, sarcoidosis, Behcet's syndrome, hypersensitivity, renal disease, rickettsial infectious disease, protozoal disease, reproduction disease, sepsis shock and the like. Other specific conditions and diseases include toothache, pain after tooth extraction, back or low back pain, periarthritis humeroscapularis, cervico-omo-brachial syndrome, tenosynovitis, acute upper respiratory inflammation, herpes zoster, fibrosis, pulmonary fibrosis, drug induced pulmonary fibrosis, pneumoconiosis, chronic interstitial pneumonia, granulomatous interstitial pneumonia, fibrosing interstitial pneumonia, renal fibrosis, nephropyelitis, various types of secondary contracted kidney, glomerular nephritis, chronic nephritis, glomerulosclerosis, hepatic fibrosis, cardiac fibrosis after myocardial infarction, idiopathic cardiomyopathy, pancreatic sclerosis, pancreatic fibrosis, pancreatolithiasis, Takayasu's arteritis, chronic thyroiditis, dermatomyositis, multiple myositis, myelofibrosis, Banti disease, retroperitoneal fibrosis, various radiation injuries and the like. Further, the medicament comprising Compound (I) of the present invention as an active ingredient can be used for the aforementioned conditions or diseases of mammals, preferably humans, pets or companion animals such as dogs and cats or farm animals together with or in combination with one or more kinds of other prophylactic or therapeutic drugs.

Examples of the drugs that can be used together or in combination with the medicament of the present invention include, for example, the following drugs: immunomodulation-type antirheumatic drugs and antimetabolite used as therapeutic drugs for rheumatoid arthritis, specifically, gold preparations, bucillamine, lobenzarit, salazosulfapyridine, methotrexate, azathiopurin, mizoribine, leflunomide, tacrolimus, cyclosporin and the like and preparations containing the same; anti-cytokine antibody preparations directed to cytokines such as interleukin (IL) 1, IL-6, and tumor necrosis factor (TNF)-α or preparations of soluble receptors for those cytokines, which are biological preparations, specifically, infliximab, etanercept and the like and preparations containing the same; steroid preparations, specifically, dexamethasone, betamethasone, prednisolone, fluticasone, beclometasone and the like and preparations containing the same; bronchodilators used as therapeutic agents for chronic bronchial asthma, specifically, salmeterol and salbutamol, which are adrenalin β2 stimulants, ipratropium, which is an anticholinergic drug, and the like and preparations containing the same; therapeutic drugs for allergic diseases, for example, theophyline, which is a xanthine analogue drug, and the like, fexoquinadine, epinastatine, cetirizine, ketotifen, disodium cromoglycate, pemirolast and the like, which are antiallergic agents, fexoquinadine, cetirizine and the like, which are antihistaminic agents, and preparations containing the same; irinotecan, 5-fluorouracil and the like, which are antitumor agents, and preparations containing the same. Examples further include use of a medicament comprising Compound (I) of the present invention as an active ingredient, for example, together with or in combination with radiotherapy.

In order to use Compound (I) of the present invention or pharmaceutically acceptable salts thereof for the medicaments described above, an effective amount of Compound (I) of the present invention or a pharmaceutically acceptable salt thereof, per se, may be used, or the substance may be mixed with a pharmaceutically acceptable carrier to form a pharmaceutical composition. The carrier may be, for example, a suspending agent such as carboxymethylcellulose, or purified water, physiological saline or the like, if desired. Other known carriers can also be used. Examples include a method of dissolving Compound (I) of the present invention or a pharmaceutically acceptable salt thereof in purified water containing 0.5% carboxymethylcellulose and using the solution.
Examples of formulations for preparing the aforementioned pharmaceutical composition include tablet, powder, granule, syrup, suspension, capsule, and injection. For the manufacture of these formulations, various carriers suitable for these preparations are used. For example, examples of the carrier for oral preparations include excipients, binders, lubricants, fluid accelerators, and colorants.

When the medicament of the present invention is formulated as a parenteral preparation such as an injection, water for injection, physiological saline, glucose aqueous solution, vegetable oil for injection, propylene glycol, polyethylene glycol and the like can generally be used as a diluent. Disinfectants, antiseptics, stabilizers, isotonic agents, soothing agents and the like may be further added, as required.
When the medicament of the present invention is administered to a mammal, e.g., human, the medicament can be orally administered in the form of a tablet, a powder, a granule, a suspension, a capsule or the like. The compound can also be parenterally administered in the form of an inhalant, a suppository, a gel, a lotion, an ointment, a cream, or a spray. A dose thereof varies depending on a disease to be applied, administration route, age, weight, degree of symptom of a patient and the like. Examples of the dose include generally an administration at a dose of 1 to 1,000 mg per day for an adult once to three times a day. Every day administration for a period of several days to two months is commonly applied. The daily dose and the administration period may be increased or decreased depending on symptoms of a patient.

### Examples

The present invention will be further specifically explained with reference to examples. However, the scope of the present invention is not limited to the following examples, and the like.
In the examples, for thin layer chromatography (TLC), Precoated Silica Gel 60 F254 (produced by Merck, product number: 5715-1M)) was used. After development with chloroform:methanol (1:0 to 1:1), acetonitrile:acetic acid:water (200:1:1 to 100:4:4) or ethyl acetate:hexane (1:0 to 0:1), observation was performed by UV irradiation (254 nm) or color development with ninhydrine or dinitrophenylhydrazine solution in hydrochloric acid. For drying organic solvent, anhydrous magnesium sulfate or anhydrous sodium sulfate was used. As for column chromatography, the indication of "Quad" means use of Quad 1 preparative chromatography system (produced by Biotage), and one or several columns selected from cartridge columns KP-Sil-12M, 40S and 40M produced by the same manufacturer were used depending on the amount of sample. For flash column chromatography, Silica gel 60N (spherical shape, neutral, 40 to 100 µm, produced by Kanto Chemicals) was used. Preparative thin layer chromatography (hereinafter abbreviated as "PTLC") was performed by using one or several plates of PLC Plate Silica Gel 60 F254 (20 x 20 cm, thickness: 2 mm, concentration zone: 4 cm, produced by Merck, product number: 13793-1M) were used depending on the amount of sample.

The indication of "LCMS" means that mass spectrum was measured by liquid chromatography-mass spectrometry (LC-MS). Platform-LC type mass spectrometry apparatus (produced by Micromass) was used as the mass spectrometer, and the measurement was performed by the electrospray ionization (ESI) method. As a liquid chromatography apparatus, an apparatus produced by GILSON was used. As a separation column, Develosil C30-UG-5 (50 × 4.6mm) (produced by Nomura Kagaku Co., Ltd.) was used. Elution was generally performed at a flow rate of 2 ml/minute, and Solution A = water [containing 0.1% (v/v) acetic acid] and Solution B = acetonitrile [containing 0.1% (v/v) acetic acid] were used as solvents.
In the tables mentioned below, data indicated by "LCMS" mean data of liquid chromatography-mass spectrometry spectra. In the columns of "Mass", data of mass spectrometry were shown (the indication "N.D" means that no molecular ion peak was detected). In the columns of "method", elution conditions of the liquid chromatography are described. In the columns of "RTime", retention times in the liquid chromatography are shown. For the indication of retention time in the liquid chromatography, the indication "A" for elution condition means that measurement was performed by elution with a linear gradient of 5 to 100% (v/v) Solution B from 0 minute to 5 minutes and then with 98% Solution B until 9 minutes. Similarly, the indication "B" for elution condition means that measurement was performed by elution with a linear gradient of 5 to 98% (v/v) Solution B from 0 minute to 4 minutes, and then with 98% Solution B until 6 minutes. For the compounds with the indication C in the columns of elution conditions, data of mass spectrometry measured by fast atomic bombardment mass spectrometry (FAB-MS) using JEOL-JMS-SX102 (produced by JEOL Co., Ltd.) were mentioned in the columns of "Mass". Further, for the compounds with the indication D in the elution conditions, an apparatus manufactured by Waters Ltd. was used as a liquid chromatography apparatus. As a column for separation, Develosil C30-UG-5 (50 x 4.6 mm, Nomura Kagaku Co., Ltd.) was used. Measurement was performed under elution condition with a linear gradient of 5 to 98% (v/v) Solution B from 0 minute to 4 minutes and then with 98% Solution B until 6 minutes.

As the liquid chromatography apparatus used for "preparation of chiral compounds", Shimadzu LC6A System (Shimadzu Corporation) was used. As the separation column, Chiralcel OJ-RH (20 mm I.D × 250 mm, Daicel Chemical Industries., Ltd.) was used. Elution was performed at a flow rate of 10 ml/minutes and 70% Solution B by using Solution A = water, and Solution B = acetonitrile.
In the columns indicated as "Exp.", compound numbers are shown. In the columns indicated as "Int.", intermediate numbers are shown. The abbreviations used in the tables have the following meanings.
n: normal, i: iso, s: secondary, t: tertiary, c: cyclo, D: di, Me: methyl, Et: ethyl, Pr: propyl, Bu: butyl, Pen: pentyl, Hex: hexyl, Hep: heptyl, Ph: phenyl, Bn: benzyl, Py: pyridyl, Indan: indanyl, Ac: acetyl, CHO: formyl, COOH: carboxyl, NO2: nitro, DMA: dimethylamino, NH2: amino, CF3: trifluoromethyl, F: fluoro, Cl: chloro, Br: bromo, OMe: methoxy, OH: hydroxy, TFA: trifluoroacetyl, SO2: sulfonyl, CO: carbonyl, Nap: naphthyl, Ind: 1H-indolyl, 1HIdz: 1H-indazolyl, 2HIdz: 2H-indazolyl, Bzt: benzothiazole, 2ABzt: 2-aminobenzothiazole, BF: benzofuranyl, BT: benzo[b]thienyl, Qu: Quinolyl, IQ: isoquinolyl
The numbers given before the substituents indicate substituting positions. The numbers given with hyphens before abbreviations of aromatic rings indicate substituting positions of the aromatic rings. (S) indicates optically active substances with S-configuration, and (R) indicates optically active substances with R-configuration. Representative examples of the substituents shown in the tables with abbreviations are listed in Table 1 mentioned below.

**[Table 1]**

| Structure | abbreviation | Structure | abbreviation | Structure | abbreviation |
|---|---|---|---|---|---|
| 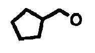 | cPenMeO | 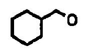 | cHexMeO | 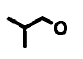 | iBuO |
| 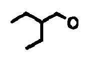 | 2EtBuO | 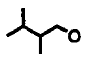 | 2,3DMeBuO | 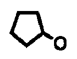 | cPenO |
| 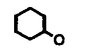 | cHexO | 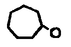 | cHepO | 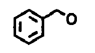 | BnO |
| 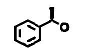 | (R)1PhEtO | 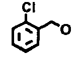 | 2ClBnO | 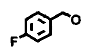 | 4FBnO |
| 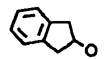 | 2-IndanO | 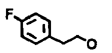 | 2(4FPh)EtO | 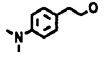 | 2(4DMAPh)EtO |
| 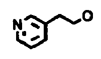 | 2(3-Py)EtO | 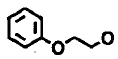 | 2(PhO)EtO | 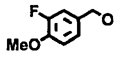 | 3F,4(OMe)BnO |
| 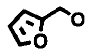 | 2FRMeO | 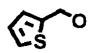 | 2TPMeO | | |
| 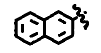 | 2-Nap | 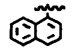 | 1-Nap | 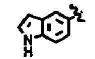 | 5-Ind |
| 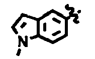 | 1Me-5-Ind | 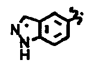 | 5-1Idz | 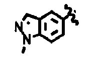 | 1Me-5-1Idz |
| 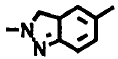 | 2Me-5-1Idz | 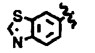 | 5-Bzt | 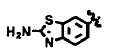 | 5-2ABzt |
| 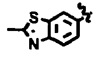 | 2Me-5-Bzt | 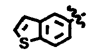 | 5-BT | 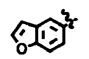 | 5-BF |
| 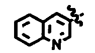 | 3-Qu | 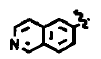 | 6-IQ | | |

The manufacturers of the regents used may sometimes be indicated with the following abbreviations.
TCI: Tokyo Kasei Kogyo Co., Ltd., Ald: Aldrich Co., KANTO: Kanto Kagaku, WAKO: Wako Pure Chemical Industries, Ltd., LANC: Lancaster Synthesis, MAYB: Maybridge, plc.

### Reference Example 1

### Synthesis of 5-cyclopentyloxy-2,3-dihydroindan-1-one (Intermediate A-1) (Preparation Method 14, Step f-2)

A solution of 5-hydroxy-1-indanone (741 mg, TCI) in anhydrous tetrahydrofuran (henceforth abbreviated as THF, 25 ml) was added with di-t-butyl azodicarboxylate (1.42 g, Ald), triphenylphosphine (1.61 g, WAKO) and cyclopentanol (500 µl, WAKO), and the mixture was stirred for 12 hours. The reaction mixture was added with water (100 ml) and ethyl acetate (20 ml × 2) for extraction, the organic layer was washed successively with saturated aqueous sodium hydrogencarbonate, saturated aqueous ammonium chloride and saturated brine, then the organic layer was dried, and the solvent was evaporated under reduced pressure. The residue was purified by column chromatography (Quad, hexane:ethyl acetate = 6:1) to obtain the title compound (Intermediate A-1, 1.13 g). Mass (LCMS): 217 (M⁺+1), Retention time: 4.20 minutes (Elution condition: B).

### Synthesis of ethyl 2-(5-cyclopentyloxy-2,3-dihydroinden-1-ylidene)acetate (Intermediate A-2) (Preparation Method 9, Step k-1)

A solution of sodium hydride (306.4 mg, WAKO) in anhydrous dimethoxyethane (henceforth abbreviated as DME, 30 mL) was added dropwise with a solution of ethyl diethylphosphonoacetate (7.6 ml, TCI) in anhydrous DME (15 ml) under ice cooling, and the mixture was stirred for 3 minutes, and then added dropwise with a solution of Intermediate A-1 (1.13 g) in anhydrous DME. The mixture was stirred for 5 minutes, and then stirred for 20 hours under reflux by heating. The reaction mixture was concentrated, then added with water (30 ml), and extracted with ethyl acetate (30 ml × 3). The organic layer was washed with water, saturated aqueous ammonium chloride and saturated brine, and dried, and then the solvent was evaporated under reduced pressure. The residue was purified by column chromatography (Quad, hexane:ethyl acetate = 8:1) to obtain the title compound (Intermediate A-2, 1.21 g). Mass (LCMS): 287 (M⁺+1), Retention time: 6.07 minutes (Elution condition: B).

### Synthesis of ethyl 2-(5-cyclopentyloxy-2,3-dihydro-1H-inden-1-yl)acetate (Intermediate A-3) (Preparation Method 9, Step j)

A solution of Intermediate A-2 (591 mg) in methanol (10 ml) was added with 10% palladium/carbon (33 mg, Merck), and the mixture was stirred at room temperature for 1.5 hours under a hydrogen atmosphere. The reaction mixture was filtered, and the solvent of the filtrate was evaporated under reduced pressure to obtain the title compound (Intermediate A-3, 563.2 mg). Mass (LCMS): N.D (M⁺+1), Retention time: 5.77 minutes (Elution condition: B).

### Synthesis of ethyl 2-(6-bromo-5-cyclopentyloxy-2,3-dihydro-1H-inden-1-yl)acetate (Intermediate A-4) (Preparation Method 8, Step h)

A solution of Intermediate A-3 (563.2 mg) in acetonitrile (6 ml) was added with N-bromosuccinimide (henceforth abbreviated as "NBS", 344 mg, WAKO) under ice cooling, and the mixture was stirred for 10 minutes, and then stirred at room temperature for 1 hour. The reaction mixture was concentrated under reduced pressure, and then added with ethyl acetate (200 ml), the organic layer was successively washed with saturated aqueous ammonium chloride, 5% aqueous sodium sulfite, saturated aqueous sodium hydrogencarbonate and saturated brine, and dried, and then the solvent was evaporated under reduced pressure. The residue was purified by column chromatography (Quad, hexane:ethyl acetate = 8:1) to obtain the title compound (Intermediate A-4, 621.3 mg). Mass (LCMS): N.D (M⁺+1), Retention time: 6.17 minutes (Elution condition: B).

### Reference Example 2

### Synthesis of 5-benzyloxy-2,3-dihydroindan-1-one (Intermediate A-5) (Preparation Method 14, Step f-1)

A solution of 5-hydroxy-1-indanone (14.8 g) in DMF (300 ml) was added with potassium carbonate (16.59 g, WAKO) and benzyl bromide (18.8 g, TCI), and the mixture was stirred at room temperature for 8 hours. The mixture was concentrated under reduced pressure, then the residue was added with water (100 ml) and ethyl acetate (200 ml × 2) for extraction, and the organic layer was water washed with (100 ml × 2). The organic layer was dried, and then the solvent was evaporated under reduced pressure. The residue was added with diethyl ether (50 ml) and hexane (30 ml), and crude crystals were taken by filtration to obtain the title compound (Intermediate A-5, 20.1 g). Mass (LCMS): 239 (M⁺+1), Retention time: 4.26 minutes (Elution condition: B).

### Synthesis of ethyl 2-(5-benzyloxy-2,3-dihydroinden-1-ylidene)acetate (Intermediate A-6) (Preparation Method 9, Step k-1)

According to the procedure described in the synthetic method of Intermediate A-2 in Reference Example 1 (Preparation Method 9, Step k-1) provided that the reaction was performed for 28 hours under a reflux condition, Intermediate A-5 (30.01 g), ethyl diethylphosphonoacetate (85.2 g, TCI) and sodium hydride (9.12 g, WAKO) were reacted and treated to obtain the title compound (Intermediate A-6, 28.6 g). Mass (LCMS): N.D (M⁺+1), Retention time: 5.60 minutes (Elution condition: B).

### Synthesis of ethyl 2-(5-hydroxy-2,3-dihydro-1H-inden-1-yl)acetate (Intermediate A-7) (Preparation Method 9, Step j)

A solution of Intermediate A-6 (28.7 g) in ethanol (500 ml) was added with 10% palladium hydroxide (1.21 g, NE Chemcat), and the mixture was stirred at room temperature for 8.5 hours under a hydrogen atmosphere. The reaction mixture was filtered, and the solvent of the filtrate was evaporated under reduced pressure to obtain the title compound (Intermediate A-7, 16.3 g). Mass (LCMS): N.D (M⁺+1), Retention time: 3.70 minutes (Elution condition: B).

### Synthesis of ethyl 2-(5-benzyloxy-2,3-dihydro-1H-inden-1-yl)acetate (Intermediate A-8) (Preparation Method 4, Step f-1)

According to the procedure described in the synthetic method of Intermediate A-5 in Reference Example 2 (Preparation Method 14, Step f-1) provided that the reaction was performed at room temperature for 15 hours, Intermediate A-7 (19.18 g), benzyl bromide (18.2 g, TCI) and potassium carbonate (14.7 g, WAKO) were reacted and treated to obtain the title compound (Intermediate A-8, 20.4 g). Mass (LCMS): N.D (M⁺+1), Retention time: 5.59 minutes (Elution condition: B).

### Synthesis of ethyl 2-(5-benzyloxy-6-bromo-2,3-dihydro-1H-inden-1-yl)acetate (Intermediate A-9) (Preparation Method 8, Step h)

According to the procedure described in the synthetic method of Intermediate A-4 in Reference Example 1 (Preparation Method 8, Step h) provided that the reaction was performed at room temperature for 3 hours, Intermediate A-8 (22.2 g) and NBS (12.85 g, WAKO) were reacted and treated to obtain the title compound (Intermediate A-9, 25.02 g). Mass (LCMS): N.D (M⁺+1), Retention time: 5.91 minutes (Elution condition: B).

### Reference Example 3

### Synthesis of ethyl 2-(5-t-butyldiphenylsilyloxy-2,3-dihydro-1H-inden-1-yl)acetate (Intermediate A-12)

A solution of Intermediate A-7 (1.02 g) in anhydrous DMF (10 ml) was added with imidazole (368.5 mg, TCI), and added dropwise with a solution of t-butyldiphenylsilyl chloride (1.03 g, TCI) in DMF (5 ml) under ice cooling, and the mixture was stirred for 30 minutes, then warmed to room temperature, and further stirred for 16.5 hours. The reaction mixture was added with water (30 ml), and extracted with ethyl acetate (50 ml). The organic layer was successively washed with water and saturated brine, and dried, and then the solvent was evaporated under reduced pressure. The residue was purified by flash column chromatography (hexane:ethyl acetate = 9:1) to obtain the title compound (Intermediate A-12, 727.5 mg). Mass (LCMS): N.D (M⁺+1), Retention time: 7.69 minutes (Elution condition: A).

### Synthesis of ethyl 2-(6-bromo-5-t-butyldiphenylsilyloxy-2,3-dihydro-1H-inden-1-yl)acetate (Intermediate A-13) (Preparation Method 8, Step h)

According to the procedure described in the synthetic method of Intermediate A-4 in Reference Example 1 (Preparation Method 8, Step h) provided that the reaction was performed at room temperature for 3 hours, Intermediate A-12 (726.4 mg) and NBS (273.7 mg, WAKO) were reacted and treated to obtain the title compound (Intermediate A-13, 769.9 mg). Mass (LCMS): N.D (M-1), Retention time: 8.05 minutes (Elution condition: A).

### Synthesis of ethyl 2-(6-bromo-5-hydroxy-2,3-dihydro-1H-inden-1-yl)acetate (Intermediate A-14)

A solution of Intermediate A-13 (768.1 mg) in THF (15 ml) was added with acetic acid (850 µl, WAKO) and a 1 M solution of tetrabutylammonium fluoride/THF (13 ml, TCI), and the mixture was stirred for 2 hours. The reaction mixture was added with saturated aqueous sodium hydrogencarbonate (100 ml), and extracted with ethyl acetate (30 ml). The organic layer was washed with saturated brine, and dried, and then the solvent was evaporated under reduced pressure. The residue was purified by flash column chromatography (hexane:ethyl acetate = 7:1) to obtain the title compound (Intermediate A-14, 629.5 mg). Mass (LCMS): N.D (M-1), Retention time: 4.30 minutes (Elution condition: A).

### Synthesis of ethyl 2-(6-bromo-5-cyclohexylmethyloxy-2,3-dihydro-1H-inden-1-yl)acetate (Intermediate A-15) (Preparation Method 4, Step f-1)

According to the procedure described in the synthetic method of Intermediate A-5 in Reference Example 2 (Preparation Method 4, Step f-1) provided that the reaction was performed for 23 hours, and the purification was performed by flash column chromatography (hexane:ethyl acetate = 10:1), Intermediate A-14 (100 mg), potassium carbonate (70 mg) and cyclohexylmethyl bromide (72 µl, Tokyo Kasei Kogyo) were reacted and treated to obtain the title compound (Intermediate A-15, 102 mg). Mass (LCMS): 395 (M⁺), Retention time: N.D (Elution condition: C).

Typical examples of the intermediates that can be obtained by reacting and treating corresponding starting compounds using any of the methods described in the present specification including the examples described above are shown in Table-Int.A-1. In the tables, the intermediate numbers are shown in the columns of "Exp.". In the tables, used methods among the aforementioned synthetic methods are shown in the columns of "Syn" with symbols, the starting compounds are shown in the columns of "SM", and the reagents are shown in the columns of "Regent". In the columns of "Reagent", the halide regents shown with symbols "Hal (No.)" are those mentioned in Table-Hal, and the alcohol regents shown with symbols "Al (No.)" are those mentioned in Table-Al. Those regents for which cells of the columns of "Manufacturer" are blank were synthesized according to methods described in ordinary chemical literatures.

**[Table 2]**

| Table-Al | | | | | |
|---|---|---|---|---|---|
| Reagent | The name of reagent | manifacture | Reagent | The name of reagent | manifacture |
| Al1 | Cyclopentanol | TCI | Al19 | 5-Methoxy-2-indanol | |
| Al2 | Cyclopentanemethanol | Ald | Al20 | 5,6-Dimethoxy-2-indanol | |
| Al3 | Cyclohexanol | WAKO | Al21 | 5-Fluoro-2-indanol | |
| Al4 | Cycloheptanol | TCI | Al22 | 1,2,3,4-Tetrahydro-2-naphthol | Acros |
| Al5 | 2-Ethyl-1-butanol | TCI | Al23 | 1,2,3,4-Tetrahydro-1-naphthol | WAKO |
| Al6 | 2-[4-(Dimethylamino)phenyl]ethan | Ald | Al24 | 2-(2-Methylphenyl)ethyl alcohol | TCI |
| Al7 | 2-Phenoxyethanol | TCI | Al25 | 3-Fluorophenylethyl alcohol | Ald |
| Al8 | Phenethyl Alcohol | TCI | Al26 | 2-(o-Chlorophenyl)ethanol | WAKO |
| Al9 | 2-Fluorophenylethyl alcohol | TCI | Al27 | 2-Naphthaleneethanol | TCI |
| Al10 | 4-Chlorophenylethyl alcohol | Ald | Al28 | 1-Naphthaleneethanol | Ald |
| Al11 | 4-Methylcyclohexanol | WAKO | Al29 | 2-Hydroxymethyl-1,4-benzodioxan | WAKO |
| Al12 | 1-Pheny-2-propanol | TCI | Al30 | 2-(Phenylthio)ethanol | WAKO |
| Al13 | 2-Phenylpropane-1-ol | | Al31 | (1-Methyl-1H-imidazol-5yl)methanol | MAYB |
| Al14 | 1-(2-Fluorophenyl)-2-Propylalcohol | | Al32 | (1,5-Dimethyl-1H-pyrazol-3-yl)methanol | MAYB |
| Al15 | 1-(3-Trifluoromethylphenyl)-2-Propylalcohol | | Al33 | 4-Pyridinemethanol | TCI |
| Al16 | 3-Pheny-1-butanol | Ald | Al34 | 6-Methyl-2-pyridinemethanol | TCI |
| Al17 | 1-Indanol | TCI | Al35 | 2-Quinolinylmethanol | MAYB |
| Al18 | 2-Hydroxyindan | TCI | Al36 | 3-(2-Hydroxyethyl)pyridine | WAKO |

**[Table 3]**

| Table-Hal | | |
|---|---|---|
| Reagent | The name of reagent | Manufacture |
| Hal1 | Benzyl bromide | TCI |
| Hal2 | Cyclohexylmathyl bromide | TCI |
| Hal3 | n-Propyl bromide | TCI |
| Hal4 | Isopropyl bromide | TCI |
| Hal5 | n-Butyl bromide | TCI |
| Hal6 | Isobutyl bromide | WAKO |
| Hal7 | 2-Fluorobenzyl bromide | TCI |
| Hal8 | 3-Fluorobenzyl bromide | TCI |
| Hal9 | 4-Fluorobenzyl bromide | TCI |
| Hal10 | 2-Chlorobenzyl bromide | TCI |
| Hal11 | 3-Chlorobenzyl bromide | TCI |
| Hal12 | 4-Methylbenzyl chloride | TCI |
| Hal13 | 4-(Trifluoromethyl)benzyl bromide | TCI |

**[Table 4]**

| Table-Int.A-1 | | | | | | | | | |
|---|---|---|---|---|---|---|---|---|---|
| 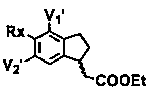 | | | | | | | | | |

| Exp. | Syn. | SM | Reagent | Rx | V1' | V2' | LCMS | | |
|---|---|---|---|---|---|---|---|---|---|
| | | | | | | | method | RTime | Mass |
| Int.A-10 | 4f-2 | Int.A-7 | Al2 | cPenMeO | H | H | C | | 303 (M⁺+1) |
| Int.A-11 | 4-h | Int.A-10 | | cPenMeO | H | Br | C | | 381 (M⁺) |
| Int.A-15 | 4f-1 | Int.A-14 | Hal2 | cHexMeO | H | Br | C | | 395 (M⁺) |
| Int.A-16 | 4f-2 | Int.A-14 | Al3 | cHexO | H | Br | C | | 381 (M⁺) |
| Int.A-17 | 4f-2 | Int.A-14 | Al4 | cHepO | H | Br | C | | 395 (M⁺) |
| Int.A-18 | 4f-1 | Int.A-14 | Hal3 | nPrO | H | Br | C | | 341 (M⁺) |
| Int.A-19 | 4f-1 | Int.A-14 | Hal4 | iPrO | H | Br | C | | 341 (M⁺) |
| Int.A-20 | 4f-1 | Int.A-14 | Hal5 | nBuO | H | Br | C | | 355 (M⁺) |
| Int.A-21 | 4f-1 | Int.A-14 | Hal6 | iBuO | H | Br | C | | 355 (M⁺) |

### Example A-a-1

### Synthesis of ethyl 2-[5-cyclopentyloxy-6-(naphthalen-2-yl)-2,3-dihydro-1H-inden-1-yl]acetate (Compound No. A-a-1) (Preparation Method 4, Step e-1)

A solution of Intermediate A-4 (100 mg) in toluene (1.1 ml) was added with a solution of 2-naphthaleneboronic acid (84.3 mg, TCI) in ethanol (0.4 ml), 2 M aqueous sodium carbonate (0.3 ml) and tetrakistriphenylphosphinepalladium(0) (henceforth abbreviated as "(Ph₃P)₄Pd", 34.7 mg, Nakalai Tesque), and the mixture was stirred at 90°C for 18 hours. The reaction mixture was added with water (30 ml) and ethyl acetate (30 ml × 2) for extraction, and then successively washed with saturated aqueous sodium hydrogencarbonate, saturated aqueous ammonium chloride and saturated brine, the organic layer was dried, and then the solvent was evaporated under reduced pressure. The residue was purified by column chromatography (Quad, hexane:ethyl acetate = 7:1) to obtain the title compound (Compound No. A-a-1, 102 mg).

### Example A-a-2

### Synthesis of 2-[5-cyclopentyloxy-6-(naphthalen-2-yl)-2,3-dihydro-1H-inden-1-yl]acetic acid (Compound No. A-a-2) (Preparation Method 1, Step a)

A solution of Example Compound A-a-1 (102 mg) in a mixture of methanol (600 µl) and THF (600 µl) was added with 2 N aqueous sodium hydroxide (600 µ1), and the mixture was stirred at 60°C for 17 hours. The reaction mixture was concentrated under reduced pressure, then neutralized with 1 N aqueous hydrochloric acid under ice cooling, and then extracted with methylene chloride (2 ml × 3). The organic layer was washed with saturated brine and dried, and then the solvent was evaporated under reduced pressure to obtain the title compound (Compound No. A-a-2, 86 mg).

### Example A-a-21

### Synthesis of ethyl 2-[6-(benzo[b]thiazol-5-yl)5-cyclopentyloxy-2,3-dihydro-1H-inden-1-yl]acetate (Compound No. A-a-21) (Preparation Method 4, Step e-2)

According to the procedure described in a literature [N. Miyaura et al., Tetrahedron Lett., 1997, p.3447], Intermediate A-4 (354 mg), bis(pinacolate)diboron (471 mg, Ald), [1,1'-bis(diphenylphosphino)ferrocene]palladium(II) dichloride (henceforth abbreviated as "PdCl₂(dppf)", 87 mg, Ald) and potassium acetate (216.5 mg, Ald) were added to 1,4-dioxane (6 ml), and the mixture was stirred with heating at 80°C for 7 hours under a nitrogen atmosphere. The reaction mixture was cooled to room temperature, and then added with 5-bromobenzo[b]thiazole (131 mg), PdCl₂(dppf) (20 mg) and 2 M aqueous sodium carbonate (0.9 ml), and the mixture was stirred with heating at 80°C for 14 hours under a nitrogen atmosphere. The reaction mixture was added with water (15 ml) and ethyl acetate (20 ml × 2) for extraction, and then washed with saturated brine and dried, and the solvent was evaporated under reduced pressure. The residue was purified by column chromatography (Quad, hexane:ethyl acetate = 5:1) to obtain the title compound (Compound No. A-a-21, 12 mg).

### Example A-a-22

### Synthesis of 2-[6-(benzo[b]thiazol-5-yl)5-cyclopentyloxy-2,3-dihydro-1H-inden-1-yl]acetic acid (Compound No. A-a-22) (Preparation Method 1, Step a)

According to the procedure described in the synthetic method of Example Compound A-a-2 (Preparation Method 1, Step a) provided that the reaction was performed for 2 hours, Example Compound A-a-21 (12 mg) and 2 N aqueous sodium hydroxide (0.1 ml) were reacted and treated to obtain the title compound (Compound No. A-a-22, 9.2 mg).

### Example A-a-107

### Synthesis of ethyl 2-[5-hydroxy-6-(naphthalen-2-yl)-2,3-dihydro-1H-inden-1-yl]acetate (Intermediate A-22)

According to the procedure described in the synthetic method of Intermediate A-7 provided that the reaction was performed for 12 hours in a mixed solvent of ethanol (20 ml) and THF (20 ml), Example Compound A-a-29 (1.45 g) and 10% palladium hydroxide (131.4 mg) were reacted and treated to obtain the title compound (Intermediate A-22, 1.14 g).

### Synthesis of ethyl 2-[5-(2-fluorobenzyloxy)-6-(naphthalen-2-yl)-2,3-dihydro-1H-inden-1-yl]acetate (Compound No. A-a-107) (Preparation Method 4, Step f-1)

According to the procedure described in the synthetic method of Intermediate A-5 in Reference Example 2 (Preparation Method 4, Step f-1) provided that the reaction was performed for 18 hours, Intermediate A-22 (116.3 mg), 2-fluorobenzyl bromide (48.25 µl, Ald) and potassium carbonate (88.2 mg) were reacted and treated to obtain the title compound (Compound No. A-a-107, 122.4 mg).

### Example A-a-108

### Synthesis of 2-[5-(2-fluorobenzyloxy)-6-(naphthalen-2-yl)-2,3-dihydro-1H-inden-1-yl]acetic acid (Compound No. A-a-108) (Preparation Method 1, Step a)

According to the procedure described in the synthetic method of Example Compound A-a-2 (Preparation Method 1, Step a) provided that the reaction was performed for 2 hours, Example Compound A-a-107 (121.2 mg) and 2 N aqueous sodium hydroxide (0.45 ml) were reacted and treated to obtain the title compound (Compound No. A-a-108, 98.9 mg).

### Example A-a-153

### Synthesis of ethyl 2-[5-(4-dimethylaminophenyloxy)-6-(naphthalen-2-yl)-2,3-dihydro-1H-inden-1-yl]acetate (Compound No. A-a-153) (Preparation Method 4, Step f-2)

According to the procedure described in the synthetic method of Intermediate A-1 in Reference Example 1 (Preparation Method 4, Step f-2) provided that the reaction was performed for 28 hours, Intermediate A-22 (112.2 mg), 2-(4-dimethylamino)dimethylphenyl alcohol (59.6 mg, Ald), triphenylphosphine (101.2 mg) and di-t-butyl azodicarboxylate (97.6 mg, Ald) were reacted and treated to obtain the title compound (Compound No. A-a-153, 110.4 mg).

### Example A-a-154

### Synthesis of 2-[5-(4-dimethylaminophenyloxy)-6-(naphthalen-2-yl)-2,3-dihydro-1H-inden-1-yl]acetic acid (Compound No. A-a-154) (Preparation Method 1, Step a)

According to the procedure described in the synthetic method of Example Compound A-a-2 (Preparation Method 1, Step a) provided that the reaction was performed for 5 hours, Example Compound A-a-153 (110.2 mg) and 2 N aqueous sodium hydroxide (0.45 ml) were reacted and treated to obtain the title compound (Compound No. A-a-154, 99.2 mg).

Typical examples of the intermediates that can be obtained by reacting and treating corresponding starting compounds using any of the methods described in the present specification including the examples described above are shown in Table-Int.A-2. In the columns of "Reagent", the halide regents shown with symbols "Hal (No.)" are those mentioned in Table-Hal, the alcohol regents shown with symbols "Al (No.)" are those mentioned in Table-Al, the boronic acid regents shown with symbols "BRA (No.)" are those mentioned in Table-BRA, and the bromoheterocyclic ring regents mentioned with the symbols "Het (No.)" are those mentioned in Table-Het. Those regents for which cells of the columns of "Manufacturer" are blank were synthesized according to methods described in ordinary chemical literatures.

**[Table 5]**

| Table-BRA-1 | | | | | |
|---|---|---|---|---|---|
| Reagent | The name of reagent | Manufacture | Reagent | The name of reagent | Manufacture |
| BRA1 | 2-Naphthalene boronic acid | TCI | BRA30 | 4-Isopropyloxyphenyl boronic acid | Ald |
| BRA2 | 5-1H-Indole boronic acid | Frontier | BRA31 | 3-n-Propyloxyphenyl boronic acid | Ald |
| BRA3 | 1-Methyl-5-Indole boronic acid | Frontier | BRA32 | 4-n-Propyloxyphenyl boronic acid | Ald |
| BRA4 | 1-Ethyl-5-Indole boronic acid | | BRA33 | 3-n-Butyloxyphenyl boronic | Ald |
| BRA5 | 1-Methyl-4-indiole boronic | | BRA34 | 4-n-Butyloxyphenyl boronic | Ald |
| BRA6 | 1-Methyl-6-indiole boronic acid | | BRA35 | 3-(Dimethylamino) phenyl boronic acid | Frontier |
| BRA7 | 5-1 H-Indazole boronic acid | | BRA36 | 4-(Dimethylamino) phenyl boronic acid | Ald |
| BRA8 | 1-Methyl-5-1H-Indazole-boronic acid | | BRA37 | 2-Fluoropheny boronic acid | Ald |
| BRA9 | 1-Ethyll-5-1H-Indazole-boronic acid | | BRA38 | 3-Fluoropheny boronic acid | Ald |
| BRA10 | 2-Methyl-5-2H-indazole boronic acid | | BRA39 | 4-Fluoropheny boronic acid | Ald |
| BRA11 | 5-Benzofurane boronic acis | Maybridg | BRA40 | 2-Chlorophenyl boronic acid | Aid |
| BRA12 | 5-Benzothiophene boronic | Ald | BRA41 | 3-Chlorophenyl boronic acid | Ald |
| BRA13 | 6-(4,4,5,5-tetramethyl-1,3,2-dioxaborolan-2-yl)quinoline | Ald | BRA42 | 4-Chlorophenyl boronic acid | Ald |
| BRA14 | Phenyl boronic acid | Ald | BRA43 | 2-(Trifluoromethyl) phenyl boronic acid | TCI |
| BRA15 | 2-Methylphenyl boronic acid | Ald | BRA44 | 3-(Trifluoromethyl) phenyl boronic acid | TCI |
| BRA16 | 3-Methylphenyl boronic acid | Ald | BRA45 | 4-(Trifluoromethyl) phenyl boronic acid | Ald |
| BRA17 | 4-Methylphenyl boronic acid | Ald | BRA46 | 2,3-Difluorophenyl boronic | Ald |
| BRA18 | 2-Ethylphenyl boronic acid | Ald | BRA47 | 2,4-Difluorophenyl boronic | Ald |
| BRA19 | 4-Ethylphenyl boronic acid | Ald | BRA48 | 2,5-Difluorophenyl boronic | Ald |
| BRA20 | 2-Isopropylphenyl boronic | Ald | BRA49 | 2,6-Difluorophenyl boronic | Ald |
| BRA21 | 4-Isopropylphenyl boronic | WAKO | BRA50 | 3,4-Difluorophenyl boronic | Ald |
| BRA22 | 1-n-Butylphenyl boronic acid | Ald | BRA51 | 3.5-Difluorophenyl boronic | Ald |
| BRA23 | 4-t-Butylphenyl boronic acid | Ald | BRA52 | 2,4-Dichlorophenyl boronic | Ald |
| BRA24 | 2-Methoxypheny boronic acid | Ald | BRA53 | 3,5-Dichlorophenyl boronic | Ald |
| BRA25 | 3-Methoxyphenyl boronic acid | Ald | BRA54 | 3,4-Dichlorophenyl boronic | Ald |
| BRA26 | 4-Methoxypheny boronic acid | Ald | BRA55 | 2,3-Dimethylphenyl boronic | Lancaster |
| BRA27 | 2-Ethyloxypheny boronic acid | Ald | BRA56 | 2,4-Dimethylphenyl boronic | WAKO |
| BRA28 | 3-Ethyloxypheny boronic acid | Ald | BRA57 | 2,6-Dimethylphenyl boronic | Ald |
| BRA29 | 3-Isopropyloxyphenyl boronic acid | Ald | | | |

**[Table 6]**

| Table-BRA-2 | | | | | |
|---|---|---|---|---|---|
| Reagent | The name of reagent | Manufacture | Reagent | The name of reagent | Manufacture |
| BRA58 | 2-Furyl boronic acid | Ald | BRA68 | Trans-2-(4-Chlorophenyl)vinylboronic acid | Ald |
| BRA59 | 3-Furyl boronic acid | Ald | BRA69 | 2-Cyclohexylvinylboronic acid | Ald |
| BRA60 | 2-Thienyl boronic acid | Ald | BRA70 | Trans-2-{4(Trifluoromethyl)-phenyl}vinylboronic acid | Ald |
| BRA61 | 3-Thienyl boronic acid | Ald | BRA71 | Trans-2-(4-Methylphenyl)vinylboronic acid | Ald |
| BRA62 | Pyridine-3-boronic acid | Ald | BRA72 | (E)-2-(3-Methoxyphenyl)-4,4,5,5-tetramethyl-(1,3,2)-dioxaborolate | Aid |
| BRA63 | Pyridine-4-boronic acid | Ald | BRA73 | 2-(cis-1-ethyl-1-butenyl)benzo- | Ald |
| BRA64 | Biphenyl-3-boronic acid | Ald | BRA74 | trans-1-Propen-1-ylboronic acid | Ald |
| BRA65 | Biphenyl-4-boronic acid | Ald | BRA75 | Cis-1-Propen-1-ylboronic | Ald |
| BRA66 | Trans-1-Hexene-1-ylboronic acid | Ald | BRA76 | 1-Pentenylboronic acid | Ald |
| BRA67 | Trans-2-(4-Fluorophenyl)vinylboronic acid | Ald | BRA77 | Trimethylboroxine | Ald |

**[Table 7]**

| Table-Het | | |
|---|---|---|
| Reagent | The name of reagent | manufacture |
| Het1 | 5-Bromobenzothiazole | TCI |
| Het2 | 3-Bromoquinoline | TCI |
| Het3 | 6-Bromoisoquinoline | |

Typical examples of the compounds of the present invention that can be obtained by reacting and treating corresponding starting compounds using any of the methods described in the present specification including the examples described above are shown in Table-A-a-1 to Table-A-a-8. The compounds were prepared according to the preparation methods of the compounds of the compound numbers (e.g., "A-a-1") or the intermediate numbers (e.g., "Int. A-1") shown in the columns of "Syn" in the tables. "Int" means an intermediate compound number. When the preparation required a plurality of steps, a plurality of compound numbers or intermediate compound numbers are mentioned in the columns of "Syn". For example, an indication of "4e-1" in a column of "Syn" means that "the compound is prepared according to the procedure described in the synthetic method e-1 of Preparation Method 4". In the tables, used methods among the aforementioned synthetic methods are shown in the columns of "Syn" with symbols, the starting compounds are shown in the columns of "SM", and the reagents are shown in the columns of "Regent". The halide regents mentioned in the columns of "Reagent" with symbols "Hal (No.)" are those mentioned in Table-Hal, the alcohol regents shown with symbols "Al (No.)" are those mentioned in Table-Al, the boronic acid regents shown with symbols "BRA (No.)" are those mentioned in Table-BRA, and the bromoheterocyclic ring regents mentioned with the symbols "Het (No.)" are those mentioned in Table-Het. Those regents for which cells of the columns of "Manufacturer" are blank were synthesized according to methods described in ordinary chemical literatures. For the compounds for which cells of the columns of "Syn" are blank, deprotection was performed with Pd/C and hydrogen.

**[Table 8]**

| Table-IntA-2 | | | | | | | | | |
|---|---|---|---|---|---|---|---|---|---|
| 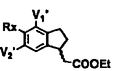 | | | | | | | | | |

| Exp. | Syn. | SM | Reagent | Rx | V1' | V2' | LCMS | | |
|---|---|---|---|---|---|---|---|---|---|
| | | | | | | | method | RTime | Mass |
| Int.A-22 | | A-a-29 | | HO | H | 2-Nap | C | | 347 (M⁺+1) |
| Int.A-23 | | A-a-31 | | HO | H | 5-Ind | C | | 336 (M⁺+1) |
| Int.A-24 | | A-a-33 | | HO | H | 1Me-5-Ind | C | | 350(M⁺+1) |
| Int.A-25 | | A-a-35 | | HO | H | 5-1Idz | C | | 337(M⁺+1) |
| Int.A-26 | | A-a-37 | | HO | H | 1Me-5-Idz | C | | 351(M⁺+1) |
| Int.A-27 | | A-a-39 | | HO | H | 1Et-5-Idz | C | | 365(M⁺+1) |
| Int.A-28 | | A-a-41 | | HO | H | 5-BF | C | | 337(M⁺+1) |
| Int.A-29 | | A-a-43 | | HO | H | 6-Qu | C | | 348(M⁺+1) |

**[Table 9]**

| Table-A-a-1 | | | | | | | | | | |
|---|---|---|---|---|---|---|---|---|---|---|
| 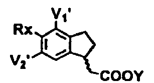 | | | | | | | | | | |

| Exp. | Syn. | SM | Reagent | Rx | Y | V1' | V2' | LCMS | | |
|---|---|---|---|---|---|---|---|---|---|---|
| | | | | | | | | method | RTime | Mass |
| A-a-1 | 4e-1 | Int.A-4 | BRA1 | cPenO | Et | H | 2-Nap | C | | 415(M⁺+1) |
| A-a-2 | 1-a | A-a-1 | | cPenO | H | H | 2-Nap | C | | 387(M⁺+1) |
| A-a-3 | 4e-1 | Int.A-4 | BRA2 | cPenO | Et | H | 5-Ind | C | | 404(M⁺+1) |
| A-a-4 | 1-a | A-a-3 | | cPenO | H | H | 5-Ind | C | | 376(M⁺+1) |
| A-a-5 | 4e-1 | Int.A-4 | BRA3 | cPenO | Et | H | 1Me-5-Ind | C | | 418(M⁺+1) |
| A-a-6 | 1-a | A-a-5 | | cPenO | H | H | 1Me-5-Ind | C | | 390(M⁺+1) |
| A-a-7 | 4e-1 | Int.A-4 | BRA4 | cPenO | Et | H | 1Et-5-Ind | C | | 432(M⁺+1) |
| A-a-8 | 1-a | A-a-7 | | cPenO | H | H | 1Et-5-Ind | C | | 404(M⁺+1) |
| A-a-9 | 4e-1 | Int.A-4 | BRA7 | cPenO | Et | H | 5-1Idz | C | | 405M⁺+1) |
| A-a-10 | 1-a | A-a-9 | | cPenO | H | H | 5-1Idz | C | | 377(M⁺+1) |
| A-a-11 | 4e-1 | Int.A-4 | BRA8 | cPenO | Et | H | 1Me-5-Idz | C | | 419M⁺+1) |
| A-a-12 | 1-a | A-a-11 | | cPenO | H | H | 1Me-5-Idz | C | | 391(M⁺+1) |
| A-a-13 | 4e-1 | Int.A-4 | BRA9 | cPenO | Et | H | 1Et-5-Idz | C | | 433(M⁺+1) |
| A-a-14 | 1-a | A-a-13 | | cPenO | H | H | 1Et-5-Idz | C | | 405(M⁺+1) |
| A-a-15 | 4e-1 | Int.A-4 | BRA10 | cPenO | Et | H | 2Me-5-Idz | C | | 419(M⁺+1) |
| A-a-16 | 1-a | A-a-15 | | cPenO | H | H | 2Me-5-Idz | C | | 391(M⁺+1) |
| A-a-17 | 4e-1 | Int.A-4 | BRA11 | cPenO | Et | H | 5-BF | C | | 405(M⁺+1) |
| A-a-18 | 1-a | A-a-17 | | cPenO | H | H | 5-BF | C | | 377(M⁺+1) |
| A-a-19 | 4e-1 | Int.A-4 | BRA12 | cPenO | Et | H | 5-BT | C | | 421(M⁺+1) |
| A-a-20 | 1-a | A-a-19 | | cPenO | H | H | 5-BT | C | | 393(M⁺+1) |
| A-a-21 | 4e-2 | Int.A-4 | Het1 | cPenO | Et | H | 5-Bzt | C | | 422(M⁺+1) |
| A-a-22 | 1-a | A-a-21 | | cPenO | H | H | 5-Bzt | C | | 394(M⁺+1) |
| A-a-23 | 4e-2 | Int.A-4 | Het2 | cPenO | Et | H | 3-Qu | C | | 416(M⁺+1) |
| A-a-24 | 1-a | A-a-23 | | cPenO | H | H | 3-Qu | C | | 388(M⁺+1) |
| A-a-25 | 4e-1 | Int.A-4 | BRA13 | cPenO | Et | H | 6-Qu | C | | 416(M⁺+1) |
| A-a-26 | 1-a | A-a-25 | | cPenO | H | H | 6-Qu | C | | 388(M⁺+1) |
| A-a-27 | 4e-2 | Int.A-4 | Het3 | cPenO | Et | H | 6-IQ | C | | 416(M⁺+1) |
| A-a-28 | 1-a | A-a-27 | | cPenO | H | H | 6-IQ | C | | 388(M⁺+1) |
| A-a-29 | 4e-1 | Int.A-9 | BRA1 | BnO | Et | H | 2-Nap | C | | 437(M⁺+1) |
| A-a-30 | 1-a | A-a-29 | | BnO | H | H | 2-Nap | C | | 409(M⁺+1) |
| A-a-31 | 4e-1 | Int.A-9 | BRA2 | BnO | Et | H | 5-Ind | C | | 426(M⁺+1) |
| A-a-32 | 1-a | A-a-31 | | BnO | H | H | 5-Ind | C | | 398(M⁺+1) |
| A-a-33 | 4e-1 | Int.A-9 | BRA3 | BnO | Et | H | 1Me-5-Ind | C | | 440(M⁺+1) |
| A-a-34 | 1-a | A-a-33 | | BnO | H | H | 1Me-5-Ind | C | | 412(M⁺+1) |
| A-a-35 | 4e-1 | Int.A-9 | BRA7 | BnO | Et | H | 5-1Idz | C | | 427(M⁺+1) |
| A-a-36 | 1-a | A-a-35 | | BnO | H | H | 5-1Idz | C | | 399(M⁺+1) |
| A-a-37 | 4e-1 | Int.A-9 | BRA8 | BnO | Et | H | 1Me-5-Idz | C | | 441(M⁺+1) |
| A-a-38 | 1-a | A-a-37 | | BnO | H | H | 1Me-5-Idz | C | | 413(M⁺+1) |
| A-a-39 | 4e-1 | Int.A-9 | BRA9 | BnO | Et | H | 1Et-5-Idz | C | | 455(M⁺+1) |
| A-a-40 | 1-a | A-a-39 | | BnO | H | H | 1Et-5-Idz | C | | 427(M⁺+1) |
| A-a-41 | 4e-1 | Int.A-9 | BRA11 | BnO | Et | H | 5-BF | C | | 427(M⁺+1) |
| A-a-42 | 1-a | A-a-41 | | BnO | H | H | 5-BF | C | | 399(M⁺+1) |
| A-a-43 | 4e-2 | Int.A-9 | BRA13 | BnO | Et | H | 6-Qu | C | | 438(M⁺+1) |
| A-a-44 | 1-a | A-a-43 | | BnO | H | H | 6-Qu | C | | 410(M⁺+1) |
| A-a-45 | 4e-1 | Int.A-11 | BRA2 | cPenMeO | Et | H | 5-Ind | C | | 418(M⁺+1) |
| A-a-46 | 1-a | A-a-45 | | cPenMeO | H | H | 5-Ind | C | | 390(M⁺+1) |

**[Table 10]**

| Table-A-a-2 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-a-47 | 4e-1 | Int.A-11 | BRA4 | cPenMeO | Et | H | 1 Et-5-Ind | C | | 446(M⁺+1) |
| A-a-48 | 1-a | A-a-47 | | cPenMeO | H | H | 1 Et-5-Ind | C | | 418M⁺+1) |
| A-a-49 | 4e-1 | Int.A-11 | BRA8 | cPenMeO | Et | H | 1Me-5-Idz | C | | 433(M⁺+1) |
| A-a-50 | 1-a | A-a-49 | | cPenMeO | H | H | 1Me-5-Idz | C | | 405(M⁺+1) |
| A-a-51 | 4e-2 | Int.A-11 | Het2 | cPenMeO | Et | H | 3-Qu | C | | 430(M⁺+1) |
| A-a-52 | 1-a | A-a-51 | | cPenMeO | H | H | 3-Qu | C | | 402(M⁺+1) |
| A-a-53 | 4e-1 | Int.A-15 | BRA1 | cHexMeO | Et | H | 2-Nap | C | | 443(M⁺+1) |
| A-a-54 | 1-a | A-a-53 | | cHexMeO | H | H | 2-Nap | C | | 415(M⁺+1) |
| A-a-55 | 4e-1 | Int.A-15 | BRA3 | cHexMeO | Et | H | 1Me-5-Ind | C | | 446(M⁺+1) |
| A-a-56 | 1-a | A-a-55 | | cHexMeO | H | H | 1Me-5-Ind | C | | 418(M⁺+1) |
| A-a-57 | 4e-1 | Int.A-15 | BRA7 | cHexMeO | Et | H | 5-1Idz | C | | 433(M⁺+1) |
| A-a-58 | 1-a | A-a-57 | | cHexMeO | H | H | 5-1Idz | C | | 405(M⁺+1) |
| A-a-59 | 4e-2 | Int.A-15 | Het3 | cHexMeO | Et | H | 6-IQ | C | | 444(M⁺+1) |
| A-a-60 | 1-a | A-a-59 | | cHexMeO | H | H | 6-IQ | C | | 416(M⁺+1) |
| A-a-61 | 4e-1 | Int.A-16 | BRA3 | cHexO | Et | H | 1Me-5-Ind | C | | 432(M⁺+1) |
| A-a-62 | 1-a | A-a-61 | | cHexO | H | H | 1Me-5-Ind | C | | 404(M⁺+1) |
| A-a-63 | 4e-1 | Int.A-16 | BRA8 | cHexO | Et | H | 1Me-5-Idz | C | | 433(M⁺+1) |
| A-a-64 | 1-a | A-a-63 | | cHexO | H | H | 1Me-5-Idz | C | | 405(M⁺+1) |
| A-a-65 | 4e-2 | Int.A-16 | Het2 | cHexO | Et | H | 3-Qu | C | | 430(M⁺+1) |
| A-a-66 | 1-a | A-a-65 | | cHexO | H | H | 3-Qu | C | | 402(M⁺+1) |
| A-a-67 | 4e-1 | Int.A-16 | BRA13 | cHexO | Et | H | 6-Qu | C | | 430(M⁺+1) |
| A-a-68 | 1-a | A-a-67 | | cHexO | H | H | 6-Qu | C | | 402(M⁺+1) |
| A-a-69 | 4e-1 | Int.A-17 | BRA1 | cHepO | Et | H | 2-Nap | C | | 443(M⁺+1) |
| A-a-70 | 1-a | A-a-69 | | cHepO | H | H | 2-Nap | C | | 415(M⁺+1) |
| A-a-71 | 4e-1 | Int.A-17 | BRA2 | cHepO | Et | H | 5-Ind | C | | 432(M⁺+1) |
| A-a-72 | 1-a | A-a-71 | | cHepO | H | H | 5-Ind | C | | 404(M⁺+1) |
| A-a-73 | 4e-1 | Int.A-17 | BRA8 | cHepO | Et | H | 1Me-5-Idz | C | | 447(M⁺+1) |
| A-a-74 | 1-a | A-a-73 | | cHepO | H | H | 1Me-5-Idz | C | | 419(M⁺+1) |
| A-a-75 | 4e-1 | Int.A-17 | BRA13 | cHepO | Et | H | 6-Qu | C | | 444(M⁺+1) |
| A-a-76 | 1-a | A-a-75 | | cHepO | H | H | 6-Qu | C | | 416(M⁺+1) |
| A-a-77 | 4e-1 | Int.A-18 | BRA2 | nPrO | Et | H | 5-Ind | C | | 378(M⁺+1) |
| A-a-78 | 1-a | A-a-77 | | nPrO | H | H | 5-Ind | C | | 350(M⁺+1) |
| A-a-79 | 4e-1 | Int.A-18 | BRA3 | nPrO | Et | H | 1Me-5-Ind | C | | 392(M⁺+1) |
| A-a-80 | 1-a | A-a-79 | | nPrO | H | H | 1Me-5-Ind | C | | 364(M⁺+1) |
| A-a-81 | 4e-1 | Int.A-18 | BRA7 | nPrO | Et | H | 5-1Idz | C | | 379(M⁺+1) |
| A-a-82 | 1-a | A-a-81 | | nPrO | H | H | 5-1Idz | C | | 351(M⁺+1) |
| A-a-83 | 4e-1 | Int.A-18 | BRA8 | nPrO | Et | H | 1Me-5-Idz | C | | 393(M⁺+1) |
| A-a-84 | 1-a | A-a-83 | | nPrO | H | H | 1Me-5-Idz | C | | 365(M⁺+1) |
| A-a-85 | 4e-1 | Int.A-19 | BRA1 | iPrO | Et | H | 2-Nap | C | | 389(M⁺+1) |
| A-a-86 | 1-a | A-a-85 | | iPrO | H | H | 2-Nap | C | | 361(M⁺+1) |
| A-a-87 | 4e-1 | Int.A-19 | BRA3 | iPrO | Et | H | 1Me-5-Ind | C | | 392(M⁺+1) |
| A-a-88 | 1-a | A-a-87 | | iPrO | H | H | 1Me-5-Ind | C | | 364(M⁺+1) |
| A-a-89 | 4e-1 | Int.A-19 | BRA8 | iPrO | Et | H | 1Me-5-Idz | C | | 393(M⁺+1) |
| A-a-90 | 1-a | A-a-89 | | iPrO | H | H | 1Me-5-Idz | C | | 365(M⁺+1) |
| A-a-91 | 4e-1 | Int.A-19 | BRA13 | iPrO | Et | H | 6-Qu | C | | 390(M⁺+1) |
| A-a-92 | 1-a | A-a-91 | | iPrO | H | H | 6-Qu | C | | 362(M⁺+1) |
| A-a-93 | 4e-1 | Int.A-20 | BRA2 | nBuO | Et | H | 5-Ind | C | | 392(M⁺+1) |
| A-a-94 | 1-a | A-a-93 | | nBuO | H | H | 5-Ind | C | | 364(M⁺+1) |

**[Table 11]**

| Table-A-a-3 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-a-95 | 4e-1 | Int.A-20 | BRA3 | nBuO | Et | H | 1Me-5-Ind | C | | 406(M⁺+1) |
| A-a-96 | 1-a | A-a-95 | | nBuO | H | H | 1Me-5-Ind | C | | 378(M⁺+1) |
| A-a-97 | 4e-1 | Int.A-20 | BRA8 | nBuO | Et | H | 1Me-5-Idz | C | | 407(M⁺+1) |
| A-a-98 | 1-a | A-a-97 | | nBuO | H | H | 1Me-5-Idz | C | | 379(M⁺+1) |
| A-a-99 | 4e-1 | Int.A-21 | BRA1 | iBuO | Et | H | 2-Nap | C | | 403(M⁺+1) |
| A-a-100 | 1-a | A-a-99 | | iBuO | H | H | 2-Nap | C | | 375(M⁺+1) |
| A-a-101 | 4e-1 | Int.A-21 | BRA3 | iBuO | Et | H | 1Me-5-Ind | C | | 406(M⁺+1) |
| A-a-102 | 1-a | A-a-101 | | iBuO | H | H | 1Me-5-Ind | C | | 378(M⁺+1) |
| A-a-103 | 4e-1 | Int.A-21 | BRA7 | iBuO | Et | H | 5-1Idz | C | | 393(M⁺+1) |
| A-a-104 | 1-a | A-a-103 | | iBuO | H | H | 5-1Idz | C | | 365(M⁺+1) |
| A-a-105 | 4e-2 | Int.A-21 | Het3 | iBuO | Et | H | 6-IQ | C | | 404(M⁺+1) |
| A-a-106 | 1-a | A-a-105 | | iBuO | H | H | 6-IQ | C | | 376(M⁺+1) |
| A-a-107 | 4f-1 | Int.A-22 | Hal7 | 2FBnO | Et | H | 2-Nap | C | | 455(M⁺+1) |
| A-a-108 | 1-a | A-a-107 | | 2FBnO | H | H | 2-Nap | C | | 427(M⁺+1) |
| A-a-109 | 4f-1 | Int.A-23 | Hal7 | 2FBnO | Et | H | 5-Ind | C | | 444(M⁺+1) |
| A-a-110 | 1-a | A-a-109 | | 2FBnO | H | H | 5-Ind | C | | 416(M⁺+1) |
| A-a-111 | 4f-1 | Int.A-26 | Hal7 | 2FBnO | Et | H | 1Me-5-Idz | C | | 459(M⁺+1) |
| A-a-112 | 1-a | A-a-111 | | 2FBnO | H | H | 1Me-5-Idz | C | | 430(M⁺+1) |
| A-a-113 | 4f-1 | Int.A-24 | Hal8 | 3FBnO | Et | H | 1Me-5-Ind | C | | 458(M⁺+1) |
| A-a-114 | 1-a | A-a-113 | | 3FBnO | H | H | 1Me-5-Ind | C | | 430(M⁺+1) |
| A-a-115 | 4f-1 | Int.A-26 | Hal8 | 3FBnO | Et | H | 1Me-5-Idz | C | | 459(M⁺+1) |
| A-a-116 | 1-a | A-a-115 | | 3FBnO | H | H | 1Me-5-Idz | C | | 431(M⁺+1) |
| A-a-117 | 4f-1 | Int.A-29 | Hal8 | 3FBnO | Et | H | 6-Qu | C | | 456(M⁺+1) |
| A-a-118 | 1-a | A-a-117 | | 3FBnO | H | H | 6-Qu | C | | 428(M⁺+1) |
| A-a-119 | 4f-1 | Int.A-22 | Hal9 | 4FBnO | Et | H | 2-Nap | C | | 455(M⁺+1) |
| A-a-120 | 1-a | A-a-119 | | 4FBnO | H | H | 2-Nap | C | | 427(M⁺+1) |
| A-a-121 | 4f-1 | Int.A-27 | Hal9 | 4FBnO | Et | H | 1Et-5-Idz | C | | 473(M⁺+1) |
| A-a-122 | 1-a | A-a-121 | | 4FBnO | H | H | 1Et-5-Idz | C | | 445(M⁺+1) |
| A-a-123 | 4f-1 | Int.A-28 | Hal9 | 4FBnO | Et | H | 5-BF | C | | 445(M⁺+1) |
| A-a-124 | 1-a | A-a-123 | | 4FBnO | H | H | 5-BF | C | | 417(M⁺+1) |
| A-a-125 | 4f-1 | Int.A-29 | Hal9 | 4FBnO | Et | H | 6-Qu | C | | 456(M⁺+1) |
| A-a-126 | 1-a | A-a-125 | | 4FBnO | H | H | 6-Qu | C | | 428(M⁺+1) |
| A-a-127 | 4f-1 | Int.A-22 | Hal10 | 2ClBnO | Et | H | 2-Nap | C | | 471(M⁺+1) |
| A-a-128 | 1-a | A-a-127 | | 2ClBnO | H | H | 2-Nap | C | | 443(M⁺+1) |
| A-a-129 | 4f-1 | Int.A-27 | Hal10 | 2ClBnO | Et | H | 1Et-5-Idz | C | | 489(M⁺+1) |
| A-a-130 | 1-a | A-a-129 | | 2ClBnO | H | H | 1Et-5-Idz | C | | 461(M⁺+1) |
| A-a-131 | 4f-1 | Int.A-28 | Hal10 | 2ClBnO | Et | H | 5-BF | C | | 461(M⁺+1) |
| A-a-132 | 1-a | A-a-131 | | 2ClBnO | H | H | 5-BF | C | | 433(M⁺+1) |
| A-a-133 | 4f-1 | Int.A-22 | Hal11 | 3ClBnO | Et | H | 2-Nap | C | | 471(M⁺+1) |
| A-a-134 | 1-a | A-a-133 | | 3ClBnO | H | H | 2-Nap | C | | 443(M⁺+1) |
| A-a-135 | 4f-1 | Int.A-23 | Hal11 | 3ClBnO | Et | H | 5-Ind | C | | 460(M⁺+1) |
| A-a-136 | 1-a | A-a-135 | | 3ClBnO | H | H | 5-Ind | C | | 432(M⁺+1) |
| A-a-137 | 4f-1 | Int.A-24 | Hal11 | 3ClBnO | Et | H | 1Me-5-Ind | C | | 474(M⁺+1) |
| A-a-138 | 1-a | A-a-137 | | 3ClBnO | H | H | 1Me-5-Ind | C | | 446(M⁺+1) |
| A-a-139 | 4f-1 | Int.A-26 | Hal11 | 3ClBnO | Et | H | 1Me-5-Idz | C | | 475(M⁺+1) |
| A-a-140 | 1-a | A-a-139 | | 3ClBnO | H | H | 1Me-5-Idz | C | | 447(M⁺+1) |
| A-a-141 | 4f-1 | Int.A-23 | Hal12 | 4MeBnO | Et | H | 5-Ind | C | | 440(M⁺+1) |
| A-a-142 | 1-a | A-a-141 | | 4MeBnO | H | H | 5-Ind | C | | 412(M⁺+1) |

**[Table 12]**

| Table-A-a-4 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-a-143 | 4f-1 | Int.A-24 | Hal12 | 4MeBnO | Et | H | 1Me-5-Ind | C | | 454(M⁺+1) |
| A-a-144 | 1-a | A-a-143 | | 4MeBnO | H | H | 1Me-5-Ind | C | | 426(M⁺+1) |
| A-a-145 | 4f-1 | Int.A-26 | Hal12 | 4MeBnO | Et | H | 1Me-5-Idz | C | | 455(M⁺+1) |
| A-a-146 | 1-a | A-a-145 | | 4MeBnO | H | H | 1Me-5-Idz | C | | 427(M⁺+1) |
| A-a-147 | 4f-1 | Int.A-22 | Hal13 | 4CF3BnO | Et | H | 2-Nap | C | | 505(M⁺+1) |
| A-a-148 | 1-a | A-a-147 | | 4CF3BnO | H | H | 2-Nap | C | | 477(M⁺+1) |
| A-a-149 | 4f-1 | Int.A-24 | Hal13 | 4CF3BnO | Et | H | 1Me-5-Ind | C | | 508(M⁺+1) |
| A-a-150 | 1-a | A-a-149 | | 4CF3BnO | H | H | 1Me-5-Ind | C | | 480(M⁺+1) |
| A-a-151 | 4f-1 | Int.A-25 | Hal13 | 4CF3BnO | Et | H | 5-1Idz | C | | 495(M⁺+1) |
| A-a-152 | 1-a | A-a-151 | | 4CF3BnO | H | H | 5-1Idz | C | | 467(M⁺+1) |
| A-a-153 | 4f-2 | Int.A-22 | Al5 | 2EtBuO | Et | H | 2-Nap | C | | 431(M⁺+1) |
| A-a-154 | 1-a | A-a-153 | | 2EtBuO | H | H | 2-Nap | C | | 403(M⁺+1) |
| A-a-155 | 4f-2 | Int.A-23 | Al5 | 2EtBuO | Et | H | 5-Ind | C | | 420(M⁺+1) |
| A-a-156 | 1-a | A-a-155 | | 2EtBuO | H | H | 5-Ind | C | | 392(M⁺+1) |
| A-a-157 | 4f-2 | Int.A-26 | Al5 | 2EtBuO | Et | H | 1Me-5-Idz | C | | 435(M⁺+1) |
| A-a-158 | 1-a | A-a-157 | | 2EtBuO | H | H | 1Me-5-Idz | C | | 407(M⁺+1) |
| A-a-159 | 4f-2 | Int.A-23 | Al6 | 2(4DMAPh)EtO | Et | H | 5-Ind | C | | 483(M⁺+1) |
| A-a-160 | 1-a | A-a-159 | | 2(4DMAPh)EtO | H | H | 5-Ind | C | | 455(M⁺+1) |
| A-a-161 | 4f-2 | Int.A-24 | Al6 | 2(4DMAPh)EtO | Et | H | 1Me-5-Ind | C | | 497(M⁺+1) |
| A-a-162 | 1-a | A-a-161 | | 2(4DMAPh)EtO | H | H | 1Me-5-Ind | C | | 469(M⁺+1) |
| A-a-163 | 4f-2 | Int.A-27 | Al6 | 2(4DMAPh)EtO | Et | H | 1Et-5-Idz | C | | 512(M⁺+1) |
| A-a-164 | 1-a | A-a-163 | | 2(4DMAPh)EtO | H | H | 1Et-5-Idz | C | | 484(M⁺+1) |
| A-a-165 | 4f-2 | Int.A-22 | Al7 | 2(PhO)EtO | Et | H | 2-Nap | C | | 467(M⁺+1) |
| A-a-166 | 1-a | A-a-165 | | 2(PhO)EtO | H | H | 2-Nap | C | | 439(M⁺+1) |
| A-a-167 | 4f-2 | Int.A-24 | Al7 | 2(PhO)EtO | Et | H | 1Me-5-Ind | C | | 470(M⁺+1) |
| A-a-168 | 1-a | A-a-167 | | 2(PhO)EtO | H | H | 1Me-5-Ind | C | | 442(M⁺+1) |
| A-a-169 | 4f-2 | Int.A-29 | Al7 | 2(PhO)EtO | Et | H | 6-Qu | C | | 468(M⁺+1) |
| A-a-170 | 1-a | A-a-169 | | 2(PhO)EtO | H | H | 6-Qu | C | | 440(M⁺+1) |
| A-a-171 | 4f-2 | Int.A-22 | Al8 | 1PhEtO | Et | H | 2-Nap | C | | 451(M⁺+1) |
| A-a-172 | 1-a | A-a-171 | | 1PhEtO | H | H | 2-Nap | C | | 423(M⁺+1) |
| A-a-173 | 4f-2 | Int.A-24 | Al8 | 1PhEtO | Et | H | 1Me-5-Ind | C | | 454(M⁺+1) |
| A-a-174 | 1-a | A-a-173 | | 1PhEtO | H | H | 1Me-5-Ind | C | | 426(M⁺+1) |
| A-a-175 | 4f-2 | Int.A-25 | Al8 | 1PhEtO | Et | H | 5-1Idz | C | | 441(M⁺+1) |
| A-a-176 | 1-a | A-a-175 | | 1PhEtO | H | H | 5-1Idz | C | | 413(M⁺+1) |
| A-a-177 | 4f-2 | Int.A-23 | Al9 | 1(2FPh)EtO | Et | H | 5-Ind | C | | 458(M⁺+1) |
| A-a-178 | 1-a | A-a-177 | | 1(2FPh)EtO | H | H | 5-Ind | C | | 430(M⁺+1) |
| A-a-179 | 4f-2 | Int.A-27 | Al9 | 1(2FPh)EtO | Et | H | 1Et-5-Idz | C | | 487(M⁺+1) |
| A-a-180 | 1-a | A-a-179 | | 1(2FPh)EtO | H | H | 1Et-5-Idz | C | | 459(M⁺+1) |
| A-a-181 | 4f-2 | Int.A-29 | Al9 | 1(2FPh)EtO | Et | H | 6-Qu | C | | 470(M⁺+1) |
| A-a-182 | 1-a | A-a-181 | | 1(2FPh)EtO | H | H | 6-Qu | C | | 442(M⁺+1) |
| A-a-183 | 4f-2 | Int.A-23 | Al10 | 1(4ClPh)EtO | Et | H | 5-Ind | C | | 475(M⁺+1) |
| A-a-184 | 1-a | A-a-183 | | 1(4ClPh)EtO | H | H | 5-Ind | C | | 446(M⁺+1) |
| A-a-185 | 4f-2 | Int.A-24 | Al10 | 1(4ClPh)EtO | Et | H | 1Me-5-Ind | C | | 489(M⁺+1) |
| A-a-186 | 1-a | A-a-185 | | 1(4ClPh)EtO | H | H | 1Me-5-Ind | C | | 460(M⁺+1) |
| A-a-187 | 4f-2 | Int.A-27 | Al10 | 1(4ClPh)EtO | Et | H | 1Et-5-Idz | C | | 504(M⁺+1) |
| A-a-188 | 1-a | A-a-187 | | 1(4ClPh)EtO | H | H | 1Et-5-Idz | C | | 476(M⁺+1) |
| A-a-189 | 4f-2 | Int.A-22 | Al11 | 4Me,cHexO | Et | H | 2-Nap | C | | 443(M⁺+1) |
| A-a-190 | 1-a | A-a-189 | | 4Me,cHexO | H | H | 2-Nap | C | | 415(M⁺+1) |

**[Table 13]**

| Table-A-a-5 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-a-191 | 4f-2 | Int.A-24 | Al11 | 4Me,cHexO | Et | H | 1Me-5-Ind | C | | 446(M⁺+1) |
| A-a-192 | 1-a | A-a-191 | | 4Me,cHexO | H | H | 1Me-5-Ind | C | | 418(M⁺+1) |
| A-a-193 | 4f-2 | Int.A-23 | Al12 | 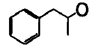 | Et | H | 5-Ind | C | | 454(M⁺+1) |
| A-a-194 | 1-a | A-a-193 | | 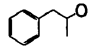 | H | H | 5-Ind | C | | 426(M⁺+1) |
| A-a-195 | 4f-2 | Int.A-26 | Al12 | 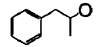 | Et | H | 1Me-5-Idz | C | | 469(M⁺+1) |
| A-a-196 | 1-a | A-a-195 | | 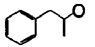 | H | H | 1Me-5-Idz | C | | 441(M⁺+1) |
| A-a-197 | 4f-2 | Int.A-22 | Al13 | 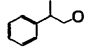 | Et | H | 2-Nap | C | | 465(M⁺+1) |
| A-a-198 | 1-a | A-a-197 | | 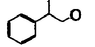 | H | H | 2-Nap | C | | 437(M⁺+1) |
| A-a-199 | 4f-2 | Int.A-27 | Al13 | 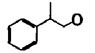 | Et | H | 1Et-5-Idz | C | | 483(M⁺+1) |
| A-a-200 | 1-a | A-a-199 | | 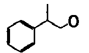 | H | H | 1Et-5-Idz | C | | 455(M⁺+1) |
| A-a-201 | 4f-2 | Int.A-23 | Al14 | 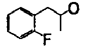 | Et | H | 5-Ind | C | | 472(M⁺+1) |
| A-a-202 | 1-a | A-a-201 | | 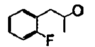 | H | H | 5-Ind | C | | 444(M⁺+1) |
| A-a-203 | 4f-2 | Int.A-24 | Al14 | 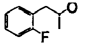 | Et | H | 1Me-5-Ind | C | | 486(M⁺+1) |
| A-a-204 | 1-a | A-a-203 | | 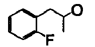 | H | H | 1Me-5-Ind | C | | 458(M⁺+1) |
| A-a-205 | 4f-2 | Int.A-23 | Al15 | 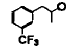 | Et | H | 5-Ind | C | | 522(M⁺+1) |
| A-a-206 | 1-a | A-a-205 | | 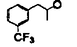 | H | H | 5-Ind | C | | 494(M⁺+1) |
| A-a-207 | 4f-2 | Int.A-24 | Al15 | 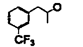 | Et | H | 1Me-5-Ind | C | | 536(M⁺+1) |
| A-a-208 | 1-a | A-a-207 | | 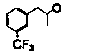 | H | H | 1Me-5-Ind | C | | 508(M⁺+1) |
| A-a-209 | 41-2 | Int.A-24 | Al16 | 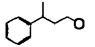 | Et | H | 1Me-5-Ind | C | | 482(M⁺+1) |
| A-a-210 | 1-a | A-a-209 | | 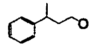 | H | H | 1Me-5-Ind | C | | 454(M⁺+1) |
| A-a-211 | 4f-2 | Int.A-26 | Al16 | 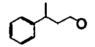 | Et | H | 1Me-5-Idz | C | | 483(M⁺+1) |
| A-a-212 | 1-a | A-a-211 | | 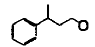 | H | H | 1Me-5-Idz | C | | 455(M⁺+1) |
| A-a-213 | 4f-2 | Int.A-22 | Al17 | 1IndanO | Et | H | 2-Nap | C | | 463(M⁺+1) |
| A-a-214 | 1-a | A-a-213 | | 1IndanO | H | H | 2-Nap | C | | 435(M⁺+1) |
| A-a-215 | 4f-2 | Int.A-24 | Al17 | 1IndanO | Et | H | 1Me-5-Ind | C | | 466(M⁺+1) |
| A-a-216 | 1-a | A-a-215 | | 1IndanO | H | H | 1Me-5-Ind | C | | 438(M⁺+1) |
| A-a-217 | 4f-2 | Int.A-25 | Al17 | 1IndanO | Et | H | 5-1Idz | C | | 453(M⁺+1) |
| A-a-218 | 1-a | A-a-217 | | 1IndanO | H | H | 5-1Idz | C | | 425(M⁺+1) |

**[Table 14]**

| Table-A-a-6 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-a-219 | 4f-2 | Int.A-22 | Al18 | 2IndanO | Et | H | 2-Nap | C | | 463(M⁺+1) |
| A-a-220 | 1-a | A-a-219 | | 2IndanO | H | H | 2-Nap | C | | 435(M⁺+1) |
| A-a-221 | 4f-2 | Int.A-23 | Al18 | 2IndanO | Et | H | 5-Ind | C | | 452(M⁺+1) |
| A-a-222 | 1-a | A-a-221 | | 2IndanO | H | H | 5-Ind | C | | 424(M⁺+1) |
| A-a-223 | 4f-2 | Int.A-26 | Al18 | 2IndanO | Et | H | 1Me-5-Idz | C | | 467(M⁺+1) |
| A-a-224 | 1-a | A-a-223 | | 2IndanO | H | H | 1Me-5-Idz | C | | 439(M⁺+1) |
| A-a-225 | 4f-2 | Int.A-24 | Al19 | 5OMe-2-IndanO | Et | H | 1Me-5-Ind | C | | 496(M⁺+1) |
| A-a-226 | 1-a | A-a-225 | | 5OMe-2-IndanO | H | H | 1Me-5-Ind | C | | 468(M⁺+1) |
| A-a-227 | 4f-2 | Int.A-25 | Al19 | 5OMe-2-IndanO | Et | H | 5-1Idz | C | | 483(M⁺+1) |
| A-a-228 | 1-a | A-a-227 | | 5OMe-2-IndanO | H | H | 5-1Idz | C | | 455(M⁺+1) |
| A-a-229 | 4f-2 | Int.A-27 | Al19 | 5OMe-2-IndanO | Et | H | 1Et-5-Idz | C | | 511(M⁺+1) |
| A-a-230 | 1-a | A-a-229 | | 5OMe-2-IndanO | H | H | 1Et-5-Idz | C | | 483(M⁺+1) |
| A-a-231 | 4f-2 | Int.A-22 | Al20 | 5,6D(OMe)-2-IndanO | Et | H | 2-Nap | C | | 523(M⁺+1) |
| A-a-232 | 1-a | A-a-231 | | 5,6D(OMe)-2-IndanO | H | H | 2-Nap | C | | 495(M⁺+1) |
| A-a-233 | 4f-2 | Int.A-23 | Al20 | 5,6D(OMe)-2-IndanO | Et | H | 5-Ind | C | | 512(M⁺+1) |
| A-a-234 | 1-a | A-a-233 | | 5,6D(OMe)-2-IndanO | H | H | 5-Ind | C | | 484(M⁺+1) |
| A-a-235 | 4f-2 | Int.A-22 | Al21 | 5F-2-IndanO | Et | H | 2-Nap | C | | 481(M⁺+1) |
| A-a-236 | 1-a | A-a-235 | | 5F-2-IndanO | H | H | 2-Nap | C | | 453(M⁺+1) |
| A-a-237 | 4f-2 | Int.A-24 | Al21 | 5F-2-IndanO | Et | H | 1Me-5-Ind | C | | 484(M⁺+1) |
| A-a-238 | 1-a | A-a-237 | | 5F-2-IndanO | H | H | 1Me-5-Ind | C | | 456(M⁺+1) |
| A-a-239 | 4f-2 | Int.A-26 | Al21 | 5F-2-IndanO | Et | H | 1Me-5-Idz | C | | 485(M⁺+1) |
| A-a-240 | 1-a | A-a-239 | | 5F-2-IndanO | H | H | 1Me-5-Idz | C | | 457(M⁺+1) |
| A-a-241 | 4f-2 | Int.A-27 | Al21 | 5F-2-IndanO | Et | H | 1Et-5-Idz | C | | 499(M⁺+1) |
| A-a-242 | 1-a | A-a-241 | | 5F-2-IndanO | H | H | 1Et-5-Idz | C | | 471(M⁺+1) |
| A-a-243 | 4f-2 | Int.A-23 | Al22 | 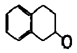 | Et | H | 5-Ind | C | | 466(M⁺+1) |
| A-a-244 | 1-a | A-a-243 | | 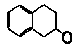 | H | H | 5-Ind | C | | 438(M⁺+1) |
| A-a-245 | 4f-2 | Int.A-24 | Al22 | 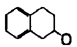 | Et | H | 1Me-5-Ind | C | | 480(M⁺+1) |
| A-a-246 | 1-a | A-a-245 | | 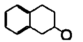 | H | H | 1Me-5-Ind | C | | 452(M⁺+1) |
| A-a-247 | 4f-2 | Int.A-22 | Al23 | 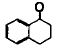 | Et | H | 2-Nap | C | | 477(M⁺+1) |
| A-a-248 | 1-a | A-a-247 | | 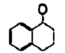 | H | H | 2-Nap | C | | 449(M⁺+1) |
| A-a-249 | 4f-2 | Int.A-25 | Al23 | 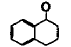 | Et | H | 5-1Idz | C | | 467(M⁺+1) |
| A-a-250 | 1-a | A-a-249 | | 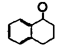 | H | H | 5-1Idz | C | | 439(M⁺+1) |
| A-a-251 | 4f-2 | Int.A-24 | Al24 | 2(2MePh)EtO | Et | H | 1Me-5-Ind | C | | 468(M⁺+1) |
| A-a-252 | 1-a | A-a-251 | | 2(2MePh)EtO | H | H | 1Me-5-Ind | C | | 440(M⁺+1) |
| A-a-253 | 4f-2 | Int.A-29 | Al24 | 2(2MePh)EtO | Et | H | 6-Qu | C | | 466(M⁺+1) |
| A-a-254 | 1-a | A-a-253 | | 2(2MePh)EtO | H | H | 6-Qu | C | | 438(M⁺+1) |

**[Table 15]**

| Table-A-a-7 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-a-255 | 4f-2 | Int.A-22 | Al25 | 2(3FPh)EtO | Et | H | 2-Nap | C | | 469(M⁺+1) |
| A-a-256 | 1-a | A-a-255 | | 2(3FPh)EtO | H | H | 2-Nap | C | | 441(M⁺+1) |
| A-a-257 | 4f-2 | Int.A-23 | Al25 | 2(3FPh)EtO | Et | H | 5-Ind | C | | 458(M⁺+1) |
| A-a-258 | 1-a | A-a-257 | | 2(3FPh)EtO | H | H | 5-Ind | C | | 430(M⁺+1) |
| A-a-259 | 4f-2 | Int.A-26 | Al26 | 2(2ClPh)EtO | Et | H | 1 Me-5-Idz | C | | 490(M⁺+1) |
| A-a-260 | 1-a | A-a-259 | | 2(2ClPh)EtO | H | H | 1Me-5-Idz | C | | 461(M⁺+1) |
| A-a-261 | 4f-2 | Int.A-29 | Al26 | 2(2ClPh)EtO | Et | H | 6-Qu | C | | 487(M⁺+1) |
| A-a-262 | 1-a | A-a-261 | | 2(2ClPh)EtO | H | H | 6-Qu | C | | 458(M⁺+1) |
| A-a-263 | 4f-2 | Int.A-22 | Al28 | 2(1-Nap)EtO | Et | H | 2-Nap | C | | 501(M⁺+1) |
| A-a-264 | 1-a | A-a-263 | | 2(1-Nap)EtO | H | H | 2-Nap | C | | 473(M⁺+1) |
| A-a-265 | 4f-2 | Int.A-23 | Al28 | 2(1-Nap)EtO | Et | H | 5-Ind | C | | 490(M⁺+1) |
| A-a-266 | 1-a | A-a-265 | | 2(1-Nap)EtO | H | H | 5-Ind | C | | 462(M⁺+1) |
| A-a-267 | 4f-2 | Int.A-24 | Al27 | 2(2-Nap)EtO | Et | H | 1Me-5-Ind | C | | 504(M⁺+1) |
| A-a-268 | 1-a | A-a-267 | | 2(2-Nap)EtO | H | H | 1Me-5-Ind | C | | 476(M⁺+1) |
| A-a-269 | 4f-2 | Int.A-25 | Al27 | 2(2-Nap)EtO | Et | H | 5-1Idz | C | | 491(M⁺+1) |
| A-a-270 | 1-a | A-a-269 | | 2(2-Nap)EtO | H | H | 5-1Idz | C | | 463(M⁺+1) |
| A-a-271 | 4f-2 | Int.A-22 | Al29 | 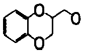 | Et | H | 2-Nap | C | | 495(M⁺+1) |
| A-a-272 | 1-a | A-a-271 | | 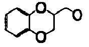 | H | H | 2-Nap | C | | 467(M⁺+1) |
| A-a-273 | 4f-2 | Int.A-23 | Al29 | 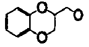 | Et | H | 5-Ind | C | | 484(M⁺+1) |
| A-a-274 | 1-a | A-a-273 | | 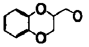 | H | H | 5-Ind | C | | 456(M⁺+1) |
| A-a-275 | 4f-2 | Int.A-22 | Al30 | 2(PhS)EtO | Et | H | 2-Nap | C | | 483(M⁺+1) |
| A-a-276 | 1-a | A-a-275 | | 2(PhS)EtO | H | H | 2-Nap | C | | 454(M⁺+1) |
| A-a-277 | 4f-2 | Int.A-23 | Al30 | 2(PhS)EtO | Et | H | 5-Ind | C | | 472(M⁺+1) |
| A-a-278 | 1-a | A-a-277 | | 2(PhS)EtO | H | H | 5-Ind | C | | 444(M⁺+1) |
| A-a-279 | 4f-2 | Int.A-24 | Al31 | 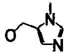 | Et | H | 1Me-5-Ind | C | | 444(M⁺+1) |
| A-a-280 | 1-a | A-a-279 | | 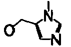 | H | H | 1Me-5-Ind | C | | 416(M⁺+1) |
| A-a-281 | 4f-2 | Int.A-27 | Al32 | 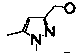 | Et | H | 1Et-5-Idz | C | | 458(M⁺+1) |
| A-a-282 | 1-a | A-a-281 | | 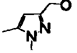 | H | H | 1Et-5-Idz | C | | 430(M⁺+1) |

**[Table 16]**

| Table-A-a-8 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-a-283 | 4f-2 | Int.A-22 | Al33 | 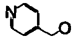 | Et | H | 2-Nap | C | | 438(M⁺+1) |
| A-a-284 | 1-a | A-a-283 | | 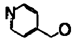 | H | H | 2-Nap | C | | 410(M⁺+1) |
| A-a-285 | 4f-2 | Int.A-23 | Al34 | 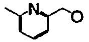 | Et | H | 5-Ind | C | | 441(M⁺+1) |
| A-a-286 | 1-a | A-a-285 | | 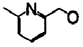 | H | H | 5-Ind | C | | 413(M⁺+1) |
| A-a-287 | 4f-2 | Int.A-23 | Al35 | 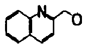 | Et | H | 5-Ind | C | | 477(M⁺+1) |
| A-a-288 | 1-a | A-a-287 | | 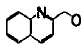 | H | H | 5-Ind | C | | 449(M⁺+1) |
| A-a-289 | 4f-2 | Int.A-26 | Al36 | 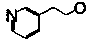 | Et | H | 1Me-5-Idz | C | | 456(M⁺+1) |
| A-a-290 | 1-a | A-a-289 | | 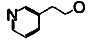 | H | H | 1Me-5-Idz | C | | 428(M⁺+1) |
| A-a-291 | 4e-1 | Int.A-4 | BRA5 | cPen | Et | H | 1Me-4-Ind | C | | 402(M⁺+1) |
| A-a-292 | 1-a | A-a-291 | | cPen | H | H | 1Me-4-Ind | C | | 374(M⁺+1) |
| A-a-293 | 4e-1 | Int.A-4 | BRA6 | cPen | Et | H | 1Me-6-Ind | C | | 402(M⁺+1) |
| A-a-294 | 1-a | A-a-293 | | cPen | H | H | 1Me-6-Ind | C | | 374(M⁺+1) |

### Reference Example 4

### Synthesis of ethyl 2-[4-chloro-5-hydroxy-6-(naphthalen-2-yl)-2,3-dihydro-1H-inden-1-yl]acetate (Intermediate A-30) (Preparation Method 4, Step h-1)

A solution of Intermediate A-22 (117.7 mg) in chloroform (3 ml) was added with sulfuryl chloride (41 µl, Ald) under ice cooling, and then the mixture was stirred for 10 minutes and stirred at room temperature for 17 hours. The mixture was concentrated under reduced pressure, and then added with water (10 ml) and chloroform (20 ml) for extraction, and then the organic layer was successively washed with saturated aqueous ammonium chloride, saturated aqueous sodium hydrogencarbonate and saturated brine. The organic layer was dried, and then the solvent was evaporated under reduced pressure to obtain the title compound (Intermediate A-30, 119.2 mg). Mass (LCMS): 381 (M⁺+1), Retention time: N.D (Elution condition: C).

### Reference Example 5

### Synthesis of ethyl 2-[4-bromo-5-hydroxy-6-(naphthalen-2-yl)-2,3-dihydro-1H-inden-1-yl]acetate (Intermediate A-34) (Preparation Method 4, Step h-2)

A solution of Intermediate A-22 (702.3 mg) in acetonitrile (10 ml) was added with NBS (370.6 mg) under ice cooling, and then the mixture was stirred for 10 minutes and stirred at room temperature 17 hours. The mixture was concentrated under reduced pressure, and then added with water (30 ml) and ethyl acetate (50 ml) for extraction, and then the organic layer was successively washed with saturated aqueous ammonium chloride, 5% aqueous sodium sulfite, saturated aqueous sodium hydrogencarbonate and saturated brine. The organic layer was dried, and then the solvent was evaporated under reduced pressure. The residue was purified by column chromatography (Quad, hexane:ethyl acetate = 8:1) to obtain the title compound (Intermediate A-34, 813.3 mg). Mass (LCMS): 425 (M⁺), Retention time: N.D (Elution condition: C).

Typical examples of the intermediates that can be obtained by reacting and treating corresponding starting compounds using any of the methods described in the present specification including the examples described above are shown in Table-Int.A-3. In the tables, the intermediate numbers are shown in the columns of "Exp.". In the tables, used methods among the aforementioned synthetic methods are shown in the columns of "Syn" with symbols, the starting compounds are shown in the columns of "SM", and the reagents are shown in the columns of "Regent".

**[Table 17]**

| Table-Int.A-3 | | | | | | | | | |
|---|---|---|---|---|---|---|---|---|---|
| 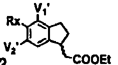 | | | | | | | | | |

| Exp. | Syn. | SM | Reagent | Rx | V1' | V2' | LCMS | | |
|---|---|---|---|---|---|---|---|---|---|
| | | | | | | | method | RTime | Mass |
| Int.A-31 | 4h-1 | A-a-24 | | HO | Cl | 1Me-5-Ind | C | | 384(M⁺+1) |
| Int.A-32 | 4h-1 | A-a-26 | | HO | Cl | 1Me-5-Idz | C | | 385(M⁺+1) |
| Int.A-33 | 4h-1 | A-a-27 | | HO | Cl | 1Et-5-Idz | C | | 399(M⁺+1) |
| Int.A-35 | 4h-2 | A-a-24 | | HO | Br | 1Me-5-Ind | C | | 427(M⁺) |
| Int.A-36 | 4h-2 | A-a-26 | | HO | Br | 1Me-5-Idz | C | | 428(M⁺) |
| Int.A-37 | 4h-2 | A-a-27 | | HO | Br | 1Et-5-Idz | C | | 442(M⁺) |

### Example A-b-1

### Synthesis of ethyl 2-[4-chloro-5-cyclopentyloxy-6-(naphthalen-2-yl)-2,3-dihydro-1H-inden-1-yl]acetate (Compound No. A-b-1) (Preparation Method 4, Step f-2)

According to the procedure described in the synthetic method of Intermediate A-1 in Reference Example 1 (Preparation Method 4, Step f-2) provided that the reaction was performed for 12 hours, Intermediate A-30 (115.6 mg), cyclopentanol (64.5 µl, Ald), triphenylphosphine (210.3 mg) and di-t-butyl azodicarboxylate (221.4 mg, Ald) were reacted and treated to obtain the title compound (Compound No. A-b-1, 134.7 mg).

### Example A-b-2

### Synthesis of 2-[4-chloro-5-cyclopentyloxy-6-(naphthalen-2-yl)-2,3-dihydro-1H-inden-1-yl]acetic acid (Compound No. A-b-2) (Preparation Method 1, Step a)

According to the procedure described in the synthetic method of Example Compound A-a-2 (Preparation Method 1, Step a) provided that the reaction was performed for 13 hours, Example Compound A-b-1 (134.5 mg) and 2 N aqueous sodium hydroxide (0.65 ml) were reacted and treated to obtain the title compound (Compound No. A-b-2, 121.3 mg).

### Example A-b-243

### Synthesis of ethyl 2-[4-bromo-5-cyclopentyloxy-6-(naphthalen-2-yl)-2,3-dihydro-1H-inden-1-yl]acetate (Compound No. A-b-243) (Preparation Method 4, Step f-2)

According to the procedure described in the synthetic method of Intermediate A-1 in Reference Example 1 (Preparation Method 4, Step f-2) provided that the reaction was performed for 12 hours, Intermediate A-34 (813.3 mg), cyclopentanol (200 µl, Ald), triphenylphosphine (561.4 mg) and di-t-butyl azodicarboxylate (611.3 mg, Ald) were reacted and treated to obtain the title compound (Compound No. A-b-243, 1.02 g).

### Example A-b-244

### Synthesis of 2-[4-bromo-5-cyclopentyloxy-6-(naphthalen-2-yl)-2,3-dihydro-1H-inden-1-yl]acetic acid (Compound No. A-b-244) (Preparation Method 1, Step a)

According to the procedure described in the synthetic method of Example Compound A-a-2 (Preparation Method 1, Step a) provided that the reaction was performed for 3 hours, Example Compound A-b-243 (142.3 mg) and 2 N aqueous sodium hydroxide (0.65 ml) were reacted and treated to obtain the title compound (Compound No. A-b-244, 118.8 mg).

### Example A-b-445

### Synthesis of ethyl 2-[5-cyclopentyloxy-4-methyl-6-(naphthalen-2-yl)-2,3-dihydro-1H-inden-1-yl]acetate (Compound No. A-b-445) (Preparation Method 4, Step e-1)

According to the procedure described in the synthetic method of Example Compound A-a-1 (Preparation Method 4, Step e-1) provided that the reaction was performed for 16 hours, Example Compound A-a-243 (356.6 mg), trimethylboroxine (600 µl, Ald), 2 M aqueous sodium carbonate (0.8 ml) and (Ph₃P)₄Pd (115.5 mg, Nakalai Tesque) were reacted and treated to obtain the title compound (Compound No. A-b-445, 101.3 mg).

### Example A-b-446

### Synthesis of 2-[5-cyclopentyloxy-4-methyl-6-(naphthalen-2-yl)-2,3-dihydro-1H-inden-1-yl]acetic acid (Compound No. A-b-446) (Preparation Method 1, Step a)

According to the procedure described in the synthetic method of Example Compound A-a-2 (Preparation Method 1, Step a) provided that the reaction was performed for 15 hours, Example Compound A-b-445 (98.6 mg) and 2 N aqueous sodium hydroxide (0.65 ml) were reacted and treated to obtain the title compound (Compound No. A-b-446, 73.4 mg).

Typical examples of the compounds of the present invention that can be obtained by reacting and treating corresponding starting compounds using any of the methods described in the present specification including the examples described above are shown in Table-A-b-1 to Table-A-b-14. The compounds were synthesized according to the preparation methods of the compounds of the compound numbers shown in the columns of "Syn" in the tables. "Int" means an intermediate compound number. In the tables, used methods among the aforementioned synthetic methods are shown in the columns of "Syn" with symbols, the starting compounds are shown in the columns of "SM", and the reagents are shown in the columns of "Regent". The halide regents mentioned in the columns of "Reagent" with symbols "Hal (No.)" are those mentioned in Table-Hal, the alcohol regents shown with symbols "Al (No.)" are those mentioned in Table-Al, and the boronic acid regents shown with symbols "BRA (No.)" are those mentioned in Table-BRA. Those regents for which cells of the columns of "Manufacturer" are blank were synthesized according to methods described in ordinary chemical literatures.

**[Table 18]**

| Table-A-b-1 | | | | | | | | | | |
|---|---|---|---|---|---|---|---|---|---|---|
| 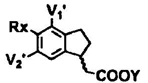 | | | | | | | | | | |

| Exp. | Syn. | SM | Reagent | Rx | Y | V1' | V2' | LCMS | | |
|---|---|---|---|---|---|---|---|---|---|---|
| | | | | | | | | method | RTime | Mass |
| A-b-1 | 4f-2 | Int.A-30 | Al1 | cPenO | Et | Cl | 2-Nap | C | | 449(M⁺+1) |
| A-b-2 | 1-a | A-b-1 | | cPenO | H | Cl | 2-Nap | C | | 421(M⁺+1) |
| A-b-3 | 4f-2 | Int.A-30 | Al2 | cPenMeO | Et | Cl | 2-Nap | C | | 463(M⁺+1) |
| A-b-4 | 1-a | A-b-3 | | cPenMeO | H | Cl | 2-Nap | C | | 431(M⁺+1) |
| A-b-5 | 4f-1 | Int.A-30 | Hal2 | cHexMeO | Et | Cl | 2-Nap | C | | 477(M⁺+1) |
| A-b-6 | 1-a | A-b-5 | | cHexMeO | H | Cl | 2-Nap | C | | 449(M⁺+1) |
| A-b-7 | 4f-2 | Int.A-30 | Al3 | cHexO | Et | Cl | 2-Nap | C | | 463(M⁺+1) |
| A-b-8 | 1-a | A-b-7 | | cHexO | H | Cl | 2-Nap | C | | 435(M⁺+1) |
| A-b-9 | 4f-2 | Int.A-30 | Al4 | cHepO | Et | Cl | 2-Nap | C | | 477(M⁺+1) |
| A-b-10 | 1-a | A-b-9 | | cHepO | H | Cl | 2-Nap | C | | 449(M⁺+1) |
| A-b-11 | 4f-1 | Int.A-30 | Hal3 | nPrO | Et | Cl | 2-Nap | C | | 422(M⁺+1) |
| A-b-12 | 1-a | A-b-11 | | nPrO | H | Cl | 2-Nap | C | | 394(M⁺+1) |
| A-b-13 | 4f-1 | Int.A-30 | Hal4 | iPrO | Et | Cl | 2-Nap | C | | 422(M⁺+1) |
| A-b-14 | 1-a | A-b-13 | | iPrO | H | Cl | 2-Nap | C | | 394(M⁺+1) |
| A-b-15 | 4f-1 | Int.A-30 | Hal5 | nBuO | Et | Cl | 2-Nap | C | | 437(M⁺+1) |
| A-b-16 | 1-a | A-b-15 | | nBuO | H | Cl | 2-Nap | C | | 409(M⁺+1) |
| A-b-17 | 4f-1 | Int.A-30 | Hal6 | iBuO | Et | Cl | 2-Nap | C | | 437(M⁺+1) |
| A-b-18 | 1-a | A-b-17 | | iBuO | H | Cl | 2-Nap | C | | 409(M⁺+1) |
| A-b-19 | 4f-1 | Int.A-30 | Hal1 | BnO | Et | Cl | 2-Nap | C | | 471(M⁺+1) |
| A-b-20 | 1-a | A-b-19 | | BnO | H | Cl | 2-Nap | C | | 443(M⁺+1) |
| A-b-21 | 4f-1 | Int.A-30 | Hal7 | 2FBnO | Et | Cl | 2-Nap | C | | 489(M⁺+1) |
| A-b-22 | 1-a | A-b-21 | | 2FBnO | H | Cl | 2-Nap | C | | 461(M⁺+1) |
| A-b-23 | 4f-1 | Int.A-30 | Hal8 | 3FBnO | Et | Cl | 2-Nap | C | | 489(M⁺+1) |
| A-b-24 | 1-a | A-b-23 | | 3FBnO | H | Cl | 2-Nap | C | | 461(M⁺+1) |
| A-b-25 | 4f-1 | Int.A-30 | Hal9 | 4FBnO | Et | Cl | 2-Nap | C | | 489(M⁺+1) |
| A-b-26 | 1-a | A-b-25 | | 4FBnO | H | Cl | 2-Nap | C | | 461(M⁺+1) |
| A-b-27 | 4f-1 | Int.A-30 | Hal10 | 2ClBnO | Et | Cl | 2-Nap | C | | 505(M⁺+1) |
| A-b-28 | 1-a | A-b-27 | | 2ClBnO | H | Cl | 2-Nap | C | | 487(M⁺+1) |
| A-b-29 | 4f-1 | Int.A-30 | Hal11 | 3ClBnO | Et | Cl | 2-Nap | C | | 505(M⁺+1) |
| A-b-30 | 1-a | A-b-29 | | 3ClBnO | H | Cl | 2-Nap | C | | 487(M⁺+1) |
| A-b-31 | 4f-1 | Int.A-30 | Hal12 | 4MeBnO | Et | Cl | 2-Nap | C | | 485(M⁺+1) |
| A-b-32 | 1-a | A-b-31 | | 4MeBnO | H | Cl | 2-Nap | C | | 457(M⁺+1) |
| A-b-33 | 4f-1 | Int.A-30 | Hal13 | 4CF3BnO | Et | Cl | 2-Nap | C | | 538(M⁺+1) |
| A-b-34 | 1-a | A-b-33 | | 4CF3BnO | H | Cl | 2-Nap | C | | 510(M⁺+1) |
| A-b-35 | 4f-2 | Int.A-30 | Al5 | 2EtBuO | Et | Cl | 2-Nap | C | | 465(M⁺+1) |
| A-b-36 | 1-a | A-b-35 | | 2EtBuO | H | Cl | 2-Nap | C | | 437(M⁺+1) |
| A-b-37 | 4f-2 | Int.A-30 | Al6 | 2(4DMAPh)EtO | Et | Cl | 2-Nap | C | | 528(M⁺+1) |
| A-b-38 | 1-a | A-b-37 | | 2(4DMAPh)EtO | H | Cl | 2-Nap | C | | 500(M⁺+1) |
| A-b-39 | 4f-2 | Int.A-30 | Al7 | 2(PhO)EtO | Et | Cl | 2-Nap | C | | 501(M⁺+1) |
| A-b-40 | 1-a | A-b-39 | | 2(PhO)EtO | H | Cl | 2-Nap | C | | 473(M⁺+1) |
| A-b-41 | 4f-2 | Int.A-30 | Al8 | 1PhEtO | Et | Cl | 2-Nap | C | | 485(M⁺+1) |
| A-b-42 | 1-a | A-b-41 | | 1PhEtO | H | Cl | 2-Nap | C | | 457(M⁺+1) |
| A-b-43 | 4f-2 | Int.A-30 | Al11 | 4Me,cHexO | Et | Cl | 2-Nap | C | | 477(M⁺+1) |
| A-b-44 | 1-a | A-b-43 | | 4Me,cHexO | H | Cl | 2-Nap | C | | 449(M⁺+1) |

**[Table 19]**

| Table-A-b-2 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-b-45 | 4f-2 | Int.A-30 | Al17 | 1IndanO | Et | Cl | 2-Nap | C | | 497(M⁺+1) |
| A-b-46 | 1-a | A-b-45 | | 1IndanO | H | Cl | 2-Nap | C | | 469(M⁺+1) |
| A-b-47 | 4f-2 | Int.A-30 | Al18 | 2IndanO | Et | Cl | 2-Nap | C | | 497(M⁺+1) |
| A-b-48 | 1-a | A-b-47 | | 2IndanO | H | Cl | 2-Nap | C | | 469(M⁺+1) |
| A-b-49 | 4f-2 | Int.A-30 | Al19 | 5OMe-2-IndanO | Et | Cl | 2-Nap | C | | 527(M⁺+1) |
| A-b-50 | 1-a | A-b-49 | | 5OMe-2-IndanO | H | Cl | 2-Nap | C | | 499(M⁺+1) |
| A-b-51 | 4f-2 | Int.A-30 | Al20 | 5,6D(OMe)-2-IndanO | Et | Cl | 2-Nap | C | | 557(M⁺+1) |
| A-b-52 | 1-a | A-b-51 | | 5,6D(OMe)-2-IndanO | H | Cl | 2-Nap | C | | 529(M⁺+1) |
| A-b-53 | 4f-2 | Int.A-30 | Al21 | 5F-2-IndanO | Et | Cl | 2-Nap | C | | 515(M⁺+1) |
| A-b-54 | 1-a | A-b-53 | | 5F-2-IndanO | H | Cl | 2-Nap | C | | 487(M⁺+1) |
| A-b-55 | 4f-2 | Int.A-30 | Al22 | 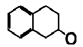 | Et | Cl | 2-Nap | C | | 511(M⁺+1) |
| A-b-56 | 1-a | A-b-55 | | 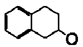 | H | Cl | 2-Nap | C | | 483(M⁺+1) |
| A-b-57 | 4f-2 | Int.A-30 | Al23 | 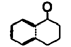 | Et | Cl | 2-Nap | C | | 511(M⁺+1) |
| A-b-58 | 1-a | A-b-57 | | 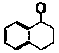 | H | Cl | 2-Nap | C | | 483(M⁺+1) |
| A-b-59 | 4f-2 | Int.A-30 | Al25 | 2(3FPh)EtO | Et | Cl | 2-Nap | C | | 503(M⁺+1) |
| A-b-60 | 1-a | A-b-59 | | 2(3FPh)EtO | H | Cl | 2-Nap | C | | 475(M⁺+1) |
| A-b-61 | 4f-2 | Int.A-30 | Al27 | 2(2-Nap)EtO | Et | Cl | 2-Nap | C | | 535(M⁺+1) |
| A-b-62 | 1-a | A-b-61 | | 2(2-Nap)EtO | H | Cl | 2-Nap | C | | 507(M⁺+1) |
| A-b-63 | 4f-2 | Int.A-31 | Al1 | cPenO | Et | Cl | 1Me-5-Ind | C | | 452(M⁺+1) |
| A-b-64 | 1-a | A-b-63 | | cPenO | H | Cl | 1Me-5-Ind | C | | 424(M⁺+1) |
| A-b-65 | 4f-2 | Int.A-31 | Al2 | cPenMeO | Et | Cl | 1Me-5-Ind | C | | 466(M⁺+1) |
| A-b-66 | 1-a | A-b-65 | | cPenMeO | H | Cl | 1Me-5-Ind | C | | 438(M⁺+1) |
| A-b-67 | 4f-1 | Int.A-31 | Hal2 | cHexMeO | Et | Cl | 1Me-5-Ind | C | | 480(M⁺+1) |
| A-b-68 | 1-a | A-b-67 | | cHexMeO | H | Cl | 1Me-5-Ind | C | | 452(M⁺+1) |
| A-b-69 | 4f-2 | Int.A-31 | Al3 | cHexO | Et | Cl | 1Me-5-Ind | C | | 466(M⁺+1) |
| A-b-70 | 1-a | A-b-69 | | cHexO | H | Cl | 1Me-5-Ind | C | | 438(M⁺+1) |
| A-b-71 | 4f-2 | Int.A-31 | Al4 | cHepO | Et | Cl | 1Me-5-Ind | C | | 480(M⁺+1) |
| A-b-72 | 1-a | A-b-71 | | cHepO | H | Cl | 1Me-5-Ind | C | | 452(M⁺+1) |
| A-b-73 | 4f-1 | Int.A-31 | Hal3 | nPrO | Et | Cl | 1Me-5-Ind | C | | 425(M⁺+1) |
| A-b-74 | 1-a | A-b-73 | | nPrO | H | Cl | 1Me-5-Ind | C | | 397(M⁺+1) |
| A-b-75 | 4f-1 | Int.A-31 | Hal4 | iPrO | Et | Cl | 1Me-5-Ind | C | | 425(M⁺+1) |
| A-b-76 | 1-a | A-b-75 | | iPrO | H | Cl | 1Me-5-Ind | C | | 397(M⁺+1) |
| A-b-77 | 4f-1 | Int.A-31 | Hal5 | nBuO | Et | Cl | 1Me-5-Ind | C | | 440(M⁺+1) |
| A-b-78 | 1-a | A-b-77 | | nBuO | H | Cl | 1Me-5-Ind | C | | 412(M⁺+1) |
| A-b-79 | 4f-1 | Int.A-31 | Hal6 | iBuO | Et | Cl | 1Me-5-Ind | C | | 440(M⁺+1) |
| A-b-80 | 1-a | A-b-79 | | iBuO | H | Cl | 1Me-5-Ind | C | | 412(M⁺+1) |
| A-b-81 | 4f-1 | Int.A-31 | Hal1 | BnO | Et | Cl | 1Me-5-Ind | C | | 474(M⁺+1) |
| A-b-82 | 1-a | A-b-81 | | BnO | H | Cl | 1Me-5-Ind | C | | 446(M⁺+1) |
| A-b-83 | 4f-1 | Int.A-31 | Hal7 | 2FBnO | Et | Cl | 1Me-5-Ind | C | | 492(M⁺+1) |
| A-b-84 | 1-a | A-b-83 | | 2FBnO | H | Cl | 1Me-5-Ind | C | | 464(M⁺+1) |
| A-b-85 | 4f-1 | Int.A-31 | Hal9 | 4FBnO | Et | Cl | 1Me-5-Ind | C | | 492(M⁺+1) |
| A-b-86 | 1-a | A-b-85 | | 4FBnO | H | Cl | 1Me-5-Ind | C | | 464(M⁺+1) |
| A-b-87 | 4f-1 | Int.A-31 | Hal11 | 3ClBnO | Et | Cl | 1Me-5-Ind | C | | 508(M⁺+1) |
| A-b-88 | 1-a | A-b-87 | | 3ClBnO | H | Cl | 1Me-5-Ind | C | | 480(M⁺+1) |

**[Table 20]**

| Table-A-b-3 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-b-89 | 4f-1 | Int.A-31 | Hal12 | 4MeBnO | Et | Cl | 1Me-5-Ind | C | | 488(M⁺+1) |
| A-b-90 | 1-a | A-b-89 | | 4MeBnO | H | Cl | 1Me-5-Ind | C | | 460(M⁺+1) |
| A-b-91 | 4f-1 | Int.A-31 | Hal13 | 4CF3BnO | Et | Cl | 1Me-5-Ind | C | | 541(M⁺+1) |
| A-b-92 | 1-a | A-b-91 | | 4CF3BnO | H | Cl | 1Me-5-Ind | C | | 513(M⁺+1) |
| A-b-93 | 4f-2 | Int.A-31 | Al5 | 2EtBuO | Et | Cl | 1Me-5-Ind | C | | 468(M⁺+1) |
| A-b-94 | 1-a | A-b-93 | | 2EtBuO | H | Cl | 1Me-5-Ind | C | | 440(M⁺+1) |
| A-b-95 | 4f-2 | Int.A-31 | Al6 | 2(4DMAPh)EtO | Et | Cl | 1Me-5-Ind | C | | 531(M⁺+1) |
| A-b-96 | 1-a | A-b-95 | | 2(4DMAPh)EtO | H | Cl | 1Me-5-Ind | C | | 503(M⁺+1) |
| A-b-97 | 4f-2 | Int.A-31 | Al8 | 1PhEtO | Et | Cl | 1Me-5-Ind | C | | 488(M⁺+1) |
| A-b-98 | 1-a | A-b-97 | | 1PhEtO | H | Cl | 1Me-5-Ind | C | | 460(M⁺+1) |
| A-b-99 | 4f-2 | Int.A-31 | Al11 | 4Me,cHexO | Et | Cl | 1Me-5-Ind | C | | 480(M⁺+1) |
| A-b-100 | 1-a | A-b-99 | | 4Me,cHexO | H | Cl | 1Me-5-Ind | C | | 452(M⁺+1) |
| A-b-101 | 4f-2 | Int.A-31 | Al17 | 1IndanO | Et | Cl | 1Me-5-Ind | C | | 500(M⁺+1) |
| A-b-102 | 1-a | A-b-101 | | 1IndanO | H | Cl | 1Me-5-Ind | C | | 472(M⁺+1) |
| A-b-103 | 4f-2 | Int.A-31 | Al18 | 2IndanO | Et | Cl | 1Me-5-Ind | C | | 500(M⁺+1) |
| A-b-104 | 1-a | A-b-103 | | 2IndanO | H | Cl | 1Me-5-Ind | C | | 472(M⁺+1) |
| A-b-105 | 4f-2 | Int.A-31 | Al19 | 5OMe-2-IndanO | Et | Cl | 1Me-5-Ind | C | | 530(M⁺+1) |
| A-b-106 | 1-a | A-b-105 | | 5OMe-2-IndanO | H | Cl | 1Me-5-Ind | C | | 502(M⁺+1) |
| A-b-107 | 4f-2 | Int.A-31 | Al20 | 5,6D(OMe)-2-IndanO | Et | Cl | 1Me-5-Ind | C | | 560(M⁺+1) |
| A-b-108 | 1-a | A-b-107 | | 5,6D(OMe)-2-IndanO | H | Cl | 1Me-5-Ind | C | | 532(M⁺+1) |
| A-b-109 | 4f-2 | Int.A-31 | Al21 | 5F-2-IndanO | Et | Cl | 1Me-5-Ind | C | | 518(M⁺+1) |
| A-b-110 | 1-a | A-b-109 | | 5F-2-IndanO | H | Cl | 1Me-5-Ind | C | | 490(M⁺+1) |
| A-b-111 | 4f-2 | Int.A-31 | Al22 | 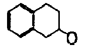 | Et | Cl | 1Me-5-Ind | C | | 514(M⁺+1) |
| A-b-112 | 1-a | A-b-111 | | 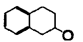 | H | Cl | 1Me-5-Ind | C | | 486(M⁺+1) |
| A-b-113 | 4f-2 | Int.A-31 | Al23 | 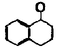 | Et | Cl | 1Me-5-Ind | C | | 514(M⁺+1) |
| A-b-114 | 1-a | A-b-113 | | 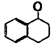 | H | Cl | 1Me-5-Ind | C | | 486(M⁺+1) |
| A-b-115 | 4f-2 | Int.A-31 | A25 | 2(3FPh)EtO | Et | Cl | 1Me-5-Ind | C | | 506(M⁺+1) |
| A-b-116 | 1-a | A-b-115 | | 2(3FPh)EtO | H | Cl | 1Me-5-Ind | C | | 478(M⁺+1) |
| A-b-117 | 4f-2 | Int.A-31 | Al27 | 2(2-Nap)EtO | Et | Cl | 1Me-5-Ind | C | | 538(M⁺+1) |
| A-b-118 | 1-a | A-b-117 | | 2(2-Nap)EtO | H | Cl | 1Me-5-Ind | C | | 510(M⁺+1) |
| A-b-119 | 4f-2 | Int.A-32 | Al1 | cPenO | Et | Cl | 1Me-5-Idz | C | | 453(M⁺+1) |
| A-b-120 | 1-a | A-b-119 | | cPenO | H | Cl | 1Me-5-Idz | C | | 425(M⁺+1) |
| A-b-121 | 4f-2 | Int.A-32 | Al2 | cPenMeO | Et | Cl | 1Me-5-Idz | C | | 467(M⁺+1) |
| A-b-122 | 1-a | A-b-121 | | cPenMeO | H | Cl | 1Me-5-Idz | C | | 439(M⁺+1) |
| A-b-123 | 4f-1 | Int.A-32 | Hal2 | cHexMeO | Et | Cl | 1Me-5-Idz | C | | 481(M⁺+1) |
| A-b-124 | 1-a | A-b-123 | | cHexMeO | H | Cl | 1Me-5-Idz | C | | 453(M⁺+1) |
| A-b-125 | 4f-2 | Int.A-32 | Al3 | cHexO | Et | Cl | 1Me-5-Idz | C | | 467(M⁺+1) |
| A-b-126 | 1-a | A-b-125 | | cHexO | H | Cl | 1Me-5-Idz | C | | 439(M⁺+1) |
| A-b-127 | 4f-2 | Int.A-32 | Al4 | cHepO | Et | Cl | 1Me-5-Idz | C | | 481(M⁺+1) |
| A-b-128 | 1-a | A-b-127 | | cHepO | H | Cl | 1Me-5-Idz | C | | 453(M⁺+1) |
| A-b-129 | 4f-1 | Int.A-32 | Hal3 | nPrO | Et | Cl | 1Me-5-Idz | C | | 426(M⁺+1) |
| A-b-130 | 1-a | A-b-129 | | nPrO | H | Cl | 1Me-5-Idz | C | | 398(M⁺+1) |
| A-b-131 | 4f-1 | Int.A-32 | Hal4 | iPrO | Et | Cl | 1Me-5-Idz | C | | 426(M⁺+1) |
| A-b-132 | 1-a | A-b-131 | | iPrO | H | Cl | 1Me-5-Idz | C | | 398(M⁺+1) |

**[Table 21]**

| Table-A-b-4 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-b-133 | 4f-1 | Int.A-32 | Hal5 | nBuO | Et | Cl | 1Me-5-Idz | C | | 440(M⁺+1) |
| A-b-134 | 1-a | A-b-133 | | nBuO | H | Cl | 1Me-5-Idz | C | | 412(M⁺+1) |
| A-b-135 | 4f-1 | Int.A-32 | Hal6 | iBuO | Et | Cl | 1Me-5-Idz | C | | 440(M⁺+1) |
| A-b-136 | 1-a | A-b-135 | | iBuO | H | Cl | 1Me-5-Idz | C | | 412(M⁺+1) |
| A-b-137 | 4f-1 | Int.A-32 | Hal1 | BnO | Et | Cl | 1Me-5-Idz | C | | 475(M⁺+1) |
| A-b-138 | 1-a | A-b-137 | | BnO | H | Cl | 1Me-5-Idz | C | | 447(M⁺+1) |
| A-b-139 | 4f-1 | Int.A-32 | Hal7 | 2FBnO | Et | Cl | 1Me-5-Idz | C | | 493(M⁺+1) |
| A-b-140 | 1-a | A-b-139 | | 2FBnO | H | Cl | 1Me-5-Idz | C | | 465(M⁺+1) |
| A-b-141 | 4f-1 | Int.A-32 | Hal8 | 3FBnO | Et | Cl | 1Me-5-Idz | C | | 493(M⁺+1) |
| A-b-142 | 1-a | A-b-141 | | 3FBnO | H | Cl | 1Me-5-Idz | C | | 465(M⁺+1) |
| A-b-143 | 4f-1 | Int.A-32 | Hal9 | 4FBnO | Et | Cl | 1Me-5-Idz | C | | 492(M⁺+1) |
| A-b-144 | 1-a | A-b-143 | | 4FBnO | H | Cl | 1Me-5-Idz | C | | 464(M⁺+1) |
| A-b-145 | 4f-1 | Int.A-32 | Hal10 | 2ClBnO | Et | Cl | 1Me-5-Idz | C | | 509(M⁺+1) |
| A-b-146 | 1-a | A-b-145 | | 2ClBnO | H | Cl | 1Me-5-Idz | C | | 481(M⁺+1) |
| A-b-147 | 4f-1 | Int.A-32 | Hal11 | 3ClBnO | Et | Cl | 1Me-5-Idz | C | | 509(M⁺+1) |
| A-b-148 | 1-a | A-b-147 | | 3ClBnO | H | Cl | 1Me-5-Idz | C | | 481(M⁺+1) |
| A-b-149 | 4f-1 | Int.A-32 | Hal12 | 4MeBnO | Et | Cl | 1Me-5-Idz | C | | 489(M⁺+1) |
| A-b-150 | 1-a | A-b-149 | | 4MeBnO | H | Cl | 1Me-5-Idz | C | | 461(M⁺+1) |
| A-b-151 | 4f-1 | Int.A-32 | Hal13 | 4CF3BnO | Et | Cl | 1Me-5-Idz | C | | 542(M⁺+1) |
| A-b-152 | 1-a | A-b-151 | | 4CF3BnO | H | Cl | 1Me-5-Idz | C | | 514(M⁺+1) |
| A-b-153 | 4f-2 | Int.A-32 | Al5 | 2EtBuO | Et | Cl | 1Me-5-Idz | C | | 469(M⁺+1) |
| A-b-154 | 1-a | A-b-153 | | 2EtBuO | H | Cl | 1Me-5-Idz | C | | 441(M⁺+1) |
| A-b-155 | 4f-2 | Int.A-32 | Al6 | 2(4DMAPh)EtO | Et | Cl | 1Me-5-Idz | C | | 533(M⁺+1) |
| A-b-156 | 1-a | A-b-155 | | 2(4DMAPh)EtO | H | Cl | 1Me-5-Idz | C | | 505(M⁺+1) |
| A-b-157 | 4f-2 | Int.A-32 | Al7 | 2(PhO)EtO | Et | Cl | 1Me-5-Idz | C | | 505(M⁺+1) |
| A-b-158 | 1-a | A-b-157 | | 2(PhO)EtO | H | Cl | 1Me-5-Idz | C | | 477(M⁺+1) |
| A-b-159 | 4f-2 | Int.A-32 | Al8 | 1PhEtO | Et | Cl | 1Me-5-Idz | C | | 489(M⁺+1) |
| A-b-160 | 1-a | A-b-159 | | 1PhEtO | H | Cl | 1Me-5-Idz | C | | 461(M⁺+1) |
| A-b-161 | 4f-2 | Int.A-32 | Al9 | 1(2FPh)EtO | Et | Cl | 1Me-5-Idz | C | | 507(M⁺+1) |
| A-b-162 | 1-a | A-b-161 | | 1(2FPh)EtO | H | Cl | 1Me-5-Idz | C | | 479(M⁺+1) |
| A-b-163 | 4f-2 | Int.A-32 | Al10 | 1(4ClPh)EtO | Et | Cl | 1Me-5-Idz | C | | 524(M⁺+1) |
| A-b-164 | 1-a | A-b-163 | | 1(4ClPh)EtO | H | Cl | 1Me-5-Idz | C | | 496(M⁺+1) |
| A-b-165 | 4f-2 | Int.A-32 | Al11 | 4Me,cHexO | Et | Cl | 1Me-5-Idz | C | | 481(M⁺+1) |
| A-b-166 | 1-a | A-b-165 | | 4Me,cHexO | H | Cl | 1Me-5-Idz | C | | 453(M⁺+1) |
| A-b-167 | 4f-2 | Int.A-32 | Al12 | 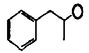 | Et | Cl | 1Me-5-Idz | C | | 517(M⁺+1) |
| A-b-168 | 1-a | A-b-167 | | 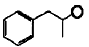 | H | Cl | 1Me-5-Idz | C | | 489(M⁺+1) |
| A-b-169 | 4f-2 | Int.A-32 | Al13 | 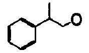 | Et | Cl | 1Me-5-Idz | C | | 517(M⁺+1) |
| A-b-170 | 1-a | A-b-169 | | 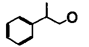 | H | Cl | 1Me-5-Idz | C | | 489(M⁺+1) |
| A-b-171 | 4f-2 | Int.A-32 | Al15 | 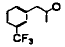 | Et | Cl | 1Me-5-Idz | C | | 585(M⁺+1) |
| A-b-172 | 1-a | A-b-171 | | 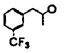 | H | Cl | 1Me-5-Idz | C | | 557(M⁺+1) |
| A-b-173 | 4f-2 | Int.A-32 | Al16 | 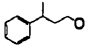 | Et | Cl | 1Me-5-Idz | C | | 517(M⁺+1) |

**[Table 22]**

| Table-A-b-5 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-b-174 | 1-a | A-b-173 | | 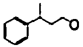 | H | Cl | 1Me-5-Idz | C | | 489(M⁺+1) |
| A-b-175 | 4f-2 | Int.A-32 | Al17 | 1IndanO | Et | Cl | 1Me-5-Idz | C | | 501(M⁺+1) |
| A-b-176 | 1-a | A-b-175 | | 1IndanO | H | Cl | 1Me-5-Idz | C | | 473(M⁺+1) |
| A-b-177 | 4f-2 | Int.A-32 | Al18 | 2IndanO | Et | Cl | 1Me-5-Idz | C | | 501(M⁺+1) |
| A-b-178 | 1-a | A-b-177 | | 2IndanO | H | Cl | 1Me-5-Idz | C | | 473(M⁺+1) |
| A-b-179 | 4f-2 | Int.A-32 | Al19 | 50Me-2-IndanO | Et | Cl | 1Me-5-Idz | C | | 531(M⁺+1) |
| A-b-180 | 1-a | A-b-179 | | 50Me-2-IndanO | H | Cl | 1Me-5-Idz | C | | 503(M⁺+1) |
| A-b-181 | 4f-2 | Int.A-32 | Al20 | 5,6D(OMe)-2-IndanO | Et | Cl | 1Me-5-Idz | C | | 561(M⁺+1) |
| A-b-182 | 1-a | A-b-181 | | 5,6D(OMe)-2-IndanO | H | Cl | 1Me-5-Idz | C | | 533(M⁺+1) |
| A-b-183 | 4f-2 | Int.A-32 | Al21 | 5F-2-IndanO | Et | Cl | 1Me-5-Idz | C | | 519(M⁺+1) |
| A-b-184 | 1-a | A-b-183 | | 5F-2-IndanO | H | Cl | 1Me-5-Idz | C | | 491(M⁺+1) |
| A-b-185 | 4f-2 | Int.A-32 | Al22 | 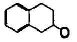 | Et | Cl | 1Me-5-Idz | C | | 515(M⁺+1) |
| A-b-186 | 1-a | A-b-185 | | 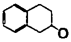 | H | Cl | 1Me-5-Idz | C | | 587(M⁺+1) |
| A-b-187 | 4f-2 | Int.A-32 | Al23 | 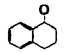 | Et | Cl | 1Me-5-Idz | C | | 515(M⁺+1) |
| A-b-188 | 1-a | A-b-187 | | 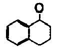 | H | Cl | 1Me-5-Idz | C | | 587(M⁺+1) |
| A-b-189 | 4f-2 | Int.A-32 | Al25 | 2(3FPh)EtO | Et | Cl | 1Me-5-Idz | C | | 507(M⁺+1) |
| A-b-190 | 1-a | A-b-141 | | 2(3FPh)EtO | H | Cl | 1Me-5-Idz | C | | 479(M⁺+1) |
| A-b-191 | 4f-2 | Int.A-32 | Al27 | 2(2-Nap)EtO | Et | Cl | 1Me-5-Idz | C | | 539(M⁺+1) |
| A-b-192 | 1-a | A-b-143 | | 2(2-Nap)EtO | H | Cl | 1Me-5-Idz | C | | 511(M⁺+1) |
| A-b-193 | 4f-2 | Int.A-33 | Al1 | cPenO | Et | Cl | 1Et-5-Idz | C | | 467(M⁺+1) |
| A-b-194 | 1-a | A-b-145 | | cPenO | H | Cl | 1Et-5-Idz | C | | 439(M⁺+1) |
| A-b-195 | 4f-2 | Int.A-33 | Al2 | cPenMeO | Et | Cl | 1Et-5-Idz | C | | 481(M⁺+1) |
| A-b-196 | 1-a | A-b-147 | | cPenMeO | H | Cl | 1Et-5-Idz | C | | 453(M⁺+1) |
| A-b-197 | 4f-2 | Int.A-33 | Al3 | cHexO | Et | Cl | 1Et-5-Idz | C | | 481(M⁺+1) |
| A-b-198 | 1-a | A-b-149 | | cHexO | H | Cl | 1Et-5-Idz | C | | 553(M⁺+1) |
| A-b-199 | 4f-1 | Int.A-33 | Hal3 | nPrO | Et | Cl | 1Et-5-Idz | C | | 441(M⁺+1) |
| A-b-200 | 1-a | A-b-151 | | nPrO | H | Cl | 1Et-5-Idz | C | | 413(M⁺+1) |
| A-b-201 | 4f-1 | Int.A-33 | Hal4 | iPrO | Et | Cl | 1Et-5-Idz | C | | 441(M⁺+1) |
| A-b-202 | 1-a | A-b-153 | | iPrO | H | Cl | 1Et-5-Idz | C | | 413(M⁺+1) |
| A-b-203 | 4f-1 | Int.A-33 | Hal5 | nBuO | Et | Cl | 1Et-5-Idz | C | | 455(M⁺+1) |
| A-b-204 | 1-a | A-b-155 | | nBuO | H | Cl | 1Et-5-Idz | C | | 427(M⁺+1) |
| A-b-205 | 4f-1 | Int.A-33 | Hal6 | iBuO | Et | Cl | 1Et-5-Idz | C | | 455(M⁺+1) |
| A-b-206 | 1-a | A-b-157 | | iBuO | H | Cl | 1Et-5-Idz | C | | 427(M⁺+1) |
| A-b-207 | 4f-1 | Int.A-33 | Hal1 | BnO | Et | Cl | 1Et-5-Idz | C | | 489(M⁺+1) |
| A-b-208 | 1-a | A-b-159 | | BnO | H | Cl | 1Et-5-Idz | C | | 461(M⁺+1) |
| A-b-209 | 4f-1 | Int.A-33 | Hal7 | 2FBnO | Et | Cl | 1Et-5-Idz | C | | 507(M⁺+1) |
| A-b-210 | 1-a | A-b-161 | | 2FBnO | H | Cl | 1Et-5-Idz | C | | 479(M⁺+1) |
| A-b-211 | 4f-1 | Int.A-33 | Hal9 | 4FBnO | Et | Cl | 1Et-5-Idz | C | | 507(M⁺+1) |
| A-b-212 | 1-a | A-b-163 | | 4FBnO | H | Cl | 1Et-5-Idz | C | | 479(M⁺+11) |
| A-b-213 | 4f-1 | Int.A-33 | Hal11 | 3ClBnO | Et | Cl | 1Et-5-Idz | C | | 523(M⁺+1) |
| A-b-214 | 1-a | A-b-165 | | 3ClBnO | H | Cl | 1Et-5-Idz | C | | 495(M⁺+1) |
| A-b-215 | 4f-1 | Int.A-33 | Hal12 | 4MeBnO | Et | Cl | 1Et-5-Idz | C | | 503(M⁺+1) |
| A-b-216 | 1-a | A-b-167 | | 4MeBnO | H | Cl | 1Et-5-Idz | C | | 475(M⁺+1) |
| A-b-217 | 4f-2 | Int.A-33 | Al5 | 2EtBuO | Et | Cl | 1Et-5-Idz | C | | 483(M⁺+1) |

**[Table 23]**

| Table-A-b-6 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-b-218 | 1-a | A-b-169 | | 2EtBuO | H | Cl | 1Et-5-Idz | C | | 456(M⁺+1) |
| A-b-219 | 4f-2 | Int.A-33 | Al6 | 2(4DMAPh)EtO | Et | Cl | 1Et-5-Idz | C | | 547(M⁺+1) |
| A-b-220 | 1-a | A-b-171 | | 2(4DMAPh)EtO | H | Cl | 1Et-5-Idz | C | | 519(M⁺+1) |
| A-b-221 | 4f-2 | Int.A-33 | Al8 | 1PhEtO | Et | Cl | 1Et-5-Idz | C | | 503(M⁺+1) |
| A-b-222 | 1-a | A-b-173 | | 1PhEtO | H | Cl | 1Et-5-Idz | C | | 475(M⁺+1) |
| A-b-223 | 4f-2 | Int.A-33 | Al11 | 4Me,cHexO | Et | Cl | 1Et-5-Idz | C | | 495(M⁺+1) |
| A-b-224 | 1-a | A-b-175 | | 4Me,cHexO | H | Cl | 1Et-5-Idz | C | | 467(M⁺+1) |
| A-b-225 | 4f-2 | Int.A-33 | Al17 | 1IndanO | Et | Cl | 1Et-5-Idz | C | | 515(M⁺+1) |
| A-b-226 | 1-a | A-b-177 | | 1IndanO | H | Cl | 1Et-5-Idz | C | | 487(M⁺+1) |
| A-b-227 | 4f-2 | Int.A-33 | Al18 | 2IndanO | Et | Cl | 1Et-5-Idz | C | | 515(M⁺+1) |
| A-b-228 | 1-a | A-b-179 | | 2IndanO | H | Cl | 1Et-5-Idz | C | | 487(M⁺+1) |
| A-b-229 | 4f-2 | Int.A-33 | Al19 | 5OMe-2-IndanO | Et | Cl | 1Et-5-Idz | C | | 545(M⁺+1) |
| A-b-230 | 1-a | A-b-181 | | 5OMe-2-IndanO | H | Cl | 1Et-5-Idz | C | | 517(M⁺+1) |
| A-b-231 | 4f-2 | Int.A-33 | Al20 | 5,6D(OMe)-2-IndanO | Et | Cl | 1Et-5-Idz | C | | 575(M⁺+1) |
| A-b-232 | 1-a | A-b-183 | | 5,6D(OMe)-2-IndanO | H | Cl | 1Et-5-Idz | C | | 547(M⁺+1) |
| A-b-233 | 4f-2 | Int.A-33 | Al21 | 5F-2-IndanO | Et | Cl | 1Et-5-Idz | C | | 533(M⁺+1) |
| A-b-234 | 1-a | A-b-185 | | 5F-2-IndanO | H | Cl | 1Et-5-Idz | C | | 505(M⁺+1) |
| A-b-235 | 4f-2 | Int.A-33 | Al22 | 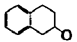 | Et | Cl | 1Et-5-Idz | C | | 529(M⁺+1) |
| A-b-236 | 1-a | A-b-187 | | 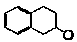 | H | Cl | 1Et-5-Idz | C | | 501(M⁺+1) |
| A-b-237 | 4f-2 | Int.A-33 | Al23 | 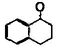 | Et | Cl | 1Et-5-Idz | C | | 529(M⁺+1) |
| A-b-238 | 1-a | A-b-153 | | 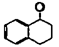 | H | Cl | 1Et-5-Idz | C | | 501(M⁺+1) |
| A-b-239 | 4f-2 | Int.A-33 | Al25 | 2(3FPh)EtO | Et | Cl | 1Et-5-Idz | C | | 521(M⁺+1) |
| A-b-240 | 1-a | A-b-155 | | 2(3FPh)EtO | H | Cl | 1Et-5-Idz | C | | 493(M⁺+1) |
| A-b-241 | 4f-2 | Int.A-33 | Al27 | 2(2-Nap)EtO | Et | Cl | 1Et-5-Idz | C | | 553(M⁺+1) |
| A-b-242 | 1-a | A-b-157 | | 2(2-Nap)EtO | H | Cl | 1Et-5-Idz | C | | 525(M⁺+1) |
| A-b-243 | 4f-2 | Int.A-34 | Al1 | cPenO | Et | Br | 2-Nap | C | | 492(M⁺) |
| A-b-244 | 1-a | A-b-159 | | cPenO | H | Br | 2-Nap | C | | 464(M⁺) |
| A-b-245 | 4f-2 | Int.A-34 | Al2 | cPenMeO | Et | Br | 2-Nap | C | | 506(M⁺) |
| A-b-246 | 1-a | A-b-161 | | cPenMeO | H | Br | 2-Nap | C | | 478(M⁺) |
| A-b-247 | 4f-1 | Int.A-34 | Hal2 | cHexMeO | Et | Br | 2-Nap | C | | 520(M⁺) |
| A-b-248 | 1-a | A-b-163 | | cHexMeO | H | Br | 2-Nap | C | | 492(M⁺) |
| A-b-249 | 4f-2 | Int.A-34 | Al3 | cHexO | Et | Br | 2-Nap | C | | 506(M⁺) |
| A-b-250 | 1-a | A-b-165 | | cHexO | H | Br | 2-Nap | C | | 478(M⁺) |
| A-b-251 | 4f-2 | Int.A-34 | Al4 | cHepO | Et | Br | 2-Nap | C | | 520(M⁺) |
| A-b-252 | 1-a | A-b-167 | | cHepO | H | Br | 2-Nap | C | | 492(M⁺) |
| A-b-253 | 4f-1 | Int.A-34 | Hal3 | nPrO | Et | Br | 2-Nap | C | | 466(M⁺) |
| A-b-254 | 1-a | A-b-169 | | nPrO | H | Br | 2-Nap | C | | 438(M⁺) |
| A-b-255 | 4f-1 | Int.A-34 | Hal4 | iPrO | Et | Br | 2-Nap | C | | 466(M⁺) |
| A-b-256 | 1-a | A-b-171 | | iPrO | H | Br | 2-Nap | C | | 438(M⁺) |
| A-b-257 | 4f-1 | Int.A-34 | Hal5 | nBuO | Et | Br | 2-Nap | C | | 480(M⁺) |
| A-b-258 | 1-a | A-b-173 | | nBuO | H | Br | 2-Nap | C | | 452(M⁺) |
| A-b-259 | 4f-1 | Int.A-34 | Hal6 | iBuO | Et | Br | 2-Nap | C | | 480(M⁺) |
| A-b-260 | 1-a | A-b-175 | | iBuO | H | Br | 2-Nap | C | | 452(M⁺) |
| A-b-261 | 4f-1 | Int.A-34 | Hal1 | BnO | Et | Br | 2-Nap | C | | 514(M⁺) |
| A-b-262 | 1-a | A-b-177 | | BnO | H | Br | 2-Nap | C | | 486(M⁺) |

**[Table 24]**

| Table-A-b-7 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-b-263 | 4f-1 | Int.A-34 | Hal7 | 2FBnO | Et | Br | 2-Nap | C | | 532(M⁺) |
| A-b-264 | 1-a | A-b-263 | | 2FBnO | H | Br | 2-Nap | C | | 504(M⁺) |
| A-b-265 | 4f-1 | Int.A-34 | Hal8 | 3FBnO | Et | Br | 2-Nap | C | | 532(M⁺) |
| A-b-266 | 1-a | A-b-265 | | 3FBnO | H | Br | 2-Nap | C | | 504(M⁺) |
| A-b-267 | 4f-1 | Int.A-34 | Hal9 | 4FBnO | Et | Br | 2-Nap | C | | 532(M⁺) |
| A-b-268 | 1-a | A-b-267 | | 4FBnO | H | Br | 2-Nap | C | | 504(M⁺) |
| A-b-269 | 4f-1 | Int.A-34 | Hal10 | 2ClBnO | Et | Br | 2-Nap | C | | 548(M⁺) |
| A-b-270 | 1-a | A-b-269 | | 2ClBnO | H | Br | 2-Nap | C | | 520(M⁺) |
| A-b-271 | 4f-1 | Int.A-34 | Hal11 | 3ClBnO | Et | Br | 2-Nap | C | | 548(M⁺) |
| A-b-272 | 1-a | A-b-271 | | 3ClBnO | H | Br | 2-Nap | C | | 520(M⁺) |
| A-b-273 | 4f-1 | Int.A-34 | Hal12 | 4MeBnO | Et | Br | 2-Nap | C | | 528(M⁺) |
| A-b-274 | 1-a | A-b-273 | | 4MeBnO | H | Br | 2-Nap | C | | 500(M⁺) |
| A-b-275 | 4f-1 | Int.A-34 | Hal13 | 4CF3BnO | Et | Br | 2-Nap | C | | 582(M⁺) |
| A-b-276 | 1-a | A-b-275 | | 4CF3BnO | H | Br | 2-Nap | C | | 554(M⁺) |
| A-b-277 | 4f-2 | Int.A-34 | Al5 | 2EtBuO | Et | Br | 2-Nap | C | | 508(M⁺) |
| A-b-278 | 1-a | A-b-277 | | 2EtBuO | H | Br | 2-Nap | C | | 480(M⁺) |
| A-b-279 | 4f-2 | Int.A-34 | Al6 | 2(4DMAPh)EtO | Et | Br | 2-Nap | C | | 572(M⁺) |
| A-b-280 | 1-a | A-b-279 | | 2(4DMAPh)EtO | H | Br | 2-Nap | C | | 544(M⁺) |
| A-b-281 | 4f-2 | Int.A-34 | Al7 | 2(PhO)EtO | Et | Br | 2-Nap | C | | 544(M⁺) |
| A-b-282 | 1-a | A-b-281 | | 2(PhO)EtO | H | Br | 2-Nap | C | | 516(M⁺) |
| A-b-283 | 4f-2 | Int.A-34 | Al8 | 1PhEtO | Et | Br | 2-Nap | C | | 528(M⁺) |
| A-b-284 | 1-a | A-b-283 | | 1PhEtO | H | Br | 2-Nap | C | | 500(M⁺) |
| A-b-285 | 4f-2 | Int.A-34 | Al11 | 4Me,cHexO | Et | Br | 2-Nap | C | | 520(M⁺) |
| A-b-286 | 1-a | A-b-285 | | 4Me,cHexO | H | Br | 2-Nap | C | | 492(M⁺) |
| A-b-287 | 4f-2 | Int.A-34 | Al17 | 1IndanO | Et | Br | 2-Nap | C | | 540(M⁺) |
| A-b-288 | 1-a | A-b-287 | | 1IndanO | H | Br | 2-Nap | C | | 512(M⁺) |
| A-b-289 | 4f-2 | Int.A-34 | Al18 | 2IndanO | Et | Br | 2-Nap | C | | 540(M⁺) |
| A-b-290 | 1-a | A-b-289 | | 2IndanO | H | Br | 2-Nap | C | | 512(M⁺) |
| A-b-291 | 4f-2 | Int.A-34 | Al19 | 5OMe-2-IndanO | Et | Br | 2-Nap | C | | 570(M⁺) |
| A-b-292 | 1-a | A-b-291 | | 5OMe-2-IndanO | H | Br | 2-Nap | C | | 542(M⁺) |
| A-b-293 | 4f-2 | Int.A-34 | Al20 | 5,6D(OMe)-2-IndanO | Et | Br | 2-Nap | C | | 600(M⁺) |
| A-b-294 | 1-a | A-b-293 | | 5,6D(OMe)-2-IndanO | H | Br | 2-Nap | C | | 572(M⁺) |
| A-b-295 | 4f-2 | Int.A-34 | Al21 | 5F-2-IndanO | Et | Br | 2-Nap | C | | 558(M⁺) |
| A-b-296 | 1-a | A-b-295 | | 5F-2-IndanO | H | Br | 2-Nap | C | | 530(M⁺) |
| A-b-297 | 4f-2 | Int.A-34 | Al22 | 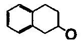 | Et | Br | 2-Nap | C | | 554(M⁺) |
| A-b-298 | 1-a | A-b-297 | | 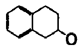 | H | Br | 2-Nap | C | | 526(M⁺) |
| A-b-299 | 4f-2 | Int.A-34 | Al23 | 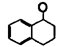 | Et | Br | 2-Nap | C | | 554(M⁺) |
| A-b-300 | 1-a | A-b-299 | | 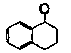 | H | Br | 2-Nap | C | | 526(M⁺) |
| A-b-301 | 4f-2 | Int.A-34 | Al25 | 2(3FPh)EtO | Et | Br | 2-Nap | C | | 546(M⁺) |
| A-b-302 | 1-a | A-b-301 | | 2(3FPh)EtO | H | Br | 2-Nap | C | | 518(M⁺) |
| A-b-303 | 4f-2 | Int.A-34 | Al27 | 2(2-Nap)EtO | Et | Br | 2-Nap | C | | 578(M⁺) |
| A-b-304 | 1-a | A-b-303 | | 2(2-Nap)EtO | H | Br | 2-Nap | C | | 550(M⁺) |
| A-b-305 | 4f-2 | Int.A-35 | Al1 | cPenO | Et | Br | 1Me-5-Ind | C | | 495(M⁺) |
| A-b-306 | 1-a | A-b-305 | | cPenO | H | Br | 1Me-5-Ind | C | | 467(M⁺) |

**[Table 25]**

| Table-A-b-8 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-b-307 | 4f-1 | Int.A-35 | Hal2 | cHexMeO | Et | Br | 1Me-5-Ind | C | | 523(M⁺) |
| A-b-308 | 1-a | A-b-307 | | cHexMeO | H | Br | 1Me-5-Ind | C | | 495(M⁺) |
| A-b-309 | 4f-2 | Int.A-35 | Al3 | cHexO | Et | Br | 1Me-5-Ind | C | | 509(M⁺) |
| A-b-310 | 1-a | A-b-309 | | cHexO | H | Br | 1Me-5-Ind | C | | 481(M⁺) |
| A-b-311 | 4f-1 | Int.A-35 | Hal3 | nPrO | Et | Br | 1Me-5-Ind | C | | 469(M⁺) |
| A-b-312 | 1-a | A-b-311 | | nPrO | H | Br | 1Me-5-Ind | C | | 441(M⁺) |
| A-b-313 | 4f-1 | Int.A-35 | Hal4 | iPrO | Et | Br | 1Me-5-Ind | C | | 469(M⁺) |
| A-b-314 | 1-a | A-b-313 | | iPrO | H | Br | 1Me-5-Ind | C | | 441(M⁺) |
| A-b-315 | 4f-1 | Int.A-35 | Hal5 | nBuO | Et | Br | 1Me-5-Ind | C | | 483(M⁺) |
| A-b-316 | 1-a | A-b-315 | | nBuO | H | Br | 1Me-5-Ind | C | | 455(M⁺) |
| A-b-317 | 4f-1 | Int.A-35 | Hal6 | iBuO | Et | Br | 1Me-5-Ind | C | | 483(M⁺) |
| A-b-318 | 1-a | A-b-317 | | iBuO | H | Br | 1Me-5-Ind | C | | 455(M⁺) |
| A-b-319 | 4f-1 | Int.A-35 | Hal1 | BnO | Et | Br | 1Me-5-Ind | C | | 517(M⁺) |
| A-b-320 | 1-a | A-b-319 | | BnO | H | Br | 1Me-5-Ind | C | | 489(M⁺) |
| A-b-321 | 4f-1 | Int.A-35 | Hal7 | 2FBnO | Et | Br | 1Me-5-Ind | C | | 535(M⁺) |
| A-b-322 | 1-a | A-b-321 | | 2FBnO | H | Br | 1Me-5-Ind | C | | 507(M⁺) |
| A-b-323 | 4f-1 | Int.A-35 | Hal8 | 3FBnO | Et | Br | 1Me-5-Ind | C | | 535(M⁺) |
| A-b-324 | 1-a | A-b-323 | | 3FBnO | H | Br | 1Me-5-Ind | C | | 507(M⁺) |
| A-b-325 | 4f-1 | Int.A-35 | Hal9 | 4FBnO | Et | Br | 1Me-5-Ind | C | | 535(M⁺) |
| A-b-326 | 1-a | A-b-325 | | 4FBnO | H | Br | 1Me-5-Ind | C | | 507(M⁺) |
| A-b-327 | 4f-1 | Int.A-35 | Hal11 | 3ClBnO | Et | Br | 1Me-5-Ind | C | | 551(M⁺) |
| A-b-328 | 1-a | A-b-327 | | 3ClBnO | H | Br | 1Me-5-Ind | C | | 523(M⁺) |
| A-b-329 | 4f-1 | Int.A-35 | Hal12 | 4MeBnO | Et | Br | 1Me-5-Ind | C | | 531(M⁺) |
| A-b-330 | 1-a | A-b-329 | | 4MeBnO | H | Br | 1Me-5-Ind | C | | 503(M⁺) |
| A-b-331 | 4f-2 | Int.A-35 | Al5 | 2EtBuO | Et | Br | 1Me-5-Ind | C | | 511(M⁺) |
| A-b-332 | 1-a | A-b-331 | | 2EtBuO | H | Br | 1Me-5-Ind | C | | 483(M⁺) |
| A-b-333 | 4f-2 | Int.A-35 | Al6 | 2(4DMAPh)EtO | Et | Br | 1Me-5-Ind | C | | 574(M⁺) |
| A-b-334 | 1-a | A-b-333 | | 2(4DMAPh)EtO | H | Br | 1Me-5-Ind | C | | 546(M⁺) |
| A-b-335 | 4f-2 | Int.A-35 | Al8 | 1PhEtO | Et | Br | 1Me-5-Ind | C | | 531(M⁺) |
| A-b-336 | 1-a | A-b-335 | | 1PhEtO | H | Br | 1Me-5-Ind | C | | 503(M⁺) |
| A-b-337 | 4f-2 | Int.A-35 | Al11 | 4Me,cHexO | Et | Br | 1Me-5-Ind | C | | 523(M⁺) |
| A-b-338 | 1-a | A-b-337 | | 4Me,cHexO | H | Br | 1Me-5-Ind | C | | 495(M⁺) |
| A-b-339 | 4f-2 | Int.A-35 | Al17 | 1IndanO | Et | Br | 1Me-5-Ind | C | | 543(M⁺) |
| A-b-340 | 1-a | A-b-339 | | 1IndanO | H | Br | 1Me-5-Ind | C | | 515(M⁺) |
| A-b-341 | 4f-2 | Int.A-35 | Al18 | 2IndanO | Et | Br | 1Me-5-Ind | C | | 543(M⁺) |
| A-b-342 | 1-a | A-b-341 | | 2IndanO | H | Br | 1Me-5-Ind | C | | 515(M⁺) |
| A-b-343 | 4f-2 | Int.A-35 | Al19 | 5OMe-2-IndanO | Et | Br | 1Me-5-Ind | C | | 573(M⁺) |
| A-b-344 | 1-a | A-b-343 | | 5OMe-2-IndanO | H | Br | 1Me-5-Ind | C | | 545(M⁺) |
| A-b-345 | 4f-2 | Int.A-35 | Al20 | 5,6D(OMe)-2-IndanO | Et | Br | 1Me-5-Ind | C | | 603(M⁺) |
| A-b-346 | 1-a | A-b-345 | | 5,6D(OMe)-2-IndanO | H | Br | 1Me-5-Ind | C | | 575(M⁺) |
| A-b-347 | 4f-2 | Int.A-35 | Al21 | 5F-2-IndanO | Et | Br | 1Me-5-Ind | C | | 561(M⁺) |
| A-b-348 | 1-a | A-b-347 | | 5F-2-IndanO | H | Br | 1Me-5-Ind | C | | 533(M⁺) |
| A-b-349 | 4f-2 | Int.A-35 | Al22 | 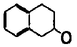 | Et | Br | 1Me-5-Ind | C | | 557(M⁺) |
| A-b-350 | 1-a | A-b-349 | | 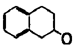 | H | Br | 1Me-5-Ind | C | | 529(M⁺) |

**[Table 26]**

| Table-A-b-9 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-b-351 | 4f-2 | Int.A-35 | Al23 | 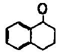 | Et | Br | 1Me-5-Ind | C | | 557(M⁺) |
| A-b-352 | 1a | A-b-351 | | 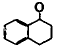 | H | Br | 1Me-5-Ind | C | | 529(M⁺) |
| A-b-353 | 4f-2 | Int.A-35 | Al27 | 2(2-Nap)EtO | Et | Br | 1Me-5-Ind | C | | 581(M⁺) |
| A-b-354 | 1a | A-b-353 | | 2(2-Nap)EtO | H | Br | 1Me-5-Ind | C | | 553M⁺) |
| A-b-355 | 4f-2 | Int.A-36 | Al1 | cPenO | Et | Br | 1Me-5-Idz | C | | 496(M⁺) |
| A-b-356 | 1a | A-b-355 | | cPenO | H | Br | 1Me-5-Idz | C | | 468(M⁺) |
| A-b-357 | 4f-2 | Int.A-36 | Al2 | cPenMeO | Et | Br | 1Me-5-Idz | C | | 510(M⁺) |
| A-b-358 | 1a | A-b-357 | | cPenMeO | H | Br | 1Me-5-Idz | C | | 482(M⁺) |
| A-b-359 | 4f-2 | Int.A-36 | Al3 | cHexO | Et | Br | 1Me-5-Idz | C | | 510(M⁺) |
| A-b-360 | 1a | A-b-359 | | cHexO | H | Br | 1Me-5-Idz | C | | 482(M⁺) |
| A-b-361 | 4f-2 | Int.A-36 | Al4 | cHepO | Et | Br | 1Me-5-Idz | C | | 524(M⁺) |
| A-b-362 | 1a | A-b-361 | | cHepO | H | Br | 1Me-5-Idz | C | | 496(M⁺) |
| A-b-363 | 4f-1 | Int.A-36 | Hal3 | nPrO | Et | Br | 1Me-5-Idz | C | | 470(M⁺) |
| A-b-364 | 1a | A-b-363 | | nPrO | H | Br | 1Me-5-Idz | C | | 442(M⁺) |
| A-b-365 | 4f-1 | Int.A-36 | Hal4 | iPrO | Et | Br | 1Me-5-Idz | C | | 470(M⁺) |
| A-b-366 | 1a | A-b-365 | | iPrO | H | Br | 1 Me-5-Idz | C | | 442(M⁺) |
| A-b-367 | 4f-1 | Int.A-36 | Hal5 | nBuO | Et | Br | 1 Me-5-Idz | C | | 484(M⁺) |
| A-b-368 | 1a | A-b-367 | | nBuO | H | Br | 1Me-5-Idz | C | | 456(M⁺) |
| A-b-369 | 4f-1 | Int.A-36 | Hal6 | iBuO | Et | Br | 1Me-5-Idz | C | | 484(M⁺) |
| A-b-370 | 1a | A-b-369 | | iBuO | H | Br | 1Me-5-Idz | C | | 456(M⁺) |
| A-b-371 | 4f-1 | Int.A-36 | Hal1 | BnO | Et | Br | 1Me-5-Idz | C | | 518(M⁺) |
| A-b-372 | 1a | A-b-371 | | BnO | H | Br | 1Me-5-Idz | C | | 490(M⁺) |
| A-b-373 | 4f-1 | Int.A-36 | Hal7 | 2FBnO | Et | Br | 1Me-5-Idz | C | | 536(M⁺) |
| A-b-374 | 1a | A-b-373 | | 2FBnO | H | Br | 1Me-5-Idz | C | | 508(M⁺) |
| A-b-375 | 4f-1 | Int.A-36 | Hal9 | 4FBnO | Et | Br | 1Me-5-Idz | C | | 536(M⁺) |
| A-b-376 | 1a | A-b-375 | | 4FBnO | H | Br | 1Me-5-Idz | C | | 508(M⁺) |
| A-b-377 | 4f-1 | Int.A-36 | Hal11 | 3ClBnO | Et | Br | 1Me-5-Idz | C | | 552(M⁺) |
| A-b-378 | 1a | A-b-377 | | 3ClBnO | H | Br | 1Me-5-Idz | C | | 524(M⁺) |
| A-b-379 | 4f-1 | Int.A-36 | Hal13 | 4CF3BnO | Et | Br | 1Me-5-Idz | C | | 586(M⁺) |
| A-b-380 | 1a | A-b-379 | | 4CF3BnO | H | Br | 1Me-5-Idz | C | | 558(M⁺) |
| A-b-381 | 4f-2 | Int.A-36 | Al5 | 2EtBuO | Et | Br | 1Me-5-Idz | C | | 512(M⁺) |
| A-b-382 | 1a | A-b-381 | | 2EtBuO | H | Br | 1Me-5-Idz | C | | 484(M⁺) |
| A-b-383 | 4f-2 | Int.A-36 | Al6 | 2(4DMAPh)EtO | Et | Br | 1Me-5-Idz | C | | 575(M⁺) |
| A-b-384 | 1a | A-b-383 | | 2(4DMAPh)EtO | H | Br | 1Me-5-Idz | C | | 547(M⁺) |
| A-b-385 | 4f-2 | Int.A-36 | Al8 | 1PhEtO | Et | Br | 1Me-5-Idz | C | | 532(M⁺) |
| A-b-386 | 1a | A-b-385 | | 1PhEtO | H | Br | 1Me-5-Idz | C | | 504(M⁺) |
| A-b-387 | 4f-2 | Int.A-36 | Al11 | 4Me,cHexO | Et | Br | 1Me-5-Idz | C | | 524(M⁺) |
| A-b-388 | 1a | A-b-387 | | 4Me,cHexO | H | Br | 1Me-5-Idz | C | | 496(M⁺) |
| A-b-389 | 4f-2 | Int.A-36 | Al17 | 1IndanO | Et | Br | 1Me-5-Idz | C | | 544(M⁺) |
| A-b-390 | 1a | A-b-389 | | 1IndanO | H | Br | 1Me-5-Idz | C | | 516(M⁺) |
| A-b-391 | 4f-2 | Int.A-36 | Al18 | 2IndanO | Et | Br | 1Me-5-Idz | C | | 544(M⁺) |
| A-b-392 | 1a | A-b-391 | | 2IndanO | H | Br | 1Me-5-Idz | C | | 516(M⁺) |
| A-b-393 | 4f-2 | Int.A-36 | Al19 | 5OMe-2-IndanO | Et | Br | 1Me-5-Idz | C | | 574(M⁺) |
| A-b-394 | 1a | A-b-393 | | 5OMe-2-IndanO | H | Br | 1Me-5-Idz | C | | 546(M⁺) |
| A-b-395 | 4f-2 | Int.A-36 | Al20 | 5,6D(OMe)-2-IndanO | Et | Br | 1Me-5-Idz | C | | 604(M⁺) |
| A-b-396 | 1a | A-b-395 | | 5,6D(OMe)-2-IndanO | H | Br | 1Me-5-Idz | C | | 576(M⁺) |

**[Table 27]**

| Table-A-b-10 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-b-397 | 4f-2 | Int.A-36 | Al21 | 5F-2-IndanO | Et | Br | 1Me-5-Idz | C | | 562(M⁺) |
| A-b-398 | 1a | A-b-397 | | 5F-2-IndanO | H | Br | 1Me-5-Idz | C | | 534(M⁺) |
| A-b-399 | 4f-2 | Int.A-36 | Al22 | 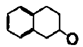 | Et | Br | 1Me-5-Idz | C | | 558(M⁺) |
| A-b-400 | 1a | A-b-399 | | 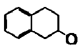 | H | Br | 1Me-5-Idz | C | | 530(M⁺) |
| A-b-401 | 4f-2 | Int.A-36 | Al23 | 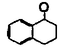 | Et | Br | 1Me-5-Idz | C | | 558(M⁺) |
| A-b-402 | 1a | A-b-401 | | 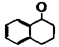 | H | Br | 1Me-5-Idz | C | | 530(M⁺) |
| A-b-403 | 4f-2 | Int.A-36 | Al25 | 2(3FPh)EtO | Et | Br | 1Me-5-Idz | C | | 550(M⁺) |
| A-b-404 | 1a | A-b-403 | | 2(3FPh)EtO | H | Br | 1Me-5-Idz | C | | 522(M⁺) |
| A-b-405 | 4f-2 | Int.A-36 | Al27 | 2(2-Nap)EtO | Et | Br | 1Me-5-Idz | C | | 582(M⁺) |
| A-b-406 | 1a | A-b-405 | | 2(2-Nap)EtO | H | Br | 1Me-5-Idz | C | | 554(M⁺) |
| A-b-407 | 4f-2 | Int.A-37 | Al1 | cPenO | Et | Br | 1Et-5-Idz | C | | 510(M⁺) |
| A-b-408 | 1a | A-b-407 | | cPenO | H | Br | 1Et-5-Idz | C | | 482(M⁺) |
| A-b-409 | 4f-2 | Int.A-37 | Al3 | cHexO | Et | Br | 1Et-5-Idz | C | | 524(M⁺) |
| A-b-410 | 1a | A-b-409 | | cHexO | H | Br | 1Et-5-Idz | C | | 496(M⁺) |
| A-b-411 | 4f-1 | Int.A-37 | Hal3 | nPrO | Et | Br | 1Et-5-Idz | C | | 484(M⁺) |
| A-b-412 | 1a | A-b-411 | | nPrO | H | Br | 1Et-5-Idz | C | | 456(M⁺) |
| A-b-413 | 4f-1 | Int.A-37 | Hal4 | iPrO | Et | Br | 1Et-5-Idz | C | | 484(M⁺) |
| A-b-414 | 1a | A-b-413 | | iPrO | H | Br | 1Et-5-Idz | C | | 456(M⁺) |
| A-b-415 | 4f-1 | Int.A-37 | Hal5 | nBuO | Et | Br | 1Et-5-Idz | C | | 498(M⁺) |
| A-b-416 | 1a | A-b-415 | | nBuO | H | Br | 1Et-5-Idz | C | | 470(M⁺) |
| A-b-417 | 4f-1 | Int.A-37 | Hal6 | iBuO | Et | Br | 1Et-5-Idz | C | | 498(M⁺) |
| A-b-418 | 1a | A-b-417 | | iBuO | H | Br | 1Et-5-Idz | C | | 470(M⁺) |
| A-b-419 | 4f-1 | Int.A-37 | Hal8 | 3FBnO | Et | Br | 1Et-5-Idz | C | | 550(M⁺) |
| A-b-420 | 1a | A-b-419 | | 3FBnO | H | Br | 1Et-5-Idz | C | | 522(M⁺) |
| A-b-421 | 4f-1 | Int.A-37 | Hal9 | 4FBnO | Et | Br | 1Et-5-Idz | C | | 550(M⁺) |
| A-b-422 | 1a | A-b-421 | | 4FBnO | H | Br | 1Et-5-Idz | C | | 522(M⁺) |
| A-b-423 | 4f-1 | Int.A-37 | Hal12 | 4MeBnO | Et | Br | 1Et-5-Idz | C | | 546(M⁺) |
| A-b-424 | 1a | A-b-423 | | 4MeBnO | H | Br | 1Et-5-Idz | C | | 518(M⁺) |
| A-b-425 | 4f-1 | Int.A-37 | Hal13 | 4CF3BnO | Et | Br | 1Et-5-Idz | C | | 600(M⁺) |
| A-b-426 | 1a | A-b-425 | | 4CF3BnO | H | Br | 1Et-5-Idz | C | | 572(M⁺) |
| A-b-427 | 4f-2 | Int.A-37 | Al5 | 2EtBuO | Et | Br | 1Et-5-Idz | C | | 526(M⁺) |
| A-b-428 | 1a | A-b-427 | | 2EtBuO | H | Br | 1Et-5-Idz | C | | 498(M⁺) |
| A-b-429 | 4f-2 | Int.A-37 | Al6 | 2(4DMAPh)EtO | Et | Br | 1Et-5-Idz | C | | 589(M⁺) |
| A-b-430 | 1a | A-b-429 | | 2(4DMAPh)EtO | H | Br | 1Et-5-Idz | C | | 561(M⁺) |
| A-b-431 | 4f-2 | Int.A-37 | Al7 | 2(PhO)EtO | Et | Br | 1Et-5-Idz | C | | 562(M⁺) |
| A-b-432 | 1a | A-b-431 | | 2(PhO)EtO | H | Br | 1Et-5-Idz | C | | 534(M⁺) |
| A-b-433 | 4f-2 | Int.A-37 | Al17 | 1IndanO | Et | Br | 1Et-5-Idz | C | | 558(M⁺) |
| A-b-434 | 1a | A-b-433 | | 1IndanO | H | Br | 1Et-5-Idz | C | | 530(M⁺) |
| A-b-435 | 4f-2 | Int.A-37 | Al18 | 2IndanO | Et | Br | 1Et-5-Idz | C | | 558(M⁺) |
| A-b-436 | 1a | A-b-435 | | 2IndanO | H | Br | 1Et-5-Idz | C | | 530(M⁺) |
| A-b-437 | 4f-2 | Int.A-37 | Al19 | 5OMe-2-IndanO | Et | Br | 1Et-5-Idz | C | | 588(M⁺) |
| A-b-438 | 1a | A-b-437 | | 5OMe-2-IndanO | H | Br | 1Et-5-Idz | C | | 560(M⁺) |
| A-b-439 | 4f-2 | Int.A-37 | Al21 | 5F-2-IndanO | Et | Br | 1Et-5-Idz | C | | 576(M⁺) |
| A-b-440 | 1a | A-b-439 | | 5F-2-IndanO | H | Br | 1Et-5-Idz | C | | 548(M⁺) |

**[Table 28]**

| Table-A-b-11 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-b-441 | 4f-2 | Int.A-37 | Al22 | 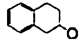 | Et | Br | 1Et-5-Idz | C | | 572(M⁺) |
| A-b-442 | 1-a | A-b-441 | | 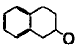 | H | Br | 1Et-5-Idz | C | | 544(M⁺) |
| A-b-443 | 4f-2 | Int.A-37 | Al23 | 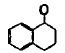 | Et | Br | 1Et-5-Idz | C | | 572(M⁺) |
| A-b-444 | 1-a | A-b-443 | | 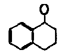 | H | Br | 1Et-5-Idz | C | | 544(M⁺) |
| A-b-445 | 4e-1 | A-b-243 | BRA77 | cPenO | Et | Me | 2-Nap | C | | 429(M⁺+1) |
| A-b-446 | 1-a | A-b-445 | | cPenO | H | Me | 2-Nap | C | | 401(M⁺+1) |
| A-b-447 | 4e-1 | A-b-245 | BRA77 | cPenMeO | Et | Me | 2-Nap | C | | 443(M⁺+1) |
| A-b-448 | 1-a | A-b-447 | | cPenMeO | H | Me | 2-Nap | C | | 415(M⁺+1) |
| A-b-449 | 4e-1 | A-b-247 | BRA77 | cHexMeO | Et | Me | 2-Nap | C | | 457(M⁺+1) |
| A-b-450 | 1-a | A-b-449 | | cHexMeO | H | Me | 2-Nap | C | | 429(M⁺+1) |
| A-b-451 | 4e-1 | A-b-249 | BRA77 | cHexO | Et | Me | 2-Nap | C | | 443(M⁺+1) |
| A-b-452 | 1-a | A-b-451 | | cHexO | H | Me | 2-Nap | C | | 415(M⁺+1) |
| A-b-453 | 4e-1 | A-b-253 | BRA77 | nPrO | Et | Me | 2-Nap | C | | 403(M⁺+1) |
| A-b-454 | 1-a | A-b-453 | | nPrO | H | Me | 2-Nap | C | | 375(M⁺+1) |
| A-b-455 | 4e-1 | A-b-255 | BRA77 | iPrO | Et | Me | 2-Nap | C | | 403(M⁺+1) |
| A-b-456 | 1-a | A-b-455 | | iPrO | H | Me | 2-Nap | C | | 375(M⁺+1) |
| A-b-457 | 4e-1 | A-b-257 | BRA77 | nBuO | Et | Me | 2-Nap | C | | 417(M⁺+1) |
| A-b-458 | 1-a | A-b-457 | | nBuO | H | Me | 2-Nap | C | | 389(M⁺+1) |
| A-b-459 | 4e-1 | A-b-259 | BRA77 | iBuO | Et | Me | 2-Nap | C | | 417(M⁺+1) |
| A-b-460 | 1-a | A-b-459 | | iBuO | H | Me | 2-Nap | C | | 389(M⁺+1) |
| A-b-461 | 4e-1 | A-b-263 | BRA77 | 2FBnO | Et | Me | 2-Nap | C | | 469(M⁺+1) |
| A-b-462 | 1-a | A-b-461 | | 2FBnO | H | Me | 2-Nap | C | | 4413(M⁺+ |
| A-b-463 | 4e-1 | A-b-265 | BRA77 | 3FBnO | Et | Me | 2-Nap | C | | 469(M⁺+1) |
| A-b-464 | 1-a | A-b-463 | | 3FBnO | H | Me | 2-Nap | C | | 441(M⁺+1) |
| A-b-465 | 4e-1 | A-b-269 | BRA77 | 2ClBnO | Et | Me | 2-Nap | C | | 484(M⁺+1) |
| A-b-466 | 1-a | A-b-465 | | 2ClBnO | H | Me | 2-Nap | C | | 457(M⁺+1) |
| A-b-467 | 4e-1 | A-b-271 | BRA77 | 3ClBnO | Et | Me | 2-Nap | C | | 485(M⁺+1) |
| A-b-468 | 1-a | A-b-467 | | 3ClBnO | H | Me | 2-Nap | C | | 457(M⁺+1) |
| A-b-469 | 4e-1 | A-b-273 | BRA77 | 4MeBnO | Et | Me | 2-Nap | C | | 465(M⁺+1) |
| A-b-470 | 1-a | A-b-469 | | 4MeBnO | H | Me | 2-Nap | C | | 437(M⁺+1) |
| A-b-471 | 4e-1 | A-b-277 | BRA77 | 2EtBuO | Et | Me | 2-Nap | C | | 445(M⁺+1) |
| A-b-472 | 1-a | A-b-471 | | 2EtBuO | H | Me | 2-Nap | C | | 417(M⁺+1) |
| A-b-473 | 4e-1 | A-b-281 | BRA77 | 2(PhO)EtO | Et | Me | 2-Nap | C | | 481(M⁺+1) |
| A-b-474 | 1-a | A-b-473 | | 2(PhO)EtO | H | Me | 2-Nap | C | | 453(M⁺+1) |
| A-b-475 | 4e-1 | A-b-285 | BRA77 | 4Me,cHexO | Et | Me | 2-Nap | C | | 457(M⁺+1) |
| A-b-476 | 1-a | A-b-475 | | 4Me,cHexO | H | Me | 2-Nap | C | | 429(M⁺+1) |
| A-b-477 | 4e-1 | A-b-287 | BRA77 | 1IndanO | Et | Me | 2-Nap | C | | 477(M⁺+1) |
| A-b-478 | 1-a | A-b-477 | | 1IndanO | H | Me | 2-Nap | C | | 449(M⁺+1) |
| A-b-479 | 4e-1 | A-b-289 | BRA77 | 2IndanO | Et | Me | 2-Nap | C | | 429(M⁺+1) |
| A-b-480 | 1-a | A-b-479 | | 2IndanO | H | Me | 2-Nap | C | | 449(M⁺+1) |
| A-b-481 | 4e-1 | A-b-291 | BRA77 | 5OMe-2-IndanO | Et | Me | 2-Nap | C | | 507(M⁺+1) |
| A-b-482 | 1-a | A-b-481 | | 5OMe-2-IndanO | H | Me | 2-Nap | C | | 479(M⁺+1) |
| A-b-483 | 4e-1 | A-b-295 | BRA77 | 5F-2-IndanO | Et | Me | 2-Nap | C | | 495(M⁺+1) |
| A-b-484 | 1-a | A-b-483 | | 5F-2-IndanO | H | Me | 2-Nap | C | | 467(M⁺+1) |

**[Table 29]**

| Table-A-b-12 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-b-485 | 4e-1 | A-b-297 | BRA77 | 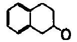 | Et | Me | 2-Nap | C | | 491(M⁺+1) |
| A-b-486 | 1-a | A-b-485 | | 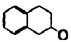 | H | Me | 2-Nap | C | | 463(M⁺+1) |
| A-b-487 | 4e-1 | A-b-299 | BRA77 | 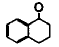 | Et | Me | 2-Nap | C | | 491(M⁺+1) |
| A-b-488 | 1-a | A-b-487 | | 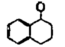 | H | Me | 2-Nap | C | | 463(M⁺+1) |
| A-b-489 | 4e-1 | A-b-305 | BRA77 | cPenO | Et | Me | 1Me-5-Ind | C | | 432(M⁺+1) |
| A-b-490 | 1-a | A-b-489 | | cPenO | H | Me | 1Me-5-Ind | C | | 404(M⁺+1) |
| A-b-491 | 4e-1 | A-b-307 | BRA77 | cHexMeO | Et | Me | 1Me-5-Ind | C | | 460(M⁺+1) |
| A-b-492 | 1-a | A-b-491 | | cHexMeO | H | Me | 1Me-5-Ind | C | | 432(M⁺+1) |
| A-b-493 | 4e-1 | A-b-309 | BRA77 | cHexO | Et | Me | 1Me-5-Ind | C | | 446(M⁺+1) |
| A-b-494 | 1-a | A-b-493 | | cHexO | H | Me | 1Me-5-Ind | C | | 418(M⁺+1) |
| A-b-495 | 4e-1 | A-b-311 | BRA77 | nPrO | Et | Me | 1Me-5-Ind | C | | 406(M⁺+1) |
| A-b-496 | 1-a | A-b-495 | | nPrO | H | Me | 1Me-5-Ind | C | | 378(M⁺+1) |
| A-b-497 | 4e-1 | A-b-313 | BRA77 | iPrO | Et | Me | 1Me-5-Ind | C | | 406(M⁺+1) |
| A-b-498 | 1-a | A-b-497 | | iPrO | H | Me | 1Me-5-Ind | C | | 378(M⁺+1) |
| A-b-499 | 4e-1 | A-b-315 | BRA77 | nBuO | Et | Me | 1Me-5-Ind | C | | 420(M⁺+1) |
| A-b-500 | 1-a | A-b-499 | | nBuO | H | Me | 1Me-5-Ind | C | | 392(M⁺+1) |
| A-b-501 | 4e-1 | A-b-317 | BRA77 | iBuO | Et | Me | 1Me-5-Ind | C | | 420(M⁺+1) |
| A-b-502 | 1-a | A-b-501 | | iBuO | H | Me | 1Me-5-Ind | C | | 392(M⁺+1) |
| A-b-503 | 4e-1 | A-b-319 | BRA77 | BnO | Et | Me | 1Me-5-Ind | C | | 454(M⁺+1) |
| A-b-504 | 1-a | A-b-503 | | BnO | H | Me | 1Me-5-Ind | C | | 426(M⁺+1) |
| A-b-505 | 4e-1 | A-b-321 | BRA77 | 2FBnO | Et | Me | 1Me-5-Ind | C | | 472(M⁺+1) |
| A-b-506 | 1-a | A-b-505 | | 2FBnO | H | Me | 1Me-5-Ind | C | | 444(M⁺+1) |
| A-b-507 | 4e-1 | A-b-323 | BRA77 | 3FBnO | Et | Me | 1Me-5-Ind | C | | 472(M⁺+1) |
| A-b-508 | 1-a | A-b-507 | | 3FBnO | H | Me | 1Me-5-Ind | C | | 444(M⁺+1) |
| A-b-509 | 4e-1 | A-b-327 | BRA77 | 3ClBnO | Et | Me | 1Me-5-Ind | C | | 488(M⁺+1) |
| A-b-510 | 1-a | A-b-509 | | 3ClBnO | H | Me | 1Me-5-Ind | C | | 460(M⁺+1) |
| A-b-511 | 4e-1 | A-b-329 | BRA77 | 4MeBnO | Et | Me | 1Me-5-Ind | C | | 468(M⁺+1) |
| A-b-512 | 1-a | A-b-511 | | 4MeBnO | H | Me | 1Me-5-Ind | C | | 440(M⁺+1) |
| A-b-513 | 4e-1 | A-b-331 | BRA77 | 2EtBuO | Et | Me | 1Me-5-Ind | C | | 448(M⁺+1) |
| A-b-514 | 1-a | A-b-513 | | 2EtBuO | H | Me | 1Me-5-Ind | C | | 420(M⁺+1) |
| A-b-515 | 4e-1 | A-b-333 | BRA77 | 2(4DMAPh)EtO | Et | Me | 1Me-5-Ind | C | | 511(M⁺+1) |
| A-b-516 | 1-a | A-b-515 | | 2(4DMAPh)EtO | H | Me | 1Me-5-Ind | C | | 483(M⁺+1) |
| A-b-517 | 4e-1 | A-b-337 | BRA77 | 4Me,cHexO | Et | Me | 1Me-5-Ind | C | | 460(M⁺+1) |
| A-b-518 | 1-a | A-b-517 | | 4Me,cHexO | H | Me | 1Me-5-Ind | C | | 432(M⁺+1) |
| A-b-519 | 4e-1 | A-b-339 | BRA77 | 1IndanO | Et | Me | 1Me-5-Ind | C | | 480(M⁺+1) |
| A-b-520 | 1-a | A-b-519 | | 1IndanO | H | Me | 1Me-5-Ind | C | | 452(M⁺+1) |
| A-b-521 | 4e-1 | A-b-341 | BRA77 | 2IndanO | Et | Me | 1Me-5-Ind | C | | 480(M⁺+1) |
| A-b-522 | 1-a | A-b-521 | | 2IndanO | H | Me | 1Me-5-Ind | C | | 452(M⁺+1) |
| A-b-523 | 4e-1 | A-b-343 | BRA77 | 5OMe-2-IndanO | Et | Me | 1Me-5-Ind | C | | 510(M⁺+1) |
| A-b-524 | 1-a | A-b-523 | | 5OMe-2-IndanO | H | Me | 1Me-5-Ind | C | | 482(M⁺+1) |
| A-b-525 | 4e-1 | A-b-345 | BRA77 | 5,6D(OMe)-2-IndanO | Et | Me | 1Me-5-Ind | C | | 540(M⁺+1) |
| A-b-526 | 1-a | A-b-525 | | 5,6D(OMe)-2-IndanO | H | Me | 1Me-5-Ind | C | | 512(M⁺+1) |
| A-b-527 | 4e-1 | A-b-347 | BRA77 | 5F-2-IndanO | Et | Me | 1Me-5-Ind | C | | 498(M⁺+1) |
| A-b-528 | 1-a | A-b-527 | | 5F-2-IndanO | H | Me | 1Me-5-Ind | C | | 470(M⁺+1) |

**[Table 30]**

| Table-A-b-13 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-b-529 | 4e-1 | A-b-349 | BRA77 | 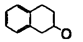 | Et | Me | 1Me-5-Ind | C | | 494(M⁺+1) |
| A-b-530 | 1-a | A-b-529 | | 2-tetra | H | Me | 1Me-5-Ind | C | | 466(M⁺+1) |
| A-b-531 | 4e-1 | A-b-351 | BRA77 | 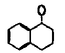 | Et | Me | 1Me-5-Ind | C | | 494(M⁺+1) |
| A-b-532 | 1-a | A-b-531 | | 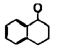 | H | Me | 1Me-5-Ind | C | | 466(M⁺+1) |
| A-b-533 | 4e-1 | A-b-353 | BRA77 | 2(2-Nap)EtO | Et | Me | 1Me-5-Ind | C | | 518(M⁺+1) |
| A-b-534 | 1-a | A-b-533 | | 2(2-Nap)EtO | H | Me | 1Me-5-Ind | C | | 490(M⁺+1) |
| A-b-535 | 4e-1 | A-b-355 | BRA77 | cPenO | Et | Me | 1Me-5-Idz | C | | 433(M⁺+1) |
| A-b-536 | 1-a | A-b-535 | | cPenO | H | Me | 1Me-5-Idz | C | | 405(M⁺+1) |
| A-b-537 | 4e-1 | A-b-357 | BRA77 | cPenMeO | Et | Me | 1Me-5-Idz | C | | 447(M⁺+1) |
| A-b-538 | 1-a | A-b-537 | | cPenMeO | H | Me | 1Me-5-Idz | C | | 419(M⁺+1) |
| A-b-539 | 4e-1 | A-b-359 | BRA77 | cHexO | Et | Me | 1Me-5-Idz | C | | 447(M⁺+1) |
| A-b-540 | 1-a | A-b-539 | | cHexO | H | Me | 1Me-5-Idz | C | | 419(M⁺+1) |
| A-b-541 | 4e-1 | A-b-363 | BRA77 | nPrO | Et | Me | 1Me-5-Idz | C | | 407(M⁺+1) |
| A-b-542 | 1-a | A-b-541 | | nPrO | H | Me | 1Me-5-Idz | C | | 379(M⁺+1) |
| A-b-543 | 4e-1 | A-b-365 | BRA77 | iPrO | Et | Me | 1Me-5-Idz | C | | 407(M⁺+1) |
| A-b-544 | 1-a | A-b-543 | | iPrO | H | Me | 1Me-5-Idz | C | | 379(M⁺+1) |
| A-b-545 | 4e-1 | A-b-367 | BRA77 | nBuO | Et | Me | 1Me-5-Idz | C | | 421(M⁺+1) |
| A-b-546 | 1-a | A-b-545 | | nBuO | H | Me | 1Me-5-Idz | C | | 393(M⁺+1) |
| A-b-547 | 4e-1 | A-b-369 | BRA77 | iBuO | Et | Me | 1Me-5-Idz | C | | 421(M⁺+1) |
| A-b-548 | 1-a | A-b-547 | | iBuO | H | Me | 1Me-5-Idz | C | | 393(M⁺+1) |
| A-b-549 | 4e-1 | A-b-371 | BRA77 | BnO | Et | Me | 1Me-5-Idz | C | | 455(M⁺+1) |
| A-b-550 | 1-a | A-b-549 | | BnO | H | Me | 1Me-5-Idz | C | | 427(M⁺+1) |
| A-b-551 | 4e-1 | A-b-373 | BRA77 | 2FBnO | Et | Me | 1Me-5-Idz | C | | 473(M⁺+1) |
| A-b-552 | 1-a | A-b-551 | | 2FBnO | H | Me | 1Me-5-Idz | C | | 445(M⁺+1) |
| A-b-553 | 4e-1 | A-b-375 | BRA77 | 4FBnO | Et | Me | 1Me-5-Idz | C | | 473(M⁺+1) |
| A-b-554 | 1-a | A-b-553 | | 4FBnO | H | Me | 1Me-5-Idz | C | | 445(M⁺+1) |
| A-b-555 | 4e-1 | A-b-377 | BRA77 | 3ClBnO | Et | Me | 1Me-5-Idz | C | | 489(M⁺+1) |
| A-b-556 | 1-a | A-b-555 | | 3ClBnO | H | Me | 1Me-5-Idz | C | | 461(M⁺+1) |
| A-b-557 | 4e-1 | A-b-379 | BRA77 | 4CF3BnO | Et | Me | 1Me-5-Idz | C | | 522(M⁺+1) |
| A-b-558 | 1-a | A-b-557 | | 4CF3BnO | H | Me | 1Me-5-Idz | C | | 494(M⁺+1) |
| A-b-559 | 4e-1 | A-b-381 | BRA77 | 2EtBuO | Et | Me | 1Me-5-Idz | C | | 449(M⁺+1) |
| A-b-560 | 1-a | A-b-559 | | 2EtBuO | H | Me | 1Me-5-Idz | C | | 421(M⁺+1) |
| A-b-561 | 4e-1 | A-b-383 | BRA77 | 2(4DMAPh)EtO | Et | Me | 1Me-5-Idz | C | | 512(M⁺+1) |
| A-b-562 | 1-a | A-b-561 | | 2(4DMAPh)EtO | H | Me | 1Me-5-Idz | C | | 484(M⁺+1) |
| A-b-563 | 4e-1 | A-b-387 | BRA77 | 4Me,cHexO | Et | Me | 1Me-5-Idz | C | | 461(M⁺+1) |
| A-b-564 | 1-a | A-b-563 | | 4Me,cHexO | H | Me | 1Me-5-Idz | C | | 433(M⁺+1) |
| A-b-565 | 4e-1 | A-b-389 | BRA77 | 1IndanO | Et | Me | 1Me-5-Idz | C | | 481(M⁺+1) |
| A-b-566 | 1-a | A-b-565 | | 1IndanO | H | Me | 1Me-5-Idz | C | | 453(M⁺+1) |
| A-b-567 | 4e-1 | A-b-391 | BRA77 | 2IndanO | Et | Me | 1Me-5-Idz | C | | 481(M⁺+1) |
| A-b-568 | 1-a | A-b-567 | | 2IndanO | H | Me | 1Me-5-Idz | C | | 453(M⁺+1) |
| A-b-569 | 4e-1 | A-b-393 | BRA77 | 5OMe-2-IndanO | Et | Me | 1Me-5-Idz | C | | 511(M⁺+1) |
| A-b-570 | 1-a | A-b-569 | | 50Me-2-IndanO | H | Me | 1Me-5-Idz | C | | 483(M⁺+1) |
| A-b-571 | 4e-1 | A-b-395 | BRA77 | 5,6D(OMe)-2-IndanO | Et | Me | 1Me-5-Idz | C | | 541(M⁺+1) |
| A-b-572 | 1-a | A-b-571 | | 5,6D(OMe)-2-IndanO | H | Me | 1Me-5-Idz | C | | 513(M⁺+1) |

**[Table 31]**

| Table-A-b-14 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-b-573 | 4e-1 | A-b-397 | BRA77 | 5F-2-IndanO | Et | Me | 1Me-5-Idz | C | | 499(M⁺+1) |
| A-b-574 | 1-a | A-b-573 | | 5F-2-IndanO | H | Me | 1Me-5-Idz | C | | 471(M⁺+1) |
| A-b-575 | 4e-1 | A-b-399 | BRA77 | 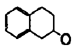 | Et | Me | 1Me-5-Idz | C | | 495(M⁺+1) |
| A-b-576 | 1-a | A-b-575 | | 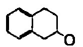 | H | Me | 1Me-5-Idz | C | | 467(M⁺+1) |
| A-b-577 | 4e-1 | A-b-401 | BRA77 | 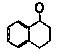 | Et | Me | 1Me-5-Idz | C | | 495(M⁺+1) |
| A-b-578 | 1-a | A-b-577 | | 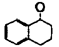 | H | Me | 1Me-5-Idz | C | | 467(M⁺+1) |
| A-b-579 | 4e-1 | A-b-403 | BRA77 | 2(3FPh)EtO | Et | Me | 1Me-5-Idz | C | | 487(M⁺+1) |
| A-b-580 | 1-a | A-b-579 | | 2(3FPh)EtO | H | Me | 1 Me-5-Idz | C | | 459(M⁺+1) |
| A-b-581 | 4e-1 | A-b-407 | BRA77 | cPenO | Et | Me | 1Et-5-Idz | C | | 447(M⁺+1) |
| A-b-582 | 1-a | A-b-581 | | cPenO | H | Me | 1Et-5-Idz | C | | 419(M⁺+1) |
| A-b-583 | 4e-1 | A-b-409 | BRA77 | cHexO | Et | Me | 1Et-5-Idz | C | | 461(M⁺+1) |
| A-b-584 | 1-a | A-b-583 | | cHexO | H | Me | 1Et-5-Idz | C | | 433(M⁺+1) |
| A-b-585 | 4e-1 | A-b-411 | BRA77 | nPrO | Et | Me | 1Et-5-Idz | C | | 421(M⁺+1) |
| A-b-586 | 1-a | A-b-585 | | nPrO | H | Me | 1Et-5-Idz | C | | 393(M⁺+1) |
| A-b-587 | 4e-1 | A-b-413 | BRA77 | iPrO | Et | Me | 1Et-5-Idz | C | | 421(M⁺+1) |
| A-b-588 | 1-a | A-b-587 | | iPrO | H | Me | 1Et-5-Idz | C | | 393(M⁺+1) |
| A-b-589 | 4e-1 | A-b-415 | BRA77 | nBuO | Et | Me | 1Et-5-Idz | C | | 435(M⁺+1) |
| A-b-590 | 1-a | A-b-589 | | nBuO | H | Me | 1Et-5-Idz | C | | 407(M⁺+1) |
| A-b-591 | 4e-1 | A-b-417 | BRA77 | iBuO | Et | Me | 1Et-5-Idz | C | | 435(M⁺+1) |
| A-b-592 | 1-a | A-b-591 | | iBuO | H | Me | 1Et-5-Idz | C | | 407(M⁺+1) |
| A-b-593 | 4e-1 | A-b-419 | BRA77 | 3FBnO | Et | Me | 1Et-5-Idz | C | | 487(M⁺+1) |
| A-b-594 | 1-a | A-b-593 | | 3FBnO | H | Me | 1Et-5-Idz | C | | 459(M⁺+1) |
| A-b-595 | 4e-1 | A-b-423 | BRA77 | 4MeBnO | Et | Me | 1Et-5-Idz | C | | 483(M⁺+1) |
| A-b-596 | 1-a | A-b-595 | | 4MeBnO | H | Me | 1Et-5-Idz | C | | 455(M⁺+1) |
| A-b-597 | 4e-1 | A-b-425 | BRA77 | 4CF3BnO | Et | Me | 1Et-5-Idz | C | | 536(M⁺+1) |
| A-b-598 | 1-a | A-b-597 | | 4CF3BnO | H | Me | 1Et-5-Idz | C | | 508(M⁺+1) |
| A-b-599 | 4e-1 | A-b-427 | BRA77 | 2EtBuO | Et | Me | 1Et-5-Idz | C | | 463(M⁺+1) |
| A-b-600 | 1-a | A-b-599 | | 2EtBuO | H | Me | 1Et-5-Idz | C | | 435(M⁺+1) |
| A-b-601 | 4e-1 | A-b-429 | BRA77 | 2(4DMAPh)EtO | Et | Me | 1Et-5-Idz | C | | 526(M⁺+1) |
| A-b-602 | 1-a | A-b-601 | | 2(4DMAPh)EtO | H | Me | 1Et-5-Idz | C | | 498(M⁺+1) |
| A-b-603 | 4e-1 | A-b-433 | BRA77 | 1IndanO | Et | Me | 1Et-5-Idz | C | | 495(M⁺+1) |
| A-b-604 | 1-a | A-b-603 | | 1IndanO | H | Me | 1Et-5-Idz | C | | 467(M⁺+1) |
| A-b-605 | 4e-1 | A-b-435 | BRA77 | 2IndanO | Et | Me | 1Et-5-Idz | C | | 495(M⁺+1) |
| A-b-606 | 1-a | A-b-605 | | 2IndanO | H | Me | 1Et-5-Idz | C | | 467(M⁺+1) |
| A-b-607 | 4e-1 | A-b-437 | BRA77 | 5OMe-2-IndanO | Et | Me | 1Et-5-Idz | C | | 525(M⁺+1) |
| A-b-608 | 1-a | A-b-607 | | 5OMe-2-IndanO | H | Me | 1Et-5-Idz | C | | 497(M⁺+1) |
| A-b-609 | 4e-1 | A-b-439 | BRA77 | 5F-2-IndanO | Et | Me | 1Et-5-Idz | C | | 513(M⁺+1) |
| A-b-610 | 1-a | A-b-609 | | 5F-2-IndanO | H | Me | 1Et-5-Idz | C | | 485(M⁺+1) |
| A-b-611 | 4e-1 | A-b-441 | BRA77 | 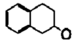 | Et | Me | 1Et-5-Idz | C | | 509(M⁺+1) |
| A-b-612 | 1-a | A-b-611 | | 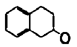 | H | Me | 1Et-5-Idz | C | | 481(M⁺+1) |

### Reference Example 6

### Synthesis of (R)-5-benzyloxy-2,3-dihydro-1H-inden-1-ol (Intermediate A-101) (Preparation Method 15, Step o)

According to the procedure described in a literature [Hashiguchi et al., Journal of American Chemical Society, p.7562, 1995], a solution of Intermediate A-5 (47.7 mg) in formic acid (714 µl, WAKO) and triethylamine (226 µl, WAKO) was added with ruthenium chloride/[(R,R)-N- (p-methanesulfonyl)-1,2-diphenylethylenediamine](p-cymene) (2.2 mg) under a nitrogen atmosphere, and stirred for 5 minutes, and then stirred at 40°C for 6 days. The reaction mixture was added with ethyl acetate (20 ml), and extracted with saturated aqueous sodium hydrogencarbonate (10 ml × 2), the organic layer was dried, and then the solvent was evaporated under reduced pressure. The residue was purified by flash column chromatography (hexane:ethyl acetate = 6:1) to obtain the title compound (Intermediate A-101, 14.1 mg). Mass (LCMS): 241 (M⁺+1), Retention time: 4.44 minutes (Elution condition: D).

### Synthesis of ethyl (R)-2-(5-benzyloxy-2,3-dihydro-1H-inden-1-yl)ethane-1,1,1-triacetate (Intermediate A-102) (Preparation Method 15, Step f-2)

According to the procedure described in a literature [Michael et al., Organic Letters, p.573, 2004], a solution of Intermediate A-101 (14.1 mg) in anhydrous toluene (1 ml) was cooled to -50°C under a nitrogen atmosphere, added with trimethylphosphine (12.4 µl, KANTO), and added dropwise with triethyl methanetricarboxylate (27.9 mg, TCI). The mixture was stirred for 5 minutes, and then added dropwise with a solution of diethyl azodicarboxylate (henceforth abbreviated as "DEAD") in 43% toluene (54.5 µl, WAKO), stirred for 10 minutes, and then warmed to room temperature.
The reaction mixture was stirred for 5 hours, and then concentrated, and the residue was purified by flash column chromatography (hexane:ethyl acetate = 15:1) to obtain a crude product of the title compound (Intermediate A-102, 65.2 mg). Mass (LCMS): 455 (M⁺+1), Retention time: 5.86 minutes (Elution condition: D).

### Synthesis of (R)-2-(5-benzyloxy-2,3-dihydro-1H-inden-1-yl)ethane-1,1,1-triacetic acid (Intermediate A-103) (Preparation Method 15, Step f-2)

According to the procedure described in a literature [Michael et al., Organic Letters, p.573, 2004], a solution of Intermediate A-102 (65.2 mg) in methanol (2 ml) was added with 2 N aqueous sodium hydroxide (236 µl), the mixture was stirred at 70°C for 19 hours, and then added with 2 N aqueous sodium hydroxide (236 µl), and the mixture was stirred at 110°C for 24 hours. The reaction mixture was then concentrated under reduced pressure, and then extracted with water (10 ml) and ethyl acetate (5 ml × 2), and the aqueous layer was neutralized with 2 N aqueous hydrochloric acid (500 µl) under ice cooling, and then extracted with ethyl acetate (5 ml × 3). The organic layer was washed with saturated brine and dried, and then the solvent was evaporated under reduced pressure to obtain the title compound (Compound No.: Intermediate A-103, 23.8 mg).

### Synthesis of (R)-2-(5-benzyloxy-2,3-dihydro-1H-inden-1-yl)acetic acid (Intermediate A-104) (Preparation Method 15, Step f-2)

According to the procedure described in a literature [Michael et al., Organic Letters, p.573, 2004], Intermediate A-103 (23.8 mg) was added with acetic acid (2.5 ml), and the mixture was stirred at 130°C for 26 hours. The reaction mixture was concentrated under reduced pressure, and then extracted with water (10 ml) and ethyl acetate (10 ml), the organic layer was washed with saturated aqueous sodium hydrogencarbonate and saturated brine, and dried, and then the solvent was evaporated under reduced pressure to obtain the title compound (Compound No. Intermediate A-104, 14.9 mg). Mass (LCMS): 283 (M⁺+1), Retention time: 4.75 minutes (Elution condition: D).

### Synthesis of ethyl (R)-2-(5-benzyloxy-2,3-dihydro-1H-inden-1-yl)acetate (Intermediate A-105) (Preparation Method 2, Step b)

A solution prepared beforehand by adding thionyl chloride (200 µl, WAKO) dropwise to ethanol (30 ml) and mixing the mixture was added dropwise with a solution of Intermediate A-104 (14.9 mg) in methanol (10 ml) under ice cooling, and the mixture was stirred for 30 minutes, and then warmed to room temperature, and further stirred for 1.5 hours. The reaction mixture was concentrated under reduced pressure, and then extracted with water (20 ml) and methylene chloride (20 ml), and the organic layer was successively washed with saturated aqueous sodium hydrogencarbonate, saturated aqueous ammonium chloride and saturated brine. The organic layer was dried, and then the solvent was evaporated under reduced pressure to obtain the title compound (Intermediate A-105, 15.1 mg). Mass (LCMS): 311 (M⁺+1), Retention time: 5.82 minutes (Elution condition: D).

### Synthesis of ethyl (R)-2-(5-benzyloxy-6-bromo-2,3-dihydro-1H-inden-1-yl)acetate (Intermediate A-106) (Preparation Method 8, Step h)

According to the procedure described in the synthetic method of Intermediate A-4 in Reference Example 1 (Preparation Method 8, Step h), Intermediate A-105 (15.1 mg) and NBS (8.67 mg, WAKO) were reacted and treated to obtain the title compound (Intermediate A-106, 17.69 mg). Mass (LCMS): 391 (M⁺+1), Retention time: 6.06 minutes (Elution condition: D).

### Example A-c-1

Example Compound A-a-2 was subjected to chiral separation to obtain the title compound (Compound No. A-c-1, 25 mg).

### Example A-c-15

### Synthesis of ethyl (R)-2-[5-benzyloxy-6-(naphthalen-2-yl)-2,3-dihydroinden-1-ylidene]acetate (Compound No. A-c-15) (Preparation Method 4, Step e-1)

According to the procedure described in the synthetic method of Example Compound A-a-1 (Preparation Method 4, Step e-1) provided that the reaction was performed for 12 hours, Intermediate A-106 (210.3 mg), 2-naphthaleneboronic acid (324.2 mg, Ald), 2 M aqueous sodium carbonate (1.0 ml) and (Ph₃P)₄Pd (121.3 mg, Nakalai Tesque) were reacted and treated to obtain the title compound (Compound No. A-c-15, 200.3 mg).

### Example A-c-16

### Synthesis of (R)-2-[5-benzyloxy-6-(naphthalen-2-yl)-2,3-dihydroinden-1-ylidene]acetic acid (Compound No. A-c-16) (Preparation Method 1, Step a)

According to the procedure described in the synthetic method of Example Compound A-a-2 (Preparation Method 1, Step a) provided that the reaction was performed for 2 hours, Example Compound A-c-15 (102.2 mg) and 2 N aqueous sodium hydroxide (0.45 ml) were reacted and treated to obtain the title compound (Compound No. A-c-16, 91.6 mg).

Typical examples of the intermediates that can be obtained by reacting and treating corresponding starting compounds using any of the methods described in the present specification including the examples described above are shown in Table-Int.A-4, and typical examples of the compounds of the present invention that can be obtained by reacting and treating corresponding starting compounds using any of the methods described in the present specification are shown in Table-A-c-1 to Table-A-c-5. The compounds were synthesized according to the preparation methods of the compounds of the compound numbers shown in the columns of "Syn" in the tables. "Int" means an intermediate compound number. In the tables, used methods among the aforementioned synthetic methods are shown in the columns of "Syn" with symbols, the starting compounds are shown in the columns of "SM", and the reagents are shown in the columns of "Regent". The halide regents mentioned in the columns of "Reagent" with symbols "Hal (No.)" are those mentioned in Table-Hal, the alcohol regents shown with symbols "Al (No.)" are those mentioned in Table-Al, the boronic acid regents shown with symbols "BRA (No.)" are those mentioned in Table-BRA, and the bromoheterocyclic ring regents mentioned with the symbols "Het (No.)" are those mentioned in Table-Het. Those regents for which cells of the columns of "Manufacturer" are blank were synthesized according to methods described in ordinary chemical literatures.
In Table-Int.A-4, for the compounds for which cells of "Syn" are blank, a deprotection reaction was performed with Pd/C and hydrogen. In Table-A-c-1 to Table-A-c-5, for the compounds for which cells of "Syn" are blank, the objective compounds were obtained by HPLC fractionation.

**[Table 32]**

| Table-IntA-4 | | | | | | | | | | |
|---|---|---|---|---|---|---|---|---|---|---|
| 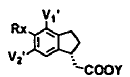 | | | | | | | | | | |

| Exp. | Syn. | SM | Reagent | Rx | Y | V1' | V2' | LCMS | | |
|---|---|---|---|---|---|---|---|---|---|---|
| | | | | | | | | method | RTime | Mass |
| Int.A-107 | | A-c-15 | | HO | Et | H | 2-Nap | C | | 347 (M⁺+1) |
| In.tA-108 | | A-c-17 | | HO | Et | H | 5-Ind | C | | 336 (M⁺+1) |
| Int.A-109 | | A-c-19 | | HO | Et | H | 1Me-5-Ind | C | | 350(M⁺+1) |
| Int.A-110 | | A-c-21 | | HO | Et | H | 5-1Idz | C | | 337(M⁺+1) |
| Int.A-111 | | A-c-23 | | HO | Et | H | 1Me-5-Idz | C | | 351(M⁺+1) |
| Int.A-112 | | A-c-25 | | HO | Et | H | 1Et-5-Idz | C | | 365(M⁺+1) |
| Int.A-113 | | A-c-29 | | HO | Et | H | 6-Qu | C | | 348(M⁺+1) |

**[Table 33]**

| Table-A-c-1 | | | | | | | | | | |
|---|---|---|---|---|---|---|---|---|---|---|
| 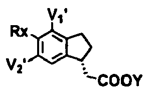 | | | | | | | | | | |

| Exp. | Syn. | SM | Reagent | Rx | Y | V1' | V2' | LCMS | | |
|---|---|---|---|---|---|---|---|---|---|---|
| | | | | | | | | method | RTime | Mass |
| A-c-1 | | A-a-2 | | cPenO | H | H | 2-Nap | C | | 387(M⁺+1) |
| A-c-2 | | A-a-4 | | cPenO | H | H | 5-Ind | C | | 376(M⁺+1) |
| A-c-3 | | A-a-6 | | cPenO | H | H | 1Me-5-Ind | C | | 390(M⁺+1) |
| A-c-4 | | A-a-8 | | cPenO | H | H | 1Et-5-Ind | C | | 404(M⁺+1) |
| A-c-5 | | A-a-10 | | cPenO | H | H | 5-1Idz | C | | 377(M⁺+1) |
| A-c-6 | | A-a-12 | | cPenO | H | H | 1Me-5-Idz | C | | 391(M⁺+1) |
| A-c-7 | | A-a-14 | | cPenO | H | H | 1Et-5-Idz | C | | 405(M⁺+1) |
| A-c-8 | | A-a-16 | | cPenO | H | H | 2Me-5-Idz | C | | 391(M⁺+1) |
| A-c-9 | | A-a-18 | | cPenO | H | H | 5-BF | C | | 377(M⁺+1) |
| A-c-10 | | A-a-20 | | cPenO | H | H | 5-BT | C | | 393(M⁺+1) |
| A-c-11 | | A-a-22 | | cPenO | H | H | 5-Bzt | C | | 394(M⁺+1) |
| A-c-12 | | A-a-24 | | cPenO | H | H | 3-Qu | C | | 388(M⁺+1) |
| A-c-13 | | A-a-26 | | cPenO | H | H | 6-Qu | C | | 388(M⁺+1) |
| A-c-14 | | A-a-28 | | cPenO | H | H | 6-IQ | C | | 388(M⁺+1) |
| A-c-15 | 4e-1 | Int.A-106 | BRA1 | BnO | Et | H | 2-Nap | C | | 437(M⁺+1) |
| A-c-16 | 1-a | A-c-15 | | BnO | H | H | 2-Nap | C | | 409(M⁺+1) |
| A-c-17 | 4e-1 | Int.A-106 | BRA2 | BnO | Et | H | 5-Ind | C | | 426(M⁺+1) |
| A-c-18 | 1-a | A-c-17 | | BnO | H | H | 5-Ind | C | | 398(M⁺+1) |
| A-c-19 | 4e-1 | In.tA-106 | BRA3 | BnO | Et | H | 1Me-5-Ind | C | | 440(M⁺+1) |
| A-c-20 | 1-a | A-c-19 | | BnO | H | H | 1Me-5-Ind | C | | 412(M⁺+1) |
| A-c-21 | 4e-1 | Int.A-106 | BRA7 | BnO | Et | H | 5-1Idz | C | | 427(M⁺+1) |
| A-c-22 | 1-a | A-c-21 | | BnO | H | H | 5-1Idz | C | | 399(M⁺+1) |
| A-c-23 | 4e-1 | Int.A-106 | BRA8 | BnO | Et | H | 1Me-5-Idz | C | | 441(M⁺+1) |
| A-c-24 | 1-a | A-c-23 | | BnO | H | H | 1Me-5-Idz | C | | 413(M⁺+1) |
| A-c-25 | 4e-1 | Int.A-106 | BRA9 | BnO | Et | H | 1Et-5-Idz | C | | 455(M⁺+1) |
| A-c-26 | 1-a | A-c-25 | | BnO | H | H | 1Et-5-Idz | C | | 427(M⁺+1) |
| A-c-27 | 4e-1 | Int.A-106 | BRA11 | BnO | Et | H | 5-BF | C | | 427(M⁺+1) |
| A-c-28 | 1-a | A-c-27 | | BnO | H | H | 5-BF | C | | 399(M⁺+1) |
| A-c-29 | 4e-2 | Int.A-106 | Het2 | BnO | Et | H | 3-Qu | C | | 438(M⁺+1) |
| A-c-30 | 1-a | A-c-29 | | BnO | H | H | 3-Qu | C | | 410(M⁺+1) |
| A-c-31 | 4f-2 | Int.A-108 | Al2 | cPenMeO | Et | H | 5-Ind | C | | 418(M⁺+1) |
| A-c-32 | 1-a | A-c-31 | | cPenMeO | H | H | 5-Ind | C | | 390(M⁺+1) |
| A-c-33 | | A-a-48 | | cPenMeO | H | H | 1Et-5-Ind | C | | 418(M⁺+1) |
| A-c-34 | 4f-2 | Int.A-111 | Al2 | cPenMeO | Et | H | 1Me-5-Idz | C | | 433(M⁺+1) |
| A-c-35 | 1-a | A-c-34 | | cPenMeO | H | H | 1 Me-5-Idz | C | | 405(M⁺+1) |
| A-c-36 | | A-a-52 | | cPenMeO | H | H | 3-Qu | C | | 402(M⁺+1) |
| A-c-37 | 4f-1 | Int.A-107 | Hal2 | cHexMeO | Et | H | 2-Nap | C | | 443(M⁺+1) |
| A-c-38 | 1-a | A-c-37 | | cHexMeO | H | H | 2-Nap | C | | 415(M⁺+1) |
| A-c-39 | | A-a-56 | | cHexMeO | H | H | 1Me-5-Ind | C | | 418(M⁺+1) |
| A-c-40 | | A-a-58 | | cHexMeO | H | H | 5-1Idz | C | | 405(M⁺+1) |
| A-c-41 | | A-a-60 | | cHexMeO | H | H | 6-IQ | C | | 416(M⁺+1) |
| A-c-42 | 4f-2 | Int.A-109 | Al3 | cHexO | Et | H | 1Me-5-Ind | C | | 432(M⁺+1) |
| A-c-43 | 1-a | A-c-42 | | cHexO | H | H | 1Me-5-Ind | C | | 404(M⁺+1) |
| A-c-44 | | A-a-64 | | cHexO | H | H | 1Me-5-Idz | C | | 405(M⁺+1) |

**[Table 34]**

| Table-A-c-2 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-c-45 | | A-a-66 | | cHexO | H | H | 3-Qu | C | | 402(M⁺+1) |
| A-c-46 | 4f-2 | Int.A-113 | Al3 | cHexO | Et | H | 6-Qu | C | | 430(M⁺+1) |
| A-c-47 | 1-a | A-c-46 | | cHexO | H | H | 6-Qu | C | | 402(M⁺+1) |
| A-c-48 | 4f-2 | Int.A-107 | Al4 | cHepO | Et | H | 2-Nap | C | | 443(M⁺+1) |
| A-c-49 | 1-a | A-c-48 | | cHepO | H | H | 2-Nap | C | | 415(M⁺+1) |
| A-c-50 | 4f-2 | Int.A-108 | Al4 | cHepO | Et | H | 5-Ind | C | | 432(M⁺+1) |
| A-c-51 | 1-a | A-c-50 | | cHepO | H | H | 5-Ind | C | | 404(M⁺+1) |
| A-c-52 | | A-a-74 | | cHepO | H | H | 1Me-5-Idz | C | | 419(M⁺+1) |
| A-c-53 | | A-a-76 | | cHepO | H | H | 6-Qu | C | | 416(M⁺+1) |
| A-c-54 | | A-a-78 | | nPrO | H | H | 5-Ind | C | | 350(M⁺+1) |
| A-c-55 | 4f-1 | Int.A-109 | Hal3 | nPrO | Et | H | 1Me-5-Ind | C | | 392(M⁺+1) |
| A-c-56 | 1-a | A-c-55 | | nPrO | H | H | 1Me-5-Ind | C | | 364(M⁺+1) |
| A-c-57 | | A-a-82 | | nPrO | H | H | 5-1Idz | C | | 351(M⁺+1) |
| A-c-58 | | A-a-84 | | nPrO | H | H | 1Me-5-Idz | C | | 365(M⁺+1) |
| A-c-59 | | A-a-86 | | iPrO | H | H | 2-Nap | C | | 361(M⁺+1) |
| A-c-60 | | A-a-88 | | iPrO | H | H | 1Me-5-Ind | C | | 364(M⁺+1) |
| A-c-61 | | A-a-90 | | iPrO | H | H | 1Me-5-Idz | C | | 365(M⁺+1) |
| A-c-62 | | A-a-92 | | iPrO | H | H | 6-Qu | C | | 362(M⁺+1) |
| A-c-63 | | A-a-94 | | nBuO | H | H | 5-Ind | C | | 364(M⁺+1) |
| A-c-64 | | A-a-96 | | nBuO | H | H | 1Me-5-Ind | C | | 378(M⁺+1) |
| A-c-65 | 4f-1 | Int.A-111 | Hal5 | nBuO | Et | H | 1Me-5-Idz | C | | 407(M⁺+1) |
| A-c-66 | 1-a | A-c-65 | | nBuO | H | H | 1Me-5-Idz | C | | 379(M⁺+1) |
| A-c-67 | 4f-1 | Int.A-107 | Hal6 | iBuO | Et | H | 2-Nap | C | | 403(M⁺+1) |
| A-c-68 | 1-a | A-c-67 | | iBuO | H | H | 2-Nap | C | | 375(M⁺+1) |
| A-c-69 | 4f-1 | Int.A-109 | Hal6 | iBuO | Et | H | 1Me-5-Ind | C | | 406(M⁺+1) |
| A-c-70 | 1-a | A-c-69 | | iBuO | H | H | 1Me-5-Ind | C | | 378(M⁺+1) |
| A-c-71 | | A-a-104 | | iBuO | H | H | 5-1Idz | C | | 365(M⁺+1) |
| A-c-72 | | A-a-106 | | iBuO | H | H | 6-IQ | C | | 376(M⁺+1) |
| A-c-73 | 4f-1 | Int.A-107 | Hal7 | 2FBnO | Et | H | 2-Nap | C | | 455(M⁺+1) |
| A-c-74 | 1-a | A-c-73 | | 2FBnO | H | H | 2-Nap | C | | 427(M⁺+1) |
| A-c-75 | 4f-1 | Int.A-108 | Hal7 | 2FBnO | Et | H | 5-Ind | C | | 444(M⁺+1) |
| A-c-76 | 1-a | A-c-75 | | 2FBnO | H | H | 5-Ind | C | | 416(M⁺+1) |
| A-c-77 | | A-a-112 | | 2FBnO | H | H | 1Me-5-Idz | C | | 431(M⁺+1) |
| A-c-78 | 4f-1 | Int.A-109 | Hal8 | 3FBnO | Et | H | 1Me-5-Ind | C | | 458(M⁺+1) |
| A-c-79 | 1-a | A-c-78 | | 3FBnO | H | H | 1Me-5-Ind | C | | 430(M⁺+1) |
| A-c-80 | | A-a-116 | | 3FBnO | H | H | 1Me-5-Idz | C | | 431(M⁺+1) |
| A-c-81 | 4f-1 | Int.A-113 | Hal8 | 3FBnO | Et | H | 6-Qu | C | | 456(M⁺+1) |
| A-c-82 | 1-a | A-c-81 | | 3FBnO | H | H | 6-Qu | C | | 428(M⁺+1) |
| A-c-83 | | A-a-120 | | 4FBnO | H | H | 2-Nap | C | | 427(M⁺+1) |
| A-c-84 | | A-a-122 | | 4FBnO | H | H | 1Et-5-Idz | C | | 445(M⁺+1) |
| A-c-85 | | A-a-124 | | 4FBnO | H | H | 5-BF | C | | 417(M⁺+1) |
| A-c-86 | | A-a-126 | | 4FBnO | H | H | 6-Qu | C | | 428(M⁺+1) |
| A-c-87 | | A-a-128 | | 2ClBnO | H | H | 2-Nap | C | | 443(M⁺+1) |
| A-c-88 | | A-a-130 | | 2ClBnO | H | H | 1Et-5-Idz | C | | 461(M⁺+1) |
| A-c-89 | | A-a-132 | | 2ClBnO | H | H | 5-BF | C | | 433(M⁺+1) |
| A-c-90 | | A-a-134 | | 3ClBnO | H | H | 2-Nap | C | | 443(M⁺+1) |
| A-c-91 | 4f-1 | Int.A-108 | Hal11 | 3ClBnO | Et | H | 5-Ind | C | | 460(M⁺+1) |
| A-c-92 | 1-a | A-c-81 | | 3ClBnO | H | H | 5-Ind | C | | 432(M⁺+1) |

**[Table 35]**

| Table-A-c-3 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-c-93 | 4f-1 | Int.A-109 | Hal11 | 3ClBnO | Et | H | 1Me-5-Ind | C | | 474(M⁺+1) |
| A-c-94 | 1-a | A-c-93 | | 3ClBnO | H | H | 1Me-5-Ind | C | | 446(M⁺+1) |
| A-c-95 | | A-a-140 | | 3ClBnO | H | H | 1Me-5-Idz | C | | 447(M⁺+1) |
| A-c-96 | | A-a-142 | | 4MeBnO | H | H | 5-Ind | C | | 412(M⁺+1) |
| A-c-97 | | A-a-144 | | 4MeBnO | H | H | 1Me-5-Ind | C | | 426(M⁺+1) |
| A-c-98 | | A-a-146 | | 4MeBnO | H | H | 1Me-5-Idz | C | | 427(M⁺+1) |
| A-c-99 | 4f-1 | Int.A-107 | Hal13 | 4CF3BnO | Et | H | 2-Nap | C | | 504(M⁺+1) |
| A-c-100 | 1-a | A-c-99 | | 4CF3BnO | H | H | 2-Nap | C | | 476(M⁺+1) |
| A-c-101 | 4f-1 | Int.A-109 | Hal13 | 4CF3BnO | Et | H | 1Me-5-Ind | C | | 507(M⁺+1) |
| A-c-102 | 1-a | A-c-101 | | 4CF3BnO | H | H | 1 Me-5-Ind | C | | 479(M⁺+1) |
| A-c-103 | 4f-1 | Int.A-110 | Hal13 | 4CF3BnO | Et | H | 5-1Idz | C | | 494(M⁺+1) |
| A-c-104 | 1-a | A-c-103 | | 4CF3BnO | H | H | 5-1Idz | C | | 466(M⁺+1) |
| A-c-105 | | A-a-154 | | 2EtBuO | H | H | 2-Nap | C | | 403(M⁺+1) |
| A-c-106 | 4f-2 | Int.A-108 | A15 | 2EtBuO | Et | H | 5-Ind | C | | 420(M⁺+1) |
| A-c-107 | 1-a | A-c-106 | | 2EtBuO | H | H | 5-Ind | C | | 392(M⁺+1) |
| A-c-108 | | A-a-158 | | 2EtBuO | H | H | 1 Me-5-Idz | C | | 407(M⁺+1) |
| A-c-109 | | A-a-160 | | 2(4DMAPh)EtO | H | H | 5-Ind | C | | 455(M⁺+1) |
| A-c-110 | | A-a-162 | | 2(4DMAPh)EtO | H | H | 1 Me-5-Ind | C | | 469(M⁺+1) |
| A-c-111 | 4f-2 | Int.A-112 | A16 | 2(4DMAPh)EtO | Et | H | 1 Et-5-Idz | C | | 512(M⁺+1) |
| A-c-112 | 1-a | A-c-111 | | 2(4DMAPh)EtO | H | H | 1 Et-5-Idz | C | | 484(M⁺+1) |
| A-c-113 | 4f-2 | Int.A-107 | A17 | 2(PhO)EtO | Et | H | 2-Nap | C | | 467(M⁺+1) |
| A-c-114 | 1-a | A-c-111 | | 2(PhO)EtO | H | H | 2-Nap | C | | 439(M⁺+1) |
| A-c-115 | | A-a-168 | | 2(PhO)EtO | H | H | 1 Me-5-Ind | C | | 442(M⁺+1) |
| A-c-116 | | A-a-170 | | 2(PhO)EtO | H | H | 6-Qu | C | | 440(M⁺+1) |
| A-c-117 | | A-a-172 | | 1PhEtO | H | H | 2-Nap | C | | 423(M⁺+1) |
| A-c-118 | | A-a-174 | | 1PhEtO | H | H | 1 Me-5-Ind | C | | 426(M⁺+1) |
| A-c-119 | | A-a-176 | | 1PhEtO | H | H | 5-1Idz | C | | 413(M⁺+1) |
| A-c-120 | | A-a-178 | | 1(2FPh)EtO | H | H | 5-Ind | C | | 430(M⁺+1) |
| A-c-121 | | A-a-180 | | 1(2FPh)EtO | H | H | 1Et-5-Idz | C | | 459(M⁺+1) |
| A-c-122 | | A-a-182 | | 1(2FPh)EtO | H | H | 6-Qu | C | | 442(M⁺+1) |
| A-c-123 | | A-a-184 | | 1(4ClPh)EtO | H | H | 5-Ind | C | | 446(M⁺+1) |
| A-c-124 | 4f-2 | Int.A-109 | Al10 | 1(4ClPh)EtO | Et | H | 1Me-5-Ind | C | | 489(M⁺+1) |
| A-c-125 | 1-a | A-c-124 | | 1(4ClPh)EtO | H | H | 1 Me-5-Ind | C | | 460(M⁺+1) |
| A-c-126 | | A-a-188 | | 1(4ClPh)EtO | H | H | 1Et-5-Idz | C | | 476(M⁺+1) |
| A-c-127 | | A-a-190 | | 4Me,cHexO | H | H | 2-Nap | C | | 415(M⁺+1) |
| A-c-128 | | A-a-192 | | 4Me,cHexO | H | H | 1 Me-5-Ind | C | | 418(M⁺+1) |
| A-c-129 | 4f-2 | Int.A-108 | Al12 | 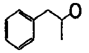 | Et | H | 5-Ind | C | | 454(M⁺+1) |
| A-c-130 | 1-a | A-c-129 | | 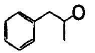 | H | H | 5-Ind | C | | 426(M⁺+1) |
| A-c-131 | | A-a-196 | | 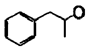 | H | H | 1Me-5-Idz | C | | 441(M⁺+1) |
| A-c-132 | 4f-2 | Int.A-107 | Al13 | 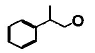 | Et | H | 2-Nap | C | | 465(M⁺+1) |
| A-c-133 | 1-a | A-c-132 | | 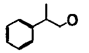 | H | H | 2-Nap | C | | 437(M⁺+1) |
| A-c-134 | | A-a-200 | | 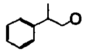 | H | H | 1Et-5-Idz | C | | 455(M⁺+1) |

**[Table 36]**

| Table-A-c-4 | | | | | | | | | | |
|---|---|---|---|---|---|---|---|---|---|---|
| Exp | Syn. | SM | Reagent | Rx | Y | V1' | V2' | LCMS | | |
| | | | | | | | | method | RTime | Mass |
| A-c-135 | | A-a-202 | | 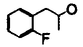 | H | H | 5-Ind | C | | 444(M⁺+1) |
| A-c-136 | | A-a-204 | | 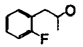 | H | H | 1Me-5-Ind | C | | 458(M⁺+1) |
| A-c-137 | | A-a-206 | | 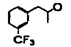 | H | H | 5-Ind | C | | 494(M⁺+1) |
| A-c-138 | | A-a-208 | | 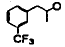 | H | H | 1 Me-5-Ind | C | | 508(M⁺+1) |
| A-c-139 | | A-a-210 | | 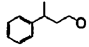 | H | H | 1Me-5-Ind | C | | 454(M⁺+1) |
| A-c-140 | | A-a-212 | | 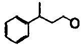 | H | H | 1Me-5-Idz | C | | 455(M⁺+1) |
| A-c-141 | 4f-2 | Int.A-107 | Al17 | 1IndanO | Et | H | 2-Nap | C | | 464(M⁺+1) |
| A-c-142 | 1-a | A-c-141 | | 1IndanO | H | H | 2-Nap | C | | 436(M⁺+1) |
| A-c-143 | | A-a-216 | | 1IndanO | H | H | 1 Me-5-Ind | C | | 438(M⁺+1) |
| A-c-144 | | A-a-218 | | 1IndanO | H | H | 5-1Idz | C | | 425(M⁺+1) |
| A-c-145 | | A-a-220 | | 2IndanO | H | H | 2-Nap | C | | 435(M⁺+1) |
| A-c-146 | 4f-2 | Int.A-108 | Al18 | 2IndanO | Et | H | 5-Ind | C | | 452(M⁺+1) |
| A-c-147 | 1-a | A-c-146 | | 2IndanO | H | H | 5-Ind | C | | 424(M⁺+1) |
| A-c-148 | | A-a-224 | | 2IndanO | H | H | 1Me-5-Idz | C | | 439(M⁺+1) |
| A-c-149 | | A-a-226 | | 5OMe-2-IndanO | H | H | 1Me-5-Ind | C | | 468(M⁺+1) |
| A-c-150 | | A-a-228 | | 5OMe-2-IndanO | H | H | 5-1Idz | C | | 455(M⁺+1) |
| A-c-151 | 4f-2 | Int.A-112 | Al19 | 5OMe-2-IndanO | Et | H | 1Et-5-Idz | C | | 511(M⁺+1) |
| A-c-152 | 1-a | A-c-151 | | 5OMe-2-IndanO | H | H | 1Et-5-Idz | C | | 483(M⁺+1) |
| A-c-153 | | A-a-232 | | 5,6D(OMe)-2-IndanO | H | H | 2-Nap | C | | 495(M⁺+1) |
| A-c-154 | | A-a-234 | | 5,6D(OMe)-2-IndanO | H | H | 5-Ind | C | | 484(M⁺+1) |
| A-c-155 | | A-a-236 | | 5F-2-IndanO | H | H | 2-Nap | C | | 453(M⁺+1) |
| A-c-156 | 4f-2 | Int.A-109 | | 5F-2-IndanO | Et | H | 1 Me-5-Ind | C | | 484(M⁺+1) |
| A-c-157 | 1-a | A-c-156 | | 5F-2-IndanO | H | H | 1 Me-5-Ind | C | | 456(M⁺+1) |
| A-c-158 | | A-a-240 | | 5F-2-IndanO | H | H | 1Me-5-Idz | C | | 457(M⁺+1) |
| A-c-159 | 4f-2 | Int.A-112 | Al21 | 5F-2-IndanO | Et | H | 1Et-5-Idz | C | | 499(M⁺+1) |
| A-c-160 | 1-a | A-c-159 | | 5F-2-IndanO | H | H | 1Et-5-Idz | C | | 471(M⁺+1) |
| A-c-161 | | A-a-244 | | 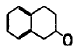 | H | H | 5-Ind | C | | 438(M⁺+1) |
| A-c-162 | | A-a-246 | | 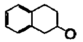 | H | H | 1 Me-5-Ind | C | | 452(M⁺+1) |
| A-c-163 | | A-a-248 | | 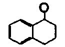 | H | H | 2-Nap | C | | 449(M⁺+1) |
| A-c-164 | | A-a-250 | | 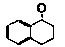 | H | H | 5-1Idz | C | | 439(M⁺+1) |
| A-c-165 | | A-a-252 | | 2(2MePh)EtO | H | H | 1 Me-5-Ind | C | | 440(M⁺+1) |
| A-c-166 | 4f-2 | Int.A-113 | Al24 | 2(2MePh)EtO | Et | H | 6-Qu | C | | 466(M⁺+1) |
| A-c-167 | 1-a | A-c-166 | | 2(2MePh)EtO | H | H | 6-Qu | C | | 438(M⁺+1) |
| A-c-168 | 4f-2 | Int.A-107 | Al25 | 2(3FPh)EtO | Et | H | 2-Nap | C | | 469(M⁺+1) |
| A-c-169 | 1-a | A-c-168 | | 2(3FPh)EtO | H | H | 2-Nap | C | | 441(M⁺+1) |
| A-c-170 | | A-a-258 | | 2(3FPh)EtO | H | H | 5-Ind | C | | 430(M⁺+1) |
| A-c-171 | | A-a-260 | | 2(2ClPh)EtO | H | H | 1Me-5-Idz | C | | 461(M⁺+1) |
| A-c-172 | | A-a-262 | | 2(2ClPh)EtO | H | H | 6-Qu | C | | 458(M⁺+1) |

**[Table 37]**

| Table-A-c-5 | | | | | | | | | | |
|---|---|---|---|---|---|---|---|---|---|---|
| A-bxp. | Syn. | SM | Reagent | Rx | Y | V1' | V2' | LCMS | | |
| | | | | | | | | method | RTime | Mass |
| A-c-173 | | A-a-264 | | 2(1-Nap)EtO | H | H | 2-Nap | C | | 473(M⁺+1) |
| A-c-174 | | A-a-266 | | 2(1-Nap)EtO | H | H | 5-Ind | C | | 462(M⁺+1) |
| A-c-175 | | A-a-268 | | 2(2-Nap)EtO | H | H | 1 Me-5-Ind | C | | 476(M⁺+1) |
| A-c-176 | | A-a-270 | | 2(2-Nap)EtO | H | H | 5-1Idz | C | | 463(M⁺+1) |
| A-c-177 | | A-a-272 | | 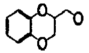 | H | H | 2-Nap | C | | 467(M⁺+1) |
| A-c-178 | | A-a-274 | | 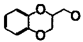 | H | H | 5-Ind | C | | 456(M⁺+1) |
| A-c-179 | | A-a-276 | | 2(PhS)EtO | H | H | 2-Nap | C | | 455(M⁺+1) |
| A-c-180 | | A-a-278 | | 2(PhS)EtO | H | H | 5-Ind | C | | 444(M⁺+1) |
| A-c-181 | | A-a-280 | | 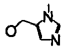 | H | H | 1Me-5-Ind | C | | 416(M⁺+1) |

### Reference Example 7: S-isomer

### Synthesis of (S)-5-benzyloxy-2,3-dihydro-1H-inden-1-ol (Intermediate A-201) (Preparation Method 15, Step o)

According to the procedure described in the synthetic method of Intermediate A-101 in Reference Example 6, a solution of Intermediate A-5 (93.0 mg) in formic acid (13.92 ml), triethylamine (5.08 ml) and ruthenium chloride/[(S,S)-N-(p-methanesulfonyl)-1,2-diphenylethylenediamine] (p-cymene) (109 mg) were reacted and treated to obtain the title compound (Intermediate A-201, 960.9 mg). Mass (LCMS): 241 (M⁺+1), Retention time: 4.45 minutes (Elution condition: D).

### Synthesis of ethyl (S)-2-(5-benzyloxy-2,3-dihydro-1H-inden-1-yl)ethane-1,1,1-triacetate (Intermediate A-202) (Preparation Method 15, Step f-2)

According to the procedure described in the synthetic method of Intermediate A-102 in Reference Example 6, Intermediate A-201 (960.9 mg), trimethylphosphine (241.8 µl), triethyl methanetricarboxylate (544 mg) and DEAD (1.06 ml) were reacted and treated to obtain the title compound a crude product of the title compound (Intermediate A-202, 1.363 g). Mass (LCMS): 455 (M⁺+1), Retention time: 5.83 minutes (Elution condition: D).

### Synthesis of (S)-2-(5-benzyloxy-2,3-dihydro-1H-inden-1-yl)ethane-1,1,1-triacetic acid (Intermediate A-203) (Preparation Method 15, Step f-2)

According to the procedure described in the synthetic method of Intermediate A-103 in Reference Example 6 provided that the reaction was performed at 130°C for 38 hours, Intermediate A-202 (1.363 g) and 2 N aqueous sodium hydroxide (11.15 ml) were reacted and treated to obtain the title compound (Compound No. Intermediate A-203, 376 mg).

### Synthesis of (S)-2-(5-benzyloxy-2,3-dihydro-1H-inden-1-yl)acetic acid (Intermediate A-204) (Preparation Method 15, Step f-2)

According to the procedure described in the synthetic method of Intermediate A-104 in Reference Example 6, Intermediate A-203 (376 mg) and acetic acid (50 ml) were reacted and treated to obtain the title compound (Compound No.: Intermediate A-204, 276.2 mg). Mass (LCMS): 283 (M⁺+1), Retention time: 4.76 minutes (Elution condition: D).

### Synthesis of ethyl (S)-2-(5-benzyloxy-2,3-dihydro-1H-inden-1-yl)acetate (Intermediate A-205) (Preparation Method 2, Step b)

According to the procedure described in the synthetic method of Intermediate A-105 in Reference Example 6 (Preparation Method 2, Step b), Intermediate A-204 (275 mg) and thionyl chloride (5 ml) were reacted and treated to obtain the title compound (Compound No.: Intermediate A-205, 281.1 mg). Mass (LCMS): 311 (M⁺+1), Retention time: 5.80 minutes (Elution condition: D).

### Synthesis of ethyl (S)-2-(5-benzyloxy-6-bromo-2,3-dihydro-1H-inden-1-yl)acetate (Intermediate A-206) (Preparation Method 8, Step h)

According to the procedure described in the synthetic method of Intermediate A-4 in Reference Example 1 (Preparation Method 8, Step h), Intermediate A-205 (281 mg) and NBS (161.3 mg) were reacted and treated to obtain the title compound (Intermediate A-206, 341.3 mg). Mass (LCMS): 391 (M⁺+1), Retention time: 6.04 minutes (Elution condition: D).

### Example A-d-1

### Example Compound A-a-2 was subjected to chiral separation to obtain the title compound (Compound No. A-d-1, 30 mg)

### Example A-d-15

### Synthesis of ethyl (S)-2-[5-benzyloxy-6-(naphthalen-2-yl)-2,3-dihydroinden-1-ylidene]acetate (Compound No. A-d-15) (Preparation Method 4, Step e-1)

According to the procedure described in the synthetic method of Example Compound A-a-1 (Preparation Method 4, Step e-1) provided that the reaction was performed for 17 hours, Intermediate A-206 (411.2 mg), 2-naphthaleneboronic acid (603.2 mg, Ald), 2 M aqueous sodium carbonate (2.2 ml) and (Ph₃P)₄Pd (272.3 mg, Nakalai Tesque) were reacted and treated to obtain the title compound (Compound No. A-d-15, 401.2 mg).

### Example A-d-16

### Synthesis of (S)-2-[5-benzyloxy-6-(naphthalen-2-yl)-2,3-dihydroinden-1-ylidene]acetic acid (Compound No. A-d-16) (Preparation Method 1, Step a)

According to the procedure described in the synthetic method of Example Compound A-a-2 (Preparation Method 1, Step a) provided that the reaction was performed for 1 hour, Example Compound A-d-15 (86.3 mg) and 2 N aqueous sodium hydroxide (0.40 ml) were reacted and treated to obtain the title compound (Compound No. A-d-16, 79.9 mg).

Typical examples of the intermediates that can be obtained by reacting and treating corresponding starting compounds using any of the methods described in the present specification including the examples described above are shown in Table-Int.A-5, and typical examples of the compounds of the present invention that can be obtained by reacting and treating corresponding starting compounds using any of the methods described in the present specification including the examples described above are shown in Table-A-d-1 to Table-A-d-5. The compounds were prepared according to the preparation methods of the compounds of the compound numbers shown in the columns of "Syn" in the tables. "Int" means an intermediate compound number. In the tables, used methods among the aforementioned synthetic methods are shown in the columns of "Syn" with symbols, the starting compounds are shown in the columns of "SM", and the reagents are shown in the columns of "Regent". The halide regents mentioned in the columns of "Reagent" with symbols "Hal (No.)" are those mentioned in Table-Hal, the alcohol regents shown with symbols "Al (No.)" are those mentioned in Table-Al, the boronic acid regents shown with symbols "BRA (No.)" are those mentioned in Table-BRA, and the bromoheterocyclic ring regents mentioned with the symbols "Het (No.)" are those mentioned in Table-Het. Those regents for which cells of the columns of "Manufacturer" are blank were synthesized according to methods described in ordinary chemical literatures.
In Table-Int.A-5, for the compounds for which cells of "Syn" are blank, a deprotection reaction was performed with Pd/C and hydrogen. In Table-A-d-1 to Table-A-d-5, for the compounds for which cells of "Syn" are blank, the objective compounds were obtained by HPLC fractionation.

**[Table 38]**

| Table-Int.A-5 | | | | | | | | | | |
|---|---|---|---|---|---|---|---|---|---|---|
| 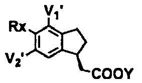 | | | | | | | | | | |

| Exp. | Syn. | SM | Reagent | Rx | Y | V1' | V2' | LCMS | | |
|---|---|---|---|---|---|---|---|---|---|---|
| | | | | | | | | method | RTime | Mass |
| Int.A-207 | | A-c-15 | | HO | Et | H | 2-Nap | C | | 347 (M⁺+1) |
| Int.A-208 | | A-c-17 | | HO | Et | H | 5-Ind | C | | 336 (M⁺+1) |
| Int.A-209 | | A-c-19 | | HO | Et | H | 1Me-5-Inc | C | | 350(M⁺+1) |
| Int.A-210 | | A-c-21 | | HO | Et | H | 5-1Idz | C | | 337(M⁺+1) |
| Int.A-211 | | A-c-23 | | HO | Et | H | 1Me-5-Idz | C | | 351(M⁺+1) |
| Int.A-212 | | A-c-25 | | HO | Et | H | 1Et-5-Idz | C | | 365(M⁺+1) |
| Int.A-213 | | A-c-29 | | HO | Et | H | 6-Qu | C | | 348(M⁺+1) |

**[Table 39]**

| Table-A-d-1 | | | | | | | | | | |
|---|---|---|---|---|---|---|---|---|---|---|
| 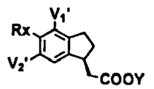 | | | | | | | | | | |

| Exp. | Syn. | SM | Reagent | Rx | Y | V1' | V2' | LCMS | | |
|---|---|---|---|---|---|---|---|---|---|---|
| | | | | | | | | method | RTime | Mass |
| A-d-1 | | A-a-2 | | cPenO | H | H | 2-Nap | C | | 387(M⁺+1) |
| A-d-2 | | A-a-4 | | cPenO | H | H | 5-Ind | C | | 376(M⁺+1) |
| A-d-3 | | A-a-6 | | cPenO | H | H | 1Me-5-Ind | C | | 390(M⁺+1) |
| A-d-4 | | A-a-8 | | cPenO | H | H | 1Et-5-Ind | C | | 404(M⁺+1) |
| A-d-5 | | A-a-10 | | cPenO | H | H | 5-1Idz | C | | 377(M⁺+1) |
| A-d-6 | | A-a-12 | | cPenO | H | H | 1Me-5-Idz | C | | 391(M⁺+1) |
| A-d-7 | | A-a-14 | | cPenO | H | H | 1Et-5-Idz | C | | 405(M⁺+1) |
| A-d-8 | | A-a-16 | | cPenO | H | H | 2Me-5-Idz | C | | 391(M⁺+1) |
| A-d-9 | | A-a-18 | | cPenO | H | H | 5-BF | C | | 377(M⁺+1) |
| A-d-10 | | A-a-20 | | cPenO | H | H | 5-BT | C | | 393(M⁺+1) |
| A-d-11 | | A-a-22 | | cPenO | H | H | 5-Bzt | C | | 394(M⁺+1) |
| A-d-12 | | A-a-24 | | cPenO | H | H | 3-Qu | C | | 388(M⁺+1) |
| A-d-13 | | A-a-26 | | cPenO | H | H | 6-Qu | C | | 388(M⁺+1) |
| A-d-14 | | A-a-28 | | cPenO | H | H | 6-IQ | C | | 388(M⁺+1) |
| A-d-15 | 4e-1 | Int.A-206 | BRA1 | BnO | Et | H | 2-Nap | C | | 437(M⁺+1) |
| A-d-16 | 1-a | A-d-15 | | BnO | H | H | 2-Nap | C | | 409(M⁺+1) |
| A-d-17 | 4e-1 | Int.A-206 | BRA2 | BnO | Et | H | 5-Ind | C | | 426(M⁺+1) |
| A-d-18 | 1-a | A-d-17 | | BnO | H | H | 5-Ind | C | | 398(M⁺+1) |
| A-d-19 | 4e-1 | Int.A-206 | BRA3 | BnO | Et | H | 1Me-5-Ind | C | | 440(M⁺+1) |
| A-d-20 | 1-a | A-d-19 | | BnO | H | H | 1Me-5-Ind | C | | 412(M⁺+1) |
| A-d-21 | 4e-1 | Int.A-206 | BRA7 | BnO | Et | H | 5-1Idz | C | | 427(M⁺+1) |
| A-d-22 | 1-a | A-d-21 | | BnO | H | H | 5-1Idz | C | | 399(M⁺+1) |
| A-d-23 | 4e-1 | Int.A-206 | BRA8 | BnO | Et | H | 1Me-5-Idz | C | | 441(M⁺+1) |
| A-d-24 | 1-a | A-d-23 | | BnO | H | H | 1Me-5-Idz | C | | 413(M⁺+1) |
| A-d-25 | 4e-1 | Int.A-206 | BRA9 | BnO | Et | H | 1Et-5-Idz | C | | 455(M⁺+1) |
| A-d-26 | 1-a | A-d-25 | | BnO | H | H | 1Et-5-Idz | C | | 427(M⁺+1) |
| A-d-27 | 4e-1 | Int.A-206 | BRA11 | BnO | Et | H | 5-BF | C | | 427(M⁺+1) |
| A-d-28 | 1-a | A-d-27 | | BnO | H | H | 5-BF | C | | 399(M⁺+1) |
| A-d-29 | 4e-2 | Int.A-206 | Het2 | BnO | Et | H | 3-Qu | C | | 438(M⁺+1) |
| A-d-30 | 1-a | A-d-29 | | BnO | H | H | 3-Qu | C | | 410(M⁺+1) |
| A-d-31 | 4f-2 | Int.A-208 | A12 | cPenMeO | Et | H | 5-Ind | C | | 418(M⁺+1) |
| A-d-32 | 1-a | A-d-31 | | cPenMeO | H | H | 5-Ind | C | | 390(M⁺+1) |
| A-d-32 | | A-a-48 | | cPenMeO | H | H | 1Et-5-Ind | C | | 418(M⁺+1) |
| A-d-33 | 4f-2 | Int.A-211 | A12 | cPenMeO | Et | H | 1Me-5-Idz | C | | 433(M⁺+1) |
| A-d-34 | 1-a | A-d-33 | | cPenMeO | H | H | 1Me-5-Idz | C | | 405(M⁺+1) |
| A-d-35 | | A-a-52 | | cPenMeO | H | H | 3-Qu | C | | 402(M⁺+1) |
| A-d-36 | 4f-1 | Int.A-208 | Hal2 | cHexMeO | Et | H | 5-Ind | C | | 432(M⁺+1) |
| A-d-37 | 1-a | A-d-33 | | cHexMeO | H | H | 5-Ind | C | | 404(M⁺+1) |
| A-d-38 | | A-a-56 | | cHexMeO | H | H | 1Me-5-Ind | C | | 418(M⁺+1) |
| A-d-39 | | A-a-58 | | cHexMeO | H | H | 5-1Idz | C | | 405(M⁺+1) |
| A-d-40 | | A-a-60 | | cHexMeO | H | H | 6-IQ | C | | 416(M⁺+1) |
| A-d-41 | 4f-2 | Int.A-207 | Al3 | cHexO | Et | H | 2-Nap | C | | 429(M⁺+1) |
| A-d-42 | 1-a | A-d-41 | | cHexO | H | H | 2-Nap | C | | 401(M⁺+1) |
| A-d-43 | 4f-2 | Int.A-209 | Al3 | cHexO | Et | H | 1Me-5-Ind | C | | 432(M⁺+1) |

**[Table 40]**

| Table-A-d-2 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-d-44 | 1-a | A-d-43 | | cHexO | H | H | 1Me-5-Ind | C | | 404(M⁺+1) |
| A-d-45 | | A-a-64 | | cHexO | H | H | 1Me-5-Idz | C | | 405(M⁺+1) |
| A-d-46 | | A-a-66 | | cHexO | H | H | 3-Qu | C | | 402(M⁺+1) |
| A-d-47 | 4f-2 | Int.A-208 | Al4 | cHepO | Et | H | 5-Ind | C | | 432(M⁺+1) |
| A-d-48 | 1-a | A-d-47 | | cHepO | H | H | 5-Ind | C | | 404(M⁺+1) |
| A-d-49 | 4f-2 | Int.A-210 | Al4 | cHepO | Et | H | 5-1Idz | C | | 433(M⁺+1) |
| A-d-50 | 1-a | A-d-49 | | cHepO | H | H | 5-1Idz | C | | 405(M⁺+1) |
| A-d-51 | | A-a-74 | | cHepO | H | H | 1Me-5-Idz | C | | 419(M⁺+1) |
| A-d-52 | | A-a-76 | | cHepO | H | H | 6-Qu | C | | 416(M⁺+1) |
| A-d-53 | 4f-1 | Int.A-207 | Hal3 | nPrO | Et | H | 2-Nap | C | | 389(M⁺+1) |
| A-d-54 | 1-a | A-d-53 | | nPrO | H | H | 2-Nap | C | | 361(M⁺+1) |
| A-d-55 | | A-a-78 | | nPrO | H | H | 5-Ind | C | | 350(M⁺+1) |
| A-d-56 | | A-a-82 | | nPrO | H | H | 5-1Idz | C | | 351(M⁺+1) |
| A-d-57 | | A-a-84 | | nPrO | H | H | 1 Me-5-Idz | C | | 365(M⁺+1) |
| A-d-58 | | A-a-86 | | iPrO | H | H | 2-Nap | C | | 361(M⁺+1) |
| A-d-59 | | A-a-88 | | iPrO | H | H | 1Me-5-Ind | C | | 364(M⁺+1) |
| A-d-60 | | A-a-90 | | iPrO | H | H | 1Me-5-Idz | C | | 365(M⁺+1) |
| A-d-61 | | A-a-92 | | iPrO | H | H | 6-Qu | C | | 362(M⁺+1) |
| A-d-62 | | A-a-94 | | nBuO | H | H | 5-Ind | C | | 364(M⁺+1) |
| A-d-63 | | A-a-96 | | nBuO | H | H | 1Me-5-Ind | C | | 378(M⁺+1) |
| A-d-64 | 4f-1 | Int.A-211 | Hal5 | nBuO | Et | H | 1 Me-5-Idz | C | | 407(M⁺+1) |
| A-d-65 | 1-a | A-d-64 | | nBuO | H | H | 1Me-5-Idz | C | | 379(M⁺+1) |
| A-d-66 | 4f-1 | Int.A-207 | Hal6 | iBuO | Et | H | 2-Nap | C | | 403(M⁺+1) |
| A-d-67 | 1-a | A-d-66 | | iBuO | H | H | 2-Nap | C | | 375(M⁺+1) |
| A-d-68 | 4f-1 | Int.A-207 | Hal6 | iBuO | Et | H | 5-Ind | C | | 392(M⁺+1) |
| A-d-69 | 1-a | A-d-68 | | iBuO | H | H | 5-Ind | C | | 364(M⁺+1) |
| A-d-70 | | A-a-104 | | iBuO | H | H | 5-1Idz | C | | 365(M⁺+1) |
| A-d-71 | 4f-1 | Int.A-211 | | iBuO | Et | H | 1Me-5-Idz | C | | 407(M⁺+1) |
| A-d-72 | 1-a | A-d-71 | | iBuO | H | H | 1Me-5-Idz | C | | 379(M⁺+1) |
| A-d-73 | 4f-1 | Int.A-207 | Hal7 | 2FBnO | Et | H | 2-Nap | C | | 455(M⁺+1) |
| A-d-74 | 1-a | A-d-73 | | 2FBnO | H | H | 2-Nap | C | | 427(M⁺+1) |
| A-d-75 | 4f-1 | Int.A-208 | Hal7 | 2FBnO | Et | H | 5-Ind | C | | 444(M⁺+1) |
| A-d-76 | 1-a | A-d-75 | | 2FBnO | H | H | 5-Ind | C | | 416(M⁺+1) |
| A-d-77 | | A-a-112 | | 2FBnO | H | H | 1 Me-5-Idz | C | | 431(M⁺+1) |
| A-d-78 | 4f-1 | Int.A-209 | Hal8 | 3FBnO | Et | H | 1Me-5-Ind | C | | 458(M⁺+1) |
| A-d-79 | 1-a | A-d-78 | | 3FBnO | H | H | 1Me-5-Ind | C | | 430(M⁺+1) |
| A-d-80 | 4f-1 | Int.A-210 | Hal8 | 3FBnO | Et | H | 5-1Idz | C | | 445(M⁺+1) |
| A-d-81 | 1-a | A-d-80 | | 3FBnO | H | H | 5-1Idz | C | | 417(M⁺+1) |
| A-d-82 | | A-a-116 | | 3FBnO | H | H | 1Me-5-Idz | C | | 431(M⁺+1) |
| A-d-83 | | A-a-120 | | 4FBnO | H | H | 2-Nap | C | | 427(M⁺+1) |
| A-d-84 | | A-a-122 | | 4FBnO | H | H | 1 Et-5-Idz | C | | 445(M⁺+1) |
| A-d-85 | | A-a-124 | | 4FBnO | H | H | 5-BF | C | | 417(M⁺+1) |
| A-d-86 | | A-a-126 | | 4FBnO | H | H | 6-Qu | C | | 443(M⁺+1) |
| A-d-87 | | A-a-128 | | 2ClBnO | H | H | 2-Nap | C | | 443(M⁺+1) |
| A-d-88 | | A-a-130 | | 2ClBnO | H | H | 1Et-5-Idz | C | | 461(M⁺+1) |
| A-d-89 | | A-a-132 | | 2ClBnO | H | H | 5-BF | C | | 433(M⁺+1) |
| A-d-90 | | A-a-134 | | 3ClBnO | H | H | 2-Nap | C | | 443(M⁺+1) |
| A-d-91 | 4f-1 | Int.A-209 | Hal11 | 3ClBnO | Et | H | 1Me-5-Ind | C | | 474(M⁺+1) |

**[Table 41]**

| Table-A-d-3 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-d-92 | 1-a | A-d-91 | | 3ClBnO | H | H | 1Me-5-Ind | C | | 446(M⁺+1) |
| A-d-93 | 4f-1 | Int.A-210 | | 3ClBnO | Et | H | 5-1Idz | C | | 461(M⁺+1) |
| A-d-94 | 1-a | A-d-93 | | 3ClBnO | H | H | 5-1Idz | C | | 433(M⁺+1) |
| A-d-95 | | A-a-140 | | 3ClBnO | H | H | 1Me-5-Idz | C | | 447(M⁺+1) |
| A-d-96 | | A-a-142 | | 4MeBnO | H | H | 5-Ind | C | | 412(M⁺+1) |
| A-d-97 | | A-a-144 | | 4MeBnO | H | H | 1Me-5-Ind | C | | 426(M⁺+1) |
| A-d-98 | | A-a-146 | | 4MeBnO | H | H | 1Me-5-Idz | C | | 427(M⁺+1) |
| A-d-99 | 4f-1 | Int.A-207 | Hal13 | 4CF3BnO | Et | H | 2-Nap | C | | 504(M⁺+1) |
| A-d-100 | 1-a | A-d-99 | | 4CF3BnO | H | H | 2-Nap | C | | 476(M⁺+1) |
| A-d-101 | 4f-1 | Int.A-209 | Hal13 | 4CF3BnO | Et | H | 1Me-5-Ind | C | | 507(M⁺+1) |
| A-d-102 | 1-a | A-d-101 | | 4CF3BnO | H | H | 1Me-5-Ind | C | | 479(M⁺+1) |
| A-d-103 | 4f-1 | Int.A-210 | Hal13 | 4CF3BnO | Et | H | 5-1Idz | C | | 494(M⁺+1) |
| A-d-104 | 1-a | A-d-103 | | 4CF3BnO | H | H | 5-1Idz | C | | 466(M⁺+1) |
| A-d-105 | | A-a-154 | | 2EtBuO | H | H | 2-Nap | C | | 403(M⁺+1) |
| A-d-106 | 4f-2 | Int.A-209 | Al5 | 2EtBuO | Et | H | 1 Me-5-Ind | C | | 434(M⁺+1) |
| A-d-107 | 1-a | A-d-106 | | 2EtBuO | H | H | 1Me-5-Ind | C | | 406(M⁺+1) |
| A-d-108 | | A-a-158 | | 2EtBuO | H | H | 1 Me-5-Idz | C | | 407(M⁺+1) |
| A-d-109 | | A-a-160 | | 2(4DMAPh)EtO | H | H | 5-Ind | C | | 455(M⁺+1) |
| A-d-110 | | A-a-162 | | 2(4DMAPh)EtO | H | H | 1 Me-5-Ind | C | | 469(M⁺+1) |
| A-d-111 | 4f-2 | Int.A-212 | Al6 | 2(4DMAPh)EtO | Et | H | 1Et-5-Idz | C | | 512(M⁺+1) |
| A-d-112 | 1-a | A-d-106 | | 2(4DMAPh)EtO | H | H | 1Et-5-Idz | C | | 484(M⁺+1) |
| A-d-113 | 4f-2 | Int.A-207 | Al7 | 2(PhO)EtO | Et | H | 2-Nap | C | | 467(M⁺+1) |
| A-d-114 | 1-a | A-d-106 | | 2(PhO)EtO | H | H | 2-Nap | C | | 439(M⁺+1) |
| A-d-115 | | A-a-168 | | 2(PhO)EtO | H | H | 1Me-5-Ind | C | | 442(M⁺+1) |
| A-d-116 | | A-a-170 | | 2(PhO)EtO | H | H | 6-Qu | C | | 440(M⁺+1) |
| A-d-117 | | A-a-172 | | 1PhEtO | H | H | 2-Nap | C | | 423(M⁺+1) |
| A-d-118 | | A-a-174 | | 1PhEtO | H | H | 1 Me-5-Ind | C | | 426(M⁺+1) |
| A-d-119 | | A-a-176 | | 1PhEtO | H | H | 5-1Idz | C | | 413(M⁺+1) |
| A-d-120 | | A-a-178 | | 1(2FPh)EtO | H | H | 5-Ind | C | | 430(M⁺+1) |
| A-d-121 | | A-a-180 | | 1(2FPh)EtO | H | H | 1Et-5-Idz | C | | 459(M⁺+1) |
| A-d-122 | | A-a-182 | | 1(2FPh)EtO | H | H | 6-Qu | C | | 442(M⁺+1) |
| A-d-123 | 4f-2 | Int.A-207 | Al10 | 1(4ClPh)EtO | Et | H | 2-Nap | C | | 489(M⁺+1) |
| A-d-124 | 1-a | A-d-123 | | 1(4ClPh)EtO | H | H | 2-Nap | C | | 457(M⁺+1) |
| A-d-125 | 4f-2 | Int.A-209 | Al10 | 1(4ClPh)EtO | Et | H | 1 Me-5-Ind | C | | 489(M⁺+1) |
| A-d-126 | 1-a | A-d-125 | | 1(4ClPh)EtO | H | H | 1 Me-5-Ind | C | | 460(M⁺+1) |
| A-d-127 | | A-a-188 | | 1(4ClPh)EtO | H | H | 1 Et-5-Idz | C | | 476(M⁺+1) |
| A-d-128 | | A-a-190 | | 4Me,cHexO | H | H | 2-Nap | C | | 415(M⁺+1) |
| A-d-129 | | A-a-192 | | 4Me,cHexO | H | H | 1 Me-5-Ind | C | | 418(M⁺+1) |
| A-d-130 | 4f-2 | Int.A-209 | Al12 | 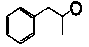 | Et | H | 1 Me-5-Ind | C | | 468(M⁺+1) |
| A-d-131 | 1-a | A-d-130 | | 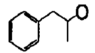 | H | H | 1 Me-5-Ind | C | | 440(M⁺+1) |
| A-d-132 | | A-a-196 | | 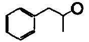 | H | H | 1Me-5-Idz | C | | 441(M⁺+1) |
| A-d-133 | 4f-2 | Int.A-207 | Al13 | 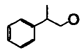 | Et | H | 2-Nap | C | | 465(M⁺+1) |
| A-d-134 | 1-a | A-d-133 | | 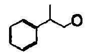 | H | H | 2-Nap | C | | 437(M⁺+1) |

**[Table 42]**

| Table-A-d-4 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-d-135 | | A-a-200 | | 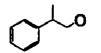 | H | H | 1 Et-5-Idz | C | | 455(M⁺+1) |
| A-d-136 | | A-a-202 | | 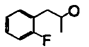 | H | H | 5-Ind | C | | 444(M⁺+1) |
| A-d-137 | | A-a-204 | | 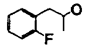 | H | H | 1Me-5-Ind | C | | 458(M⁺+1) |
| A-d-138 | | A-a-206 | | 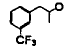 | H | H | 5-Ind | C | | 494(M⁺+1) |
| A-d-139 | | A-a-208 | | 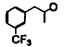 | H | H | 1 Me-5-Ind | C | | 508(M⁺+1) |
| A-d-140 | | A-a-210 | | 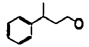 | H | H | 1 Me-5-Ind | C | | 454(M⁺+1) |
| A-d-141 | | A-a-212 | | 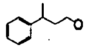 | H | H | 1 Me-5-Idz | C | | 455(M⁺+1) |
| A-d-142 | 4f-2 | Int.A-207 | Al17 | 1IndanO | Et | H | 2-Nap | C | | 463(M⁺+1) |
| A-d-143 | 1-a | A-d-142 | | 1IndanO | H | H | 2-Nap | C | | 435(M⁺+1) |
| A-d-144 | | A-a-216 | | 1IndanO | H | H | 1 Me-5-Ind | C | | 438(M⁺+1) |
| A-d-145 | | A-a-218 | | 1IndanO | H | H | 5-1Idz | C | | 425(M⁺+1) |
| A-d-146 | | A-a-220 | | 2IndanO | H | H | 2-Nap | C | | 435(M⁺+1) |
| A-d-147 | 4f-2 | Int.A-208 | Al18 | 2IndanO | Et | H | 5-Ind | C | | 452(M⁺+1) |
| A-d-148 | 1-a | A-d-147 | | 2IndanO | H | H | 5-Ind | C | | 424(M⁺+1) |
| A-d-149 | 4f-2 | Int.A-209 | Al18 | 2IndanO | Et | H | 1Me-5-Ind | C | | 466(M⁺+1) |
| A-d-150 | 1-a | A-d-149 | | 2IndanO | H | H | 1Me-5-Ind | C | | 438(M⁺+1) |
| A-d-151 | | A-a-224 | | 2IndanO | H | H | 1Me-5-Idz | C | | 439(M⁺+1) |
| A-d-152 | | A-a-226 | | 5OMe-2-IndanO | H | H | 1Me-5-Ind | C | | 468(M⁺+1) |
| A-d-153 | | A-a-228 | | 5OMe-2-IndanO | H | H | 5-1Idz | C | | 455(M⁺+1) |
| A-d-154 | 4f-2 | Int.A-212 | Al19 | 5OMe-2-IndanO | Et | H | 1Et-5-Idz | C | | 511(M⁺+1) |
| A-d-155 | 1-a | A-d-154 | | 5OMe-2-IndanO | H | H | 1Et-5-Idz | C | | 483(M⁺+1) |
| A-d-156 | | A-a-232 | | 5,6D(OMe)-2-IndanO | H | H | 2-Nap | C | | 495(M⁺+1) |
| A-d-157 | | A-a-234 | | 5,6D(OMe)-2-IndanO | H | H | 5-Ind | C | | 484(M⁺+1) |
| A-d-158 | | A-a-236 | | 5F-2-IndanO | H | H | 2-Nap | C | | 453(M⁺+1) |
| A-d-159 | 4f-2 | Int.A-209 | Al21 | 5F-2-IndanO | Et | H | 1Me-5-Ind | C | | 484(M⁺+1) |
| A-d-160 | 1-a | A-d-159 | | 5F-2-IndanO | H | H | 1Me-5-Ind | C | | 456(M⁺+1) |
| A-d-161 | 4f-2 | Int.A-210 | Al21 | 5F-2-IndanO | Et | H | 5-1Idz | C | | 471(M⁺+1) |
| A-d-162 | 1-a | A-d-162 | | 5F-2-IndanO | H | H | 5-1Idz | C | | 443(M⁺+1) |
| A-d-163 | | A-a-240 | | 5F-2-IndanO | H | H | 1Me-5-Idz | C | | 457(M⁺+1) |
| A-d-164 | 4f-2 | Int.A-212 | Al21 | 5F-2-IndanO | Et | H | 1Et-5-Idz | C | | 499(M⁺+1) |
| A-d-165 | 1-a | A-d-164 | | 5F-2-IndanO | H | H | 1Et-5-Idz | C | | 471(M⁺+1) |
| A-d-166 | | A-a-244 | | 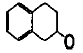 | H | H | 5-Ind | C | | 438(M⁺+1) |
| A-d-167 | | A-a-246 | | 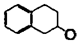 | H | H | 1 Me-5-Ind | C | | 452(M⁺+1) |
| A-d-168 | | A-a-248 | | 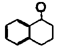 | H | H | 2-Nap | C | | 449(M⁺+1) |
| A-d-169 | | A-a-250 | | 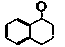 | H | H | 5-1Idz | C | | 439(M⁺+1) |
| A-d-170 | 4f-2 | Int.A-208 | Al24 | 2(2MePh)EtO | Et | H | 5-Ind | C | | 454(M⁺+1) |
| A-d-171 | 1-a | A-d-170 | | 2(2MePh)EtO | H | H | 5-Ind | C | | 426(M⁺+1) |

**[Table 43]**

| Table-A-d-5 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| A-d-172 | 4f-2 | Int.A-211 | Al24 | 2(2MePh)EtO | Et | H | 1Me-5-Idz | C | | 469(M⁺+1) |
| A-d-173 | 1-a | A-d-164 | | 2(2MePh)EtO | H | H | 1Me-5-Idz | C | | 441(M⁺+1) |
| A-d-174 | 4f-2 | Int.A-213 | Al24 | 2(2MePh)EtO | Et | H | 6-Qu | C | | 466(M⁺+1) |
| A-d-175 | 1-a | A-d-164 | | 2(2MePh)EtO | H | H | 6-Qu | C | | 438(M⁺+1) |
| A-d-176 | 4f-2 | Int.A-207 | Al25 | 2(3FPh)EtO | Et | H | 2-Nap | C | | 469(M⁺+1) |
| A-d-177 | 1-a | A-d-164 | | 2(3FPh)EtO | H | H | 2-Nap | C | | 441(M⁺+1) |
| A-d-178 | | A-a-258 | | 2(3FPh)EtO | H | H | 5-Ind | C | | 430(M⁺+1) |
| A-d-179 | | A-a-260 | | 2(2ClPh)EtO | H | H | 1Me-5-Idz | C | | 461(M⁺+1) |
| A-d-180 | | A-a-262 | | 2(2ClPh)EtO | H | H | 6-Qu | C | | 458(M⁺+1) |
| A-d-181 | | A-a-264 | | 2(1-Nap)EtO | H | H | 2-Nap | C | | 473(M⁺+1) |
| A-d-182 | | A-a-266 | | 2(1-Nap)EtO | H | H | 5-Ind | C | | 462(M⁺+1) |
| A-d-183 | | A-a-268 | | 2(2-Nap)EtO | H | H | 1Me-5-Ind | C | | 476(M⁺+1) |
| A-d-184 | | A-a-270 | | 2(2-Nap)EtO | H | H | 5-1Idz | C | | 463(M⁺+1) |
| A-d-185 | | A-a-272 | | 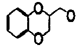 | H | H | 2-Nap | C | | 467(M⁺+1) |
| A-d-186 | | A-a-274 | | 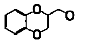 | H | H | 5-Ind | C | | 456(M⁺+1) |
| A-d-187 | | A-a-276 | | 2(PhS)EtO | H | H | 2-Nap | C | | 455(M⁺+1) |
| A-d-188 | | A-a-278 | | 2(PhS)EtO | H | H | 5-Ind | C | | 444(M⁺+1) |
| A-d-189 | | A-a-280 | | 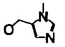 | H | H | 1Me-5-Ind | C | | 416(M⁺+1) |

### Reference Example 8

### Synthesis of 6-bromo-5-cyclopentyloxy-2,3-dihydroindan-1-one (Intermediate B-1) (Preparation Method 13, Step h)

According to the procedure described in the synthetic method of Intermediate
A-4 in Reference Example 1 (Preparation Method 8, Step h) provided that the reaction was performed at room temperature for 1 hour, Intermediate A-1 (1.19 g) and NBS (1.08 g, WAKO) were reacted and treated to obtain the title compound (Intermediate
B-1, 366.2 mg). Mass (LCMS): 295 (M⁺), Retention time: 4.88 minutes (Elution condition: B).

### Synthesis of 5-cyclopentyloxy-6-(naphthalen-2-yl)-2,3-dihydroinden-1-one (Intermediate B-2) (Preparation Method 11, Step e-1)

According to the procedure described in the synthetic method of Example Compound A-a-1 (Preparation Method 4, Step e-1) provided that the reaction was performed for 12 hours, Intermediate B-1 (363.2 mg), 2-naphthaleneboronic acid (311.2 mg, TCI), 2 M aqueous sodium carbonate (0.8 ml) and (Ph₃P)₄Pd (88.4 mg, Nakalai Tesque) were reacted and treated to obtain the title compound (Intermediate B-2, 388.5 mg). Mass (LCMS): 343 (M⁺), Retention time: N.D (Elution condition: C).

Typical examples of the intermediates that can be obtained by reacting and treating corresponding starting compounds using any of the methods described in the present specification including the examples described above are shown in Table-Int.B-1. The compounds were synthesized according to the preparation methods of the compounds of the compound numbers shown in the columns of "Syn" in the tables. "Int" means an intermediate compound number. In the tables, used methods among the aforementioned synthetic methods are shown in the columns of "Syn" with symbols, the starting compounds are shown in the columns of "SM", and the reagents are shown in the columns of "Regent". In the columns of "Reagent", the halide regents shown with symbols "Hal (No.)" are those mentioned in Table-Hal, the alcohol regents shown with symbols "Al (No.)" are those mentioned in Table-Al, the boronic acid regents shown with symbols "BRA (No.)" are those mentioned in Table-BRA, and the bromoheterocyclic ring regents mentioned with the symbols "Het (No.)" are those mentioned in Table-Het.

**[Table 44]**

| Table-Int.B-1 | | | | | | | | | |
|---|---|---|---|---|---|---|---|---|---|
| 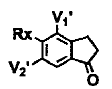 | | | | | | | | | |

| Exp. | Syn. | SM | Reagent | Rx | V1' | V2' | LCMS | | |
|---|---|---|---|---|---|---|---|---|---|
| | | | | | | | method | RTime | Mass |
| Int.B-3 | 11e-1 | Int.B-1 | BRA3 | cPenO | H | 1 Me-5-Ind | C | | 346 (M⁺+1) |
| Int.B-4 | 11e-1 | Int.B-1 | BRA4 | cPenO | H | 1Et-5-Ind | C | | 360 (M⁺+1) |
| Int.B-5 | 11e-1 | Int.B-1 | BRA5 | cPenO | H | 1Me-4-Ind | C | | 346 (M⁺+1) |
| Int.B-6 | 11e-1 | Int.B-1 | BRA6 | cPenO | H | 1Me-6-Ind | C | | 346 (M⁺+1) |
| Int.B-7 | 11e-1 | Int.B-1 | BRA8 | cPenO | H | 1Me-5-Idz | C | | 347 (M⁺+1) |
| Int.B-8 | 11e-1 | Int.B-1 | BRA9 | cPenO | H | 1Et-5-Idz | C | | 361 (M⁺+1) |
| Int.B-9 | 11e-1 | Int.B-1 | BRA11 | cPenO | H | 5-BF | C | | 333 (M⁺+1) |
| Int.B-10 | 11e-1 | Int.B-1 | BRA13 | cPenO | H | 6-Qu | C | | 344 (M⁺+1) |
| Int.B-11 | 11e-2 | Int.B-1 | Het3 | cPenO | H | 6-IQ | C | | 344 (M⁺+1) |
| Int.B-12 | 14f-1 | | Hal1 | BnO | H | H | C | | 239 (M⁺+1) |
| Int.B-13 | 13h-1 | Int.B-12 | | BnO | H | Br | C | | 317 (M⁺) |
| Int.B-14 | 11e-1 | Int.B-13 | BRA1 | BnO | H | 2-Nap | C | | 365(M⁺+1) |
| Int.B-15 | 11e-1 | Int.B-13 | BRA3 | BnO | H | 1Me-5-Ind | C | | 368 (M⁺+1) |
| Int.B-16 | 11e-1 | Int.B-13 | BRA4 | BnO | H | 1Et-5-Ind | C | | 382(M⁺+1) |
| Int.B-17 | 11e-1 | Int.B-13 | BRA5 | BnO | H | 1Me-4-Ind | C | | 368 (M⁺+1) |
| Int.B-18 | 11e-1 | Int.B-13 | BRA6 | BnO | H | 1Me-6-Ind | C | | 368 (M⁺+1) |
| Int.B-19 | 11e-1 | Int.B-13 | BRA8 | BnO | H | 1Me-5-Idz | C | | 369 (M⁺+1) |
| Int.B-20 | 11e-1 | Int.B-13 | BRA9 | BnO | H | 1Et-5-Idz | C | | 383 (M⁺+1) |
| Int.B-21 | 11e-1 | Int.B-13 | BRA11 | BnO | H | 5-BF | C | | 355(M⁺+1) |
| Int.B-22 | 11e-1 | Int.B-13 | BRA13 | BnO | H | 6-Qu | C | | 366 (M⁺+1) |
| Int.B-23 | 11e-2 | Int.B-13 | Het3 | BnO | H | 6-IQ | C | | 366 (M⁺+1) |

### Reference Example 9

### Synthesis of 5-benzyloxy-6-bromo-2,3-dihydroindan-1-one (Intermediate B-13) (Preparation Method 13, Step h)

According to the procedure described in the synthetic method of Intermediate A-4 in Reference Example 1 (Preparation Method 8, Step h) provided that the reaction was performed at room temperature for 15 hours, Intermediate B-12 (4.10 g) and NBS (3.22 g, WAKO) were reacted and treated to obtain the title compound (Intermediate B-13, 388.5 mg). Mass (LCMS): 317 (M⁺), Retention time: 4.77 minutes (Elution condition: B).

### Synthesis of 5-benzyloxy-6-(naphthalen-2-yl)-2,3-dihydroinden-1-one (Intermediate B-14) (Preparation Method 11, Step e-1)

According to the procedure described in the synthetic method of Example Compound A-a-1 (Preparation Method 4, Step e-1) provided that the reaction was performed for 12 hours, Intermediate B-13 (388.5 mg), 2-naphthaleneboronic acid (376.4 mg, TCI), 2 M aqueous sodium carbonate (0.9 ml) and (Ph₃P)₄Pd (121.5 mg, Nakalai Tesque) were reacted and treated to obtain the title compound (Intermediate B-14, 395.8 mg). Mass (LCMS): 365 (M⁺+1), Retention time: N.D (Elution condition: C).

### Synthesis of 5-hydroxy-6-(naphthalen-2-yl)-2,3-dihydroinden-1-one (Intermediate B-24)

According to the procedure described in the synthetic method of Intermediate A-22 in Example A-a-107 provided that the reaction was performed for 12 hours, Intermediate B-14 (391.3 mg) and 10% palladium hydroxide (212.2 mg, NE Chemcat) were reacted and treated to obtain the title compound (Intermediate B-24, 314.2 mg). Mass (LCMS): 275 (M⁺+1), Retention time: N.D (Elution condition: C).

### Synthesis of 5-cyclopentylmethyloxy-6-(naphthalen-2-yl)-2,3-dihydroinden-1-one (Intermediate B-34) (Preparation Method 4, Step f-2)

According to the procedure described in the synthetic method of Intermediate A-1 in Reference Example 1 (Preparation Method 4, Step f-2) provided that the reaction was performed for 12 hours, Intermediate B-24 (100.0 mg), cyclopentanemethanol (53.5 µl, Ald), di-t-butyl azodicarboxylate (165.3 mg, Ald) and triphenylphosphine (143.3 mg, WAKO) were reacted and treated to obtain the title compound (Intermediate B-34, 110.2 mg). Mass (LCMS): 357 (M⁺+1), Retention time: N.D (Elution condition: C).

Typical examples of the intermediates that can be obtained by reacting and treating corresponding starting compounds using any of the methods described in the present specification including the examples described above are shown in Table-Int.B-2 to Table-Int.B-6. The compounds were synthesized according to the preparation methods of the compounds of the compound numbers shown in the columns of "Syn" in the tables. "Int" means an intermediate compound number. In the tables, used methods among the aforementioned synthetic methods are shown in the columns of "Syn" with symbols, the starting compounds are shown in the columns of "SM", and the reagents are shown in the columns of "Regent". In the columns of "Reagent", the halide regents shown with symbols "Hal (No.)" are those mentioned in Table-Hal, the alcohol regents shown with symbols "Al (No.)" are those mentioned in Table-Al, the boronic acid regents shown with symbols "BRA (No.)" are those mentioned in Table-BRA, and the bromoheterocyclic ring regents mentioned with the symbols "Het (No.)" are those mentioned in Table-Het. For the compounds for which cells of the columns of "Syn" are blank, deprotection was performed with Pd/C and hydrogen.

**[Table 45]**

| Table-Int.B-2 | | | | | | | | | |
|---|---|---|---|---|---|---|---|---|---|
| 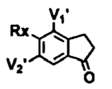 | | | | | | | | | |

| Exp. | Syn. | SM | Reagent | Rx | V1' | V2' | LCMS | | |
|---|---|---|---|---|---|---|---|---|---|
| | | | | | | | method | RTime | Mass |
| Int.B-24 | | Int.B-14 | | HO | H | 2-Nap | C | | 275 (M⁺+1) |
| Int.B-25 | | Int.B-15 | | HO | H | 1 Me-5-Ind | C | | 278(M⁺+1) |
| Int.B-26 | | Int.B-16 | | HO | H | 1 Et-5-Ind | C | | 292 (M⁺+1) |
| Int.B-27 | | Int.B-17 | | HO | H | 1Me-4-Ind | C | | 278 (M⁺+1) |
| Int.B-28 | | Int.B-18 | | HO | H | 1Me-6-Ind | C | | 278 (M⁺+1) |
| Int.B-29 | | Int.B-19 | | HO | H | 1Me-5-Idz | C | | 279 (M⁺+1) |
| Int.B-30 | | Int.B-20 | | HO | H | 1 Et-5-Idz | C | | 293 (M⁺+1) |
| Int.B-31 | | Int.B-21 | | HO | H | 5-BF | C | | 265 (M⁺+1) |
| Int.B-32 | | Int.B-22 | | HO | H | 6-Qu | C | | 276 (M⁺+1) |
| Int.B-33 | | Int.B-23 | | HO | H | 6-IQ | C | | 276 (M⁺+1) |
| Int.B-34 | f-2 | Int.B-24 | Al2 | cPenMeO | H | 2-Nap | C | | 357 (M⁺+1) |
| Int.B-35 | f-2 | Int.B-25 | Al2 | cPenMeO | H | 1Me-5-Ind | C | | 360 (M⁺+1) |
| Int.B-36 | f-2 | Int.B-27 | Al2 | cPenMeO | H | 1Me-4-Ind | C | | 360 (M⁺+1) |
| Int.B-37 | f-2 | Int.B-29 | Al2 | cPenMeO | H | 1 Me-5-Idz | C | | 361 (M⁺+1) |
| Int.B-38 | f-2 | Int.B-30 | Al2 | cPenMeO | H | 1Et-5-Idz | C | | 375(M⁺+1 |
| Int.B-39 | f-2 | Int.B-32 | Al2 | cPenMeO | H | 6-Qu | C | | 358 (M⁺+1) |
| Int.B-40 | f-1 | Int.B-24 | Hal2 | cHexMeO | H | 2-Nap | C | | 371 (M⁺+1) |
| Int.B-41 | f-1 | Int.B-26 | Hal2 | cHexMeO | H | 1Et-5-Ind | C | | 388 (M⁺+1) |
| Int.B-42 | f-1 | Int.B-28 | Hal2 | cHexMeO | H | 1Me-6-Ind | C | | 374 (M⁺+1) |
| Int.B-43 | f-1 | Int.B-29 | Hal2 | cHexMeO | H | 1 Me-5-Idz | C | | 375 (M⁺+1) |
| Int.B-44 | f-1 | Int.B-30 | Hal2 | cHexMeO | H | 1Et-5-Idz | C | | 389 (M⁺+1) |
| Int.B-45 | f-1 | Int.B-31 | Hal2 | cHexMeO | H | 5-BF | C | | 361 (M⁺+1) |
| Int.B-46 | f-1 | Int.B-33 | Hal2 | cHexMeO | H | 6-IQ | C | | 372 (M⁺+1 |
| Int.B-47 | f-2 | Int.B-24 | Al3 | cHexO | H | 2-Nap | C | | 357 (M⁺+1) |
| Int.B-48 | f-2 | Int.B-25 | Al3 | cHexO | H | 1 Me-5-Ind | C | | 360 (M⁺+1) |
| Int.B-49 | f-2 | Int.B-26 | Al3 | cHexO | H | 1 Et-5-Ind | C | | 374 (M⁺+1) |
| Int.B-50 | f-2 | Int.B-27 | Al3 | cHexO | H | 1Me-4-Ind | C | | 360 (M⁺+1) |
| Int.B-51 | f-2 | Int.B-29 | Al3 | cHexO | H | 1Me-5-Idz | C | | 361 (M⁺+1 |
| Int.B-52 | f-2 | Int.B-30 | Al3 | cHexO | H | 1Et-5-Idz | C | | 375 (M⁺+1) |
| Int.B-53 | f-2 | Int.B-32 | Al3 | cHexO | H | 6-Qu | C | | 358 (M⁺+1) |
| Int.B-54 | f-2 | Int.B-24 | Al4 | cHepO | H | 2-Nap | C | | 371 (M⁺+1) |
| Int.B-55 | f-2 | Int.B-25 | Al4 | cHepO | H | 1 Me-5-Ind | C | | 374 (M⁺+1) |
| Int.B-56 | f-2 | Int.B-29 | Al4 | cHepO | H | 1Me-5-Idz | C | | 375 (M⁺+1) |
| Int.B-57 | f-2 | Int.B-33 | Al4 | cHepO | H | 6-IQ | C | | 372 (M⁺+1) |
| Int.B-58 | f-1 | Int.B-25 | Hal3 | nPrO | H | 1Me-5-Ind | C | | 320 (M⁺+1) |
| Int.B-59 | f-1 | Int.B-26 | Hal3 | nPrO | H | 1Et-5-Ind | C | | 334 (M⁺+1 |
| Int.B-60 | f-1 | Int.B-30 | Hal3 | nPrO | H | 1 Et-5-Idz | C | | 335 (M⁺+1) |
| Int.B-61 | f-1 | Int.B-32 | Hal3 | nPrO | H | 6-Qu | C | | 318(M⁺+1 |
| Int.B-62 | f-1 | Int.B-24 | Hal4 | iPrO | H | 2-Nap | C | | 317 (M⁺+1) |
| Int.B-63 | f-1 | Int.B-26 | Hal4 | iPrO | H | 1 Et-5-Ind | C | | 334 (M⁺+1) |
| Int.B-64 | f-1 | Int.B-29 | Hal4 | iPrO | H | 1Me-5-Idz | C | | 321 (M⁺+1) |
| Int.B-65 | f-1 | Int.B-33 | Hal4 | iPrO | H | 6-IQ | C | | 318 (M⁺+1) |
| Int.B-66 | f-1 | Int.B-24 | Hal5 | nBuO | H | 2-Nap | C | | 331 (M⁺+1) |
| Int.B-67 | f-1 | Int.B-25 | Hal5 | nBuO | H | 1 Me-5-Ind | C | | 334 (M⁺+1) |
| Int.B-68 | f-1 | Int.B-30 | Hal5 | nBuO | H | 1 Et-5-Idz | C | | 349 (M⁺+1) |
| Int.B-69 | f-1 | Int.B-31 | Hal5 | nBuO | H | 5-B F | C | | 321 (M⁺+1) |

**[Table 46]**

| Table-IntB-3 | | | | | | | | | |
|---|---|---|---|---|---|---|---|---|---|
| Exp. | Syn. | SM | Reagent | Rx | V1' | V2' | LCMS | | |
| | | | | | | | method | RTime | Mass |
| Int.B-70 | f-1 | Int.B-24 | Hal6 | iBuO | H | 2-Nap | C | | 331 (M⁺+1) |
| Int.B-71 | f-1 | Int.B-25 | Hal6 | iBuO | H | 1Me-5-Ind | C | | 334 (M⁺+1) |
| Int.B-72 | f-1 | Int.B-26 | Hal6 | iBuO | H | 1Et-5-Ind | C | | 348 (M⁺+1) |
| Int.B-73 | f-1 | Int.B-29 | Hal6 | iBuO | H | 1Me-5-Idz | C | | 335 (M⁺+1) |
| Int.B-74 | f-1 | Int.B-30 | Hal6 | iBuO | H | 1Et-5-Idz | C | | 349 (M⁺+1) |
| Int.B-75 | f-1 | Int.B-24 | Hal7 | 2FBnO | H | 2-Nap | C | | 383 (M⁺+1) |
| Int.B-76 | f-1 | Int.B-25 | Hal7 | 2FBnO | H | 1Me-5-Ind | C | | 386 (M⁺+1) |
| Int.B-77 | f-1 | Int.B-26 | Hal7 | 2FBnO | H | 1Et-5-Ind | C | | 400 (M⁺+1) |
| Int.B-78 | f-1 | Int.B-29 | Hal7 | 2FBnO | H | 1Me-5-Idz | C | | 387(M⁺+1) |
| Int.B-79 | f-1 | Int.B-30 | Hal7 | 2FBnO | H | 1Et-5-Idz | C | | 401 (M⁺+1) |
| Int.B-80 | f-1 | Int.B-32 | Hal7 | 2FBnO | H | 6-Qu | C | | 384 (M⁺+1) |
| Int.B-81 | f-1 | Int.B-33 | Hal7 | 2FBnO | H | 6-IQ | C | | 384 (M⁺+1) |
| Int.B-82 | f-1 | Int.B-24 | Hal8 | 3FBnO | H | 2-Nap | C | | 383 (M⁺+1) |
| Int.B-83 | f-1 | Int.B-26 | Hal8 | 3FBnO | H | 1Et-5-Ind | C | | 400 (M⁺+1) |
| Int.B-84 | f-1 | Int.B-28 | Hal8 | 3FBnO | H | 1Me-6-Ind | C | | 386 (M⁺+1) |
| Int.B-85 | f-1 | Int.B-29 | Hal8 | 3FBnO | H | 1Me-5-Idz | C | | 387 (M⁺+1) |
| Int.B-86 | f-1 | Int.B-31 | Hal8 | 3FBnO | H | 5-BF | C | | 373 (M⁺+1) |
| Int.B-87 | f-1 | Int.B-32 | Hal8 | 3FBnO | H | 6-Qu | C | | 384 (M⁺+1) |
| Int.B-88 | f-1 | Int.B-25 | Hal9 | 4FBnO | H | 1Me-5-Ind | C | | 386 (M⁺+1) |
| Int.B-89 | f-1 | Int.B-27 | Hal9 | 4FBnO | H | 1Me-4-Ind | C | | 386 (M⁺+1) |
| Int.B-90 | f-1 | Int.B-28 | Hal9 | 4FBnO | H | 1Me-6-Ind | C | | 386 (M⁺+1) |
| Int.B-91 | f-1 | Int.B-30 | Hal9 | 4FBnO | H | 1Et-5-Idz | C | | 401 (M⁺+1) |
| Int.B-92 | f-1 | Int.B-32 | Hal9 | 4FBnO | H | 6-Qu | C | | 384(M⁺+1) |
| Int.B-93 | f-1 | Int.B-24 | Hal10 | 2ClBnO | H | 2-Nap | C | | 399(M⁺+1) |
| Int.B-94 | f-1 | Int.B-26 | Hal10 | 2ClBnO | H | 1Et-5-Ind | C | | 416 (M⁺+1) |
| Int.B-95 | f-1 | Int.B-27 | Hal10 | 2ClBnO | H | 1Me-4-Ind | C | | 402 (M⁺+1) |
| Int.B-96 | f-1 | Int.B-28 | Hal10 | 2ClBnO | H | 1Me-6-Ind | C | | 402 (M⁺+1) |
| Int.B-97 | f-1 | Int.B-30 | Hal10 | 2ClBnO | H | 1Et-5-Idz | C | | 417 (M⁺+1) |
| Int.B-98 | f-1 | Int.B-24 | Hal11 | 3ClBnO | H | 2-Nap | C | | 399 (M⁺+1) |
| Int.B-99 | f-1 | Int.B-25 | Hal11 | 3ClBnO | H | 1Me-5-Ind | C | | 402 (M⁺+1) |
| Int.B-100 | f-1 | Int.B-26 | Hal11 | 3ClBnO | H | 1Et-5-Ind | C | | 416 (M⁺+1) |
| Int.B-101 | f-1 | Int.B-29 | Hal11 | 3ClBnO | H | 1Me-5-Idz | C | | 403 (M⁺+1) |
| IntB-102 | f-1 | Int.B-30 | Hal11 | 3ClBnO | H | 1Et-5-Idz | C | | 417 (M⁺+1) |
| IntB-103 | f-1 | Int.B-31 | Hal11 | 3ClBnO | H | 5-BF | C | | 389 (M⁺+1) |
| IntB-104 | f-1 | Int.B-32 | Hal11 | 3ClBnO | H | 6-Qu | C | | 400 (M⁺+1) |
| Int.B-105 | f-1 | Int.B-24 | Hal12 | 4MeBnO | H | 2-Nap | C | | 379 (M⁺+1) |
| Int.B-106 | f-1 | Int.B-25 | Hal12 | 4MeBnO | H | 1Me-5-Ind | C | | 382 (M⁺+1) |
| Int.B-107 | f-1 | Int.B-26 | Hal12 | 4MeBnO | H | 1Et-5-Ind | C | | 396 (M⁺+1) |
| Int.B-108 | f-1 | Int.B-28 | Hal12 | 4MeBnO | H | 1Me-6-Ind | C | | 382 (M⁺+1) |
| IntB-109 | f-1 | Int.B-29 | Hal12 | 4MeBnO | H | 1Me-5-Idz | C | | 383 (M⁺+1) |
| Int.B-110 | f-1 | Int.B-33 | Hal12 | 4MeBnO | H | 6-IQ | C | | 380 (M⁺+1) |
| Int.B-111 | f-1 | Int.B-25 | Hal13 | 4CF3BnO | H | 1Me-5-Ind | C | | 435 (M⁺+1) |
| Int.B-112 | f-1 | Int.B-27 | Hal13 | 4CF3BnO | H | 1Me-4-Ind | C | | 435 (M⁺+1) |
| Int.B-113 | f-1 | Int.B-29 | Hal13 | 4CF3BnO | H | 1Me-5-Idz | C | | 436 (M⁺+1) |
| Int.B-114 | f-1 | Int.B-30 | Hal13 | 4CF3BnO | H | 1Et-5-Idz | C | | 450 (M⁺+1) |
| Int.B-115 | f-1 | Int.B-32 | Hal13 | 4CF3BnO | H | 6-Qu | C | | 433 (M⁺+1) |

**[Table 47]**

| Table-Int.B-4 | | | | | | | | | |
|---|---|---|---|---|---|---|---|---|---|
| Exp. | Syn. | SM | Reagent | Rx | V1' | V2' | LCMS | | |
| | | | | | | | method | RTime | Mass |
| Int.B-116 | f-2 | Int.B-24 | Al5 | 2EtBuO | H | 2-Nap | C | | 359 (M⁺+1) |
| Int.B-117 | f-2 | Int.B-25 | Al5 | 2EtBuO | H | 1Me-5-Ind | C | | 362 (M⁺+1) |
| Int.B-118 | f-2 | Int.B-26 | Al5 | 2EtBuO | H | 1Et-5-Ind | C | | 376 (M⁺+1) |
| Int.B-119 | f-2 | Int.B-29 | Al5 | 2EtBuO | H | 1Me-5-Idz | C | | 363 (M⁺+1) |
| Int.B-120 | f-2 | Int.B-30 | Al5 | 2EtBuO | H | 1Et-5-Idz | C | | 377 (M⁺+1) |
| Int.B-121 | f-2 | Int.B-32 | Al5 | 2EtBuO | H | 6-Qu | C | | 360 (M⁺+1) |
| IntB-122 | f-2 | Int.B-33 | Al5 | 2EtBuO | H | 6-IQ | C | | 360 (M⁺+1) |
| IntB-123 | f-2 | Int.B-24 | Al6 | 2(4DMAPh)EtO | H | 2-Nap | C | | 422 (M⁺+1) |
| IntB-124 | f-2 | Int.B-25 | Al6 | 2(4DMAPh)EtO | H | 1Me-5-Ind | C | | 425 (M⁺+1) |
| IntB-125 | f-2 | Int.B-27 | Al6 | 2(4DMAPh)EtO | H | 1Me-4-Ind | C | | 425 (M⁺+1) |
| IntB-126 | f-2 | Int.B-30 | Al6 | 2(4DMAPh)EtO | H | 1 Et-5-Idz | C | | 440 (M⁺+1) |
| IntB-127 | f-2 | Int.B-31 | Al6 | 2(4DMAPh)EtO | H | 5-BF | C | | 412 (M⁺+1) |
| IntB-128 | f-2 | Int.B-32 | Al6 | 2(4DMAPh)EtO | H | 6-Qu | C | | 423 (M⁺+1) |
| IntB-129 | f-2 | Int.B-26 | Al7 | 2(PhO)EtO | H | 1Et-5-Ind | C | | 412 (M⁺+1) |
| IntB-130 | f-2 | Int.B-27 | Al7 | 2(PhO)EtO | H | 1Me-4-Ind | C | | 398 (M⁺+1) |
| Int.B-131 | f-2 | Int.B-28 | Al7 | 2(PhO)EtO | H | 1Me-6-Ind | C | | 398 (M⁺+1) |
| IntB-132 | f-2 | Int.B-29 | Al7 | 2(PhO)EtO | H | 1Me-5-Idz | C | | 399 (M⁺+1) |
| IntB-133 | f-2 | Int.B-33 | Al7 | 2(PhO)EtO | H | 6-IQ | C | | 306 (M⁺+1) |
| Int.B-134 | f-2 | Int.B-24 | Al8 | 1PhEtO | H | 2-Nap | C | | 379 (M⁺+1) |
| IntB-135 | f-2 | Int.B-25 | Al8 | 1PhEtO | H | 1Me-5-Ind | C | | 382 (M⁺+1) |
| IntB-136 | f-2 | Int.B-26 | Al8 | 1PhEtO | H | 1Et-5-Ind | C | | 396 (M⁺+1) |
| IntB-137 | f-2 | Int.B-29 | Al8 | 1PhEtO | H | 1Me-5-Idz | C | | 383 (M⁺+1) |
| IntB-138 | f-2 | Int.B-30 | Al8 | 1PhEtO | H | 1Et-5-Idz | C | | 397 (M⁺+1) |
| IntB-139 | f-2 | Int.B-24 | Al9 | 1(2FPh)EtO | H | 2-Nap | C | | 397 (M⁺+1) |
| IntB-140 | f-2 | Int.B-26 | Al9 | 1(2FPh)EtO | H | 1Et-5-Ind | C | | 414 (M⁺+1) |
| Int.B-141 | f-2 | Int.B-28 | Al9 | 1(2FPh)EtO | H | 1Me-6-Ind | C | | 400 (M⁺+1) |
| Int.B-142 | f-2 | Int.B-29 | Al9 | 1(2FPh)EtO | H | 1Me-5-Idz | C | | 401 (M⁺+1) |
| IntB-143 | f-2 | Int.B-30 | Al9 | 1(2FPh)EtO | H | 1Et-5-Idz | C | | 415 (M⁺+1) |
| Int.B-144 | f-2 | Int.B-33 | Al9 | 1(2FPh)EtO | H | 6-IQ | C | | 398 (M⁺+1) |
| Int.B-145 | f-2 | Int.B-25 | Al10 | 1(4ClPh)EtO | H | 1Me-5-Ind | C | | 416 (M⁺+1) |
| Int.B-146 | f-2 | Int.B-27 | Al10 | 1(4ClPh)EtO | H | 1Me-4-Ind | C | | 416 (M⁺+1) |
| Int.B-147 | f-2 | Int.B-28 | Al10 | 1(4ClPh)EtO | H | 1Me-6-Ind | C | | 416 (M⁺+1) |
| IntB-148 | f-2 | Int.B-29 | Al10 | 1(4ClPh)EtO | H | 1Me-5-Idz | C | | 417 (M⁺+1) |
| IntB-149 | f-2 | Int.B-30 | Al10 | 1(4ClPh)EtO | H | 1Et-5-Idz | C | | 431 (M⁺+1) |
| IntB-150 | f-2 | Int.B-31 | Al10 | 1(4ClPh)EtO | H | 5-BF | C | | 403 (M⁺+1) |
| Int.B-151 | f-2 | Int.B-24 | Al11 | 4Me,cHexO | H | 2-Nap | C | | 371 (M⁺+1) |
| IntB-152 | f-2 | Int.B-25 | Al11 | 4Me,cHexO | H | 1Me-5-Ind | C | | 374 (M⁺+1) |
| IntB-153 | f-2 | Int.B-26 | Al11 | 4Me,cHexO | H | 1Et-5-Ind | C | | 388 (M⁺+1) |
| IntB-154 | f-2 | Int.B-29 | Al11 | 4Me,cHexO | H | 1Me-5-Idz | C | | 375 (M⁺+1) |
| IntB-155 | f-2 | Int.B-30 | Al11 | 4Me,cHexO | H | 1Et-5-Idz | C | | 389 (M⁺+1) |
| IntB-156 | f-2 | Int.B-32 | Al11 | 4Me,cHexO | H | 6-Qu | C | | 372 (M⁺+1) |
| IntB-157 | f-2 | Int.B-24 | Al17 | 1IndanO | H | 2-Nap | C | | 391 (M⁺+1) |
| IntB-158 | f-2 | Int.B-25 | Al17 | 1IndanO | H | 1Me-5-Ind | C | | 394 (M⁺+1) |
| IntB-159 | f-2 | Int.B-27 | Al17 | 1IndanO | H | 1Me-4-Ind | C | | 394 (M⁺+1) |
| IntB-160 | f-2 | Int.B-28 | Al17 | 1IndanO | H | 1Me-6-Ind | C | | 394 (M⁺+1) |
| Int.B-161 | f-2 | Int.B-29 | Al17 | 1IndanO | H | 1Me-5-Idz | C | | 395 (M⁺+1) |

**[Table 48]**

| Table-Int.B-5 | | | | | | | | | |
|---|---|---|---|---|---|---|---|---|---|
| Exp. | Syn. | SM | Reagent | Rx | V1' | V2' | LCMS | | |
| | | | | | | | method | RTime | Mass |
| Int.B-162 | f-2 | Int.B-30 | Al17 | 1IndanO | H | 1Et-5-Idz | C | | 409 (M⁺+1) |
| Int.B-163 | f-2 | Int.B-31 | Al17 | 1IndanO | H | 5-B F | C | | 381 (M⁺+1) |
| Int.B-164 | f-2 | Int.B-32 | Al17 | 1IndanO | H | 6-Qu | C | | 392 (M⁺+1) |
| Int.B-165 | f-2 | Int.B-24 | Al18 | 2IndanO | H | 2-Nap | C | | 391 (M⁺+1) |
| Int.B-166 | f-2 | Int.B-25 | Al18 | 2IndanO | H | 1Me-5-Ind | C | | 394 (M⁺+1) |
| Int.B-167 | f-2 | Int.B-26 | Al18 | 2IndanO | H | 1Et-5-Ind | C | | 408 (M⁺+1) |
| Int.B-168 | f-2 | Int.B-29 | Al18 | 2IndanO | H | 1Me-5-Idz | C | | 395 (M⁺+1) |
| Int.B-169 | f-2 | Int.B-30 | Al18 | 2IndanO | H | 1Et-5-Idz | C | | 409 (M⁺+1) |
| Int.B-170 | f-2 | Int.B-33 | Al18 | 2IndanO | H | 6-IQ | C | | 392 (M⁺+1) |
| Int.B-171 | f-2 | Int.B-24 | Al19 | 5OMe-2-IndanO | H | 2-Nap | C | | 421 (M⁺+1) |
| Int.B-172 | f-2 | Int.B-26 | Al19 | 5OMe-2-IndanO | H | 1Et-5-Ind | C | | 438 (M⁺+1) |
| Int.B-173 | f-2 | Int.B-29 | Al19 | 5OMe-2-IndanO | H | 1Me-5-Idz | C | | 425 (M⁺+1) |
| Int.B-174 | f-2 | Int.B-32 | Al19 | 5OMe-2-IndanO | H | 6-Qu | C | | 422 (M⁺+1) |
| Int.B-175 | f-2 | Int.B-25 | Al20 | 5,6D(OMe)-2-IndanO | H | 1Me-5-Ind | C | | 454 (M⁺+1) |
| Int.B-176 | f-2 | Int.B-27 | Al20 | 5,6D(OMe)-2-IndanO | H | 1Me-4-Ind | C | | 454 (M⁺+1) |
| Int.B-177 | f-2 | Int.B-28 | Al20 | 5,6D(OMe)-2-IndanO | H | 1Me-6-Ind | C | | 454 (M⁺+1) |
| Int.B-178 | f-2 | Int.B-29 | Al20 | 5,6D(OMe)-2-IndanO | H | 1Me-5-Idz | C | | 455 (M⁺+1) |
| Int.B-179 | f-2 | Int.B-33 | Al20 | 5,6D(OMe)-2-IndanO | H | 6-IQ | C | | 452 (M⁺+1) |
| Int.B-180 | f-2 | Int.B-24 | Al21 | 5F-2-IndanO | H | 2-Nap | C | | 409 (M⁺+1) |
| Int.B-181 | f-2 | Int.B-25 | Al21 | 5F-2-IndanO | H | 1Me-5-Ind | C | | 412 (M⁺+1) |
| Int.B-182 | f-2 | Int.B-26 | Al21 | 5F-2-IndanO | H | 1Et-5-Ind | C | | 426 (M⁺+1) |
| Int.B-183 | f-2 | Int.B-29 | Al21 | 5F-2-IndanO | H | 1Me-5-Idz | C | | 413 (M⁺+1) |
| Int.B-184 | f-2 | Int.B-30 | Al21 | 5F-2-IndanO | H | 1Et-5-Idz | C | | 427 (M⁺+1) |
| Int.B-185 | f-2 | Int.B-32 | Al21 | 5F-2-IndanO | H | 6-Qu | C | | 410 (M⁺+1) |
| Int.B-186 | f-2 | Int.B-33 | Al21 | 5F-2-IndanO | H | 6-IQ | C | | 410 (M⁺+1) |
| Int.B-187 | f-2 | Int.B-24 | Al22 | 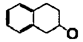 | H | 2-Nap | C | | 405 (M⁺+1) |
| Int.B-188 | f-2 | Int.B-25 | Al22 | 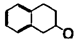 | H | 1Me-5-Ind | C | | 408 (M⁺+1) |
| Int.B-189 | f-2 | Int.B-28 | Al22 | 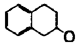 | H | 1Me-6-Ind | C | | 408 (M⁺+1) |
| Int.B-190 | f-2 | Int.B-29 | Al22 | 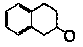 | H | 1Me-5-Idz | C | | 409 (M⁺+1) |
| Int.B-191 | f-2 | Int.B-32 | Al22 | 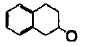 | H | 6-Qu | C | | 423 (M⁺+1) |
| Int.B-192 | f-2 | Int.B-25 | Al23 | 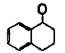 | H | 1Me-5-Ind | C | | 408 (M⁺+1) |
| Int.B-193 | f-2 | Int.B-26 | Al23 | 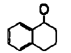 | H | 1Et-5-Ind | C | | 422 (M⁺+1) |
| Int.B-194 | f-2 | Int.B-27 | Al23 | 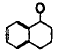 | H | 1Me-4-Ind | C | | 408 (M⁺+1) |
| Int.B-195 | f-2 | Int.B-30 | Al23 | 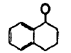 | H | 1Et-5-Idz | C | | 423 (M⁺+1) |
| Int.B-196 | f-2 | Int.B-31 | Al23 | 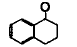 | H | 5-BF | C | | 395 (M⁺+1) |
| Int.B-197 | f-2 | Int.B-24 | Al24 | 2(2MePh)EtO | H | 2-Nap | C | | 393 (M⁺+1) |

**[Table 49]**

| Table-Int.B-6 | | | | | | | | | |
|---|---|---|---|---|---|---|---|---|---|
| Exp. | Syn. | SM | Reagent | Rx | V1' | V2' | LCMS | | |
| | | | | | | | method | RTime | Mass |
| Int.B-198 | f-2 | Int.B-25 | Al24 | 2(2MePh)EtO | H | 1Me-5-Ind | C | | 396 (M⁺+1) |
| Int.B-199 | f-2 | Int.B-26 | Al24 | 2(2MePh)EtO | H | 1Et-5-Ind | C | | 410 (M⁺+1) |
| Int.B-200 | f-2 | Int.B-30 | Al24 | 2(2MePh)EtO | H | 1Et-5-Idz | C | | 410 (M⁺+1) |
| Int.B-201 | f-2 | Int.B-32 | Al24 | 2(2MePh)EtO | H | 6-Qu | C | | 394 (M⁺+1) |
| Int.B-202 | f-2 | Int.B-24 | Al25 | 2(3FPh)EtO | H | 2-Nap | C | | 397 (M⁺+1) |
| Int.B-203 | f-2 | Int.B-25 | Al25 | 2(3FPh)EtO | H | 1Me-5-Ind | C | | 400 (M⁺+1) |
| Int.B-204 | f-2 | Int.B-27 | Al25 | 2(3FPh)EtO | H | 1Me-4-Ind | C | | 400 (M⁺+1) |
| Int.B-205 | f-2 | Int.B-30 | Al25 | 2(3FPh)EtO | H | 1Et-5-Idz | C | | 414 (M⁺+1) |
| Int.B-206 | f-2 | Int.B-33 | Al25 | 2(3FPh)EtO | H | 6-IQ | C | | 398 (M⁺+1) |
| Int.B-207 | f-2 | Int.B-26 | Al26 | 2(2ClPh)EtO | H | 1Et-5-Ind | C | | 430 (M⁺+1) |
| Int.B-208 | f-2 | Int.B-27 | Al26 | 2(2ClPh)EtO | H | 1Me-4-Ind | C | | 416 (M⁺+1) |
| Int.B-209 | f-2 | Int.B-28 | Al26 | 2(2ClPh)EtO | H | 1Me-6-Ind | C | | 416 (M⁺+1) |
| IntB-210 | f-2 | Int.B-30 | Al26 | 2(2ClPh)EtO | H | 1Et-5-Idz | C | | 430 (M⁺+1) |
| Int.B-211 | f-2 | Int.B-31 | Al26 | 2(2ClPh)EtO | H | 5-BF | C | | 403 (M⁺+1) |
| Int.B-212 | f-2 | Int.B-24 | Al28 | 2(1-Nap)EtO | H | 2-Nap | C | | 429 (M⁺+1) |
| Int.B-213 | f-2 | Int.B-25 | Al28 | 2(1-Nap)EtO | H | 1Me-5-Ind | C | | 432 (M⁺+1) |
| Int.B-214 | f-2 | Int.B-29 | Al28 | 2(1-Nap)EtO | H | 1Me-5-Idz | C | | 433 (M⁺+1) |
| Int.B-215 | f-2 | Int.B-30 | Al28 | 2(1-Nap)EtO | H | 1Et-5-Idz | C | | 446 (M⁺+1) |
| Int.B-216 | f-2 | Int.B-33 | Al28 | 2(1-Nap)EtO | H | 6-IQ | C | | 430 (M⁺+1) |
| Int.B-217 | f-2 | Int.B-25 | Al30 | 2(PhS)EtO | H | 1Me-5-Ind | C | | 414 (M⁺+1) |
| Int.B-218 | f-2 | Int.B-28 | Al30 | 2(PhS)EtO | H | 1Me-6-Ind | C | | 414 (M⁺+1) |
| Int.B-219 | f-2 | Int.B-30 | Al30 | 2(PhS)EtO | H | 1Et-5-Idz | C | | 428 (M⁺+1) |
| Int.B-220 | f-2 | Int.B-32 | Al30 | 2(PhS)EtO | H | 6-Qu | C | | 412 (M⁺+1) |

### Example B-a-1

### Synthesis of ethyl 2-[5-cyclopentyloxy-6-(naphthalen-2-yl)-2,3-dihydroinden-1-ylidene]acetate (Compound No. B-a-1) (Preparation Method 10, Step k-1)

According to the procedure described in the synthetic method of Intermediate A-2 in Reference Example 1 (Preparation Method 9, Step k-1) provided that the reaction was performed for 18.5 hours under a reflux condition, Intermediate B-2 (68.1 mg), ethyl diethylphosphonoacetate (1.5 ml, TCI) and sodium hydride (152.2 mg, WAKO) were reacted and treated to obtain the title compound (Compound No. B-a-1, 26.7 mg).

### Example B-a-2

### Synthesis of 2-[5-cyclopentyloxy-6-(naphthalen-2-yl)-2,3-dihydroinden-1-ylidene]acetic acid (Compound No. B-a-2) (Preparation Method 1, Step a)

According to the procedure described in the synthetic method of Example Compound A-a-2 (Preparation Method 1, Step a) provided that the reaction was performed for 3 hours, Example Compound B-a-1 (26.7 mg) and 2 N aqueous sodium hydroxide (0.20 ml) were reacted and treated to obtain the title compound (Compound No. B-a-2, 22.5 mg).

Typical examples of the compounds of the present invention that can be obtained by reacting and treating corresponding starting compounds using any of the methods described in the present specification including the examples described above are shown in Table-B-a-1 to Table-B-a-10. The compounds were synthesized according to the preparation methods of the compounds of the compound numbers shown in the columns of "Syn" in the tables. "Int" means an intermediate compound number. In the tables, used methods among the aforementioned synthetic methods are shown in the columns of "Syn" with symbols, the starting compounds are shown in the columns of "SM", and the reagents are shown in the columns of "Regent".

**[Table 50]**

| Table-B-a-1 | | | | | | | | | | |
|---|---|---|---|---|---|---|---|---|---|---|
| 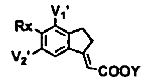 | | | | | | | | | | |

| Exp. | Syn. | SM | Reagent | Rx | Y | V1' | V2' | LCMS | | |
|---|---|---|---|---|---|---|---|---|---|---|
| | | | | | | | | method | RTime | Mass |
| B-a-1 | 10k-1 | Int.B-2 | | cPenO | Et | H | 2-Nap | C | | 413(M⁺+1) |
| B-a-2 | 1-a | B-a-1 | | cPenO | H | H | 2-Nap | C | | 385(M⁺+1) |
| B-a-3 | 10k-1 | Int.B-3 | | cPenO | Et | H | 1Me-5-Ind | C | | 416(M⁺+1) |
| B-a-4 | 1-a | B-a-3 | | cPenO | H | H | 1Me-5-Ind | C | | 388(M⁺+1) |
| B-a-5 | 10k-1 | Int.B-4 | | cPenO | Et | H | 1Et-5-Ind | C | | 430(M⁺+1) |
| B-a-6 | 1-a | B-a-5 | | cPenO | H | H | 1Et-5-Ind | C | | 302(M⁺+1) |
| B-a-7 | 10k-1 | Int.B-5 | | cPenO | Et | H | 1Me-4-Ind | C | | 416(M⁺+1) |
| B-a-8 | 1-a | B-a-7 | | cPenO | H | H | 1Me-4-Ind | C | | 388(M⁺+1) |
| B-a-9 | 10k-1 | Int.B-6 | | cPenO | Et | H | 1Me-6-Ind | C | | 416(M⁺+1) |
| B-a-10 | 1-a | B-a-9 | | cPenO | H | H | 1Me-6-Ind | C | | 388(M⁺+1) |
| B-a-11 | 10k-1 | Int.B-7 | | cPenO | Et | H | 1Me-5-Idz | C | | 417M⁺+1) |
| B-a-12 | 1-a | B-a-11 | | cPenO | H | H | 1Me-5-Idz | C | | 389(M⁺+1) |
| B-a-13 | 10k-1 | Int.B-8 | | cPenO | Et | H | 1Et-5-Idz | C | | 431(M⁺+1) |
| B-a-14 | 1-a | B-a-13 | | cPenO | H | H | 1Et-5-Idz | C | | 403(M⁺+1) |
| B-a-15 | 10k-1 | Int.B-9 | | cPenO | Et | H | 5-B F | C | | 403(M⁺+1) |
| B-a-16 | 1-a | B-a-15 | | cPenO | H | H | 5-B F | C | | 375(M⁺+1) |
| B-a-17 | 10k-1 | Int.B-10 | | cPenO | Et | H | 6-Qu | C | | 414(M⁺+1) |
| B-a-18 | 1-a | B-a-17 | | cPenO | H | H | 6-Qu | C | | 386(M⁺+1) |
| B-a-19 | 10k-1 | Int.B-11 | | cPenO | Et | H | 6-IQ | C | | 414(M⁺+1) |
| B-a-20 | 1-a | B-a-19 | | cPenO | H | H | 6-IQ | C | | 386(M⁺+1) |
| B-a-21 | 10k-1 | IntB-14 | | BnO | Et | H | 2-Nap | C | | 435(M⁺+1) |
| B-a-22 | 1-a | B-a-21 | | BnO | H | H | 2-Nap | C | | 407(M⁺+1) |
| B-a-23 | 10k-1 | Int.B-15 | | BnO | Et | H | 1Me-5-Ind | C | | 438(M⁺+1) |
| B-a-24 | 1-a | B-a-23 | | BnO | H | H | 1Me-5-Ind | C | | 410(M⁺+1) |
| B-a-25 | 10k-1 | Int.B-16 | | BnO | Et | H | 1Et-5-Ind | C | | 452(M⁺+1) |
| B-a-26 | 1-a | B-a-25 | | BnO | H | H | 1Et-5-Ind | C | | 424(M⁺+1) |
| B-a-27 | 10k-1 | Int.B-17 | | BnO | Et | H | 1Me-4-Ind | C | | 438(M⁺+1) |
| B-a-28 | 1-a | B-a-27 | | BnO | H | H | 1Me-4-Ind | C | | 410(M⁺+1) |
| B-a-29 | 10k-1 | Int.B-18 | | BnO | Et | H | 1Me-6-Ind | C | | 438(M⁺+1) |
| B-a-30 | 1-a | B-a-29 | | BnO | H | H | 1Me-6-Ind | C | | 410(M⁺+1) |
| B-a-31 | 10k-1 | Int.B-19 | | BnO | Et | H | 1Me-5-Idz | C | | 439(M⁺+1) |
| B-a-32 | 1-a | B-a-31 | | BnO | H | H | 1Me-5-Idz | C | | 411(M⁺+1) |
| B-a-33 | 10k-1 | Int.B-20 | | BnO | Et | H | 1Et-5-Idz | C | | 453(M⁺+1) |
| B-a-34 | 1-a | B-a-33 | | BnO | H | H | 1Et-5-Idz | C | | 425(M⁺+1) |
| B-a-35 | 10k-1 | Int.B-21 | | BnO | Et | H | 5-BF | C | | 425(M⁺+1) |
| B-a-36 | 1-a | B-a-35 | | BnO | H | H | 5-BF | C | | 397(M⁺+1) |
| B-a-37 | 10k-1 | Int B-22 | | BnO | Et | H | 6-Qu | C | | 436(M⁺+1) |
| B-a-38 | 1-a | B-a-37 | | BnO | H | H | 6-Qu | C | | 408(M⁺+1) |
| B-a-39 | 10k-1 | Int.B-23 | | BnO | Et | H | 6-IQ | C | | 436(M⁺+1) |
| B-a-40 | 1-a | B-a-39 | | BnO | H | H | 6-IQ | C | | 408(M⁺+1) |
| B-a-41 | 10k-1 | Int.B-34 | | cPenMeO | Et | H | 2-Nap | C | | 427(M⁺+1) |
| B-a-42 | 1-a | B-a-41 | | cPenMeO | H | H | 2-Nap | C | | 399(M⁺+1) |
| B-a-43 | 10k-1 | Int.B-35 | | cPenMeO | Et | H | 1Me-5-Ind | C | | 430(M⁺+1) |
| B-a-44 | 1a | B-a-43 | | cPenMeO | H | H | 1Me-5-Ind | C | | 402(M⁺+1) |

**[Table 51]**

| Table-B-a-2 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-a-45 | 10k-1 | Int.B-36 | | cPenMeO | Et | H | 1Me-4-Ind | C | | 430(M⁺+1) |
| B-a-46 | 1-a | B-a-45 | | cPenMeO | H | H | 1Me-4-Ind | C | | 402(M⁺+1) |
| B-a-47 | 10k-1 | Int.B-37 | | cPenMeO | Et | H | 1Me-5-Idz | C | | 431(M⁺+1) |
| B-a-48 | 1-a | B-a-47 | | cPenMeO | H | H | 1Me-5-Idz | C | | 403(M⁺+1) |
| B-a-49 | 10k-1 | Int.B-38 | | cPenMeO | Et | H | 1Et-5-Idz | C | | 445(M⁺+1) |
| B-a-50 | 1-a | B-a-49 | | cPenMeO | H | H | 1Et-5-Idz | C | | 417(M⁺+1) |
| B-a-51 | 10k-1 | Int.B-39 | | cPenMeO | Et | H | 6-Qu | C | | 428(M⁺+1) |
| B-a-52 | 1-a | B-a-51 | | cPenMeO | H | H | 6-Qu | C | | 400(M⁺+1) |
| B-a-53 | 10k-1 | Int.B-40 | | cHexMeO | Et | H | 2-Nap | C | | 441(M⁺+1) |
| B-a-54 | 1-a | B-a-53 | | cHexMeO | H | H | 2-Nap | C | | 413(M⁺+1) |
| B-a-55 | 10k-1 | Int.B-41 | | cHexMeO | Et | H | 1Et-5-Ind | C | | 458M⁺+1) |
| B-a-56 | 1-a | B-a-55 | | cHexMeO | H | H | 1Et-5-Ind | C | | 430(M⁺+1) |
| B-a-57 | 10k-1 | Int.B-42 | | cHexMeO | Et | H | 1Me-6-Ind | C | | 444(M⁺+1) |
| B-a-58 | 1-a | B-a-57 | | cHexMeO | H | H | 1Me-6-Ind | C | | 416(M⁺+1) |
| B-a-59 | 10k-1 | Int.B-43 | | cHexMeO | Et | H | 1Me-5-Idz | C | | 445(M⁺+1) |
| B-a-60 | 1-a | B-a-59 | | cHexMeO | H | H | 1Me-5-Idz | C | | 417(M⁺+1) |
| B-a-61 | 10k-1 | Int.B-44 | | cHexMeO | Et | H | 1Et-5-Idz | C | | 459(M⁺+1) |
| B-a-62 | 1-a | B-a-61 | | cHexMeO | H | H | 1Et-5-Idz | C | | 431(M⁺+1) |
| B-a-63 | 10k-1 | Int.B-45 | | cHexMeO | Et | H | 5-BF | C | | 431(M⁺+1) |
| B-a-64 | 1-a | B-a-63 | | cHexMeO | H | H | 5-BF | C | | 403(M⁺+1) |
| B-a-65 | 10k-1 | Int.B-46 | | cHexMeO | Et | H | 6-IQ | C | | 442(M⁺+1) |
| B-a-66 | 1-a | B-a-65 | | cHexMeO | H | H | 6-IQ | C | | 414(M⁺+1) |
| B-a-67 | 10k-1 | Int.B-47 | | cHexO | Et | H | 2-Nap | C | | 427(M⁺+1) |
| B-a-68 | 1-a | B-a-67 | | cHexO | H | H | 2-Nap | C | | 399(M⁺+1) |
| B-a-69 | 10k-1 | Int.B-48 | | cHexO | Et | H | 1Me-5-Ind | C | | 430(M⁺+1) |
| B-a-70 | 1-a | B-a-69 | | cHexO | H | H | 1Me-5-Ind | C | | 402(M⁺+1) |
| B-a-71 | 10k-1 | Int.B-49 | | cHexO | Et | H | 1Et-5-Ind | C | | 444(M⁺+1) |
| B-a-72 | 1-a | B-a-71 | | cHexO | H | H | 1Et-5-Ind | C | | 416(M⁺+1) |
| B-a-73 | 10k-1 | Int.B-50 | | cHexO | Et | H | 1Me-4-Ind | C | | 430(M⁺+1) |
| B-a-74 | 1-a | B-a-73 | | cHexO | H | H | 1Me-4-Ind | C | | 402(M⁺+1) |
| B-a-75 | 10k-1 | Int.B-51 | | cHexO | Et | H | 1Me-5-Idz | C | | 431(M⁺+1) |
| B-a-76 | 1-a | B-a-75 | | cHexO | H | H | 1Me-5-Idz | C | | 403(M⁺+1) |
| B-a-77 | 10k-1 | Int.B-52 | | cHexO | Et | H | 1Et-5-Idz | C | | 445(M⁺+1) |
| B-a-78 | 1-a | B-a-77 | | cHexO | H | H | 1Et-5-Idz | C | | 417(M⁺+1) |
| B-a-79 | 10k-1 | Int.B-53 | | cHexO | Et | H | 6-Qu | C | | 428(M⁺+1) |
| B-a-80 | 1-a | B-a-79 | | cHexO | H | H | 6-Qu | C | | 400(M⁺+1) |
| B-a-81 | 10k-1 | Int.B-54 | | cHepO | Et | H | 2-Nap | C | | 441(M⁺+1) |
| B-a-82 | 1-a | B-a-81 | | cHepO | H | H | 2-Nap | C | | 413(M⁺+1) |
| B-a-83 | 10k-1 | Int.B-55 | | cHepO | Et | H | 1Me-5-Ind | C | | 444(M⁺+1) |
| B-a-84 | 1-a | B-a-83 | | cHepO | H | H | 1Me-5-Ind | C | | 416(M⁺+1) |
| B-a-85 | 10k-1 | Int.B-56 | | cHepO | Et | H | 1 Me-5-Idz | C | | 445(M⁺+1) |
| B-a-86 | 1-a | B-a-85 | | cHepO | H | H | 1 Me-5-Idz | C | | 417(M⁺+1) |
| B-a-87 | 10k-1 | Int.B-57 | | cHepO | Et | H | 6-IQ | C | | 442(M⁺+1) |
| B-a-88 | 1-a | B-a-87 | | cHepO | H | H | 6-IQ | C | | 414(M⁺+1) |
| B-a-89 | 10k-1 | Int.B-58 | | nPrO | Et | H | 1Me-5-Ind | C | | 390(M⁺+1) |
| B-a-90 | 1-a | B-a-89 | | nPrO | H | H | 1Me-5-Ind | C | | 362(M⁺+1) |
| B-a-91 | 10k-1 | Int.B-59 | | nPrO | Et | H | 1 Et-5-Ind | C | | 404(M⁺+1) |
| B-a-92 | 1-a | B-a-91 | | nPrO | H | H | 1Et-5-Ind | C | | 376(M⁺+1) |

**[Table 52]**

| Table-B-a-3 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-a-93 | 10k-1 | Int.B-60 | | nPrO | Et | H | 1Et-5-Idz | C | | 405(M⁺+1) |
| B-a-94 | 1-a | B-a-93 | | nPrO | H | H | 1Et-5-Idz | C | | 377(M⁺+1) |
| B-a-95 | 10k-1 | Int.B-61 | | nPrO | Et | H | 6-Qu | C | | 388(M⁺+1) |
| B-a-96 | 1-a | B-a-95 | | nPrO | H | H | 6-Qu | C | | 360(M⁺+1) |
| B-a-97 | 10k-1 | Int.B-62 | | iPrO | Et | H | 2-Nap | C | | 387(M⁺+1) |
| B-a-98 | 1-a | B-a-97 | | iPrO | H | H | 2-Nap | C | | 359(M⁺+1) |
| B-a-99 | 10k-1 | Int.B-63 | | iPrO | Et | H | 1Et-5-Ind | C | | 404(M⁺+1) |
| B-a-100 | 1-a | B-a-99 | | iPrO | H | H | 1Et-5-Ind | C | | 376(M⁺+1) |
| B-a-101 | 10k-1 | Int.B-64 | | iPrO | Et | H | 1Me-5-Idz | C | | 391(M⁺+1) |
| B-a-102 | 1-a | B-a-101 | | iPrO | H | H | 1Me-5-Idz | C | | 363(M⁺+1) |
| B-a-103 | 10k-1 | Int.B-65 | | iPrO | Et | H | 6-IQ | C | | 388(M⁺+1) |
| B-a-104 | 1-a | B-a-103 | | iPrO | H | H | 6-IQ | C | | 360(M⁺+1) |
| B-a-105 | 10k-1 | Int.B-66 | | nBuO | Et | H | 2-Nap | C | | 401(M⁺+1) |
| B-a-106 | 1-a | B-a-105 | | nBuO | H | H | 2-Nap | C | | 376(M⁺+1) |
| B-a-107 | 10k-1 | Int.B-67 | | nBuO | Et | H | 1Me-5-Ind | C | | 404(M⁺+1) |
| B-a-108 | 1-a | B-a-107 | | nBuO | H | H | 1Me-5-Ind | C | | 376(M⁺+1) |
| B-a-109 | 10k-1 | Int.B-68 | | nBuO | Et | H | 1Et-5-Idz | C | | 419(M⁺+1) |
| B-a-110 | 1-a | B-a-109 | | nBuO | H | H | 1Et-5-Idz | C | | 391(M⁺+1) |
| B-a-111 | 10k-1 | Int.B-69 | | nBuO | Et | H | 5-BF | C | | 391(M⁺+1) |
| B-a-112 | 1-a | B-a-111 | | nBuO | H | H | 5-BF | C | | 363(M⁺+1) |
| B-a-113 | 10k-1 | Int.B-70 | | iBuO | Et | H | 2-Nap | C | | 401(M⁺+1) |
| B-a-114 | 1-a | B-a-113 | | iBuO | H | H | 2-Nap | C | | 373(M⁺+1) |
| B-a-115 | 10k-1 | Int.B-71 | | iBuO | Et | H | 1Me-5-Ind | C | | 404(M⁺+1) |
| B-a-116 | 1-a | B-a-115 | | iBuO | H | H | 1Me-5-Ind | C | | 376(M⁺+1) |
| B-a-117 | 10k-1 | Int.B-72 | | iBuO | Et | H | 1Et-5-Ind | C | | 418(M⁺+1) |
| B-a-118 | 1-a | B-a-117 | | iBuO | H | H | 1Et-5-Ind | C | | 390(M⁺+1) |
| B-a-119 | 10k-1 | Int.B-73 | | iBuO | Et | H | 1Me-5-Idz | C | | 405(M⁺+1) |
| B-a-120 | 1-a | B-a-119 | | iBuO | H | H | 1Me-5-Idz | C | | 377(M⁺+1) |
| B-a-121 | 10k-1 | Int.B-74 | | iBuO | Et | H | 1Et-5-Idz | C | | 419(M⁺+1) |
| B-a-122 | 1-a | B-a-121 | | iBuO | H | H | 1Et-5-Idz | C | | 391(M⁺+1) |
| B-a-123 | 10k-1 | Int.B-75 | | 2FBnO | Et | H | 2-Nap | C | | 453(M⁺+1) |
| B-a-124 | 1-a | B-a-123 | | 2FBnO | H | H | 2-Nap | C | | 425(M⁺+1) |
| B-a-125 | 10k-1 | Int.B-76 | | 2FBnO | Et | H | 1Me-5-Ind | C | | 456(M⁺+1) |
| B-a-126 | 1-a | B-a-125 | | 2FBnO | H | H | 1Me-5-Ind | C | | 428(M⁺+1) |
| B-a-127 | 10k-1 | Int.B-77 | | 2FBnO | Et | H | 1Et-5-Ind | C | | 470(M⁺+1) |
| B-a-128 | 1-a | B-a-127 | | 2FBnO | H | H | 1Et-5-Ind | C | | 442(M⁺+1) |
| B-a-129 | 10k-1 | Int.B-78 | | 2FBnO | Et | H | 1Me-5-Idz | C | | 457(M⁺+1) |
| B-a-130 | 1-a | B-a-129 | | 2FBnO | H | H | 1Me-5-Idz | C | | 429(M⁺+1) |
| B-a-131 | 10k-1 | Int.B-79 | | 2FBnO | Et | H | 1Et-5-Idz | C | | 471(M⁺+1) |
| B-a-132 | 1-a | B-a-131 | | 2FBnO | H | H | 1Et-5-Idz | C | | 443(M⁺+1) |
| B-a-133 | 10k-1 | Int.B-80 | | 2FBnO | Et | H | 6-Qu | C | | 454(M⁺+1) |
| B-a-134 | 1-a | B-a-133 | | 2FBnO | H | H | 6-Qu | C | | 426(M⁺+1) |
| B-a-135 | 10k-1 | Int.B-81 | | 2FBnO | Et | H | 6-IQ | C | | 454(M⁺+1) |
| B-a-136 | 1-a | B-a-135 | | 2FBnO | H | H | 6-IQ | C | | 426(M⁺+1) |
| B-a-137 | 10k-1 | Int.B-82 | | 3FBnO | Et | H | 2-Nap | C | | 453(M⁺+1) |
| B-a-138 | 1-a | B-a-137 | | 3FBnO | H | H | 2-Nap | C | | 425(M⁺+1) |
| B-a-139 | 10k-1 | Int.B-83 | | 3FBnO | Et | H | 1Et-5-Ind | C | | 470(M⁺+1) |
| B-a-140 | 1-a | B-a-139 | | 3FBnO | H | H | 1Et-5-Ind | C | | 442(M⁺+1) |

**[Table 53]**

| Table-B-a-4 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-a-141 | 10k-1 | Int.B-84 | | 3FBnO | Et | H | 1 Me-6-Ind | C | | 456(M⁺+1) |
| B-a-142 | 1-a | B-a-141 | | 3FBnO | H | H | 1Me-6-Ind | C | | 428(M⁺+1) |
| B-a-143 | 10k-1 | Int.B-85 | | 3FBnO | Et | H | 1Me-5-Idz | C | | 457(M⁺+1) |
| B-a-144 | 1-a | B-a-143 | | 3FBnO | H | H | 1Me-5-Idz | C | | 429(M⁺+1) |
| B-a-145 | 10k-1 | Int.B-86 | | 3FBnO | Et | H | 5-BF | C | | 443(M⁺+1) |
| B-a-146 | 1-a | B-a-145 | | 3FBnO | H | H | 5-BF | C | | 415(M⁺+1) |
| B-a-147 | 10k-1 | Int.B-87 | | 3FBnO | Et | H | 6-Qu | C | | 454(M⁺+1) |
| B-a-148 | 1-a | B-a-147 | | 3FBnO | H | H | 6-Qu | C | | 426(M⁺+1) |
| B-a-149 | 10k-1 | Int.B-88 | | 4FBnO | Et | H | 1Me-5-Ind | C | | 456(M⁺+1) |
| B-a-150 | 1-a | B-a-149 | | 4FBnO | H | H | 1 Me-5-Ind | C | | 428(M⁺+1) |
| B-a-151 | 10k-1 | Int.B-89 | | 4FBnO | Et | H | 1 Me-4-Ind | C | | 456(M⁺+1) |
| B-a-152 | 1-a | B-a-151 | | 4FBnO | H | H | 1Me-4-Ind | C | | 428(M⁺+1) |
| B-a-153 | 10k-1 | Int.B-90 | | 4FBnO | Et | H | 1Me-6-Ind | C | | 456(M⁺+1) |
| B-a-154 | 1-a | B-a-153 | | 4FBnO | H | H | 1Me-6-Ind | C | | 428(M⁺+1) |
| B-a-155 | 10k-1 | Int.B-91 | | 4FBnO | Et | H | 1Et-5-Idz | C | | 471(M⁺+1) |
| B-a-156 | 1-a | B-a-155 | | 4FBnO | H | H | 1Et-5-Idz | C | | 443(M⁺+1) |
| B-a-157 | 10k-1 | Int.B-92 | | 4FBnO | Et | H | 6-Qu | C | | 454(M⁺+1) |
| B-a-158 | 1-a | B-a-157 | | 4FBnO | H | H | 6-Qu | C | | 426(M⁺+1) |
| B-a-159 | 10k-1 | Int.B-93 | | 2ClBnO | Et | H | 2-Nap | C | | 469(M⁺+1) |
| B-a-160 | 1-a | B-a-159 | | 2ClBnO | H | H | 2-Nap | C | | 441(M⁺+1) |
| B-a-161 | 10k-1 | Int.B-94 | | 2ClBnO | Et | H | 1Et-5-Ind | C | | 486(M⁺+1) |
| B-a-162 | 1-a | B-a-161 | | 2ClBnO | H | H | 1Et-5-Ind | C | | 458(M⁺+1) |
| B-a-163 | 10k-1 | Int.B-95 | | 2ClBnO | Et | H | 1Me-4-Ind | C | | 472(M⁺+1) |
| B-a-164 | 1-a | B-a-163 | | 2ClBnO | H | H | 1Me-4-Ind | C | | 444(M⁺+1) |
| B-a-165 | 10k-1 | Int.B-96 | | 2ClBnO | Et | H | 1Me-6-Ind | C | | 472(M⁺+1) |
| B-a-166 | 1-a | B-a-165 | | 2ClBnO | H | H | 1Me-6-Ind | C | | 444(M⁺+1) |
| B-a-167 | 10k-1 | Int.B-97 | | 2ClBnO | Et | H | 1Et-5-Idz | C | | 487(M⁺+1) |
| B-a-168 | 1-a | B-a-167 | | 2ClBnO | H | H | 1Et-5-Idz | C | | 459(M⁺+1) |
| B-a-169 | 10k-1 | Int.B-98 | | 3ClBnO | Et | H | 2-Nap | C | | 469(M⁺+1) |
| B-a-170 | 1-a | B-a-169 | | 3ClBnO | H | H | 2-Nap | C | | 441(M⁺+1) |
| B-a-171 | 10k-1 | Int.B-99 | | 3ClBnO | Et | H | 1Me-5-Ind | C | | 472(M⁺+1) |
| B-a-172 | 1-a | B-a-171 | | 3ClBnO | H | H | 1Me-5-Ind | C | | 444(M⁺+1) |
| B-a-173 | 10k-1 | IntB-100 | | 3ClBnO | Et | H | 1Et-5-Ind | C | | 486(M⁺+1) |
| B-a-174 | 1-a | B-a-173 | | 3ClBnO | H | H | 1Et-5-Ind | C | | 458(M⁺+1) |
| B-a-175 | 10k-1 | Int.B-101 | | 3ClBnO | Et | H | 1Me-5-Idz | C | | 473(M⁺+1) |
| B-a-176 | 1-a | B-a-175 | | 3ClBnO | H | H | 1Me-5-Idz | C | | 442(M⁺+1) |
| B-a-177 | 10k-1 | IntB-102 | | 3ClBnO | Et | H | 1Et-5-Idz | C | | 487(M⁺+1) |
| B-a-178 | 1-a | B-a-177 | | 3ClBnO | H | H | 1Et-5-Idz | C | | 459(M⁺+1) |
| B-a-179 | 10k-1 | IntB-103 | | 3ClBnO | Et | H | 5-BF | C | | 459(M⁺+1) |
| B-a-180 | 1-a | B-a-179 | | 3ClBnO | H | H | 5-BF | C | | 431(M⁺+1) |
| B-a-181 | 10k-1 | IntB-104 | | 3ClBnO | Et | H | 6-Qu | C | | 470(M⁺+1) |
| B-a-182 | 1-a | B-a-181 | | 3ClBnO | H | H | 6-Qu | C | | 442(M⁺+1) |
| B-a-183 | 10k-1 | Int.B-105 | | 4MeBnO | Et | H | 2-Nap | C | | 449(M⁺+1) |
| B-a-184 | 1-a | B-a-183 | | 4MeBnO | H | H | 2-Nap | C | | 421(M⁺+1) |
| B-a-185 | 10k-1 | Int.B-106 | | 4MeBnO | Et | H | 1Me-5-Ind | C | | 452(M⁺+1) |
| B-a-186 | 1-a | B-a-185 | | 4MeBnO | H | H | 1Me-5-Ind | C | | 424(M⁺+1) |
| B-a-187 | 10k-1 | Int.B-107 | | 4MeBnO | Et | H | 1Et-5-Ind | C | | 466(M⁺+1) |
| B-a-188 | 1-a | B-a-187 | | 4MeBnO | H | H | 1Et-5-Ind | C | | 438(M⁺+1) |

**[Table 54]**

| Table-B-a-5 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-a-189 | 10k-1 | Int.B-108 | | 4MeBnO | Et | H | 1Me-6-Ind | C | | 452(M⁺+1) |
| B-a-190 | 1-a | B-a-189 | | 4MeBnO | H | H | 1Me-6-Ind | C | | 424(M⁺+1) |
| B-a-191 | 10k-1 | Int.B-109 | | 4MeBnO | Et | H | 1Me-5-Idz | C | | 453(M⁺+1) |
| B-a-192 | 1-a | B-a-191 | | 4MeBnO | H | H | 1Me-5-Idz | C | | 425(M⁺+1) |
| B-a-193 | 10k-1 | Int.B-110 | | 4MeBnO | Et | H | 6-IQ | C | | 450(M⁺+1) |
| B-a-194 | 1-a | B-a-193 | | 4MeBnO | H | H | 6-IQ | C | | 422(M⁺+1) |
| B-a-195 | 10k-1 | Int.B-111 | | 4CF3BnO | Et | H | 1Me-5-Ind | C | | 505(M⁺+1) |
| B-a-196 | 1-a | B-a-195 | | 4CF3BnO | H | H | 1Me-5-Ind | C | | 477(M⁺+1) |
| B-a-197 | 10k-1 | Int.B-112 | | 4CF3BnO | Et | H | 1Me-4-Ind | C | | 505(M⁺+1) |
| B-a-198 | 1-a | B-a-197 | | 4CF3BnO | H | H | 1Me-4-Ind | C | | 477(M⁺+1) |
| B-a-199 | 10k-1 | Int.B-113 | | 4CF3BnO | Et | H | 1Me-5-Idz | C | | 506(M⁺+1) |
| B-a-200 | 1-a | B-a-199 | | 4CF3BnO | H | H | 1Me-5-Idz | C | | 478(M⁺+1) |
| B-a-201 | 10k-1 | Int.B-114 | | 4CF3BnO | Et | H | 1Et-5-Idz | C | | 520(M⁺+1) |
| B-a-202 | 1-a | B-a-201 | | 4CF3BnO | H | H | 1Et-5-Idz | C | | 492(M⁺+1) |
| B-a-203 | 10k-1 | Int.B-115 | | 4CF3BnO | Et | H | 6-Qu | C | | 503(M⁺+1) |
| B-a-204 | 1-a | B-a-203 | | 4CF3BnO | H | H | 6-Qu | C | | 475(M⁺+1) |
| B-a-205 | 10k-1 | Int.B-116 | | 2EtBuO | Et | H | 2-Nap | C | | 429(M⁺+1) |
| B-a-206 | 1-a | B-a-205 | | 2EtBuO | H | H | 2-Nap | C | | 401(M⁺+1) |
| B-a-207 | 10k-1 | Int.B-117 | | 2EtBuO | Et | H | 1Me-5-Ind | C | | 432(M⁺+1) |
| B-a-208 | 1-a | B-a-207 | | 2EtBuO | H | H | 1Me-5-Ind | C | | 404(M⁺+1) |
| B-a-209 | 10k-1 | Int.B-118 | | 2EtBuO | Et | H | 1Et-5-Ind | C | | 446(M⁺+1) |
| B-a-210 | 1-a | B-a-209 | | 2EtBuO | H | H | 1Et-5-Ind | C | | 418(M⁺+1) |
| B-a-211 | 10k-1 | Int.B-119 | | 2EtBuO | Et | H | 1Me-5-Idz | C | | 433(M⁺+1) |
| B-a-212 | 1-a | B-a-211 | | 2EtBuO | H | H | 1Me-5-Idz | C | | 405(M⁺+1) |
| B-a-213 | 10k-1 | Int.B-120 | | 2EtBuO | Et | H | 1Et-5-Idz | C | | 447(M⁺+1) |
| B-a-214 | 1-a | B-a-213 | | 2EtBuO | H | H | 1Et-5-Idz | C | | 419(M⁺+1) |
| B-a-215 | 10k-1 | Int.B-121 | | 2EtBuO | Et | H | 6-Qu | C | | 430(M⁺+1) |
| B-a-216 | 1-a | B-a-215 | | 2EtBuO | H | H | 6-Qu | C | | 402(M⁺+1) |
| B-a-217 | 10k-1 | Int.B-122 | | 2EtBuO | Et | H | 6-IQ | C | | 430(M⁺+1) |
| B-a-218 | 1-a | B-a-217 | | 2EtBuO | H | H | 6-IQ | C | | 402(M⁺+1) |
| B-a-219 | 10k-1 | Int.B-123 | | 2(4DMAPh)EtO | Et | H | 2-Nap | C | | 492(M⁺+1) |
| B-a-220 | 1-a | B-a-219 | | 2(4DMAPh)EtO | H | H | 2-Nap | C | | 464(M⁺+1) |
| B-a-221 | 10k-1 | IntB-124 | | 2(4DMAPh)EtO | Et | H | 1Me-5-Ind | C | | 495(M⁺+1) |
| B-a-222 | 1-a | B-a-221 | | 2(4DMAPh)EtO | H | H | 1Me-5-Ind | C | | 467(M⁺+1) |
| B-a-223 | 10k-1 | Int.B-125 | | 2(4DMAPh)EtO | Et | H | 1Me-4-Ind | C | | 495(M⁺+1) |
| B-a-224 | 1-a | B-a-223 | | 2(4DMAPh)EtO | H | H | 1Me-4-Ind | C | | 467(M⁺+1) |
| B-a-225 | 10k-1 | Int.B-126 | | 2(4DMAPh)EtO | Et | H | 1Et-5-Idz | C | | 510(M⁺+1) |
| B-a-226 | 1-a | B-a-225 | | 2(4DMAPh)EtO | H | H | 1Et-5-Idz | C | | 482(M⁺+1) |
| B-a-227 | 10k-1 | Int.B-127 | | 2(4DMAPh)EtO | Et | H | 5-BF | C | | 482(M⁺+1) |
| B-a-228 | 1-a | B-a-227 | | 2(4DMAPh)EtO | H | H | 5-BF | C | | 454(M⁺+1) |
| B-a-229 | 10k-1 | Int.B-128 | | 2(4DMAPh)EtO | Et | H | 6-Qu | C | | 193(M⁺+1) |
| B-a-230 | 1-a | B-a-229 | | 2(4DMAPh)EtO | H | H | 6-Qu | C | | 165(M⁺+1) |
| B-a-231 | 10k-1 | Int.B-129 | | 2(PhO)EtO | Et | H | 1Et-5-Ind | C | | 482(M⁺+1) |
| B-a-232 | 1-a | B-a-231 | | 2(PhO)EtO | H | H | 1Et-5-Ind | C | | 454(M⁺+1) |
| B-a-233 | 10k-1 | Int.B-130 | | 2(PhO)EtO | Et | H | 1Me-4-Ind | C | | 468(M⁺+1) |
| B-a-234 | 1-a | B-a-233 | | 2(PhO)EtO | H | H | 1Me-4-Ind | C | | 440(M⁺+1) |
| B-a-235 | 10k-1 | Int.B-131 | | 2(PhO)EtO | Et | H | 1Me-6-Ind | C | | 468(M⁺+1) |
| B-a-236 | 1-a | B-a-235 | | 2(PhO)EtO | H | H | 1Me-6-Ind | C | | 440(M⁺+1) |

**[Table 55]**

| Table-B-a-6 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-a-237 | 10k-1 | Int.B-132 | | 2(PhO)EtO | Et | H | 1Me-5-Idz | C | | 469(M⁺+1) |
| B-a-238 | 1-a | B-a-237 | | 2(PhO)EtO | H | H | 1Me-5-Idz | C | | 441(M⁺+1) |
| B-a-239 | 10k-1 | Int.B-133 | | 2(PhO)EtO | Et | H | 6-IQ | C | | 466(M⁺+1) |
| B-a-240 | 1-a | B-a-239 | | 2(PhO)EtO | H | H | 6-IQ | C | | 438(M⁺+1) |
| B-a-241 | 10k-1 | Int.B-134 | | 1PhEtO | Et | H | 2-Nap | C | | 449(M⁺+1) |
| B-a-242 | 1-a | B-a-241 | | 1PhEtO | H | H | 2-Nap | C | | 421(M⁺+1) |
| B-a-243 | 10k-1 | IntB-135 | | 1PhEtO | Et | H | 1Me-5-Ind | C | | 452(M⁺+1) |
| B-a-244 | 1-a | B-a-243 | | 1PhEtO | H | H | 1Me-5-Ind | C | | 424(M⁺+1) |
| B-a-245 | 10k-1 | IntB-136 | | 1PhEtO | Et | H | 1Et-5-Ind | C | | 466(M⁺+1) |
| B-a-246 | 1-a | B-a-245 | | 1PhEtO | H | H | 1Et-5-Ind | C | | 438(M⁺+1) |
| B-a-247 | 10k-1 | IntB-137 | | 1PhEtO | Et | H | 1Me-5-Idz | C | | 453(M⁺+1) |
| B-a-248 | 1-a | B-a-247 | | 1PhEtO | H | H | 1Me-5-Idz | C | | 425(M⁺+1) |
| B-a-249 | 10k-1 | Int B-138 | | 1PhEtO | Et | H | 1Et-5-Idz | C | | 467(M⁺+1) |
| B-a-250 | 1-a | B-a-249 | | 1PhEtO | H | H | 1Et-5-Idz | C | | 439(M⁺+1) |
| B-a-251 | 10k-1 | IntB-139 | | 1(2FPh)EtO | Et | H | 2-Nap | C | | 467(M⁺+1) |
| B-a-252 | 1-a | B-a-251 | | 1(2FPh)EtO | H | H | 2-Nap | C | | 439(M⁺+1) |
| B-a-253 | 10k-1 | IntB-140 | | 1(2FPh)EtO | Et | H | 1Et-5-Ind | C | | 484(M⁺+1) |
| B-a-254 | 1-a | B-a-253 | | 1(2FPh)EtO | H | H | 1Et-5-Ind | C | | 456(M⁺+1) |
| B-a-255 | 10k-1 | IntB-141 | | 1(2FPh)EtO | Et | H | 1Me-6-Ind | C | | 470(M⁺+1) |
| B-a-256 | 1-a | B-a-255 | | 1(2FPh)EtO | H | H | 1Me-6-Ind | C | | 442(M⁺+1) |
| B-a-257 | 10k-1 | IntB-142 | | 1(2FPh)EtO | Et | H | 1Me-5-Idz | C | | 471(M⁺+1) |
| B-a-258 | 1-a | B-a-257 | | 1(2FPh)EtO | H | H | 1Me-5-Idz | C | | 443(M⁺+1) |
| B-a-259 | 10k-1 | Int B-143 | | 1(2FPh)EtO | Et | H | 1Et-5-Idz | C | | 485(M⁺+1) |
| B-a-260 | 1-a | B-a-259 | | 1(2FPh)EtO | H | H | 1Et-5-Idz | C | | 457(M⁺+1) |
| B-a-261 | 10k-1 | Int.B-144 | | 1(2FPh)EtO | Et | H | 6-IQ | C | | 468(M⁺+1) |
| B-a-262 | 1-a | B-a-261 | | 1(2FPh)EtO | H | H | 6-IQ | C | | 440(M⁺+1) |
| B-a-263 | 10k-1 | Int.B-145 | | 1(4ClPh)EtO | Et | H | 1Me-5-Ind | C | | 487(M⁺+1) |
| B-a-264 | 1-a | B-a-263 | | 1(4ClPh)EtO | H | H | 1Me-5-Ind | C | | 458(M⁺+1) |
| B-a-265 | 10k-1 | Int.B-146 | | 1(4ClPh)EtO | Et | H | 1Me-4-Ind | C | | 487(M⁺+1) |
| B-a-266 | 1-a | B-a-265 | | 1(4ClPh)EtO | H | H | 1Me-4-Ind | C | | 458(M⁺+1) |
| B-a-267 | 10k-1 | Int.B-147 | | 1(4ClPh)EtO | Et | H | 1Me-6-Ind | C | | 487(M⁺+1) |
| B-a-268 | 1-a | B-a-267 | | 1(4ClPh)EtO | H | H | 1Me-6-Ind | C | | 458(M⁺+1) |
| B-a-269 | 10k-1 | Int.B-148 | | 1(4ClPh)EtO | Et | H | 1Me-5-Idz | C | | 488(M⁺+1) |
| B-a-270 | 1-a | B-a-269 | | 1(4ClPh)EtO | H | H | 1Me-5-Idz | C | | 459(M⁺+1) |
| B-a-271 | 10k-1 | Int.B-149 | | 1(4ClPh)EtO | Et | H | 1Et-5-Idz | C | | 502(M⁺+1) |
| B-a-272 | 1-a | B-a-271 | | 1(4ClPh)EtO | H | H | 1Et-5-Idz | C | | 473(M⁺+1) |
| B-a-273 | 10k-1 | Int.B-150 | | 1(4ClPh)EtO | Et | H | 5-BF | C | | 473(M⁺+1) |
| B-a-274 | 1-a | B-a-273 | | 1(4ClPh)EtO | H | H | 5-BF | C | | 445(M⁺+1) |
| B-a-275 | 10k-1 | Int.B-151 | | 4Me,cHexO | Et | H | 2-Nap | C | | 441(M⁺+1) |
| B-a-276 | 1-a | B-a-275 | | 4Me,cHexO | H | H | 2-Nap | C | | 413(M⁺+1) |
| B-a-277 | 10k-1 | IntB-152 | | 4Me,cHexO | Et | H | 1Me-5-Ind | C | | 444(M⁺+1) |
| B-a-278 | 1-a | B-a-277 | | 4Me,cHexO | H | H | 1Me-5-Ind | C | | 416(M⁺+1) |
| B-a-279 | 10k-1 | Int.B-153 | | 4Me,cHexO | Et | H | 1Et-5-Ind | C | | 458(M⁺+1) |
| B-a-280 | 1-a | B-a-279 | | 4Me,cHexO | H | H | 1Et-5-Ind | C | | 430(M⁺+1) |
| B-a-281 | 10k-1 | IntB-154 | | 4Me,cHexO | Et | H | 1Me-5-Idz | C | | 445(M⁺+1) |
| B-a-282 | 1-a | B-a-281 | | 4Me,cHexO | H | H | 1Me-5-Idz | C | | 417(M⁺+1) |
| B-a-283 | 10k-1 | IntB-155 | | 4Me,cHexO | Et | H | 1Et-5-Idz | C | | 459(M⁺+1) |
| B-a-284 | 1-a | B-a-283 | | 4Me,cHexO | H | H | 1Et-5-Idz | C | | 431(M⁺+1) |

**[Table 56]**

| Table-B-a-7 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-a-285 | 10k-1 | Int.B-156 | | 4Me,cHexO | Et | H | 6-Qu | C | | 442 (M⁺+1) |
| B-a-286 | 1-a | B-a-285 | | 4Me,cHexO | H | H | 6-Qu | C | | 414 (M⁺+1) |
| B-a-287 | 10k-1 | Int.B-157 | | 1IndanO | Et | H | 2-Nap | C | | 461 (M⁺+1) |
| B-a-288 | 1-a | B-a-287 | | 1IndanO | H | H | 2-Nap | C | | 433 (M⁺+1) |
| B-a-289 | 10k-1 | Int.B-158 | | 1IndanO | Et | H | 1Me-5-Ind | C | | 464 (M⁺+1) |
| B-a-290 | 1-a | B-a-289 | | 1IndanO | H | H | 1Me-5-Ind | C | | 436 (M⁺+1) |
| B-a-291 | 10k-1 | Int.B-159 | | 1IndanO | Et | H | 1Me-4-Ind | C | | 464 (M⁺+1) |
| B-a-292 | 1-a | B-a-291 | | 1IndanO | H | H | 1Me-4-Ind | C | | 436 (M⁺+1) |
| B-a-293 | 10k-1 | Int.B-160 | | 1IndanO | Et | H | 1Me-6-Ind | C | | 464 (M⁺+1) |
| B-a-294 | 1-a | B-a-293 | | 1IndanO | H | H | 1Me-6-Ind | C | | 436 (M⁺+1) |
| B-a-295 | 10k-1 | Int.B-161 | | 1IndanO | Et | H | 1Me-5-Idz | C | | 465 (M⁺+1) |
| B-a-296 | 1-a | B-a-295 | | 1IndanO | H | H | 1Me-5-Idz | C | | 437 (M⁺+1) |
| B-a-297 | 10k-1 | Int.B-162 | | 1IndanO | Et | H | 1Et-5-Idz | C | | 479 (M⁺+1) |
| B-a-298 | 1-a | B-a-297 | | 1IndanO | H | H | 1Et-5-Idz | C | | 451 (M⁺+1) |
| B-a-299 | 10k-1 | Int.B-163 | | 1IndanO | Et | H | 5-BF | C | | 451 (M⁺+1) |
| B-a-300 | 1-a | B-a-299 | | 1IndanO | H | H | 5-BF | C | | 423 (M⁺+1) |
| B-a-301 | 10k-1 | Int.B-164 | | 1IndanO | Et | H | 6-Qu | C | | 462 (M⁺+1) |
| B-a-302 | 1-a | B-a-301 | | 1IndanO | H | H | 6-Qu | C | | 434 (M⁺+1) |
| B-a-303 | 10k-1 | Int.B-165 | | 2IndanO | Et | H | 2-Nap | C | | 461 (M⁺+1) |
| B-a-304 | 1-a | B-a-303 | | 2IndanO | H | H | 2-Nap | C | | 433 (M⁺+1) |
| B-a-305 | 10k-1 | Int.B-166 | | 2IndanO | Et | H | 1Me-5-Ind | C | | 464 (M⁺+1) |
| B-a-306 | 1-a | B-a-305 | | 2IndanO | H | H | 1Me-5-Ind | C | | 436 (M⁺+1) |
| B-a-307 | 10k-1 | Int.B-167 | | 2IndanO | Et | H | 1Et-5-Ind | C | | 478 (M⁺+1) |
| B-a-308 | 1-a | B-a-307 | | 2IndanO | H | H | 1Et-5-Ind | C | | 450 (M⁺+1) |
| B-a-309 | 10k-1 | Int.B-168 | | 2IndanO | Et | H | 1Me-5-Idz | C | | 465 (M⁺+1) |
| B-a-310 | 1-a | B-a-309 | | 2IndanO | H | H | 1Me-5-Idz | C | | 437 (M⁺+1) |
| B-a-311 | 10k-1 | Int.B-169 | | 2IndanO | Et | H | 1Et-5-Idz | C | | 479 (M⁺+1) |
| B-a-312 | 1-a | B-a-311 | | 2IndanO | H | H | 1Et-5-Idz | C | | 451 (M⁺+1) |
| B-a-313 | 10k-1 | Int.B-170 | | 2IndanO | Et | H | 6-IQ | C | | 462 (M⁺+1) |
| B-a-314 | 1-a | B-a-313 | | 2IndanO | H | H | 6-IQ | C | | 434 (M⁺+1) |
| B-a-315 | 10k-1 | Int.B-171 | | 5OMe-2-IndanO | Et | H | 2-Nap | C | | 491 (M⁺+1) |
| B-a-316 | 1-a | B-a-315 | | 5OMe-2-IndanO | H | H | 2-Nap | C | | 463 (M⁺+1) |
| B-a-317 | 10k-1 | Int.B-172 | | 5OMe-2-IndanO | Et | H | 1Et-5-Ind | C | | 508 (M⁺+1) |
| B-a-318 | 1-a | B-a-317 | | 5OMe-2-IndanO | H | H | 1Et-5-Ind | C | | 480 (M⁺+1) |
| B-a-319 | 10k-1 | Int.B-173 | | 5OMe-2-IndanO | Et | H | 1Me-5-Idz | C | | 495 (M⁺+1) |
| B-a-320 | 1-a | B-a-319 | | 5OMe-2-IndanO | H | H | 1Me-5-Idz | C | | 467 (M⁺+1) |
| B-a-321 | 10k-1 | Int.B-174 | | 5OMe-2-IndanO | Et | H | 6-Qu | C | | 492 (M⁺+1) |
| B-a-322 | 1-a | B-a-321 | | 5OMe-2-IndanO | H | H | 6-Qu | C | | 464 (M⁺+1) |
| B-a-323 | 10k-1 | Int.B-175 | | 5,6D(OMe)-2-IndanO | Et | H | 1Me-5-Ind | C | | 524 (M⁺+1) |
| B-a-324 | 1-a | B-a-323 | | 5,6D(OMe)-2-IndanO | H | H | 1Me-5-Ind | C | | 496 (M⁺+1) |
| B-a-325 | 10k-1 | Int.B-176 | | 5,6D(OMe)-2-IndanO | Et | H | 1Me-4-Ind | C | | 524 (M⁺+1) |
| B-a-326 | 1-a | B-a-325 | | 5,6D(OMe)-2-IndanO | H | H | 1Me-4-Ind | C | | 496 (M⁺+1) |
| B-a-327 | 10k-1 | Int.B-177 | | 5,6D(OMe)-2-IndanO | Et | H | 1Me-6-Ind | C | | 524 (M⁺+1) |
| B-a-328 | 1-a | B-a-327 | | 5,6D(OMe)-2-IndanO | H | H | 1Me-6-Ind | C | | 496 (M⁺+1) |
| B-a-329 | 10k-1 | Int.B-178 | | 5,6D(OMe)-2-IndanO | Et | H | 1Me-5-Idz | C | | 525 (M⁺+1) |
| B-a-330 | 1-a | B-a-329 | | 5,6D(OMe)-2-IndanO | H | H | 1Me-5-Idz | C | | 497 (M⁺+1) |
| B-a-331 | 10k-1 | Int.B-179 | | 5,6D(OMe)-2-IndanO | Et | H | 6-IQ | C | | 522 (M⁺+1) |
| B-a-332 | 1-a | B-a-331 | | 5,6D(OMe)-2-IndanO | H | H | 6-IQ | C | | 494 (M⁺+1) |

**[Table 57]**

| Table-B-a-8 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-a-333 | 10k-1 | Int.B-180 | | 5F-2-IndanO | Et | H | 2-Nap | C | | 479 (M⁺+1) |
| B-a-334 | 1-a | B-a-333 | | 5F-2-IndanO | H | H | 2-Nap | C | | 451 (M⁺+1) |
| B-a-335 | 10k-1 | Int.B-181 | | 5F-2-IndanO | Et | H | 1Me-5-Ind | C | | 482 (M⁺+1) |
| B-a-336 | 1-a | B-a-335 | | 5F-2-IndanO | H | H | 1Me-5-Ind | C | | 454 (M⁺+1) |
| B-a-337 | 10k-1 | Int.B-182 | | 5F-2-IndanO | Et | H | 1Et-5-Ind | C | | 496 (M⁺+1) |
| B-a-338 | 1-a | B-a-337 | | 5F-2-IndanO | H | H | 1Et-5-Ind | C | | 468 (M⁺+1) |
| B-a-339 | 10k-1 | Int.B-183 | | 5F-2-IndanO | Et | H | 1Me-5-Idz | C | | 483 (M⁺+1) |
| B-a-340 | 1-a | B-a-339 | | 5F-2-IndanO | H | H | 1Me-5-Idz | C | | 455 (M⁺+1) |
| B-a-341 | 10k-1 | Int.B-184 | | 5F-2-IndanO | Et | H | 1Et-5-Idz | C | | 497 (M⁺+1) |
| B-a-342 | 1-a | B-a-341 | | 5F-2-IndanO | H | H | 1Et-5-Idz | C | | 469 (M⁺+1) |
| B-a-343 | 10k-1 | Int.B-185 | | 5F-2-IndanO | Et | H | 6-Qu | C | | 480 (M⁺+1) |
| B-a-344 | 1-a | B-a-343 | | 5F-2-IndanO | H | H | 6-Qu | C | | 452 (M⁺+1) |
| B-a-345 | 10k-1 | Int.B-186 | | 5F-2-IndanO | Et | H | 6-IQ | C | | 480 (M⁺+1) |
| B-a-346 | 1-a | B-a-345 | | 5F-2-IndanO | H | H | 6-IQ | C | | 452 (M⁺+1) |
| B-a-347 | 10k-1 | Int.B-187 | | 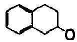 | Et | H | 2-Nap | C | | 475 (M⁺+1) |
| B-a-348 | 1-a | B-a-347 | | 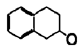 | H | H | 2-Nap | C | | 447 (M⁺+1) |
| B-a-349 | 10k-1 | Int.B-188 | | 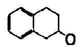 | Et | H | 1Me-5-Ind | C | | 478 (M⁺+1) |
| B-a-350 | 1-a | B-a-349 | | 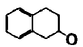 | H | H | 1Me-5-Ind | C | | 450 (M⁺+1) |
| B-a-351 | 10k-1 | Int.B-189 | | 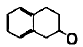 | Et | H | 1Me-6-Ind | C | | 478 (M⁺+1) |
| B-a-352 | 1-a | B-a-351 | | 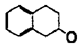 | H | H | 1Me-6-Ind | C | | 450 (M⁺+1) |
| B-a-353 | 10k-1 | Int.B-190 | | 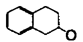 | Et | H | 1Me-5-Idz | C | | 479 (M⁺+1) |
| B-a-354 | 1-a | B-a-353 | | 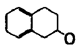 | H | H | 1Me-5-Idz | C | | 451 (M⁺+1) |
| B-a-355 | 10k-1 | Int.B-191 | | 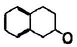 | Et | H | 6-Qu | C | | 476 (M⁺+1) |
| B-a-356 | 1-a | B-a-355 | | 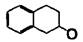 | H | H | 6-Qu | C | | 448 (M⁺+1) |
| B-a-357 | 10k-1 | Int.B-192 | | 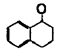 | Et | H | 1Me-5-Ind | C | | 478 (M⁺+1) |
| B-a-358 | 1-a | B-a-357 | | 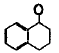 | H | H | 1Me-5-Ind | C | | 450 (M⁺+1) |
| B-a-359 | 10k-1 | Int.B-193 | | 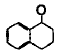 | Et | H | 1Et-5-Ind | C | | 492 (M⁺+1) |
| B-a-360 | 1-a | B-a-359 | | 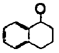 | H | H | 1Et-5-Ind | C | | 464 (M⁺+1) |
| B-a-361 | 10k-1 | Int.B-194 | | 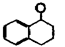 | Et | H | 1Me-4-Ind | C | | 478 (M⁺+1) |
| B-a-362 | 1-a | B-a-361 | | 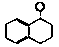 | H | H | 1Me-4-Ind | C | | 450 (M⁺+1) |
| B-a-363 | 10k-1 | Int.B-195 | | 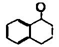 | Et | H | 1Et-5-Idz | C | | 465 (M⁺+1) |

**[Table 58]**

| Table-B-a-9 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-a-364 | 1-a | B-a-363 | | 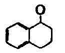 | H | H | 1Et-5-Idz | C | | 465 (M⁺+1) |
| B-a-365 | 10k-1 | Int.B-196 | | 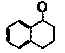 | Et | H | 5-BF | C | | 465 (M⁺+1) |
| B-a-366 | 1-a | B-a-365 | | 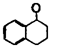 | H | H | 5-BF | C | | 437 (M⁺+1) |
| B-a-367 | 10k-1 | Int.B-197 | | 2(2MePh)EtO | Et | H | 2-Nap | C | | 463 (M⁺+1) |
| B-a-368 | 1-a | B-a-367 | | 2(2MePh)EtO | H | H | 2-Nap | C | | 435 (M⁺+1) |
| B-a-369 | 10k-1 | IntB-198 | | 2(2MePh)EtO | Et | H | 1Me-5-Ind | C | | 466 (M⁺+1) |
| B-a-370 | 1-a | B-a-369 | | 2(2MePh)EtO | H | H | 1Me-5-Ind | C | | 438 (M⁺+1) |
| B-a-371 | 10k-1 | Int.B-199 | | 2(2MePh)EtO | Et | H | 1Et-5-Ind | C | | 480 (M⁺+1) |
| B-a-372 | 1-a | B-a-371 | | 2(2MePh)EtO | H | H | 1Et-5-Ind | C | | 452 (M⁺+1) |
| B-a-373 | 10k-1 | Int.B-200 | | 2(2MePh)EtO | Et | H | 1Et-5-Idz | C | | 481 (M⁺+1) |
| B-a-374 | 1-a | B-a-373 | | 2(2MePh)EtO | H | H | 1Et-5-Idz | C | | 453 (M⁺+1) |
| B-a-375 | 10k-1 | Int.B-201 | | 2(2MePh)EtO | Et | H | 6-Qu | C | | 464 (M⁺+1) |
| B-a-376 | 1-a | B-a-375 | | 2(2MePh)EtO | H | H | 6-Qu | C | | 436 (M⁺+1) |
| B-a-377 | 10k-1 | Int.B-202 | | 2(3FPh)EtO | Et | H | 2-Nap | C | | 467 (M⁺+1) |
| B-a-378 | 1-a | B-a-377 | | 2(3FPh)EtO | H | H | 2-Nap | C | | 439 (M⁺+1) |
| B-a-379 | 10k-1 | Int.B-203 | | 2(3FPh)EtO | Et | H | 1Me-5-Ind | C | | 470 (M⁺+1) |
| B-a-380 | 1-a | B-a-379 | | 2(3FPh)EtO | H | H | 1Me-5-Ind | C | | 442 (M⁺+1) |
| B-a-381 | 10k-1 | Int.B-204 | | 2(3FPh)EtO | Et | H | 1Me-4-Ind | C | | 470 (M⁺+1) |
| B-a-382 | 1-a | B-a-381 | | 2(3FPh)EtO | H | H | 1Me-4-Ind | C | | 442 (M⁺+1) |
| B-a-383 | 10k-1 | Int.B-205 | | 2(3FPh)EtO | Et | H | 1Et-5-Idz | C | | 485 (M⁺+1) |
| B-a-384 | 1-a | B-a-383 | | 2(3FPh)EtO | H | H | 1Et-5-Idz | C | | 457 (M⁺+1) |
| B-a-385 | 10k-1 | Int.B-206 | | 2(3FPh)EtO | Et | H | 6-IQ | C | | 468 (M⁺+1) |
| B-a-386 | 1-a | B-a-385 | | 2(3FPh)EtO | H | H | 6-IQ | C | | 440 (M⁺+1) |
| B-a-387 | 10k-1 | Int B-207 | | 2(2ClPh)EtO | Et | H | 1Et-5-Ind | C | | 501 (M⁺+1) |
| B-a-388 | 1-a | B-a-387 | | 2(2ClPh)EtO | H | H | 1Et-5-Ind | C | | 473 (M⁺+1) |
| B-a-389 | 10k-1 | Int.B-208 | | 2(2ClPh)EtO | Et | H | 1Me-4-Ind | C | | 487 (M⁺+1) |
| B-a-390 | 1-a | B-a-389 | | 2(2ClPh)EtO | H | H | 1Me-4-Ind | C | | 458 (M⁺+1) |
| B-a-391 | 10k-1 | Int.B-209 | | 2(2ClPh)EtO | Et | H | 1Me-6-Ind | C | | 487 (M⁺+1) |
| B-a-392 | 1-a | B-a-391 | | 2(2ClPh)EtO | H | H | 1Me-6-Ind | C | | 458 (M⁺+1) |
| B-a-393 | 10k-1 | Int.B-210 | | 2(2ClPh)EtO | Et | H | 1Et-5-Idz | C | | 502 (M⁺+1) |
| B-a-394 | 1-a | B-a-393 | | 2(2ClPh)EtO | H | H | 1Et-5-Idz | C | | 473 (M⁺+1) |
| B-a-395 | 10k-1 | Int.B-211 | | 2(2ClPh)EtO | Et | H | 5-BF | C | | 473 (M⁺+1) |
| B-a-396 | 1-a | B-a-395 | | 2(2ClPh)EtO | H | H | 5-BF | C | | 445 (M⁺+1) |

**[Table 59]**

| Table-B-a-10 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-a-397 | 10k-1 | Int.B-212 | | 2(1-Nap)EtO | Et | H | 2-Nap | C | | 499 (M⁺+1) |
| B-a-398 | 1-a | B-a-397 | | 2(1-Nap)EtO | H | H | 2-Nap | C | | 471 (M⁺+1) |
| B-a-399 | 10k-1 | Int.B-213 | | 2(1-Nap)EtO | Et | H | 1Me-5-Ind | C | | 502 (M⁺+1) |
| B-a-400 | 1-a | B-a-399 | | 2(1-Nap)EtO | H | H | 1Me-5-Ind | C | | 474 (M⁺+1) |
| B-a-401 | 10k-1 | Int.B-214 | | 2(1-Nap)EtO | Et | H | 1Me-5-Idz | C | | 503 (M⁺+1) |
| B-a-402 | 1-a | B-a-401 | | 2(1-Nap)EtO | H | H | 1Me-5-Idz | C | | 475 (M⁺+1) |
| B-a-403 | 10k-1 | Int.B-215 | | 2(1-Nap)EtO | Et | H | 1Et-5-Idz | C | | 517 (M⁺+1) |
| B-a-404 | 1-a | B-a-403 | | 2(1-Nap)EtO | H | H | 1 Et-5-Idz | C | | 489 (M⁺+1) |
| B-a-405 | 10k-1 | Int.B-216 | | 2(1-Nap)EtO | Et | H | 6-IQ | C | | 500 (M⁺+1) |
| B-a-406 | 1-a | B-a-405 | | 2(1-Nap)EtO | H | H | 6-IQ | C | | 472 (M⁺+1) |
| B-a-407 | 10k-1 | Int.B-217 | | 2(PhS)EtO | Et | H | 1Me-5-Ind | C | | 484 (M⁺+1) |
| B-a-408 | 1-a | B-a-407 | | 2(PhS)EtO | H | H | 1Me-5-Ind | C | | 456 (M⁺+1) |
| B-a-409 | 10k-1 | Int.B-218 | | 2(PhS)EtO | Et | H | 1Me-6-Ind | C | | 484 (M⁺+1) |
| B-a-410 | 1-a | B-a-409 | | 2(PhS)EtO | H | H | 1Me-6-Ind | C | | 456 (M⁺+1) |
| B-a-411 | 10k-1 | Int.B-219 | | 2(PhS)EtO | Et | H | 1Et-5-Idz | C | | 499 (M⁺+1) |
| B-a-412 | 1-a | B-a-411 | | 2(PhS)EtO | H | H | 1Et-5-Idz | C | | 471 (M⁺+1) |
| B-a-413 | 10k-1 | Int.B-220 | | 2(PhS)EtO | Et | H | 6-Qu | C | | 482 (M⁺+1) |
| B-a-414 | 1-a | B-a-413 | | 2(PhS)EtO | H | H | 6-Qu | C | | 454 (M⁺+1) |

### Reference Example 10

### Synthesis of 4-bromo-5-cyclopentyloxy-2,3-dihydroindan-1-one (Intermediate B-221) (Preparation Method 13, Step h)

According to the procedure described in the synthetic method of Intermediate A-4 in Reference Example 1 (Preparation Method 8, Step h) provided that the reaction was performed at room temperature for 1 hour, Intermediate A-1 (1.19 g) and NBS (1.08 g, WAKO) were reacted and treated to obtain the title compound (Intermediate B-221, 774.5 mg). Mass (LCMS): 295 (M⁺), Retention time: 4.98 minutes (Elution condition: B).

### Synthesis of 5-cyclopentyloxy-4-(naphthalen-2-yl)-2,3-dihydroinden-1-one (Intermediate B-222) (Preparation Method 11, Step e-1)

According to the procedure described in the synthetic method of Example Compound A-a-1 (Preparation Method 4, Step e-1) provided that the reaction was performed for 12 hours, Intermediate B-221 (770.1 mg), 2-naphthaleneboronic acid (476.2 mg, TCI), 2 M aqueous sodium carbonate (1.9 ml) and (Ph₃P)₄Pd (287.5 mg, Nakalai Tesque) were reacted and treated to obtain the title compound (Intermediate B-222, 811.2 mg).

Typical examples of the intermediates that can be obtained by reacting and treating corresponding starting compounds using any of the methods described in the present specification including the examples described above are shown in Table-Int.B-7. The compounds were synthesized according to the preparation methods of the compounds of the compound numbers shown in the columns of "Syn" in the tables. "Int" means an intermediate compound number. In the tables, used methods among the aforementioned synthetic methods are shown in the columns of "Syn" with symbols, the starting compounds are shown in the columns of "SM", and the reagents are shown in the columns of "Regent". In the columns of "Reagent", the halide regents shown with symbols "Hal (No.)" are those mentioned in Table-Hal, the alcohol regents shown with symbols "Al (No.)" are those mentioned in Table-Al, the boronic acid regents shown with symbols "BRA (No.)" are those mentioned in Table-BRA, and the bromoheterocyclic ring regents mentioned with the symbols "Het (No.)" are those mentioned in Table-Het.

**[Table 60]**

| Table-Int.B-7 | | | | | | | | | |
|---|---|---|---|---|---|---|---|---|---|
| 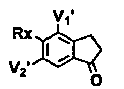 | | | | | | | | | |

| Exp. | Syn. | SM | Reagent | Rx | V1' | V2' | LCMS | | |
|---|---|---|---|---|---|---|---|---|---|
| | | | | | | | method | RTime | Mass |
| Int.B-223 | 11e-1 | Int.B-221 | BRA3 | cPenO | 1Me-5-Ind | H | C | | 346 (M⁺+1) |
| Int.B-224 | 11e-1 | Int.B-221 | BRA4 | cPenO | 1Et-5-Ind | H | C | | 360 (M⁺+1) |
| Int.B-225 | 11e-1 | Int.B-221 | BRA5 | cPenO | 1Me-4-Ind | H | C | | 346 (M⁺+1) |
| Int.B-226 | 11e-1 | Int.B-221 | BRA6 | cPenO | 1Me-6-Ind | H | C | | 346 (M⁺+1) |
| Int.B-227 | 11e-1 | Int.B-221 | BRA8 | cPenO | 1Me-5-Idz | H | C | | 347 (M⁺+1) |
| Int.B-228 | 11e-1 | Int.B-221 | BRA9 | cPenO | 1Et-5-Idz | H | C | | 361 (M⁺+1) |
| Int.B-229 | 11e-1 | Int.B-221 | BRA11 | cPenO | 2-BF | H | C | | 333 (M⁺+1) |
| Int.B-230 | 11e-1 | Int.B-221 | BRA13 | cPenO | 6-Qu | H | C | | 344 (M⁺+1) |
| Int.B-231 | 11e-2 | Int.B-221 | Het3 | cPenO | 6-IQ | H | C | | 344 (M⁺+1) |
| Int.B-233 | 11e-1 | Int.B-232 | BRA1 | BnO | 2-Nap | H | C | | 365(M⁺+1) |
| Int.B-234 | 11e-1 | Int.B-232 | BRA3 | BnO | 1Me-5-Ind | H | C | | 368 (M⁺+1) |
| Int.B-235 | 11e-1 | Int.B-232 | BRA4 | BnO | 1Et-5-Ind | H | C | | 382(M⁺+1) |
| Int.B-236 | 11e-1 | Int.B-232 | BRA5 | BnO | 1Me-4-Ind | H | C | | 368 (M⁺+1) |
| Int.B-237 | 11e-1 | Int.B-232 | BRA6 | BnO | 1Me-6-Ind | H | C | | 368 (M⁺+1) |
| Int.B-238 | 11e-1 | Int.B-232 | BRA8 | BnO | 1Me-5-Idz | H | C | | 369 (M⁺+1) |
| Int.B-239 | 11e-1 | Int.B-232 | BRA9 | BnO | 1Et-5-Idz | H | C | | 383 (M⁺+1) |
| Int.B-240 | 11e-1 | Int.B-232 | BRA11 | BnO | 2-BF | H | C | | 355 (M⁺+1) |
| Int.B-241 | 11e-1 | Int.B-232 | BRA13 | BnO | 6-Qu | H | C | | 366 (M⁺+1) |
| Int.B-242 | 11e-2 | Int.B-232 | Het3 | BnO | 6-IQ | H | C | | 366 (M⁺+1) |

### Reference Example 11

### Synthesis of 5-benzyloxy-4-bromo-2,3-dihydroindan-1-one (Intermediate B-232) (Preparation Method 13, Step h)

According to the procedure described in the synthetic method of Intermediate A-4 in Reference Example 1 (Preparation Method 8, Step h) provided that the reaction was performed at room temperature for 15 hours, Intermediate B-12 (4.10 g) and NBS (3.22 g, WAKO) were reacted and treated to obtain the title compound (Intermediate B-232, 2.17 g). Mass (LCMS): 317 (M⁺), Retention time: 4.89 minutes (Elution condition: B).

### Synthesis of 5-benzyloxy-4-(naphthalen-2-yl)-2,3-dihydroinden-1-one (Intermediate B-233) (Preparation Method 11, Step e-1)

According to the procedure described in the synthetic method of Example Compound A-a-1 (Preparation Method 4, Step e-1) provided that the reaction was performed for 12 hours, Intermediate B-232 (2.16 g), 2-naphthaleneboronic acid (2.34 g, TCI), 2 M aqueous sodium carbonate (6.5 ml) and (Ph₃P)₄Pd (821.3 mg, Nakalai Tesque) were reacted and treated to obtain the title compound (Intermediate B-233, 2.76 g). Mass (LCMS): 365 (M⁺+1), Retention time: N.D (Elution condition: C).

### Synthesis of 5-hydroxy-6-(naphthalen-2-yl)-2,3-dihydroinden-1-one (Intermediate B-243)

According to the procedure described in the synthetic method of Intermediate A-22 in Example A-a-107 provided that the reaction was performed for 12 hours, Intermediate B-233 (2.76 g) and 10% palladium hydroxide (1.43 g, NE Chemcat) were reacted and treated to obtain the title compound (Intermediate B-243, 1.98 g). Mass (LCMS): 275 (M⁺+1), Retention time: N.D (Elution condition: C).

### Synthesis of 5-cyclopentylmethyloxy-6-(naphthalen-2-yl)-2,3-dihydroinden-1-one (Intermediate B-253) (Preparation Method 4, Step f-2)

According to the procedure described in the synthetic method of Intermediate A-1 in Reference Example 1 (Preparation Method 4, Step f-2) provided that the reaction was performed for 12 hours, Intermediate B-243 (1.03 g), cyclopentanemethanol (202.3 µl, Ald), di-t-butyl azodicarboxylate (297.3 mg, Ald) and triphenylphosphine (276.2 mg, WAKO) were reacted and treated to obtain the title compound (Intermediate B-253, 1.14 g). Mass (LCMS): 357 (M⁺+1), Retention time: N.D (Elution condition: C).

Typical examples of the intermediates that can be obtained by reacting and treating corresponding starting compounds using any of the methods described in the present specification including the examples described above are shown in Table-Int.B-8 to Table-Int.B-11. In the columns of "Reagent", the halide regents shown with symbols "Hal (No.)" are those mentioned in Table-Hal, the alcohol regents shown with symbols "Al (No.)" are those mentioned in Table-Al, the boronic acid regents shown with symbols "BRA (No.)" are those mentioned in Table-BRA, and the bromoheterocyclic ring regents mentioned with the symbols "Het (No.)" are those mentioned in Table-Het. For the compounds for which cells of the columns of "Syn" are blank, deprotection was performed with Pd/C and hydrogen.

**[Table 61]**

| Table-Int.B-8 | | | | | | | | | |
|---|---|---|---|---|---|---|---|---|---|
| Exp. | Syn. | SM | Reagent | Rx | V1' | V2' | LCMS | | |
| | | | | | | | method | RTime | Mass |
| Int.B-243 | | Int.B-233 | | HO | 2-Nap | H | C | | 275 (M⁺+1) |
| Int.B-244 | | Int.B-234 | | HO | 1Me-5-Ind | H | C | | 278 (M⁺+1) |
| Int.B-245 | | Int.B-235 | | HO | 1Et-5-Ind | H | C | | 292 (M⁺+1) |
| Int.B-246 | | Int.B-236 | | HO | 1 Me-4-Ind | H | C | | 278 (M⁺+1) |
| Int.B-247 | | Int.B-237 | | HO | 1Me-6-Ind | H | C | | 278 (M⁺+1) |
| Int.B-248 | | Int.B-238 | | HO | 1Me-5-Idz | H | C | | 279 (M⁺+1) |
| Int.B-249 | | Int.B-239 | | HO | 1Et-5-Idz | H | C | | 293 (M⁺+1) |
| Int.B-250 | | Int.B-240 | | HO | 5-BF | H | C | | 265 (M⁺+1) |
| Int.B-251 | | Int.B-241 | | HO | 6-Qu | H | C | | 276 (M⁺+1) |
| Int.B-252 | | Int.B-242 | | HO | 6-IQ | H | C | | 276 (M⁺+1) |
| Int.B-253 | f-2 | Int.B-243 | Al2 | cPenMeO | 2-Nap | H | C | | 357 (M⁺+1) |
| Int.B-254 | f-2 | Int.B-244 | Al2 | cPenMeO | 1Me-5-Ind | H | C | | 360 (M⁺+1) |
| Int.B-255 | f-2 | Int.B-247 | Al2 | cPenMeO | 1Me-6-Ind | H | C | | 360 (M⁺+1) |
| Int.B-256 | f-2 | Int.B-248 | Al2 | cPenMeO | 1Me-5-Idz | H | C | | 361 (M⁺+1) |
| Int.B-257 | f-2 | Int.B-249 | Al2 | cPenMeO | 1Et-5-Idz | H | C | | 375 (M⁺+1) |
| Int.B-258 | f-2 | Int.B-251 | Al2 | cPenMeO | 6-Qu | H | C | | 358 (M⁺+1) |
| Int.B-259 | f-1 | Int.B-243 | Hal2 | cHexMeO | 2-Nap | H | C | | 371 (M⁺+1) |
| Int.B-260 | f-1 | Int.B-245 | Hal2 | cHexMeO | 1Et-5-Ind | H | C | | 388 (M⁺+1) |
| Int.B-261 | f-1 | Int.B-246 | Hal2 | cHexMeO | 1 Me-4-Ind | H | C | | 374 (M⁺+1) |
| Int.B-262 | f-1 | Int.B-248 | Hal2 | cHexMeO | 1Me-5-Idz | H | C | | 375 (M⁺+1) |
| Int.B-263 | f-1 | Int.B-249 | Hal2 | cHexMeO | 1Et-5-Idz | H | C | | 389 (M⁺+1) |
| Int.B-264 | f-1 | Int.B-250 | Hal2 | cHexMeO | 5-B F | H | C | | 361 (M⁺+1) |
| Int.B-265 | f-1 | Int.B-252 | Hal2 | cHexMeO | 6-IQ | H | C | | 372 (M⁺+1) |
| Int.B-266 | f-2 | Int.B-243 | Al3 | cHexO | 2-Nap | H | C | | 357 (M⁺+1) |
| Int.B-267 | f-2 | Int.B-244 | Al3 | cHexO | 1 Me-5-Ind | H | C | | 360 (M⁺+1) |
| Int.B-268 | f-2 | Int.B-245 | Al3 | cHexO | 1Et-5-Ind | H | C | | 374 (M⁺+1) |
| Int.B-269 | f-2 | Int.B-246 | Al3 | cHexO | 1Me-4-Ind | H | C | | 360 (M⁺+1) |
| Int.B-270 | f-2 | Int.B-248 | Al3 | cHexO | 1Me-5-Idz | H | C | | 361 (M⁺+1) |
| Int.B-271 | f-2 | Int.B-249 | Al3 | cHexO | 1Et-5-Idz | H | C | | 375 (M⁺+1) |
| Int.B-272 | f-2 | Int.B-251 | Al3 | cHexO | 6-Qu | H | C | | 358 (M⁺+1) |
| Int.B-273 | f-2 | Int.B-243 | Al4 | cHepO | 2-Nap | H | C | | 371 (M⁺+1) |
| Int.B-274 | f-2 | Int.B-244 | Al4 | cHepO | 1 Me-5-Ind | H | C | | 374 (M⁺+1) |
| Int.B-275 | f-2 | Int.B-249 | Al4 | cHepO | 1Et-5-Idz | H | C | | 389 (M⁺+1) |
| Int.B-276 | f-2 | Int.B-252 | Al4 | cHepO | 6-IQ | H | C | | 372 (M⁺+1) |
| Int.B-277 | f-1 | Int.B-245 | Hal3 | nPrO | 1Et-5-Ind | H | C | | 334 (M⁺+1) |
| Int.B-278 | f-1 | Int.B-246 | Hal3 | nPrO | 1Me-4-Ind | H | C | | 320 (M⁺+1) |
| Int.B-279 | f-1 | Int.B-249 | Hal3 | nPrO | 1Et-5-Idz | H | C | | 335 (M⁺+1) |
| Int.B-280 | f-1 | Int.B-251 | Hal3 | nPrO | 6-Qu | H | C | | 318 (M⁺+1) |
| Int.B-281 | f-1 | Int.B-243 | Hal4 | iPrO | 2-Nap | H | C | | 317 (M⁺+1) |
| Int.B-282 | f-1 | Int.B-245 | Hal4 | iPrO | 1Et-5-Ind | H | C | | 334 (M⁺+1) |
| Int.B-283 | f-1 | Int.B-248 | Hal4 | iPrO | 1 Me-5-Idz | H | C | | 321 (M⁺+1) |
| Int.B-284 | f-1 | Int.B-252 | Hal4 | iPrO | 6-IQ | H | C | | 318 (M⁺+1) |
| Int.B-285 | f-1 | Int.B-243 | Hal5 | nBuO | 2-Nap | H | C | | 331 (M⁺+1) |
| Int.B-286 | f-1 | Int.B-244 | Hal5 | nBuO | 1 Me-5-Ind | H | C | | 334 (M⁺+1) |
| Int.B-287 | f-1 | Int.B-245 | Hal5 | nBuO | 1Et-5-Ind | H | C | | 348 (M⁺) |
| Int.B-288 | f-1 | Int.B-244 | Hal6 | iBuO | 1 Me-5-Ind | H | C | | 334 (M⁺+1) |

**[Table 62]**

| Table-Int.B-9 | | | | | | | | | |
|---|---|---|---|---|---|---|---|---|---|
| Exp. | Syn. | SM | Reagent | Rx | V1' | V2' | LCMS | | |
| | | | | | | | method | RTime | Mass |
| Int.B-289 | f-1 | Int.B-245 | Hal6 | iBuO | 1Et-5-Ind | H | C | | 348 (M⁺+1) |
| Int.B-290 | f-1 | Int.B-248 | Hal6 | iBuO | 1Me-5-Idz | H | C | | 335 (M⁺+1) |
| Int.B-291 | f-1 | Int.B-249 | Hal6 | iBuO | 1Et-5-Idz | H | C | | 349 (M⁺+1) |
| Int.B-292 | f-1 | Int.B-243 | Hal7 | 2FBnO | 2-Nap | H | C | | 383 (M⁺+1) |
| Int.B-293 | f-1 | Int.B-246 | Hal7 | 2FBnO | 1 Me-4-Ind | H | C | | 386 (M⁺+1) |
| Int.B-294 | f-1 | Int.B-247 | Hal7 | 2FBnO | 1Me-6-Ind | H | C | | 386 (M⁺+1) |
| Int.B-295 | f-1 | Int.B-248 | Hal7 | 2FBnO | 1Me-5-Idz | H | C | | 387 (M⁺+1) |
| Int.B-296 | f-1 | Int.B-251 | Hal7 | 2FBnO | 6-Qu | H | C | | 384 (M⁺+1) |
| Int.B-297 | f-1 | Int.B-244 | Hal8 | 3FBnO | 1Me-5-Ind | H | C | | 386 (M⁺+1) |
| Int.B-298 | f-1 | Int.B-245 | Hal8 | 3FBnO | 1Et-5-Ind | H | C | | 400 (M⁺+1) |
| Int.B-299 | f-1 | Int.B-247 | Hal8 | 3FBnO | 1 Me-6-Ind | H | C | | 386 (M⁺+1) |
| Int.B-300 | f-1 | Int.B-250 | Hal8 | 3FBnO | 5-B F | H | C | | 373 (M⁺+1) |
| Int.B-301 | f-1 | Int.B-243 | Hal9 | 4FBnO | 2-Nap | H | C | | 383 (M⁺+1) |
| Int.B-302 | f-1 | Int.B-246 | Hal9 | 4FBnO | 1 Me-4-Ind | H | C | | 386 (M⁺+1) |
| Int.B-303 | f-1 | Int.B-247 | Hal9 | 4FBnO | 1 Me-6-Ind | H | C | | 386 (M⁺+1) |
| Int.B-304 | f-1 | Int.B-251 | Hal9 | 4FBnO | 6-Qu | H | C | | 384 (M⁺+1) |
| Int.B-305 | f-1 | Int.B-243 | Hal10 | 2ClBnO | 2-Nap | H | C | | 399 (M⁺+1) |
| Int.B-306 | f-1 | Int.B-245 | Hal10 | 2ClBnO | 1Et-5-Ind | H | C | | 416 (M⁺+1) |
| Int.B-307 | f-1 | Int.B-248 | Hal10 | 2ClBnO | 1Me-5-Idz | H | C | | 403 (M⁺+1) |
| Int.B-308 | f-1 | Int.B-249 | Hal10 | 2ClBnO | 1Et-5-Idz | H | C | | 417 (M⁺+1) |
| Int.B-309 | f-1 | Int.B-244 | Hal11 | 3ClBnO | 1Me-5-Ind | H | C | | 402 (M⁺+1) |
| Int.B-310 | f-1 | Int.B-247 | Hal11 | 3ClBnO | 1 Me-6-Ind | H | C | | 402 (M⁺+1) |
| Int.B-311 | f-1 | Int.B-249 | Hal11 | 3ClBnO | 1 Et-5-Idz | H | C | | 417 (M⁺+1) |
| Int.B-312 | f-1 | Int.B-252 | Hal11 | 3ClBnO | 6-IQ | H | C | | 400 (M⁺+1) |
| Int.B-313 | f-1 | Int.B-243 | Hal12 | 4MeBnO | 2-Nap | H | C | | 379 (M⁺+1) |
| Int.B-314 | f-1 | Int.B-245 | Hal12 | 4MeBnO | 1Et-5-Ind | H | C | | 396 (M⁺+1) |
| Int.B-315 | f-1 | Int.B-248 | Hal12 | 4MeBnO | 1Me-5-Idz | H | C | | 383 (M⁺+1) |
| Int.B-316 | f-1 | Int.B-251 | Hal12 | 4MeBnO | 6-Qu | H | C | | 380 (M⁺+1) |
| Int.B-317 | f-1 | Int.B-244 | Hal13 | 4CF3BnO | 1Me-5-Ind | H | C | | 435 (M⁺+1) |
| Int.B-318 | f-1 | Int.B-246 | Hal13 | 4CF3BnO | 1 Me-4-Ind | H | C | | 435 (M⁺+1) |
| Int.B-319 | f-1 | Int.B-249 | Hal13 | 4CF3BnO | 1Et-5-Idz | H | C | | 450 (M⁺+1) |
| Int.B-320 | f-1 | Int.B-251 | Hal13 | 4CF3BnO | 6-Qu | H | C | | 433 (M⁺+1) |
| Int.B-321 | f-2 | Int.B-243 | Al5 | 2EtBuO | 2-Nap | H | C | | 359 (M⁺+1) |
| Int.B-322 | f-2 | Int.B-244 | Al5 | 2EtBuO | 1Me-5-Ind | H | C | | 362 (M⁺+1) |
| Int.B-323 | f-2 | Int.B-245 | Al5 | 2EtBuO | 1Et-5-Ind | H | C | | 376 (M⁺+1) |
| Int.B-324 | f-2 | Int.B-248 | Al5 | 2EtBuO | 1 Me-5-Idz | H | C | | 363 (M⁺+1) |
| Int.B-325 | f-2 | Int.B-252 | Al5 | 2EtBuO | 6-IQ | H | C | | 360 (M⁺+1) |
| Int.B-326 | f-2 | Int.B-243 | Al6 | 2(4DMAPh)EtO | 2-Nap | H | C | | 422 (M⁺+1) |
| Int.B-327 | f-2 | Int.B-244 | Al6 | 2(4DMAPh)EtO | 1Me-5-Ind | H | C | | 425 (M⁺+1) |
| Int.B-328 | f-2 | Int.B-246 | Al6 | 2(4DMAPh)EtO | 1 Me-4-Ind | H | C | | 425 (M⁺+1) |
| Int.B-329 | f-2 | Int.B-249 | Al6 | 2(4DMAPh)EtO | 1Et-5-Idz | H | C | | 440 (M⁺+1) |
| Int.B-330 | f-2 | Int.B-246 | Al7 | 2(PhO)EtO | 1 Me-4-Ind | H | C | | 398 (M⁺+1) |
| Int.B-331 | f-2 | Int.B-247 | Al7 | 2(PhO)EtO | 1 Me-6-Ind | H | C | | 398 (M⁺+1) |
| Int.B-332 | f-2 | Int.B-248 | Al7 | 2(PhO)EtO | 1 Me-5-Idz | H | C | | 399 (M⁺+1) |
| Int.B-333 | f-2 | Int.B-251 | Al7 | 2(PhO)EtO | 6-Qu | H | C | | 306 (M⁺+1) |
| Int.B-334 | f-2 | Int.B-243 | Al8 | 1PhEtO | 2-Nap | H | C | | 379 (M⁺+1) |

**[Table 63]**

| Table-Int.B-10 | | | | | | | | | |
|---|---|---|---|---|---|---|---|---|---|
| Exp. | Syn. | SM | Reagent | Rx | V1' | V2' | LCMS | | |
| | | | | | | | method | RTime | Mass |
| Int.B-335 | f-2 | Int.B-245 | Al8 | 1 PhEtO | 1Et-5-Ind | H | C | | 396 (M⁺+1) |
| Int.B-336 | f-2 | Int.B-246 | Al8 | 1PhEtO | 1Me-4-Ind | H | C | | 382 (M⁺+1) |
| Int.B-337 | f-2 | Int.B-248 | Al8 | 1PhEtO | 1Me-5-Idz | H | C | | 383 (M⁺+1) |
| Int.B-338 | f-2 | Int.B-249 | Al8 | 1PhEtO | 1Et-5-Idz | H | C | | 397 (M⁺+1) |
| Int.B-339 | f-2 | Int.B-243 | Al9 | 1(2FPh)EtO | 2-Nap | H | C | | 397 (M⁺+1) |
| Int.B-340 | f-2 | Int.B-245 | Al9 | 1(2FPh)EtO | 1Et-5-Ind | H | C | | 414 (M⁺+1) |
| Int.B-341 | f-2 | Int.B-248 | Al9 | 1(2FPh)EtO | 1Me-5-Idz | H | C | | 401 (M⁺+1) |
| Int.B-342 | f-2 | Int.B-252 | Al9 | 1(2FPh)EtO | 6-IQ | H | C | | 398 (M⁺+1) |
| Int.B-343 | f-2 | Int.B-244 | Al10 | 1(4ClPh)EtO | 1Me-5-Ind | H | C | | 416 (M⁺+1) |
| Int.B-344 | f-2 | Int.B-246 | Al10 | 1(4ClPh)EtO | 1Me-4-Ind | H | C | | 416 (M⁺+1) |
| Int.B-345 | f-2 | Int.B-247 | Al10 | 1(4ClPh)EtO | 1Me-6-Ind | H | C | | 416 (M⁺+1) |
| Int.B-346 | f-2 | Int.B-249 | Al10 | 1(4ClPh)EtO | 1Et-5-Idz | H | C | | 431 (M⁺+1) |
| Int.B-347 | f-2 | Int.B-243 | Al11 | 4Me,cHexO | 2-Nap | H | C | | 371 (M⁺+1) |
| Int.B-348 | f-2 | Int.B-245 | Al11 | 4Me,cHexO | 1Et-5-Ind | H | C | | 388 (M⁺+1) |
| Int.B-349 | f-2 | Int.B-248 | Al11 | 4Me,cHexO | 1Me-5-Idz | H | C | | 375 (M⁺+1) |
| Int.B-350 | f-2 | Int.B-250 | Al11 | 4Me,cHexO | 5-BF | H | C | | 361 (M⁺+1) |
| Int.B-351 | f-2 | Int.B-243 | Al17 | 1IndanO | 2-Nap | H | C | | 391 (M⁺+1) |
| Int.B-352 | f-2 | Int.B-244 | Al17 | 1IndanO | 1Me-5-Ind | H | C | | 394 (M⁺+1) |
| Int.B-353 | f-2 | Int.B-246 | Al17 | 1IndanO | 1Me-4-Ind | H | C | | 394 (M⁺+1) |
| Int.B-354 | f-2 | Int.B-248 | Al17 | 1IndanO | 1Me-5-Idz | H | C | | 395 (M⁺+1) |
| Int.B-355 | f-2 | Int.B-251 | Al17 | 1IndanO | 6-Qu | H | C | | 392 (M⁺+1) |
| Int.B-356 | f-2 | Int.B-243 | Al18 | 2IndanO | 2-Nap | H | C | | 391 (M⁺+1) |
| Int.B-357 | f-2 | Int.B-244 | Al18 | 2IndanO | 1Me-5-Ind | H | C | | 394 (M⁺+1) |
| Int.B-358 | f-2 | Int.B-245 | Al18 | 2IndanO | 1Et-5-Ind | H | C | | 408 (M⁺+1) |
| Int.B-359 | f-2 | Int.B-248 | Al18 | 2IndanO | 1 Me-5-Idz | H | C | | 395 (M⁺+1) |
| Int.B-360 | f-2 | Int.B-249 | Al18 | 2IndanO | 1Et-5-Idz | H | C | | 409 (M⁺+1) |
| Int.B-361 | f-2 | Int.B-252 | Al18 | 2IndanO | 6-IQ | H | C | | 392 (M⁺+1) |
| Int.B-362 | f-2 | Int.B-243 | Al19 | 50Me-2-IndanO | 2-Nap | H | C | | 421 (M⁺+1) |
| Int.B-363 | f-2 | Int.B-245 | Al19 | 50Me-2-IndanO | 1Et-5-Ind | H | C | | 438 (M⁺+1) |
| Int.B-364 | f-2 | Int.B-248 | Al19 | 50Me-2-IndanO | 1Me-5-Idz | H | C | | 425 (M⁺+1) |
| Int.B-365 | f-2 | Int.B-251 | Al19 | 50Me-2-IndanO | 6-Qu | H | C | | 422 (M⁺+1) |
| Int.B-366 | f-2 | Int.B-244 | Al20 | 5,6D(OMe)-2-IndanO | 1Me-5-Ind | H | C | | 454 (M⁺+1) |
| Int.B-367 | f-2 | Int.B-246 | Al20 | 5,6D(OMe)-2-IndanO | 1Me-4-Ind | H | C | | 454 (M⁺+1) |
| Int.B-368 | f-2 | Int.B-249 | Al20 | 5,6D(OMe)-2-IndanO | 1Et-5-Idz | H | C | | 455 (M⁺+1) |
| Int.B-369 | f-2 | Int.B-252 | Al20 | 5,6D(OMe)-2-IndanO | 6-IQ | H | C | | 452 (M⁺+1) |
| Int.B-370 | f-2 | Int.B-243 | Al21 | 5F-2-IndanO | 2-Nap | H | C | | 409 (M⁺+1) |
| Int.B-371 | f-2 | Int.B-244 | Al21 | 5F-2-IndanO | 1Me-5-Ind | H | C | | 412 (M⁺+1) |
| Int.B-372 | f-2 | Int.B-248 | Al21 | 5F-2-IndanO | 1Me-5-Idz | H | C | | 413 (M⁺+1) |
| Int.B-373 | f-2 | Int.B-249 | Al21 | 5F-2-IndanO | 1Et-5-Idz | H | C | | 427 (M⁺+1) |
| Int.B-374 | f-2 | Int.B-243 | Al22 | 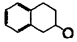 | 2-Nap | H | C | | 405 (M⁺+1) |
| Int.B-375 | f-2 | Int.B-244 | Al22 | 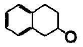 | 1Me-5-Ind | H | C | | 408 (M⁺+1) |
| Int.B-376 | f-2 | Int.B-247 | Al22 | 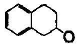 | 1Me-6-Ind | H | C | | 408 (M⁺+1) |
| Int.B-377 | f-2 | Int.B-248 | Al22 | 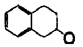 | 1Me-5-Idz | H | C | | 409 (M⁺+1) |

**[Table 64]**

| Table-Int.B-11 | | | | | | | | | |
|---|---|---|---|---|---|---|---|---|---|
| Exp. | Syn. | SM | Reagent | Rx | V1' | V2' | LCMS | | |
| | | | | | | | method | RTime | Mass |
| Int.B-378 | f-2 | Int.B-251 | Al22 | 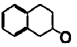 | 6-Qu | H | C | | 423 (M⁺+1) |
| Int.B-379 | f-2 | Int.B-244 | Al23 | 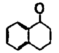 | 1Me-5-Ind | H | C | | 408 (M⁺+1) |
| Int.B-380 | f-2 | Int.B-245 | Al23 | 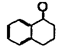 | 1 Et-5-Ind | H | C | | 422 (M⁺+1) |
| Int.B-381 | f-2 | Int.B-246 | Al23 | 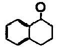 | 1Me-4-Ind | H | C | | 408 (M⁺+1) |
| Int.B-382 | f-2 | Int.B-249 | Al23 | 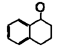 | 1Et-5-Idz | H | C | | 423 (M⁺+1) |
| Int.B-383 | f-2 | Int.B-250 | Al23 | 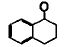 | 5-BF | H | C | | 395 (M⁺+1) |
| Int.B-384 | f-2 | Int.B-243 | Al24 | 2(2MePh)EtO | 2-Nap | H | C | | 393 (M⁺+1) |
| Int.B-385 | f-2 | Int.B-245 | Al24 | 2(2MePh)EtO | 1Et-5-Ind | H | C | | 410 (M⁺+1) |
| Int.B-386 | f-2 | Int.B-247 | Al24 | 2(2MePh)EtO | 1Me-6-Ind | H | C | | 396 (M⁺+1) |
| Int.B-387 | f-2 | Int.B-249 | Al24 | 2(2MePh)EtO | 1 Et-5-Idz | H | C | | 410 (M⁺+1) |
| Int.B-388 | f-2 | Int.B-244 | Al25 | 2(3FPh)EtO | 1Me-5-Ind | H | C | | 400 (M⁺+1) |
| Int.B-389 | f-2 | Int.B-246 | Al25 | 2(3FPh)EtO | 1Me-4-Ind | H | C | | 400 (M⁺+1) |
| Int.B-390 | f-2 | Int.B-248 | Al25 | 2(3FPh)EtO | 1Me-5-Idz | H | C | | 401 (M⁺+1) |
| Int.B-391 | f-2 | Int.B-252 | Al25 | 2(3FPh)EtO | 6-IQ | H | C | | 398 (M⁺+1) |
| Int.B-392 | f-2 | Int.B-243 | Al26 | 2(2ClPh)EtO | 2-Nap | H | C | | 413 (M⁺+1) |
| Int.B-393 | f-2 | Int.B-245 | Al26 | 2(2ClPh)EtO | 1Et-5-Ind | H | C | | 430 (M⁺+1) |
| Int.B-394 | f-2 | Int.B-246 | Al26 | 2(2ClPh)EtO | 1 Me-4-Ind | H | C | | 416 (M⁺+1) |
| Int.B-395 | f-2 | Int.B-247 | Al26 | 2(2ClPh)EtO | 1 Me-6-Ind | H | C | | 416 (M⁺+1) |
| Int.B-396 | f-2 | Int.B-249 | Al26 | 2(2ClPh)EtO | 1Et-5-Idz | H | C | | 430 (M⁺+1) |
| Int.B-397 | f-2 | Int.B-250 | Al26 | 2(2ClPh)EtO | 5-BF | H | C | | 403 (M⁺+1) |
| Int.B-398 | f-2 | Int.B-243 | Al28 | 2(1-Nap)EtO | 2-Nap | H | C | | 429 (M⁺+1) |
| Int.B-399 | f-2 | Int.B-244 | Al28 | 2(1-Nap)EtO | 1Me-5-Ind | H | C | | 432 (M⁺+1) |
| Int.B-400 | f-2 | Int.B-248 | Al28 | 2(1-Nap)EtO | 1Me-5-Idz | H | C | | 433 (M⁺+1) |
| Int.B-401 | f-2 | Int.B-251 | Al28 | 2(1-Nap)EtO | 6-Qu | H | C | | 430 (M⁺+1) |
| Int.B-402 | f-2 | Int.B-252 | Al28 | 2(1-Nap)EtO | 6-IQ | H | C | | 430 (M⁺+1) |
| Int.B-403 | f-2 | Int.B-244 | Al30 | 2(PhS)EtO | 1 Me-5-Ind | H | C | | 414 (M⁺+1) |
| Int.B-404 | f-2 | Int.B-247 | Al30 | 2(PhS)EtO | 1 Me-6-Ind | H | C | | 414 (M⁺+1) |
| Int.B-405 | f-2 | Int.B-249 | Al30 | 2(PhS)EtO | 1Et-5-Idz | H | C | | 428 (M⁺+1) |
| Int.B-406 | f-2 | Int.B-251 | Al30 | 2(PhS)EtO | 6-Qu | H | C | | 412 (M⁺+1) |

### Example B-b-1

### Synthesis of ethyl 2-[5-cyclopentyloxy-4-(naphthalen-2-yl)-2,3-dihydroinden-1-ylidene]acetate (Compound No. B-b-1) (Preparation Method 10, Step k-1)

According to the procedure described in the synthetic method of Intermediate A-2 in Reference Example 1 (Preparation Method 9, Step k-1) provided that the reaction was performed for 18.5 hours under a reflux condition, Intermediate B-222 (254.0 mg) and ethyl diethylphosphonoacetate (2 ml, TCI) and sodium hydride (241.3 mg, WAKO) were reacted and treated to obtain the title compound (Compound No. B-b-1, 202.0 mg).

### Example B-b-2

### Synthesis of 2-[5-cyclopentyloxy-4-(naphthalen-2-yl)-2,3-dihydroinden-1-ylidene]acetic acid (Compound No. B-b-2) (Preparation Method 1, Step a)

According to the procedure described in the synthetic method of Example Compound A-a-2 (Preparation Method 1, Step a) provided that the reaction was performed for 3 hours, Example Compound B-b-1 (70.3 mg) and 2 N aqueous sodium hydroxide (0.45 ml) were reacted and treated to obtain the title compound (Compound No. B-b-2, 58.9 mg).

Typical examples of the compounds of the present invention that can be obtained by reacting and treating corresponding starting compounds using any of the methods described in the present specification including the examples described above are shown in Table-B-b-1 to Table-B-b-8. The compounds were synthesized according to the preparation methods of the compounds of the compound numbers shown in the columns of "Syn" in the tables. "Int" means an intermediate compound number. In the tables, used methods among the aforementioned synthetic methods are shown in the columns of "Syn" with symbols, the starting compounds are shown in the columns of "SM", and the reagents are shown in the columns of "Regent".

**[Table 65]**

| Table-B-b-1 | | | | | | | | | | |
|---|---|---|---|---|---|---|---|---|---|---|
| 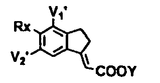 | | | | | | | | | | |

| Exp. | Syn. | SM | Reagent | Rx | Y | V1' | V2' | LCMS | | |
|---|---|---|---|---|---|---|---|---|---|---|
| | | | | | | | | method | RTime | Mass |
| B-b-1 | 10k-1 | Int.B-222 | | cPenO | Et | 2-Nap | H | C | | 413 (M⁺+1) |
| B-b-2 | 1-a | B-b-1 | | cPenO | H | 2-Nap | H | C | | 385 (M⁺+1) |
| B-b-3 | 10k-1 | Int.B-223 | | cPenO | Et | 1Me-5-Ind | H | C | | 416 (M⁺+1) |
| B-b-4 | 1-a | B-b-3 | | cPenO | H | 1Me-5-Ind | H | C | | 388 (M⁺+1) |
| B-b-5 | 10k-1 | Int.B-224 | | cPenO | Et | 1Et-5-Ind | H | C | | 430 (M⁺+1) |
| B-b-6 | 1-a | B-b-5 | | cPenO | H | 1Et-5-Ind | H | C | | 402 (M⁺+1) |
| B-b-7 | 10k-1 | Int.B-225 | | cPenO | Et | 1Me-4-Ind | H | C | | 416 (M⁺+1) |
| B-b-8 | 1-a | B-b-7 | | cPenO | H | 1Me-4-Ind | H | C | | 388 (M⁺+1) |
| B-b-9 | 10k-1 | Int.B-226 | | cPenO | Et | 1Me-6-Ind | H | C | | 416 (M⁺+1) |
| B-b-10 | 1-a | B-b-9 | | cPenO | H | 1Me-6-Ind | H | C | | 388 (M⁺+1) |
| B-b-11 | 10k-1 | Int.B-227 | | cPenO | Et | 1Me-5-Idz | H | C | | 417 (M⁺+1) |
| B-b-12 | 1-a | B-b-11 | | cPenO | H | 1Me-5-Idz | H | C | | 389 (M⁺+1) |
| B-b-13 | 10k-1 | Int.B-228 | | cPenO | Et | 1Et-5-Idz | H | C | | 431 (M⁺+1) |
| B-b-14 | 1-a | B-b-13 | | cPenO | H | 1Et-5-Idz | H | C | | 403 (M⁺+1) |
| B-b-15 | 10k-1 | Int.B-229 | | cPenO | Et | 5-BF | H | C | | 403 (M⁺+1) |
| B-b-16 | 1-a | B-b-15 | | cPenO | H | 5-B F | H | C | | 375 (M⁺+1) |
| B-b-17 | 10k-1 | Int.B-230 | | cPenO | Et | 6-Qu | H | C | | 414 (M⁺+1) |
| B-b-18 | 1-a | B-b-17 | | cPenO | H | 6-Qu | H | C | | 386 (M⁺+1) |
| B-b-19 | 10k-1 | Int.B-231 | | cPenO | Et | 6-IQ | H | C | | 414 (M⁺+1) |
| B-b-20 | 1-a | B-b-19 | | cPenO | H | 6-IQ | H | C | | 386 (M⁺+1) |
| B-b-21 | 10k-1 | Int.B-233 | | BnO | Et | 2-Nap | H | C | | 435 (M⁺+1) |
| B-b-22 | 1-a | B-b-21 | | BnO | H | 2-Nap | H | C | | 407 (M⁺+1) |
| B-b-23 | 10k-1 | Int.B-234 | | BnO | Et | 1Me-5-Ind | H | C | | 438 (M⁺+1) |
| B-b-24 | 1-a | B-b-23 | | BnO | H | 1Me-5-Ind | H | C | | 410 (M⁺+1) |
| B-b-25 | 10k-1 | Int.B-235 | | BnO | Et | 1Et-5-Ind | H | C | | 452 (M⁺+1) |
| B-b-26 | 1-a | B-b-25 | | BnO | H | 1Et-5-Ind | H | C | | 424 (M⁺+1) |
| B-b-27 | 10k-1 | Int.B-236 | | BnO | Et | 1Me-4-Ind | H | C | | 438 (M⁺+1) |
| B-b-28 | 1-a | B-b-27 | | BnO | H | 1Me-4-Ind | H | C | | 410 (M⁺+1) |
| B-b-29 | 10k-1 | Int.B-237 | | BnO | Et | 1Me-6-Ind | H | C | | 438 (M⁺+1) |
| B-b-30 | 1-a | B-b-29 | | BnO | H | 1Me-6-Ind | H | C | | 410 (M⁺+1) |
| B-b-31 | 10k-1 | Int.B-238 | | BnO | Et | 1Me-5-Idz | H | C | | 439 (M⁺+1) |
| B-b-32 | 1-a | B-b-31 | | BnO | H | 1Me-5-Idz | H | C | | 411 (M⁺+1) |
| B-b-33 | 10k-1 | Int.B-239 | | BnO | Et | 1Et-5-Idz | H | C | | 453 (M⁺+1) |
| B-b-34 | 1-a | B-b-33 | | BnO | H | 1Et-5-Idz | H | C | | 425 (M⁺+1) |
| B-b-35 | 10k-1 | Int.B-240 | | BnO | Et | 5-BF | H | C | | 425 (M⁺+1) |
| B-b-36 | 1-a | B-b-35 | | BnO | H | 5-B F | H | C | | 397 (M⁺+1) |
| B-b-37 | 10k-1 | Int.B-241 | | BnO | Et | 6-Qu | H | C | | 436 (M⁺+1) |
| B-b-38 | 1-a | B-b-37 | | BnO | H | 6-Qu | H | C | | 408 (M⁺+1) |
| B-b-39 | 10k-1 | Int.B-242 | | BnO | Et | 6-IQ | H | C | | 436 (M⁺+1) |
| B-b-40 | 1-a | B-b-39 | | BnO | H | 6-IQ | H | C | | 408 (M⁺+1) |
| B-b-41 | 10k-1 | Int.B-253 | | cPenMeO | Et | 2-Nap | H | C | | 427 (M⁺+1) |
| B-b-42 | 1-a | B-b-41 | | cPenMeO | H | 2-Nap | H | C | | 399 (M⁺+1) |
| B-b-43 | 10k-1 | Int.B-254 | | cPenMeO | Et | 1 Me-5-Ind | H | C | | 430 (M⁺+1) |
| B-b-44 | 1-a | B-b-43 | | cPenMeO | H | 1Me-5-Ind | H | C | | 402 (M⁺+1) |

**[Table 66]**

| Table-B-b-2 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-b-45 | 10k-1 | Int.B-255 | | cPenMeO | Et | 1Me-6-Ind | H | C | | 430 (M⁺+1) |
| B-b-46 | 1-a | B-b-45 | | cPenMeO | H | 1Me-6-Ind | H | C | | 402 (M⁺+1) |
| B-b-47 | 10k-1 | Int.B-256 | | cPenMeO | Et | 1Me-5-Idz | H | C | | 431 (M⁺+1) |
| B-b-48 | 1-a | B-b-47 | | cPenMeO | H | 1Me-5-Idz | H | C | | 403 (M⁺+1) |
| B-b-49 | 10k-1 | Int.B-257 | | cPenMeO | Et | 1Et-5-Idz | H | C | | 445 (M⁺+1) |
| B-b-50 | 1-a | B-b-49 | | cPenMeO | H | 1Et-5-Idz | H | C | | 417 (M⁺+1) |
| B-b-51 | 10k-1 | Int.B-258 | | cPenMeO | Et | 6-Qu | H | C | | 428 (M⁺+1) |
| B-b-52 | 1-a | B-b-51 | | cPenMeO | H | 6-Qu | H | C | | 400 (M⁺+1) |
| B-b-53 | 10k-1 | Int.B-259 | | cHexMeO | Et | 2-Nap | H | C | | 441 (M⁺+1) |
| B-b-54 | 1-a | B-b-53 | | cHexMeO | H | 2-Nap | H | C | | 413 (M⁺+1) |
| B-b-55 | 10k-1 | Int.B-260 | | cHexMeO | Et | 1Et-5-Ind | H | C | | 458 (M⁺+1) |
| B-b-56 | 1-a | B-b-55 | | cHexMeO | H | 1Et-5-Ind | H | C | | 430 (M⁺+1) |
| B-b-57 | 10k-1 | Int.B-261 | | cHexMeO | Et | 1Me-4-Ind | H | C | | 444 (M⁺+1) |
| B-b-58 | 1-a | B-b-57 | | cHexMeO | H | 1Me-4-Ind | H | C | | 416 (M⁺+1) |
| B-b-59 | 10k-1 | Int.B-262 | | cHexMeO | Et | 1Me-5-Idz | H | C | | 445 (M⁺+1) |
| B-b-60 | 1-a | B-b-59 | | cHexMeO | H | 1Me-5-Idz | H | C | | 417 (M⁺+1) |
| B-b-61 | 10k-1 | Int.B-263 | | cHexMeO | Et | 1Et-5-Idz | H | C | | 459 (M⁺+1) |
| B-b-62 | 1-a | B-b-61 | | cHexMeO | H | 1Et-5-Idz | H | C | | 431 (M⁺+1) |
| B-b-63 | 10k-1 | Int.B-264 | | cHexMeO | Et | 5-BF | H | C | | 431 (M⁺+1) |
| B-b-64 | 1-a | B-b-63 | | cHexMeO | H | 5-BF | H | C | | 403 (M⁺+1) |
| B-b-65 | 10k-1 | Int.B-265 | | cHexMeO | Et | 6-IQ | H | C | | 442 (M⁺+1) |
| B-b-66 | 1-a | B-b-65 | | cHexMeO | H | 6-IQ | H | C | | 414 (M⁺+1) |
| B-b-67 | 10k-1 | Int.B-266 | | cHexO | Et | 2-Nap | H | C | | 427 (M⁺+1) |
| B-b-68 | 1-a | B-b-67 | | cHexO | H | 2-Nap | H | C | | 399 (M⁺+1) |
| B-b-69 | 10k-1 | Int.B-267 | | cHexO | Et | 1Me-5-Ind | H | C | | 430 (M⁺+1) |
| B-b-70 | 1-a | B-b-69 | | cHexO | H | 1Me-5-Ind | H | C | | 402 (M⁺+1) |
| B-b-71 | 10k-1 | Int.B-268 | | cHexO | Et | 1Et-5-Ind | H | C | | 444 (M⁺+1) |
| B-b-72 | 1-a | B-b-71 | | cHexO | H | 1Et-5-Ind | H | C | | 416 (M⁺+1) |
| B-b-73 | 10k-1 | Int.B-269 | | cHexO | Et | 1Me-4-Ind | H | C | | 430 (M⁺+1) |
| B-b-74 | 1-a | B-b-73 | | cHexO | H | 1Me-4-Ind | H | C | | 402 (M⁺+1) |
| B-b-75 | 10k-1 | Int.B-270 | | cHexO | Et | 1Me-5-Idz | H | C | | 431 (M⁺+1) |
| B-b-76 | 1-a | B-b-75 | | cHexO | H | 1Me-5-Idz | H | C | | 403 (M⁺+1) |
| B-b-77 | 10k-1 | Int.B-271 | | cHexO | Et | 1Et-5-Idz | H | C | | 445 (M⁺+1) |
| B-b-78 | 1-a | B-b-77 | | cHexO | H | 1Et-5-Idz | H | C | | 417 (M⁺+1) |
| B-b-79 | 10k-1 | Int.B-272 | | cHexO | Et | 6-Qu | H | C | | 428 (M⁺+1) |
| B-b-80 | 1-a | B-b-79 | | cHexO | H | 6-Qu | H | C | | 400 (M⁺+1) |
| B-b-81 | 10k-1 | Int.B-273 | | cHepO | Et | 2-Nap | H | C | | 441 (M⁺+1) |
| B-b-82 | 1-a | B-b-81 | | cHepO | H | 2-Nap | H | C | | 413 (M⁺+1) |
| B-b-83 | 10k-1 | Int.B-274 | | cHepO | Et | 1Me-5-Ind | H | C | | 444 (M⁺+1) |
| B-b-84 | 1-a | B-b-83 | | cHepO | H | 1Me-5-Ind | H | C | | 416 (M⁺+1) |
| B-b-85 | 10k-1 | Int.B-275 | | cHepO | Et | 1Me-5-Idz | H | C | | 445 (M⁺+1) |
| B-b-86 | 1-a | B-b-85 | | cHepO | H | 1 Me-5-Idz | H | C | | 417 (M⁺+1) |
| B-b-87 | 10k-1 | Int.B-276 | | cHepO | Et | 6-IQ | H | C | | 442 (M⁺+1) |
| B-b-88 | 1-a | B-b-87 | | cHepO | H | 6-IQ | H | C | | 414 (M⁺+1) |
| B-b-89 | 10k-1 | Int.B-277 | | nPrO | Et | 1Et-5-Ind | H | C | | 404 (M⁺+1) |
| B-b-90 | 1-a | B-b-89 | | nPrO | H | 1Et-5-Ind | H | C | | 376 (M⁺+1) |
| B-b-91 | 10k-1 | Int.B-278 | | nPrO | Et | 1Me-4-Ind | H | C | | 390 (M⁺+1) |
| B-b-92 | 1-a | B-b-91 | | nPrO | H | 1 Me-4-Ind | H | C | | 362 (M⁺+1) |

**[Table 67]**

| Table-B-b-3 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-b-93 | 10k-1 | Int.B-279 | | nPrO | Et | 1Et-5-Idz | H | C | | 405 (M⁺+1) |
| B-b-94 | 1-a | B-b-93 | | nPrO | H | 1Et-5-Idz | H | C | | 377 (M⁺+1) |
| B-b-95 | 10k-1 | Int.B-280 | | nPrO | Et | 6-Qu | H | C | | 388 (M⁺+1) |
| B-b-96 | 1-a | B-b-95 | | nPrO | H | 6-Qu | H | C | | 360 (M⁺+1) |
| B-b-97 | 10k-1 | Int.B-281 | | iPrO | Et | 2-Nap | H | C | | 387 (M⁺+1) |
| B-b-98 | 1-a | B-b-97 | | iPrO | H | 2-Nap | H | C | | 359 (M⁺+1) |
| B-b-99 | 10k-1 | Int.B-282 | | iPrO | Et | 1Et-5-Ind | H | C | | 404 (M⁺+1) |
| B-b-100 | 1-a | B-b-99 | | iPrO | H | 1Et-5-Ind | H | C | | 376 (M⁺+1) |
| B-b-101 | 10k-1 | Int.B-283 | | iPrO | Et | 1Me-5-Idz | H | C | | 391 (M⁺+1) |
| B-b-102 | 1-a | B-b-101 | | iPrO | H | 1Me-5-Idz | H | C | | 363 (M⁺+1) |
| B-b-103 | 10k-1 | Int.B-284 | | iPrO | Et | 6-IQ | H | C | | 388 (M⁺+1) |
| B-b-104 | 1-a | B-b-103 | | iPrO | H | 6-IQ | H | C | | 360 (M⁺+1) |
| B-b-105 | 10k-1 | Int.B-285 | | nBuO | Et | 2-Nap | H | C | | 401 (M⁺+1) |
| B-b-106 | 1-a | B-b-105 | | nBuO | H | 2-Nap | H | C | | 373 (M⁺+1) |
| B-b-107 | 10k-1 | Int.B-286 | | nBuO | Et | 1Me-5-Ind | H | C | | 404 (M⁺+1) |
| B-b-108 | 1-a | B-b-107 | | nBuO | H | 1Me-5-Ind | H | C | | 376 (M⁺+1) |
| B-b-109 | 10k-1 | Int.B-287 | | nBuO | Et | 1Et-5-Idz | H | C | | 419 (M⁺+1) |
| B-b-110 | 1-a | B-b-109 | | nBuO | H | 1Et-5-Idz | H | C | | 391 (M⁺+1) |
| B-b-111 | 10k-1 | Int.B-288 | | iBuO | Et | 1Me-5-Ind | H | C | | 404 (M⁺+1) |
| B-b-112 | 1-a | B-b-111 | | iBuO | H | 1Me-5-Ind | H | C | | 376 (M⁺+1) |
| B-b-113 | 10k-1 | Int. B-289 | | iBuO | Et | 1Et-5-Ind | H | C | | 418 (M⁺+1) |
| B-b-114 | 1-a | B-b-113 | | iBuO | H | 1Et-5-Ind | H | C | | 390 (M⁺+1) |
| B-b-115 | 10k-1 | Int.B-290 | | iBuO | Et | 1Me-5-Idz | H | C | | 405 (M⁺+1) |
| B-b-118 | 1-a | B-b-115 | | iBuO | H | 1Me-5-Idz | H | C | | 377 (M⁺+1) |
| B-b-117 | 10k-1 | Int.B-291 | | iBuO | Et | 1Et-5-Idz | H | C | | 419 (M⁺+1) |
| B-b-118 | 1-a | B-b-117 | | iBuO | H | 1Et-5-Idz | H | C | | 391 (M⁺+1) |
| B-b-119 | 10k-1 | Int.B-292 | | 2FBnO | Et | 2-Nap | H | C | | 453 (M⁺+1) |
| B-b-120 | 1-a | B-b-119 | | 2FBnO | H | 2-Nap | H | C | | 425 (M⁺+1) |
| B-b-121 | 10k-1 | Int.B-293 | | 2FBnO | Et | 1Me-4-Ind | H | C | | 456 (M⁺+1) |
| B-b-122 | 1-a | B-b-121 | | 2FBnO | H | 1Me-4-Ind | H | C | | 428 (M⁺+1) |
| B-b-123 | 10k-1 | Int.B-294 | | 2FBnO | Et | 1Me-6-Ind | H | C | | 456 (M⁺+1) |
| B-b-124 | 1-a | B-b-123 | | 2FBnO | H | 1Me-6-Ind | H | C | | 428 (M⁺+1) |
| B-b-125 | 10k-1 | Int.B-295 | | 2FBnO | Et | 1Me-5-Idz | H | C | | 457 (M⁺+1) |
| B-b-126 | 1-a | B-b-125 | | 2FBnO | H | 1Me-5-Idz | H | C | | 429 (M⁺+1) |
| B-b-127 | 10k-1 | Int.B-296 | | 2FBnO | Et | 6-Qu | H | C | | 454 (M⁺+1) |
| B-b-128 | 1-a | B-b-127 | | 2FBnO | H | 6-Qu | H | C | | 426 (M⁺+1) |
| B-b-129 | 10k-1 | Int.B-297 | | 3FBnO | Et | 1Me-5-Ind | H | C | | 456 (M⁺+1) |
| B-b-130 | 1-a | B-b-129 | | 3FBnO | H | 1Me-5-Ind | H | C | | 428 (M⁺+1) |
| B-b-131 | 10k-1 | Int.B-298 | | 3FBnO | Et | 1Et-5-Ind | H | C | | 470 (M⁺+1) |
| B-b-132 | 1-a | B-b-131 | | 3FBnO | H | 1Et-5-Ind | H | C | | 442 (M⁺+1) |
| B-b-133 | 10k-1 | Int.B-299 | | 3FBnO | Et | 1Me-6-Ind | H | C | | 456 (M⁺+1) |
| B-b-134 | 1-a | B-b-133 | | 3FBnO | H | 1Me-6-Ind | H | C | | 428 (M⁺+1) |
| B-b-135 | 10k-1 | Int.B-300 | | 3FBnO | Et | 5-BF | H | C | | 443 (M⁺+1) |
| B-b-136 | 1-a | B-b-135 | | 3FBnO | H | 5-BF | H | C | | 415 (M⁺+1) |
| B-b-137 | 10k-1 | Int.B-301 | | 4FBnO | Et | 2-Nap | H | C | | 453 (M⁺+1) |
| B-b-138 | 1-a | B-b-137 | | 4FBnO | H | 2-Nap | H | C | | 425 (M⁺+1) |
| B-b-139 | 10k-1 | Int.B-302 | | 4FBnO | Et | 1Me-4-Ind | H | C | | 456 (M⁺+1) |
| B-b-140 | 1-a | B-b-139 | | 4FBnO | H | 1Me-4-Ind | H | C | | 428 (M⁺+1) |

**[Table 68]**

| Table-B-b-4 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-b-141 | 10k-1 | Int.B-303 | | 4FBnO | Et | 1Me-6-Ind | H | C | | 456 (M⁺+1) |
| B-b-142 | 1-a | B-b-141 | | 4FBnO | H | 1Me-6-Ind | H | C | | 428 (M⁺+1) |
| B-b-143 | 10k-1 | Int.B-304 | | 4FBnO | Et | 6-Qu | H | C | | 454 (M⁺+1) |
| B-b-144 | 1-a | B-b-143 | | 4FBnO | H | 6-Qu | H | C | | 426 (M⁺+1) |
| B-b-145 | 10k-1 | Int.B-305 | | 2ClBnO | Et | 2-Nap | H | C | | 469 (M⁺+1) |
| B-b-146 | 1-a | B-b-145 | | 2ClBnO | H | 2-Nap | H | C | | 441 (M⁺+1) |
| B-b-147 | 10k-1 | Int.B-306 | | 2ClBnO | Et | 1Et-5-Ind | H | C | | 486 (M⁺+1) |
| B-b-148 | 1-a | B-b-147 | | 2ClBnO | H | 1Et-5-Ind | H | C | | 458 (M⁺+1) |
| B-b-149 | 10k-1 | Int.B-307 | | 2ClBnO | Et | 1Me-5-Idz | H | C | | 473 (M⁺+1) |
| B-b-150 | 1-a | B-b-149 | | 2ClBnO | H | 1Me-5-Idz | H | C | | 445 (M⁺+1) |
| B-b-151 | 10k-1 | Int.B-308 | | 2ClBnO | Et | 1Et-5-Idz | H | C | | 487 (M⁺+1) |
| B-b-152 | 1-a | B-b-151 | | 2ClBnO | H | 1Et-5-Idz | H | C | | 459 (M⁺+1) |
| B-b-153 | 10k-1 | Int.B-309 | | 3ClBnO | Et | 1Me-5-Ind | H | C | | 472 (M⁺+1) |
| B-b-154 | 1-a | B-b-153 | | 3ClBnO | H | 1Me-5-Ind | H | C | | 444 (M⁺+1) |
| B-b-155 | 10k-1 | Int.B-310 | | 3ClBnO | Et | 1Me-6-Ind | H | C | | 472 (M⁺+1) |
| B-b-156 | 1-a | B-b-155 | | 3ClBnO | H | 1Me-6-Ind | H | C | | 444 (M⁺+1) |
| B-b-157 | 10k-1 | Int.B-311 | | 3ClBnO | Et | 1Et-5-Idz | H | C | | 487 (M⁺+1) |
| B-b-158 | 1-a | B-b-157 | | 3ClBnO | H | 1Et-5-Idz | H | C | | 459 (M⁺+1) |
| B-b-159 | 10k-1 | Int.B-312 | | 3ClBnO | Et | 6-IQ | H | C | | 470 (M⁺+1) |
| B-b-160 | 1-a | B-b-159 | | 3ClBnO | H | 6-IQ | H | C | | 442 (M⁺+1) |
| B-b-161 | 10k-1 | Int.B-313 | | 4MeBnO | Et | 2-Nap | H | C | | 449 (M⁺+1) |
| B-b-162 | 1-a | B-b-161 | | 4MeBnO | H | 2-Nap | H | C | | 421 (M⁺+1) |
| B-b-163 | 10k-1 | Int.B-314 | | 4MeBnO | Et | 1Et-5-Ind | H | C | | 466 (M⁺+1) |
| B-b-164 | 1-a | B-b-163 | | 4MeBnO | H | 1Et-5-Ind | H | C | | 438 (M⁺+1) |
| B-b-165 | 10k-1 | Int.B-315 | | 4MeBnO | Et | 1Me-5-Idz | H | C | | 453 (M⁺+1) |
| B-b-166 | 1-a | B-b-165 | | 4MeBnO | H | 1Me-5-Idz | H | C | | 425 (M⁺+1) |
| B-b-167 | 10k-1 | Int.B-316 | | 4MeBnO | Et | 6-Qu | H | C | | 450 (M⁺+1) |
| B-b-168 | 1-a | B-b-167 | | 4MeBnO | H | 6-Qu | H | C | | 422 (M⁺+1) |
| B-b-169 | 10k-1 | Int.B-317 | | 4CF3BnO | Et | 1Me-5-Ind | H | C | | 505 (M⁺+1) |
| B-b-170 | 1-a | B-b-169 | | 4CF3BnO | H | 1Me-5-Ind | H | C | | 477 (M⁺+1) |
| B-b-171 | 10k-1 | Int.B-318 | | 4CF3BnO | Et | 1Me-4-Ind | H | C | | 505 (M⁺+1) |
| B-b-172 | 1-a | B-b-171 | | 4CF3BnO | H | 1Me-4-Ind | H | C | | 477 (M⁺+1) |
| B-b-173 | 10k-1 | Int.B-319 | | 4CF3BnO | Et | 1Et-5-Idz | H | C | | 520 (M⁺+1) |
| B-b-174 | 1-a | B-b-173 | | 4CF3BnO | H | 1Et-5-Idz | H | C | | 492 (M⁺+1) |
| B-b-175 | 10k-1 | Int.B-320 | | 4CF3BnO | Et | 6-Qu | H | C | | 503 (M⁺+1) |
| B-b-176 | 1-a | B-b-175 | | 4CF3BnO | H | 6-Qu | H | C | | 475 (M⁺+1) |
| B-b-177 | 10k-1 | Int.B-321 | | 2EtBuO | Et | 2-Nap | H | C | | 429 (M⁺+1) |
| B-b-178 | 1-a | B-b-177 | | 2EtBuO | H | 2-Nap | H | C | | 401 (M⁺+1) |
| B-b-179 | 10k-1 | Int.B-322 | | 2EtBuO | Et | 1Me-5-Ind | H | C | | 432 (M⁺+1) |
| B-b-180 | 1-a | B-b-179 | | 2EtBuO | H | 1Me-5-Ind | H | C | | 404 (M⁺+1) |
| B-b-181 | 10k-1 | Int.B-323 | | 2EtBuO | Et | 1Et-5-Ind | H | C | | 446 (M⁺+1) |
| B-b-182 | 1-a | B-b-181 | | 2EtBuO | H | 1Et-5-Ind | H | C | | 418 (M⁺+1) |
| B-b-183 | 10k-1 | Int.B-324 | | 2EtBuO | Et | 1Me-5-Idz | H | C | | 433 (M⁺+1) |
| B-b-184 | 1-a | B-b-183 | | 2EtBuO | H | 1Me-5-Idz | H | C | | 405 (M⁺+1) |
| B-b-185 | 10k-1 | Int.B-325 | | 2EtBuO | Et | 6-IQ | H | C | | 430 (M⁺+1) |
| B-b-186 | 1-a | B-b-185 | | 2EtBuO | H | 6-IQ | H | C | | 402 (M⁺+1) |
| B-b-187 | 10k-1 | Int.B-326 | | 2(4DMAPh)EtO | Et | 2-Nap | H | C | | 464 (M⁺+1) |
| B-b-188 | 1-a | B-b-187 | | 2(4DMAPh)EtO | H | 2-Nap | H | C | | 464 (M⁺+1) |

**[Table 69]**

| Table-B-b-5 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-b-189 | 10k-1 | Int.B-327 | | 2(4DMAPh)EtO | Et | 1Me-5-Ind | H | C | | 495 (M⁺+1) |
| B-b-190 | 1-a | B-b-189 | | 2(4DMAPh)EtO | H | 1Me-5-Ind | H | C | | 467 (M⁺+1) |
| B-b-191 | 10k-1 | Int.B-328 | | 2(4DMAPh)EtO | Et | 1Me-4-Ind | H | C | | 495 (M⁺+1) |
| B-b-192 | 1-a | B-b-191 | | 2(4DMAPh)EtO | H | 1Me-4-Ind | H | C | | 467 (M⁺+1) |
| B-b-193 | 10k-1 | Int.B-329 | | 2(4DMAPh)EtO | Et | 1Et-5-Idz | H | C | | 510 (M⁺+1) |
| B-b-194 | 1-a | B-b-193 | | 2(4DMAPh)EtO | H | 1Et-5-Idz | H | C | | 482 (M⁺+1) |
| B-b-195 | 10k-1 | Int.B-330 | | 2(PhO)EtO | Et | 1Me-4-Ind | H | C | | 468 (M⁺+1) |
| B-b-196 | 1-a | B-b-195 | | 2(PhO)EtO | H | 1Me-4-Ind | H | C | | 440 (M⁺+1) |
| B-b-197 | 10k-1 | Int.B-331 | | 2(PhO)EtO | Et | 1Me-6-Ind | H | C | | 468 (M⁺+1) |
| B-b-198 | 1-a | B-b-197 | | 2(PhO)EtO | H | 1Me-6-Ind | H | C | | 440 (M⁺+1) |
| B-b-199 | 10k-1 | Int.B-332 | | 2(PhO)EtO | Et | 1Me-5-Idz | H | C | | 469 (M⁺+1) |
| B-b-200 | 1-a | B-b-199 | | 2(PhO)EtO | H | 1Me-5-Idz | H | C | | 441 (M⁺+1) |
| B-b-201 | 10k-1 | Int.B-333 | | 2(PhO)EtO | Et | 6-Qu | H | C | | 466 (M⁺+1) |
| B-b-202 | 1-a | B-b-201 | | 2(PhO)EtO | H | 6-Qu | H | C | | 438 (M⁺+1) |
| B-b-203 | 10k-1 | Int.B-334 | | 1PhEtO | Et | 2-Nap | H | C | | 449 (M⁺+1) |
| B-b-204 | 1-a | B-b-203 | | 1PhEtO | H | 2-Nap | H | C | | 421 (M⁺+1) |
| B-b-205 | 10k-1 | Int.B-335 | | 1PhEtO | Et | 1Et-5-Ind | H | C | | 466 (M⁺+1) |
| B-b-206 | 1-a | B-b-205 | | 1PhEtO | H | 1Et-5-Ind | H | C | | 438 (M⁺+1) |
| B-b-207 | 10k-1 | Int.B-336 | | 1PhEtO | Et | 1Me-4-Ind | H | C | | 452 (M⁺+1) |
| B-b-208 | 1-a | B-b-207 | | 1PhEtO | H | 1Me-4-Ind | H | C | | 424 (M⁺+1) |
| B-b-209 | 10k-1 | Int.B-337 | | 1PhEtO | Et | 1Me-5-Idz | H | C | | 453 (M⁺+1) |
| B-b-210 | 1-a | B-b-209 | | 1PhEtO | H | 1Me-5-Idz | H | C | | 425 (M⁺+1) |
| B-b-211 | 10k-1 | Int.B-338 | | 1PhEtO | Et | 1Et-5-ldz | H | C | | 467 (M⁺+1) |
| B-b-212 | 1-a | B-b-211 | | 1PhEtO | H | 1Et-5-Idz | H | C | | 439 (M⁺+1) |
| B-b-213 | 10k-1 | Int.B-339 | | 1(2FPh)EtO | Et | 2-Nap | H | C | | 467 (M⁺+1) |
| B-b-214 | 1-a | B-b-213 | | 1(2FPh)EtO | H | 2-Nap | H | C | | 439 (M⁺+1) |
| B-b-215 | 10k-1 | Int.B-340 | | 1(2FPh)EtO | Et | 1Et-5-Ind | H | C | | 484 (M⁺+1) |
| B-b-216 | 1-a | B-b-215 | | 1(2FPh)EtO | H | 1Et-5-Ind | H | C | | 456 (M⁺+1) |
| B-b-217 | 10k-1 | Int.B-341 | | 1(2FPh)EtO | Et | 1Me-5-Idz | H | C | | 471 (M⁺+1) |
| B-b-218 | 1-a | B-b-217 | | 1(2FPh)EtO | H | 1Me-5-Idz | H | C | | 443 (M⁺+1) |
| B-b-219 | 10k-1 | Int.B-342 | | 1(2FPh)EtO | Et | 6-IQ | H | C | | 468 (M⁺+1) |
| B-b-220 | 1-a | B-b-219 | | 1(2FPh)EtO | H | 6-IQ | H | C | | 440 (M⁺+1) |
| B-b-221 | 10k-1 | Int.B-343 | | 1(4ClPh)EtO | Et | 1Me-5-Ind | H | C | | 487 (M⁺+1) |
| B-b-222 | 1-a | B-b-221 | | 1(4ClPh)EtO | H | 1Me-5-Ind | H | C | | 458 (M⁺+1) |
| B-b-223 | 10k-1 | Int.B-344 | | 1(4ClPh)EtO | Et | 1Me-4-Ind | H | C | | 487 (M⁺+1) |
| B-b-224 | 1-a | B-b-223 | | 1(4ClPh)EtO | H | 1Me-4-Ind | H | C | | 458 (M⁺+1) |
| B-b-225 | 10k-1 | Int.B-345 | | 1(4ClPh)EtO | Et | 1Me-6-Ind | H | C | | 487 (M⁺+1) |
| B-b-226 | 1-a | B-b-225 | | 1(4ClPh)EtO | H | 1Me-6-Ind | H | C | | 458 (M⁺+1) |
| B-b-227 | 10k-1 | Int.B-346 | | 1(4ClPh)EtO | Et | 1Et-5-Idz | H | C | | 502 (M⁺+1) |
| B-b-228 | 1-a | B-b-227 | | 1(4ClPh)EtO | H | 1Et-5-Idz | H | C | | 473 (M⁺+1) |
| B-b-229 | 10k-1 | Int.B-347 | | 4Me,cHexO | Et | 2-Nap | H | C | | 441 (M⁺+1) |
| B-b-230 | 1-a | B-b-229 | | 4Me,cHexO | H | 2-Nap | H | C | | 413 (M⁺+1) |
| B-b-231 | 10k-1 | Int.B-348 | | 4Me,cHexO | Et | 1Et-5-Ind | H | C | | 458 (M⁺+1) |
| B-b-232 | 1-a | B-b-231 | | 4Me,cHexO | H | 1Et-5-Ind | H | C | | 430 (M⁺+1) |
| B-b-233 | 10k-1 | Int.B-349 | | 4Me,cHexO | Et | 1Me-5-Idz | H | C | | 445 (M⁺+1) |
| B-b-234 | 1-a | B-b-233 | | 4Me,cHexO | H | 1Me-5-Idz | H | C | | 417 (M⁺+1) |
| B-b-235 | 10k-1 | Int.B-350 | | 4Me,cHexO | Et | 5-B F | H | C | | 431 (M⁺+1) |
| B-b-236 | 1-a | B-b-235 | | 4Me,cHexO | H | 5-BF | H | C | | 403 (M⁺+1) |

**[Table 70]**

| Table-B-b-6 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-b-237 | 10k-1 | Int.B-351 | | 1IndanO | Et | 2-Nap | H | C | | 461 (M⁺+1) |
| B-b-238 | 1-a | B-b-237 | | 1IndanO | H | 2-Nap | H | C | | 433 (M⁺+1) |
| B-b-239 | 10k-1 | Int.B-352 | | 1IndanO | Et | 1Me-5-Ind | H | C | | 464 (M⁺+1) |
| B-b-240 | 1-a | B-b-239 | | 1IndanO | H | 1Me-5-Ind | H | C | | 436 (M⁺+1) |
| B-b-241 | 10k-1 | Int.B-353 | | 1IndanO | Et | 1Me-4-Ind | H | C | | 464 (M⁺+1) |
| B-b-242 | 1-a | B-b-241 | | 1IndanO | H | 1Me-4-Ind | H | C | | 436 (M⁺+1) |
| B-b-243 | 10k-1 | Int.B-354 | | 1IndanO | Et | 1Me-5-Idz | H | C | | 465 (M⁺+1) |
| B-b-244 | 1-a | B-b-243 | | 1IndanO | H | 1Me-5-Idz | H | C | | 437 (M⁺+1) |
| B-b-245 | 10k-1 | Int.B-355 | | 1IndanO | Et | 6-Qu | H | C | | 462 (M⁺+1) |
| B-b-246 | 1-a | B-b-245 | | 1IndanO | H | 6-Qu | H | C | | 434 (M⁺+1) |
| B-b-247 | 10k-1 | Int.B-356 | | 2IndanO | Et | 2-Nap | H | C | | 461 (M⁺+1) |
| B-b-248 | 1-a | B-b-247 | | 2IndanO | H | 2-Nap | H | C | | 433 (M⁺+1) |
| B-b-249 | 10k-1 | Int.B-357 | | 2IndanO | Et | 1 Me-5-Ind | H | C | | 464 (M⁺+1) |
| B-b-250 | 1-a | B-b-249 | | 2IndanO | H | 1 Me-5-Ind | H | C | | 436 (M⁺+1) |
| B-b-251 | 10k-1 | Int.B-358 | | 2IndanO | Et | 1Et-5-Ind | H | C | | 478 (M⁺+1) |
| B-b-252 | 1-a | B-b-251 | | 2IndanO | H | 1Et-5-Ind | H | C | | 450 (M⁺+1) |
| B-b-253 | 10k-1 | Int.B-359 | | 2IndanO | Et | 1Me-5-Idz | H | C | | 465 (M⁺+1) |
| B-b-254 | 1-a | B-b-253 | | 2IndanO | H | 1Me-5-Idz | H | C | | 437 (M⁺+1) |
| B-b-255 | 10k-1 | Int.B-360 | | 2IndanO | Et | 1Et-5-Idz | H | C | | 479 (M⁺+1) |
| B-b-256 | 1-a | B-b-255 | | 2IndanO | H | 1Et-5-Idz | H | C | | 451 (M⁺+1) |
| B-b-257 | 10k-1 | Int.B-361 | | 2IndanO | Et | 6-IQ | H | C | | 462 (M⁺+1) |
| B-b-258 | 1-a | B-b-257 | | 2IndanO | H | 6-IQ | H | C | | 434 (M⁺+1) |
| B-b-259 | 10k-1 | Int.B-362 | | 5OMe-2-IndanO | Et | 2-Nap | H | C | | 491 (M⁺+1) |
| B-b-260 | 1-a | B-b-259 | | 5OMe-2-IndanO | H | 2-Nap | H | C | | 463 (M⁺+1) |
| B-b-261 | 10k-1 | Int.B-363 | | 5OMe-2-IndanO | Et | 1Et-5-Ind | H | C | | 508 (M⁺+1) |
| B-b-262 | 1-a | B-b-261 | | 5OMe-2-IndanO | H | 1Et-5-Ind | H | C | | 480 (M⁺+1) |
| B-b-263 | 10k-1 | Int.B-364 | | 5OMe-2-IndanO | Et | 1Me-5-Idz | H | C | | 495 (M⁺+1) |
| B-b-264 | 1-a | B-b-263 | | 5OMe-2-IndanO | H | 1Me-5-Idz | H | C | | 467 (M⁺+1) |
| B-b-265 | 10k-1 | Int.B-365 | | 5OMe-2-IndanO | Et | 6-Qu | H | C | | 492 (M⁺+1) |
| B-b-266 | 1-a | B-b-265 | | 5OMe-2-IndanO | H | 6-Qu | H | C | | 464 (M⁺+1) |
| B-b-267 | 10k-1 | Int.B-366 | | 5,6D(OMe)-2-Indano | Et | 1Me-5-Ind | H | C | | 524 (M⁺+1) |
| B-b-268 | 1-a | B-b-267 | | 5,6D(OMe)-2-IndanO | H | 1Me-5-Ind | H | C | | 496 (M⁺+1) |
| B-b-269 | 10k-1 | Int.B-367 | | 5,6D(OMe)-2-IndanO | Et | 1Me-4-Ind | H | C | | 524 (M⁺+1) |
| B-b-270 | 1-a | B-b-269 | | 5,6D(OMe)-2-IndanO | H | 1Me-4-Ind | H | C | | 496 (M⁺+1) |
| B-b-271 | 10k-1 | Int.B-368 | | 5,6D(OMe)-2-IndanO | Et | 1Et-5-Idz | H | C | | 539 (M⁺+1) |
| B-b-272 | 1-a | B-b-271 | | 5,6D(OMe)-2-IndanO | H | 1Et-5-Idz | H | C | | 511 (M⁺+1) |
| B-b-273 | 10k-1 | Int.B-369 | | 5,6D(OMe)-2-IndanO | Et | 6-IQ | H | C | | 522 (M⁺+1) |
| B-b-274 | 1-a | B-b-273 | | 5,6D(OMe)-2-IndanO | H | 6-IQ | H | C | | 494 (M⁺+1) |
| B-b-275 | 10k-1 | Int.B-370 | | 5F-2-IndanO | Et | 2-Nap | H | C | | 479 (M⁺+1) |
| B-b-276 | 1-a | B-b-275 | | 5F-2-IndanO | H | 2-Nap | H | C | | 451 (M⁺+1) |
| B-b-277 | 10k-1 | Int.B-371 | | 5F-2-IndanO | Et | 1Me-5-Ind | H | C | | 482 (M⁺+1) |
| B-b-278 | 1-a | B-b-277 | | 5F-2-IndanO | H | 1Me-5-Ind | H | C | | 454 (M⁺+1) |
| B-b-279 | 10k-1 | Int.B-372 | | 5F-2-IndanO | Et | 1Me-5-Idz | H | C | | 483 (M⁺+1) |
| B-b-280 | 1-a | B-b-279 | | 5F-2-IndanO | H | 1Me-5-Idz | H | C | | 455 (M⁺+1) |
| B-b-281 | 10k-1 | Int.B-373 | | 5F-2-IndanO | Et | 1Et-5-Idz | H | C | | 497 (M⁺+1) |
| B-b-282 | 1-a | B-b-281 | | 5F-2-IndanO | H | 1Et-5-Idz | H | C | | 469 (M⁺+1) |

**[Table 71]**

| Tbble-B-b-7 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-b-283 | 10k-1 | Int.B-374 | | 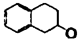 | Et | 2-Nap | H | C | | 475 (M⁺+1) |
| B-b-284 | 1-a | B-b-283 | | 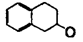 | H | 2-Nap | H | C | | 447 (M⁺+1) |
| B-b-285 | 10k-1 | Int.B-375 | | 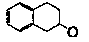 | Et | 1Me-5-Ind | H | C | | 478 (M⁺+1) |
| B-b-286 | 1-a | B-b-285 | | 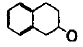 | H | 1Me-5-Ind | H | C | | 450 (M⁺+1) |
| B-b-287 | 10k-1 | Int.B-376 | | 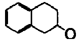 | Et | 1Me-6-Ind | H | C | | 478 (M⁺+1) |
| B-b-288 | 1-a | B-b-287 | | 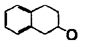 | H | 1Me-6-Ind | H | C | | 450 (M⁺+1) |
| B-b-289 | 10k-1 | Int.B-377 | | 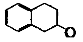 | Et | 1Me-5-Idz | H | C | | 479 (M⁺+1) |
| B-b-290 | 1-a | B-b-289 | | 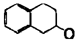 | H | 1Me-5-Idz | H | C | | 451 (M⁺+1) |
| B-b-291 | 10k-1 | Int.B-378 | | 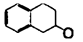 | Et | 6-Qu | H | C | | 476 (M⁺+1) |
| B-b-292 | 1-a | B-b-291 | | 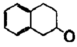 | H | 6-Qu | H | C | | 448 (M⁺+1) |
| B-b-293 | 10k-1 | Int.B-379 | | 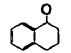 | Et | 1Me-5-Ind | H | C | | 478 (M⁺+1) |
| B-b-294 | 1-a | B-b-293 | | 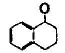 | H | 1Me-5-Ind | H | C | | 450 (M⁺+1) |
| B-b-295 | 10k-1 | Int.B-380 | | 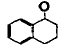 | Et | 1Et-5-Ind | H | C | | 492 (M⁺+1) |
| B-b-296 | 1-a | B-b-295 | | 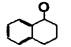 | H | 1Et-5-Ind | H | C | | 464 (M⁺+1) |
| B-b-297 | 10k-1 | Int.B-381 | | 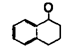 | Et | 1Me-4-Ind | H | C | | 478 (M⁺+1) |
| B-b-298 | 1-a | B-b-297 | | 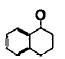 | H | 1Me-4-Ind | H | C | | 450 (M⁺+1) |
| B-b-299 | 10k-1 | Int.B-382 | | 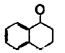 | Et | 1Et-5-Idz | H | C | | 493 (M⁺+1) |
| B-b-300 | 1-a | B-b-299 | | 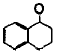 | H | 1Et-5-Idz | H | C | | 465 (M⁺+1) |
| B-b-301 | 10k-1 | Int.B-383 | | 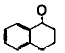 | Et | 5-B F | H | C | | 465 (M⁺+1) |
| B-b-302 | 1-a | B-b-301 | | 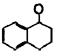 | H | 5-B F | H | C | | 437 (M⁺+1) |
| B-b-303 | 10k-1 | Int.B-384 | | 2(2MePh)EtO | Et | 2-Nap | H | C | | 463 (M⁺+1) |
| B-b-304 | 1-a | B-b-303 | | 2(2MePh)EtO | H | 2-Nap | H | C | | 435 (M⁺+1) |
| B-b-305 | 10k-1 | Int.B-385 | | 2(2MePh)EtO | Et | 1Et-5-Ind | H | C | | 480 (M⁺+1) |
| B-b-306 | 1-a | B-b-305 | | 2(2MePh)EtO | H | 1Et-5-Ind | H | C | | 452 (M⁺+1) |
| B-b-307 | 10k-1 | Int.B-386 | | 2(2MePh)EtO | Et | 1 Me-6-Ind | H | C | | 466 (M⁺+1) |
| B-b-308 | 1-a | B-b-307 | | 2(2MePh)EtO | H | 1Me-6-Inc | H | C | | 438 (M⁺+1) |
| B-b-309 | 10k-1 | Int.B-387 | | 2(2MePh)EtO | Et | 1Et-5-Idz | H | C | | 481 (M⁺+1) |
| B-b-310 | 1-a | B-b-309 | | 2(2MePh)EtO | H | 1Et-5-Idz | H | C | | 453 (M⁺+1) |

**[Table 72]**

| Table-B-b-8 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-b-311 | 10k-1 | Int.B-388 | | 2(3FPh)EtO | Et | 1Me-5-Ind | H | C | | 470 (M⁺+1) |
| B-b-312 | 1-a | B-b-311 | | 2(3FPh)EtO | H | 1Me-5-Ind | H | C | | 442 (M⁺+1) |
| B-b-313 | 10k-1 | Int.B-389 | | 2(3FPh)EtO | Et | 1 Me-4-Ind | H | C | | 470 (M⁺+1) |
| B-b-314 | 1-a | B-b-313 | | 2(3FPh)EtO | H | 1Me-4-Ind | H | C | | 442 (M⁺+1) |
| B-b-315 | 10k-1 | Int.B-390 | | 2(3FPh)EtO | Et | 1Me-5-Idz | H | C | | 471 (M⁺+1) |
| B-b-316 | 1-a | B-b-315 | | 2(3FPh)EtO | H | 1Me-5-Idz | H | C | | 443 (M⁺+1) |
| B-b-317 | 10k-1 | Int.B-391 | | 2(3FPh)EtO | Et | 6-IQ | H | C | | 468 (M⁺+1) |
| B-b-318 | 1-a | B-b-317 | | 2(3FPh)EtO | H | 6-IQ | H | C | | 440 (M⁺+1) |
| B-b-319 | 10k-1 | Int.B-392 | | 2(2ClPh)EtO | Et | 2-Nap | H | C | | 484 (M⁺+1) |
| B-b-320 | 1-a | B-b-319 | | 2(2ClPh)EtO | H | 2-Nap | H | C | | 455 (M⁺+1) |
| B-b-321 | 10k-1 | Int.B-393 | | 2(2ClPh)EtO | Et | 1 Et-5-Ind | H | C | | 501 (M⁺+1) |
| B-b-322 | 1-a | B-b-321 | | 2(2ClPh)EtO | H | 1Et-5-Ind | H | C | | 473 (M⁺+1) |
| B-b-323 | 10k-1 | Int.B-394 | | 2(2ClPh)EtO | Et | 1Me-4-Ind | H | C | | 487 (M⁺+1) |
| B-b-324 | 1-a | B-b-323 | | 2(2ClPh)EtO | H | 1Me-4-Ind | H | C | | 458 (M⁺+1) |
| B-b-325 | 10k-1 | Int.B-395 | | 2(2ClPh)EtO | Et | 1Me-6-Ind | H | C | | 487 (M⁺+1) |
| B-b-326 | 1-a | B-b-325 | | 2(2ClPh)EtO | H | 1Me-6-Ind | H | C | | 458 (M⁺+1) |
| B-b-327 | 10k-1 | Int.B-396 | | 2(2ClPh)EtO | Et | 1Et-5-Idz | H | C | | 502 (M⁺+1) |
| B-b-328 | 1-a | B-b-327 | | 2(2ClPh)EtO | H | 1Et-5-Idz | H | C | | 473 (M⁺+1) |
| B-b-329 | 10k-1 | Int.B-397 | | 2(2ClPh)EtO | Et | 5-BF | H | C | | 473 (M⁺+1) |
| B-b-330 | 1-a | B-b-329 | | 2(2ClPh)EtO | H | 5-BF | H | C | | 445 (M⁺+1) |
| B-b-331 | 10k-1 | Int.B-398 | | 2(1-Nap)EtO | Et | 2-Nap | H | C | | 499 (M⁺+1) |
| B-b-332 | 1-a | B-b-331 | | 2(1-Nap)EtO | H | 2-Nap | H | C | | 471 (M⁺+1) |
| B-b-333 | 10k-1 | Int.B-399 | | 2(1-Nap)EtO | Et | 1Me-5-Ind | H | C | | 502 (M⁺+1) |
| B-b-334 | 1-a | B-b-333 | | 2(1-Nap)EtO | H | 1Me-5-Ind | H | C | | 474 (M⁺+1) |
| B-b-335 | 10k-1 | Int.B-400 | | 2(1-Nap)EtO | Et | 1Me-5-Idz | H | C | | 503 (M⁺+1) |
| B-b-336 | 1-a | B-b-335 | | 2(1-Nap)EtO | H | 1Me-5-Idz | H | C | | 475 (M⁺+1) |
| B-b-337 | 10k-1 | Int.B-401 | | 2(1-Nap)EtO | Et | 6-Qu | H | C | | 500 (M⁺+1) |
| B-b-338 | 1-a | B-b-337 | | 2(1-Nap)EtO | H | 6-Qu | H | C | | 472 (M⁺+1) |
| B-b-339 | 10k-1 | Int.B-402 | | 2(1-Nap)EtO | Et | 6-IQ | H | C | | 500 (M⁺+1) |
| B-b-340 | 1-a | B-b-339 | | 2(1-Nap)EtO | H | 6-IQ | H | C | | 472 (M⁺+1) |
| B-b-341 | 10k-1 | Int.B-403 | | 2(PhS)EtO | Et | 1Me-5-Ind | H | C | | 484 (M⁺+1) |
| B-b-342 | 1-a | B-b-341 | | 2(PhS)EtO | H | 1Me-5-Ind | H | C | | 456 (M⁺+1) |
| B-b-343 | 10k-1 | Int.B-404 | | 2(PhS)EtO | Et | 1Me-6-Ind | H | C | | 484 (M⁺+1) |
| B-b-344 | 1-a | B-b-343 | | 2(PhS)EtO | H | 1 Me-6-Ind | H | C | | 456 (M⁺+1) |
| B-b-345 | 10k-1 | Int.B-405 | | 2(PhS)EtO | Et | 1Et-5-Idz | H | C | | 499 (M⁺+1) |
| B-b-346 | 1-a | B-b-345 | | 2(PhS)EtO | H | 1 Et-5-Idz | H | C | | 471 (M⁺+1) |
| B-b-347 | 10k-1 | Int.B-406 | | 2(PhS)EtO | Et | 6-Qu | H | C | | 482 (M⁺+1) |
| B-b-348 | 1-a | B-b-347 | | 2(PhS)EtO | H | 6-Qu | H | C | | 454 (M⁺+1) |

### Example B-c-1

### Synthesis of ethyl 2-[5-cyclopentyloxy-4-(naphthalen-2-yl)-2,3-dihydro-1H-inden-1-yl]acetate (Compound No. B-c-1) (Preparation Method 9, Step j)

According to the procedure described in the synthetic method of Intermediate A-3 in Reference Example 1 (Preparation Method 9, Step j) provided that the reaction was performed for 1.5 hours, Example Compound B-b-1 (121.4 mg) and Pd/C (26.6 mg, Merck) were reacted and treated to obtain the title compound (Compound No. B-c-1, 108.6 mg).

### Example B-c-2

### Synthesis of 2-[5-cyclopentyloxy-4-(naphthalen-2-yl)-2,3-dihydro-1H-inden-1-yl]acetic acid (Compound No. B-c-2) (Preparation Method 1, Step a)

According to the procedure described in the synthetic method of Example Compound A-a-2 (Preparation Method 1, Step a) provided that the reaction was performed for 14 hours, Example Compound B-c-1 (105.2 mg) and 2 N aqueous sodium hydroxide (0.63 ml) were reacted and treated to obtain the title compound (Compound No. B-c-2, 95.8 mg).

Typical examples of the compounds of the present invention that can be obtained by reacting and treating corresponding starting compounds using any of the methods described in the present specification including the examples described above are shown in Table-B-c-1 to Table-B-c-6. The compounds were prepared according to the preparation methods of the compounds of the compound numbers shown in the columns of "Syn" in the tables. "Int" means an intermediate compound number. In the tables, used methods among the aforementioned synthetic methods are shown in the columns of "Syn" with symbols, the starting compounds are shown in the columns of "SM", and the reagents are shown in the columns of "Regent".

**[Table 73]**

| Table-B-c-1 | | | | | | | | | | |
|---|---|---|---|---|---|---|---|---|---|---|
| 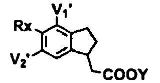 | | | | | | | | | | |

| Exp. | Syn. | SM | Reagent | Rx | Y | V1' | V2' | LCMS | | |
|---|---|---|---|---|---|---|---|---|---|---|
| | | | | | | | | method | RTime | Mass |
| B-c-1 | 9-j | B-b-1 | | cPenO | Et | 2-Nap | H | C | | 415 (M⁺+1) |
| B-c-2 | 1-a | B-c-1 | | cPenO | H | 2-Nap | H | C | | 387 (M⁺+1) |
| B-c-3 | 9-j | B-b-3 | | cPenO | Et | 1Me-5-Ind | H | C | | 418 (M⁺+1) |
| B-c-4 | 1-a | B-c-3 | | cPenO | H | 1Me-5-Ind | H | C | | 390 (M⁺+1) |
| B-c-5 | 9-j | B-b-5 | | cPenO | Et | 1Et-5-Ind | H | C | | 432 (M⁺+1) |
| B-c-6 | 1-a | B-c-5 | | cPenO | H | 1Et-5-Ind | H | C | | 404 (M⁺+1) |
| B-c-7 | 9-j | B-b-7 | | cPenO | Et | 1Me-4-Ind | H | C | | 418 (M⁺+1) |
| B-c-8 | 1-a | B-c-7 | | cPenO | H | 1Me-4-Ind | H | C | | 390 (M⁺+1) |
| B-c-9 | 9-j | B-b-9 | | cPenO | Et | 1Me-6-Ind | H | C | | 418 (M⁺+1) |
| B-c-10 | 1-a | B-c-9 | | cPenO | H | 1Me-6-Ind | H | C | | 390 (M⁺+1) |
| B-c-11 | 9-j | B-b-11 | | cPenO | Et | 1Me-5-Idz | H | C | | 419 (M⁺+1) |
| B-c-12 | 1-a | B-c-11 | | cPenO | H | 1Me-5-Idz | H | C | | 391 (M⁺+1) |
| B-c-13 | 9-j | B-b-13 | | cPenO | Et | 1Et-5-Idz | H | C | | 433 (M⁺+1) |
| B-c-14 | 1-a | B-c-13 | | cPenO | H | 1Et-5-Idz | H | C | | 405 (M⁺+1) |
| B-c-15 | 9-j | B-b-15 | | cPenO | Et | 5-BF | H | C | | 405 (M⁺+1) |
| B-c-16 | 1-a | B-c-15 | | cPenO | H | 5-BF | H | C | | 377 (M⁺+1) |
| B-c-17 | 9-j | B-b-17 | | cPenO | Et | 6-Qu | H | C | | 416 (M⁺+1) |
| B-c-18 | 1-a | B-c-17 | | cPenO | H | 6-Qu | H | C | | 388 (M⁺+1) |
| B-c-19 | 9-j | B-b-19 | | cPenO | Et | 6-IQ | H | C | | 416 (M⁺+1) |
| B-c-20 | 1-a | B-c-19 | | cPenO | H | 6-IQ | H | C | | 388 (M⁺+1) |
| B-c-21 | 9-j | B-b-41 | | cPenMeO | Et | 2-Nap | H | C | | 429 (M⁺+1) |
| B-c-22 | 1-a | B-c-21 | | cPenMeO | H | 2-Nap | H | C | | 401 (M⁺+1) |
| B-c-23 | 9-j | B-b-43 | | cPenMeO | Et | 1Me-5-Ind | H | C | | 432 (M⁺+1) |
| B-c-24 | 1-a | B-c-23 | | cPenMeO | H | 1Me-5-Ind | H | C | | 404 (M⁺+1) |
| B-c-25 | 9-j | B-b-45 | | cPenMeO | Et | 1Me-6-Ind | H | C | | 432 (M⁺+1) |
| B-c-26 | 1-a | B-c-25 | | cPenMeO | H | 1Me-6-Ind | H | C | | 404 (M⁺+1) |
| B-c-27 | 9-j | B-b-47 | | cPenMeO | Et | 1Me-5-Idz | H | C | | 433 (M⁺+1) |
| B-c-28 | 1-a | B-c-27 | | cPenMeO | H | 1Me-5-Idz | H | C | | 405 (M⁺+1) |
| B-c-29 | 9-j | B-b-49 | | cPenMeO | Et | 1 Et-5-Idz | H | C | | 447 (M⁺+1) |
| B-c-30 | 1-a | B-c-29 | | cPenMeO | H | 1Et-5-Idz | H | C | | 419 (M⁺+1) |
| B-c-31 | 9-j | B-b-51 | | cPenMeO | Et | 6-Qu | H | C | | 430 (M⁺+1) |
| B-c-32 | 1-a | B-c-31 | | cPenMeO | H | 6-Qu | H | C | | 402 (M⁺+1) |
| B-c-33 | 9-j | B-b-53 | | cHexMeO | Et | 2-Nap | H | C | | 443 (M⁺+1) |
| B-c-34 | 1-a | B-c-33 | | cHexMeO | H | 2-Nap | H | C | | 415 (M⁺+1) |
| B-c-35 | 9-j | B-b-55 | | cHexMeO | Et | 1Et-5-Ind | H | C | | 460 (M⁺+1) |
| B-c-36 | 1-a | B-c-35 | | cHexMeO | H | 1Et-5-Ind | H | C | | 432 (M⁺+1) |
| B-c-37 | 9-j | B-b-57 | | cHexMeO | Et | 1Me-4-Ind | H | C | | 446 (M⁺+1) |
| B-c-38 | 1-a | B-c-37 | | cHexMeO | H | 1Me-4-Ind | H | C | | 418 (M⁺+1) |
| B-c-39 | 9-j | B-b-59 | | cHexMeO | Et | 1Me-5-Idz | H | C | | 447 (M⁺+1) |
| B-c-40 | 1-a | B-c-39 | | cHexMeO | H | 1Me-5-Idz | H | C | | 419 (M⁺+1) |
| B-c-41 | 9-j | B-b-61 | | cHexMeO | Et | 1Et-5-Idz | H | C | | 461 (M⁺+1) |
| B-c-42 | 1-a | B-c-41 | | cHexMeO | H | 1Et-5-Idz | H | C | | 433 (M⁺+1) |
| B-c-43 | 9-j | B-b-63 | | cHexMeO | Et | 5-BF | H | C | | 433 (M⁺+1) |
| B-c-44 | 1-a | B-c-43 | | cHexMeO | H | 5-BF | H | C | | 405 (M⁺+1) |

**[Table 74]**

| Table-B-c-2 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-c-45 | 9-j | B-b-65 | | cHexMeO | Et | 6-IQ | H | C | | 444 (M⁺+1) |
| B-c-46 | 1-a | B-c-45 | | cHexMeO | H | 6-IQ | H | C | | 416 (M⁺+1) |
| B-c-47 | 9-j | B-b-67 | | cHexO | Et | 2-Nap | H | C | | 429 (M⁺+1) |
| B-c-48 | 1-a | B-c-47 | | cHexO | H | 2-Nap | H | C | | 401 (M⁺+1) |
| B-c-49 | 9-j | B-b-69 | | cHexO | Et | 1Me-5-Ind | H | C | | 432 (M⁺+1) |
| B-c-50 | 1-a | B-c-49 | | cHexO | H | 1Me-5-Ind | H | C | | 404 (M⁺+1) |
| B-c-51 | 9-j | B-b-73 | | cHexO | Et | 1Me-4-Ind | H | C | | 432 (M⁺+1) |
| B-c-52 | 1-a | B-c-51 | | cHexO | H | 1Me-4-Ind | H | C | | 404 (M⁺+1) |
| B-c-53 | 9-j | B-b-75 | | cHexO | Et | 1Me-5-Idz | H | C | | 433 (M⁺+1) |
| B-c-54 | 1-a | B-c-53 | | cHexO | H | 1Me-5-Idz | H | C | | 405 (M⁺+1) |
| B-c-55 | 9-j | B-b-77 | | cHexO | Et | 1Et-5-Idz | H | C | | 447 (M⁺+1) |
| B-c-56 | 1-a | B-c-55 | | cHexO | H | 1Et-5-Idz | H | C | | 419 (M⁺+1) |
| B-c-57 | 9-j | B-b-81 | | cHepO | Et | 2-Nap | H | C | | 443 (M⁺+1) |
| B-c-58 | 1-a | B-c-57 | | cHepO | H | 2-Nap | H | C | | 415 (M⁺+1) |
| B-c-59 | 9-j | B-b-83 | | cHepO | Et | 1Me-5-Ind | H | C | | 446 (M⁺+1) |
| B-c-60 | 1-a | B-c-59 | | cHepO | H | 1Me-5-Ind | H | C | | 418 (M⁺+1) |
| B-c-61 | 9-j | B-b-85 | | cHepO | Et | 1Me-5-Idz | H | C | | 447 (M⁺+1) |
| B-c-62 | 1-a | B-c-61 | | cHepO | H | 1Me-5-Idz | H | C | | 419 (M⁺+1) |
| B-c-63 | 9-j | B-b-87 | | cHepO | Et | 6-IQ | H | C | | 444 (M⁺+1) |
| B-c-64 | 1-a | B-c-63 | | cHepO | H | 6-IQ | H | C | | 416 (M⁺+1) |
| B-c-65 | 9-j | B-b-89 | | nPrO | Et | 1Et-5-Ind | H | C | | 406 (M⁺+1) |
| B-c-66 | 1-a | B-c-65 | | nPrO | H | 1Et-5-Ind | H | C | | 378 (M⁺+1) |
| B-c-67 | 9-j | B-b-93 | | nPrO | Et | 1Et-5-Idz | H | C | | 407 (M⁺+1) |
| B-c-68 | 1-a | B-c-67 | | nPrO | H | 1Et-5-Idz | H | C | | 379 (M⁺+1) |
| B-c-69 | 9-j | B-b-95 | | nPrO | Et | 6-Qu | H | C | | 390 (M⁺+1) |
| B-c-70 | 1-a | B-c-69 | | nPrO | H | 6-Qu | H | C | | 362 (M⁺+1) |
| B-c-71 | 9-j | B-b-97 | | iPrO | Et | 2-Nap | H | C | | 389 (M⁺+1) |
| B-c-72 | 1-a | B-c-71 | | iPrO | H | 2-Nap | H | C | | 361 (M⁺+1) |
| B-c-73 | 9-j | B-b-99 | | iPrO | Et | 1Et-5-Ind | H | C | | 406 (M⁺+1) |
| B-c-74 | 1-a | B-c-73 | | iPrO | H | 1Et-5-Ind | H | C | | 378 (M⁺+1) |
| B-c-75 | 9-j | B-b-101 | | iPrO | Et | 1Me-5-Idz | H | C | | 393 (M⁺+1) |
| B-c-76 | 1-a | B-c-75 | | iPrO | H | 1Me-5-Idz | H | C | | 365 (M⁺+1) |
| B-c-77 | 9-j | B-b-103 | | iPrO | Et | 6-IQ | H | C | | 390 (M⁺+1) |
| B-c-78 | 1-a | B-c-77 | | iPrO | H | 6-IQ | H | C | | 362 (M⁺+1) |
| B-c-79 | 9-j | B-b-105 | | nBuO | Et | 2-Nap | H | C | | 403 (M⁺+1) |
| B-c-80 | 1-a | B-c-79 | | nBuO | H | 2-Nap | H | C | | 375 (M⁺+1) |
| B-c-81 | 9-j | B-b-107 | | nBuO | Et | 1Me-5-Ind | H | C | | 406 (M⁺+1) |
| B-c-82 | 1-a | B-c-81 | | nBuO | H | 1Me-5-Ind | H | C | | 378 (M⁺+1) |
| B-c-83 | 9-j | B-b-109 | | nBuO | Et | 1Et-5-Idz | H | C | | 421 (M⁺+1) |
| B-c-84 | 1-a | B-c-83 | | nBuO | H | 1Et-5-Idz | H | C | | 393 (M⁺+1) |
| B-c-85 | 9-j | B-b-111 | | iBuO | Et | 1Me-5-Ind | H | C | | 406 (M⁺+1) |
| B-c-86 | 1-a | B-c-85 | | iBuO | H | 1Me-5-Ind | H | C | | 378 (M⁺+1) |
| B-c-87 | 9-j | B-b-113 | | iBuO | Et | 1Et-5-Ind | H | C | | 420 (M⁺+1) |
| B-c-88 | 1-a | B-c-87 | | iBuO | H | 1Et-5-Ind | H | C | | 392 (M⁺+1) |
| B-c-89 | 9-j | B-b-115 | | iBuO | Et | 1Me-5-Idz | H | C | | 407 (M⁺+1) |
| B-c-90 | 1-a | B-c-89 | | iBuO | H | 1Me-5-Idz | H | C | | 379 (M⁺+1) |
| B-c-91 | 9-j | B-b-117 | | iBuO | Et | 1Et-5-Idz | H | C | | 421 (M⁺+1) |
| B-c-92 | 1-a | B-c-91 | | iBuO | H | 1Et-5-Idz | H | C | | 393 (M⁺+1) |

**[Table 75]**

| Tacle-B-c-3 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-c-93 | 9-j | B-b-177 | | 2EtBuO | Et | 2-Nap | H | C | | 431 (M⁺+1) |
| B-c-94 | 1-a | B-c-93 | | 2EtBuO | H | 2-Nap | H | C | | 403 (M⁺+1) |
| B-c-95 | 9-j | B-b-179 | | 2EtBuO | Et | 1Me-5-Ind | H | C | | 434 (M⁺+1) |
| B-c-96 | 1-a | B-c-95 | | 2EtBuO | H | 1Me-5-Ind | H | C | | 406 (M⁺+1) |
| B-c-97 | 9-j | B-b-181 | | 2EtBuO | Et | 1Et-5-Ind | H | C | | 448 (M⁺+1) |
| B-c-98 | 1-a | B-c-97 | | 2EtBuO | H | 1Et-5-Ind | H | C | | 420 (M⁺+1) |
| B-c-99 | 9-j | B-b-183 | | 2EtBuO | Et | 1Me-5-Idz | H | C | | 435 (M⁺+1) |
| B-c-100 | 1-a | B-c-99 | | 2EtBuO | H | 1Me-5-Idz | H | C | | 407 (M⁺+1) |
| B-c-101 | 9-j | B-b-185 | | 2EtBuO | Et | 6-IQ | H | C | | 432 (M⁺+1) |
| B-c-102 | 1-a | B-c-101 | | 2EtBuO | H | 6-IQ | H | C | | 404 (M⁺+1) |
| B-c-103 | 9-j | B-b-187 | | 2(4DMAPh)EtO | Et | 2-Nap | H | C | | 494 (M⁺+1) |
| B-c-104 | 1-a | B-c-103 | | 2(4DMAPh)EtO | H | 2-Nap | H | C | | 466 (M⁺+1) |
| B-c-105 | 9-j | B-b-189 | | 2(4DMAPh)EtO | Et | 1Me-5-Ind | H | C | | 497 (M⁺+1) |
| B-c-106 | 1-a | B-c-105 | | 2(4DMAPh)EtO | H | 1Me-5-Ind | H | C | | 469 (M⁺+1) |
| B-c-107 | 9-j | B-b-191 | | 2(4DMAPh)EtO | Et | 1Me-4-Ind | H | C | | 497 (M⁺+1) |
| B-c-108 | 1-a | B-c-107 | | 2(4DMAPh)EtO | H | 1Me-4-Ind | H | C | | 469 (M⁺+1) |
| B-c-109 | 9-j | B-b-193 | | 2(4DMAPh)EtO | Et | 1Et-5-Idz | H | C | | 512 (M⁺+1) |
| B-c-110 | 1-a | B-c-109 | | 2(4DMAPh)EtO | H | 1Et-5-Idz | H | C | | 484 (M⁺+1) |
| B-c-111 | 9-j | B-b-195 | | 2(PhO)EtO | Et | 1Me-4-Ind | H | C | | 470 (M⁺+1) |
| B-c-112 | 1-a | B-c-111 | | 2(PhO)EtO | H | 1Me-4-Ind | H | C | | 442 (M⁺+1) |
| B-c-113 | 9-j | B-b-197 | | 2(PhO)EtO | Et | 1Me-6-Ind | H | C | | 470 (M⁺+1) |
| B-c-114 | 1-a | B-c-113 | | 2(PhO)EtO | H | 1Me-6-Ind | H | C | | 442 (M⁺+1) |
| B-c-115 | 9-j | B-b-199 | | 2(PhO)EtO | Et | 1Me-5-Idz | H | C | | 471 (M⁺+1) |
| B-c-116 | 1-a | B-c-115 | | 2(PhO)EtO | H | 1Me-5-Idz | H | C | | 443 (M⁺+1) |
| B-c-117 | 9-j | B-b-203 | | 1PhEtO | Et | 2-Nap | H | C | | 451 (M⁺+1) |
| B-c-118 | 1-a | B-c-117 | | 1PhEtO | H | 2-Nap | H | C | | 423 (M⁺+1) |
| B-c-119 | 9-j | B-b-205 | | 1PhEtO | Et | 1Et-5-Ind | H | C | | 468 (M⁺+1) |
| B-c-120 | 1-a | B-c-119 | | 1PhEtO | H | 1Et-5-Ind | H | C | | 440 (M⁺+1) |
| B-c-121 | 9-j | B-b-207 | | 1PhEtO | Et | 1Me-4-Ind | H | C | | 454 (M⁺+1) |
| B-c-122 | 1-a | B-c-121 | | 1PhEtO | H | 1Me-4-Ind | H | C | | 426 (M⁺+1) |
| B-c-123 | 9-j | B-b-209 | | 1PhEtO | Et | 1Me-5-Idz | H | C | | 455 (M⁺+1) |
| B-c-124 | 1-a | B-c-123 | | 1PhEtO | H | 1Me-5-Idz | H | C | | 427 (M⁺+1) |
| B-c-125 | 9-j | B-b-211 | | 1PhEtO | Et | 1Et-5-Idz | H | C | | 469 (M⁺+1) |
| B-c-126 | 1-a | B-c-125 | | 1PhEtO | H | 1Et-5-Idz | H | C | | 441 (M⁺+1) |
| B-c-127 | 9-j | B-b-213 | | 1(2FPh)EtO | Et | 2-Nap | H | C | | 469 (M⁺+1) |
| B-c-128 | 1-a | B-c-127 | | 1(2FPh)EtO | H | 2-Nap | H | C | | 441 (M⁺+1) |
| B-c-129 | 9-j | B-b-215 | | 1(2FPh)EtO | Et | 1Et-5-Ind | H | C | | 486 (M⁺+1) |
| B-c-130 | 1-a | B-c-129 | | 1(2FPh)EtO | H | 1Et-5-Ind | H | C | | 458 (M⁺+1) |
| B-c-131 | 9-j | B-b-217 | | 1(2FPh)EtO | Et | 1Me-5-Idz | H | C | | 473 (M⁺+1) |
| B-c-132 | 1-a | B-c-131 | | 1(2FPh)EtO | H | 1Me-5-Idz | H | C | | 445 (M⁺+1) |
| B-c-133 | 9-j | B-b-229 | | 4Me,cHexO | Et | 2-Nap | H | C | | 443 (M⁺+1) |
| B-c-134 | 1-a | B-c-133 | | 4Me,cHexO | H | 2-Nap | H | C | | 415 (M⁺+1) |
| B-c-135 | 9-j | B-b-231 | | 4Me,cHexO | Et | 1Et-5-Ind | H | C | | 460 (M⁺+1) |
| B-c-136 | 1-a | B-c-135 | | 4Me,cHexO | H | 1Et-5-Ind | H | C | | 432 (M⁺+1) |
| B-c-137 | 9-j | B-b-233 | | 4Me,cHexO | Et | 1Me-5-Idz | H | C | | 447 (M⁺+1) |
| B-c-138 | 1-a | B-c-137 | | 4Me,cHexO | H | 1Me-5-Idz | H | C | | 419 (M⁺+1) |
| B-c-139 | 9-j | B-b-235 | | 4Me,cHexO | Et | 5-BF | H | C | | 433 (M⁺+1) |
| B-c-140 | 1-a | B-c-139 | | 4Me,cHexO | H | 5-BF | H | C | | 405 (M⁺+1) |

**[Table 76]**

| Table-B-c-4 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-c-141 | 9-j | B-b-237 | | 1IndanO | Et | 2-Nap | H | C | | 463 (M⁺+1) |
| B-c-142 | 1-a | B-c-141 | | 1IndanO | H | 2-Nap | H | C | | 435 (M⁺+1) |
| B-c-143 | 9-j | B-b-239 | | 1IndanO | Et | 1Me-5-Ind | H | C | | 466 (M⁺+1) |
| B-c-144 | 1-a | B-c-143 | | 1IndanO | H | 1Me-5-Ind | H | C | | 438 (M⁺+1) |
| B-c-145 | 9-j | B-b-241 | | 1IndanO | Et | 1Me-4-Ind | H | C | | 466 (M⁺+1) |
| B-c-146 | 1-a | B-c-145 | | 1IndanO | H | 1Me-4-Ind | H | C | | 438 (M⁺+1) |
| B-c-147 | 9-j | B-b-243 | | 1IndanO | Et | 1Me-5-Idz | H | C | | 467 (M⁺+1) |
| B-c-148 | 1-a | B-c-147 | | 1IndanO | H | 1Me-5-Idz | H | C | | 439 (M⁺+1) |
| B-c-149 | 9-j | B-b-245 | | 1IndanO | Et | 6-Qu | H | C | | 464 (M⁺+1) |
| B-c-150 | 1-a | B-c-149 | | 1IndanO | H | 6-Qu | H | C | | 436 (M⁺+1) |
| B-c-151 | 9-j | B-b-247 | | 2IndanO | Et | 2-Nap | H | C | | 463 (M⁺+1) |
| B-c-152 | 1-a | B-c-151 | | 2IndanO | H | 2-Nap | H | C | | 435 (M⁺+1) |
| B-c-153 | 9-j | B-b-249 | | 2IndanO | Et | 1Me-5-Ind | H | C | | 466 (M⁺+1) |
| B-c-154 | 1-a | B-c-153 | | 2IndanO | H | 1Me-5-Ind | H | C | | 438 (M⁺+1) |
| B-c-155 | 9-j | B-b-251 | | 2IndanO | Et | 1Et-5-Ind | H | C | | 480 (M⁺+1) |
| B-c-156 | 1-a | B-c-155 | | 2IndanO | H | 1Et-5-Ind | H | C | | 452 (M⁺+1) |
| B-c-157 | 9-j | B-b-253 | | 2IndanO | Et | 1Me-5-Idz | H | C | | 467 (M⁺+1) |
| B-c-158 | 1-a | B-c-157 | | 2IndanO | H | 1Me-5-Idz | H | C | | 439 (M⁺+1) |
| B-c-159 | 9-j | B-b-255 | | 2IndanO | Et | 1Et-5-Idz | H | C | | 481 (M⁺+1) |
| B-c-160 | 1-a | B-c-159 | | 2IndanO | H | 1Et-5-Idz | H | C | | 453 (M⁺+1) |
| B-c-161 | 9-j | B-b-257 | | 2IndanO | Et | 6-IQ | H | C | | 464 (M⁺+1) |
| B-c-162 | 1-a | B-c-161 | | 2IndanO | H | 6-IQ | H | C | | 436 (M⁺+1) |
| B-c-163 | 9-j | B-b-259 | | 5OMe-2-IndanO | Et | 2-Nap | H | C | | 493 (M⁺+1) |
| B-c-164 | 1-a | B-c-163 | | 5OMe-2-IndanO | H | 2-Nap | H | C | | 465 (M⁺+1) |
| B-c-165 | 9-j | B-b-261 | | 5OMe-2-IndanO | Et | 1Et-5-Ind | H | C | | 510 (M⁺+1) |
| B-c-166 | 1-a | B-c-165 | | 5OMe-2-IndanO | H | 1Et-5-Ind | H | C | | 482 (M⁺+1) |
| B-c-167 | 9-j | B-b-263 | | 5OMe-2-IndanO | Et | 1Me-5-Idz | H | C | | 497 (M⁺+1) |
| B-c-168 | 1-a | B-c-167 | | 5OMe-2-IndanO | H | 1Me-5-Idz | H | C | | 469 (M⁺+1) |
| B-c-169 | 9-j | B-b-267 | | 5,6D(OMe)-2-IndanO | Et | 1Me-5-Ind | H | C | | 526 (M⁺+1) |
| B-c-170 | 1-a | B-c-169 | | 5,6D(OMe)-2-IndanO | H | 1Me-5-Ind | H | C | | 498 (M⁺+1) |
| B-c-171 | 9-j | B-b-269 | | 5,6D(OMe)-2-IndanO | Et | 1Me-4-Ind | H | C | | 526 (M⁺+1) |
| B-c-172 | 1-a | B-c-171 | | 5,6D(OMe)-2-IndanO | H | 1Me-4-Ind | H | C | | 498 (M⁺+1) |
| B-c-173 | 9-j | B-b-271 | | 5,6D(OMe)-2-IndanO | Et | 1Et-5-Idz | H | C | | 541 (M⁺+1) |
| B-c-174 | 1-a | B-c-173 | | 5,6D(OMe)-2-IndanO | H | 1Et-5-Idz | H | C | | 513 (M⁺+1) |
| B-c-175 | 9-j | B-b-273 | | 5,6D(OMe)-2-IndanO | Et | 6-IQ | H | C | | 524 (M⁺+1) |
| B-c-176 | 1-a | B-c-175 | | 5,6D(OMe)-2-IndanO | H | 6-IQ | H | C | | 496 (M⁺+1) |
| B-c-177 | 9-j | B-b-275 | | 5F-2-IndanO | Et | 2-Nap | H | C | | 481 (M⁺+1) |
| B-c-178 | 1-a | B-c-177 | | 5F-2-IndanO | H | 2-Nap | H | C | | 453 (M⁺+1) |
| B-c-179 | 9-j | B-b-277 | | 5F-2-IndanO | Et | 1 Me-5-Ind | H | C | | 484 (M⁺+1) |
| B-c-180 | 1-a | B-c-179 | | 5F-2-IndanO | H | 1Me-5-Ind | H | C | | 456 (M⁺+1) |
| B-c-181 | 9-j | B-b-279 | | 5F-2-IndanO | Et | 1Me-5-Idz | H | C | | 485 (M⁺+1) |
| B-c-182 | 1-a | B-c-181 | | 5F-2-IndanO | H | 1Me-5-Idz | H | C | | 457 (M⁺+1) |
| B-c-183 | 9-j | B-b-281 | | 5F-2-IndanO | Et | 1Et-5-Idz | H | C | | 499 (M⁺+1) |
| B-c-184 | 1-a | B-c-183 | | 5F-2-IndanO | H | 1Et-5-Idz | H | C | | 471 (M⁺+1) |
| B-c-185 | 9-j | B-b-283 | | 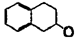 | Et | 2-Nap | H | C | | 477 (M⁺+1) |
| B-c-186 | 1-a | B-c-185 | | 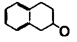 | H | 2-Nap | H | C | | 449 (M⁺+1) |

**[Table 77]**

| Table-B-c-5 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-c-187 | 9-j | B-b-285 | | 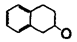 | Et | 1Me-5-Ind | H | C | | 480 (M⁺+1) |
| B-c-188 | 1-a | B-c-187 | | 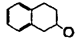 | H | 1Me-5-Ind | H | C | | 452 (M⁺+1) |
| B-c-189 | 9-j | B-b-287 | | 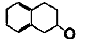 | Et | 1Me-6-Ind | H | C | | 480 (M⁺+1) |
| B-c-190 | 1-a | B-c-189 | | 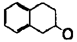 | H | 1Me-6-Ind | H | C | | 452 (M⁺+1) |
| B-c-191 | 9-j | B-b-289 | | 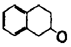 | Et | 1Me-5-Idz | H | C | | 481 (M⁺+1) |
| B-c-192 | 1-a | B-c-191 | | 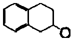 | H | 1Me-5-Idz | H | C | | 453 (M⁺+1) |
| B-c-193 | 9-j | B-b-291 | | 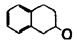 | Et | 6-Qu | H | C | | 478 (M⁺+1) |
| B-c-194 | 1-a | B-c-193 | | 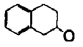 | H | 6-Qu | H | C | | 450 (M⁺+1) |
| B-c-195 | 9-j | B-b-293 | | 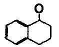 | Et | 1Me-5-Ind | H | C | | 480 (M⁺+1) |
| B-c-196 | 1-a | B-c-195 | | 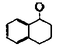 | H | 1Me-5-Ind | H | C | | 452 (M⁺+1) |
| B-c-197 | 9-j | B-b-295 | | 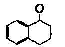 | Et | 1Et-5-Ind | H | C | | 494 (M⁺+1) |
| B-c-198 | 1-a | B-c-197 | | 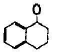 | H | 1Et-5-Ind | H | C | | 466 (M⁺+1) |
| B-c-199 | 9-j | B-b-297 | | 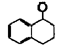 | Et | 1Me-4-Ind | H | C | | 480 (M⁺+1) |
| B-c-200 | 1-a | B-c-199 | | 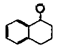 | H | 1Me-4-Ind | H | C | | 452 (M⁺+1) |
| B-c-201 | 9-j | B-b-299 | | 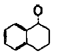 | Et | 1Et-5-Idz | H | C | | 495 (M⁺+1) |
| B-c-202 | 1-a | B-c-201 | | 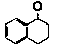 | H | 1Et-5-Idz | H | C | | 467 (M⁺+1) |
| B-c-203 | 9-j | B-b-303 | | 2(2MePh)EtO | Et | 2-Nap | H | C | | 465 (M⁺+1) |
| B-c-204 | 1-a | B-c-203 | | 2(2MePh)EtO | H | 2-Nap | H | C | | 437 (M⁺+1) |
| B-c-205 | 9-j | B-b-305 | | 2(2MePh)EtO | Et | 1Et-5-Ind | H | C | | 482 (M⁺+1) |
| B-c-206 | 1-a | B-c-205 | | 2(2MePh)EtO | H | 1Et-5-Ind | H | C | | 454 (M⁺+1) |
| B-c-207 | 9-j | B-b-307 | | 2(2MePh)EtO | Et | 1Me-6-Ind | H | C | | 468 (M⁺+1) |
| B-c-208 | 1-a | B-c-207 | | 2(2MePh)EtO | H | 1Me-6-Ind | H | C | | 440 (M⁺+1) |
| B-c-209 | 9-j | B-b-309 | | 2(2MePh)EtO | Et | 1Et-5-Idz | H | C | | 483 (M⁺+1) |
| B-c-210 | 1-a | B-c-209 | | 2(2MePh)EtO | H | 1Et-5-Idz | H | C | | 455 (M⁺+1) |
| B-c-211 | 9-j | B-b-311 | | 2(3FPh)EtO | Et | 1Me-5-Ind | H | C | | 472 (M⁺+1) |
| B-c-212 | 1-a | B-c-211 | | 2(3FPh)EtO | H | 1Me-5-Ind | H | C | | 444 (M⁺+1) |
| B-c-213 | 9-j | B-b-313 | | 2(3FPh)EtO | Et | 1Me-4-Ind | H | C | | 472 (M⁺+1) |
| B-c-214 | 1-a | B-c-213 | | 2(3FPh)EtO | H | 1Me-4-Ind | H | C | | 444 (M⁺+1) |
| B-c-215 | 9-j | B-b-315 | | 2(3FPh)EtO | Et | 1Me-5-Idz | H | C | | 473 (M⁺+1) |
| B-c-216 | 1-a | B-c-215 | | 2(3FPh)EtO | H | 1Me-5-Idz | H | C | | 445 (M⁺+1) |
| B-c-217 | 9-j | B-b-317 | | 2(3FPh)EtO | Et | 6-IQ | H | C | | 470 (M⁺+1) |
| B-c-218 | 1-a | B-c-217 | | 2(3FPh)EtO | H | 6-IQ | H | C | | 442 (M⁺+1) |
| B-c-219 | 9-j | B-b-331 | | 2(1-NapEt)O | Et | 2-Nap | H | C | | 501 (M⁺+1) |
| B-c-220 | 1-a | B-c-219 | | 2(1-NapEt)O | H | 2-Nap | H | C | | 473 (M⁺+1) |

**[Table 78]**

| Table-B-c-6 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-c-221 | 9-j | B-b-333 | | 2(1-NapEt)O | Et | 1Me-5-Ind | H | C | | 504 (M⁺+1) |
| B-c-222 | 1-a | B-c-221 | | 2(1-NapEt)O | H | 1Me-5-Ind | H | C | | 476 (M⁺+1) |
| B-c-223 | 9-j | B-b-335 | | 2(1-NapEt)O | Et | 1Me-5-Idz | H | C | | 505 (M⁺+1) |
| B-c-224 | 1-a | B-c-223 | | 2(1-NapEt)O | H | 1Me-5-Idz | H | C | | 477 (M⁺+1) |
| B-c-225 | 9-j | B-b-337 | | 2(1-NapEt)O | Et | 6-Qu | H | C | | 502 (M⁺+1) |
| B-c-226 | 1-a | B-c-225 | | 2(1-NapEt)O | H | 6-Qu | H | C | | 474 (M⁺+1) |
| B-c-227 | 9-j | B-b-341 | | 2(PhS)EtO | Et | 1Me-5-Ind | H | C | | 486 (M⁺+1) |
| B-c-228 | 1-a | B-c-227 | | 2(PhS)EtO | H | 1Me-5-Ind | H | C | | 458 (M⁺+1) |
| B-c-229 | 9-j | B-b-343 | | 2(PhS)EtO | Et | 1Me-6-Ind | H | C | | 486 (M⁺+1) |
| B-c-230 | 1-a | B-c-229 | | 2(PhS)EtO | H | 1Me-6-Ind | H | C | | 458 (M⁺+1) |
| B-c-231 | 9-j | B-b-345 | | 2(PhS)EtO | Et | 1Et-5-Idz | H | C | | 501 (M⁺+1) |
| B-c-232 | 1-a | B-c-231 | | 2(PhS)EtO | H | 1Et-5-Idz | H | C | | 473 (M⁺+1) |

### Example B-d-1

### Synthesis of (R)-2-[5-cyclopentyloxy-4-(naphthalen-2-yl)-2,3-dihydro-1H-inden-1-yl]acetic acid (Compound No. B-d-1)

Example Compound B-c-2 was subjected to chiral separation to obtain the title compound (Compound No. B-d-1, 18 mg).
Typical examples of the compounds of the present invention that can be obtained by reacting and treating corresponding starting compounds using any of the methods described in the present specification including the examples described above are shown in Table-B-d-1 to Table-B-d-3. As for the preparation of the compounds, all of the objective compounds were obtained by using HPLC separation. In the tables, used methods among the aforementioned synthetic methods are shown in the columns of "Syn" with symbols, the starting compounds are shown in the columns of "SM", and the reagents are shown in the columns of "Regent". For the compounds for which cells of the columns of "Syn" are blank, the objective compounds were obtained by using HPLC separation.

**[Table 79]**

| Table-B-d-1 | | | | | | | | | | |
|---|---|---|---|---|---|---|---|---|---|---|
| 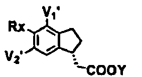 | | | | | | | | | | |

| Exp. | Syn. | SM | Reagent | Rx | Y | V1' | V2' | LCMS | | |
|---|---|---|---|---|---|---|---|---|---|---|
| | | | | | | | | method | RTime | Mass |
| B-d-1 | | B-c-2 | | cPenO | H | 2-Nap | H | C | | 387 (M⁺+1) |
| B-d-2 | | B-c-4 | | cPenO | H | 1Me-5-Ind | H | C | | 390 (M⁺+1) |
| B-d-3 | | B-c-6 | | cPenO | H | 1Et-5-Ind | H | C | | 404 (M⁺+1) |
| B-d-4 | | B-c-8 | | cPenO | H | 1Me-4-Ind | H | C | | 390 (M⁺+1) |
| B-d-5 | | B-c-10 | | cPenO | H | 1Me-6-Ind | H | C | | 390 (M⁺+1) |
| B-d-6 | | B-c-12 | | cPenO | H | 1Me-5-Idz | H | C | | 391 (M⁺+1) |
| B-d-7 | | B-c-14 | | cPenO | H | 1Et-5-Idz | H | C | | 405 (M⁺+1) |
| B-d-8 | | B-c-16 | | cPenO | H | 5-BF | H | C | | 377 (M⁺+1) |
| B-d-9 | | B-c-18 | | cPenO | H | 6-Qu | H | C | | 388 (M⁺+1) |
| B-d-10 | | B-c-20 | | cPenO | H | 6-IQ | H | C | | 388 (M⁺+1) |
| B-d-11 1 | | B-c-22 | | cPenMeO | H | 2-Nap | H | C | | 401 (M⁺+1) |
| B-d-12 | | B-c-24 | | cPenMeO | H | 1Me-5-Ind | H | C | | 404 (M⁺+1) |
| B-d-13 | | B-c-26 | | cPenMeO | H | 1Me-6-Ind | H | C | | 404 (M⁺+1) |
| B-d-14 | | B-c-28 | | cPenMeO | H | 1Me-5-Idz | H | C | | 405 (M⁺+1) |
| B-d-15 | | B-c-30 | | cPenMeO | H | 1Et-5-Idz | H | C | | 419 (M⁺+1) |
| B-d-16 | | B-c-32 | | cPenMeO | H | 6-Qu | H | C | | 402 (M⁺+1) |
| B-d-17 | | B-c-34 | | cHexMeO | H | 2-Nap | H | C | | 415 (M⁺+1) |
| B-d-18 | | B-c-36 | | cHexMeO | H | 1 Et-5-Ind | H | C | | 432 (M⁺+1) |
| B-d-19 | | B-c-38 | | cHexMeO | H | 1Me-4-Ind | H | C | | 418 (M⁺+1) |
| B-d-20 | | B-c-40 | | cHexMeO | H | 1Me-5-Idz | H | C | | 419 (M⁺+1) |
| B-d-21 | | B-c-42 | | cHexMeO | H | 1Et-5-Idz | H | C | | 433 (M⁺+1) |
| B-d-22 | | B-c-44 | | cHexMeO | H | 5-BF | H | C | | 405 (M⁺+1) |
| B-d-23 | | B-c-48 | | cHexO | H | 2-Nap | H | C | | 401 (M⁺+1) |
| B-d-24 | | B-c-50 | | cHexO | H | 1Me-5-Ind | H | C | | 404 (M⁺+1) |
| B-d-25 | | B-c-52 | | cHexO | H | 1Me-4-Ind | H | C | | 404 (M⁺+1) |
| B-d-26 | | B-c-54 | | cHexO | H | 1Me-5-Idz | H | C | | 405 (M⁺+1) |
| B-d-27 | | B-c-56 | | cHexO | H | 1Et-5-Idz | H | C | | 419 (M⁺+1) |
| B-d-28 | | B-c-58 | | cHepO | H | 2-Nap | H | C | | 415 (M⁺+1) |
| B-d-29 | | B-c-60 | | cHepO | H | 1Me-5-Ind | H | C | | 418 (M⁺+1) |
| B-d-30 | | B-c-62 | | cHepO | H | 1Me-5-Idz | H | C | | 419 (M⁺+1) |
| B-d-31 | | B-c-64 | | cHepO | H | 6-IQ | H | C | | 416 (M⁺+1) |
| B-d-32 | | B-c-66 | | nPrO | H | 1Et-5-Ind | H | C | | 378 (M⁺+1) |
| B-d-33 | | B-c-68 | | nPrO | H | 1Et-5-Idz | H | C | | 379 (M⁺+1) |
| B-d-34 | | B-c-70 | | nPrO | H | 6-Qu | H | C | | 362 (M⁺+1) |
| B-d-35 | | B-c-72 | | iPrO | H | 2-Nap | H | C | | 361 (M⁺+1) |
| B-d-36 | | B-c-74 | | iPrO | H | 1Et-5-Ind | H | C | | 378 (M⁺+1) |
| B-d-37 | | B-c-76 | | iPrO | H | 1Me-5-Idz | H | C | | 365 (M⁺+1) |
| B-d-38 | | B-c-80 | | nBuO | H | 2-Nap | H | C | | 375 (M⁺+1) |
| B-d-39 | | B-c-82 | | nBuO | H | 1Me-5-Ind | H | C | | 378 (M⁺+1) |
| B-d-40 | | B-c-84 | | nBuO | H | 1Et-5-Idz | H | C | | 393 (M⁺+1) |
| B-d-41 | | B-c-86 | | iBuO | H | 1Me-5-Ind | H | C | | 378 (M⁺+1) |
| B-d-42 | | B-c-88 | | iBuO | H | 1Et-5-Ind | H | C | | 392 (M⁺+1) |
| B-d-43 | | B-c-90 | | iBuO | H | 1Me-5-Idz | H | C | | 379 (M⁺+1) |
| B-d-44 | | B-c-92 | | iBuO | H | 1Et-5-Idz | H | C | | 393 (M⁺+1) |

**[Table 80]**

| Table-B-d-2 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-d-45 | | B-c-94 | | 2EtBuO | H | 2-Nap | H | C | | 403 (M⁺+1) |
| B-d-46 | | B-c-96 | | 2EtBuO | H | 1Me-5-Ind | H | C | | 406 (M⁺+1) |
| B-d-47 | | B-c-98 | | 2EtBuO | H | 1Et-5-Ind | H | C | | 420 (M⁺+1) |
| B-d-48 | | B-c-100 | | 2EtBuO | H | 1Me-5-Idz | H | C | | 407 (M⁺+1) |
| B-d-49 | | B-c-102 | | 2EtBuO | H | 6-IQ | H | C | | 404 (M⁺+1) |
| B-d-50 | | B-c-104 | | 2(4DMAPh)EtO | H | 2-Nap | H | C | | 466 (M⁺+1) |
| B-d-51 | | B-c-106 | | 2(4DMAPh)EtO | H | 1Me-5-Ind | H | C | | 469 (M⁺+1) |
| B-d-52 | | B-c-110 | | 2(4DMAPh)EtO | H | 1Et-5-Idz | H | C | | 484 (M⁺+1) |
| B-d-53 | | B-c-112 | | 2(PhO)EtO | H | 1Me-4-Ind | H | C | | 442 (M⁺+1) |
| B-d-54 | | B-c-114 | | 2(PhO)EtO | H | 1Me-6-Ind | H | C | | 442 (M⁺+1) |
| B-d-55 | | B-c-116 | | 2(PhO)EtO | H | 1Me-5-Idz | H | C | | 443 (M⁺+1) |
| B-d-56 | | B-c-118 | | 1PhEtO | H | 2-Nap | H | C | | 423 (M⁺+1) |
| B-d-57 | | B-c-120 | | 1PhEtO | H | 1Et-5-Ind | H | C | | 440 (M⁺+1) |
| B-d-58 | | B-c-124 | | 1PhEtO | H | 1Me-5-Idz | H | C | | 427 (M⁺+1) |
| B-d-59 | | B-c-128 | | 1(2FPh)EtO | H | 2-Nap | H | C | | 441 (M⁺+1) |
| B-d-60 | | B-c-130 | | 1(2FPh)EtO | H | 1Et-5-Ind | H | C | | 458 (M⁺+1) |
| B-d-61 | | B-c-136 | | 4Me,cHexO | H | 1Et-5-Ind | H | C | | 432 (M⁺+1) |
| B-d-62 | | B-c-138 | | 4Me,cHexO | H | 1Me-5-Idz | H | C | | 419 (M⁺+1) |
| B-d-63 | | B-c-140 | | 4Me,cHexO | H | 5-BF | H | C | | 405 (M⁺+1) |
| B-d-64 | | B-c-142 | | 1IndanO | H | 2-Nap | H | C | | 435 (M⁺+1) |
| B-d-65 | | B-c-144 | | 1IndanO | H | 1Me-5-Ind | H | C | | 438 (M⁺+1) |
| B-d-66 | | B-c-146 | | 1IndanO | H | 1Me-4-Ind | H | C | | 438 (M⁺+1) |
| B-d-67 | | B-c-148 | | 1IndanO | H | 1Me-5-Idz | H | C | | 439 (M⁺+1) |
| B-d-68 | | B-c-150 | | 1IndanO | H | 6-Qu | H | C | | 436 (M⁺+1) |
| B-d-69 | | B-c-154 | | 2IndanO | H | 1Me-5-Ind | H | C | | 438 (M⁺+1) |
| B-d-70 | | B-c-156 | | 2IndanO | H | 1Et-5-Ind | H | C | | 452 (M⁺+1) |
| B-d-71 | | B-c-158 | | 2IndanO | H | 1Me-5-Idz | H | C | | 439 (M⁺+1) |
| B-d-72 | | B-c-160 | | 2IndanO | H | 1Et-5-Idz | H | C | | 453 (M⁺+1) |
| B-d-73 | | B-c-164 | | 50Me-2-IndanO | H | 2-Nap | H | C | | 465 (M⁺+1) |
| B-d-74 | | B-c-166 | | 50Me-2-IndanO | H | 1Et-5-Ind | H | C | | 482 (M⁺+1) |
| B-d-75 | | B-c-168 | | 50Me-2-IndanO | H | 1Me-5-Idz | H | C | | 469 (M⁺+1) |
| B-d-76 | | B-c-170 | | 5,6D(OMe)-2-IndanO | H | 1Me-5-Ind | H | C | | 498 (M⁺+1) |
| B-d-77 | | B-c-172 | | 5,6D(OMe)-2-IndanO | H | 1Me-4-Ind | H | C | | 498 (M⁺+1) |
| B-d-78 | | B-c-176 | | 5,6D(OMe)-2-IndanO | H | 6-IQ | H | C | | 496 (M⁺+1) |
| B-d-79 | | B-c-178 | | 5F-2-IndanO | H | 2-Nap | H | C | | 453 (M⁺+1) |
| B-d-80 | | B-c-180 | | 5F-2-IndanO | H | 1Me-5-Ind | H | C | | 456 (M⁺+1) |
| B-d-81 | | B-c-182 | | 5F-2-IndanO | H | 1Me-5-Idz | H | C | | 457 (M⁺+1) |
| B-d-82 | | B-c-184 | | 5F-2-IndanO | H | 1Et-5-Idz | H | C | | 471 (M⁺+1) |
| B-d-83 | | B-c-186 | | 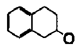 | H | 2-Nap | H | C | | 449 (M⁺+1) |
| B-d-84 | | B-c-190 | | 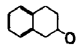 | H | 1Me-6-Ind | H | C | | 452 (M⁺+1) |
| B-d-85 | | B-c-192 | | 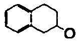 | H | 1Me-5-Idz | H | C | | 453 (M⁺+1) |
| B-d-86 | | B-c-196 | | 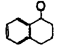 | H | 1Me-5-Ind | H | C | | 452 (M⁺+1) |
| B-d-87 | | B-c-202 | | 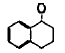 | H | 1Et-5-Idz | H | C | | 467 (M⁺+1) |

**[Table 81]**

| Table-B-d-3 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-d-88 | | B-c-204 | | 2(2MePh)EtO | H | 2-Nap | H | C | | 437 (M⁺+1) |
| B-d-89 | | B-c-206 | | 2(2MePh)EtO | H | 1Et-5-Ind | H | C | | 454 (M⁺+1) |
| B-d-90 | | B-c-210 | | 2(2MePh)EtO | H | 1Et-5-Idz | H | C | | 455 (M⁺+1) |
| B-d-91 | | B-c-212 | | 2(3FPh)EtO | H | 1Me-5-Ind | H | C | | 444 (M⁺+1) |
| B-d-92 | | B-c-214 | | 2(3FPh)EtO | H | 1 Me-4-Ind | H | C | | 444 (M⁺+1) |
| B-d-93 | | B-c-216 | | 2(3FPh)EtO | H | 1Me-5-Idz | H | C | | 445 (M⁺+1) |
| B-d-94 | | B-c-220 | | 2(1-NapEt)O | H | 2-Nap | H | C | | 473 (M⁺+1) |
| B-d-95 | | B-c-222 | | 2(1-NapEt)O | H | 1Me-5-Ind | H | C | | 476 (M⁺+1) |
| B-d-96 | | B-c-224 | | 2(1-NapEt)O | H | 1M e-5-Idz | H | C | | 477 (M⁺+1) |
| B-d-97 | | B-c-226 | | 2(1-NapEt)O | H | 6-Qu | H | C | | 474 (M⁺+1) |
| B-d-98 | | B-c-228 | | 2(PhS)EtO | H | 1 Me-5-Ind | H | C | | 458 (M⁺+1) |
| B-d-99 | | B-c-230 | | 2(PhS)EtO | H | 1 Me-6-Ind | H | C | | 458 (M⁺+1) |
| B-d-100 | | B-c-232 | | 2(PhS)EtO | H | 1Et-5-Idz | H | C | | 473 (M⁺+1) |

### Example B-e-1

### Synthesis of (S)-2-[5-cyclopentyloxy-4-(naphthalen-2-yl)-2,3-dihydro-1H-inden-1-yl]acetic acid (Compound No. B-e-1)

Example Compound B-c-2 was subjected to chiral separation to obtain the title compound (Compound No. B-e-1, 19.2 mg)
Typical examples of the compounds of the present invention that can be obtained by reacting and treating corresponding starting compounds using any of the methods described in the present specification including the examples described above are shown in Table-B-e-1 to Table-B-e-3. As for the preparation of the compounds, all of the objective compounds were obtained by using HPLC separation. In the tables, used methods among the aforementioned synthetic methods are shown in the columns of "Syn" with symbols, the starting compounds are shown in the columns of "SM", and the reagents are shown in the columns of "Regent". For the compounds for which cells of the columns of "Syn" are blank, the objective compounds were obtained by using HPLC separation.

**[Table 82]**

| Table-B-e-1 | | | | | | | | | | |
|---|---|---|---|---|---|---|---|---|---|---|
| 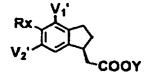 | | | | | | | | | | |

| Exp. | Syn. | SM | Reagent | Rx | Y | V1' | V2' | LCMS | | |
|---|---|---|---|---|---|---|---|---|---|---|
| | | | | | | | | method | RTime | Mass |
| B-e-1 | | B-c-2 | | cPenO | H | 2-Nap | H | C | | 387 (M⁺+1) |
| B-e-2 | | B-c-4 | | cPenO | H | 1Me-5-Ind | H | C | | 390 (M⁺+1) |
| B-e-3 | | B-c-6 | | cPenO | H | 1Et-5-Ind | H | C | | 404 (M⁺+1) |
| B-e-4 | | B-c-8 | | cPenO | H | 1Me-4-Ind | H | C | | 390 (M⁺+1) |
| B-e-5 | | B-c-10 | | cPenO | H | 1Me-6-Ind | H | C | | 390 (M⁺+1) |
| B-e-6 | | B-c-12 | | cPenO | H | 1Me-5-Idz | H | C | | 391 (M⁺+1) |
| B-e-7 | | B-c-14 | | cPenO | H | 1 Et-5-Idz | H | C | | 405 (M⁺+1) |
| B-e-8 | | B-c-16 | | cPenO | H | 5-B F | H | C | | 377 (M⁺+1) |
| B-e-9 | | B-c-18 | | cPenO | H | 6-Qu | H | C | | 388 (M⁺+1) |
| B-e-10 | | B-c-20 | | cPenO | H | 6-IQ | H | C | | 388 (M⁺+1) |
| B-e-11 | | B-c-22 | | cPenMeO | H | 2-Nap | H | C | | 401 (M⁺+1) |
| B-e-12 | | B-c-24 | | cPenMeO | H | 1Me-5-Ind | H | C | | 404 (M⁺+1) |
| B-e-13 | | B-c-26 | | cPenMeO | H | 1Me-6-Ind | H | C | | 404 (M⁺+1) |
| B-e-14 | | B-c-28 | | cPenMeO | H | 1Me-5-Idz | H | C | | 405 (M⁺+1) |
| B-e-15 | | B-c-30 | | cPenMeO | H | 1Et-5-Idz | H | C | | 419 (M⁺+1) |
| B-e-16 | | B-c-32 | | cPenMeO | H | 6-Qu | H | C | | 402 (M⁺+1) |
| B-e-17 | | B-c-34 | | cHexMeO | H | 2-Nap | H | C | | 415 (M⁺+1) |
| B-e-18 | | B-c-36 | | cHexMeO | H | 1Et-5-Ind | H | C | | 432 (M⁺+1) |
| B-e-19 | | B-c-38 | | cHexMeO | H | 1Me-4-Ind | H | C | | 418 (M⁺+1) |
| B-e-20 | | B-c-40 | | cHexMeO | H | 1Me-5-Idz | H | C | | 419 (M⁺+1) |
| B-e-21 | | B-c-42 | | cHexMeO | H | 1Et-5-Idz | H | C | | 433 (M⁺+1) |
| B-e-22 | | B-c-44 | | cHexMeO | H | 5-BF | H | C | | 405 (M⁺+1) |
| B-e-23 | | B-c-48 | | cHexO | H | 2-Nap | H | C | | 401 (M⁺+1) |
| B-e-24 | | B-c-50 | | cHexO | H | 1Me-5-Ind | H | C | | 404 (M⁺+1) |
| B-e-25 | | B-c-52 | | cHexO | H | 1Me-4-Ind | H | C | | 404 (M⁺+1) |
| B-e-26 | | B-c-54 | | cHexO | H | 1Me-5-Idz | H | C | | 405 (M⁺+1) |
| B-e-27 | | B-c-56 | | cHexO | H | 1Et-5-Idz | H | C | | 419 (M⁺+1) |
| B-e-28 | | B-c-58 | | cHepO | H | 2-Nap | H | C | | 415 (M⁺+1) |
| B-e-29 | | B-c-60 | | cHepO | H | 1Me-5-Ind | H | C | | 418 (M⁺+1) |
| B-e-30 | | B-c-62 | | cHepO | H | 1Me-5-Idz | H | C | | 419 (M⁺+1) |
| B-e-31 | | B-c-64 | | cHepO | H | 6-IQ | H | C | | 416 (M⁺+1) |
| B-e-32 | | B-c-66 | | nPrO | H | 1Et-5-Ind | H | C | | 378 (M⁺+1) |
| B-e-33 | | B-c-68 | | nPrO | H | 1Et-5-Idz | H | C | | 379 (M⁺+1) |
| B-e-34 | | B-c-70 | | nPrO | H | 6-Qu | H | C | | 362 (M⁺+1) |
| B-e-35 | | B-c-72 | | iPrO | H | 2-Nap | H | C | | 361 (M⁺+1) |
| B-e-36 | | B-c-74 | | iPrO | H | 1Et-5-Ind | H | C | | 378 (M⁺+1) |
| B-e-37 | | B-c-76 | | iPrO | H | 1Me-5-Idz | H | C | | 365 (M⁺+1) |
| B-e-38 | | B-c-80 | | nBuO | H | 2-Nap | H | C | | 375 (M⁺+1) |
| B-e-39 | | B-c-82 | | nBuO | H | 1Me-5-Ind | H | C | | 378 (M⁺+1) |
| B-e-40 | | B-c-84 | | nBuO | H | 1Et-5-Idz | H | C | | 393 (M⁺+1) |
| B-e-41 | | B-c-86 | | iBuO | H | 1Me-5-Ind | H | C | | 378 (M⁺+1) |
| B-e-42 | | B-c-88 | | iBuO | H | 1Et-5-Ind | H | C | | 392 (M⁺+1) |
| B-e-43 | | B-c-90 | | iBuO | H | 1Me-5-Idz | H | C | | 379 (M⁺+1) |
| B-e-44 | | B-c-92 | | iBuO | H | 1Et-5-Idz | H | C | | 393 (M⁺+1) |

**[Table 83]**

| Table-B-e-2 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-e-45 | | B-c-94 | | 2EtBuO | H | 2-Nap | H | C | | 403 (M⁺+1) |
| B-e-46 | | B-c-96 | | 2EtBuO | H | 1Me-5-Ind | H | C | | 406 (M⁺+1) |
| B-e-47 | | B-c-98 | | 2EtBuO | H | 1Et-5-Ind | H | C | | 420 (M⁺+1) |
| B-e-48 | | B-c-100 | | 2EtBuO | H | 1Me-5-Idz | H | C | | 407 (M⁺+1) |
| B-e-49 | | B-c-102 | | 2EtBuO | H | 6-IQ | H | C | | 404 (M⁺+1) |
| B-e-50 | | B-c-104 | | 2(4DMAPh)EtO | H | 2-Nap | H | C | | 466 (M⁺+1) |
| B-e-51 | | B-c-106 | | 2(4DMAPh)EtO | H | 1Me-5-Ind | H | C | | 469 (M⁺+1) |
| B-e-52 | | B-c-110 | | 2(4DMAPh)EtO | H | 1Et-5-Idz | H | C | | 484 (M⁺+1) |
| B-e-53 | | B-c-112 | | 2(PhO)EtO | H | 1Me-4-Ind | H | C | | 442 (M⁺+1) |
| B-e-54 | | B-c-114 | | 2(PhO)EtO | H | 1Me-6-Ind | H | C | | 442 (M⁺+1) |
| B-e-55 | | B-c-116 | | 2(PhO)EtO | H | 1Me-5-Idz | H | C | | 443 (M⁺+1) |
| B-e-56 | | B-c-118 | | 1PhEtO | H | 2-Nap | H | C | | 423 (M⁺+1) |
| B-e-57 | | B-c-120 | | 1PhEtO | H | 1Et-5-Ind | H | C | | 440 (M⁺+1) |
| B-e-58 | | B-c-124 | | 1 PhEtO | H | 1Me-5-Idz | H | C | | 427 (M⁺+1) |
| B-e-59 | | B-c-128 | | 1(2FPh)EtO | H | 2-Nap | H | C | | 441 (M⁺+1) |
| B-e-60 | | B-c-130 | | 1(2FPh)EtO | H | 1Et-5-Ind | H | C | | 458 (M⁺+1) |
| B-e-61 | | B-c-136 | | 4Me,cHexO | H | 1Et-5-Ind | H | C | | 432 (M⁺+1) |
| B-e-62 | | B-c-138 | | 4Me,cHexO | H | 1Me-5-Idz | H | C | | 419 (M⁺+1) |
| B-e-63 | | B-c-140 | | 4Me,cHexO | H | 5-B F | H | C | | 405 (M⁺+1) |
| B-e-64 | | B-c-142 | | 1IndanO | H | 2-Nap | H | C | | 435 (M⁺+1) |
| B-e-65 | | B-c-144 | | 1IndanO | H | 1Me-5-Ind | H | C | | 438 (M⁺+1) |
| B-e-66 | | B-c-146 | | 1IndanO | H | 1Me-4-Ind | H | C | | 438 (M⁺+1) |
| B-e-67 | | B-c-148 | | 1IndanO | H | 1Me-5-Idz | H | C | | 439 (M⁺+1) |
| B-e-68 | | B-c-150 | | 1IndanO | H | 6-Qu | H | C | | 436 (M⁺+1) |
| B-e-69 | | B-c-154 | | 2IndanO | H | 1Me-5-Ind | H | C | | 438 (M⁺+1) |
| B-e-70 | | B-c-156 | | 2IndanO | H | 1Et-5-Ind | H | C | | 452 (M⁺+1) |
| B-e-71 | | B-c-158 | | 2IndanO | H | 1Me-5-Idz | H | C | | 439 (M⁺+1) |
| B-e-72 | | B-c-160 | | 2IndanO | H | 1Et-5-Idz | H | C | | 453 (M⁺+1) |
| B-e-73 | | B-c-164 | | 50Me-2-IndanO | H | 2-Nap | H | C | | 465 (M⁺+1) |
| B-e-74 | | B-c-166 | | 50Me-2-IndanO | H | 1Et-5-Ind | H | C | | 482 (M⁺+1) |
| B-e-75 | | B-c-168 | | 50Me-2-IndanO | H | 1Me-5-Idz | H | C | | 469 (M⁺+1) |
| B-e-76 | | B-c-170 | | 5,6D(OMe)-2-IndanO | H | 1Me-5-Ind | H | C | | 498 (M⁺+1) |
| B-e-77 | | B-c-172 | | 5,6D(OMe)-2-IndanO | H | 1Me-4-Ind | H | C | | 498 (M⁺+1) |
| B-e-78 | | B-c-176 | | 5,6D(OMe)-2-IndanO | H | 6-IQ | H | C | | 496 (M⁺+1) |
| B-e-79 | | B-c-178 | | 5F-2-IndanO | H | 2-Nap | H | C | | 453 (M⁺+1) |
| B-e-80 | | B-c-180 | | 5F-2-IndanO | H | 1Me-5-Ind | H | C | | 456 (M⁺+1) |
| B-e-81 | | B-c-182 | | 5F-2-IndanO | H | 1Me-5-Idz | H | C | | 457 (M⁺+1) |
| B-e-82 | | B-c-184 | | 5F-2-IndanO | H | 1Et-5-Idz | H | C | | 471 (M⁺+1) |
| B-e-83 | | B-c-186 | | 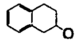 | H | 2-Nap | H | C | | 449 (M⁺+1) |
| B-e-84 | | B-c-190 | | 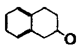 | H | 1Me-6-Ind | H | C | | 452 (M⁺+1) |
| B-e-85 | | B-c-192 | | 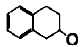 | H | 1Me-5-Idz | H | C | | 453 (M⁺+1) |
| B-e-86 | | B-c-196 | | 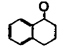 | H | 1Me-5-Ind | H | C | | 452 (M⁺+1) |
| B-e-87 | | B-c-202 | | 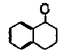 | H | 1Et-5-Idz | H | C | | 467 (M⁺+1) |

**[Table 84]**

| Table-B-e-3 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| B-e-88 | | B-c-204 | | 2(2MePh)EtO | H | 2-Nap | H | C | | 437 (M⁺+1) |
| B-e-89 | | B-c-206 | | 2(2MePh)EtO | H | 1Et-5-Ind | H | C | | 454 (M⁺+1) |
| B-e-90 | | B-c-210 | | 2(2MePh)EtO | H | 1Et-5-Idz | H | C | | 455 (M⁺+1) |
| B-e-91 | | B-c-212 | | 2(3FPh)EtO | H | 1Me-5-Ind | H | C | | 444 (M⁺+1) |
| B-e-92 | | B-c-214 | | 2(3FPh)EtO | H | 1Me-4-Ind | H | C | | 444 (M⁺+1) |
| B-e-93 | | B-c-216 | | 2(3FPh)EtO | H | 1Me-5-Idz | H | C | | 445 (M⁺+1) |
| B-e-94 | | B-c-220 | | 2(1-NapEt)O | H | 2-Nap | H | C | | 473 (M⁺+1) |
| B-e-95 | | B-c-222 | | 2(1-NapEt)O | H | 1Me-5-Ind | H | C | | 476 (M⁺+1) |
| B-e-96 | | B-c-224 | | 2(1-NapEt)O | H | 1Me-5-Idz | H | C | | 477 (M⁺+1) |
| B-e-97 | | B-c-226 | | 2(1-NapEt)O | H | 6-Qu | H | C | | 474 (M⁺+1) |
| B-e-98 | | B-c-228 | | 2(PhS)EtO | H | 1Me-5-Ind | H | C | | 458 (M⁺+1) |
| B-e-99 | | B-c-230 | | 2(PhS)EtO | H | 1Me-6-Ind | H | C | | 458 (M⁺+1) |
| B-e-100 | | B-c-232 | | 2(PhS)EtO | H | 1Et-5-Idz | H | C | | 473 (M⁺+1) |

### Reference Example 12

### Synthesis of ethyl 2-[6-(naphthalen-2-yl)-5-trifluoromethanesulfonyl-2,3-dihydro-1H-inden-1-yl]acetate (Intermediate c-a-1) (Preparation Method 5, Step g)

A solution of Intermediate A-22 (973.5 mg) in dehydrated pyridine (15 ml) was added with trifluoromethanesulfonic anhydride (543 µl, ALD) under ice cooling, and then the mixture was warmed to room temperature, and stirred for 2.5 hours. The reaction mixture was concentrated under reduced pressure, and then extracted with ethyl acetate (800 ml), the organic layer was successively washed with 1 N hydrochloric acid, saturated aqueous ammonium chloride and saturated brine, and dried, and then the solvent was evaporated under reduced pressure. The residue was purified by flash column chromatography (hexane:ethyl acetate = 9:1) to obtain the title compound (Intermediate c-a-1, 839.5 mg).
Typical examples of the intermediates that can be obtained by reacting and treating corresponding starting compounds using any of the methods described in the present specification including the examples described above are shown in Table-Int.C-a-1 to Table-Int.C-a-3. The compounds were synthesized according to the preparation methods of the compounds of the compound numbers shown in the columns of "Syn" in the tables. "Int" means an intermediate compound number. In the tables, used methods among the aforementioned synthetic methods are shown in the columns of "Syn" with symbols, the starting compounds are shown in the columns of "SM", and the reagents are shown in the columns of "Regent".

**[Table 85]**

| Table-C-a-1 | | | | | | | | | |
|---|---|---|---|---|---|---|---|---|---|
| 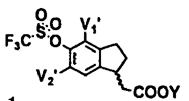 | | | | | | | | | |

| Exp. | Syn. | SM1 | Reagent | Y | V₁' | V₂' | LCMS | | |
|---|---|---|---|---|---|---|---|---|---|
| | | | | | | | method | RTime | Mass |
| Int.c-a-2 | 5-g | Int.A-23 | | Et | H | 5-Ind | C | | 468 (M⁺+1) |
| Int.c-a-3 | 5-g | Int.A-24 | | Et | H | 1Me-5-Ind | C | | 482 (M⁺+1) |
| Int.c-a-4 | 5-g | Int.A-25 | | Et | H | 5-1Idz | C | | 469 (M⁺+1) |
| Int.c-a-5 | 5-g | Int.A-26 | | Et | H | 1 Me-5-Idz | C | | 443 (M⁺+1) |
| Int.c-a-6 | 5-g | Int.A-27 | | Et | H | 1Et-5-Idz | C | | 497 (M⁺+1) |
| Int.c-a-7 | 5-g | Int.A-28 | | Et | H | 5-BF | C | | 469 (M⁺+1) |
| Int.c-a-8 | 5-g | Int.A-29 | | Et | H | 6-Qu | C | | 480 (M⁺+1) |

**[Table 86]**

| Table-C-b-1 | | | | | | | | | |
|---|---|---|---|---|---|---|---|---|---|
| 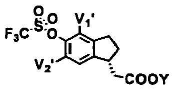 | | | | | | | | | |

| Exp. | Syn. | SM1 | Reagent | Y | V₁' | V₂' | LCMS | | |
|---|---|---|---|---|---|---|---|---|---|
| | | | | | | | method | RTime | Mass |
| Int.c-b-1 | 5-g | Int.A-106 | | Et | H | 2-Nap | C | | 479 (M⁺+1) |
| Int.c-b-2 | 5-g | Int.A-107 | | Et | H | 5-Ind | C | | 468 (M⁺+1) |
| Int.c-b-3 | 5-g | Int.A-108 | | Et | H | 1Me-5-Ind | C | | 482 (M⁺+1) |
| Int.c-b-4 | 5-g | Int.A-109 | | Et | H | 5-1Idz | C | | 469 (M⁺+1) |
| Int.c-b-5 | 5-g | Int.A-110 | | Et | H | 1Me-5-Idz | C | | 443 (M⁺+1) |
| Int.c-b-6 | 5-g | Int.A-111 | | Et | H | 1Et-5-Idz | C | | 497 (M⁺+1) |
| Int.c-b-7 | 5-g | Int.A-112 | | Et | H | 5-BF | C | | 469 (M⁺+1) |
| Int.c-b-8 | 5-g | Int.A-113 | | Et | H | 6-Qu | C | | 480 (M⁺+1) |

**[Table 87]**

| Table-C-c-1 | | | | | | | | | |
|---|---|---|---|---|---|---|---|---|---|
| 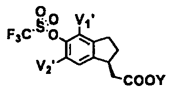 | | | | | | | | | |

| Exp. | Syn. | SM1 | Reagent | Y | V₁' | V₂' | LCMS | | |
|---|---|---|---|---|---|---|---|---|---|
| | | | | | | | method | RTime | Mass |
| Int.c-c-1 | 5-g | Int.A-206 | | Et | H | 2-Nap | C | | 479 (M⁺+1) |
| Int.c-c-2 | 5-g | Int.A-207 | | Et | H | 5-Ind | C | | 468 (M⁺+1) |
| Int.c-c-3 | 5-g | Int.A-208 | | Et | H | 1Me-5-Ind | C | | 482 (M⁺+1) |
| Int.c-c-4 | 5-g | Int.A-209 | | Et | H | 5-1Idz | C | | 469 (M⁺+1) |
| Int.c-c-5 | 5-g | Int.A-210 | | Et | H | 1Me-5-Idz | C | | 443 (M⁺+1) |
| Int.c-c-6 | 5-g | Int.A-211 | | Et | H | 1Et-5-Idz | C | | 497 (M⁺+1) |
| Int.c-c-7 | 5-g | Int.A-212 | | Et | H | 5-BF | C | | 469 (M⁺+1) |
| Int.c-c-8 | 5-g | Int.A-213 | | Et | H | 6-Qu | C | | 480 (M⁺+1) |

### Example Ca-a-1

### Synthesis of ethyl 2-[6-(naphthalen-2-yl)-5-phenyl-2,3-dihydro-1H-inden-1-yl]acetate (Compound No. Ca-a-1) (Preparation Method 5, Step e)

Intermediate C-a-1 (110.4 mg), phenylboronic acid (74.3 mg, Ald), cesium carbonate (254.9 mg) and PdCl₂(dppf) (27.6 mg) were added with toluene (600 µl), methanol (1.2 ml) and water (1.2 ml), and the mixture was stirred at 90°C for 15.5 hours under a nitrogen atmosphere. The reaction mixture was added with ethyl acetate (30 ml), and successively washed with water and saturated brine. The organic layer was dried, and then the solvent was evaporated under reduced pressure. The residue was purified by flash column chromatography (Quad, hexane:ethyl acetate = 9:1) to obtain the title compound (Compound No. Ca-a-1, 69.4 mg).

### Example Ca-a-2

### Synthesis of 2-[6-(naphthalen-2-yl)-5-phenyl-2,3-dihydro-1H-inden-1-yl]acetic acid (Compound No. Ca-a-2) (Preparation Method 1, Step a)

According to the procedure described in the synthetic method of Example Compound A-a-2 (Preparation Method 1, Step a) provided that the reaction was performed for 2 hours, Example Compound Ca-a-1 (63.3 mg), and 2 N aqueous sodium hydroxide (250 µl) were reacted and treated to obtain the title compound (Compound No. Ca-a-2, 53.4 mg).
Typical examples of the compounds of the present invention that can be obtained by reacting and treating corresponding starting compounds using any of the methods described in the present specification including the examples described above are shown in Table-Ca-A-1 to Table-Ca-A-14, Table-Ca-B-1 to Table-Ca-B-11, and Table-Ca-C-1 to Table-Ca-C-11. The compounds were synthesized according to the preparation methods of the compounds of the compound numbers shown in the columns of "Syn" in the tables. "Int" means an intermediate compound number. In the tables, used methods among the aforementioned synthetic methods are shown in the columns of "Syn" with symbols, the starting compounds are shown in the columns of "SM", and the reagents are shown in the columns of "Regent". The boronic acid regents mentioned in the columns of "Reagent" with symbols "BRA (No.)" are those mentioned in Table-BRA, and the bromoheterocyclic ring regents mentioned with the symbols "Het (No.)" are those mentioned in Table-Het. The blank cells in the columns of "Syn" means that the objective compounds were obtained by using HPLC separation.

**[Table 88]**

| Table-Ca-A-1 | | | | | | | | | | |
|---|---|---|---|---|---|---|---|---|---|---|
| 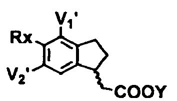 | | | | | | | | | | |

| Exp. | Syn. | SM1 | Reagent | Rx | Y | V₁' | V₂' | LCMS | | |
|---|---|---|---|---|---|---|---|---|---|---|
| | | | | | | | | method | RTime | Mass |
| Ca-a-1 | 5-e | Int.c-a-1 | BRA14 | Ph | Et | H | 2-Nap | C | | 407 (M⁺+1) |
| Ca-a-2 | 1-a | Ca-a-1 | | Ph | H | H | 2-Nap | C | | 379 (M⁺+1) |
| Ca-a-3 | 5-e | Int.c-a-1 | BRA15 | 2-MePh | Et | H | 2-Nap | C | | 421 (M⁺+1) |
| Ca-a-4 | 1-a | Ca-a-3 | | 2-MePh | H | H | 2-Nap | C | | 393 (M⁺+1) |
| Ca-a-5 | 5-e | Int.c-a-1 | BRA16 | 3-MePh | Et | H | 2-Nap | C | | 421 (M⁺+1) |
| Ca-a-6 | 1-a | Ca-a-5 | | 3-MePh | H | H | 2-Nap | C | | 393 (M⁺+1) |
| Ca-a-7 | 5-e | Int.c-a-1 | BRA17 | 4-MePh | Et | H | 2-Nap | C | | 421 (M⁺+1) |
| Ca-a-8 | 1-a | Ca-a-7 | | 4-MePh | H | H | 2-Nap | C | | 393 (M⁺+1) |
| Ca-a-9 | 5-e | Int.c-a-1 | BRA18 | 2-EtPh | Et | H | 2-Nap | C | | 435 (M⁺+1) |
| Ca-a-10 | 1-a | Ca-a-9 | | 2-EtPh | H | H | 2-Nap | C | | 407 (M⁺+1) |
| Ca-a-11 | 5-e | Int.c-a-1 | BRA20 | 2-iPrPh | Et | H | 2-Nap | C | | 449 (M⁺+1) |
| Ca-a-12 | 1-a | Ca-a-11 | | 2-iPrPh | H | H | 2-Nap | C | | 421 (M⁺+1) |
| Ca-a-13 | 5-e | Int.c-a-1 | BRA21 | 4-iPrPh | Et | H | 2-Nap | C | | 449 (M⁺+1) |
| Ca-a-14 | 1-a | Ca-a-13 | | 4-iPrPh | H | H | 2-Nap | C | | 421 (M⁺+1) |
| Ca-a-15 | 5-e | Int.c-a-1 | BRA22 | 1-nBuPh | Et | H | 2-Nap | C | | 463 (M⁺+1) |
| Ca-a-16 | 1-a | Ca-a-15 | | 1-nBuPh | H | H | 2-Nap | C | | 435 (M⁺+1) |
| Ca-a-17 | 5-e | Int.c-a-1 | BRA24 | 2-MeOPh | Et | H | 2-Nap | C | | 437 (M⁺+1) |
| Ca-a-18 | 1-a | Ca-a-17 | | 2-MeOPh | H | H | 2-Nap | C | | 409 (M⁺+1) |
| Ca-a-19 | 5-e | Int.c-a-1 | BRA25 | 3-MeOPh | Et | H | 2-Nap | C | | 437 (M⁺+1) |
| Ca-a-20 | 1-a | Ca-a-19 | | 3-MeOPh | H | H | 2-Nap | C | | 409 (M⁺+1) |
| Ca-a-21 | 5-e | Int.c-a-1 | BRA27 | 2-EtOPh | Et | H | 2-Nap | C | | 451 (M⁺+1) |
| Ca-a-22 | 1-a | Ca-a-21 | | 2-EtOPh | H | H | 2-Nap | C | | 423 (M⁺+1) |
| Ca-a-23 | 5-e | Int.c-a-1 | BRA30 | 4-iPrOPh | Et | H | 2-Nap | C | | 465 (M⁺+1) |
| Ca-a-24 | 1-a | Ca-a-23 | | 4-iPrOPh | H | H | 2-Nap | C | | 437 (M⁺+1) |
| Ca-a-25 | 5-e | Int.c-a-1 | BRA31 | 3-nPrOPh | Et | H | 2-Nap | C | | 465 (M⁺+1) |
| Ca-a-26 | 1-a | Ca-a-25 | | 3-nPrOPh | H | H | 2-Nap | C | | 437 (M⁺+1) |
| Ca-a-27 | 5-e | Int.c-a-1 | BRA34 | 4-nBuOPh | Et | H | 2-Nap | C | | 479 (M⁺+1) |
| Ca-a-28 | 1-a | Ca-a-27 | | 4-nBuOPh | H | H | 2-Nap | C | | 451 (M⁺+1) |
| Ca-a-29 | 5-e | Int.c-a-1 | BRA36 | 4-DMAPh | Et | H | 2-Nap | C | | 450 (M⁺+1) |
| Ca-a-30 | 1-a | Ca-a-29 | | 4-DMAPh | H | H | 2-Nap | C | | 422 (M⁺+1) |
| Ca-a-31 | 5-e | Int.c-a-1 | BRA37 | 2-FPh | Et | H | 2-Nap | C | | 425 (M⁺+1) |
| Ca-a-32 | 1-a | Ca-a-31 | | 2-FPh | H | H | 2-Nap | C | | 397 (M⁺+1) |
| Ca-a-33 | 5-e | Int.c-a-1 | BRA38 | 3-FPh | Et | H | 2-Nap | C | | 425 (M⁺+1) |
| Ca-a-34 | 1-a | Ca-a-33 | | 3-FPh | H | H | 2-Nap | C | | 397 (M⁺+1) |
| Ca-a-35 | 5-e | Int.c-a-1 | BRA39 | 4-FPh | Et | H | 2-Nap | C | | 425 (M⁺+1) |
| Ca-a-36 | 1-a | Ca-a-35 | | 4-FPh | H | H | 2-Nap | C | | 397 (M⁺+1) |
| Ca-a-37 | 5-e | Int.c-a-1 | BRA40 | 2-ClPh | Et | H | 2-Nap | C | | 441 (M⁺+1) |
| Ca-a-38 | 1-a | Ca-a-37 | | 2-ClPh | H | H | 2-Nap | C | | 413 (M⁺+1) |
| Ca-a-39 | 5-e | Int.c-a-1 | BRA42 | 4-ClPh | Et | H | 2-Nap | C | | 441 (M⁺+1) |
| Ca-a-40 | 1-a | Ca-a-39 | | 4-ClPh | H | H | 2-Nap | C | | 413 (M⁺+1) |
| Ca-a-41 | 5-e | Int.c-a-1 | BRA44 | 3-CF3Ph | Et | H | 2-Nap | C | | 475 (M⁺+1) |
| Ca-a-42 | 1-a | Ca-a-41 | | 3-CF3Ph | H | H | 2-Nap | C | | 447 (M⁺+1) |
| Ca-a-43 | 5-e | Int.c-a-1 | BRA46 | 2,3-DFPh | Et | H | 2-Nap | C | | 443 (M⁺+1) |

**[Table 89]**

| Table-Ca-A-2 | | | | | | | | | | |
|---|---|---|---|---|---|---|---|---|---|---|
| Exp. | Syn | SM1 | Reagent | Rx | Y | V₁' | V₂' | LCMS | | |
| | | | | | | | | method | RTime | Mass |
| Ca-a-44 | 1-a | Ca-a-43 | | 2,3-DFPh | H | H | 2-Nap | C | | 415 (M⁺+1) |
| Ca-a-45 | 5-e | Int.c-a-1 | BRA47 | 2,4-DFPh | Et | H | 2-Nap | C | | 443 (M⁺+1) |
| Ca-a-46 | 1-a | Ca-a-45 | | 2,4-DFPh | H | H | 2-Nap | C | | 415 (M⁺+1) |
| Ca-a-47 | 5-e | Int.c-a-1 | BRA49 | 2,6-DFPh | Et | H | 2-Nap | C | | 443 (M⁺+1) |
| Ca-a-48 | 1-a | Ca-a-47 | | 2,6-DFPh | H | H | 2-Nap | C | | 415 (M⁺+1) |
| Ca-a-49 | 5-e | Int.c-a-1 | BRA50 | 3,4-DFPh | Et | H | 2-Nap | C | | 443 (M⁺+1) |
| Ca-a-50 | 1-a | Ca-a-49 | | 3,4-DFPh | H | H | 2-Nap | C | | 415 (M⁺+1) |
| Ca-a-51 | 5-e | Int.c-a-1 | BRA51 | 3,5-DFPh | Et | H | 2-Nap | C | | 443 (M⁺+1) |
| Ca-a-52 | 1-a | Ca-a-51 | | 3,5-DFPh | H | H | 2-Nap | C | | 415 (M⁺+1) |
| Ca-a-53 | 5-e | Int.c-a-1 | BRA52 | 2,4-DClPh | Et | H | 2-Nap | C | | 476 (M⁺+1) |
| Ca-a-54 | 1-a | Ca-a-53 | | 2,4-DClPh | H | H | 2-Nap | C | | 448 (M⁺+1) |
| Ca-a-55 | 5-e | Int.c-a-1 | BRA54 | 3,4-DClPh | Et | H | 2-Nap | C | | 476 (M⁺+1) |
| Ca-a-56 | 1-a | Ca-a-55 | | 3,4-DClPh | H | H | 2-Nap | C | | 448 (M⁺+1) |
| Ca-a-57 | 5-e | Int.c-a-1 | BRA55 | 2,3-DMePh | Et | H | 2-Nap | C | | 435 (M⁺+1) |
| Ca-a-58 | 1-a | Ca-a-57 | | 2,3-DMePh | H | H | 2-Nap | C | | 407 (M⁺+1) |
| Ca-a-59 | 5-e | Int.c-a-1 | BRA56 | 2,4-DMePh | Et | H | 2-Nap | C | | 435 (M⁺+1) |
| Ca-a-60 | 1-a | Ca-a-59 | | 2,4-DMePh | H | H | 2-Nap | C | | 407 (M⁺+1) |
| Ca-a-61 | 5-e | Int.c-a-1 | BRA58 | 2-Furan | Et | H | 2-Nap | C | | 397 (M⁺+1) |
| Ca-a-62 | 1-a | Ca-a-61 | | 2-Furan | H | H | 2-Nap | C | | 369 (M⁺+1) |
| Ca-a-63 | 5-e | Int.c-a-1 | BRA59 | 3-Furan | Et | H | 2-Nap | C | | 397 (M⁺+1) |
| Ca-a-64 | 1-a | Ca-a-63 | | 3-Furan | H | H | 2-Nap | C | | 369 (M⁺+1) |
| Ca-a-65 | 5-e | Int.c-a-1 | BRA60 | 2-Thiophene | Et | H | 2-Nap | C | | 413 (M⁺+1) |
| Ca-a-66 | 1-a | Ca-a-65 | | 2-Thiophene | H | H | 2-Nap | C | | 385 (M⁺+1) |
| Ca-a-67 | 5-e | Int.c-a-1 | BRA61 | 3-Thiophene | Et | H | 2-Nap | C | | 413 (M⁺+1) |
| Ca-a-68 | 1-a | Ca-a-67 | | 3-Thiophene | H | H | 2-Nap | C | | 385 (M⁺+1) |
| Ca-a-69 | 5-e | Int.c-a-1 | BRA62 | 3-Pyridine | Et | H | 2-Nap | C | | 408 (M⁺+1) |
| Ca-a-70 | 1-a | Ca-a-69 | | 3-Pyridine | H | H | 2-Nap | C | | 380 (M⁺+1) |
| Ca-a-71 | 5-e | Int.c-a-1 | BRA63 | 4-Pyridine | Et | H | 2-Nap | C | | 408 (M⁺+1) |
| Ca-a-72 | 1-a | Ca-a-71 | | 4-Pyridine | H | H | 2-Nap | C | | 380 (M⁺+1) |
| Ca-a-73 | 5-e | Int.c-a-1 | BRA65 | 4-PhPh | Et | H | 2-Nap | C | | 483 (M⁺+1) |
| Ca-a-74 | 1-a | Ca-a-73 | | 4-PhPh | H | H | 2-Nap | C | | 455 (M⁺+1) |
| Ca-a-75 | 5-e | Int.c-a-1 | BRA66 | C₄H₉CH=CH- | Et | H | 2-Nap | C | | 413 (M⁺+1) |
| Ca-a-76 | 1-a | Ca-a-75 | | C₄H₉CH=CH- | H | H | 2-Nap | C | | 385 (M⁺+1) |
| Ca-a-77 | 9-j | Ca-a-75 | | nHex- | Et | H | 2-Nap | C | | 415 (M⁺+1) |
| Ca-a-78 | 1-a | Ca-a-77 | | nHex- | H | H | 2-Nap | C | | 387 (M⁺+1) |
| Ca-a-79 | 5-e | Int.c-a-1 | BRA67 | 4FPhCH=CH- | Et | H | 2-Nap | C | | 451 (M⁺+1) |
| Ca-a-80 | 1-a | Ca-a-79 | | 4FPhCH=CH- | H | H | 2-Nap | C | | 423 (M⁺+1) |
| Ca-a-81 | 9-j | Ca-a-79 | | 4FPhC₂H₄ | Et | H | 2-Nap | C | | 453 (M⁺+1) |
| Ca-a-82 | 1-a | Ca-a-80 | | 4FPhC₂H₄ | H | H | 2-Nap | C | | 425 (M⁺+1) |
| Ca-a-83 | 5-e | Int.c-a-1 | BRA68 | 4ClPhCH=CH- | Et | H | 2-Nap | C | | 468 (M⁺+1) |
| Ca-a-84 | 1-a | Ca-a-80 | | 4ClPhCH=CH- | H | H | 2-Nap | C | | 439 (M⁺+1) |
| Ca-a-85 | 5-e | Int.c-a-1 | BRA71 | 4MePhCH=CH- | Et | H | 2-Nap | C | | 447 (M⁺+1) |
| Ca-a-86 | 1-a | Ca-a-85 | | 4MePhCH=CH- | H | H | 2-Nap | C | | 419 (M⁺+1) |
| Ca-a-87 | 9-j | Ca-a-85 | | 4MePhC₂H₄ | Et | H | 2-Nap | C | | 449 (M⁺+1) |
| Ca-a-88 | 1-a | Ca-a-87 | | 4MePhC₂H₄ | H | H | 2-Nap | C | | 421 (M⁺+1) |
| Ca-a-89 | 5-e | Int.c-a-1 | BRA72 | MeOCH₂CH=CH- | Et | H | 2-Nap | C | | 401 (M⁺+1) |
| Ca-a-90 | 1-a | Ca-a-89 | | MeOCH₂CH=CH- | H | H | 2-Nap | C | | 373 (M⁺+1) |

**[Table 90]**

| Table-Ca-A-3 | | | | | | | | | | |
|---|---|---|---|---|---|---|---|---|---|---|
| Exp. | Syn | SM1 | Reagent | Rx | Y | V₁' | V₂' | LCMS | | |
| | | | | | | | | method | RTime | Mass |
| Ca-a-91 | 9-j | Ca-a-89 | | I MeOCH₂C₂H₄- | Et | H | 2-Nap | C | | 403 (M⁺+1) |
| Ca-a-92 | 1-a | Ca-a-91 | | I MeOCH₂C₂H₄- | H | H | 2-Nap | C | | 375 (M⁺+1) |
| Ca-a-93 | 5-e | Int.c-a-1 | BRA73 | 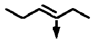 | Et | H | 2-Nap | C | | 413 (M⁺+1) |
| Ca-a-94 | 1-a | Ca-a-93 | | 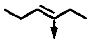 | H | H | 2-Nap | C | | 385 (M⁺+1) |
| Ca-a-95 | 9-j | Ca-a-93 | | 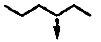 | Et | H | 2-Nap | C | | 415 (M⁺+1) |
| Ca-a-96 | 1-a | Ca-a-71 | | 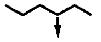 | H | H | 2-Nap | C | | 387 (M⁺+1) |
| Ca-a-97 | 5-e | Int.c-a-2 | BRA14 | Ph | Et | H | 5-Ind | C | | 396 (M⁺+1) |
| Ca-a-98 | 1-a | Ca-a-97 | | Ph | H | H | 5-Ind | C | | 368 (M⁺+1) |
| Ca-a-99 | 5-e | Int.c-a-2 | BRA16 | 3-MePh | Et | H | 5-Ind | C | | 410 (M⁺+1) |
| Ca-a-100 | 1-a | Ca-a-99 | | 3-MePh | H | H | 5-Ind | C | | 382 (M⁺+1) |
| Ca-a-101 | 5-e | Int.c-a-2 | BRA18 | 2-EtPh | Et | H | 5-Ind | C | | 424 (M⁺+1) |
| Ca-a-102 | 1-a | Ca-a-101 | | 2-EtPh | H | H | 5-Ind | C | | 396 (M⁺+1) |
| Ca-a-103 | 5-e | Int.c-a-2 | BRA19 | 4-EtPh | Et | H | 5-Ind | C | | 424 (M⁺+1) |
| Ca-a-104 | 1-a | Ca-a-103 | | 4-EtPh | H | H | 5-Ind | C | | 396 (M⁺+1) |
| Ca-a-105 | 5-e | Int.c-a-2 | BRA20 | 2-iPrPh | Et | H | 5-Ind | C | | 438 (M⁺+1) |
| Ca-a-106 | 1-a | Ca-a-105 | | 2-iPrPh | H | H | 5-Ind | C | | 410 (M⁺+1) |
| Ca-a-107 | 5-e | Int.c-a-2 | BRA23 | 4-tBuPh | Et | H | 5-Ind | C | | 452 (M⁺+1) |
| Ca-a-108 | 1-a | Ca-a-107 | | 4-tBuPh | H | H | 5-Ind | C | | 424 (M⁺+1) |
| Ca-a-109 | 5-e | Int.c-a-2 | BRA25 | 3-MeOPh | Et | H | 5-Ind | C | | 426 (M⁺+1) |
| Ca-a-110 | 1-a | Ca-a-109 | | 3-MeOPh | H | H | 5-Ind | C | | 398 (M⁺+1) |
| Ca-a-111 | 5-e | Int.c-a-2 | BRA26 | 4-MeOPh | Et | H | 5-Ind | C | | 426 (M⁺+1) |
| Ca-a-112 | 1-a | Ca-a-111 | | 4-MeOPh | H | H | 5-Ind | C | | 398 (M⁺+1) |
| Ca-a-113 | 5-e | Int.c-a-2 | BRA28 | 3-EtOPh | Et | H | 5-Ind | C | | 440 (M⁺+1) |
| Ca-a-114 | 1-a | Ca-a-113 | | 3-EtOPh | H | H | 5-Ind | C | | 412 (M⁺+1) |
| Ca-a-115 | 5-e | Int.c-a-2 | BRA29 | 3-iPrOPh | Et | H | 5-Ind | C | | 454 (M⁺+1) |
| Ca-a-116 | 1-a | Ca-a-115 | | 3-iPrOPh | H | H | 5-Ind | C | | 426 (M⁺+1) |
| Ca-a-117 | 5-e | Int.c-a-2 | BRA31 | 3-nPrOPh | Et | H | 5-Ind | C | | 454 (M⁺+1) |
| Ca-a-118 | 1-a | Ca-a-116 | | 3-nPrOPh | H | H | 5-Ind | C | | 426 (M⁺+1) |
| Ca-a-119 | 5-e | Int.c-a-2 | BRA32 | 4-nPrOPh | Et | H | 5-Ind | C | | 454 (M⁺+1) |
| Ca-a-120 | 1-a | Ca-a-120 | | 4-nPrOPh | H | H | 5-Ind | C | | 426 (M⁺+1) |
| Ca-a-121 | 5-e | Int.c-a-2 | BRA33 | 3-nBuOPh | Et | H | 5-Ind | C | | 468 (M⁺+1) |
| Ca-a-122 | 1-a | Ca-a-122 | | 3-nBuOPh | H | H | 5-Ind | C | | 440 (M⁺+1) |
| Ca-a-123 | 5-e | Int.c-a-2 | BRA36 | 4-DMAPh | Et | H | 5-Ind | C | | 439 (M⁺+1) |
| Ca-a-124 | 1-a | Ca-a-123 | | 4-DMAPh | H | H | 5-Ind | C | | 411 (M⁺+1) |
| Ca-a-125 | 5-e | Int.c-a-2 | BRA37 | 2-FPh | Et | H | 5-Ind | C | | 414 (M⁺+1) |
| Ca-a-126 | 1-a | Ca-a-125 | | 2-FPh | H | H | 5-Ind | C | | 386 (M⁺+1) |
| Ca-a-127 | 5-e | Int.c-a-2 | BRA38 | 3-FPh | Et | H | 5-Ind | C | | 414 (M⁺+1) |
| Ca-a-128 | 1-a | Ca-a-127 | | 3-FPh | H | H | 5-Ind | C | | 386 (M⁺+1) |
| Ca-a-129 | 5-e | Int.c-a-2 | BRA41 | 3-ClPh | Et | H | 5-Ind | C | | 430 (M⁺+1) |
| Ca-a-130 | 1-a | Ca-a-129 | | 3-ClPh | H | H | 5-Ind | C | | 402 (M⁺+1) |
| Ca-a-131 | 5-e | Int.c-a-2 | BRA42 | 4-ClPh | Et | H | 5-Ind | C | | 430 (M⁺+1) |
| Ca-a-132 | 1-a | Ca-a-131 | | 4-ClPh | H | H | 5-Ind | C | | 402 (M⁺+1) |
| Ca-a-133 | 5-e | Int.c-a-2 | BRA43 | 2-CF3Ph | Et | H | 5-Ind | C | | 464 (M⁺+1) |
| Ca-a-134 | 1-a | Ca-a-133 | | 2-CF3Ph | H | H | 5-Ind | C | | 436 (M⁺+1) |
| Ca-a-135 | 5-e | Int.c-a-2 | BRA45 | 4-CF3Ph | Et | H | 5-Ind | C | | 464 (M⁺+1) |

**[Table 91]**

| Table-Ca-A-4 | | | | | | | | | | |
|---|---|---|---|---|---|---|---|---|---|---|
| Exp. | Syn | SM1 | Reagent | Rx | Y | V₁' | V₂' | LCMS | | |
| | | | | | | | | method | RTime | Mass |
| Ca-a-136 | 1-a | Ca-a-135 | | 4-CF3Ph | H | H | 5-Ind | C | | 436 (M⁺+1) |
| Ca-a-137 | 5-e | Int.c-a-2 | BRA47 | 2.4-DFPh | Et | H | 5-Ind | C | | 432 (M⁺+1) |
| Ca-a-138 | 1-a | Ca-a-137 | | 2,4-DFPh | H | H | 5-Ind | C | | 404 (M⁺+1) |
| Ca-a-139 | 5-e | Int.c-a-2 | BRA48 | 2,5-DFPh | Et | H | 5-Ind | C | | 432 (M⁺+1) |
| Ca-a-140 | 1-a | Ca-a-139 | | 2,5-DFPh | H | H | 5-Ind | C | | 404 (M⁺+1) |
| Ca-a-141 | 5-e | Int.c-a-2 | BRA49 | 2,6-DFPh | Et | H | 5-Ind | C | | 432 (M⁺+1) |
| Ca-a-142 | 1-a | Ca-a-141 | | 2,6-DFPh | H | H | 5-Ind | C | | 404 (M⁺+1) |
| Ca-a-143 | 5-e | Int.c-a-2 | BRA53 | 3,5-DClPh | Et | H | 5-Ind | C | | 465 (M⁺+1) |
| Ca-a-144 | 1-a | Ca-a-143 | | 3,5-DCIPh | H | H | 5-Ind | C | | 437 (M⁺+1) |
| Ca-a-145 | 5-e | Int.c-a-2 | BRA54 | 3,4-DClPh | Et | H | 5-Ind | C | | 465 (M⁺+1) |
| Ca-a-146 | 1-a | Ca-a-145 | | 3,4-DClPh | H | H | 5-Ind | C | | 437 (M⁺+1) |
| Ca-a-147 | 5-e | Int.c-a-2 | BRA56 | 2,4-DMePh | Et | H | 5-Ind | C | | 424 (M⁺+1) |
| Ca-a-148 | 1-a | Ca-a-147 | | 2,4-DMePh | H | H | 5-Ind | C | | 396 (M⁺+1) |
| Ca-a-149 | 5-e | Int.c-a-2 | BRA57 | 2,6-DMePh | Et | H | 5-Ind | C | | 424 (M⁺+1) |
| Ca-a-150 | 1-a | Ca-a-149 | | 2,6-DMePh | H | H | 5-Ind | C | | 396 (M⁺+1) |
| Ca-a-151 | 5-e | Int.c-a-2 | BRA58 | 2-Furan | Et | H | 5-Ind | C | | 386 (M⁺+1) |
| Ca-a-152 | 1-a | Ca-a-151 | | 2-Furan | H | H | 5-Ind | C | | 358 (M⁺+1) |
| Ca-a-153 | 5-e | Int.c-a-2 | BRA59 | 3-Furan | Et | H | 5-Ind | C | | 386 (M⁺+1) |
| Ca-a-154 | 1-a | Ca-a-153 | | 3-Furan | H | H | 5-Ind | C | | 358 (M⁺+1) |
| Ca-a-155 | 5-e | Int.c-a-2 | BRA60 | 2-Thiophene | Et | H | 5-Ind | C | | 402 (M⁺+1) |
| Ca-a-156 | 1-a | Ca-a-155 | | 2-Thiophene | H | H | 5-Ind | C | | 374 (M⁺+1) |
| Ca-a-157 | 5-e | Int.c-a-2 | BRA61 | 3-Thiophene | Et | H | 5-Ind | C | | 402 (M⁺+1) |
| Ca-a-158 | 1-a | Ca-a-157 | | 3-Thiophene | H | H | 5-Ind | C | | 374 (M⁺+1) |
| Ca-a-159 | 5-e | Int.c-a-2 | BRA63 | 4-Pyridine | Et | H | 5-Ind | C | | 397 (M⁺+1) |
| Ca-a-160 | 1-a | Ca-a-159 | | 4-Pyridine | H | H | 5-Ind | C | | 369 (M⁺+1) |
| Ca-a-161 | 5-e | Int.c-a-2 | BRA66 | C₄H₉CH=CH- | Et | H | 5-Ind | C | | 402 (M⁺+1) |
| Ca-a-162 | 1-a | Ca-a-161 | | C₄H₉CH=CH- | H | H | 5-Ind | C | | 374 (M⁺+1) |
| Ca-a-163 | 5-e | Int.c-a-2 | BRA67 | 4FPhCH=CH- | Et | H | 5-Ind | C | | 440 (M⁺+1) |
| Ca-a-164 | 1-a | Ca-a-163 | | 4FPhCH=CH- | H | H | 5-Ind | C | | 412 (M⁺+1) |
| Ca-a-165 | 9-j | Ca-a-163 | | 4FPhC₂H₄ | Et | H | 5-Ind | C | | 442 (M⁺+1) |
| Ca-a-166 | 1-a | Ca-a-165 | | 4FPhC₂H₄ | H | H | 5-Ind | C | | 414 (M⁺+1) |
| Ca-a-167 | 5-e | Int.c-a-2 | BRA71 | 4MePhCH=CH- | Et | H | 5-Ind | C | | 436 (M⁺+1) |
| Ca-a-168 | 1-a | Ca-a-167 | | 4MePhCH=CH- | H | H | 5-Ind | C | | 408 (M⁺+1) |
| Ca-a-169 | 5-e | Int.c-a-2 | BRA73 | 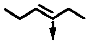 | Et | H | 5-Ind | C | | 402 (M⁺+1) |
| Ca-a-170 | 1-a | Ca-a-169 | | 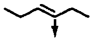 | H | H | 5-Ind | C | | 374 (M⁺+1) |
| Ca-a-171 | 9-j | Ca-a-169 | | 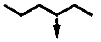 | Et | H | 5-Ind | C | | 404 (M⁺+1) |
| Ca-a-172 | 1-a | Ca-a-171 | | 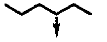 | H | H | 5-Ind | C | | 376 (M⁺+1) |
| Ca-a-173 | 5-e | Int.c-a-3 | BRA15 | 2-MePh | Et | H | 1Me-5-Ind | C | | 424 (M⁺+1) |
| Ca-a-174 | 1-a | Ca-a-173 | | 2-MePh | H | H | 1Me-5-Ind | C | | 396 (M⁺+1) |
| Ca-a-175 | 5-e | Int.c-a-3 | BRA17 | 4-MePh | Et | H | 1Me-5-Ind | C | | 424 (M⁺+1) |
| Ca-a-176 | 1-a | Ca-a-175 | | 4-MePh | H | H | 1Me-5-Ind | C | | 396 (M⁺+1) |
| Ca-a-177 | 5-e | Int.c-a-3 | BRA19 | 4-EtPh | Et | H | 1Me-5-Ind | C | | 438 (M⁺+1) |
| Ca-a-178 | 1-a | Ca-a-177 | | 4-EtPh | H | H | 1Me-5-Ind | C | | 410 (M⁺+1) |
| Ca-a-179 | 5-e | Int.c-a-3 | BRA21 | 4-iPrPh | Et | H | 1Me-5-Ind | C | | 452 (M⁺+1) |

**[Table 92]**

| Table-Ca-A-5 | | | | | | | | | | |
|---|---|---|---|---|---|---|---|---|---|---|
| Exp. | Syn. | SM1 | Reagent | Rx | Y | V₁' | V₂' | LCMS | | |
| | | | | | | | | method | RTime | Mass |
| Ca-a-180 | 1-a | Ca-a-179 | | 4-iPrPh | H | H | 1Me-5-Ind | C | | 424 (M⁺+1) |
| Ca-a-181 | 5-e | Int.c-a-3 | BRA22 | 1-nBuPh | Et | H | 1Me-5-Ind | C | | 466 (M⁺+1) |
| Ca-a-182 | 1-a | Ca-a-181 | | 1-nBuPh | H | H | 1Me-5-Ind | C | | 438 (M⁺+1) |
| Ca-a-183 | 5-e | Int.c-a-3 | BRA24 | 2-MeOPh | Et | H | 1Me-5-Ind | C | | 440 (M⁺+1) |
| Ca-a-184 | 1-a | Ca-a-183 | | 2-MeOPh | H | H | 1Me-5-Ind | C | | 412 (M⁺+1) |
| Ca-a-185 | 5-e | Int.c-a-3 | BRA26 | 4-MeOPh | Et | H | 1Me-5-Ind | C | | 440 (M⁺+1) |
| Ca-a-186 | 1-a | Ca-a-185 | | 4-MeOPh | H | H | 1Me-5-Ind | C | | 412 (M⁺+1) |
| Ca-a-187 | 5-e | Int.c-a-3 | BRA27 | 2-EtOPh | Et | H | 1Me-5-Ind | C | | 454 (M⁺+1) |
| Ca-a-188 | 1-a | Ca-a-187 | | 2-EtOPh | H | H | 1Me-5-Ind | C | | 426 (M⁺+1) |
| Ca-a-189 | 5-e | Int.c-a-3 | BRA30 | 4-iPrOPh | Et | H | 1Me-5-Ind | C | | 468 (M⁺+1) |
| Ca-a-190 | 1-a | Ca-a-189 | | 4-iPrOPh | H | H | 1Me-5-Ind | C | | 440 (M⁺+1) |
| Ca-a-191 | 5-e | Int.c-a-3 | BRA32 | 4-nPrOPh | Et | H | 1Me-5-Ind | C | | 468 (M⁺+1) |
| Ca-a-192 | 1-a | Ca-a-191 | | 4-nPrOPh | H | H | 1Me-5-Ind | C | | 440 (M⁺+1) |
| Ca-a-193 | 5-e | Int.c-a-3 | BRA33 | 3-nBuOPh | Et | H | 1Me-5-Ind | C | | 482 (M⁺+1) |
| Ca-a-194 | 1-a | Ca-a-193 | | 3-nBuOPh | H | H | 1Me-5-Ind | C | | 454 (M⁺+1) |
| Ca-a-195 | 5-e | Int.c-a-3 | BRA35 | 3-DMAPh | Et | H | 1Me-5-Ind | C | | 453 (M⁺+1) |
| Ca-a-196 | 1-a | Ca-a-195 | | 3-DMAPh | H | H | 1Me-5-Ind | C | | 425 (M⁺+1) |
| Ca-a-197 | 5-e | Int.c-a-3 | BRA37 | 2-FPh | Et | H | 1Me-5-Ind | C | | 428 (M⁺+1) |
| Ca-a-198 | 1-a | Ca-a-197 | | 2-FPh | H | H | 1Me-5-Ind | C | | 400 (M⁺+1) |
| Ca-a-199 | 5-e | Int.c-a-3 | BRA38 | 3-FPh | Et | H | 1Me-5-Ind | C | | 428 (M⁺+1) |
| Ca-a-200 | 1-a | Ca-a-199 | | 3-FPh | H | H | 1Me-5-Ind | C | | 400 (M⁺+1) |
| Ca-a-201 | 5-e | Int.c-a-3 | BRA39 | 4-FPh | Et | H | 1Me-5-Ind | C | | 428 (M⁺+1) |
| Ca-a-202 | 1-a | Ca-a-201 | | 4-FPh | H | H | 1Me-5-Ind | C | | 400 (M⁺+1) |
| Ca-a-203 | 5-e | Int.c-a-3 | BRA40 | 2-ClPh | Et | H | 1Me-5-Ind | C | | 444 (M⁺+1) |
| Ca-a-204 | 1-a | Ca-a-203 | | 2-ClPh | H | H | 1Me-5-Ind | C | | 416 (M⁺+1) |
| Ca-a-205 | 5-e | Int.c-a-3 | BRA41 | 3-ClPh | Et | H | 1Me-5-Ind | C | | 444 (M⁺+1) |
| Ca-a-206 | 1-a | Ca-a-205 | | 3-ClPh | H | H | 1Me-5-Ind | C | | 416 (M⁺+1) |
| Ca-a-207 | 5-e | Int.c-a-3 | BRA44 | 3-CF3Ph | Et | H | 1Me-5-Ind | C | | 478 (M⁺+1) |
| Ca-a-208 | 1-a | Ca-a-207 | | 3-CF3Ph | H | H | 1Me-5-Ind | C | | 450 (M⁺+1) |
| Ca-a-209 | 5-e | Int.c-a-3 | BRA45 | 4-CF3Ph | Et | H | 1Me-5-Ind | C | | 478 (M⁺+1) |
| Ca-a-210 | 1-a | Ca-a-209 | | 4-CF3Ph | H | H | 1Me-5-Ind | C | | 450 (M⁺+1) |
| Ca-a-211 | 5-e | Int.c-a-3 | BRA47 | 2,4-DFPh | Et | H | 1Me-5-Ind | C | | 446 (M⁺+1) |
| Ca-a-212 | 1-a | Ca-a-211 | | 2,4-DFPh | H | H | 1Me-5-Ind | C | | 418 (M⁺+1) |
| Ca-a-213 | 5-e | Int.c-a-3 | BRA48 | 2,5-DFPh | Et | H | 1Me-5-Ind | C | | 446 (M⁺+1) |
| Ca-a-214 | 1-a | Ca-a-213 | | 2,5-DFPh | H | H | 1Me-5-Ind | C | | 418 (M⁺+1) |
| Ca-a-215 | 5-e | Int.c-a-3 | BRA50 | 3,4-DFPh | Et | H | 1Me-5-Ind | C | | 446 (M⁺+1) |
| Ca-a-216 | 1-a | Ca-a-215 | | 3,4-DFPh | H | H | 1Me-5-Ind | C | | 418 (M⁺+1) |
| Ca-a-217 | 5-e | Int.c-a-3 | BRA52 | 2,4-DClPh | Et | H | 1Me-5-Ind | C | | 479 (M⁺+1) |
| Ca-a-218 | 1-a | Ca-a-217 | | 2,4-DClPh | H | H | 1Me-5-Ind | C | | 451 (M⁺+1) |
| Ca-a-219 | 5-e | Int.c-a-3 | BRA53 | 3,5-DClPh | Et | H | 1Me-5-Ind | C | | 479 (M⁺+1) |
| Ca-a-220 | 1-a | Ca-a-219 | | 3,5-DClPh | H | H | 1Me-5-Ind | C | | 451 (M⁺+1) |
| Ca-a-221 | 5-e | Int.c-a-3 | BRA54 | 3,4-DClPh | Et | H | 1Me-5-Ind | C | | 479 (M⁺+1) |
| Ca-a-222 | 1-a | Ca-a-221 | | 3,4-DClPh | H | H | 1Me-5-Ind | C | | 451 (M⁺+1) |
| Ca-a-223 | 5-e | Int.c-a-3 | BRA56 | 2,4-DMePh | Et | H | 1Me-5-Ind | C | | 438 (M⁺+1) |
| Ca-a-224 | 1-a | Ca-a-223 | | 2,4-DMePh | H | H | 1Me-5-Ind | C | | 410 (M⁺+1) |
| Ca-a-225 | 5-e | Int.c-a-3 | BRA57 | 2,6-DMePh | Et | H | 1Me-5-Ind | C | | 438 (M⁺+1) |
| Ca-a-226 | 1-a | Ca-a-225 | | 2.6-DMePh | H | H | 1Me-5-Ind | C | | 410 (M⁺+1) |

**[Table 93]**

| Table-Ca-A-6 | | | | | | | | | | |
|---|---|---|---|---|---|---|---|---|---|---|
| Exp. | Syn. | SM1 | Reagent | Rx | Y | V₁' | V₂' | LCMS | | |
| | | | | | | | | method | RTime | Mass |
| Ca-a-227 | 5-e | Int.c-a-3 | BRA59 | 3-Furan | Et | H | 1 Me-5-Ind | C | | 400 (M⁺+1) |
| Ca-a-228 | 1-a | Ca-a-227 | | 3-Furan | H | H | 1 Me-5-Ind | C | | 372 (M⁺+1) |
| Ca-a-229 | 5-e | Int.c-a-3 | BRA60 | 2-Thiophene | Et | H | 1 Me-5-Ind | C | | 416 (M⁺+1) |
| Ca-a-230 | 1-a | Ca-a-229 | | 2-Thiophene | H | H | 1 Me-5-Ind | C | | 388 (M⁺+1) |
| Ca-a-231 | 5-e | Int.c-a-3 | BRA61 | 3-Thiophene | Et | H | 1 Me-5-Ind | C | | 416 (M⁺+1) |
| Ca-a-232 | 1-a | Ca-a-231 | | 3-Thiophene | H | H | 1 Me-5-Ind | C | | 388 (M⁺+1) |
| Ca-a-233 | 5-e | Int.c-a-3 | BRA63 | 4-Pyridine | Et | H | 1 Me-5-Ind | C | | 411 (M⁺+1) |
| Ca-a-234 | 1-a | Ca-a-233 | | 4-Pyridine | H | H | 1 Me-5-Ind | C | | 383 (M⁺+1) |
| Ca-a-235 | 5-e | Int.c-a-3 | BRA65 | 4-PhPh | Et | H | 1 Me-5-Ind | C | | 486 (M⁺+1) |
| Ca-a-236 | 1-a | Ca-a-235 | | 4-PhPh | H | H | 1 Me-5-Ind | C | | 458 (M⁺+1) |
| Ca-a-237 | 5-e | Int.c-a-3 | BRA67 | 4FPhCH=CH- | Et | H | 1 Me-5-Ind | C | | 454 (M⁺+1) |
| Ca-a-238 | 1-a | Ca-a-237 | | 4FPhCH=CH- | H | H | 1 Me-5-Ind | C | | 426 (M⁺+1) |
| Ca-a-239 | 9-j | Ca-a-237 | | 4FPhC₂H₄ | Et | H | 1 Me-5-Ind | C | | 456 (M⁺+1) |
| Ca-a-240 | 1-a | Ca-a-239 | | 4FPhC₂H₄ | H | H | 1 Me-5-Ind | C | | 428 (M⁺+1) |
| Ca-a-241 | 5-e | Int.c-a-3 | BRA68 | 4ClPhCH=CH- | Et | H | 1 Me-5-Ind | C | | 471 (M⁺+1) |
| Ca-a-242 | 1-a | Ca-a-241 | | 4ClPhCH=CH- | H | H | 1 Me-5-Ind | C | | 442 (M⁺+1) |
| Ca-a-243 | 5-e | Int.c-a-3 | BRA72 | MeOCH₂CH=CH- | Et | H | 1 Me-5-Ind | C | | 404 (M⁺+1) |
| Ca-a-244 | 1-a | Ca-a-243 | | MeOCH₂CH=CH- | H | H | 1 Me-5-Ind | C | | 376 (M⁺+1) |
| Ca-a-245 | 9-j | Ca-a-243 | | MeOCH₂C₂H₄ | Et | H | 1 Me-5-Ind | C | | 406 (M⁺+1) |
| Ca-a-246 | 1-a | Ca-a-245 | | MeOCH₂C₂H₄ | H | H | 1 Me-5-Ind | C | | 378 (M⁺+1) |
| Ca-a-247 | 5-e | Int.c-a-3 | BRA73 | 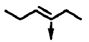 | Et | H | 1 Me-5-Ind | C | | 416 (M⁺+1) |
| Ca-a-248 | 1-a | Ca-a-247 | | 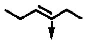 | H | H | 1 Me-5-Ind | C | | 388 (M⁺+1) |
| Ca-a-249 | 9-j | Ca-a-247 | | 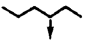 | Et | H | 1 Me-5-Ind | C | | 418 (M⁺+1) |
| Ca-a-250 | 1-a | Ca-a-249 | | 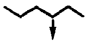 | H | H | 1 Me-5-Ind | C | | 390 (M⁺+1) |
| Ca-a-251 | 5-e | Int.c-a-4 | BRA14 | Ph | Et | H | 5-1Idz | C | | 397 (M⁺+1) |
| Ca-a-252 | 1-a | Ca-a-251 | | Ph | H | H | 5-1Idz | C | | 369 (M⁺+1) |
| Ca-a-253 | 5-e | Int.c-a-4 | BRA16 | 3-MePh | Et | H | 5-1Idz | C | | 411 (M⁺+1) |
| Ca-a-254 | 1-a | Ca-a-253 | | 3-MePh | H | H | 5-1Idz | C | | 383 (M⁺+1) |
| Ca-a-255 | 5-e | Int.c-a-4 | BRA17 | 4-MePh | Et | H | 5-1Idz | C | | 411 (M⁺+1) |
| Ca-a-256 | 1-a | Ca-a-255 | | 4-MePh | H | H | 5-1Idz | C | | 383 (M⁺+1) |
| Ca-a-257 | 5-e | Int.c-a-4 | BRA18 | 2-EtPh | Et | H | 5-1Idz | C | | 425 (M⁺+1) |
| Ca-a-258 | 1-a | Ca-a-257 | | 2-EtPh | H | H | 5-1Idz | C | | 397 (M⁺+1) |
| Ca-a-259 | 5-e | Int.c-a-4 | BRA20 | 2-iPrPh | Et | H | 5-1Idz | C | | 439 (M⁺+1) |
| Ca-a-260 | 1-a | Ca-a-259 | | 2-iPrPh | H | H | 5-1Idz | C | | 411 (M⁺+1) |
| Ca-a-261 | 5-e | Int.c-a-4 | BRA23 | 4-tBuPh | Et | H | 5-1Idz | C | | 453 (M⁺+1) |
| Ca-a-262 | 1-a | Ca-a-261 | | 4-tBuPh | H | H | 5-1Idz | C | | 425 (M⁺+1) |
| Ca-a-263 | 5-e | Int.c-a-4 | BRA25 | 3-MeOPh | Et | H | 5-1Idz | C | | 427 (M⁺+1) |
| Ca-a-264 | 1-a | Ca-a-263 | | 3-MeOPh | H | H | 5-1Idz | C | | 399 (M⁺+1) |
| Ca-a-265 | 5-e | Int.c-a-4 | BRA28 | 3-EtOPh | Et | H | 5-1Idz | C | | 441 (M⁺+1) |
| Ca-a-266 | 1-a | Ca-a-265 | | 3-EtOPh | H | H | 5-1Idz | C | | 413 (M⁺+1) |
| Ca-a-267 | 5-e | Int.c-a-4 | BRA29 | 3-iPrOPh | Et | H | 5-1Idz | C | | 455 (M⁺+1) |
| Ca-a-268 | 1-a | Ca-a-267 | | 3-iPrOPh | H | H | 5-1Idz | C | | 427 (M⁺+1) |
| Ca-a-269 | 5-e | Int.c-a-4 | BRA32 | 4-nPrOPh | Et | H | 5-1Idz | C | | 455 (M⁺+1) |

**[Table 94]**

| Table-Ca-A-7 | | | | | | | | | | |
|---|---|---|---|---|---|---|---|---|---|---|
| Exp. | Syn. | SM1 | Reagent | Rx | Y | V₁' | V₂' | LCMS | | |
| | | | | | | | | method | RTime | Mass |
| Ca-a-270 | 1-a | Ca-a-269 | | 4-nPrOPh | H | H | 5-1Idz | C | | 427 (M⁺+1) |
| Ca-a-271 | 5-e | Int.c-a-4 | BRA33 | 3-nBuOPh | Et | H | 5-1Idz | C | | 469 (M⁺+1) |
| Ca-a-272 | 1-a | Ca-a-271 | | 3-nBuOPh | H | H | 5-1Idz | C | | 441 (M⁺+1) |
| Ca-a-273 | 5-e | Int.c-a-4 | BRA35 | 3-DMAPh | Et | H | 5-1Idz | C | | 440 (M⁺+1) |
| Ca-a-274 | 1-a | Ca-a-273 | | 3-DMAPh | H | H | 5-1Idz | C | | 412 (M⁺+1) |
| Ca-a-275 | 5-e | Int.c-a-4 | BRA36 | 4-DMAPh | Et | H | 5-1Idz | C | | 440 (M⁺+1) |
| Ca-a-276 | 1-a | Ca-a-275 | | 4-DMAPh | H | H | 5-1Idz | C | | 412 (M⁺+1) |
| Ca-a-277 | 5-e | Int.c-a-4 | BRA38 | 3-FPh | Et | H | 5-1Idz | C | | 415 (M⁺+1) |
| Ca-a-278 | 1-a | Ca-a-277 | | 3-FPh | H | H | 5-1Idz | C | | 387 (M⁺+1) |
| Ca-a-279 | 5-e | Int.c-a-4 | BRA39 | 4-FPh | Et | H | 5-1Idz | C | | 415 (M⁺+1) |
| Ca-a-280 | 1-a | Ca-a-279 | | 4-FPh | H | H | 5-1Idz | C | | 387 (M⁺+1) |
| Ca-a-281 | 5-e | Int.c-a-4 | BRA40 | 2-ClPh | Et | H | 5-1Idz | C | | 431 (M⁺+1) |
| Ca-a-282 | 1-a | Ca-a-281 | | 2-ClPh | H | H | 5-1Idz | C | | 403 (M⁺+1) |
| Ca-a-283 | 5-e | Int.c-a-4 | BRA41 | 3-ClPh | Et | H | 5-1Idz | C | | 431 (M⁺+1) |
| Ca-a-284 | 1-a | Ca-a-283 | | 3-ClPh | H | H | 5-1Idz | C | | 403 (M⁺+1) |
| Ca-a-285 | 5-e | Int.c-a-4 | BRA42 | 4-ClPh | Et | H | 5-1Idz | C | | 431 (M⁺+1) |
| Ca-a-286 | 1-a | Ca-a-285 | | 4-ClPh | H | H | 5-1Idz | C | | 403 (M⁺+1) |
| Ca-a-287 | 5-e | Int.c-a-4 | BRA43 | 2-CF3Ph | Et | H | 5-1Idz | C | | 465 (M⁺+1) |
| Ca-a-288 | 1-a | Ca-a-287 | | 2-CF3Ph | H | H | 5-1Idz | C | | 437 (M⁺+1) |
| Ca-a-289 | 5-e | Int.c-a-4 | BRA45 | 4-CF3Ph | Et | H | 5-1Idz | C | | 465 (M⁺+1) |
| Ca-a-290 | 1-a | Ca-a-289 | | 4-CF3Ph | H | H | 5-1Idz | C | | 437 (M⁺+1) |
| Ca-a-291 | 5-e | Int.c-a-4 | BRA48 | 2,5-DFPh | Et | H | 5-1Idz | C | | 433 (M⁺+1) |
| Ca-a-292 | 1-a | Ca-a-291 | | 2,5-DFPh | H | H | 5-1Idz | C | | 405 (M⁺+1) |
| Ca-a-293 | 5-e | Int.c-a-4 | BRA49 | 2,6-DFPh | Et | H | 5-1Idz | C | | 433 (M⁺+1) |
| Ca-a-294 | 1-a | Ca-a-293 | | 2,6-DFPh | H | H | 5-1Idz | C | | 405 (M⁺+1) |
| Ca-a-295 | 5-e | Int.c-a-4 | BRA51 | 3,5-DFPh | Et | H | 5-1Idz | C | | 433 (M⁺+1) |
| Ca-a-296 | 1-a | Ca-a-295 | | 3,5-DFPh | H | H | 5-1Idz | C | | 405 (M⁺+1) |
| Ca-a-297 | 5-e | Ca-a-295 | BRA52 | 2,4-DClPh | Et | H | 5-1Idz | C | | 466 (M⁺+1) |
| Ca-a-298 | 1-a | Ca-a-297 | | 2,4-DClPh | H | H | 5-1Idz | C | | 438 (M⁺+1) |
| Ca-a-299 | 5-e | Int.c-a-4 | BRA53 | 3,5-DClPh | Et | H | 5-1Idz | C | | 466 (M⁺+1) |
| Ca-a-300 | 1-a | Ca-a-299 | | 3,5-DClPh | H | H | 5-1Idz | C | | 438 (M⁺+1) |
| Ca-a-301 | 5-e | Int.c-a-4 | BRA54 | 3,4-DClPh | Et | H | 5-1Idz | C | | 466 (M⁺+1) |
| Ca-a-302 | 1-a | Ca-a-301 | | 3,4-DClPh | H | H | 5-1Idz | C | | 438 (M⁺+1) |
| Ca-a-303 | 5-e | Int.c-a-4 | BRA55 | 2,3-DMePh | Et | H | 5-1Idz | C | | 425 (M⁺+1) |
| Ca-a-304 | 1-a | Ca-a-303 | | 2,3-DMePh | H | H | 5-1Idz | C | | 397 (M⁺+1) |
| Ca-a-305 | 5-e | Int.c-a-4 | BRA57 | 2,6-DMePh | Et | H | 5-1Idz | C | | 425 (M⁺+1) |
| Ca-a-306 | 1-a | Ca-a-305 | | 2,6-DMePh | H | H | 5-1Idz | C | | 397 (M⁺+1) |
| Ca-a-307 | 5-e | Int.c-a-4 | BRA58 | 2-Furan | Et | H | 5-1Idz | C | | 387 (M⁺+1) |
| Ca-a-308 | 1-a | Ca-a-307 | | 2-Furan | H | H | 5-1Idz | C | | 359 (M⁺+1) |
| Ca-a-309 | 5-e | Int.c-a-4 | BRA59 | 3-Furan | Et | H | 5-1Idz | C | | 387 (M⁺+1) |
| Ca-a-310 | 1-a | Ca-a-309 | | 3-Furan | H | H | 5-1Idz | C | | 359 (M⁺+1) |
| Ca-a-311 | 5-e | Int.c-a-4 | BRA60 | 2-Thiophene | Et | H | 5-1Idz | C | | 403 (M⁺+1) |
| Ca-a-312 | 1-a | Ca-a-311 | | 2-Thiophene | H | H | 5-1Idz | C | | 375 (M⁺+1) |
| Ca-a-313 | 5-e | Int.c-a-4 | BRA61 | 3-Thiophene | Et | H | 5-1Idz | C | | 403 (M⁺+1) |
| Ca-a-314 | 1-a | Ca-a-313 | | 3-Thiophene | H | H | 5-1Idz | C | | 375 (M⁺+1) |
| Ca-a-315 | 5-e | Int.c-a-4 | BRA62 | 3-Pyridine | Et | H | 5-1Idz | C | | 398 (M⁺+1) |
| Ca-a-316 | 1-a | Ca-a-315 | | 3-Pyridine | H | H | 5-1Idz | C | | 370 (M⁺+1) |

**[Table 95]**

| Table-Ca-A-8 | | | | | | | | | | |
|---|---|---|---|---|---|---|---|---|---|---|
| Exp. | Syn. | SM1 | Reagent | Rx | Y | V₁' | V₂' | LCMS | | |
| | | | | | | | | method | RTime | Mass |
| Ca-a-317 | 5-e | Int.c-a-4 | BRA63 | 4-Pyridine | Et | H | 5-1Idz | C | | 398 (M⁺+1) |
| Ca-a-318 | 1-a | Ca-a-317 | | 4-Pyridine | H | H | 5-1Idz | C | | 370 (M⁺+1) |
| Ca-a-319 | 5-e | Int.c-a-4 | BRA65 | 4-PhPh | Et | H | 5-1Idz | C | | 473 (M⁺+1) |
| Ca-a-320 | 1-a | Ca-a-319 | | 4-PhPh | H | H | 5-1Idz | C | | 445 (M⁺+1) |
| Ca-a-321 | 5-e | Int.c-a-4 | BRA66 | C₄H₉CH=CH- | Et | H | 5-1Idz | C | | 403 (M⁺+1) |
| Ca-a-322 | 1-a | Ca-a-321 | | C₄H₉CH=CH- | H | H | 5-1Idz | C | | 375 (M⁺+1) |
| Ca-a-323 | 9-j | Ca-a-321 | | nHex- | Et | H | 5-1Idz | C | | 405 (M⁺+1) |
| Ca-a-324 | 1-a | Ca-a-323 | | nHex- | H | H | 5-1Idz | C | | 377 (M⁺+1) |
| Ca-a-325 | 5-e | Int.c-a-4 | BRA67 | 4FPhCH=CH- | Et | H | 5-1Idz | C | | 441 (M⁺+1) |
| Ca-a-326 | 1-a | Ca-a-325 | | 4FPhCH=CH- | H | H | 5-1Idz | C | | 413 (M⁺+1) |
| Ca-a-327 | 9-j | Ca-a-325 | | 4FPhC₂H₄- | Et | H | 5-1Idz | C | | 443 (M⁺+1) |
| Ca-a-328 | 1-a | Ca-a-327 | | 4FPhC₂H₄- | H | H | 5-1Idz | C | | 415 (M⁺+1) |
| Ca-a-329 | 5-e | Int.c-a-4 | BRA68 | 4ClPhCH=CH- | Et | H | 5-1Idz | C | | 457 (M⁺+1) |
| Ca-a-330 | 1-a | Ca-a-329 | | 4ClPhCH=CH- | H | H | 5-1Idz | C | | 429 (M⁺+1) |
| Ca-a-331 | 5-e | Int.c-a-4 | BRA69 | 2cHexCH=CH- | Et | H | 5-1Idz | C | | 429 (M⁺+1) |
| Ca-a-332 | 1-a | Ca-a-331 | | 2cHexCH=CH- | H | H | 5-1Idz | C | | 401 (M⁺+1) |
| Ca-a-333 | 9-j | Ca-a-331 | | 2cHexC₂H₄- | Et | H | 5-1Idz | C | | 431 (M⁺+1) |
| Ca-a-334 | 1-a | Ca-a-333 | | 2cHexC₂H₄- | H | H | 5-1Idz | C | | 403 (M⁺+1) |
| Ca-a-335 | 5-e | Int.c-a-4 | BRA71 | 4MePhCH=CH- | Et | H | 5-1Idz | C | | 437 (M⁺+1) |
| Ca-a-336 | 1-a | Ca-a-335 | | 4MePhCH=CH- | H | H | 5-1Idz | C | | 409 (M⁺+1) |
| Ca-a-337 | 9-j | Ca-a-335 | | 4MePhC₂H₄- | Et | H | 5-1Idz | C | | 439 (M⁺+1) |
| Ca-a-338 | 1-a | Ca-a-337 | | 4MePhC₂H₄- | H | H | 5-1Idz | C | | 411 (M⁺+1) |
| Ca-a-339 | 5-e | Int.c-a-5 | BRA14 | Ph | Et | H | 1Me-5-Idz | C | | 411 (M⁺+1) |
| Ca-a-340 | 1-a | Ca-a-339 | | Ph | H | H | 1Me-5-Idz | C | | 383 (M⁺+1) |
| Ca-a-341 | 5-e | Int.c-a-5 | BRA15 | 2-MePh | Et | H | 1Me-5-Idz | C | | 425 (M⁺+1) |
| Ca-a-342 | 1-a | Ca-a-341 | | 2-MePh | H | H | 1Me-5-Idz | C | | 397 (M⁺+1) |
| Ca-a-343 | 5-e | Int.c-a-5 | BRA16 | 3-MePh | Et | H | 1Me-5-Idz | C | | 425 (M⁺+1) |
| Ca-a-344 | 1-a | Ca-a-343 | | 3-MePh | H | H | 1Me-5-Idz | C | | 397 (M⁺+1) |
| Ca-a-345 | 5-e | Int.c-a-5 | BRA17 | 4-MePh | Et | H | 1Me-5-Idz | C | | 425 (M⁺+1) |
| Ca-a-346 | 1-a | Ca-a-345 | | 4-MePh | H | H | 1Me-5-Idz | C | | 397 (M⁺+1) |
| Ca-a-347 | 5-e | Int.c-a-5 | BRA18 | 2-EtPh | Et | H | 1Me-5-Idz | C | | 439 (M⁺+1) |
| Ca-a-348 | 1-a | Ca-a-347 | | 2-EtPh | H | H | 1Me-5-Idz | C | | 411 (M⁺+1) |
| Ca-a-349 | 5-e | Int.c-a-5 | BRA20 | 2-iPrPh | Et | H | 1Me-5-ldz | C | | 453 (M⁺+1) |
| Ca-a-350 | 1-a | Ca-a-349 | | 2-iPrPh | H | H | 1Me-5-Idz | C | | 425 (M⁺+1) |
| Ca-a-351 | 5-e | Int.c-a-5 | BRA22 | 1-nBuPh | Et | H | 1Me-5-Idz | C | | 467 (M⁺+1) |
| Ca-a-352 | 1-a | Ca-a-351 | | 1-nBuPh | H | H | 1Me-5-Idz | C | | 439 (M⁺+1) |
| Ca-a-353 | 5-e | Int.c-a-5 | BRA23 | 4-tBuPh | Et | H | 1Me-5-Idz | C | | 467 (M⁺+1) |
| Ca-a-354 | 1-a | Ca-a-353 | | 4-tBuPh | H | H | 1Me-5-Idz | C | | 439 (M⁺+1) |
| Ca-a-355 | 5-e | Int.c-a-5 | BRA24 | 2-MeOPh | Et | H | 1Me-5-Idz | C | | 441 (M⁺+1) |
| Ca-a-356 | 1-a | Ca-a-355 | | 2-MeOPh | H | H | 1Me-5-Idz | C | | 413 (M⁺+1) |
| Ca-a-357 | 5-e | Int.c-a-5 | BRA26 | 4-MeOPh | Et | H | 1Me-5-Idz | C | | 441 (M⁺+1) |
| Ca-a-358 | 1-a | Ca-a-357 | | 4-MeOPh | H | H | 1Me-5-Idz | C | | 413 (M⁺+1) |
| Ca-a-359 | 5-e | Int.c-a-5 | BRA27 | 2-EtOPh | Et | H | 1Me-5-Idz | C | | 455 (M⁺+1) |
| Ca-a-360 | 1-a | Ca-a-359 | | 2-EtOPh | H | H | 1Me-5-Idz | C | | 427 (M⁺+1) |
| Ca-a-361 | 5-e | Int.c-a-5 | BRA29 | 3-iPrOPh | Et | H | 1Me-5-Idz | C | | 469 (M⁺+1) |
| Ca-a-362 | 1-a | Ca-a-361 | | 3-iPrOPh | H | H | 1Me-5-Idz | C | | 441 (M⁺+1) |

**[Table 96]**

| Table-Ca-A-9 | | | | | | | | | | |
|---|---|---|---|---|---|---|---|---|---|---|
| Exp. | Syn. | SM1 | Reagent | Rx | Y | V₁' | V₂' | LCMS | | |
| | | | | | | | | method | RTime | Mass |
| Ca-a-363 | 5-e | Int.c-a-5 | BRA30 | 4-iPrOPh | Et | H | 1Me-5-Idz | C | | 469 (M⁺+1) |
| Ca-a-364 | 1-a | Ca-a-363 | | 4-iPrOPh | H | H | 1Me-5-Idz | C | | 441 (M⁺+1) |
| Ca-a-365 | 5-e | Int.c-a-5 | BRA31 | 3-nPrOPh | Et | H | 1Me-5-Idz | C | | 469 (M⁺+1) |
| Ca-a-366 | 1-a | Ca-a-365 | | 3-nPrOPh | H | H | 1Me-5-Idz | C | | 441 (M⁺+1) |
| Ca-a-367 | 5-e | Int.c-a-5 | BRA32 | 4-nPrOPh | Et | H | 1Me-5-Idz | C | | 469 (M⁺+1) |
| Ca-a-368 | 1-a | Ca-a-367 | | 4-nPrOPh | H | H | 1Me-5-Idz | C | | 441 (M⁺+1) |
| Ca-a-369 | 5-e | Int.c-a-5 | BRA34 | 4-nBuOPh | Et | H | 1Me-5-Idz | C | | 483 (M⁺+1) |
| Ca-a-370 | 1-a | Ca-a-369 | | 4-nBuOPh | H | H | 1Me-5-Idz | C | | 455 (M⁺+1) |
| Ca-a-371 | 5-e | Int.c-a-5 | BRA35 | 3-DMAPh | Et | H | 1Me-5-Idz | C | | 454 (M⁺+1) |
| Ca-a-372 | 1-a | Ca-a-371 | | 3-DMAPh | H | H | 1Me-5-Idz | C | | 426 (M⁺+1) |
| Ca-a-373 | 5-e | Int.c-a-5 | BRA36 | 4-DMAPh | Et | H | 1Me-5-Idz | C | | 454 (M⁺+1) |
| Ca-a-374 | 1-a | Ca-a-373 | | 4-DMAPh | H | H | 1Me-5-Idz | C | | 426 (M⁺+1) |
| Ca-a-375 | 5-e | Int.c-a-5 | BRA37 | 2-FPh | Et | H | 1Me-5-Idz | C | | 429 (M⁺+1) |
| Ca-a-376 | 1-a | Ca-a-375 | | 2-FPh | H | H | 1Me-5-Idz | C | | 401 (M⁺+1) |
| Ca-a-377 | 5-e | Int.c-a-5 | BRA38 | 3-FPh | Et | H | 1Me-5-Idz | C | | 429 (M⁺+1) |
| Ca-a-378 | 1-a | Ca-a-377 | | 3-FPh | H | H | 1Me-5-Idz | C | | 401 (M⁺+1) |
| Ca-a-379 | 5-e | Int.c-a-5 | BRA39 | 4-FPh | Et | H | 1Me-5-Idz | C | | 429 (M⁺+1) |
| Ca-a-380 | 1-a | Ca-a-379 | | 4-FPh | H | H | 1Me-5-Idz | C | | 401 (M⁺+1) |
| Ca-a-381 | 5-e | Int.c-a-5 | BRA41 | 3-ClPh | Et | H | 1Me-5-Idz | C | | 445 (M⁺+1) |
| Ca-a-382 | 1-a | Ca-a-381 | | 3-ClPh | H | H | 1Me-5-Idz | C | | 417 (M⁺+1) |
| Ca-a-383 | 5-e | Int.c-a-5 | BRA42 | 4-ClPh | Et | H | 1Me-5-Idz | C | | 445 (M⁺+1) |
| Ca-a-384 | 1-a | Ca-a-383 | | 4-ClPh | H | H | 1Me-5-Idz | C | | 417 (M⁺+1) |
| Ca-a-385 | 5-e | Int.c-a-5 | BRA44 | 3-CF3Ph | Et | H | 1Me-5-Idz | C | | 479 (M⁺+1) |
| Ca-a-386 | 1-a | Ca-a-385 | | 3-CF3Ph | H | H | 1Me-5-Idz | C | | 451 (M⁺+1) |
| Ca-a-387 | 5-e | Int.c-a-5 | BRA46 | 2,3-DFPh | Et | H | 1Me-5-Idz | C | | 447 (M⁺+1) |
| Ca-a-388 | 1-a | Ca-a-387 | | 2,3-DFPh | H | H | 1Me-5-Idz | C | | 419 (M⁺+1) |
| Ca-a-389 | 5-e | Int.c-a-5 | BRA47 | 2,4-DFPh | Et | H | 1Me-5-Idz | C | | 447 (M⁺+1) |
| Ca-a-390 | 1-a | Ca-a-389 | | 2,4-DFPh | H | H | 1Me-5-Idz | C | | 419 (M⁺+1) |
| Ca-a-391 | 5-e | Int.c-a-5 | BRA49 | 2,6-DFPh | Et | H | 1Me-5-Idz | C | | 447 (M⁺+1) |
| Ca-a-392 | 1-a | Ca-a-391 | | 2,6-DFPh | H | H | 1Me-5-Idz | C | | 419 (M⁺+1) |
| Ca-a-393 | 5-e | Int.c-a-5 | BRA52 | 2,4-DClPh | Et | H | 1Me-5-Idz | C | | 480 (M⁺+1) |
| Ca-a-394 | 1-a | Ca-a-393 | | 2,4-DClPh | H | H | 1Me-5-Idz | C | | 452 (M⁺+1) |
| Ca-a-395 | 5-e | Int.c-a-5 | BRA53 | 3,5-DClPh | Et | H | 1Me-5-Idz | C | | 480 (M⁺+1) |
| Ca-a-396 | 1-a | Ca-a-395 | | 3,5-DClPh | H | H | 1Me-5-Idz | C | | 452 (M⁺+1) |
| Ca-a-397 | 5-e | Int.c-a-5 | BRA55 | 2,3-DMePh | Et | H | 1Me-5-Idz | C | | 439 (M⁺+1) |
| Ca-a-398 | 1-a | Ca-a-397 | | 2,3-DMePh | H | H | 1Me-5-Idz | C | | 411 (M⁺+1) |
| Ca-a-399 | 5-e | Int.c-a-5 | BRA56 | 2,4-DMePh | Et | H | 1Me-5-Idz | C | | 439 (M⁺+1) |
| Ca-a-400 | 1-a | Ca-a-399 | | 2,4-DMePh | H | H | 1Me-5-Idz | C | | 411 (M⁺+1) |
| Ca-a-401 | 5-e | Int.c-a-5 | BRA58 | 2-Furan | Et | H | 1Me-5-Idz | C | | 401 (M⁺+1) |
| Ca-a-402 | 1-a | Ca-a-401 | | 2-Furan | H | H | 1Me-5-Idz | C | | 373 (M⁺+1) |
| Ca-a-403 | 5-e | Int.c-a-5 | BRA59 | 3-Furan | Et | H | 1Me-5-Idz | C | | 401 (M⁺+1) |
| Ca-a-404 | 1-a | Ca-a-403 | | 3-Furan | H | H | 1Me-5-Idz | C | | 373 (M⁺+1) |
| Ca-a-405 | 5-e | Int.c-a-5 | BRA60 | 2-Thiophene | Et | H | 1Me-5-Idz | C | | 417 (M⁺+1) |
| Ca-a-406 | 1-a | Ca-a-405 | | 2-Thiophene | H | H | 1Me-5-Idz | C | | 389 (M⁺+1) |
| Ca-a-407 | 5-e | Int.c-a-5 | BRA61 | 3-Thiophene | Et | H | 1Me-5-Idz | C | | 417 (M⁺+1) |
| Ca-a-408 | 1-a | Ca-a-407 | | 3-Thiophene | H | H | 1Me-5-Idz | C | | 389 (M⁺+1) |

**[Table 97]**

| Table-Ca-A-10 | | | | | | | | | | |
|---|---|---|---|---|---|---|---|---|---|---|
| Exp. | Syn. | SM1 | Reagent | Rx | Y | V₁' | V₂' | LCMS | | |
| | | | | | | | | method | RTime | Mass |
| Ca-a-409 | 5-e | Int.c-a-5 | BRA62 | 3-Pyridine | Et | H | 1Me-5-Idz | C | | 412 (M⁺+1) |
| Ca-a-410 | 1-a | Ca-a-409 | | 3-Pyridine | H | H | 1Me-5-Idz | C | | 384 (M⁺+1) |
| Ca-a-411 | 5-e | Int.c-a-5 | BRA63 | 4-Pyridine | Et | H | 1Me-5-Idz | C | | 412 (M⁺+1) |
| Ca-a-412 | 1-a | Ca-a-411 | | 4-Pyridine | H | H | 1Me-5-Idz | C | | 384 (M⁺+1) |
| Ca-a-413 | 5-e | Int.c-a-5 | BRA65 | 4-PhPh | Et | H | 1Me-5-Idz | C | | 487 (M⁺+1) |
| Ca-a-414 | 1-a | Ca-a-413 | | 4-PhPh | H | H | 1Me-5-Idz | C | | 459 (M⁺+1) |
| Ca-a-415 | 5-e | Int.c-a-5 | BRA66 | C₄H₉CH=CH- | Et | H | 1Me-5-Idz | C | | 417 (M⁺+1) |
| Ca-a-416 | 1-a | Ca-a-415 | | C₄H₉CH=CH- | H | H | 1Me-5-Idz | C | | 389 (M⁺+1) |
| Ca-a-417 | 9-j | Ca-a-415 | | nHex- | Et | H | 1Me-5-Idz | C | | 419 (M⁺+1) |
| Ca-a-418 | 1-a | Ca-a-417 | | nHex- | H | H | 1Me-5-Idz | C | | 391 (M⁺+1) |
| Ca-a-419 | 5-e | Int.c-a-5 | BRA67 | 4FPhCH=CH- | Et | H | 1Me-5-Idz | C | | 455 (M⁺+1) |
| Ca-a-420 | 1-a | Ca-a-419 | | 4FPhCH=CH- | H | H | 1Me-5-Idz | C | | 427 (M⁺+1) |
| Ca-a-421 | 9-j | Ca-a-419 | | 4FPhC₂H₄- | Et | H | 1Me-5-Idz | C | | 457 (M⁺+1) |
| Ca-a-422 | 1-a | Ca-a-421 | | 4FPhC₂H₄- | H | H | 1Me-5-Idz | C | | 429 (M⁺+1) |
| Ca-a-423 | 5-e | Int.c-a-5 | BRA69 | 2cHexCH=CH- | Et | H | 1Me-5-Idz | C | | 443 (M⁺+1) |
| Ca-a-424 | 1-a | Ca-a-423 | | 2cHexCH=CH- | H | H | 1Me-5-Idz | C | | 415 (M⁺+1) |
| Ca-a-425 | 9-j | Ca-a-423 | | 2cHexC₂H₄- | Et | H | 1Me-5-Idz | C | | 445 (M⁺+1) |
| Ca-a-426 | 1-a | Ca-a-425 | | 2cHexC₂H₄- | H | H | 1Me-5-Idz | C | | 417 (M⁺+1) |
| Ca-a-427 | 5-e | Int.c-a-5 | BRA70 | 4CF3PhCH=CH- | Et | H | 1Me-5-Idz | C | | 505 (M⁺+1) |
| Ca-a-428 | 1-a | Ca-a-427 | | 4CF3PhCH=CH- | H | H | 1Me-5-Idz | C | | 477 (M⁺+1) |
| Ca-a-429 | 9-j | Ca-a-427 | | 4CF3PhC₂H₄- | Et | H | 1Me-5-Idz | C | | 507 (M⁺+1) |
| Ca-a-430 | 1-a | Ca-a-429 | | 4CF3PhC₂H₄- | H | H | 1Me-5-Idz | C | | 479 (M⁺+1) |
| Ca-a-431 | 5-e | Int.c-a-5 | BRA73 | 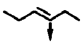 | Et | H | 1Me-5-Idz | C | | 417 (M⁺+1) |
| Ca-a-432 | 1-a | Ca-a-431 | | 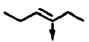 | H | H | 1Me-5-Idz | C | | 389 (M⁺+1) |
| Ca-a-433 | 9-j | Ca-a-431 | | 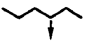 | Et | H | 1Me-5-Idz | C | | 419 (M⁺+1) |
| Ca-a-434 | 1-a | Ca-a-433 | | 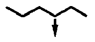 | H | H | 1Me-5-Idz | C | | 391 (M⁺+1) |
| Ca-a-435 | 5-e | Int.c-a-6 | BRA14 | Ph | Et | H | 1Et-5-Idz | C | | 425 (M⁺+1) |
| Ca-a-436 | 1-a | Ca-a-435 | | Ph | H | H | 1Et-5-Idz | C | | 397 (M⁺+1) |
| Ca-a-437 | 5-e | Int.c-a-6 | BRA16 | 3-MePh | Et | H | 1Et-5-Idz | C | | 439 (M⁺+1) |
| Ca-a-438 | 1-a | Ca-a-437 | | 3-MePh | H | H | 1Et-5-Idz | C | | 411 (M⁺+1) |
| Ca-a-439 | 5-e | Int.c-a-6 | BRA18 | 2-EtPh | Et | H | 1Et-5-Idz | C | | 453 (M⁺+1) |
| Ca-a-440 | 1-a | Ca-a-439 | | 2-EtPh | H | H | 1Et-5-Idz | C | | 425 (M⁺+1) |
| Ca-a-441 | 5-e | Int.c-a-6 | BRA20 | 2-iPrPh | Et | H | 1Et-5-Idz | C | | 467 (M⁺+1) |
| Ca-a-442 | 1-a | Ca-a-441 | | 2-iPrPh | H | H | 1Et-5-Idz | C | | 439 (M⁺+1) |
| Ca-a-443 | 5-e | Int.c-a-6 | BRA31 | 3-nPrOPh | Et | H | 1Et-5-Idz | C | | 483 (M⁺+1) |
| Ca-a-444 | 1-a | Ca-a-443 | | 3-nPrOPh | H | H | 1Et-5-Idz | C | | 455 (M⁺+1) |
| Ca-a-445 | 5-e | Int.c-a-6 | BRA35 | 3-DMAPh | Et | H | 1Et-5-Idz | C | | 468 (M⁺+1) |
| Ca-a-446 | 1-a | Ca-a-445 | | 3-DMAPh | H | H | 1Et-5-Idz | C | | 440 (M⁺+1) |
| Ca-a-447 | 5-e | Int.c-a-6 | BRA37 | 2-FPh | Et | H | 1Et-5-Idz | C | | 443 (M⁺+1) |
| Ca-a-448 | 1-a | Ca-a-447 | | 2-FPh | H | H | 1Et-5-Idz | C | | 415 (M⁺+1) |
| Ca-a-449 | 5-e | Int.c-a-6 | BRA38 | 3-FPh | Et | H | 1Et-5-Idz | C | | 443 (M⁺+1) |
| Ca-a-450 | 1-a | Ca-a-449 | | 3-FPh | H | H | 1Et-5-Idz | C | | 415 (M⁺+1) |
| Ca-a-451 | 5-e | Int.c-a-6 | BRA40 | 2-ClPh | Et | H | 1Et-5-Idz | C | | 460 (M⁺+1) |
| Ca-a-452 | 1-a | Ca-a-451 | | 2-ClPh | H | H | 1Et-5-Idz | C | | 431 (M⁺+1) |
| Ca-a-453 | 5-e | Int.c-a-6 | BRA41 | 3-ClPh | Et | H | 1Et-5-Idz | C | | 460 (M⁺+1) |
| Ca-a-454 | 1-a | Ca-a-453 | | 3-ClPh | H | H | 1Et-5-Idz | C | | 431 (M⁺+1) |

**[Table 98]**

| Table-Ca-A-11 | | | | | | | | | | |
|---|---|---|---|---|---|---|---|---|---|---|
| Exp. | Syn. | SM1 | Reagent | Rx | Y | V₁' | V₂' | LCMS | | |
| | | | | | | | | method | RTime | Mass |
| Ca-a-455 | 5-e | Int.c-a-6 | BRA43 | 2-CF3Ph | Et | H | 1Et-5-Idz | C | | 493 (M⁺+1) |
| Ca-a-456 | 1-a | Ca-a-455 | | 2-CF3Ph | H | H | 1Et-5-Idz | C | | 465 (M⁺+1) |
| Ca-a-457 | 5-e | Int.c-a-6 | BRA47 | 2,4-DFPh | Et | H | 1Et-5-Idz | C | | 461 (M⁺+1) |
| Ca-a-458 | 1-a | Ca-a-457 | | 2,4-DFPh | H | H | 1Et-5-Idz | C | | 433 (M⁺+1) |
| Ca-a-459 | 5-e | Int.c-a-6 | BRA48 | 2.5-DFPh | Et | H | 1Et-5-Idz | C | | 461 (M⁺+1) |
| Ca-a-460 | 1-a | Ca-a-459 | | 2,5-DFPh | H | H | 1Et-5-Idz | C | | 433 (M⁺+1) |
| Ca-a-461 | 5-e | Int.c-a-6 | BRA50 | 3,4-DFPh | Et | H | 1Et-5-Idz | C | | 461 (M⁺+1) |
| Ca-a-462 | 1-a | Ca-a-461 | | 3,4-DFPh | H | H | 1Et-5-Idz | C | | 433 (M⁺+1) |
| Ca-a-463 | 5-e | Int.c-a-6 | BRA52 | 2,4-DClPh | Et | H | 1Et-5-Idz | C | | 494 (M⁺+1) |
| Ca-a-464 | 1-a | Ca-a-463 | | 2,4-DClPh | H | H | 1Et-5-Idz | C | | 466 (M⁺+1) |
| Ca-a-465 | 5-e | Int.c-a-6 | BRA54 | 3,4-DClPh | Et | H | 1Et-5-Idz | C | | 494 (M⁺+1) |
| Ca-a-466 | 1-a | Ca-a-465 | | 3,4-DClPh | H | H | 1Et-5-Idz | C | | 466 (M⁺+1) |
| Ca-a-467 | 5-e | Int.c-a-6 | BRA55 | 2,3-DMePh | Et | H | 1Et-5-Idz | C | | 453 (M⁺+1) |
| Ca-a-468 | 1-a | Ca-a-467 | | 2,3-DMePh | H | H | 1Et-5-Idz | C | | 425 (M⁺+1) |
| Ca-a-469 | 5-e | Int.c-a-6 | BRA57 | 2,6-DMePh | Et | H | 1Et-5-Idz | C | | 453 (M⁺+1) |
| Ca-a-470 | 1-a | Ca-a-469 | | 2,6-DMePh | H | H | 1Et-5-Idz | C | | 425 (M⁺+1) |
| Ca-a-471 | 5-e | Int.c-a-6 | BRA58 | 2-Furan | Et | H | 1Et-5-Idz | C | | 415 (M⁺+1) |
| Ca-a-472 | 1-a | Ca-a-471 | | 2-Furan | H | H | 1Et-5-Idz | C | | 387 (M⁺+1) |
| Ca-a-473 | 5-e | Int.c-a-6 | BRA60 | 2-Thiophene | Et | H | 1Et-5-Idz | C | | 431 (M⁺+1) |
| Ca-a-474 | 1-a | Ca-a-473 | | 2-Thiophene | H | H | 1Et-5-Idz | C | | 403 (M⁺+1) |
| Ca-a-475 | 5-e | Int.c-a-6 | BRA63 | 4-Pyridine | Et | H | 1Et-5-Idz | C | | 426 (M⁺+1) |
| Ca-a-476 | 1-a | Ca-a-475 | | 4-Pyridine | H | H | 1Et-5-Idz | C | | 398 (M⁺+1) |
| Ca-a-477 | 5-e | Int.c-a-6 | BRA65 | 4-PhPh | Et | H | 1Et-5-Idz | C | | 501 (M⁺+1) |
| Ca-a-478 | 1-a | Ca-a-477 | | 4-PhPh | H | H | 1Et-5-Idz | C | | 473 (M⁺+1) |
| Ca-a-479 | 5-e | Int.c-a-6 | BRA67 | 4FPhCH=CH- | Et | H | 1Et-5-Idz | C | | 469 (M⁺+1) |
| Ca-a-480 | 1-a | Ca-a-479 | | 4FPhCH=CH- | H | H | 1Et-5-Idz | C | | 441 (M⁺+1) |
| Ca-a-481 | 9-j | Ca-a-479 | | 4FPhC₂H₄- | Et | H | 1Et-5-Idz | C | | 471 (M⁺+1) |
| Ca-a-482 | 1-a | Ca-a-481 | | 4FPhC₂H₄- | H | H | 1Et-5-Idz | C | | 443 (M⁺+1) |
| Ca-a-483 | 5-e | Int.c-a-6 | BRA69 | 2cHexCH=CH- | Et | H | 1Et-5-Idz | C | | 457 (M⁺+1) |
| Ca-a-484 | 1-a | Ca-a-483 | | 2cHexCH=CH- | H | H | 1Et-5-Idz | C | | 429 (M⁺+1) |
| Ca-a-485 | 9-j | Ca-a-483 | | 2cHexC₂H₄- | Et | H | 1Et-5-Idz | C | | 459 (M⁺+1) |
| Ca-a-486 | 1-a | Ca-a-485 | | 2cHexC₂H₄- | H | H | 1Et-5-Idz | C | | 431 (M⁺+1) |
| Ca-a-487 | 5-e | Int.c-a-6 | BRA71 | 4MePhCH=CH- | Et | H | 1Et-5-Idz | C | | 465 (M⁺+1) |
| Ca-a-488 | 1-a | Ca-a-487 | | 4MePhCH=CH- | H | H | 1Et-5-Idz | C | | 437 (M⁺+1) |
| Ca-a-489 | 9-j | Ca-a-487 | | 4MePhC₂H₄- | Et | H | 1Et-5-Idz | C | | 467 (M⁺+1) |
| Ca-a-490 | 1-a | Ca-a-489 | | 4MePhC₂H₄- | H | H | 1Et-5-Idz | C | | 439 (M⁺+1) |
| Ca-a-491 | 5-e | Int.c-a-7 | BRA14 | Ph | Et | H | 5-BF | C | | 397 (M⁺+1) |
| Ca-a-492 | 1-a | Ca-a-491 | | Ph | H | H | 5-BF | C | | 369 (M⁺+1) |
| Ca-a-493 | 5-e | Int.c-a-7 | BRA16 | 3-MePh | Et | H | 5-BF | C | | 411 (M⁺+1) |
| Ca-a-494 | 1-a | Ca-a-493 | | 3-MePh | H | H | 5-BF | C | | 383 (M⁺+1) |
| Ca-a-495 | 5-e | Int.c-a-7 | BRA17 | 4-MePh | Et | H | 5-BF | C | | 411 (M⁺+1) |
| Ca-a-496 | 1-a | Ca-a-495 | | 4-MePh | H | H | 5-BF | C | | 383 (M⁺+1) |
| Ca-a-497 | 5-e | Int.c-a-7 | BRA20 | 2-iPrPh | Et | H | 5-BF | C | | 439 (M⁺+1) |
| Ca-a-498 | 1-a | Ca-a-497 | | 2-iPrPh | H | H | 5-BF | C | | 411 (M⁺+1) |
| Ca-a-499 | 5-e | Int.c-a-7 | BRA23 | 4-tBuPh | Et | H | 5-BF | C | | 453 (M⁺+1) |
| Ca-a-500 | 1-a | Ca-a-499 | | 4-tBuPh | H | H | 5-BF | C | | 425 (M⁺+1) |

**[Table 99]**

| Table-Ca-A-12 | | | | | | | | | | |
|---|---|---|---|---|---|---|---|---|---|---|
| Exp. | Syn. | SM1 | Reagent | Rx | Y | V₁' | V₂' | LCMS | | |
| | | | | | | | | method | RTime | Mass |
| Ca-a-501 | 5-e | Int.c-a-7 | BRA25 | 3-MeOPh | Et | H | 5-BF | C | | 427 (M⁺+1) |
| Ca-a-502 | 1-a | Ca-a-501 | | 3-MeOPh | H | H | 5-BF | C | | 399 (M⁺+1) |
| Ca-a-503 | 5-e | Int.c-a-7 | BRA27 | 2-EtOPh | Et | H | 5-BF | C | | 441 (M⁺+1) |
| Ca-a-504 | 1-a | Ca-a-503 | | 2-EtOPh | H | H | 5-BF | C | | 413 (M⁺+1) |
| Ca-a-505 | 5-e | Int.c-a-7 | BRA31 | 3-nPrOPh | Et | H | 5-BF | C | | 455 (M⁺+1) |
| Ca-a-506 | 1-a | Ca-a-505 | | 3-nPrOPh | H | H | 5-BF | C | | 427 (M⁺+1) |
| Ca-a-507 | 5-e | Int.c-a-7 | BRA33 | 3-nBuOPh | Et | H | 5-BF | C | | 469 (M⁺+1) |
| Ca-a-508 | 1-a | Ca-a-507 | | 3-nBuOPh | H | H | 5-BF | C | | 441 (M⁺+1) |
| Ca-a-509 | 5-e | Int.c-a-7 | BRA35 | 3-DMAPh | Et | H | 5-BF | C | | 440 (M⁺+1) |
| Ca-a-510 | 1-a | Ca-a-509 | | 3-DMAPh | H | H | 5-BF | C | | 412 (M⁺+1) |
| Ca-a-511 | 5-e | Int.c-a-7 | BRA37 | 2-FPh | Et | H | 5-BF | C | | 415 (M⁺+1) |
| Ca-a-512 | 1-a | Ca-a-511 | | 2-FPh | H | H | 5-BF | C | | 387 (M⁺+1) |
| Ca-a-513 | 5-e | Int.c-a-7 | BRA39 | 4-FPh | Et | H | 5-BF | C | | 415 (M⁺+1) |
| Ca-a-514 | 1-a | Ca-a-513 | | 4-FPh | H | H | 5-BF | C | | 387 (M⁺+1) |
| Ca-a-515 | 5-e | Int.c-a-7 | BRA41 | 3-ClPh | Et | H | 5-BF | C | | 431 (M⁺+1) |
| Ca-a-516 | 1-a | Ca-a-515 | | 3-ClPh | H | H | 5-BF | C | | 403 (M⁺+1) |
| Ca-a-517 | 5-e | Int.c-a-7 | BRA43 | 2-CF3Ph | Et | H | 5-BF | C | | 465 (M⁺+1) |
| Ca-a-518 | 1-a | Ca-a-517 | | 2-CF3Ph | H | H | 5-BF | C | | 437 (M⁺+1) |
| Ca-a-519 | 5-e | Int.c-a-7 | BRA48 | 2.5-DFPh | Et | H | 5-BF | C | | 433 (M⁺+1) |
| Ca-a-520 | 1-a | Ca-a-519 | | 2,5-DFPh | H | H | 5-BF | C | | 405 (M⁺+1) |
| Ca-a-521 | 5-e | Int.c-a-7 | BRA49 | 2,6-DFPh | Et | H | 5-BF | C | | 433 (M⁺+1) |
| Ca-a-522 | 1-a | Ca-a-521 | | 2,6-DFPh | H | H | 5-BF | C | | 405 (M⁺+1) |
| Ca-a-523 | 5-e | Int.c-a-7 | BRA50 | 3,4-DFPh | Et | H | 5-BF | C | | 433 (M⁺+1) |
| Ca-a-524 | 1-a | Ca-a-523 | | 3,4-DFPh | H | H | 5-BF | C | | 405 (M⁺+1) |
| Ca-a-525 | 5-e | Int.c-a-7 | BRA53 | 3,5-DClPh | Et | H | 5-BF | C | | 466 (M⁺+1) |
| Ca-a-526 | 1-a | Ca-a-525 | | 3,5-DClPh | H | H | 5-BF | C | | 438 (M⁺+1) |
| Ca-a-527 | 5-e | Int.c-a-7 | BRA55 | 2,3-DMePh | Et | H | 5-BF | C | | 425 (M⁺+1) |
| Ca-a-528 | 1-a | Ca-a-527 | | 2,3-DMePh | H | H | 5-BF | C | | 397 (M⁺+1) |
| Ca-a-529 | 5-e | Int.c-a-7 | BRA57 | 2,6-DMePh | Et | H | 5-BF | C | | 425 (M⁺+1) |
| Ca-a-530 | 1-a | Ca-a-529 | | 2,6-DMePh | H | H | 5-BF | C | | 397 (M⁺+1) |
| Ca-a-531 | 5-e | Int.c-a-7 | BRA58 | 2-Furan | Et | H | 5-BF | C | | 387 (M⁺+1) |
| Ca-a-532 | 1-a | Ca-a-531 | | 2-Furan | H | H | 5-BF | C | | 359 (M⁺+1) |
| Ca-a-533 | 5-e | Int.c-a-7 | BRA61 | 3-Thiophene | Et | H | 5-BF | C | | 403 (M⁺+1) |
| Ca-a-534 | 1-a | Ca-a-533 | | 3-Thiophene | H | H | 5-BF | C | | 375 (M⁺+1) |
| Ca-a-535 | 5-e | Int.c-a-7 | BRA64 | 3-PhPh | Et | H | 5-BF | C | | 473 (M⁺+1) |
| Ca-a-536 | 1-a | Ca-a-535 | | 3-PhPh | H | H | 5-BF | C | | 445 (M⁺+1) |
| Ca-a-537 | 5-e | Int.c-a-8 | BRA14 | Ph | Et | H | 6-Qu | C | | 408 (M⁺+1) |
| Ca-a-538 | 1-a | Ca-a-537 | | Ph | H | H | 6-Qu | C | | 380 (M⁺+1) |
| Ca-a-539 | 5-e | Int.c-a-8 | BRA15 | 2-MePh | Et | H | 6-Qu | C | | 422 (M⁺+1) |
| Ca-a-540 | 1-a | Ca-a-539 | | 2-MePh | H | H | 6-Qu | C | | 394 (M⁺+1) |
| Ca-a-541 | 5-e | Int.c-a-8 | BRA17 | 4-MePh | Et | H | 6-Qu | C | | 422 (M⁺+1) |
| Ca-a-542 | 1-a | Ca-a-541 | | 4-MePh | H | H | 6-Qu | C | | 394 (M⁺+1) |
| Ca-a-543 | 5-e | Int.c-a-8 | BRA22 | 1-nBuPh | Et | H | 6-Qu | C | | 464 (M⁺+1) |
| Ca-a-544 | 1-a | Ca-a-543 | | 1-nBuPh | H | H | 6-Qu | C | | 436 (M⁺+1) |
| Ca-a-545 | 5-e | Int.c-a-8 | BRA25 | 3-MeOPh | Et | H | 6-Qu | C | | 438 (M⁺+1) |
| Ca-a-546 | 1-a | Ca-a-545 | | 3-MeOPh | H | H | 6-Qu | C | | 410 (M⁺+1) |

**[Table 100]**

| Table-Ca-A-13 | | | | | | | | | | |
|---|---|---|---|---|---|---|---|---|---|---|
| Exp. | Syn. | SM1 | Reagent | Rx | Y | V₁' | V₂' | LCMS | | |
| | | | | | | | | method | RTime | Mass |
| Ca-a-547 | 5-e | Int.c-a-8 | BRA28 | 3-EtOPh | Et | H | 6-Qu | C | | 452 (M⁺+1) |
| Ca-a-548 | 1-a | Ca-a-547 | | 3-EtOPh | H | H | 6-Qu | C | | 424 (M⁺+1) |
| Ca-a-549 | 5-e | Int.c-a-8 | BRA30 | 4-iPrOPh | Et | H | 6-Qu | C | | 466 (M⁺+1) |
| Ca-a-550 | 1-a | Ca-a-549 | | 4-iPrOPh | H | H | 6-Qu | C | | 438 (M⁺+1) |
| Ca-a-551 | 5-e | Int.c-a-8 | BRA36 | 4-DMAPh | Et | H | 6-Qu | C | | 451 (M⁺+1) |
| Ca-a-552 | 1-a | Ca-a-551 | | 4-DMAPh | H | H | 6-Qu | C | | 423 (M⁺+1) |
| Ca-a-553 | 5-e | Int.c-a-8 | BRA38 | 3-FPh | Et | H | 6-Qu | C | | 426 (M⁺+1) |
| Ca-a-554 | 1-a | Ca-a-553 | | 3-FPh | H | H | 6-Qu | C | | 398 (M⁺+1) |
| Ca-a-555 | 5-e | Int.c-a-8 | BRA40 | 2-ClPh | Et | H | 6-Qu | C | | 442 (M⁺+1) |
| Ca-a-556 | 1-a | Ca-a-555 | | 2-ClPh | H | H | 6-Qu | C | | 414 (M⁺+1) |
| Ca-a-557 | 5-e | Int.c-a-8 | BRA45 | 4-CF3Ph | Et | H | 6-Qu | C | | 476 (M⁺+1) |
| Ca-a-558 | 1-a | Ca-a-557 | | 4-CF3Ph | H | H | 6-Qu | C | | 448 (M⁺+1) |
| Ca-a-559 | 5-e | Int.c-a-8 | BRA47 | 2,4-DFPh | Et | H | 6-Qu | C | | 444 (M⁺+1) |
| Ca-a-560 | 1-a | Ca-a-559 | | 2,4-DFPh | H | H | 6-Qu | C | | 416 (M⁺+1) |
| Ca-a-561 | 5-e | Int.c-a-8 | BRA50 | 3,4-DFPh | Et | H | 6-Qu | C | | 444 (M⁺+1) |
| Ca-a-562 | 1-a | Ca-a-561 | | 3,4-DFPh | H | H | 6-Qu | C | | 416 (M⁺+1) |
| Ca-a-563 | 5-e | Int.c-a-8 | BRA51 | 3,5-DFPh | Et | H | 6-Qu | C | | 444 (M⁺+1) |
| Ca-a-564 | 1-a | Ca-a-563 | | 3,5-DFPh | H | H | 6-Qu | C | | 416 (M⁺+1) |
| Ca-a-565 | 5-e | Int.c-a-8 | BRA52 | 2,4-DClPh | Et | H | 6-Qu | C | | 477 (M⁺+1) |
| Ca-a-566 | 1-a | Ca-a-565 | | 2,4-DClPh | H | H | 6-Qu | C | | 449 (M⁺+1) |
| Ca-a-567 | 5-e | Int.c-a-8 | BRA54 | 3,4-DClPh | Et | H | 6-Qu | C | | 477 (M⁺+1) |
| Ca-a-568 | 1-a | Ca-a-567 | | 3,4-DClPh | H | H | 6-Qu | C | | 449 (M⁺+1) |
| Ca-a-569 | 5-e | Int.c-a-8 | BRA56 | 2,4-DMePh | Et | H | 6-Qu | C | | 436 (M⁺+1) |
| Ca-a-570 | 1-a | Ca-a-569 | | 2,4-DMePh | H | H | 6-Qu | C | | 408 (M⁺+1) |
| Ca-a-571 | 5-e | Int.c-a-8 | BRA58 | 2-Furan | Et | H | 6-Qu | C | | 398 (M⁺+1) |
| Ca-a-572 | 1-a | Ca-a-571 | | 2-Furan | H | H | 6-Qu | C | | 370 (M⁺+1) |
| Ca-a-573 | 5-e | Int.c-a-8 | BRA60 | 2-Thiophene | Et | H | 6-Qu | C | | 414 (M⁺+1) |
| Ca-a-574 | 1-a | Ca-a-573 | | 2-Thiophene | H | H | 6-Qu | C | | 386 (M⁺+1) |
| Ca-a-575 | 5-e | Int.c-a-8 | BRA62 | 3-Pyridine | Et | H | 6-Qu | C | | 409 (M⁺+1) |
| Ca-a-576 | 1-a | Ca-a-575 | | 3-Pyridine | H | H | 6-Qu | C | | 381 (M⁺+1) |
| Ca-a-577 | 5-e | Int.c-a-8 | BRA65 | 4-PhPh | Et | H | 6-Qu | C | | 484 (M⁺+1) |
| Ca-a-578 | 1-a | Ca-a-577 | | 4-PhPh | H | H | 6-Qu | C | | 456 (M⁺+1) |
| Ca-a-579 | 5-e | Int.c-a-8 | BRA67 | 4FPhCH=CH- | Et | H | 6-Qu | C | | 452 (M⁺+1) |
| Ca-a-580 | 1-a | Ca-a-579 | | 4FPhCH=CH- | H | H | 6-Qu | C | | 424 (M⁺+1) |
| Ca-a-581 | 9-j | Ca-a-579 | | 4FPhC₂H₄- | Et | H | 6-Qu | C | | 454 (M⁺+1) |
| Ca-a-582 | 1-a | Ca-a-581 | | 4FPhC₂H₄- | H | H | 6-Qu | C | | 426 (M⁺+1) |
| Ca-a-583 | 5-e | Int.c-a-8 | BRA71 | 4MePhCH=CH- | Et | H | 6-Qu | C | | 448 (M⁺+1) |
| Ca-a-584 | 1-a | Ca-a-583 | | 4MePhCH=CH- | H | H | 6-Qu | C | | 420 (M⁺+1) |
| Ca-a-585 | 9-j | Ca-a-583 | | 4MePhC₂H₄- | Et | H | 6-Qu | C | | 450 (M⁺+1) |
| Ca-a-586 | 1-a | Ca-a-585 | | 4MePhC₂H₄- | H | H | 6-Qu | C | | 422 (M⁺+1) |
| Ca-a-587 | 5-e | Int.c-a-8 | BRA73 | 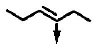 | Et | H | 6-Qu | C | | 414 (M⁺+1) |
| Ca-a-588 | 1-a | Ca-a-587 | | 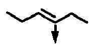 | H | H | 6-Qu | C | | 386 (M⁺+1) |
| Ca-a-589 | 5-e | Int.c-a-1 | BRA74 | Trans-CH3_{C}H=CH- | Et | H | 2-Nap | C | | 371 (M⁺+1) |
| Ca-a-590 | 1-a | Ca-a-589 | | Trans-CH₃CH=CH- | H | H | 2-Nap | C | | 343 (M⁺+1) |

**[Table 101]**

| Table-Ca-A-14 | | | | | | | | | | |
|---|---|---|---|---|---|---|---|---|---|---|
| Exp. | Syn. | SM1 | SM2 | Rx | Y | V₁' | V₂' | LCMS | | |
| | | | | | | | | method | RTime | Mass |
| Ca-a-591 | 9-j | Ca-a-589 | | nPr | Et | H | 2-Nap | C | | 373 (M⁺+1) |
| Ca-a-592 | 1-a | Ca-a-591 | | nPr | H | H | 2-Nap | C | | 345 (M⁺+1) |
| Ca-a-593 | 5-e | Int.c-a-1 | BRA75 | Cis-CH₃CH=CH- | Et | H | 2-Nap | C | | 371 (M⁺+1) |
| Ca-a-594 | 1-a | Ca-a-593 | | Cis-CH₃CH=CH- | H | H | 2-Nap | C | | 343 (M⁺+1) |
| Ca-a-595 | 5-e | Int.c-a-1 | BRA76 | C₃H₇CH=CH- | Et | H | 2-Nap | C | | 399 (M⁺+1) |
| Ca-a-596 | 1-a | Ca-a-595 | | C₃H₇CH=CH- | H | H | 2-Nap | C | | 371 (M⁺+1) |
| Ca-a-597 | 9-j | Ca-a-595 | | nPen | Et | H | 2-Nap | C | | 401 (M⁺+1) |
| Ca-a-598 | 1-a | Ca-a-597 | | nPen | H | H | 2-Nap | C | | 373 (M⁺+1) |
| Ca-a-599 | 5-e | Int.c-a-2 | BRA74 | Trans-CH₃CH=CH- | Et | H | 5-Ind | C | | 360 (M⁺+1) |
| Ca-a-600 | 1-a | Ca-a-599 | | Trans-CH₃CH=CH- | H | H | 5-Ind | C | | 332 (M⁺+1) |
| Ca-a-601 | 9-j | Ca-a-599 | | nPr | Et | H | 5-Ind | C | | 362 (M⁺+1) |
| Ca-a-602 | 1-a | Ca-a-601 | | nPr | H | H | 5-Ind | C | | 334 (M⁺+1) |
| Ca-a-603 | 5-e | Int.c-a-2 | BRA76 | C₃H₇CH=CH- | Et | H | 5-Ind | C | | 388 (M⁺+1) |
| Ca-a-604 | 1-a | Ca-a-603 | | C₃H₇CH=CH- | H | H | 5-Ind | C | | 360 (M⁺+1) |
| Ca-a-605 | 5-e | Int.c-a-3 | BRA75 | Cis-CH₃CH=CH- | Et | H | 1Me-5-Ind | C | | 374 (M⁺+1) |
| Ca-a-606 | 1-a | Ca-a-605 | | Cis-CH₃CH=CH- | H | H | 1Me-5-Ind | C | | 346 (M⁺+1) |
| Ca-a-607 | 9-j | Ca-a-605 | | nPr | Et | H | 1Me-5-Ind | C | | 376 (M⁺+1) |
| Ca-a-608 | 1-a | Ca-a-607 | | nPr | H | H | 1Me-5-Ind | C | | 348 (M⁺+1) |
| Ca-a-609 | 5-e | Int.c-a-3 | BRA76 | C₃H₇CH=CH- | Et | H | 1Me-5-Ind | C | | 402 (M⁺+1) |
| Ca-a-610 | 1-a | Ca-a-609 | | C₃H₇CH=CH- | H | H | 1Me-5-Ind | C | | 374 (M⁺+1) |
| Ca-a-611 | 5-e | Int.c-a-4 | BRA74 | Trans-CH₃CH=CH- | Et | H | 5-1Idz | C | | 361 (M⁺+1) |
| Ca-a-612 | 1-a | Ca-a-611 | | Trans-CH₃CH=CH- | H | H | 5-1Idz | C | | 333 (M⁺+1) |
| Ca-a-613 | 9-j | Ca-a-611 | | nPr | Et | H | 5-1Idz | C | | 363 (M⁺+1) |
| Ca-a-614 | 1-a | Ca-a-613 | | nPr | H | H | 5-1Idz | C | | 335 (M⁺+1) |
| Ca-a-615 | 5-e | Int.c-a-4 | BRA76 | C₃H₇CH=CH- | Et | H | 5-1Idz | C | | 389 (M⁺+1) |
| Ca-a-616 | 1-a | Ca-a-615 | | C₃H₇CH=CH- | H | H | 5-1Idz | C | | 361 (M⁺+1) |
| Ca-a-617 | 9-i | Ca-a-615 | | nPen | Et | H | 5-1Idz | C | | 391 (M⁺+1) |
| Ca-a-618 | 1-a | Ca-a-617 | | nPen | H | H | 5-1Idz | C | | 363 (M⁺+1) |
| Ca-a-619 | 5-e | Int.c-a-5 | BRA74 | Trans-CH₃CH=CH- | Et | H | 1Me-5-Idz | C | | 375 (M⁺+1) |
| Ca-a-620 | 1-a | Ca-a-619 | | Trans-CH₃CH=CH- | H | H | 1Me-5-Idz | C | | 347 (M⁺+1) |
| Ca-a-621 | 9-j | Ca-a-619 | | nPr | Et | H | 1Me-5-Idz | C | | 377 (M⁺+1) |
| Ca-a-622 | 1-a | Ca-a-621 | | nPr | H | H | 1Me-5-Idz | C | | 349 (M⁺+1) |
| Ca-a-623 | 5-e | Int.c-a-5 | BRA75 | Cis-CH₃CH=CH- | Et | H | 1Me-5-Idz | C | | 375 (M⁺+1) |
| Ca-a-624 | 1-a | Ca-a-623 | | Cis-CH₃CH=CH- | H | H | 1Me-5-Idz | C | | 347 (M⁺+1) |
| Ca-a-625 | 5-e | Int.c-a-6 | BRA74 | Trans-CH₃CH=CH- | Et | H | 1Et-5-Idz | C | | 389 (M⁺+1) |
| Ca-a-626 | 1-a | Ca-a-625 | | Trans-CH₃CH=CH- | H | H | 1Et-5-Idz | C | | 361 (M⁺+1) |
| Ca-a-627 | 9-j | Ca-a-625 | | nPr | Et | H | 1Et-5-Idz | C | | 391 (M⁺+1) |
| Ca-a-628 | 1-a | Ca-a-627 | | nPr | H | H | 1Et-5-Idz | C | | 363 (M⁺+1) |
| Ca-a-629 | 5-e | Int.c-a-7 | BRA74 | Trans-CH₃CH=CH- | Et | H | 5-BF | C | | 361 (M⁺+1) |
| Ca-a-630 | 1-a | Ca-a-629 | | Trans-CH₃CH=CH- | H | H | 5-BF | C | | 333 (M⁺+1) |
| Ca-a-631 | 5-e | Int.c-a-7 | BRA76 | C₃H₇CH=CH- | Et | H | 5-BF | C | | 389 (M⁺+1) |
| Ca-a-632 | 1-a | Ca-a-631 | | C₃H₇CH=CH- | H | H | 5-BF | C | | 361 (M⁺+1) |
| Ca-a-633 | 5-e | Int.c-a-8 | BRA74 | Trans-CH₃CH=CH- | Et | H | 6-Qu | C | | 372 (M⁺+1) |
| Ca-a-634 | 1-a | Ca-a-633 | | Trans-CH₃CH=CH- | H | H | 6-Qu | C | | 344 (M⁺+1) |
| Ca-a-635 | 5-e | Int.c-a-8 | BRA76 | C₃H₇CH=CH- | Et | H | 6-Qu | C | | 400 (M⁺+1) |
| Ca-a-636 | 1-a | Ca-a-635 | | C₃H₇CH=CH- | H | H | 6-Qu | C | | 372 (M⁺+1) |

**[Table 102]**

| Table-Ca-B-1 | | | | | | | | | | |
|---|---|---|---|---|---|---|---|---|---|---|
| 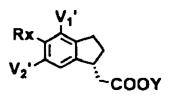 | | | | | | | | | | |

| Exp. | Syn. | SM1 | Reagent | Rx | Y | V₁' | V₂' | LCMS | | |
|---|---|---|---|---|---|---|---|---|---|---|
| | | | | | | | | method | RTime | Mass |
| Ca-b-1 | 5-e | Int.c-b-1 | BRA14 | Ph | Et | H | 2-Nap | C | | 407 (M⁺+1) |
| Ca-b-2 | 1-a | Ca-b-1 | | Ph | H | H | 2-Nap | C | | 379 (M⁺+1) |
| Ca-b-3 | 5-e | Int.c-b-1 | BRA15 | 2-MePh | Et | H | 2-Nap | C | | 421 (M⁺+1) |
| Ca-b-4 | 1-a | Ca-b-3 | | 2-MePh | H | H | 2-Nap | C | | 393 (M⁺+1) |
| Ca-b-5 | 5-e | Int.c-b-1 | BRA17 | 4-MePh | Et | H | 2-Nap | C | | 421 (M⁺+1) |
| Ca-b-6 | 1-a | Ca-b-5 | | 4-MePh | H | H | 2-Nap | C | | 393 (M⁺+1) |
| Ca-b-7 | 5-e | Int.c-b-1 | BRA19 | 4-EtPh | Et | H | 2-Nap | C | | 435 (M⁺+1) |
| Ca-b-8 | 1-a | Ca-b-7 | | 4-EtPh | H | H | 2-Nap | C | | 407 (M⁺+1) |
| Ca-b-9 | 5-e | Int.c-b-1 | BRA20 | 2-iPrPh | Et | H | 2-Nap | C | | 449 (M⁺+1) |
| Ca-b-10 | 1-a | Ca-b-9 | | 2-iPrPh | H | H | 2-Nap | C | | 421 (M⁺+1) |
| Ca-b-11 | 5-e | Int.c-b-1 | BRA22 | 1-nBuPh | Et | H | 2-Nap | C | | 463 (M⁺+1) |
| Ca-b-12 | 1-a | Ca-b-11 | | 1-nBuPh | H | H | 2-Nap | C | | 435 (M⁺+1) |
| Ca-b-13 | 5-e | Int.c-b-1 | BRA23 | 4-tBuPh | Et | H | 2-Nap | C | | 463 (M⁺+1) |
| Ca-b-14 | 1-a | Ca-b-13 | | 4-tBuPh | H | H | 2-Nap | C | | 435 (M⁺+1) |
| Ca-b-15 | 5-e | Int.c-b-1 | BRA24 | 2-MeOPh | Et | H | 2-Nap | C | | 437 (M⁺+1) |
| Ca-b-16 | 1-a | Ca-b-15 | | 2-MeOPh | H | H | 2-Nap | C | | 409 (M⁺+1) |
| Ca-b-17 | 5-e | Int.c-b-1 | BRA26 | 4-MeOPh | Et | H | 2-Nap | C | | 437 (M⁺+1) |
| Ca-b-18 | 1-a | Ca-b-17 | | 4-MeOPh | H | H | 2-Nap | C | | 409 (M⁺+1) |
| Ca-b-19 | 5-e | Int.c-b-1 | BRA28 | 3-EtOPh | Et | H | 2-Nap | C | | 451 (M⁺+1) |
| Ca-b-20 | 1-a | Ca-b-19 | | 3-EtOPh | H | H | 2-Nap | C | | 423 (M⁺+1) |
| Ca-b-21 | 5-e | Int.c-b-1 | BRA29 | 3-iPrOPh | Et | H | 2-Nap | C | | 465 (M⁺+1) |
| Ca-b-22 | 1-a | Ca-b-21 | | 3-iPrOPh | H | H | 2-Nap | C | | 437 (M⁺+1) |
| Ca-b-23 | 5-e | Int.c-b-1 | BRA31 | 3-nPrOPh | Et | H | 2-Nap | C | | 465 (M⁺+1) |
| Ca-b-24 | 1-a | Ca-b-23 | | 3-nPrOPh | H | H | 2-Nap | C | | 437 (M⁺+1) |
| Ca-b-25 | 5-e | Int.c-b-1 | BRA34 | 4-nBuOPh | Et | H | 2-Nap | C | | 479 (M⁺+1) |
| Ca-b-26 | 1-a | Ca-b-25 | | 4-nBuOPh | H | H | 2-Nap | C | | 451 (M⁺+1) |
| Ca-b-27 | 5-e | Int.c-b-1 | BRA35 | 3-DMAPh | Et | H | 2-Nap | C | | 450 (M⁺+1) |
| Ca-b-28 | 1-a | Ca-b-27 | | 3-DMAPh | H | H | 2-Nap | C | | 422 (M⁺+1) |
| Ca-b-29 | 5-e | Int.c-b-1 | BRA37 | 2-FPh | Et | H | 2-Nap | C | | 425 (M⁺+1) |
| Ca-b-30 | 1-a | Ca-b-29 | | 2-FPh | H | H | 2-Nap | C | | 397 (M⁺+1) |
| Ca-b-31 | 5-e | Int.c-b-1 | BRA39 | 4-FPh | Et | H | 2-Nap | C | | 425 (M⁺+1) |
| Ca-b-32 | 1-a | Ca-b-31 | | 4-FPh | H | H | 2-Nap | C | | 397 (M⁺+1) |
| Ca-b-33 | 5-e | Int.c-b-1 | BRA40 | 2-CIPh | Et | H | 2-Nap | C | | 441 (M⁺+1) |
| Ca-b-34 | 1-a | Ca-b-33 | | 2-CIPh | H | H | 2-Nap | C | | 413 (M⁺+1) |
| Ca-b-35 | | Ca-a-40 | | 4-CIPh | H | H | 2-Nap | C | | 413 (M⁺+1) |
| Ca-b-36 | | Ca-a-42 | | 3-CF3Ph | H | H | 2-Nap | C | | 447 (M⁺+1) |
| Ca-b-37 | 5-e | Int.c-b-1 | BRA46 | 2,3-DFPh | Et | H | 2-Nap | C | | 443 (M⁺+1) |
| Ca-b-38 | 1-a | Ca-b-37 | | 2,3-DFPh | H | H | 2-Nap | C | | 415 (M⁺+1) |
| Ca-b-39 | | Ca-a-48 | | 2,6-DFPh | H | H | 2-Nap | C | | 415 (M⁺+1) |
| Ca-b-40 | | Ca-a-54 | | 2,4-DClPh | H | H | 2-Nap | C | | 448 (M⁺+1) |
| Ca-b-41 | 5-e | Int.c-b-1 | BRA55 | 2,3-DMePh | Et | H | 2-Nap | C | | 435 (M⁺+1) |
| Ca-b-42 | 1-a | Ca-b-41 | | 2,3-DMePh | H | H | 2-Nap | C | | 407 (M⁺+1) |
| Ca-b-43 | 5-e | Int.c-b-1 | BRA58 | 2-Furan | Et | H | 2-Nap | C | | 397 (M⁺+1) |
| Ca-b-44 | 1-a | Ca-b-43 | | 2-Furan | H | H | 2-Nap | C | | 369 (M⁺+1) |

**[Table 103]**

| Table-Ca-B-2 | | | | | | | | | | |
|---|---|---|---|---|---|---|---|---|---|---|
| Exp. | Syn | SM1 | Reagent | Rx | Y | V₁' | V₂' | LCMS | | |
| | | | | | | | | method | RTime | Mass |
| Ca-b-45 | | Ca-a-64 | | 3-Furan | H | H | 2-Nap | C | | 369 (M⁺+1) |
| Ca-b-46 | 5-e | Int.c-b-1 | BRA60 | 2-Thiophene | Et | H | 2-Nap | C | | 413 (M⁺+1) |
| Ca-b-47 | 1-a | Ca-b-46 | | 2-Thiophene | H | H | 2-Nap | C | | 385 (M⁺+1) |
| Ca-b-48 | | Ca-a-68 | | 3-Thiophene | H | H | 2-Nap | C | | 385 (M⁺+1) |
| Ca-b-49 | 5-e | Int.c-b-1 | BRA63 | 4-Pyridine | Et | H | 2-Nap | C | | 408 (M⁺+1) |
| Ca-b-50 | 1-a | Ca-b-49 | | 4-Pyridine | H | H | 2-Nap | C | | 380 (M⁺+1) |
| Ca-b-51 | 5-e | Int.c-b-1 | BRA64 | 3-PhPh | Et | H | 2-Nap | C | | 483 (M⁺+1) |
| Ca-b-52 | 1-a | Ca-b-51 | | 3-PhPh | H | H | 2-Nap | C | | 455 (M⁺+1) |
| Ca-b-53 | 5-e | Int.c-b-1 | BRA66 | C₄H₉CH=CH- | Et | H | 2-Nap | C | | 413 (M⁺+1) |
| Ca-b-54 | 1-a | Ca-b-53 | | C₄H₉CH=CH- | H | H | 2-Nap | C | | 385 (M⁺+1) |
| Ca-b-55 | 9-j | Ca-b-53 | | nHex- | Et | H | 2-Nap | C | | 415 (M⁺+1) |
| Ca-b-56 | 1-a | Ca-b-55 | | nHex- | H | H | 2-Nap | C | | 387 (M⁺+1) |
| Ca-b-57 | 5-e | Int.c-b-1 | BRA67 | 4FPhCH=CH- | Et | H | 2-Nap | C | | 451 (M⁺+1) |
| Ca-b-58 | 1-a | Ca-b-57 | | 4FPhCH=CH- | H | H | 2-Nap | C | | 423 (M⁺+1) |
| Ca-b-59 | 9-j | Ca-b-57 | | 4FPhC₂H₄- | Et | H | 2-Nap | C | | 453 (M⁺+1) |
| Ca-b-60 | 1-a | Ca-b-59 | | 4FPhC₂H₄- | H | H | 2-Nap | C | | 425 (M⁺+1) |
| Ca-b-61 | 5-e | Int.c-b-1 | BRA71 | 4MePhCH=CH- | Et | H | 2-Nap | C | | 447 (M⁺+1) |
| Ca-b-62 | 1-a | Ca-b-61 | | 4MePhCH=CH- | H | H | 2-Nap | C | | 419 (M⁺+1) |
| Ca-b-63 | 9-j | Ca-b-61 | | 4MePhC₂H₄- | Et | H | 2-Nap | C | | 449 (M⁺+1) |
| Ca-b-64 | 1-a | Ca-b-63 | | 4MePhC₂H₄- | H | H | 2-Nap | C | | 421 (M⁺+1) |
| Ca-b-65 | 5-e | Int.c-b-1 | BRA73 | 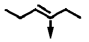 | Et | H | 2-Nap | C | | 413 (M⁺+1) |
| Ca-b-66 | 1-a | Ca-b-65 | | 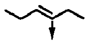 | H | H | 2-Nap | C | | 385 (M⁺+1) |
| Ca-b-67 | 9-j | Ca-b-65 | | 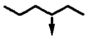 | Et | H | 2-Nap | C | | 415 (M⁺+1) |
| Ca-b-68 | 1-a | Ca-b-67 | | 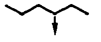 | H | H | 2-Nap | C | | 387 (M⁺+1) |
| Ca-b-69 | 5-e | Int.c-b-2 | BRA14 | Ph | Et | H | 5-Ind | C | | 396 (M⁺+1) |
| Ca-b-70 | 1-a | Ca-b-69 | | Ph | H | H | 5-Ind | C | | 368 (M⁺+1) |
| Ca-b-71 | 5-e | Int.c-b-2 | BRA15 | 2-MePh | Et | H | 5-Ind | C | | 410 (M⁺+1) |
| Ca-b-72 | 1-a | Ca-b-71 | | 2-MePh | H | H | 5-Ind | C | | 382 (M⁺+1) |
| Ca-b-73 | 5-e | Int.c-b-2 | BRA18 | 2-EtPh | Et | H | 5-Ind | C | | 424 (M⁺+1) |
| Ca-b-74 | 1-a | Ca-b-73 | | 2-EtPh | H | H | 5-Ind | C | | 396 (M⁺+1) |
| Ca-b-75 | | Ca-a-106 | | 2-iPrPh | H | H | 5-Ind | C | | 410 (M⁺+1) |
| Ca-b-76 | 5-e | Int.c-b-2 | BRA25 | 3-MeOPh | Et | H | 5-Ind | C | | 426 (M⁺+1) |
| Ca-b-77 | 1-a | Ca-b-76 | | 3-MeOPh | H | H | 5-Ind | C | | 398 (M⁺+1) |
| Ca-b-78 | 5-e | Int.c-b-2 | BRA28 | 3-EtOPh | Et | H | 5-Ind | C | | 440 (M⁺+1) |
| Ca-b-79 | 1-a | Ca-b-78 | | 3-EtOPh | H | H | 5-Ind | C | | 412 (M⁺+1) |
| Ca-b-80 | 5-e | Int.c-b-2 | BRA30 | 4-iPrOPh | Et | H | 5-Ind | C | | 454 (M⁺+1) |
| Ca-b-81 | 1-a | Ca-b-80 | | 4-iPrOPh | H | H | 5-Ind | C | | 426 (M⁺+1) |
| Ca-b-82 | 5-e | Int.c-b-2 | BRA32 | 4-nPrOPh | Et | H | 5-Ind | C | | 454 (M⁺+1) |
| Ca-b-83 | 1-a | Ca-b-82 | | 4-nPrOPh | H | H | 5-Ind | C | | 426 (M⁺+1) |
| Ca-b-84 | 5-e | Int.c-b-2 | BRA35 | 3-DMAPh | Et | H | 5-Ind | C | | 439 (M⁺+1) |
| Ca-b-85 | 1-a | Ca-b-84 | | 3-DMAPh | H | H | 5-Ind | C | | 411 (M⁺+1) |
| Ca-b-86 | | Ca-a-126 | | 2-FPh | H | H | 5-Ind | C | | 386 (M⁺+1) |
| Ca-b-87 | 5-e | Int.c-b-2 | BRA38 | 3-FPh | Et | H | 5-Ind | C | | 414 (M⁺+1) |
| Ca-b-88 | 1-a | Ca-b-87 | | 3-FPh | H | H | 5-Ind | C | | 386 (M⁺+1) |
| Ca-b-89 | 5-e | Int.c-b-2 | BRA41 | 3-CIPh | Et | H | 5-Ind | C | | 430 (M⁺+1) |
| Ca-b-90 | 1-a | Ca-b-89 | | 3-CIPh | H | H | 5-Ind | C | | 402 (M⁺+1) |

**[Table 104]**

| Table-Ca-B-3 | | | | | | | | | | |
|---|---|---|---|---|---|---|---|---|---|---|
| Exp. | Syn | SM1 | Reagent | Rx | Y | V₁' | V₂' | LCMS | | |
| | | | | | | | | method | RTime | Mass |
| Ca-b-89 | 5-e | Int.c-b-2 | BRA41 | 3-CIPh | Et | H | 5-Ind | C | | 430 (M⁺+1) |
| Ca-b-90 | 1-a | Ca-b-89 | | 3-CIPh | H | H | 5-Ind | C | | 402 (M⁺+1) |
| Ca-b-91 | 5-e | Int.c-b-2 | BRA42 | 4-CIPh | Et | H | 5-Ind | C | | 430 (M⁺+1) |
| Ca-b-92 | 1-a | Ca-b-91 | | 4-CIPh | H | H | 5-Ind | C | | 402 (M⁺+1) |
| Ca-b-93 | 5-e | Int.c-b-2 | BRA43 | 2-CF3Ph | Et | H | 5-Ind | C | | 464 (M⁺+1) |
| Ca-b-94 | 1-a | Ca-b-93 | | 2-CF3Ph | H | H | 5-Ind | C | | 436 (M⁺+1) |
| Ca-b-95 | 5-e | Int.c-b-2 | BRA46 | 2,3-DFPh | Et | H | 5-Ind | C | | 432 (M⁺+1) |
| Ca-b-96 | 1-a | Ca-b-95 | | 2,3-DFPh | H | H | 5-Ind | C | | 404 (M⁺+1) |
| Ca-b-97 | 5-e | Int.c-b-2 | BRA49 | 2,6-DFPh | Et | H | 5-Ind | C | | 432 (M⁺+1) |
| Ca-b-98 | 1-a | Ca-b-97 | | 2,6-DFPh | H | H | 5-Ind | C | | 404 (M⁺+1) |
| Ca-b-99 | 5-e | Int.c-b-2 | BRA51 | 3,5-DFPh | Et | H | 5-Ind | C | | 432 (M⁺+1) |
| Ca-b-100 | 1-a | Ca-b-99 | | 3,5-DFPh | H | H | 5-Ind | C | | 404 (M⁺+1) |
| Ca-b-101 | | Ca-a-144 | | 3,5-DClPh | H | H | 5-Ind | C | | 437 (M⁺+1) |
| Ca-b-102 | 5-e | Int.c-b-2 | BRA54 | 3,4-DClPh | Et | H | 5-Ind | C | | 465 (M⁺+1) |
| Ca-b-103 | 1-a | Ca-b-102 | | 3,4-DClPh | H | H | 5-Ind | C | | 437 (M⁺+1) |
| Ca-b-104 | 5-e | Int.c-b-2 | BRA55 | 2,3-DMePh | Et | H | 5-Ind | C | | 424 (M⁺+1) |
| Ca-b-105 | 1-a | Ca-b-104 | | 2,3-DMePh | H | H | 5-Ind | C | | 396 (M⁺+1) |
| Ca-b-106 | 5-e | Int.c-b-2 | BRA58 | 2-Furan | Et | H | 5-Ind | C | | 386 (M⁺+1) |
| Ca-b-107 | 1-a | Ca-b-106 | | 2-Furan | H | H | 5-Ind | C | | 358 (M⁺+1) |
| Ca-b-108 | 5-e | Int.c-b-2 | BRA59 | 3-Furan | Et | H | 5-Ind | C | | 386 (M⁺+1) |
| Ca-b-109 | 1-a | Ca-b-108 | | 3-Furan | H | H | 5-Ind | C | | 358 (M⁺+1) |
| Ca-b-110 | 5-e | Int.c-b-2 | BRA60 | 2-Thiophene | Et | H | 5-Ind | C | | 402 (M⁺+1) |
| Ca-b-111 | 1-a | Ca-b-110 | | 2-Thiophene | H | H | 5-Ind | C | | 374 (M⁺+1) |
| Ca-b-112 | | Ca-a-158 | | 3-Thiophene | H | H | 5-Ind | C | | 374 (M⁺+1) |
| Ca-b-113 | 5-e | Int.c-b-2 | BRA62 | 3-Pyridine | Et | H | 5-Ind | C | | 397 (M⁺+1) |
| Ca-b-114 | 1-a | Ca-b-113 | | 3-Pyridine | H | H | 5-Ind | C | | 369 (M⁺+1) |
| Ca-b-115 | 5-e | Int.c-b-2 | BRA64 | 3-PhPh | Et | H | 5-Ind | C | | 472 (M⁺+1) |
| Ca-b-116 | 1-a | Ca-b-115 | | 3-PhPh | H | H | 5-Ind | C | | 444 (M⁺+1) |
| Ca-b-117 | 5-e | Int.c-b-2 | BRA66 | C₄H₉CH=CH- | Et | H | 5-Ind | C | | 402 (M⁺+1) |
| Ca-b-118 | 1-a | Ca-b-116 | | C₄H₉CH=CH- | H | H | 5-Ind | C | | 374 (M⁺+1) |
| Ca-b-119 | 9-j | Ca-b-116 | | nHex- | Et | H | 5-Ind | C | | 404 (M⁺+1) |
| Ca-b-12C | 1-a | Ca-b-120 | | nHex- | H | H | 5-Ind | C | | 376 (M⁺+1) |
| Ca-b-121 | 5-e | Int.c-b-2 | BRA67 | 4FPhCH=CH- | Et | H | 5-Ind | C | | 440 (M⁺+1) |
| Ca-b-122 | 1-a | Ca-b-122 | | 4FPhCH=CH- | H | H | 5-Ind | C | | 412 (M⁺+1) |
| Ca-b-123 | 5-e | Int.c-b-2 | BRA73 | 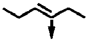 | Et | H | 5-Ind | C | | 402 (M⁺+1) |
| Ca-b-124 | 1-a | Ca-b-123 | | 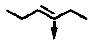 | H | H | 5-Ind | C | | 374 (M⁺+1) |
| Ca-b-125 | 9-j | Ca-b-123 | | 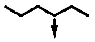 | Et | H | 5-Ind | C | | 404 (M⁺+1) |
| Ca-b-126 | 1-a | Ca-b-125 | | 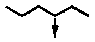 | H | H | 5-Ind | C | | 376 (M⁺+1) |
| Ca-b-127 | 5-e | Int.c-b-3 | BRA14 | Ph | Et | H | 1Me-5-Ind | C | | 410 (M⁺+1) |
| Ca-b-128 | 1-a | Ca-b-127 | | Ph | H | H | 1Me-5-Ind | C | | 382 (M⁺+1) |
| Ca-b-129 | 5-e | Ca-a-174 | BRA15 | 2-MePh | Et | H | 1Me-5-Ind | C | | 396 (M⁺+1) |
| Ca-b-13C | 1-a | Ca-a-176 | | 2-MePh | H | H | 1Me-5-Ind | C | | 396 (M⁺+1) |
| Ca-b-131 | 5-e | Int.c-b-3 | BRA19 | 4-EtPh | Et | H | 1Me-5-Ind | C | | 438 (M⁺+1) |
| Ca-b-132 | 1-a | Ca-b-131 | | 4-EtPh | H | H | 1Me-5-Ind | C | | 410 (M⁺+1) |
| Ca-b-133 | 5-e | Int.c-b-3 | BRA22 | 1-nBuPh | Et | H | 1Me-5-Ind | C | | 466 (M⁺+1) |
| Ca-b-134 | 1-a | Ca-b-133 | | 1-nBuPh | H | H | 1Me-5-Ind | C | | 438 (M⁺+1) |

**[Table 105]**

| Table-Ca-b-4 | | | | | | | | | | |
|---|---|---|---|---|---|---|---|---|---|---|
| Exp. | Syn | SM1 | Reagent | Rx | Y | V₁' | V₂' | LCMS | | |
| | | | | | | | | method | RTime | Mass |
| Ca-b-135 | 5-e | Int.c-b-3 | BRA24 | 2-MeOPh | Et | H | 1Me-5-Ind | C | | 440 (M⁺+1) |
| Ca-b-136 | 1-a | Ca-b-135 | | 2-MeOPh | H | H | 1Me-5-Ind | C | | 412 (M⁺+1) |
| Ca-b-137 | | Ca-a-188 | | 2-EtOPh | H | H | 1Me-5-Ind | C | | 426 (M⁺+1) |
| Ca-b-138 | 5-e | Int.c-b-3 | BRA29 | 3-iPrOPh | Et | H | 1Me-5-Ind | C | | 468 (M⁺+1) |
| Ca-b-139 | 1-a | Ca-b-138 | | 3-iPrOPh | H | H | 1Me-5-Ind | C | | 440 (M⁺+1) |
| Ca-b-140 | 5-e | Int.c-b-3 | BRA32 | 4-nPrOPh | Et | H | 1Me-5-Ind | C | | 468 (M⁺+1) |
| Ca-b-141 | 1-a | Ca-b-140 | | 4-nPrOPh | H | H | 1Me-5-Ind | C | | 440 (M⁺+1) |
| Ca-b-142 | 5-e | Int.c-b-3 | BRA34 | 4-nBuOPh | Et | H | 1 Me-5-Ind | C | | 482 (M⁺+1) |
| Ca-b-143 | 1-a | Ca-b-142 | | 4-nBuOPh | H | H | 1Me-5-Ind | C | | 454 (M⁺+1) |
| Ca-b-144 | 5-e | Int.c-b-3 | BRA36 | 4-DMAPh | Et | H | 1Me-5-Ind | C | | 453 (M⁺+1) |
| Ca-b-145 | 1-a | Ca-b-144 | | 4-DMAPh | H | H | 1Me-5-Ind | C | | 425 (M⁺+1) |
| Ca-b-146 | 5-e | Int.c-b-3 | BRA37 | 2-FPh | Et | H | 1Me-5-Ind | C | | 428 (M⁺+1) |
| Ca-b-147 | 1-a | Ca-b-146 | | 2-FPh | H | H | 1Me-5-Ind | C | | 400 (M⁺+1) |
| Ca-b-148 | 5-e | Int.c-b-3 | BRA39 | 4-FPh | Et | H | 1Me-5-Ind | C | | 428 (M⁺+1) |
| Ca-b-149 | 1-a | Ca-b-148 | | 4-FPh | H | H | 1Me-5-Ind | C | | 400 (M⁺+1) |
| Ca-b-150 | 5-e | Int.c-b-3 | BRA42 | 4-ClPh | Et | H | 1Me-5-Ind | C | | 444 (M⁺+1) |
| Ca-b-151 | 1-a | Ca-b-150 | | 4-ClPh | H | H | 1Me-5-Ind | C | | 416 (M⁺+1) |
| Ca-b-152 | 5-e | Int.c-b-3 | BRA44 | 3-CF3Ph | Et | H | 1Me-5-Ind | C | | 478 (M⁺+1) |
| Ca-b-153 | 1-a | Ca-b-152 | | 3-CF3Ph | H | H | 1Me-5-Ind | C | | 450 (M⁺+1) |
| Ca-b-154 | | Ca-a-212 | | 2,4-DFPh | H | H | 1Me-5-Ind | C | | 418 (M⁺+1) |
| Ca-b-155 | 5-e | Int.c-b-3 | BRA49 | 2,6-DFPh | Et | H | 1Me-5-Ind | C | | 446 (M⁺+1) |
| Ca-b-156 | 1-a | Ca-b-155 | | 2,6-DFPh | H | H | 1Me-5-Ind | C | | 418 (M⁺+1) |
| Ca-b-157 | 5-e | Int.c-b-3 | BRA50 | 3,4-DFPh | Et | H | 1 Me-5-Ind | C | | 446 (M⁺+1) |
| Ca-b-158 | 1-a | Ca-b-157 | | 3,4-DFPh | H | H | 1Me-5-Ind | C | | 418 (M⁺+1) |
| Ca-b-159 | 5-e | Int.c-b-3 | BRA51 | 3,5-DClPh | Et | H | 1Me-5-Ind | C | | 479 (M⁺+1) |
| Ca-b-160 | 1-a | Ca-b-159 | | 3,5-DClPh | H | H | 1Me-5-Ind | C | | 451 (M⁺+1) |
| Ca-b-161 | 5-e | Int.c-b-3 | BRA54 | 3,4-DClPh | Et | H | 1Me-5-Ind | C | | 479 (M⁺+1) |
| Ca-b-162 | 1-a | Ca-b-161 | | 3,4-DClPh | H | H | 1Me-5-Ind | C | | 451 (M⁺+1) |
| Ca-b-163 | 5-e | Int.c-b-3 | BRA55 | 2,3-DMePh | Et | H | 1Me-5-Ind | C | | 438 (M⁺+1) |
| Ca-b-164 | 1-a | Ca-b-163 | | 2,3-DMePh | H | H | 1Me-5-Ind | C | | 410 (M⁺+1) |
| Ca-b-165 | | Ca-a-224 | | 2,4-DMePh | H | H | 1Me-5-Ind | C | | 410 (M⁺+1) |
| Ca-b-166 | 5-e | Int.c-b-3 | BRA58 | 2-Furan | Et | H | 1Me-5-Ind | C | | 400 (M⁺+1) |
| Ca-b-167 | 1-a | Ca-b-166 | | 2-Furan | H | H | 1Me-5-Ind | C | | 372 (M⁺+1) |
| Ca-b-168 | 5-e | Int.c-b-3 | BRA59 | 3-Furan | Et | H | 1Me-5-Ind | C | | 400 (M⁺+1) |
| Ca-b-169 | 1-a | Ca-b-168 | | 3-Furan | H | H | 1Me-5-Ind | C | | 372 (M⁺+1) |
| Ca-b-170 | 5-e | Int.c-b-3 | BRA60 | 2-Thiophene | Et | H | 1Me-5-Ind | C | | 416 (M⁺+1) |
| Ca-b-171 | 1-a | Ca-b-170 | | 2-Thiophene | H | H | 1Me-5-Ind | C | | 388 (M⁺+1) |
| Ca-b-172 | 5-e | Int.c-b-3 | BRA61 | 3-Thiophene | Et | H | 1Me-5-Ind | C | | 416 (M⁺+1) |
| Ca-b-173 | 1-a | Ca-b-172 | | 3-Thiophene | H | H | 1Me-5-Ind | C | | 388 (M⁺+1) |
| Ca-b-174 | | Ca-a-234 | | 4-Pyridine | H | H | 1Me-5-Ind | C | | 383 (M⁺+1) |
| Ca-b-175 | 5-e | Int.c-b-3 | BRA64 | 3-PhPh | Et | H | 1Me-5-Ind | C | | 486 (M⁺+1) |
| Ca-b-176 | 1-a | Ca-b-175 | | 3-PhPh | H | H | 1Me-5-Ind | C | | 458 (M⁺+1) |
| Ca-b-177 | | Ca-a-238 | | 4FPhCH=CH- | H | H | 1Me-5-Ind | C | | 426 (M⁺+1) |
| Ca-b-178 | | Ca-a-240 | | 4FPhC₂H₄- | H | H | 1Me-5-Ind | C | | 428 (M⁺+1) |
| Ca-b-179 | 5-e | Int.c-b-3 | BRA68 | 4ClPhCH=CH- | Et | H | 1Me-5-Ind | C | | 471 (M⁺+1) |
| Ca-b-180 | 1-a | Ca-b-179 | | 4ClPhCH=CH- | H | H | 1Me-5-Ind | C | | 442 (M⁺+1) |

**[Table 106]**

| Table-Ca-B-5 | | | | | | | | | | |
|---|---|---|---|---|---|---|---|---|---|---|
| Exp. | Syn. | SM1 | SM2 | Rx | Y | V₁' | V₂' | LCMS | | |
| | | | | | | | | method | RTime | Mass |
| Ca-b-181 | 5-e | Int.c-b-3 | BRA73 | 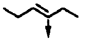 | Et | H | 1Me-5-Ind | C | | 416 (M⁺+1) |
| Ca-b-182 | 1-a | Ca-b-181 | | 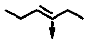 | H | H | 1Me-5-Ind | C | | 388 (M⁺+1) |
| Ca-b-183 | 9-j | Ca-b-181 | | 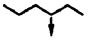 | Et | H | 1Me-5-Ind | C | | 418 (M⁺+1) |
| Ca-b-184 | 1-a | Ca-b-183 | | 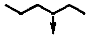 | H | H | 1Me-5-Ind | C | | 390 (M⁺+1) |
| Ca-b-185 | | Ca-a-252 | | Ph | H | H | 5-1Idz | C | | 369 (M⁺+1) |
| Ca-b-186 | 5-e | Int.c-b-4 | BRA17 | 4-MePh | Et | H | 5-1Idz | C | | 411 (M⁺+1) |
| Ca-b-187 | 1-a | Ca-b-186 | | 4-MePh | H | H | 5-1Idz | C | | 383 (M⁺+1) |
| Ca-b-188 | 5-e | Int.c-b-4 | BRA19 | 4-EtPh | Et | H | 5-1Idz | C | | 425 (M⁺+1) |
| Ca-b-189 | 1-a | Ca-b-188 | | 4-EtPh | H | H | 5-1Idz | C | | 397 (M⁺+1) |
| Ca-b-190 | | Ca-a-260 | | 2-iPrPh | H | H | 5-1Idz | C | | 411 (M⁺+1) |
| Ca-b-191 | 5-e | Int.c-b-4 | BRA22 | 1-nBuPh | Et | H | 5-1Idz | C | | 453 (M⁺+1) |
| Ca-b-192 | 1-a | Ca-b-191 | | 1-nBuPh | H | H | 5-1Idz | C | | 425 (M⁺+1) |
| Ca-b-193 | 5-e | Int.c-b-4 | BRA25 | 3-MeOPh | Et | H | 5-1Idz | C | | 427 (M⁺+1) |
| Ca-b-194 | 1-a | Ca-b-193 | | 3-MeOPh | H | H | 5-1Idz | C | | 399 (M⁺+1) |
| Ca-b-195 | 5-e | Int.c-b-4 | BRA28 | 3-EtOPh | Et | H | 5-1Idz | C | | 441 (M⁺+1) |
| Ca-b-196 | 1-a | Ca-b-195 | | 3-EtOPh | H | H | 5-1Idz | C | | 413 (M⁺+1) |
| Ca-b-197 | 5-e | Int.c-b-4 | BRA30 | 4-iPrOPh | Et | H | 5-1Idz | C | | 455 (M⁺+1) |
| Ca-b-198 | 1-a | Ca-b-197 | | 4-iPrOPh | H | H | 5-1Idz | C | | 427 (M⁺+1) |
| Ca-b-199 | 5-e | Int.c-b-4 | BRA31 | 3-nPrOPh | Et | H | 5-1Idz | C | | 455 (M⁺+1) |
| Ca-b-200 | 1-a | Ca-b-199 | | 3-nPrOPh | H | H | 5-1Idz | C | | 427 (M⁺+1) |
| Ca-b-201 | | Ca-a-276 | | 4-DMAPh | H | H | 5-1Idz | C | | 412 (M⁺+1) |
| Ca-b-202 | 5-e | Int.c-b-4 | BRA37 | 2-FPh | Et | H | 5-1Idz | C | | 415 (M⁺+1) |
| Ca-b-203 | 1-a | Ca-b-202 | | 2-FPh | H | H | 5-1Idz | C | | 387 (M⁺+1) |
| Ca-b-204 | 5-e | Int.c-b-4 | BRA41 | 3-ClPh | Et | H | 5-1Idz | C | | 431 (M⁺+1) |
| Ca-b-205 | 1-a | Ca-b-204 | | 3-ClPh | H | H | 5-1Idz | C | | 403 (M⁺+1) |
| Ca-b-206 | 5-e | Int.c-b-4 | BRA42 | 4-ClPh | Et | H | 5-1Idz | C | | 431 (M⁺+1) |
| Ca-b-207 | 1-a | Ca-b-206 | | 4-ClPh | H | H | 5-1Idz | C | | 403 (M⁺+1) |
| Ca-b-208 | 5-e | Int.c-b-4 | BRA44 | 3-CF3Ph | Et | H | 5-1Idz | C | | 465 (M⁺+1) |
| Ca-b-209 | 1-a | Ca-b-208 | | 3-CF3Ph | H | H | 5-1Idz | C | | 437 (M⁺+1) |
| Ca-b-210 | 5-e | Int.c-b-4 | BRA46 | 2,3-DFPh | Et | H | 5-1Idz | C | | 433 (M⁺+1) |
| Ca-b-211 | 1-a | Ca-b-210 | | 2,3-DFPh | H | H | 5-1Idz | C | | 405 (M⁺+1) |
| Ca-b-212 | 5-e | Int.c-b-4 | BRA48 | 2,5-DFPh | Et | H | 5-1Idz | C | | 433 (M⁺+1) |
| Ca-b-213 | 1-a | Ca-b-212 | | 2,5-DFPh | H | H | 5-1Idz | C | | 405 (M⁺+1) |
| Ca-b-214 | | Ca-a-294 | | 2,6-DFPh | H | H | 5-1Idz | C | | 405 (M⁺+1) |
| Ca-b-215 | | Ca-a-298 | | 2,4-DClPh | H | H | 5-1Idz | C | | 438 (M⁺+1) |
| Ca-b-216 | | Ca-a-302 | | 3,4-DClPh | H | H | 5-1Idz | C | | 438 (M⁺+1) |
| Ca-b-217 | 5-e | Int.c-b-4 | BRA58 | 2-Furan | Et | H | 5-1Idz | C | | 387 (M⁺+1) |
| Ca-b-218 | 1-a | Ca-b-217 | | 2-Furan | H | H | 5-1Idz | C | | 359 (M⁺+1) |
| Ca-b-219 | 5-e | Int.c-b-4 | BRA59 | 3-Furan | Et | H | 5-1Idz | C | | 387 (M⁺+1) |
| Ca-b-220 | 1-a | Ca-b-219 | | 3-Furan | H | H | 5-1Idz | C | | 359 (M⁺+1) |
| Ca-b-221 | 5-e | Int.c-b-4 | BRA60 | 2-Thiophene | Et | H | 5-1Idz | C | | 403 (M⁺+1) |
| Ca-b-222 | 1-a | Ca-b-221 | | 2-Thiophene | H | H | 5-1Idz | C | | 375 (M⁺+1) |
| Ca-b-223 | 5-e | Int.c-b-4 | BRA61 | 3-Thiophene | Et | H | 5-1Idz | C | | 403 (M⁺+1) |
| Ca-b-224 | 1-a | Ca-b-223 | | 3-Thiophene | H | H | 5-1Idz | C | | 375 (M⁺+1) |
| Ca-b-225 | | Ca-a-316 | | 3-Pyridine | H | H | 5-1Idz | C | | 398 (M⁺+1) |
| Ca-b-226 | | Ca-a-318 | | 4-Pyridine | H | H | 5-1Idz | C | | 370 (M⁺+1) |

**[Table 107]**

| Table-Ca-B-6 | | | | | | | | | | |
|---|---|---|---|---|---|---|---|---|---|---|
| Exp. | Syn. | SM1 | Reagent | Rx | Y | V₁' | V₂' | LCMS | | |
| | | | | | | | | method | RTime | Mass |
| Ca-b-225 | | Ca-a-316 | | 3-Pyridine | H | H | 5-1Idz | C | | 398 (M⁺+1) |
| Ca-b-226 | | Ca-a-318 | | 4-Pyridine | H | H | 5-1Idz | C | | 370 (M⁺+1) |
| Ca-b-227 | 5-e | Int.c-b-4 | BRA66 | C₄H₉CH=CH- | Et | H | 5-1Idz | C | | 403 (M⁺+1) |
| Ca-b-228 | 1-a | Ca-b-227 | | C₄H₉CH=CH- | H | H | 5-1Idz | C | | 375 (M⁺+1) |
| Ca-b-229 | 9-j | Ca-b-227 | | nHex- | Et | H | 5-1Idz | C | | 405 (M⁺+1) |
| Ca-b-230 | 1-a | Ca-b-229 | | nHex- | H | H | 5-1Idz | C | | 377 (M⁺+1) |
| Ca-b-231 | 5-e | Intc-b-4 | BRA67 | 4FPhCH=CH- | Et | H | 5-1Idz | C | | 441 (M⁺+1) |
| Ca-b-232 | 1-a | Ca-b-231 | | 4FPhCH=CH- | H | H | 5-1Idz | C | | 413 (M⁺+1) |
| Ca-b-233 | 9-j | Ca-b-231 | | 4FPhC₂H₄- | Et | H | 5-1Idz | C | | 443 (M⁺+1) |
| Ca-b-234 | 1-a | Ca-b-233 | | 4FPhC₂H₄- | H | H | 5-1Idz | C | | 415 (M⁺+1) |
| Ca-b-235 | 5-e | Int.c-b-4 | BRA68 | 4CIPhCH=CH- | Et | H | 5-1Idz | C | | 457 (M⁺+1) |
| Ca-b-236 | 1-a | Ca-b-235 | | 4ClPhCH=CH- | H | H | 5-1Idz | C | | 429 (M⁺+1) |
| Ca-b-237 | 5-e | Int.c-b-4 | BRA70 | 4CF3PhCH=CH- | Et | H | 5-1Idz | C | | 491 (M⁺+1) |
| Ca-b-238 | 1-a | Ca-b-237 | | 4CF3PhCH=CH- | H | H | 5-1Idz | C | | 463 (M⁺+1) |
| Ca-b-239 | 9-j | Ca-b-237 | | 4CF3PhC₂H₄- | Et | H | 5-1Idz | C | | 493 (M⁺+1) |
| Ca-b-240 | 1-a | Ca-b-239 | | 4CF3PhC₂H₄- | H | H | 5-1Idz | C | | 465 (M⁺+1) |
| Ca-b-241 | 5-e | Int.c-b-4 | BRA71 | 4MePhCH=CH- | Et | H | 5-1Idz | C | | 437 (M⁺+1) |
| Ca-b-242 | 1-a | Ca-b-241 | | 4MePhCH=CH- | H | H | 5-1Idz | C | | 409 (M⁺+1) |
| Ca-b-243 | 9-j | Ca-b-241 | | 4MePhC₂H₄- | Et | H | 5-1Idz | C | | 439 (M⁺+1) |
| Ca-b-244 | 1-a | Ca-b-243 | | 4MePhC₂H₄- | H | H | 5-1Idz | C | | 411 (M⁺+1) |
| Ca-b-245 | | Ca-a-340 | | Ph | H | H | 1Me-5-Idz | C | | 383 (M⁺+1) |
| Ca-b-246 | 5-e | Int.c-b-5 | BRA15 | 2-MePh | Et | H | 1Me-5-Idz | C | | 425 (M⁺+1) |
| Ca-b-247 | 1-a | Ca-b-246 | | 2-MePh | H | H | 1Me-5-Idz | C | | 397 (M⁺+1) |
| Ca-b-248 | 5-e | Int.c-b-5 | BRA16 | 3-MePh | Et | H | 1Me-5-Idz | C | | 425 (M⁺+1) |
| Ca-b-249 | 1-a | Ca-b-248 | | 3-MePh | H | H | 1Me-5-Idz | C | | 397 (M⁺+1) |
| Ca-b-250 | 5-e | Ca-b-249 | BRA18 | 2-EtPh | Et | H | 1Me-5-Idz | C | | 439 (M⁺+1) |
| Ca-b-251 | 1-a | Int.c-b-5 | | 2-EtPh | H | H | 1Me-5-Idz | C | | 411 (M⁺+1) |
| Ca-b-252 | 5-e | Ca-b-251 | BRA20 | 2-iPrPh | Et | H | 1Me-5-Idz | C | | 453 (M⁺+1) |
| Ca-b-253 | 1-a | Int.c-b-5 | | 2-iPrPh | H | H | 1Me-5-Idz | C | | 425 (M⁺+1) |
| Ca-b-254 | | Ca-a-352 | | 1-nBuPh | H | H | 1Me-5-Idz | C | | 439 (M⁺+1) |
| Ca-b-255 | 5-e | lnt.c-b-5 | BRA23 | 4-tBuPh | Et | H | 1Me-5-Idz | C | | 467 (M⁺+1) |
| Ca-b-256 | 1-a | Ca-b-255 | | 4-tBuPh | H | H | 1Me-5-Idz | C | | 439 (M⁺+1) |
| Ca-b-257 | 5-e | Int.c-b-5 | BRA25 | 3-MeOPh | Et | H | 1Me-5-Idz | C | | 441 (M⁺+1) |
| Ca-b-258 | 1-a | Ca-b-257 | | 3-MeOPh | H | H | 1Me-5-Idz | C | | 413 (M⁺+1) |
| Ca-b-259 | 5-e | Int.c-b-5 | BRA26 | 4-MeOPh | Et | H | 1Me-5-Idz | C | | 441 (M⁺+1) |
| Ca-b-260 | 1-a | Ca-b-259 | | 4-MeOPh | H | H | 1Me-5-Idz | C | | 413 (M⁺+1) |
| Ca-b-261 | 5-e | Int.c-b-5 | BRA28 | 3-EtOPh | Et | H | 1Me-5-Idz | C | | 455 (M⁺+1) |
| Ca-b-262 | 1-a | Ca-b-261 | | 3-EtOPh | H | H | 1Me-5-Idz | C | | 427 (M⁺+1) |
| Ca-b-263 | 5-e | Int.c-b-5 | BRA29 | 3-iPrOPh | Et | H | 1Me-5-Idz | C | | 469 (M⁺+1) |
| Ca-b-264 | 1-a | Ca-b-263 | | 3-iPrOPh | H | H | 1Me-5-Idz | C | | 441 (M⁺+1) |
| Ca-b-265 | 5-e | Int.c-b-5 | BRA30 | 4-iPrOPh | Et | H | 1Me-5-Idz | C | | 469 (M⁺+1) |
| Ca-b-266 | 1-a | Ca-b-265 | | 4-iPrOPh | H | H | 1Me-5-Idz | C | | 441 (M⁺+1) |
| Ca-b-267 | 5-e | Int.c-b-5 | BRA32 | 4-nPrOPh | Et | H | 1Me-5-Idz | C | | 469 (M⁺+1) |
| Ca-b-268 | 1-a | Ca-b-267 | | 4-nPrOPh | H | H | 1Me-5-Idz | C | | 441 (M⁺+1) |
| Ca-b-269 | 5-e | Int.c-b-5 | BRA33 | 3-nBuOPh | Et | H | 1Me-5-Idz | C | | 483 (M⁺+1) |
| Ca-b-270 | 1-a | Ca-b-269 | | 3-nBuOPh | H | H | 1Me-5-Idz | C | | 455 (M⁺+1) |

**[Table 108]**

| Tabte-Ca-B-7 | | | | | | | | | | |
|---|---|---|---|---|---|---|---|---|---|---|
| Exp. | Syn. | SM1 | Reagent | Rx | Y | V₁' | V₂' | LCMS | | |
| | | | | | | | | method | RTime | Mass |
| Ca-b-271 | 5-e | Int.c-b-5 | BRA36 | 4-DMAPh | Et | H | 1Me-5-Idz | C | | 454 (M⁺+1) |
| Ca-b-272 | 1-a | Ca-b-271 | | 4-DMAPh | H | H | 1Me-5-Idz | C | | 426 (M⁺+1) |
| Ca-b-273 | | Ca-a-376 | | 2-FPh | H | H | 1Me-5-Idz | C | | 401 (M⁺+1) |
| Ca-b-274 | | Ca-a-378 | | 3-FPh | H | H | 1Me-5-Idz | C | | 401 (M⁺+1) |
| Ca-b-275 | | Ca-a-380 | | 4-FPh | H | H | 1Me-5-Idz | C | | 401 (M⁺+1) |
| Ca-b-276 | 5-e | Int.c-b-5 | BRA42 | 4-ClPh | Et | H | 1Me-5-Idz | C | | 445 (M⁺+1) |
| Ca-b-277 | 1-a | Ca-b-276 | | 4-ClPh | H | H | 1Me-5-Idz | C | | 417 (M⁺+1) |
| Ca-b-278 | 5-e | Int.c-b-5 | BRA43 | 2-CF3Ph | Et | H | 1Me-5-Idz | C | | 479 (M⁺+1) |
| Ca-b-279 | 1-a | Ca-b-278 | | 2-CF3Ph | H | H | 1Me-5-Idz | C | | 451 (M⁺+1) |
| Ca-b-280 | 5-e | Int.c-b-5 | BRA46 | 2,3-DFPh | Et | H | 1Me-5-Idz | C | | 447 (M⁺+1) |
| Ca-b-281 | 1-a | Ca-b-280 | | 2,3-DFPh | H | H | 1Me-5-Idz | C | | 419 (M⁺+1) |
| Ca-b-282 | 5-e | Int.c-b-5 | BRA48 | 2,5-DFPh | Et | H | 1Me-5-Idz | C | | 447 (M⁺+1) |
| Ca-b-283 | 1-a | Ca-b-282 | | 2,5-DFPh | H | H | 1Me-5-Idz | C | | 419 (M⁺+1) |
| Ca-b-284 | 5-e | Int.c-b-5 | BRA49 | 2,6-DFPh | Et | H | 1Me-5-Idz | C | | 447 (M⁺+1) |
| Ca-b-285 | 1-a | Ca-b-284 | | 2,6-DFPh | H | H | 1Me-5-Idz | C | | 419 (M⁺+1) |
| Ca-b-286 | 5-e | Int.c-b-5 | BRA51 | 3,5-DFPh | Et | H | 1Me-5-Idz | C | | 447 (M⁺+1) |
| Ca-b-287 | 1-a | Ca-b-286 | | 3,5-DFPh | H | H | 1Me-5-Idz | C | | 419 (M⁺+1) |
| Ca-b-288 | | Ca-a-394 | | 2,4-DClPh | H | H | 1 Me-5-Idz | C | | 452 (M⁺+1) |
| Ca-b-289 | | Ca-a-396 | | 3,5-DClPh | H | H | 1Me-5-Idz | C | | 452 (M⁺+1) |
| Ca-b-290 | 5-e | Int.c-b-5 | BRA55 | 2,3-DMePh | Et | H | 1 Me-5-Idz | C | | 439 (M⁺+1) |
| Ca-b-291 | 1-a | Ca-b-290 | | 2,3-DMePh | H | H | 1Me-5-Idz | C | | 411 (M⁺+1) |
| Ca-b-292 | 5-e | Int.c-b-5 | BRA57 | 2.6-DMePh | Et | H | 1Me-5-Idz | C | | 439 (M⁺+1) |
| Ca-b-293 | 1-a | Ca-b-292 | | 2,6-DMePh | H | H | 1Me-5-Idz | C | | 411 (M⁺+1) |
| Ca-b-294 | 5-e | Ca-b-293 | BRA58 | 2-Furan | Et | H | 1Me-5-Idz | C | | 401 (M⁺+1) |
| Ca-b-295 | 1-a | Ca-b-294 | | 2-Furan | H | H | 1Me-5-Idz | C | | 373 (M⁺+1) |
| Ca-b-296 | 5-e | Ca-b-295 | BRA59 | 3-Furan | Et | H | 1Me-5-Idz | C | | 401 (M⁺+1) |
| Ca-b-297 | 1-a | Ca-b-296 | | 3-Furan | H | H | 1Me-5-Idz | C | | 373 (M⁺+1) |
| Ca-b-298 | 5-e | Ca-b-297 | BRA60 | 2-Thiophene | Et | H | 1Me-5-Idz | C | | 417 (M⁺+1) |
| Ca-b-299 | 1-a | Int.c-b-5 | | 2-Thiophene | H | H | 1Me-5-Idz | C | | 389 (M⁺+1) |
| Ca-b-300 | 5-e | Ca-b-299 | BRA61 | 3-Thiophene | Et | H | 1Me-5-Idz | C | | 417 (M⁺+1) |
| Ca-b-301 | 1-a | Int.c-b-5 | | 3-Thiophene | H | H | 1Me-5-Idz | C | | 389 (M⁺+1) |
| Ca-b-302 | | Ca-a-410 | | 3-Pyridine | H | H | 1Me-5-Idz | C | | 384 (M⁺+1) |
| Ca-b-303 | | Ca-a-412 | | 4-Pyridine | H | H | 1Me-5-Idz | C | | 384 (M⁺+1) |
| Ca-b-304 | 5-e | Int.c-b-5 | BRA64 | 3-PhPh | Et | H | 1Me-5-Idz | C | | 487 (M⁺+1) |
| Ca-b-305 | 1-a | Ca-b-304 | | 3-PhPh | H | H | 1Me-5-Idz | C | | 459 (M⁺+1) |
| Ca-b-306 | 5-e | Int.c-b-5 | BRA66 | C₄H₉CH=CH- | Et | H | 1Me-5-Idz | C | | 417 (M⁺+1) |
| Ca-b-307 | 1-a | Ca-b-306 | | C₄H₉CH=CH- | H | H | 1Me-5-Idz | C | | 389 (M⁺+1) |
| Ca-b-308 | 9-j | Ca-b-306 | | nHex- | Et | H | 1Me-5-Idz | C | | 419 (M⁺+1) |
| Ca-b-309 | 1-a | Ca-b-308 | | nHex- | H | H | 1Me-5-Idz | C | | 391 (M⁺+1) |
| Ca-b-310 | 5-e | Int.c-b-5 | BRA67 | 4FPhCH=CH- | Et | H | 1Me-5-Idz | C | | 455 (M⁺+1) |
| Ca-b-311 | 1-a | Ca-b-310 | | 4FPhCH=CH- | H | H | 1Me-5-Idz | C | | 427 (M⁺+1) |
| Ca-b-312 | 9-j | Ca-b-310 | | 4FPhC₂H₄- | Et | H | 1Me-5-Idz | C | | 457 (M⁺+1) |
| Ca-b-313 | 1-a | Ca-b-312 | | 4FPhC₂H₄- | H | H | 1Me-5-Idz | C | | 429 (M⁺+1) |
| Ca-b-314 | 5-e | Int.c-b-5 | BRA69 | 2cHexCH=CH- | Et | H | 1Me-5-Idz | C | | 443 (M⁺+1) |
| Ca-b-315 | 1-a | Ca-b-314 | | 2cHexCH=CH- | H | H | 1Me-5-Idz | C | | 415 (M⁺+1) |
| Ca-b-316 | 9-j | Ca-b-314 | | 2cHexC₂H₄- | Et | H | 1Me-5-Idz | C | | 445 (M⁺+1) |

**[Table 109]**

| Table-Ca-B-8 | | | | | | | | | | |
|---|---|---|---|---|---|---|---|---|---|---|
| Exp. | Syn. | SM1 | Reagent | Rx | Y | V₁' | V₂' | LCMS | | |
| | | | | | | | | method | RTime | Mass |
| Ca-b-317 | 1-a | Ca-b-316 | | 2cHexC₂H₄- | H | H | 1Me-5-Idz | C | | 417 (M⁺+1) |
| Ca-b-318 | 5-e | Intc-b-5 | BRA70 | 4CF₃PhCH=CH- | Et | H | 1Me-5-Idz | C | | 505 (M⁺+1) |
| Ca-b-319 | 1-a | Ca-b-318 | | 4CF3PhCH=CH- | H | H | 1Me-5-Idz | C | | 477 (M⁺+1) |
| Ca-b-320 | 5-e | Ca-b-318 | | 4CF3PhC₂H₄- | Et | H | 1Me-5-Idz | C | | 507 (M⁺+1) |
| Ca-b-321 | 1-a | Ca-b-320 | | 4CF3PhC₂H₄- | H | H | 1Me-5-Idz | C | | 479 (M⁺+1) |
| Ca-b-322 | 5-e | Int.c-b-5 | BRA71 | 4MePhCH=CH- | Et | H | 1Me-5-Idz | C | | 451 (M⁺+1) |
| Ca-b-323 | 1-a | Ca-b-322 | | 4MePhCH=CH- | H | H | 1Me-5-Idz | C | | 423 (M⁺+1) |
| Ca-b-324 | 9₋j | Ca-b-322 | | 4MePhC₂H₄- | Et | H | 1Me-5-Idz | C | | 453 (M⁺+1) |
| Ca-b-325 | 1-a | Ca-b-324 | | 4MePhC₂H₄- | H | H | 1Me-5-Idz | C | | 425 (M⁺+1) |
| Ca-b-326 | 5-e | Int.c-b-5 | BRA73 | 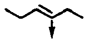 | Et | H | 1Me-5-Idz | C | | 417 (M⁺+1) |
| Ca-b-327 | 1-a | Ca-b-326 | | 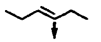 | H | H | 1Me-5-Idz | C | | 389 (M⁺+1) |
| Ca-b-328 | 9-j | Ca-b-326 | | 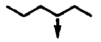 | Et | H | 1Me-5-Idz | C | | 419 (M⁺+1) |
| Ca-b-329 | 1-a | Ca-b-328 | | 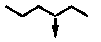 | H | H | 1Me-5-Idz | C | | 391 (M⁺+1) |
| Ca-b-330 | 5-e | Int.c-b-6 | BRA14 | Ph | Et | H | 1Et-5-Idz | C | | 425 (M⁺+1) |
| Ca-b-331 | 1-a | Ca-b-330 | | Ph | H | H | 1Et-5-Idz | C | | 397 (M⁺+1) |
| Ca-b-332 | 5-e | Int.c-b-6 | BRA15 | 2-MePh | Et | H | 1Et-5-Idz | C | | 439 (M⁺+1) |
| Ca-b-333 | 1-a | Ca-b-332 | | 2-MePh | H | H | 1Et-5-Idz | C | | 411 (M⁺+1) |
| Ca-b-334 | | Ca-a-438 | | 3-MePh | H | H | 1Et-5-Idz | C | | 411 (M⁺+1) |
| Ca-b-335 | | Ca-a-440 | | 2-EtPh | H | H | 1Et-5-Idz | C | | 425 (M⁺+1) |
| Ca-b-336 | 5-e | Int.c-b-6 | BRA20 | 2-iPrPh | Et | H | 1Et-5-Idz | C | | 467 (M⁺+1) |
| Ca-b-337 | 1-a | Ca-b-336 | | 2-iPrPh | H | H | 1Et-5-Idz | C | | 439 (M⁺+1) |
| Ca-b-338 | 5-e | Int.c-b-6 | BRA27 | 2-EtOPh | Et | H | 1Et-5-Idz | C | | 469 (M⁺+1) |
| Ca-b-339 | 1-a | Ca-b-338 | | 2-EtOPh | H | H | 1Et-5-Idz | C | | 441 (M⁺+1) |
| Ca-b-340 | | Ca-a-444 | | 3-nPrOPh | H | H | 1Et-5-Idz | C | | 455 (M⁺+1) |
| Ca-b-341 | 5-e | Int.c-b-6 | BRA35 | 3-DMAPh | Et | H | 1Et-5-Idz | C | | 468 (M⁺+1) |
| Ca-b-342 | 1-a | Ca-b-341 | | 3-DMAPh | H | H | 1Et-5-Idz | C | | 440 (M⁺+1) |
| Ca-b-343 | | Ca-a-450 | | 3-FPh | H | H | 1Et-5-Idz | C | | 415 (M⁺+1) |
| Ca-b-344 | 5-e | Int.c-b-6 | BRA40 | 2-ClPh | Et | H | 1Et-5-Idz | C | | 460 (M⁺+1) |
| Ca-b-345 | 1-a | Ca-b-344 | | 2-ClPh | H | H | 1Et-5-Idz | C | | 431 (M⁺+1) |
| Ca-b-346 | | Ca-a-454 | | 3-ClPh | H | H | 1Et-5-Idz | C | | 431 (M⁺+1) |
| Ca-b-347 | 5-e | Int.c-b-6 | BRA47 | 2,4-DFPh | Et | H | 1Et-5-Idz | C | | 461 (M⁺+1) |
| Ca-b-348 | 1-a | Ca-b-347 | | 2,4-DFPh | H | H | 1Et-5-Idz | C | | 433 (M⁺+1) |
| Ca-b-349 | 5-e | Int.c-b-6 | BRA48 | 2.5-DFPh | Et | H | 1Et-5-Idz | C | | 461 (M⁺+1) |
| Ca-b-350 | 1-a | Ca-b-349 | | 2.5-DFPh | H | H | 1Et-5-Idz | C | | 433 (M⁺+1) |
| Ca-b-351 | | Ca-a-462 | | 3,4-DFPh | H | H | 1Et-5-Idz | C | | 433 (M⁺+1) |
| Ca-b-352 | 5-e | Ca-b-351 | BRA53 | 3,5-DClPh | Et | H | 1Et-5-Idz | C | | 494 (M⁺+1) |
| Ca-b-353 | 1-a | Int.c-b-6 | | 3,5-DClPh | H | H | 1Et-5-Idz | C | | 466 (M⁺+1) |
| Ca-b-354 | 5-e | Ca-b-353 | BRA54 | 3,4-DClPh | Et | H | 1Et-5-Idz | C | | 494 (M⁺+1) |
| Ca-b-355 | 1-a | Ca-b-354 | | 3,4-DClPh | H | H | 1Et-5-Idz | C | | 466 (M⁺+1) |
| Ca-b-356 | | Ca-a-470 | | 2,6-DMePh | H | H | 1Et-5-Idz | C | | 425 (M⁺+1) |
| Ca-b-357 | 5-e | Ca-b-356 | BRA58 | 2-Furan | Et | H | 1Et-5-Idz | C | | 415 (M⁺+1) |
| Ca-b-358 | 1-a | Ca-b-357 | | 2-Furan | H | H | 1Et-5-Idz | C | | 387 (M⁺+1) |
| Ca-b-359 | 5-e | Ca-b-358 | BRA61 | 3-Thiophene | Et | H | 1Et-5-Idz | C | | 431 (M⁺+1) |
| Ca-b-360 | 1-a | Ca-b-359 | | 3-Thiophene | H | H | 1Et-5-Idz | C | | 403 (M⁺+1) |

**[Table 110]**

| Table-Ca-B-9 | | | | | | | | | | |
|---|---|---|---|---|---|---|---|---|---|---|
| Exp. | Syn. | SM1 | Reagent | Rx | Y | V₁' | V₂' | LCMS | | |
| | | | | | | | | method | RTime | Mass |
| Ca-b-361 | | Ca-a-476 | | 4-Pyridine | H | H | 1Et-5-Idz | C | | 398 (M⁺+1) |
| Ca-b-362 | | Ca-a-478 | | 4-PhPh | H | H | 1Et-5-Idz | C | | 473 (M⁺+1) |
| Ca-b-363 | 5-e | Int.c-b-6 | BRA69 | 2cHexCH=CH- | Et | H | 1Et-5-Idz | C | | 457 (M⁺+1) |
| Ca-b-364 | 1-a | Ca-b-363 | | 2cHexCH=CH- | H | H | 1Et-5-Idz | C | | 429 (M⁺+1) |
| Ca-b-365 | 9-j | Ca-b-363 | | 2cHexC₂H₄- | Et | H | 1Et-5-Idz | C | | 459 (M⁺+1) |
| Ca-b-366 | 1-a | Ca-b-365 | | 2cHexC₂H₄- | H | H | 1Et-5-Idz | C | | 431 (M⁺+1) |
| Ca-b-367 | 5-e | Int.c-b-6 | BRA71 | 4MePhCH=CH- | Et | H | 1Et-5-Idz | C | | 465 (M⁺+1) |
| Ca-b-368 | 1-a | Ca-b-367 | | 4MePhCH=CH- | H | H | 1Et-5-Idz | C | | 437 (M⁺+1) |
| Ca-b-369 | 5-e | Ca-b-367 | | 4MePhC₂H₄- | Et | H | 1Et-5-Idz | C | | 467 (M⁺+1) |
| Ca-b-370 | 1-a | Ca-b-369 | | 4MePhC₂H₄- | H | H | 1Et-5-Idz | C | | 439 (M⁺+1) |
| Ca-b-371 | | Ca-a-492 | | Ph | H | H | 5-BF | C | | 369 (M⁺+1) |
| Ca-b-372 | | Ca-a-494 | | 3-MePh | H | H | 5-BF | C | | 383 (M⁺+1) |
| Ca-b-373 | | Ca-a-498 | | 2-iPrPh | H | H | 5-BF | C | | 411 (M⁺+1) |
| Ca-b-374 | | Ca-a-500 | | 4-tBuPh | H | H | 5-BF | C | | 425 (M⁺+1) |
| Ca-b-375 | 5-e | Int.c-b-7 | BRA25 | 3-MeOPh | Et | H | 5-BF | C | | 427 (M⁺+1) |
| Ca-b-376 | 1-a | Ca-b-375 | | 3-MeOPh | H | H | 5-BF | C | | 399 (M⁺+1) |
| Ca-b-377 | 5-e | Int.c-b-7 | BRA27 | 2-EtOPh | Et | H | 5-BF | C | | 441 (M⁺+1) |
| Ca-b-378 | 1-a | Ca-b-377 | | 2-EtOPh | H | H | 5-BF | C | | 413 (M⁺+1) |
| Ca-b-379 | | Ca-a-506 | | 3-nPrOPh | H | H | 5-BF | C | | 427 (M⁺+1) |
| Ca-b-380 | | Ca-a-508 | | 3-nBuOPh | H | H | 5-BF | C | | 441 (M+ +1) |
| Ca-b-381 | | Ca-a-510 | | 3-DMAPh | H | H | 5-BF | C | | 412 (M⁺+1) |
| Ca-b-382 | 5-e | Int.c-b-7 | BRA36 | 4-DMAPh | Et | H | 5-BF | C | | 412 (M⁺+1) |
| Ca-b-383 | 1-a | Ca-b-382 | | 4-DMAPh | H | H | 5-BF | C | | 440 (M⁺+1) |
| Ca-b-384 | | Ca-a-512 | | 2-FPh | H | H | 5-BF | C | | 387 (M⁺+1) |
| Ca-b-385 | | Ca-a-514 | | 4-FPh | H | H | 5-BF | C | | 387 (M⁺+1) |
| Ca-b-386 | 5-e | Int.c-b-7 | BRA42 | 4-ClPh | Et | H | 5-BF | C | | 403 (M⁺+1) |
| Ca-b-387 | 1-a | Ca-b-386 | | 4-ClPh | H | H | 5-BF | C | | 431 (M+ +1) |
| Ca-b-388 | 5-e | Int.c-b-7 | BRA45 | 4-CF3Ph | Et | H | 5-BF | C | | 465 (M⁺+1) |
| Ca-b-389 | 1-a | Ca-b-388 | | 4-CF3Ph | H | H | 5-BF | C | | 437 (M⁺+1) |
| Ca-b-390 | 5-e | Int.c-b-7 | BRA47 | 2,4-DFPh | Et | H | 5-BF | C | | 433 (M⁺+1) |
| Ca-b-391 | 1-a | Ca-b-390 | | 2,4-DFPh | H | H | 5-BF | C | | 405 (M⁺+1) |
| Ca-b-392 | 5-e | Int.c-b-7 | BRA49 | 2.6-DFPh | Et | H | 5-BF | C | | 433 (M⁺+1) |
| Ca-b-393 | 1-a | Ca-b-392 | | 2,6-DFPh | H | H | 5-BF | C | | 405 (M⁺+1) |
| Ca-b-394 | | Ca-a-524 | | 3,4-DFPh | H | H | 5-BF | C | | 405 (M⁺+1) |
| Ca-b-395 | | Ca-a-526 | | 3,5-DCIPh | H | H | 5-BF | C | | 438 (M⁺+1) |
| Ca-b-396 | 5-e | Int.c-b-7 | BRA57 | 2,6-DMePh | Et | H | 5-BF | C | | 425 (M⁺+1) |
| Ca-b-397 | 1-a | Ca-b-396 | | 2,6-DMePh | H | H | 5-BF | C | | 397 (M⁺+1) |
| Ca-b-398 | | Ca-a-532 | | 2-Furan | H | H | 5-BF | C | | 359 (M⁺+1) |
| Ca-b-399 | | Ca-a-534 | | 3-Thiophene | H | H | 5-BF | C | | 375 (M⁺+1) |
| Ca-b-400 | | Ca-a-538 | | Ph | H | H | 6-Qu | C | | 380 (M⁺+1) |
| Ca-b-401 | | Ca-a-542 | | 4-MePh | H | H | 6-Qu | C | | 394 (M⁺+1) |
| Ca-b-402 | 5-e | Int.c-b-8 | BRA22 | 1-nBuPh | Et | H | 6-Qu | C | | 464 (M⁺+1) |
| Ca-b-403 | 1-a | Ca-b-402 | | 1-nBuPh | H | H | 6-Qu | C | | 436 (M⁺+1) |
| Ca-b-404 | | Ca-a-546 | | 3-MeOPh | H | H | 6-Qu | C | | 410 (M⁺+1) |
| Ca-b-405 | | Ca-a-550 | | 4-iPrOPh | H | H | 6-Qu | C | | 438 (M⁺+1) |
| Ca-b-406 | 5-e | Ca-b-405 | BRA35 | 3-DMAPh | Et | H | 6-Qu | C | | 451 (M⁺+1) |

**[Table 111]**

| Table-Ca-B-10 | | | | | | | | | | |
|---|---|---|---|---|---|---|---|---|---|---|
| Exp. | Syn. | SM1 | Reagent | Rx | Y | V₁' | V₂' | LCMS | | |
| | | | | | | | | method | RTime | Mass |
| Ca-b-407 | 1-a | Ca-b-406 | | 3-DMAPh | H | H | 6-Qu | C | | 423 (M⁺+1) |
| Ca-b-408 | | Ca-a-554 | | 3-FPh | H | H | 6-Qu | C | | 398 (M⁺+1) |
| Ca-b-409 | 5-e | Ca-b-408 | BRA42 | 4-ClPh | Et | H | 6-Qu | C | | 442 (M⁺+1) |
| Ca-b-410 | 1-a | Ca-b-409 | | 4-ClPh | H | H | 6-Qu | C | | 414 (M⁺+1) |
| Ca-b-411 | 5-e | Int.c-b-8 | BRA44 | 3-CF3Ph | Et | H | 6-Qu | C | | 476 (M⁺+1) |
| Ca-b-412 | 1-a | Ca-b-411 | | 3-CF3Ph | H | H | 6-Qu | C | | 448 (M⁺+1) |
| Ca-b-413 | | Ca-a-560 | | 2,4-DFPh | H | H | 6-Qu | C | | 416 (M⁺+1) |
| Ca-b-414 | 5-e | Int.c-b-8 | BRA49 | 2,6-DFPh | Et | H | 6-Qu | C | | 444 (M⁺+1) |
| Ca-b-415 | 1-a | Ca-b-414 | | 2,6-DFPh | H | H | 6-Qu | C | | 416 (M⁺+1) |
| Ca-b-416 | | Ca-a-562 | | 3,4-DFPh | H | H | 6-Qu | C | | 416 (M⁺+1) |
| Ca-b-417 | | Ca-a-566 | | 2,4-DClPh | H | H | 6-Qu | C | | 449 (M⁺+1) |
| Ca-b-418 | 5-e | Int.c-b-8 | BRA55 | 2,3-DMePh | Et | H | 6-Qu | C | | 436 (M⁺+1) |
| Ca-b-419 | 1-a | Ca-b-418 | | 2,3-DMePh | H | H | 6-Qu | C | | 408 (M⁺+1) |
| Ca-b-420 | | Ca-a-572 | | 2-Furan | H | H | 6-Qu | C | | 370 (M⁺+1) |
| Ca-b-421 | 5-e | Int.c-b-8 | BRA59 | 3-Furan | Et | H | 6-Qu | C | | 398 (M⁺+1) |
| Ca-b-422 | 1-a | Ca-b-421 | | 3-Furan | H | H | 6-Qu | C | | 370 (M⁺+1) |
| Ca-b-423 | | Ca-a-574 | | 2-Thiophene | H | H | 6-Qu | C | | 386 (M⁺+1) |
| Ca-b-424 | 5-e | Int.c-b-8 | BRA61 | 3-Thiophene | Et | H | 6-Qu | C | | 414 (M⁺+1) |
| Ca-b-425 | 1-a | Ca-b-424 | | 3-Thiophene | H | H | 6-Qu | C | | 386 (M⁺+1) |
| Ca-b-426 | | Ca-a-576 | | 3-Pyridine | H | H | 6-Qu | C | | 381 (M⁺+1) |
| Ca-b-427 | | Ca-a-578 | | 4-PhPh | H | H | 6-Qu | C | | 456 (M⁺+1) |
| Ca-b-428 | 5-e | Int.c-b-8 | BRA69 | 2cHexCH=CH- | Et | H | 6-Qu | C | | 440 (M⁺+1) |
| Ca-b-429 | 1-a | Ca-b-428 | | 2cHexCH=CH- | H | H | 6-Qu | C | | 412 (M⁺+1) |
| Ca-b-430 | 9-j | Ca-b-428 | | 2cHexC₂H₄- | Et | H | 6-Qu | C | | 442 (M⁺+1) |
| Ca-b-431 | 1-a | Ca-b-430 | | 2cHexC₂H₄- | H | H | 6-Qu | C | | 414 (M⁺+1) |
| Ca-b-432 | | Ca-a-584 | | 4MePhCH=CH- | H | H | 6-Qu | C | | 448 (M⁺+1) |
| Ca-b-433 | | Ca-a-586 | | 4MePhC₂H₄- | H | H | 6-Qu | C | | 422 (M⁺+1) |
| Ca-b-434 | 5-e | Int.c-b-8 | BRA73 | 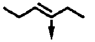 | Et | H | 6-Qu | C | | 414 (M⁺+1) |
| Ca-b-435 | 1-a | Ca-b-434 | | 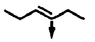 | H | H | 6-Qu | C | | 386 (M⁺+1) |
| Ca-b-436 | 5-e | Int.c-b-1 | BRA74 | Trans-CH₃CH=CH- | Et | H | 2-Nap | C | | 371 (M⁺+1) |
| Ca-b-437 | 1-a | Ca-b-436 | | Trans-CH₃CH=CH- | H | H | 2-Nap | C | | 343 (M⁺+1) |
| Ca-b-438 | 9-j | Ca-b-436 | | nPr | Et | H | 2-Nap | C | | 373 (M⁺+1) |
| Ca-b-439 | 1-a | Ca-b-438 | | nPr | H | H | 2-Nap | C | | 345 (M⁺+1) |
| Ca-b-440 | 5-e | Int.c-b-1 | BRA75 | Cis-CH₃CH=CH- | Et | H | 2-Nap | C | | 371 (M⁺+1) |
| Ca-b-441 | 1-a | Ca-b-440 | | Cis-CH₃CH=CH- | H | H | 2-Nap | C | | 343 (M⁺+1) |
| Ca-b-442 | 5-e | Int.c-b-1 | BRA76 | C₃H₇CH=CH- | Et | H | 2-Nap | C | | 399 (M⁺+1) |
| Ca-b-443 | 1-a | Ca-b-442 | | C₃H₇CH=CH- | H | H | 2-Nap | C | | 371 (M⁺+1) |
| Ca-b-444 | 9-j | Ca-b-442 | | nPen | Et | H | 2-Nap | C | | 401 (M⁺+1) |
| Ca-b-445 | 1-a | Ca-b-444 | | nPen | H | H | 2-Nap | C | | 373 (M⁺+1) |
| Ca-b-446 | 5-e | Int.c-b-2 | BRA74 | Trans-CH₃CH=CH- | Et | H | 5-Ind | C | | 360 (M⁺+1) |
| Ca-b-447 | 1-a | Ca-b-446 | | Trans-CH₃CH=CH- | H | H | 5-Ind | C | | 332 (M⁺+1) |
| Ca-b-448 | | Ca-b-446 | | nPr | H | H | 5-Ind | C | | 362 (M⁺+1) |
| Ca-b-449 | 1-a | Ca-b-448 | | nPr | H | H | 5-Ind | C | | 334 (M⁺+1) |
| Ca-b-45C | | Ca-a-604 | | C₃H₇CH=CH- | H | H | 5-Ind | C | | 360 (M⁺+1) |

**[Table 112]**

| Table-Ca-B-11 | | | | | | | | | | |
|---|---|---|---|---|---|---|---|---|---|---|
| Exp. | Syn. | SM1 | Reagent | Rx | Y | V₁' | V₂' | LCMS | | |
| | | | | | | | | method | RTime | Mass |
| Ca-b-451 | 5-e | Int.c-b-3 | BRA75 | Cis-CH₃CH=CH- | Et | H | 1Me-5-Ind | C | | 374 (M⁺+1) |
| Ca-b-452 | 1-a | Ca-b-451 | | Cis-CH₃CH=CH- | H | H | 1Me-5-Ind | C | | 346 (M⁺+1) |
| Ca-b-453 | 9-j | Ca-b-451 | | nPr | Et | H | 1Me-5-Ind | C | | 376 (M⁺+1) |
| Ca-b-454 | 1-a | Ca-b-453 | | nPr | H | H | 1Me-5-Ind | C | | 348 (M⁺+1) |
| Ca-b-455 | 5-e | Int.c-b-3 | BRA76 | C₃H₇CH=CH- | Et | H | 1Me-5-Ind | C | | 402 (M⁺+1) |
| Ca-b-456 | 1-a | Ca-b-455 | | C₃H₇CH=CH- | H | H | 1Me-5-Ind | C | | 374 (M⁺+1) |
| Ca-b-457 | 9-j | Ca-b-455 | | nPen | Et | H | 1Me-5-Ind | C | | 404 (M⁺+1) |
| Ca-b-458 | 1-a | Ca-b-457 | | nPen | H | H | 1Me-5-Ind | C | | 376 (M⁺+1) |
| Ca-b-459 | 5-e | Int.c-b-4 | BRA74 | Trans-CH₃CH=CH- | Et | H | 5-1Idz | C | | 361 (M⁺+1) |
| Ca-b-460 | 1-a | Ca-b-459 | | Trans-CH₃CH=CH- | H | H | 5-1Idz | C | | 333 (M⁺+1) |
| Ca-b-461 | 9-j | Ca-b-459 | | nPr | Et | H | 5-1Idz | C | | 363 (M⁺+1) |
| Ca-b-462 | 1-a | Ca-b-461 | | nPr | H | H | 5-1Idz | C | | 335 (M⁺+1) |
| Ca-b-463 | 5-e | Int.c-b-4 | BRA75 | Cis-CH₃CH=CH- | Et | H | 5-1Idz | C | | 361 (M⁺+1) |
| Ca-b-464 | 1-a | Ca-b-463 | | Cis-CH₃CH=CH- | H | H | 5-1Idz | C | | 333 (M⁺+1) |
| Ca-b-465 | | Ca-a-616 | | C₃H₇CH=CH- | H | H | 5-1Idz | C | | 389 (M⁺+1) |
| Ca-b-466 | | Ca-a-618 | | nPen | H | H | 5-1Idz | C | | 363 (M⁺+1) |
| Ca-b-467 | 5-e | Int.c-b-6 | BRA74 | Trans-CH₃CH=CH- | Et | H | 1Me-5-Idz | C | | 375 (M⁺+1) |
| Ca-b-468 | 1-a | Ca-b-467 | | Trans-CH3_{C}H=CH- | H | H | 1Me-5-Idz | C | | 347 (M⁺+1) |
| Ca-b-469 | 9-j | Ca-b-467 | | nPr | Et | H | 1Me-5-Idz | C | | 377 (M⁺+1) |
| Ca-b-470 | 1-a | Ca-b-469 | | nPr | H | H | 1Me-5-Idz | C | | 349 (M⁺+1) |
| Ca-b-471 | 5-e | Int.c-b-6 | BRA75 | Cis-CH₃CH=CH- | Et | H | 1Me-5-Idz | C | | 375 (M⁺+1) |
| Ca-b-472 | 1-a | Ca-b-471 | | Cis-CH₃CH=CH- | H | H | 1Me-5-Idz | C | | 347 (M⁺+1) |
| Ca-b-473 | 5-e | Int.c-b-6 | BRA76 | C₃H₇CH=CH- | Et | H | 1Me-5-Idz | C | | 403 (M⁺+1) |
| Ca-b-474 | 1-a | Ca-b-473 | | C₃H₇CH=CH- | H | H | 1Me-5-Idz | C | | 375 (M⁺+1) |
| Ca-b-475 | 9-j | Ca-b-473 | | nPen | Et | H | 1Me-5-Idz | C | | 405 (M⁺+1) |
| Ca-b-476 | 1-a | Ca-b-475 | | nPen | H | H | 1Me-5-Idz | C | | 377 (M⁺+1) |
| Ca-b-477 | | Ca-a-626 | | Trans-CH₃CH=CH- | H | H | 1Et-5-Idz | C | | 389 (M⁺+1) |
| Ca-b-478 | | Ca-a-628 | | nPr | H | H | 1Et-5-Idz | C | | 363 (M⁺+1) |
| Ca-b-479 | | Ca-a-630 | | Trans-CH₃CH=CH- | H | H | 5-BF | C | | 333 (M⁺+1) |
| Ca-b-480 | 5-e | Int.c-b-7 | BRA75 | Cis-CH₃CH=CH- | Et | H | 5-BF | C | | 361 (M⁺+1) |
| Ca-b-481 | 1-a | Ca-b-479 | | Cis-CH₃CH=CH- | H | H | 5-BF | C | | 333 (M⁺+1) |
| Ca-b-482 | 5-e | Int.c-b-7 | BRA76 | C₃H₇CH=CH- | Et | H | 5-BF | C | | 361 (M⁺+1) |
| Ca-b-483 | 1-a | Ca-b-482 | | C₃H₇CH=CH- | H | H | 5-BF | C | | 389 (M⁺+1) |
| Ca-b-484 | 5-e | Int.c-b-8 | BRA74 | Trans-CH₃CH=CH- | Et | H | 6-Qu | C | | 372 (M⁺+1) |
| Ca-b-485 | 1-a | Ca-b-483 | | Trans-CH₃CH=CH- | H | H | 6-Qu | C | | 344 (M⁺+1) |
| Ca-b-486 | 9-j | Ca-b-483 | | nPr | Et | H | 6-Qu | C | | 346 (M⁺+1) |
| Ca-b-487 | 1-a | Ca-b-486 | | nPr | H | H | 6-Qu | C | | 374 (M⁺+1) |
| Ca-b-488 | | Ca-a-636 | | C₃H₇CH=CH- | H | H | 6-Qu | C | | 372 (M⁺+1) |

**[Table 113]**

| Table-Ca-C-1 | | | | | | | | | | |
|---|---|---|---|---|---|---|---|---|---|---|
| 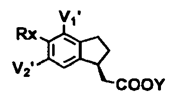 | | | | | | | | | | |

| Exp. | Syn. | SM1 | Reagent | Rx | Y | V₁' | V₂' | LCMS | | |
|---|---|---|---|---|---|---|---|---|---|---|
| | | | | | | | | method | RTime | Mass |
| Ca-c-1 | 5-e | Int.c-c-1 | BRA14 | Ph | Et | H | 2-Nap | C | | 407 (M⁺+1) |
| Ca-c-2 | 1-a | Ca-c-1 | | Ph | H | H | 2-Nap | C | | 379 (M⁺+1) |
| Ca-c-3 | 5-e | Int.c-c-1 | BRA16 | 3-MePh | Et | H | 2-Nap | C | | 421 (M⁺+1) |
| Ca-c-4 | 1-a | Ca-c-3 | | 3-MePh | H | H | 2-Nap | C | | 393 (M⁺+1) |
| Ca-c-5 | 5-e | Int.c-c-1 | BRA17 | 4-MePh | Et | H | 2-Nap | C | | 421 (M⁺+1) |
| Ca-c-6 | 1-a | Ca-c-5 | | 4-MePh | H | H | 2-Nap | C | | 393 (M⁺+1) |
| Ca-c-7 | 5-e | Int.c-c-1 | BRA18 | 2-EtPh | Et | H | 2-Nap | C | | 435 (M⁺+1) |
| Ca-c-8 | 1-a | Ca-c-7 | | 2-EtPh | H | H | 2-Nap | C | | 407 (M⁺+1) |
| Ca-c-9 | 5-e | Int.c-c-1 | BRA20 | 2-iPrPh | Et | H | 2-Nap | C | | 449 (M⁺+1) |
| Ca-c-10 | 1-a | Ca-c-9 | | 2-iPrPh | H | H | 2-Nap | C | | 421 (M⁺+1) |
| Ca-c-11 | 5-e | Int.c-c-1 | BRA21 | 4-iPrPh | Et | H | 2-Nap | C | | 449 (M⁺+1) |
| Ca-c-12 | 1-a | Ca-c-11 | | 4-iPrPh | H | H | 2-Nap | C | | 421 (M⁺+1) |
| Ca-c-13 | 5-e | Int.c-c-1 | BRA23 | 4-tBuPh | Et | H | 2-Nap | C | | 463 (M⁺+1) |
| Ca-c-14 | 1-a | Ca-c-13 | | 4-tBuPh | H | H | 2-Nap | C | | 435 (M⁺+1) |
| Ca-c-15 | 5-e | Int.c-c-1 | BRA24 | 2-MeOPh | Et | H | 2-Nap | C | | 437 (M⁺+1) |
| Ca-c-16 | 1-a | Ca-c-15 | | 2-MeOPh | H | H | 2-Nap | C | | 409 (M⁺+1) |
| Ca-c-17 | 5-e | Int.c-c-1 | BRA25 | 3-MeOPh | Et | H | 2-Nap | C | | 437 (M⁺+1) |
| Ca-c-18 | 1-a | Ca-c-17 | | 3-MeOPh | H | H | 2-Nap | C | | 409 (M⁺+1) |
| Ca-c-19 | 5-e | Int.c-c-1 | BRA27 | 2-EtOPh | Et | H | 2-Nap | C | | 451 (M⁺+1) |
| Ca-c-20 | 1-a | Ca-c-19 | | 2-EtOPh | H | H | 2-Nap | C | | 423 (M⁺+1) |
| Ca-c-21 | 5-e | Int.c-c-1 | BRA30 | 4-iPrOPh | Et | H | 2-Nap | C | | 465 (M⁺+1) |
| Ca-c-22 | 1-a | Ca-c-21 | | 4-iPrOPh | H | H | 2-Nap | C | | 437 (M⁺+1) |
| Ca-c-23 | 5-e | Int.c-c-1 | BRA31 | 3-nPrOPh | Et | H | 2-Nap | C | | 465 (M⁺+1) |
| Ca-c-24 | 1-a | Ca-c-23 | | 3-nPrOPh | H | H | 2-Nap | C | | 437 (M⁺+1) |
| Ca-c-25 | 5-e | Int.c-c-1 | BRA33 | 3-nBuOPh | Et | H | 2-Nap | C | | 479 (M⁺+1) |
| Ca-c-26 | 1-a | Ca-c-25 | | 3-nBuOPh | H | H | 2-Nap | C | | 451 (M⁺+1) |
| Ca-c-27 | 5-e | Int.c-c-1 | BRA36 | 4-DMAPh | Et | H | 2-Nap | C | | 450 (M⁺+1) |
| Ca-c-28 | 1-a | Ca-c-27 | | 4-DMAPh | H | H | 2-Nap | C | | 422 (M⁺+1) |
| Ca-c-29 | 5-e | Int.c-c-1 | BRA37 | 2-FPh | Et | H | 2-Nap | C | | 425 (M⁺+1) |
| Ca-c-30 | 1-a | Ca-c-29 | | 2-FPh | H | H | 2-Nap | C | | 397 (M⁺+1) |
| Ca-c-31 | 5-e | Int.c-c-1 | BRA38 | 3-FPh | Et | H | 2-Nap | C | | 425 (M⁺+1) |
| Ca-c-32 | 1-a | Ca-c-31 | | 3-FPh | H | H | 2-Nap | C | | 397 (M⁺+1) |
| Ca-c-33 | 5-e | Int.c-c-1 | BRA41 | 3-ClPh | Et | H | 2-Nap | C | | 441 (M⁺+1) |
| Ca-c-34 | 1-a | Ca-c-33 | | 3-ClPh | H | H | 2-Nap | C | | 413 (M⁺+1) |
| Ca-c-35 | | Ca-a-40 | | 4-ClPh | H | H | 2-Nap | C | | 413 (M⁺+1) |
| Ca-c-36 | | Ca-a-42 | | 3-CF3Ph | H | H | 2-Nap | C | | 447 (M⁺+1) |
| Ca-c-37 | 5-e | Int.c-c-1 | BRA48 | 2,5-DFPh | Et | H | 2-Nap | C | | 443 (M⁺+1) |
| Ca-c-38 | 1-a | Ca-c-37 | | 2,5-DFPh | H | H | 2-Nap | C | | 415 (M⁺+1) |
| Ca-c-39 | | Ca-a-48 | | 2.6-DFPh | H | H | 2-Nap | C | | 415 (M⁺+1) |
| Ca-c-40 | | Ca-a-54 | | 2,4-DClPh | H | H | 2-Nap | C | | 448 (M⁺+1) |
| Ca-c-41 | 5-e | Int.c-c-1 | BRA56 | 2,4-DMePh | Et | H | 2-Nap | C | | 435 (M⁺+1) |
| Ca-c-42 | 1-a | Ca-c-41 | | 2,4-DMePh | H | H | 2-Nap | C | | 407 (M⁺+1) |
| Ca-c-43 | 5-e | Int.c-c-1 | BRA58 | 2-Furan | Et | H | 2-Nap | C | | 397 (M⁺+1) |
| Ca-c-44 | 1-a | Ca-c-43 | | 2-Furan | H | H | 2-Nap | C | | 369 (M⁺+1) |

**[Table 114]**

| Table-Ca-C-2 | | | | | | | | | | |
|---|---|---|---|---|---|---|---|---|---|---|
| Exp. | Syn | SM1 | Reagent | Rx | Y | V₁' | V₂' | LCMS | | |
| | | | | | | | | method | RTime | Mass |
| Ca-c-45 | | Ca-a-64 | | 3-Furan | H | H | 2-Nap | C | | 369 (M⁺+1) |
| Ca-c-46 | 5-e | Int.c-c-1 | BRA60 | 2-Thiophene | Et | H | 2-Nap | C | | 413 (M⁺+1) |
| Ca-c-47 | 1-a | Ca-c-46 | | 2-Thiophene | H | H | 2-Nap | C | | 385 (M⁺+1) |
| Ca-c-48 | | Ca-a-68 | | 3-Thiophene | H | H | 2-Nap | C | | 385 (M⁺+1) |
| Ca-c-49 | 5-e | Int.c-c-1 | BRA62 | 3-Pyridine | Et | H | 2-Nap | C | | 408 (M⁺+1) |
| Ca-c-50 | 1-a | Ca-c-49 | | 3-Pyridine | H | H | 2-Nap | C | | 380 (M⁺+1) |
| Ca-c-51 | 5-e | Int.c-c-1 | BRA65 | 4-PhPh | Et | H | 2-Nap | C | | 483 (M⁺+1) |
| Ca-c-52 | 1-a | Ca-c-51 | | 4-PhPh | H | H | 2-Nap | C | | 455 (M⁺+1) |
| Ca-c-53 | 5-e | Int.c-c-1 | BRA66 | C₄H₉CH=CH- | Et | H | 2-Nap | C | | 413 (M⁺+1) |
| Ca-c-54 | 1-a | Ca-c-53 | | C₄H₉CH=CH- | H | H | 2-Nap | C | | 385 (M⁺+1) |
| Ca-c-55 | 9-j | Ca-c-53 | | nHex- | Et | H | 2-Nap | C | | 415 (M⁺+1) |
| Ca-c-56 | 1-a | Ca-c-55 | | nHex- | H | H | 2-Nap | C | | 387 (M⁺+1) |
| Ca-c-57 | 5-e | Int.c-c-1 | BRA67 | 4FPhCH=CH- | Et | H | 2-Nap | C | | 451 (M⁺+1) |
| Ca-c-58 | 1-a | Ca-c-57 | | 4FPhCH=CH- | H | H | 2-Nap | C | | 423 (M⁺+1) |
| Ca-c-59 | 9-j | Ca-c-57 | | 4FPhC₂H₄- | Et | H | 2-Nap | C | | 453 (M⁺+1) |
| Ca-c-60 | 1-a | Ca-c-59 | | 4FPhC₂H₄- | H | H | 2-Nap | C | | 425 (M⁺+1) |
| Ca-c-61 | 5-e | Int.c-c-1 | BRA69 | 2cHexCH=CH- | Et | H | 2-Nap | C | | 439 (M⁺+1) |
| Ca-c-62 | 1-a | Ca-c-61 | | 2cHexCH=CH- | H | H | 2-Nap | C | | 411 (M⁺+1) |
| Ca-c-63 | 9-j | Ca-c-61 | | 2cHexC₂H₄- | Et | H | 2-Nap | C | | 441 (M⁺+1) |
| Ca-c-64 | 1-a | Ca-c-63 | | 2cHexC₂H₄- | H | H | 2-Nap | C | | 413 (M⁺+1) |
| Ca-c-65 | 5-e | Int.c-c-1 | BRA73 | 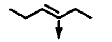 | Et | H | 2-Nap | C | | 413 (M⁺+1) |
| Ca-c-66 | 1-a | Ca-c-65 | | 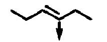 | H | H | 2-Nap | C | | 385 (M⁺+1) |
| Ca-c-67 | 9-j | Ca-c-65 | | 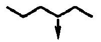 | Et | H | 2-Nap | C | | 415 (M⁺+1) |
| Ca-c-68 | 1-a | Ca-c-67 | | 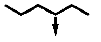 | H | H | 2-Nap | C | | 387 (M⁺+1) |
| Ca-c-69 | 5-e | Int.c-c-2 | BRA14 | Ph | Et | H | 5-Ind | C | | 396 (M⁺+1) |
| Ca-c-70 | 1-a | Ca-c-69 | | Ph | H | H | 5-Ind | C | | 368 (M⁺+1) |
| Ca-c-71 | 5-e | Int.c-c-2 | BRA17 | 4-MePh | Et | H | 5-Ind | C | | 410 (M⁺+1) |
| Ca-c-72 | 1-a | Ca-c-71 | | 4-MePh | H | H | 5-Ind | C | | 382 (M⁺+1) |
| Ca-c-73 | 5-e | Int.c-c-2 | BRA19 | 4-EtPh | Et | H | 5-Ind | C | | 424 (M⁺+1) |
| Ca-c-74 | 1-a | Ca-c-73 | | 4-EtPh | H | H | 5-Ind | C | | 396 (M⁺+1) |
| Ca-c-75 | | Ca-a-106 | | 2-iPrPh | H | H | 5-Ind | C | | 410 (M⁺+1) |
| Ca-c-76 | 5-e | Int.c-c-2 | BRA26 | 4-MeOPh | Et | H | 5-Ind | C | | 426 (M⁺+1) |
| Ca-c-77 | 1-a | Ca-c-76 | | 4-MeOPh | H | H | 5-Ind | C | | 398 (M⁺+1) |
| Ca-c-78 | 5-e | Int.c-c-2 | BRA28 | 3-EtOPh | Et | H | 5-Ind | C | | 440 (M⁺+1) |
| Ca-c-79 | 1-a | Ca-c-78 | | 3-EtOPh | H | H | 5-Ind | C | | 412 (M⁺+1) |
| Ca-c-80 | 5-e | Int.c-c-2 | BRA30 | 4-iPrOPh | Et | H | 5-Ind | C | | 454 (M⁺+1) |
| Ca-c-81 | 1-a | Ca-c-80 | | 4-iPrOPh | H | H | 5-Ind | C | | 426 (M⁺+1) |
| Ca-c-82 | 5-e | Int.c-c-2 | BRA31 | 3-nPrOPh | Et | H | 5-Ind | C | | 454 (M⁺+1) |
| Ca-c-83 | 1-a | Ca-c-82 | | 3-nPrOPh | H | H | 5-Ind | C | | 426 (M⁺+1) |
| Ca-c-84 | 5-e | Int.c-c-2 | BRA35 | 3-DMAPh | Et | H | 5-Ind | C | | 439 (M⁺+1) |
| Ca-c-85 | 1-a | Ca-c-84 | | 3-DMAPh | H | H | 5-Ind | C | | 411 (M⁺+1) |
| Ca-c-86 | | Ca-a-126 | | 2-FPh | H | H | 5-Ind | C | | 414 (M⁺+1) |
| Ca-c-87 | 5-e | Int.c-c-2 | BRA39 | 4-FPh | H | H | 5-Ind | C | | 386 (M⁺+1) |
| Ca-c-88 | 1-a | Ca-c-87 | | 4-FPh | Et | H | 5-Ind | C | | 414 (M⁺+1) |

**[Table 115]**

| Table-Ca-C-3 | | | | | | | | | | |
|---|---|---|---|---|---|---|---|---|---|---|
| Exp. | Syn | SM1 | Reagent | Rx | Y | V₁' | V₂' | LCMS | | |
| | | | | | | | | method | RTime | Mass |
| Ca-c-89 | 5-e | Int.c-c-2 | BRA41 | 3-ClPh | Et | H | 5-Ind | C | | 430 (M⁺+1) |
| Ca-c-90 | 1-a | Ca-c-89 | | 3-ClPh | H | H | 5-Ind | C | | 402 (M⁺+1) |
| Ca-c-91 | 5-e | Int.c-c-2 | BRA41 | 3-ClPh | Et | H | 5-Ind | C | | 430 (M⁺+1) |
| Ca-c-92 | 1-a | Ca-c-91 | | 3-ClPh | H | H | 5-Ind | C | | 402 (M⁺+1) |
| Ca-c-93 | 5-e | Intc-c-2 | BRA44 | 3-CF3Ph | Et | H | 5-Ind | C | | 464 (M⁺+1) |
| Ca-c-94 | 1-a | Ca-c-93 | | 3-CF3Ph | H | H | 5-Ind | C | | 436 (M⁺+1) |
| Ca-c-95 | 5-e | Int.c-c-2 | BRA46 | 2,3-DFPh | Et | H | 5-Ind | C | | 432 (M⁺+1) |
| Ca-c-96 | 1-a | Ca-c-95 | | 2.3-DFPh | H | H | 5-Ind | C | | 404 (M⁺+1) |
| Ca-c-97 | 5-e | Int.c-c-2 | BRA48 | 2,5-DFPh | Et | H | 5-Ind | C | | 432 (M⁺+1) |
| Ca-c-98 | 1-a | Ca-c-97 | | 2,5-DFPh | H | H | 5-Ind | C | | 404 (M⁺+1) |
| Ca-c-99 | 5-e | Int.c-c-2 | BRA50 | 3,4-DFPh | Et | H | 5-Ind | C | | 432 (M⁺+1) |
| Ca-c-100 | 1-a | Ca-c-99 | | 3,4-DFPh | H | H | 5-Ind | C | | 404 (M⁺+1) |
| Ca-c-101 | | Ca-a-144 | | 3,5-DClPh | H | H | 5-Ind | C | | 437 (M⁺+1) |
| Ca-c-102 | 5-e | Intc-c-2 | BRA54 | 3,4-DClPh | Et | H | 5-Ind | C | | 465 (M⁺+1) |
| Ca-c-103 | 1-a | Ca-c-102 | | 3,4-DClPh | H | H | 5-Ind | C | | 437 (M⁺+1) |
| Ca-c-104 | 5-e | Intc-c-2 | BRA56 | 2,4-DMePh | Et | H | 5-Ind | C | | 424 (M⁺+1) |
| Ca-c-105 | 1-a | Ca-c-104 | | 2,4-DMePh | H | H | 5-Ind | C | | 396 (M⁺+1) |
| Ca-c-106 | 5-e | Intc-c-2 | BRA58 | 2-Furan | Et | H | 5-Ind | C | | 386 (M⁺+1) |
| Ca-c-107 | 1-a | Ca-c-106 | | 2-Furan | H | H | 5-Ind | C | | 358 (M⁺+1) |
| Ca-c-108 | 5-e | Int.c-c-2 | BRA59 | 3-Furan | Et | H | 5-Ind | C | | 386 (M⁺+1) |
| Ca-c-109 | 1-a | Ca-c-108 | | 3-Furan | H | H | 5-Ind | C | | 358 (M⁺+1) |
| Ca-c-110 | 5-e | Intc-c-2 | BRA60 | 2-Thiophene | Et | H | 5-Ind | C | | 402 (M⁺+1) |
| Ca-c-111 | 1-a | Ca-c-110 | | 2-Thiophene | H | H | 5-Ind | C | | 374 (M⁺+1) |
| Ca-c-112 | | Ca-a-158 | | 3-Thiophene | H | H | 5-Ind | C | | 374 (M⁺+1) |
| Ca-c-113 | 5-e | Intc-c-2 | BRA62 | 3-Pyridine | Et | H | 5-Ind | C | | 397 (M⁺+1) |
| Ca-c-114 | 1-a | Ca-c-113 | | 3-Pyridine | H | H | 5-Ind | C | | 369 (M⁺+1) |
| Ca-c-115 | 5-e | Intc-c-2 | BRA65 | 4-PhPh | Et | H | 5-Ind | C | | 472 (M⁺+1) |
| Ca-c-116 | 1-a | Ca-c-115 | | 4-PhPh | H | H | 5-Ind | C | | 444 (M⁺+1) |
| Ca-c-117 | 5-e | Intc-c-2 | BRA66 | C₄H₉CH=CH- | Et | H | 5-Ind | C | | 402 (M⁺+1) |
| Ca-c-118 | 1-a | Ca-c-116 | | C₄H₉CH=CH- | H | H | 5-Ind | C | | 374 (M⁺+1) |
| Ca-c-119 | 9-j | Ca-c-116 | | nHex- | Et | H | 5-Ind | C | | 404 (M⁺+1) |
| Ca-c-120 | 1-a | Ca-c-120 | | nHex- | H | H | 5-Ind | C | | 376 (M⁺+1) |
| Ca-c-121 | 5-e | Int.c-c-2 | BRA67 | 4FPhCH=CH- | Et | H | 5-Ind | C | | 440 (M⁺+1) |
| Ca-c-122 | 1-a | Ca-c-122 | | 4FPhCH=CH- | H | H | 5-Ind | C | | 412 (M⁺+1) |
| Ca-c-123 | 5-e | Int.c-c-2 | BRA73 | 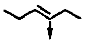 | Et | H | 5-Ind | C | | 402 (M⁺+1) |
| Ca-c-124 | 1-a | Ca-c-123 | | 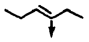 | H | H | 5-In d | C | | 374 (M⁺+1) |
| Ca-c-125 | 9-j | Ca-c-123 | | 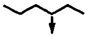 | Et | H | 5-Ind | C | | 404 (M⁺+1) |
| Ca-c-126 | 1-a | Ca-c-125 | | 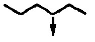 | H | H | 5-Ind | C | | 376 (M⁺+1) |
| Ca-c-127 | 5-e | Int.c-c-3 | BRA14 | Ph | Et | H | 1Me-5-Ind | C | | 410 (M⁺+1) |
| Ca-c-128 | 1-a | Ca-c-127 | | Ph | H | H | 1Me-5-Ind | C | | 382 (M⁺+1) |
| Ca-c-129 | 5-e | Int.c-c-3 | BRA17 | 4-MePh | Et | H | 1Me-5-Ind | C | | 396 (M⁺+1) |
| Ca-c-130 | 1-a | Ca-c-129 | | 4-MePh | H | H | 1Me-5-Ind | C | | 396 (M⁺+1) |
| Ca-c-131 | 5-e | Int.c-c-3 | BRA18 | 2-EtPh | Et | H | 1Me-5-Ind | C | | 438 (M⁺+1) |
| Ca-c-132 | 1-a | Ca-c-131 | | 2-EtPh | H | H | 1Me-5-Ind | C | | 410 (M⁺+1) |

**[Table 116]**

| Table-Ca-C-4 | | | | | | | | | | |
|---|---|---|---|---|---|---|---|---|---|---|
| Exp. | Syn | SM1 | Reagent | Rx | Y | V₁' | V₂' | LCMS | | |
| | | | | | | | | method | RTime | Mass |
| Ca-c-133 | 5-e | Int.c-c-3 | BRA22 | 1-nBuPh | Et | H | 1Me-5-Ind | C | | 466 (M⁺+1) |
| Ca-c-134 | 1-a | Ca-c-133 | | 1-nBuPh | H | H | 1Me-5-Ind | C | | 438 (M⁺+1) |
| Ca-c-135 | 5-e | Int.c-c-3 | BRA25 | 3-MeOPh | Et | H | 1Me-5-Ind | C | | 440 (M⁺+1) |
| Ca-c-136 | 1-a | Ca-c-135 | | 3-MeOPh | H | H | 1Me-5-Ind | C | | 412 (M⁺+1) |
| Ca-c-137 | | Ca-a-188 | | 2-EtOPh | H | H | 1Me-5-Ind | C | | 426 (M⁺+1) |
| Ca-c-138 | 5-e | Int.c-c-3 | BRA30 | 4-iPrOPh | Et | H | 1Me-5-Ind | C | | 468 (M⁺+1) |
| Ca-c-139 | 1-a | Ca-c-138 | | 4-iPrOPh | H | H | 1Me-5-Ind | C | | 440 (M⁺+1) |
| Ca-c-14C | 5-e | Int.c-c-3 | BRA31 | 3-nPrOPh | Et | H | 1Me-5-Ind | C | | 468 (M⁺+1) |
| Ca-c-141 | 1-a | Ca-c-140 | | 3-nPrOPh | H | H | 1Me-5-Ind | C | | 440 (M⁺+1) |
| Ca-c-142 | 5-e | Int.c-c-3 | BRA34 | 4-nBuOPh | Et | H | 1Me-5-Ind | C | | 482 (M⁺+1) |
| Ca-c-143 | 1-a | Ca-c-142 | | 4-nBuOPh | H | H | 1Me-5-Ind | C | | 454 (M⁺+1) |
| Ca-c-144 | 5-e | Int.c-c-3 | BRA36 | 4-DMAPh | Et | H | 1Me-5-Ind | C | | 453 (M⁺+1) |
| Ca-c-145 | 1-a | Ca-c-144 | | 4-DMAPh | H | H | 1Me-5-Ind | C | | 425 (M⁺+1) |
| Ca-c-146 | 5-e | Int.c-c-3 | BRA38 | 3-FPh | Et | H | 1Me-5-Ind | C | | 428 (M⁺+1) |
| Ca-c-147 | 1-a | Ca-c-146 | | 3-FPh | H | H | 1Me-5-Ind | C | | 400 (M⁺+1) |
| Ca-c-148 | 5-e | Int.c-c-3 | BRA39 | 4-FPh | Et | H | 1Me-5-Ind | C | | 428 (M⁺+1) |
| Ca-c-149 | 1-a | Ca-c-148 | | 4-FPh | H | H | 1Me-5-Ind | C | | 400 (M⁺+1) |
| Ca-c-15C | 5-e | Int.c-c-3 | BRA40 | 2-ClPh | Et | H | 1Me-5-Ind | C | | 444 (M⁺+1) |
| Ca-c-151 | 1-a | Ca-c-150 | | 2-ClPh | H | H | 1Me-5-Ind | C | | 416 (M⁺+1) |
| Ca-c-152 | 5-e | Int.c-c-3 | BRA45 | 4-CF3Ph | Et | H | 1Me-5-Ind | C | | 478 (M⁺+1) |
| Ca-c-153 | 1-a | Ca-c-152 | | 4-CF3Ph | H | H | 1Me-5-Ind | C | | 450 (M⁺+1) |
| Ca-c-154 | | Ca-a-212 | | 2,4-DFPh | H | H | 1Me-5-Ind | C | | 418 (M⁺+1) |
| Ca-c-155 | 5-e | Int.c-c-3 | BRA49 | 2,6-DFPh | Et | H | 1Me-5-Ind | C | | 446 (M⁺+1) |
| Ca-c-156 | 1-a | Ca-c-155 | | 2,6-DFPh | H | H | 1Me-5-Ind | C | | 418 (M⁺+1) |
| Ca-c-157 | 5-e | Int.c-c-3 | BRA51 | 3,5-DClPh | Et | H | 1Me-5-Ind | C | | 479 (M⁺+1) |
| Ca-c-158 | 1-a | Ca-c-157 | | 3,5-DClPh | H | H | 1Me-5-Ind | C | | 451 (M⁺+1) |
| Ca-c-159 | 5-e | Int.c-c-3 | BRA52 | 2,4-DClPh | Et | H | 1Me-5-Ind | C | | 479 (M⁺+1) |
| Ca-c-16C | 1-a | Ca-c-159 | | 2,4-DClPh | H | H | 1Me-5-Ind | C | | 451 (M⁺+1) |
| Ca-c-161 | 5-e | Int.c-c-3 | BRA54 | 3,4-DClPh | Et | H | 1Me-5-Ind | C | | 479 (M⁺+1) |
| Ca-c-162 | 1-a | Ca-c-161 | | 3,4-DClPh | H | H | 1Me-5-Ind | C | | 451 (M⁺+1) |
| Ca-c-163 | 5-e | Int.c-c-3 | BRA55 | 2,3-DMePh | Et | H | 1Me-5-Ind | C | | 438 (M⁺+1) |
| Ca-c-164 | 1-a | Ca-c-163 | | 2,3-DMePh | H | H | 1Me-5-Ind | C | | 410 (M⁺+1) |
| Ca-c-165 | | Ca-a-224 | | 2,4-DMePh | H | H | 1Me-5-Ind | C | | 410 (M⁺+1) |
| Ca-c-166 | 5-e | Int.c-c-3 | BRA58 | 2-Furan | Et | H | 1Me-5-Ind | C | | 400 (M⁺+1) |
| Ca-c-167 | 1-a | Ca-c-166 | | 2-Furan | H | H | 1Me-5-Ind | C | | 372 (M⁺+1) |
| Ca-c-168 | 5-e | Int.c-c-3 | BRA59 | 3-Furan | Et | H | 1Me-5-Ind | C | | 400 (M⁺+1) |
| Ca-c-169 | 1-a | Ca-c-168 | | 3-Furan | H | H | 1Me-5-Ind | C | | 372 (M⁺+1) |
| Ca-c-170 | 5-e | Int.c-c-3 | BRA60 | 2-Thiophene | Et | H | 1Me-5-Ind | C | | 416 (M⁺+1) |
| Ca-c-171 | 1-a | Ca-c-170 | | 2-Thiophene | H | H | 1Me-5-Ind | C | | 388 (M⁺+1) |
| Ca-c-172 | 5-e | Int.c-c-3 | BRA61 | 3-Thiophene | Et | H | 1Me-5-Ind | C | | 416 (M⁺+1) |
| Ca-c-173 | 1-a | Ca-c-172 | | 3-Thiophene | H | H | 1Me-5-Ind | C | | 388 (M⁺+1) |
| Ca-c-174 | | Ca-a-234 | | 4-Pyridine | H | H | 1Me-5-Ind | C | | 383 (M⁺+1) |
| Ca-c-175 | 5-e | Int.c-c-3 | BRA64 | 3-PhPh | Et | H | 1Me-5-Ind | C | | 486 (M⁺+1) |
| Ca-c-176 | 1-a | Ca-c-175 | | 3-PhPh | H | H | 1Me-5-Ind | C | | 458 (M⁺+1) |
| Ca-c-177 | | Ca-a-238 | | 4FPhCH=CH- | H | H | 1Me-5-Ind | C | | 426 (M⁺+1) |
| Ca-c-178 | | Ca-a-240 | | 4FPhC₂H₄- | H | H | 1Me-5-Ind | C | | 428 (M⁺+1) |

**[Table 117]**

| Table-Ca-C-5 | | | | | | | | | | |
|---|---|---|---|---|---|---|---|---|---|---|
| Exp. | Syn. | SM1 | Reagent | Rx | Y | V₁' | V₂' | LCMS | | |
| | | | | | | | | method | RTime | Mass |
| Ca-c-179 | 5-e | Int.c-c-3 | BRA70 | 4CF₃PhCH=CH- | Et | H | 1Me-5-Ind | C | | 504 (M⁺+1) |
| Ca-c-18C | 1-a | Ca-c-179 | | 4CF₃PhCH=CH- | H | H | 1Me-5-Ind | C | | 476 (M⁺+1) |
| Ca-c-181 | 5-e | Int.c-c-3 | BRA73 | 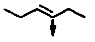 | Et | H | 1Me-5-Ind | C | | 416 (M⁺+1) |
| Ca-c-182 | 1-a | Ca-c-181 | | 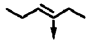 | H | H | 1Me-5-Ind | C | | 388 (M⁺+1) |
| Ca-c-183 | 9-j | Ca-c-181 | | 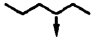 | Et | H | 1Me-5-Ind | C | | 418 (M⁺+1) |
| Ca-c-184 | 1-a | Ca-c-183 | | 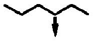 | H | H | 1Me-5-Ind | C | | 390 (M⁺+1) |
| Ca-c-185 | | Ca-a-252 | | Ph | H | H | 5-1Idz | C | | 369 (M⁺+1) |
| Ca-c-186 | 5-e | Int.c-c-4 | BRA16 | 3-MePh | Et | H | 5-1Idz | C | | 411 (M⁺+1) |
| Ca-c-187 | 1-a | Ca-c-186 | | 3-MePh | H | H | 5-1Idz | C | | 383 (M⁺+1) |
| Ca-c-188 | 5-e | Int.c-c-4 | BRA19 | 4-EtPh | Et | H | 5-1Idz | C | | 425 (M⁺+1) |
| Ca-c-189 | 1-a | Ca-c-188 | | 4-EtPh | H | H | 5-1Idz | C | | 397 (M⁺+1) |
| Ca-c-190 | | Ca-a-260 | | 2-iPrPh | H | H | 5-1Idz | C | | 411 (M⁺+1) |
| Ca-c-191 | 5-e | Int.c-c-4 | BRA23 | 4-tBuPh | Et | H | 5-1Idz | C | | 453 (M⁺+1) |
| Ca-c-192 | 1-a | Ca-c-191 | | 4-tBuPh | H | H | 5-1Idz | C | | 425 (M⁺+1) |
| Ca-c-193 | 5-e | Int.c-c-4 | BRA24 | 2-MeOPh | Et | H | 5-1Idz | C | | 427 (M⁺+1) |
| Ca-c-194 | 1-a | Ca-c-193 | | 2-MeOPh | H | H | 5-1Idz | C | | 399 (M⁺+1) |
| Ca-c-195 | 5-e | Int.c-c-4 | BRA28 | 3-EtOPh | Et | H | 5-1Idz | C | | 441 (M⁺+1) |
| Ca-c-196 | 1-a | Ca-c-195 | | 3-EtOPh | H | H | 5-1Idz | C | | 413 (M⁺+1) |
| Ca-c-197 | 5-e | Int.c-c-4 | BRA30 | 4-iPrOPh | Et | H | 5-1Idz | C | | 455 (M⁺+1) |
| Ca-c-198 | 1-a | Ca-c-197 | | 4-iPrOPh | H | H | 5-1Idz | C | | 427 (M⁺+1) |
| Ca-c-199 | 5-e | Int.c-c-4 | BRA33 | 3-nBuOPh | Et | H | 5-1Idz | C | | 469 (M⁺+1) |
| Ca-c-200 | 1-a | Ca-c-199 | | 3-nBuOPh | H | H | 5-1Idz | C | | 441 (M⁺+1) |
| Ca-c-201 | | Ca-a-276 | | 4-DMAPh | H | H | 5-1Idz | C | | 412 (M⁺+1) |
| Ca-c-202 | 5-e | Int.c-c-4 | BRA38 | 3-FPh | Et | H | 5-1Idz | C | | 415 (M⁺+1) |
| Ca-c-203 | 1-a | Ca-c-202 | | 3-FPh | H | H | 5-1Idz | C | | 387 (M⁺+1) |
| Ca-c-204 | 5-e | Int.c-c-4 | BRA42 | 4-ClPh | Et | H | 5-1Idz | C | | 431 (M⁺+1) |
| Ca-c-205 | 1-a | Ca-c-204 | | 4-ClPh | H | H | 5-1Idz | C | | 403 (M⁺+1) |
| Ca-c-206 | 5-e | Int.c-c-4 | BRA43 | 2-CF3Ph | Et | H | 5-1Idz | C | | 465 (M⁺+1) |
| Ca-c-207 | 1-a | Ca-c-206 | | 2-CF3Ph | H | H | 5-1Idz | C | | 437 (M⁺+1) |
| Ca-c-208 | 5-e | Int.c-c-4 | BRA44 | 3-CF3Ph | Et | H | 5-1Idz | C | | 465 (M⁺+1) |
| Ca-c-209 | 1-a | Ca-c-208 | | 3-CF3Ph | H | H | 5-1Idz | C | | 437 (M⁺+1) |
| Ca-c-210 | 5-e | Int.c-c-4 | BRA46 | 2,3-DFPh | Et | H | 5-1Idz | C | | 433 (M⁺+1) |
| Ca-c-211 1 | 1-a | Ca-c-210 | | 2,3-DFPh | H | H | 5-1Idz | C | | 405 (M⁺+1) |
| a-c-212 | 5-e | Int.c-c-4 | BRA47 | 2,4-DFPh | Et | H | 5-1Idz | C | | 433 (M⁺+1) |
| Ca-c-213 | 1-a | Ca-c-212 | | 2,4-DFPh | H | H | 5-1Idz | C | | 405 (M⁺+1) |
| Ca-c-214 | | Ca-a-294 | | 2,6-DFPh | H | H | 5-1Idz | C | | 405 (M⁺+1) |
| Ca-c-215 | | Ca-a-298 | | 2,4-DClPh | H | H | 5-1Idz | C | | 438 (M⁺+1) |
| Ca-c-216 | | Ca-a-302 | | 3,4-DClPh | H | H | 5-1Idz | C | | 438 (M⁺+1) |
| Ca-c-217 | 5-e | Int.c-c-4 | BRA58 | 2-Furan | Et | H | 5-1Idz | C | | 387 (M⁺+1) |
| Ca-c-218 | 1-a | Ca-c-217 | | 2-Furan | H | H | 5-1Idz | C | | 359 (M⁺+1) |
| Ca-c-219 | 5-e | Int.c-c-4 | BRA59 | 3-Furan | Et | H | 5-1Idz | C | | 387 (M⁺+1) |
| Ca-c-22C | 1-a | Ca-c-219 | | 3-Furan | H | H | 5-1Idz | C | | 359 (M⁺+1) |
| Ca-c-221 | 5-e | Int.c-c-4 | BRA60 | 2-Thiophene | Et | H | 5-1Idz | C | | 403 (M⁺+1) |
| Ca-c-222 | 1-a | Ca-c-221 | | 2-Thiophene | H | H | 5-1Idz | C | | 375 (M⁺+1) |

**[Table 118]**

| Table-Ca-C-6 | | | | | | | | | | |
|---|---|---|---|---|---|---|---|---|---|---|
| Exp. | Syn. | SM1 | Reagent | Rx | Y | V₁' | V₂ | LCMS | | |
| | | | | | | | | method | RTime | Mass |
| Ca-c-223 | 5-e | Intc-c-4 | BRA61 | 3-Thiophene | Et | H | 5-1Idz | C | | 403 (M⁺+1) |
| Ca-c-224 | 1-a | Ca-c-223 | | 3-Thiophene | H | H | 5-1Idz | C | | 375 (M⁺+1) |
| Ca-c-225 | | Ca-a-316 | | 3-Pyridine | H | H | 5-1Idz | C | | 370 (M⁺+1) |
| Ca-c-226 | | Ca-a-318 | | 4-Pyridine | H | H | 5-1Idz | C | | 370 (M⁺+1) |
| Ca-c-227 | 5-e | Intc-c-4 | BRA66 | C₄H₉CH=CH- | Et | H | 5-1Idz | C | | 403 (M⁺+1) |
| Ca-c-228 | 1-a | Ca-c-227 | | C₄H₉CH=CH- | H | H | 5-1Idz | C | | 375 (M⁺+1) |
| Ca-c-229 | 9-j | Ca-c-227 | | nHex- | Et | H | 5-1Idz | C | | 405 (M⁺+1) |
| Ca-c-230 | 1-a | Ca-c-229 | | nHex- | H | H | 5-1Idz | C | | 377 (M⁺+1) |
| Ca-c-231 | 5-e | Int.c-c-4 | BRA67 | 4FPhCH=CH- | Et | H | 5-1Idz | C | | 441 (M⁺+1) |
| Ca-c-232 | 1-a | Ca-c-231 | | 4FPhCH=CH- | H | H | 5-1Idz | C | | 413 (M⁺+1) |
| Ca-c-233 | 9-j | Ca-c-231 | | 4FPhC₂H₄- | Et | H | 5-1Idz | C | | 443 (M⁺+1) |
| Ca-c-234 | 1-a | Ca-c-233 | | 4FPhC₂H₄- | H | H | 5-1Idz | C | | 415 (M⁺+1) |
| Ca-c-235 | 5-e | Intc-c-4 | BRA68 | 4ClPhCH=CH- | Et | H | 5-1Idz | C | | 457 (M⁺+1) |
| Ca-c-236 | 1-a | Ca-c-235 | | 4ClPhCH=CH- | H | H | 5-1Idz | C | | 429 (M⁺+1) |
| Ca-c-237 | 5-e | Int.c-c-4 | BRA69 | 2cHexCH=CH- | Et | H | 5-1Idz | C | | 429 (M⁺+1) |
| Ca-c-238 | 1-a | Ca-c-237 | | 2cHexCH=CH- | H | H | 5-1Idz | C | | 401 (M⁺+1) |
| Ca-c-239 | 9-j | Ca-c-237 | | 2cHexC₂H₄- | Et | H | 5-1Idz | C | | 431 (M⁺+1) |
| Ca-c-240 | 1-a | Ca-c-239 | | 2cHexC₂H₄- | H | H | 5-1Idz | C | | 403 (M⁺+1) |
| Ca-c-241 | 5-e | Intc-c-4 | BRA70 | 4CF3PhCH=CH- | Et | H | 5-1Idz | C | | 491 (M⁺+1) |
| Ca-c-242 | 1-a | Ca-c-241 | | 4CF3PhCH=CH- | H | H | 5-1Idz | C | | 463 (M⁺+1) |
| Ca-c-243 | 9-j | Ca-c-241 | | 4CF3PhC₂H₄- | Et | H | 5-1Idz | C | | 493 (M⁺+1) |
| Ca-c-244 | 1-a | Ca-c-243 | | 4CF3PhC₂H₄- | H | H | 5-1Idz | C | | 465 (M⁺+1) |
| Ca-c-245 | | Ca-a-340 | | Ph | H | H | 1Me-5-Idz | C | | 383 (M⁺+1) |
| Ca-c-246 | 5-e | Intc-c-5 | BRA16 | 3-MePh | Et | H | 1Me-5-Idz | C | | 425 (M⁺+1) |
| Ca-c-247 | 1-a | Ca-c-246 | | 3-MePh | H | H | 1Me-5-Idz | C | | 397 (M⁺+1) |
| Ca-c-248 | 5-e | Int.c-c-5 | BRA17 | 4-MePh | Et | H | 1Me-5-Idz | C | | 425 (M⁺+1) |
| Ca-c-249 | 1-a | Ca-c-248 | | 4-MePh | H | H | 1Me-5-Idz | C | | 397 (M⁺+1) |
| Ca-c-250 | 5-e | Ca-c-249 | BRA18 | 2-EtPh | Et | H | 1Me-5-Idz | C | | 439 (M⁺+1) |
| Ca-c-251 | 1-a | Intc-c-5 | | 2-EtPh | H | H | 1Me-5-Idz | C | | 411 (M⁺+1) |
| Ca-c-252 | 5-e | Ca-c-251 | BRA21 | 4-iPrPh | Et | H | 1Me-5-Idz | C | | 453 (M⁺+1) |
| Ca-c-253 | 1-a | Intc-c-5 | | 4-iPrPh | H | H | 1Me-5-Idz | C | | 425 (M⁺+1) |
| Ca-c-254 | | Ca-a-352 | | 1-nBuPh | H | H | 1Me-5-Idz | C | | 439 (M⁺+1) |
| Ca-c-255 | 5-e | Int.c-c-5 | BRA23 | 4-tBuPh | Et | H | 1Me-5-Idz | C | | 467 (M⁺+1) |
| Ca-c-256 | 1-a | Ca-c-255 | | 4-tBuPh | H | H | 1Me-5-Idz | C | | 439 (M⁺+1) |
| Ca-c-257 | 5-e | Intc-c-5 | BRA24 | 2-MeOPh | Et | H | 1Me-5-Idz | C | | 441 (M⁺+1) |
| Ca-c-258 | 1-a | Ca-c-257 | | 2-MeOPh | H | H | 1Me-5-Idz | C | | 413 (M⁺+1) |
| Ca-c-259 | 5-e | Intc-c-5 | BRA26 | 4-MeOPh | Et | H | 1Me-5-Idz | C | | 441 (M⁺+1) |
| Ca-c-260 | 1-a | Ca-c-259 | | 4-MeOPh | H | H | 1Me-5-Idz | C | | 413 (M⁺+1) |
| Ca-c-261 | 5-e | Intc-c-5 | BRA27 | 2-EtOPh | Et | H | 1Me-5-Idz | C | | 455 (M⁺+1) |
| Ca-c-262 | 1-a | Ca-c-261 | | 2-EtOPh | H | H | 1Me-5-Idz | C | | 427 (M⁺+1) |
| Ca-c-263 | 5-e | Intc-c-5 | BRA29 | 3-iPrOPh | Et | H | 1Me-5-Idz | C | | 469 (M⁺+1) |
| Ca-c-264 | 1-a | Ca-c-263 | | 3-iPrOPh | H | H | 1Me-5-Idz | C | | 441 (M⁺+1) |
| Ca-c-265 | 5-e | Int.c-c-5 | BRA30 | 4-iPrOPh | Et | H | 1Me-5-Idz | C | | 469 (M⁺+1) |
| Ca-c-266 | 1-a | Ca-c-265 | | 4-iPrOPh | H | H | 1Me-5-Idz | C | | 441 (M⁺+1) |
| Ca-c-267 | 5-e | Int.c-c-5 | BRA31 | 3-nPrOPh | Et | H | 1Me-5-Idz | C | | 469 (M⁺+1) |
| Ca-c-268 | 1-a | Ca-c-267 | | 3-nPrOPh | H | H | 1Me-5-Idz | C | | 441 (M⁺+1) |

**[Table 119]**

| Table-Ca-C-7 | | | | | | | | | | |
|---|---|---|---|---|---|---|---|---|---|---|
| Exp. | Syn. | SM1 | Reagent | Rx | Y | V₁' | V₂' | LCMS | | |
| | | | | | | | | method | RTime | Mass |
| Ca-c-269 | 5-e | Int.c-c-5 | BRA33 | 3-nBuOPh | Et | H | 1Me-5-Idz | C | | 483 (M⁺+1) |
| Ca-c-270 | 1-a | Ca-c-269 | | 3-nBuOPh | H | H | 1Me-5-Idz | C | | 455 (M⁺+1) |
| Ca-c-271 | 5-e | Int.c-c-5 | BRA35 | 3-DMAPh | Et | H | 1Me-5-Idz | C | | 454 (M⁺+1) |
| Ca-c-272 | 1-a | Ca-c-271 | | 3-DMAPh | H | H | 1Me-5-Idz | C | | 426 (M⁺+1) |
| Ca-c-273 | | Ca-a-376 | | 2-FPh | H | H | 1Me-5-Idz | C | | 401 (M⁺+1) |
| Ca-c-274 | | Ca-a-378 | | 3-FPh | H | H | 1Me-5-Idz | C | | 401 (M⁺+1) |
| Ca-c-275 | | Ca-a-380 | | 4-FPh | H | H | 1Me-5-Idz | C | | 401 (M⁺+1) |
| Ca-c-276 | 5-e | Int.c-c-5 | BRA40 | 2-ClPh | Et | H | 1Me-5-Idz | C | | 445 (M⁺+1) |
| Ca-c-277 | 1-a | Ca-c-276 | | 2-ClPh | H | H | 1Me-5-Idz | C | | 417 (M⁺+1) |
| Ca-c-278 | 5-e | Int.c-c-5 | BRA43 | 2-CF3Ph | Et | H | 1Me-5-Idz | C | | 479 (M⁺+1) |
| Ca-c-279 | 1-a | Ca-c-278 | | 2-CF3Ph | H | H | 1Me-5-Idz | C | | 451 (M⁺+1) |
| Ca-c-28C | 5-e | Int.c-c-5 | BRA45 | 4-CF3Ph | Et | H | 1Me-5-Idz | C | | 479 (M⁺+1) |
| Ca-c-281 | 1-a | Ca-c-280 | | 4-CF3Ph | H | H | 1Me-5-Idz | C | | 451 (M⁺+1) |
| Ca-c-282 | 5-e | Int.c-c-5 | BRA47 | 2,4-DFPh | Et | H | 1Me-5-Idz | C | | 447 (M⁺+1) |
| Ca-c-283 | 1-a | Ca-c-282 | | 2,4-DFPh | H | H | 1Me-5-Idz | C | | 419 (M⁺+1) |
| Ca-c-284 | 5-e | Int.c-c-5 | BRA49 | 2,6-DFPh | Et | H | 1Me-5-Idz | C | | 447 (M⁺+1) |
| Ca-c-285 | 1-a | Ca-c-284 | | 2,6-DFPh | H | H | 1Me-5-Idz | C | | 419 (M⁺+1) |
| Ca-c-286 | 5-e | Int.c-c-5 | BRA50 | 3,4-DFPh | Et | H | 1Me-5-Idz | C | | 447 (M⁺+1) |
| Ca-c-287 | 1-a | Ca-c-286 | | 3,4-DFPh | H | H | 1Me-5-Idz | C | | 419 (M⁺+1) |
| Ca-c-288 | | Ca-a-394 | | 2,4-DClPh | H | H | 1Me-5-Idz | C | | 452 (M⁺+1) |
| Ca-c-289 | | Ca-a-396 | | 3,5-DClPh | H | H | 1Me-5-Idz | C | | 452 (M⁺+1) |
| Ca-c-290 | 5-e | Int.c-c-5 | BRA56 | 2,4-DMePh | Et | H | 1Me-5-Idz | C | | 439 (M⁺+1) |
| Ca-c-291 | 1-a | Ca-c-290 | | 2,4-DMePh | H | H | 1Me-5-Idz | C | | 411 (M⁺+1) |
| Ca-c-292 | 5-e | Int.c-c-5 | BRA57 | 2,6-DMePh | Et | H | 1Me-5-Idz | C | | 439 (M⁺+1) |
| Ca-c-293 | 1-a | Ca-c-292 | | 2,6-DMePh | H | H | 1Me-5-Idz | C | | 411 (M⁺+1) |
| Ca-c-294 | 5-e | Ca-c-293 | BRA58 | 2-Furan | Et | H | 1Me-5-Idz | C | | 401 (M⁺+1) |
| Ca-c-295 | 1-a | Ca-c-294 | | 2-Furan | H | H | 1Me-5-Idz | C | | 373 (M⁺+1) |
| Ca-c-296 | 5-e | Ca-c-295 | BRA59 | 3-Furan | Et | H | 1Me-5-Idz | C | | 401 (M⁺+1) |
| Ca-c-297 | 1-a | Ca-c-296 | | 3-Furan | H | H | 1Me-5-Idz | C | | 373 (M⁺+1) |
| Ca-c-298 | 5-e | Ca-c-297 | BRA60 | 2-Thiophene | Et | H | 1Me-5-Idz | C | | 417 (M⁺+1) |
| Ca-c-299 | 1-a | Int.c-c-5 | | 2-Thiophene | H | H | 1Me-5-Idz | C | | 389 (M⁺+1) |
| Ca-c-30C | 5-e | Ca-c-299 | BRA61 | 3-Thiophene | Et | H | 1Me-5-Idz | C | | 417 (M⁺+1) |
| Ca-c-301 | 1-a | Int.c-c-5 | | 3-Thiophene | H | H | 1Me-5-Idz | C | | 389 (M⁺+1) |
| Ca-c-302 | | Ca-a-410 | | 3-Pyridine | H | H | 1Me-5-Idz | C | | 384 (M⁺+1) |
| Ca-c-303 | | Ca-a-412 | | 4-Pyridine | H | H | 1Me-5-Idz | C | | 384 (M⁺+1) |
| Ca-c-304 | 5-e | Int.c-c-5 | BRA64 | 3-PhPh | Et | H | 1Me-5-Idz | C | | 487 (M⁺+1) |
| Ca-c-305 | 1-a | Ca-c-304 | | 3-PhPh | H | H | 1Me-5-Idz | C | | 459 (M⁺+1) |
| Ca-c-306 | 5-e | Int.c-c-5 | BRA66 | C₄H₉CH=CH- | Et | H | 1Me-5-Idz | C | | 417 (M⁺+1) |
| Ca-c-307 | 1-a | Ca-c-306 | | C₄H₉CH=CH- | H | H | 1Me-5-ldz | C | | 389 (M⁺+1) |
| Ca-c-308 | 9-j | Ca-c-306 | | nHex- | Et | H | 1Me-5-Idz | C | | 419 (M⁺+1) |
| Ca-c-309 | 1-a | Ca-c-308 | | nHex- | H | H | 1Me-5-Idz | C | | 391 (M⁺+1) |
| Ca-c-310 | 5-e | Int.c-c-5 | BRA67 | 4FPhCH=CH- | Et | H | 1Me-5-Idz | C | | 455 (M⁺+1) |
| Ca-c-311 | 1-a | Ca-c-310 | | 4FPhCH=CH- | H | H | 1Me-5-Idz | C | | 427 (M⁺+1) |
| Ca-c-312 | 9-j | Ca-c-310 | | 4FPhC₂H₄- | Et | H | 1Me-5-Idz | C | | 457 (M⁺+1) |
| Ca-c-313 | 1-a | Ca-c-312 | | 4FPhC₂H₄- | H | H | 1Me-5-Idz | C | | 429 (M⁺+1) |
| Ca-c-314 | 5-e | Int.c-c-5 | BRA69 | 2cHexCH=CH- | Et | H | 1Me-5-Idz | C | | 443 (M⁺+1) |

**[Table 120]**

| Table-Ca-C-8 | | | | | | | | | | |
|---|---|---|---|---|---|---|---|---|---|---|
| Exp. | Syn. | SM1 | Reagent | Rx | Y | V₁' | V₂' | LCMS | | |
| | | | | | | | | method | RTime | Mass |
| Ca-c-315 | 1-a | Ca-c-314 | | 2cHexCH=CH- | H | H | 1Me-5-Idz | C | | 415 (M⁺+1) |
| Ca-c-316 | 9-j | Ca-c-314 | | 2cHexC₂H₄- | Et | H | 1Me-5-Idz | C | | 445 (M⁺+1) |
| Ca-c-317 | 1-a | Ca-c-316 | | 2cHexC₂H₄- | H | H | 1Me-5-Idz | C | | 417 (M⁺+1) |
| Ca-c-318 | 5-e | Intc-c-5 | BRA70 | 4CF₃PhCH=CH- | Et | H | 1Me-5-Idz | C | | 505 (M⁺+1) |
| Ca-c-319 | 1-a | Ca-c-318 | | 4CF3PhCH=CH- | H | H | 1Me-5-Idz | C | | 477 (M⁺+1) |
| Ca-c-320 | 9-j | Ca-c-318 | | 4CF3PhC₂H₄- | Et | H | 1Me-5-Idz | C | | 507 (M⁺+1) |
| Ca-c-321 | 1-a | Ca-c-320 | | 4CF3PhC₂H₄- | H | H | 1Me-5-Idz | C | | 479 (M⁺+1) |
| Ca-c-322 | 5-e | Int.c-c-5 | BRA72 | 3MeOPhCH=CH- | Et | H | 1Me-5-Idz | C | | 467 (M⁺+1) |
| Ca-c-323 | 1-a | Ca-c-322 | | 3MeOPhCH=CH- | H | H | 1Me-5-Idz | C | | 439 (M⁺+1) |
| Ca-c-324 | 9-j | Ca-c-322 | | 3MeOPhC₂H₄- | Et | H | 1Me-5-Idz | C | | 469 (M⁺+1) |
| Ca-c-325 | 1-a | Ca-c-324 | | 3MeOPhC₂H₄- | H | H | 1Me-5-Idz | C | | 441 (M⁺+1) |
| Ca-c-326 | 5-e | Int.c-c-5 | BRA73 | 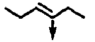 | Et | H | 1Me-5-Idz | C | | 417 (M⁺+1) |
| Ca-c-327 | 1-a | Ca-c-326 | | 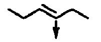 | H | H | 1Me-5-Idz | C | | 389 (M⁺+1) |
| Ca-c-328 | 9-j | Ca-c-326 | | 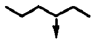 | Et | H | 1Me-5-Idz | C | | 419 (M⁺+1) |
| Ca-c-329 | 1-a | Ca-c-328 | | 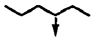 | H | H | 1Me-5-Idz | C | | 391 (M⁺+1) |
| Ca-c-330 | 5-e | Int.c-c-6 | BRA14 | Ph | Et | H | 1Et-5-Idz | C | | 425 (M⁺+1) |
| Ca-c-331 | 1-a | Ca-c-330 | | Ph | H | H | 1Et-5-Idz | C | | 397 (M⁺+1) |
| Ca-c-332 | 5-e | Int.c-c-6 | BRA15 | 2-MePh | Et | H | 1Et-5-Idz | C | | 439 (M⁺+1) |
| Ca-c-333 | 1-a | Ca-c-332 | | 2-MePh | H | H | 1Et-5-Idz | C | | 411 (M⁺+1) |
| Ca-c-334 | | Ca-a-438 | | 3-MePh | H | H | 1Et-5-Idz | C | | 411 (M⁺+1) |
| Ca-c-335 | | Ca-a-440 | | 2-EtPh | H | H | 1Et-5-Idz | C | | 425 (M⁺+1) |
| Ca-c-336 | 5-e | Int.c-c-6 | BRA21 | 4-iPrPh | Et | H | 1Et-5-Idz | C | | 467 (M⁺+1) |
| Ca-c-337 | 1-a | Ca-c-336 | | 4-iPrPh | H | H | 1Et-5-Idz | C | | 439 (M⁺+1) |
| Ca-c-338 | 5-e | Int.c-c-6 | BRA28 | 3-EtOPh | Et | H | 1Et-5-Idz | C | | 469 (M⁺+1) |
| Ca-c-339 | 1-a | Ca-c-338 | | 3-EtOPh | H | H | 1Et-5-Idz | C | | 441 (M⁺+1) |
| Ca-c-340 | | Ca-a-444 | | 3-nPrOPh | H | H | 1Et-5-Idz | C | | 455 (M⁺+1) |
| Ca-c-341 | 5-e | Int.c-c-6 | BRA36 | 4-DMAPh | Et | H | 1Et-5-Idz | C | | 468 (M⁺+1) |
| Ca-c-342 | 1-a | Ca-c-341 | | 4-DMAPh | H | H | 1Et-5-Idz | C | | 440 (M⁺+1) |
| Ca-c-343 | | Ca-a-450 | | 3-FPh | H | H | 1Et-5-Idz | C | | 415 (M⁺+1) |
| Ca-c-344 | 5-e | Int.c-c-6 | BRA39 | 4-FPh | Et | H | 1Et-5-Idz | C | | 443 (M⁺+1) |
| Ca-c-345 | 1-a | Ca-c-344 | | 4-FPh | H | H | 1Et-5-Idz | C | | 415 (M⁺+1) |
| Ca-c-346 | | Ca-a-454 | | 3-ClPh | H | H | 1Et-5-Idz | C | | 431 (M⁺+1) |
| Ca-c-347 | 5-e | Int.c-c-6 | BRA46 | 2,3-DFPh | Et | H | 1Et-5-Idz | C | | 461 (M⁺+1) |
| Ca-c-348 | 1-a | Ca-c-347 | | 2,3-DFPh | H | H | 1Et-5-Idz | C | | 433 (M⁺+1) |
| Ca-c-349 | 5-e | Int.c-c-6 | BRA48 | 2,5-DFPh | Et | H | 1Et-5-Idz | C | | 461 (M⁺+1) |
| Ca-c-35C | 1-a | Ca-c-349 | | 2.5-DFPh | H | H | 1Et-5-Idz | C | | 433 (M⁺+1) |
| Ca-c-351 | | Ca-a-462 | | 3,4-DFPh | H | H | 1Et-5-Idz | C | | 433 (M⁺+1) |
| Ca-c-352 | 5-e | Ca-c-351 | BRA51 | 3,5-DFPh | Et | H | 1Et-5-Idz | C | | 461 (M⁺+1) |
| Ca-c-353 | 1-a | Int.c-c-6 | | 3,5-DFPh | H | H | 1Et-5-Idz | C | | 433 (M⁺+1) |
| Ca-c-354 | 5-e | Ca-c-353 | BRA53 | 3,5-DClPh | Et | H | 1 Et-5-Idz | C | | 494 (M⁺+1) |
| Ca-c-355 | 1-a | Ca-c-354 | | 3,5-DClPh | H | H | 1Et-5-Idz | C | | 466 (M⁺+1) |
| Ca-c-356 | | Ca-a-470 | | 2.6-DMePh | H | H | 1Et-5-Idz | C | | 425 (M⁺+1) |
| Ca-c-357 | 5-e | Ca-c-356 | BRA59 | 3-Furan | Et | H | 1Et-5-Idz | C | | 415 (M⁺+1) |
| Ca-c-358 | 1-a | Ca-c-357 | | 3-Furan | H | H | 1Et-5-Idz | C | | 387 (M⁺+1) |

**[Table 121]**

| Table-Ca-C-9 | | | | | | | | | | |
|---|---|---|---|---|---|---|---|---|---|---|
| Exp. | Syn. | SM1 | Reagent | Rx | Y | V₁' | V₂' | LCMS | | |
| | | | | | | | | method | RTime | Mass |
| Ca-c-359 | 5-e | Ca-c-358 | BRA60 | 2-Thiophene | Et | H | 1Et-5-Idz | C | | 431 (M⁺+1) |
| Ca-c-36C | 1-a | Ca-c-359 | | 2-Thiophene | H | H | 1Et-5-Idz | C | | 403 (M⁺+1) |
| Ca-c-361 | | Ca-a-476 | | 4-Pyridine | H | H | 1Et-5-Idz | C | | 398 (M⁺+1) |
| Ca-c-362 | | Ca-a-478 | | 4-PhPh | H | H | 1Et-5-Idz | C | | 473 (M⁺+1) |
| Ca-c-363 | 5-e | Int.c-c-6 | BRA69 | 2cHexCH=CH- | Et | H | 1Et-5-Idz | C | | 457 (M⁺+1) |
| Ca-c-364 | 1-a | Ca-c-363 | | 2cHexCH=CH- | H | H | 1Et-5-Idz | C | | 429 (M⁺+1) |
| Ca-c-365 | 9-j | Ca-c-363 | | 2cHexC₂H₄- | Et | H | 1Et-5-Idz | C | | 459 (M⁺+1) |
| Ca-c-366 | 1-a | Ca-c-365 | | 2cHexC₂H₄- | H | H | 1Et-5-Idz | C | | 431 (M⁺+1) |
| Ca-c-367 | 5-e | Int.c-c-6 | BRA72 | 3MeOPhCH=CH- | Et | H | 1Et-5-Idz | C | | 481 (M⁺+1) |
| Ca-c-368 | 1-a | Ca-c-367 | | 3MeOPhCH=CH- | H | H | 1Et-5-Idz | C | | 453 (M⁺+1) |
| Ca-c-369 | 5-e | Ca-c-367 | | 3MeOPhC₂H₄- | Et | H | 1Et-5-Idz | C | | 483 (M⁺+1) |
| Ca-c-37C | 1-a | Ca-c-369 | | 3MeOPhC₂H₄- | H | H | 1Et-5-Idz | C | | 455 (M⁺+1) |
| Ca-c-371 | | Ca-a-492 | | Ph | H | H | 5-BF | C | | 369 (M⁺+1) |
| Ca-c-372 | | Ca-a-494 | | 3-MePh | H | H | 5-BF | C | | 383 (M⁺+1) |
| Ca-c-373 | | Ca-a-498 | | 2-iPrPh | H | H | 5-BF | C | | 411 (M⁺+1) |
| Ca-c-374 | | Ca-a-500 | | 4-tBuPh | H | H | 5-BF | C | | 425 (M⁺+1) |
| Ca-c-375 | 5-e | Int.c-c-7 | BRA25 | 3-MeOPh | Et | H | 5-BF | C | | 427 (M⁺+1) |
| Ca-c-376 | 1-a | Ca-c-375 | | 3-MeOPh | H | H | 5-BF | C | | 399 (M⁺+1) |
| Ca-c-377 | 5-e | Int.c-c-7 | BRA28 | 3-EtOPh | Et | H | 5-BF | C | | 441 (M⁺+1) |
| Ca-c-378 | 1-a | Ca-c-377 | | 3-EtOPh | H | H | 5-BF | C | | 413 (M⁺+1) |
| Ca-c-379 | | Ca-a-506 | | 3-nPrOPh | H | H | 5-BF | C | | 427 (M⁺+1) |
| Ca-c-38C | | Ca-a-508 | | 3-nBuOPh | H | H | 5-BF | C | | 441 (M⁺+1) |
| Ca-c-381 | | Ca-a-510 | | 3-DMAPh | H | H | 5-BF | C | | 412 (M⁺+1) |
| Ca-c-382 | 5-e | Int.c-c-7 | BRA36 | 4-DMAPh | Et | H | 5-BF | C | | 440 (M⁺+1) |
| Ca-c-383 | 1-a | Ca-c-382 | | 4-DMAPh | H | H | 5-BF | C | | 412 (M⁺+1) |
| Ca-c-384 | | Ca-a-512 | | 2-FPh | H | H | 5-BF | C | | 387 (M⁺+1) |
| Ca-c-385 | | Ca-a-514 | | 4-FPh | H | H | 5-BF | C | | 387 (M⁺+1) |
| Ca-c-386 | 5-e | Int.c-c-7 | BRA41 | 3-ClPh | Et | H | 5-BF | C | | 431 (M⁺+1) |
| Ca-c-387 | 1-a | Ca-c-386 | | 3-ClPh | H | H | 5-BF | C | | 403 (M⁺+1) |
| Ca-c-388 | 5-e | Int.c-c-7 | BRA45 | 4-CF3Ph | Et | H | 5-BF | C | | 465 (M⁺+1) |
| Ca-c-389 | 1-a | Ca-c-388 | | 4-CF3Ph | H | H | 5-BF | C | | 437 (M⁺+1) |
| Ca-c-39C | 5-e | Int.c-c-7 | BRA46 | 2,3-DFPh | Et | H | 5-BF | C | | 433 (M⁺+1) |
| Ca-c-391 | 1-a | Ca-c-39C | | 2,3-DFPh | H | H | 5-BF | C | | 405 (M⁺+1) |
| Ca-c-392 | 5-e | Int.c-c-7 | BRA47 | 2,4-DFPh | Et | H | 5-BF | C | | 433 (M⁺+1) |
| Ca-c-393 | 1-a | Ca-c-392 | | 2,4-DFPh | H | H | 5-BF | C | | 405 (M⁺+1) |
| Ca-c-394 | | Ca-a-524 | | 3,4-DFPh | H | H | 5-BF | C | | 405 (M⁺+1) |
| Ca-c-395 | | Ca-a-526 | | 3,5-DClPh | H | H | 5-BF | C | | 438 (M⁺+1) |
| Ca-c-396 | 5-e | Int.c-c-7 | BRA56 | 2,4-DMePh | Et | H | 5-BF | C | | 425 (M⁺+1) |
| Ca-c-397 | 1-a | Ca-c-396 | | 2,4-DMePh | H | H | 5-BF | C | | 397 (M⁺+1) |
| Ca-c-398 | | Ca-a-532 | | 2-Furan | H | H | 5-BF | C | | 359 (M⁺+1) |
| Ca-c-399 | | Ca-a-534 | | 3-Thiophene | H | H | 5-BF | C | | 375 (M⁺+1) |
| Ca-c-40C | | Ca-a-538 | | Ph | H | H | 6-Qu | C | | 380 (M⁺+1) |
| Ca-c-401 | | Ca-a-542 | | 4-MePh | H | H | 6-Qu | C | | 394 (M⁺+1) |
| Ca-c-402 | 5-e | Int.c-c-8 | BRA23 | 4-tBuPh | Et | H | 6-Qu | C | | 464 (M⁺+1) |
| Ca-c-403 | 1-a | Ca-c-402 | | 4-tBuPh | H | H | 6-Qu | C | | 436 (M⁺+1) |
| Ca-c-404 | | Ca-a-546 | | 3-MeOPh | H | H | 6-Qu | C | | 410 (M⁺+1) |

**[Table 122]**

| Table-Ca-C-10 | | | | | | | | | | |
|---|---|---|---|---|---|---|---|---|---|---|
| Exp. | Syn. | SM1 | Reagent | Rx | Y | V₁' | V₂' | LCMS | | |
| | | | | | | | | method | RTime | Mass |
| Ca-c-405 | | Ca-a-550 | | 4-iPrOPh | H | H | 6-Qu | C | | 438 (M⁺+1) |
| Ca-c-406 | 5-e | Int.c-c-8 | BRA36 | 4-DMAPh | Et | H | 6-Qu | C | | 451 (M⁺+1) |
| Ca-c-407 | 1-a | Ca-c-406 | | 4-DMAPh | H | H | 6-Qu | C | | 423 (M⁺+1) |
| Ca-c-408 | | Ca-a-554 | | 3-FPh | H | H | 6-Qu | C | | 398 (M⁺+1) |
| Ca-c-409 | 5-e | Int.c-c-8 | BRA40 | 2-ClPh | Et | H | 6-Qu | C | | 442 (M⁺+1) |
| Ca-c-410 | 1-a | Ca-c-409 | | 2-ClPh | H | H | 6-Qu | C | | 414 (M⁺+1) |
| Ca-c-411 | 5-e | Int.c-c-8 | BRA45 | 4-CF3Ph | Et | H | 6-Qu | C | | 476 (M⁺+1) |
| Ca-c-412 | 1-a | Ca-c-411 | | 4-CF3Ph | H | H | 6-Qu | C | | 448 (M⁺+1) |
| Ca-c-413 | | Ca-a-560 | | 2,4-DFPh | H | H | 6-Qu | C | | 416 (M⁺+1) |
| Ca-c-414 | 5-e | Int.c-c-8 | BRA49 | 2,6-DFPh | Et | H | 6-Qu | C | | 444 (M⁺+1) |
| Ca-c-415 | 1-a | Ca-c-414 | | 2,6-DFPh | H | H | 6-Qu | C | | 416 (M⁺+1) |
| Ca-c-416 | | Ca-a-562 | | 3,4-DFPh | H | H | 6-Qu | C | | 416 (M⁺+1) |
| Ca-c-417 | | Ca-a-566 | | 2,4-DClPh | H | H | 6-Qu | C | | 449 (M⁺+1) |
| Ca-c-418 | 5-e | Int.c-c-8 | BRA57 | 2,6-DMePh | Et | H | 6-Qu | C | | 436 (M⁺+1) |
| Ca-c-419 | 1-a | Ca-c-418 | | 2,6-DMePh | H | H | 6-Qu | C | | 408 (M⁺+1) |
| Ca-c-420 | | Ca-a-572 | | 2-Furan | H | H | 6-Qu | C | | 370 (M⁺+1) |
| Ca-c-421 | 5-e | Int.c-c-8 | BRA59 | 3-Furan | Et | H | 6-Qu | C | | 398 (M⁺+1) |
| Ca-c-422 | 1-a | Ca-c-421 | | 3-Furan | H | H | 6-Qu | C | | 370 (M⁺+1) |
| Ca-c-423 | | Ca-a-574 | | 2-Thiophene | H | H | 6-Qu | C | | 386 (M⁺+1) |
| Ca-c-424 | 5-e | Int.c-c-8 | BRA61 | 3-Thiophene | Et | H | 6-Qu | C | | 414 (M⁺+1) |
| Ca-c-425 | 1-a | Ca-c-424 | | 3-Thiophene | H | H | 6-Qu | C | | 386 (M⁺+1) |
| Ca-c-426 | | Ca-a-576 | | 3-Pyridine | H | H | 6-Qu | C | | 381 (M⁺+1) |
| Ca-c-427 | | Ca-a-578 | | 4-PhPh | H | H | 6-Qu | C | | 456 (M⁺+1) |
| Ca-c-428 | 5-e | Int.c-c-8 | BRA68 | 4ClPhCH=CH- | Et | H | 6-Qu | C | | 469 (M⁺+1) |
| Ca-c-429 | 1-a | Ca-c-427 | | 4ClPhCH=CH- | H | H | 6-Qu | C | | 440 (M⁺+1) |
| Ca-c-430 | 5-e | Int.c-c-8 | BRA69 | 2cHexCH=CH- | Et | H | 6-Qu | C | | 440 (M⁺+1) |
| Ca-c-431 | 1-a | Ca-c-430 | | 2cHexCH=CH- | H | H | 6-Qu | C | | 412 (M⁺+1) |
| Ca-c-432 | | Ca-a-584 | | 4MePhCH=CH- | H | H | 6-Qu | C | | 420 (M⁺+1) |
| Ca-c-433 | | Ca-a-586 | | 4MePhC₂H₄- | H | H | 6-Qu | C | | 422 (M⁺+1) |
| Ca-c-434 | 5-e | Int.c-c-8 | BRA73 | 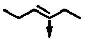 | Et | H | 6-Qu | C | | 414 (M⁺+1) |
| Ca-c-435 | 1-a | Ca-c-434 | | 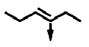 | H | H | 6-Qu | C | | 386 (M⁺+1) |
| Ca-c-436 | 5-e | Int.c-c-1 | BRA74 | Trans-CH₃CH=CH- | Et | H | 2-Nap | C | | 371 (M⁺+1) |
| Ca-c-437 | 1-a | Ca-c-436 | | Trans-CH₃CH=CH- | H | H | 2-Nap | C | | 343 (M⁺+1) |
| Ca-c-438 | 9-j | Ca-c-436 | | nPr | Et | H | 2-Nap | C | | 373 (M⁺+1) |
| Ca-c-439 | 1-a | Ca-c-438 | | nPr | H | H | 2-Nap | C | | 345 (M⁺+1) |
| Ca-c-440 | 5-e | Int.c-c-1 | BRA75 | Cis-CH₃CH=CH- | Et | H | 2-Nap | C | | 371 (M⁺+1) |
| Ca-c-441 | 1-a | Ca-c-440 | | Cis-CH₃CH=CH- | H | H | 2-Nap | C | | 343 (M⁺+1) |
| Ca-c-442 | 5-e | Int.c-c-1 | BRA76 | C₃H₇CH=CH- | Et | H | 2-Nap | C | | 399 (M⁺+1) |
| Ca-c-443 | 1-a | Ca-c-442 | | C₃H₇CH=CH- | H | H | 2-Nap | C | | 371 (M⁺+1) |
| Ca-c-444 | 9-j | Ca-c-442 | | nPen | Et | H | 2-Nap | C | | 401 (M⁺+1) |
| Ca-c-445 | 1-a | Ca-c-444 | | nPen | H | H | 2-Nap | C | | 373 (M⁺+1) |
| Ca-c-446 | 5-e | Int.c-c-2 | BRA74 | Trans-CH₃CH=CH- | Et | H | 5-Ind | C | | 360 (M⁺+1) |
| Ca-c-447 | 1-a | Ca-c-446 | | Trans-CH₃CH=CH- | H | H | 5-Ind | C | | 332 (M⁺+1) |
| Ca-c-448 | 9-j | Ca-c-446 | | nPr | Et | H | 5-Ind | C | | 362 (M⁺+1) |

**[Table 123]**

| Table-Ca-C-11 | | | | | | | | | | |
|---|---|---|---|---|---|---|---|---|---|---|
| Exp. | Syn. | SM1 | Reagent | Rx | Y | V₁' | V₂' | LCMS | | |
| | | | | | | | | method | RTime | Mass |
| Ca-c-449 | 1-a | Ca-c-448 | | nPr | H | H | 5-Ind | C | | 334 (M⁺+1) |
| Ca-c-450 | | Ca-a-604 | | C₃H₇CH=CH- | H | H | 5-Ind | C | | 360 (M⁺+1) |
| Ca-c-451 | 5-e | Int.c-c-3 | BRA75 | Cis-CH₃CH=CH- | Et | H | 1Me-5-Ind | C | | 374 (M⁺+1) |
| Ca-c-452 | 1-a | Ca-c-451 | | Cis-CH₃CH=CH- | H | H | 1Me-5-Ind | C | | 346 (M⁺+1) |
| Ca-c-453 | 9-j | Ca-c-451 | | nPr | Et | H | 1Me-5-Ind | C | | 376 (M⁺+1) |
| Ca-c-454 | 1-a | Ca-c-453 | | nPr | H | H | 1Me-5-Ind | C | | 348 (M⁺+1) |
| Ca-c-455 | 5-e | Int.c-c-3 | BRA76 | C₃H₇CH=CH- | Et | H | 1Me-5-Ind | C | | 402 (M⁺+1) |
| Ca-c-456 | 1-a | Ca-c-455 | | C₃H₇CH=CH- | H | H | 1Me-5-Ind | C | | 374 (M⁺+1) |
| Ca-c-457 | 9-j | Ca-c-455 | | nPen | Et | H | 1Me-5-Ind | C | | 404 (M⁺+1) |
| Ca-c-458 | 1-a | Ca-c-457 | | nPen | H | H | 1Me-5-Ind | C | | 376 (M⁺+1) |
| Ca-c-459 | 5-e | Int.c-c-4 | BRA74 | Trans-CH₃CH=CH- | Et | H | 5-1Idz | C | | 361 (M⁺+1) |
| Ca-c-460 | 1-a | Ca-c-459 | | Trans-CH₃CH=CH- | H | H | 5-1Idz | C | | 333 (M⁺+1) |
| Ca-c-461 | 9-j | Ca-c-459 | | nPr | Et | H | 5-1Idz | C | | 363 (M⁺+1) |
| Ca-c-462 | 1-a | Ca-c-461 | | nPr | H | H | 5-1Idz | C | | 335 (M⁺+1) |
| Ca-c-463 | 5-e | Int.c-c-4 | BRA75 | Cis-CH₃CH=CH- | Et | H | 5-1Idz | C | | 361 (M⁺+1) |
| Ca-c-464 | 1-a | Ca-c-463 | | Cis-CH₃CH=CH- | H | H | 5-1Idz | C | | 333 (M⁺+1) |
| Ca-c-465 | | Ca-a-616 | | C₃H₇CH=CH- | H | H | 5-1Idz | C | | 361 (M⁺+1) |
| Ca-c-466 | | Ca-a-618 | | nPen | H | H | 5-1Idz | C | | 363 (M⁺+1) |
| Ca-c-467 | 5-e | Int.c-c-5 | BRA74 | Trans-CH₃CH=CH- | Et | H | 1Me-5-Idz | C | | 375 (M⁺+1) |
| Ca-c-468 | 1-a | Ca-c-467 | | Trans-CH₃CH=CH- | H | H | 1Me-5-Idz | C | | 347 (M⁺+1) |
| Ca-c-469 | 9-j | Ca-c-467 | | nPr | Et | H | 1Me-5-Idz | C | | 377 (M⁺+1) |
| Ca-c-470 | 1-a | Ca-c-469 | | nPr | H | H | 1Me-5-Idz | C | | 349 (M⁺+1) |
| Ca-c-471 | 5-e | Int.c-c-5 | BRA75 | Cis-CH₃CH=CH- | Et | H | 1Me-5-Idz | C | | 375 (M⁺+1) |
| Ca-c-472 | 1-a | Ca-c-471 | | Cis-CH₃CH=CH- | H | H | 1Me-5-Idz | C | | 347 (M⁺+1) |
| Ca-c-473 | 5-e | Int.c-c-5 | BRA76 | C₃H₇CH=CH- | Et | H | 1Me-5-Idz | C | | 403 (M⁺+1) |
| Ca-c-474 | 1-a | Ca-c-473 | | C₃H₇CH=CH- | H | H | 1Me-5-Idz | C | | 375 (M⁺+1) |
| Ca-c-475 | 9-j | Ca-c-473 | | nPen | Et | H | 1Me-5-Idz | C | | 405 (M⁺+1) |
| Ca-c-476 | 1-a | Ca-c-475 | | nPen | H | H | 1Me-5-Idz | C | | 377 (M⁺+1) |
| Ca-c-477 | | Ca-a-626 | | Trans-CH₃CH=CH- | H | H | 1Et-5-Idz | C | | 361 (M⁺+1) |
| Ca-c-478 | | Ca-a-628 | | nPr | H | H | 1Et-5-Idz | C | | 363 (M⁺+1) |
| Ca-c-479 | | Ca-a-630 | | Trans-CH₃CH=CH- | H | H | 5-BF | C | | 333 (M⁺+1) |
| Ca-c-480 | 5-e | Int.c-c-7 | BRA75 | Cis-CH₃CH=CH- | Et | H | 5-BF | C | | 361 (M⁺+1) |
| Ca-c-481 | 1-a | Ca-c-479 | | Cis-CH₃CH=CH- | H | H | 5-BF | C | | 333 (M⁺+1) |
| Ca-c-482 | 5-e | Int.c-c-7 | BRA76 | C₃H₇CH=CH- | Et | H | 5-BF | C | | 389 (M⁺+1) |
| Ca-c-483 | 1-a | Ca-c-482 | | C₃H₇CH=CH- | H | H | 5-BF | C | | 361 (M⁺+1) |
| Ca-c-484 | 5-e | Int.c-c-8 | BRA74 | Trans-CH₃CH=CH- | Et | H | 6-Qu | C | | 372 (M⁺+1) |
| Ca-c-485 | 1-a | Ca-c-483 | | Trans-CH₃CH=CH- | H | H | 6-Qu | C | | 344 (M⁺+1) |
| Ca-c-486 | 9-j | Ca-c-483 | | nPr | Et | H | 6-Qu | C | | 374 (M⁺+1) |
| Ca-c-487 | 1-a | Ca-c-486 | | nPr | H | H | 6-Qu | C | | 346 (M⁺+1) |
| Ca-c-488 | | Ca-a-636 | | C₃H₇CH=CH- | H | H | 6-Qu | C | | 372 (M⁺+1) |

### Reference Example 13

### Synthesis of 5-benzylmethylamino-2,3-dihydroinden-1-one (Intermediate n-a-1) (Preparation Method 14, Step n-1)

A solution of 5-fluoro-1-indanone (1.56 g, Frontier) in dimethyl sulfoxide (3 ml) was added with potassium carbonate (341.3 mg, WAKO) and benzylmethylamine (2 ml, TCI), and stirred at 100°C for 17 hours. The reaction mixture was added with water (30 ml) and extracted with ethyl acetate (20 ml × 2), and then the organic layer was washed with water and saturated brine. The organic layer was dried, and then the solvent was evaporated under reduced pressure. The residue was purified by column chromatography (Quad, hexane:ethyl acetate = 6:1) to obtain the title compound (Intermediate n-a-1, 1.56 g). Mass (LCMS): 252 (M⁺+1), Retention time: 4.46 minutes (Elution condition: B).

### Synthesis of 5-(piperidin-1-yl)-2,3-dihydroinden-1-one (Intermediate n-a-3) (Preparation Method 14, Step n-1)

According to the procedure described in the synthetic method of Intermediate n-a-1 in Reference Example 13, 5-fluoro-1-indanone (1.40 g, Frontier), potassium carbonate (311.4 mg, WAKO) and piperidine (1.5 ml, TCI) were reacted and treated to obtain the title compound (Intermediate n-c-1, 1.33 g). Mass (LCMS): 216 (M⁺+1), Retention time: 4.34 minutes (Elution condition: D).

### Synthesis of ethyl 2-(5-benzylmethylamino-2,3-dihydroinden-1-yl-ylidene)acetate (Intermediate n-b-1) (Preparation Method 9, Step k-1)

According to the procedure described in the synthetic method of Intermediate A-2 in Reference Example 1 (Preparation Method 9, Step k-1), Intermediate n-a-1 (1.56 g), ethyl diethylphosphonoacetate (7.5 ml, TCI) and sodium hydride (1.28 g, WAKO) were reacted and treated to obtain the title compound (Intermediate n-b-1, 991.2 mg). Mass (LCMS): 322 (M⁺+1), Retention time: 5.99 minutes (Elution condition: B).

### Synthesis of ethyl 2-(5-methylamino-2,3-dihydro-1H-inden-1-yl)acetate (Intermediate n-b-2) (Preparation Method 9, Step j)

According to the procedure described in the synthetic method of Intermediate A-3 in Reference Example 1 (Preparation Method 9, Step j), Intermediate n-b-1 (991.2 mg) and 10% palladium/carbon (76.3 mg, Merck) were reacted and treated to obtain the title compound (Intermediate n-b-2, 786.2 mg). Mass (LCMS): 234 (M⁺+1), Retention time: 3.51 minutes (Elution condition: D).

### Synthesis of ethyl 2-(4-bromo-5-methylamino-2,3-dihydro-1H-inden-1-yl)acetate (Intermediate n-b-3) (Preparation Method 8, Step h)

According to the procedure described in the synthetic method of Intermediate A-4 in Reference Example 1 (Preparation Method 8, Step h) provided that the reaction was performed at room temperature for 3 hours, Intermediate n-b-2 (236.2 mg) and NBS (182.1 g) were reacted and treated to obtain the title compound (Intermediate n-b-3, 255.4 mg). Mass (LCMS): 312 (M⁺+1), Retention time: 5.25 minutes (Elution condition: D).

### Reference Example 14

### Synthesis of ethyl 2-[5-t-butyloxycarbonyl-6-(naphthalen-2-yl)-2,3-dihydro-1H-inden-1-yl]acetate (Intermediate n-b-7) (Preparation Method 4, Step d)

A solution of Intermediate c-a-1 (2.05 g) in toluene (88 ml) was added with t-butyl carbamate (2.56 g, WAKO), xanthophos (1.01 g, Strem) and cesium carbonate (2.14 g, WAKO), and added with tris(dibenzylideneacetone)dipalladium (800 mg, WAKO) under a nitrogen atmosphere, and the mixture was stirred at 115°C for 16 hours in an autoclave. The reaction mixture was filtered, and concentrated under reduced pressure, and the residue was purified by column chromatography (Quad, hexane:ethyl acetate = 5:1) to obtain the title compound (Intermediate n-b-7, 1.00 g). Mass (LCMS): 446 (M⁺+1), Retention time: N.D (Elution condition: C).

### Synthesis of ethyl 2-[5-amino-6-(naphthalen-2-yl)-2,3-dihydro-1H-inden-1-yl]acetate (Intermediate n-b-8) (Preparation Method 4, Step d)

A solution of Intermediate n-b-7 (1.00 g) was added with 4 N hydrochloric acid in dioxane (23 ml), and the mixture was stirred at room temperature for 4 hours. The reaction mixture was concentrated, and then added with ethyl acetate (50 ml), and the organic layer was washed with saturated aqueous sodium hydrogencarbonate, and concentrated under reduced pressure to obtain the title compound (Intermediate n-b-8, 863 mg). Mass (LCMS): 346 (M⁺+1), Retention time: N.D (Elution condition: D).

Typical examples of the intermediates that can be obtained by reacting and treating corresponding starting compounds using any of the methods described in the present specification including the examples described above are shown in Table-Int.N-a-1 and Table-Int.N-b-1. The compounds were synthesized according to the preparation methods of the compounds of the compound numbers shown in the columns of "Syn" in the tables. "Int" means an intermediate compound number. In the tables, used methods among the aforementioned synthetic methods are shown in the columns of "Syn" with symbols, the starting compounds are shown in the columns of "SM", and the reagents are shown in the columns of "Regent". The halide regents mentioned in the columns of "Reagent" with symbols "AMN (No.)" are those mentioned in Table-AMN. Further, the compounds indicated as "S" in the columns of "S/D" in Table-Int.N-b-1 are compound in which two of the carbon atoms binding the indan ring and carbonyl group in the compounds are bound with a single bond, and those indicated as "D" in the same are compounds in which two of the carbon atoms binding the benzene ring and carbonyl group in the compounds are bound with a double bond. The blank cells of the columns of "Syn" indicate that a reduction reaction with Pd/C was performed for the compounds.

**[Table 124]**

| Table-AMN | | |
|---|---|---|
| Reagent | The name of reagent | manufacture |
| AMN1 | BnMeNH | TCI |
| AMN2 | BnEtNH | TCI |
| AMN3 | Piperidine | TCI |
| AMN4 | 4-Methylpiperidine | TCI |
| AMN5 | 2-Methylpiperidine | TCI |
| AMN6 | 3-Methylpiperidine | TCI |
| AMN7 | 3,5-Dimethylpiperidine | TCI |
| AMN8 | 4-Phenylpiperidine | WAKO |
| AMN9 | 4-Benzylpiperidine | TCI |
| AMN10 | Morpholine | TCI |
| AMN11 | 2,6-Dimethylmorpholine | WAKO |
| AMN12 | cis-2,6-Dimethylmorpholine | TCI |
| AMN13 | 1-Phenylpiperazine | TCI |
| AMN14 | 1-(2-Pyridyl)piperazine | TCI |
| AMN15 | 1-(4-Fluorophenyll)piperazine | WAKO |
| AMN16 | 1-(2-Fluorophenyll)piperazine | WAKO |
| AMN17 | 1-(3-Chlorophenyll)piperazine | WAKO |
| AMN18 | Hexamethyleneimine | TCI |
| AMN19 | Pyrrolidine | TCI |

**[Table 125]**

| Table-CHO | | |
|---|---|---|
| Reagent | The name of reagent | manufacture |
| CHO1 | Formaldehyde | WAKO |
| CHO2 | acetaldehyde | WAKO |
| CHO3 | n-Propylaldehyde | TCI |
| CHO4 | acetone | TCI |
| CHO5 | n-Butyraldehyde | TCI |
| CHO6 | Isobutyraldehyde | TCI |
| CHO7 | cyclopentanone | WAKO |
| CHO8 | cyclohexanone | WAKO |
| CHO9 | 2-Methylcyclohexanone | TCI |
| CHO10 | 2-Indanone | WAKO |
| CHO11 | 4-Fluorobenzaldehyde | TCI |
| CHO12 | 2-Fluorobenzaldehyde | TCI |
| CHO13 | 3-Fluorobenzaldehyde | TCI |
| CHO14 | 2-ChloroBenzaldehyde | TCI |
| CHO15 | 4-Chlorobenzaldehyde | TCI |
| CHO16 | 2-Bromobenzaldehyde | TCI |
| CHO17 | 2-Trifluoromethylbenzaldehyde | TCI |
| CHO18 | 2,3-difluorobenzaldehyde | TCI |
| CHO19 | 4-Methylbenzaldehyde | TCI |
| CHO20 | 2-Phenylbenzaldehyde | Ald |
| CHO21 | p-anisaldehyde | TCI |
| CHO22 | o-anisaldehyde | TCI |
| CHO23 | 4-Dimethylaminobenzaldehyde | TCI |
| CHO24 | 4-Methylthiobenzaldehyde | TCI |
| CHO25 | 1-Naphthylaldehyde | TCI |
| CHO26 | 2-Naphthylaldehyde | TCI |
| CHO27 | 2-Furaldehyde | TCI |
| CHO28 | 3-Furaldehyde | TCI |
| CHO29 | 2-Thiophenaldehyde | TCI |

**[Table 126]**

| Table-Int.N-a-1 | | | | | | | | | | |
|---|---|---|---|---|---|---|---|---|---|---|
| 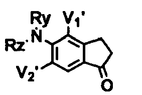 | | | | | | | | | | |

| Exp. | Syn. | SM1 | Reagent | Rz | Ry | V₁' | V₂' | LCMS | | |
|---|---|---|---|---|---|---|---|---|---|---|
| | | | | | | | | method | RTime | Mass |
| Int.n-a-2 | 14n-1 | AMN2 | | Et | Bn | H | H | C | | 266 (M⁺+1) |
| Int.n-a-4 | 14n-1 | AMN4 | | 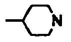 | | H | H | C | | 230 (M⁺+1) |
| Int.n-a-5 | 14n-1 | AMN5 | | 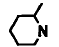 | | H | H | C | | 230 (M⁺+1) |
| Int.n-a-6 | 14n-1 | AMN6 | | 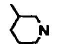 | | H | H | C | | 230 (M⁺+1) |
| Int.n-a-7 | 14n-1 | AMN7 | | 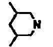 | | H | H | C | | 244 (M⁺+1) |
| Int.n-a-8 | 14n-1 | AMN8 | | 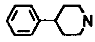 | | H | H | C | | 292 (M⁺+1) |
| Int.n-a-9 | 14n-1 | AMN9 | | 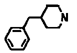 | | H | H | C | | 306 (M⁺+1) |
| Int.n-a-10 | 14n-1 | AMN10 | | 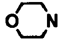 | | H | H | C | | 218 (M⁺+1) |
| Int.n-a-11 | 14n-1 | AMN11 | | 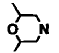 | | H | H | C | | 246 (M⁺+1) |
| Int.n-a-12 | 14n-1 | AMN12 | | 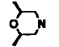 | | H | H | C | | 246 (M⁺+1) |
| Int.n-a-13 | 14n-1 | AMN13 | | 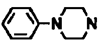 | | H | H | C | | 293 (M⁺+1) |
| Int.n-a-14 | 14n-1 | AMN14 | | 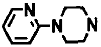 | | H | H | C | | 294 (M⁺+1) |
| Int.n-a-15 | 14n-1 | AMN15 | | 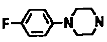 | | H | H | C | | 311 (M⁺+1) |
| Int.n-a-16 | 14n-1 | AMN16 | | 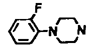 | | H | H | C | | 311 (M⁺+1) |
| Int.n-a-17 | 14n-1 | AMN17 | | 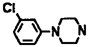 | | H | H | C | | 327 (M⁺+1) |
| Int.n-a-18 | 14n-1 | AMN18 | | 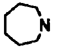 | | H | H | C | | 244 (M⁺+1) |

**[Table 127]**

| Table-Int.N-b-1 | | | | | | | | | | | | |
|---|---|---|---|---|---|---|---|---|---|---|---|---|
| 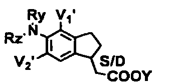 | | | | | | | | | | | | |

| Exp. | Syn. | SM1 | SM2 | Rz | Ry | Y | V₁' | V₂' | S/D | LCMS | | |
|---|---|---|---|---|---|---|---|---|---|---|---|---|
| | | | | | | | | | | method | RTime | Mass |
| Int.n-b-4 | 10k-1 | Int.n-a-2 | | Et | Bn | Et | H | H | D | C | | 336 (M⁺+1) |
| Int.n-b-5 | | Int.n-b-4 | | Et | H | Et | H | H | S | C | | 248 (M⁺+1) |
| Int.n-b-6 | 13-h | Int.n-b-5 | | Et | H | Et | H | Br | S | C | | 326 (M⁺) |
| Int.n-b-9 | 5-d | Int.c-a-2 | | H | H | Et | H | 5-Ind | S | C | | 335 (M⁺+1) |
| Int.n-b-10 | 5-d | Int.c-a-3 | | H | H | Et | H | 1Me-5-Ind | S | C | | 349 (M⁺+1) |
| Int.n-b-11 | 5-d | Int.c-a-4 | | H | H | Et | H | 5-1Idz | S | C | | 336 (M⁺+1) |
| Int.n-b-12 | 5-d | Int.c-a-5 | | H | H | Et | H | 1Me-5-Idz | S | C | | 350 (M⁺+1) |
| Int.n-b-13 | 5-d | Int.c-a-6 | | H | H | Et | H | 1Et-5-Idz | S | C | | 364 (M⁺+1) |
| lnt.n-b-14 | 5-d | Int.c-a-7 | | H | H | Et | H | 5-BF | S | C | | 336 (M⁺+1) |
| Int.n-b-15 | 5-d | Int.c-a-8 | | H | H | Et | H | 6-Qu | S | C | | 347 (M⁺+1) |

### Example N-a-1

### Synthesis of ethyl 2-[5-methylamino-6-(naphthalen-2-yl)-2,3-dihydro-1H-inden-1-yl]acetate (Compound No. N-a-1) (Preparation Method 4, Step e-1)

According to the procedure described in the synthetic method of Example Compound A-a-1 (Preparation Method 4, Step e-1) provided that the reaction was performed for 15 hours, Intermediate n-b-3 (439.4 mg), 2-naphthaleneboronic acid (684.2 mg, Ald), 2 M aqueous sodium carbonate (2.2 ml) and (Ph₃P)₄Pd (276.3 mg, Nakalai Tesque) were reacted and treated to obtain the title compound (Compound No.
N-a-1, 632.3 mg).

### Example N-a-2

### Synthesis of 2-[5-methylamino-6-(naphthalen-2-yl)-2,3-dihydro-1H-inden-1-yl]acetic acid (Compound No. N-a-2) (Preparation Method 1, Step a)

According to the procedure described in the synthetic method of Example Compound A-a-2 (Preparation Method 1, Step a) provided that the reaction was performed for 1 hour, Example Compound N-a-1 (98.6 mg) and 2 N aqueous sodium hydroxide (0.45 ml) were reacted and treated to obtain the title compound (Compound No. N-a-2, 88.7 mg).

### Example N-a-31

### Synthesis of ethyl 2-[5-dimethylamino-6-(naphthalen-2-yl)-2,3-dihydro-1H-inden-1-yl]acetate (Compound No. N-a-31) (Preparation Method 14, Step n-2)

A solution of Example Compound N-a-1 (99.5 mg) in methanol (10 ml) was added with 30% aqueous formaldehyde (350 µl, WAKO) and sodium cyanotrihydroborate (42.2 mg, WAKO), and the mixture was stirred at 60°C to 1 hour. The reaction mixture was concentrated under reduced pressure, and then extracted with dichloromethane (150 ml), the organic layer was washed with saturated brine, and dried, and then the solvent was evaporated under reduced pressure. The residue was purified by column chromatography (Quad, hexane:ethyl acetate = 6:1) to obtain the title compound (Compound No. N-a-31, 45.2 mg).

### Example N-a-43

### Synthesis of ethyl 2-[5-ethylmethylamino-6-(naphthalen-2-yl)-2,3-dihydro-1H-inden-1-yl]acetate (Compound No. N-a-43) (Preparation Method 14, Step n-2)

A solution of Example Compound N-a-1 (99.5 mg) in dichloromethane (5 ml) was added with acetaldehyde (40 µl, TCI), sodium triaceoxyborohydride (224.1 mg, Ald) and acetic acid (100 µL), and the mixture was stirred at room temperature for 13 hours. The reaction mixture was extracted with dichloromethane (20 ml × 2), the organic layer was washed with saturated aqueous sodium hydrogencarbonate and saturated brine, and dried, and then the solvent was evaporated under reduced pressure. The residue was purified by column chromatography (Quad, hexane:ethyl acetate = 6:1) to obtain the title compound (Compound No. N-a-43, 87.3 mg).

Typical examples of the compounds of the present invention that can be obtained by reacting and treating corresponding starting compounds using any of the methods described in the present specification including the examples described above are shown in Table-N-A-1 to Table-N-A-6. The compounds were synthesized according to the preparation methods of the compounds of the compound numbers shown in the columns of "Syn" in the tables. "Int" means an intermediate compound number. As for the preparation of the compounds, used methods among the aforementioned synthetic methods are shown in the columns of "Syn" with symbols, the starting compounds are shown in the columns of "SM", and the reagents are shown in the columns of "Regent" in the tables. The boronic acid regents mentioned in the columns of "Reagent" with symbols "BRA (No.)" are those mentioned in Table-BRA, the bromoheterocyclic ring regents mentioned with the symbols "Het (No.)" are those mentioned in Table-Het, and the formyl regents mentioned with the symbols "CHO (No.)" are those mentioned in Table-CHO.

**[Table 128]**

| Table-N-A-1 | | | | | | | | | | | |
|---|---|---|---|---|---|---|---|---|---|---|---|
| 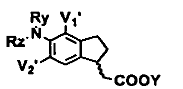 | | | | | | | | | | | |

| Exp. | Syn. | SM1 | Reagent | Ry | Rz | Y | V₁' | V₂' | LCMS | | |
|---|---|---|---|---|---|---|---|---|---|---|---|
| | | | | | | | | | method | RTime | Mass |
| N-a-1 | 4e-1 | Int.n-b-3 | BRA1 | H | Me | Et | H | 2-Nap | C | | 360 (M⁺+1) |
| N-a-2 | 1-a | N-a-1 | | H | Me | H | H | 2-Nap | C | | 332 (M⁺+1) |
| N-a-3 | 4e-1 | Int.n-b-3 | BRA2 | H | Me | Et | H | 5-Ind | C | | 349 (M⁺+1) |
| N-a-4 | 1-a | N-a-3 | | H | Me | H | H | 5-Ind | C | | 321 (M⁺+1) |
| N-a-5 | 4e-1 | Int.n-b-3 | BRA3 | H | Me | Et | H | 1Me-5-Ind | C | | 363 (M⁺+1) |
| N-a-6 | 1-a | N-a-5 | | H | Me | H | H | 1Me-5-Ind | C | | 335 (M⁺+1) |
| N-a-7 | 4e-1 | Int.n-b-3 | BRA4 | H | Me | Et | H | 1Et-5-Ind | C | | 377 (M⁺+1) |
| N-a-8 | 1-a | N-a-7 | | H | Me | H | H | 1Et-5-Ind | C | | 349 (M⁺+1) |
| N-a-9 | 4e-1 | Int.n-b-3 | BRA5 | H | Me | Et | H | 1Me-4-Ind | C | | 363 (M⁺+1) |
| N-a-10 | 1-a | N-a-9 | | H | Me | H | H | 1Me-4-Ind | C | | 335 (M⁺+1) |
| N-a-11 | 4e-1 | Int.n-b-3 | BRA6 | H | Me | Et | H | 1Me-6-Ind | C | | 363 (M⁺+1) |
| N-a-12 | 1-a | N-a-11 | | H | Me | H | H | 1Me-6-Ind | C | | 335 (M⁺+1) |
| N-a-13 | 4e-1 | Int.n-b-3 | BRA7 | H | Me | Et | H | 5-1Idz | C | | 350 (M⁺+1) |
| N-a-14 | 1-a | N-a-13 | | H | Me | H | H | 5-1Idz | C | | 322 (M⁺+1) |
| N-a-15 | 4e-1 | Int.n-b-3 | BRA8 | H | Me | Et | H | 1Me-5-Idz | C | | 364 (M⁺+1) |
| N-a-16 | 1-a | N-a-15 | | H | Me | H | H | 1 Me-5-Idz | C | | 336 (M⁺+1) |
| N-a-17 | 4e-1 | Int.n-b-3 | BRA9 | H | Me | Et | H | 1 Et-5-Idz | C | | 378 (M⁺+1) |
| N-a-18 | 1-a | N-a-17 | | H | Me | H | H | 1Et-5-Idz | C | | 350 (M⁺+1) |
| N-a-19 | 4e-1 | Int.n-b-3 | BRA10 | H | Me | Et | H | 2Me-5-Idz | C | | 364 (M⁺+1) |
| N-a-20 | 1-a | N-a-19 | | H | Me | H | H | 2Me-5-Idz | C | | 336 (M⁺+1) |
| N-a-21 | 4e-1 | Int.n-b-3 | BRA11 | H | Me | Et | H | 5-BF | C | | 350 (M⁺+1) |
| N-a-22 | 1-a | N-a-21 | | H | Me | H | H | 5-BF | C | | 322 (M⁺+1) |
| N-a-23 | 4e-2 | Int.n-b-3 | Het1 | H | Me | Et | H | 5-Bzt | C | | 367 (M⁺+1) |
| N-a-24 | 1-a | N-a-23 | | H | Me | H | H | 5-Bzt | C | | 339 (M⁺+1) |
| N-a-25 | 4e-2 | Int.n-b-3 | Het2 | H | Me | Et | H | 3-Qu | C | | 361 (M⁺+1) |
| N-a-26 | 1-a | N-a-25 | | H | Me | H | H | 3-Qu | C | | 333 (M⁺+1) |
| N-a-27 | 4e-1 | Int.n-b-3 | BRA13 | H | Me | Et | H | 6-Qu | C | | 361 (M⁺+1) |
| N-a-28 | 1-a | N-a-27 | | H | Me | H | H | 6-Qu | C | | 333 (M⁺+1) |
| N-a-29 | 4e-2 | Int.n-b-3 | Het3 | H | Me | Et | H | 6-IQ | C | | 361 (M⁺+1) |
| N-a-30 | 1-a | N-a-29 | | H | Me | H | H | 6-IQ | C | | 333 (M⁺+1) |
| N-a-31 | 14n-2 | N-a-1 | CHO1 | Me | Me | Et | H | 2-Nap | C | | 374 (M⁺+1) |
| N-a-32 | 1-a | N-a-31 | | Me | Me | H | H | 2-Nap | C | | 346 (M⁺+1) |
| N-a-33 | 14n-2 | N-a-3 | CHO1 | Me | Me | Et | H | 5-Ind | C | | 363 (M⁺+1) |
| N-a-34 | 1-a | N-a-33 | | Me | Me | H | H | 5-Ind | C | | 335 (M⁺+1) |
| N-a-35 | 14n-2 | N-a-5 | CHO1 | Me | Me | Et | H | 1Me-5-Ind | C | | 377 (M⁺+1) |
| N-a-36 | 1-a | N-a-35 | | Me | Me | H | H | 1Me-5-Ind | C | | 349 (M⁺+1) |
| N-a-37 | 14n-2 | N-a-13 | CHO1 | Me | Me | Et | H | 5-1Idz | C | | 364 (M⁺+1) |
| N-a-38 | 1-a | N-a-37 | | Me | Me | H | H | 5-1Idz | C | | 336 (M⁺+1) |
| N-a-39 | 14n-2 | N-a-15 | CHO1 | Me | Me | Et | H | 1 Me-5-Idz | C | | 378 (M⁺+1) |
| N-a-40 | 1-a | N-a-39 | | Me | Me | H | H | 1Me-5-Idz | C | | 350 (M⁺+1) |
| N-a-41 | 14n-2 | N-a-27 | CHO1 | Me | Me | Et | H | 6-Qu | C | | 375 (M⁺+1) |
| N-a-42 | 1-a | N-a-41 | | Me | Me | H | H | 6-Qu | C | | 347 (M⁺+1) |
| N-a-43 | 14n-2 | N-a-1 | CHO2 | Et | Me | Et | H | 2-Nap | C | | 388 (M⁺+1) |
| N-a-44 | 1-a | N-a-43 | | Et | Me | H | H | 2-Nap | C | | 360 (M⁺+1) |

**[Table 129]**

| Table-N-A-2 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-a-45 | 14n-2 | N-a-3 | CHO2 | Et | Me | Et | H | 5-Ind | C | | 377 (M⁺+1) |
| N-a-46 | 1-a | N-a-45 | | Et | Me | H | H | 5-Ind | C | | 349 (M⁺+1) |
| N-a-47 | 14n-2 | N-a-5 | CHO2 | Et | Me | Et | H | 1Me-5-Ind | C | | 391 (M⁺+1) |
| N-a-48 | 1-a | N-a-47 | | Et | Me | H | H | 1Me-5-Ind | C | | 363 (M⁺+1) |
| N-a-49 | 14n-2 | N-a-7 | CHO2 | Et | Me | Et | H | 1Et-5-Ind | C | | 405 (M⁺+1) |
| N-a-50 | 1-a | N-a-49 | | Et | Me | H | H | 1Et-5-Ind | C | | 377 (M⁺+1) |
| N-a-51 | 14n-2 | N-a-9 | CHO2 | Et | Me | Et | H | 1Me-4-Ind | C | | 391 (M⁺+1) |
| N-a-52 | 1-a | N-a-51 | | Et | Me | H | H | 1Me-4-Ind | C | | 363 (M⁺+1) |
| N-a-53 | 14n-2 | N-a-11 | CHO2 | Et | Me | Et | H | 1Me-6-Ind | C | | 391 (M⁺+1) |
| N-a-54 | 1-a | N-a-53 | | Et | Me | H | H | 1Me-6-Ind | C | | 363 (M⁺+1) |
| N-a-55 | 14n-2 | N-a-13 | CHO2 | Et | Me | Et | H | 5-1Idz | C | | 378 (M⁺+1) |
| N-a-56 | 1-a | N-a-55 | | Et | Me | H | H | 5-1Idz | C | | 350 (M⁺+1) |
| N-a-57 | 14n-2 | N-a-15 | CHO2 | Et | Me | Et | H | 1Me-5-Idz | C | | 392 (M⁺+1) |
| N-a-58 | 1-a | N-a-57 | | Et | Me | H | H | 1Me-5-Idz | C | | 364 (M⁺+1) |
| N-a-59 | 14n-2 | N-a-25 | CHO2 | Et | Me | Et | H | 3-Qu | C | | 389 (M⁺+1) |
| N-a-60 | 1-a | N-a-59 | | Et | Me | H | H | 3-Qu | C | | 361 (M⁺+1) |
| N-a-61 | 14n-2 | N-a-29 | CHO2 | Et | Me | Et | H | 6-IQ | C | | 389 (M⁺+1) |
| N-a-62 | 1-a | N-a-61 | | Et | Me | H | H | 6-IQ | C | | 361 (M⁺+1) |
| N-a-63 | 14n-2 | N-a-1 | CHO3 | nPr | Me | Et | H | 2-Nap | C | | 402 (M⁺+1) |
| N-a-64 | 1-a | N-a-63 | | nPr | Me | H | H | 2-Nap | C | | 374 (M⁺+1) |
| N-a-65 | 14n-2 | N-a-3 | CHO3 | nPr | Me | Et | H | 5-Ind | C | | 391 (M⁺+1) |
| N-a-66 | 1-a | N-a-65 | | nPr | Me | H | H | 5-Ind | C | | 363 (M⁺+1) |
| N-a-67 | 14n-2 | N-a-5 | CHO3 | nPr | Me | Et | H | 1Me-5-Ind | C | | 405 (M⁺+1) |
| N-a-68 | 1-a | N-a-67 | | nPr | Me | H | H | 1Me-5-Ind | C | | 377 (M⁺+1) |
| N-a-69 | 14n-2 | N-a-13 | CHO3 | nPr | Me | Et | H | 5-1Idz | C | | 392 (M⁺+1) |
| N-a-70 | 1-a | N-a-69 | | nPr | Me | H | H | 5-1Idz | C | | 364 (M⁺+1) |
| N-a-71 | 14n-2 | N-a-15 | CHO3 | nPr | Me | Et | H | 1Me-5-Idz | C | | 406 (M⁺+1) |
| N-a-72 | 1-a | N-a-71 | | nPr | Me | H | H | 1Me-5-Idz | C | | 378 (M⁺+1) |
| N-a-73 | 14n-2 | N-a-21 | CHO3 | nPr | Me | Et | H | 5-BF | C | | 392 (M⁺+1) |
| N-a-74 | 1-a | N-a-73 | | nPr | Me | H | H | 5-BF | C | | 364 (M⁺+1) |
| N-a-75 | 14n-2 | N-a-25 | CHO3 | nPr | Me | Et | H | 3-Qu | C | | 403 (M⁺+1) |
| N-a-76 | 1-a | N-a-75 | | nPr | Me | H | H | 3-Qu | C | | 375 (M⁺+1) |
| N-a-77 | 14n-2 | N-a-1 | CHO4 | iPr | Me | Et | H | 2-Nap | C | | 402 (M⁺+1) |
| N-a-78 | 1-a | N-a-77 | | iPr | Me | H | H | 2-Nap | C | | 374 (M⁺+1) |
| N-a-79 | 14n-2 | N-a-3 | CHO4 | iPr | Me | Et | H | 5-Ind | C | | 391 (M⁺+1) |
| N-a-80 | 1-a | N-a-79 | | iPr | Me | H | H | 5-Ind | C | | 363 (M⁺+1) |
| N-a-81 | 14n-2 | N-a-5 | CHO4 | iPr | Me | Et | H | 1Me-5-Ind | C | | 405 (M⁺+1) |
| N-a-82 | 1-a | N-a-81 | | iPr | Me | H | H | 1Me-5-Ind | C | | 377 (M⁺+1) |
| N-a-83 | 14n-2 | N-a-7 | CHO4 | iPr | Me | Et | H | 1Et-5-Ind | C | | 419 (M⁺+1) |
| N-a-84 | 1-a | N-a-83 | | iPr | Me | H | H | 1 Et-5-Ind | C | | 391 (M⁺+1) |
| N-a-85 | 14n-2 | N-a-11 | CHO4 | iPr | Me | Et | H | 1Me-6-Ind | C | | 405 (M⁺+1) |
| N-a-86 | 1-a | N-a-85 | | iPr | Me | H | H | 1Me-6-Ind | C | | 377 (M⁺+1) |
| N-a-87 | 14n-2 | N-a-13 | CHO4 | iPr | Me | Et | H | 5-1Idz | C | | 392 (M⁺+1) |
| N-a-88 | 1-a | N-a-87 | | iPr | Me | H | H | 5-1Idz | C | | 364 (M⁺+1) |
| N-a-89 | 14n-2 | N-a-15 | CHO4 | iPr | Me | Et | H | 1Me-5-Idz | C | | 406 (M⁺+1) |
| N-a-90 | 1-a | N-a-89 | | iPr | Me | H | H | 1Me-5-Idz | C | | 378 (M⁺+1) |

**[Table 130]**

| Table-N-A-3 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-a-91 | 14n-2 | N-a-1 | CHO5 | nBu | Me | Et | H | 2-Nap | C | | 416 (M⁺+1) |
| N-a-92 | 1-a | N-a-91 | | nBu | Me | H | H | 2-Nap | C | | 388 (M⁺+1) |
| N-a-93 | 14n-2 | N-a-3 | CHO5 | nBu | Me | Et | H | 5-Ind | C | | 405 (M⁺+1) |
| N-a-94 | 1-a | N-a-93 | | nBu | Me | H | H | 5-Ind | C | | 377 (M⁺+1) |
| N-a-95 | 14n-2 | N-a-5 | CHO5 | nBu | Me | Et | H | 1Me-5-Ind | C | | 419 (M⁺+1) |
| N-a-96 | 1-a | N-a-95 | | nBu | Me | H | H | 1Me-5-Ind | C | | 391 (M⁺+1) |
| N-a-97 | 14n-2 | N-a-11 | CHO5 | nBu | Me | Et | H | 1Me-6-Ind | C | | 419 (M⁺+1) |
| N-a-98 | 1-a | N-a-97 | | nBu | Me | H | H | 1Me-6-Ind | C | | 391 (M⁺+1) |
| N-a-99 | 14n-2 | N-a-13 | CHO5 | nBu | Me | Et | H | 5-1Idz | C | | 406 (M⁺+1) |
| N-a-100 | 1-a | N-a-99 | | nBu | Me | H | H | 5-1Idz | C | | 378 (M⁺+1) |
| N-a-101 | 14n-2 | N-a-15 | CHO5 | nBu | Me | Et | H | 1Me-5-Idz | C | | 420 (M⁺+1) |
| N-a-102 | 1-a | N-a-101 | | nBu | Me | H | H | 1Me-5-Idz | C | | 392 (M⁺+1) |
| N-a-103 | 14n-2 | N-a-25 | CHO5 | nBu | Me | Et | H | 3-Qu | C | | 417 (M⁺+1) |
| N-a-104 | 1-a | N-a-103 | | nBu | Me | H | H | 3-Qu | C | | 389 (M⁺+1) |
| N-a-105 | 14n-2 | N-a-27 | CHO5 | nBu | Me | Et | H | 6-Qu | C | | 417 (M⁺+1) |
| N-a-106 | 1-a | N-a-105 | | nBu | Me | H | H | 6-Qu | C | | 389 (M⁺+1) |
| N-a-107 | 14n-2 | N-a-1 | CHO6 | iBu | Me | Et | H | 2-Nap | C | | 416 (M⁺+1) |
| N-a-108 | 1-a | N-a-107 | | iBu | Me | H | H | 2-Nap | C | | 388 (M⁺+1) |
| N-a-109 | 14n-2 | N-a-3 | CHO6 | iBu | Me | Et | H | 5-Ind | C | | 405 (M⁺+1) |
| N-a-110 | 1-a | N-a-109 | | iBu | Me | H | H | 5-Ind | C | | 377 (M⁺+1) |
| N-a-111 | 14n-2 | N-a-5 | CHO6 | iBu | Me | Et | H | 1Me-5-Ind | C | | 419 (M⁺+1) |
| N-a-112 | 1-a | N-a-111 | | iBu | Me | H | H | 1Me-5-Ind | C | | 391 (M⁺+1) |
| N-a-113 | 14n-2 | N-a-7 | CHO6 | iBu | Me | Et | H | 1 Et-5-Ind | C | | 433 (M⁺+1) |
| N-a-114 | 1-a | N-a-113 | | iBu | Me | H | H | 1 Et-5-Ind | C | | 405 (M⁺+1) |
| N-a-115 | 14n-2 | N-a-11 | CHO6 | iBu | Me | Et | H | 1Me-6-Ind | C | | 419 (M⁺+1) |
| N-a-116 | 1-a | N-a-115 | | iBu | Me | H | H | 1Me-6-Ind | C | | 391 (M⁺+1) |
| N-a-117 | 14n-2 | N-a-13 | CHO6 | iBu | Me | Et | H | 5-1Idz | C | | 406 (M⁺+1) |
| N-a-118 | 1-a | N-a-117 | | iBu | Me | H | H | 5-1Idz | C | | 378 (M⁺+1) |
| N-a-119 | 14n-2 | N-a-15 | CHO6 | iBu | Me | Et | H | 1Me-5-Idz | C | | 420 (M⁺+1) |
| N-a-120 | 1-a | N-a-119 | | iBu | Me | H | H | 1Me-5-Idz | C | | 392 (M⁺+1) |
| N-a-121 | 14n-2 | N-a-17 | CHO6 | iBu | Me | Et | H | 1Et-5-Idz | C | | 434 (M⁺+1) |
| N-a-122 | 1-a | N-a-121 | | iBu | Me | H | H | 1Et-5-Idz | C | | 406 (M⁺+1) |
| N-a-123 | 14n-2 | N-a-1 | CHO7 | cPen | Me | Et | H | 2-Nap | C | | 428 (M⁺+1) |
| N-a-124 | 1-a | N-a-123 | | cPen | Me | H | H | 2-Nap | C | | 400 (M⁺+1) |
| N-a-125 | 14n-2 | N-a-3 | CHO7 | cPen | Me | Et | H | 5-Ind | C | | 417 (M⁺+1) |
| N-a-126 | 1-a | N-a-125 | | cPen | Me | H | H | 5-Ind | C | | 389 (M⁺+1) |
| N-a-127 | 14n-2 | N-a-5 | CHO7 | cPen | Me | Et | H | 1Me-5-Ind | C | | 431 (M⁺+1) |
| N-a-128 | 1-a | N-a-127 | | cPen | Me | H | H | 1Me-5-Ind | C | | 403 (M⁺+1) |
| N-a-129 | 14n-2 | N-a-7 | CHO7 | cPen | Me | Et | H | 1 Et-5-Ind | C | | 445 (M⁺+1) |
| N-a-130 | 1-a | N-a-129 | | cPen | Me | H | H | 1 Et-5-Ind | C | | 417 (M⁺+1) |
| N-a-131 | 14n-2 | N-a-13 | CHO7 | cPen | Me | Et | H | 5-1Idz | C | | 418 (M⁺+1) |
| N-a-132 | 1-a | N-a-131 | | cPen | Me | H | H | 5-1Idz | C | | 390 (M⁺+1) |
| N-a-133 | 14n-2 | N-a-15 | CHO7 | cPen | Me | Et | H | 1Me-5-Idz | C | | 432 (M⁺+1) |
| N-a-134 | 1-a | N-a-133 | | cPen | Me | H | H | 1Me-5-Idz | C | | 404 (M⁺+1) |
| N-a-135 | 14n-2 | N-a-17 | CHO7 | cPen | Me | Et | H | 1Et-5-Idz | C | | 446 (M⁺+1) |
| N-a-136 | 1-a | N-a-135 | | cPen | Me | H | H | 1Et-5-Idz | C | | 418 (M⁺+1) |

**[Table 131]**

| Table-N-A-4 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-a-137 | 14n-2 | N-a-1 | CHO8 | cHex | Me | Et | H | 2-Nap | C | | 442 (M⁺+1) |
| N-a-138 | 1-a | N-a-137 | | cHex | Me | H | H | 2-Nap | C | | 414 (M⁺+1) |
| N-a-139 | 14n-2 | N-a-3 | CHO8 | cHex | Me | Et | H | 5-Ind | C | | 431 (M⁺+1) |
| N-a-140 | 1-a | N-a-139 | | cHex | Me | H | H | 5-Ind | C | | 403 (M⁺+1) |
| N-a-141 | 14n-2 | N-a-5 | CHO8 | cHex | Me | Et | H | 1Me-5-Ind | C | | 445 (M⁺+1) |
| N-a-142 | 1-a | N-a-141 | | cHex | Me | H | H | 1Me-5-Ind | C | | 417 (M⁺+1) |
| N-a-143 | 14n-2 | N-a-9 | CHO8 | cHex | Me | Et | H | 1Me-4-Ind | C | | 445 (M⁺+1) |
| N-a-144 | 1-a | N-a-143 | | cHex | Me | H | H | 1Me-4-Ind | C | | 417 (M⁺+1) |
| N-a-145 | 14n-2 | N-a-11 | CHO8 | cHex | Me | Et | H | 1Me-6-Ind | C | | 445 (M⁺+1) |
| N-a-146 | 1-a | N-a-145 | | cHex | Me | H | H | 1Me-6-Ind | C | | 417 (M⁺+1) |
| N-a-147 | 14n-2 | N-a-13 | CHO8 | cHex | Me | Et | H | 5-1Idz | C | | 432 (M⁺+1) |
| N-a-148 | 1-a | N-a-147 | | cHex | Me | H | H | 5-1Idz | C | | 404 (M⁺+1) |
| N-a-149 | 14n-2 | N-a-15 | CHO8 | cHex | Me | Et | H | 1Me-5-Idz | C | | 446 (M⁺+1) |
| N-a-150 | 1-a | N-a-149 | | cHex | Me | H | H | 1Me-5-Idz | C | | 418 (M⁺+1) |
| N-a-151 | 14n-2 | N-a-17 | CHO8 | cHex | Me | Et | H | 1Et-5-Idz | C | | 460 (M⁺+1) |
| N-a-152 | 1-a | N-a-151 | | cHex | Me | H | H | 1Et-5-Idz | C | | 432 (M⁺+1) |
| N-a-153 | 14n-2 | N-a-1 | CHO9 | 2MecHex | Me | Et | H | 2-Nap | C | | 456 (M⁺+1) |
| N-a-154 | 1-a | N-a-153 | | 2MecHex | Me | H | H | 2-Nap | C | | 428 (M⁺+1) |
| N-a-155 | 14n-2 | N-a-3 | CHO9 | 2MecHex | Me | Et | H | 5-Ind | C | | 445 (M⁺+1) |
| N-a-156 | 1-a | N-a-155 | | 2MecHex | Me | H | H | 5-Ind | C | | 417 (M⁺+1) |
| N-a-157 | 14n-2 | N-a-5 | CHO9 | 2MecHex | Me | Et | H | 1Me-5-Ind | C | | 459 (M⁺+1) |
| N-a-158 | 1-a | N-a-157 | | 2MecHex | Me | H | H | 1Me-5-Ind | C | | 431 (M⁺+1) |
| N-a-159 | 14n-2 | N-a-11 | CHO9 | 2MecHex | Me | Et | H | 1Me-6-Ind | C | | 459 (M⁺+1) |
| N-a-160 | 1-a | N-a-159 | | 2MecHex | Me | H | H | 1Me-6-Ind | C | | 431 (M⁺+1) |
| N-a-161 | 14n-2 | N-a-13 | CHO9 | 2MecHex | Me | Et | H | 5-1Idz | C | | 446 (M⁺+1) |
| N-a-162 | 1-a | N-a-161 | | 2MecHex | Me | H | H | 5-1 Idz | C | | 418 (M⁺+1) |
| N-a-163 | 14n-2 | N-a-15 | CHO9 | 2MecHex | Me | Et | H | 1Me-5-Idz | C | | 460 (M⁺+1) |
| N-a-164 | 1-a | N-a-163 | | 2MecHex | Me | H | H | 1Me-5-Idz | C | | 432 (M⁺+1) |
| N-a-165 | 14n-2 | N-a-1 | CHO10 | 2Indane | Me | Et | H | 2-Nap | C | | 476 (M⁺+1) |
| N-a-166 | 1-a | N-a-165 | | 2Indane | Me | H | H | 2-Nap | C | | 448 (M⁺+1) |
| N-a-167 | 14n-2 | N-a-3 | CHO10 | 2Indane | Me | Et | H | 5-Ind | C | | 465 (M⁺+1) |
| N-a-168 | 1-a | N-a-167 | | 2Indane | Me | H | H | 5-Ind | C | | 437 (M⁺+1) |
| N-a-169 | 14n-2 | N-a-5 | CHO10 | 2Indane | Me | Et | H | 1Me-5-Ind | C | | 479 (M⁺+1) |
| N-a-170 | 1-a | N-a-169 | | 2Indane | Me | H | H | 1Me-5-Ind | C | | 451 (M⁺+1) |
| N-a-171 | 14n-2 | N-a-13 | CHO10 | 2Indane | Me | Et | H | 5-1Idz | C | | 466 (M⁺+1) |
| N-a-172 | 1-a | N-a-171 | | 2Indane | Me | H | H | 5-1Idz | C | | 438 (M⁺+1) |
| N-a-173 | 14n-2 | N-a-15 | CHO10 | 2Indane | Me | Et | H | 1Me-5-Idz | C | | 480 (M⁺+1) |
| N-a-174 | 1-a | N-a-173 | | 2Indane | Me | H | H | 1Me-5-Idz | C | | 452 (M⁺+1) |
| N-a-175 | 4e-1 | Int.n-b-6 | BRA1 | H | Et | Et | H | 2-Nap | C | | 374 (M⁺+1) |
| N-a-176 | 1-a | N-a-175 | | H | Et | H | H | 2-Nap | C | | 346 (M⁺+1) |
| N-a-177 | 4e-1 | Int.n-b-6 | BRA2 | H | Et | Et | H | 5-Ind | C | | 363 (M⁺+1) |
| N-a-178 | 1-a | N-a-177 | | H | Et | H | H | 5-Ind | C | | 335 (M⁺+1) |
| N-a-179 | 4e-1 | Int.n-b-6 | BRA3 | H | Et | Et | H | 1Me-5-Ind | C | | 377 (M⁺+1) |
| N-a-180 | 1-a | N-a-179 | | H | Et | H | H | 1Me-5-Ind | C | | 349 (M⁺+1) |
| N-a-181 | 4e-1 | Int.n-b-6 | BRA4 | H | Et | Et | H | 1Et-5-Ind | C | | 391 (M⁺+1) |
| N-a-182 | 1-a | N-a-181 | | H | Et | H | H | 1Et-5-Ind | C | | 363 (M⁺+1) |

**[Table 132]**

| Table-N-A-5 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-a-183 | 4e-1 | Int.n-b-6 | BRA7 | H | Et | Et | H | 5-1Idz | C | | 364 (M⁺+1) |
| N-a-184 | 1-a | N-a-183 | | H | Et | H | H | 5-1Idz | C | | 336 (M⁺+1) |
| N-a-185 | 4e-1 | Int.n-b-6 | BRA8 | H | Et | Et | H | 1Me-5-Idz | C | | 378 (M⁺+1) |
| N-a-186 | 1-a | N-a-185 | | H | Et | H | H | 1Me-5-Idz | C | | 350 (M⁺+1) |
| N-a-187 | 4e-1 | Int.n-b-6 | BRA9 | H | Et | Et | H | 1Et-5-Idz | C | | 392 (M⁺+1) |
| N-a-188 | 1-a | N-a-187 | | H | Et | H | H | 1Et-5-Idz | C | | 364 (M⁺+1) |
| N-a-189 | 14n-2 | N-a-175 | CHO2 | Et | Et | Et | H | 2-Nap | C | | 402 (M⁺+1) |
| N-a-190 | 1-a | N-a-189 | | Et | Et | H | H | 2-Nap | C | | 374 (M⁺+1) |
| N-a-191 | 14n-2 | N-a-177 | CHO2 | Et | Et | Et | H | 5-Ind | C | | 391 (M⁺+1) |
| N-a-192 | 1-a | N-a-191 | | Et | Et | H | H | 5-Ind | C | | 363 (M⁺+1) |
| N-a-193 | 14n-2 | N-a-179 | CHO2 | Et | Et | Et | H | 1Me-5-Ind | C | | 405 (M⁺+1) |
| N-a-194 | 1-a | N-a-193 | | Et | Et | H | H | 1Me-5-Ind | C | | 377 (M⁺+1) |
| N-a-195 | 14n-2 | N-a-183 | CHO2 | Et | Et | Et | H | 5-1Idz | C | | 392 (M⁺+1) |
| N-a-196 | 1-a | N-a-195 | | Et | Et | H | H | 5-1Idz | C | | 364 (M⁺+1) |
| N-a-197 | 14n-2 | N-a-185 | CHO2 | Et | Et | Et | H | 1Me-5-Idz | C | | 406 (M⁺+1) |
| N-a-198 | 1-a | N-a-197 | | Et | Et | H | H | 1Me-5-Idz | C | | 378 (M⁺+1) |
| N-a-199 | 14n-2 | N-a-187 | CHO2 | Et | Et | Et | H | 1Et-5-Idz | C | | 420 (M⁺+1) |
| N-a-200 | 1-a | N-a-199 | | Et | Et | H | H | 1Et-5-Idz | C | | 392 (M⁺+1) |
| N-a-201 | 14n-2 | N-a-175 | CHO3 | nPr | Et | Et | H | 2-Nap | C | | 416 (M⁺+1) |
| N-a-202 | 1-a | N-a-201 | | nPr | Et | H | H | 2-Nap | C | | 388 (M⁺+1) |
| N-a-203 | 14n-2 | N-a-177 | CHO3 | nPr | Et | Et | H | 5-Ind | C | | 405 (M⁺+1) |
| N-a-204 | 1-a | N-a-203 | | nPr | Et | H | H | 5-Ind | C | | 377 (M⁺+1) |
| N-a-205 | 14n-2 | N-a-179 | CHO3 | nPr | Et | Et | H | 1 Me-5-Ind | C | | 419 (M⁺+1) |
| N-a-206 | 1-a | N-a-205 | | nPr | Et | H | H | 1 Me-5-Ind | C | | 391 (M⁺+1) |
| N-a-207 | 14n-2 | N-a-183 | CHO3 | nPr | Et | Et | H | 5-1Idz | C | | 406 (M⁺+1) |
| N-a-208 | 1-a | N-a-207 | | nPr | Et | H | H | 5-1Idz | C | | 378 (M⁺+1) |
| N-a-209 | 14n-2 | N-a-185 | CHO3 | nPr | Et | Et | H | 1Me-5-Idz | C | | 420 (M⁺+1) |
| N-a-210 | 1-a | N-a-209 | | nPr | Et | H | H | 1Me-5-Idz | C | | 392 (M⁺+1) |
| N-a-211 | 14n-2 | N-a-175 | CHO4 | iPr | Et | Et | H | 2-Nap | C | | 416 (M⁺+1) |
| N-a-212 | 1-a | N-a-211 | | iPr | Et | H | H | 2-Nap | C | | 388 (M⁺+1) |
| N-a-213 | 14n-2 | N-a-177 | CHO4 | iPr | Et | Et | H | 5-Ind | C | | 405 (M⁺+1) |
| N-a-214 | 1-a | N-a-213 | | iPr | Et | H | H | 5-Ind | C | | 377 (M⁺+1) |
| N-a-215 | 14n-2 | N-a-181 | CHO4 | iPr | Et | Et | H | 1Et-5-Ind | C | | 433 (M⁺+1) |
| N-a-216 | 1-a | N-a-215 | | iPr | Et | H | H | 1Et-5-Ind | C | | 405 (M⁺+1) |
| N-a-217 | 14n-2 | N-a-183 | CHO4 | iPr | Et | Et | H | 5-1Idz | C | | 406 (M⁺+1) |
| N-a-218 | 1-a | N-a-217 | | iPr | Et | H | H | 5-1Idz | C | | 378 (M⁺+1) |
| N-a-219 | 14n-2 | N-a-187 | CHO4 | iPr | Et | Et | H | 1Et-5-Idz | C | | 434 (M⁺+1) |
| N-a-220 | 1-a | N-a-219 | | iPr | Et | H | H | 1Et-5-Idz | C | | 406 (M⁺+1) |
| N-a-221 | 14n-2 | N-a-175 | CHO5 | nBu | Et | Et | H | 2-Nap | C | | 430 (M⁺+1) |
| N-a-222 | 1-a | N-a-221 | | nBu | Et | H | H | 2-Nap | C | | 402 (M⁺+1) |
| N-a-223 | 14n-2 | N-a-177 | CHO5 | nBu | Et | Et | H | 5-Ind | C | | 419 (M⁺+1) |
| N-a-224 | 1-a | N-a-223 | | nBu | Et | H | H | 5-Ind | C | | 391 (M⁺+1) |
| N-a-225 | 14n-2 | N-a-183 | CHO5 | nBu | Et | Et | H | 5-1Idz | C | | 420 (M⁺+1) |
| N-a-226 | 1-a | N-a-225 | | nBu | Et | H | H | 5-1Idz | C | | 392 (M⁺+1) |
| N-a-227 | 14n-2 | N-a-185 | CHO5 | nBu | Et | Et | H | 1Me-5-Idz | C | | 434 (M⁺+1) |
| N-a-228 | 1-a | N-a-227 | | nBu | Et | H | H | 1Me-5-Idz | C | | 406 (M⁺+1) |

**[Table 133]**

| Table-N-A-6 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-a-229 | 14n-2 | N-a-175 | CHO6 | iBu | Et | Et | H | 2-Nap | C | | 430 (M⁺+1) |
| N-a-230 | 1-a | N-a-229 | | iBu | Et | H | H | 2-Nap | C | | 402 (M⁺+1) |
| N-a-231 | 14n-2 | N-a-177 | CHO6 | iBu | Et | Et | H | 5-Ind | C | | 419 (M⁺+1) |
| N-a-232 | 1-a | N-a-231 | | iBu | Et | H | H | 5-Ind | C | | 391 (M⁺+1) |
| N-a-233 | 14n-2 | N-a-179 | CHO6 | iBu | Et | Et | H | 1Me-5-Ind | C | | 433 (M⁺+1) |
| N-a-234 | 1-a | N-a-233 | | iBu | Et | H | H | 1Me-5-Ind | C | | 405 (M⁺+1) |
| N-a-235 | 14n-2 | N-a-183 | CHO6 | iBu | Et | Et | H | 5-1Idz | C | | 420 (M⁺+1) |
| N-a-236 | 1-a | N-a-235 | | iBu | Et | H | H | 5-1Idz | C | | 392 (M⁺+1) |
| N-a-237 | 14n-2 | N-a-185 | CHO6 | iBu | Et | Et | H | 1Me-5-Idz | C | | 434 (M⁺+1) |
| N-a-238 | 1-a | N-a-237 | | iBu | Et | H | H | 1Me-5-Idz | C | | 406 (M⁺+1) |
| N-a-239 | 14n-2 | N-a-187 | CHO6 | iBu | Et | Et | H | 1Et-5-Idz | C | | 448 (M⁺+1) |
| N-a-240 | 1-a | N-a-239 | | iBu | Et | H | H | 1Et-5-Idz | C | | 420 (M⁺+1) |
| N-a-241 | 14n-2 | N-a-175 | CHO7 | cPen | Et | Et | H | 2-Nap | C | | 442 (M⁺+1) |
| N-a-242 | 1-a | N-a-241 | | cPen | Et | H | H | 2-Nap | C | | 414 (M⁺+1) |
| N-a-243 | 14n-2 | N-a-177 | CHO7 | cPen | Et | Et | H | 5-Ind | C | | 431 (M⁺+1) |
| N-a-244 | 1-a | N-a-243 | | cPen | Et | H | H | 5-Ind | C | | 403 (M⁺+1) |
| N-a-245 | 14n-2 | N-a-179 | CHO7 | cPen | Et | Et | H | 1Me-5-Ind | C | | 445 (M⁺+1) |
| N-a-246 | 1-a | N-a-245 | | cPen | Et | H | H | 1Me-5-Ind | C | | 417 (M⁺+1) |
| N-a-247 | 14n-2 | N-a-181 | CHO7 | cPen | Et | Et | H | 1Et-5-Ind | C | | 459 (M⁺+1) |
| N-a-248 | 1-a | N-a-247 | | cPen | Et | H | H | 1Et-5-Ind | C | | 431 (M⁺+1) |
| N-a-249 | 14n-2 | N-a-183 | CHO7 | cPen | Et | Et | H | 5-1Idz | C | | 432 (M⁺+1) |
| N-a-250 | 1-a | N-a-249 | | cPen | Et | H | H | 5-1Idz | C | | 404 (M⁺+1) |
| N-a-251 | 14n-2 | N-a-185 | CHO7 | cPen | Et | Et | H | 1Me-5-Idz | C | | 446 (M⁺+1) |
| N-a-252 | 1-a | N-a-251 | | cPen | Et | H | H | 1Me-5-Idz | C | | 418 (M⁺+1) |
| N-a-253 | 14n-2 | N-a-187 | CHO7 | cPen | Et | Et | H | 1Et-5-Idz | C | | 460 (M⁺+1) |
| N-a-254 | 1-a | N-a-253 | | cPen | Et | H | H | 1Et-5-Idz | C | | 432 (M⁺+1) |
| N-a-255 | 14n-2 | N-a-175 | CHO8 | cHex | Et | Et | H | 2-Nap | C | | 456 (M⁺+1) |
| N-a-256 | 1-a | N-a-255 | | cHex | Et | H | H | 2-Nap | C | | 428 (M⁺+1) |
| N-a-257 | 14n-2 | N-a-177 | CHO8 | cHex | Et | Et | H | 5-Ind | C | | 445 (M⁺+1) |
| N-a-258 | 1-a | N-a-257 | | cHex | Et | H | H | 5-Ind | C | | 417 (M⁺+1) |
| N-a-259 | 14n-2 | N-a-179 | CH08 | cHex | Et | Et | H | 1Me-5-Ind | C | | 459 (M⁺+1) |
| N-a-260 | 1-a | N-a-259 | | cHex | Et | H | H | 1Me-5-Ind | C | | 431 (M⁺+1) |
| N-a-261 | 14n-2 | N-a-183 | CHO8 | cHex | Et | Et | H | 5-1Idz | C | | 446 (M⁺+1) |
| N-a-262 | 1-a | N-a-261 | | cHex | Et | H | H | 5-1Idz | C | | 418 (M⁺+1) |
| N-a-263 | 14n-2 | N-a-185 | CH08 | cHex | Et | Et | H | 1Me-5-Idz | C | | 460 (M⁺+1) |
| N-a-264 | 1-a | N-a-263 | | cHex | Et | H | H | 1Me-5-Idz | C | | 432 (M⁺+1) |
| N-a-265 | 14n-2 | N-a-175 | CHO10 | 2Indane | Et | Et | H | 2-Nap | C | | 490 (M⁺+1) |
| N-a-266 | 1-a | N-a-265 | | 2Indane | Et | H | H | 2-Nap | C | | 462 (M⁺+1) |
| N-a-267 | 14n-2 | N-a-177 | CHO10 | 2Indane | Et | Et | H | 5-Ind | C | | 479 (M⁺+1) |
| N-a-268 | 1-a | N-a-267 | | 2Indane | Et | H | H | 5-Ind | C | | 451 (M⁺+1) |
| N-a-269 | 14n-2 | N-a-179 | CHO10 | 2Indane | Et | Et | H | 1Me-5-Ind | C | | 493 (M⁺+1) |
| N-a-270 | 1-a | N-a-269 | | 2Indane | Et | H | H | 1Me-5-Ind | C | | 465 (M⁺+1) |
| N-a-271 | 14n-2 | N-a-183 | CHO10 | 2Indane | Et | Et | H | 5-1Idz | C | | 480 (M⁺+1) |
| N-a-272 | 1-a | N-a-271 | | 2Indane | Et | H | H | 5-1Idz | C | | 452 (M⁺+1) |
| N-a-273 | 14n-2 | N-a-185 | CHO10 | 2Indane | Et | Et | H | 1Me-5-Idz | C | | 494 (M⁺+1) |
| N-a-274 | 1-a | N-a-273 | | 2Indane | Et | H | H | 1Me-5-Idz | C | | 466 (M⁺+1) |

### Example N-b-1

### Synthesis of ethyl 2-[5-benzylamino-6-(naphthalen-2-yl)-2,3-dihydro-1H-inden-1-yl]acetate (Compound No. N-b-1) (Preparation Method 5, Step d)

A solution of Intermediate c-a-1 (110.4 mg) in toluene (2.5 ml) was added with 2,2'-bis(diphenylphosphino)-1,1'-binaphthalene (henceforth abbreviated as "BINAP", 48.8 mg, Ald), potassium phosphate (91.8 mg, WAKO) and benzylamine (70 µl, TCI), and added with tris(dibenzylideneacetone)dipalladium(0) (henceforth abbreviated as "Pd₂(dba)₃", 31.6 mg, Ald) under a nitrogen atmosphere, and then the reaction mixture was stirred at 100°C for 14 hours, then added with 2-(di-t-butylphosphino)biphenyl (31.6 mg, WAKO), and further stirred for 24 hours. The reaction mixture was added with ethyl acetate (30 ml) and water (15 ml) for extraction, and then the organic layer was washed with saturated brine, dried, and then concentrated under reduced pressure. The residue was purified by column chromatography (Quad, hexane:ethyl acetate = 4:1) to obtain the title compound (Compound No. N-b-1, 24.4 mg).

### Example N-b-45

### Synthesis of ethyl 2-[5-(2-fluorobenzylamino)-6-(naphthalen-2-yl)-2,3-dihydro-1H-inden-1-yl]acetate (Compound No. N-b-45) (Preparation Method 14, Step n-2)

According to the procedure described in the synthetic method of Example Compound N-a-43 (Preparation Method 14, Step n-2) provided that the reaction was performed for 18 hours, Intermediate n-b-8 (115.3 mg), 2-fluorobenzaldehyde (75 µl, TCI), sodium triacetoxyborohydride (231.2 mg, Ald) and acetic acid (90 µL) were reacted and treated to obtain the title compound (Compound No. N-b-45, 82.2 mg).
Typical examples of the compounds of the present invention that can be obtained by reacting and treating corresponding starting compounds using any of the methods described in the present specification including the examples described above are shown in Table-N-B-1 to Table-N-B-9. The compounds were synthesized according to the preparation methods of the compounds of the compound numbers shown in the columns of "Syn" in the tables. "Int" means an intermediate compound number. As for the preparation of the compounds, used methods among the aforementioned synthetic methods are shown in the columns of "Syn" with symbols, the starting compounds are shown in the columns of "SM", and the reagents are shown in the columns of "Regent" in the tables. The formyl regents mentioned in the columns of "Reagent" with the symbols "CHO (No.)" are those mentioned in Table-CHO.

**[Table 134]**

| Table-N-B-1 | | | | | | | | | | | |
|---|---|---|---|---|---|---|---|---|---|---|---|
| 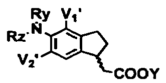 | | | | | | | | | | | |

| Exp. | Syn. | SM1 | Reagent | Ry | Rz | Y | V₁' | V₂' | LCMS | | |
|---|---|---|---|---|---|---|---|---|---|---|---|
| | | | | | | | | | method | RTime | Mass |
| N-b-1 | 4e-1 | Int.c-a-1 | | Bn | H | Et | H | 2-Nap | C | | 436 (M⁺+1) |
| N-b-2 | 1-a | N-b-1 | | Bn | H | H | H | 2-Nap | C | | 408 (M⁺+1) |
| N-b-3 | 4e-1 | Int.c-a-2 | | Bn | H | Et | H | 5-Ind | C | | 425 (M⁺+1) |
| N-b-4 | 1-a | N-b-3 | | Bn | H | H | H | 5-Ind | C | | 397 (M⁺+1) |
| N-b-5 | 4e-1 | Int.c-a-3 | | Bn | H | Et | H | 1Me-5-Ind | C | | 439 (M⁺+1) |
| N-b-6 | 1-a | N-b-5 | | Bn | H | H | H | 1Me-5-Ind | C | | 411 (M⁺+1) |
| N-b-7 | 4e-1 | Int.c-a-4 | | Bn | H | Et | H | 5-1Idz | C | | 426 (M⁺+1) |
| N-b-8 | 1-a | N-b-7 | | Bn | H | H | H | 5-1Ids | C | | 398 (M⁺+1) |
| N-b-9 | 4e-1 | Int.c-a-5 | | Bn | H | Et | H | 1Me-5-Idz | C | | 440 (M⁺+1) |
| N-b-10 | 1-a | N-b-9 | | Bn | H | H | H | 1Me-5-Idz | C | | 412 (M⁺+1) |
| N-b-11 | 4e-1 | Int.c-a-6 | | Bn | H | Et | H | 1Et-5-Idz | C | | 454 (M⁺+1) |
| N-b-12 | 1-a | N-b-11 | | Bn | H | H | H | 1Et-5-Idz | C | | 426 (M⁺+1) |
| N-b-13 | 4e-1 | Int.c-a-7 | | Bn | H | Et | H | 5-BF | C | | 426 (M⁺+1) |
| N-b-14 | 1-a | N-b-13 | | Bn | H | H | H | 5-BF | C | | 398 (M⁺+1) |
| N-b-15 | 4e-1 | Int.c-a-8 | | Bn | H | Et | H | 6-Qu | C | | 437 (M⁺+1) |
| N-b-16 | 1-a | N-b-15 | | Bn | H | H | H | 6-Qu | C | | 409 (M⁺+1) |
| N-b-17 | 14n-2 | N-b-1 | CHO1 | Bn | Me | Et | H | 2-Nap | C | | 450 (M⁺+1) |
| N-b-18 | 1-a | N-b-17 | | Bn | Me | H | H | 2-Nap | C | | 422 (M⁺+1) |
| N-b-19 | 14n-2 | N-b-3 | CHO1 | Bn | Me | Et | H | 5-Ind | C | | 439 (M⁺+1) |
| N-b-20 | 1-a | N-b-19 | | Bn | Me | H | H | 5-Ind | C | | 411 (M⁺+1) |
| N-b-21 | 14n-2 | N-b-5 | CHO1 | Bn | Me | Et | H | 1Me-5-Ind | C | | 453 (M⁺+1) |
| N-b-22 | 1-a | N-b-21 | | Bn | Me | H | H | 1Me-5-Ind | C | | 425 (M⁺+1) |
| N-b-23 | 14n-2 | N-b-7 | CHO1 | Bn | Me | Et | H | 5-1Idz | C | | 440 (M⁺+1) |
| N-b-24 | 1-a | N-b-23 | | Bn | Me | H | H | 5-1Idz | C | | 412 (M⁺+1) |
| N-b-25 | 14n-2 | N-b-9 | CHO1 | Bn | Me | Et | H | 1Me-5-Idz | C | | 454 (M⁺+1) |
| N-b-26 | 1-a | N-b-25 | | Bn | Me | H | H | 1Me-5-Idz | C | | 426 (M⁺+1) |
| N-b-27 | 14n-2 | N-b-11 | CHO1 | Bn | Me | Et | H | 1Et-5-Idz | C | | 468 (M⁺+1) |
| N-b-28 | 1-a | N-b-27 | | Bn | Me | H | H | 1Et-5-Idz | C | | 440 (M⁺+1) |
| N-b-29 | 14n-2 | N-b-13 | CHO1 | Bn | Me | Et | H | 5-BF | C | | 440 (M⁺+1) |
| N-b-30 | 1-a | N-b-29 | | Bn | Me | H | H | 5-BF | C | | 412 (M⁺+1) |
| N-b-31 | 14n-2 | N-b-1 | CHO2 | Bn | Et | Et | H | 2-Nap | C | | 464 (M⁺+1) |
| N-b-32 | 1-a | N-b-31 | | Bn | Et | H | H | 2-Nap | C | | 436 (M⁺+1) |
| N-b-33 | 14n-2 | N-b-3 | CHO2 | Bn | Et | Et | H | 5-Ind | C | | 453 (M⁺+1) |
| N-b-34 | 1-a | N-b-33 | | Bn | Et | H | H | 5-Ind | C | | 425 (M⁺+1) |
| N-b-35 | 14n-2 | N-b-5 | CHO2 | Bn | Et | Et | H | 1Me-5-Ind | C | | 467 (M⁺+1) |
| N-b-36 | 1-a | N-b-35 | | Bn | Et | H | H | 1Me-5-Ind | C | | 439 (M⁺+1) |
| N-b-37 | 14n-2 | N-b-7 | CHO2 | Bn | Et | Et | H | 5-1Idz | C | | 454 (M⁺+1) |
| N-b-38 | 1-a | N-b-37 | | Bn | Et | H | H | 5-1Idz | C | | 426 (M⁺+1) |
| N-b-39 | 14n-2 | N-b-9 | CHO2 | Bn | Et | Et | H | 1Me-5-Idz | C | | 468 (M⁺+1) |
| N-b-40 | 1-a | N-b-39 | | Bn | Et | H | H | 1Me-5-Idz | C | | 440 (M⁺+1) |
| N-b-41 | 14n-2 | N-b-11 | CHO2 | Bn | Et | Et | H | 1Et-5-Idz | C | | 482 (M⁺+1) |
| N-b-42 | 1-a | N-b-41 | | Bn | Et | H | H | 1Et-5-Idz | C | | 454 (M⁺+1) |
| N-b-43 | 14n-2 | N-b-15 | CHO2 | Bn | Et | Et | H | 6-Qu | C | | 465 (M⁺+1) |
| N-b-44 | 1-a | N-b-43 | | Bn | Et | H | H | 6-Qu | C | | 437 (M⁺+1) |

**[Table 135]**

| Table-N-B-2 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-b-45 | 14n-2 | Int.n-b-8 | CHO11 | 4FBn | H | Et | H | 2-Nap | C | | 454 (M⁺+1) |
| N-b-46 | 1-a | N-b-45 | | 4FBn | H | H | H | 2-Nap | C | | 426 (M⁺+1) |
| N-b-47 | 14n-2 | Int.n-b-9 | CHO11 | 4FBn | H | Et | H | 5-Ind | C | | 443 (M⁺+1) |
| N-b-48 | 1-a | N-b-47 | | 4FBn | H | H | H | 5-Ind | C | | 415 (M⁺+1) |
| N-b-49 | 14n-2 | Int.n-b-10 | CHO11 | 4FBn | H | Et | H | 1Me-5-Ind | C | | 457 (M⁺+1) |
| N-b-50 | 1-a | N-b-49 | | 4FBn | H | H | H | 1Me-5-Ind | C | | 429 (M⁺+1) |
| N-b-51 | 14n-2 | Int.n-b-11 | CHO11 | 4FBn | H | Et | H | 5-1Idz | C | | 444 (M⁺+1) |
| N-b-52 | 1-a | N-b-51 | | 4FBn | H | H | H | 5-1Idz | C | | 416 (M⁺+1) |
| N-b-53 | 14n-2 | Int.n-b-12 | CHO11 | 4FBn | H | Et | H | 1Me-5-Idz | C | | 458 (M⁺+1) |
| N-b-54 | 1-a | N-b-53 | | 4FBn | H | H | H | 1Me-5-Idz | C | | 430 (M⁺+1) |
| N-b-55 | 14n-2 | N-b-45 | CHO1 | 4FBn | Me | Et | H | 2-Nap | C | | 468 (M⁺+1) |
| N-b-56 | 1-a | N-b-55 | | 4FBn | Me | H | H | 2-Nap | C | | 440 (M⁺+1) |
| N-b-57 | 14n-2 | N-b-47 | CHO1 | 4FBn | Me | Et | H | 5-Ind | C | | 457 (M⁺+1) |
| N-b-58 | 1-a | N-b-57 | | 4FBn | Me | H | H | 5-Ind | C | | 429 (M⁺+1) |
| N-b-59 | 14n-2 | N-b-49 | CHO1 | 4FBn | Me | Et | H | 1Me-5-Ind | C | | 471 (M⁺+1) |
| N-b-60 | 1-a | N-b-59 | | 4FBn | Me | H | H | 1Me-5-Ind | C | | 443 (M⁺+1) |
| N-b-61 | 14n-2 | N-b-51 | CHO1 | 4FBn | Me | Et | H | 5-1Idz | C | | 458 (M⁺+1) |
| N-b-62 | 1-a | N-b-61 | | 4FBn | Me | H | H | 5-1Idz | C | | 430 (M⁺+1) |
| N-b-63 | 14n-2 | N-b-53 | CHO1 | 4FBn | Me | Et | H | 1Me-5-Idz | C | | 472 (M⁺+1) |
| N-b-64 | 1-a | N-b-63 | | 4FBn | Me | H | H | 1Me-5-Idz | C | | 444 (M⁺+1) |
| N-b-65 | 14n-2 | N-b-45 | CHO2 | 4FBn | Et | Et | H | 2-Nap | C | | 482 (M⁺+1) |
| N-b-66 | 1-a | N-b-65 | | 4FBn | Et | H | H | 2-Nap | C | | 454 (M⁺+1) |
| N-b-67 | 14n-2 | N-b-47 | CHO2 | 4FBn | Et | Et | H | 5-Ind | C | | 471 (M⁺+1) |
| N-b-68 | 1-a | N-b-67 | | 4FBn | Et | H | H | 5-Ind | C | | 443 (M⁺+1) |
| N-b-69 | 14n-2 | N-b-49 | CHO2 | 4FBn | Et | Et | H | 1Me-5-Ind | C | | 485 (M⁺+1) |
| N-b-70 | 1-a | N-b-69 | | 4FBn | Et | H | H | 1Me-5-Ind | C | | 457 (M⁺+1) |
| N-b-71 | 14n-2 | N-b-51 | CHO2 | 4FBn | Et | Et | H | 5-1Idz | C | | 472 (M⁺+1) |
| N-b-72 | 1-a | N-b-71 | | 4FBn | Et | H | H | 5-1Idz | C | | 444 (M⁺+1) |
| N-b-73 | 14n-2 | N-b-53 | CHO2 | 4FBn | Et | Et | H | 1Me-5-Idz | C | | 486 (M⁺+1) |
| N-b-74 | 1-a | N-b-73 | | 4FBn | Et | H | H | 1 Me-5-Idz | C | | 458 (M⁺+1) |
| N-b-75 | 14n-2 | Int.n-b-8 | CHO12 | 2FBn | H | Et | H | 2-Nap | C | | 454 (M⁺+1) |
| N-b-76 | 1-a | N-b-75 | | 2FBn | H | H | H | 2-Nap | C | | 426 (M⁺+1) |
| N-b-77 | 14n-2 | Int.n-b-9 | CHO12 | 2FBn | H | Et | H | 5-Ind | C | | 443 (M⁺+1) |
| N-b-78 | 1-a | N-b-77 | | 2FBn | H | H | H | 5-Ind | C | | 415 (M⁺+1) |
| N-b-79 | 14n-2 | Int.n-b-10 | CHO12 | 2FBn | H | Et | H | 1Me-5-Ind | C | | 457 (M⁺+1) |
| N-b-80 | 1-a | N-b-79 | | 2FBn | H | H | H | 1Me-5-Ind | C | | 429 (M⁺+1) |
| N-b-81 | 14n-2 | Int.n-b-12 | CHO12 | 2FBn | H | Et | H | 1Me-5-Idz | C | | 458 (M⁺+1) |
| N-b-82 | 1-a | N-b-80 | | 2FBn | H | H | H | 1Me-5-Idz | C | | 430 (M⁺+1) |
| N-b-83 | 14n-2 | Int.n-b-13 | CHO12 | 2FBn | H | Et | H | 1Et-5-Idz | C | | 472 (M⁺+1) |
| N-b-84 | 1-a | N-b-80 | | 2FBn | H | H | H | 1Et-5-Idz | C | | 444 (M⁺+1) |
| N-b-85 | 14n-2 | Int.n-b-14 | CHO12 | 2FBn | H | Et | H | 5-BF | C | | 444 (M⁺+1) |
| N-b-86 | 1-a | N-b-85 | | 2FBn | H | H | H | 5-BF | C | | 416 (M⁺+1) |
| N-b-87 | 14n-2 | N-b-75 | CHO1 | 2FBn | Me | Et | H | 2-Nap | C | | 468 (M⁺+1) |
| N-b-88 | 1-a | N-b-87 | | 2FBn | H | H | H | 2-Nap | C | | 426 (M⁺+1) |
| N-b-89 | 14n-2 | N-b-77 | CHO1 | 2FBn | Me | Et | H | 5-Ind | C | | 457 (M⁺+1) |
| N-b-90 | 1-a | N-b-89 | | 2FBn | H | H | H | 5-Ind | C | | 415 (M⁺+1) |

**[Table 136]**

| Table-N-B-3 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-b-91 | 14n-2 | N-b-79 | CHO1 | 2FBn | Me | Et | H | 1Me-5-Ind | C | | 471 (M⁺+1) |
| N-b-92 | 1-a | N-b-91 | | 2FBn | Me | H | H | 1Me-5-Ind | C | | 443 (M⁺+1) |
| N-b-93 | 14n-2 | N-b-81 | CHO1 | 2FBn | Me | Et | H | 1Me-5-Idz | C | | 472 (M⁺+1) |
| N-b-94 | 1-a | N-b-93 | | 2FBn | Me | H | H | 1Me-5-Idz | C | | 444 (M⁺+1) |
| N-b-95 | 14n-2 | N-b-83 | CHO1 | 2FBn | Me | Et | H | 1Et-5-Idz | C | | 486 (M⁺+1) |
| N-b-96 | 1-a | N-b-71 | | 2FBn | Me | H | H | 1Et-5-Idz | C | | 458 (M⁺+1) |
| N-b-97 | 14n-2 | N-b-75 | CHO2 | 2FBn | Et | Et | H | 2-Nap | C | | 482 (M⁺+1) |
| N-b-98 | 1-a | N-b-97 | | 2FBn | Et | H | H | 2-Nap | C | | 454 (M⁺+1) |
| N-b-99 | 14n-2 | N-b-77 | CHO2 | 2FBn | Et | Et | H | 5-Ind | C | | 471 (M⁺+1) |
| N-b-100 | 1-a | N-b-99 | | 2FBn | Et | H | H | 5-Ind | C | | 443 (M⁺+1) |
| N-b-101 | 14n-2 | N-b-79 | CHO2 | 2FBn | Et | Et | H | 1Me-5-Ind | C | | 485 (M⁺+1) |
| N-b-102 | 1-a | N-b-101 | | 2FBn | Et | H | H | 1 Me-5-Ind | C | | 457 (M⁺+1) |
| N-b-103 | 14n-2 | N-b-81 | CHO2 | 2FBn | Et | Et | H | 1Me-5-Idz | C | | 486 (M⁺+1) |
| N-b-104 | 1-a | N-b-103 | | 2FBn | Et | H | H | 1Me-5-Idz | C | | 458 (M⁺+1) |
| N-b-105 | 14n-2 | N-b-83 | CHO2 | 2FBn | Et | Et | H | 1Et-5-Idz | C | | 500 (M⁺+1) |
| N-b-106 | 1-a | N-b-105 | | 2FBn | Et | H | H | 1Et-5-Idz | C | | 472 (M⁺+1) |
| N-b-107 | 14n-2 | Int.n-b-8 | CHO13 | 3FBn | H | Et | H | 2-Nap | C | | 454 (M⁺+1) |
| N-b-108 | 1-a | N-b-107 | | 3FBn | H | H | H | 2-Nap | C | | 426 (M⁺+1) |
| N-b-109 | 14n-2 | Int.n-b-10 | CHO13 | 3FBn | H | Et | H | 1Me-5-Ind | C | | 457 (M⁺+1) |
| N-b-110 | 1-a | N-b-109 | | 3FBn | H | H | H | 1Me-5-Ind | C | | 429 (M⁺+1) |
| N-b-111 | 14n-2 | Int.n-b-11 | CHO13 | 3FBn | H | Et | H | 5-1Idz | C | | 444 (M⁺+1) |
| N-b-112 | 1-a | N-b-111 | | 3FBn | H | H | H | 5-1Idz | C | | 416 (M⁺+1) |
| N-b-113 | 14n-2 | Int.n-b-12 | CHO13 | 3FBn | H | Et | H | 1Me-5-Idz | C | | 458 (M⁺+1) |
| N-b-114 | 1-a | N-b-113 | | 3FBn | H | H | H | 1Me-5-Idz | C | | 430 (M⁺+1) |
| N-b-115 | 14n-2 | N-b-107 | CHO1 | 3FBn | Me | Et | H | 2-Nap | C | | 468 (M⁺+1) |
| N-b-116 | 1-a | N-b-115 | | 3FBn | Me | H | H | 2-Nap | C | | 440 (M⁺+1) |
| N-b-117 | 14n-2 | N-b-109 | CHO1 | 3FBn | Me | Et | H | 1Me-5-Ind | C | | 471 (M⁺+1) |
| N-b-118 | 1-a | N-b-116 | | 3FBn | Me | H | H | 1Me-5-Ind | C | | 443 (M⁺+1) |
| N-b-119 | 14n-2 | N-b-111 | CHO1 | 3FBn | Me | Et | H | 5-1Idz | C | | 458 (M⁺+1) |
| N-b-120 | 1-a | N-b-120 | | 3FBn | Me | H | H | 5-1Idz | C | | 430 (M⁺+1) |
| N-b-121 | 14n-2 | N-b-113 | CHO1 | 3FBn | Me | Et | H | 1Me-5-Idz | C | | 472 (M⁺+1) |
| N-b-122 | 1-a | N-b-122 | | 3FBn | Me | H | H | 1Me-5-Idz | C | | 444 (M⁺+1) |
| N-b-123 | 14n-2 | N-b-107 | CHO2 | 3FBn | Et | Et | H | 2-Nap | C | | 482 (M⁺+1) |
| N-b-124 | 1-a | N-b-123 | | 3FBn | Et | H | H | 2-Nap | C | | 454 (M⁺+1) |
| N-b-125 | 14n-2 | N-b-109 | CHO2 | 3FBn | Et | Et | H | 1Me-5-Ind | C | | 485 (M⁺+1) |
| N-b-126 | 1-a | N-b-125 | | 3FBn | Et | H | H | 1Me-5-Ind | C | | 457 (M⁺+1) |
| N-b-127 | 14n-2 | N-b-111 | CHO2 | 3FBn | Et | Et | H | 5-1 Idz | C | | 472 (M⁺+1) |
| N-b-128 | 1-a | N-b-127 | | 3FBn | Et | H | H | 5-1Idz | C | | 444 (M⁺+1) |
| N-b-129 | 14n-2 | N-b-113 | CHO2 | 3FBn | Et | Et | H | 1Me-5-Idz | C | | 486 (M⁺+1) |
| N-b-130 | 1-a | N-b-129 | | 3FBn | Et | H | H | 1 Me-5-Idz | C | | 458 (M⁺+1) |
| N-b-131 | 14n-2 | Int.n-b-8 | CHO14 | 2ClBn | H | Et | H | 2-Nap | C | | 471 (M⁺+1) |
| N-b-132 | 1-a | N-b-131 | | 2ClBn | H | H | H | 2-Nap | C | | 442 (M⁺+1) |
| N-b-133 | 14n-2 | Int.n-b-9 | CHO14 | 2ClBn | H | Et | H | 5-Ind | C | | 460 (M⁺+1) |
| N-b-134 | 1-a | N-b-133 | | 2ClBn | H | H | H | 5-Ind | C | | 431 (M⁺+1) |
| N-b-135 | 14n-2 | Int.n-b-10 | CHO14 | 2ClBn | H | Et | H | 1Me-5-Ind | C | | 474 (M⁺+1) |
| N-b-136 | 1-a | N-b-135 | | 2ClBn | H | H | H | 1Me-5-Ind | C | | 445 (M⁺+1) |

**[Table 137]**

| Table-N-B-4 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-b-137 | 14n-2 | Int.n-b-11 | CHO14 | 2ClBn | H | Et | H | 5-1Idz | C | | 460 (M⁺+1) |
| N-b-138 | 1-a | N-b-137 | | 2ClBn | H | H | H | 5-1Idz | C | | 432 (M⁺+1) |
| N-b-139 | 14n-2 | Int.n-b-12 | CHO14 | 2ClBn | H | Et | H | 1 Me-5-Idz | C | | 475 (M⁺+1) |
| N-b-140 | 1-a | N-b-139 | | 2ClBn | Me | H | H | 1 Me-5-Idz | C | | 460 (M⁺+1) |
| N-b-141 | 14n-2 | N-b-131 | CHO1 | 2ClBn | Me | Et | H | 2-Nap | C | | 485 (M⁺+1) |
| N-b-142 | 1-a | N-b-141 | | 2ClBn | Me | H | H | 2-Nap | C | | 457 (M⁺+1) |
| N-b-143 | 14n-2 | N-b-135 | CHO1 | 2ClBn | Me | Et | H | 1Me-5-Ind | C | | 488 (M⁺+1) |
| N-b-144 | 1-a | N-b-143 | | 2ClBn | Me | H | H | 1Me-5-Ind | C | | 460 (M⁺+1) |
| N-b-145 | 14n-2 | N-b-137 | CHO1 | 2ClBn | Me | Et | H | 5-1Idz | C | | 475 (M⁺+1) |
| N-b-146 | 1-a | N-b-145 | | 2ClBn | Me | H | H | 5-1Idz | C | | 446 (M⁺+1) |
| N-b-147 | 14n-2 | N-b-131 | CHO2 | 2ClBn | Et | Et | H | 2-Nap | C | | 499 (M⁺+1) |
| N-b-148 | 1-a | N-b-147 | | 2ClBn | Et | H | H | 2-Nap | C | | 471 (M⁺+1) |
| N-b-149 | 14n-2 | N-b-135 | CHO2 | 2ClBn | Et | Et | H | 1Me-5-Ind | C | | 502 (M⁺+1) |
| N-b-150 | 1-a | N-b-149 | | 2ClBn | Et | H | H | 1Me-5-Ind | C | | 474 (M⁺+1) |
| N-b-151 | 14n-2 | N-b-139 | CHO2 | 2ClBn | Et | Et | H | 1Me-5-Idz | C | | 503 (M⁺+1) |
| N-b-152 | 1-a | N-b-151 | | 2ClBn | Et | H | H | 1 Me-5-Idz | C | | 475 (M⁺+1) |
| N-b-153 | 14n-2 | Int.n-b-8 | CHO15 | 4ClBn | H | Et | H | 2-Nap | C | | 471 (M⁺+1) |
| N-b-154 | 1-a | N-b-153 | | 4ClBn | H | H | H | 2-Nap | C | | 442 (M⁺+1) |
| N-b-155 | 14n-2 | Int.n-b-9 | CHO15 | 4ClBn | H | Et | H | 5-Ind | C | | 460 (M⁺+1) |
| N-b-156 | 1-a | N-b-155 | | 4ClBn | H | H | H | 5-Ind | C | | 431 (M⁺+1) |
| N-b-157 | 14n-2 | Int.n-b-10 | CHO15 | 4ClBn | H | Et | H | 1Me-5-Ind | C | | 474 (M⁺+1) |
| N-b-158 | 1-a | N-b-157 | | 4ClBn | H | H | H | 1Me-5-Ind | C | | 445 (M⁺+1) |
| N-b-159 | 14n-2 | Int.n-b-11 | CHO15 | 4ClBn | H | Et | H | 5-1Idz | C | | 460 (M⁺+1) |
| N-b-160 | 1-a | N-b-159 | | 4ClBn | H | H | H | 5-1Idz | C | | 432 (M⁺+1) |
| N-b-161 | 14n-2 | Int.n-b-12 | CHO15 | 4ClBn | H | Et | H | 1Me-5-Idz | C | | 475 (M⁺+1) |
| N-b-162 | 1-a | N-b-161 | | 4ClBn | H | H | H | 1Me-5-Idz | C | | 446 (M⁺+1) |
| N-b-163 | 14n-2 | N-a-1 | CHO15 | 4ClBn | Me | Et | H | 2-Nap | C | | 485 (M⁺+1) |
| N-b-164 | 1-a | N-b-163 | | 4ClBn | Me | H | H | 2-Nap | C | | 457 (M⁺+1) |
| N-b-165 | 14n-2 | N-a-3 | CHO15 | 4ClBn | Me | Et | H | 5-Ind | C | | 474 (M⁺+1) |
| N-b-166 | 1-a | N-b-165 | | 4ClBn | Me | H | H | 5-Ind | C | | 445 (M⁺+1) |
| N-b-167 | 14n-2 | N-a-5 | CHO15 | 4ClBn | Me | Et | H | 1Me-5-Ind | C | | 488 (M⁺+1) |
| N-b-168 | 1-a | N-b-167 | | 4ClBn | Me | H | H | 1Me-5-Ind | C | | 460 (M⁺+1) |
| N-b-169 | 14n-2 | N-a-13 | CHO15 | 4ClBn | Me | Et | H | 5-1Idz | C | | 475 (M⁺+1) |
| N-b-170 | 1-a | N-b-169 | | 4ClBn | Me | H | H | 5-1Idz | C | | 446 (M⁺+1) |
| N-b-171 | 14n-2 | N-a-15 | CHO15 | 4ClBn | Me | Et | H | 1Me-5-Idz | C | | 489 (M⁺+1) |
| N-b-172 | 1-a | N-b-171 | | 4ClBn | Me | H | H | 1Me-5-Idz | C | | 460 (M⁺+1) |
| N-b-173 | 14n-2 | N-a-175 | CHO15 | 4ClBn | Et | Et | H | 2-Nap | C | | 499 (M⁺+1) |
| N-b-174 | 1-a | N-b-173 | | 4ClBn | Et | H | H | 2-Nap | C | | 471 (M⁺+1) |
| N-b-175 | 14n-2 | N-a-177 | CHO15 | 4ClBn | Et | Et | H | 5-Ind | C | | 488 (M⁺+1) |
| N-b-176 | 1-a | N-b-175 | | 4ClBn | Et | H | H | 5-Ind | C | | 460 (M⁺+1) |
| N-b-177 | 14n-2 | N-a-179 | CHO15 | 4ClBn | Et | Et | H | 1Me-5-Ind | C | | 502 (M⁺+1) |
| N-b-178 | 1-a | N-b-177 | | 4ClBn | Et | H | H | 1Me-5-Ind | C | | 474 (M⁺+1) |
| N-b-179 | 14n-2 | N-a-183 | CHO15 | 4ClBn | Et | Et | H | 5-1Idz | C | | 489 (M⁺+1) |
| N-b-180 | 1-a | N-b-179 | | 4ClBn | Et | H | H | 5-1Idz | C | | 460 (M⁺+1) |
| N-b-181 | 14n-2 | N-a-185 | CHO15 | 4ClBn | Et | Et | H | 1Me-5-Idz | C | | 503 (M⁺+1) |
| N-b-182 | 1-a | N-b-181 | | 4ClBn | Et | H | H | 1Me-5-Idz | C | | 475 (M⁺+1) |

**[Table 138]**

| Table-N-B-5 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-b-183 | 14n-2 | N-a-1 | CHO16 | 2BrBn | Me | Et | H | 2-Nap | C | | 528 (M⁺) |
| N-b-184 | 1-a | N-b-183 | | 2BrBn | Me | H | H | 2-Nap | C | | 500 (M⁺) |
| N-b-185 | 14n-2 | N-a-3 | CHO16 | 2BrBn | Me | Et | H | 5-Ind | C | | 517 (M⁺) |
| N-b-186 | 1-a | N-b-185 | | 2BrBn | Me | H | H | 5-Ind | C | | 489 (M⁺) |
| N-b-187 | 14n-2 | N-a-5 | CHO16 | 2BrBn | Me | Et | H | 1Me-5-Ind | C | | 531 (M⁺) |
| N-b-188 | 1-a | N-b-187 | | 2BrBn | Me | H | H | 1Me-5-Ind | C | | 503 (M⁺) |
| N-b-189 | 14n-2 | N-a-7 | CHO16 | 2BrBn | Me | Et | H | 1Et-5-Ind | C | | 545 (M⁺) |
| N-b-190 | 1-a | N-b-189 | | 2BrBn | Me | H | H | 1Et-5-Ind | C | | 517 (M⁺) |
| N-b-191 | 14n-2 | N-a-13 | CHO16 | 2BrBn | Me | Et | H | 5-1Idz | C | | 518 (M⁺) |
| N-b-192 | 1-a | N-b-191 | | 2BrBn | Me | H | H | 5-1Idz | C | | 491 (M⁺) |
| N-b-193 | 14n-2 | N-a-15 | CHO16 | 2BrBn | Me | Et | H | 1 Me-5-Idz | C | | 532 (M⁺) |
| N-b-194 | 1-a | N-b-193 | | 2BrBn | Me | H | H | 1Me-5-Idz | C | | 504 (M⁺) |
| N-b-195 | 14n-2 | N-a-17 | CHO16 | 2BrBn | Me | Et | H | 1Et-5-Idz | C | | 546 (M⁺) |
| N-b-196 | 1-a | N-b-195 | | 2BrBn | Me | H | H | 1Et-5-Idz | C | | 518 (M⁺) |
| N-b-197 | 14n-2 | N-a-1 | CHO17 | 2CF3Bn | Me | Et | H | 2-Nap | C | | 518 (M⁺+1) |
| N-b-198 | 1-a | N-b-197 | | 2CF3Bn | Me | H | H | 2-Nap | C | | 490 (M⁺+1) |
| N-b-199 | 14n-2 | N-a-3 | CHO17 | 2CF3Bn | Me | Et | H | 5-Ind | C | | 507 (M⁺+1) |
| N-b-200 | 1-a | N-b-199 | | 2CF3Bn | Me | H | H | 5-Ind | C | | 479 (M⁺+1) |
| N-b-201 | 14n-2 | N-a-5 | CHO17 | 2CF3Bn | Me | Et | H | 1Me-5-Ind | C | | 521 (M⁺+1) |
| N-b-202 | 1-a | N-b-201 | | 2CF3Bn | Me | H | H | 1Me-5-Ind | C | | 493 (M⁺+1) |
| N-b-203 | 14n-2 | N-a-13 | CHO17 | 2CF3Bn | Me | Et | H | 5-1Idz | C | | 508 (M⁺+1) |
| N-b-204 | 1-a | N-b-203 | | 2CF3Bn | Me | H | H | 5-1Idz | C | | 480 (M⁺+1) |
| N-b-205 | 14n-2 | N-a-15 | CHO17 | 2CF3Bn | Me | Et | H | 1Me-5-Idz | C | | 522 (M⁺+1) |
| N-b-206 | 1-a | N-b-205 | | 2CF3Bn | Me | H | H | 1Me-5-Idz | C | | 494 (M⁺+1) |
| N-b-207 | 14n-2 | Int.n-b-8 | CHO18 | 2,3DFBn | H | Et | H | 2-Nap | C | | 472 (M⁺+1) |
| N-b-208 | 1-a | N-b-207 | | 2,3DFBn | H | H | H | 2-Nap | C | | 444 (M⁺+1) |
| N-b-209 | 14n-2 | Int.n-b-9 | CHO18 | 2,3DFBn | H | Et | H | 5-Ind | C | | 461 (M⁺+1) |
| N-b-210 | 1-a | N-b-209 | | 2,3DFBn | H | H | H | 5-Ind | C | | 433 (M⁺+1) |
| N-b-211 | 14n-2 | Int.n-b-10 | CHO18 | 2,3DFBn | H | Et | H | 1Me-5-Ind | C | | 475 (M⁺+1) |
| N-b-212 | 1-a | N-b-211 | | 2,3DFBn | H | H | H | 1Me-5-Ind | C | | 447 (M⁺+1) |
| N-b-213 | 14n-2 | Int.n-b-12 | CHO18 | 2,3DFBn | H | Et | H | 1Me-5-Idz | C | | 476 (M⁺+1) |
| N-b-214 | 1-a | N-b-213 | | 2,3DFBn | H | H | H | 1Me-5-Idz | C | | 448 (M⁺+1) |
| N-b-215 | 14n-2 | N-a-1 | CHO18 | 2,3DFBn | Me | Et | H | 2-Nap | C | | 486 (M⁺+1) |
| N-b-216 | 1-a | N-b-215 | | 2,3DFBn | Me | H | H | 2-Nap | C | | 458 (M⁺+1) |
| N-b-217 | 14n-2 | N-a-3 | CHO18 | 2,3DFBn | Me | Et | H | 5-Ind | C | | 475 (M⁺+1) |
| N-b-218 | 1-a | N-b-217 | | 2,3DFBn | Me | H | H | 5-Ind | C | | 447 (M⁺+1) |
| N-b-219 | 14n-2 | N-a-5 | CHO18 | 2,3DFBn | Me | Et | H | 1Me-5-Ind | C | | 489 (M⁺+1) |
| N-b-220 | 1-a | N-b-219 | | 2,3DFBn | Me | H | H | 1Me-5-Ind | C | | 461 (M⁺+1) |
| N-b-221 | 14n-2 | N-a-15 | CHO18 | 2,3DFBn | Me | Et | H | 1 Me-5-Idz | C | | 490 (M⁺+1) |
| N-b-222 | 1-a | N-b-221 | | 2,3DFBn | Me | H | H | 1 Me-5-Idz | C | | 462 (M⁺+1) |
| N-b-223 | 14n-2 | Int.n-b-8 | CHO19 | 4MeBn | H | Et | H | 2-Nap | C | | 450 (M⁺+1) |
| N-b-224 | 1-a | N-b-223 | | 4MeBn | H | H | H | 2-Nap | C | | 422 (M⁺+1) |
| N-b-225 | 14n-2 | Int.n-b-9 | CHO19 | 4MeBn | H | Et | H | 5-Ind | C | | 439 (M⁺+1) |
| N-b-226 | 1-a | N-b-225 | | 4MeBn | H | H | H | 5-Ind | C | | 411 (M⁺+1) |
| N-b-227 | 14n-2 | Int.n-b-10 | CHO19 | 4MeBn | H | Et | H | 1Me-5-Ind | C | | 453 (M⁺+1) |
| N-b-228 | 1-a | N-b-227 | | 4MeBn | H | H | H | 1Me-5-Ind | C | | 425 (M⁺+1) |

**[Table 139]**

| Table-N-B-6 | | | | | | | | | | | |
|---|---|---|---|---|---|---|---|---|---|---|---|
| Exp. | Syn. | SM1 | Reagent | Rz | Ry | Y | V₁' | V₂' | LCMS | | |
| | | | | | | | | | method | RTime | Mass |
| N-b-229 | 14n-2 | Int.n-b-12 | CHO19 | 4MeBn | H | Et | H | 1Me-5-Idz | C | | 454 (M⁺+1) |
| N-b-230 | 1-a | N-b-229 | | 4MeBn | H | H | H | 1Me-5-Idz | C | | 426 (M⁺+1) |
| N-b-231 | 14n-2 | Int.n-b-13 | CHO19 | 4MeBn | H | Et | H | 1Et-5-Idz | C | | 468 (M⁺+1) |
| N-b-232 | 1-a | N-b-231 | | 4MeBn | H | H | H | 1Et-5-Idz | C | | 440 (M⁺+1) |
| N-b-233 | 14n-2 | N-b-223 | CHO1 | 4MeBn | Me | Et | H | 2-Nap | C | | 464 (M⁺+1) |
| N-b-234 | 1-a | N-b-233 | | 4MeBn | Me | H | H | 2-Nap | C | | 436 (M⁺+1) |
| N-b-235 | 14n-2 | N-b-225 | CHO1 | 4MeBn | Me | Et | H | 5-Ind | C | | 453 (M⁺+1) |
| N-b-236 | 1-a | N-b-235 | | 4MeBn | Me | H | H | 5-Ind | C | | 425 (M⁺+1) |
| N-b-237 | 14n-2 | N-b-227 | CHO1 | 4MeBn | Me | Et | H | 1Me-5-Ind | C | | 467 (M⁺+1) |
| N-b-238 | 1-a | N-b-237 | | 4MeBn | Me | H | H | 1Me-5-Ind | C | | 439 (M⁺+1) |
| N-b-239 | 14n-2 | N-b-229 | CHO1 | 4MeBn | Me | Et | H | 1 Me-5-Idz | C | | 468 (M⁺+1) |
| N-b-240 | 1-a | N-b-239 | | 4MeBn | Me | H | H | 1Me-5-Idz | C | | 440 (M⁺+1) |
| N-b-241 | 14n-2 | N-b-231 | CHO1 | 4MeBn | Me | Et | H | 1Et-5-Idz | C | | 482 (M⁺+1) |
| N-b-242 | 1-a | N-b-241 | | 4MeBn | Me | H | H | 1Et-5-Idz | C | | 454 (M⁺+1) |
| N-b-243 | 14n-2 | N-a-1 | CHO20 | 2PhBn | Me | Et | H | 2-Nap | C | | 526 (M⁺+1) |
| N-b-244 | 1-a | N-b-243 | | 2PhBn | Me | H | H | 2-Nap | C | | 498 (M⁺+1) |
| N-b-245 | 14n-2 | N-a-3 | CHO20 | 2PhBn | Me | Et | H | 5-Ind | C | | 515 (M⁺+1) |
| N-b-246 | 1-a | N-b-245 | | 2PhBn | Me | H | H | 5-Ind | C | | 487 (M⁺+1) |
| N-b-247 | 14n-2 | N-a-5 | CHO20 | 2PhBn | Me | Et | H | 1 Me-5-Ind | C | | 529 (M⁺+1) |
| N-b-248 | 1-a | N-b-247 | | 2PhBn | Me | H | H | 1Me-5-Ind | C | | 501 (M⁺+1) |
| N-b-249 | 14n-2 | N-a-13 | CHO20 | 2PhBn | Me | Et | H | 5-1Idz | C | | 516 (M⁺+1) |
| N-b-250 | 1-a | N-b-249 | | 2PhBn | Me | H | H | 5-1Idz | C | | 488 (M⁺+1) |
| N-b-251 | 14n-2 | N-a-15 | CHO20 | 2PhBn | Me | Et | H | 1Me-5-Idz | C | | 530 (M⁺+1) |
| N-b-252 | 1-a | N-b-251 | | 2PhBn | Me | H | H | 1Me-5-Idz | C | | 502 (M⁺+1) |
| N-b-253 | 14n-2 | N-a-17 | CHO20 | 2PhBn | Me | Et | H | 1Et-5-Idz | C | | 544 (M⁺+1) |
| N-b-254 | 1-a | N-b-253 | | 2PhBn | Me | H | H | 1Et-5-Idz | C | | 516 (M⁺+1) |
| N-b-255 | 14n-2 | N-a-175 | CHO20 | 2PhBn | Et | Et | H | 2-Nap | C | | 540 (M⁺+1) |
| N-b-256 | 1-a | N-b-255 | | 2PhBn | Et | H | H | 2-Nap | C | | 512 (M⁺+1) |
| N-b-257 | 14n-2 | N-a-179 | CHO20 | 2PhBn | Et | Et | H | 1Me-5-Ind | C | | 543 (M⁺+1) |
| N-b-258 | 1-a | N-b-257 | | 2PhBn | Et | H | H | 1Me-5-Ind | C | | 515 (M⁺+1) |
| N-b-259 | 14n-2 | N-a-185 | CHO20 | 2PhBn | Et | Et | H | 1Me-5-Idz | C | | 544 (M⁺+1) |
| N-b-260 | 1-a | N-b-259 | | 2PhBn | Et | H | H | 1Me-5-Idz | C | | 516 (M⁺+1) |
| N-b-261 | 14n-2 | N-a-1 | CHO21 | 40MeBn | Me | Et | H | 2-Nap | C | | 480 (M⁺+1) |
| N-b-262 | 1-a | N-b-261 | | 40MeBn | Me | H | H | 2-Nap | C | | 452 (M⁺+1) |
| N-b-263 | 14n-2 | N-a-3 | CHO21 | 40MeBn | Me | Et | H | 5-Ind | C | | 469 (M⁺+1) |
| N-b-264 | 1-a | N-b-263 | | 40MeBn | Me | H | H | 5-Ind | C | | 441 (M⁺+1) |
| N-b-265 | 14n-2 | N-a-5 | CHO21 | 40MeBn | Me | Et | H | 1Me-5-Ind | C | | 483 (M⁺+1) |
| N-b-266 | 1-a | N-b-265 | | 40MeBn | Me | H | H | 1 Me-5-Ind | C | | 455 (M⁺+1) |
| N-b-267 | 14n-2 | N-a-7 | CHO21 | 40MeBn | Me | Et | H | 1Et-5-Ind | C | | 497 (M⁺+1) |
| N-b-268 | 1-a | N-b-267 | | 40MeBn | Me | H | H | 1Et-5-Ind | C | | 469 (M⁺+1) |
| N-b-269 | 14n-2 | N-a-13 | CHO21 | 40MeBn | Me | Et | H | 5-1Idz | C | | 470 (M⁺+1) |
| N-b-270 | 1-a | N-b-269 | | 40MeBn | Me | H | H | 5-1Idz | C | | 442 (M⁺+1) |
| N-b-271 | 14n-2 | N-a-175 | CHO21 | 40MeBn | Et | Et | H | 2-Nap | C | | 494 (M⁺+1) |
| N-b-272 | 1-a | N-b-271 | | 40MeBn | Et | H | H | 2-Nap | C | | 466 (M⁺+1) |
| N-b-273 | 14n-2 | N-a-185 | CHO21 | 40MeBn | Et | Et | H | 1Me-5-Idz | C | | 498 (M⁺+1) |
| N-b-274 | 1-a | N-b-273 | | 40MeBn | Et | H | H | 1Me-5-Idz | C | | 470 (M⁺+1) |

**[Table 140]**

| Table-N-B-7 | | | | | | | | | | | |
|---|---|---|---|---|---|---|---|---|---|---|---|
| Exp. | Syn. | SM1 | Reagent | Rz | Ry | Y | V₁' | V₂' | LCMS | | |
| | | | | | | | | | method | RTime | Mass |
| N-b-275 | 14n-2 | Int.n-b-8 | CHO22 | 20MeBn | H | Et | H | 2-Nap | C | | 466 (M⁺+1) |
| N-b-276 | 1-a | N-b-275 | | 20MeBn | H | H | H | 2-Nap | C | | 438 (M⁺+1) |
| N-b-277 | 14n-2 | Int.n-b-9 | CHO22 | 20MeBn | H | Et | H | 5-Ind | C | | 455 (M⁺+1) |
| N-b-278 | 1-a | N-b-277 | | 20MeBn | H | H | H | 5-Ind | C | | 427 (M⁺+1) |
| N-b-279 | 14n-2 | Int.n-b-10 | CHO22 | 20MeBn | H | Et | H | 1Me-5-Ind | C | | 469 (M⁺+1) |
| N-b-280 | 1-a | N-b-279 | | 20MeBn | H | H | H | 1Me-5-Ind | C | | 441 (M⁺+1) |
| N-b-281 | 14n-2 | Int.n-b-11 | CHO22 | 20MeBn | H | Et | H | 5-1Idz | C | | 456 (M⁺+1) |
| N-b-282 | 1-a | N-b-281 | | 20MeBn | H | H | H | 5-1Idz | C | | 428 (M⁺+1) |
| N-b-283 | 14n-2 | Int.n-b-12 | CHO22 | 20MeBn | H | Et | H | 1Me-5-Idz | C | | 470 (M⁺+1) |
| N-b-284 | 1-a | N-b-283 | | 20MeBn | H | H | H | 1Me-5-Idz | C | | 442 (M⁺+1) |
| N-b-285 | 14n-2 | N-a-1 | CHO22 | 20MeBn | Me | Et | H | 2-Nap | C | | 480 (M⁺+1) |
| N-b-286 | 1-a | N-b-285 | | 20MeBn | Me | H | H | 2-Nap | C | | 452 (M⁺+1) |
| N-b-287 | 14n-2 | N-a-15 | CHO22 | 20MeBn | Me | Et | H | 1Me-5-Idz | C | | 484 (M⁺+1) |
| N-b-288 | 1-a | N-b-287 | | 20MeBn | Me | H | H | 1Me-5-Idz | C | | 456 (M⁺+1) |
| N-b-289 | 14n-2 | Int.n-b-8 | CHO23 | 4DMABn | H | Et | H | 2-Nap | C | | 479 (M⁺+1) |
| N-b-290 | 1-a | N-b-289 | | 4DMABn | H | H | H | 2-Nap | C | | 451 (M⁺+1) |
| N-b-291 | 14n-2 | Int.n-b-9 | CHO23 | 4DMABn | H | Et | H | 5-Ind | C | | 468 (M⁺+1) |
| N-b-292 | 1-a | N-b-291 | | 4DMABn | H | H | H | 5-Ind | C | | 440 (M⁺+1) |
| N-b-293 | 14n-2 | Int.n-b-10 | CHO23 | 4DMABn | H | Et | H | 1Me-5-Ind | C | | 482 (M⁺+1) |
| N-b-294 | 1-a | N-b-293 | | 4DMABn | H | H | H | 1Me-5-Ind | C | | 454 (M⁺+1) |
| N-b-295 | 14n-2 | Int.n-b-11 | CHO23 | 4DMABn | H | Et | H | 5-1Idz | C | | 469 (M⁺+1) |
| N-b-296 | 1-a | N-b-295 | | 4DMABn | H | H | H | 5-1Idz | C | | 441 (M⁺+1) |
| N-b-297 | 14n-2 | Int.n-b-12 | CHO23 | 4DMABn | H | Et | H | 1Me-5-Idz | C | | 483 (M⁺+1) |
| N-b-298 | 1-a | N-b-297 | | 4DMABn | H | H | H | 1Me-5-Idz | C | | 455 (M⁺+1) |
| N-b-299 | 14n-2 | N-a-1 | CHO23 | 4DMABn | Me | Et | H | 2-Nap | C | | 493 (M⁺+1) |
| N-b-300 | 1-a | N-b-299 | | 4DMABn | Me | H | H | 2-Nap | C | | 465 (M⁺+1) |
| N-b-301 | 14n-2 | N-a-5 | CHO23 | 4DMABn | Me | Et | H | 1Me-5-Ind | C | | 496 (M⁺+1) |
| N-b-302 | 1-a | N-b-301 | | 4DMABn | Me | H | H | 1Me-5-Ind | C | | 468 (M⁺+1) |
| N-b-303 | 14n-2 | N-a-13 | CHO23 | 4DMABn | Me | Et | H | 5-1Idz | C | | 483 (M⁺+1) |
| N-b-304 | 1-a | N-b-303 | | 4DMABn | Me | H | H | 5-1Idz | C | | 455 (M⁺+1) |
| N-b-305 | 14n-2 | N-a-15 | CHO23 | 4DMABn | Me | Et | H | 1Me-5-Idz | C | | 497 (M⁺+1) |
| N-b-306 | 1-a | N-b-305 | | 4DMABn | Me | H | H | 1Me-5-Idz | C | | 469 (M⁺+1) |
| N-b-307 | 14n-2 | N-a-1 | CHO24 | 4SMeBn | Me | Et | H | 2-Nap | C | | 496 (M⁺+1) |
| N-b-308 | 1-a | N-b-307 | | 4SMeBn | Me | H | H | 2-Nap | C | | 468 (M⁺+1) |
| N-b-309 | 14n-2 | N-a-3 | CHO24 | 4SMeBn | Me | Et | H | 5-Ind | C | | 485 (M⁺+1) |
| N-b-310 | 1-a | N-b-309 | | 4SMeBn | Me | H | H | 5-Ind | C | | 457 (M⁺+1) |
| N-b-311 | 14n-2 | N-a-5 | CHO24 | 4SMeBn | Me | Et | H | 1Me-5-Ind | C | | 499 (M⁺+1) |
| N-b-312 | 1-a | N-b-311 | | 4SMeBn | Me | H | H | 1Me-5-Ind | C | | 471 (M⁺+1) |
| N-b-313 | 14n-2 | N-a-7 | CHO24 | 4SMeBn | Me | Et | H | 1Et-5-Ind | C | | 513 (M⁺+1) |
| N-b-314 | 1-a | N-b-313 | | 4SMeBn | Me | H | H | 1Et-5-Ind | C | | 485 (M⁺+1) |
| N-b-315 | 14n-2 | N-a-13 | CHO24 | 4SMeBn | Me | Et | H | 5-1Idz | C | | 486 (M⁺+1) |
| N-b-316 | 1-a | N-b-315 | | 4SMeBn | Me | H | H | 5-1Idz | C | | 458 (M⁺+1) |
| N-b-317 | 14n-2 | N-a-1 | CHO25 | 1NapMe | Me | Et | H | 2-Nap | C | | 500 (M⁺+1) |
| N-b-318 | 1-a | N-b-317 | | 1NapMe | Me | H | H | 2-Nap | C | | 472 (M⁺+1) |
| N-b-319 | 14n-2 | N-a-5 | CHO25 | 1NapMe | Me | Et | H | 1Me-5-Ind | C | | 503 (M⁺+1) |
| N-b-320 | 1-a | N-b-319 | | 1NapMe | Me | H | H | 1Me-5-Ind | C | | 475 (M⁺+1) |

**[Table 141]**

| Table-N-B-8 | | | | | | | | | | | |
|---|---|---|---|---|---|---|---|---|---|---|---|
| Exp. | Syn. | SM1 | Reagent | Rz | Ry | Y | V₁' | V₂' | LCMS | | |
| | | | | | | | | | method | RTime | Mass |
| N-b-321 | 14n-2 | N-a-15 | CHO25 | 1NapMe | Me | Et | H | 1 Me-5-Idz | C | | 504 (M⁺+1) |
| N-b-322 | 1-a | N-b-321 | | 1NapMe | Me | H | H | 1 Me-5-Idz | C | | 476 (M⁺+1) |
| N-b-323 | 14n-2 | N-a-1 | CHO26 | 2NapMe | Me | Et | H | 2-Nap | C | | 500 (M⁺+1) |
| N-b-324 | 1-a | N-b-323 | | 2NapMe | Me | H | H | 2-Nap | C | | 472 (M⁺+1) |
| N-b-325 | 14n-2 | N-a-3 | CHO26 | 2NapMe | Me | Et | H | 5-Ind | C | | 489 (M⁺+1) |
| N-b-326 | 1-a | N-b-325 | | 2NapMe | Me | H | H | 5-Ind | C | | 461 (M⁺+1) |
| N-b-327 | 14n-2 | N-a-5 | CHO26 | 2NapMe | Me | Et | H | 1Me-5-Ind | C | | 503 (M⁺+1) |
| N-b-328 | 1-a | N-b-327 | | 2NapMe | Me | H | H | 1Me-5-Ind | C | | 475 (M⁺+1) |
| N-b-329 | 14n-2 | N-a-7 | CHO26 | 2NapMe | Me | Et | H | 1Et-5-Ind | C | | 517 (M⁺+1) |
| N-b-330 | 1-a | N-b-329 | | 2NapMe | Me | H | H | 1Et-5-Ind | C | | 489 (M⁺+1) |
| N-b-331 | 14n-2 | N-a-13 | CHO26 | 2NapMe | Me | Et | H | 5-1Idz | C | | 490 (M⁺+1) |
| N-b-332 | 1-a | N-b-331 | | 2NapMe | Me | H | H | 5-1Idz | C | | 462 (M⁺+1) |
| N-b-333 | 14n-2 | N-a-15 | CHO26 | 2NapMe | Me | Et | H | 1Me-5-Idz | C | | 504 (M⁺+1) |
| N-b-334 | 1-a | N-b-333 | | 2NapMe | Me | H | H | 1Me-5-Idz | C | | 476 (M⁺+1) |
| N-b-335 | 14n-2 | N-a-1 | CHO27 | 2FRMe | Me | Et | H | 2-Nap | C | | 440 (M⁺+1) |
| N-b-336 | 1-a | N-b-335 | | 2FRMe | Me | H | H | 2-Nap | C | | 412 (M⁺+1) |
| N-b-337 | 14n-2 | N-a-5 | CHO27 | 2FRMe | Me | Et | H | 1Me-5-Ind | C | | 443 (M⁺+1) |
| N-b-338 | 1-a | N-b-337 | | 2FRMe | Me | H | H | 1Me-5-Ind | C | | 415 (M⁺+1) |
| N-b-339 | 14n-2 | N-a-7 | CHO27 | 2FRMe | Me | Et | H | 1Et-5-Ind | C | | 457 (M⁺+1) |
| N-b-340 | 1-a | N-b-339 | | 2FRMe | Me | H | H | 1Et-5-Ind | C | | 429 (M⁺+1) |
| N-b-341 | 14n-2 | N-a-13 | CHO27 | 2FRMe | Me | Et | H | 5-1Idz | C | | 430 (M⁺+1) |
| N-b-342 | 1-a | N-b-341 | | 2FRMe | Me | H | H | 5-1Idz | C | | 402 (M⁺+1) |
| N-b-343 | 14n-2 | N-a-15 | CHO27 | 2FRMe | Me | Et | H | 1Me-5-Idz | C | | 444 (M⁺+1) |
| N-b-344 | 1-a | N-b-343 | | 2FRMe | Me | H | H | 1Me-5-Idz | C | | 416 (M⁺+1) |
| N-b-345 | 14n-2 | N-a-175 | CHO27 | 2FRMe | Et | Et | H | 2-Nap | C | | 454 (M⁺+1) |
| N-b-346 | 1-a | N-b-345 | | 2FRMe | Et | H | H | 2-Nap | C | | 426 (M⁺+1) |
| N-b-347 | 14n-2 | N-a-179 | CHO27 | 2FRMe | Et | Et | H | 1Me-5-Ind | C | | 457 (M⁺+1) |
| N-b-348 | 1-a | N-b-347 | | 2FRMe | Et | H | H | 1Me-5-Ind | C | | 429 (M⁺+1) |
| N-b-349 | 14n-2 | N-a-181 | CHO27 | 2FRMe | Et | Et | H | 1Et-5-Ind | C | | 471 (M⁺+1) |
| N-b-350 | 1-a | N-b-349 | | 2FRMe | Et | H | H | 1Et-5-Ind | C | | 443 (M⁺+1) |
| N-b-351 | 14n-2 | N-a-183 | CHO27 | 2FRMe | Et | Et | H | 5-1Idz | C | | 444 (M⁺+1) |
| N-b-352 | 1-a | N-b-351 | | 2FRMe | Et | H | H | 5-1Idz | C | | 416 (M⁺+1) |
| N-b-353 | 14n-2 | N-a-185 | CHO27 | 2FRMe | Et | Et | H | 1Me-5-Idz | C | | 458 (M⁺+1) |
| N-b-354 | 1-a | N-b-353 | | 2FRMe | Et | H | H | 1Me-5-Idz | C | | 430 (M⁺+1) |
| N-b-355 | 14n-2 | N-a-1 | CHO28 | 3FRMe | Me | Et | H | 2-Nap | C | | 440 (M⁺+1) |
| N-b-356 | 1-a | N-b-355 | | 3FRMe | Me | H | H | 2-Nap | C | | 412 (M⁺+1) |
| N-b-357 | 14n-2 | N-a-3 | CHO28 | 3FRMe | Me | Et | H | 1Me-5-Ind | C | | 443 (M⁺+1) |
| N-b-358 | 1-a | N-b-357 | | 3FRMe | Me | H | H | 1Me-5-Ind | C | | 415 (M⁺+1) |
| N-b-359 | 14n-2 | N-a-13 | CHO28 | 3FRMe | Me | Et | H | 5-1Idz | C | | 430 (M⁺+1) |
| N-b-360 | 1-a | N-b-359 | | 3FRMe | Me | H | H | 5-1Idz | C | | 402 (M⁺+1) |
| N-b-361 | 14n-2 | N-a-15 | CHO28 | 3FRMe | Me | Et | H | 1Me-5-Idz | C | | 444 (M⁺+1) |
| N-b-362 | 1-a | N-b-361 | | 3FRMe | Me | H | H | 1Me-5-Idz | C | | 416 (M⁺+1) |
| N-b-363 | 14n-2 | N-a-175 | CHO28 | 3FRMe | Et | Et | H | 2-Nap | C | | 454 (M⁺+1) |
| N-b-364 | 1-a | N-b-363 | | 3FRMe | Et | H | H | 2-Nap | C | | 426 (M⁺+1) |
| N-b-365 | 14n-2 | N-a-179 | CHO28 | 3FRMe | Et | Et | H | 1Me-5-Ind | C | | 457 (M⁺+1) |
| N-b-366 | 1-a | N-b-365 | | 3FRMe | Et | H | H | 1Me-5-Ind | C | | 429 (M⁺+1) |

**[Table 142]**

| Table-N-B-9 | | | | | | | | | | | |
|---|---|---|---|---|---|---|---|---|---|---|---|
| Exp. | Syn. | SM1 | Reagent | Rz | Ry | Y | V₁' | V₂' | LCMS | | |
| | | | | | | | | | method | RTime | Mass |
| N-b-367 | 14n-2 | N-a-183 | CHO28 | 3FRMe | Et | Et | H | 5-1Idz | C | | 444 (M⁺+1) |
| N-b-368 | 1-a | N-b-367 | | 3FRMe | Et | H | H | 5-1Idz | C | | 416 (M⁺+1) |
| N-b-369 | 14n-2 | N-a-185 | CHO28 | 3FRMe | Et | Et | H | 1Me-5-Idz | C | | 458 (M⁺+1) |
| N-b-370 | 1-a | N-b-369 | | 3FRMe | Et | H | H | 1Me-5-Idz | C | | 430 (M⁺+1) |
| N-b-371 | 14n-2 | N-a-187 | CHO28 | 3FRMe | Et | Et | H | 1Et-5-Idz | C | | 472 (M⁺+1) |
| N-b-372 | 1-a | N-b-371 | | 3FRMe | Et | H | H | 1Et-5-Idz | C | | 444 (M⁺+1) |
| N-b-373 | 14n-2 | N-a-1 | CHO29 | 2TPMe | Me | Et | H | 2-Nap | C | | 456 (M⁺+1) |
| N-b-374 | 1-a | N-b-373 | | 2TPMe | Me | H | H | 2-Nap | C | | 428 (M⁺+1) |
| N-b-375 | 14n-2 | N-a-5 | CHO29 | 2TPMe | Me | Et | H | 1Me-5-Ind | C | | 459 (M⁺+1) |
| N-b-376 | 1-a | N-b-375 | | 2TPMe | Me | H | H | 1Me-5-Ind | C | | 431 (M⁺+1) |
| N-b-377 | 14n-2 | N-a-7 | CHO29 | 2TPMe | Me | Et | H | 1Et-5-Ind | C | | 473 (M⁺+1) |
| N-b-378 | 1-a | N-b-377 | | 2TPMe | Me | H | H | 1Et-5-Ind | C | | 445 (M⁺+1) |
| N-b-379 | 14n-2 | N-a-13 | CHO29 | 2TPMe | Me | Et | H | 5-1Idz | C | | 446 (M⁺+1) |
| N-b-380 | 1-a | N-b-379 | | 2TPMe | Me | H | H | 5-1Idz | C | | 418 (M⁺+1) |
| N-b-381 | 14n-2 | N-a-15 | CHO29 | 2TPMe | Me | Et | H | 1Me-5-Idz | C | | 460 (M⁺+1) |
| N-b-382 | 1-a | N-b-381 | | 2TPMe | Me | H | H | 1Me-5-Idz | C | | 432 (M⁺+1) |
| N-b-383 | 14n-2 | N-a-175 | CHO29 | 2TPMe | Et | Et | H | 2-Nap | C | | 470 (M⁺+1) |
| N-b-384 | 1-a | N-b-383 | | 2TPMe | Et | H | H | 2-Nap | C | | 442 (M⁺+1) |
| N-b-385 | 14n-2 | N-a-177 | CHO29 | 2TPMe | Et | Et | H | 5-Ind | C | | 459 (M⁺+1) |
| N-b-386 | 1-a | N-b-385 | | 2TPMe | Et | H | H | 5-Ind | C | | 431 (M⁺+1) |
| N-b-387 | 14n-2 | N-a-179 | CHO29 | 2TPMe | Et | Et | H | 1Me-5-Ind | C | | 473 (M⁺+1) |
| N-b-388 | 1-a | N-b-387 | | 2TPMe | Et | H | H | 1Me-5-Ind | C | | 445 (M⁺+1) |
| N-b-389 | 14n-2 | N-a-181 | CHO29 | 2TPMe | Et | Et | H | 1Et-5-Ind | C | | 487 (M⁺+1) |
| N-b-390 | 1-a | N-b-389 | | 2TPMe | Et | H | H | 1Et-5-Ind | C | | 459 (M⁺+1) |
| N-b-391 | 14n-2 | N-a-183 | CHO29 | 2TPMe | Et | Et | H | 5-1Idz | C | | 460 (M⁺+1) |
| N-b-392 | 1-a | N-b-391 | | 2TPMe | Et | H | H | 5-1Idz | C | | 432 (M⁺+1) |
| N-b-393 | 14n-2 | N-a-185 | CHO29 | 2TPMe | Et | Et | H | 1Me-5-Idz | C | | 474 (M⁺+1) |
| N-b-394 | 1-a | N-b-393 | | 2TPMe | Et | H | H | 1Me-5-Idz | C | | 446 (M⁺+1) |

### Reference Example 15

### Synthesis of ethyl 2-[5-(piperidin-l-yl)-2,3-dihydroinden-1-yl-ylidene]acetate (Intermediate n-c-1) (Preparation Method 9, Step k-1)

According to the procedure described in the synthetic method of Intermediate A-2 in Reference Example 1 (Preparation Method 9, Step k-1), Intermediate n-a-3 (1.33 g), ethyl diethylphosphonoacetate (7.5 ml, TCI) and sodium hydride (1.23 g, WAKO) were reacted and treated to obtain the title compound (Intermediate n-c-2, 1.208 g). Mass (LCMS): 286 (M⁺+1), Retention time: 5.93 minutes (Elution condition: B).

### Synthesis of ethyl 2-(5-(piperidin-1-yl)-2,3-dihydro-1H-inden-1-yl)acetate (Intermediate n-c-2) (Preparation Method 9, Step j)

According to the procedure described in the synthetic method of Intermediate A-3 in Reference Example 1 (Preparation Method 9, Step j), Intermediate n-c-1 (1.20 g) and 10% palladium/carbon (82.2 mg, Merck) were reacted and treated to obtain the title compound (Intermediate n-c-2, 1.286 mg). Mass (LCMS): 288 (M⁺+1), Retention time: 3.55 minutes (Elution condition: D).

### Synthesis of ethyl 2-[6-bromo-5-(piperidin-1-yl)-2,3-dihydro-1H-inden-1-yl]acetate (Intermediate n-c-3) (Preparation Method 8, Step h)

According to the procedure described in the synthetic method of Intermediate A-4 in Reference Example 1 (Preparation Method 8, Step h) provided that the reaction was performed at room temperature for 1 hour, Intermediate n-c-2 (272.3 mg) and NBS (180.0 mg) were reacted and treated to obtain the title compound (Intermediate n-c-3, 165.3 mg). Mass (LCMS): 365 (M⁺), Retention time: 3.97 minutes (Elution condition: B).

### Reference Example 16

### Synthesis of ethyl 2-(5-fluoro-2,3-dihydroinden-1-yl-ylidene)acetate (Intermediate n-c-46) (Preparation Method 9, Step k-1)

According to the procedure described in the synthetic method of Intermediate A-2 in Reference Example 1 (Preparation Method 9, Step k-1), 5-fluoro-1-indanone (5.32 g), ethyl diethylphosphonoacetate (23 ml, TCI) and sodium hydride (4.13 g, WAKO) were reacted and treated to obtain the title compound (Intermediate N-c-46, 5.24 g). Mass (LCMS): 221 (M⁺+1), Retention time: 5.13 minutes (Elution condition: B).

### Synthesis of ethyl 2-[5-(piperazin-1-yl)-2,3-dihydroinden-1-yl-ylidene]acetate (Intermediate n-c-47) (Preparation Method 14, Step n)

According to the procedure described in the synthetic method of Intermediate n-a-1 in Reference Example 12 (Preparation Method 14, Step n), Intermediate n-c-46 (2.29 g), potassium carbonate (1.21 g, WAKO) and piperazine (1.2 ml, TCI) were reacted and treated to obtain the title compound (Intermediate N-c-47, 1.28 g). Mass (LCMS): 272 (M⁺+1), Retention time: 5.98 minutes (Elution condition: B).

Typical examples of the intermediates that can be obtained by reacting and treating corresponding starting compounds using any of the methods described in the present specification including the examples described above are shown in Table-Int.N-c-1 and Table-Int.N-c-2. The compounds were synthesized according to the preparation methods of the compounds of the compound numbers shown in the columns of "Syn" in the tables. "Int" means an intermediate compound number. In the tables, used methods among the aforementioned synthetic methods are shown in the columns of "Syn" with symbols, the starting compounds are shown in the columns of "SM", and the reagents are shown in the columns of "Regent". The halide regents mentioned in the columns of "Reagent" with symbols "AMN (No.)" are those mentioned in Table-AMN. Further, the compounds indicated as "S" in the columns of "S/D" in Table-Int.N-b-1 are compound in which two of the carbon atoms binding the indane ring and carbonyl group in the compounds are bound with a single bond, and those indicated as "D" in the same are compounds in which two of the carbon atoms binding the benzene ring and carbonyl group in the compounds are bound with a double bond.

**[Table 143]**

| Table-Int.N-c-1 | | | | | | | | | | | |
|---|---|---|---|---|---|---|---|---|---|---|---|
| 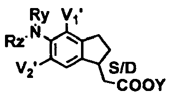 | | | | | | | | | | | |

| Exp. | Syn. | SM1 | SM2 | RyRz | Y | V₁' | V₂' | S/D | LCMS | | |
|---|---|---|---|---|---|---|---|---|---|---|---|
| | | | | | | | | | method | RTime | Mass |
| Int.n-c-4 | 10k- 1 | Int.n-a-4 | | 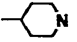 | Et | H | H | D | C | | 300 (M⁺+1) |
| Int.n-c-5 | 10-j | Int.n-c-4 | | 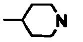 | Et | H | H | S | C | | 302 (M⁺+1) |
| Int.n-c-6 | 13-h | Int.n-c-5 | | 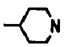 | Et | H | Br | S | C | | 380 (M⁺) |
| Int.n-c-7 | 10k- 1 | Int.n-a-5 | | 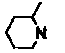 | Et | H | H | D | C | | 300 (M⁺+1) |
| Int.n-c-8 | 10-j | Int.n-c-7 | | 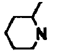 | Et | H | H | S | C | | 302 (M⁺+1) |
| Int.n-c-9 | 13-h | Int.n-c-8 | | 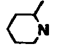 | Et | H | Br | S | C | | 380 (M⁺) |
| Int.n-c-10 | 10k- 1 | Int.n-a-6 | | 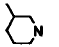 | Et | H | H | D | C | | 300 (M⁺+1) |
| Int.n-c-11 | 10-j | Int.n-c-10 | | 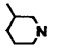 | Et | H | H | S | C | | 302 (M⁺+1) |
| Int.n-c-12 | 13-h | Int.n-c-11 | | 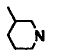 | Et | H | Br | S | C | | 380 (M⁺) |
| Int.n-c-13 | 10k- 1 | Int.n-a-7 | | 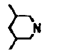 | Et | H | H | D | C | | 314 (M⁺+1) |
| Int.n-c-14 | 10-j | Int.n-c-13 | | 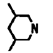 | Et | H | H | S | C | | 316 (M⁺+1) |
| Int.n-c-15 | 13-h | Int.n-c-14 | | 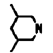 | Et | H | Br | S | C | | 394 (M⁺) |
| Int.n-c-16 | 10k- 1 | Int.n-a-8 | | 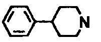 | Et | H | H | D | C | | 362 (M⁺+1) |
| Int.n-c-17 | 10-j | Int.n-c-16 | | 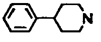 | Et | H | H | S | C | | 364 (M⁺+1) |
| Int.n-c-18 | 13-h | Int.n-c-17 | | 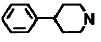 | Et | H | Br | S | C | | 442 (M⁺) |
| Int.n-c-19 | 10k- 1 | Int.n-a-9 | | 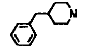 | Et | H | H | D | C | | 376 (M⁺+1) |
| Int.n-c-20 | 10-j | Int.n-c-19 | | 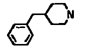 | Et | H | H | S | C | | 378 (M⁺+1) |
| Int.n-c-21 | 13-h | Int.n-c-20 | | 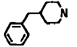 | Et | H | Br | S | C | | 456 (M⁺) |
| Int.n-c-22 | 10k- 1 | Int.n-a-10 | | 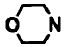 | Et | H | H | D | C | | 288 (M⁺+1) |
| Int.n-c-23 | 10-j | Int.n-c-22 | | 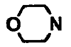 | Et | H | H | S | C | | 290 (M⁺+1) |
| Int.n-c-24 | 13-h | Int.n-c-23 | | 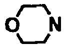 | Et | H | Br | S | C | | 368 (M⁺) |
| Int.n-c-25 | 10k- 1 | Int.n-a-11 | | 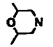 | Et | H | H | D | C | | 316 (M⁺+1) |
| Int.n-c-26 | 10-j | Int.n-c-25 | | 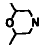 | Et | H | H | S | C | | 318 (M⁺+1) |

**[Table 144]**

| Table-Int.N-c-2 | | | | | | | | | | | |
|---|---|---|---|---|---|---|---|---|---|---|---|
| Exp. | Syn. | SM1 | SM2 | RyRz | Y | V₁' | V₂' | S/D | LCMS | | |
| | | | | | | | | | method | RTime | Mass |
| Int.n-c-27 | 13-h | Int.n-c-26 | | 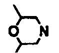 | Et | H | Br | S | C | | 396 (M⁺) |
| Int.n-c-28 | 10k- 1 | Int.n-a-12 | | 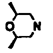 | Et | H | H | D | C | | 316 (M⁺+1) |
| Int.n-c-29 | 10-j | Int.n-c-28 | | 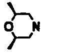 | Et | H | H | S | C | | 318 (M⁺+1) |
| Int.n-c-30 | 13-h | Int.n-c-29 | | 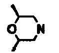 | Et | H | Br | S | C | | 396 (M⁺) |
| Int.n-c-31 | 10k- 1 | Int.n-a-13 | | 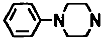 | Et | H | H | D | C | | 363 (M⁺+1) |
| Int.n-c-32 | 10-j | Int.n-c-31 | | 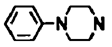 | Et | H | H | S | C | | 365 (M⁺+1) |
| Int.n-c-33 | 13-h | Int.n-c-32 | | 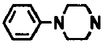 | Et | H | Br | S | C | | 443 (M⁺) |
| Int.n-c-34 | 10k- 1 | Int.n-a-14 | | 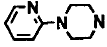 | Et | H | H | D | C | | 364 (M⁺+1) |
| Int.n-c-35 | 10-j | Int.n-c-34 | | 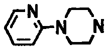 | Et | H | H | S | C | | 366 (M⁺+1) |
| Int.n-c-36 | 13-h | Int.n-c-35 | | 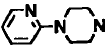 | Et | H | Br | S | C | | 444 (M⁺) |
| Int.n-c-37 | 10k- 1 | Int.n-a-15 | | 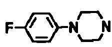 | Et | H | H | D | C | | 381 (M⁺+1) |
| Int.n-c-38 | 10-j | Int.n-c-37 | | 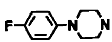 | Et | H | H | S | C | | 383 (M⁺+1) |
| Int.n-c-39 | 13-h | Int.n-c-38 | | 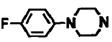 | Et | H | Br | S | C | | 461 (M⁺) |
| Int.n-c-40 | 10k- 1 | Int.n-a-16 | | 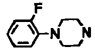 | Et | H | H | D | C | | 381 (M⁺+1) |
| Int.n-c-41 | 10-j | Int.n-c-40 | | 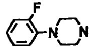 | Et | H | H | S | C | | 383 (M⁺+1) |
| Int.n-c-42 | 13-h | Int.n-c-41 | | 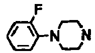 | Et | H | Br | S | C | | 461 (M⁺) |
| Int.n-c-43 | 10k- 1 | Int.n-a-17 | | 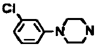 | Et | H | H | D | C | | 397 (M⁺+1) |
| Int.n-c-44 | 10-j | Int.n-c-43 | | 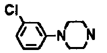 | Et | H | H | S | C | | 399 (M⁺+1) |
| Int.n-c-45 | 13-h | Int.n-c-44 | | 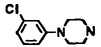 | Et | H | Br | S | C | | 477 (M⁺) |
| Int.n-c-48 | 10-j | Int.n-c-47 | | 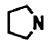 | Et | H | H | S | C | | 274 (M⁺) |
| Int.n-c-49 | 13-h | Int.n-c-48 | | 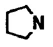 | Et | H | Br | S | C | | 352 (M⁺) |
| Int.n-c-50 | 10k- 1 | Int.n-a-18 | | 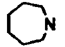 | Et | H | H | D | C | | 300 (M⁺+1) |
| Int.n-c-51 | 10-j | Int.n-c-50 | | 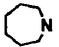 | Et | H | H | S | C | | 302 (M⁺+1) |
| Int.n-c-52 | 13-h | Int.n-c-51 | | 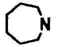 | Et | H | Br | S | C | | 380 (M⁺) |

### Example N-c-1

### Synthesis of ethyl 2-[6-(naphthalen-2-yl)-5-(piperidin-1-yl)-2,3-dihydro-1H-inden-1-yl]acetate (Compound No. N-c-1) (Preparation Method 4, Step e-1)

According to the procedure described in the synthetic method of Example Compound A-a-1 (Preparation Method 4, Step e-1) provided that the reaction was performed for 18 hours, Intermediate n-c-3 (143.3 mg), 2-naphthaleneboronic acid (254.6 mg, Aid), 2 M aqueous sodium carbonate (0.45 ml) and (Ph₃P)₄Pd (65.4 mg, Nakalai Tesque) were reacted and treated to obtain the title compound (Compound No. N-c-1, 79.4 mg).

### Example N-c-2

### Synthesis of 2-[6-(naphthalen-2-yl)-5-(piperidin-1-yl)-2,3-dihydro-1H-inden-1-yl]acetic acid (Compound No. N-b-2) (Preparation Method 1, Step a)

According to the procedure described in the synthetic method of Example Compound A-a-2 (Preparation Method 1, Step a) provided that the reaction was performed for 2 hours, Example Compound N-c-1 (65.7 mg) and 2 N aqueous sodium hydroxide (0.40 ml) were reacted and treated to obtain the title compound (Compound No. N-c-2, 57.7 mg).
Typical examples of the compounds of the present invention that can be obtained by reacting and treating corresponding starting compounds using any of the methods described in the present specification including the examples described above are shown in Table-N-C-1 to Table-N-C-8. The compounds were synthesized according to the preparation methods of the compounds of the compound numbers shown in the columns of "Syn" in the tables. "Int" means an intermediate compound number. As for the preparation of the compounds, used methods among the aforementioned synthetic methods are shown in the columns of "Syn" with symbols, the starting compounds are shown in the columns of "SM", and the reagents are shown in the columns of "Regent" in the tables. The boronic acid regents mentioned in the columns of "Reagent" with symbols "BRA (No.)" are those mentioned in Table. BRA.

**[Table 145]**

| Table-N-C-1 | | | | | | | | | | |
|---|---|---|---|---|---|---|---|---|---|---|
| 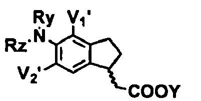 | | | | | | | | | | |

| Exp. | Syn. | SM1 | Reagent | NRyRz | Y | V₁' | V₂' | LCMS | | |
|---|---|---|---|---|---|---|---|---|---|---|
| | | | | | | | | method | RTime | Mass |
| N-c-1 | 4e-1 | Int.n-c-3 | BRA1 | 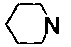 | Et | H | 2-Nap | C | | 414 (M⁺+1) |
| N-c-2 | 1-a | N-c-1 | | 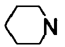 | H | H | 2-Nap | C | | 386 (M⁺+1) |
| N-c-3 | 4e-1 | Intn-c-3 | BRA2 | 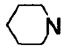 | Et | H | 5-Ind | C | | 403 (M⁺+1) |
| N-c-4 | 1-a | N-c-3 | | 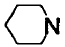 | H | H | 5-Ind | C | | 375 (M⁺+1) |
| N-c-5 | 4e-1 | Int.n-c-3 | BRA3 | 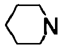 | Et | H | 1 Me-5-Ind | C | | 417 (M⁺+1) |
| N-c-6 | 1-a | N-c-5 | | 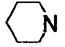 | H | H | 1 Me-5-Ind | C | | 389 (M⁺+1) |
| N-c-7 | 4e-1 | Int.n-c-3 | BRA4 | 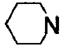 | Et | H | 1Et-5-Ind | C | | 431 (M⁺+1) |
| N-c-8 | 1-a | N-c-7 | | 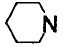 | H | H | 1Et-5-Ind | C | | 403 (M⁺+1) |
| N-c-9 | 4e-1 | Int.n-c-3 | BRA5 | 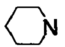 | Et | H | 1Me-4-Ind | C | | 417 (M⁺+1) |
| N-c-10 | 1-a | N-c-9 | | 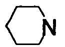 | H | H | 1Me-4-Ind | C | | 389 (M⁺+1) |
| N-c-11 | 4e-1 | Intn-c-3 | BRA6 | 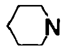 | Et | H | 1Me-6-Ind | C | | 417 (M⁺+1) |
| N-c-12 | 1-a | N-c-11 | | 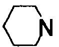 | H | H | 1Me-6-Ind | C | | 389 (M⁺+1) |
| N-c-13 | 4e-1 | Int.n-c-3 | BRA7 | 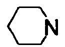 | Et | H | 5-1Idz | C | | 404 (M⁺+1) |
| N-c-14 | 1-a | N-c-13 | | 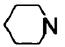 | H | H | 5-1Idz | C | | 376 (M⁺+1) |
| N-c-15 | 4e-1 | Intn-c-3 | BRA8 | 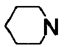 | Et | H | 1Me-5-Idz | C | | 418 (M⁺+1) |
| N-c-16 | 1-a | N-c-15 | | 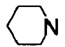 | H | H | 1Me-5-Idz | C | | 390 (M⁺+1) |
| N-c-17 | 4e-1 | Int.n-c-3 | BRA9 | 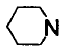 | Et | H | 1Et-5-Idz | C | | 432 (M⁺+1) |
| N-c-18 | 1-a | N-c-17 | | 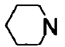 | H | H | 1Et-5-Idz | C | | 404 (M⁺+1) |
| N-c-19 | 4e-1 | Int.n-c-6 | BRA1 | 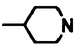 | Et | H | 2-Nap | C | | 428 (M⁺+1) |
| N-c-20 | 1-a | N-c-19 | | 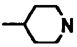 | H | H | 2-Nap | C | | 400 (M⁺+1) |
| N-c-21 | 4e-1 | Int.n-c-6 | BRA2 | 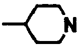 | Et | H | 5-Ind | C | | 417 (M⁺+1) |
| N-c-22 | 1-a | N-c-21 | | 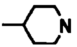 | H | H | 5-Ind | C | | 389 (M⁺+1) |

**[Table 146]**

| Table-N-C-2 | | | | | | | | | | |
|---|---|---|---|---|---|---|---|---|---|---|
| Exp. | Syn. | SM1 | Reagent | NRzRy | Y | Z | AR | LCMS | | |
| | | | | | | | | method | RTime | Mass |
| N-c-23 | 4e-1 | Int.n-c-6 | BRA3 | 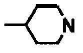 | Et | H | 1Me-5-Ind | C | | 431 (M⁺+1) |
| N-c-24 | 1-a | N-c-23 | | 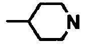 | H | H | 1Me-5-Ind | C | | 403 (M⁺+1) |
| N-c-25 | 4e-1 | Int.n-c-6 | BRA4 | 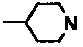 | Et | H | 1Et-5-Ind | C | | 445 (M⁺+1) |
| N-c-26 | 1-a | N-c-25 | | 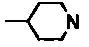 | H | H | 1Et-5-Ind | C | | 417 (M⁺+1) |
| N-c-27 | 4e-1 | Int.n-c-6 | BRA7 | 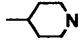 | Et | H | 5-1Idz | C | | 418 (M⁺+1) |
| N-c-28 | 1-a | N-c-27 | | 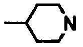 | H | H | 5-1Idz | C | | 390 (M⁺+1) |
| N-c-29 | 4e-1 | Intn-c-6 | BRA8 | 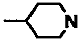 | Et | H | 1Me-5-Idz | C | | 432 (M⁺+1) |
| N-c-30 | 1-a | N-c-29 | | 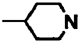 | H | H | 1Me-5-Idz | C | | 404 (M⁺+1) |
| N-c-31 | 4e-1 | Intn-c-6 | BRA9 | 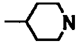 | Et | H | 1Et-5-Idz | C | | 446 (M⁺+1) |
| N-c-32 | 1-a | N-c-31 | | | H | H | 1Et-5-Idz | C | | 418 (M⁺+1) |
| N-c-33 | 4e-1 | Intn-c-9 | BRA1 | 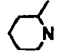 | Et | H | 2-Nap | C | | 428 (M⁺+1) |
| N-c-34 | 1-a | N-c-33 | | 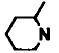 | H | H | 2-Nap | C | | 400 (M⁺+1) |
| N-c-35 | 4e-1 | Intn-c-9 | BRA2 | 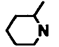 | Et | H | 5-Ind | C | | 417 (M⁺+1) |
| N-c-36 | 1-a | N-c-35 | | 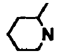 | H | H | 5-Ind | C | | 389 (M⁺+1) |
| N-c-37 | 4e-1 | Intn-c-9 | BRA3 | 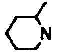 | Et | H | 1Me-5-Ind | C | | 431 (M⁺+1) |
| N-c-38 | 1-a | N-c-37 | | 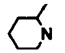 | H | H | 1Me-5-Ind | C | | 403 (M⁺+1) |
| N-c-39 | 4e-1 | Intn-c-9 | BRA7 | 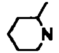 | Et | H | 5-1Idz | C | | 418 (M⁺+1) |
| N-c-40 | 1-a | N-c-39 | | 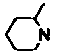 | H | H | 5-1Idz | C | | 390 (M⁺+1) |
| N-c-41 | 4e-1 | Intn-c-9 | BRA8 | 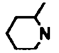 | Et | H | 1Me-5-Idz | C | | 432 (M⁺+1) |
| N-c-42 | 1-a | N-c-41 | | 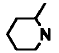 | H | H | 1Me-5-Idz | C | | 404 (M⁺+1) |
| N-c-43 | 4e-1 | Int.n-c-12 | BRA1 | 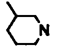 | Et | H | 2-Nap | C | | 428 (M⁺+1) |
| N-c-44 | 1-a | N-c-43 | | 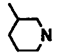 | H | H | 2-Nap | C | | 400 (M⁺+1) |

**[Table 147]**

| Table-N-C-3 | | | | | | | | | | |
|---|---|---|---|---|---|---|---|---|---|---|
| Exp. | Syn. | SM1 | Reagent | NRzRy | Y | Z | AR | LCMS | | |
| | | | | | | | | method | RTime | Mass |
| N-c-45 | 4e-1 | Int.n-c-12 | BRA3 | 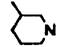 | Et | H | 1Me-5-Ind | C | | 431 (M⁺+1) |
| N-c-46 | 1-a | N-c-45 | | 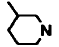 | H | H | 1Me-5-Ind | C | | 403 (M⁺+1) |
| N-c-47 | 4e-1 | Int.n-c- 12 | BRA8 | 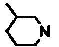 | Et | H | 1Me-5-Idz | C | | 432 (M⁺+1) |
| N-c-48 | 1-a | N-c-47 | | 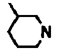 | H | H | 1Me-5-Idz | C | | 404 (M⁺+1) |
| N-c-49 | 4e-1 | Int.n-c- 12 | BRA9 | 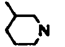 | Et | H | 1Et-5-Idz | C | | 446 (M⁺+1) |
| N-c-50 | 1-a | N-c-49 | | 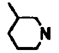 | H | H | 1Et-5-Idz | C | | 418 (M⁺+1) |
| N-c-51 | 4e-1 | Int.n-c- 15 | BRA1 | 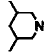 | Et | H | 2-Nap | C | | 442 (M⁺+1) |
| N-c-52 | 1-a | N-c-51 | | 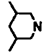 | H | H | 2-Nap | C | | 414 (M⁺+1) |
| N-c-53 | 4e-1 | Int.n-c- 15 | BRA2 | 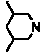 | Et | H | 5-Ind | C | | 431 (M⁺+1) |
| N-c-54 | 1-a | N-c-53 | | 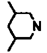 | H | H | 5-Ind | C | | 403 (M⁺+1) |
| N-c-55 | 4e-1 | Int.n-c- 15 | BRA3 | 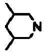 | Et | H | 1Me-5-Ind | C | | 445 (M⁺+1) |
| N-c-56 | 1-a | N-c-55 | | 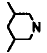 | H | H | 1Me-5-Ind | C | | 417 (M⁺+1) |
| N-c-57 | 4e-1 | Int.n-c- 15 | BRA7 | 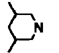 | Et | H | 5-1 Idz | C | | 432 (M⁺+1) |
| N-c-58 | 1-a | N-c-57 | | 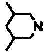 | H | H | 5-1 Idz | C | | 404 (M⁺+1) |
| N-c-59 | 4e-1 | Int.n-c- 15 | BRA8 | 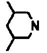 | Et | H | 1Me-5-Idz | C | | 446 (M⁺+1) |
| N-c-60 | 1-a | N-c-59 | | 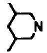 | H | H | 1Me-5-Idz | C | | 418 (M⁺+1) |
| N-c-61 | 4e-1 | Int.n-c- 18 | BRA1 | 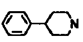 | Et | H | 2-Nap | C | | 490 (M⁺+1) |
| N-c-62 | 1-a | N-c-61 | | 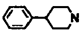 | H | H | 2-Nap | C | | 462 (M⁺+1) |
| N-c-63 | 4e-1 | Int.n-c- 18 | BRA2 | 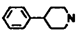 | Et | H | 5-Ind | C | | 479 (M⁺+1) |
| N-c-64 | 1-a | N-c-63 | | 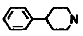 | H | H | 5-Ind | C | | 451 (M⁺+1) |
| N-c-65 | 4e-1 | Int.n-c- 18 | BRA3 | 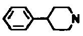 | Et | H | 1Me-5-Ind | C | | 493 (M⁺+1) |
| N-c-66 | 1-a | N-c-65 | | 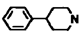 | H | H | 1Me-5-Ind | C | | 465 (M⁺+1) |

**[Table 148]**

| Table-N-C-4 | | | | | | | | | | |
|---|---|---|---|---|---|---|---|---|---|---|
| Exp. | Syn. | SM1 | Reagent | NRzRy | Y | Z | AR | LCMS | | |
| | | | | | | | | method | RTime | Mass |
| N-c-67 | 4e-1 | Int.n-c- 18 | BRA7 | 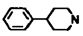 | Et | H | 5-1 Idz | C | | 480 (M⁺+1) |
| N-c-68 | 1-a | N-c-67 | | 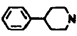 | H | H | 5-1Idz | C | | 452 (M⁺+1) |
| N-c-69 | 4e-1 | Intn-c- 18 | BRA8 | 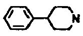 | Et | H | 1Me-5-Idz | C | | 494 (M⁺+1) |
| N-c-70 | 1-a | N-c-69 | | 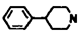 | H | H | 1Me-5-Idz | C | | 466 (M⁺+1) |
| N-c-71 | 4e-1 | Int.n-c- 21 | BRA1 | 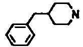 | Et | H | 2-Nap | C | | 504 (M⁺+1) |
| N-c-72 | 1-a | N-c-71 | | 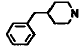 | H | H | 2-Nap | C | | 476 (M⁺+1) |
| N-c-73 | 4e-1 | Int.n-c- 21 | BRA2 | 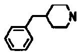 | Et | H | 5-Ind | C | | 493 (M⁺+1) |
| N-c-74 | 1-a | N-c-73 | | 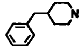 | H | H | 5-Ind | C | | 465 (M⁺+1) |
| N-c-75 | 4e-1 | Int.n-c- 21 | BRA3 | 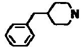 | Et | H | 1Me-5-Ind | C | | 507 (M⁺+1) |
| N-c-76 | 1-a | N-c-75 | | 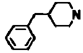 | H | H | 1Me-5-Ind | C | | 479 (M⁺+1) |
| N-c-77 | 4e-1 | Int.n-c- 21 | BRA8 | 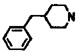 | Et | H | 1Me-5-Idz | C | | 508 (M⁺+1) |
| N-c-78 | 1-a | N-c-77 | | 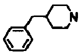 | H | H | 1Me-5-Idz | C | | 480 (M⁺+1) |
| N-c-79 | 4e-1 | Intn-c- 24 | BRA1 | 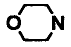 | Et | H | 2-Nap | C | | 416 (M⁺+1) |
| N-c-80 | 1-a | N-c-79 | | 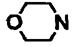 | H | H | 2-Nap | C | | 388 (M⁺+1) |
| N-c-81 | 4e-1 | Intn-c- 24 | BRA2 | 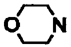 | Et | H | 5-Ind | C | | 405 (M⁺+1) |
| N-c-82 | 1-a | N-c-81 | | 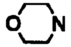 | H | H | 5-Ind | C | | 377 (M⁺+1) |
| N-c-83 | 4e-1 | Int.n-c- 24 | BRA3 | 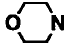 | Et | H | 1Me-5-Ind | C | | 419 (M⁺+1) |
| N-c-84 | 1-a | N-c-83 | | 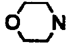 | H | H | 1Me-5-Ind | C | | 391 (M⁺+1) |
| N-c-85 | 4e-1 | Int.n-c- 24 | BRA7 | 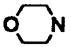 | Et | H | 5-1Idz | C | | 406 (M⁺+1) |
| N-c-86 | 1-a | N-c-85 | | 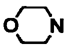 | H | H | 5-1Idz | C | | 378 (M⁺+1) |
| N-c-87 | 4e-1 | Int.n-c- 24 | BRA8 | 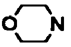 | Et | H | 1Me-5-Idz | C | | 420 (M⁺+1) |
| N-c-88 | 1-a | N-c-87 | | 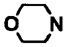 | H | H | 1Me-5-Idz | C | | 392 (M⁺+1) |

**[Table 149]**

| Table-N-C-5 | | | | | | | | | | |
|---|---|---|---|---|---|---|---|---|---|---|
| Exp. | Syn. | SM1 | Reagent | NRzRy | Y | Z | AR | LCMS | | |
| | | | | | | | | method | RTime | Mass |
| N-c-89 | 4e-1 | Int.n-c-27 | BRA1 | 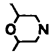 | Et | H | 2-Nap | C | | 444 (M⁺+1) |
| N-c-90 | 1-a | N-c-89 | | 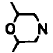 | H | H | 2-Nap | C | | 416 (M⁺+1) |
| N-c-91 | 4e-1 | Int.n-c-27 | BRA2 | 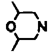 | Et | H | 5-Ind | C | | 433 (M⁺+1) |
| N-c-92 | 1-a | N-c-91 | | 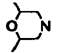 | H | H | 5-Ind | C | | 405 (M⁺+1) |
| N-c-93 | 4e-1 | Int.n-c-27 | BRA3 | 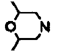 | Et | H | 1Me-5-Ind | C | | 447 (M⁺+1) |
| N-c-94 | 1-a | N-c-93 | | 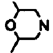 | H | H | 1Me-5-Ind | C | | 419 (M⁺+1) |
| N-c-95 | 4e-1 | Int.n-c-27 | BRA7 | 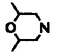 | Et | H | 5-1Idz | C | | 434 (M⁺+1) |
| N-c-96 | 1-a | N-c-95 | | 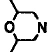 | H | H | 5-1Idz | C | | 406 (M⁺+1) |
| N-c-97 | 4e-1 | Int.n-c-27 | BRA8 | 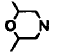 | Et | H | 1Me-5-Idz | C | | 448 (M⁺+1) |
| N-c-98 | 1-a | N-c-97 | | 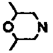 | H | H | 1Me-5-Idz | C | | 420 (M⁺+1) |
| N-c-99 | 4e-1 | Int.n-c-30 | BRA1 | 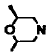 | Et | H | 2-Nap | C | | 444 (M⁺+1) |
| N-c-100 | 1-a | N-c-99 | | 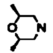 | H | H | 2-Nap | C | | 416 (M⁺+1) |
| N-c-101 | 4e-1 | Int.n-c-30 | BRA2 | 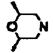 | Et | H | 5-Ind | C | | 433 (M⁺+1) |
| N-c-102 | 1-a | N-c-101 | | 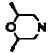 | H | H | 5-Ind | C | | 405 (M⁺+1) |
| N-c-103 | 4e-1 | Int.n-c-30 | BRA7 | 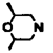 | Et | H | 5-1Idz | C | | 434 (M⁺+1) |
| N-c-104 | 1-a | N-c-103 | | 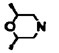 | H | H | 5-1Idz | C | | 406 (M⁺+1) |
| N-c-105 | 4e-1 | Int.n-c-33 | BRA1 | 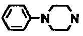 | Et | H | 2-Nap | C | | 491 (M⁺+1) |
| N-c-106 | 1-a | N-c-105 | | 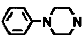 | H | H | 2-Nap | C | | 463 (M⁺+1) |
| N-c-107 | 4e-1 | Int.n-c-33 | BRA2 | 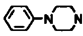 | Et | H | 5-Ind | C | | 480 (M⁺+1) |
| N-c-108 | 1-a | N-c-107 | | 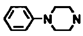 | H | H | 5-Ind | C | | 452 (M⁺+1) |
| N-c-109 | 4e-1 | Int.n-c-33 | BRA3 | 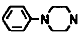 | Et | H | 1Me-5-Ind | C | | 494 (M⁺+1) |
| N-c-110 | 1-a | N-c-109 | | 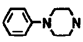 | H | H | 1Me-5-Ind | C | | 466 (M⁺+1) |

**[Table 150]**

| Table-N-C-6 | | | | | | | | | | |
|---|---|---|---|---|---|---|---|---|---|---|
| Exp. | Syn. | SM1 | Reagent | NRzRy | Y | Z | AR | LCMS | | |
| | | | | | | | | method | RTime | Mass |
| N-c-111 | 4e-1 | Int.n-c-33 | BRA7 | 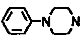 | Et | H | 5-1Idz | C | | 481 (M⁺+1) |
| N-c-112 | 1-a | N-c-111 | | 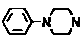 | H | H | 5-1Idz | C | | 453 (M⁺+1) |
| N-c-113 | 4e-1 | Int.n-c-33 | BRA8 | 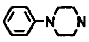 | Et | H | 1Me-5-Idz | C | | 495 (M⁺+1) |
| N-c-114 | 1-a | N-c-113 | | 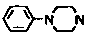 | H | H | 1Me-5-Idz | C | | 467 (M⁺+1) |
| N-c-115 | 4e-1 | Int.n-c-36 | BRA1 | 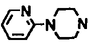 | Et | H | 2-Nap | C | | 492 (M⁺+1) |
| N-c-116 | 1-a | N-c-115 | | 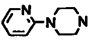 | H | H | 2-Nap | C | | 464 (M⁺+1) |
| N-c-117 | 4e-1 | Int.n-c-36 | BRA3 | 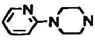 | Et | H | 1Me-5-Ind | C | | 495 (M⁺+1) |
| N-c-118 | 1-a | N-c-117 | | 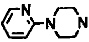 | H | H | 1Me-5-Ind | C | | 467 (M⁺+1) |
| N-c-119 | 4e-1 | Int.n-c-36 | BRA8 | 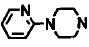 | Et | H | 1Me-5-Idz | C | | 496 (M⁺+1) |
| N-c-120 | 1-a | N-c-119 | | 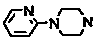 | H | H | 1Me-5-Idz | C | | 468 (M⁺+1) |
| N-c-121 | 4e-1 | Int.n-c-39 | BRA1 | 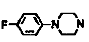 | Et | H | 2-Nap | C | | 509 (M⁺+1) |
| N-c-122 | 1-a | N-c-121 | | 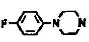 | H | H | 2-Nap | C | | 481 (M⁺+1) |
| N-c-123 | 4e-1 | Int.n-c-39 | BRA2 | 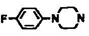 | Et | H | 5-Ind | C | | 498 (M⁺+1) |
| N-c-124 | 1-a | N-c-123 | | 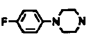 | H | H | 5-Ind | C | | 470 (M⁺+1) |
| N-c-125 | 4e-1 | Int.n-c-39 | BRA7 | 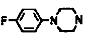 | Et | H | 5-1Idz | C | | 499 (M⁺+1) |
| N-c-126 | 1-a | N-c-125 | | 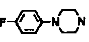 | H | H | 5-1Idz | C | | 471 (M⁺+1) |
| N-c-127 | 4e-1 | Int.n-c-42 | BRA1 | 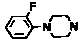 | Et | H | 2-Nap | C | | 509 (M⁺+1) |
| N-c-128 | 1-a | N-c-127 | | 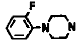 | H | H | 2-Nap | C | | 481 (M⁺+1) |
| N-c-129 | 4e-1 | Int.n-c-42 | BRA3 | 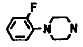 | Et | H | 1Me-5-Ind | C | | 512 (M⁺+1) |
| N-c-130 | 1-a | N-c-129 | | 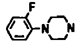 | H | H | 1Me-5-Ind | C | | 484 (M⁺+1) |
| N-c-131 | 4e-1 | Int.n-c-42 | BRA8 | 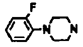 | Et | H | 1Me-5-Idz | C | | 513 (M⁺+1) |
| N-c-132 | 1-a | N-c-131 | | 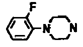 | H | H | 1Me-5-Idz | C | | 485 (M⁺+1) |

**[Table 151]**

| Table-N-C-7 | | | | | | | | | | |
|---|---|---|---|---|---|---|---|---|---|---|
| Exp. | Syn. | SM1 | Reagent | NRzRy | Y | Z | AR | LCMS | | |
| | | | | | | | | method | RTime | Mass |
| N-c-133 | 4e-1 | Intn-c-45 | BRA1 | 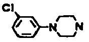 | Et | H | 2-Nap | C | | 526 (M⁺+1) |
| N-c-134 | 1-a | N-c-133 | | 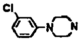 | H | H | 2-Nap | C | | 498 (M⁺+1) |
| N-c-135 | 4e-1 | Intn-c-45 | BRA2 | 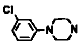 | Et | H | 5-Ind | C | | 515 (M⁺+1) |
| N-c-136 | 1-a | N-c-135 | | 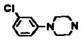 | H | H | 5-Ind | C | | 487 (M⁺+1) |
| N-c-137 | 4e-1 | Intn-c-45 | BRA3 | 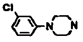 | Et | H | 1Me-5-Ind | C | | 529 (M⁺+1) |
| N-c-138 | 1-a | N-c-137 | | 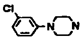 | H | H | 1Me-5-Ind | C | | 501 (M⁺+1) |
| N-c-139 | 4e-1 | Int.n-c-45 | BRA5 | 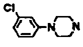 | Et | H | 1Me-4-Ind | C | | 529 (M⁺+1) |
| N-c-140 | 1-a | N-c-139 | | 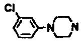 | H | H | 1Me-4-Ind | C | | 501 (M⁺+1) |
| N-c-141 | 4e-1 | Intn-c-45 | BRA7 | 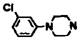 | Et | H | 5-1Idz | C | | 516 (M⁺+1) |
| N-c-142 | 1-a | N-c-141 | | 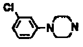 | H | H | 5-1Idz | C | | 488 (M⁺+1) |
| N-c-143 | 4e-1 | Int.n-c-45 | BRA8 | 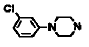 | Et | H | 1Me-5-Idz | C | | 530 (M⁺+1) |
| N-c-144 | 1-a | N-c-143 | | 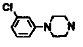 | H | H | 1Me-5-Idz | C | | 502 (M⁺+1) |
| N-c-145 | 4e-1 | Int.n-c-49 | BRA1 | 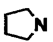 | Et | H | 2-Nap | C | | 400 (M⁺+1) |
| N-c-146 | 1-a | N-c-145 | | 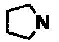 | H | H | 2-Nap | C | | 372 (M⁺+1) |
| N-c-147 | 4e-1 | Int.n-c-49 | BRA2 | 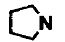 | Et | H | 5-Ind | C | | 389 (M⁺+1) |
| N-c-148 | 1-a | N-c-147 | | 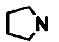 | H | H | 5-Ind | C | | 361 (M⁺+1) |
| N-c-149 | 4e-1 | Intn-c-49 | BRA3 | 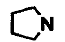 | Et | H | 1Me-5-Ind | C | | 403 (M⁺+1) |
| N-c-150 | 1-a | N-c-149 | | 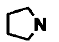 | H | H | 1Me-5-Ind | C | | 375 (M⁺+1) |
| N-c-151 | 4e-1 | Intn-c-49 | BRA7 | 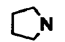 | Et | H | 5-1Idz | C | | 390 (M⁺+1) |
| N-c-152 | 1-a | N-c-151 | | 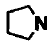 | H | H | 5-1Idz | C | | 362 (M⁺+1) |
| N-c-153 | 4e-1 | Intn-c-49 | BRA8 | 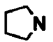 | Et | H | 1Me-5-Idz | C | | 404 (M⁺+1) |
| N-c-154 | 1-a | N-c-153 | | 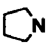 | H | H | 1Me-5-Idz | C | | 376 (M⁺+1) |

**[Table 152]**

| Table-N-C-8 | | | | | | | | | | |
|---|---|---|---|---|---|---|---|---|---|---|
| Exp. | Syn. | SM1 | Reagent | NRzRy | Y | Z | AR | LCMS | | |
| | | | | | | | | method | RTime | Mass |
| N-c-155 | 4e-1 | Int.n-c-52 | BRA1 | 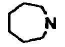 | Et | H | 2-Nap | C | | 428 (M⁺+1) |
| N-c-156 | 1-a | N-c-155 | | 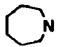 | H | H | 2-Nap | C | | 400 (M⁺+1) |
| N-c-157 | 4e-1 | Int.n-c-52 | BRA2 | 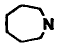 | Et | H | 5-Ind | C | | 417 (M⁺+1) |
| N-c-158 | 1-a | N-c-157 | | 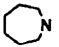 | H | H | 5-Ind | C | | 389 (M⁺+1) |
| N-c-159 | 4e-1 | Int.n-c-52 | BRA3 | 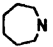 | Et | H | 1Me-5-Ind | C | | 431 (M⁺+1) |
| N-c-160 | 1-a | N-c-159 | | 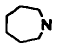 | H | H | 1Me-5-Ind | C | | 403 (M⁺+1) |
| N-c-161 | 4e-1 | Int.n-c-52 | BRA7 | 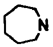 | Et | H | 5-1Idz | C | | 418 (M⁺+1) |
| N-c-162 | 1-a | N-c-161 | | 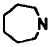 | H | H | 5-1Idz | C | | 390 (M⁺+1) |
| N-c-163 | 4e-1 | Int.n-c-52 | BRA8 | 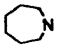 | Et | H | 1Me-5-Idz | C | | 432 (M⁺+1) |
| N-c-164 | 1-a | N-c-163 | | 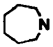 | H | H | 1Me-5-Idz | C | | 404 (M⁺+1) |

### Example N-d-1

### Example Compound N-a-2 was subjected to chiral separation to obtain the title compound (Compound No. N-d-1, 10.3 mg).

Typical examples of the compounds of the present invention that can be obtained by reacting and treating corresponding starting compounds using any of the methods described in the present specification including the examples described above are shown in Table-N-D-1 to Table-N-D-3. As for the preparation of the compounds, all of the objective compounds were obtained by using HPLC separation. In the tables, used methods among the aforementioned synthetic methods are shown in the columns of "Syn" with symbols, the starting compounds are shown in the columns of "SM", and the reagents are shown in the columns of "Regent".

**[Table 153]**

| Table-N-D-1 | | | | | | | | | | | |
|---|---|---|---|---|---|---|---|---|---|---|---|
| 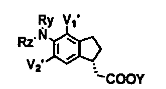 | | | | | | | | | | | |

| Exp. | Syn. | SM1 | Reagent | Ry | Rz | Y | V₁' | V₂' | LCMS | | |
|---|---|---|---|---|---|---|---|---|---|---|---|
| | | | | | | | | | method | RTime | Mass |
| N-d-1 | | N-a-2 | | H | Me | H | H | 2-Nap | C | | 332 (M⁺+1) |
| N-d-2 | | N-a-4 | | H | Me | H | H | 5-Ind | C | | 321 (M⁺+1) |
| N-d-3 | | N-a-6 | | H | Me | H | H | 1Me-5-Ind | C | | 335 (M⁺+1) |
| N-d-4 | | N-a-8 | | H | Me | H | H | 1Et-5-Ind | C | | 349 (M⁺+1) |
| N-d-5 | | N-a-10 | | H | Me | H | H | 1Me-4-Ind | C | | 335 (M⁺+1) |
| N-d-6 | | N-a-12 | | H | Me | H | H | 1Me-6-Ind | C | | 335 (M⁺+1) |
| N-d-7 | | N-a-14 | | H | Me | H | H | 5-1Idz | C | | 322 (M⁺+1) |
| N-d-8 | | N-a-16 | | H | Me | H | H | 1Me-5-Idz | C | | 336 (M⁺+1) |
| N-d-9 | | N-a-18 | | H | Me | H | H | 1Et-5-Idz | C | | 350 (M⁺+1) |
| N-d-10 | | N-a-20 | | H | Me | H | H | 2Me-5-Idz | C | | 336 (M⁺+1) |
| N-d-11 | | N-a-22 | | H | Me | H | H | 5-BF | C | | 322 (M⁺+1) |
| N-d-12 | | N-a-24 | | H | Me | H | H | 5-Bzt | C | | 339 (M⁺+1) |
| N-d-13 | | N-a-26 | | H | Me | H | H | 3-Qu | C | | 333 (M⁺+1) |
| N-d-14 | | N-a-28 | | H | Me | H | H | 6-Qu | C | | 333 (M⁺+1) |
| N-d-15 | | N-a-30 | | Me | Me | H | H | 6-IQ | C | | 347 (M⁺+1) |
| N-d-16 | | N-a-32 | | Me | Me | H | H | 2-Nap | C | | 346 (M⁺+1) |
| N-d-17 | | N-a-34 | | Me | Me | H | H | 5-Ind | C | | 335 (M⁺+1) |
| N-d-18 | | N-a-36 | | Me | Me | H | H | 1Me-5-Ind | C | | 349 (M⁺+1) |
| N-d-19 | | N-a-38 | | Me | Me | H | H | 5-1Idz | C | | 336 (M⁺+1) |
| N-d-20 | | N-a-40 | | Me | Me | H | H | 1Me-5-Idz | C | | 350 (M⁺+1) |
| N-d-21 | | N-a-42 | | Me | Me | H | H | 6-Qu | C | | 347 (M⁺+1) |
| N-d-22 | | N-a-44 | | Et | Me | H | H | 2-Nap | C | | 360 (M⁺+1) |
| N-d-23 | | N-a-46 | | Et | Me | H | H | 5-Ind | C | | 349 (M⁺+1) |
| N-d-24 | | N-a-48 | | Et | Me | H | H | 1Me-5-Ind | C | | 363 (M⁺+1) |
| N-d-25 | | N-a-50 | | Et | Me | H | H | 1Et-5-Ind | C | | 377 (M⁺+1) |
| N-d-26 | | N-a-52 | | Et | Me | H | H | 1Me-4-Ind | C | | 363 (M⁺+1) |
| N-d-27 | | N-a-54 | | Et | Me | H | H | 1Me-6-Ind | C | | 363 (M⁺+1) |
| N-d-28 | | N-a-56 | | Et | Me | H | H | 5-1Idz | C | | 350 (M⁺+1) |
| N-d-29 | | N-a-58 | | Et | Me | H | H | 1Me-5-Idz | C | | 364 (M⁺+1) |
| N-d-30 | | N-a-60 | | Et | Me | H | H | 3-Qu | C | | 361 (M⁺+1) |
| N-d-31 | | N-a-62 | | Et | Me | H | H | 6-IQ | C | | 361 (M⁺+1) |
| N-d-32 | | N-a-64 | | nPr | Me | H | H | 2-Nap | C | | 374 (M⁺+1) |
| N-d-33 | | N-a-66 | | nPr | Me | H | H | 5-Ind | C | | 363 (M⁺+1) |
| N-d-34 | | N-a-68 | | nPr | Me | H | H | 1Me-5-Ind | C | | 377 (M⁺+1) |
| N-d-35 | | N-a-70 | | nPr | Me | H | H | 5-1Idz | C | | 364 (M⁺+1) |
| N-d-36 | | N-a-72 | | nPr | Me | H | H | 1Me-5-Idz | C | | 378 (M⁺+1) |
| N-d-37 | | N-a-74 | | nPr | Me | H | H | 5-BF | C | | 364 (M⁺+1) |
| N-d-38 | | N-a-76 | | nPr | Me | H | H | 3-Qu | C | | 375 (M⁺+1) |
| N-d-39 | | N-a-78 | | iPr | Me | H | H | 2-Nap | C | | 374 (M⁺+1) |
| N-d-40 | | N-a-80 | | iPr | Me | H | H | 5-Ind | C | | 363 (M⁺+1) |
| N-d-41 | | N-a-82 | | iPr | Me | H | H | 1Me-5-Ind | C | | 377 (M⁺+1) |
| N-d-42 | | N-a-84 | | iPr | Me | H | H | 1Et-5-Ind | C | | 391 (M⁺+1) |
| N-d-43 | | N-a-86 | | iPr | Me | H | H | 1Me-6-Ind | C | | 377 (M⁺+1) |
| N-d-44 | | N-a-88 | | iPr | Me | H | H | 5-1Idz | C | | 364 (M⁺+1) |

**[Table 154]**

| Table-N-D-2 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-d-45 | | N-a-90 | | iPr | Me | H | H | 1Me-5-Idz | C | | 378 (M⁺+1) |
| N-d-46 | | N-a-92 | | nBu | Me | H | H | 2-Nap | C | | 388 (M⁺+1) |
| N-d-47 | | N-a-94 | | nBu | Me | H | H | 5-Ind | C | | 377 (M⁺+1) |
| N-d-48 | | N-a-96 | | nBu | Me | H | H | 1Me-5-Ind | C | | 391 (M⁺+1) |
| N-d-49 | | N-a-98 | | nBu | Me | H | H | 1Me-6-Ind | C | | 391 (M⁺+1) |
| N-d-50 | | N-a-100 | | nBu | Me | H | H | 5-1Idz | C | | 378 (M⁺+1) |
| N-d-51 | | N-a-102 | | nBu | Me | H | H | 1Me-5-Idz | C | | 392 (M⁺+1) |
| N-d-52 | | N-a-104 | | nBu | Me | H | H | 3-Qu | C | | 389 (M⁺+1) |
| N-d-53 | | N-a-106 | | nBu | Me | H | H | 6-Qu | C | | 389 (M⁺+1) |
| N-d-54 | | N-a-108 | | iBu | Me | H | H | 2-Nap | C | | 388 (M⁺+1) |
| N-d-55 | | N-a-110 | | iBu | Me | H | H | 5-Ind | C | | 377 (M⁺+1) |
| N-d-56 | | N-a-112 | | iBu | Me | H | H | 1Me-5-Ind | C | | 391 (M⁺+1) |
| N-d-57 | | N-a-114 | | iBu | Me | H | H | 1Et-5-Ind | C | | 405 (M⁺+1) |
| N-d-58 | | N-a-116 | | iBu | Me | H | H | 1Me-6-Ind | C | | 391 (M⁺+1) |
| N-d-59 | | N-a-118 | | iBu | Me | H | H | 5-1Idz | C | | 378 (M⁺+1) |
| N-d-60 | | N-a-120 | | iBu | Me | H | H | 1Me-5-Idz | C | | 392 (M⁺+1) |
| N-d-61 | | N-a-122 | | iBu | Me | H | H | 1Et-5-Idz | C | | 406 (M⁺+1) |
| N-d-62 | | N-a-124 | | iBu | Me | H | H | 2-Nap | C | | 388 (M⁺+1) |
| N-d-63 | | N-a-126 | | cPen | Me | H | H | 5-Ind | C | | 389 (M⁺+1) |
| N-d-64 | | N-a-128 | | cPen | Me | H | H | 1Me-5-Ind | C | | 403 (M⁺+1) |
| N-d-65 | | N-a-130 | | cPen | Me | H | H | 1Et-5-Ind | C | | 417 (M⁺+1) |
| N-d-66 | | N-a-132 | | cPen | Me | H | H | 5-1Idz | C | | 390 (M⁺+1) |
| N-d-67 | | N-a-134 | | cPen | Me | H | H | 1Me-5-Idz | C | | 404 (M⁺+1) |
| N-d-68 | | N-a-136 | | cPen | Me | H | H | 1Et-5-Idz | C | | 418 (M⁺+1) |
| N-d-69 | | N-a-138 | | cHex | Me | H | H | 2-Nap | C | | 414 (M⁺+1) |
| N-d-70 | | N-a-140 | | cHex | Me | H | H | 5-Ind | C | | 403 (M⁺+1) |
| N-d-71 | | N-a-142 | | cHex | Me | H | H | 1Me-5-Ind | C | | 417 (M⁺+1) |
| N-d-72 | | N-a-144 | | cHex | Me | H | H | 1Me-4-Ind | C | | 417 (M⁺+1) |
| N-d-73 | | N-a-146 | | cHex | Me | H | H | 1Me-6-Ind | C | | 417 (M⁺+1) |
| N-d-74 | | N-a-148 | | cHex | Me | H | H | 5-1Idz | C | | 404 (M⁺+1) |
| N-d-75 | | N-a-150 | | cHex | Me | H | H | 1Me-5-Idz | C | | 418 (M⁺+1) |
| N-d-76 | | N-a-152 | | cHex | Me | H | H | 1Et-5-Idz | C | | 432 (M⁺+1) |
| N-d-77 | | N-a-154 | | 2MecHex | Me | H | H | 2-Nap | C | | 428 (M⁺+1) |
| N-d-78 | | N-a-156 | | 2MecHex | Me | H | H | 5-Ind | C | | 417 (M⁺+1) |
| N-d-79 | | N-a-158 | | 2MecHex | Me | H | H | 1Me-5-Ind | C | | 431 (M⁺+1) |
| N-d-80 | | N-a-160 | | 2MecHex | Me | H | H | 1Me-6-Ind | C | | 431 (M⁺+1) |
| N-d-81 | | N-a-162 | | 2MecHex | Me | H | H | 5-1Idz | C | | 418 (M⁺+1) |
| N-d-82 | | N-a-164 | | 2MecHex | Me | H | H | 1Me-5-Idz | C | | 432 (M⁺+1) |
| N-d-83 | | N-a-166 | | 2Indane | Me | H | H | 2-Nap | C | | 448 (M⁺+1) |
| N-d-84 | | N-a-168 | | 2Indane | Me | H | H | 5-Ind | C | | 437 (M⁺+1) |
| N-d-85 | | N-a-170 | | 2Indane | Me | H | H | 1Me-5-Ind | C | | 451 (M⁺+1) |
| N-d-86 | | N-a-172 | | 2Indane | Me | H | H | 5-1Idz | C | | 438 (M⁺+1) |
| N-d-87 | | N-a-174 | | 2Indane | Me | H | H | 1Me-5-Idz | C | | 452 (M⁺+1) |
| N-d-88 | | N-a-176 | | H | Et | H | H | 2-Nap | C | | 346 (M⁺+1) |
| N-d-89 | | N-a-178 | | H | Et | H | H | 5-Ind | C | | 335 (M⁺+1) |
| N-d-90 | | N-a-180 | | H | Et | H | H | 1Me-5-Ind | C | | 349 (M⁺+1) |

**[Table 155]**

| Table-N-D-3 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-d-91 | | N-a-182 | | H | Et | H | H | 1Et-5-Ind | C | | 363 (M⁺+1) |
| N-d-92 | | N-a-184 | | H | Et | H | H | 5-1Idz | C | | 336 (M⁺+1) |
| N-d-93 | | N-a-186 | | H | Et | H | H | 1Me-5-Idz | C | | 350 (M⁺+1) |
| N-d-94 | | N-a-188 | | H | Et | H | H | 1Et-5-Idz | C | | 364 (M⁺+1) |
| N-d-95 | | N-a-190 | | Et | Et | H | H | 2-Nap | C | | 374 (M⁺+1) |
| N-d-96 | | N-a-192 | | Et | Et | H | H | 5-Ind | C | | 363 (M⁺+1) |
| N-d-97 | | N-a-194 | | Et | Et | H | H | 1Me-5-Ind | C | | 377 (M⁺+1) |
| N-d-98 | | N-a-196 | | Et | Et | H | H | 5-1Idz | C | | 364 (M⁺+1) |
| N-d-99 | | N-a-198 | | Et | Et | H | H | 1Me-5-Idz | C | | 378 (M⁺+1) |
| N-d-100 | | N-a-200 | | Et | Et | H | H | 1Et-5-Idz | C | | 392 (M⁺+1) |
| N-d-101 | | N-a-202 | | nPr | Et | H | H | 2-Nap | C | | 388 (M⁺+1) |
| N-d-102 | | N-a-204 | | nPr | Et | H | H | 5-Ind | C | | 377 (M⁺+1) |
| N-d-103 | | N-a-206 | | nPr | Et | H | H | 1Me-5-Ind | C | | 391 (M⁺+1) |
| N-d-104 | | N-a-208 | | nPr | Et | H | H | 5-1Idz | C | | 378 (M⁺+1) |
| N-d-105 | | N-a-210 | | nPr | Et | H | H | 1Me-5-Idz | C | | 392 (M⁺+1) |
| N-d-106 | | N-a-212 | | iPr | Et | H | H | 2-Nap | C | | 388 (M⁺+1) |
| N-d-107 | | N-a-214 | | iPr | Et | H | H | 5-Ind | C | | 377 (M⁺+1) |
| N-d-108 | | N-a-216 | | iPr | Et | H | H | 1Et-5-Ind | C | | 405 (M⁺+1) |
| N-d-109 | | N-a-218 | | iPr | Et | H | H | 5-1Idz | C | | 378 (M⁺+1) |
| N-d-110 | | N-a-220 | | iPr | Et | H | H | 1Et-5-Idz | C | | 406 (M⁺+1) |
| N-d-111 | | N-a-222 | | nBu | Et | H | H | 2-Nap | C | | 402 (M⁺+1) |
| N-d-112 | | N-a-224 | | nBu | Et | H | H | 5-Ind | C | | 391 (M⁺+1) |
| N-d-113 | | N-a-226 | | nBu | Et | H | H | 5-1Idz | C | | 392 (M⁺+1) |
| N-d-114 | | N-a-228 | | nBu | Et | H | H | 1Me-5-Idz | C | | 406 (M⁺+1) |
| N-d-115 | | N-a-230 | | iBu | Et | H | H | 2-Nap | C | | 402 (M⁺+1) |
| N-d-116 | | N-a-232 | | iBu | Et | H | H | 5-Ind | C | | 391 (M⁺+1) |
| N-d-117 | | N-a-234 | | iBu | Et | H | H | 1Me-5-Ind | C | | 405 (M⁺+1) |
| N-d-118 | | N-a-236 | | iBu | Et | H | H | 5-1Idz | C | | 392 (M⁺+1) |
| N-d-119 | | N-a-238 | | iBu | Et | H | H | 1Me-5-Idz | C | | 406 (M⁺+1) |
| N-d-120 | | N-a-240 | | iBu | Et | H | H | 1Et-5-Idz | C | | 420 (M⁺+1) |
| N-d-121 | | N-a-242 | | cPen | Et | H | H | 2-Nap | C | | 414 (M⁺+1) |
| N-d-122 | | N-a-244 | | cPen | Et | H | H | 5-Ind | C | | 403 (M⁺+1) |
| N-d-123 | | N-a-246 | | cPen | Et | H | H | 1Me-5-Ind | C | | 417 (M⁺+1) |
| N-d-124 | | N-a-248 | | cPen | Et | H | H | 1Et-5-Ind | C | | 431 (M⁺+1) |
| N-d-125 | | N-a-250 | | cPen | Et | H | H | 5-1Idz | C | | 404 (M⁺+1) |
| N-d-126 | | N-a-252 | | cPen | Et | H | H | 1Me-5-Idz | C | | 418 (M⁺+1) |
| N-d-127 | | N-a-254 | | cPen | Et | H | H | 1Et-5-Idz | C | | 432 (M⁺+1) |
| N-d-128 | | N-a-256 | | cHex | Et | H | H | 2-Nap | C | | 428 (M⁺+1) |
| N-d-129 | | N-a-258 | | cHex | Et | H | H | 5-Ind | C | | 417 (M⁺+1) |
| N-d-130 | | N-a-260 | | cHex | Et | H | H | 1Me-5-Ind | C | | 431 (M⁺+1) |
| N-d-131 | | N-a-262 | | cHex | Et | H | H | 5-1Idz | C | | 418 (M⁺+1) |
| N-d-132 | | N-a-264 | | cHex | Et | H | H | 1Me-5-Idz | C | | 432 (M⁺+1) |
| N-d-133 | | N-a-266 | | 2Indane | Et | H | H | 2-Nap | C | | 462 (M⁺+1) |
| N-d-134 | | N-a-268 | | 2Indane | Et | H | H | 5-Ind | C | | 451 (M⁺+1) |
| N-d-135 | | N-a-270 | | 2Indane | Et | H | H | 1Me-5-Ind | C | | 465 (M⁺+1) |
| N-d-136 | | N-a-272 | | 2Indane | Et | H | H | 5-1Idz | C | | 452 (M⁺+1) |
| N-d-137 | | N-a-274 | | 2Indane | Et | H | H | 1Me-5-Idz | C | | 466 (M⁺+1) |

### Example N-e-1

### Example Compound N-a-2 was subjected to chiral separation to obtain the title compound (Compound No. N-e-1, 12.1 mg).

Typical example compounds that can be obtained by reacting and treating corresponding starting compounds using any of the methods described in the present specification including the examples described above are shown in Table-N-E-1 to Table-N-E-3. As for the preparation of the compounds, all of the objective compounds were obtained by using HPLC separation. In the tables, used methods among the aforementioned synthetic methods are shown in the columns of "Syn" with symbols, the starting compounds are shown in the columns of "SM", and the reagents are shown in the columns of "Regent".

**[Table 156]**

| Table-N-E-1 | | | | | | | | | | | |
|---|---|---|---|---|---|---|---|---|---|---|---|
| 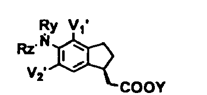 | | | | | | | | | | | |

| Exp. | Syn. | SM1 | Reagent | Ry | Rz | Y | V₁' | V₂' | LCMS | | |
|---|---|---|---|---|---|---|---|---|---|---|---|
| | | | | | | | | | method | RTime | Mass |
| N-e-1 | | N-a-2 | | H | Me | H | H | 2-Nap | C | | 332 (M⁺+1) |
| N-e-2 | | N-a-4 | | H | Me | H | H | 5-Ind | C | | 321 (M⁺+1) |
| N-e-3 | | N-a-6 | | H | Me | H | H | 1Me-5-Ind | C | | 335 (M⁺+1) |
| N-e-4 | | N-a-8 | | H | Me | H | H | 1Et-5-Ind | C | | 349 (M⁺+1) |
| N-e-5 | | N-a-10 | | H | Me | H | H | 1Me-4-Ind | C | | 335 (M⁺+1) |
| N-e-6 | | N-a-12 | | H | Me | H | H | 1Me-6-Ind | C | | 335 (M⁺+1) |
| N-e-7 | | N-a-14 | | H | Me | H | H | 5-1Idz | C | | 322 (M⁺+1) |
| N-e-8 | | N-a-16 | | H | Me | H | H | 1Me-5-Idz | C | | 336 (M⁺+1) |
| N-e-9 | | N-a-18 | | H | Me | H | H | 1Et-5-Idz | C | | 350 (M⁺+1) |
| N-e-10 | | N-a-20 | | H | Me | H | H | 2Me-5-Idz | C | | 336 (M⁺+1) |
| N-e-11 | | N-a-22 | | H | Me | H | H | 5-BF | C | | 322 (M⁺+1) |
| N-e-12 | | N-a-24 | | H | Me | H | H | 5-Bzt | C | | 339 (M⁺+1) |
| N-e-13 | | N-a-26 | | H | Me | H | H | 3-Qu | C | | 333 (M⁺+1) |
| N-e-14 | | N-a-28 | | H | Me | H | H | 6-Qu | C | | 333 (M⁺+1) |
| N-e-15 | | N-a-30 | | Me | Me | H | H | 6-IQ | C | | 347 (M⁺+1) |
| N-e-16 | | N-a-32 | | Me | Me | H | H | 2-Nap | C | | 346 (M⁺+1) |
| N-e-17 | | N-a-34 | | Me | Me | H | H | 5-Ind | C | | 335 (M⁺+1) |
| N-e-18 | | N-a-36 | | Me | Me | H | H | 1Me-5-Ind | C | | 349 (M⁺+1) |
| N-e-19 | | N-a-38 | | Me | Me | H | H | 5-1Idz | C | | 336 (M⁺+1) |
| N-e-20 | | N-a-40 | | Me | Me | H | H | 1Me-5-Idz | C | | 350 (M⁺+1) |
| N-e-21 | | N-a-42 | | Me | Me | H | H | 6-Qu | C | | 347 (M⁺+1) |
| N-e-22 | | N-a-44 | | Et | Me | H | H | 2-Nap | C | | 360 (M⁺+1) |
| N-e-23 | | N-a-46 | | Et | Me | H | H | 5-Ind | C | | 349 (M⁺+1) |
| N-e-24 | | N-a-48 | | Et | Me | H | H | 1Me-5-Ind | C | | 363 (M⁺+1) |
| N-e-25 | | N-a-50 | | Et | Me | H | H | 1Et-5-Ind | C | | 377 (M⁺+1) |
| N-e-26 | | N-a-52 | | Et | Me | H | H | 1Me-4-Ind | C | | 363 (M⁺+1) |
| N-e-27 | | N-a-54 | | Et | Me | H | H | 1Me-6-Ind | C | | 363 (M⁺+1) |
| N-e-28 | | N-a-56 | | Et | Me | H | H | 5-1Idz | C | | 350 (M⁺+1) |
| N-e-29 | | N-a-58 | | Et | Me | H | H | 1Me-5-Idz | C | | 364 (M⁺+1) |
| N-e-30 | | N-a-60 | | Et | Me | H | H | 3-Qu | C | | 361 (M⁺+1) |
| N-e-31 | | N-a-62 | | Et | Me | H | H | 6-IQ | C | | 361 (M⁺+1) |
| N-e-32 | | N-a-64 | | nPr | Me | H | H | 2-Nap | C | | 374 (M⁺+1) |
| N-e-33 | | N-a-66 | | nPr | Me | H | H | 5-Ind | C | | 363 (M⁺+1) |
| N-e-34 | | N-a-68 | | nPr | Me | H | H | 1Me-5-Ind | C | | 377 (M⁺+1) |
| N-e-35 | | N-a-70 | | nPr | Me | H | H | 5-1Idz | C | | 364 (M⁺+1) |
| N-e-36 | | N-a-72 | | nPr | Me | H | H | 1Me-5-Idz | C | | 378 (M⁺+1) |
| N-e-37 | | N-a-74 | | nPr | Me | H | H | 5-BF | C | | 364 (M⁺+1) |
| N-e-38 | | N-a-76 | | nPr | Me | H | H | 3-Qu | C | | 375 (M⁺+1) |
| N-e-39 | | N-a-78 | | iPr | Me | H | H | 2-Nap | C | | 374 (M⁺+1) |
| N-e-40 | | N-a-80 | | iPr | Me | H | H | 5-Ind | C | | 363 (M⁺+1) |
| N-e-41 | | N-a-82 | | iPr | Me | H | H | 1 Me-5-Ind | C | | 377 (M⁺+1) |
| N-e-42 | | N-a-84 | | iPr | Me | H | H | 1Et-5-Ind | C | | 391 (M⁺+1) |
| N-e-43 | | N-a-86 | | iPr | Me | H | H | 1Me-6-Ind | C | | 377 (M⁺+1) |
| N-e-44 | | N-a-88 | | iPr | Me | H | H | 5-1Idz | C | | 364 (M⁺+1) |

**[Table 157]**

| Table-N-E-2 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-e-45 | | N-a-90 | | iPr | Me | H | H | 1Me-5-Idz | C | | 378 (M⁺+1) |
| N-e-46 | | N-a-92 | | nBu | Me | H | H | 2-Nap | C | | 388 (M⁺+1) |
| N-e-47 | | N-a-94 | | nBu | Me | H | H | 5-Ind | C | | 377 (M⁺+1) |
| N-e-48 | | N-a-96 | | nBu | Me | H | H | 1Me-5-Ind | C | | 391 (M⁺+1) |
| N-e-49 | | N-a-98 | | nBu | Me | H | H | 1Me-6-Ind | C | | 391 (M⁺+1) |
| N-e-50 | | N-a-100 | | nBu | Me | H | H | 5-1Idz | C | | 378 (M⁺+1) |
| N-e-51 | | N-a-102 | | nBu | Me | H | H | 1Me-5-Idz | C | | 392 (M⁺+1) |
| N-e-52 | | N-a-104 | | nBu | Me | H | H | 3-Qu | C | | 389 (M⁺+1) |
| N-e-53 | | N-a-106 | | nBu | Me | H | H | 6-Qu | C | | 389 (M⁺+1) |
| N-e-54 | | N-a-108 | | iBu | Me | H | H | 2-Nap | C | | 388 (M⁺+1) |
| N-e-55 | | N-a-110 | | iBu | Me | H | H | 5-Ind | C | | 377 (M⁺+1) |
| N-e-56 | | N-a-112 | | iBu | Me | H | H | 1Me-5-Ind | C | | 391 (M⁺+1) |
| N-e-57 | | N-a-114 | | iBu | Me | H | H | 1Et-5-Ind | C | | 405 (M⁺+1) |
| N-e-58 | | N-a-116 | | iBu | Me | H | H | 1Me-6-Ind | C | | 391 (M⁺+1) |
| N-e-59 | | N-a-118 | | iBu | Me | H | H | 5-1Idz | C | | 378 (M⁺+1) |
| N-e-60 | | N-a-120 | | iBu | Me | H | H | 1Me-5-Idz | C | | 392 (M⁺+1) |
| N-e-61 | | N-a-122 | | iBu | Me | H | H | 1Et-5-Idz | C | | 406 (M⁺+1) |
| N-e-62 | | N-a-124 | | iBu | Me | H | H | 2-Nap | C | | 388 (M⁺+1) |
| N-e-63 | | N-a-126 | | cPen | Me | H | H | 5-Ind | C | | 389 (M⁺+1) |
| N-e-64 | | N-a-128 | | cPen | Me | H | H | 1Me-5-Ind | C | | 403 (M⁺+1) |
| N-e-65 | | N-a-130 | | cPen | Me | H | H | 1Et-5-Ind | C | | 417 (M⁺+1) |
| N-e-66 | | N-a-132 | | cPen | Me | H | H | 5-1Idz | C | | 390 (M⁺+1) |
| N-e-67 | | N-a-134 | | cPen | Me | H | H | 1Me-5-Idz | C | | 404 (M⁺+1) |
| N-e-68 | | N-a-136 | | cPen | Me | H | H | 1Et-5-Idz | C | | 418 (M⁺+1) |
| N-e-69 | | N-a-138 | | cHex | Me | H | H | 2-Nap | C | | 414 (M⁺+1) |
| N-e-70 | | N-a-140 | | cHex | Me | H | H | 5-Ind | C | | 403 (M⁺+1) |
| N-e-71 | | N-a-142 | | cHex | Me | H | H | 1Me-5-Ind | C | | 417 (M⁺+1) |
| N-e-72 | | N-a-144 | | cHex | Me | H | H | 1Me-4-Ind | C | | 417 (M⁺+1) |
| N-e-73 | | N-a-146 | | cHex | Me | H | H | 1Me-6-Ind | C | | 417 (M⁺+1) |
| N-e-74 | | N-a-148 | | cHex | Me | H | H | 5-1Idz | C | | 404 (M⁺+1) |
| N-e-75 | | N-a-150 | | cHex | Me | H | H | 1Me-5-Idz | C | | 418 (M⁺+1) |
| N-e-76 | | N-a-152 | | cHex | Me | H | H | 1Et-5-Idz | C | | 432 (M⁺+1) |
| N-e-77 | | N-a-154 | | 2MecHex | Me | H | H | 2-Nap | C | | 428 (M⁺+1) |
| N-e-78 | | N-a-156 | | 2MecHex | Me | H | H | 5-Ind | C | | 417 (M⁺+1) |
| N-e-79 | | N-a-158 | | 2MecHex | Me | H | H | 1Me-5-Ind | C | | 431 (M⁺+1) |
| N-e-80 | | N-a-160 | | 2MecHex | Me | H | H | 1Me-6-Ind | C | | 431 (M⁺+1) |
| N-e-81 | | N-a-162 | | 2MecHex | Me | H | H | 5-1Idz | C | | 418 (M⁺+1) |
| N-e-82 | | N-a-164 | | 2MecHex | Me | H | H | 1Me-5-Idz | C | | 432 (M⁺+1) |
| N-e-83 | | N-a-166 | | 2Indane | Me | H | H | 2-Nap | C | | 448 (M⁺+1) |
| N-e-84 | | N-a-168 | | 2Indane | Me | H | H | 5-Ind | C | | 437 (M⁺+1) |
| N-e-85 | | N-a-170 | | 2Indane | Me | H | H | 1Me-5-Ind | C | | 451 (M⁺+1) |
| N-e-86 | | N-a-172 | | 2Indane | Me | H | H | 5-1Idz | C | | 438 (M⁺+1) |
| N-e-87 | | N-a-174 | | 2Indane | Me | H | H | 1Me-5-Idz | C | | 452 (M⁺+1) |
| N-e-88 | | N-a-176 | | H | Et | H | H | 2-Nap | C | | 346 (M⁺+1) |
| N-e-89 | | N-a-178 | | H | Et | H | H | 5-Ind | C | | 335 (M⁺+1) |
| N-e-90 | | N-a-180 | | H | Et | H | H | 1Me-5-Ind | C | | 349 (M⁺+1) |

**[Table 158]**

| Table-N-E-3 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-e-91 | | N-a-182 | | H | Et | H | H | 1Et-5-Ind | C | | 363 (M⁺+1) |
| N-e-92 | | N-a-184 | | H | Et | H | H | 5-1Idz | C | | 336 (M⁺+1) |
| N-e-93 | | N-a-186 | | H | Et | H | H | 1Me-5-Idz | C | | 350 (M⁺+1) |
| N-e-94 | | N-a-188 | | H | Et | H | H | 1Et-5-Idz | C | | 364 (M⁺+1) |
| N-e-95 | | N-a-190 | | Et | Et | H | H | 2-Nap | C | | 374 (M⁺+1) |
| N-e-96 | | N-a-192 | | Et | Et | H | H | 5-Ind | C | | 363 (M⁺+1) |
| N-e-97 | | N-a-194 | | Et | Et | H | H | 1Me-5-Ind | C | | 377 (M⁺+1) |
| N-e-98 | | N-a-196 | | Et | Et | H | H | 5-1Idz | C | | 364 (M⁺+1) |
| N-e-99 | | N-a-198 | | Et | Et | H | H | 1Me-5-Idz | C | | 378 (M⁺+1) |
| N-e-100 | | N-a-200 | | Et | Et | H | H | 1Et-5-Idz | C | | 392 (M⁺+1) |
| N-e-101 | | N-a-202 | | nPr | Et | H | H | 2-Nap | C | | 388 (M⁺+1) |
| N-e-102 | | N-a-204 | | nPr | Et | H | H | 5-Ind | C | | 377 (M⁺+1) |
| N-e-103 | | N-a-206 | | nPr | Et | H | H | 1Me-5-Ind | C | | 391 (M⁺+1) |
| N-e-104 | | N-a-208 | | nPr | Et | H | H | 5-1Idz | C | | 378 (M⁺+1) |
| N-e-105 | | N-a-210 | | nPr | Et | H | H | 1Me-5-Idz | C | | 392 (M⁺+1) |
| N-e-106 | | N-a-212 | | iPr | Et | H | H | 2-Nap | C | | 388 (M⁺+1) |
| N-e-107 | | N-a-214 | | iPr | Et | H | H | 5-Ind | C | | 377 (M⁺+1) |
| N-e-108 | | N-a-216 | | iPr | Et | H | H | 1Et-5-Ind | C | | 405 (M⁺+1) |
| N-e-109 | | N-a-218 | | iPr | Et | H | H | 5-1Idz | C | | 378 (M⁺+1) |
| N-e-110 | | N-a-220 | | iPr | Et | H | H | 1Et-5-Idz | C | | 406 (M⁺+1) |
| N-e-111 | | N-a-222 | | nBu | Et | H | H | 2-Nap | C | | 402 (M⁺+1) |
| N-e-112 | | N-a-224 | | nBu | Et | H | H | 5-Ind | C | | 391 (M⁺+1) |
| N-e-113 | | N-a-226 | | nBu | Et | H | H | 5-1 Idz | C | | 392 (M⁺+1) |
| N-e-114 | | N-a-228 | | nBu | Et | H | H | 1Me-5-Idz | C | | 406 (M⁺+1) |
| N-e-115 | | N-a-230 | | iBu | Et | H | H | 2-Nap | C | | 402 (M⁺+1) |
| N-e-116 | | N-a-232 | | iBu | Et | H | H | 5-Ind | C | | 391 (M⁺+1) |
| N-e-117 | | N-a-234 | | iBu | Et | H | H | 1Me-5-Ind | C | | 405 (M⁺+1) |
| N-e-118 | | N-a-236 | | iBu | Et | H | H | 5-1Idz | C | | 392 (M⁺+1) |
| N-e-119 | | N-a-238 | | iBu | Et | H | H | 1Me-5-Idz | C | | 406 (M⁺+1) |
| N-e-120 | | N-a-240 | | iBu | Et | H | H | 1Et-5-Idz | C | | 420 (M⁺+1) |
| N-e-121 | | N-a-242 | | cPen | Et | H | H | 2-Nap | C | | 414 (M⁺+1) |
| N-e-122 | | N-a-244 | | cPen | Et | H | H | 5-Ind | C | | 403 (M⁺+1) |
| N-e-123 | | N-a-246 | | cPen | Et | H | H | 1Me-5-Ind | C | | 417 (M⁺+1) |
| N-e-124 | | N-a-248 | | cPen | Et | H | H | 1Et-5-Ind | C | | 431 (M⁺+1) |
| N-e-125 | | N-a-250 | | cPen | Et | H | H | 5-1Idz | C | | 404 (M⁺+1) |
| N-e-126 | | N-a-252 | | cPen | Et | H | H | 1Me-5-Idz | C | | 418 (M⁺+1) |
| N-e-127 | | N-a-254 | | cPen | Et | H | H | 1Et-5-Idz | C | | 432 (M⁺+1) |
| N-e-128 | | N-a-256 | | cHex | Et | H | H | 2-Nap | C | | 428 (M⁺+1) |
| N-e-129 | | N-a-258 | | cHex | Et | H | H | 5-Ind | C | | 417 (M⁺+1) |
| N-e-130 | | N-a-260 | | cHex | Et | H | H | 1Me-5-Ind | C | | 431 (M⁺+1) |
| N-e-131 | | N-a-262 | | cHex | Et | H | H | 5-1Idz | C | | 418 (M⁺+1) |
| N-e-132 | | N-a-264 | | cHex | Et | H | H | 1Me-5-Idz | C | | 432 (M⁺+1) |
| N-e-133 | | N-a-266 | | 2Indane | Et | H | H | 2-Nap | C | | 462 (M⁺+1) |
| N-e-134 | | N-a-268 | | 2Indane | Et | H | H | 5-Ind | C | | 451 (M⁺+1) |
| N-e-135 | | N-a-270 | | 2Indane | Et | H | H | 1Me-5-Ind | C | | 465 (M⁺+1) |
| N-e-136 | | N-a-272 | | 2Indane | Et | H | H | 5-1Idz | C | | 452 (M⁺+1) |
| N-e-137 | | N-a-274 | | 2Indane | Et | H | H | 1Me-5-Idz | C | | 466 (M⁺+1) |

### Example N-f-1

### Example Compound N-b-2 was subjected to chiral separation to obtain the title compound (Compound No. N-f-1, 14.4 mg).

Typical example compounds that can be obtained by reacting and treating corresponding starting compounds using any of the methods described in the present specification including the examples described above are shown in Table-N-F-1 to Table-N-F-5. As for the preparation of the compounds, all of the objective compounds were obtained by using HPLC separation. In the tables, used methods among the aforementioned synthetic methods are shown in the columns of "Syn" with symbols, the starting compounds are shown in the columns of "SM", and the reagents are shown in the columns of "Regent".

**[Table 159]**

| Table-N-F-1 | | | | | | | | | | | |
|---|---|---|---|---|---|---|---|---|---|---|---|
| 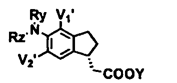 | | | | | | | | | | | |

| Exp. | Syn. | SM1 | Reagent | Ry | Rz | Y | V₁' | V₂' | LCMS | | |
|---|---|---|---|---|---|---|---|---|---|---|---|
| | | | | | | | | | method | RTime | Mass |
| N-f-1 | | N-b-2 | | Bn | H | H | H | 2-Nap | C | | 408 (M⁺+1) |
| N-f-2 | | N-b-4 | | Bn | H | H | H | 5-Ind | C | | 397 (M⁺+1) |
| N-f-3 | | N-b-6 | | Bn | H | H | H | 1Me-5-Ind | C | | 411 (M⁺+1) |
| N-f-4 | | N-b-8 | | Bn | H | H | H | 5-1Idz | C | | 398 (M⁺+1) |
| N-f-5 | | N-b-10 | | Bn | H | H | H | 1Me-5-Idz | C | | 412 (M⁺+1) |
| N-f-6 | | N-b-12 | | Bn | H | H | H | 1Et-5-Idz | C | | 426 (M⁺+1) |
| N-f-7 | | N-b-14 | | Bn | H | H | H | 5-BF | C | | 398 (M⁺+1) |
| N-f-8 | | N-b-16 | | Bn | H | H | H | 6-Qu | C | | 409 (M⁺+1) |
| N-f-9 | | N-b-18 | | Bn | Me | H | H | 2-Nap | C | | 422 (M⁺+1) |
| N-f-10 | | N-b-20 | | Bn | Me | H | H | 5-Ind | C | | 411 (M⁺+1) |
| N-f-11 | | N-b-22 | | Bn | Me | H | H | 1Me-5-Ind | C | | 425 (M⁺+1) |
| N-f-12 | | N-b-24 | | Bn | Me | H | H | 5-1Idz | C | | 412 (M⁺+1) |
| N-f-13 | | N-b-26 | | Bn | Me | H | H | 1Me-5-Idz | C | | 426 (M⁺+1) |
| N-f-14 | | N-b-28 | | Bn | Me | H | H | 1Et-5-Idz | C | | 440 (M⁺+1) |
| N-f-15 | | N-b-30 | | Bn | Me | H | H | 5-BF | C | | 412 (M⁺+1) |
| N-f-16 | | N-b-32 | | Bn | Et | H | H | 2-Nap | C | | 436 (M⁺+1) |
| N-f-17 | | N-b-34 | | Bn | Et | H | H | 5-Ind | C | | 425 (M⁺+1) |
| N-f-18 | | N-b-36 | | Bn | Et | H | H | 1Me-5-Ind | C | | 439 (M⁺+1) |
| N-f-19 | | N-b-38 | | Bn | Et | H | H | 5-1Idz | C | | 426 (M⁺+1) |
| N-f-20 | | N-b-40 | | Bn | Et | H | H | 1Me-5-Idz | C | | 440 (M⁺+1) |
| N-f-21 | | N-b-42 | | Bn | Et | H | H | 1Et-5-Idz | C | | 454 (M⁺+1) |
| N-f-22 | | N-b-44 | | 4FBn | Et | H | H | 6-Qu | C | | 455 (M⁺+1) |
| N-f-23 | | N-b-46 | | 4FBn | H | H | H | 2-Nap | C | | 426 (M⁺+1) |
| N-f-24 | | N-b-48 | | 4FBn | H | H | H | 5-Ind | C | | 415 (M⁺+1) |
| N-f-25 | | N-b-50 | | 4FBn | H | H | H | 1Me-5-Ind | C | | 429 (M⁺+1) |
| N-f-26 | | N-b-52 | | 4FBn | H | H | H | 5-1Idz | C | | 416 (M⁺+1) |
| N-f-27 | | N-b-54 | | 4FBn | H | H | H | 1Me-5-Idz | C | | 430 (M⁺+1) |
| N-f-28 | | N-b-56 | | 4FBn | Me | H | H | 2-Nap | C | | 440 (M⁺+1) |
| N-f-29 | | N-b-58 | | 4FBn | Me | H | H | 5-Ind | C | | 429 (M⁺+1) |
| N-f-30 | | N-b-60 | | 4FBn | Me | H | H | 1Me-5-Ind | C | | 443 (M⁺+1) |
| N-f-31 | | N-b-62 | | 4FBn | Me | H | H | 5-1Idz | C | | 430 (M⁺+1) |
| N-f-32 | | N-b-64 | | 4FBn | Me | H | H | 1Me-5-Idz | C | | 444 (M⁺+1) |
| N-f-33 | | N-b-66 | | 4FBn | Et | H | H | 2-Nap | C | | 454 (M⁺+1) |
| N-f-34 | | N-b-68 | | 4FBn | Et | H | H | 5-Ind | C | | 443 (M⁺+1) |
| N-f-35 | | N-b-70 | | 4FBn | Et | H | H | 1Me-5-Ind | C | | 457 (M⁺+1) |
| N-f-36 | | N-b-72 | | 4FBn | Et | H | H | 5-1Idz | C | | 444 (M⁺+1) |
| N-f-37 | | N-b-74 | | 4FBn | Et | H | H | 1Me-5-Idz | C | | 458 (M⁺+1) |
| N-f-38 | | N-b-76 | | 2FBn | H | H | H | 2-Nap | C | | 426 (M⁺+1) |
| N-f-39 | | N-b-78 | | 2FBn | H | H | H | 5-Ind | C | | 415 (M⁺+1) |
| N-f-40 | | N-b-80 | | 2FBn | H | H | H | 1Me-5-Ind | C | | 429 (M⁺+1) |
| N-f-41 | | N-b-82 | | 2FBn | H | H | H | 1Me-5-Idz | C | | 430 (M⁺+1) |
| N-f-42 | | N-b-84 | | 2FBn | H | H | H | 1Et-5-Idz | C | | 444 (M⁺+1) |
| N-f-43 | | N-b-86 | | 2FBn | H | H | H | 5-BF | C | | 416 (M⁺+1) |
| N-f-44 | | N-b-88 | | 2FBn | Me | H | H | 2-Nap | C | | 440 (M⁺+1) |

**[Table 160]**

| Table-N-F-2 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-f-45 | | N-b-90 | | 2FBn | Me | H | H | 5-Ind | C | | 429 (M⁺+1) |
| N-f-46 | | N-b-92 | | 2FBn | Me | H | H | 1Me-5-Ind | C | | 443 (M⁺+1) |
| N-f-47 | | N-b-94 | | 2FBn | Me | H | H | 1Me-5-Idz | C | | 444 (M⁺+1) |
| N-f-48 | | N-b-96 | | 2FBn | Me | H | H | 1Et-5-Idz | C | | 458 (M⁺+1) |
| N-f-49 | | N-b-98 | | 2FBn | Et | H | H | 2-Nap | C | | 454 (M⁺+1) |
| N-f-50 | | N-b-100 | | 2FBn | Et | H | H | 5-Ind | C | | 443 (M⁺+1) |
| N-f-51 | | N-b-102 | | 2FBn | Et | H | H | 1Me-5-Ind | C | | 457 (M⁺+1) |
| N-f-52 | | N-b-104 | | 2FBn | Et | H | H | 1Me-5-Idz | C | | 458 (M⁺+1) |
| N-f-53 | | N-b-106 | | 2FBn | Et | H | H | 1Et-5-Idz | C | | 472 (M⁺+1) |
| N-f-54 | | N-b-108 | | 3FBn | H | H | H | 2-Nap | C | | 426 (M⁺+1) |
| N-f-55 | | N-b-110 | | 3FBn | H | H | H | 1Me-5-Ind | C | | 429 (M⁺+1) |
| N-f-56 | | N-b-112 | | 3FBn | H | H | H | 5-1Idz | C | | 416 (M⁺+1) |
| N-f-57 | | N-b-114 | | 3FBn | H | H | H | 1Me-5-Idz | C | | 430 (M⁺+1) |
| N-f-58 | | N-b-116 | | 3FBn | Me | H | H | 2-Nap | C | | 440 (M⁺+1) |
| N-f-59 | | N-b-118 | | 3FBn | Me | H | H | 1Me-5-Ind | C | | 443 (M⁺+1) |
| N-f-60 | | N-b-120 | | 3FBn | Me | H | H | 5-1Idz | C | | 430 (M⁺+1) |
| N-f-61 | | N-b-122 | | 3FBn | Me | H | H | 1Me-5-Idz | C | | 444 (M⁺+1) |
| N-f-62 | | N-b-124 | | 3FBn | Et | H | H | 2-Nap | C | | 454 (M⁺+1) |
| N-f-63 | | N-b-126 | | 3FBn | Et | H | H | 1Me-5-Ind | C | | 457 (M⁺+1) |
| N-f-64 | | N-b-128 | | 3FBn | Et | H | H | 5-1Idz | C | | 444 (M⁺+1) |
| N-f-65 | | N-b-130 | | 3FBn | Et | H | H | 1Me-5-Idz | C | | 458 (M⁺+1) |
| N-f-66 | | N-b-132 | | 2ClBn | H | H | H | 2-Nap | C | | 442 (M⁺+1) |
| N-f-67 | | N-b-134 | | 2ClBn | H | H | H | 5-Ind | C | | 431 (M⁺+1) |
| N-f-68 | | N-b-136 | | 2ClBn | H | H | H | 1Me-5-Ind | C | | 445 (M⁺+1) |
| N-f-69 | | N-b-138 | | 2ClBn | H | H | H | 5-1Idz | C | | 432 (M⁺+1) |
| N-f-70 | | N-b-140 | | 2ClBn | H | H | H | 1Me-5-Idz | C | | 446 (M⁺+1) |
| N-f-71 | | N-b-142 | | 2ClBn | Me | H | H | 2-Nap | C | | 457 (M⁺+1) |
| N-f-72 | | N-b-144 | | 2ClBn | Me | H | H | 1Me-5-Ind | C | | 460 (M⁺+1) |
| N-f-73 | | N-b-146 | | 2ClBn | Me | H | H | 5-1Idz | C | | 446 (M⁺+1) |
| N-f-74 | | N-b-148 | | 2ClBn | Et | H | H | 2-Nap | C | | 471 (M⁺+1) |
| N-f-75 | | N-b-150 | | 2ClBn | Et | H | H | 1Me-5-Ind | C | | 474 (M⁺+1) |
| N-f-76 | | N-b-152 | | 2ClBn | Et | H | H | 1Me-5-Idz | C | | 475 (M⁺+1) |
| N-f-77 | | N-b-154 | | 4ClBn | H | H | H | 2-Nap | C | | 442 (M⁺+1) |
| N-f-78 | | N-b-156 | | 4ClBn | H | H | H | 5-Ind | C | | 431 (M⁺+1) |
| N-f-79 | | N-b-158 | | 4ClBn | H | H | H | 1Me-5-Ind | C | | 445 (M⁺+1) |
| N-f-80 | | N-b-160 | | 4ClBn | H | H | H | 5-1Idz | C | | 432 (M⁺+1) |
| N-f-81 | | N-b-162 | | 4ClBn | H | H | H | 1Me-5-Idz | C | | 446 (M⁺+1) |
| N-f-82 | | N-b-164 | | 4ClBn | Me | H | H | 2-Nap | C | | 457 (M⁺+1) |
| N-f-83 | | N-b-166 | | 4ClBn | Me | H | H | 5-Ind | C | | 445 (M⁺+1) |
| N-f-84 | | N-b-168 | | 4ClBn | Me | H | H | 1Me-5-Ind | C | | 460 (M⁺+1) |
| N-f-85 | | N-b-170 | | 4ClBn | Me | H | H | 5-1Idz | C | | 446 (M⁺+1) |
| N-f-86 | | N-b-172 | | 4ClBn | Me | H | H | 1Me-5-Idz | C | | 460 (M⁺+1) |
| N-f-87 | | N-b-174 | | 4ClBn | Et | H | H | 2-Nap | C | | 471 (M⁺+1) |
| N-f-88 | | N-b-176 | | 4ClBn | Et | H | H | 5-Ind | C | | 460 (M⁺+1) |
| N-f-89 | | N-b-178 | | 4ClBn | Et | H | H | 1Me-5-Ind | C | | 474 (M⁺+1) |
| N-f-90 | | N-b-180 | | 4ClBn | Et | H | H | 5-1Idz | C | | 460 (M⁺+1) |

**[Table 161]**

| Table-N-F-3 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-f-91 | | N-b-182 | | 4ClBn | Et | H | H | 1 Me-5-Idz | C | | 475 (M⁺+1) |
| N-f-92 | | N-b-184 | | 2BrBn | Me | H | H | 2-Nap | C | | 500 (M⁺) |
| N-f-93 | | N-b-186 | | 2BrBn | Me | H | H | 5-Ind | C | | 489 (M⁺) |
| N-f-94 | | N-b-188 | | 2BrBn | Me | H | H | 1Me-5-Ind | C | | 503 (M⁺) |
| N-f-95 | | N-b-190 | | 2BrBn | Me | H | H | 1Et-5-Ind | C | | 517 (M⁺) |
| N-f-96 | | N-b-192 | | 2BrBn | Me | H | H | 5-1Idz | C | | 490 (M⁺) |
| N-f-97 | | N-b-194 | | 2BrBn | Me | H | H | 1Me-5-Idz | C | | 504 (M⁺) |
| N-f-98 | | N-b-196 | | 2BrBn | Me | H | H | 1Et-5-Idz | C | | 518 (M⁺) |
| N-f-99 | | N-b-198 | | 2CF3Bn | Me | H | H | 2-Nap | C | | 490 (M⁺+1) |
| N-f-100 | | N-b-200 | | 2CF3Bn | Me | H | H | 5-Ind | C | | 479 (M⁺+1) |
| N-f-101 | | N-b-202 | | 2CF3Bn | Me | H | H | 1Me-5-Ind | C | | 493 (M⁺+1) |
| N-f-102 | | N-b-204 | | 2CF3Bn | Me | H | H | 5-1Idz | C | | 480 (M⁺+1) |
| N-f-103 | | N-b-206 | | 2CF3Bn | Me | H | H | 1Me-5-Idz | C | | 494 (M⁺+1) |
| N-f-104 | | N-b-208 | | 2,3DFBn | H | H | H | 2-Nap | C | | 444 (M⁺+1) |
| N-f-105 | | N-b-210 | | 2,3DFBn | H | H | H | 5-Ind | C | | 433 (M⁺+1) |
| N-f-106 | | N-b-212 | | 2,3DFBn | H | H | H | 1Me-5-Ind | C | | 447 (M⁺+1) |
| N-f-107 | | N-b-214 | | 2,3DFBn | H | H | H | 1Me-5-Idz | C | | 448 (M⁺+1) |
| N-f-108 | | N-b-216 | | 2,3DFBn | Me | H | H | 2-Nap | C | | 458 (M⁺+1) |
| N-f-109 | | N-b-218 | | 2,3DFBn | Me | H | H | 5-Ind | C | | 447 (M⁺+1) |
| N-f-110 | | N-b-220 | | 2,3DFBn | Me | H | H | 1Me-5-Ind | C | | 461 (M⁺+1) |
| N-f-111 | | N-b-222 | | 2,3DFBn | Me | H | H | 1Me-5-Idz | C | | 462 (M⁺+1) |
| N-f-112 | | N-b-224 | | 4MeBn | H | H | H | 2-Nap | C | | 422 (M⁺+1) |
| N-f-113 | | N-b-226 | | 4MeBn | H | H | H | 5-Ind | C | | 411 (M⁺+1) |
| N-f-114 | | N-b-228 | | 4MeBn | H | H | H | 1Me-5-Ind | C | | 425 (M⁺+1) |
| N-f-115 | | N-b-230 | | 4MeBn | H | H | H | 1Me-5-Idz | C | | 426 (M⁺+1) |
| N-f-116 | | N-b-232 | | 4MeBn | H | H | H | 1Et-5-Idz | C | | 440 (M⁺+1) |
| N-f-117 | | N-b-234 | | 4MeBn | Me | H | H | 2-Nap | C | | 436 (M⁺+1) |
| N-f-118 | | N-b-236 | | 4MeBn | Me | H | H | 5-Ind | C | | 425 (M⁺+1) |
| N-f-119 | | N-b-238 | | 4MeBn | Me | H | H | 1Me-5-Ind | C | | 439 (M⁺+1) |
| N-f-120 | | N-b-240 | | 4MeBn | Me | H | H | 1Me-5-Idz | C | | 440 (M⁺+1) |
| N-f-121 | | N-b-242 | | 4MeBn | Me | H | H | 1Et-5-Idz | C | | 454 (M⁺+1) |
| N-f-122 | | N-b-244 | | 2PhBn | Me | H | H | 2-Nap | C | | 498 (M⁺+1) |
| N-f-123 | | N-b-246 | | 2PhBn | Me | H | H | 5-Ind | C | | 487 (M⁺+1) |
| N-f-124 | | N-b-248 | | 2PhBn | Me | H | H | 1Me-5-Ind | C | | 501 (M⁺+1) |
| N-f-125 | | N-b-250 | | 2PhBn | Me | H | H | 5-1Idz | C | | 488 (M⁺+1) |
| N-f-126 | | N-b-252 | | 2PhBn | Me | H | H | 1Me-5-Idz | C | | 502 (M⁺+1) |
| N-f-127 | | N-b-254 | | 2PhBn | Me | H | H | 1Et-5-Idz | C | | 516 (M⁺+1) |
| N-f-128 | | N-b-256 | | 2PhBn | Et | H | H | 2-Nap | C | | 512 (M⁺+1) |
| N-f-129 | | N-b-258 | | 2PhBn | Et | H | H | 1Me-5-Ind | C | | 515 (M⁺+1) |
| N-f-130 | | N-b-260 | | 2PhBn | Et | H | H | 1Me-5-Idz | C | | 516 (M⁺+1) |
| N-f-131 | | N-b-262 | | 40MeBn | Me | H | H | 2-Nap | C | | 452 (M⁺+1) |
| N-f-132 | | N-b-264 | | 40MeBn | Me | H | H | 5-Ind | C | | 441 (M⁺+1) |
| N-f-133 | | N-b-266 | | 40MeBn | Me | H | H | 1Me-5-Ind | C | | 455 (M⁺+1) |
| N-f-134 | | N-b-268 | | 40MeBn | Me | H | H | 1Et-5-Ind | C | | 469 (M⁺+1) |
| N-f-135 | | N-b-270 | | 40MeBn | Me | H | H | 5-1Idz | C | | 442 (M⁺+1) |
| N-f-136 | | N-b-272 | | 40MeBn | Et | H | H | 2-Nap | C | | 466 (M⁺+1) |

**[Table 162]**

| Table-N-F-4 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-f-137 | | N-b-274 | | 40MeBn | Et | H | H | 1Me-5-Idz | C | | 470 (M⁺+1) |
| N-f-138 | | N-b-276 | | 20MeBn | H | H | H | 2-Nap | C | | 438 (M⁺+1) |
| N-f-139 | | N-b-278 | | 20MeBn | H | H | H | 5-Ind | C | | 427 (M⁺+1) |
| N-f-140 | | N-b-280 | | 20MeBn | H | H | H | 1Me-5-Ind | C | | 441 (M⁺+1) |
| N-f-141 | | N-b-282 | | 20MeBn | H | H | H | 5-1Idz | C | | 428 (M⁺+1) |
| N-f-142 | | N-b-284 | | 20MeBn | H | H | H | 1Me-5-Idz | C | | 442 (M⁺+1) |
| N-f-143 | | N-b-286 | | 20MeBn | Me | H | H | 2-Nap | C | | 452 (M⁺+1) |
| N-f-144 | | N-b-288 | | 20MeBn | Me | H | H | 1Me-5-Idz | C | | 456 (M⁺+1) |
| N-f-145 | | N-b-290 | | 4DMABn | H | H | H | 2-Nap | C | | 451 (M⁺+1) |
| N-f-146 | | N-b-292 | | 4DMABn | H | H | H | 5-Ind | C | | 440 (M⁺+1) |
| N-f-147 | | N-b-294 | | 4DMABn | H | H | H | 1Me-5-Ind | C | | 454 (M⁺+1) |
| N-f-148 | | N-b-296 | | 4DMABn | H | H | H | 5-1Idz | C | | 441 (M⁺+1) |
| N-f-149 | | N-b-298 | | 4DMABn | H | H | H | 1Me-5-Idz | C | | 455 (M⁺+1) |
| N-f-150 | | N-b-300 | | 4DMABn | Me | H | H | 2-Nap | C | | 465 (M⁺+1) |
| N-f-151 | | N-b-302 | | 4DMABn | Me | H | H | 1Me-5-Ind | C | | 468 (M⁺+1) |
| N-f-152 | | N-b-304 | | 4DMABn | Me | H | H | 5-1Idz | C | | 455 (M⁺+1) |
| N-f-153 | | N-b-306 | | 4DMABn | Me | H | H | 1Me-5-Idz | C | | 469 (M⁺+1) |
| N-f-154 | | N-b-308 | | 4SMeBn | Me | H | H | 2-Nap | C | | 468 (M⁺+1) |
| N-f-155 | | N-b-310 | | 4SMeBn | Me | H | H | 5-Ind | C | | 457 (M⁺+1) |
| N-f-156 | | N-b-312 | | 4SMeBn | Me | H | H | 1Me-5-Ind | C | | 471 (M⁺+1) |
| N-f-157 | | N-b-314 | | 4SMeBn | Me | H | H | 1Et-5-Ind | C | | 485 (M⁺+1) |
| N-f-158 | | N-b-316 | | 4SMeBn | Me | H | H | 5-1Idz | C | | 458 (M⁺+1) |
| N-f-159 | | N-b-318 | | 1NapMe | Me | H | H | 2-Nap | C | | 472 (M⁺+1) |
| N-f-160 | | N-b-320 | | 1NapMe | Me | H | H | 1Me-5-Ind | C | | 475 (M⁺+1) |
| N-f-161 | | N-b-322 | | 1NapMe | Me | H | H | 1Me-5-Idz | C | | 476 (M⁺+1) |
| N-f-162 | | N-b-324 | | 2NapMe | Me | H | H | 2-Nap | C | | 472 (M⁺+1) |
| N-f-163 | | N-b-326 | | 2NapMe | Me | H | H | 5-Ind | C | | 461 (M⁺+1) |
| N-f-164 | | N-b-328 | | 2NapMe | Me | H | H | 1Me-5-Ind | C | | 475 (M⁺+1) |
| N-f-165 | | N-b-330 | | 2NapMe | Me | H | H | 1Et-5-Ind | C | | 489 (M⁺+1) |
| N-f-166 | | N-b-332 | | 2NapMe | Me | H | H | 5-1Idz | C | | 462 (M⁺+1) |
| N-f-167 | | N-b-334 | | 2NapMe | Me | H | H | 1Me-5-Idz | C | | 476 (M⁺+1) |
| N-f-168 | | N-b-336 | | 2FRMe | Me | H | H | 2-Nap | C | | 412 (M⁺+1) |
| N-f-169 | | N-b-338 | | 2FRMe | Me | H | H | 1Me-5-Ind | C | | 415 (M⁺+1) |
| N-f-170 | | N-b-340 | | 2FRMe | Me | H | H | 1Et-5-Ind | C | | 429 (M⁺+1) |
| N-f-171 | | N-b-342 | | 2FRMe | Me | H | H | 5-1Idz | C | | 402 (M⁺+1) |
| N-f-172 | | N-b-344 | | 2FRMe | Me | H | H | 1Me-5-Idz | C | | 416 (M⁺+1) |
| N-f-173 | | N-b-346 | | 2FRMe | Et | H | H | 2-Nap | C | | 426 (M⁺+1) |
| N-f-174 | | N-b-348 | | 2FRMe | Et | H | H | 1Me-5-Ind | C | | 429 (M⁺+1) |
| N-f-175 | | N-b-350 | | 2FRMe | Et | H | H | 1Et-5-Ind | C | | 443 (M⁺+1) |
| N-f-176 | | N-b-352 | | 2FRMe | Et | H | H | 5-1Idz | C | | 416 (M⁺+1) |
| N-f-177 | | N-b-354 | | 2FRMe | Et | H | H | 1Me-5-Idz | C | | 430 (M⁺+1) |
| N-f-178 | | N-b-356 | | 3FRMe | Me | H | H | 2-Nap | C | | 412 (M⁺+1) |
| N-f-179 | | N-b-358 | | 3FRMe | Me | H | H | 1Me-5-Ind | C | | 415 (M⁺+1) |
| N-f-180 | | N-b-360 | | 3FRMe | Me | H | H | 5-1Idz | C | | 402 (M⁺+1) |
| N-f-181 | | N-b-362 | | 3FRMe | Me | H | H | 1Me-5-Idz | C | | 416 (M⁺+1) |
| N-f-182 | | N-b-364 | | 3FRMe | Et | H | H | 2-Nap | C | | 426 (M⁺+1) |

**[Table 163]**

| Table-N-F-5 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-f-183 | | N-b-366 | | 3FRMe | Et | H | H | 1Me-5-Ind | C | | 429 (M⁺+1) |
| N-f-184 | | N-b-368 | | 3FRMe | Et | H | H | 5-1Idz | C | | 416 (M⁺+1) |
| N-f-185 | | N-b-370 | | 3FRMe | Et | H | H | 1Me-5-Idz | C | | 430 (M⁺+1) |
| N-f-186 | | N-b-372 | | 3FRMe | Et | H | H | 1Et-5-Idz | C | | 444 (M⁺+1) |
| N-f-187 | | N-b-374 | | 2TPMe | Me | H | H | 2-Nap | C | | 428 (M⁺+1) |
| N-f-188 | | N-b-376 | | 2TPMe | Me | H | H | 1Me-5-Ind | C | | 431 (M⁺+1) |
| N-f-189 | | N-b-378 | | 2TPMe | Me | H | H | 1Et-5-Ind | C | | 445 (M⁺+1) |
| N-f-190 | | N-b-380 | | 2TPMe | Me | H | H | 5-1Idz | C | | 418 (M⁺+1) |
| N-f-191 | | N-b-382 | | 2TPMe | Me | H | H | 1Me-5-Idz | C | | 432 (M⁺+1) |
| N-f-192 | | N-b-384 | | 2TPMe | Et | H | H | 2-Nap | C | | 442 (M⁺+1) |
| N-f-193 | | N-b-386 | | 2TPMe | Et | H | H | 5-Ind | C | | 431 (M⁺+1) |
| N-f-194 | | N-b-388 | | 2TPMe | Et | H | H | 1Me-5-Ind | C | | 445 (M⁺+1) |
| N-f-195 | | N-b-390 | | 2TPMe | Et | H | H | 1Et-5-Ind | C | | 459 (M⁺+1) |
| N-f-196 | | N-b-392 | | 2TPMe | Et | H | H | 5-1Idz | C | | 432 (M⁺+1) |
| N-f-197 | | N-b-394 | | 2TPMe | Et | H | H | 1Me-5-Idz | C | | 446 (M⁺+1) |

### Example N-g-1

### Example Compound N-b-2 was subjected to chiral separation to obtain the title compound (Compound No. N-g-1, 10.1 mg).

Typical example compounds that can be obtained by reacting and treating corresponding starting compounds using any of the methods described in the present specification including the examples described above are shown in Table-N-G-1 to Table-N-G-5. As for the preparation of the compounds, all of the objective compounds were obtained by using HPLC separation. In the tables, used methods among the aforementioned synthetic methods are shown in the columns of "Syn" with symbols, the starting compounds are shown in the columns of "SM", and the reagents are shown in the columns of "Regent".

**[Table 164]**

| Table-N-G-1 | | | | | | | | | | | |
|---|---|---|---|---|---|---|---|---|---|---|---|
| 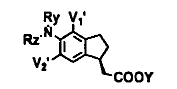 | | | | | | | | | | | |

| Exp. | Syn. | SM1 | Reagent | Ry | Rz | Y | V₁' | V₂' | LCMS | | |
|---|---|---|---|---|---|---|---|---|---|---|---|
| | | | | | | | | | method | RTime | Mass |
| N-g-1 | | N-b-2 | | Bn | H | H | H | 2-Nap | C | | 408 (M⁺+1) |
| N-g-2 | | N-b-4 | | Bn | H | H | H | 5-Ind | C | | 397 (M⁺+1) |
| N-g-3 | | N-b-6 | | Bn | H | H | H | 1Me-5-Ind | C | | 411 (M⁺+1) |
| N-g-4 | | N-b-8 | | Bn | H | H | H | 5-1Idz | C | | 398 (M⁺+1) |
| N-g-5 | | N-b-10 | | Bn | H | H | H | 1Me-5-Idz | C | | 412 (M⁺+1) |
| N-g-6 | | N-b-12 | | Bn | H | H | H | 1Et-5-Idz | C | | 426 (M⁺+1) |
| N-g-7 | | N-b-14 | | Bn | H | H | H | 5-BF | C | | 398 (M⁺+1) |
| N-g-8 | | N-b-16 | | Bn | H | H | H | 6-Qu | C | | 409 (M⁺+1) |
| N-g-9 | | N-b-18 | | Bn | Me | H | H | 2-Nap | C | | 422 (M⁺+1) |
| N-g-10 | | N-b-20 | | Bn | Me | H | H | 5-Ind | C | | 411 (M⁺+1) |
| N-g-11 | | N-b-22 | | Bn | Me | H | H | 1Me-5-Ind | C | | 425 (M⁺+1) |
| N-g-12 | | N-b-24 | | Bn | Me | H | H | 5-1Idz | C | | 412 (M⁺+1) |
| N-g-13 | | N-b-26 | | Bn | Me | H | H | 1Me-5-Idz | C | | 426 (M⁺+1) |
| N-g-14 | | N-b-28 | | Bn | Me | H | H | 1Et-5-Idz | C | | 440 (M⁺+1) |
| N-g-15 | | N-b-30 | | Bn | Me | H | H | 5-BF | C | | 412 (M⁺+1) |
| N-g-16 | | N-b-32 | | Bn | Et | H | H | 2-Nap | C | | 436 (M⁺+1) |
| N-g-17 | | N-b-34 | | Bn | Et | H | H | 5-Ind | C | | 425 (M⁺+1) |
| N-g-18 | | N-b-36 | | Bn | Et | H | H | 1Me-5-Ind | C | | 439 (M⁺+1) |
| N-g-19 | | N-b-38 | | Bn | Et | H | H | 5-1Idz | C | | 426 (M⁺+1) |
| N-g-20 | | N-b-40 | | Bn | Et | H | H | 1Me-5-ldz | C | | 440 (M⁺+1) |
| N-g-21 | | N-b-42 | | Bn | Et | H | H | 1Et-5-Idz | C | | 454 (M⁺+1) |
| N-g-22 | | N-b-44 | | 4FBn | Et | H | H | 6-Qu | C | | 455 (M⁺+1) |
| N-g-23 | | N-b-46 | | 4FBn | H | H | H | 2-Nap | C | | 426 (M⁺+1) |
| N-g-24 | | N-b-48 | | 4FBn | H | H | H | 5-Ind | C | | 415 (M⁺+1) |
| N-g-25 | | N-b-50 | | 4FBn | H | H | H | 1Me-5-Ind | C | | 429 (M⁺+1) |
| N-g-26 | | N-b-52 | | 4FBn | H | H | H | 5-1Idz | C | | 416 (M⁺+1) |
| N-g-27 | | N-b-54 | | 4FBn | H | H | H | 1Me-5-Idz | C | | 430 (M⁺+1) |
| N-g-28 | | N-b-56 | | 4FBn | Me | H | H | 2-Nap | C | | 440 (M⁺+1) |
| N-g-29 | | N-b-58 | | 4FBn | Me | H | H | 5-Ind | C | | 429 (M⁺+1) |
| N-g-30 | | N-b-60 | | 4FBn | Me | H | H | 1Me-5-Ind | C | | 443 (M⁺+1) |
| N-g-31 | | N-b-62 | | 4FBn | Me | H | H | 5-1Idz | C | | 430 (M⁺+1) |
| N-g-32 | | N-b-64 | | 4FBn | Me | H | H | 1Me-5-Idz | C | | 444 (M⁺+1) |
| N-g-33 | | N-b-66 | | 4FBn | Et | H | H | 2-Nap | C | | 454 (M⁺+1) |
| N-g-34 | | N-b-68 | | 4FBn | Et | H | H | 5-Ind | C | | 443 (M⁺+1) |
| N-g-35 | | N-b-70 | | 4FBn | Et | H | H | 1Me-5-Ind | C | | 457 (M⁺+1) |
| N-g-36 | | N-b-72 | | 4FBn | Et | H | H | 5-1Idz | C | | 444 (M⁺+1) |
| N-g-37 | | N-b-74 | | 4FBn | Et | H | H | 1Me-5-Idz | C | | 458 (M⁺+1) |
| N-g-38 | | N-b-76 | | 2FBn | H | H | H | 2-Nap | C | | 426 (M⁺+1) |
| N-g-39 | | N-b-78 | | 2FBn | H | H | H | 5-Ind | C | | 415 (M⁺+1) |
| N-g-40 | | N-b-80 | | 2FBn | H | H | H | 1Me-5-Ind | C | | 429 (M⁺+1) |
| N-g-41 | | N-b-82 | | 2FBn | H | H | H | 1Me-5-Idz | C | | 430 (M⁺+1) |
| N-g-42 | | N-b-84 | | 2FBn | H | H | H | 1Et-5-Idz | C | | 444 (M⁺+1) |
| N-g-43 | | N-b-86 | | 2FBn | H | H | H | 5-BF | C | | 416 (M⁺+1) |
| N-g-44 | | N-b-88 | | 2FBn | Me | H | H | 2-Nap | C | | 440 (M⁺+1) |

**[Table 165]**

| Table-N-G-2 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-g-45 | | N-b-90 | | 2FBn | Me | H | H | 5-Ind | C | | 429 (M⁺+1) |
| N-g-46 | | N-b-92 | | 2FBn | Me | H | H | 1Me-5-Ind | C | | 443 (M⁺+1) |
| N-g-47 | | N-b-94 | | 2FBn | Me | H | H | 1Me-5-Idz | C | | 444 (M⁺+1) |
| N-g-48 | | N-b-96 | | 2FBn | Me | H | H | 1Et-5-Idz | C | | 458 (M⁺+1) |
| N-g-49 | | N-b-98 | | 2FBn | Et | H | H | 2-Nap | C | | 454 (M⁺+1) |
| N-g-50 | | N-b-100 | | 2FBn | Et | H | H | 5-1Ind | C | | 443 (M⁺+1) |
| N-g-51 | | N-b-102 | | 2FBn | Et | H | H | 1Me-5-Ind | C | | 457 (M⁺+1) |
| N-g-52 | | N-b-104 | | 2FBn | Et | H | H | 1Me-5-Idz | C | | 458 (M⁺+1) |
| N-g-53 | | N-b-106 | | 2FBn | Et | H | H | 1Et-5-Idz | C | | 472 (M⁺+1) |
| N-g-54 | | N-b-108 | | 3FBn | H | H | H | 2-Nap | C | | 426 (M⁺+1) |
| N-g-55 | | N-b-110 | | 3FBn | H | H | H | 1Me-5-Ind | C | | 429 (M⁺+1) |
| N-g-56 | | N-b-112 | | 3FBn | H | H | H | 5-Idz | C | | 416 (M⁺+1) |
| N-g-57 | | N-b-114 | | 3FBn | H | H | H | 1Me-5-Idz | C | | 430 (M⁺+1) |
| N-g-58 | | N-b-116 | | 3FBn | Me | H | H | 2-Nap | C | | 440 (M⁺+1) |
| N-g-59 | | N-b-118 | | 3FBn | Me | H | H | 1Me-5-Ind | C | | 443 (M⁺+1) |
| N-g-60 | | N-b-120 | | 3FBn | Me | H | H | 5-Idz | C | | 430 (M⁺+1) |
| N-g-61 | | N-b-122 | | 3FBn | Me | H | H | 1Me-5-Idz | C | | 444 (M⁺+1) |
| N-g-62 | | N-b-124 | | 3FBn | Et | H | H | 2-Nap | C | | 454 (M⁺+1) |
| N-g-63 | | N-b-126 | | 3FBn | Et | H | H | 1Me-5-Ind | C | | 457 (M⁺+1) |
| N-g-64 | | N-b-128 | | 3FBn | Et | H | H | 5-Idz | C | | 444 (M⁺+1) |
| N-g-65 | | N-b-130 | | 3FBn | Et | H | H | 1Me-5-Idz | C | | 458 (M⁺+1) |
| N-g-66 | | N-b-132 | | 2ClBn | H | H | H | 2-Nap | C | | 442 (M⁺+1) |
| N-g-67 | | N-b-134 | | 2ClBn | H | H | H | 5-1Ind | C | | 431 (M⁺+1) |
| N-g-68 | | N-b-136 | | 2ClBn | H | H | H | 1Me-5-Ind | C | | 445 (M⁺+1) |
| N-g-69 | | N-b-138 | | 2ClBn | H | H | H | 5-Idz | C | | 432 (M⁺+1) |
| N-g-70 | | N-b-140 | | 2ClBn | H | H | H | 1Me-5-Idz | C | | 446 (M⁺+1) |
| N-g-71 | | N-b-142 | | 2ClBn | Me | H | H | 2-Nap | C | | 457 (M⁺+1) |
| N-g-72 | | N-b-144 | | 2ClBn | Me | H | H | 1Me-5-Ind | C | | 460 (M⁺+1) |
| N-g-73 | | N-b-146 | | 2ClBn | Me | H | H | 5-Idz | C | | 446 (M⁺+1) |
| N-g-74 | | N-b-148 | | 2ClBn | Et | H | H | 2-Nap | C | | 471 (M⁺+1) |
| N-g-75 | | N-b-150 | | 2ClBn | Et | H | H | 1Me-5-Ind | C | | 474 (M⁺+1) |
| N-g-76 | | N-b-152 | | 2ClBn | Et | H | H | 1Me-5-Idz | C | | 475 (M⁺+1) |
| N-g-77 | | N-b-154 | | 4ClBn | H | H | H | 2-Nap | C | | 442 (M⁺+1) |
| N-g-78 | | N-b-156 | | 4ClBn | H | H | H | 5-1Ind | C | | 431 (M⁺+1) |
| N-g-79 | | N-b-158 | | 4ClBn | H | H | H | 1Me-5-Ind | C | | 445 (M⁺+1) |
| N-g-80 | | N-b-160 | | 4ClBn | H | H | H | 5-Idz | C | | 432 (M⁺+1) |
| N-g-81 | | N-b-162 | | 4ClBn | H | H | H | 1Me-5-Idz | C | | 446 (M⁺+1) |
| N-g-82 | | N-b-164 | | 4ClBn | Me | H | H | 2-Nap | C | | 457 (M⁺+1) |
| N-g-83 | | N-b-166 | | 4ClBn | Me | H | H | 5-1Ind | C | | 445 (M⁺+1) |
| N-g-84 | | N-b-168 | | 4ClBn | Me | H | H | 1Me-5-Ind | C | | 460 (M⁺+1) |
| N-g-85 | | N-b-170 | | 4ClBn | Me | H | H | 5-Idz | C | | 446 (M⁺+1) |
| N-g-86 | | N-b-172 | | 4ClBn | Me | H | H | 1Me-5-Idz | C | | 460 (M⁺+1) |
| N-g-87 | | N-b-174 | | 4ClBn | Et | H | H | 2-Nap | C | | 471 (M⁺+1) |
| N-g-88 | | N-b-176 | | 4ClBn | Et | H | H | 5-1Ind | C | | 460 (M⁺+1) |
| N-g-89 | | N-b-178 | | 4ClBn | Et | H | H | 1Me-5-Ind | C | | 474 (M⁺+1) |
| N-g-90 | | N-b-180 | | 4ClBn | Et | H | H | 5-Idz | C | | 460 (M⁺+1) |

**[Table 166]**

| Table-N-G-3 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-g-91 | | N-f-182 | | 4ClBn | Et | H | H | 1Me-5-Idz | C | | 475 (M⁺+1) |
| N-g-92 | | N-f-184 | | 2BrBn | Me | H | H | 2-Nap | C | | 500 (M⁺) |
| N-g-93 | | N-f-186 | | 2BrBn | Me | H | H | 5-Ind | C | | 489 (M⁺) |
| N-g-94 | | N-f-188 | | 2BrBn | Me | H | H | 1Me-5-Ind | C | | 503 (M⁺) |
| N-g-95 | | N-f-190 | | 2BrBn | Me | H | H | 1Et-5-Ind | C | | 517 (M⁺) |
| N-g-96 | | N-f-192 | | 2BrBn | Me | H | H | 5-1Idz | C | | 490 (M⁺) |
| N-g-97 | | N-f-194 | | 2BrBn | Me | H | H | 1Me-5-Idz | C | | 504 (M⁺) |
| N-g-98 | | N-f-196 | | 2BrBn | Me | H | H | 1Et-5-Idz | C | | 518 (M⁺) |
| N-g-99 | | N-f-198 | | 2CF3Bn | Me | H | H | 2-Nap | C | | 490 (M⁺+1) |
| N-g-100 | | N-f-200 | | 2CF3Bn | Me | H | H | 5-Ind | C | | 479 (M⁺+1) |
| N-g-101 | | N-f-202 | | 2CF3Bn | Me | H | H | 1Me-5-Ind | C | | 493 (M⁺+1) |
| N-g-102 | | N-f-204 | | 2CF3Bn | Me | H | H | 5-1Idz | C | | 480 (M⁺+1) |
| N-g-103 | | N-f-206 | | 2CF3Bn | Me | H | H | 1Me-5-Idz | C | | 494 (M⁺+1) |
| N-g-104 | | N-f-208 | | 2,3DFBn | H | H | H | 2-Nap | C | | 444 (M⁺+1) |
| N-g-105 | | N-f-210 | | 2,3DFBn | H | H | H | 5-Ind | C | | 433 (M⁺+1) |
| N-g-106 | | N-f-212 | | 2,3DFBn | H | H | H | 1Me-5-Ind | C | | 447 (M⁺+1) |
| N-g-107 | | N-f-214 | | 2,3DFBn | H | H | H | 1Me-5-Idz | C | | 448 (M⁺+1) |
| N-g-108 | | N-f-216 | | 2,3DFBn | Me | H | H | 2-Nap | C | | 458 (M⁺+1) |
| N-g-109 | | N-f-218 | | 2,3DFBn | Me | H | H | 5-Ind | C | | 447 (M⁺+1) |
| N-g-110 | | N-f-220 | | 2,3DFBn | Me | H | H | 1Me-5-Ind | C | | 461 (M⁺+1) |
| N-g-111 | | N-f-222 | | 2,3DFBn | Me | H | H | 1Me-5-Idz | C | | 462 (M⁺+1) |
| N-g-112 | | N-f-224 | | 4MeBn | H | H | H | 2-Nap | C | | 422 (M⁺+1) |
| N-g-113 | | N-f-226 | | 4MeBn | H | H | H | 5-Ind | C | | 411 (M⁺+1) |
| N-g-114 | | N-f-228 | | 4MeBn | H | H | H | 1Me-5-Ind | C | | 425 (M⁺+1) |
| N-g-115 | | N-f-230 | | 4MeBn | H | H | H | 1Me-5-Idz | C | | 426 (M⁺+1) |
| N-g-116 | | N-f-232 | | 4MeBn | H | H | H | 1Et-5-Idz | C | | 440 (M⁺+1) |
| N-g-117 | | N-f-234 | | 4MeBn | Me | H | H | 2-Nap | C | | 436 (M⁺+1) |
| N-g-118 | | N-f-236 | | 4MeBn | Me | H | H | 5-Ind | C | | 425 (M⁺+1) |
| N-g-119 | | N-f-238 | | 4MeBn | Me | H | H | 1Me-5-Ind | C | | 439 (M⁺+1) |
| N-g-120 | | N-f-240 | | 4MeBn | Me | H | H | 1Me-5-Idz | C | | 440 (M⁺+1) |
| N-g-121 | | N-f-242 | | 4MeBn | Me | H | H | 1Et-5-Idz | C | | 454 (M⁺+1) |
| N-g-122 | | N-f-244 | | 2PhBn | Me | H | H | 2-Nap | C | | 498 (M⁺+1) |
| N-g-123 | | N-f-246 | | 2PhBn | Me | H | H | 5-Ind | C | | 487 (M⁺+1) |
| N-g-124 | | N-f-248 | | 2PhBn | Me | H | H | 1Me-5-Ind | C | | 501 (M⁺+1) |
| N-g-125 | | N-f-250 | | 2PhBn | Me | H | H | 5-1Idz | C | | 488 (M⁺+1) |
| N-g-126 | | N-f-252 | | 2PhBn | Me | H | H | 1Me-5-Idz | C | | 502 (M⁺+1) |
| N-g-127 | | N-f-254 | | 2PhBn | Me | H | H | 1Et-5-Idz | C | | 516 (M⁺+1) |
| N-g-128 | | N-f-256 | | 2PhBn | Et | H | H | 2-Nap | C | | 512 (M⁺+1) |
| N-g-129 | | N-f-258 | | 2PhBn | Et | H | H | 1Me-5-Ind | C | | 515 (M⁺+1) |
| N-g-130 | | N-f-260 | | 2PhBn | Et | H | H | 1Me-5-Idz | C | | 516 (M⁺+1) |
| N-g-131 | | N-f-262 | | 40MeBn | Me | H | H | 2-Nap | C | | 452 (M⁺+1) |
| N-g-132 | | N-f-264 | | 40MeBn | Me | H | H | 5-Ind | C | | 441 (M⁺+1) |
| N-g-133 | | N-f-266 | | 40MeBn | Me | H | H | 1Me-5-Ind | C | | 455 (M⁺+1) |
| N-g-134 | | N-f-268 | | 40MeBn | Me | H | H | 1Et-5-Ind | C | | 469 (M⁺+1) |
| N-g-135 | | N-f-270 | | 40MeBn | Me | H | H | 5-1Idz | C | | 442 (M⁺+1) |
| N-g-136 | | N-f-272 | | 40MeBn | Et | H | H | 2-Nap | C | | 466 (M⁺+1) |

**[Table 167]**

| Table-N-G-4 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-g-137 | | N-b-274 | | 40MeBn | Et | H | H | 1Me-5-Idz | C | | 470 (M⁺+1) |
| N-g-138 | | N-b-276 | | 20MeBn | H | H | H | 2-Nap | C | | 438 (M⁺+1) |
| N-g-139 | | N-b-278 | | 20MeBn | H | H | H | 5-Ind | C | | 427 (M⁺+1) |
| N-g-140 | | N-b-280 | | 20MeBn | H | H | H | 1Me-5-Ind | C | | 441 (M⁺+1) |
| N-g-141 | | N-b-282 | | 20MeBn | H | H | H | 5-1Idz | C | | 428 (M⁺+1) |
| N-g-142 | | N-b-284 | | 20MeBn | H | H | H | 1Me-5-Idz | C | | 442 (M⁺+1) |
| N-g-143 | | N-b-286 | | 20MeBn | Me | H | H | 2-Nap | C | | 452 (M⁺+1) |
| N-g-144 | | N-b-288 | | 20MeBn | Me | H | H | 1Me-5-Idz | C | | 456 (M⁺+1) |
| N-g-145 | | N-b-290 | | 4DMABn | H | H | H | 2-Nap | C | | 451 (M⁺+1) |
| N-g-146 | | N-b-292 | | 4DMABn | H | H | H | 5-Ind | C | | 440 (M⁺+1) |
| N-g-147 | | N-b-294 | | 4DMABn | H | H | H | 1Me-5-Ind | C | | 454 (M⁺+1) |
| N-g-148 | | N-b-296 | | 4DMABn | H | H | H | 5-1Idz | C | | 441 (M⁺+1) |
| N-g-149 | | N-b-298 | | 4DMABn | H | H | H | 1Me-5-Idz | C | | 455 (M⁺+1) |
| N-g-150 | | N-b-300 | | 4DMABn | Me | H | H | 2-Nap | C | | 465 (M⁺+1) |
| N-g-151 | | N-b-302 | | 4DMABn | Me | H | H | 1Me-5-Ind | C | | 468 (M⁺+1) |
| N-g-152 | | N-b-304 | | 4DMABn | Me | H | H | 5-1Idz | C | | 455 (M⁺+1) |
| N-g-153 | | N-b-306 | | 4DMABn | Me | H | H | 1Me-5-Idz | C | | 469 (M⁺+1) |
| N-g-154 | | N-b-308 | | 4SMeBn | Me | H | H | 2-Nap | C | | 468 (M⁺+1) |
| N-g-155 | | N-b-310 | | 4SMeBn | Me | H | H | 5-Ind | C | | 457 (M⁺+1) |
| N-g-156 | | N-b-312 | | 4SMeBn | Me | H | H | 1Me-5-Ind | C | | 471 (M⁺+1) |
| N-g-157 | | N-b-314 | | 4SMeBn | Me | H | H | 1Et-5-Ind | C | | 485 (M⁺+1) |
| N-g-158 | | N-b-316 | | 4SMeBn | Me | H | H | 5-1Idz | C | | 458 (M⁺+1) |
| N-g-159 | | N-b-318 | | 1NapMe | Me | H | H | 2-Nap | C | | 472 (M⁺+1) |
| N-g-160 | | N-b-320 | | 1NapMe | Me | H | H | 1Me-5-Ind | C | | 475 (M⁺+1) |
| N-g-161 | | N-b-322 | | 1NapMe | Me | H | H | 1Me-5-Idz | C | | 476 (M⁺+1) |
| N-g-162 | | N-b-324 | | 2NapMe | Me | H | H | 2-Nap | C | | 472 (M⁺+1) |
| N-g-163 | | N-b-326 | | 2NapMe | Me | H | H | 5-Ind | C | | 461 (M⁺+1) |
| N-g-164 | | N-b-328 | | 2NapMe | Me | H | H | 1Me-5-Ind | C | | 475 (M⁺+1) |
| N-g-165 | | N-b-330 | | 2NapMe | Me | H | H | 1Et-5-Ind | C | | 489 (M⁺+1) |
| N-g-166 | | N-b-332 | | 2NapMe | Me | H | H | 5-1Idz | C | | 462 (M⁺+1) |
| N-g-167 | | N-b-334 | | 2NapMe | Me | H | H | 1Me-5-Idz | C | | 476 (M⁺+1) |
| N-g-168 | | N-b-336 | | 2FRMe | Me | H | H | 2-Nap | C | | 412 (M⁺+1) |
| N-g-169 | | N-b-338 | | 2FRMe | Me | H | H | 1Me-5-Ind | C | | 415 (M⁺+1) |
| N-g-170 | | N-b-340 | | 2FRMe | Me | H | H | 1Et-5-Ind | C | | 429 (M⁺+1) |
| N-g-171 | | N-b-342 | | 2FRMe | Me | H | H | 5-1Idz | C | | 402 (M⁺+1) |
| N-g-172 | | N-b-344 | | 2FRMe | Me | H | H | 1Me-5-Idz | C | | 416 (M⁺+1) |
| N-g-173 | | N-b-346 | | 2FRMe | Et | H | H | 2-Nap | C | | 426 (M⁺+1) |
| N-g-174 | | N-b-348 | | 2FRMe | Et | H | H | 1Me-5-Ind | C | | 429 (M⁺+1) |
| N-g-175 | | N-b-350 | | 2FRMe | Et | H | H | 1Et-5-Ind | C | | 443 (M⁺+1) |
| N-g-176 | | N-b-352 | | 2FRMe | Et | H | H | 5-1Idz | C | | 416 (M⁺+1) |
| N-g-177 | | N-b-354 | | 2FRMe | Et | H | H | 1Me-5-Idz | C | | 430 (M⁺+1) |
| N-g-178 | | N-b-356 | | 3FRMe | Me | H | H | 2-Nap | C | | 412 (M⁺+1) |
| N-g-179 | | N-b-358 | | 3FRMe | Me | H | H | 1Me-5-Ind | C | | 415 (M⁺+1) |
| N-g-180 | | N-b-360 | | 3FRMe | Me | H | H | 5-1Idz | C | | 402 (M⁺+1) |
| N-g-181 | | N-b-362 | | 3FRMe | Me | H | H | 1Me-5-Idz | C | | 416 (M⁺+1) |
| N-g-182 | | N-b-364 | | 3FRMe | Et | H | H | 2-Nap | C | | 426 (M⁺+1) |

**[Table 168]**

| Table-N-G-5 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-g-183 | | N-b-366 | | 3FRMe | Et | H | H | 1Me-5-Ind | C | | 429 (M⁺+1) |
| N-g-184 | | N-b-368 | | 3FRMe | Et | H | H | 5-1Idz | C | | 416 (M⁺+1) |
| N-g-185 | | N-b-370 | | 3FRMe | Et | H | H | 1Me-5-Idz | C | | 430 (M⁺+1) |
| N-g-186 | | N-b-372 | | 3FRMe | Et | H | H | 1Et-5-Idz | C | | 444 (M⁺+1) |
| N-g-187 | | N-b-374 | | 2TPMe | Me | H | H | 2-Nap | C | | 428 (M⁺+1) |
| N-g-188 | | N-b-376 | | 2TPMe | Me | H | H | 1Me-5-Ind | C | | 431 (M⁺+1) |
| N-g-189 | | N-b-378 | | 2TPMe | Me | H | H | 1Et-5-Ind | C | | 445 (M⁺+1) |
| N-g-190 | | N-b-380 | | 2TPMe | Me | H | H | 5-1Idz | C | | 418 (M⁺+1) |
| N-g-191 | | N-b-382 | | 2TPMe | Me | H | H | 1Me-5-Idz | C | | 432 (M⁺+1) |
| N-g-192 | | N-b-384 | | 2TPMe | Et | H | H | 2-Nap | C | | 442 (M⁺+1) |
| N-g-193 | | N-b-386 | | 2TPMe | Et | H | H | 5-Ind | C | | 431 (M⁺+1) |
| N-g-194 | | N-b-388 | | 2TPMe | Et | H | H | 1Me-5-Ind | C | | 445 (M⁺+1) |
| N-g-195 | | N-b-390 | | 2TPMe | Et | H | H | 1Et-5-Ind | C | | 459 (M⁺+1) |
| N-g-196 | | N-b-392 | | 2TPMe | Et | H | H | 5-1Idz | C | | 432 (M⁺+1) |
| N-g-197 | | N-b-394 | | 2TPMe | Et | H | H | 1Me-5-Idz | C | | 446 (M⁺+1) |

### Example N-h-1

### Example Compound N-c-2 was subjected to chiral separation to obtain the title compound (Compound No. N-h-1, 9.3 mg).

Typical example compounds that can be obtained by reacting and treating corresponding starting compounds using any of the methods described in the present specification including the examples described above are shown in Table-N-H-1 to Table-N-H-4. As for the preparation of the compounds, all of the objective compounds were obtained by using HPLC separation. In the tables, used methods among the aforementioned synthetic methods are shown in the columns of "Syn" with symbols, the starting compounds are shown in the columns of "SM", and the reagents are shown in the columns of "Regent".

**[Table 169]**

| Table-N-H-1 | | | | | | | | | | |
|---|---|---|---|---|---|---|---|---|---|---|
| 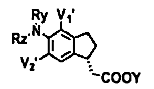 | | | | | | | | | | |

| Exp. | Syn. | SM1 | Reagent | NRyRz | Y | V₁' | V₂' | LCMS | | |
|---|---|---|---|---|---|---|---|---|---|---|
| | | | | | | | | method | RTime | Mass |
| N-h-1 | | N-c-2 | | 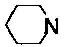 | H | H | 2-Nap | C | | 386 (M⁺+1) |
| N-h-2 | | N-c-4 | | 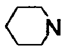 | H | H | 5-Ind | C | | 375 (M⁺+1) |
| N-h-3 | | N-c-6 | | 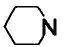 | H | H | 1Me-5-Ind | C | | 389 (M⁺+1) |
| N-h-4 | | N-c-8 | | 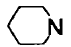 | H | H | 1Et-5-Ind | C | | 403 (M⁺+1) |
| N-h-5 | | N-c-10 | | 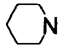 | H | H | 1Me-4-Ind | C | | 389 (M⁺+1) |
| N-h-6 | | N-c-12 | | 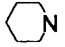 | H | H | 1Me-6-Ind | C | | 389 (M⁺+1) |
| N-h-7 | | N-c-14 | | 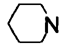 | H | H | 5-1Idz | C | | 376 (M⁺+1) |
| N-h-8 | | N-c-16 | | 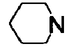 | H | H | 1Me-5-Idz | C | | 390 (M⁺+1) |
| N-h-9 | | N-c-18 | | 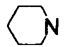 | H | H | 1Et-5-Idz | C | | 404 (M⁺+1) |
| N-h-10 | | N-c-20 | | 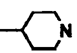 | H | H | 2-Nap | C | | 400 (M⁺+1) |
| N-h-11 | | N-c-22 | | 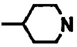 | H | H | 5-Ind | C | | 389 (M⁺+1) |
| N-h-12 | | N-c-24 | | 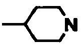 | H | H | 1Me-5-Ind | C | | 403 (M⁺+1) |
| N-h-13 | | N-c-26 | | 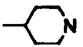 | H | H | 1Et-5-Ind | C | | 417 (M⁺+1) |
| N-h-14 | | N-c-28 | | 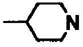 | H | H | 5-1Idz | C | | 390 (M⁺+1) |
| N-h-15 | | N-c-30 | | 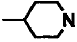 | H | H | 1Me-5-Idz | C | | 404 (M⁺+1) |
| N-h-16 | | N-c-32 | | 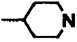 | H | H | 1Et-5-Idz | C | | 418 (M⁺+1) |
| N-h-17 | | N-c-34 | | 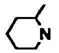 | H | H | 2-Nap | C | | 400 (M⁺+1) |
| N-h-18 | | N-c-36 | | 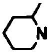 | H | H | 5-Ind | C | | 389 (M⁺+1) |
| N-h-19 | | N-c-38 | | 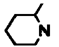 | H | H | 1Me-5-Ind | C | | 403 (M⁺+1) |
| N-h-20 | | N-c-40 | | 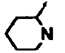 | H | H | 5-1Idz | C | | 390 (M⁺+1) |
| N-h-21 | | N-c-42 | | 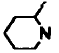 | H | H | 1Me-5-Idz | C | | 404 (M⁺+1) |

**[Table-170]**

| Table-N-H-2 | | | | | | | | | | |
|---|---|---|---|---|---|---|---|---|---|---|
| Exp. | Syn. | SM1 | Reagent | NRzRy | Y | Z | AR | LCMS | | |
| | | | | | | | | method | RTime | Mass |
| N-h-22 | | N-c-44 | | 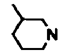 | H | H | 2-Nap | C | | 400 (M⁺+1) |
| N-h-23 | | N-c-46 | | 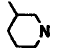 | H | H | 1Me-5-Ind | C | | 403 (M⁺+1) |
| N-h-24 | | N-c-48 | | 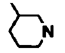 | H | H | 1Me-5-Idz | C | | 404 (M⁺+1) |
| N-h-25 | | N-c-50 | | 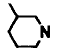 | H | H | 1Et-5-Idz | C | | 418 (M⁺+1) |
| N-h-26 | | N-c-52 | | 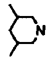 | H | H | 2-Nap | C | | 414 (M⁺+1) |
| N-h-27 | | N-c-54 | | 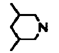 | H | H | 5-Ind | C | | 403 (M⁺+1) |
| N-h-28 | | N-c-56 | | 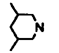 | H | H | 1Me-5-Ind | C | | 417 (M⁺+1) |
| N-h-29 | | N-c-58 | | 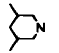 | H | H | 5-1Idz | C | | 404 (M⁺+1) |
| N-h-30 | | N-c-60 | | 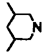 | H | H | 1Me-5-Idz | C | | 418 (M⁺+1) |
| N-h-31 | | N-c-62 | | 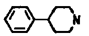 | H | H | 2-Nap | C | | 462 (M⁺+1) |
| N-h-32 | | N-c-64 | | 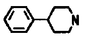 | H | H | 5-Ind | C | | 451 (M⁺+1) |
| N-h-33 | | N-c-66 | | 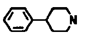 | H | H | 1Me-5-Ind | C | | 465 (M⁺+1) |
| N-h-34 | | N-c-68 | | 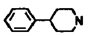 | H | H | 5-1 Idz | C | | 452 (M⁺+1) |
| N-h-35 | | N-c-70 | | 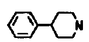 | H | H | 1Me-5-Idz | C | | 466 (M⁺+1) |
| N-h-36 | | N-c-72 | | 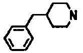 | H | H | 2-Nap | C | | 476 (M⁺+1) |
| N-h-37 | | N-c-74 | | 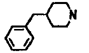 | H | H | 5-Ind | C | | 465 (M⁺+1) |
| N-h-38 | | N-c-76 | | 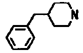 | H | H | 1Me-5-Ind | C | | 479 (M⁺+1) |
| N-h-39 | | N-c-78 | | 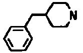 | H | H | 1Me-5-Idz | C | | 480 (M⁺+1) |
| N-h-40 | | N-c-80 | | 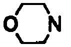 | H | H | 2-Nap | C | | 388 (M⁺+1) |
| N-h-41 | | N-c-82 | | 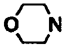 | H | H | 5-Ind | C | | 377 (M⁺+1) |
| N-h-42 | | N-c-84 | | 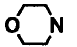 | H | H | 1Me-5-Ind | C | | 391 (M⁺+1) |
| N-h-43 | | N-c-86 | | 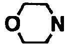 | H | H | 5-1Idz | C | | 378 (M⁺+1) |
| N-h-44 | | N-c-88 | | 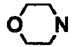 | H | H | 1Me-5-Idz | C | | 392 (M⁺+1) |

**[Table 171]**

| Table-N-H-3 | | | | | | | | | | |
|---|---|---|---|---|---|---|---|---|---|---|
| Exp. | Syn. | SM1 | Reagent | NRzRy | Y | Z | AR | LCMS | | |
| | | | | | | | | method | RTime | Mass |
| N-h-45 | | N-c-90 | | 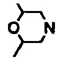 | H | H | 2-Nap | C | | 416 (M⁺+1) |
| N-h-46 | | N-c-92 | | 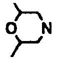 | H | H | 5-Ind | C | | 405 (M⁺+1) |
| N-h-47 | | N-c-94 | | 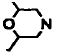 | H | H | 1Me-5-Ind | C | | 419 (M⁺+1) |
| N-h-48 | | N-c-96 | | 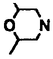 | H | H | 5-1Idz | C | | 406 (M⁺+1) |
| N-h-49 | | N-c-98 | | 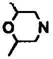 | H | H | 1Me-5-Idz | C | | 420 (M⁺+1) |
| N-h-50 | | N-c-100 | | 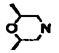 | H | H | 2-Nap | C | | 416 (M⁺+1) |
| N-h-51 | | N-c-102 | | 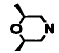 | H | H | 5-Ind | C | | 405 (M⁺+1) |
| N-h-52 | | N-c-104 | | 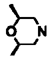 | H | H | 5-1Idz | C | | 406 (M⁺+1) |
| N-h-53 | | N-c-106 | | 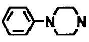 | H | H | 2-Nap | C | | 463 (M⁺+1) |
| N-h-54 | | N-c-108 | | 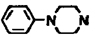 | H | H | 5-Ind | C | | 452 (M⁺+1) |
| N-h-55 | | N-c-110 | | 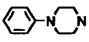 | H | H | 1Me-5-Ind | C | | 466 (M⁺+1) |
| N-h-56 | | N-c-112 | | 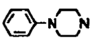 | H | H | 5-1Idz | C | | 453 (M⁺+1) |
| N-h-57 | | N-c-114 | | 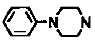 | H | H | 1Me-5-Idz | C | | 467 (M⁺+1) |
| N-h-58 | | N-c-116 | | 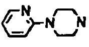 | H | H | 2-Nap | C | | 464 (M⁺+1) |
| N-h-59 | | N-c-118 | | 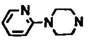 | H | H | 1Me-5-Ind | C | | 467 (M⁺+1) |
| N-h-60 | | N-c-120 | | 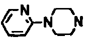 | H | H | 1Me-5-Idz | C | | 468 (M⁺+1) |
| N-h-61 | | N-c-122 | | 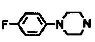 | H | H | 2-Nap | C | | 481 (M⁺+1) |
| N-h-62 | | N-c-124 | | 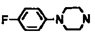 | H | H | 5-Ind | C | | 470 (M⁺+1) |
| N-h-63 | | N-c-126 | | 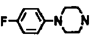 | H | H | 5-1Idz | C | | 471 (M⁺+1) |
| N-h-64 | | N-c-128 | | 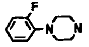 | H | H | 2-Nap | C | | 481 (M⁺+1) |
| N-h-65 | | N-c-130 | | 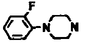 | H | H | 1Me-5-Ind | C | | 484 (M⁺+1) |
| N-h-66 | | N-c-132 | | 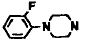 | H | H | 1Me-5-Idz | C | | 485 (M⁺+1) |

**[Table 172]**

| Table-N-H-4 | | | | | | | | | | |
|---|---|---|---|---|---|---|---|---|---|---|
| Exp. | Syn. | SM1 | Reagent | NRzRy | Y | Z | AR | LCMS | | |
| | | | | | | | | method | RTime | Mass |
| N-h-67 | | N-c-134 | | 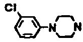 | H | H | 2-Nap | C | | 498 (M⁺+1) |
| N-h-68 | | N-c-136 | | 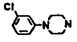 | H | H | 5-Ind | C | | 487 (M⁺+1) |
| N-h-69 | | N-c-138 | | 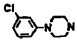 | H | H | 1Me-5-Ind | C | | 501 (M⁺+1) |
| N-h-70 | | N-c-140 | | 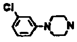 | H | H | 1Me-4-Ind | C | | 501 (M⁺+1) |
| N-h-71 | | N-c-142 | | 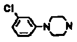 | H | H | 5-1Idz | C | | 488 (M⁺+1) |
| N-h-72 | | N-c-144 | | 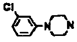 | H | H | 1Me-5-Idz | C | | 502 (M⁺+1) |
| N-h-73 | | N-c-146 | | 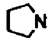 | H | H | 2-Nap | C | | 372 (M⁺+1) |
| N-h-74 | | N-c-148 | | 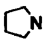 | H | H | 5-Ind | C | | 361 (M⁺+1) |
| N-h-75 | | N-c-150 | | 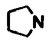 | H | H | 1Me-5-Ind | C | | 375 (M⁺+1) |
| N-h-76 | | N-c-152 | | 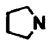 | H | H | 5-1Idz | C | | 362 (M⁺+1) |
| N-h-77 | | N-c-154 | | 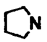 | H | H | 1Me-5-Idz | C | | 376 (M⁺+1) |
| N-h-78 | | N-c-156 | | 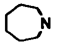 | H | H | 2-Nap | C | | 400 (M⁺+1) |
| N-h-79 | | N-c-158 | | 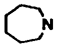 | H | H | 5-Ind | C | | 389 (M⁺+1) |
| N-h-80 | | N-c-160 | | 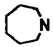 | H | H | 1Me-5-Ind | C | | 403 (M⁺+1) |
| N-h-81 | | N-c-162 | | 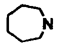 | H | H | 5-1Idz | C | | 390 (M⁺+1) |
| N-h-82 | | N-c-164 | | 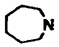 | H | H | 1Me-5-Idz | C | | 404 (M⁺+1) |

### Example N-i-1

### Example Compound N-c-2 was subjected to chiral separation to obtain the title compound (Compound No. N-h-1, 9.0 mg).

Typical example compounds that can be obtained by reacting and treating corresponding starting compounds using any of the methods described in the present specification including the examples described above are shown in Table-N-I-1 to Table-N-I-4. As for the preparation of the compounds, all of the objective compounds were obtained by using HPLC separation. In the tables, used methods among the aforementioned synthetic methods are shown in the columns of "Syn" with symbols, the starting compounds are shown in the columns of "SM", and the reagents are shown in the columns of "Regent".

**[Table 173]**

| Table-N-I-1 | | | | | | | | | | |
|---|---|---|---|---|---|---|---|---|---|---|
| 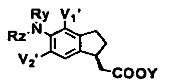 | | | | | | | | | | |

| Exp. | Syn. | SM1 | Reagent | NRyRz | Y | V₁' | V₂' | LCMS | | |
|---|---|---|---|---|---|---|---|---|---|---|
| | | | | | | | | method | RTime | Mass |
| N-i-1 | | N-c-2 | | 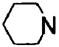 | H | H | 2-Nap | C | | 386 (M⁺+1) |
| N-i-2 | | N-c-4 | | 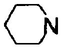 | H | H | 5-Ind | C | | 375 (M⁺+1) |
| N-i-3 | | N-c-6 | | 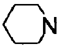 | H | H | 1Me-5-Ind | C | | 389 (M⁺+1) |
| N-i-4 | | N-c-8 | | 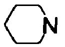 | H | H | 1Et-5-Ind | C | | 403 (M⁺+1) |
| N-i-5 | | N-c-10 | | 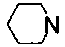 | H | H | 1Me-4-Ind | C | | 389 (M⁺+1) |
| N-i-6 | | N-c-12 | | 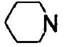 | H | H | 1Me-6-Ind | C | | 389 (M⁺+1) |
| N-i-7 | | N-c-14 | | 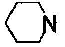 | H | H | 5-1Idz | C | | 376 (M⁺+1) |
| N-i-8 | | N-c-16 | | 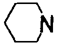 | H | H | 1Me-5-Idz | C | | 390 (M⁺+1) |
| N-i-9 | | N-c-18 | | 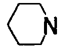 | H | H | 1Et-5-Idz | C | | 404 (M⁺+1) |
| N-i-10 | | N-c-20 | | 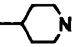 | H | H | 2-Nap | C | | 400 (M⁺+1) |
| N-i-11 | | N-c-22 | | 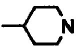 | H | H | 5-Ind | C | | 389 (M⁺+1) |
| N-i-12 | | N-c-24 | | 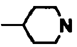 | H | H | 1Me-5-Ind | C | | 403 (M⁺+1) |
| N-i-13 | | N-c-26 | | 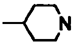 | H | H | 1Et-5-Ind | C | | 417 (M⁺+1) |
| N-i-14 | | N-c-28 | | 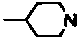 | H | H | 5-1Idz | C | | 390 (M⁺+1) |
| N-i-15 | | N-c-30 | | 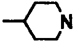 | H | H | 1Me-5-Idz | C | | 404 (M⁺+1) |
| N-i-16 | | N-c-32 | | 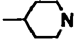 | H | H | 1Et-5-Idz | C | | 418 (M⁺+1) |
| N-i-17 | | N-c-34 | | 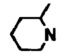 | H | H | 2-Nap | C | | 400 (M⁺+1) |
| N-i-18 | | N-c-36 | | 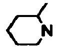 | H | H | 5-Ind | C | | 389 (M⁺+1) |
| N-i-19 | | N-c-38 | | 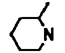 | H | H | 1Me-5-Ind | C | | 403 (M⁺+1) |
| N-i-20 | | N-c-40 | | 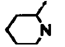 | H | H | 5-1Idz | C | | 390 (M⁺+1) |
| N-i-21 | | N-c-42 | | 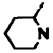 | H | H | 1Me-5-Idz | C | | 404 (M⁺+1) |

**[Table 174]**

| Table-N-I-2 | | | | | | | | | | |
|---|---|---|---|---|---|---|---|---|---|---|
| Exp. | Syn. | SM1 | Reagent | NRzRy | Y | Z | AR | LCMS | | |
| | | | | | | | | method | RTime | Mass |
| N-i-22 | | N-c-44 | | 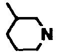 | H | H | 2-Nap | C | | 400 (M⁺+1) |
| N-i-23 | | N-c-46 | | 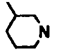 | H | H | 1Me-5-Ind | C | | 403 (M⁺+1) |
| N-i-24 | | N-c-48 | | 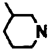 | H | H | 1Me-5-Idz | C | | 404 (M⁺+1) |
| N-i-25 | | N-c-50 | | 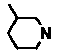 | H | H | 1Et-5-Idz | C | | 418 (M⁺+1) |
| N-i-26 | | N-c-52 | | 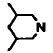 | H | H | 2-Nap | C | | 414 (M⁺+1) |
| N-i-27 | | N-c-54 | | 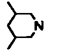 | H | H | 5-Ind | C | | 403 (M⁺+1) |
| N-i-28 | | N-c-56 | | 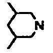 | H | H | 1Me-5-Ind | C | | 417 (M⁺+1) |
| N-i-29 | | N-c-58 | | 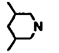 | H | H | 5-1Idz | C | | 404 (M⁺+1) |
| N-i-30 | | N-c-60 | | 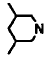 | H | H | 1Me-5-Idz | C | | 418 (M⁺+1) |
| N-i-31 | | N-c-62 | | 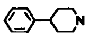 | H | H | 2-Nap | C | | 462 (M⁺+1) |
| N-i-32 | | N-c-64 | | 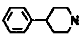 | H | H | 5-Ind | C | | 451 (M⁺+1) |
| N-i-33 | | N-c-66 | | 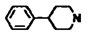 | H | H | 1Me-5-Ind | C | | 465 (M⁺+1) |
| N-i-34 | | N-c-68 | | 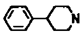 | H | H | 5-1Idz | C | | 452 (M⁺+1) |
| N-i-35 | | N-c-70 | | 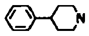 | H | H | 1Me-5-Idz | C | | 466 (M⁺+1) |
| N-i-36 | | N-c-72 | | 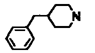 | H | H | 2-Nap | C | | 476 (M⁺+1) |
| N-i-37 | | N-c-74 | | 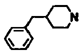 | H | H | 5-Ind | C | | 465 (M⁺+1) |
| N-i-38 | | N-c-76 | | 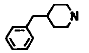 | H | H | 1Me-5-Ind | C | | 479 (M⁺+1) |
| N-i-39 | | N-c-78 | | 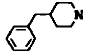 | H | H | 1Me-5-Idz | C | | 480 (M⁺+1) |
| N-i-40 | | N-c-80 | | 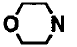 | H | H | 2-Nap | C | | 388 (M⁺+1) |
| N-i-41 | | N-c-82 | | 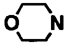 | H | H | 5-Ind | C | | 377 (M⁺+1) |
| N-i-42 | | N-c-84 | | 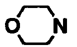 | H | H | 1Me-5-Ind | C | | 391 (M⁺+1) |
| N-i-43 | | N-c-86 | | 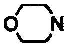 | H | H | 5-1Idz | C | | 378 (M⁺+1) |
| N-i-44 | | N-c-88 | | 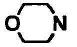 | H | H | 1Me-5-Idz | C | | 392 (M⁺+1) |

**[Table 175]**

| Table-N-I-3 | | | | | | | | | | |
|---|---|---|---|---|---|---|---|---|---|---|
| Exp. | Syn. | SM1 | Reagent | NRzRy | Y | Z | AR | LCMS | | |
| | | | | | | | | method | RTime | Mass |
| N-i-45 | | N-c-90 | | 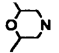 | H | H | 2-Nap | C | | 416 (M⁺+1) |
| N-i-46 | | N-c-92 | | 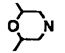 | H | H | 5-Ind | C | | 405 (M⁺+1) |
| N-i-47 | | N-c-94 | | 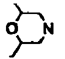 | H | H | 1Me-5-Ind | C | | 419 (M⁺+1) |
| N-i-48 | | N-c-96 | | 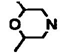 | H | H | 5-1Idz | C | | 406 (M⁺+1) |
| N-i-49 | | N-c-98 | | 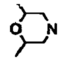 | H | H | 1Me-5-Idz | C | | 420 (M⁺+1) |
| N-i-50 | | N-c-100 | | 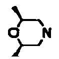 | H | H | 2-Nap | C | | 416 (M⁺+1) |
| N-i-51 | | N-c-102 | | 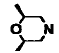 | H | H | 5-Ind | C | | 405 (M⁺+1) |
| N-i-52 | | N-c-104 | | 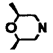 | H | H | 5-1Idz | C | | 406 (M⁺+1) |
| N-i-53 | | N-c-106 | | 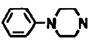 | H | H | 2-Nap | C | | 463 (M⁺+1) |
| N-i-54 | | N-c-108 | | 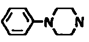 | H | H | 5-Ind | C | | 452 (M⁺+1) |
| N-i-55 | | N-c-110 | | 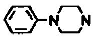 | H | H | 1Me-5-Ind | C | | 466 (M⁺+1) |
| N-i-56 | | N-c-112 | | 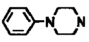 | H | H | 5-1Idz | C | | 453 (M⁺+1) |
| N-i-57 | | N-c-114 | | 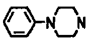 | H | H | 1Me-5-Idz | C | | 467 (M⁺+1) |
| N-i-58 | | N-c-116 | | 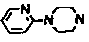 | H | H | 2-Nap | C | | 464 (M⁺+1) |
| N-i-59 | | N-c-118 | | 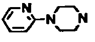 | H | H | 1Me-5-Ind | C | | 467 (M⁺+1) |
| N-i-60 | | N-c-120 | | 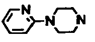 | H | H | 1Me-5-Idz | C | | 468 (M⁺+1) |
| N-i-61 | | N-c-122 | | 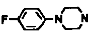 | H | H | 2-Nap | C | | 481 (M⁺+1) |
| N-i-62 | | N-c-124 | | 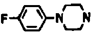 | H | H | 5-Ind | C | | 470 (M⁺+1) |
| N-i-63 | | N-c-126 | | 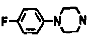 | H | H | 5-1Idz | C | | 471 (M⁺+1) |
| N-i-64 | | N-c-128 | | 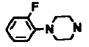 | H | H | 2-Nap | C | | 481 (M⁺+1) |
| N-i-65 | | N-c-130 | | 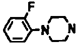 | H | H | 1Me-5-Ind | C | | 484 (M⁺+1) |
| N-i-66 | | N-c-132 | | 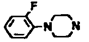 | H | H | 1Me-5-Idz | C | | 485 (M⁺+1) |

**[Table 176]**

| Table-N-I-4 | | | | | | | | | | |
|---|---|---|---|---|---|---|---|---|---|---|
| Exp. | Syn. | SM1 | Reagent | NRzRy | Y | Z | AR | LCMS | | |
| | | | | | | | | method | RTime | Mass |
| N-i-67 | | N-c-134 | | 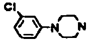 | H | H | 2-Nap | C | | 498 (M⁺+1) |
| N-i-68 | | N-c-136 | | 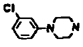 | H | H | 5-Ind | C | | 487 (M⁺+1) |
| N-i-69 | | N-c-138 | | 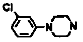 | H | H | 1Me-5-Ind | C | | 501 (M⁺+1) |
| N-i-70 | | N-c-140 | | 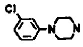 | H | H | 1Me-4-Ind | C | | 501 (M⁺+1) |
| N-i-71 | | N-c-142 | | 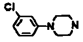 | H | H | 5-1Idz | C | | 488 (M⁺+1) |
| N-i-72 | | N-c-144 | | 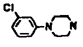 | H | H | 1Me-5-Idz | C | | 502 (M⁺+1) |
| N-i-73 | | N-c-146 | | 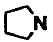 | H | H | 2-Nap | C | | 372 (M⁺+1) |
| N-i-74 | | N-c-148 | | 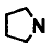 | H | H | 5-Ind | C | | 361 (M⁺+1) |
| N-i-75 | | N-c-150 | | 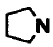 | H | H | 1Me-5-Ind | C | | 375 (M⁺+1) |
| N-i-76 | | N-c-152 | | 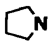 | H | H | 5-1Idz | C | | 362 (M⁺+1) |
| N-i-77 | | N-c-154 | | 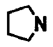 | H | H | 1Me-5-Idz | C | | 376 (M⁺+1) |
| N-i-78 | | N-c-156 | | 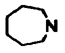 | H | H | 2-Nap | C | | 400 (M⁺+1) |
| N-i-79 | | N-c-158 | | 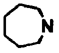 | H | H | 5-Ind | C | | 389 (M⁺+1) |
| N-i-80 | | N-c-160 | | 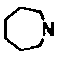 | H | H | 1Me-5-Ind | C | | 403 (M⁺+1) |
| N-i-81 | | N-c-162 | | 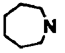 | H | H | 5-1Idz | C | | 390 (M⁺+1) |
| N-i-82 | | N-c-164 | | 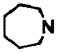 | H | H | 1Me-5-Idz | C | | 404 (M⁺+1) |

### Reference Example 17

### Synthesis of 4-bromo-5-benzylmethylamino-2,3-dihydroinden-1-one (Intermediate n-d-1) (Preparation Method 13, Step h)

According to the procedure described in the synthetic method of Intermediate A-4 in Reference Example 1 (Preparation Method 8, Step h) provided that the reaction was performed at room temperature for 1 hour, Intermediate n-a-1 (514.9 mg) and NBS (387.5 mg, WAKO) were reacted and treated to obtain the title compound (Intermediate n-d-1, 538.9 mg). Mass (LCMS): 320 (M⁺+1), Retention time: 5.48 minutes (Elution condition: B).

### Synthesis of 5-benzylmethylamino-4-(naphthalen-2-yl)-2,3-dihydroinden-1-one (Intermediate n-d-2) (Preparation Method 11, Step e-1)

According to the procedure described in the synthetic method of Example Compound A-a-1 (Preparation Method 4, Step e-1) provided that the reaction was performed for 12 hours, Intermediate n-d-1 (538.9 mg), 2-naphthaleneboronic acid (465.4 mg, TCI), 2 M aqueous sodium carbonate (1.5 ml) and (Ph₃P)₄Pd (211.2 mg, Nakalai Tesque) were reacted and treated to obtain the title compound (Intermediate n-d-2, 507.4 mg). Mass (LCMS): 364 (M⁺+1), Retention time: N.D (Elution condition: C).

### Synthesis of 5-methylamino-4-(naphthalen-2-yl)-2,3-dihydroinden-1-one (Intermediate n-d-3)

According to the procedure described in the synthetic method of Intermediate A-22 in Example A-a-107, Intermediate n-d-2 (310.4 mg) and 10% palladium/carbon (37.5 mg, Merck) were reacted and treated to obtain the title compound (Intermediate n-d-3, 251.2 mg). Mass (LCMS): 274 (M⁺+1), Retention time: N.D (Elution condition: C).
Typical examples of the intermediates that can be obtained by reacting and treating corresponding starting compounds using any of the methods described in the present specification including the examples described above are shown in Table-Int.N-d-1 and Table-Int.N-d-2. The compounds were synthesized according to the preparation methods of the compounds of the compound numbers shown in the columns of "Syn" in the tables. "Int" means an intermediate compound number. In the tables, used methods among the aforementioned synthetic methods are shown in the columns of "Syn" with symbols, the starting compounds are shown in the columns of "SM", and the reagents are shown in the columns of "Regent". The boronic acid regents mentioned in the columns of "Reagent" with symbols "BRA (No.)" are those mentioned in Table-BRA, and the formyl regents mentioned with the symbols "CHO (No.)" are those mentioned in Table-CHO. The blank cells of the columns of "Syn" indicate that a reduction reaction with Pd/C was performed for the compounds.

**[Table 177]**

| Table-IntN-d-1 | | | | | | | | | | |
|---|---|---|---|---|---|---|---|---|---|---|
| 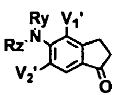 | | | | | | | | | | |

| Exp. | Syn. | SM1 | Reagent | Ry | Rz | V₁' | V₂' | LCMS | | |
|---|---|---|---|---|---|---|---|---|---|---|
| | | | | | | | | method | RTime | Mass |
| Int.n-d-4 | 14n-2 | Int.n-d-3 | CHO3 | nPr | Me | 2-Nap | H | C | | 330 (M⁺+1) |
| Int.n-d-5 | 14n-2 | Int.n-d-3 | CHO4 | iPr | Me | 2-Nap | H | C | | 330 (M⁺+1) |
| Int.n-d-6 | 14n-2 | Int.n-d-3 | CHO5 | nBu | Me | 2-Nap | H | C | | 344 (M⁺+1) |
| Int.n-d-7 | 14n-2 | Int.n-d-3 | CHO6 | iBu | Me | 2-Nap | H | C | | 344 (M⁺+1) |
| Int.n-d-8 | 14n-2 | Int.n-d-3 | CHO7 | cPen | Me | 2-Nap | H | C | | 356 (M⁺+1) |
| Int.n-d-9 | 14n-2 | Int.n-d-3 | CHO8 | cHex | Me | 2-Nap | H | C | | 370 (M⁺+1) |
| Int.n-d-10 | 11e-1 | Int.n-a-1 | BRA2 | Bn | Me | 5-Ind | H | C | | 367 (M⁺+1) |
| Int.n-d-11 | | Int.n-d-10 | | H | Me | 5-Ind | H | C | | 277 (M⁺+1) |
| Int.n-d-12 | 14n-2 | Int.n-d-11 | CHO3 | nPr | Me | 5-Ind | H | C | | 319 (M⁺+1) |
| Int.n-d-13 | 14n-2 | Int.n-d-11 | CHO4 | iPr | Me | 5-Ind | H | C | | 319 (M⁺+1) |
| Int.n-d-14 | 14n-2 | Int.n-d-11 | CHO5 | nBu | Me | 5-Ind | H | C | | 333 (M⁺+1) |
| Int.n-d-15 | 14n-2 | Int.n-d-11 | CHO6 | iBu | Me | 5-Ind | H | C | | 333 (M⁺+1) |
| Int.n-d-16 | 14n-2 | Int.n-d-11 | CHO7 | cPen | Me | 5-Ind | H | C | | 345 (M⁺+1) |
| Int.n-d-17 | 14n-2 | Int.n-d-11 | CHO8 | cHex | Me | 5-Ind | H | C | | 359 (M⁺+1) |
| Int.n-d-18 | 11e-1 | Int.n-a-1 | BRA3 | Bn | Me | 1Me-5-Ind | H | C | | 381 (M⁺+1) |
| Int.n-d-19 | | Int.n-d-18 | | H | Me | 1Me-5-Ind | H | C | | 291 (M⁺+1) |
| Int.n-d-20 | 14n-2 | Int.n-d-19 | CHO3 | nPr | Me | 1Me-5-Ind | H | C | | 333 (M⁺+1) |
| Int.n-d-21 | 14n-2 | Int.n-d-19 | CHO6 | iBu | Me | 1Me-5-Ind | H | C | | 347 (M⁺+1) |
| Int.n-d-22 | 14n-2 | Int.n-d-19 | CHO7 | cPen | Me | 1Me-5-Ind | H | C | | 359 (M⁺+1) |
| Int.n-d-23 | 14n-2 | Int.n-d-19 | CHO8 | cHex | Me | 1Me-5-Ind | H | C | | 373 (M⁺+1) |
| Int.n-d-24 | 11e-1 | Int.n-a-1 | BRA4 | Bn | Me | 1Et-5-Ind | H | C | | 395 (M⁺+1) |
| Int.n-d-25 | | Int.n-d-24 | | H | Me | 1Et-5-Ind | H | C | | 305 (M⁺+1) |
| Int.n-d-26 | 14n-2 | Int.n-d-25 | CHO4 | iPr | Me | 1Et-5-Ind | H | C | | 347 (M⁺+1) |
| Int.n-d-27 | 14n-2 | Int.n-d-25 | CHO5 | nBu | Me | 1Et-5-Ind | H | C | | 361 (M⁺+1) |
| Int.n-d-28 | 14n-2 | Int.n-d-25 | CHO6 | iBu | Me | 1Et-5-Ind | H | C | | 361 (M⁺+1) |
| Int.n-d-29 | 14n-2 | Int.n-d-25 | CHO7 | cPen | Me | 1Et-5-Ind | H | C | | 373 (M⁺+1) |
| Int.n-d-30 | 11e-1 | Int.n-a-1 | BRA8 | Bn | Me | 1Me-5-Idz | H | C | | 382 (M⁺+1) |
| Int.n-d-31 | | Int.n-d-30 | | H | Me | 1Me-5-Idz | H | C | | 292 (M⁺+1) |
| Int.n-d-32 | 14n-2 | Int.n-d-31 | CHO3 | nPr | Me | 1Me-5-Idz | H | C | | 334 (M⁺+1) |
| Int.n-d-33 | 14n-2 | Int.n-d-31 | CHO4 | iPr | Me | 1Me-5-Idz | H | C | | 334 (M⁺+1) |
| Int.n-d-34 | 14n-2 | Int.n-d-31 | CHO5 | nBu | Me | 1Me-5-Idz | H | C | | 348 (M⁺+1) |
| Int.n-d-35 | 14n-2 | Int.n-d-31 | CHO6 | iBu | Me | 1Me-5-Idz | H | C | | 348 (M⁺+1) |
| Int.n-d-36 | 14n-2 | Int.n-d-31 | CHO7 | cPen | Me | 1Me-5-Idz | H | C | | 360 (M⁺+1) |
| Int.n-d-37 | 14n-2 | Int.n-d-31 | CHO8 | cHex | Me | 1Me-5-Idz | H | C | | 374 (M⁺+1) |
| Int.n-d-38 | 11e-1 | Int.n-a-1 | BRA9 | Bn | Me | 1Et-5-Idz | H | C | | 396 (M⁺+1) |
| Int.n-d-39 | | Int.n-d-38 | | H | Me | 1Et-5-Idz | H | C | | 306 (M⁺+1) |
| Int.n-d-40 | 14n-2 | Int.n-d-39 | CHO3 | nPr | Me | 1Et-5-Idz | H | C | | 348 (M⁺+1) |
| Int.n-d-41 | 14n-2 | Int.n-d-39 | CHO4 | iPr | Me | 1Et-5-Idz | H | C | | 348 (M⁺+1) |
| Int.n-d-42 | 14n-2 | Int.n-d-39 | CHO8 | cHex | Me | 1Et-5-Idz | H | C | | 388 (M⁺+1) |
| Int.n-d-43 | 11e-1 | Int.n-a-2 | BRA1 | Bn | Et | 2-Nap | H | C | | 392 (M⁺+1) |
| Int.n-d-44 | | Int.n-d-43 | | H | Et | 2-Nap | H | C | | 302 (M⁺+1) |
| Int.n-d-45 | 14n-2 | Int.n-d-44 | CHO3 | nPr | Et | 2-Nap | H | C | | 344 (M⁺+1) |
| Int.n-d-46 | 14n-2 | Int.n-d-44 | CHO4 | iPr | Et | 2-Nap | H | C | | 344 (M⁺+1) |
| Int.n-d-47 | 14n-2 | Int.n-d-44 | CHO5 | nBu | Et | 2-Nap | H | C | | 358 (M⁺+1) |
| Int.n-d-48 | 14n-2 | Int.n-d-44 | CHO6 | iBu | Et | 2-Nap | H | C | | 358 (M⁺+1) |

**[Table 178]**

| Table-Int.N-d-2 | | | | | | | | | | |
|---|---|---|---|---|---|---|---|---|---|---|
| Exp. | Syn. | SM1 | Reagent | Ry | Rz | V₁' | V₂' | LCMS | | |
| | | | | | | | | method | RTime | Mass |
| Int.n-d-49 | 14n-2 | Int.n-d-44 | CHO7 | cPen | Et | 2-Nap | H | C | | 370 (M⁺+1) |
| Int.n-d-50 | 14n-2 | Int.n-d-44 | CHO8 | cHex | Et | 2-Nap | H | C | | 384 (M⁺+1) |
| Int.n-d-51 | 11e-1 | Int.n-a-2 | BRA2 | Bn | Et | 5-Ind | H | C | | 381 (M⁺+1) |
| Int.n-d-52 | | Int.n-d-51 | | H | Et | 5-Ind | H | C | | 291 (M⁺+1) |
| Int.n-d-53 | 14n-2 | Int.n-d-52 | CHO3 | nPr | Et | 5-Ind | H | C | | 333 (M⁺+1) |
| Int.n-d-54 | 14n-2 | Int.n-d-52 | CHO4 | iPr | Et | 5-Ind | H | C | | 333 (M⁺+1) |
| Int.n-d-55 | 14n-2 | Int.n-d-52 | CHO5 | nBu | Et | 5-Ind | H | C | | 347 (M⁺+1) |
| Int.n-d-56 | 14n-2 | Int.n-d-52 | CHO6 | iBu | Et | 5-Ind | H | C | | 347 (M⁺+1) |
| Int.n-d-57 | 14n-2 | Int.n-d-52 | CHO7 | cPen | Et | 5-Ind | H | C | | 359 (M⁺+1) |
| Int.n-d-58 | 11e-1 | Int.n-a-2 | BRA3 | Bn | Et | 1Me-5-Ind | H | C | | 395 (M⁺+1) |
| Int.n-d-59 | | Int.n-d-58 | | H | Et | 1Me-5-Ind | H | C | | 305 (M⁺+1) |
| Int.n-d-60 | 14n-2 | Int.n-d-59 | CHO3 | nPr | Et | 1Me-5-Ind | H | C | | 347 (M⁺+1) |
| Int.n-d-61 | 14n-2 | Int.n-d-59 | CHO4 | iPr | Et | 1Me-5-Ind | H | C | | 347 (M⁺+1) |
| Int.n-d-62 | 14n-2 | Int.n-d-59 | CHO5 | nBu | Et | 1Me-5-Ind | H | C | | 361 (M⁺+1) |
| Int.n-d-63 | 14n-2 | Int.n-d-59 | CHO6 | iBu | Et | 1Me-5-Ind | H | C | | 361 (M⁺+1) |
| Int.n-d-64 | 14n-2 | Int.n-d-59 | CHO7 | cPen | Et | 1Me-5-Ind | H | C | | 373 (M⁺+1) |
| Int.n-d-65 | 14n-2 | Int.n-d-59 | CHO8 | cHex | Et | 1Me-5-Ind | H | C | | 387 (M⁺+1) |
| Int.n-d-66 | 11e-1 | Int.n-a-2 | BRA4 | Bn | Et | 1Et-5-Ind | H | C | | 409 (M⁺+1) |
| Int.n-d-67 | | Int.n-d-66 | | H | Et | 1Et-5-Ind | H | C | | 319 (M⁺+1) |
| Int.n-d-68 | 14n-2 | Int.n-d-67 | CHO4 | iPr | Et | 1Et-5-Ind | H | C | | 361 (M⁺+1) |
| Int.n-d-69 | 14n-2 | Int.n-d-67 | CHO5 | nBu | Et | 1Et-5-Ind | H | C | | 375 (M⁺+1) |
| Int.n-d-70 | 14n-2 | Int.n-d-67 | CHO7 | cPen | Et | 1Et-5-Ind | H | C | | 387 (M⁺+1) |
| Int.n-d-71 | 11e-1 | Int.n-a-2 | BRA8 | Bn | Et | 1Me-5-Idz | H | C | | 396 (M⁺+1) |
| Int.n-d-72 | | Int.n-d-71 | | H | Et | 1Me-5-Idz | H | C | | 306 (M⁺+1) |
| Int.n-d-73 | 14n-2 | Int.n-d-72 | CHO3 | nPr | Et | 1Me-5-Idz | H | C | | 348 (M⁺+1) |
| Int.n-d-74 | 14n-2 | Int.n-d-72 | CHO4 | iPr | Et | 1Me-5-Idz | H | C | | 348 (M⁺+1) |
| Int.n-d-75 | 14n-2 | Int.n-d-72 | CHO5 | nBu | Et | 1Me-5-Idz | H | C | | 362 (M⁺+1) |
| Int.n-d-76 | 14n-2 | Int.n-d-72 | CHO6 | iBu | Et | 1Me-5-Idz | H | C | | 362 (M⁺+1) |
| Int.n-d-77 | 14n-2 | Int.n-d-72 | CHO7 | cPen | Et | 1Me-5-Idz | H | C | | 374 (M⁺+1) |
| Int.n-d-78 | 14n-2 | Int.n-d-72 | CHO8 | cHex | Et | 1Me-5-Idz | H | C | | 388 (M⁺+1) |
| Int.n-d-79 | 11e-1 | Int.n-a-2 | BRA9 | Bn | Et | 1Et-5-Idz | H | C | | 410 (M⁺+1) |
| Int.n-d-80 | | Int.n-d-79 | | H | Et | 1Et-5-Idz | H | C | | 320 (M⁺+1) |
| Int.n-d-81 | 14n-2 | Int.n-d-80 | CHO3 | nPr | Et | 1Et-5-Idz | H | C | | 362 (M⁺+1) |
| Int.n-d-82 | 14n-2 | Int.n-d-80 | CHO4 | iPr | Et | 1Et-5-Idz | H | C | | 362 (M⁺+1) |
| Int.n-d-83 | 14n-2 | Int.n-d-80 | CHO5 | nBu | Et | 1Et-5-Idz | H | C | | 376 (M⁺+1) |

### Example N-j-1

### Synthesis of ethyl 2-[5-benzylmethylamino-4-(naphthalen-2-yl)-2,3-dihydroinden-1-ylidene]acetate (Compound No. N-j-1) (Preparation Method 10, Step k)

According to the procedure described in the synthetic method of Intermediate A-2 in Reference Example 1 (Preparation Method 9, Step k-1), Intermediate n-d-2 (247.3 mg), ethyl diethylphosphonoacetate (1 ml, TCI) and sodium hydride (159.4 mg, WAKO) were reacted and treated to obtain the title compound (Compound No. N-j-1, 176.4 mg).

### Example N-j-2

### Synthesis of 2-[5-benzylmethylamino-4-(naphthalen-2-yl)-2,3-dihydroinden-1-ylidene]acetic acid (Compound No. N-j-2) (Preparation Method 1, Step a)

According to the procedure described in the synthetic method of Example Compound A-a-2 (Preparation Method 1, Step a) provided that the reaction was performed for 1 hour, Example Compound N-j-1 (56.4 mg) and 2 N aqueous sodium hydroxide (0.30 ml) were reacted and treated to obtain the title compound (Compound No. N-j-2, 21.3 mg).
Typical examples of the compounds of the present invention that can be obtained by reacting and treating corresponding starting compounds using any of the methods described in the present specification including the examples described above are shown in Table-N-J-1 to Table-N-J-3. The compounds were synthesized according to the preparation methods of the compounds of the compound numbers shown in the columns of "Syn" in the tables. "Int" means an intermediate compound number. As for the preparation of the compounds, used methods among the aforementioned synthetic methods are shown in the columns of "Syn" with symbols, the starting compounds are shown in the columns of "SM", and the reagents are shown in the columns of "Regent" in the tables.

**[Table 179]**

| Table-N-J-1 | | | | | | | | | | | |
|---|---|---|---|---|---|---|---|---|---|---|---|
| 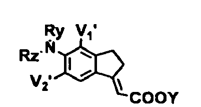 | | | | | | | | | | | |

| Exp. | Syn. | SM1 | Reagent | Ry | Rz | Y | V₁' | V₂' | LCMS | | |
|---|---|---|---|---|---|---|---|---|---|---|---|
| | | | | | | | | | method | RTime | Mass |
| N-j-1 | 10k-1 | Int.n-d-2 | | Bn | Me | Et | 2-Nap | H | C | | 448 (M⁺+1) |
| N₋j-2 | 1-a | N**-**j-1 | | Bn | Me | H | 2-Nap | H | C | | 420 (M⁺+1) |
| N-j-3 | 10k-1 | Int.n-d-4 | | nPr | Me | Et | 2-Nap | H | C | | 400 (M⁺+1) |
| N-j-4 | 1-a | N-j-3 | | nPr | Me | H | 2-Nap | H | C | | 372 (M⁺+1) |
| N-j-5 | 10k-1 | Int.n-d-5 | | iPr | Me | Et | 2-Nap | H | C | | 400 (M⁺+1) |
| N-j-6 | 1-a | N-j-5 | | iPr | Me | H | 2-Nap | H | C | | 372 (M⁺+1) |
| N-j-7 | 10k-1 | Int.n-d-6 | | nBu | Me | Et | 2-Nap | H | C | | 414 (M⁺+1) |
| N-j-8 | 1-a | N-j-7 | | nBu | Me | H | 2-Nap | H | C | | 386 (M⁺+1) |
| N-j-9 | 10k-1 | Int.n-d-7 | | iBu | Me | Et | 2-Nap | H | C | | 414 (M⁺+1) |
| N-j-10 | 1-a | N-j-9 | | iBu | Me | H | 2-Nap | H | C | | 386 (M⁺+1) |
| N-j-11 | 10k-1 | Int.n-d-8 | | cPen | Me | Et | 2-Nap | H | C | | 426 (M⁺+1) |
| N-j-12 | 1-a | N-j-11 | | cPen | Me | H | 2-Nap | H | C | | 398 (M⁺+1) |
| N-j-13 | 10k-1 | Int.n-d-9 | | cHex | Me | Et | 2-Nap | H | C | | 440 (M⁺+1) |
| N-j-14 | 1-a | N-j-13 | | cHex | Me | H | 2-Nap | H | C | | 412 (M⁺+1) |
| N-j-15 | 10k-1 | Int.n-d-10 | | Bn | Me | Et | 5-Ind | H | C | | 437 (M⁺+1) |
| N-j-16 | 1-a | N-j-15 | | Bn | Me | H | 5-Ind | H | C | | 409 (M⁺+1) |
| N-j-17 | 10k-1 | Int.n-d-12 | | nPr | Me | Et | 5-Ind | H | C | | 389 (M⁺+1) |
| N-j-18 | 1-a | N-J-17 | | nPr | Me | H | 5-Ind | H | C | | 361 (M⁺+1) |
| N-j-19 | 10k-1 | Int.n-d-13 | | iPr | Me | Et | 5-Ind | H | C | | 389 (M⁺+1) |
| N-j-20 | 1-a | N-j-19 | | iPr | Me | H | 5-Ind | H | C | | 361 (M⁺+1) |
| N-j-21 | 10k-1 | Int.n-d-14 | | nBu | Me | Et | 5-Ind | H | C | | 403 (M⁺+1) |
| N-j-22 | 1-a | N-j-21 | | nBu | Me | H | 5-Ind | H | C | | 375 (M⁺+1) |
| N-j-23 | 10k-1 | Int.n-d-15 | | iBu | Me | Et | 5-Ind | H | C | | 403 (M⁺+1) |
| N-j-24 | 1-a | N-j-23 | | iBu | Me | H | 5-Ind | H | C | | 375 (M⁺+1) |
| N-j-25 | 10k-1 | Int.n-d-16 | | cPen | Me | Et | 5-Ind | H | C | | 415 (M⁺+1) |
| N-j-26 | 1-a | N-j-25 | | cPen | Me | H | 5-Ind | H | C | | 387 (M⁺+1) |
| N-j-27 | 10k-1 | Int.n-d-17 | | cHex | Me | Et | 5-Ind | H | C | | 429 (M⁺+1) |
| N-j-28 | 1-a | N-j-27 | | cHex | Me | H | 5-Ind | H | C | | 401 (M⁺+1) |
| N-j-29 | 10k-1 | Int.n-d-18 | | Bn | Me | Et | 1Me-5-Ind | H | C | | 451 (M⁺+1) |
| N-j-30 | 1-a | N-j-29 | | Bn | Me | H | 1Me-5-Ind | H | C | | 423 (M⁺+1) |
| N-j-31 | 10k-1 | Int.n-d-20 | | nPr | Me | Et | 1Me-5-Ind | H | C | | 403 (M⁺+1) |
| N-j-32 | 1-a | N-j-31 | | nPr | Me | H | 1Me-5-Ind | H | C | | 375 (M⁺+1) |
| N-j-33 | 10k-1 | Int.n-d-21 | | iBu | Me | Et | 1Me-5-Ind | H | C | | 417 (M⁺+1) |
| N-j-34 | 1-a | N-j-33 | | iBu | Me | H | 1Me-5-Ind | H | C | | 389 (M⁺+1) |
| N-j-35 | 10k-1 | Int.n-d-22 | | cPen | Me | Et | 1Me-5-Ind | H | C | | 429 (M⁺+1) |
| N-j-36 | 1-a | N-j-35 | | cPen | Me | H | 1Me-5-Ind | H | C | | 401 (M⁺+1) |
| N-j-37 | 10k-1 | Int.n-d-23 | | cHex | Me | Et | 1Me-5-Ind | H | C | | 443 (M⁺+1) |
| N-j-38 | 1-a | N-j-37 | | cHex | Me | H | 1Me-5-Ind | H | C | | 415 (M⁺+1) |
| N-j-39 | 10k-1 | Int.n-d-24 | | Bn | Me | Et | 1Et-5-Ind | H | C | | 465 (M⁺+1) |
| N-j-40 | 1-a | N-j-39 | | Bn | Me | H | 1Et-5-Ind | H | C | | 437 (M⁺+1) |
| N-j-41 | 10k-1 | Int.n-d-26 | | iPr | Me | Et | 1Et-5-Ind | H | C | | 417 (M⁺+1) |
| N-j-42 | 1-a | N-j-41 | | iPr | Me | H | 1Et-5-Ind | H | C | | 389 (M⁺+1) |
| N-j-43 | 10k-1 | Int.n-d-27 | | nBu | Me | Et | 1Et-5-Ind | H | C | | 431 (M⁺+1) |
| N-j-44 | 1-a | N-j-43 | | nBu | Me | H | 1Et-5-Ind | H | C | | 403 (M⁺+1) |

**[Table 180]**

| Table-N-J-2 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-j-45 | 10k-1 | Int.n-d-28 | | iBu | Me | Et | 1Et-5-Ind | H | C | | 431 (M⁺+1) |
| N-j-46 | 1-a | N-j-45 | | iBu | Me | H | 1Et-5-Ind | H | C | | 403 (M⁺+1) |
| N-j-47 | 10k-1 | Int.n-d-29 | | cPen | Me | Et | 1Et-5-Ind | H | C | | 443 (M⁺+1) |
| N-j-48 | 1-a | N-j-47 | | cPen | Me | H | 1Et-5-Ind | H | C | | 415 (M⁺+1) |
| N-j-49 | 10k-1 | Int.n-d-30 | | Bn | Me | Et | 1Me-5-Idz | H | C | | 452 (M⁺+1) |
| N-j-50 | 1-a | N-j-49 | | Bn | Me | H | 1Me-5-Idz | H | C | | 424 (M⁺+1) |
| N-j-51 | 10k-1 | Int.n-d-32 | | nPr | Me | Et | 1Me-5-Idz | H | C | | 404 (M⁺+1) |
| N-j-52 | 1-a | N-j-51 | | nPr | Me | H | 1Me-5-Idz | H | C | | 376 (M⁺+1) |
| N-j-53 | 10k-1 | Int.n-d-33 | | iPr | Me | Et | 1Me-5-Idz | H | C | | 404 (M⁺+1) |
| N-j-54 | 1-a | N-j-53 | | iPr | Me | H | 1Me-5-Idz | H | C | | 376 (M⁺+1) |
| N-j-55 | 10k-1 | Int.n-d-34 | | nBu | Me | Et | 1Me-5-Idz | H | C | | 418 (M⁺+1) |
| N-j-56 | 1-a | N-j-55 | | nBu | Me | H | 1Me-5-Idz | H | C | | 390 (M⁺+1) |
| N-j-57 | 10k-1 | Int.n-d-35 | | iBu | Me | Et | 1Me-5-Idz | H | C | | 418 (M⁺+1) |
| N-j-58 | 1-a | N-j-57 | | iBu | Me | H | 1Me-5-Idz | H | C | | 390 (M⁺+1) |
| N-j-59 | 10k-1 | Int.n-d-36 | | cPen | Me | Et | 1Me-5-Idz | H | C | | 430 (M⁺+1) |
| N-j-60 | 1-a | N-j-59 | | cPen | Me | H | 1Me-5-Idz | H | C | | 402 (M⁺+1) |
| N-j-61 | 10k-1 | Int.n-d-37 | | cHex | Me | Et | 1Me-5-Idz | H | C | | 444 (M⁺+1) |
| N-j-62 | 1-a | N-j-61 | | cHex | Me | H | 1Me-5-Idz | H | C | | 416 (M⁺+1) |
| N-j-63 | 10k-1 | Int.n-d-38 | | Bn | Me | Et | 1Et-5-Idz | H | C | | 466 (M⁺+1) |
| N-j-64 | 1-a | N-j-63 | | Bn | Me | H | 1Et-5-Idz | H | C | | 438 (M⁺+1) |
| N-j-65 | 10k-1 | Int.n-d-40 | | nPr | Me | Et | 1Et-5-Idz | H | C | | 418 (M⁺+1) |
| N-j-66 | 1-a | N-j-65 | | nPr | Me | H | 1Et-5-Idz | H | C | | 390 (M⁺+1) |
| N-j-67 | 10k-1 | Int.n-d-41 | | iPr | Me | Et | 1Et-5-Idz | H | C | | 418 (M⁺+1) |
| N-j-68 | 1-a | N-j-67 | | iPr | Me | H | 1Et-5-Idz | H | C | | 390 (M⁺+1) |
| N-j-69 | 10k-1 | Int.n-d-42 | | cHex | Me | Et | 1Et-5-Idz | H | C | | 458 (M⁺+1) |
| N-j-70 | 1-a | N-j-69 | | cHex | Me | H | 1Et-5-Idz | H | C | | 430 (M⁺+1) |
| N-j-71 | 10k-1 | Int.n-d-43 | | Bn | Et | Et | 2-Nap | H | C | | 462 (M⁺+1) |
| N-j-72 | 1-a | N-j-71 | | Bn | Et | H | 2-Nap | H | C | | 434 (M⁺+1) |
| N-j-73 | 10k-1 | Int.n-d-45 | | nPr | Et | Et | 2-Nap | H | C | | 414 (M⁺+1) |
| N-j-74 | 1-a | N-j-73 | | nPr | Et | H | 2-Nap | H | C | | 386 (M⁺+1) |
| N-j-75 | 10k-1 | Int.n-d-46 | | iPr | Et | Et | 2-Nap | H | C | | 414 (M⁺+1) |
| N-j-76 | 1-a | N-j-75 | | iPr | Et | H | 2-Nap | H | C | | 386 (M⁺+1) |
| N-j-77 | 10k-1 | Int.n-d-47 | | nBu | Et | Et | 2-Nap | H | C | | 428 (M⁺+1) |
| N-j-78 | 1-a | N-j-77 | | nBu | Et | H | 2-Nap | H | C | | 400 (M⁺+1) |
| N-j-79 | 10k-1 | Int.n-d-48 | | iBu | Et | Et | 2-Nap | H | C | | 428 (M⁺+1) |
| N-j-80 | 1-a | N-j-79 | | iBu | Et | H | 2-Nap | H | C | | 400 (M⁺+1) |
| N-j-81 | 10k-1 | Int.n-d-49 | | cPen | Et | Et | 2-Nap | H | C | | 440 (M⁺+1) |
| N-j-82 | 1-a | N-j-81 | | cPen | Et | H | 2-Nap | H | C | | 412 (M⁺+1) |
| N-j-83 | 10k-1 | Int.n-d-50 | | cHex | Et | Et | 2-Nap | H | C | | 454 (M⁺+1) |
| N-j-84 | 1-a | N-j-83 | | cHex | Et | H | 2-Nap | H | C | | 426 (M⁺+1) |
| N-j-85 | 10k-1 | Int.n-d-51 | | Bn | Et | Et | 5-Ind | H | C | | 451 (M⁺+1) |
| N-j-86 | 1-a | N-j-85 | | Bn | Et | H | 5-Ind | H | C | | 423 (M⁺+1) |
| N-j-87 | 10k-1 | Int.n-d-53 | | nPr | Et | Et | 5-Ind | H | C | | 403 (M⁺+1) |
| N-j-88 | 1-a | N-j-87 | | nPr | Et | H | 5-Ind | H | C | | 375 (M⁺+1) |
| N-j-89 | 10k-1 | Int.n-d-54 | | iPr | Et | Et | 5-Ind | H | C | | 403 (M⁺+1) |
| N-j-90 | 1-a | N-j-89 | | iPr | Et | H | 5-Ind | H | C | | 375 (M⁺+1) |
| N-j-91 | 10k-1 | Int.n-d-55 | | nBu | Et | Et | 5-Ind | H | C | | 417 (M⁺+1) |
| N-j-92 | 1-a | N-j-91 | | nBu | Et | H | 5-Ind | H | C | | 389 (M⁺+1) |

**[Table 181]**

| Table-N-J-3 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-j-93 | 10k-1 | Int.n-d-56 | | iBu | Et | Et | 5-Ind | H | C | | 417 (M⁺+1) |
| N-j-94 | 1-a | N-j-93 | | iBu | Et | H | 5-Ind | H | C | | 389 (M⁺+1) |
| N-j-95 | 10k-1 | Int.n-d-57 | | cPen | Et | Et | 5-Ind | H | C | | 429 (M⁺+1) |
| N-j-96 | 1-a | N-j-95 | | cPen | Et | H | 5-Ind | H | C | | 401 (M⁺+1) |
| N-j-97 | 10k-1 | Int.n-d-58 | | Bn | Et | Et | 1Me-5-Ind | H | C | | 465 (M⁺+1) |
| N-j-98 | 1-a | N-j-97 | | Bn | Et | H | 1Me-5-Ind | H | C | | 437 (M⁺+1) |
| N-j-99 | 10k-1 | Int.n-d-60 | | nPr | Et | Et | 1Me-5-Ind | H | C | | 417 (M⁺+1) |
| N-j-100 | 1-a | N-j-99 | | nPr | Et | H | 1Me-5-Ind | H | C | | 389 (M⁺+1) |
| N-j-101 | 10k-1 | Int.n-d-61 | | iPr | Et | Et | 1Me-5-Ind | H | C | | 417 (M⁺+1) |
| N-j-102 | 1-a | N-j-101 | | iPr | Et | H | 1Me-5-Ind | H | C | | 389 (M⁺+1) |
| N-j-103 | 10k-1 | Int.n-d-62 | | nBu | Et | Et | 1Me-5-Ind | H | C | | 431 (M⁺+1) |
| N-j-104 | 1-a | N-j-103 | | nBu | Et | H | 1Me-5-Ind | H | C | | 403 (M⁺+1) |
| N-j-105 | 10k-1 | Int.n-d-63 | | iBu | Et | Et | 1Me-5-Ind | H | C | | 431 (M⁺+1) |
| N-j-106 | 1-a | N-j-105 | | iBu | Et | H | 1Me-5-Ind | H | C | | 403 (M⁺+1) |
| N-j-107 | 10k-1 | Int.n-d-64 | | cPen | Et | Et | 1Me-5-Ind | H | C | | 443 (M⁺+1) |
| N-j-108 | 1-a | N-j-107 | | cPen | Et | H | 1Me-5-Ind | H | C | | 415 (M⁺+1) |
| N-j-109 | 10k-1 | Int.n-d-65 | | cHex | Et | Et | 1Me-5-Ind | H | C | | 457 (M⁺+1) |
| N-j-110 | 1-a | N-j-109 | | cHex | Et | H | 1Me-5-Ind | H | C | | 429 (M⁺+1) |
| N-j-111 | 10k-1 | Int.n-d-66 | | Bn | Et | Et | 1Et-5-Ind | H | C | | 479 (M⁺+1) |
| N-j-112 | 1-a | N-j-111 | | Bn | Et | H | 1Et-5-Ind | H | C | | 451 (M⁺+1) |
| N-j-113 | 10k-1 | Int.n-d-68 | | iPr | Et | Et | 1Et-5-Ind | H | C | | 431 (M⁺+1) |
| N-j-114 | 1-a | N-j-113 | | iPr | Et | H | 1Et-5-Ind | H | C | | 403 (M⁺+1) |
| N-j-115 | 10k-1 | Int.n-d-69 | | nBu | Et | Et | 1Et-5-Ind | H | C | | 445 (M⁺+1) |
| N-j-116 | 1-a | N-j-115 | | nBu | Et | H | 1Et-5-Ind | H | C | | 417 (M⁺+1) |
| N-j-117 | 10k-1 | Int.n-d-70 | | cPen | Et | Et | 1Et-5-Ind | H | C | | 457 (M⁺+1) |
| N-j-118 | 1-a | N-j-117 | | cPen | Et | H | 1Et-5-Ind | H | C | | 429 (M⁺+1) |
| N-j-119 | 10k-1 | Int.n-d-71 | | Bn | Et | Et | 1Me-5-Idz | H | C | | 466 (M⁺+1) |
| N-j-120 | 1-a | N-j-119 | | Bn | Et | H | 1Me-5-Idz | H | C | | 438 (M⁺+1) |
| N-j-121 | 10k-1 | Int.n-d-73 | | nPr | Et | Et | 1Me-5-Idz | H | C | | 418 (M⁺+1) |
| N-j-122 | 1-a | N-j-121 | | nPr | Et | H | 1Me-5-Idz | H | C | | 390 (M⁺+1) |
| N-j-123 | 10k-1 | Int.n-d-74 | | iPr | Et | Et | 1Me-5-Idz | H | C | | 418 (M⁺+1) |
| N-j-124 | 1-a | N-j-123 | | iPr | Et | H | 1Me-5-Idz | H | C | | 390 (M⁺+1) |
| N-j-125 | 10k-1 | Int.n-d-75 | | nBu | Et | Et | 1Me-5-Idz | H | C | | 432 (M⁺+1) |
| N-j-126 | 1-a | N-j-125 | | nBu | Et | H | 1Me-5-Idz | H | C | | 404 (M⁺+1) |
| N-j-127 | 10k-1 | Int.n-d-76 | | iBu | Et | Et | 1Me-5-Idz | H | C | | 432 (M⁺+1) |
| N-j-128 | 1-a | N-j-127 | | iBu | Et | H | 1Me-5-Idz | H | C | | 404 (M⁺+1) |
| N-j-129 | 10k-1 | Int.n-d-77 | | cPen | Et | Et | 1Me-5-Idz | H | C | | 444 (M⁺+1) |
| N-j-130 | 1-a | N-j-129 | | cPen | Et | H | 1Me-5-Idz | H | C | | 416 (M⁺+1) |
| N-j-131 | 10k-1 | Int.n-d-78 | | cHex | Et | Et | 1Me-5-Idz | H | C | | 458 (M⁺+1) |
| N-j-132 | 1-a | N-j-131 | | cHex | Et | H | 1Me-5-Idz | H | C | | 430 (M⁺+1) |
| N-j-133 | 10k-1 | Int.n-d-79 | | Bn | Et | Et | 1Et-5-Idz | H | C | | 480 (M⁺+1) |
| N-j-134 | 1-a | N-j-133 | | Bn | Et | H | 1Et-5-Idz | H | C | | 452 (M⁺+1) |
| N-j-135 | 10k-1 | Int.n-d-81 | | nPr | Et | Et | 1Et-5-Idz | H | C | | 432 (M⁺+1) |
| N-j-136 | 1-a | N-j-135 | | nPr | Et | H | 1Et-5-Idz | H | C | | 404 (M⁺+1) |
| N-j-137 | 10k-1 | Int.n-d-82 | | iPr | Et | Et | 1Et-5-Idz | H | C | | 432 (M⁺+1) |
| N-j-138 | 1-a | N-j-135 | | iPr | Et | H | 1Et-5-Idz | H | C | | 404 (m⁺+1) |
| N-j-139 | 10k-1 | Int.n-d-83 | | nBu | Et | Et | 1Et-5-Idz | H | C | | 446 (M⁺+1) |
| N-j-140 | 1-a | N-j-135 | | nBu | Et | H | 1Et-5-Idz | H | C | | 418 (M⁺+1) |

### Reference Example 18

### Synthesis of 4-bromo-5-(piperidin-1-yl)-2,3-dihydroinden-1-one (Intermediate n-e-1) (Preparation Method 13, Step h)

According to the procedure described in the synthetic method of Intermediate A-4 in Reference Example 1 (Preparation Method 8, Step h) provided that the reaction was performed at room temperature for 3 hours, Intermediate n-a-3 (986.4 mg) and NBS (781.3 mg, WAKO) were reacted and treated to obtain the title compound (Intermediate n-e-1, 1.183 g). Mass (LCMS): 294 (M⁺), Retention time: N.D (Elution condition: C).

### Synthesis of 4-(naphthalen-2-yl)-5-(piperidin-1-yl)-2,3-dihydroinden-1-one (Intermediate n-e-2) (Preparation Method 11, Step e-1)

According to the procedure described in the synthetic method of Example Compound A-a-1 (Preparation Method 4, Step e-1) provided that the reaction was performed for 16 hours, Intermediate n-e-1 (198.3 mg), 2-naphthaleneboronic acid (267.3 mg, TCI), 2 M aqueous sodium carbonate (0.8 ml) and (Ph₃P)₄Pd (109.2 mg, Nakalai Tesque) were reacted and treated to obtain the title compound (Intermediate n-e-2, 201.1 mg). Mass (LCMS): 342 (M⁺+1), Retention time: N.D (Elution condition: C).

Typical examples of the intermediates that can be obtained by reacting and treating corresponding starting compounds using any of the methods described in the present specification including the examples described above are shown in Table-Int.N-e-1 to Table-Int.N-e-4. The compounds were synthesized according to the preparation methods of the compounds of the compound numbers shown in the columns of "Syn" in the tables. "Int" means an intermediate compound number. In the tables, used methods among the aforementioned synthetic methods are shown in the columns of "Syn" with symbols, the starting compounds are shown in the columns of "SM", and the reagents are shown in the columns of "Regent". The boronic acid regents mentioned in the columns of "Reagent" with symbols "BRA (No.)" are those mentioned in Table-BRA.

**[Table 182]**

| Table-Int.N-e-1 | | | | | | | | | |
|---|---|---|---|---|---|---|---|---|---|
| 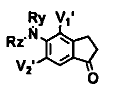 | | | | | | | | | |

| Exp. | Syn. | SM1 | Reagent | RyRz | V₁' | V₂' | LCMS | | |
|---|---|---|---|---|---|---|---|---|---|
| | | | | | | | method | RTime | Mass |
| Int.n-e-3 | 11e-1 | Int.n-e-1 | BRA2 | 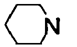 | 5-Ind | H | C | | 331 (M⁺+1) |
| Int.n-e-4 | 11e-1 | Int.n-e-1 | BRA3 | 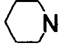 | 1Me-5-Ind | H | C | | 345 (M⁺+1) |
| Int.n-e-5 | 11e-1 | Int.n-e-1 | BRA4 | 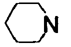 | 1 Et-5-Ind | H | C | | 359 (M⁺+1) |
| Int.n-e-6 | 11e-1 | Int.n-e-1 | BRA7 | 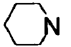 | 5-1Idz | H | C | | 332 (M⁺+1) |
| Int.n-e-7 | 11e-1 | Int.n-e-1 | BRA8 | 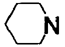 | 1 Me-5-Idz | H | C | | 346 (M⁺+1) |
| Int.n-e-8 | 11e-1 | Int.n-e-1 | BRA9 | 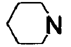 | 1Et-5-Idz | H | C | | 360 (M⁺+1) |
| Int.n-e-9 | 13-h | Int.n-a-4 | | 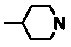 | Br | H | C | | 308 (M⁺) |
| Int.n-e-10 | 11e-1 | Int.n-e-8 | BRA1 | 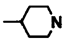 | 2-Nap | H | C | | 356 (M⁺+1) |
| Int.n-e-11 | 11e-1 | Int.n-e-8 | BRA3 | 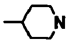 | 1 Me-5-Ind | H | C | | 359 (M⁺+1) |
| Int.n-e-12 | 11e-1 | Int.n-e-8 | BRA7 | 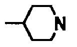 | 5-1 Idz | H | C | | 346 (M⁺+1) |
| Int.n-e-13 | 13-h | Int.n-a-5 | | 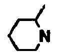 | Br | H | C | | 308 (M⁺) |
| Int.n-e-14 | 11e-1 | Int.n-e-13 | BRA1 | 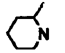 | 2-Nap | H | C | | 356 (M⁺+1) |
| Int.n-e-15 | 11e-1 | Int.n-e-13 | BRA2 | 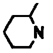 | 5-Ind | H | C | | 345 (M⁺+1) |
| Int.n-e-16 | 11e-1 | Int.n-e-13 | BRA3 | 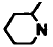 | 1 Me-5-Ind | H | C | | 359 (M⁺+1) |
| Int.n-e-17 | 11e-1 | Int.n-e-13 | BRA8 | 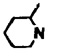 | 1 Me-5-Idz | H | C | | 360 (M⁺+1) |
| Int.n-e-18 | 11e-1 | Int.n-e-13 | BRA9 | 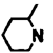 | 1Et-5-Idz | H | C | | 374 (M⁺+1) |
| Int.n-e-19 | 13-h | Int.n-a-6 | | 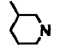 | Br | H | C | | 308 (M⁺) |
| Int.n-e-20 | 11e-1 | Int.n-e-19 | BRA1 | 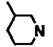 | 2-Nap | H | C | | 356 (M⁺+1) |
| Int.n-e-21 | 11e-1 | Int.n-e-19 | BRA2 | 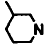 | 5-Ind | H | C | | 345 (M⁺+1) |
| Int.n-e-22 | 11e-1 | Int.n-e-19 | BRA7 | 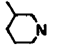 | 5-1Idz | H | C | | 346 (M⁺+1) |

**[Table 183]**

| Table-Int.N-e-2 | | | | | | | | | |
|---|---|---|---|---|---|---|---|---|---|
| Exp. | Syn. | SM1 | Reagent | RyRz | V₁' | V₂' | LCMS | | |
| | | | | | | | method | RTime | Mass |
| Int.n-e-23 | 11e-1 | Int.n-e-19 | BRA8 | 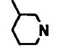 | 1Me-5-Idz | H | C | | 360 (M⁺+1) |
| Int.n-e-24 | 13-h | Int.n-a-7 | | 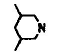 | Br | H | C | | 322 (M⁺) |
| Int.n-e-25 | 11e-1 | Int.n-e-24 | BRA1 | 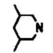 | 2-Nap | H | C | | 370 (M⁺+1) |
| Int.n-e-26 | 11e-1 | Int.n-e-24 | BRA3 | 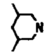 | 1Me-5-Ind | H | C | | 373 (M⁺+1) |
| Int.n-e-27 | 11e-1 | Int.n-e-24 | BRA8 | 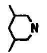 | 1Me-5-Idz | H | C | | 374 (M⁺+1) |
| Int.n-e-28 | 11e-1 | Int.n-e-24 | BRA9 | 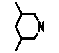 | 1Et-5-Idz | H | C | | 388 (M⁺+1) |
| Int.n-e-29 | 13-h | Int.n-a-8 | | 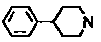 | Br | H | C | | 370 (M⁺) |
| Int.n-e-30 | 11e-1 | Int.n-e-29 | BRA1 | 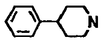 | 2-Nap | H | C | | 418 (M⁺+1) |
| Int.n-e-31 | 11e-1 | Int.n-e-29 | BRA2 | 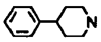 | 5-Ind | H | C | | 407 (M⁺+1) |
| Int.n-e-32 | 11e-1 | Int.n-e-29 | BRA3 | 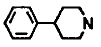 | 1Me-5-Ind | H | C | | 421 (M⁺+1) |
| Int.n-e-33 | 11e-1 | Int.n-e-29 | BRA7 | 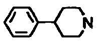 | 5-1Idz | H | C | | 408 (M⁺+1) |
| Int.n-e-34 | 11e-1 | Int.n-e-29 | BRA8 | 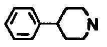 | 1Me-5-Idz | H | C | | 422 (M⁺+1) |
| Int.n-e-35 | 13-h | Int.n-a-9 | | 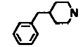 | Br | H | C | | 384 (M⁺) |
| Int.n-e-36 | 11e-1 | Int.n-e-35 | BRA1 | 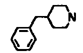 | 2-Nap | H | C | | 432 (M⁺+1) |
| Int.n-e-37 | 11e-1 | Int.n-e-35 | BRA2 | 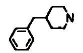 | 5-Ind | H | C | | 421 (M⁺+1) |
| Int.n-e-38 | 11e-1 | Int.n-e-35 | BRA7 | 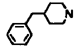 | 5-1Idz | H | C | | 422 (M⁺+1) |
| Int.n-e-39 | 13-h | Int.n-a-10 | | 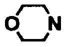 | Br | H | C | | 296 (M⁺) |
| Int.n-e-40 | 11e-1 | Int.n-e-39 | BRA1 | 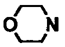 | 2-Nap | H | C | | 344 (M⁺+1) |
| Int.n-e-41 | 11e-1 | Int.n-e-39 | BRA2 | 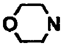 | 5-Ind | H | C | | 333 (M⁺+1) |
| Int.n-e-42 | 11e-1 | Int.n-e-39 | BRA3 | 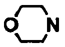 | 1 Me-5-Ind | H | C | | 347 (M⁺+1) |
| Int.n-e-43 | 11e-1 | Int.n-e-39 | BRA7 | 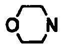 | 5-1Idz | H | C | | 334 (M⁺+1) |
| Int.n-e-44 | 11e-1 | Int.n-e-39 | BRA8 | 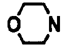 | 1Me-5-Idz | H | C | | 348 (M⁺+1) |
| Int.n-e-45 | 11e-1 | Int.n-e-39 | BRA9 | 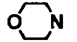 | 1Et-5-Idz | H | C | | 362 (M⁺+1) |
| Int.n-e-46 | 13-h | Int.n-a-11 | | | Br | H | C | | 324 (M⁺) |

**[Table 184]**

| Table-Int.N-e-3 | | | | | | | | | |
|---|---|---|---|---|---|---|---|---|---|
| Exp. | Syn. | SM1 | Reagent | RyRz | V₁' | V₂' | LCMS | | |
| | | | | | | | method | RTime | Mass |
| Int.n-e-47 | 11e-1 | Int.n-e-46 | BRA1 | 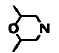 | 2-Nap | H | C | | 372 (M⁺+1) |
| Int.n-e-48 | 11e-1 | Int.n-e-46 | BRA2 | 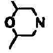 | 5-Ind | H | C | | 361 (M⁺+1) |
| Int.n-e-49 | 11e-1 | Int.n-e-46 | BRA3 | 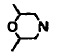 | 1Me-5-Ind | H | C | | 375 (M⁺+1) |
| Int.n-e-50 | 11e-1 | Int.n-e-46 | BRA7 | 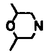 | 5-1Idz | H | C | | 362 (M⁺+1) |
| Int.n-e-51 | 11e-1 | Int.n-e-46 | BRA8 | 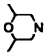 | 1Me-5-Idz | H | C | | 376 (M⁺+1) |
| Int.n-e-52 | 13-h | Int.n-a-12 | | 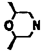 | Br | H | C | | 324 (M⁺) |
| Int.n-e-53 | 11e-1 | Int.n-e-52 | BRA2 | 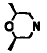 | 5-Ind | H | C | | 361 (M⁺+1) |
| Int.n-e-54 | 11e-1 | Int.n-e-52 | BRA3 | 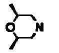 | 1Me-5-Ind | H | C | | 375 (M⁺+1) |
| Int.n-e-55 | 11e-1 | Int.n-e-52 | BRA8 | 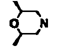 | 1Me-5-Idz | H | C | | 376 (M⁺+1) |
| Int.n-e-56 | 13-h | Int.n-a-13 | | 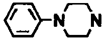 | Br | H | C | | 371 (M⁺) |
| Int.n-e-57 | 11e-1 | Int.n-e-56 | BRA1 | 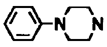 | 2-Nap | H | C | | 419 (M⁺+1) |
| Int.n-e-58 | 11e-1 | Int.n-e-56 | BRA2 | 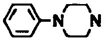 | 5-Ind | H | C | | 408 (M⁺+1) |
| Int.n-e-59 | 11e-1 | Int.n-e-56 | BRA3 | 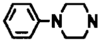 | 1Me-5-Ind | H | C | | 422 (M⁺+1) |
| Int.n-e-60 | 11e-1 | Int.n-e-56 | BRA7 | 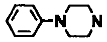 | 5-1Idz | H | C | | 409 (M⁺+1) |
| Int.n-e-61 | 11e-1 | Int.n-e-56 | BRA8 | 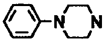 | 1Me-5-Idz | H | C | | 423 (M⁺+1) |
| Int.n-e-62 | 13-h | Int.n-a-14 | | 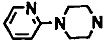 | Br | H | C | | 372 (M⁺) |
| Int.n-e-63 | 11e-1 | Int.n-e-62 | BRA1 | 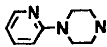 | 2-Nap | H | C | | 420 (M⁺+1) |
| Int.n-e-64 | 11e-1 | Int.n-e-62 | BRA3 | 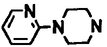 | 1Me-5-Ind | H | C | | 423 (M⁺+1) |
| Int.n-e-65 | 11e-1 | Int.n-e-62 | BRA8 | 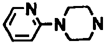 | 1Me-5-Idz | H | C | | 424 (M⁺+1) |
| Int.n-e-66 | 11e-1 | Int.n-e-62 | BRA9 | 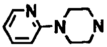 | 1Et-5-Idz | H | C | | 438 (M⁺+1) |
| Int.n-e-67 | 13-h | Int.n-a-15 | | 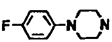 | Br | H | C | | 389 (M⁺) |
| Int.n-e-68 | 11e-1 | Int.n-e-67 | BRA1 | 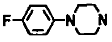 | 2-Nap | H | C | | 437 (M⁺+1) |
| Int.n-e-69 | 11e-1 | Int.n-e-67 | BRA2 | 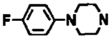 | 5-Ind | H | C | | 426 (M⁺+1) |
| Int.n-e-70 | 11e-1 | Int.n-e-67 | BRA4 | 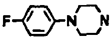 | 1Et-5-Ind | H | C | | 454 (M⁺+1) |

**[Table 185]**

| Table-Int.N-e-4 | | | | | | | | | |
|---|---|---|---|---|---|---|---|---|---|
| Exp. | Syn. | SM1 | Reagent | RyRz | V₁' | V₂' | LCMS | | |
| | | | | | | | method | RTime | Mass |
| Int.n-e-71 | 11e-1 | Int.n-e-67 | BRA7 | 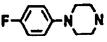 | 5-1Idz | H | C | | 427 (M⁺+1) |
| Int.n-e-72 | 11e-1 | Int.n-e-67 | BRA8 | 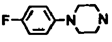 | 1 Me-5-Idz | H | C | | 441 (M⁺+1) |
| Int.n-e-73 | 13-h | Int.n-a-16 | | 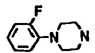 | Br | H | C | | 389 (M⁺) |
| Int.n-e-74 | 11e-1 | Int.n-e-73 | BRA1 | 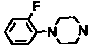 | 2-Nap | H | C | | 437 (M⁺+1) |
| Int.n-e-75 | 11e-1 | Int.n-e-73 | BRA2 | 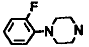 | 5-Ind | H | C | | 426 (M⁺+1) |
| Int.n-e-76 | 11e-1 | Int.n-e-73 | BRA4 | 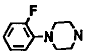 | 1Et-5-Ind | H | C | | 454 (M⁺+1) |
| Int.n-e-77 | 11e-1 | Int.n-e-73 | BRA8 | 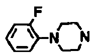 | 1Me-5-Idz | H | C | | 441 (M⁺+1) |
| Int.n-e-78 | 11e-1 | Int.n-e-73 | BRA9 | 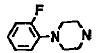 | 1Et-5-Idz | H | C | | 455 (M⁺+1) |
| Int.n-e-79 | 13-h | Int.n-a-17 | | 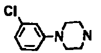 | Br | H | C | | 405 (M⁺) |
| Int.n-e-80 | 11e-1 | Int.n-e-79 | BRA1 | 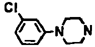 | 2-Nap | H | C | | 453 (M⁺+1) |
| Int.n-e-81 | 11e-1 | Int.n-e-79 | BRA2 | 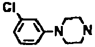 | 5-Ind | H | C | | 442 (M⁺+1) |
| Int.n-e-82 | 11e-1 | Int.n-e-79 | BRA3 | 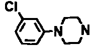 | 1 Me-5-Ind | H | C | | 456 (M⁺+1) |
| Int.n-e-83 | 11e-1 | Int.n-e-79 | BRA7 | 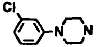 | 5-1Idz | H | C | | 443 (M⁺+1) |
| Int.n-e-84 | 11e-1 | Int.n-e-79 | BRA8 | 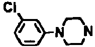 | 1Me-5-Idz | H | C | | 457 (M⁺+1) |
| Int.n-e-85 | 13-h | Int.n-a-18 | | 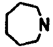 | Br | H | C | | 308 (M⁺) |
| Int.n-e-86 | 11e-1 | Int.n-a-85 | BRA1 | 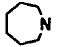 | 2-Nap | H | C | | 356 (M⁺+1) |
| Int.n-e-87 | 11e-1 | Int.n-a-85 | BRA2 | 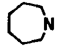 | 5-Ind | H | C | | 345 (M⁺+1) |
| Int.n-e-88 | 11e-1 | Int.n-a-85 | BRA3 | 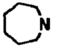 | 1Me-5-Ind | H | C | | 359 (M⁺+1) |
| Int.n-e-89 | 11e-1 | Int.n-a-85 | BRA7 | 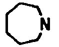 | 5-1Idz | H | C | | 346 (M⁺+1) |
| Int.n-e-90 | 11e-1 | Int.n-a-85 | BRA8 | 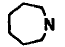 | 1Me-5-Idz | H | C | | 360 (M⁺+1) |

### Example N-k-1

### Synthesis of ethyl 2-[4-(naphthalen-2-yl)-5-(piperidin-1-yl)-2,3-dihydroylidene]acetate (Compound No. N-k-1) (Preparation Method 10, Step k-1)

According to the procedure described in the synthetic method of Intermediate A-2 in Reference Example 1 (Preparation Method 9, Step k-1), Intermediate n-e-2 (198.3 mg), ethyl diethylphosphonoacetate (1.2 ml, TCI) and sodium hydride (160.1 mg, WAKO) were reacted and treated to obtain the title compound (Compound No. N-k-1, 112.2 mg).

### Example N-k-2

### Synthesis of 2-[4-(naphthalen-2-yl)-5-(piperidin-1-yl)-2,3-dihydro-ylidene]acetic acid (Compound No. N-e-2) (Preparation Method 1, Step a)

According to the procedure described in the synthetic method of Example Compound A-a-2 (Preparation Method 1, Step a) provided that the reaction was performed for 1 hour, Example Compound N-k-1 (103.4 mg) and 2 N aqueous sodium hydroxide (0.70 ml) were reacted and treated to obtain the title compound (Compound No. N-k-2, 89.3 mg).

Typical examples of the compounds of the present invention that can be obtained by reacting and treating corresponding starting compounds using any of the methods described in the present specification including the examples described above are shown in Table-N-K-1 to Table-N-K-7. The compounds were synthesized according to the preparation methods of the compounds of the compound numbers shown in the columns of "Syn" in the tables. "Int" means an intermediate compound number. As for the preparation of the compounds, used methods among the aforementioned synthetic methods are shown in the columns of "Syn" with symbols, the starting compounds are shown in the columns of "SM", and the reagents are shown in the columns of "Regent" in the tables.

**[Table 186]**

| Table-N-K-1 | | | | | | | | | | |
|---|---|---|---|---|---|---|---|---|---|---|
| 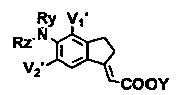 | | | | | | | | | | |

| Exp. | Syn. | SM1 | Reagent | NRzRy | Y | V₁' | V₂' | LCMS | | |
|---|---|---|---|---|---|---|---|---|---|---|
| | | | | | | | | method | RTime | Mass |
| N-k-1 | 10k-1 | Int.n-e-2 | | 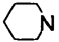 | Et | 2-Nap | H | C | | 412 (M⁺+1) |
| N-k-2 | 1-a | N-k-1 | | 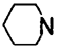 | H | 2-Nap | H | C | | 384 (M⁺+1) |
| N-k-3 | 10k-1 | Int.n-e-3 | | 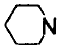 | Et | 5-Ind | H | C | | 401 (M⁺+1) |
| N-k-4 | 1-a | N-k-3 | | 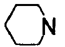 | H | 5-Ind | H | C | | 373 (M⁺+1) |
| N-k-5 | 10k-1 | Int.n-e-4 | | 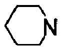 | Et | 1 Me-5-Ind | H | C | | 415 (M⁺+1) |
| N-k-6 | 1-a | N-k-5 | | 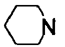 | H | 1Me-5-Ind | H | C | | 387 (M⁺+1) |
| N-k-7 | 10k-1 | Int.n-e-5 | | 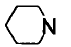 | Et | 1 Et-5-Ind | H | C | | 429 (M⁺+1) |
| N-k-8 | 1-a | N-k-7 | | 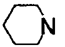 | H | 1Et-5-Ind | H | C | | 401 (M⁺+1) |
| N-k-9 | 10k-1 | Int.n-e-6 | | 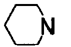 | Et | 5-1Idz | H | C | | 402 (M⁺+1) |
| N-k-10 | 1-a | N-k-9 | | 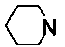 | H | 5-1Idz | H | C | | 374 (M⁺+1) |
| N-k-11 | 10k-1 | Int.n-e-7 | | 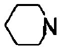 | Et | 1Me-5-Idz | H | C | | 416 (M⁺+1) |
| N-k-12 | 1-a | N-k-11 | | 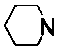 | H | 1Me-5-Idz | H | C | | 388 (M⁺+1) |
| N-k-13 | 10k-1 | Int.n-e-8 | | 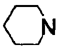 | Et | 1Et-5-Idz | H | C | | 430 (M⁺+1) |
| N-k-14 | 1-a | N-k-13 | | 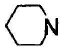 | H | 1Et-5-Idz | H | C | | 402 (M⁺+1) |
| N-k-15 | 10k-1 | Int.n-e-10 | | 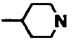 | Et | 2-Nap | H | C | | 426 (M⁺+1) |
| N-k-16 | 1-a | N-k-15 | | 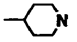 | H | 2-Nap | H | C | | 398 (M⁺+1) |
| N-k-17 | 10k-1 | Int.n-e-11 | | 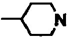 | Et | 1Me-5-Ind | H | C | | 429 (M⁺+1) |
| N-k-18 | 1-a | N-k-17 | | 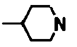 | H | 1Me-5-Ind | H | C | | 401 (M⁺+1) |
| N-k-19 | 10k-1 | Int,n-e-12 | | 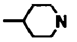 | Et | 5-1Idz | H | C | | 416 (M⁺+1) |
| N-k-20 | 1-a | N-k-19 | | 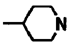 | H | 5-1Idz | H | C | | 388 (M⁺+1) |
| N-k-21 | 10k-1 | Int.n-e-14 | | 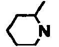 | Et | 2-Nap | H | C | | 426 (M⁺+1) |
| N-k-22 | 1-a | N-k-21 | | 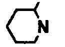 | H | 2-Nap | H | C | | 398 (M⁺+1) |

**[Table 187]**

| Table-N-K-2 | | | | | | | | | | |
|---|---|---|---|---|---|---|---|---|---|---|
| Exp. | Syn. | SM1 | Reagent | NRzRy | Y | V₁' | V₂' | LCMS | | |
| | | | | | | | | method | RTime | Mass |
| N-k-23 | 10k-1 | Int.n-e-15 | | 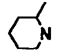 | Et | 5-Ind | H | C | | 415 (M⁺+1) |
| N-k-24 | 1-a | N-k-23 | | 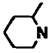 | H | 5-Ind | H | C | | 387 (M⁺+1) |
| N-k-25 | 10k-1 | Int.n-e-16 | | 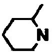 | Et | 1Me-5-Ind | H | C | | 429 (M⁺+1) |
| N-k-26 | 1-a | N-k-25 | | 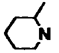 | H | 1Me-5-Ind | H | C | | 401 (M⁺+1) |
| N-k-27 | 10k-1 | Int.n-e-17 | | 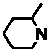 | Et | 1Me-5-Idz | H | C | | 430 (M⁺+1) |
| N-k-28 | 1-a | N-k-27 | | 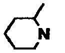 | H | 1Me-5-Idz | H | C | | 402 (M⁺+1) |
| N-k-29 | 10k-1 | Int.n-e-18 | | 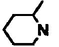 | Et | 1Et-5-Idz | H | C | | 444 (M⁺+1) |
| N-k-30 | 1-a | N-k-29 | | 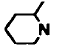 | H | 1Et-5-Idz | H | C | | 416 (M⁺+1) |
| N-k-31 | 10k-1 | Int.n-e-20 | | 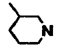 | Et | 2-Nap | H | C | | 426 (M⁺+1) |
| N-k-32 | 1-a | N-k-31 | | 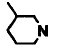 | H | 2-Nap | H | C | | 398 (M⁺+1) |
| N-k-33 | 10k-1 | Int.n-e-21 | | 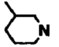 | Et | 5-Ind | H | C | | 415 (M⁺+1) |
| N-k-34 | 1-a | N-k-33 | | 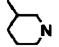 | H | 5-Ind | H | C | | 387 (M⁺+1) |
| N-k-35 | 10k-1 | Int.n-e-22 | | 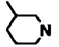 | Et | 5-1Idz | H | C | | 416 (M⁺+1) |
| N-k-36 | 1-a | N-k-35 | | 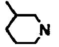 | H | 5-1Idz | H | C | | 388 (M⁺+1) |
| N-k-37 | 10k-1 | Intn-e-23 | | 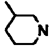 | Et | 1Me-5-Idz | H | C | | 430 (M⁺+1) |
| N-k-38 | 1-a | N-k-37 | | 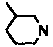 | H | 1Me-5-Idz | H | C | | 402 (M⁺+1) |
| N-k-39 | 10k-1 | Intn-e-25 | | 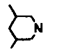 | Et | 2-Nap | H | C | | 440 (M⁺+1) |
| N-k-40 | 1-a | N-k-39 | | 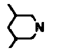 | H | 2-Nap | H | C | | 412 (M⁺+1) |
| N-k-41 | 10k-1 | Int.n-e-26 | | 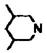 | Et | 1Me-5-Ind | H | C | | 443 (M⁺+1) |
| N-k-42 | 1-a | N-k-41 | | 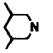 | H | 1Me-5-Ind | H | C | | 415 (M⁺+1) |
| N-k-43 | 10k-1 | Intn-e-27 | | 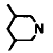 | Et | 1Me-5-Idz | H | C | | 444 (M⁺+1) |
| N-k-44 | 1-a | N-k-43 | | 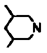 | H | 1Me-5-Idz | H | C | | 416 (M⁺+1) |

**[Table 188]**

| Table-N-K-3 | | | | | | | | | | |
|---|---|---|---|---|---|---|---|---|---|---|
| Exp. | Syn. | SM1 | Reagent | NRzRy | Y | V₁' | V₂' | LCMS | | |
| | | | | | | | | method | RTime | Mass |
| N-k-45 | 10k-1 | Int.n-e-28 | | 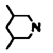 | Et | 1Et-5-Idz | H | C | | 458 (M⁺+1) |
| N-k-46 | 1-a | N-k-45 | | 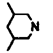 | H | 1Et-5-Idz | H | C | | 430 (M⁺+1) |
| N-k-47 | 10k-1 | Int.n-e-30 | | 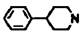 | Et | 2-Nap | H | C | | 488 (M⁺+1) |
| N-k-48 | 1-a | N-k-47 | | 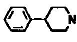 | H | 2-Nap | H | C | | 460 (M⁺+1) |
| N-k-49 | 10k-1 | Int.n-e-31 | | 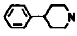 | Et | 5-Ind | H | C | | 477 (M⁺+1) |
| N-k-50 | 1-a | N-k-49 | | 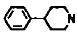 | H | 5-Ind | H | C | | 449 (M⁺+1) |
| N-k-51 | 10k-1 | Int.n-e-32 | | 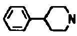 | Et | 1Me-5-Ind | H | C | | 491 (M⁺+1) |
| N-k-52 | 1-a | N-k-51 | | 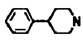 | H | 1Me-5-Ind | H | C | | 463 (M⁺+1) |
| N-k-53 | 10k-1 | Int.n-e-33 | | 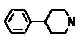 | Et | 5-1Idz | H | C | | 478 (M⁺+1) |
| N-k-54 | 1-a | N-k-53 | | 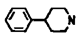 | H | 5-1Idz | H | C | | 450 (M⁺+1) |
| N-k-55 | 10k-1 | Int.n-e-34 | | 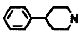 | Et | 1Me-5-Idz | H | C | | 492 (M⁺+1) |
| N-k-56 | 1-a | N-k-55 | | 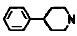 | H | 1Me-5-Idz | H | C | | 464 (M⁺+1) |
| N-k-57 | 10k-1 | Int.n-e-36 | | 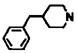 | Et | 2-Nap | H | C | | 502 (M⁺+1) |
| N-k-58 | 1-a | N-k-57 | | 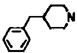 | H | 2-Nap | H | C | | 474 (M⁺+1) |
| N-k-59 | 10k-1 | Int.n-e-37 | | 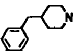 | Et | 5-Ind | H | C | | 491 (M⁺+1) |
| N-k-60 | 1-a | N-k-59 | | 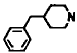 | H | 5-Ind | H | C | | 463 (M⁺+1) |
| N-k-61 | 10k-1 | Int.n-e-38 | | 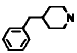 | Et | 5-1Idz | H | C | | 492 (M⁺+1) |
| N-k-62 | 1-a | N-k-61 | | 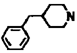 | H | 5-1Idz | H | C | | 464 (M⁺+1) |
| N-k-63 | 10k-1 | Int.n-e-40 | | 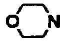 | Et | 2-Nap | H | C | | 414 (M⁺+1) |
| N-k-64 | 1-a | N-k-63 | | 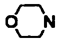 | H | 2-Nap | H | C | | 386 (M⁺+1) |
| N-k-65 | 10k-1 | Int.n-e-41 | | 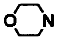 | Et | 5-Ind | H | C | | 403 (M⁺+1) |
| N-k-66 | 1-a | N-k-65 | | 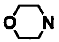 | H | 5-Ind | H | C | | 375 (M⁺+1) |

**[Table 189]**

| Table-N-K-4 | | | | | | | | | | |
|---|---|---|---|---|---|---|---|---|---|---|
| Exp. | Syn. | SM1 | Reagent | NRzRy | Y | V₁' | V₂' | LCMS | | |
| | | | | | | | | method | RTime | Mass |
| N-k-67 | 10k-1 | Int.n-e-42 | | 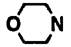 | Et | 1Me-5-Ind | H | C | | 417 (M⁺+1) |
| N-k-68 | 1-a | N-k-67 | | 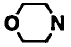 | H | 1Me-5-Ind | H | C | | 389 (M⁺+1) |
| N-k-69 | 10k-1 | Int.n-e-43 | | 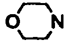 | Et | 5-1Idz | H | C | | 404 (M⁺+1) |
| N-k-70 | 1-a | N-k-69 | | 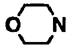 | H | 5-1Idz | H | C | | 376 (M⁺+1) |
| N-k-71 | 10k-1 | Int.n-e-44 | | 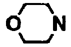 | Et | 1Me-5-Idz | H | C | | 418 (M⁺+1) |
| N-k-72 | 1-a | N-k-71 | | 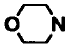 | H | 1Me-5-Idz | H | C | | 390 (M⁺+1) |
| N-k-73 | 1 Ok-1 | Int.n-e-45 | | 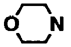 | Et | 1Et-5-Idz | H | C | | 432 (M⁺+1) |
| N-k-74 | 1-a | N-k-73 | | 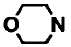 | H | 1Et-5-Idz | H | C | | 404 (M⁺+1) |
| N-k-75 | 10k-1 | Int.n-e-47 | | 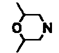 | Et | 2-Nap | H | C | | 442 (M⁺+1) |
| N-k-76 | 1-a | N-k-75 | | 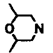 | H | 2-Nap | H | C | | 414 (M⁺+1) |
| N-k-77 | 10k-1 | Int.n-e-48 | | 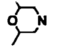 | Et | 5-Ind | H | C | | 431 (M⁺+1) |
| N-k-78 | 1-a | N-k-77 | | 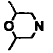 | H | 5-Ind | H | C | | 403 (M⁺+1) |
| N-k-79 | 10k-1 | Int.n-e-49 | | 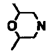 | Et | 1Me-5-Ind | H | C | | 445 (M⁺+1) |
| N-k-80 | 1-a | N-k-79 | | 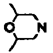 | H | 1Me-5-Ind | H | C | | 417 (M⁺+1) |
| N-k-81 | 10k-1 | Int.n-e-50 | | 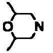 | Et | 5-1Idz | H | C | | 432 (M⁺+1) |
| N-k-82 | 1-a | N-k-81 | | 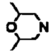 | H | 5-1Idz | H | C | | 404 (M⁺+1) |
| N-k-83 | 10k-1 | Int.n-e-51 | | 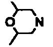 | Et | 1Me-5-Idz | H | C | | 446 (M⁺+1) |
| N-k-84 | 1-a | N-k-83 | | 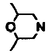 | H | 1Me-5-Idz | H | C | | 418 (M⁺+1) |
| N-k-85 | 10k-1 | Int.n-e-53 | | 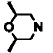 | Et | 5-Ind | H | C | | 431 (M⁺+1) |
| N-k-86 | 1-a | N-k-85 | | 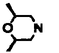 | H | 5-Ind | H | C | | 403 (M⁺+1) |
| N-k-87 | 10k-1 | Int.n-e-54 | | 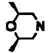 | Et | 1Me-5-Ind | H | C | | 445 (M⁺+1) |
| N-k-88 | 1-a | N-k-87 | | 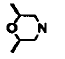 | H | 1Me-5-Ind | H | C | | 417 (M⁺+1) |

**[Table 190]**

| Table-N-K-5 | | | | | | | | | | |
|---|---|---|---|---|---|---|---|---|---|---|
| Exp. | Syn. | SM1 | Reagent | NRzRy | Y | V₁' | V₂' | LCMS | | |
| | | | | | | | | method | RTime | Mass |
| N-k-89 | 10k-1 | Int.n-e-55 | | 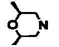 | Et | 1Me-5-Idz | H | C | | 446 (M⁺+1) |
| N-k-90 | 1-a | N-k-89 | | 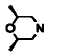 | H | 1Me-5-Idz | H | C | | 418 (M⁺+1) |
| N-k-91 | 10k-1 | Int.n-e-57 | | 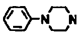 | Et | 2-Nap | H | C | | 489 (M⁺+1) |
| N-k-92 | 1-a | N-k-91 | | 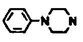 | H | 2-Nap | H | C | | 461 (M⁺+1) |
| N-k-93 | 10k-1 | Int.n-e-58 | | 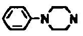 | Et | 5-Ind | H | C | | 478 (M⁺+1) |
| N-k-94 | 1-a | N-k-93 | | 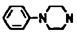 | H | 5-Ind | H | C | | 450 (M⁺+1) |
| N-k-95 | 10k-1 | Int.n-e-59 | | 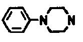 | Et | 1Me-5-Ind | H | C | | 492 (M⁺+1) |
| N-k-96 | 1-a | N-k-95 | | 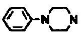 | H | 1Me-5-Ind | H | C | | 464 (M⁺+1) |
| N-k-97 | 10k-1 | Int.n-e-60 | | 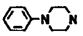 | Et | 5-1Idz | H | C | | 479 (M⁺+1) |
| N-k-98 | 1-a | N-k-97 | | 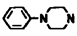 | H | 5-1Idz | H | C | | 451 (M⁺+1) |
| N-k-99 | 10k-1 | Int.n-e-61 | | 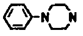 | Et | 1Me-5-Idz | H | C | | 493 (M⁺+1) |
| N-k-100 | 1-a | N-k-99 | | 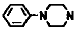 | H | 1Me-5-Idz | H | C | | 465 (M⁺+1) |
| N-k-101 | 10k-1 | Int.n-e-63 | | 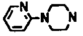 | Et | 2-Nap | H | C | | 490 (M⁺+1) |
| N-k-102 | 1-a | N-k-101 | | 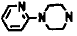 | H | 2-Nap | H | C | | 462 (M⁺+1) |
| N-k-103 | 10k-1 | Int.n-e-64 | | 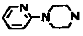 | Et | 1Me-5-Ind | H | C | | 493 (M⁺+1) |
| N-k-104 | 1-a | N-k-103 | | 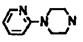 | H | 1Me-5-Ind | H | C | | 465 (M⁺+1) |
| N-k-105 | 10k-1 | Int.n-e-65 | | 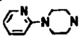 | Et | 1Me-5-Idz | H | C | | 494 (M⁺+1) |
| N-k-106 | 1-a | N-k-105 | | 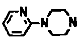 | H | 1Me-5-Idz | H | C | | 466 (M⁺+1) |
| N-k-107 | 10k-1 | Int.n-e-66 | | 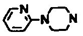 | Et | 1Et-5-Idz | H | C | | 508 (M⁺+1) |
| N-k-108 | 1-a | N-k-107 | | 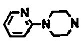 | H | 1Et-5-Idz | H | C | | 480 (M⁺+1) |
| N-k-109 | 10k-1 | Int.n-e-68 | | 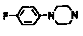 | Et | 2-Nap | H | C | | 507 (M⁺+1) |
| N-k-110 | 1-a | N-k-109 | | 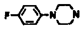 | H | 2-Nap | H | C | | 479 (M⁺+1) |

**[Table 191]**

| Table-N-K-6 | | | | | | | | | | |
|---|---|---|---|---|---|---|---|---|---|---|
| Exp. | Syn. | SM1 | Reagent | NRzRy | Y | V₁' | V₂' | LCMS | | |
| | | | | | | | | method | RTime | Mass |
| N-k-111 | 10k-1 | Int.n-e-69 | | 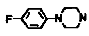 | Et | 5-Ind | H | C | | 496 (M⁺+1) |
| N-k-112 | 1-a | N-k-111 | | 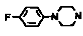 | H | 5-Ind | H | C | | 468 (M⁺+1) |
| N-k-113 | 10k-1 | Int.n-e-70 | | 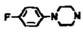 | Et | 1Et-5-Ind | H | C | | 524 (M⁺+1) |
| N-k-114 | 1-a | N-k-113 | | 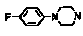 | H | 1Et-5-Ind | H | C | | 496 (M⁺+1) |
| N-k-115 | 10k-1 | Int.n-e-71 | | 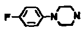 | Et | 5-1Idz | H | C | | 497 (M⁺+1) |
| N-k-116 | 1-a | N-k-115 | | 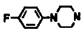 | H | 5-1Idz | H | C | | 469 (M⁺+1) |
| N-k-117 | 10k-1 | Int.n-e-72 | | 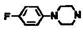 | Et | 1Me-5-Idz | H | C | | 511 (M⁺+1) |
| N-k-118 | 1-a | N-k-117 | | 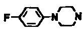 | H | 1Me-5-Idz | H | C | | 483 (M⁺+1) |
| N-k-119 | 10k-1 | Int.n-e-74 | | 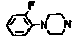 | Et | 2-Nap | H | C | | 507 (M⁺+1) |
| N-k-120 | 1-a | N-k-119 | | 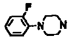 | H | 2-Nap | H | C | | 479 (M⁺+1) |
| N-k-121 | 10k-1 | Int.n-e-75 | | 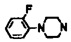 | Et | 5-Ind | H | C | | 496 (M⁺+1) |
| N-k-122 | 1-a | N-k-121 | | 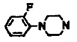 | H | 5-Ind | H | C | | 468 (M⁺+1) |
| N-k-123 | 10k-1 | Int.n-e-76 | | 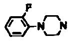 | Et | 1 Et-5-Ind | H | C | | 524 (M⁺+1) |
| N-k-124 | 1-a | N-k-123 | | 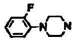 | H | 1 Et-5-Ind | H | C | | 496 (M⁺+1) |
| N-k-125 | 10k-1 | Int.n-e-77 | | 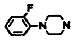 | Et | 1Me-5-Idz | H | C | | 511 (M⁺+1) |
| N-k-126 | 1-a | N-k-125 | | 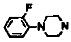 | H | 1Me-5-Idz | H | C | | 483 (M⁺+1) |
| N-k-127 | 10k-1 | Int.n-e-78 | | 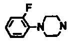 | Et | 1Et-5-Idz | H | C | | 525 (M⁺+1) |
| N-k-128 | 1-a | N-k-127 | | 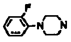 | H | 1Et-5-Idz | H | C | | 497 (M⁺+1) |
| N-k-129 | 10k-1 | Int.n-e-80 | | 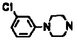 | Et | 2-Nap | H | C | | 524 (M⁺+1) |
| N-k-130 | 1-a | N-k-129 | | 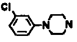 | H | 2-Nap | H | C | | 496 (M⁺+1) |
| N-k-131 | 10k-1 | Int.n-e-81 | | 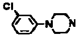 | Et | 5-Ind | H | C | | 513 (M⁺+1) |
| N-k-132 | 1-a | N-k-131 | | 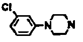 | H | 5-Ind | H | C | | 485 (M⁺+1) |

**[Table 192]**

| Table-N-K-7 | | | | | | | | | | |
|---|---|---|---|---|---|---|---|---|---|---|
| Exp. | Syn. | SM1 | Reagent | NRzRy | Y | V₁' | V₂' | LCMS | | |
| | | | | | | | | method | RTime | Mass |
| N-k-133 | 10k-1 | Intn-e-82 | | 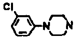 | Et | 1Me-5-Ind | H | C | | 527 (M⁺+1) |
| N-k-134 | 1-a | N-k-133 | | 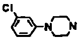 | H | 1Me-5-Ind | H | C | | 499 (M⁺+1) |
| N-k-135 | 10k-1 | Int.n-e-83 | | 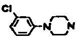 | Et | 5-1Idz | H | C | | 514 (M⁺+1) |
| N-k-136 | 1-a | N-k-135 | | 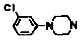 | H | 5-1Idz | H | C | | 486 (M⁺+1) |
| N-k-137 | 10k-1 | Int.n-e-84 | | 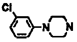 | Et | 1Me-5-Idz | H | C | | 528 (M⁺+1) |
| N-k-138 | 1-a | N-k-137 | | 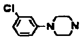 | H | 1Me-5-Idz | H | C | | 500 (M⁺+1) |
| N-k-139 | 10k-1 | Int.n-e-86 | | 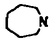 | Et | 2-Nap | H | C | | 426 (M⁺+1) |
| N-k-140 | 1-a | N-k-139 | | 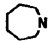 | H | 2-Nap | H | C | | 398 (M⁺+1) |
| N-k-141 | 10k-1 | Int.n-e-87 | | 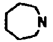 | Et | 5-Ind | H | C | | 415 (M⁺+1) |
| N-k-142 | 1-a | N-k-141 | | 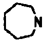 | H | 5-Ind | H | C | | 387 (M⁺+1) |
| N-k-143 | 10k-1 | Int.n-e-88 | | 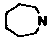 | Et | 1Me-5-Ind | H | C | | 429 (M⁺+1) |
| N-k-144 | 1-a | N-k-143 | | 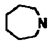 | H | 1Me-5-Ind | H | C | | 401 (M⁺+1) |
| N-k-145 | 10k-1 | Intn-e-89 | | 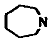 | Et | 5-1Idz | H | C | | 416 (M⁺+1) |
| N-k-146 | 1-a | N-k-145 | | 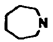 | H | 5-1Idz | H | C | | 388 (M⁺+1) |
| N-k-147 | 10k-1 | Int.n-e-90 | | 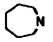 | Et | 1Me-5-Idz | H | C | | 430 (M⁺+1) |
| N-k-148 | 1-a | N-k-147 | | 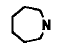 | H | 1Me-5-Idz | H | C | | 402 (M⁺+1) |

### Example N-l-1

### Synthesis of ethyl 2-[5-methylamino-4-(naphthalen-2-yl)-2,3-dihydro-1H-inden-1-yl]acetate (Compound No. N-l-1) (Preparation Method 9, Step j)

According to the procedure described in the synthetic method of Intermediate A-3 in Reference Example 1 (Preparation Method 9, Step j) provided that the reaction was performed for 9 hours, Example Compound N-j-1 (70.6 mg) and Pd/C (17.2 mg, Merck) were reacted and treated to obtain the title compound (Compound No. N-l-1, 41.2 mg).

### Example N-l-2

### Synthesis of 2-[5-methylamino-4-(naphthalen-2-yl)-2,3-dihydro-1H-inden-1-yl]acetic acid (Compound No. N-l-2) (Preparation Method 1, Step a)

According to the procedure described in the synthetic method of Example Compound A-a-2 (Preparation Method 1, Step a) provided that the reaction was performed for 1 hour, Example Compound N-l-1 (39.1 mg) and 2 N aqueous sodium hydroxide (0.40 ml) were reacted and treated to obtain the title compound (Compound No. N-l-2, 30.2 mg).

Typical examples of the compounds of the present invention that can be obtained by reacting and treating corresponding starting compounds using any of the methods described in the present specification including the examples described above are shown in Table-N-L-1 to Table-N-L-3 and Table-N-M-1 to Table-N-M-5. As for the preparation of the compounds, used methods among the aforementioned synthetic methods are shown in the columns of "Syn" with symbols, the starting compounds are shown in the columns of "SM", and the reagents are shown in the columns of "Regent" in the tables.

**[Table 193]**

| Table-N-L-1 | | | | | | | | | | | |
|---|---|---|---|---|---|---|---|---|---|---|---|
| 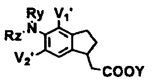 | | | | | | | | | | | |

| Exp. | Syn. | SM1 | Reagent | Ry | Rz | Y | V₁' | V₂' | LCMS | | |
|---|---|---|---|---|---|---|---|---|---|---|---|
| | | | | | | | | | method | RTime | Mass |
| N-l-1 | 9-j | N-j-1 | | Me | H | Et | 2-Nap | H | C | | 360 (M⁺+1) |
| N-l-2 | 1-a | N-l-1 | | Me | H | H | 2-Nap | H | C | | 332 (M⁺+1) |
| N-l-3 | 9-j | N-j-3 | | nPr | Me | Et | 2-Nap | H | C | | 402 (M⁺+1) |
| N-l-4 | 1-a | N-l-3 | | nPr | Me | H | 2-Nap | H | C | | 374 (M⁺+1) |
| N-l-5 | 9-j | N-j-5 | | iPr | Me | Et | 2-Nap | H | C | | 402 (M⁺+1) |
| N-l-6 | 1-a | N-l-5 | | iPr | Me | H | 2-Nap | H | C | | 374 (M⁺+1) |
| N-l-7 | 9-j | N-j-7 | | nBu | Me | Et | 2-Nap | H | C | | 416 (M⁺+1) |
| N-l-8 | 1-a | N-l-7 | | nBu | Me | H | 2-Nap | H | C | | 388 (M⁺+1) |
| N-l-9 | 9-j | N-j-9 | | iBu | Me | Et | 2-Nap | H | C | | 416 (M⁺+1) |
| N-l-10 | 1-a | N-l-9 | | iBu | Me | H | 2-Nap | H | C | | 388 (M⁺+1) |
| N-l-11 | 9-j | N-j-11 | | cPen | Me | Et | 2-Nap | H | C | | 428 (M⁺+1) |
| N-l-12 | 1-a | N-l-11 | | cPen | Me | H | 2-Nap | H | C | | 400 (M⁺+1) |
| N-l-13 | 9-j | N-j-13 | | cHex | Me | Et | 2-Nap | H | C | | 442 (M⁺+1) |
| N-l-14 | 1-a | N-l-13 | | cHex | Me | H | 2-Nap | H | C | | 414 (M⁺+1) |
| N-l-15 | 9-j | N-j-15 | | Me | H | Et | 5-Ind | H | C | | 349 (M⁺+1) |
| N-l-16 | 1-a | N-l-15 | | Me | H | H | 5-Ind | H | C | | 321 (M⁺+1) |
| N-l-17 | 9-j | N-j-17 | | nPr | Me | Et | 5-Ind | H | C | | 391 (M⁺+1) |
| N-l-18 | 1-a | N-l-17 | | nPr | Me | H | 5-Ind | H | C | | 363 (M⁺+1) |
| N-l-19 | 9-j | N-j-19 | | iPr | Me | Et | 5-Ind | H | C | | 391 (M⁺+1) |
| N-l-20 | 1-a | N-l-19 | | iPr | Me | H | 5-Ind | H | C | | 363 (M⁺+1) |
| N-l-21 | 9-j | N-j-21 | | nBu | Me | Et | 5-Ind | H | C | | 405 (M⁺+1) |
| N-l-22 | 1-a | N-l-21 | | nBu | Me | H | 5-Ind | H | C | | 377 (M⁺+1) |
| N-l-23 | 9-j | N-j-23 | | iBu | Me | Et | 5-Ind | H | C | | 405 (M⁺+1) |
| N-l-24 | 1-a | N-l-23 | | iBu | Me | H | 5-Ind | H | C | | 377 (M⁺+1) |
| N-l-25 | 9-j | N-j-25 | | cPen | Me | Et | 5-Ind | H | C | | 417 (M⁺+1) |
| N-l-26 | 1-a | N-l-25 | | cPen | Me | H | 5-Ind | H | C | | 389 (M⁺+1) |
| N-l-27 | 9-j | N-j-27 | | cHex | Me | Et | 5-Ind | H | C | | 431 (M⁺+1) |
| N-l-28 | 1-a | N-l-27 | | cHex | Me | H | 5-Ind | H | C | | 403 (M⁺+1) |
| N-l-29 | 9-j | N-j-31 | | nPr | Me | Et | 1Me-5-Ind | H | C | | 405 (M⁺+1) |
| N-l-30 | 1-a | N-l-29 | | nPr | Me | H | 1Me-5-Ind | H | C | | 377 (M⁺+1) |
| N-l-31 | 9-j | N-j-33 | | iBu | Me | Et | 1Me-5-Ind | H | C | | 419 (M⁺+1) |
| N-l-32 | 1-a | N-l-31 | | iBu | Me | H | 1Me-5-Ind | H | C | | 391 (M⁺+1) |
| N-l-33 | 9-j | N-j-35 | | cPen | Me | Et | 1Me-5-Ind | H | C | | 431 (M⁺+1) |
| N-l-34 | 1-a | N-l-33 | | cPen | Me | H | 1Me-5-Ind | H | C | | 403 (M⁺+1) |
| N-l-35 | 9-j | N-j-37 | | cHex | Me | Et | 1Me-5-Ind | H | C | | 445 (M⁺+1) |
| N-l-36 | 1-a | N-l-35 | | cHex | Me | H | 1Me-5-Ind | H | C | | 417 (M⁺+1) |
| N-l-37 | 9-j | N-j-41 | | iPr | Me | Et | 1Et-5-Ind | H | C | | 419 (M⁺+1) |
| N-l-38 | 1-a | N-l-37 | | iPr | Me | H | 1Et-5-Ind | H | C | | 391 (M⁺+1) |
| N-l-39 | 9-j | N-j-43 | | nBu | Me | Et | 1Et-5-Ind | H | C | | 433 (M⁺+1) |
| N-l-40 | 1-a | N-l-39 | | nBu | Me | H | 1Et-5-Ind | H | C | | 405 (M⁺+1) |
| N-l-41 | 9-j | N-j-45 | | iBu | Me | Et | 1Et-5-Ind | H | C | | 433 (M⁺+1) |
| N-l-42 | 1-a | N-l-41 | | iBu | Me | H | 1Et-5-Ind | H | C | | 405 (M⁺+1) |
| N-l-43 | 9-j | N-j-47 | | cPen | Me | Et | 1Et-5-Ind | H | C | | 445 (M⁺+1) |
| N-l-44 | 1-a | N-l-43 | | cPen | Me | H | 1Et-5-Ind | H | C | | 417 (M⁺+1) |

**[Table 194]**

| Table-N-L-2 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-l-45 | 9-j | N-j-49 | | Me | H | Et | 1Me-5-Idz | H | C | | 364 (M⁺+1) |
| N-l-46 | 1-a | N-l-45 | | Me | H | H | 1Me-5-Idz | H | C | | 336 (M⁺+1) |
| N-l-47 | 9-j | N-j-51 | | nPr | Me | Et | 1Me-5-Idz | H | C | | 406 (M⁺+1) |
| N-l-48 | 1-a | N-l-47 | | nPr | Me | H | 1Me-5-Idz | H | C | | 378 (M⁺+1) |
| N-l-49 | 9-j | N-j-53 | | iPr | Me | Et | 1Me-5-Idz | H | C | | 406 (M⁺+1) |
| N-l-50 | 1-a | N-l-49 | | iPr | Me | H | 1Me-5-Idz | H | C | | 378 (M⁺+1) |
| N-l-51 | 9-j | N-j-55 | | nBu | Me | Et | 1Me-5-Idz | H | C | | 420 (M⁺+1) |
| N-l-52 | 1-a | N-l-51 | | nBu | Me | H | 1Me-5-Idz | H | C | | 392 (M⁺+1) |
| N-l-53 | 9-j | N-j-57 | | iBu | Me | Et | 1Me-5-Idz | H | C | | 420 (M⁺+1) |
| N-l-54 | 1-a | N-l-53 | | iBu | Me | H | 1Me-5-Idz | H | C | | 392 (M⁺+1) |
| N-l-55 | 9-j | N-j-59 | | cPen | Me | Et | 1Me-5-Idz | H | C | | 432 (M⁺+1) |
| N-l-56 | 1-a | N-l-55 | | cPen | Me | H | 1Me-5-Idz | H | C | | 404 (M⁺+1) |
| N-l-57 | 9-j | N-j-61 | | cHex | Me | Et | 1Me-5-Idz | H | C | | 446 (M⁺+1) |
| N-l-58 | 1-a | N-l-57 | | cHex | Me | H | 1Me-5-Idz | H | C | | 418 (M⁺+1) |
| N-l-59 | 9-j | N-j-65 | | Pr | Me | Et | 1Et-5-Idz | H | C | | 420 (M⁺+1) |
| N-l-60 | 1-a | N-l-59 | | nPr | Me | H | 1Et-5-Idz | H | C | | 392 (M⁺+1) |
| N-l-61 | 9-j | N-j-67 | | iPr | Me | Et | 1Et-5-Idz | H | C | | 420 (M⁺+1) |
| N-l-62 | 1-a | N-l-61 | | iPr | Me | H | 1Et-5-Idz | H | C | | 392 (M⁺+1) |
| N-l-63 | 9-j | N-j-69 | | cHex | Me | Et | 1Et-5-Idz | H | C | | 460 (M⁺+1) |
| N-l-64 | 1-a | N-l-63 | | cHex | Me | H | 1Et-5-Idz | H | C | | 432 (M⁺+1) |
| N-l-65 | 9-j | N-j-71 | | Bn | Et | Et | 2-Nap | H | C | | 450 (M⁺+1) |
| N-l-66 | 1-a | N-l-65 | | Bn | Et | H | 2-Nap | H | C | | 422 (M⁺+1) |
| N-l-67 | 9-j | N-j-73 | | nPr | Et | Et | 2-Nap | H | C | | 402 (M⁺+1) |
| N-l-68 | 1-a | N-l-67 | | nPr | Et | H | 2-Nap | H | C | | 374 (M⁺+1) |
| N-l-69 | 9-j | N-j-75 | | iPr | Et | Et | 2-Nap | H | C | | 416 (M⁺+1) |
| N-l-70 | 1-a | N-l-69 | | iPr | Et | H | 2-Nap | H | C | | 388 (M⁺+1) |
| N-l-71 | 9-j | N-j-77 | | nBu | Et | Et | 2-Nap | H | C | | 430 (M⁺+1) |
| N-l-72 | 1-a | N-l-71 | | nBu | Et | H | 2-Nap | H | C | | 402 (M⁺+1) |
| N-l-73 | 9-j | N-j-79 | | iBu | Et | Et | 2-Nap | H | C | | 430 (M⁺+1) |
| N-l-74 | 1-a | N-l-73 | | iBu | Et | H | 2-Nap | H | C | | 402 (M⁺+1) |
| N-l-75 | 9-j | N-j-81 | | cPen | Et | Et | 2-Nap | H | C | | 442 (M⁺+1) |
| N-l-76 | 1-a | N-l-75 | | cPen | Et | H | 2-Nap | H | C | | 414 (M⁺+1) |
| N-l-77 | 9-j | N-j-87 | | nPr | Et | Et | 5-Ind | H | C | | 405 (M⁺+1) |
| N-l-78 | 1-a | N-j-69 | | nPr | Et | H | 5-Ind | H | C | | 377 (M⁺+1) |
| N-l-79 | 9-j | N-j-89 | | iPr | Et | Et | 5-Ind | H | C | | 405 (M⁺+1) |
| N-l-80 | 1-a | N-l-79 | | iPr | Et | H | 5-Ind | H | C | | 377 (M⁺+1) |
| N-l-81 | 9-j | N-j-91 | | nBu | Et | Et | 5-Ind | H | C | | 419 (M⁺+1) |
| N-l-82 | 1-a | N-l-81 | | nBu | Et | H | 5-Ind | H | C | | 391 (M⁺+1) |
| N-l-83 | 9-j | N-j-93 | | iBu | Et | Et | 5-Ind | H | C | | 419 (M⁺+1) |
| N-l-84 | 1-a | N-l-83 | | iBu | Et | H | 5-Ind | H | C | | 391 (M⁺+1) |
| N-l-85 | 9-j | N-j-95 | | cPen | Et | Et | 5-Ind | H | C | | 431 (M⁺+1) |
| N-l-86 | 1-a | N-l-85 | | cPen | Et | H | 5-Ind | H | C | | 403 (M⁺+1) |
| N-l-87 | 9-j | N-j-99 | | nPr | Et | Et | 1Me-5-Ind | H | C | | 419 (M⁺+1) |
| N-l-88 | 1-a | N-l-87 | | nPr | Et | H | 1Me-5-Ind | H | C | | 391 (M⁺+1) |
| N-l-89 | 9-j | N-j-101 | | iPr | Et | Et | 1Me-5-Ind | H | C | | 419 (M⁺+1) |
| N-l-90 | 1-a | N-l-89 | | iPr | Et | H | 1Me-5-Ind | H | C | | 391 (M⁺+1) |

**[Table 195]**

| Table-N-L-3 | | | | | | | | | | | |
|---|---|---|---|---|---|---|---|---|---|---|---|
| Exp. | Syn. | SM1 | Reagent | Ry | Rz | Y | V₁' | V₂' | LCMS | | |
| | | | | | | | | | method | RTime | Mass |
| N-l-91 | 9-j | N-j-103 | | nBu | Et | Et | 1Me-5-Ind | H | C | | 433 (M⁺+1) |
| N-l-92 | 1-a | N-l-91 | | nBu | Et | H | 1Me-5-Ind | H | C | | 405 (M⁺+1) |
| N-l-93 | 9-j | N-j-105 | | iBu | Et | Et | 1Me-5-Ind | H | C | | 433 (M⁺+1) |
| N-l-94 | 1-a | N-l-93 | | iBu | Et | H | 1Me-5-Ind | H | C | | 405 (M⁺+1) |
| N-l-95 | 9-j | N-j-107 | | cPen | Et | Et | 1Me-5-Ind | H | C | | 445 (M⁺+1) |
| N-l-96 | 1-a | N-l-95 | | cPen | Et | H | 1Me-5-Ind | H | C | | 417 (M⁺+1) |
| N-l-97 | 9-j | N-j-113 | | iPr | Et | Et | 1Et-5-Ind | H | C | | 433 (M⁺+1) |
| N-l-98 | 1-a | N-l-97 | | iPr | Et | H | 1Et-5-Ind | H | C | | 405 (M⁺+1) |
| N-l-99 | 9-j | N-j-115 | | nBu | Et | Et | 1Et-5-Ind | H | C | | 447 (M⁺+1) |
| N-l-100 | 1-a | N-l-99 | | nBu | Et | H | 1Et-5-Ind | H | C | | 419 (M⁺+1) |
| N-l-101 | 9-j | N-j-117 | | cPen | Et | Et | 1Et-5-Ind | H | C | | 459 (M⁺+1) |
| N-l-102 | 1-a | N-l-101 | | cPen | Et | H | 1Et-5-Ind | H | C | | 431 (M⁺+1) |
| N-l-103 | 9-j | N-j-119 | | Et | H | Et | 1Me-5-Idz | H | C | | 378 (M⁺+1) |
| N-l-104 | 1-a | N-l-103 | | Et | H | H | 1Me-5-Idz | H | C | | 350 (M⁺+1) |
| N-l-105 | 9-j | N-j-121 | | Pr | Et | Et | 1Me-5-Idz | H | C | | 420 (M⁺+1) |
| N-l-106 | 1-a | N-l-105 | | nPr | Et | H | 1Me-5-Idz | H | C | | 392 (M⁺+1) |
| N-l-107 | 9-j | N-j-123 | | iPr | Et | Et | 1Me-5-Idz | H | C | | 420 (M⁺+1) |
| N-l-108 | 1-a | N-l-107 | | iPr | Et | H | 1Me-5-Idz | H | C | | 392 (M⁺+1) |
| N-l-109 | 9-j | N-j-127 | | iBu | Et | Et | 1Me-5-Idz | H | C | | 434 (M⁺+1) |
| N-l-110 | 1-a | N-l-109 | | iBu | Et | H | 1Me-5-Idz | H | C | | 406 (M⁺+1) |
| N-l-111 | 9-j | N-j-131 | | cHex | Et | Et | 1Me-5-Idz | H | C | | 460 (M⁺+1) |
| N-l-112 | 1-a | N-l-111 | | cHex | Et | H | 1Me-5-Idz | H | C | | 432 (M⁺+1) |
| N-l-113 | 9-j | N-j-135 | | nPr | Et | Et | 1Et-5-Idz | H | C | | 406 (M⁺+1) |
| N-l-114 | 1-a | N-l-113 | | nPr | Et | H | 1Et-5-Idz | H | C | | 406 (M⁺+1) |
| N-l-115 | 9-j | N-j-137 | | iPr | Et | Et | 1Et-5-Idz | H | C | | 406 (M⁺+1) |
| N-l-116 | 1-a | N-l-115 | | iPr | Et | H | 1Et-5-Idz | H | C | | 406 (M⁺+1) |
| N-l-117 | 9-j | N-j-139 | | nBu | Et | Et | 1Et-5-Idz | H | C | | 420 (M⁺+1) |
| N-l-118 | 1-a | N-l-117 | | nBu | Et | H | 1Et-5-Idz | H | C | | 420 (M⁺+1) |

**[Table 196]**

| Table-N-M-1 | | | | | | | | | | |
|---|---|---|---|---|---|---|---|---|---|---|
| 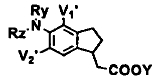 | | | | | | | | | | |

| Exp. | Syn. | SM1 | Reagent | NRzRy | Y | V₁' | V₂' | LCMS | | |
|---|---|---|---|---|---|---|---|---|---|---|
| | | | | | | | | method | RTime | Mass |
| N-m-1 | 9-j | N-k-1 | | 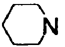 | Et | 2-Nap | H | C | | 414 (M⁺+1) |
| N-m-2 | 1-a | N-m-1 | | 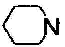 | H | 2-Nap | H | C | | 386 (M⁺+1) |
| N-m-3 | 9-j | N-k-3 | | 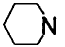 | Et | 5-Ind | H | C | | 403 (M⁺+1) |
| N-m-4 | 1-a | N-m-3 | | 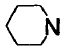 | H | 5-Ind | H | C | | 375 (M⁺+1) |
| N-m-5 | 9-j | N-k-5 | | 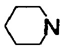 | Et | 1Me-5-Ind | H | C | | 417 (M⁺+1) |
| N-m-6 | 1-a | N-m-5 | | 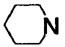 | H | 1Me-5-Ind | H | C | | 389 (M⁺+1) |
| N-m-7 | 9-j | N-k-7 | | 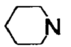 | Et | 1Et-5-Ind | H | C | | 431 (M⁺+1) |
| N-m-8 | 1-a | N-m-7 | | 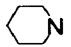 | H | 1Et-5-Ind | H | C | | 403 (M⁺+1) |
| N-m-9 | 9-j | N-k-9 | | 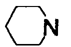 | Et | 5-1Idz | H | C | | 404 (M⁺+1) |
| N-m-10 | 1-a | N-m-9 | | 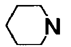 | H | 5-1Idz | H | C | | 376 (M⁺+1) |
| N-m-11 | 9-j | N-k-11 | | 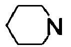 | Et | 1 Me-5-Idz | H | C | | 418 (M⁺+1) |
| N-m-12 | 1-a | N-m-11 | | 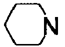 | H | 1 Me-5-Idz | H | C | | 390 (M⁺+1) |
| N-m-13 | 9-j | N-k-13 | | 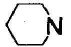 | Et | 1Et-5-Idz | H | C | | 432 (M⁺+1) |
| N-m-14 | 1-a | N-m-13 | | 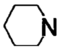 | H | 1 Et-5-Idz | H | C | | 404 (M⁺+1) |
| N-m-15 | 9-j | N-k-15 | | 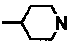 | Et | 2-Nap | H | C | | 428 (M⁺+1) |
| N-m-16 | 1-a | N-m-15 | | 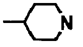 | H | 2-Nap | H | C | | 400 (M⁺+1) |
| N-m-17 | 9-j | N-k-17 | | 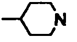 | Et | 1 Me-5-Ind | H | C | | 431 (M⁺+1) |
| N-m-18 | 1-a | N-m-17 | | 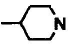 | H | 1Me-5-Ind | H | C | | 403 (M⁺+1) |
| N-m-19 | 9-j | N-k-21 | | 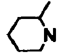 | Et | 2-Nap | H | C | | 428 (M⁺+1) |
| N-m-20 | 1-a | N-m-19 | | 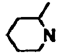 | H | 2-Nap | H | C | | 400 (M⁺+1) |
| N-m-21 | 9-j | N-k-23 | | 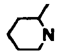 | Et | 5-Ind | H | C | | 417 (M⁺+1) |
| N-m-22 | 1-a | N-m-21 | | 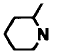 | H | 5-Ind | H | C | | 389 (M⁺+1) |

**[Table 197]**

| Table-N-M-2 | | | | | | | | | | |
|---|---|---|---|---|---|---|---|---|---|---|
| Exp. | Syn. | SM1 | Reagent | NRzRy | Y | V₁' | V₂' | LCMS | | |
| | | | | | | | | method | RTime | Mass |
| N-m-23 | 9-j | N-K-25 | | 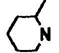 | Et | 1 Me-5-Ind | H | C | | 431 (M⁺+1) |
| N-m-24 | 1-a | N-m-23 | | 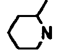 | H | 1 Me-5-Ind | H | C | | 403 (M⁺+1) |
| N-m-25 | 9-j | N-k-27 | | 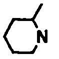 | Et | 1 Me-5-Idz | H | C | | 432 (M⁺+1) |
| N-m-26 | 1-a | N-m-25 | | 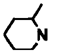 | H | 1 Me-5-Idz | H | C | | 404 (M⁺+1) |
| N-m-27 | 9-j | N-k-31 | | 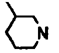 | Et | 2-Nap | H | C | | 428 (M⁺+1) |
| N-m-28 | 1-a | N-m-27 | | 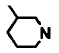 | H | 2-Nap | H | C | | 400 (M⁺+1) |
| N-m-29 | 9-j | N-k-33 | | 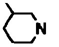 | Et | 5-Ind | H | C | | 417 (M⁺+1) |
| N-m-30 | 1-a | N-m-29 | | 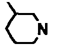 | H | 5-Ind | H | C | | 389 (M⁺+1) |
| N-m-31 | 9-j | N-k-35 | | 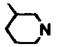 | Et | 5-1Idz | H | C | | 418 (M⁺+1) |
| N-m-32 | 1-a | N-m-31 | | 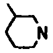 | H | 5-1Idz | H | C | | 390 (M⁺+1) |
| N-m-33 | 9-j | N-k-41 | | 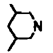 | Et | 1 Me-5-Ind | H | C | | 445 (M⁺+1) |
| N-m-34 | 1-a | N-m-33 | | 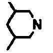 | H | 1 Me-5-Ind | H | C | | 417 (M⁺+1) |
| N-m-35 | 9-j | N-k-43 | | 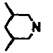 | Et | 1 Me-5-Idz | H | C | | 446 (M⁺+1) |
| N-m-36 | 1-a | N-m-35 | | 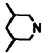 | H | 1 Me-5-Idz | H | C | | 418 (M⁺+1) |
| N-m-37 | 9-j | N-k-45 | | 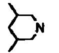 | Et | 1 Et-5-Idz | H | C | | 460 (M⁺+1) |
| N-m-38 | 1-a | N-m-37 | | 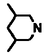 | H | 1Et-5-Idz | H | C | | 432 (M⁺+1) |
| N-m-39 | 9-j | N-k-47 | | 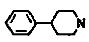 | Et | 2-Nap | H | C | | 490 (M⁺+1) |
| N-m-40 | 1-a | N-m-39 | | 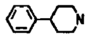 | H | 2-Nap | H | C | | 462 (M⁺+1) |
| N-m-41 | 9-j | N-k-49 | | 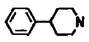 | Et | 5-Ind | H | C | | 479 (M⁺+1) |
| N-m-42 | 1-a | N-m-41 | | 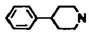 | H | 5-Ind | H | C | | 451 (M⁺+1) |
| N-m-43 | 9-j | N-k-51 | | 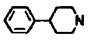 | Et | 1Me-5-Ind | H | C | | 493 (M⁺+1) |
| N-m-44 | 1-a | N-m-43 | | 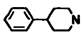 | H | 1 Me-5-Ind | H | C | | 465 (M⁺+1) |

**[Table 198]**

| Table-N-M-3 | | | | | | | | | | |
|---|---|---|---|---|---|---|---|---|---|---|
| Exp. | Syn. | SM1 | Reagent | NRzRy | Y | V₁' | V₂' | LCMS | | |
| | | | | | | | | method | RTime | Mass |
| N-m-45 | 9-j | N-k-53 | | 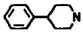 | Et | 5-1Idz | H | C | | 480 (M⁺+1) |
| N-m-46 | 1-a | N-m-45 | | 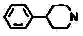 | H | 5-1 Idz | H | C | | 452 (M⁺+1) |
| N-m-47 | 9-j | N-k-57 | | 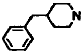 | Et | 2-Nap | H | C | | 504 (m⁺+1) |
| N-m-48 | 1-a | N-m-47 | | 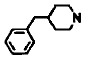 | H | 2-Nap | H | C | | 476 (M⁺+1) |
| N-m-49 | 9-j | N-k-59 | | 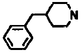 | Et | 5-Ind | H | C | | 493 (M⁺+1) |
| N-m-50 | 1-a | N-m-49 | | 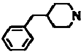 | H | 5-Ind | H | C | | 465 (M⁺+1) |
| N-m-51 | 9-j | N-k-61 | | 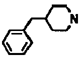 | Et | 5-1 Idz | H | C | | 494 (M⁺+1) |
| N-m-52 | 1-a | N-m-51 | | 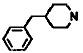 | H | 5-1 Idz | H | C | | 466 (M⁺+1) |
| N-m-53 | 9-j | N-k-63 | | 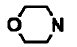 | Et | 2-Nap | H | C | | 416 (M⁺+1) |
| N-m-54 | 1-a | N-m-53 | | 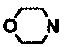 | H | 2-Nap | H | C | | 388 (M⁺+1) |
| N-m-55 | 9-j | N-k-67 | | 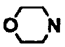 | Et | 1Me-5-Ind | H | C | | 419 (M⁺+1) |
| N-m-56 | 1-a | N-m-55 | | 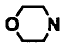 | H | 1 Me-5-Ind | H | C | | 391 (M⁺+1) |
| N-m-57 | 9-j | N-k-69 | | 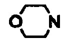 | Et | 5-1 Idz | H | C | | 406 (M⁺+1) |
| N-m-58 | 1-a | N-m-57 | | 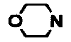 | H | 5-1 Idz | H | C | | 378 (M⁺+1) |
| N-m-59 | 9-j | N-k-71 | | 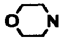 | Et | 1 Me-5-Idz | H | C | | 420 (M⁺+1) |
| N-m-60 | 1-a | N-m-59 | | 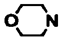 | H | 1 Me-5-Idz | H | C | | 392 (M⁺+1) |
| N-m-61 | 9-j | N-k-75 | | 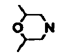 | Et | 2-Nap | H | C | | 444 (M⁺+1) |
| N-m-62 | 1-a | N-m-61 | | 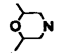 | H | 2-Nap | H | C | | 416 (M⁺+1) |
| N-m-63 | 9-j | N-k-77 | | 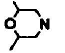 | Et | 5-Ind | H | C | | 433 (M⁺+1) |
| N-m-64 | 1-a | N-m-63 | | 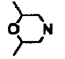 | H | 5-Ind | H | C | | 405 (M⁺+1) |
| N-m-65 | 9-j | N-k-79 | | 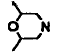 | Et | 1 Me-5-Ind | H | C | | 447 (M⁺+1) |
| N-m-66 | 1-a | N-m-65 | | 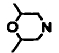 | H | 1 Me-5-Ind | H | C | | 419 (M⁺+1) |

**[Table 199]**

| Table-N-M-4 | | | | | | | | | | |
|---|---|---|---|---|---|---|---|---|---|---|
| Exp. | Syn. | SM1 | Reagent | NRzRy | Y | V₁' | V₂' | LCMS | | |
| | | | | | | | | method | RTime | Mass |
| N-m-67 | 9-j | N-k-87 | | 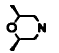 | Et | 1Me-5-Ind | H | C | | 447 (M⁺+1) |
| N-m-68 | 1-a | N-m-67 | | 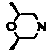 | H | 1Me-5-Ind | H | C | | 419 (M⁺+1) |
| N-m-69 | 9-j | N-K-89 | | 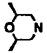 | Et | 1Me-5-Idz | H | C | | 448 (M⁺+1) |
| N-m-70 | 1-a | N-m-89 | | 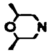 | H | 1Me-5-Idz | H | C | | 420 (M⁺+1) |
| N-m-71 | 9-j | N-K-91 | | 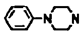 | Et | 2-Nap | H | C | | 491 (M⁺+1) |
| N-m-72 | 1-a | N-m-71 | | 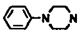 | H | 2-Nap | H | C | | 463 (M⁺+1) |
| N-m-73 | 9-j | N-K-93 | | 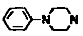 | Et | 5-Ind | H | C | | 480 (M⁺+1) |
| N-m-74 | 1-a | N-m-73 | | 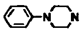 | H | 5-Ind | H | C | | 452 (M⁺+1) |
| N-m-75 | 9-j | N-K-95 | | 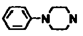 | Et | 1Me-5-Ind | H | C | | 494 (M⁺+1) |
| N-m-76 | 1-a | N-m-75 | | 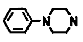 | H | 1Me-5-Ind | H | C | | 466 (M⁺+1) |
| N-m-77 | 9-j | N-K-97 | | 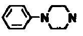 | Et | 5-1Idz | H | C | | 481 (M⁺+1) |
| N-m-78 | 1-a | N-m-77 | | 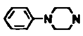 | H | 5-1Idz | H | C | | 453 (M⁺+1) |
| N-m-79 | 9-j | N-K-99 | | 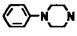 | Et | 1Me-5-Idz | H | C | | 495 (M⁺+1) |
| N-m-80 | 1-a | N-m-79 | | 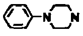 | H | 1Me-5-Idz | H | C | | 467 (M⁺+1) |
| N-m-81 | 9-j | N-K-101 | | 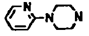 | Et | 2-Nap | H | C | | 492 (M⁺+1) |
| N-m-82 | 1-a | N-m-81 | | 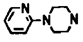 | H | 2-Nap | H | C | | 464 (M⁺+1) |
| N-m-83 | 9-j | N-K-103 | | 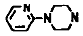 | Et | 1Me-5-Ind | H | C | | 495 (M⁺+1) |
| N-m-84 | 1-a | N-m-83 | | 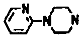 | H | 1Me-5-Ind | H | C | | 467 (M⁺+1) |
| N-m-85 | 9-j | N-K-107 | | 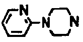 | Et | 1Et-5-Idz | H | C | | 510 (M⁺+1) |
| N-m-86 | 1-a | N-m-85 | | 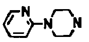 | H | 1Et-5-Idz | H | C | | 482 (M⁺+1) |
| N-m-87 | 9-j | N-K-139 | | 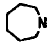 | Et | 2-Nap | H | C | | 428 (M⁺+1) |
| N-m-88 | 1-a | N-m-87 | | 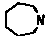 | H | 2-Nap | H | C | | 400 (M⁺+1) |

**[Table 200]**

| Table-N-M-5 | | | | | | | | | | |
|---|---|---|---|---|---|---|---|---|---|---|
| Exp. | Syn. | SM1 | Reagent | NRzRy | Y | V₁' | V₂' | LCMS | | |
| | | | | | | | | method | RTime | Mass |
| N-m-89 | 9-j | N-k-141 | | 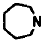 | Et | 5-Ind | H | C | | 417 (M⁺+1) |
| N-m-90 | 1-a | N-m-89 | | 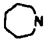 | H | 5-Ind | H | C | | 389 (M⁺+1) |
| N-m-91 | 9-j | N-k-143 | | 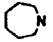 | Et | 1 Me-5-Ind | H | C | | 431 (M⁺+1) |
| N-m-92 | 1-a | N-m-91 | | 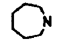 | H | 1 Me-5-Ind | H | C | | 403 (M⁺+1) |
| N-m-93 | 9-j | N-k-145 | | 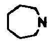 | Et | 5-1Idz | H | C | | 418 (M⁺+1) |
| N-m-94 | 1-a | N-m-93 | | 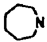 | H | 5-1Idz | H | C | | 390 (M⁺+1) |
| N-m-95 | 9-j | N-k-147 | | 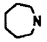 | Et | 1 Me-5-Idz | H | C | | 432 (M⁺+1) |
| N-m-96 | 1-a | N-m-95 | | 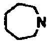 | H | 1 Me-5-Idz | H | C | | 404 (M⁺+1) |

### Reference Example 19

### Synthesis of 6-benzyloxy-3,4-dihydronaphthalen-1(2H)-one (Intermediate d-1) (Preparation Method 14, Step f-1)

According to the procedure described in the synthetic method of Intermediate A-5 in Reference Example 2 (Preparation Method 4, Step f-1), 6-hydroxy-1-tetralone (3.23 g, Ald) and benzyl bromide (2.37 ml, TCI) were reacted and treated to obtain the title compound (Intermediate d-1, 4.37 g). Mass (LCMS): 253 (M⁺), Retention time: 4.66 minutes (Elution condition: B).

### Synthesis of ethyl 2-(6-hydroxy-1,2,3,4-tetrahydronaphthalen-1-yl)acetate (Intermediate d-2) (Preparation Method 10, Step k-2)

According to the procedure described in a literature [Cannon et al., Journal of Medicinal Chemistry, p.813, 1983], zinc powder (5.74 g, WAKO) and a solution of iodine (581.4 mg, WAKO) in toluene (3 ml) were added dropwise with a solution of Intermediate d-1 (4.37 g) and ethyl bromoacetate (6 mL) in toluene (40 ml) at room temperature over 30 minutes, and the mixture was stirred for 1 hour, and further stirred for 12 hours under reflux.
The reaction mixture was added with 10% sulfuric acid (5 ml), stirred for 30 minutes under ice cooling, and then extracted with ethyl acetate (30 ml × 2), and then the organic layer was washed with saturated aqueous sodium hydrogencarbonate and saturated brine, dried, and concentrated under reduced pressure. The residue was purified by flash column chromatography (Quad, hexane:ethyl acetate = 7:1) to obtain a crude product. A solution of the resulting crude product in methanol (20 ml) was added with 10% palladium/carbon (286.2 mg, Merck), and stirred at room temperature for 16.5 hours under 5 atm. The reaction mixture was filtered, and the solvent of the filtrate was evaporated under reduced pressure to obtain the title compound (Intermediate d-2, 1.41 g). Mass (LCMS): N.D, Retention time: 3.85 minutes (Elution condition: B).

### Synthesis of ethyl 2-(6-cyclopentyloxy-1,2,3,4-tetrahydronaphthalen-1-yl)acetate (Intermediate d-3) (Preparation Method 14, Step f-2)

According to the procedure described in the synthetic method of Intermediate A-1 in Reference Example 1 (Preparation Method 4, Step f-2), Intermediate d-2 (119 mg), di-t-butyl azodicarboxylate (238.5 mg, Ald), triphenylphosphine (276.7 mg, WAKO) and cyclopentanol (141 µl, WAKO) were reacted and treated to obtain the title compound (Intermediate d-3, 142.2 mg). Mass (LCMS): N.D (M⁺+1), Retention time: 5.97 minutes (Elution condition: B).

### Synthesis of ethyl 2-(7-bromo-6-cyclopentyloxy-1,2,3,4-tetrahydronaphthalen-1-yl)acetate (Intermediate d-4) (Preparation Method 8, Step h)

According to the procedure described in the synthetic method of Intermediate A-4 in Reference Example 1 (Preparation Method 8, Step h) provided that the reaction was performed at room temperature for 1 hour and 15 minutes, Intermediate d-3 (142.2 mg) and NBS (81.3 mg) were reacted and treated to obtain the title compound (Intermediate d-4, 63.2 mg). Mass (LCMS): N.D (M⁺+1), Retention time: 6.53 minutes (Elution condition: A).

### Synthesis of ethyl 2-(5-bromo-6-cyclopentyloxy-1,2,3,4-tetrahydronaphthalen-1-yl)acetate (Intermediate d-5) (Preparation Method 8, Step h)

According to the procedure described in the synthetic method of Intermediate A-4 in Reference Example 1 (Preparation Method 8, Step h) provided that the reaction was performed at room temperature for 1 hour and 15 minutes, Intermediate d-3 (142.2 mg) and NBS (81.3 mg) were reacted and treated to obtain the title compound (Intermediate d-5, 87.2 mg). Mass (LCMS): N.D (M⁺+1), Retention time: 6.31 minutes (Elution condition: A).
Typical examples of the intermediates that can be obtained by reacting and treating corresponding starting compounds using any of the methods described in the present specification including the examples described above are shown in Table-Int.D-1. In the tables, used methods among the aforementioned synthetic methods are shown in the columns of "Syn" with symbols, the starting compounds are shown in the columns of "SM", and the reagents are shown in the columns of "Regent". The halide regents shown in the columns of "Reagent" with symbols "Hal (No.)" are those mentioned in Table-Hal, and the alcohol regents shown with symbols "A1 (No.)" are those mentioned in Table-Al.

**[Table 201]**

| Table-Int.D-1 | | | | | | | | | | |
|---|---|---|---|---|---|---|---|---|---|---|
| 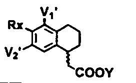 | | | | | | | | | | |

| Exp. | Syn. | SM | Reagent | Rx | Y | V1' | V2' | LCMS | | |
|---|---|---|---|---|---|---|---|---|---|---|
| | | | | | | | | method | RTime | Mass |
| Int.d-6 | 4f-1 | Int.d-2 | Hal1 | BnO | Et | H | H | C | | 325 (M⁺+1) |
| Int.d-7 | 8-h | Int.d-6 | | BnO | Et | H | Br | C | | 403 (M⁺) |
| Int.d-8 | 8-h | Int.d-6 | | BnO | Et | Br | H | C | | 403 (M⁺) |
| Int.d-9 | 4f-2 | Int.d-2 | Al2 | cPenMeO | Et | H | H | C | | 317 (M⁺+1) |
| Int.d-10 | 8-h | Int.d-9 | | cPenMeO | Et | H | Br | C | | 395 (M⁺) |
| Int.d-11 | 8-h | Int.d-9 | | cPenMeO | Et | Br | H | C | | 395 (M⁺) |
| Int.d-12 | 4f-1 | Int.d-2 | Hal2 | cHexMeO | Et | H | H | C | | 331 (M⁺+1) |
| Int.d-13 | 8-h | Int.d-12 | | cHexMeO | Et | H | Br | C | | 409 (M⁺) |
| Int.d-14 | 8-h | Int.d-12 | | cHexMeO | Et | Br | H | C | | 409 (M⁺) |
| Int.d-15 | 4f-2 | Int.d-2 | Al3 | cHexO | Et | H | H | C | | 317 (M⁺+1) |
| Int.d-16 | 8-h | Int.d-15 | | cHexO | Et | H | Br | C | | 395 (M⁺) |
| Int.d-17 | 8-h | Int.d-15 | | cHexO | Et | Br | H | C | | 395 (M⁺) |
| Int.d-18 | 4f-1 | Int.d-2 | Hal3 | nPrO | Et | H | H | C | | 277 (M⁺+1) |
| Int.d-19 | 8-h | Int.d-18 | | nPrO | Et | H | Br | C | | 355 (M⁺) |
| Int.d-20 | 8-h | Int.d-18 | | nPrO | Et | Br | H | C | | 355 (M⁺) |
| Int.d-21 | 4f-2 | Int.d-2 | Hal4 | iPrO | Et | H | H | C | | 277 (M⁺+1) |
| Int.d-22 | 8-h | Int.d-21 | | iPrO | Et | H | Br | C | | 355 (M⁺) |
| Int.d-23 | 8-h | Int.d-21 | | iPrO | Et | Br | H | C | | 355 (M⁺) |

### Example D-a-1

### Synthesis of ethyl 2-[6-cyclopentyloxy-7-(naphthalen-2-yl)-1,2,3,4-tetrahydronaphthalen-1-yl]acetate (Compound No. D-a-1) (Preparation Method 4, Step e-1)

According to the procedure described in the synthetic method of Example Compound A-a-1 (Preparation Method 4, Step e-1) provided that the reaction was performed for 12 hours, Intermediate d-4 (46.4 mg), 2-naphthaleneboronic acid (143.3 mg, Ald), 2 M aqueous sodium carbonate (0.30 ml) and (Ph₃P)₄Pd (43.3 mg, Nakalai Tesque) were reacted and treated to obtain the title compound (Compound No. D-a-1, 37.3 mg).

### Example D-a-2

### Synthesis of 2-[6-cyclopentyloxy-7-(naphthalen-2-yl)-1,2,3,4-tetrahydronaphthalen-1-yl]acetic acid (Compound No. D-a-2) (Preparation Method 1, Step a)

According to the procedure described in the synthetic method of Example Compound A-a-2 (Preparation Method 1, Step a) provided that the reaction was performed for 1 hour, Example Compound D-a-1 (37.3 mg) and 2 N aqueous sodium hydroxide (0.25 ml) were reacted and treated to obtain the title compound (Compound No. D-a-2, 27.2 mg).
Typical examples of the compounds of the present invention that can be obtained by reacting and treating corresponding starting compounds using any of the methods described in the present specification including the examples described above are shown in Table-D-a-1 to Table-D-a-10. As for the preparation of the compounds, used methods among the aforementioned synthetic methods are shown in the columns of "Syn" with symbols, the starting compounds are shown in the columns of "SM", and the reagents are shown in the columns of "Regent" in the tables. The halide regents shown in the columns of "Reagent" with symbols "Hal (No.)" are those mentioned in Table-Hal, the alcohol regents shown with symbols "Al (No.)" are those mentioned in Table-Al, the boronic acid regents shown with symbols "BRA (No.)" are those mentioned in Table-BRA, and the bromoheterocyclic ring regents mentioned with the symbols "Het (No.)" are those mentioned in Table-Het. In Table-Int.D-2, the blank cells of the columns of "Syn" indicate that a reduction reaction with Pd/C was performed for the compounds.

**[Table 202]**

| Table-Int.D-2 | | | | | | | | | | |
|---|---|---|---|---|---|---|---|---|---|---|
| 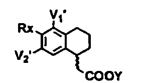 | | | | | | | | | | |

| Exp. | Syn. | SM | Reagent | Rx | Y | V1' | V2' | LCMS | | |
|---|---|---|---|---|---|---|---|---|---|---|
| | | | | | | | | method | RTime | Mass |
| Int.d-24 | | D-a-39 | | HO | Et | H | 2-Nap | C | | 361 (M⁺+1) |
| Int.d-25 | | D-a-41 | | HO | Et | H | 5-Ind | C | | 350 (M⁺+1) |
| Int.d-26 | | D-a-43 | | HO | Et | H | 1Me-5-Ind | C | | 364 (M⁺+1) |
| Int.d-27 | | D-a-45 | | HO | Et | H | 1Me-4-Ind | C | | 364 (M⁺+1) |
| Int.d-28 | | D-a-47 | | HO | Et | H | 1Me-6-Ind | C | | 364 (M⁺+1) |
| Int.d-29 | | D-a-49 | | HO | Et | H | 5-1Idz | C | | 351 (M⁺+1) |
| Int.d-30 | | D-a-51 | | HO | Et | H | 1Me-5-Idz | C | | 365 (M⁺+1) |
| Int.d-31 | | D-a-53 | | HO | Et | H | 1Et-5-Idz | C | | 379 (M⁺+1) |
| Int.d-32 | | D-a-55 | | HO | Et | H | 3-Qu | C | | 362 (M⁺+1) |
| Int.d-33 | | D-a-57 | | HO | Et | H | 6-Qu | C | | 362 (M⁺+1) |
| Int.d-34 | | D-a-59 | | HO | Et | H | 6-IQ | C | | 362 (M⁺+1) |
| Int.d-35 | | D-a-61 | | HO | Et | 2-Nap | H | C | | 361 (M⁺+1) |
| Int.d-36 | | D-a-63 | | HO | Et | 5-Ind | H | C | | 350 (M⁺+1) |
| Int.d-37 | | D-a-65 | | HO | Et | 1Me-5-Ind | H | C | | 364 (M⁺+1) |
| Int.d-38 | | D-a-67 | | HO | Et | 1Me-6-Ind | H | C | | 364 (M⁺+1) |
| Int.d-39 | | D-a-69 | | HO | Et | 1Me-5-Idz | H | C | | 365 (M⁺+1) |
| Int.d-40 | | D-a-71 | | HO | Et | 1Et-5-Idz | H | C | | 379 (M⁺+1) |

**[Table 203]**

| Table-D-a-1 | | | | | | | | | | |
|---|---|---|---|---|---|---|---|---|---|---|
| 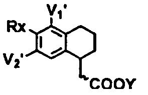 | | | | | | | | | | |

| Exp. | Syn. | SM | Reagent | Rx | Y | V1' | V2' | LCMS | | |
|---|---|---|---|---|---|---|---|---|---|---|
| | | | | | | | | method | RTime | Mass |
| D-a-1 | 4e-1 | Int.d-4 | BRA1 | cPenO | Et | H | 2-Nap | C | | 429 (M⁺+1) |
| D-a-2 | 1-a | D-a-1 | | cPenO | H | H | 2-Nap | C | | 401 (M⁺+1) |
| D-a-3 | 4e-1 | Int.d-4 | BRA2 | cPenO | Et | H | 5-Ind | C | | 418 (M⁺+1) |
| D-a-4 | 1-a | D-a-3 | | cPenO | H | H | 5-Ind | C | | 390 (M⁺+1) |
| D-a-5 | 4e-1 | Int.d-4 | BRA3 | cPenO | Et | H | 1Me-5-Ind | C | | 432 (M⁺+1) |
| D-a-6 | 1-a | D-a-5 | | cPenO | H | H | 1Me-5-Ind | C | | 404 (M⁺+1) |
| D-a-7 | 4e-1 | Int.d-4 | BRA5 | cPenO | Et | H | 1Me-4-Ind | C | | 432 (M⁺+1) |
| D-a-8 | 1-a | D-a-7 | | cPenO | H | H | 1Me-4-Ind | C | | 404 (M⁺+1) |
| D-a-9 | 4e-1 | Int.d-4 | BRA6 | cPenO | Et | H | 1Me-6-Ind | C | | 432 (M⁺+1) |
| D-a-10 | 1-a | D-a-9 | | cPenO | H | H | 1Me-6-Ind | C | | 404 (M⁺+1) |
| D-a-11 | 4e-1 | Int.d-4 | BRA8 | cPenO | Et | H | 1Me-5-Idz | C | | 433 (M⁺+1) |
| D-a-12 | 1-a | D-a-11 | | cPenO | H | H | 1Me-5-Idz | C | | 405 (M⁺+1) |
| D-a-13 | 4e-1 | Int.d-4 | BRA9 | cPenO | Et | H | 1Et-5-Idz | C | | 447 (M⁺+1) |
| D-a-14 | 1-a | D-a-13 | | cPenO | H | H | 1Et-5-Idz | C | | 419 (M⁺+1) |
| D-a-15 | 4e-1 | Int.d-4 | BRA10 | cPenO | Et | H | 2Me-5-Idz | C | | 433 (M⁺+1) |
| D-a-16 | 1-a | D-a-15 | | cPenO | H | H | 2Me-5-Idz | C | | 405 (M⁺+1) |
| D-a-17 | 4e-2 | Int.d-4 | Het2 | cPenO | Et | H | 3-Qu | C | | 430 (M⁺+1) |
| D-a-18 | 1-a | D-a-17 | | cPenO | H | H | 3-Qu | C | | 402 (M⁺+1) |
| D-a-19 | 4e-2 | Int.d-4 | Het3 | cPenO | Et | H | 6-IQ | C | | 430 (M⁺+1) |
| D-a-20 | 1-a | D-a-19 | | cPenO | H | H | 6-IQ | C | | 402 (M⁺+1) |
| D-a-21 | 4e-1 | Int.d-5 | BRA1 | cPenO | Et | 2-Nap | H | C | | 429 (M⁺+1) |
| D-a-22 | 1-a | D-a-21 | | cPenO | H | 2-Nap | H | C | | 401 (M⁺+1) |
| D-a-23 | 4e-1 | Int.d-5 | BRA2 | cPenO | Et | 5-Ind | H | C | | 418 (M⁺+1) |
| D-a-24 | 1-a | D-a-23 | | cPenO | H | 5-Ind | H | C | | 390 (M⁺+1) |
| D-a-25 | 4e-1 | Int.d-5 | BRA3 | cPenO | Et | 1Me-5-Ind | H | C | | 432 (M⁺+1) |
| D-a-26 | 1-a | D-a-25 | | cPenO | H | 1Me-5-Ind | H | C | | 404 (M⁺+1) |
| D-a-27 | 4e-1 | Int.d-5 | BRA6 | cPenO | Et | 1Me-6-Ind | H | C | | 432 (M⁺+1) |
| D-a-28 | 1-a | D-a-27 | | cPenO | H | 1Me-6-Ind | H | C | | 404 (M⁺+1) |
| D-a-29 | 4e-1 | Int.d-5 | BRA7 | cPenO | Et | 5-1Idz | H | C | | 419 (M⁺+1) |
| D-a-30 | 1-a | D-a-29 | | cPenO | H | 5-1Idz | H | C | | 391 (M⁺+1) |
| D-a-31 | 4e-1 | Int.d-5 | BRA8 | cPenO | Et | 1Me-5-Idz | H | C | | 433 (M⁺+1) |
| D-a-32 | 1-a | D-a-31 | | cPenO | H | 1Me-5-Idz | H | C | | 405 (M⁺+1) |
| D-a-33 | 4e-1 | Int.d-5 | BRA9 | cPenO | Et | 1Et-5-Idz | H | C | | 447 (M⁺+1) |
| D-a-34 | 1-a | D-a-33 | | cPenO | H | 1Et-5-Idz | H | C | | 419 (M⁺+1) |
| D-a-35 | 4e-1 | Int.d-5 | BRA11 | cPenO | Et | 5-BF | H | C | | 419 (M⁺+1) |
| D-a-36 | 1-a | D-a-35 | | cPenO | H | 5-BF | H | C | | 391 (M⁺+1) |
| D-a-37 | 4e-1 | Int.d-5 | BRA13 | cPenO | Et | 6-Qu | H | C | | 430 (M⁺+1) |
| D-a-38 | 1-a | D-a-37 | | cPenO | H | 6-Qu | H | C | | 402 (M⁺+1) |
| D-a-39 | 4e-1 | Int.d-7 | BRA1 | BnO | Et | H | 2-Nap | C | | 451 (M⁺+1) |
| D-a-40 | 1-a | D-a-39 | | BnO | H | H | 2-Nap | C | | 423 (M⁺+1) |
| D-a-41 | 4e-1 | Int.d-7 | BRA2 | BnO | Et | H | 5-Ind | C | | 440 (M⁺+1) |
| D-a-42 | 1-a | D-a-41 | | BnO | H | H | 5-Ind | C | | 412 (M⁺+1) |
| D-a-43 | 4e-1 | Int.d-7 | BRA3 | BnO | Et | H | 1Me-5-Ind | C | | 454 (M⁺+1) |
| D-a-44 | 1-a | D-a-43 | | BnO | H | H | 1Me-5-Ind | C | | 426 (M⁺+1) |

**[Table 204]**

| Table-D-a-2 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| D-a-45 | 4e-1 | Int.d-7 | BRA4 | BnO | Et | H | 1 Me-4-Ind | C | | 454 (M⁺+1) |
| D-a-46 | 1-a | D-a-45 | | BnO | H | H | 1Me-4-Ind | C | | 426 (M⁺+1) |
| D-a-47 | 4e-1 | Int.d-7 | BRA6 | BnO | Et | H | 1Me-6-Ind | C | | 454 (M⁺+1) |
| D-a-48 | 1-a | D-a-47 | | BnO | H | H | 1Me-6-Ind | C | | 426 (M⁺+1) |
| D-a-49 | 4e-1 | Int.d-7 | BRA7 | BnO | Et | H | 5-1Idz | C | | 441 (M⁺+1) |
| D-a-50 | 1-a | D-a-49 | | BnO | H | H | 5-1Idz | C | | 413 (M⁺+1) |
| D-a-51 | 4e-1 | Int.d-7 | BRA8 | BnO | Et | H | 1Me-5-Idz | C | | 455 (M⁺+1) |
| D-a-52 | 1-a | D-a-51 | | BnO | H | H | 1Me-5-Idz | C | | 427 (M⁺+1) |
| D-a-53 | 4e-1 | Int.d-7 | BRA9 | BnO | Et | H | 1Et-5-Idz | C | | 469 (M⁺+1) |
| D-a-54 | 1-a | D-a-53 | | BnO | H | H | 1Et-5-Idz | C | | 441 (M⁺+1) |
| D-a-55 | 4e-1 | Int.d-7 | Het2 | BnO | Et | H | 3-Qu | C | | 452 (M⁺+1) |
| D-a-56 | 1-a | D-a-55 | | BnO | H | H | 3-Qu | C | | 424 (M⁺+1) |
| D-a-57 | 4e-1 | Int.d-7 | BRA13 | BnO | Et | H | 6-Q | C | | 452 (M⁺+1) |
| D-a-58 | 1-a | D-a-57 | | BnO | H | H | 6-Q | C | | 424 (M⁺+1) |
| D-a-59 | 4e-2 | Int.d-7 | Het3 | BnO | Et | H | 6-IQ | C | | 452 (M⁺+1) |
| D-a-60 | 1-a | D-a-59 | | BnO | H | H | 6-IQ | C | | 424 (M⁺+1) |
| D-a-61 | 4e-1 | Int.d-8 | BRA1 | BnO | Et | 2-Nap | H | C | | 451 (M⁺+1) |
| D-a-62 | 1-a | D-a-61 | | BnO | H | 2-Nap | H | C | | 423 (M⁺+1) |
| D-a-63 | 4e-1 | Int.d-8 | BRA2 | BnO | Et | 5-Ind | H | C | | 440 (M⁺+1) |
| D-a-64 | 1-a | D-a-63 | | BnO | H | 5-Ind | H | C | | 412 (M⁺+1) |
| D-a-65 | 4e-1 | Int.d-8 | BRA3 | BnO | Et | 1Me-5-Ind | H | C | | 454 (M⁺+1) |
| D-a-66 | 1-a | D-a-65 | | BnO | H | 1Me-5-Ind | H | C | | 426 (M⁺+1) |
| D-a-67 | 4e-1 | Int.d-8 | BRA6 | BnO | Et | 1Me-6-Ind | H | C | | 454 (M⁺+1) |
| D-a-68 | 1-a | D-a-67 | | BnO | H | 1Me-6-Ind | H | C | | 426 (M⁺+1) |
| D-a-69 | 4e-1 | Int.d-8 | BRA8 | BnO | Et | 1Me-5-Idz | H | C | | 455 (M⁺+1) |
| D-a-70 | 1-a | D-a-69 | | BnO | H | 1Me-5-Idz | H | C | | 427 (M⁺+1) |
| D-a-71 | 4e-1 | Int.d-8 | BRA9 | BnO | Et | 1Et-5-Idz | H | C | | 469 (M⁺+1) |
| D-a-72 | 1-a | D-a-71 | | BnO | H | 1Et-5-Idz | H | C | | 441 (M⁺+1) |
| D-a-73 | 4e-1 | Int.d-10 | BRA1 | cPenMeO | Et | H | 2-Nap | C | | 443 (M⁺+1) |
| D-a-74 | 1-a | D-a-73 | | cPenMeO | H | H | 2-Nap | C | | 415 (M⁺+1) |
| D-a-75 | 4e-1 | Int.d-10 | BRA3 | cPenMeO | Et | H | 1Me-5-Ind | C | | 446 (M⁺+1) |
| D-a-76 | 1-a | D-a-75 | | cPenMeO | H | H | 1Me-5-Ind | C | | 418 (M⁺+1) |
| D-a-77 | 4e-1 | Int.d-10 | BRA8 | cPenMeO | Et | H | 1Me-5-Idz | C | | 447 (M⁺+1) |
| D-a-78 | 1-a | D-a-77 | | cPenMeO | H | H | 1Me-5-Idz | C | | 419 (M⁺+1) |
| D-a-79 | 4e-1 | Int.d-11 | BRA2 | cPenMeO | Et | 5-Ind | H | C | | 432 (M⁺+1) |
| D-a-80 | 1-a | D-a-79 | | cPenMeO | H | 5-Ind | H | C | | 404 (M⁺+1) |
| D-a-81 | 4e-1 | Int.d-11 | BRA3 | cPenMeO | Et | 1Me-5-Ind | H | C | | 446 (M⁺+1) |
| D-a-82 | 1-a | D-a-81 | | cPenMeO | H | 1Me-5-Ind | H | C | | 418 (M⁺+1) |
| D-a-83 | 4e-1 | Int.d-11 | BRA9 | cPenMeO | Et | 1Et-5-Idz | H | C | | 461 (M⁺+1) |
| D-a-84 | 1-a | D-a-83 | | cPenMeO | H | 1Et-5-Idz | H | C | | 433 (M⁺+1) |
| D-a-85 | 4e-1 | Int.d-13 | BRA3 | cHexMeO | Et | H | 1Me-5-Ind | C | | 460 (M⁺+1) |
| D-a-86 | 1-a | D-a-85 | | cHexMeO | H | H | 1Me-5-Ind | C | | 432 (M⁺+1) |
| D-a-87 | 4e-1 | Int.d-13 | BRA9 | cHexMeO | Et | H | 1Et-5-Idz | C | | 475 (M⁺+1) |
| D-a-88 | 1-a | D-a-87 | | cHexMeO | H | H | 1Et-5-Idz | C | | 447 (M⁺+1) |
| D-a-89 | 4e-1 | Int.d-13 | BRA13 | cHexMeO | Et | H | 6-Q | C | | 458 (M⁺+1) |
| D-a-90 | 1-a | D-a-89 | | cHexMeO | H | H | 6-Qu | C | | 430 (M⁺+1) |
| D-a-91 | 4e-2 | Int.d-13 | Het3 | cHexMeO | Et | H | 6-IQ | C | | 458 (M⁺+1) |
| D-a-92 | 1-a | D-a-91 | | cHexMeO | H | H | 6-IQ | C | | 430 (M⁺+1) |

**[Table 205]**

| Table-D-a-3 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| D-a-93 | 4e-1 | Intd-14 | BRA1 | cHexMeO | Et | 2-Nap | H | C | | 457 (M⁺+1) |
| D-a-94 | 1-a | D-a-93 | | cHexMeO | H | 2-Nap | H | C | | 429 (M⁺+1) |
| D-a-95 | 4e-1 | Int.d-14 | BRA5 | cHexMeO | Et | 1Me-4-Ind | H | C | | 460 (M⁺+1) |
| D-a-96 | 1-a | D-a-95 | | cHexMeO | H | 1Me-4-Ind | H | C | | 432 (M⁺+1) |
| D-a-97 | 4e-1 | Int.d-14 | BRA9 | cHexMeO | Et | 1Et-5-Idz | H | C | | 475 (M⁺+1) |
| D-a-98 | 1-a | D-a-97 | | cHexMeO | H | 1Et-5-Idz | H | C | | 447 (M⁺+1) |
| D-a-99 | 4e-1 | Int.d-16 | BRA1 | cHexO | Et | H | 2-Nap | C | | 443 (M⁺+1) |
| D-a-100 | 1-a | D-a-99 | | cHexO | H | H | 2-Nap | C | | 415 (M⁺+1) |
| D-a-101 | 4e-1 | Int.d-16 | BRA2 | cHexO | Et | H | 5-Ind | C | | 432 (M⁺+1) |
| D-a-102 | 1-a | D-a-101 | | cHexO | H | H | 5-Ind | C | | 404 (M⁺+1) |
| D-a-103 | 4e-1 | Int.d-16 | BRA4 | cHexO | Et | H | 1Et-5-Ind | C | | 460 (M⁺+1) |
| D-a-104 | 1-a | D-a-103 | | cHexO | H | H | 1Et-5-Ind | C | | 432 (M⁺+1) |
| D-a-105 | 4e-1 | Int.d-16 | BRA7 | cHexO | Et | H | 5-1Idz | C | | 433 (M⁺+1) |
| D-a-106 | 1-a | D-a-105 | | cHexO | H | H | 5-1Idz | C | | 405 (M⁺+1) |
| D-a-107 | 4e-1 | Int.d-17 | BRA2 | cHexO | Et | 5-Ind | H | C | | 432 (M⁺+1) |
| D-a-108 | 1-a | D-a-107 | | cHexO | H | 5-Ind | H | C | | 404 (M⁺+1) |
| D-a-109 | 4e-1 | Int.d-17 | BRA3 | cHexO | Et | 1Me-5-Ind | H | C | | 446 (M⁺+1) |
| D-a-110 | 1-a | D-a-109 | | cHexO | H | 1Me-5-Ind | H | C | | 418 (M⁺+1) |
| D-a-111 | 4e-1 | Int.d-17 | BRA8 | cHexO | Et | 1Me-5-Idz | H | C | | 447 (M⁺+1) |
| D-a-112 | 1-a | D-a-111 | | cHexO | H | 1Me-5-Idz | H | C | | 419 (M⁺+1) |
| D-a-113 | 4e-1 | Int.d-17 | BRA9 | cHexO | Et | 1Et-5-Idz | H | C | | 461 (M⁺+1) |
| D-a-114 | 1-a | D-a-113 | | cHexO | H | 1Et-5-Idz | H | C | | 433 (M⁺+1) |
| D-a-115 | 4e-1 | Int.d-19 | BRA3 | nPrO | Et | H | 1Me-5-Ind | C | | 406 (M⁺+1) |
| D-a-116 | 1-a | D-a-115 | | nPrO | H | H | 1Me-5-Ind | C | | 378 (M⁺+1) |
| D-a-117 | 4e-1 | Int.d-19 | BRA4 | nPrO | Et | H | 1Et-5-Ind | C | | 420 (M⁺+1) |
| D-a-118 | 1-a | D-a-117 | | nPrO | H | H | 1Et-5-Ind | C | | 392 (M⁺+1) |
| D-a-119 | 4e-1 | Int.d-19 | BRA7 | nPrO | Et | H | 5-1Idz | C | | 393 (M⁺+1) |
| D-a-120 | 1-a | D-a-119 | | nPrO | H | H | 5-1Idz | C | | 365 (M⁺+1) |
| D-a-121 | 4e-1 | Int.d-20 | BRA1 | nPrO | Et | 2-Nap | H | C | | 403 (M⁺+1) |
| D-a-122 | 1-a | D-a-121 | | nPrO | H | 2-Nap | H | C | | 375 (M⁺+1) |
| D-a-123 | 4e-1 | Int.d-20 | BRA4 | nPrO | Et | 1Et-5-Ind | H | C | | 420 (M⁺+1) |
| D-a-124 | 1-a | D-a-123 | | nPrO | H | 1Et-5-Ind | H | C | | 392 (M⁺+1) |
| D-a-125 | 4e-1 | Int.d-20 | BRA7 | nPrO | Et | 5-1Idz | H | C | | 393 (M⁺+1) |
| D-a-126 | 1-a | D-a-125 | | nPrO | H | 5-1Idz | H | C | | 365 (M⁺+1) |
| D-a-127 | 4e-1 | Int.d-22 | BRA1 | iPrO | Et | H | 2-Nap | C | | 403 (M⁺+1) |
| D-a-128 | 1-a | D-a-127 | | iPrO | H | H | 2-Nap | C | | 375 (M⁺+1) |
| D-a-129 | 4e-1 | Int.d-22 | BRA3 | iPrO | Et | H | 1Me-5-Ind | C | | 406 (M⁺+1) |
| D-a-130 | 1-a | D-a-129 | | iPrO | H | H | 1Me-5-Ind | C | | 378 (M⁺+1) |
| D-a-131 | 4e-1 | Int.d-22 | BRA7 | iPrO | Et | H | 5-1Idz | C | | 393 (M⁺+1) |
| D-a-132 | 1-a | D-a-131 | | iPrO | H | H | 5-1Idz | C | | 365 (M⁺+1) |
| D-a-133 | 4e-1 | Int.d-22 | BRA9 | iPrO | Et | H | 1Et-5-Idz | C | | 421 (M⁺+1) |
| D-a-134 | 1-a | D-a-133 | | iPrO | H | H | 1Et-5-Idz | C | | 393 (M⁺+1) |
| D-a-135 | 4e-1 | Int.d-22 | BRA13 | iPrO | Et | H | 6-Qu | C | | 404 (M⁺+1) |
| D-a-136 | 1-a | D-a-135 | | iPrO | H | H | 6-Qu | C | | 376 (M⁺+1) |
| D-a-137 | 4e-1 | Int.d-23 | BRA1 | iPrO | Et | 2-Nap | H | C | | 403 (M⁺+1) |
| D-a-138 | 1-a | D-a-137 | | iPrO | H | 2-Nap | H | C | | 375 (M⁺+1) |
| D-a-139 | 4e-1 | Int.d-23 | BRA8 | iPrO | Et | 1Me-5-Idz | H | C | | 407 (M⁺+1) |
| D-a-140 | 1-a | D-a-139 | | iPrO | H | 1Me-5-Idz | H | C | | 379 (M⁺+1) |

**[Table 206]**

| Table-D-a-4 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| D-a-141 | 4f-2 | Int.d-24 | Al4 | cHepO | Et | H | 2-Nap | C | | 457 (M⁺+1) |
| D-a-142 | 1-a | D-a-141 | | cHepO | H | H | 2-Nap | C | | 429 (M⁺+1) |
| D-a-143 | 4f-2 | Int.d-25 | Al4 | cHepO | Et | H | 5-Ind | C | | 446 (M⁺+1) |
| D-a-144 | 1-a | D-a-143 | | cHepO | H | H | 5-Ind | C | | 418 (M⁺+1) |
| D-a-145 | 4f-2 | Int.d-27 | Al4 | cHepO | Et | H | 1Me-4-Ind | C | | 460 (M⁺+1) |
| D-a-146 | 1-a | D-a-145 | | cHepO | H | H | 1Me-4-Ind | C | | 432 (M⁺+1) |
| D-a-147 | 4f-2 | Int.d-30 | Al4 | cHepO | Et | H | 1Me-5-Idz | C | | 461 (M⁺+1) |
| D-a-148 | 1-a | D-a-147 | | cHepO | H | H | 1Me-5-Idz | C | | 433 (M⁺+1) |
| D-a-149 | 4f-2 | Int.d-32 | Al4 | cHepO | Et | H | 3-Qu | C | | 458 (M⁺+1) |
| D-a-150 | 1-a | D-a-149 | | cHepO | H | H | 3-Qu | C | | 430 (M⁺+1) |
| D-a-151 | 4f-2 | Int.d-35 | Al4 | cHepO | Et | 2-Nap | H | C | | 457 (M⁺+1) |
| D-a-152 | 1-a | D-a-151 | | cHepO | H | 2-Nap | H | C | | 429 (M⁺+1) |
| D-a-153 | 4f-2 | Int.d-37 | Al4 | cHepO | Et | 1Me-5-Ind | H | C | | 460 (M⁺+1) |
| D-a-154 | 1-a | D-a-153 | | cHepO | H | 1Me-5-Ind | H | C | | 432 (M⁺+1) |
| D-a-155 | 4f-2 | Int.d-38 | Al4 | cHepO | Et | 1Me-6-Ind | H | C | | 460 (M⁺+1) |
| D-a-156 | 1-a | D-a-155 | | cHepO | H | 1Me-6-Ind | H | C | | 432 (M⁺+1) |
| D-a-157 | 4f-1 | Int.d-24 | Hal5 | nBuO | Et | H | 2-Nap | C | | 417 (M⁺+1) |
| D-a-158 | 1-a | D-a-157 | | nBuO | H | H | 2-Nap | C | | 389 (M⁺+1) |
| D-a-159 | 4f-1 | Int.d-26 | Hal5 | nBuO | Et | H | 1Me-5-Ind | C | | 420 (M⁺+1) |
| D-a-160 | 1-a | D-a-159 | | nBuO | H | H | 1Me-5-Ind | C | | 392 (M⁺+1) |
| D-a-161 | 4f-1 | Int.d-28 | Hal5 | nBuO | Et | H | 1Me-6-Ind | C | | 420 (M⁺+1) |
| D-a-162 | 1-a | D-a-161 | | nBuO | H | H | 1Me-6-Ind | C | | 392 (M⁺+1) |
| D-a-163 | 4f-1 | Int.d-29 | Hal5 | nBuO | Et | H | 5-1Idz | C | | 407 (M⁺+1) |
| D-a-164 | 1-a | D-a-163 | | nBuO | H | H | 5-1Idz | C | | 379 (M⁺+1) |
| D-a-165 | 4f-1 | Int.d-31 | Hal5 | nBuO | Et | H | 1Et-5-Idz | C | | 435 (M⁺+1) |
| D-a-166 | 1-a | D-a-165 | | nBuO | H | H | 1Et-5-Idz | C | | 407 (M⁺+1) |
| D-a-167 | 4f-1 | Int.d-36 | Hal5 | nBuO | Et | 5-Ind | H | C | | 406 (M⁺+1) |
| D-a-168 | 1-a | D-a-167 | | nBuO | H | 5-Ind | H | C | | 378 (M⁺+1) |
| D-a-169 | 4f-1 | Int.d-37 | Hal5 | nBuO | Et | 1Me-5-Ind | H | C | | 420 (M⁺+1) |
| D-a-170 | 1-a | D-a-169 | | nBuO | H | 1Me-5-Ind | H | C | | 392 (M⁺+1) |
| D-a-171 | 4f-1 | Int.d-39 | Hal5 | nBuO | Et | 1Me-5-Idz | H | C | | 421 (M⁺+1) |
| D-a-172 | 1-a | D-a-171 | | nBuO | H | 1Me-5-Idz | H | C | | 393 (M⁺+1) |
| D-a-173 | 4f-1 | Int.d-24 | Hal6 | iBuO | Et | H | 2-Nap | C | | 417 (M⁺+1) |
| D-a-174 | 1-a | D-a-173 | | iBuO | H | H | 2-Nap | C | | 389 (M⁺+1) |
| D-a-175 | 4f-1 | Int.d-25 | Hal6 | iBuO | Et | H | 5-Ind | C | | 406 (M⁺+1) |
| D-a-176 | 1-a | D-a-175 | | iBuO | H | H | 5-Ind | C | | 378 (M⁺+1) |
| D-a-177 | 4f-1 | Int.d-26 | Hal6 | iBuO | Et | H | 1Me-5-Ind | C | | 420 (M⁺+1) |
| D-a-178 | 1-a | D-a-177 | | iBuO | H | H | 1Me-5-Ind | C | | 392 (M⁺+1) |
| D-a-179 | 4f-1 | Int.d-31 | Hal6 | iBuO | Et | H | 1Et-5-Idz | C | | 435 (M⁺+1) |
| D-a-180 | 1-a | D-a-179 | | iBuO | H | H | 1Et-5-Idz | C | | 407 (M⁺+1) |
| D-a-181 | 4f-1 | Int.d-36 | Hal6 | iBuO | Et | 5-Ind | H | C | | 406 (M⁺+1) |
| D-a-182 | 1-a | D-a-181 | | iBuO | H | 5-Ind | H | C | | 378 (M⁺+1) |
| D-a-183 | 4f-1 | Int.d-37 | Hal6 | iBuO | Et | 1Me-5-Ind | H | C | | 420 (M⁺+1) |
| D-a-184 | 1-a | D-a-183 | | iBuO | H | 1Me-5-Ind | H | C | | 392 (M⁺+1) |
| D-a-185 | 4f-1 | Int.d-40 | Hal6 | iBuO | Et | 1Et-5-Idz | H | C | | 435 (M⁺+1) |
| D-a-186 | 1-a | D-a-185 | | iBuO | H | 1Et-5-Idz | H | C | | 407 (M⁺+1) |
| D-a-187 | 4f-1 | Int.d-25 | Hal7 | 2FBnO | Et | H | 5-Ind | C | | 458 (M⁺+1) |
| D-a-188 | 1-a | D-a-187 | | 2FBnO | H | H | 5-Ind | C | | 430 (M⁺+1) |

**[Table 207]**

| Table-D-a-5 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| D-a-189 | 4f-1 | Int.d-27 | Hal7 | 2FBnO | Et | H | 1 Me-4-Ind | C | | 472 (M⁺+1) |
| D-a-190 | 1-a | D-a-189 | | 2FBnO | H | H | 1Me-4-Ind | C | | 444 (M⁺+1) |
| D-a-191 | 4f-1 | Int.d-28 | Hal7 | 2FBnO | Et | H | 1Me-6-Ind | C | | 472 (M⁺+1) |
| D-a-192 | 1-a | D-a-191 | | 2FBnO | H | H | 1Me-6-Ind | C | | 444 (M⁺+1) |
| D-a-193 | 4f-1 | Int.d-31 | Hal7 | 2FBnO | Et | H | 1Et-5-Idz | C | | 487 (M⁺+1) |
| D-a-194 | 1-a | D-a-193 | | 2FBnO | H | H | 1Et-5-Idz | C | | 459 (M⁺+1) |
| D-a-195 | 4f-1 | Int.d-34 | Hal7 | 2FBnO | Et | H | 6-IQ | C | | 470 (M⁺+1) |
| D-a-196 | 1-a | D-a-195 | | 2FBnO | H | H | 6-IQ | C | | 442 (M⁺+1) |
| D-a-197 | 4f-1 | Int.d-35 | Hal7 | 2FBnO | Et | 2-Nap | H | C | | 469 (M⁺+1) |
| D-a-198 | 1-a | D-a-197 | | 2FBnO | H | 2-Nap | H | C | | 441 (M⁺+1) |
| D-a-199 | 4f-1 | Int.d-37 | Hal7 | 2FBnO | Et | 1Me-5-Ind | H | C | | 472 (M⁺+1) |
| D-a-200 | 1-a | D-a-199 | | 2FBnO | H | 1Me-5-Ind | H | C | | 444 (M⁺+1) |
| D-a-201 | 4f-1 | Int.d-40 | Hal7 | 2FBnO | Et | 1 Et-5-Idz | H | C | | 487 (M⁺+1) |
| D-a-202 | 1-a | D-a-201 | | 2FBnO | H | 1Et-5-Idz | H | C | | 459 (M⁺+1) |
| D-a-203 | 4f-1 | Int.d-24 | Hal9 | 4FBnO | Et | H | 2-Nap | C | | 469 (M⁺+1) |
| D-a-204 | 1-a | D-a-203 | | 4FBnO | H | H | 2-Nap | C | | 441 (M⁺+1) |
| D-a-205 | 4f-1 | Int.d-26 | Hal9 | 4FBnO | Et | H | 1Me-5-Ind | C | | 472 (M⁺+1) |
| D-a-206 | 1-a | D-a-205 | | 4FBnO | H | H | 1Me-5-Ind | C | | 444 (M⁺+1) |
| D-a-207 | 4f-1 | Int.d-29 | Hal9 | 4FBnO | Et | H | 5-1Idz | C | | 459 (M⁺+1) |
| D-a-208 | 1-a | D-a-207 | | 4FBnO | H | H | 5-1Idz | C | | 431 (M⁺+1) |
| D-a-209 | 4f-1 | Int.d-37 | Hal9 | 4FBnO | Et | 1 Me-5-Ind | H | C | | 472 (M⁺+1) |
| D-a-210 | 1-a | D-a-209 | | 4FBnO | H | 1Me-5-Ind | H | C | | 444 (M⁺+1) |
| D-a-211 | 4f-1 | Int.d-38 | Hal9 | 4FBnO | Et | 1Me-6-Ind | H | C | | 472 (M⁺+1) |
| D-a-212 | 1-a | D-a-211 | | 4FBnO | H | 1Me-6-Ind | H | C | | 444 (M⁺+1) |
| D-a-213 | 4f-1 | Int.d-40 | Hal9 | 4FBnO | Et | 1Et-5-Idz | H | C | | 487 (M⁺+1) |
| D-a-214 | 1-a | D-a-213 | | 4FBnO | H | 1Et-5-Idz | H | C | | 459 (M⁺+1) |
| D-a-215 | 4f-1 | Int.d-24 | Hal11 | 3ClBnO | Et | H | 2-Nap | C | | 485 (M⁺+1) |
| D-a-216 | 1-a | D-a-215 | | 3ClBnO | H | H | 2-Nap | C | | 457 (M⁺+1) |
| D-a-217 | 4f-1 | Int.d-26 | Hal11 | 3ClBnO | Et | H | 1Me-5-Ind | C | | 488 (M⁺+1) |
| D-a-218 | 1-a | D-a-217 | | 3ClBnO | H | H | 1Me-5-Ind | C | | 460 (M⁺+1) |
| D-a-219 | 4f-1 | Int.d-29 | Hal11 | 3ClBnO | Et | H | 5-1Idz | C | | 475 (M⁺+1) |
| D-a-220 | 1-a | D-a-219 | | 3ClBnO | H | H | 5-1Idz | C | | 447 (M⁺+1) |
| D-a-221 | 4f-1 | Int.d-37 | Hal11 | 3ClBnO | Et | 1 Me-5-Ind | H | C | | 488 (M⁺+1) |
| D-a-222 | 1-a | D-a-221 | | 3ClBnO | H | 1 Me-5-Ind | H | C | | 460 (M⁺+1) |
| D-a-223 | 4f-1 | Int.d-38 | Hal11 | 3ClBnO | Et | 1Me-6-Ind | H | C | | 488 (M⁺+1) |
| D-a-224 | 1-a | D-a-223 | | 3ClBnO | H | 1Me-6-Ind | H | C | | 460 (M⁺+1) |
| D-a-225 | 4f-1 | Int.d-40 | Hal11 | 3ClBnO | Et | 1 Et-5-Idz | H | C | | 503 (M⁺+1) |
| D-a-226 | 1-a | D-a-225 | | 3ClBnO | H | 1Et-5-Idz | H | C | | 475 (M⁺+1) |
| D-a-227 | 4f-1 | Int.d-25 | Hal12 | 4MeBnO | Et | H | 5-Ind | C | | 454 (M⁺+1) |
| D-a-228 | 1-a | D-a-227 | | 4MeBnO | H | H | 5-Ind | C | | 426 (M⁺+1) |
| D-a-229 | 4f-1 | Int.d-27 | Hal12 | 4MeBnO | Et | H | 1Me-4-Ind | C | | 468 (M⁺+1) |
| D-a-230 | 1-a | D-a-229 | | 4MeBnO | H | H | 1Me-4-Ind | C | | 440 (M⁺+1) |
| D-a-231 | 4f-1 | Int.d-30 | Hal12 | 4MeBnO | Et | H | 1Me-5-Idz | C | | 469 (M⁺+1) |
| D-a-232 | 1-a | D-a-231 | | 4MeBnO | H | H | 1Me-5-Idz | C | | 441 (M⁺+1) |
| D-a-233 | 4f-1 | Int.d-35 | Hal12 | 4MeBnO | Et | 2-Nap | H | C | | 465 (M⁺+1) |
| D-a-234 | 1-a | D-a-233 | | 4MeBnO | H | 2-Nap | H | C | | 437 (M⁺+1) |
| D-a-235 | 4f-1 | Int.d-38 | Hal12 | 4MeBnO | Et | 1Me-6-Ind | H | C | | 468 (M⁺+1) |
| D-a-236 | 1-a | D-a-235 | | 4MeBnO | H | 1 Me-6-Ind | H | C | | 440 (M⁺+1) |

**[Table 208]**

| Table-D-a-6 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| D-a-237 | 4f-2 | Int.d-24 | Al11 | 4Me,cHexO | Et | H | 2-Nap | C | | 457 (M⁺+1) |
| D-a-238 | 1-a | D-a-237 | | 4Me,cHexO | H | H | 2-Nap | C | | 429 (M⁺+1) |
| D-a-239 | 4f-2 | Int.d-28 | Al11 | 4Me,cHexO | Et | H | 1Me-6-Ind | C | | 460 (M⁺+1) |
| D-a-240 | 1-a | D-a-239 | | 4Me,cHexO | H | H | 1Me-6-Ind | C | | 432 (M⁺+1) |
| D-a-241 | 4f-2 | Int.d-30 | Al11 | 4Me,cHexO | Et | H | 1Me-5-Idz | C | | 461 (M⁺+1) |
| D-a-242 | 1-a | D-a-241 | | 4Me,cHexO | H | H | 1Me-5-Idz | C | | 433 (M⁺+1) |
| D-a-243 | 4f-2 | Int.d-32 | Al11 | 4Me,cHexO | Et | H | 3-Qu | C | | 458 (M⁺+1) |
| D-a-244 | 1-a | D-a-243 | | 4Me,cHexO | H | H | 3-Qu | C | | 430 (M⁺+1) |
| D-a-245 | 4f-2 | Int.d-36 | Al11 | 4Me,cHexO | Et | 5-Ind | H | C | | 446 (M⁺+1) |
| D-a-246 | 1-a | D-a-245 | | 4Me,cHexO | H | 5-Ind | H | C | | 418 (M⁺+1) |
| D-a-247 | 4f-2 | Int.d-37 | Al11 | 4Me,cHexO | Et | 1Me-5-Ind | H | C | | 460 (M⁺+1) |
| D-a-248 | 1-a | D-a-247 | | 4Me,cHexO | H | 1Me-5-Ind | H | C | | 432 (M⁺+1) |
| D-a-249 | 4f-2 | Int.d-24 | Al6 | 2(4DMAPh)EtO | Et | H | 2-Nap | C | | 508 (M⁺+1) |
| D-a-250 | 1-a | D-a-249 | | 2(4DMAPh)EtO | H | H | 2-Nap | C | | 480 (M⁺+1) |
| D-a-251 | 4f-2 | Int.d-25 | Al6 | 2(4DMAPh)EtO | Et | H | 5-Ind | C | | 497 (M⁺+1) |
| D-a-252 | 1-a | D-a-251 | | 2(4DMAPh)EtO | H | H | 5-Ind | C | | 469 (M⁺+1) |
| D-a-253 | 4f-2 | Int.d-29 | Al6 | 2(4DMAPh)EtO | Et | H | 5-1Idz | C | | 498 (M⁺+1) |
| D-a-254 | 1-a | D-a-253 | | 2(4DMAPh)EtO | H | H | 5-1Idz | C | | 470 (M⁺+1) |
| D-a-255 | 4f-2 | Int.d-34 | Al6 | 2(4DMAPh)EtO | Et | H | 6-IQ | C | | 509 (M⁺+1) |
| D-a-256 | 1-a | D-a-255 | | 2(4DMAPh)EtO | H | H | 6-IQ | C | | 481 (M⁺+1) |
| D-a-257 | 4f-2 | Int.d-35 | Al6 | 2(4DMAPh)EtO | Et | 2-Nap | H | C | | 508 (M⁺+1) |
| D-a-258 | 1-a | D-a-257 | | 2(4DMAPh)EtO | H | 2-Nap | H | C | | 480 (M⁺+1) |
| D-a-259 | 4f-2 | Int.d-36 | Al6 | 2(4DMAPh)EtO | Et | 5-Ind | H | C | | 497 (M⁺+1) |
| D-a-260 | 1-a | D-a-259 | | 2(4DMAPh)EtO | H | 5-Ind | H | C | | 469 (M⁺+1) |
| D-a-261 | 4f-2 | Int.d-39 | Al6 | 2(4DMAPh)EtO | Et | 1Me-5-Idz | H | C | | 512 (M⁺+1) |
| D-a-262 | 1-a | D-a-261 | | 2(4DMAPh)EtO | H | 1Me-5-Idz | H | C | | 484 (M⁺+1) |
| D-a-263 | 4f-2 | Int.d-24 | Al8 | 1PhEtO | Et | H | 2-Nap | C | | 465 (M⁺+1) |
| D-a-264 | 1-a | D-a-263 | | 1PhEtO | H | H | 2-Nap | C | | 437 (M⁺+1) |
| D-a-265 | 4f-2 | Int.d-26 | Al8 | 1PhEtO | Et | H | 1Me-5-Ind | C | | 468 (M⁺+1) |
| D-a-266 | 1-a | D-a-265 | | 1PhEtO | H | H | 1Me-5-Ind | C | | 440 (M⁺+1) |
| D-a-267 | 4f-2 | Int.d-28 | Al8 | 1PhEtO | Et | H | 1Me-6-Ind | C | | 468 (M⁺+1) |
| D-a-268 | 1-a | D-a-267 | | 1PhEtO | H | H | 1Me-6-Ind | C | | 440 (M⁺+1) |
| D-a-269 | 4f-2 | Int.d-31 | Al8 | 1PhEtO | Et | H | 1Et-5-Idz | C | | 483 (M⁺+1) |
| D-a-270 | 1-a | D-a-269 | | 1PhEtO | H | H | 1Et-5-Idz | C | | 455 (M⁺+1) |
| D-a-271 | 4f-2 | Int.d-33 | Al8 | 1PhEtO | Et | H | 6-Qu | C | | 466 (M⁺+1) |
| D-a-272 | 1-a | D-a-271 | | 1PhEtO | H | H | 6-Qu | C | | 438 (M⁺+1) |
| D-a-273 | 4f-2 | Int.d-36 | Al8 | 1PhEtO | Et | 5-Ind | H | C | | 454 (M⁺+1) |
| D-a-274 | 1-a | D-a-273 | | 1PhEtO | H | 5-Ind | H | C | | 426 (M⁺+1) |
| D-a-275 | 4f-2 | Int.d-40 | Al8 | 1PhEtO | Et | 1Et-5-Idz | H | C | | 483 (M⁺+1) |
| D-a-276 | 1-a | D-a-275 | | 1PhEtO | H | 1Et-5-Idz | H | C | | 455 (M⁺+1) |
| D-a-277 | 4f-2 | Int.d-24 | Al9 | 1(2FPh)EtO | Et | H | 2-Nap | C | | 483 (M⁺+1) |
| D-a-278 | 1-a | D-a-277 | | 1(2FPh)EtO | H | H | 2-Nap | C | | 455 (M⁺+1) |
| D-a-279 | 4f-2 | Int.d-25 | Al9 | 1(2FPh)EtO | Et | H | 5-Ind | C | | 472 (M⁺+1) |
| D-a-280 | 1-a | D-a-279 | | 1(2FPh)EtO | H | H | 5-Ind | C | | 444 (M⁺+1) |
| D-a-281 | 4f-2 | Int.d-31 | Al9 | 1(2FPh)EtO | Et | H | 1Et-5-Idz | C | | 501 (M⁺+1) |
| D-a-282 | 1-a | D-a-281 | | 1(2FPh)EtO | H | H | 1Et-5-Idz | C | | 473 (M⁺+1) |
| D-a-283 | 4f-2 | Int.d-35 | Al9 | 1(2FPh)EtO | Et | 2-Nap | H | C | | 483 (M⁺+1) |
| D-a-284 | 1-a | D-a-283 | | 1(2FPh)EtO | H | 2-Nap | H | C | | 455 (M⁺+1) |

**[Table 209]**

| Table-D-a-7 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| D-a-285 | 4f-2 | Int.d-24 | Al17 | 1IndanO | Et | H | 2-Nap | C | | 477 (M⁺+1) |
| D-a-286 | 1-a | D-a-285 | | 1IndanO | H | H | 2-Nap | C | | 449 (M⁺+1) |
| D-a-287 | 4f-2 | Int.d-25 | Al17 | 1IndanO | Et | H | 5-Ind | C | | 466 (M⁺+1) |
| D-a-288 | 1-a | D-a-287 | | 1IndanO | H | H | 5-Ind | C | | 438 (M⁺+1) |
| D-a-289 | 4f-2 | Int.d-26 | Al17 | 1IndanO | Et | H | 1Me-5-Ind | C | | 480 (M⁺+1) |
| D-a-290 | 1-a | D-a-289 | | 1IndanO | H | H | 1Me-5-In | C | | 452 (M⁺+1) |
| D-a-291 | 4f-2 | Int.d-30 | Al17 | 1IndenO | Et | H | 1Me-5-Idz | C | | 481 (M⁺+1) |
| D-a-292 | 1-a | D-a-291 | | 1IndanO | H | H | 1Me-5-Idz | C | | 453 (M⁺+1) |
| D-a-293 | 4f-2 | Int.d-31 | Al17 | 1IndanO | Et | H | 1Et-5-Idz | C | | 495 (M⁺+1) |
| D-a-294 | 1-a | D-a-293 | | 1IndanO | H | H | 1Et-5-Idz | C | | 467 (M⁺+1) |
| D-a-295 | 4f-2 | Int.d-35 | Al17 | 1IndanO | Et | 2-Nap | H | C | | 477 (M⁺+1) |
| D-a-296 | 1-a | D-a-295 | | 1IndanO | H | 2-Nap | H | C | | 449 (M⁺+1) |
| D-a-297 | 4f-2 | Int.d-39 | Al17 | 1IndanO | Et | 1Me-5-Idz | H | C | | 481 (M⁺+1) |
| D-a-298 | 1-a | D-a-297 | | 1IndanO | H | 1Me-5-Idz | H | C | | 453 (M⁺+1) |
| D-a-299 | 4f-2 | Int.d-24 | Al18 | 2IndanO | Et | H | 2-Nap | C | | 477 (M⁺+1) |
| D-a-300 | 1-a | D-a-299 | | 2IndanO | H | H | 2-Nap | C | | 449 (M⁺+1) |
| D-a-301 | 4f-2 | Int.d-26 | Al18 | 2IndanO | Et | H | 1Me-5-Ind | C | | 480 (M⁺+1) |
| D-a-302 | 1-a | D-a-301 | | 2IndanO | H | H | 1Me-5-Ind | C | | 452 (M⁺+1) |
| D-a-303 | 4f-2 | Int.d-29 | Al18 | 2IndanO | Et | H | 5-1Idz | C | | 467 (M⁺+1) |
| D-a-304 | 1-a | D-a-303 | | 2IndanO | H | H | 5-1Idz | C | | 439 (M⁺+1) |
| D-a-305 | 4f-2 | Int.d-30 | Al18 | 2IndanO | Et | H | 1Me-5-Idz | C | | 481 (M⁺+1) |
| D-a-306 | 1-a | D-a-305 | | 2IndanO | H | H | 1Me-5-Idz | C | | 453 (M⁺+1) |
| D-a-307 | 4f-2 | Int.d-35 | Al18 | 2IndanO | Et | 2-Nap | H | C | | 477 (M⁺+1) |
| D-a-308 | 1-a | D-a-307 | | 2IndanO | H | 2-Nap | H | C | | 449 (M⁺+1) |
| D-a-309 | 4f-2 | Int.d-37 | Al18 | 2IndanO | Et | 1Me-5-Ind | H | C | | 480 (M⁺+1) |
| D-a-310 | 1-a | D-a-309 | | 2IndanO | H | 1Me-5-Ind | H | C | | 452 (M⁺+1) |
| D-a-311 | 4f-2 | Int.d-40 | Al18 | 2IndanO | Et | 1Et-5-Idz | H | C | | 495 (M⁺+1) |
| D-a-312 | 1-a | D-a-311 | | 2IndanO | H | 1Et-5-Idz | H | C | | 467 (M⁺+1) |
| D-a-313 | 4f-2 | Int.d-25 | Al19 | 5OMe-2-IndanO | Et | H | 5-Ind | C | | 496 (M⁺+1) |
| D-a-314 | 1-a | D-a-313 | | 5OMe-2-IndanO | H | H | 5-Ind | C | | 468 (M⁺+1) |
| D-a-315 | 4f-2 | Int.d-26 | Al19 | 5OMe-2-IndanO | Et | H | 1Me-5-Ind | C | | 510 (M⁺+1) |
| D-a-316 | 1-a | D-a-315 | | 5OMe-2-IndanO | H | H | 1Me-5-Ind | C | | 482 (M⁺+1) |
| D-a-317 | 4f-2 | Int.d-30 | Al19 | 5OMe-2-IndanO | Et | H | 1Me-5-Idz | C | | 511 (M⁺+1) |
| D-a-318 | 1-a | D-a-317 | | 5OMe-2-IndanO | H | H | 1Me-5-Idz | C | | 483 (M⁺+1) |
| D-a-319 | 4f-2 | Int.d-31 | Al19 | 5OMe-2-IndanO | Et | H | 1Et-5-Idz | C | | 525 (M⁺+1) |
| D-a-320 | 1-a | D-a-319 | | 5OMe-2-IndanO | H | H | 1Et-5-Idz | C | | 497 (M⁺+1) |
| D-a-321 | 4f-2 | Int.d-35 | Al19 | 5OMe-2-IndanO | Et | 2-Nap | H | C | | 507 (M⁺+1) |
| D-a-322 | 1-a | D-a-321 | | 5OMe-2-IndanO | H | 2-Nap | H | C | | 479 (M⁺+1) |
| D-a-323 | 4f-2 | Int.d-39 | Al19 | 5OMe-2-IndanO | Et | 1Me-5-Idz | H | C | | 511 (M⁺+1) |
| D-a-324 | 1-a | D-a-323 | | 5OMe-2-IndanO | H | 1Me-5-Idz | H | C | | 483 (M⁺+1) |
| D-a-325 | 4f-2 | Int.d-25 | Al21 | 5F-2-IndanO | Et | H | 5-Ind | C | | 484 (M⁺+1) |
| D-a-326 | 1-a | D-a-325 | | 5F-2-IndanO | H | H | 5-Ind | C | | 456 (M⁺+1) |
| D-a-327 | 4f-2 | Int.d-26 | Al21 | 5F-2-IndanO | Et | H | 1Me-5-Ind | C | | 498 (M⁺+1) |
| D-a-328 | 1-a | D-a-327 | | 5F-2-IndanO | H | H | 1Me-5-Ind | C | | 470 (M⁺+1) |
| D-a-329 | 4f-2 | Int.d-31 | Al21 | 5F-2-IndanO | Et | H | 1Et-5-Idz | C | | 513 (M⁺+1) |
| D-a-330 | 1-a | D-a-329 | | 5F-2-IndanO | H | H | 1Et-5-Idz | C | | 485 (M⁺+1) |
| D-a-331 | 4f-2 | Int.d-35 | Al21 | 5F-2-IndanO | Et | 2-Nap | H | C | | 495 (M⁺+1) |
| D-a-332 | 1-a | D-a-331 | | 5F-2-IndanO | H | 2-Nap | H | C | | 467 (M⁺+1) |

**[Table 210]**

| Table-D-a-8 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| D-a-333 | 4f-2 | Int.d-24 | Al22 | 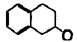 | Et | H | 2-Nap | C | | 491 (M⁺+1) |
| D-a-334 | 1-a | D-a-333 | | 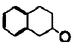 | H | H | 2-Nap | C | | 463 (M⁺+1) |
| D-a-335 | 4f-2 | Int.d-25 | Al22 | 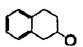 | Et | H | 5-Ind | C | | 480 (M⁺+1) |
| D-a-336 | 1-a | D-a-335 | | 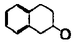 | H | H | 5-Ind | C | | 452 (M⁺+1) |
| D-a-337 | 4f-2 | Int.d-26 | Al22 | 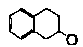 | Et | H | 1Me-5-Ind | C | | 494 (M⁺+1) |
| D-a-338 | 1-a | D-a-337 | | 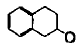 | H | H | 1Me-5-Ind | C | | 466 (M⁺+1) |
| D-a-339 | 4f-2 | Int.d-30 | Al22 | 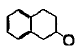 | Et | H | 1Me-5-Idz | C | | 495 (M⁺+1) |
| D-a-340 | 1-a | D-a-339 | | 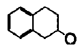 | H | H | 1Me-5-Idz | C | | 467 (M⁺+1) |
| D-a-341 | 4f-2 | Int.d-35 | Al22 | 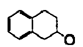 | Et | 2-Nap | H | C | | 491 (M⁺+1) |
| D-a-342 | 1-a | D-a-341 | | 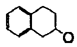 | H | 2-Nap | H | C | | 463 (M⁺+1) |
| D-a-343 | 4f-2 | Int.d-37 | Al22 | 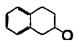 | Et | 1Me-5-Ind | H | C | | 494 (M⁺+1) |
| D-a-344 | 1-a | D-a-343 | | 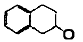 | H | 1Me-5-Ind | H | C | | 466 (M⁺+1) |
| D-a-345 | 4f-2 | Int.d-40 | Al22 | 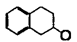 | Et | 1Et-5-Idz | H | C | | 509 (M⁺+1) |
| D-a-346 | 1-a | D-a-345 | | 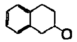 | H | 1Et-5-Idz | H | C | | 481 (M⁺+1) |
| D-a-347 | 4f-2 | Int.d-24 | Al23 | 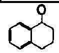 | Et | H | 2-Nap | C | | 491 (M⁺+1) |
| D-a-348 | 1-a | D-a-347 | | 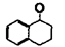 | H | H | 2-Nap | C | | 463 (M⁺+1) |
| D-a-349 | 4f-2 | Int.d-26 | Al23 | 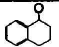 | Et | H | 1Me-5-Ind | C | | 494 (M⁺+1) |
| D-a-350 | 1-a | D-a-349 | | 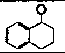 | H | H | 1Me-5-Ind | C | | 466 (M⁺+1) |
| D-a-351 | 4f-2 | Int.d-29 | Al23 | 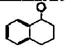 | Et | H | 5-1Idz | C | | 481 (M⁺+1) |
| D-a-352 | 1-a | D-a-351 | | 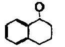 | H | H | 5-1Idz | C | | 453 (M⁺+1) |
| D-a-353 | 4f-2 | Int.d-35 | Al23 | 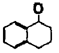 | Et | 2-Nap | H | C | | 491 (M⁺+1) |
| D-a-354 | 1-a | D-a-353 | | 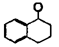 | H | 2-Nap | H | C | | 463 (M⁺+1) |
| D-a-355 | 4f-2 | Int.d-36 | Al23 | 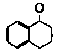 | Et | 5-Ind | H | C | | 480 (M⁺+1) |
| D-a-356 | 1-a | D-a-355 | | 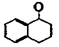 | H | 5-Ind | H | C | | 452 (M⁺+1) |

**[Table 211]**

| Table-D-a-9 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| D-a-357 | 4f-2 | Int.d-37 | Al23 | 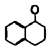 | Et | 1Me-5-Ind | H | C | | 494 (M⁺+1) |
| D-a-358 | 1-a | D-a-357 | | 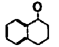 | H | 1Me-5-Ind | H | C | | 466 (M⁺+1) |
| D-a-359 | 4f-2 | Int.d-24 | Al24 | 2(2MePh)EtO | Et | H | 2-Nap | C | | 479 (M⁺+1) |
| D-a-360 | 1-a | D-a-359 | | 2(2MePh)EtO | H | H | 2-Nap | C | | 451 (M⁺+1) |
| D-a-361 | 4f-2 | Int.d-26 | Al24 | 2(2MePh)EtO | Et | H | 1Me-5-Ind | C | | 482 (M⁺+1) |
| D-a-362 | 1-a | D-a-361 | | 2(2MePh)EtO | H | H | 1Me-5-Ind | C | | 454 (M⁺+1) |
| D-a-363 | 4f-2 | Int.d-30 | Al24 | 2(2MePh)EtO | Et | H | 1Me-5-Idz | C | | 483 (M⁺+1) |
| D-a-364 | 1-a | D-a-363 | | 2(2MePh)EtO | H | H | 1Me-5-Idz | C | | 455 (M⁺+1) |
| D-a-365 | 4f-2 | Int.d-31 | Al24 | 2(2MePh)EtO | Et | H | 1Et-5-Idz | C | | 497 (M⁺+1) |
| D-a-366 | 1-a | D-a-365 | | 2(2MePh)EtO | H | H | 1Et-5-Idz | C | | 469 (M⁺+1) |
| D-a-367 | 4f-2 | Int.d-35 | Al24 | 2(2MePh)EtO | Et | 2-Nap | H | C | | 479 (M⁺+1) |
| D-a-368 | 1-a | D-a-367 | | 2(2MePh)EtO | H | 2-Nap | H | C | | 451 (M⁺+1) |
| D-a-369 | 4f-2 | Int.d-36 | Al24 | 2(2MePh)EtO | Et | 5-Ind | H | C | | 468 (M⁺+1) |
| D-a-370 | 1-a | D-a-369 | | 2(2MePh)EtO | H | 5-Ind | H | C | | 440 (M⁺+1) |
| D-a-371 | 4f-2 | Int.d-25 | Al25 | 2(3FPh)EtO | Et | H | 5-Ind | C | | 472 (M⁺+1) |
| D-a-372 | 1-a | D-a-371 | | 2(3FPh)EtO | H | H | 5-Ind | C | | 444 (M⁺+1) |
| D-a-373 | 4f-2 | Int.d-29 | Al25 | 2(3FPh)EtO | Et | H | 5-1Idz | C | | 473 (M⁺+1) |
| D-a-374 | 1-a | D-a-373 | | 2(3FPh)EtO | H | H | 5-1Idz | C | | 445 (M⁺+1) |
| D-a-375 | 4f-2 | Int.d-31 | Al25 | 2(3FPh)EtO | Et | H | 1Et-5-Idz | C | | 501 (M⁺+1) |
| D-a-376 | 1-a | D-a-375 | | 2(3FPh)EtO | H | H | 1Et-5-Idz | C | | 473 (M⁺+1) |
| D-a-377 | 4f-2 | Int.d-35 | Al25 | 2(3FPh)EtO | Et | 2-Nap | H | C | | 483 (M⁺+1) |
| D-a-378 | 1-a | D-a-377 | | 2(3FPh)EtO | H | 2-Nap | H | C | | 455 (M⁺+1) |
| D-a-379 | 4f-2 | Int.d-36 | Al25 | 2(3FPh)EtO | Et | 5-Ind | H | C | | 472 (M⁺+1) |
| D-a-380 | 1-a | D-a-379 | | 2(3FPh)EtO | H | 5-Ind | H | C | | 444 (M⁺+1) |
| D-a-381 | 4f-2 | Int.d-39 | Al25 | 2(3FPh)EtO | Et | 1Me-5-Idz | H | C | | 487 (M⁺+1) |
| D-a-382 | 1-a | D-a-381 | | 2(3FPh)EtO | H | 1Me-5-Idz | H | C | | 459 (M⁺+1) |
| D-a-383 | 4f-2 | Int.d-24 | Al26 | 2(2CIPh)EtO | Et | H | 2-Nap | C | | 500 (M⁺+1) |
| D-a-384 | 1-a | D-a-383 | | 2(2CIPh)EtO | H | H | 2-Nap | C | | 471 (M⁺+1) |
| D-a-385 | 4f-2 | Int.d-26 | Al26 | 2(2CIPh)EtO | Et | H | 1Me-5-Ind | C | | 503 (M⁺+1) |
| D-a-386 | 1-a | D-a-385 | | 2(2CIPh)EtO | H | H | 1Me-5-Ind | C | | 474 (M⁺+1) |
| D-a-387 | 4f-2 | Int.d-30 | Al26 | 2(2CIPh)EtO | Et | H | 1Me-5-Idz | C | | 504 (M⁺+1) |
| D-a-388 | 1-a | D-a-387 | | 2(2CIPh)EtO | H | H | 1Me-5-Idz | C | | 475 (M⁺+1) |
| D-a-389 | 4f-2 | Int.d-31 | Al26 | 2(2CIPh)EtO | Et | H | 1Et-5-Idz | C | | 518 (M⁺+1) |
| D-a-390 | 1-a | D-a-389 | | 2(2CIPh)EtO | H | H | 1Et-5-Idz | C | | 490 (M⁺+1) |
| D-a-391 | 4f-2 | Int.d-37 | Al26 | 2(2CIPh)EtO | Et | 1Me-5-Ind | H | C | | 503 (M⁺+1) |
| D-a-392 | 1-a | D-a-391 | | 2(2CIPh)EtO | H | 1Me-5-Ind | H | C | | 474 (M⁺+1) |
| D-a-393 | 4f-2 | Int.d-39 | Al26 | 2(2CIPh)EtO | Et | 1Me-5-Idz | H | C | | 504 (M⁺+1) |
| D-a-394 | 1-a | D-a-393 | | 2(2CIPh)EtO | H | 1Me-5-Idz | H | C | | 475 (M⁺+1) |
| D-a-395 | 4f-2 | Int.d-24 | Al28 | 2(1-Nap)EtO | Et | H | 2-Nap | C | | 515 (M⁺+1) |
| D-a-396 | 1-a | D-a-395 | | 2(1-Nap)EtO | H | H | 2-Nap | C | | 487 (M⁺+1) |
| D-a-397 | 4f-2 | Int.d-25 | Al28 | 2(1-Nap)EtO | Et | H | 5-Ind | C | | 504 (M⁺+1) |

**[Table 212]**

| Table-D-a-10 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| D-a-398 | 1-a | D-a-397 | | 2(1-Nap)EtO | H | H | 5-Ind | C | | 476 (M⁺+1) |
| D-a-399 | 4f-2 | Int.d-26 | Al28 | 2(1-Nap)EtO | Et | H | 1Me-5-Ind | C | | 518 (M⁺+1) |
| D-a-400 | 1-a | D-a-399 | | 2(1-Nap)EtO | H | H | 1Me-5-Ind | C | | 490 (M⁺+1) |
| D-a-401 | 4f-2 | Int.d-35 | Al28 | 2(1-Nap)EtO | Et | 2-Nap | H | C | | 515 (M⁺+1) |
| D-a-402 | 1-a | D-a-401 | | 2(1-Nap)EtO | H | 2-Nap | H | C | | 487 (M⁺+1) |
| D-a-403 | 4f-2 | Int.d-36 | Al28 | 2(1-Nap)EtO | Et | 5-Ind | H | C | | 504 (M⁺+1) |
| D-a-404 | 1-a | D-a-403 | | 2(1-Nap)EtO | H | 5-Ind | H | C | | 476 (M⁺+1) |
| D-a-405 | 4f-2 | Int.d-40 | Al28 | 2(1-Nap)EtO | Et | 1Et-5-Idz | H | C | | 533 (M⁺+1) |
| D-a-406 | 1-a | D-a-405 | | 2(1-Nap)EtO | H | 1Et-5-Idz | H | C | | 505 (M⁺+1) |

### Reference Example 20

### Synthesis of cyclopropyl 2-[3-(cyclopentyloxy)phenyl]acetate (Intermediate e-1) (Preparation Method 14, Step f-1)

According to the procedure described in the synthetic method of Intermediate A-5 in Reference Example 2 (Preparation Method 14, Step f-1), 3-hydroxyphenylacetic acid (5.00 g, TCI), cyclopentyl bromide (2.37 ml, TCI) and potassium carbonate (11.35 g) were reacted and treated to obtain the title compound (Intermediate e-1, 8.00 g). Mass (LCMS): 289 (M⁺+1), Retention time: 5.83 minutes (Elution condition: B). Synthesis of 2-[3-(cyclopentyloxy)phenyl]acetic acid (Intermediate e-2) (Preparation Method 1, Step a)
According to the procedure described in the synthetic method of Example Compound A-2 (Preparation Method 1, Step a) provided that the reaction was performed at room temperature for 1 hour in a mixed solvent of THF (55 ml) and methanol (55 ml), Intermediate e-1 (8.00 g) and 2 N aqueous sodium hydroxide (55.5 ml) were reacted and treated to obtain the title compound (Intermediate e-2, 6.10 g). Mass (LCMS): 219 (M-1), Retention time: 4.04 minutes (Elution condition: B).

### Synthesis of 2-[5-(cyclopentyloxy)-2-iodophenyl]acetic acid (Intermediate e-3)

According to the procedure described in a literature [Olivera et al., Journal of Organic Chemistry, p.7215, 2002], a solution of Intermediate e-2 (6.00 g) in acetic acid (120 ml) was added dropwise with a solution of iodine monochloride (6.63 g, WAKO) in acetic acid (40 ml) at room temperature over 30 minutes, and the mixture was stirred for 2 hours. The reaction mixture was added with water (80 ml), then gradually added with disodium sulfite, and after the solution changed into yellow, added with water (150 ml). The reaction mixture was extracted with ethyl acetate (250 ml × 2), and then the organic layer was washed with saturated brine, dried, and concentrated under reduced pressure to obtain the title compound (Intermediate e-3, 7.79 g). Mass (LCMS): 345 (M-1), Retention time: 4.79 minutes (Elution condition: B).

### Synthesis of 2-[5-(cyclopentyloxy)-2-iodophenyl]-N-methyloxy-N-acetamide (Intermediate e-4)

A solution of Intermediate e-3 (7.73 g) in dichloromethane (200 ml) was successively added with N,O-dimethylhydroxyamine monohydrochloride (4.35 g, WAKO), 1-hydroxy-7-azabenzotriazole (6.08 g, Watanabe Kagaku), water-soluble carbodiimide monohydrochloride (8.56 g, Watanabe Kagaku) and triethylamine (18.58 ml), and the mixture was stirred for 1 hour. The reaction mixture was added with dichloromethane (100 ml) for extraction, and the organic layer was washed with 10% citric acid, saturated aqueous sodium hydrogencarbonate and saturated brine, dried, and concentrated under reduced pressure to obtain the title compound (Intermediate e-4, 8.36 g). Mass (LCMS): 390 (M⁺+1), Retention time: 5.18 minutes (Elution condition: B).

### Synthesis of 4-cyclopentyloxy-cyclobutylbenzen-1(2H)-one (Intermediate e-5)

According to the procedure described in a literature [Aidhen et al., Tetrahedron Letters, p.5431, 1992], a solution of Intermediate e-4 (8.30 g) in THF (60 ml) was added dropwise with t-butyllithium (36.51 ml, Ald) at -78°C over 15 minutes under a nitrogen atmosphere, and the mixture was stirred for 8 hours at the same temperature. The reaction mixture was warmed to -10°C, then added with saturated aqueous ammonium chloride (150 ml), and then extracted with dichloromethane (300 ml × 2), the organic layer was dried, and the solvent was evaporated under reduced pressure. The residue was purified by column chromatography (Quad, hexane:ethyl acetate = 2:1) to obtain the title compound (Intermediate e-5, 1.00 g). Mass (LCMS): 203 (M⁺+1), Retention time: 4.70 minutes (Elution condition: B).

### Synthesis of ethyl 2-(4-cyclopentyloxy-cyclobutylbenzen-1(2H)-ylidene)acetate (Intermediate e-6) (Preparation Method 9, Step k-3)

A solution of ethyl diethylphosphonoacetate (25 ml, TCI) in DME was added with sodium hydride (0.78 g, WAKO) under ice cooling, and the mixture was stirred for 10 minutes, and then added dropwise with a solution of Intermediate e-5 (989 mg) in DME (50 ml). The reaction mixture was stirred at room temperature for 2 hours, and then added with aqueous ammonium chloride (75 ml) under ice cooling. The reaction mixture was added with water (25 ml), and extracted with ethyl acetate (150 ml × 2), the organic layer was washed with saturated brine, and dried, and the solvent was evaporated under reduced pressure. The residue was purified by column chromatography (Quad, hexane:ethyl acetate = 3:1) to obtain the title compound (Intermediate e-6, 772.1 mg). Mass (LCMS): 273 (M⁺+1), Retention time: 6.00 minutes (Elution condition: B).

### Synthesis of ethyl 2-(4-cyclopentyloxy-1,2-dihydrocyclobutylbenzen-1-yl)acetate (Intermediate e-7) (Preparation Method 9, Step j)

According to the procedure described in the synthetic method of Intermediate a-3 in Reference Example 1 (Preparation Method 9, Step j), Intermediate e-6 (387.1 mg) and 10% palladium/carbon (100 mg, Merck) were reacted and treated to obtain the title compound (Intermediate e-7, 325.3 mg). Mass (LCMS): 275 (M⁺+1), Retention time: 5.69 minutes (Elution condition: B).

### Synthesis of ethyl 2-(5-bromo-4-cyclopentyloxy-1,2-dihydrocyclobutylbenzen-1-yl)acetate (Intermediate e-8) (Preparation Method 8, Step h)

According to the procedure described in the synthetic method of Intermediate A-4 in Reference Example 1 (Preparation Method 8, Step h) provided that the reaction was performed at room temperature for 1 hour, Intermediate e-7 (220 mg) and NBS (157 mg) were reacted and treated to obtain the title compound (Intermediate e-8, 241.4 mg). Mass (LCMS): 353 (M⁺+1), Retention time: 5.15 minutes (Elution condition: B).

### Reference Example 21

### Synthesis of benzyl 2-[3-(benzyloxy)phenyl]acetate (Intermediate e-9) (Preparation Method 14, Step f-1)

According to the procedure described in the synthetic method of Intermediate A-5 in Reference Example 2 (Preparation Method 14, Step f-1), 3-hydroxyphenylacetic acid (5.04 g, TCI), benzyl bromide (3.76 ml, TCI) and potassium carbonate (12.11 g) were reacted and treated to obtain the title compound (Intermediate e-9, 8.23 g). Mass (LCMS): 333 (M⁺+1), Retention time: N.D (Elution condition: C).

### Synthesis of 2-[3-(benzyloxy)phenyl]acetic acid (Intermediate e-10) (Preparation Method 1, Step a)

According to the procedure described in the synthetic method of Example Compound A-2 (Preparation Method 1, Step a) provided that the reaction was performed at room temperature for 1 hour in a mixed solvent of THF (55 ml) and methanol (55 ml), Intermediate e-9 (8.23 g) and 2 N aqueous sodium hydroxide (62.2 ml) were reacted and treated to obtain the title compound (Intermediate e-10, 6.24 g). Mass (LCMS): 243 (M⁺+1), Retention time: N.D (Elution condition: C).

### Synthesis of 2-[5-(benzyloxy)-2-iodophenyl]acetic acid (Intermediate e-11)

According to the procedure described in the synthetic method of Intermediate e-3 in Reference Example 20, Intermediate e-10 (6.24 g) and iodine monochloride (6.93 g, WAKO) were reacted and treated to obtain the title compound (Intermediate e-11, 7.92 g). Mass (LCMS): 367 (M-1), Retention time: N.D (Elution condition: C). Synthesis of 2-[5-(benzyloxy)-2-iodophenyl]-N-methyloxy-N-acetamide (Intermediate e-12)
According to the procedure described in the synthetic method of Intermediate e-4 in Reference Example 20, Intermediate E-11 (7.90 g), N,O-dimethylhydroxylamine monohydrochloride (4.65 g, WAKO), 1-hydroxy-7-azabenzotriazole (6.22 g, Watanabe Kagaku), water-soluble carbodiimide monohydrochloride (8.76 g, Watanabe Kagaku) and triethylamine (18.97 ml) were reacted and treated to obtain the title compound (Intermediate e-12, 8.73 g). Mass (LCMS): 428 (M⁺+1), Retention time: N.D (Elution condition: C).

### Synthesis of 4-benzyloxy-cyclobutylbenzen-1(2H)-one (Intermediate e-13)

According to the procedure described in the synthetic method of Intermediate e-5 in Reference Example 20 (Preparation Method 1, Step a), Intermediate E-12 (8.70 g) and t-butyllithium (37.21 ml, Ald) were reacted and treated to obtain the title compound (Intermediate e-13, 2.03 g). Mass (LCMS): 225 (M⁺+1), Retention time: N.D (Elution condition: C).

### Synthesis of ethyl 2-(4-benzyloxy-cyclobutylbenzen-1(2H)-ylidene)acetate (Intermediate e-14) (Preparation Method 9, Step k-3)

According to the procedure described in the synthetic method of Intermediate e-6 in Reference Example 20 (Preparation Method 9, Step k-3), ethyl diethylphosphonoacetate (25 ml, TCI), sodium hydride (1.02 g, WAKO) and Intermediate E-13 (2.00 g) were reacted and treated to obtain the title compound (Intermediate e-14, 1.87 g). Mass (LCMS): 295 (M⁺+1), Retention time: N.D (Elution condition: C).

### Synthesis of ethyl 2-(1,2-dihydrocyclobutylbenzene-4-hydroxy-1-yl)acetate (Intermediate e-15) (Preparation Method 9, Step j)

According to the procedure described in the synthetic method of Intermediate A-3 in Reference Example 1 (Preparation Method 9, Step j) provided that the reaction was performed for 30 minutes, Intermediate e-14 (387.1 mg) and 10% palladium/carbon (100 mg, Merck) were reacted and treated to obtain the title compound (Intermediate e-15, 194.3 mg). Mass (LCMS): 297 (M⁺+1), Retention time: N.D (Elution condition: C).

Typical examples of the intermediates that can be obtained by reacting and treating corresponding starting compounds using any of the methods described in the present specification including the examples described above are shown in Table-Int.E-1. In the tables, used methods among the aforementioned synthetic methods are shown in the columns of "Syn" with symbols, the starting compounds are shown in the columns of "SM", and the reagents are shown in the columns of "Regent". The halide regents shown in the columns of "Reagent" with symbols "Hal (No.)" are those mentioned in Table-Hal, and the alcohol regents shown with symbols "Al (No.)" are those mentioned in Table-Al.

**[Table 213]**

| Table-IntE-1 | | | | | | | | | | |
|---|---|---|---|---|---|---|---|---|---|---|
| 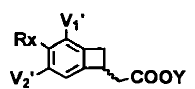 | | | | | | | | | | |

| Exp. | Syn. | SM | Reagent | Rx | Y | V1' | V2' | LCMS | | |
|---|---|---|---|---|---|---|---|---|---|---|
| | | | | | | | | method | RTime | Mass |
| Int.e-16 | 4f-1 | Int.e-15 | Hal1 | BnO | Et | H | H | C | | 297 (M⁺+1) |
| Int.e-17 | 8-h | Int.e-16 | | BnO | Et | H | Br | C | | 375 (M⁺) |
| Int.e-18 | 4f-2 | Int.e-15 | AI2 | cPenMeO | Et | H | H | C | | 289 (M⁺+1) |
| Int.e-19 | 8-h | Int.e-18 | | cPenMeO | Et | H | Br | C | | 367 (M⁺) |
| Int.e-20 | 4f-1 | Int.e-15 | Hal2 | cHexMeO | Et | H | H | C | | 303 (M⁺+1) |
| Int.e-21 | 8-h | Int.e-20 | | cHexMeO | Et | H | Br | C | | 381 (M⁺) |
| Int.e-22 | 4f-2 | Int.e-15 | Al3 | cHexO | Et | H | H | C | | 289 (M⁺+1) |
| Int.e-23 | 8-h | Int.e-22 | | cHexO | Et | H | Br | C | | 367 (M⁺) |
| Int.e-24 | 4f-1 | Int.e-15 | Hal3 | nPrO | Et | H | H | C | | 249 (M⁺+1) |
| Int.e-25 | 8-h | Int.e-24 | | nPrO | Et | H | Br | C | | 327 (M⁺) |
| Int.e-26 | 4f-1 | Int.e-15 | Hal4 | iPrO | Et | H | H | C | | 249 (M⁺+1) |
| Int.e-27 | 8-h | Int.e-26 | | iPrO | Et | H | Br | C | | 327 (M⁺) |

### Example E-a-1

### Synthesis of ethyl 2-[4-(cyclopentyloxy)-5-(naphthalen-2-yl)-1,2-dihydrocyclobutylbenzen-1-yl]acetate (Compound No. E-a-1) (Preparation Method 4, Step e-1)

According to the procedure described in the synthetic method of Example Compound A-a-1 (Preparation Method 4, Step e-1) provided that the reaction was performed for 6 hours, Intermediate e-8 (176.9 mg), 2-naphthaleneboronic acid (154.2 mg, Ald), 2 M aqueous sodium carbonate (1.2 ml) and (Ph₃P)₄Pd (184.9 mg, Nakalai Tesque) were reacted and treated to obtain the title compound (Compound No. E-a-1, 151.3 mg).

### Example E-a-2

### Synthesis of 2-[4-(cyclopentyloxy)-5-(naphthalen-2-yl)-1,2-dihydrocyclobutylbenzen-1-yl]acetic acid (Compound No. E-a-2) (Preparation Method 1, Step a)

According to the procedure described in the synthetic method of Example Compound A-a-2 (Preparation Method 1, Step a) provided that the reaction was performed for 1 hour, Example Compound E-a-1 (151.3 mg) and 2 N aqueous sodium hydroxide (0.78 ml) were reacted and treated to obtain the title compound (Compound No. E-a-2, 132.2 mg).

Typical examples of the compounds of the present invention that can be obtained by reacting and treating corresponding starting compounds using any of the methods described in the present specification including the examples described above are shown in Table-E-a-1 to Table-E-a-8, and the intermediates that can be similarly obtained are shown in Table-Int.E-2. As for the preparation of the compounds, used methods among the aforementioned synthetic methods are shown in the columns of "Syn" with symbols, the starting compounds are shown in the columns of "SM", and the reagents are shown in the columns of "Regent" in the tables. In the columns of "Reagent", the halide regents shown with symbols "Hal (No.)" are those mentioned in Table-Hal, the alcohol regents shown with symbols "Al (No.)" are those mentioned in Table-Al, the boronic acid regents shown with symbols "BRA (No.)" are those mentioned in Table-BRA, and the bromoheterocyclic ring regents shown with the symbols "Het (No.)" are those mentioned in Table-Het. The blank cells of the columns of "Syn" indicate that a reduction reaction with Pd/C was performed for the compounds.

**[Table 214]**

| Table-Int.E-2 | | | | | | | | | | |
|---|---|---|---|---|---|---|---|---|---|---|
| 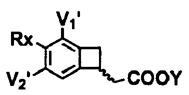 | | | | | | | | | | |

| Exp. | Syn. | SM | Reagent | Rx | Y | V1' | V2' | LCMS | | |
|---|---|---|---|---|---|---|---|---|---|---|
| | | | | | | | | method | RTime | Mass |
| Int.e-28 | | E-a-21 | | HO | Et | H | 2-Nap | C | | 333 (M⁺+1) |
| Int.e-29 | | E-a-23 | | HO | Et | H | 5-Ind | C | | 322 (M⁺+1) |
| Int.e-30 | | E-a-25 | | HO | Et | H | 1Me-5-Ind | C | | 336 (M⁺+1) |
| Int.e-31 | | E-a-27 | | HO | Et | H | 1Me-4-Ind | C | | 336 (M⁺+1) |
| Int.e-32 | | E-a-29 | | HO | Et | H | 1Me-6-Ind | C | | 336 (M⁺+1) |
| Int.e-33 | | E-a-31 | | HO | Et | H | 5-1Idz | C | | 323 (M⁺+1) |
| Int.e-34 | | E-a-33 | | HO | Et | H | 1Me-5-Idz | C | | 337 (M⁺+1) |
| Int.e-35 | | E-a-35 | | HO | Et | H | 1Et-5-Idz | C | | 351 (M⁺+1) |
| Int.e-36 | | E-a-37 | | HO | Et | H | 3-Qu | C | | 334 (M⁺+1) |
| Int.e-37 | | E-a-39 | | HO | Et | H | 6-Qu | C | | 334 (M⁺+1) |
| Int.e-38 | | E-a-41 | | HO | Et | H | 6-IQ | C | | 334 (M⁺+1) |

**[Table 215]**

| Table-E-a-1 | | | | | | | | | | |
|---|---|---|---|---|---|---|---|---|---|---|
| 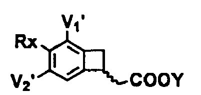 | | | | | | | | | | |

| Exp. | Syn. | SM | Reagent | Rx | Y | V1' | V2' | LCMS | | |
|---|---|---|---|---|---|---|---|---|---|---|
| | | | | | | | | method | RTime | Mass |
| E-a-1 | 4e-1 | Int.e-8 | BRA1 | cPenO | Et | H | 2-Nap | C | | 401 (M⁺+1) |
| E-a-2 | 1-a | E-a-1 | | cPenO | H | H | 2-Nap | C | | 373 (M⁺+1) |
| E-a-3 | 4e-1 | Int.e-8 | BRA2 | cPenO | Et | H | 5-Ind | C | | 390 (M⁺+1) |
| E-a-4 | 1-a | E-a-3 | | cPenO | H | H | 5-Ind | C | | 362 (M⁺+1) |
| E-a-5 | 4e-1 | Int.e-8 | BRA3 | cPenO | Et | H | 1Me-5-Ind | C | | 404 (M⁺+1) |
| E-a-6 | 1-a | E-a-5 | | cPenO | H | H | 1Me-5-Ind | C | | 376 (M⁺+1) |
| E-a-7 | 4e-1 | Int.e-8 | BRA5 | cPenO | Et | H | 1Me-4-Ind | C | | 404 (M⁺+1) |
| E-a-8 | 1-a | E-a-7 | | cPenO | H | H | 1Me-4-Ind | C | | 376 (M⁺+1) |
| E-a-9 | 4e-1 | Int.e-8 | BRA6 | cPenO | Et | H | 1Me-6-Ind | C | | 404 (M⁺+1) |
| E-a-10 | 1-a | E-a-9 | | cPenO | H | H | 1Me-6-Ind | C | | 376 (M⁺+1) |
| E-a-11 | 4e-1 | Int.e-8 | BRA8 | cPenO | Et | H | 1Me-5-Idz | C | | 405 (M⁺+1) |
| E-a-12 | 1-a | E-a-11 | | cPenO | H | H | 1Me-5-Idz | C | | 377 (M⁺+1) |
| E-a-13 | 4e-1 | Int.e-8 | BRA9 | cPenO | Et | H | 1Et-5-Idz | C | | 419 (M⁺+1) |
| E-a-14 | 1-a | E-a-13 | | cPenO | H | H | 1Et-5-Idz | C | | 391 (M⁺+1) |
| E-a-15 | 4e-1 | Int.e-8 | BRA10 | cPenO | Et | H | 2Me-5-Idz | C | | 405 (M⁺+1) |
| E-a-16 | 1-a | E-a-15 | | cPenO | H | H | 2Me-5-Idz | C | | 377 (M⁺+1) |
| E-a-17 | 4e-2 | Int.e-8 | Het2 | cPenO | Et | H | 3-Qu | C | | 402 (M⁺+1) |
| E-a-18 | 1-a | E-a-17 | | cPenO | H | H | 3-Qu | C | | 374 (M⁺+1) |
| E-a-19 | 4e-2 | Int.e-8 | Het3 | cPenO | Et | H | 6-IQ | C | | 402 (M⁺+1) |
| E-a-20 | 1-a | E-a-19 | | cPenO | H | H | 6-IQ | C | | 374 (M⁺+1) |
| E-a-21 | 4e-1 | Int.e-17 | BRA1 | BnO | Et | H | 2-Nap | C | | 423 (M⁺+1) |
| E-a-22 | 1-a | E-a-21 | | BnO | H | H | 2-Nap | C | | 395 (M⁺+1) |
| E-a-23 | 4e-1 | Int.e-17 | BRA2 | BnO | Et | H | 5-Ind | C | | 412 (M⁺+1) |
| E-a-24 | 1-a | E-a-23 | | BnO | H | H | 5-Ind | C | | 384 (M⁺+1) |
| E-a-25 | 4e-1 | Int.e-17 | BRA3 | BnO | Et | H | 1Me-5-Ind | C | | 426 (M⁺+1) |
| E-a-26 | 1-a | E-a-25 | | BnO | H | H | 1Me-5-Ind | C | | 398 (M⁺+1) |
| E-a-27 | 4e-1 | Int.e-17 | BRA5 | BnO | Et | H | 1Me-4-Ind | C | | 426 (M⁺+1) |
| E-a-28 | 1-a | E-a-27 | | BnO | H | H | 1Me-4-Ind | C | | 398 (M⁺+1) |
| E-a-29 | 4e-1 | Int.e-17 | BRA6 | BnO | Et | H | 1Me-6-Ind | C | | 426 (M⁺+1) |
| E-a-30 | 1-a | E-a-29 | | BnO | H | H | 1Me-6-Ind | C | | 398 (M⁺+1) |
| E-a-31 | 4e-1 | Int.e-17 | BRA7 | BnO | Et | H | 5-1Idz | C | | 413 (M⁺+1) |
| E-a-32 | 1-a | E-a-31 | | BnO | H | H | 5-1Idz | C | | 385 (M⁺+1) |
| E-a-33 | 4e-1 | Int.e-17 | BRA8 | BnO | Et | H | 1Me-5-Idz | C | | 427 (M⁺+1) |
| E-a-34 | 1-a | E-a-33 | | BnO | H | H | 1Me-5-Idz | C | | 399 (M⁺+1) |
| E-a-35 | 4e-1 | Int.e-17 | BRA9 | BnO | Et | H | 1Et-5-Idz | C | | 441 (M⁺+1) |
| E-a-36 | 1-a | E-a-35 | | BnO | H | H | 1Et-5-Idz | C | | 413 (M⁺+1) |
| E-a-37 | 4e-2 | Int.e-17 | Het2 | BnO | Et | H | 3-Qu | C | | 424 (M⁺+1) |
| E-a-38 | 1-a | E-a-37 | | BnO | H | H | 3-Qu | C | | 396 (M⁺+1) |
| E-a-39 | 4e-1 | Int.e-17 | BRA13 | BnO | Et | H | 6-Qu | C | | 424 (M⁺+1) |
| E-a-40 | 1-a | E-a-39 | | BnO | H | H | 6-Qu | C | | 396 (M⁺+1) |
| E-a-41 | 4e-2 | Int.e-17 | Het3 | BnO | Et | H | 6-IQ | C | | 424 (M⁺+1) |
| E-a-42 | 1-a | E-a-41 | | BnO | H | H | 6-IQ | C | | 396 (M⁺+1) |
| E-a-43 | 4e-1 | Int.e-19 | BRA1 | cPenMeO | Et | H | 2-Nap | C | | 415 (M⁺+1) |
| E-a-44 | 1-a | E-a-43 | | cPenMeO | H | H | 2-Nap | C | | 387 (M⁺+1) |

**[Table 216]**

| Table-E-a-2 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| E-a-45 | 4e-1 | Int.e-19 | BRA2 | cPenMeO | Et | H | 5-Ind | C | | 404 (M⁺+1) |
| E-a-46 | 1-a | E-a-45 | | cPenMeO | H | H | 5-Ind | C | | 376 (M⁺+1) |
| E-a-47 | 4e-1 | Int.e-19 | BRA3 | cPenMeO | Et | H | 1Me-5-Ind | C | | 418 (M⁺+1) |
| E-a-48 | 1-a | E-a-47 | | cPenMeO | H | H | 1Me-5-Ind | C | | 390 (M⁺+1) |
| E-a-49 | 4e-1 | Int.e-19 | BRA8 | cPenMeO | Et | H | 1Me-5-Idz | C | | 419 (M⁺+1) |
| E-a-50 | 1-a | E-a-49 | | cPenMeO | H | H | 1Me-5-Idz | C | | 391 (M⁺+1) |
| E-a-51 | 4e-1 | Int.e-21 | BRA3 | cHexMeO | Et | H | 1Me-5-Ind | C | | 432 (M⁺+1) |
| E-a-52 | 1-a | E-a-51 | | cHexMeO | H | H | 1Me-5-Ind | C | | 404 (M⁺+1) |
| E-a-53 | 4e-1 | Int.e-21 | BRA7 | cHexMeO | Et | H | 5-1Idz | C | | 419 (M⁺+1) |
| E-a-54 | 1-a | E-a-53 | | cHexMeO | H | H | 5-1Idz | C | | 391 (M⁺+1) |
| E-a-55 | 4e-1 | Int.e-21 | BRA9 | cHexMeO | Et | H | 1Et-5-Idz | C | | 447 (M⁺+1) |
| E-a-56 | 1-a | E-a-55 | | cHexMeO | H | H | 1Et-5-Idz | C | | 419 (M⁺+1) |
| E-a-57 | 4e-1 | Int.e-21 | BRA13 | cHexMeO | Et | H | 6-Qu | C | | 430 (M⁺+1) |
| E-a-58 | 1-a | E-a-57 | | cHexMeO | H | H | 6-Qu | C | | 402 (M⁺+1) |
| E-a-59 | 4e-2 | Int.e-21 | Het3 | cHexMeO | Et | H | 6-IQ | C | | 430 (M⁺+1) |
| E-a-60 | 1-a | E-a-59 | | cHexMeO | H | H | 6-IQ | C | | 402 (M⁺+1) |
| E-a-61 | 4e-1 | Int.e-23 | BRA1 | cHexO | Et | H | 2-Nap | C | | 415 (M⁺+1) |
| E-a-62 | 1-a | E-a-61 | | cHexO | H | H | 2-Nap | C | | 387 (M⁺+1) |
| E-a-63 | 4e-1 | Int.e-23 | BRA2 | cHexO | Et | H | 5-Ind | C | | 404 (M⁺+1) |
| E-a-64 | 1-a | E-a-63 | | cHexO | H | H | 5-Ind | C | | 376 (M⁺+1) |
| E-a-65 | 4e-1 | Int.e-23 | BRA4 | cHexO | Et | H | 1Et-5-Ind | C | | 432 (M⁺+1) |
| E-a-66 | 1-a | E-a-65 | | cHexO | H | H | 1Et-5-Ind | C | | 404 (M⁺+1) |
| E-a-67 | 4e-1 | Int.e-23 | BRA7 | cHexO | Et | H | 5-1Idz | C | | 405 (M⁺+1) |
| E-a-68 | 1-a | E-a-67 | | cHexO | H | H | 5-1Idz | C | | 377 (M⁺+1) |
| E-a-69 | 4e-1 | Int.e-25 | BRA1 | nPrO | Et | H | 2-Nap | C | | 375 (M⁺+1) |
| E-a-70 | 1-a | E-a-69 | | nPrO | H | H | 2-Nap | C | | 347 (M⁺+1) |
| E-a-71 | 4e-1 | Int.e-25 | BRA3 | nPrO | Et | H | 1Me-5-Ind | C | | 378 (M⁺+1) |
| E-a-72 | 1-a | E-a-71 | | nPrO | H | H | 1Me-5-Ind | C | | 350 (M⁺+1) |
| E-a-73 | 4e-1 | Int.e-25 | BRA4 | nPrO | Et | H | 1Et-5-Ind | C | | 392 (M⁺+1) |
| E-a-74 | 1-a | E-a-73 | | nPrO | H | H | 1Et-5-Ind | C | | 364 (M⁺+1) |
| E-a-75 | 4e-1 | Int.e-25 | BRA7 | nPrO | Et | H | 5-1Idz | C | | 365 (M⁺+1) |
| E-a-76 | 1-a | E-a-75 | | nPrO | H | H | 5-1Idz | C | | 337 (M⁺+1) |
| E-a-77 | 4e-1 | Int.e-27 | BRA1 | iPrO | Et | H | 2-Nap | C | | 375 (M⁺+1) |
| E-a-78 | 1-a | E-a-77 | | iPrO | H | H | 2-Nap | C | | 347 (M⁺+1) |
| E-a-79 | 4e-1 | Int.e-27 | BRA3 | iPrO | Et | H | 1Me-5-Ind | C | | 378 (M⁺+1) |
| E-a-80 | 1-a | E-a-79 | | iPrO | H | H | 1Me-5-Ind | C | | 350 (M⁺+1) |
| E-a-81 | 4e-1 | Int.e-27 | BRA7 | iPrO | Et | H | 5-1Idz | C | | 365 (M⁺+1) |
| E-a-82 | 1-a | E-a-81 | | iPrO | H | H | 5-1Idz | C | | 337 (M⁺+1) |
| E-a-83 | 4e-1 | Int.e-27 | BRA9 | iPrO | Et | H | 1Et-5-Idz | C | | 393 (M⁺+1) |
| E-a-84 | 1-a | E-a-83 | | iPrO | H | H | 1Et-5-Idz | C | | 365 (M⁺+1) |
| E-a-85 | 4e-1 | Int.e-27 | BRA13 | iPrO | Et | H | 6-Qu | C | | 376 (M⁺+1) |
| E-a-86 | 1-a | E-a-85 | | iPrO | H | H | 6-Qu | C | | 348 (M⁺+1) |
| E-a-87 | 4f-2 | Int.e-28 | Al4 | cHepO | Et | H | 2-Nap | C | | 429 (M⁺+1) |
| E-a-88 | 1-a | E-a-87 | | cHepO | H | H | 2-Nap | C | | 401 (M⁺+1) |
| E-a-89 | 4f-2 | Int.e-29 | Al4 | cHepO | Et | H | 5-Ind | C | | 418 (M⁺+1) |
| E-a-90 | 1-a | E-a-89 | | cHepO | H | H | 5-Ind | C | | 390 (M⁺+1) |
| E-a-91 | 4f-2 | Int.e-31 | Al4 | cHepO | Et | H | 1Me-4-Ind | C | | 432 (M⁺+1) |
| E-a-92 | 1-a | E-a-91 | | cHepO | H | H | 1Me-4-Ind | C | | 404 (M⁺+1) |

**[Table 217]**

| Table-E-a-3 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| E-a-93 | 4f-2 | Int.e-34 | Al4 | cHepO | Et | H | 1Me-5-Idz | C | | 433 (M⁺+1) |
| E-a-94 | 1-a | E-a-93 | | cHepO | H | H | 1Me-5-Idz | C | | 405 (M⁺+1) |
| E-a-95 | 4f-2 | Int.e-36 | Al4 | cHepO | Et | H | 3-Qu | C | | 430 (M⁺+1) |
| E-a-96 | 1-a | E-a-95 | | cHepO | H | H | 3-Qu | C | | 402 (M⁺+1) |
| E-a-97 | 4f-1 | Int.e-28 | Hal5 | nBuO | Et | H | 2-Nap | C | | 389 (M⁺+1) |
| E-a-98 | 1-a | E-a-97 | | nBuO | H | H | 2-Nap | C | | 361 (M⁺+1) |
| E-a-99 | 4f-1 | Int.e-30 | Hal5 | nBuO | Et | H | 1Me-5-Ind | C | | 392 (M⁺+1) |
| E-a-100 | 1-a | E-a-99 | | nBuO | H | H | 1Me-5-Ind | C | | 364 (M⁺+1) |
| E-a-101 | 4f-1 | Int.e-32 | Hal5 | nBuO | Et | H | 1Me-6-Ind | C | | 392 (M⁺+1) |
| E-a-102 | 1-a | E-a-101 | | nBuO | H | H | 1Me-6-Ind | C | | 364 (M⁺+1) |
| E-a-103 | 4f-1 | Int.e-33 | Hal5 | nBuO | Et | H | 5-1Idz | C | | 379 (M⁺+1) |
| E-a-104 | 1-a | E-a-103 | | nBuO | H | H | 5-1Idz | C | | 351 (M⁺+1) |
| E-a-105 | 4f-1 | Int.e-35 | Hal5 | nBuO | Et | H | 1Et-5-Idz | C | | 407 (M⁺+1) |
| E-a-106 | 1-a | E-a-105 | | nBuO | H | H | 1Et-5-Idz | C | | 379 (M⁺+1) |
| E-a-107 | 4f-1 | Int.e-37 | Hal5 | nBuO | Et | H | 6-Qu | C | | 390 (M⁺+1) |
| E-a-108 | 1-a | E-a-107 | | nBuO | H | H | 6-Qu | C | | 362 (M⁺+1) |
| E-a-109 | 4f-1 | Int.e-28 | Hal6 | iBuO | Et | H | 2-Nap | C | | 389 (M⁺+1) |
| E-a-110 | 1-a | E-a-109 | | iBuO | H | H | 2-Nap | C | | 361 (M⁺+1) |
| E-a-111 | 4f-1 | Int.e-29 | Hal6 | iBuO | Et | H | 5-Ind | C | | 378 (M⁺+1) |
| E-a-112 | 1-a | E-a-111 | | iBuO | H | H | 5-Ind | C | | 350 (M⁺+1) |
| E-a-113 | 4f-1 | Int.e-30 | Hal6 | iBuO | Et | H | 1Me-5-Ind | C | | 392 (M⁺+1) |
| E-a-114 | 1-a | E-a-113 | | iBuO | H | H | 1Me-5-Ind | C | | 364 (M⁺+1) |
| E-a-115 | 4f-1 | Int.e-35 | Hal6 | iBuO | Et | H | 1Et-5-Idz | C | | 407 (M⁺+1) |
| E-a-116 | 1-a | E-a-115 | | iBuO | H | H | 1Et-5-Idz | C | | 379 (M⁺+1) |
| E-a-117 | 4f-1 | Int.e-36 | Hal6 | iBuO | Et | H | 3-Qu | C | | 390 (M⁺+1) |
| E-a-118 | 1-a | E-a-117 | | iBuO | H | H | 3-Qu | C | | 362 (M⁺+1) |
| E-a-119 | 4f-1 | Int.e-29 | Hal7 | 2FBnO | Et | H | 5-Ind | C | | 430 (M⁺+1) |
| E-a-120 | 1-a | E-a-119 | | 2FBnO | H | H | 5-Ind | C | | 402 (M⁺+1) |
| E-a-121 | 4f-1 | Int.e-31 | Hal7 | 2FBnO | Et | H | 1Me-4-Ind | C | | 444 (M⁺+1) |
| E-a-122 | 1-a | E-a-121 | | 2FBnO | H | H | 1Me-4-Ind | C | | 416 (M⁺+1) |
| E-a-123 | 4f-1 | Int.e-32 | Hal7 | 2FBnO | Et | H | 1Me-6-Ind | C | | 444 (M⁺+1) |
| E-a-124 | 1-a | E-a-123 | | 2FBnO | H | H | 1Me-6-Ind | C | | 416 (M⁺+1) |
| E-a-125 | 4f-1 | Int.e-35 | Hal7 | 2FBnO | Et | H | 1Et-5-Idz | C | | 459 (M⁺+1) |
| E-a-126 | 1-a | E-a-125 | | 2FBnO | H | H | 1Et-5-Idz | C | | 431 (M⁺+1) |
| E-a-127 | 4f-1 | Int.e-38 | Hal7 | 2FBnO | Et | H | 6-IQ | C | | 442 (M⁺+1) |
| E-a-128 | 1-a | E-a-127 | | 2FBnO | H | H | 6-IQ | C | | 414 (M⁺+1) |
| E-a-129 | 4f-1 | Int.e-28 | Hal9 | 4FBnO | Et | H | 2-Nap | C | | 441 (M⁺+1) |
| E-a-130 | 1-a | E-a-129 | | 4FBnO | H | H | 2-Nap | C | | 413 (M⁺+1) |
| E-a-131 | 4f-1 | Int.e-30 | Hal9 | 4FBnO | Et | H | 1Me-5-Ind | C | | 444 (M⁺+1) |
| E-a-132 | 1-a | E-a-131 | | 4FBnO | H | H | 1Me-5-Ind | C | | 416 (M⁺+1) |
| E-a-133 | 4f-1 | Int.e-33 | Hal9 | 4FBnO | Et | H | 5-1Idz | C | | 431 (M⁺+1) |
| E-a-134 | 1-a | E-a-133 | | 4FBnO | H | H | 5-1Idz | C | | 403 (M⁺+1) |
| E-a-135 | 4f-1 | Int.e-34 | Hal9 | 4FBnO | Et | H | 1Me-5-Idz | C | | 445 (M⁺+1) |
| E-a-136 | 1-a | E-a-135 | | 4FBnO | H | H | 1Me-5-Idz | C | | 417 (M⁺+1) |
| E-a-137 | 4f-1 | Int.e-28 | I Hal11 | 3ClBnO | Et | H | 2-Nap | C | | 457 (M⁺+1) |
| E-a-138 | 1-a | E-a-137 | | 3ClBnO | H | H | 2-Nap | C | | 429 (M⁺+1) |
| E-a-139 | 4f-1 | Int.e-30 | I Hal11 | 3ClBnO | Et | H | 1Me-5-Ind | C | | 460 (M⁺+1) |
| E-a-140 | 1-a | E-a-139 | | 3ClBnO | H | H | 1Me-5-Ind | C | | 432 (M⁺+1) |

**[Table 218]**

| Table-E-a-4 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| E-a-141 | 4f-1 | Int.e-31 | Hal11 | 3ClBnO | Et | H | 1Me-4-Ind | C | | 460 (M⁺+1) |
| E-a-142 | 1-a | E-a-141 | | 3ClBnO | H | H | 1Me-4-Ind | C | | 432 (M⁺+1) |
| E-a-143 | 4f-1 | Int.e-32 | Hal11 | 3ClBnO | Et | H | 1Me-6-Ind | C | | 460 (M⁺+1) |
| E-a-144 | 1-a | E-a-143 | | 3ClBnO | H | H | 1Me-6-Ind | C | | 432 (M⁺+1) |
| E-a-145 | 4f-1 | Int.e-33 | Hal11 | 3ClBnO | Et | H | 5-1Idz | C | | 447 (M⁺+1) |
| E-a-146 | 1-a | E-a-145 | | 3ClBnO | H | H | 5-1Idz | C | | 419 (M⁺+1) |
| E-a-147 | 4f-1 | Int.e-35 | Hal11 | 3ClBnO | Et | H | 1Et-5-Idz | C | | 475 (M⁺+1) |
| E-a-148 | 1-a | E-a-147 | | 3ClBnO | H | H | 1Et-5-Idz | C | | 447 (M⁺+1) |
| E-a-149 | 4f-1 | Int.e-37 | Hal11 | 3ClBnO | Et | H | 6-Qu | C | | 458 (M⁺+1) |
| E-a-150 | 1-a | E-a-149 | | 3ClBnO | H | H | 6-Qu | C | | 430 (M⁺+1) |
| E-a-151 | 4f-1 | Int.e-29 | Hal12 | 4MeBnO | Et | H | 5-Ind | C | | 426 (M⁺+1) |
| E-a-152 | 1-a | E-a-151 | | 4MeBnO | H | H | 5-Ind | C | | 398 (M⁺+1) |
| E-a-153 | 4f-1 | Int.e-31 | Hal12 | 4MeBnO | Et | H | 1Me-4-Ind | C | | 440 (M⁺+1) |
| E-a-154 | 1-a | E-a-153 | | 4MeBnO | H | H | 1Me-4-Ind | C | | 412 (M⁺+1) |
| E-a-155 | 4f-1 | Int.e-34 | Hal12 | 4MeBnO | Et | H | 1Me-5-Idz | C | | 441 (M⁺+1) |
| E-a-156 | 1-a | E-a-155 | | 4MeBnO | H | H | 1Me-5-Idz | C | | 413 (M⁺+1) |
| E-a-157 | 4f-1 | Int.e-35 | Hal12 | 4MeBnO | Et | H | 1Et-5-Idz | C | | 455 (M⁺+1) |
| E-a-158 | 1-a | E-a-157 | | 4MeBnO | H | H | 1Et-5-Idz | C | | 427 (M⁺+1) |
| E-a-159 | 4f-1 | Int.e-38 | Hal12 | 4MeBnO | Et | H | 6-IQ | C | | 438 (M⁺+1) |
| E-a-160 | 1-a | E-a-159 | | 4MeBnO | H | H | 6-IQ | C | | 410 (M⁺+1) |
| E-a-161 | 4f-2 | Int.e-28 | Al11 | 4Me,cHexO | Et | H | 2-Nap | C | | 429 (M⁺+1) |
| E-a-162 | 1-a | E-a-161 | | 4Me,cHexO | H | H | 2-Nap | C | | 401 (M⁺+1) |
| E-a-163 | 4f-2 | Int.e-29 | Al11 | 4Me,cHexO | Et | H | 5-Ind | C | | 418 (M⁺+1) |
| E-a-164 | 1-a | E-a-163 | | 4Me,cHexO | H | H | 5-Ind | C | | 390 (M⁺+1) |
| E-a-165 | 4f-2 | Int.e-31 | Al11 | 4Me,cHexO | Et | H | 1Me-4-Ind | C | | 432 (M⁺+1) |
| E-a-166 | 1-a | E-a-165 | | 4Me,cHexO | H | H | 1Me-4-Ind | C | | 404 (M⁺+1) |
| E-a-167 | 4f-2 | Int.e-34 | Al11 | 4Me,cHexO | Et | H | 1Me-5-Idz | C | | 433 (M⁺+1) |
| E-a-168 | 1-a | E-a-167 | | 4Me,cHexO | H | H | 1Me-5-Idz | C | | 405 (M⁺+1) |
| E-a-169 | 4f-2 | Int.e-36 | Al11 | 4Me,cHexO | Et | H | 3-Qu | C | | 430 (M⁺+1) |
| E-a-170 | 1-a | E-a-169 | | 4Me,cHexO | H | H | 3-Qu | C | | 402 (M⁺+1) |
| E-a-171 | 4f-2 | Int.e-38 | Al11 | 4Me,cHexO | Et | H | 6-IQ | C | | 430 (M⁺+1) |
| E-a-172 | 1-a | E-a-171 | | 4Me,cHexO | H | H | 6-IQ | C | | 402 (M⁺+1) |
| E-a-173 | 4f-2 | Int.e-28 | Al6 | 2(4DMAPh)EtO | Et | H | 2-Nap | C | | 480 (M⁺+1) |
| E-a-174 | 1-a | E-a-173 | | 2(4DMAPh)EtO | H | H | 2-Nap | C | | 452 (M⁺+1) |
| E-a-175 | 4f-2 | Int.e-29 | Al6 | 2(4DMAPh)EtO | Et | H | 5-Ind | C | | 469 (M⁺+1) |
| E-a-176 | 1-a | E-a-175 | | 2(4DMAPh)EtO | H | H | 5-Ind | C | | 441 (M⁺+1) |
| E-a-177 | 4f-2 | Int.e-30 | Al6 | 2(4DMAPh)EtO | Et | H | 1Me-5-Ind | C | | 483 (M⁺+1) |
| E-a-178 | 1-a | E-a-177 | | 2(4DMAPh)EtO | H | H | 1Me-5-Ind | C | | 455 (M⁺+1) |
| E-a-179 | 4f-2 | Int.e-35 | Al6 | 2(4DMAPh)EtO | Et | H | 1Et-5-Idz | C | | 498 (M⁺+1) |
| E-a-180 | 1-a | E-a-179 | | 2(4DMAPh)EtO | H | H | 1Et-5-Idz | C | | 470 (M⁺+1) |
| E-a-181 | 4f-2 | Int.e-28 | Al7 | 1PhEtO | Et | H | 2-Nap | C | | 437 (M⁺+1) |
| E-a-182 | 1-a | E-a-181 | | 1PhEtO | H | H | 2-Nap | C | | 409 (M⁺+1) |
| E-a-183 | 4f-2 | Int.e-30 | Al7 | 1PhEtO | Et | H | 1Me-5-Ind | C | | 440 (M⁺+1) |
| E-a-184 | 1-a | E-a-183 | | 1PhEtO | H | H | 1Me-5-Ind | C | | 412 (M⁺+1) |
| E-a-185 | 4f-2 | Int.e-34 | Al7 | 1PhEtO | Et | H | 1Me-5-Idz | C | | 441 (M⁺+1) |
| E-a-186 | 1-a | E-a-185 | | 1PhEtO | H | H | 1Me-5-Idz | C | | 413 (M⁺+1) |
| E-a-187 | 4f-2 | Int.e-37 | Al7 | 1PhEtO | Et | H | 6-Qu | C | | 438 (M⁺+1) |
| E-a-188 | 1-a | E-a-187 | | 1PhEtO | H | H | 6-Qu | C | | 410 (M⁺+1) |

**[Table 219]**

| Table-E-a-5 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| E-a-189 | 4f-1 | Int.e-28 | Al9 | 1(2FPh)EtO | Et | H | 2-Nap | C | | 455 (m⁺+1) |
| E-a-190 | 1-a | E-a-189 | | 1(2FPh)EtO | H | H | 2-Nap | C | | 427 (M⁺+1) |
| E-a-191 | 4f-1 | Int.e-29 | Al9 | 1(2FPh)EtO | Et | H | 5-Ind | C | | 444 (M⁺+1) |
| E-a-192 | 1-a | E-a-191 | | 1(2FPh)EtO | H | H | 5-Ind | C | | 416 (M⁺+1) |
| E-a-193 | 4f-1 | Int.e-33 | Al9 | 1(2FPh)EtO | Et | H | 5-1Idz | C | | 445 (M⁺+1) |
| E-a-194 | 1-a | E-a-193 | | 1(2FPh)EtO | H | H | 5-1Idz | C | | 417 (M⁺+1) |
| E-a-195 | 4f-1 | Inte-35 | Al9 | 1(2FPh)EtO | Et | H | 1Et-5-Idz | C | | 473 (M⁺+1) |
| E-a-196 | 1-a | E-a-195 | | 1(2FPh)EtO | H | H | 1Et-5-Idz | C | | 445 (M⁺+1) |
| E-a-197 | 4f-1 | Inte-36 | Al9 | 1(2FPh)EtO | Et | H | 3-Qu | C | | 456 (m⁺+1) |
| E-a-198 | 1-a | E-a-197 | | 1(2FPh)EtO | H | H | 3-Qu | C | | 428 (M⁺+1) |
| E-a-199 | 4f-1 | Inte-28 | Al17 | 1IndanO | Et | H | 2-Nap | C | | 449 (M⁺+1) |
| E-a-200 | 1-a | E-a-199 | | 1IndanO | H | H | 2-Nap | C | | 421 (M⁺+1) |
| E-a-201 | 4f-2 | Inte-29 | Al17 | 1IndanO | Et | H | 5-Ind | C | | 438 (M⁺+1) |
| E-a-202 | 1-a | E-a-201 | | 1IndanO | H | H | 5-Ind | C | | 410 (M⁺+1) |
| E-a-203 | 4f-2 | Inte-30 | Al17 | 1IndanO | Et | H | 1Me-5-Ind | C | | 452 (M⁺+1) |
| E-a-204 | 1-a | E-a-203 | | 1Indano | H | H | 1Me-5-Ind | C | | 424 (M⁺+1) |
| E-a-205 | 4f-2 | Inte-34 | Al17 | 1IndanO | Et | H | 1Me-5-Idz | C | | 453 (M⁺+1) |
| E-a-206 | 1-a | E-a-205 | | 1IndanO | H | H | 1Me-5-Idz | C | | 425 (M⁺+1) |
| E-a-207 | 4f-2 | Int.e-35 | Al17 | 1IndanO | Et | H | 1Et-5-Idz | C | | 467 (M⁺+1) |
| E-a-208 | 1-a | E-a-207 | | 1IndanO | H | H | 1Et-5-Idz | C | | 439 (M⁺+1) |
| E-a-209 | 4f-2 | Inte-37 | Al17 | 1IndanO | Et | H | 6-Qu | C | | 450 (M⁺+1) |
| E-a-210 | 1-a | E-a-209 | | 1IndanO | H | H | 6-Qu | C | | 422 (M⁺+1) |
| E-a-211 | 4f-2 | Inte-28 | Al18 | 2IndanO | Et | H | 2-Nap | C | | 449 (M⁺+1) |
| E-a-212 | 1-a | E-a-211 | | 2IndanO | H | H | 2-Nap | C | | 421 (M⁺+1) |
| E-a-213 | 4f-2 | Int.e-30 | Al18 | 2IndanO | Et | H | 1Me-5-Ind | C | | 452 (M⁺+1) |
| E-a-214 | 1-a | E-a-213 | | 2IndanO | H | H | 1Me-5-Ind | C | | 424 (M⁺+1) |
| E-a-215 | 4f-2 | Inte-33 | Al18 | 2IndanO | Et | H | 5-1Idz | C | | 439 (M⁺+1) |
| E-a-216 | 1-a | E-a-215 | | 2IndanO | H | H | 5-1Idz | C | | 411 (M⁺+1) |
| E-a-217 | 4f-2 | Inte-34 | Al18 | 2IndanO | Et | H | 1Me-5-Idz | C | | 453 (M⁺+1) |
| E-a-218 | 1-a | E-a-217 | | 2IndanO | H | H | 1Me-5-Idz | C | | 425 (M⁺+1) |
| E-a-219 | 4f-2 | Int.e-38 | Al18 | 2IndanO | Et | H | 6-IQ | C | | 450 (M⁺+1) |
| E-a-220 | 1-a | E-a-219 | | 2IndanO | H | H | 6-IQ | C | | 422 (M⁺+1) |
| E-a-221 | 4f-2 | Int.e-29 | Al19 | 5OMe-2-IndanO | Et | H | 5-Ind | C | | 468 (M⁺+1) |
| E-a-222 | 1-a | E-a-221 | | 5OMe-2-IndanO | H | H | 5-Ind | C | | 440 (M⁺+1) |
| E-a-223 | 4f-2 | Inte-30 | Al19 | 5OMe-2-IndanO | Et | H | 1Me-5-Ind | C | | 482 (M⁺+1) |
| E-a-224 | 1-a | E-a-223 | | 5OMe-2-IndanO | H | H | 1Me-5-Ind | C | | 454 (M⁺+1) |
| E-a-225 | 4f-2 | Inte-34 | Al19 | 5OMe-2-IndanO | Et | H | 1Me-5-Idz | C | | 483 (M⁺+1) |
| E-a-226 | 1-a | E-a-225 | | 5OMe-2-IndanO | H | H | 1Me-5-Idz | C | | 455 (M⁺+1) |
| E-a-227 | 4f-2 | Int.e-35 | Al19 | 5OMe-2-IndanO | Et | H | 1Et-5-Idz | C | | 497 (M⁺+1) |
| E-a-228 | 1-a | E-a-227 | | 5OMe-2-IndanO | H | H | 1Et-5-Idz | C | | 469 (M⁺+1) |
| E-a-229 | 4f-2 | Int.e-28 | Al21 | 5F-2-IndanO | Et | H | 2-Nap | C | | 467 (M⁺+1) |
| E-a-230 | 1-a | E-a-229 | | 5F-2-IndanO | H | H | 2-Nap | C | | 439 (M⁺+1) |
| E-a-231 | 4f-2 | Int.e-30 | Al21 | 5F-2-IndanO | Et | H | 1Me-5-Ind | C | | 470 (M⁺+1) |
| E-a-232 | 1-a | E-a-231 | | 5F-2-IndanO | H | H | 1Me-5-Ind | C | | 442 (M⁺+1) |
| E-a-233 | 4f-2 | Int.e-35 | Al21 | 5F-2-IndanO | Et | H | 1Et-5-Idz | C | | 485 (M⁺+1) |
| E-a-234 | 1-a | E-a-233 | | 5F-2-IndanO | H | H | 1Et-5-Idz | C | | 457 (M⁺+1) |
| E-a-235 | 4f-2 | Int.e-28 | Al22 | 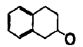 | Et | H | 2-Nap | C | | 463 (M⁺+1) |

**[Table 220]**

| Table-E-a-6 | | | | | | | | | | |
|---|---|---|---|---|---|---|---|---|---|---|
| Exp. | Syn. | SM | Reagent | Rx | Y | V1' | V2' | LCMS | | |
| | | | | | | | | method | RTime | Mass |
| E-a-236 | 1-a | E-a-235 | | 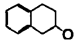 | H | H | 2-Nap | C | | 435 (M⁺+1) |
| E-a-237 | 4f-2 | Int.e-29 | Al22 | 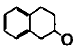 | Et | H | 5-Ind | C | | 452 (M⁺+1) |
| E-a-238 | 1-a | E-a-237 | | 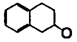 | H | H | 5-Ind | C | | 424 (M⁺+1) |
| E-a-239 | 4f-2 | Int.e-31 | Al22 | 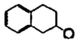 | Et | H | 1Me-4-Ind | C | | 466 (M⁺+1) |
| E-a-240 | 1-a | E-a-239 | | 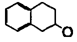 | H | H | 1Me-4-Ind | C | | 438 (M⁺+1) |
| E-a-241 | 4f-2 | Int.e-34 | Al22 | 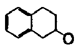 | Et | H | 1Me-5-Idz | C | | 467 (M⁺+1) |
| E-a-242 | 1-a | E-a-241 | | 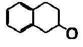 | H | H | 1 Me-5-Idz | C | | 439 (M⁺+1) |
| E-a-243 | 4f-2 | Int.e-36 | Al22 | 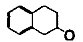 | Et | H | 3-Qu | C | | 464 (M⁺+1) |
| E-a-244 | 1-a | E-a-243 | | 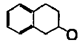 | H | H | 3-Qu | C | | 436 (M⁺+1) |
| E-a-245 | 4f-2 | Int.e-28 | Al33 | 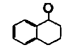 | Et | H | 2-Nap | C | | 463 (M⁺+1) |
| E-a-246 | 1-a | E-a-245 | | 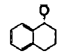 | H | H | 2-Nap | C | | 435 (M⁺+1) |
| E-a-247 | 4f-2 | Int.e-30 | Al23 | 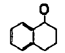 | Et | H | 1Me-5-Ind | C | | 466 (M⁺+1) |
| E-a-248 | 1-a | E-a-247 | | 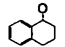 | H | H | 1Me-5-Ind | C | | 438 (M⁺+1) |
| E-a-249 | 4f-2 | Int.e-38 | Al23 | 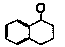 | Et | H | 6-IQ | C | | 464 (M⁺+1) |
| E-a-250 | 1-a | E-a-249 | | 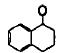 | H | H | 6-IQ | C | | 436 (M⁺+1) |
| E-a-251 | 4f-2 | Int.e-30 | Al24 | 2(2MePh)EtO | Et | H | 1Me-5-Ind | C | | 454 (M⁺+1) |
| E-a-252 | 1-a | E-a-251 | | 2(2MePh)EtO | H | H | 1Me-5-Ind | C | | 426 (M⁺+1) |
| E-a-253 | 4f-2 | Int.e-34 | Al24 | 2(2MePh)EtO | Et | H | 1Me-5-Idz | C | | 455 (M⁺+1) |
| E-a-254 | 1-a | E-a-253 | | 2(2MePh)EtO | H | H | 1Me-5-Idz | C | | 427 (M⁺+1) |
| E-a-255 | 4f-2 | Int.e-35 | Al24 | 2(2MePh)EtO | Et | H | 1Et-5-Idz | C | | 469 (M⁺+1) |
| E-a-256 | 1-a | E-a-255 | | 2(2MePh)EtO | H | H | 1Et-5-Idz | C | | 441 (M⁺+1) |
| E-a-257 | 4f-2 | Int.e-29 | Al25 | 2(3FPh)EtO | Et | H | 5-Ind | C | | 444 (M⁺+1) |
| E-a-258 | 1-a | E-a-257 | | 2(3FPh)EtO | H | H | 5-Ind | C | | 416 (M⁺+1) |
| E-a-259 | 4f-2 | Int.e-35 | Al25 | 2(3FPh)EtO | Et | H | 1 Et-5-Idz | C | | 473 (M⁺+1) |
| E-a-260 | 1-a | E-a-259 | | 2(3FPh)EtO | H | H | 1 Et-5-Idz | C | | 445 (M⁺+1) |
| E-a-261 | 4f-2 | Int.e-28 | Al26 | 2(2CIPh)EtO | Et | H | 2-Nap | C | | 472 (M⁺+1) |
| E-a-262 | 1-a | E-a-261 | | 2(2CIPh)EtO | H | H | 2-Nap | C | | 443 (M⁺+1) |
| E-a-263 | 4f-2 | Int.e-30 | Al26 | 2(2CIPh)EtO | Et | H | 1Me-5-Ind | C | | 475 (M⁺+1) |
| E-a-264 | 1-a | E-a-263 | | 2(2CIPh)EtO | H | H | 1Me-5-Ind | C | | 446 (M⁺+1) |
| E-a-265 | 4f-2 | Int.e-28 | Al28 | 2(1-Nap)EtO | Et | H | 2-Nap | C | | 487 (M⁺+1) |
| E-a-266 | 1-a | E-a-265 | | 2(1-Nap)EtO | H | H | 2-Nap | C | | 459 (M⁺+1) |
| E-a-267 | 4f-2 | Int.e-29 | Al28 | 2(1-Nap)EtO | Et | H | 5-Ind | C | | 476 (M⁺+1) |
| E-a-268 | 1-a | E-a-267 | | 2(1-Nap)EtO | H | H | 5-Ind | C | | 448 (M⁺+1) |

### [Test Examples]

### 1. Suppressing Action on PGE₂ production from IL-1β -stimulated MG-63 cells

### (1) Method for measurement

An action of suppressing PGE₂ production caused by interleukin (IL) 1β as an inflammatory stimulant was studied by the following method. Cells of MG-63, which is a human osteosarcoma cell line (purchased from Dainippon Pharmaceutical), were suspended in EMEM medium (GIBCO) containing 10% fetal bovine serum (BioFluid), and then inoculated to each well of 96-well culture plate at a density of 2 x 10⁴ cells/well and cultured overnight. The medium was changed to EMEM medium containing 0.5% fetal bovine serum, and then a test compound was added to each well. Human interleukin-1β (ENDOGEN) was further added as an inflammatory stimulant at a final concentration of 1 ng/ml. The cells were further cultured for 18 hours. Then, the culture supernatant was collected, and the PGE₂ concentration in the culture supernatant was measured by using EIA kit (CAYMAN). By using a well which was not added with the stimulant as a negative control and a well which was added only with the stimulant as a positive control, suppression ratio on PGE₂ production was calculated from the produced amount of PGE₂ in the well added with the test compound using the following equation. ${PGE}_{2} production suppression ratio = \left[1-\left(C-B\right)/\left(A-B\right)\right] \times 100$
A: PGE₂ production amount of positive control
B: PGE₂ production amount of negative control
C: PGE₂ production amount in well added with test compound
Further, cytotoxicity of the compounds was studied by using the cells after the collection of the supernatant according to the methylene blue uptake method. Specifically, the cells remained after the collection of the supernatant were fixed with glutaraldehyde and stained with a 0.05% methylene blue solution, then methylene blue taken up by the cells was extracted with 0.3 N hydrochloric acid, and absorbance of the extract was measured at 670 nm. The absorbance of the well of the aforementioned positive control was taken as 100%, and a test compound that gave absorbance in well of less than 80% was judged to be positive in cytotoxicity.

### (2) Measurement results

The test compounds (Compound Nos. A-a-1 to 294, A-b-1 to 612, A-c-1 to 181, A-d-1 to A-d-189, B-a-1 to 414, B-b-1 to 236, 247 to 291, 303 to 348, B-c-1 to 140, 151 to 194, 203 to 232, B-d-1 to 63, 69 to 85, 88 to 100, B-e-1 to 63, 69 to 85, 88 to 100, Ca-a-1 to 636, Ca-b-1 to 488, Ca-c-1 to 488, N-a-1 to 274, N-b-1 to 394, N-c-1 to 164, N-d-1 to 136, N-e-1 to 136, N-f-1 to 197, N- g-1 to 197, N-h-1 to 82, N-i-1 to 82, N-j-1 to 140, N-k-1 to 148, N-l-1 to 118, and N-m-96) suppressed the PGE₂ production caused by IL-1β by 50% or more at 1.0 µM. Moreover, all the test compounds did not exhibit cytotoxicity at that concentration.
Therefore, the novel substituted bicyclic derivatives or salts thereof according to the present invention are useful as active ingredients of medicaments for suppressing inflammatory prostaglandin production.

### 2. Suppressing action on PGD₂ and LTB₄ production from IgE-stimulated RBL-2H3 cells

### (1) Method for measurement

Suppressing action on PGD₂ and LTB₄ production caused by IgE antibody as an allergic stimulant was investigated by the following method. Cells of RBL-2H3, which is a rat mastocytoma cell line (purchased from ATCC), were suspended in DEMEM medium (GIBCO) containing 10% fetal bovine serum (BioFluid), inoculated to each well of 48-well culture plate at a density of 2 x 10⁴ cells/well and cultured overnight. Then, IgE antiserum directed to dinitrophenylated BSA (hereinafter abbreviated as "DNP-BSA") was further added to each well, and the cells were cultured for 30 minutes. Then, the medium was changed to DEMEM medium containing 0.5% fetal bovine serum, a test compound was added to each well, and DNP-BSA was further added at a final concentration of 100 ng/ml as a stimulant.
Ten minutes after the stimulant was added, the culture supernatant was collected, and the PGD₂ concentration and LTB₄ concentration in the culture supernatant were measured by using EIA kit (CAYMAN). By using a well which was not added with the stimulant as a negative control and a well which was added only with the stimulant as a positive control, suppressing ratios on mediator production were calculated from the production amounts of the mediators in the well added with the test compound using the following equation 2. ${PGD}_{2} or {LTB}_{4} production suppression ratio = \left[1-\left(C-B\right)/\left(A-B\right)\right] \times 100$
A: PGD₂ or LTB₄ production amount of positive control
B: PGD₂ or LTB₄ production amount of negative control
C: PGD₂ or LTB₄ production amount in well added with test compound
Cytotoxicity of the compounds was studied in the same manner as those described above, by using the cells after the collection of the supernatant according to the methylene blue uptake method.

### (2) Measurement results

Representative compounds of the objective Compounds (I) described in the specification suppressed the PGD₂ and LTB₄ production caused by IgE stimulation by 50% or more at 1.0 µM. Moreover, all the test compounds did not exhibit cytotoxicity at that concentration. Thus, the novel substituted bicyclic derivatives or salts thereof according to the present invention exhibit suppressing action on the allergic prostaglandin and leukotriene production, and are useful as active ingredients of medicaments for suppressing the production thereof.

### 3. Suppressing effect on mouse zymosan-stimulated footpad edema reaction

### (1) Method for measurement

A suppressing effect on footpad edema caused by zymosan as an inflammatory stimulant was studied by the following method. Groups of ICR female mice (6- to 7-week old) each consisting of eight mice were used for the test. A test compound was suspended or dissolved in purified water containing 0.5% methylcellulose and orally administered to the test animals at 0.1 to 500 mg/10 ml/kg. To the control group, purified water containing 0.5% methylcellulose was administered in a similar manner, which was not added with a test compound. One hour after the administration of the test compound, 0.02 ml of a suspension of zymosan suspended in physiological saline (Otsuka Pharmaceutical) at 1 mg/ml was subcutaneously administered to right hind leg footpad of each mouse. One and two hours after the administration of the zymosan suspension, volume of the right hind leg footpad was measured by using an apparatus for measuring a volume of mouse hind leg footpad edema (Unicom). A difference of the volume of footpad measured above and the footpad volume before the administration of the test compound measured beforehand was regarded as a volume of the edema.
For the volume of the edema at 1 hour or 2 hours after the zymosan administration, a graph was prepared by indicating time in abscissa and the edema volume in ordinate, and an edema volume AUC (area under the curve) was obtained up to 2 hours by calculation using the following equation. $Edema volume AUC \left(μ⁢1\cdot hour\right) = 1 / 2 x 1 x A + 1 x \left(A+B\right) / 2$
A: Edema volume 1 hour after zymosan administration
B: Edema volume 2 hour after zymosan administration
A suppression ratio on edema of test compound was obtained by calculation using the following equation.
$Edema suppression ratio \left(%\right) = \left[1-B/A\right] x 100$
A: Edema volume AUC of positive control
B: Edema volume AUC of test compound administered group

### (2) Measurement results

Representative compounds of the objective Compounds (I) described in the specification more effectively suppressed footpad edema caused by subcutaneous administration of zymosan compared with the positive control group by oral administration at 0.1 to 500 mg/kg.
Therefore, the novel substituted bicyclic derivatives or salts thereof according to the present invention exhibit a suppressing action on footpad edema caused by zymosan as an inflammatory stimulant, and thus they are useful as active ingredients of medicaments for prophylactic and/or therapeutic treatment of inflammatory diseases.

### 4. Suppressing effect on mouse IgE-stimulated footpad edema reaction

### (1) Method for measurement

Suppression on footpad edema caused by IgE antibody as an allergic stimulant was studied by the following method. Groups of C57BL/6 male mice (9- to 11-week old) each consisting of five mice were used for the test. Anti-DNP-BSA IgE serum was subcutaneously administered in a volume of 20 µl to right hind leg footpad of each mouse one day before the test. A test compound was suspended or dissolved in purified water containing 0.5% methylcellulose and orally administered to the test animals at 0.1 to 500 mg/10 ml/kg. To the control group, purified water containing 0.5% methylcellulose was administered in a similar manner, which was not added with any test compound. Two hours after the administration of the test compound, 0.2 ml of a solution of DNP-BSA dissolved in physiological saline (Otsuka Pharmaceutical) at 2.5 µg/ml was intravenously administered. The thickness of right hind leg footpad was measured by using a digital thickness gauge (MITSUTOYO) 10, 15, 20, and 30 minutes after the administration of DNP-BSA. A difference of the thickness of footpad measured above and the thickness before the administration of the test compound measured beforehand was regarded as a thickness of edema.
For the thickness of the edema at 10, 15, 20 and 30 minutes after the DNP-BSA administration, a graph was prepared indicating time in abscissa and the edema thickness in ordinate, and edema thickness AUC up to 2 hours was obtained by calculation according to the following equation. $Edema thickness AUC \left(mm⋅minute\right) = 1 / 2 x 10 x A + 5 x \left(A+B\right) / 2 + 5 x \left(B+C\right) / 2 + 10 x \left(C+D\right) / 2$
A: Edema thickness 10 minutes after DNP-BSA administration
B: Edema thickness 15 minutes after DNP-BSA administration
C: Edema thickness 20 minutes after DNP-BSA administration
D: Edema thickness 30 minutes after DNP-BSA administration
A suppressing ratio on edema of a test compound was obtained by calculation in accordance with the following equation.
$Edema suppression ratio \left(%\right) = \left[1-B/A\right] x 100$
A: Edema thickness AUC of positive control
B: Edema thickness AUC of test compound administered group

### (2) Measurement results

Representative compounds of the objective Compounds (I) described in the specification suppressed the footpad edema caused by IgE stimulation, i.e., footpad edema observed when DNP-BSA was administered to the mice sensitized with the anti-DNP-BSA IgE serum, compared with the positive control group by oral administration of 0.1 to 500 mg/kg.
Therefore, the novel substituted bicyclic derivatives or salts thereof according to the present invention exhibit suppressing action on footpad edema caused by IgE antibody, which is an allergic stimulant, and thus they are useful as active
ingredients of medicaments for prophylactic and/or therapeutic treatment of allergic diseases.

### 5. Suppressing effect on mouse acetic acid writhing reaction

### (1) Method for measurement

A suppressing effect on acetic acid writhing reaction, which is an acute pain model, was studied by the following method. Groups of ICR female mice (6-week old) each consisting of eight mice were used for the test. A test compound was suspended or dissolved in purified water containing 0.5% methylcellulose and orally administered to the test animals at 0.1 to 500 mg/10 ml/kg. To the control group, purified water containing 0.5% methylcellulose was administered in a similar manner, which was not added with any test compound. One hour after the administration of the test compound, 0.9% aqueous acetic acid was intraperitoneally administered to the mice in a volume of 5 ml/kg, and number of writhing reactions during 15 minutes immediately after the administration of acetic acid was counted. Suppression ratio relative to the control group was obtained by calculation according to the following equation. $Writhing suppression ratio \left(%\right) = \left[1-B/A\right] x 100$
A: Writhing number of positive control group
B: Writhing number of test compound administered group

### (2) Measurement results

The representative compounds of the objective Compounds (I) described in the specification suppressed writhing caused by administration of aqueous acetic acid compared with the positive control group at oral administration of 0.1 to 500 mg/kg.
It has been elucidated that a writhing reaction caused by intraperitoneal administration of acetic acid is caused due to production of prostaglandin [Matsumoto et al., European Journal of Pharmacology (Eur. J. Pharmacol), 1998, vol. 352, p.47; Ueno et al., Biochemical Pharmacology (Biochem. Pharmacol), 2001, vol. 15, p.157].
Therefore, the novel substituted bicyclic derivatives or salts thereof according to the present invention are useful as active ingredients of medicaments for prophylactic and/or therapeutic treatment of acute pain caused by prostaglandins.

### 6. Prophylactic and therapeutic effects for rat adjuvant arthritis

### (1) Method for measurement

A suppressing effect on footpad edema in rat adjuvant arthritis, which is a disease model of rheumatoid arthritis as being one of autoimmune diseases and also a chronic inflammatory disease, was studied by the following method. Groups of Lewis female rats (8-week old) each consisting of six mice were used for the test. The test animals were immunized by subcutaneously administering, to right hind leg footpads, 50 µl of liquid paraffin containing 10 mg/ml of M. tuberclulosis H37 RA (DIFCO) as an adjuvant. A test compound was suspended or dissolved in purified water containing 0.5% methylcellulose and orally administered to the test animals at 0.1 to 500 mg/5 ml/kg. The test compound was administered twice a day for 14 days, from the 12th day after the immunization. To the control group, purified water containing 0.5% methylcellulose was administered in a similar manner, which was not added with any test compound. Every 2 or 3 days after the administration of adjuvant, volume of left hind leg footpad, which was not administered with the adjuvant, was measured by using an apparatus for measuring a volume of edema of a rat hind leg footpad (Unicom). A suppression ratio on edema was obtained by calculation using the following equation. $Edema suppression ratio \left(%\right) = \left\{1-[\left(D-C\right)/C]/[\left(B-A\right)/A]\right\} \times 100$
A: Left hind leg footpad volume of positive control immediately before administration of adjuvant
B: Left hind leg footpad volume of positive control on each measurement day
C: Left hind leg footpad volume of test compound administered group immediately before administration of adjuvant
D: Left hind leg footpad volume of test compound administered group on each measurement day

### (2) Measurement results

The representative compounds of the objective Compound (I) described in the specification suppressed footpad edema in adjuvant arthritis compared with the positive control group.
Therefore, the novel substituted bicyclic derivatives or salts thereof according to the present invention are useful as active ingredients of medicaments for prophylactic and/or therapeutic treatment of rheumatoid arthritis and autoimmune diseases.

### 7. Effect on rat pulmonary fibrosis

### (1) Method for measurement

A suppressing effect on pulmonary fibrosing in a bleomycin-induced rat pulmonary fibrosis model, which is a pathological model of pulmonary fibrosis, was studied by the following method. Groups of BN female rats (7-week old) each consisting of seven rats were used for the test. The test animals were anesthetized with ketamine and xylazine, and the tracheae were exposed. Then, a 125 µg/0.1 ml solution of bleomycin (Nippon Kayaku) dissolved in physiological saline (Ohtsuka Pharmaceutical Factory) was injected into the tracheae by using a syringe. The negative control group was administered with 0.1 ml of saline into the tracheae.
Each test compound was suspended or dissolved in purified water containing 0.5% methylcellulose, and orally administered to the test animals at doses of 10, 30, 100 and 300 mg/5 ml/kg. The administration of the test compounds was started from the day of the bleomycin administration and performed once or twice a day for 21 days. The positive control group was administered with purified water containing 0.5% methylcellulose not added with any test compound in a similar manner. On the 21st day after the administration of bleomycin, the rats were sacrificed, and lungs were fixed with neutral buffered formalin to prepare histopathological samples. Staining of the histopathological samples was performed by the Azan method.
The histopathological samples of lungs were examined, and degree of fibrosing was represented with the following scores on the basis of formation of granulation tissues and proliferation of collagen fibers as indicators, i.e., -: no abnormality, ±: extremely mild change, +: mild change, ++: moderate change, and +++: significant change.

### (2) Measurement results

The fibrosing score of the negative control group was minus (-), and no pulmonary fibrosing was observed. The median of the fibrosing score of the positive control group was from ++ to +++, and pulmonary fibrosing was observed. The medians of the fibrosing score of the groups of rats administered with the representative compounds of the objective Compounds (I) described in the specification were from ± to +, and thus the fibrosing was milder compared with the positive control group. Therefore, the novel substituted bicyclic derivatives or salts thereof according to the present invention are useful as active ingredients of medicaments for prophylactic and/or therapeutic treatment of pulmonary fibrosis, and type 4 PLA₂ inhibitor compounds are also useful as active ingredients of medicaments for prophylactic and/or therapeutic treatment (including prevention of progression) of pulmonary fibrosis.

### Industrial Applicability

The novel substituted bicyclic derivatives or salts thereof according to the present invention have superior suppressing action on prostaglandin production and leukotriene production, and they are useful as active ingredients of medicaments for prophylactic and/or therapeutic treatment of various inflammatory diseases, autoimmune diseases, allergic diseases, pain, fibrosis and the like caused by these lipid mediators.

## Claims

1. A compound represented by the formula (I): 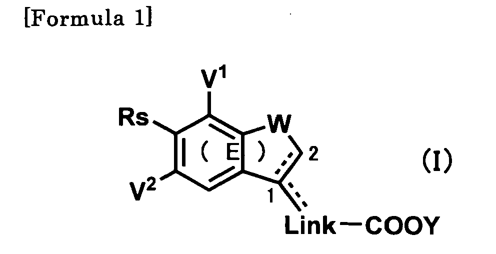 [In the formula, - - - - represents a single bond or a double bond (provided that both the bond between the ring-constituting carbon atom at the 1-position of the ring (E) and the ring-constituting carbon atom at the 2-position of the ring (E), and the bond between the ring-constituting carbon atom at the 1-position of the ring (E) and the Link moiety do not simultaneously represent a double bond, and when Link represents a single bond, the bond between the ring-constituting carbon atom at the 1-position of the ring (E) and the Link moiety represents a single bond);
Link represents a single bond, or a saturated or unsaturated straight hydrocarbon having 1 or 2 carbon atoms ;
W represents a single bond, methylene group, oxygen atom, sulfur atom, or N(Rw);
Rw represents hydrogen atom, an alkyl group having 1 to 8 carbon atoms, or - A⁶-Qp;
A⁶ represents a single bond or methylene group;
Qp represents phenyl group, and the phenyl group may be substituted with one of T¹ or two or more of the same or different T¹;
T¹ represents a linear or branched saturated alkyl group having 1 to 4 carbon atoms, hydroxyl group, fluorine atom, chlorine atom, bromine atom, trifluoromethyl group, nitro group, an alkoxyl group having 1 to 4 carbon atoms, or a mono- or dialkylamino group having 1 to 4 carbon atoms;
Rs represents -D-Rx or -N(Ry)(Rz);
D represents a single bond, oxygen atom, sulfur atom, -S(O)-, -S(O)₂-, or - C(O)-;
Rx represents a linear or branched saturated alkyl group having 3 to 8 carbon atoms, or represents Ra represented by the following formula:
R¹-A^{a}- (Ra)
Rb represented by the following formula: 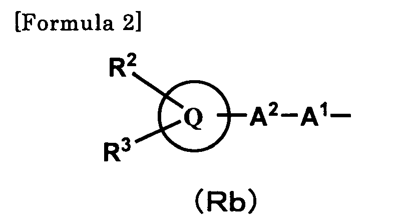 or Rc represented by the following formula: 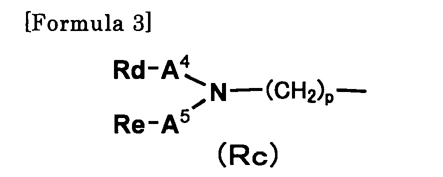
A^{a} in Ra represents a single bond, an alkylene (aa) having 1 to 3 carbon atoms, or an alkenylene (aa') having 2 or 3 carbon atoms, and the alkylene (aa) and the alkenylene (aa') may be independently substituted with a lower alkyl group having 1 to 4 carbon atoms;
R¹ represents a saturated cyclic alkyl group having 3 to 7 carbon atoms or a condensed saturated cyclic alkyl group having 6 to 8 carbon atoms, and R¹ may be substituted with one of lower alkyl group having 1 to 4 carbon atoms or two or more of the same or different lower alkyl groups having 1 to 4 carbon atoms;
Q in Rb represents a partially unsaturated or completely unsaturated monocyclic or condensed bicyclic carbon ring or a heterocyclic ring (q), and binds to A² at an arbitrary position, and the heterocyclic ring (q) contains 1 to 4 of the same or different ring-constituting heteroatoms selected from the group consisting of nitrogen atom, oxygen atom, and sulfur atom;
A¹ represents a single bond, an alkylene (a1) having 1 to 3 carbon atoms, or an alkenylene (a1') having 2 or 3 carbon atoms, and the alkylene (a1) and the alkenylene (a1') may be independently substituted with a lower alkyl group having 1 to 4 carbon atoms or phenyl group;
A² represents a single bond, oxygen atom, sulfur atom, -S(O)-, -S(O)₂-, or - N(R⁴)- (provided that when A² represents oxygen atom, sulfur atom, -S(O)-, -S(O)₂-, or -N(R⁴)-, A¹ represents ethylene or trimethylene);
R² and R³ independently represents hydrogen atom, a linear or branched saturated alkyl group having 1 to 4 carbon atoms, oxo group, thioxo group, fluorine atom, chlorine atom, bromine atom, trifluoromethyl group, -OR⁵, -N(R⁶)(R⁶'), - NHCOR⁷, -NHSO₂R⁸, or -A⁶-Qa, or they bind to each other to represent methylenedioxy group;
Qa represents a partially unsaturated or completely unsaturated monocyclic or condensed bicyclic carbon ring or heterocyclic ring (qa), binds to A⁶ at an arbitrary position on the ring, and may be substituted with one of T¹ or two or more of the same or different T¹, and the heterocyclic ring (qa) contains 1 to 4 of the same or different ring-constituting heteroatoms selected from the group consisting of nitrogen atom, oxygen atom, and sulfur atom;
R⁴ and R⁶ independently represent hydrogen atom or a lower alkyl group having 1 to 4 carbon atoms;
R⁵ and R⁷ independently represent hydrogen atom, a lower alkyl group having 1 to 4 carbon atoms, or -A⁶-Qa;
R⁸ represents a lower alkyl group having 1 to 4 carbon atoms;
R⁶ has the same meaning as that of R⁶, or binds to R⁶ to form a 3- to 6-membered ring together with the nitrogen atom to which they bind to represent a saturated nitrogen-containing cycloalkyl group, or morpholino group;
symbol p in Rc represents an integer of 2 to 4;
A⁴ represents a single bond, methylene, or ethylene;
A⁵ represents -C(O)-, -C(S)-, or -S(O)₂-;
Rd represents hydrogen atom, an alkyl group having 1 to 8 carbon atoms, or Qa;
Re represents an alkyl group having 1 to 8 carbon atoms, -A⁶-Qa, -(CH₂)ᵢR¹⁴, - OR²⁸, -SR²⁸, or -N(R²⁹)(R³⁰);
symbol i represents an integer of 1 to 3;
R¹⁴ represents hydroxyl group, an alkoxyl group having 1 to 4 carbon atoms, carboxyl group, or an N,N-dialkylcarbamoyl group having 1 to 4 carbon atoms;
R²⁸ represents an alkyl group having 1 to 8 carbon atoms or -A⁶-Qa;
R²⁹ represents an alkyl group having 1 to 8 carbon atoms, an alkoxycarbonyl group having 1 to 4 carbon atoms, or -A⁶-Qa;
R³⁰ represents hydrogen atom or a lower alkyl group having 1 to 4 carbon atoms, or binds to R²⁹ to form a 3- to 6-membered ring together with the nitrogen atom to which they bind to represent a saturated nitrogen-containing cycloalkyl group, or morpholino group;
Rz has the same meaning as that of Rx, or represents methyl group, ethyl group, or -A⁵-Re;
Ry represents hydrogen atom, an alkyl group having 1 to 8 carbon atoms, or - A⁶-Qp represents, or binds to Rz to form, together with the nitrogen atom to which they bind, a saturated or unsaturated cyclic substituent (qy) having 3 to 7 atoms, the cyclic substituent (qy) may contain one of nitrogen atom, oxygen atom, or sulfur atom besides the nitrogen atom, and the cyclic substituent (qy) may further be substituted with one of Rf or two of the same or different Rf;
Rf represents an alkyl group having 1 to 8 carbon atoms or -A⁶-Qp;
one of V¹ and V² represents Zx, and the other represents AR;
Zx represents hydrogen atom, a linear or branched saturated alkyl group having 1 to 4 carbon atoms, fluorine atom, chlorine atom, bromine atom, nitro group, OR⁹, or -N(Rn¹)(Rn²);
R⁹ represents hydrogen atom, a lower alkyl group having 1 to 4 carbon atoms, or -A⁶-Qp;
Rn¹ represents hydrogen atom or a linear or branched saturated alkyl group having 1 to 4 carbon atoms;
Rn² has the same meaning as that of Rn¹, or represents -COR²³ or -SO₂R²⁴, or binds to Rn¹ to form a 3- to 6-membered ring together with the nitrogen atom to which they bind to form a saturated nitrogen-containing cycloalkyl group, or morpholino group;
R²³ represents hydrogen atom, a lower alkyl group having 1 to 4 carbon atoms, a lower alkoxyl group having 1 to 4 carbon atoms, -O-A⁶-Qp, or -N(R²⁵)(R²⁶);
R²⁵ represents hydrogen atom or a linear or branched saturated alkyl group having 1 to 4 carbon atoms;
R²⁶ has the same meaning as that of R²⁵, or binds to R²⁵ to form a 3- to 6-membered ring together with the nitrogen atom to which they bind to form a saturated nitrogen-containing cycloalkyl group, or morpholino group;
R²⁴ represents a lower alkyl group having 1 to 4 carbon atoms, amino group, or a mono- or dialkylamino group having 1 to 4 carbon atoms;
AR represents a partially unsaturated or completely unsaturated condensed bicyclic carbon ring or heterocyclic ring (ar), and may be substituted with one of Xa or two or more of the same or different Xa;
the heterocyclic ring (ar) contains 1 to 4 of the same or different ring-constituting heteroatoms selected from the group consisting of nitrogen atom, oxygen atom, and sulfur atom;
Xa represents a linear or branched saturated alkyl group having 1 to 4 carbon atoms, a saturated cyclic alkyl group having 3 to 7 carbon atoms, oxo group, thioxo group, fluorine atom, chlorine atom, trifluoromethyl group, -(CH₂)ᵢR¹⁴, -OR¹⁰, - N(R¹¹)(R¹¹), -SO²R¹³, or -COR₂₇;
R¹⁰ represents hydrogen atom, a lower alkyl group having 1 to 4 carbon atoms or -(CH₂)ᵢR¹⁴;
R¹¹ represents hydrogen atom or a lower alkyl group having 1 to 4 carbon atoms;
R¹² represents hydrogen atom, a lower alkyl group having 1 to 4 carbon atoms, a hydroxyalkyl group having 2 to 4 carbon atoms, -COR¹⁵, or -SO₂R¹⁶, or binds to R¹¹ to form a 3- to 6-membered ring together with the nitrogen atom to which they bind to represent a saturated nitrogen-containing cycloalkyl group, or morpholino group;
R¹⁵ represents a lower alkyl group having 1 to 4 carbon atoms, a hydroxyalkyl group having 2 to 4 carbon atoms, amino group, a mono- or dialkylamino group having 1 to 4 carbon atoms, or -A⁶-Qa;
R¹³ and R¹⁶ independently represent a lower alkyl group having 1 to 4 carbon atoms, amino group, or a mono- or dialkylamino group having 1 to 4 carbon atoms;
R²⁷ represents hydrogen atom, hydroxyl group, an alkoxyl group having 1 to 4 carbon atoms, a lower alkyl group having 1 to 4 carbon atoms, amino group, or a mono- or dialkylamino group having 1 to 4 carbon atoms;
Y represents hydrogen atom, a lower alkyl group having 1 to 4 carbon atoms, -(CH₂)ₘN(R¹⁸)(R¹⁹), or -C(R²⁰)₂OC(O)A³R²¹;
symbol m represents an integer of 2 or 3;
R¹⁸ has the same meaning as that of R¹⁹, or binds to R¹⁹ to form a 3- to 6-membered ring together with the nitrogen atom to which they bind to represent a saturated nitrogen-containing cycloalkyl group, or morpholino group;
R¹⁹ represents methyl group, ethyl group, or propyl group;
R²⁰ represents hydrogen atom, methyl group, ethyl group, or propyl group;
R²¹ represents a lower alkyl group having 1 to 4 carbon atoms, a saturated cyclic alkyl group having 3 to 6 carbon atoms, or phenyl group; and
A³ represents a single bond or oxygen atom] (this compound may sometimes be hereinafter referred to simply as "Compound (I)" of the present invention"), or a salt thereof.

2. The compound or salt thereof according to claim 1, wherein V¹ is Zx, and V² is AR.

3. The compound or salt thereof according to claim 1 or 2, wherein the bond between the ring-constituting carbon atom at the 1-position of the ring (E) and the ring-constituting carbon atom at the 2-position of the ring (E) is a single bond;
the bond between the ring-constituting carbon atom at the 1-position of the ring (E) and the Link moiety is a single bond or a double bond;
when the bond between the ring-constituting carbon atom at the 1-position of the ring (E) and the Link moiety is a single bond, Link is CH₂, or when the bond between the ring-constituting carbon atom at the 1-position of the ring (E) and the Link moiety is a double bond, Link is CH; and
W is methylene group.

4. The compound or salt thereof according to claim 1 or 2, wherein the bond between the ring-constituting carbon atom at the 1-position of the ring (E) and the ring-constituting carbon atom at the 2-position of the ring (E) is a single bond;
the bond between the ring-constituting carbon atom at the 1-position of the ring (E) and the Link moiety is a single bond;
Link is CH₂; and
W is methylene group.

5. The compound or salt thereof according to any one of claims 1 to 4, wherein AR is a residue of naphthalene, benzofuran, benzo[b]thiophene, indole, benzothiazole, dihydro-3H-benzothiazole, quinoline, dihydro-1H-quinoline, benzo[d]isothiazole, 1H-indazole, benzo[c]isothiazole, 2H-indazole, imidazo[1,2-a]pyridine, 1H-pyrrolo[2,3-b]pyridine, isoquinoline, dihydro-2H-isoquinoline, cinnoline, quinazoline, quinoxaline, 1H-benzimidazole, benzoxazole, 1H-pyrrolo[3,2-b]pyridine, benzo[1,2,5]thiadiazole, 1H-benzotriazole, 1,3-dihydropyrrolo[2,3-b]pyridine, 1,3-dihydrobenzimidazole, dihydro-3H-benzoxazole, phthalazine, [1,8]naphthylidine, [1,5]naphthylidine, 1H-pyrrolo[3,2-c]pyridine, 1H-pyrrolo[2,3-c]pyridine, 1H-pyrazolo[4,3-b]pyridine, 1H-pyrazolo[4,3-c]pyridine, 1H-pyrazolo[3,4-c]pyridine, 1H-pyrazolo[3,4-b]pyridine, [1,2,4]triazolo[4,3-a]pyridine, thieno[3,2-c]pyridine, thieno[3,2-b]pyridine, 1H-thieno[3,2-c]pyrazole, benzo[d]isoxazole, benzo[c]isoxazole, indolizine, 1,3-dihydroindole, 1H-pyrazolo[3,4-d]thiazole, 2H-isoindole, [1,2,4]triazolo[1,5-a]pyrimidine, 1H-pyrazolo[3,4-b]pyrazine, 1H-imidazo[4,5-b]pyrazine, 7H-purine, or 4H-chromene (the aforementioned residue may be substituted with one of Xa or two or more of the same or different Xa).

6. The compound or salt thereof according to any one of claims 1 to 4, wherein AR is naphthalen-2-yl group, naphthalen-1-yl group, benzofuran-5-yl group, benzofuran-4-yl group, benzofuran-2-yl group, benzo[b]thiophen-5-yl group, benzo[b]thiophen-4-yl group, benzo[b]thiophen-2-yl group, indol-5-yl group, indol-4-yl group, indol-6-yl group, benzothiazol-6-yl group, benzothiazol-7-yl group, benzothiazol-5-yl group, benzothiazol-4-yl group, dihydro-3H-benzothiazol-6-yl group, dihydro-3H-benzothiazol-7-yl group, dihydro-3H-benzothiazol-5-yl group, dihydro-3H-benzothiazol-4-yl group, quinolin-6-yl group, quinolin-3-yl group, quinolin-5-yl group, quinolin-7-yl group, dihydro-1H-quinolin-6-yl group, dihydro-1H-quinolin-5-yl group, benzo[d]isothiazol-5-yl group, benzo[d]isothiazol-4-yl group, benzo[d]isothiazol-6-yl group, benzo[d]isothiazol-7-yl group, 1H-indazol-5-yl group, 1H-indazol-4-yl group, 1H-indazol-6-yl group, benzo[c]isothiazol-5-yl group, benzo[c]isothiazol-4-yl group, benzo[c]isothiazol-6-yl group, benzo[c]isothiazol-7-yl group, 2H-indazol-5-yl group, 2H-indazol-4-yl group, 2H-indazol-6-yl group, imidazo[1,2-a]pyridin-6-yl group, imidazo[1,2-a]pyridin-7-yl group, 1H-pyrrolo[2,3-b]pyridin-5-yl group, 1H-pyrrolo[2,3-b]pyridin-4-yl group, isoquinolin-6-yl group, isoquinolin-3-yl group, isoquinolin-5-yl group, isoquinolin-7-yl group, dihydro-2H-isoquinolin-6-yl group, dihydro-2H-isoquinolin-5-yl group, cinnolin-6-yl group, cinnolin-5-yl group, quinazolin-6-yl group, quinazolin-7-yl group, quinazolin-5-yl group, quinoxalin-2-yl group, quinoxalin-6-yl group, quinoxalin-5-yl group, 1H-benzimidazol-5-yl group, 1H-benzimidazol-4-yl group, benzoxazol-5-yl group, benzoxazol-6-yl group, benzoxazol-4-yl group, benzoxazol-7-yl group, 1H-pyrrolo[3,2-b]pyridin-5-yl group, 1H-pyrrolo[3,2-b]pyridin-6-yl group, benzo[1,2,5]thiadiazol-5-yl group, benzo[1,2,5]thiadiazol-4-yl group, 1H-benzotriazol-5-yl group, 1H-benzotriazol-4-yl group, 1,3-dihydropyrrolo[2,3-b]pyridin-5-yl group, 1,3-dihydropyrrolo[2,3-b]pyridin-4-yl group, 1,3-dihydrobenzimidazol-5-yl group, 1,3-dihydrobenzimidazol-4-yl group, dihydro-3H-benzoxazol-6-yl group, dihydro-3H-benzoxazol-7-yl group, dihydro-3H-benzoxazol-5-yl group, dihydro-3H-benzoxazol-4-yl group, phthalazin-6-yl group, phthalazin-5-yl group, [1,8]naphthylidin-3-yl group, [1,8]naphthylidin-4-yl group, [1,5]naphthylidin-3-yl group, [1,5]naphthylidin-4-yl group, 1H-pyrrolo[3,2-c]pyridin-6-yl group, 1H-pyrrolo[3,2-c]pyridin-4-yl group, 1H-pyrrolo[2,3-c]pyridin-5-yl group, 1H-pyrrolo[2,3-c]pyridin-4-yl group, 1H-pyrazolo[4,3-b]pyridin-5-yl group, 1H-pyrazolo[4,3-b]pyridin-6-yl group, 1H-pyrazolo[4,3-c]pyridin-6-yl group, 1H-pyrazolo[4,3-c]pyridin-4-yl group, 1H-pyrazolo[3,4-c]pyridin-5-yl group, 1H-pyrazolo[3,4-c]pyridin-4-yl group, 1H-pyrazolo[3,4-b]pyridin-5-yl group, 1H-pyrazolo[3,4-b]pyridin-4-yl group, [1,2,4]triazolo[4,3-a]pyridin-6-yl group, [1,2,4]triazolo[4,3-a]pyridin-7-yl group, thieno[3,2-c]pyridin-2-yl group, thieno[3,2-c]pyridin-3-yl group, thieno[3,2-c]pyridin-6-yl group, thieno[3,2-b]pyridin-2-yl group, thieno[3,2-b]pyridin-3-yl group, thieno[3,2-b]pyridin-5-yl group, thieno[3,2-b]pyridin-6-yl group, 1H-thieno[3,2-c]pyrazol-5-yl group, 1H-thieno[3,2-c]pyrazol-4-yl group, benzo[d]isoxazol-5-yl group, benzo[d]isoxazol-4-yl group, benzo[d]isoxazol-6-yl group, benzo[d]isoxazol-7-yl group, benzo[c]isoxazol-5-yl group, benzo[c]isoxazol-4-yl group, benzo[c]isoxazol-6-yl group, benzo[c]isoxazol-7-yl group, indolizin-7-yl group, indolizin-6-yl group, indolizin-8-yl group, 1,3-dihydroindol-5-yl group, 1,3-dihydroindol-4-yl group, 1,3-dihydroindol-6-yl group, 1H-pyrazolo[3,4-d]thiazol-5-yl group, 2H-isoindol-5-yl group, 2H-isoindol-4-yl group, [1,2,4]triazolo[1,5-a]pyrimidin-6-yl group, 1H-pyrazolo[3,4-b]pyrazin-5-yl group, 1H-imidazo[4,5-b]pyrazin-5-yl group, 7H-purin-2-yl group, 4H-chromen-6-yl group, or 4H-chromen-5-yl group (the aforementioned groups may be substituted with one of Xa or two or more of the same or different Xa).

7. The compound or salt thereof according to any one of claims 1 to 4, wherein AR is naphthalen-2-yl group, 6-hydroxynaphthalen-2-yl group, 6-methoxynaphthalen-2-yl group, 6-(2-hydroxyethyloxy)naphthalen-2-yl group, 6-aminonaphthalen-2-yl group, 6-(N-methylamino)naphthalen-2-yl group, 6-(N,N-dimethylamino)naphthalen-2-yl group, 6-(2-hydroxyethylamino)naphthalen-2-yl group, benzo[b]furan-5-yl group, 2-methylbenzo[b]furan-5-yl group, 3-methylbenzo[b]furan-5-yl group, 2,3-dimethylbenzo[b]furan-5-yl group, benzo[b]thiophen-5-yl group, 2-methylbenzo[b]thiophen-5-yl group, 3-methylbenzo[b]thiophen-5-yl group, 2,3-dimethylbenzo[b]thiophen-5-yl group, 1H-indol-5-yl group, 2-methyl-1H-indol-5-yl group, 3-methyl-1H-indol-5-yl group, 2,3-dimethyl-1H-indol-5-yl group, 1-methyl-1H-indol-5-yl group, 1,2-dimethyl-1H-indol-5-yl group, 1,3-dimethyl-1H-indol-5-yl group, 1,2,3-trimethyl-1H-indol-5-yl group, 1-ethyl-1H-indol-5-yl group, 1-ethyl-2-methyl-1H-indol-5-yl group, 1-ethyl-3-methyl-1H-indol-5-yl group, 1-ethyl-2,3-dimethyl-1H-indol-5-yl group, 1-propyl-1H-indol-5-yl group, 2-methyl-1-propyl-1H-indol-5-yl group, 3-methyl-1-propyl-1H-indol-5-yl group, 2,3-dimethyl-1-propyl-1H-indol-5-yl group, 1-(2-hydroxyethyl)-1H-indol-5-yl group, 1-(2-hydroxyethyl)-2-methyl-1H-indol-5-yl group, 1-(2-hydroxyethyl)-3-methyl-1H-indol-5-yl group, 2,3-dimethyl-1-(2-hydroxyethyl)-1H-indol-5-yl group, benzothiazol-6-yl group, 2-methylbenzothiazol-6-yl group, 2-methoxybenzothiazol-6-yl group, 2-aminobenzothiazol-6-yl group, 2-oxo-2,3-dihydrobenzothiazol-6-yl group, 2-oxo-3-methyl-2,3-dihydrobenzothiazol-6-yl group, 2-thioxo-2,3-dihydrobenzothiazol-6-yl group, 2-thioxo-3-methyl-2,3-dihydrobenzothiazol-6-yl group, quinolin-3-yl group, quinolin-6-yl group, 2-oxo-1,2-dihydroquinolin-6-yl group, benzo[d]isothiazol-5-yl group, 1H-indazol-5-yl group, 1-methyl-1H-indazol-5-yl group, 1-ethyl-1H-indazol-5-yl group, 1-propyl-1H-indazol-5-yl group, 1-(2-hydroxyethyl)-1H-indazol-5-yl group, 3-hydroxy-1H-indazol-5-yl group, 3-hydroxy-1-methyl-1H-indazol-5-yl group, 1-ethyl-3-hydroxy-1H-indazol-5-yl group, imidazo[1,2-a]pyridin-6-yl group, 1H-pyrrolo[2,3-b]pyridin-5-yl group, 1-methyl-1H-pyrrolo[2,3-b]pyridin-5-yl group, 1-ethyl-1H-pyrrolo[2,3-b]pyridin-5-yl group, 1-propyl-1H-pyrrolo[2,3-b]pyridin-5-yl group, 1-(2-hydroxyethyl)-1H-pyrrolo[2,3-b]pyridin-5-yl group, isoquinolin-6-yl group, 1-oxo-1,2-dihydroisoquinolin-6-yl group, cinnolin-6-yl group, or benzoxazol-5-yl group.

8. The compound or salt thereof according to any one of claims 1 to 4, wherein AR is naphthalen-2-yl group, 6-hydroxynaphthalen-2-yl group, 6-methoxynaphthalen-2-yl group, 6-aminonaphthalen-2-yl group, 6-(N,N-dimethylamino)naphthalen-2-yl group, benzo[b]furan-5-yl group, benzo[b]thiophen-5-yl group, 1H-indol-5-yl group, 1-methyl-1H-indol-5-yl group, 1-ethyl-1H-indol-5-yl group, benzothiazol-6-yl group, 2-aminobenzothiazol-6-yl group, 2-oxo-2,3-dihydrobenzothiazol-6-yl group, quinolin-3-yl group, quinolin-6-yl group, 2-oxo-1,2-dihydroquinolin-6-yl group, 1H-indazol-5-yl group, 1-methyl-1H-indazol-5-yl group, 1-ethyl-1H-indazol-5-yl group, isoquinolin-6-yl group, 1-oxo-1,2-dihydroisoquinolin-6-yl group, cinnolin-6-yl group, or benzoxazol-5-yl group.

9. The compound or salt thereof according to any one of claims 1 to 8, wherein Q in Rb is phenyl group, thienyl group, furyl group, pyrrolyl group, pyridyl group, oxazolyl group, isoxazolyl group, thiazolyl group, isothiazolyl group, imidazolyl group, pyrazolyl group, oxadiazolyl group, thiadiazolyl group, triazolyl group, tetrazolyl group, naphthyl group, tetrahydronaphthyl group, indanyl group, indenyl group, quinolyl group, isoquinolyl group, indolyl group, benzofuryl group, benzothienyl group, benzimidazolyl group, benzoxazolyl group, benzothiazolyl group, indazolyl group, 4H-chromenyl group, dihydrobenzodioxyl group, benzisoxazolyl group, pyrrolopyridinyl group, pyrazolopyridinyl group, triazolopyridinyl group, thienopyridinyl group, thienopyrazolyl group, 1,3-dihydrobenzimidazole group, dihydro-3H-benzoxazole group, or dihydro-3H-benzothiazole group (the aforementioned groups bind to A² at an arbitrary position on the ring); and
Qa is phenyl group, pyridyl group, oxazolyl group, isoxazolyl group, thiazolyl group, isothiazolyl group, imidazolyl group, pyrazolyl group, oxadiazolyl group, thiadiazolyl group, triazolyl group, tetrazolyl group, naphthyl group, indanyl group, indenyl group, quinolyl group, isoquinolyl group, indolyl group, benzofuryl group, benzothienyl group, benzimidazolyl group, benzoxazolyl group, benzothiazolyl group, or indazolyl group (the aforementioned groups may be substituted with one of T¹ or two or more of the same or different T¹, and bind to A⁶ at an arbitrary position on the ring).

10. The compound or salt thereof according to any one of claims 1 to 9, wherein D is a single bond, oxygen atom, or sulfur atom.

11. The compound or salt thereof according to any one of claims 1 to 9, wherein D is a single bond, oxygen atom, or sulfur atom;
Rx is propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb;
Q is phenyl group, thienyl group, furyl group, pyridyl group, oxazolyl group, naphthyl group, tetrahydronaphthyl group, indanyl group, indolyl group, or dihydrobenzodioxyl group;
A² is a single bond, oxygen atom, sulfur atom, -N(methyl)-, or -N(ethyl)-;
A¹ is a single bond, methylene group, methylmethylene group, ethylmethylene group, phenylmethylene group, ethylene group, methylethylene group, dimethylethylene group, ethylethylene group, phenylethylene group, ethenylene group, trimethylene group, or methyltrimethylene group (provided that when A² is oxygen atom, sulfur atom, -N(methyl)-, or -N(ethyl)-, A¹ is ethylene group, methylethylene group, dimethylethylene group, ethylethylene group, phenylethylene group, ethenylene group, trimethylene group, or methyltrimethylene group);
R² and R³ independently represent hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group;
when Rs is -N(Ry)(Rz), Rz has the same meaning as that of Rx, or is methyl group, or ethyl group; and
Ry is hydrogen atom, methyl group, ethyl group, propyl group, isopropyl group, butyl group, isobutyl group, pentyl group, or isopentyl group (provided that when Rz is methyl group, or ethyl group, Ry is a substituent other than hydrogen atom), or may bind to Rz to form, together with the nitrogen atom, a cyclic substituent (qy) selected from pyrrolidino group, piperidino group, homopiperidino group, morpholino group, piperazino group, homopiperazino group, pyrrol-1-yl group, imidazol-1-yl group, pyrazol-1-yl group, and thiomorpholino group, and the cyclic substituent (qy) may be substituted with one of alkyl group having 1 to 8 carbon atoms or two of the same or different alkyl groups having 1 to 8 carbon atoms, phenyl group, or phenylmethyl group (provided that the benzene ring of the phenyl group, or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group).

12. The compound or salt thereof according to any one of claims 1 to 9, wherein D is a single bond, or oxygen atom;
Rx is propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, or cycloheptylmethyl group, or Rb,
Q is phenyl group, 2-thienyl group, 3-thienyl group, 2-furyl group, 3-furyl group, or indan-2-yl group;
A² is a single bond, oxygen atom, sulfur atom, -N(methyl)-, or -N(ethyl)-;
A¹ is a single bond, or methylene group, methylmethylene group, or ethylene group when A² is a single bond, or ethylene group when A² is oxygen atom, sulfur atom, -N(methyl)-, or -N(ethyl)-;
R² and R³ independently represent hydrogen atom, methyl group, fluorine atom, chlorine atom, trifluoromethyl group, hydroxyl group, methoxy group, or dimethylamino group;
when Rs is -N(Ry)(Rz), Rz has the same meaning as that of Rx, or is methyl group, or ethyl group; and
Ry is hydrogen atom, methyl group, ethyl group, propyl group, isopropyl group, butyl group, isobutyl group, pentyl group, or isopentyl group (provided that when Rz represents methyl group, or ethyl group, Ry is a substituent other than hydrogen atom), or may bind to Rz to form, together with the nitrogen atom, a cyclic substituent (qy) selected from pyrrolidino group, piperidino group, morpholino group, and piperazino group, and the cyclic substituent (qy) may be substituted with one of substituent or two of the same or different substituents selected from methyl group, ethyl group, propyl group, isopropyl group, butyl group, isobutyl group, cyclopentyl group, cyclohexyl group, cyclopentylmethyl group, cyclohexylmethyl group, phenyl group, and phenylmethyl group (provided that the benzene ring of the phenyl group, or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group).

13. The compound or salt thereof according to any one of claims 1 to 9, wherein D is a single bond, or oxygen atom;
Rx is propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group; and
-N(Ry)(Rz) is N,N-dimethylamino group, N-ethyl-N-methylamino group, N,N-diethylamino group, N-methyl-N-propylamino group, N-ethyl-N-propylamino group, N-isopropyl-N-methylamino group, N-ethyl-N-isopropylamino group, N-butylamino group, N-butyl-N-methylamino group, N-butyl-N-ethylamino group, N-isobutylamino group, N-isobutyl-N-methylamino group, N-ethyl-N-isobutylamino group, N-isopentylamino group, N-isopentyl-N-methylamino group, N-ethyl-N-isopentylamino group, N-(2-ethylbutyl)amino group, N-(2-ethylbutyl)-N-methylamino group, N-cyclopentylamino group, N-cyclopentyl-N-methylamino group, N-cyclohexylamino group, N-cyclohexyl-N-methylamino group, N-cycloheptylamino group, N-(cyclopentylmethyl)amino group, N-(cyclopentylmethyl)-N-methylamino group, N-(cyclohexylmethyl)amino group, N-(cyclohexylmethyl)-N-methylamino group, N-phenylamino group, N-(2-methylphenyl)amino group, N-(4-methylphenyl)amino group, N-(2-fluorophenyl)amino group, N-(3-fluorophenyl)amino group, N-(4-fluorophenyl)amino group, N-(2-chlorophenyl)amino group, N-(3-chlorophenyl)amino group, N-(4-chlorophenyl)amino group, N-(indan-2-yl)amino group, N-(1-phenylethyl)amino group, N-[1-(2-fluorophenyl)ethyl]amino group, N-[1-(3-fluorophenyl)ethyl]amino group, N-[1-(4-fluorophenyl)ethyl]amino group, N-[1-(2-chlorophenyl)ethyl]amino group, N-[1-(3-chlorophenyl)ethyl]amino group, N-[1-(4-chlorophenyl)ethyl]amino group, N-benzylamino group, N-benzyl-N-methylamino group, N-benzyl-N-ethylamino group, N-(2-methylphenylmethyl)amino group, N-methyl-N-(2-methylphenylmethyl)amino group, N-(3-methylphenylmethyl)amino group, N-methyl-N-(3-methylphenylmethyl)amino group, N-(4-methylphenylmethyl)amino group, N-methyl-N-(4-methylphenylmethyl)amino group, N-(2-fluorophenylmethyl)amino group, N-(2-fluorophenylmethyl)-N-methylamino group, N-(3-fluorophenylmethyl)amino group, N-(3-fluorophenylmethyl)-N-methylamino group, N-(4-fluorophenylmethyl)amino group, N-(4-fluorophenylmethyl)-N-methylamino group, N-(2-chlorophenylmethyl)amino group, N-(2-chlorophenylmethyl)-N-methylamino group, N-(3-chlorophenylmethyl)amino group, N-(3-chlorophenylmethyl)-N-methylamino group, N-(4-chlorophenylmethyl)amino group, N-(4-chlorophenylmethyl)-N-methylamino group, N-(2,3-difluorophenylmethyl)amino group, N-(2,3-difluorophenylmethyl)-N-methylamino group, N-(2,4-difluorophenylmethyl)amino group, N-(2,4-difluorophenylmethyl)-N-methylamino group, N-(2,5-difluorophenylmethyl)amino group, N-(2,5-difluorophenylmethyl)-N-methylamino group, N-(3,4-difluorophenylmethyl)amino group, N-(3,4-difluorophenylmethyl)-N-methylamino group, N-(3,5-difluorophenylmethyl)amino group, N-(3,5-difluorophenylmethyl)-N-methylamino group, N-(2,3-dichlorophenylmethyl)amino group, N-(2,3-dichlorophenylmethyl)-N-methylamino group, N-(2,4-dichlorophenylmethyl)amino group, N-(2,4-dichlorophenylmethyl)-N-methylamino group, N-(2,5-dichlorophenylmethyl)amino group, N-(2,5-dichlorophenylmethyl)-N-methylamino group, N-(2,6-dichlorophenylmethyl)amino group, N-(2,6-dichlorophenylmethyl)-N-methylamino group, N-(3,4-dichlorophenylmethyl)amino group, N-(3,4-dichlorophenylmethyl)-N-methylamino group, N-(3,5-dichlorophenylmethyl)amino group, N-(3,5-dichlorophenylmethyl)-N-methylamino group, N-[2-(trifluoromethyl)phenylmethyl]amino group, N-methyl-N-[2-(trifluoromethyl)phenylmethyl]amino group, N-[3-(trifluoromethyl)phenylmethyl]amino group, N-methyl-N-[3-(trifluoromethyl)phenylmethyl]amino group, N-[4-(trifluoromethyl)phenylmethyl]amino group, N-methyl-N-[4-(trifluoromethyl)phenylmethyl]amino group, pyrrolidino group, 2-methylpyrrolidino group, 3-methylpyrrolidino group, 2,5-dimethylpyrrolidino group, 3,4-dimethylpyrrolidino group, piperidino group, 2-methylpiperidino group, 3-methylpiperidino group, 4-methylpiperidino group, 4-ethylpiperidino group, 4-propylpiperidino group, 4-isopropylpiperidino group, 4-butylpiperidino group, 4-isobutylpiperidino group, 4-cyclopentylpiperidino group, 4-cyclohexylpiperidino group, 4-(cyclopentylmethyl)piperidino group, 4-(cyclohexylmethyl)piperidino group, 4-phenylpiperidino group, 4-(2-methylphenyl)piperidino group, 4-(4-methylphenyl)piperidino group, 4-(2-fluorophenyl)piperidino group, 4-(3-fluorophenyl)piperidino group, 4-(4-fluorophenyl)piperidino group, 4-(2-chlorophenyl)piperidino group, 4-(3-chlorophenyl)piperidino group, 4-(4-chlorophenyl)piperidino group, 4-benzylpiperidino group, 4-(2-methylphenylmethyl)piperidino group, 4-(3-methylphenylmethyl)piperidino group, 4-methylphenylmethyl)piperidino group, 4-(2,3-dimethylphenylmethyl)piperidino group, 4-(3,5-dimethylphenylmethyl)piperidino group, 4-(2-fluorophenylmethyl)piperidino group, 4-(3-fluorophenylmethyl)piperidino group, 4-(4-fluorophenylmethyl)piperidino group, 4-(2-chlorophenylmethyl)piperidino group, 4-(3-chlorophenylmethyl)piperidino group, 4-(4-chlorophenylmethyl)piperidino group, 4-(2,3-difluorophenylmethyl)piperidino group, 4-(2,4-difluorophenylmethyl)piperidino group, 4-(2,5-difluorophenylmethyl)piperidino group, 4-(3,4-difluorophenylmethyl)piperidino group, 4-(2,3-dichlorophenylmethyl)piperidino group, 4-(2,4-dichlorophenylmethyl)piperidino group, 4-(2,5-dichlorophenylmethyl)piperidino group, 4-(2,6-dichlorophenylmethyl)piperidino group, 4-(3,4-dichlorophenylmethyl)piperidino group, 4-(3,5-dichlorophenylmethyl)piperidino group, 4-(3,6-dichlorophenylmethyl)piperidino group, 4-(2-methoxyphenylmethyl)piperidino group, 4-(3-methoxyphenylmethyl)piperidino group, 4-(4-methoxyphenylmethyl)piperidino group, 2,6-dimethylpiperidino group, 3,5-dimethylpiperidino group, 4,4-dimethylpiperidino group, homopiperidino group, morpholino group, 3,5-dimethylmorpholino group, 4-phenylpiperazino group, 4-(2-methylphenyl)piperazino group, 4-(4-methylphenyl)piperazino group, 4-(2-fluorophenyl)piperazino group, 4-(3-fluorophenyl)piperazino group, 4-(4-fluorophenyl)piperazino group, 4-(2-chlorophenyl)piperazino group, 4-(3-chlorophenyl)piperazino group, 4-(4-chlorophenyl)piperazino group, 4-benzylpiperazino group, 4-(2-methylphenylmethyl)piperazino group, 4-(3-methylphenylmethyl)piperazino group, 4-methylphenylmethyl)piperazino group, 4-(2,3-dimethylphenylmethyl)piperazino group, 4-(3,5-dimethylphenylmethyl)piperazino group, 4-(2-fluorophenylmethyl)piperazino group, 4-(3-fluorophenylmethyl)piperazino group, 4-(4-fluorophenylmethyl)piperazino group, 4-(2-chlorophenylmethyl)piperazino group, 4-(3-chlorophenylmethyl)piperazino group, 4-(4-chlorophenylmethyl)piperazino group, 4-(2,3-difluorophenylmethyl)piperazino group, 4-(2,4-difluorophenylmethyl)piperazino group, 4-(2,5-difluorophenylmethyl)piperazino group, 4-(3,4-difluorophenylmethyl)piperazino group, 4-(2,3-dichlorophenylmethyl)piperazino group, 4-(2,4-dichlorophenylmethyl)piperazino group, 4-(2,5-dichlorophenylmethyl)piperazino group, 4-(2,6-dichlorophenylmethyl)piperazino group, 4-(3,4-dichlorophenylmethyl)piperazino group, 4-(3,5-dichlorophenylmethyl)piperazino group, 4-(3,6-dichlorophenylmethyl)piperazino group, 4-(2-methoxyphenylmethyl)piperazino group, 4-(3-methoxyphenylmethyl)piperazino group, or 4-(4-methoxyphenylmethyl)piperazino group.

14. The compound or salt thereof according to any one of claims 1 to 13, wherein Zx is hydrogen atom, fluorine atom, chlorine atom, bromine atom, methyl group, hydroxyl group, amino group, or N-methylamino group.

15. The compound or salt thereof according to any one of claims 1 to 14, wherein Y is hydrogen atom, or a lower alkyl group having 1 to 4 carbon atoms.

16. The compound or salt thereof according to claim 1, wherein the bond between the ring-constituting carbon atom at the 1-position of the ring (E) and the ring-constituting carbon atom at the 2-position of the ring (E) is a single bond;
the bond between the ring-constituting carbon atom at the 1-position of the ring (E) and the Link moiety is a single bond or a double bond;
when the bond between the ring-constituting carbon atom at the 1-position of the ring (E) and the Link moiety is a single bond, Link is CH₂ or CH₂CH₂, or when the bond between the ring-constituting carbon atom at the 1-position of the ring (E) and the Link moiety is a double bond, Link is CH;
W is methylene group;
Rs is -D-Rx or -N(Ry)(Rz);
D is a single bond, oxygen atom, or sulfur atom;
Rx is propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, cycloheptylmethyl group, 2-cyclopentylethyl group, 2-cyclohexylethyl group, or 2-cycloheptylethyl group, or Rb;
Q is phenyl group, thienyl group, furyl group, pyridyl group, oxazolyl group, naphthyl group, tetrahydronaphthyl group, indanyl group, indolyl group, or dihydrobenzodioxyl group;
A² is a single bond, oxygen atom, sulfur atom, -N(methyl)-, or -N(ethyl)-;
A¹ is a single bond, methylene group, methylmethylene group, ethylmethylene group, phenylmethylene group, ethylene group, methylethylene group, dimethylethylene group, ethylethylene group, phenylethylene group, trimethylene group, or methyltrimethylene group (provided that when A² is oxygen atom, sulfur atom, -N(methyl)-, or -N(ethyl)-, A¹ is ethylene group, methylethylene group, dimethylethylene group, ethylethylene group, phenylethylene group, trimethylene group, or methyltrimethylene group);
R² and R³ independently represent hydrogen atom, methyl group, fluorine atom, chlorine atom, trifluoromethyl group, hydroxyl group, methoxy group, N,N-dimethylamino group, piperidino group, morpholino group, acetylamino group, or methylsulfonylamino group;
Rz has the same meaning as that of Rx, or is methyl group, or ethyl group;
Ry is hydrogen atom, methyl group, ethyl group, propyl group, isopropyl group, butyl group, isobutyl group, pentyl group, or isopentyl group (provided that when Rz is methyl group, or ethyl group, Ry is a substituent other than hydrogen atom), or may bind to Rz to form, together with the nitrogen atom, a cyclic substituent (qy) selected from pyrrolidino group, piperidino group, homopiperidino group, morpholino group, piperazino group, homopiperazino group, pyrrol-1-yl group, imidazol-1-yl group, pyrazol-1-yl group, and thiomorpholino group, and the cyclic substituent (qy) may be substituted with one of alkyl group having 1 to 8 carbon atoms or two of the same or different alkyl groups having 1 to 8 carbon atoms, phenyl group, or phenylmethyl group (provided that the benzene ring of the phenyl group, or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group);
V¹ is Zx, and V² is AR;
Zx is hydrogen atom, fluorine atom, chlorine atom, bromine atom, nitro group, methyl group, hydroxyl group, methoxy group, amino group, N-methylamino group, N-ethylamino group, N-propylamino group, N-isopropylamino group, or N,N-dimethylamino group;
AR is a residue of naphthalene, benzofuran, benzo[b]thiophene, indole, benzothiazole, dihydro-3H-benzothiazole, quinoline, dihydro-1H-quinoline, benzo[d]isothiazole, 1H-indazole, benzo[c]isothiazole, 2H-indazole, imidazo[1,2-a]pyridine, 1H-pyrrolo[2,3-b]pyridine, isoquinoline, dihydro-2H-isoquinoline, cinnoline, quinazoline, quinoxaline, 1H-benzimidazole, benzoxazole, 1H-pyrrolo[3,2-b]pyridine, benzo[1,2,5]thiadiazole, 1H-benzotriazole, 1,3-dihydropyrrolo[2,3-b]pyridine, 1,3-dihydrobenzimidazole, dihydro-3H-benzoxazole, phthalazine, [1,8]naphthylidine, [1,5]naphthylidine, 1H-pyrrolo[3,2-c]pyridine, 1H-pyrrolo[2,3-c]pyridine, 1H-pyrazolo[4,3-b]pyridine, 1H-pyrazolo[4,3-c]pyridine, 1H-pyrazolo[3,4-c]pyridine, 1H-pyrazolo[3,4-b]pyridine, [1,2,4]triazolo[4,3-a]pyridine, thieno[3,2-c]pyridine, thieno[3,2-b]pyridine, 1H-thieno[3,2-c]pyrazole, benzo[d]isoxazole, benzo[c]isoxazole, indolizine, 1,3-dihydroindole, 1H-pyrazolo[3,4-d]thiazole, 2H-isoindole, [1,2,4]triazolo[1,5-a]pyrimidine, 1H-pyrazolo[3,4-b]pyrazine, 1H-imidazo[4,5-b]pyrazine, 7H-purine, or 4H-chromene (the aforementioned residue may be substituted with one of Xa or two or more of the same or different Xa);
Xa is oxo group, thioxo group, fluorine atom, chlorine atom, trifluoromethyl group, methyl group, ethyl group, propyl group, 2-hydroxyethyl group, carboxymethyl group, 2-carboxyethyl group, N,N-dimethylcarbamoylmethyl group, hydroxyl group, methoxy group, 2-hydroxyethyloxy group, carboxymethyloxy group, 2-carboxyethyloxy group, N,N-dimethylcarbamoylmethyloxy group, amino group, methylamino group, dimethylamino group, 2-hydroxyethylamino group, carbamoylamino group, acetylamino group, furan-2-carboxyamino group, 2-hydroxyacetylamino group, 2-aminoacetylamino group, methylsulfonylamino group, (N,N-dimethylsulfamoyl)amino group, methanesulfonyl group, sulfamoyl group, N-methylsulfamoyl group, N,N-dimethylsulfamoyl group, carboxyl group, acetyl group, carbamoyl group, or N,N-dimethylcarbamoyl group; and
Y is hydrogen atom, or a lower alkyl group having 1 to 4 carbon atoms.

17. The compound or salt thereof according to claim 1, wherein the bond between the ring-constituting carbon atom at the 1-position of the ring (E) and the ring-constituting carbon atom at the 2-position of the ring (E) is a single bond;
the bond between the ring-constituting carbon atom at the 1-position of the ring (E) and the Link moiety is a single bond, or a double bond;
when the bond between the ring-constituting carbon atom at the 1-position of the ring (E) and the Link moiety is a single bond, Link is CH₂, or when the bond between the ring-constituting carbon atom at the 1-position of the ring (E) and the Link moiety is a double bond, Link is CH;
W is methylene group;
Rs is -D-Rx or -N(Ry)(Rz);
D represents a single bond, or oxygen atom;
Rx is a substituent which is propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, or cycloheptylmethyl group, or Rb mentioned above;
Q is phenyl group, 2-thienyl group, 3-thienyl group, 2-furyl group, 3-furyl group, or indan-2-yl group;
A² is a single bond, oxygen atom, sulfur atom, -N(methyl)-, or -N(ethyl)-;
A¹ is a single bond, or methylene group, methylmethylene group, or ethylene group when A² is a single bond, or ethylene group when A² is oxygen atom, sulfur atom, -N(methyl)-, or -N(ethyl)-;
R² and R³ independently represent hydrogen atom, methyl group, fluorine atom, chlorine atom, trifluoromethyl group, hydroxyl group, methoxy group, or dimethylamino group;
Rz has the same meaning as that of Rx, or is methyl group, or ethyl group;
Ry is hydrogen atom, methyl group, ethyl group, propyl group, isopropyl group, butyl group, isobutyl group, pentyl group, or isopentyl group (provided that when Rz is methyl group, or ethyl group, Ry is a substituent other than hydrogen atom), or may bind to Rz to form, together with the nitrogen atom, a cyclic substituent (qy) selected from pyrrolidino group, piperidino group, morpholino group, and piperazino group, and the cyclic substituent (qy) may be substituted with one of substituent or two of the same or different substituents selected from methyl group, ethyl group, propyl group, isopropyl group, butyl group, isobutyl group, cyclopentyl group, cyclohexyl group, cyclopentylmethyl group, cyclohexylmethyl group, phenyl group, and phenylmethyl group (provided that the benzene ring of the phenyl group, or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group);
V¹ is Zx, V² is AR;
Zx is hydrogen atom, fluorine atom, chlorine atom, bromine atom, methyl group, hydroxyl group, amino group, or N-methylamino group;
AR is a substituent which is naphthalen-2-yl group, naphthalen-1-yl group, benzofuran-5-yl group, benzofuran-4-yl group, benzo[b]thiophen-5-yl group, benzo[b]thiophen-4-yl group, indol-5-yl group, indol-4-yl group, benzothiazol-6-yl group, benzothiazol-7-yl group, quinolin-6-yl group, quinolin-3-yl group, dihydro-1H-quinolin-6-yl group, benzo[d]isothiazol-5-yl group, 1H-indazol-5-yl group, 1H-indazol-4-yl group, imidazo[1,2-a]pyridin-6-yl group, 1H-pyrrolo[2,3-b]pyridin-5-yl group, isoquinolin-6-yl group, dihydro-2H-isoquinolin-6-yl group, cinnolin-6-yl group, or benzoxazol-5-yl group (the aforementioned groups may be substituted with one of Xa or two or more of the same or different Xa);
Xa is oxo group, methyl group, ethyl group, propyl group, 2-hydroxyethyl group, hydroxyl group, methoxy group, 2-hydroxyethyloxy group, amino group, N-methylamino group, N,N-dimethylamino group, 2-hydroxyethylamino group, or carboxyl group; and
Y is hydrogen atom, methyl group, or ethyl group.

18. The compound or salt thereof according to claim 1, wherein the bond between the ring-constituting carbon atom at the 1-position of the ring (E) and the ring-constituting carbon atom at the 2-position of the ring (E) is a single bond;
the bond between the ring-constituting carbon atom at the 1-position of the ring (E) and the Link moiety is a single bond, or a double bond;
when the bond between the ring-constituting carbon atom at the 1-position of the ring (E) and the Link moiety is a single bond, Link is CH₂, or when the bond between the ring-constituting carbon atom at the 1-position of the ring (E) and the Link moiety is a double bond, Link is CH;
W is methylene group;
Rs is -D-Rx or -N(Ry)(Rz);
D is a single bond, or oxygen atom;
Rx is propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group;
-N(Ry)(Rz) is N,N-dimethylamino group, N-ethyl-N-methylamino group, N,N-diethylamino group, N-methyl-N-propylamino group, N-ethyl-N-propylamino group, N-isopropyl-N-methylamino group, N-ethyl-N-isopropylamino group, N-butylamino group, N-butyl-N-methylamino group, N-butyl-N-ethylamino group, N-isobutylamino group, N-isobutyl-N-methylamino group, N-ethyl-N-isobutylamino group, N-isopentylamino group, N-isopentyl-N-methylamino group, N-ethyl-N-isopentylamino group, N-(2-ethylbutyl)amino group, N-(2-ethylbutyl)-N-methylamino group, N-cyclopentylamino group, N-cyclopentyl-N-methylamino group, N-cyclohexylamino group, N-cyclohexyl-N-methylamino group, N-cycloheptylamino group, N-(cyclopentylmethyl)amino group, N-(cyclopentylmethyl)-N-methylamino group, N-(cyclohexylmethyl)amino group, N-(cyclohexylmethyl)-N-methylamino group, N-phenylamino group, N-(2-methylphenyl)amino group, N-(4-methylphenyl)amino group, N-(2-fluorophenyl)amino group, N-(3-fluorophenyl)amino group, N-(4-fluorophenyl)amino group, N-(2-chlorophenyl)amino group, N-(3-chlorophenyl)amino group, N-(4-chlorophenyl)amino group, N-(indan-2-yl)amino group, N-(1-phenylethyl)amino group, N-[1-(2-fluorophenyl)ethyl]amino group, N-[1-(3-fluorophenyl)ethyl]amino group, N-[1-(4-fluorophenyl)ethyl]amino group, N-[1-(2-chlorophenyl)ethyl]amino group, N-[1-(3-chlorophenyl)ethyl]amino group, N-[1-(4-chlorophenyl)ethyl]amino group, N-benzylamino group, N-benzyl-N-methylamino group, N-benzyl-N-ethylamino group, N-(2-methylphenylmethyl)amino group, N-methyl-N-(2-methylphenylmethyl)amino group, N-(3-methylphenylmethyl)amino group, N-methyl-N-(3-methylphenylmethyl)amino group, N-(4-methylphenylmethyl)amino group, N-methyl-N-(4-methylphenylmethyl)amino group, N-(2-fluorophenylmethyl)amino group, N-(2-fluorophenylmethyl)-N-methylamino group, N-(3-fluorophenylmethyl)amino group, N-(3-fluorophenylmethyl)-N-methylamino group, N-(4-fluorophenylmethyl)amino group, N-(4-fluorophenylmethyl)-N-methylamino group, N-(2-chlorophenylmethyl)amino group, N-(2-chlorophenylmethyl)-N-methylamino group, N-(3-chlorophenylmethyl)amino group, N-(3-chlorophenylmethyl)-N-methylamino group, N-(4-chlorophenylmethyl)amino group, N-(4-chlorophenylmethyl)-N-methylamino group, N-(2,3-difluorophenylmethyl)amino group, N-(2,3-difluorophenylmethyl)-N-methylamino group, N-(2,4-difluorophenylmethyl)amino group, N-(2,4-difluorophenylmethyl)-N-methylamino group, N-(2,5-difluorophenylmethyl)amino group, N-(2,5-difluorophenylmethyl)-N-methylamino group, N-(3,4-difluorophenylmethyl)amino group, N-(3,4-difluorophenylmethyl)-N-methylamino group, N-(3,5-difluorophenylmethyl)amino group, N-(3,5-difluorophenylmethyl)-N-methylamino group, N-(2,3-dichlorophenylmethyl)amino group, N-(2,3-dichlorophenylmethyl)-N-methylamino group, N-(2,4-dichlorophenylmethyl)amino group, N-(2,4-dichlorophenylmethyl)-N-methylamino group, N-(2,5-dichlorophenylmethyl)amino group, N-(2,5-dichlorophenylmethyl)-N-methylamino group, N-(2,6-dichlorophenylmethyl)amino group, N-(2,6-dichlorophenylmethyl)-N-methylamino group, N-(3,4-dichlorophenylmethyl)amino group, N-(3,4-dichlorophenylmethyl)-N-methylamino group, N-(3,5-dichlorophenylmethyl)amino group, N-(3,5-dichlorophenylmethyl)-N-methylamino group, N-[2-(trifluoromethyl)phenylmethyl]amino group, N-methyl-N-[2-(trifluoromethyl)phenylmethyl]amino group, N-[3-(trifluoromethyl)phenylmethyl]amino group, N-methyl-N-[3-(trifluoromethyl)phenylmethyl]amino group, N-[4-(trifluoromethyl)phenylmethyl]amino group, N-methyl-N-[4-(trifluoromethyl)phenylmethyl]amino group, pyrrolidino group, 2-methylpyrrolidino group, 3-methylpyrrolidino group, 2,5-dimethylpyrrolidino group, 3,4-dimethylpyrrolidino group, piperidino group, 2-methylpiperidino group, 3-methylpiperidino group, 4-methylpiperidino group, 4-ethylpiperidino group, 4-propylpiperidino group, 4-isopropylpiperidino group, 4-butylpiperidino group, 4-isobutylpiperidino group, 4-cyclopentylpiperidino group, 4-cyclohexylpiperidino group, 4-(cyclopentylmethyl)piperidino group, 4-(cyclohexylmethyl)piperidino group, 4-phenylpiperidino group, 4-(2-methylphenyl)piperidino group, 4-(4-methylphenyl)piperidino group, 4-(2-fluorophenyl)piperidino group, 4-(3-fluorophenyl)piperidino group, 4-(4-fluorophenyl)piperidino group, 4-(2-chlorophenyl)piperidino group, 4-(3-chlorophenyl)piperidino group, 4-(4-chlorophenyl)piperidino group, 4-benzylpiperidino group, 4-(2-methylphenylmethyl)piperidino group, 4-(3-methylphenylmethyl)piperidino group, 4-methylphenylmethyl)piperidino group, 4-(2,3-dimethylphenylmethyl)piperidino group, 4-(3,5-dimethylphenylmethyl)piperidino group, 4-(2-fluorophenylmethyl)piperidino group, 4-(3-fluorophenylmethyl)piperidino group, 4-(4-fluorophenylmethyl)piperidino group, 4-(2-chlorophenylmethyl)piperidino group, 4-(3-chlorophenylmethyl)piperidino group, 4-(4-chlorophenylmethyl)piperidino group, 4-(2,3-difluorophenylmethyl)piperidino group, 4-(2,4-difluorophenylmethyl)piperidino group, 4-(2,5-difluorophenylmethyl)piperidino group, 4-(3,4-difluorophenylmethyl)piperidino group, 4-(2,3-dichlorophenylmethyl)piperidino group, 4-(2,4-dichlorophenylmethyl)piperidino group, 4-(2,5-dichlorophenylmethyl)piperidino group, 4-(2,6-dichlorophenylmethyl)piperidino group, 4-(3,4-dichlorophenylmethyl)piperidino group, 4-(3,5-dichlorophenylmethyl)piperidino group, 4-(3,6-dichlorophenylmethyl)piperidino group, 4-(2-methoxyphenylmethyl)piperidino group, 4-(3-methoxyphenylmethyl)piperidino group, 4-(4-methoxyphenylmethyl)piperidino group, 2,6-dimethylpiperidino group, 3,5-dimethylpiperidino group, 4,4-dimethylpiperidino group, homopiperidino group, morpholino group, 3,5-dimethylmorpholino group, 4-phenylpiperazino group, 4-(2-methylphenyl)piperazino group, 4-(4-methylphenyl)piperazino group, 4-(2-fluorophenyl)piperazino group, 4-(3-fluorophenyl)piperazino group, 4-(4-fluorophenyl)piperazino group, 4-(2-chlorophenyl)piperazino group, 4-(3-chlorophenyl)piperazino group, 4-(4-chlorophenyl)piperazino group, 4-benzylpiperazino group, 4-(2-methylphenylmethyl)piperazino group, 4-(3-methylphenylmethyl)piperazino group, 4-methylphenylmethyl)piperazino group, 4-(2,3-dimethylphenylmethyl)piperazino group, 4-(3,5-dimethylphenylmethyl)piperazino group, 4-(2-fluorophenylmethyl)piperazino group, 4-(3-fluorophenylmethyl)piperazino group, 4-(4-fluorophenylmethyl)piperazino group, 4-(2-chlorophenylmethyl)piperazino group, 4-(3-chlorophenylmethyl)piperazino group, 4-(4-chlorophenylmethyl)piperazino group, 4-(2,3-difluorophenylmethyl)piperazino group, 4-(2,4-difluorophenylmethyl)piperazino group, 4-(2,5-difluorophenylmethyl)piperazino group, 4-(3,4-difluorophenylmethyl)piperazino group, 4-(2,3-dichlorophenylmethyl)piperazino group, 4-(2,4-dichlorophenylmethyl)piperazino group, 4-(2,5-dichlorophenylmethyl)piperazino group, 4-(2,6-dichlorophenylmethyl)piperazino group, 4-(3,4-dichlorophenylmethyl)piperazino group, 4-(3,5-dichlorophenylmethyl)piperazino group, 4-(3,6-dichlorophenylmethyl)piperazino group, 4-(2-methoxyphenylmethyl)piperazino group, 4-(3-methoxyphenylmethyl)piperazino group, or 4-(4-methoxyphenylmethyl)piperazino group;
V¹ is Zx, and V² is AR;
Zx is hydrogen atom, fluorine atom, chlorine atom, bromine atom, methyl group, hydroxyl group, amino group, or N-methylamino group;
AR is naphthalen-2-yl group, 6-hydroxynaphthalen-2-yl group, 6-methoxynaphthalen-2-yl group, 6-(2-hydroxyethyloxy)naphthalen-2-yl group, 6-aminonaphthalen-2-yl group, 6-(N-methylamino)naphthalen-2-yl group, 6-(N,N-dimethylamino)naphthalen-2-yl group, 6-(2-hydroxyethylamino)naphthalen-2-yl group, benzo[b]furan-5-yl group, 2-methylbenzo[b]furan-5-yl group, 3-methylbenzo[b]furan-5-yl group, 2,3-dimethylbenzo[b]furan-5-yl group, benzo[b]thiophen-5-yl group, 2-methylbenzo[b]thiophen-5-yl group, 3-methylbenzo[b]thiophen-5-yl group, 2,3-dimethylbenzo[b]thiophen-5-yl group, 1H-indol-5-yl group, 2-methyl-1H-indol-5-yl group, 3-methyl-1H-indol-5-yl group, 2,3-dimethyl-1H-indol-5-yl group, 1-methyl-1H-indol-5-yl group, 1,2-dimethyl-1H-indol-5-yl group, 1,3-dimethyl-1H-indol-5-yl group, 1,2,3-trimethyl-1H-indol-5-yl group, 1-ethyl-1H-indol-5-yl group, 1-ethyl-2-methyl-1H-indol-5-yl group, 1-ethyl-3-methyl-1H-indol-5-yl group, 1-ethyl-2,3-dimethyl-1H-indol-5-yl group, 1-propyl-1H-indol-5-yl group, 2-methyl-1-propyl-1H-indol-5-yl group, 3-methyl-1-propyl-1H-indol-5-yl group, 2,3-dimethyl-1-propyl-1H-indol-5-yl group, 1-(2-hydroxyethyl)-1H-indol-5-yl group, 1-(2-hydroxyethyl)-2-methyl-1H-indol-5-yl group, 1-(2-hydroxyethyl)-3-methyl-1H-indol-5-yl group, 2,3-dimethyl-1-(2-hydroxyethyl)-1H-indol-5-yl group, benzothiazol-6-yl group, 2-methylbenzothiazol-6-yl group, 2-methoxybenzothiazol-6-yl group, 2-aminobenzothiazol-6-yl group, 2-oxo-2,3-dihydrobenzothiazol-6-yl group, 2-oxo-3-methyl-2,3-dihydrobenzothiazol-6-yl group, 2-thioxo-2,3-dihydrobenzothiazol-6-yl group, 2-thioxo-3-methyl-2,3-dihydrobenzothiazol-6-yl group, quinolin-3-yl group, quinolin-6-yl group, 2-oxo-1,2-dihydroquinolin-6-yl group, benzo[d]isothiazol-5-yl group, 1H-indazol-5-yl group, 1-methyl-1H-indazol-5-yl group, 1-ethyl-1H-indazol-5-yl group, 1-propyl-1H-indazol-5-yl group, 1-(2-hydroxyethyl)-1H-indazol-5-yl group, 3-hydroxy-1H-indazol-5-yl group, 3-hydroxy-1-methyl-1H-indazol-5-yl group, 1-ethyl-3-hydroxy-1H-indazol-5-yl group, imidazo[1,2-a]pyridin-6-yl group, 1H-pyrrolo[2,3-b]pyridin-5-yl group, 1-methyl-1H-pyrrolo[2,3-b]pyridin-5-yl group, 1-ethyl-1H-pyrrolo[2,3-b]pyridin-5-yl group, 1-propyl-1H-pyrrolo[2,3-b]pyridin-5-yl group, 1-(2-hydroxyethyl)-1H-pyrrolo[2,3-b]pyridin-5-yl group, isoquinolin-6-yl group, 1-oxo-1,2-dihydroisoquinolin-6-yl group, cinnolin-6-yl group, or benzoxazol-5-yl group; and
Y is hydrogen atom, methyl group, or ethyl group.

19. The compound or salt thereof according to claim 1, wherein the bond between the ring-constituting carbon atom at the 1-position of the ring (E) and the ring-constituting carbon atom at the 2-position of the ring (E) is a single bond;
the bond between the ring-constituting carbon atom at the 1-position of the ring (E) and the Link moiety is a single bond;
Link is CH₂;
W is methylene group;
Rs is -D-Rx or -N(Ry)(Rz);
D is a single bond, or oxygen atom;
Rx is a substituent which is propyl group, isopropyl group, butyl group, isobutyl group, pentyl group, isopentyl group, 2-ethylbutyl group, cyclopentyl group, cyclohexyl group, cycloheptyl group, cyclopentylmethyl group, cyclohexylmethyl group, or cycloheptylmethyl group, or Rb;
Q is phenyl group, 2-thienyl group, 3-thienyl group, 2-furyl group, 3-furyl group, or indan-2-yl group;
A² is a single bond, oxygen atom, sulfur atom, -N(methyl)-, or -N(ethyl)-;
A¹ is a single bond, or methylene group, methylmethylene group, or ethylene group when A² is a single bond, or ethylene group when A² is oxygen atom, sulfur atom, -N(methyl)-, or -N(ethyl)-;
R² and R³ independently represent hydrogen atom, methyl group, fluorine atom, chlorine atom, trifluoromethyl group, hydroxyl group, methoxy group, or dimethylamino group;
Rz has the same meaning as that of Rx, or is methyl group, or ethyl group;
Ry is hydrogen atom, methyl group, ethyl group, propyl group, isopropyl group, butyl group, isobutyl group, pentyl group, or isopentyl group (provided that when Rz is methyl group, or ethyl group, Ry is a substituent other than hydrogen atom), or may bind to Rz to form, together with the nitrogen atom, a cyclic substituent (qy) selected from pyrrolidino group, piperidino group, morpholino group, and piperazino group, and the cyclic substituent (qy) may be substituted with one of substituent or two of the same or different substituents selected from methyl group, ethyl group, propyl group, isopropyl group, butyl group, isobutyl group, cyclopentyl group, cyclohexyl group, cyclopentylmethyl group, cyclohexylmethyl group, phenyl group, and phenylmethyl group (provided that the benzene ring of the phenyl group, or the phenylmethyl group may be substituted with one of substituent or two of the same or different substituents selected from the group consisting of methyl group, fluorine atom, chlorine atom, trifluoromethyl group, hydroxyl group, methoxy group, and dimethylamino group);
V¹ is Zx, and V² is AR;
Zx is hydrogen atom, fluorine atom, chlorine atom, bromine atom, methyl group, hydroxyl group, amino group, or N-methylamino group;
AR is a substituent which is naphthalen-2-yl group, benzofuran-5-yl group, benzo[b]thiophen-5-yl group, indol-5-yl group, benzothiazol-6-yl group, quinolin-6-yl group, quinolin-3-yl group, 1H-indazol-5-yl group, isoquinolin-6-yl group, cinnolin-6-yl group, or benzoxazol-5-yl group (the aforementioned groups may be substituted with one of Xa or two or more of the same or different Xa);
Xa is oxo group, methyl group, ethyl group, propyl group, 2-hydroxyethyl group, hydroxyl group, methoxy group, 2-hydroxyethyloxy group, amino group, N-methylamino group, N,N-dimethylamino group, 2-hydroxyethylamino group, or carboxyl group; and
Y is hydrogen atom, methyl group, or ethyl group.

20. The compound or salt thereof according to claim 1, wherein the bond between the ring-constituting carbon atom at the 1-position of the ring (E) and the ring-constituting carbon atom at the 2-position of the ring (E) is a single bond;
the bond between the ring-constituting carbon atom at the 1-position of the ring (E) and the Link moiety is a single bond;
Link is CH₂;
W is methylene group;
Rs is -D-Rx or -N(Ry)(Rz);
D is a single bond, or oxygen atom;
Rx is propyl group, isopropyl group, butyl group, isobutyl group, isopentyl group, 2-ethylbutyl group, cyclopentyl group, cyclohexyl group, cycloheptyl group, cyclopentylmethyl group, cyclohexylmethyl group, cycloheptylmethyl group, phenyl group, 2-methylphenyl group, 4-methylphenyl group, 2-fluorophenyl group, 3-fluorophenyl group, 4-fluorophenyl group, 2-chlorophenyl group, 3-chlorophenyl group, 4-chlorophenyl group, 2-thienyl group, 3-thienyl group, 2-furyl group, 3-furyl group, indan-2-yl group, 4-methylindan-2-yl group, 5-methylindan-2-yl group, 4,7-dimethylindan-2-yl group, 5,6-dimethylindan-2-yl group, 4-fluoroindan-2-yl group, 5-fluoroindan-2-yl group, 4,7-difluoroindan-2-yl group, 5,6-difluoroindan-2-yl group, 4-chloroindan-2-yl group, 5-chloroindan-2-yl group, 4,7-dichloroindan-2-yl group, 5,6-dichloroindan-2-yl group, 4-hydroxyindan-2-yl group, 5-hydroxyindan-2-yl group, 4,7-dihydroxyindan-2-yl group, 5,6-dihydroxyindan-2-yl group, 4-methoxyindan-2-yl group, 5-methoxyindan-2-yl group, 4,7-dimethoxyindan-2-yl group, 5,6-dimethoxyindan-2-yl group, 1-phenylethyl group, 1-(2-fluorophenyl)ethyl group, 1-(3-fluorophenyl)ethyl group, 1-(4-fluorophenyl)ethyl group, 1-(2-chlorophenyl)ethyl group, 1-(3-chlorophenyl)ethyl group, 1-(4-chlorophenyl)ethyl group, 1-(2-methoxyphenyl)ethyl group, 1-(3-methoxyphenyl)ethyl group, 1-(4-methoxyphenyl)ethyl group, benzyl group, 2-methylphenylmethyl group, 3-methylphenylmethyl group, 4-methylphenylmethyl group, 2,3-dimethylphenylmethyl group, 3,5-dimethylphenylmethyl group, 2-fluorophenylmethyl group, 3-fluorophenylmethyl group, 4-fluorophenylmethyl group, 2-chlorophenylmethyl group, 3-chlorophenylmethyl group, 4-chlorophenylmethyl group, 2,3-difluorophenylmethyl group, 2,4-difluorophenylmethyl group, 2,5-difluorophenylmethyl group, 3,4-difluorophenylmethyl group, 2,3-dichlorophenylmethyl group, 2,4-dichlorophenylmethyl group, 2,5-dichlorophenylmethyl group, 2,6-dichlorophenylmethyl group, 3,4-dichlorophenylmethyl group, 3,5-dichlorophenylmethyl group, 3,6-dichlorophenylmethyl group, 2-(trifluoromethyl)phenylmethyl group, 3-(trifluoromethyl)phenylmethyl group, 4-(trifluoromethyl)phenylmethyl group, 2-hydroxyphenylmethyl group, 3-hydroxyphenylmethyl group, 4-hydroxyphenylmethyl group, 2-methoxyphenylmethyl group, 3-methoxyphenylmethyl group, 4-methoxyphenylmethyl group, 2-(2-methylphenyl)ethyl group, 2-(3-methylphenyl)ethyl group, 2-(4-methylphenyl)ethyl group, 2-(2-methoxyphenyl)ethyl group, 2-(3-methoxyphenyl)ethyl group, 2-(4-methoxyphenyl)ethyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-[2-(trifluoromethyl)phenyl]ethyl group, 2-[3-(trifluoromethyl)phenyl]ethyl group, 2-[4-(trifluoromethyl)phenyl]ethyl group, 2-[4-(N,N-dimethylamino)phenyl]ethyl group, 2-phenyloxyethyl group, 2-(2-chlorophenyloxy)ethyl group, 2-(3-chlorophenyloxy)ethyl group, 2-(4-chlorophenyloxy)ethyl group, 2-(phenylthio)ethyl group, 2-(N-phenyl-N-methylamino)ethyl group, or 2-(N-ethyl-N-phenylamino)ethyl group;
the substituent -N(Ry)(Rz) is N,N-dimethylamino group, N-ethyl-N-methylamino group, N,N-diethylamino group, N-methyl-N-propylamino group, N-ethyl-N-propylamino group, N-isopropyl-N-methylamino group, N-ethyl-N-isopropylamino group, N-butylamino group, N-butyl-N-methylamino group, N-butyl-N-ethylamino group, N-isobutylamino group, N-isobutyl-N-methylamino group, N-ethyl-N-isobutylamino group, N-isopentylamino group, N-isopentyl-N-methylamino group, N-ethyl-N-isopentylamino group, N-(2-ethylbutyl)amino-group, N-(2-ethylbutyl)-N-methylamino group, N-cyclopentylamino group, N-cyclopentyl-N-methylamino group, N-cyclohexylamino group, N-cyclohexyl-N-methylamino group, N-cycloheptylamino group, N-(cyclopentylmethyl)amino group, N-(cyclopentylmethyl)-N-methylamino group, N-(cyclohexylmethyl)amino group, N-(cyclohexylmethyl)-N-methylamino group, N-phenylamino group, N-(2-methylphenyl)amino group, N-(4-methylphenyl)amino group, N-(2-fluorophenyl)amino group, N-(3-fluorophenyl)amino group, N-(4-fluorophenyl)amino group, N-(2-chlorophenyl)amino group, N-(3-chlorophenyl)amino group, N-(4-chlorophenyl)amino group, N-(indan-2-yl)amino group, N-(1-phenylethyl)amino group, N-[1-(2-fluorophenyl)ethyl]amino group, N-[1-(3-fluorophenyl)ethyl]amino group, N-[1-(4-fluorophenyl)ethyl]amino group, N-[1-(2-chlorophenyl)ethyl]amino group, N-[1-(3-chlorophenyl)ethyl]amino group, N-[1-(4-chlorophenyl)ethyl]amino group, N-benzylamino group, N-benzyl-N-methylamino group, N-benzyl-N-ethylamino group, N-(2-methylphenylmethyl)amino group, N-methyl-N-(2-methylphenylmethyl)amino group, N-(3-methylphenylmethyl)amino group, N-methyl-N-(3-methylphenylmethyl)amino group, N-(4-methylphenylmethyl)amino group, N-methyl-N-(4-methylphenylmethyl)amino group, N-(2-fluorophenylmethyl)amino group, N-(2-fluorophenylmethyl)-N-methylamino group, N-(3-fluorophenylmethyl)amino group, N-(3-fluorophenylmethyl)-N-methylamino group, N-(4-fluorophenylmethyl)amino group, N-(4-fluorophenylmethyl)-N-methylamino group, N-(2-chlorophenylmethyl)amino group, N-(2-chlorophenylmethyl)-N-methylamino group, N-(3-chlorophenylmethyl)amino group, N-(3-chlorophenylmethyl)-N-methylamino group, N-(4-chlorophenylmethyl)amino group, N-(4-chlorophenylmethyl)-N-methylamino group, N-(2,3-difluorophenylmethyl)amino group, N-(2,3-difluorophenylmethyl)-N-methylamino group, N-(2,4-difluorophenylmethyl)amino group, N-(2,4-difluorophenylmethyl)-N-methylamino group, N-(2,5-difluorophenylmethyl)amino group, N-(2,5-difluorophenylmethyl)-N-methylamino group, N-(3,4-difluorophenylmethyl)amino group, N-(3,4-difluorophenylmethyl)-N-methylamino group, N-(3,5-difluorophenylmethyl)amino group, N-(3,5-difluorophenylmethyl)-N-methylamino group, N-(2,3-dichlorophenylmethyl)amino group, N-(2,3-dichlorophenylmethyl)-N-methylamino group, N-(2,4-dichlorophenylmethyl)amino group, N-(2,4-dichlorophenylmethyl)-N-methylamino group, N-(2,5-dichlorophenylmethyl)amino group, N-(2,5-dichlorophenylmethyl)-N-methylamino group, N-(2,6-dichlorophenylmethyl)amino group, N-(2,6-dichlorophenylmethyl)-N-methylamino group, N-(3,4-dichlorophenylmethyl)amino group, N-(3,4-dichlorophenylmethyl)-N-methylamino group, N-(3,5-dichlorophenylmethyl)amino group, N-(3,5-dichlorophenylmethyl)-N-methylamino group, N-[2-(trifluoromethyl)phenylmethyl]amino group, N-methyl-N-[2-(trifluoromethyl)phenylmethyl]amino group, N-[3-(trifluoromethyl)phenylmethyl]amino group, N-methyl-N-[3-(trifluoromethyl)phenylmethyl]amino group, N-[4-(trifluoromethyl)phenylmethyl]amino group, N-methyl-N-[4-(trifluoromethyl)phenylmethyl]amino group, pyrrolidino group, 2-methylpyrrolidino group, 3-methylpyrrolidino group, 2,5-dimethylpyrrolidino group, 3,4-dimethylpyrrolidino group, piperidino group, 2-methylpiperidino group, 3-methylpiperidino group, 4-methylpiperidino group, 4-ethylpiperidino group, 4-propylpiperidino group, 4-isopropylpiperidino group, 4-butylpiperidino group, 4-isobutylpiperidino group, 4-cyclopentylpiperidino group, 4-cyclohexylpiperidino group, 4-(cyclopentylmethyl)piperidino group, 4-(cyclohexylmethyl)piperidino group, 4-phenylpiperidino group, 4-(2-methylphenyl)piperidino group, 4-(4-methylphenyl)piperidino group, 4-(2-fluorophenyl)piperidino group, 4-(3-fluorophenyl)piperidino group, 4-(4-fluorophenyl)piperidino group, 4-(2-chlorophenyl)piperidino group, 4-(3-chlorophenyl)piperidino group, 4-(4-chlorophenyl)piperidino group, 4-benzylpiperidino group, 4-(2-methylphenylmethyl)piperidino group, 4-(3-methylphenylmethyl)piperidino group, 4-methylphenylmethyl)piperidino group, 4-(2,3-dimethylphenylmethyl)piperidino group, 4-(3,5-dimethylphenylmethyl)piperidino group, 4-(2-fluorophenylmethyl)piperidino group, 4-(3-fluorophenylmethyl)piperidino group, 4-(4-fluorophenylmethyl)piperidino group, 4-(2-chlorophenylmethyl)piperidino group, 4-(3-chlorophenylmethyl)piperidino group, 4-(4-chlorophenylmethyl)piperidino group, 4-(2,3-difluorophenylmethyl)piperidino group, 4-(2,4-difluorophenylmethyl)piperidino group, 4-(2,5-difluorophenylmethyl)piperidino group, 4-(3,4-difluorophenylmethyl)piperidino group, 4-(2,3-dichlorophenylmethyl)piperidino group, 4-(2,4-dichlorophenylmethyl)piperidino group, 4-(2,5-dichlorophenylmethyl)piperidino group, 4-(2,6-dichlorophenylmethyl)piperidino group, 4-(3,4-dichlorophenylmethyl)piperidino group, 4-(3,5-dichlorophenylmethyl)piperidino group, 4-(3,6-dichlorophenylmethyl)piperidino group, 4-(2-methoxyphenylmethyl)piperidino group, 4-(3-methoxyphenylmethyl)piperidino group, 4-(4-methoxyphenylmethyl)piperidino group, 2,6-dimethylpiperidino group, 3,5-dimethylpiperidino group, 4,4-dimethylpiperidino group, homopiperidino group, morpholino group, 3,5-dimethylmorpholino group, 4-phenylpiperazino group, 4-(2-methylphenyl)piperazino group, 4-(4-methylphenyl)piperazino group, 4-(2-fluorophenyl)piperazino group, 4-(3-fluorophenyl)piperazino group, 4-(4-fluorophenyl)piperazino group, 4-(2-chlorophenyl)piperazino group, 4-(3-chlorophenyl)piperazino group, 4-(4-chlorophenyl)piperazino group, 4-benzylpiperazino group, 4-(2-methylphenylmethyl)piperazino group, 4-(3-methylphenylmethyl)piperazino group, 4-methylphenylmethyl)piperazino group, 4-(2,3-dimethylphenylmethyl)piperazino group, 4-(3,5-dimethylphenylmethyl)piperazino group, 4-(2-fluorophenylmethyl)piperazino group, 4-(3-fluorophenylmethyl)piperazino group, 4-(4-fluorophenylmethyl)piperazino group, 4-(2-chlorophenylmethyl)piperazino group, 4-(3-chlorophenylmethyl)piperazino group, 4-(4-chlorophenylmethyl)piperazino group, 4-(2,3-difluorophenylmethyl)piperazino group, 4-(2,4-difluorophenylmethyl)piperazino group, 4-(2,5-difluorophenylmethyl)piperazino group, 4-(3,4-difluorophenylmethyl)piperazino group, 4-(2,3-dichlorophenylmethyl)piperazino group, 4-(2,4-dichlorophenylmethyl)piperazino group, 4-(2,5-dichlorophenylmethyl)piperazino group, 4-(2,6-dichlorophenylmethyl)piperazino group, 4-(3,4-dichlorophenylmethyl)piperazino group, 4-(3,5-dichlorophenylmethyl)piperazino group, 4-(3,6-dichlorophenylmethyl)piperazino group, 4-(2-methoxyphenylmethyl)piperazino group, 4-(3-methoxyphenylmethyl)piperazino group, or 4-(4-methoxyphenylmethyl)piperazino group;
V¹ is Zx, and V² is AR;
Zx is hydrogen atom, fluorine atom, chlorine atom, bromine atom, methyl group, hydroxyl group, amino group, or N-methylamino group;
AR is naphthalen-2-yl group, 6-hydroxynaphthalen-2-yl group, 6-methoxynaphthalen-2-yl group, 6-aminonaphthalen-2-yl group, 6-(N,N-dimethylamino)naphthalen-2-yl group, benzo[b]furan-5-yl group, benzo[b]thiophen-5-yl group, 1H-indol-5-yl group, 1-methyl-1H-indol-5-yl group, 1-ethyl-1H-indol-5-yl group, benzothiazol-6-yl group, 2-aminobenzothiazol-6-yl group, 2-oxo-2,3-dihydrobenzothiazol-6-yl group, quinolin-3-yl group, quinolin-6-yl group, 2-oxo-1,2-dihydroquinolin-6-yl group, 1H-indazol-5-yl group, 1-methyl-1H-indazol-5-yl group, 1-ethyl-1H-indazol-5-yl group, isoquinolin-6-yl group, 1-oxo-1,2-dihydroisoquinolin-6-yl group, cinnolin-6-yl group, or benzoxazol-5-yl group; and
Y is hydrogen atom, methyl group, or ethyl group.

21. A medicament comprising the compound or pharmacologically acceptable salt thereof according to any one of claims 1 to 20 as an active ingredient.

22. An agent for suppressing production of a prostaglandin and/or a leukotriene, which comprises the compound or pharmacologically acceptable salt thereof according to any one of claims 1 to 20 as an active ingredient.

23. The medicament according to claim 21, which is for prophylactic and/or therapeutic treatment of a disease induced by production of a prostaglandin and/or a leukotriene.

24. The medicament according to claim 21, which is for prophylactic and/or therapeutic treatment of an inflammatory disease of mammal.

25. The medicament according to claim 21, which is for prophylactic and/or therapeutic treatment of an autoimmune disease of mammal.

26. The medicament according to claim 21, which is for prophylactic and/or therapeutic treatment of an allergic disease of mammal.

27. The medicament according to claim 21, which is for antipyresis and/or analgesis of mammal.

28. A pharmaceutical composition for prophylactic and/or therapeutic treatment of a biological condition of mammal in which an acute or chronic inflammatory reaction is observed, which comprises a prophylactically and/or therapeutically effective amount of the compound or pharmaceutically acceptable salt thereof according to any one of claims 1 to 20 and a pharmaceutically acceptable carrier.

29. A method for prophylactic and/or therapeutic treatment of a biological condition of a mammal in which an acute or chronic inflammatory reaction is observed, which comprises administering a prophylactically and/or therapeutically effective amount of the compound or pharmaceutically acceptable salt thereof according to any one of claims 1 to 20 to the mammal.
